# EUROPEAN PATENT APPLICATION

(11) **EP 1 352 962 A1**
(43) Date of publication of application: **15.10.2003**
(21) Application number: 03006291.3
(22) Date of filing: 06.03.1998
(51) Int. Cl.: C12N 15/12, C12N 5/10, C12N 1/21, C07K 14/47, C07K 16/18, C12Q 1/68, G01N 33/50, G01N 33/53, G01N 33/68, A61K 38/17

(54) **186 human secreted proteins**

(30) Priority: 02.10.1997 US 61060 P; 07.03.1997 US 40162 P; 07.03.1997 US 40333 P; 07.03.1997 US 38621 P; 07.03.1997 US 40161 P; 07.03.1997 US 40626 P; 07.03.1997 US 40334 P; 07.03.1997 US 40336 P; 07.03.1997 US 40163 P; 23.05.1997 US 47600 P; 23.05.1997 US 47615 P; 23.05.1997 US 47597 P; 23.05.1997 US 47502 P; 23.05.1997 US 47633 P; 23.05.1997 US 47583 P; 23.05.1997 US 47617 P; 23.05.1997 US 47618 P; 23.05.1997 US 47503 P; 23.05.1997 US 47592 P; 23.05.1997 US 47581 P; 23.05.1997 US 47584 P; 23.05.1997 US 47500 P; 23.05.1997 US 47587 P; 23.05.1997 US 47492 P; 23.05.1997 US 47598 P; 23.05.1997 US 47613 P; 23.05.1997 US 47582 P; 23.05.1997 US 47596 P; 23.05.1997 US 47612 P; 23.05.1997 US 47632 P; 23.05.1997 US 47601 P; 11.04.1997 US 43580 P; 11.04.1997 US 43568 P; 11.04.1997 US 43314 P; 11.04.1997 US 43569 P; 11.04.1997 US 43311 P; 11.04.1997 US 43671 P; 11.04.1997 US 43674 P; 11.04.1997 US 43669 P; 11.04.1997 US 43312 P; 11.04.1997 US 43313 P; 11.04.1997 US 43672 P; 11.04.1997 US 43315 P; 06.06.1997 US 48974 P; 22.08.1997 US 56886 P; 22.08.1997 US 56877 P; 22.08.1997 US 56889 P; 22.08.1997 US 56893 P; 22.08.1997 US 56630 P; 22.08.1997 US 56878 P; 22.08.1997 US 56662 P; 22.08.1997 US 56872 P; 22.08.1997 US 56882 P; 22.08.1997 US 56637 P; 22.08.1997 US 56903 P; 22.08.1997 US 56888 P; 22.08.1997 US 56879 P; 22.08.1997 US 56880 P; 22.08.1997 US 56894 P; 22.08.1997 US 56911 P; 22.08.1997 US 56636 P; 22.08.1997 US 56874 P; 22.08.1997 US 56910 P; 22.08.1997 US 56864 P; 22.08.1997 US 56631 P; 22.08.1997 US 56845; 22.08.1997 US 56892 P; 05.09.1997 US 57761 P; 23.05.1997 US 47595 P; 23.05.1997 US 47599 P; 23.05.1997 US 47588 P; 23.05.1997 US 47585 P; 23.05.1997 US 47586 P; 23.05.1997 US 47590 P; 23.05.1997 US 47594 P; 23.05.1997 US 47589 P; 23.05.1997 US 47593 P; 23.05.1997 US 47614 P; 11.04.1997 US 43578 P; 11.04.1997 US 43576 P; 23.05.1997 US 47501 P; 11.04.1997 US 43670 P; 22.08.1997 US 56632 P; 22.08.1997 US 56664 P; 22.08.1997 US 56876 P; 22.08.1997 US 56881 P; 22.08.1997 US 56909 P; 22.08.1997 US 56875 P; 22.08.1997 US 56862 P; 22.08.1997 US 56887 P; 22.08.1997 US 56908 P; 06.06.1997 US 48964 P; 05.09.1997 US 57650 P; 22.08.1997 US 56884 P; 13.06.1997 US 49610 P; 08.07.1997 US 51926 P; 16.07.1997 US 52874 P; 18.08.1997 US 55724 P; 05.09.1997 US 57669 P; 12.09.1997 US 58785 P
(62) Divisional of application: 98905127.1
(71) Applicant: HUMAN GENOME SCIENCES, INC., Rockville, MD 20850-3338 (US)
(72) Inventor: Olsen, Henrik S., Gaithersburg, MD 207878 (US); Yu, Guo-Liang, Berkeley, CA 94705 (US); Endress, Gregory A., Florence, MA 01062 (US); Bednarik, Daniel P., Columbia, MD 21046 (US); Carter, Kenneth C., North Potomac, MD 20878 (US); Feng, Ping, Germantown, MD 20874 (US); Soppet, Daniel R., Centreville, VA 20120 (US); Young, Paul E., Gaithersburg, MD 20878 (US); Duan, D. Roxanne, Gaithersburg, MD 20878 (US); Florence, Kimberly A., Rockville, MD 20851 (US); Greene, John M., Gaithersburg, MD 20878 (US); Fischer, Carrie L., Burke, VA 22015 (US); Hu, Jing-Shan, Mountain View, CA 94040 (US); Ruben, Steven M., Brookeville, MD 20833 (US); Ebner, Reinhard, Gaithersburg, MD 20878 (US); Brewer, Laurie A., St. Paul, MN 55119 (US); Ferrie, Ann M., Painted Post, NY 14870 (US); Moore, Paul A., Germantown, MD 20874 (US); Ni, Jian, Germantown, MD 20874 (US); Shi, Yanggu, Gaithersburg, MD 20878 (US); Lafleur, David W., Washington, DC 20015 (US); Li, Yi, Sunnyvale, CA 94086 (US); Zeng, Zhizhen, Lansdale, PA 19446 (US); Kyaw, Hla, Boonsboro, MD 21713 (US); Rosen, Craig A., Laytonsville, MD 20882 (US)
(74) Representative: VOSSIUS & PARTNER

(57) **Abstract**

The present invention relates to 186 novel human secreted proteins and isolated nucleic acids containing the coding regions of the genes encoding such proteins. Also provided are vectors, host cells, antibodies, and recombinant methods for producing human secreted proteins. The invention further relates to diagnostic and therapeutic methods useful for diagnosing and treating disorders related to these novel human secreted proteins.

## Description

### Field of the Invention

This invention relates to newly identified polynucleotides and the polypeptides encoded by these polynucleotides, uses of such polynucleotides and polypeptides, and their production.

### Background of the Invention

Unlike bacterium, which exist as a single compartment surrounded by a membrane, human cells and other eucaryotes are subdivided by membranes into many functionally distinct compartments. Each membrane-bounded compartment, or organelle, contains different proteins essential for the function of the organelle. The cell uses "sorting signals," which are amino acid motifs located within the protein, to target proteins to particular cellular organelles.

One type of sorting signal, called a signal sequence, a signal peptide, or a leader sequence, directs a class of proteins to an organelle called the endoplasmic reticulum (ER). The ER separates the membrane-bounded proteins from all other types of proteins. Once localized to the ER, both groups of proteins can be further directed to another organelle called the Golgi apparatus. Here, the Golgi distributes the proteins to vesicles, including secretory vesicles, the cell membrane, lysosomes, and the other organelles.

Proteins targeted to the ER by a signal sequence can be released into the extracellular space as a secreted protein. For example, vesicles containing secreted proteins can fuse with the cell membrane and release their contents into the extracellular space - a process called exocytosis. Exocytosis can occur constitutively or after receipt of a triggering signal. In the latter case, the proteins are stored in secretory vesicles (or secretory granules) until exocytosis is triggered. Similarly, proteins residing on the cell membrane can also be secreted into the extracellular space by proteolytic cleavage of a "linker" holding the protein to the membrane.

Despite the great progress made in recent years, only a small number of genes encoding human secreted proteins have been identified. These secreted proteins include the commercially valuable human insulin, interferon, Factor VIII, human growth hormone, tissue plasminogen activator, and erythropoeitin. Thus, in light of the pervasive role of secreted proteins in human physiology, a need exists for identifying and characterizing novel human secreted proteins and the genes that encode them. This knowledge will allow one to detect, to treat, and to prevent medical disorders by using secreted proteins or the genes that encode them.

### Summary of the Invention

The present invention relates to novel polynucleotides and the encoded polypeptides. Moreover, the present invention relates to vectors, host cells, antibodies, and recombinant methods for producing the polypeptides and polynucleotides. Also provided are diagnostic methods for detecting disorders related to the polypeptides, and therapeutic methods for treating such disorders. The invention further relates to screening methods for identifying binding partners of the polypeptides.

### Detailed Description

### Definitions

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide.

In the present invention, a "secreted" protein refers to those proteins capable of being directed to the ER, secretory vesicles, or the extracellular space as a result of a signal sequence, as well as those proteins released into the extracellular space without necessarily containing a signal sequence. If the secreted protein is released into the extracellular space, the secreted protein can undergo extracellular processing to produce a "mature" protein. Release into the extracellular space can occur by many mechanisms, including exocytosis and proteolytic cleavage.

As used herein, a "polynucleotide" refers to a molecule having a nucleic acid sequence contained in SEQ ID NO:X or the cDNA contained within the clone deposited with the ATCC. For example, the polynucleotide can contain the nucleotide sequence of the full length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, a "polypeptide" refers to a molecule having the translated amino acid sequence generated from the polynucleotide as broadly defined.

In the present invention, the full length sequence identified as SEQ ID NO:X was often generated by overlapping sequences contained in multiple clones (contig analysis). A representative clone containing all or most of the sequence for SEQ ID NO:X was deposited with the American Type Culture Collection ("ATCC"). As shown in Table 1, each clone is identified by a cDNA Clone ID (Identifier) and the ATCC Deposit Number. The ATCC is located at 12301 Park Lawn Drive, Rockville, Maryland 20852, USA. The ATCC deposit was made pursuant to the terms of the Budapest Treaty on the international recognition of the deposit of microorganisms for purposes of patent procedure.

A "polynucleotide" of the present invention also includes those polynucleotides capable of hybridizing, under stringent hybridization conditions, to sequences contained in SEQ ID NO:X, the complement thereof, or the cDNA contained within the clone deposited with the ATCC. "Stringent hybridization conditions" refers to an overnight incubation at 42° C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C.

Also contemplated are nucleic acid molecules that hybridize to the polynucleotides of the present invention at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO₄; 0.02M EDTA, pH 7.4), 0.5% SDS, 30% formamide, 100 ug/ml salmon sperm blocking DNA; followed by washes at 50°C with 1XSSPE, 0.1% SDS. In addition, to achieve even lower stringency, washes performed following stringent hybridization can be done at higher salt concentrations (e.g. 5X SSC).

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Of course, a polynucleotide which hybridizes only to polyA+ sequences (such as any 3' terminal polyA+ tract of a cDNA shown in the sequence listing), or to a complementary stretch of T (or U) residues, would not be included in the definition of "polynucleotide," since such a polynucleotide would hybridize to any nucleic acid molecule containing a poly (A) stretch or the complement thereof (e.g., practically any double-stranded cDNA clone).

The polynucleotide of the present invention can be composed of any polyribonucleotide or polydeoxribonucleotide, which may be unmodified RNA or DNA or modified RNA or DNA. For example, polynucleotides can be composed of single-and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

The polypeptide of the present invention can be composed of amino acids joined to each other by peptide bonds or modified peptide bonds, i.e., peptide isosteres, and may contain amino acids other than the 20 gene-encoded amino acids. The polypeptides may be modified by either natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched , for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

"SEQ ID NO:X" refers to a polynucleotide sequence while "SEQ ID NO:Y" refers to a polypeptide sequence, both sequences identified by an integer specified in Table 1.

"A polypeptide having biological activity" refers to polypeptides exhibiting activity similar, but not necessarily identical to, an activity of a polypeptide of the present invention, including mature forms, as measured in a particular biological assay, with or without dose dependency. In the case where dose dependency does exist, it need not be identical to that of the polypeptide, but rather substantially similar to the dose-dependence in a given activity as compared to the polypeptide of the present invention (i.e., the candidate polypeptide will exhibit greater activity or not more than about 25-fold less and, preferably, not more than about tenfold less activity, and most preferably, not more than about three-fold less activity relative to the polypeptide of the present invention.)

### Polynucleotides and Polypeptides of the Invention

### FEATURES OF PROTEIN ENCODED BY GENE NO: 1

This gene is expressed primarily in testes tumor and to a lesser extent in fetal brain.

Therefore, polynucleotides and polypeptides of the invention are useful as reagents for differential identification of the tissue(s) or cell type(s) present in a biological sample and for diagnosis of diseases and conditions which include, but are not limited to, cancer particularly of the testes, and defects of the central nervous system such as seizure and neurodegenerative disorders. Similarly, polypeptides and antibodies directed to these polypeptides are useful in providing immunological probes for differential identification of the tissue(s) or cell type(s). For a number of disorders of the above tissues or cells, particularly cancer of the testes and central nervous system, expression of this gene at significantly higher or lower levels may be routinely detected in certain tissues and cell types (e.g., testes and other reproductive tissue, brain and other tissue of the nervous system, and blood cells, and spleen, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment/diagnosis of testicular cancer and treatment of central nervous system disorders since this gene is primarily expressed in the testes tumor and developing brain.

### FEATURES OF PROTEIN ENCODED BY GENE NO: 2

This gene is expressed primarily in cancer tissues, such as breast cancer and Wilm's tumor, and to a lesser extent in fetal tissues.

Therefore, polynucleotides and polypeptides of the invention are useful as reagents for differential identification of the tissue(s) or cell type(s) present in a biological sample and for diagnosis of diseases and conditions which include, but are not limited to, and/or tumors, particularly, those found in the breast, and developmental abnormalities or disorders. Similarly, polypeptides and antibodies directed to these polypeptides are useful in providing immunological probes for differential identification of the tissue(s) or cell type(s). For a number of disorders of the above tissues or cells, particularly of the glandular tissues, expression of this gene at significantly higher or lower levels may be routinely detected in certain tissues and cell types (e.g., mammary tissue, and fetal tissue and, cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 314 as residues: Pro-11 to Thr-18, Leu-43 to Pro-50, Gly-64 to Leu-72, and Leu-81 to Lys-86.

The tissue distribution indicates that polynucleotides and polypeptides corresponding to this gene are useful for treatment/diagnosis of cancers and/or tumors, particularly, those found in the breast since expression is mainly in cancer/tumor tissues. May serve as therapeutic proteins for proliferation/differentiation of fetal tissues.

### FEATURES OF PROTEIN ENCODED BY GENE NO: 3

This gene is expressed primarily in CD34 depleted huffy coat and to a lesser extent in spleen, chronic lymphocytic leukemia.

Therefore, polynucleotides and polypeptides of the invention are useful as reagents for differential identification of the tissue(s) or cell type(s) present in a biological sample and for diagnosis of diseases and conditions: blood disorders or leukemias, diseases of the immune system. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., blood cells, and spleen, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment/diagnosis of blood disorders or leukemias, diseases of the immune system since expression is in tissues related to immune function.

### FEATURES OF PROTEIN ENCODED BY GENE NO: 4

This gene is expressed primarily in CD34 depleted buffy coat.

Therefore, polynucleotides and polypeptides of the invention are useful as reagents for differential identification of the tissue(s) or cell type(s) present in a biological sample and for diagnosis of diseases and conditions: blood disorders or lymphocytic diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment/diagnosis of blood disorders since expression is in tissues related to immune function.

### FEATURES OF PROTEIN ENCODED BY GENE NO: 5

This gene is expressed primarily in CD34 depleted buffy coat.

Therefore, polynucleotides and polypeptides of the invention are useful as reagents for differential identification of the tissue(s) or cell type(s) present in a biological sample and for diagnosis of diseases and conditions: blood or immune diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 317 as residues: Pro-13 to Lys-21.

The tissue distribution indicates that polynucleotides and polypeptides corresponding to this gene are useful for treatment/diagnosis of blood disorders since expression is in tissues related to immune function.

### FEATURES OF PROTEIN ENCODED BY GENE NO: 6

This gene is expressed primarily in CD34 depleted buffy coat.

Therefore, polynucleotides and polypeptides of the invention are useful as reagents for differential identification of the tissue(s) or cell type(s) present in a biological sample and for diagnosis of diseases and conditions: blood or immune diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 318 as residues: Lys-31 to Lys-39.

The tissue distribution indicates that polynucleotides and polypeptides corresponding to this gene are useful for treatment/diagnosis of blood diseases since it is expressed in tissues related to immune function.

### FEATURES OF PROTEIN ENCODED BY GENE NO: 7

This gene is expressed primarily in CD34 depleted buffy coat and to a lesser extent in pineal gland.

Therefore, polynucleotides and polypeptides of the invention are useful as reagents for differential identification of the tissue(s) or cell type(s) present in a biological sample and for diagnosis of diseases and conditions: diseases of the immune system and brain associated diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., blood cells, and pineal gland, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment/diagnosis of blood disorders, immune diseases or brain associated diseases (specifically of the pineal gland) since expression is in tissues related to immune function.

### FEATURES OF PROTEIN ENCODED BY GENE NO: 8

The translation product of this gene shares sequence homology with an organic cation transporter which is thought to be important in organic cation uptake in the kidney and liver. (See Accession No. 2343059.) Preferred polypeptide fragments comprise the amino acid sequence ITIAIQMICLVNXELYPTFVRNXGVMVCSSLCDIGGIITP FIVFRLREVWQALPLILFAVLGLLAAGVTLLLPETKGVALPETMKDAENLGRKAKPKENTIYLK VQTSEPSGT (SEQ ID NO: 615) or TMKDAENLGRKAKPKENT (SEQ ID NO: 616) as well as N-terminal and C-terminal deletions of these fragments. Also preferred are polynucleotide fragments encoding these polypeptide fragments.

This gene is expressed primarily in liver.

Therefore, polynucleotides and polypeptides of the invention are useful as reagents for differential identification of the tissue(s) or cell type(s) present in a biological sample and for diagnosis of diseases and conditions: hepatic and renal diseases where drug elimination/cation exchange (organic cation uptake) in the liver and kidney are problematic. Similarly, polypeptides and antibodies directed to these polypeptides are useful in providing immunological probes for differential identification of the tissue(s) or cell type(s). For a number of disorders of the above tissues or cells, particularly of the hepatic or renal system, expression of this gene at significantly higher or lower levels may be routinely detected in certain tissues and cell types (e.g., kidney and liver, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 320 as residues: Asn-64 to Asn-74, and Gln-81 to Gly-87.

The tissue distribution and homology to organic cation transporter indicate that polynucleotides and polypeptides corresponding to this gene are useful as a polyspecific transporter that is important for drug elimination in the liver (and possibly kidney) since expression is found in the liver.

### FEATURES OF PROTEIN ENCODED BY GENE NO: 9

This gene is expressed primarily in eosinophil induced with IL-5 and to a lesser extent in fetal liver and spleen. This gene also maps to chromosome 15, and therefore can be used in linkage analysis as a marker for chromosome 15.

Therefore, polynucleotides and polypeptides of the invention are useful as reagents for differential identification of the tissue(s) or cell type(s) present in a biological sample and for diagnosis of diseases and conditions: diseases of the immune system, particularly allergies or asthma. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., blood cells, liver, and spleen, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating/diagnosis of diseases involving esosinphil reactions since expression seems to be concentrated in eosinophils and other tissues involved in immunity such as the liver and spleen.

### FEATURES OF PROTEIN ENCODED BY GENE NO: 10

This gene is expressed primarily in tissues of hematopoietic lineage and to a lesser extent in Hodgkins lymphoma. Any frame shifts in this sequence can easily be clarified using known molecular biology techniques.

Therefore, polynucleotides and polypeptides of the invention are useful as reagents for differential identification of the tissue(s) or cell type(s) present in a biological sample and for diagnosis of diseases and conditions which include, but are not limited to, and immune deficiency or dysfunction. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., hematopoietic cells, lymphoid and reticuloendothelial tissues, and cancerous tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment/ diagnosis for lymphomas or immune dysfuction or as a therapeutic protein useful in immune modulation based on expression in anergic T-cells and lymphomas.

### FEATURES OF PROTEIN ENCODED BY GENE NO: 11

This gene is expressed primarily in neutrophils and to a lesser extent in activated lymphoid cells.

Therefore, polynucleotides and polypeptides of the invention are useful as reagents for differential identification of the cell type present in a biological sample and for diagnosis of diseases and conditions: inflamation. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., blood cells and lymphoid tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 323 as residues: Glu-40 to Lys-46.

The tissue distribution indicates that polynucleotides and polypeptides corresponding to this gene are useful for modulation of an immune reaction or as a growth factor for the differentiation or proliferation of neutrphils for the treatment of neutropenia.

### FEATURES OF PROTEIN ENCODED BY GENE NO: 12

This gene is expressed primarily in brain and to a lesser extent in activated T-cells. It is likely that the open reading frame containing the predicted signal peptide continues in the 5' direction. Preferred polypeptide fragments comprise the amino acid sequence PRVRNSPEDLGLSLTGDSCKL (SEQ ID NO:617).

Therefore, polynucleotides and polypeptides of the invention are useful as reagents for differential identification of the tissue(s) or cell type(s) present in a biological sample and for diagnosis of diseases and conditions: neurodegenerative disorders including ischemic shock, alzheimers and cognitive disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues and cell types (e.g., blood cells, and brain, and other tissue of the nervous system and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 324 as residues: Ser-5 to Glu-14, Ile-21 to Pro-35, Ser-65 to Asp-81, Cys-89 to Val-96, Lys-136 to Ser-145, Ile-152 to Met-169, and Arg-189 to Lys-196.

The tissue distribution indicates that polynucleotides and polypeptides corresponding to this gene are useful for diagnostic/treatment for cancers of the given tissue or in the treatment of neurological disorders of the CNS.

### FEATURES OF PROTEIN ENCODED BY GENE NO: 13

This gene was also recently cloned by other groups, naming this calcium-activated potassium channel gene, hKCa4. (See Accession No. AF033021, see also, Accession No. 2584866.) This gene is mapped to human chromosome 19q13.2. A second signal sequence likely exists upstream from the predicted signal sequence as described in Table 1. Preferred polypeptide fragments comprise: QADDLQATVAALCVLRGGGPWAG SWLSPKTPGAMGGDLVLGLGALRRRKRLL (SEQ NO: 618); or EQEKSLAGWALVLAXXGIGL MVLHAEMLWFGGCSAVNATGHLSDTLWLIPITFLTIGYGDVVPGTMWGKIVCLCTGVMGVCC TALLVAVVARKLEFNKAEKHVHNFMMDIQYTKEMKESAARVLQEAWMFYKHTRRKESHAAR XHQRXLLAAINAFRQVRLKHRKLREQVNSMVDISKMHMILYDLQQNLSSSHRALEKQIDTLAG KLDALTELLSTALGPRQLPEPSQQSK (SEQ ID NO: 619), as well as N-terminal and C-terminal deletions. Also preferred are polynulcleotide fragments encoding these polypeptide fragments.

This gene is expressed primarily in breast lymph node and T-cells, and to a lesser extent in placenta.

Therefore, polynucleotides and polypeptides of the invention are useful as reagents for differential identification of the tissue(s) or cell type(s) present in a biological sample and for diagnosis of diseases and conditions: hematologic and immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., lymphoid tissue, blood cells and placenta, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 325 as residues: Arg-13 to Lys-23.

The tissue distribution indicates that polynucleotides and polypeptides corresponding to this gene are useful for the treatment/diagnosis of hematologic and diseases involving immune modulation based or distribution in the lymph node and T-cells.

### FEATURES OF PROTEIN ENCODED BY GENE NO: 14

This gene was recently cloned by another group, calling it PAPS synethase. (See Accession No. e1204135.) Preferred polypeptide fragments comprise the amino acid sequence YQAHHVSRNKRGQVVGTRGGFRGCTVWLTGLSGAGK (SEQ ID NO: 620). Also preferred are the polynucleotide fragments encoding this polypeptide fragment.

It has been discovered that this gene is expressed primarily in benign prostate hyperplasia, Human Umbilical Vein Endothelial Cells and to a lesser extent in smooth muscle and Human endometrial stromal cells-treated with estradiol.

Therefore, polynucleotides and polypeptides of the invention are useful as reagents for differential identification of the tissue(s) or cell type(s) present in a biological sample and for diagnosis of diseases and conditions: inflamation, ischemia, and restenosis, based on endothelial cell and smooth muscle cell expression, and prostate diseases such as benign prostate hyperplasia or prostate cancer. Similarly, polypeptides and antibodies directed to these polypeptides are useful in providing immunological probes for differential identification of the tissue(s) or cell type(s). For a number of disorders of the above tissues or cells, particularly of the prostate or vessels of the circulatory system, expression of this gene at significantly higher or lower levels may be routinely detected in certain tissues and cell types (e.g., prostate, endothelial cells, smooth muscle, and endometrium, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 326 as residues: Arg-21 to Asp-26, Lys-35 to Lys-44, Glu-49 to Asn-58.

The tissue distribution indicates that polynucleotides and polypeptides corresponding to this gene are useful for treating/diagnosing diseases or conditions where the endothelial cell lining of the veins and arteries of underlying smooth muscle are involved.

### FEATURES OF PROTEIN ENCODED BY GENE NO: 15

This gene is expressed primarily in human 6 week embryo and to a lesser extent in placenta.

Therefore, polynucleotides and polypeptides of the invention are useful as reagents for differential identification of the tissue(s) or cell type(s) present in a biological sample and for diagnosis of diseases and conditions: developmental anomalies or fetal deficiencies. Similarly, polypeptides and antibodies directed to these polypeptides are useful in providing immunological probes for differential identification of the tissue(s) or cell type(s). For a number of disorders of the above tissues or cells, particularly developmental in nature, expression of this gene at significantly higher or lower levels may be routinely detected in certain tissues and cell types (e.g., embryonic tissue, and placenta, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 327 as residues Lys-50 to Glu-57.

The tissue distribution indicates that polynucleotides and polypeptides corresponding to this gene are useful for detection of developmental abnormalities.

### FEATURES OF PROTEIN ENCODED BY GENE NO: 16

This gene is expressed primarily in kidney and amygdala and to a lesser extent in fetal tissues. This gene is mapped to chromosome 14, and therefore is useful in linkage analysis as a marker for chromosome 14.

Therefore, polynucleotides and polypeptides of the invention are useful as reagents for differential identification of the tissue(s) present in a biological sample and for diagnosis of diseases and conditions: kidney diseases, neurological disorders and developmental abnormalities. Similarly, polypeptides and antibodies directed to these polypeptides are useful in providing immunological probes for differential identification of the tissue(s). For a number of disorders of the above tissues, particularly of the renal system or developing fetal tissues, expression of this gene at significantly higher or lower levels may be routinely detected in certain tissues and cell types (e.g., kidney, amygdala, and fetal tissues, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment or diagnosis of conditions affecting the brain, kidneys and fetal development.

### FEATURES OF PROTEIN ENCODED BY GENE NO: 17

This gene is expressed primarily in ovarian cancer.

Therefore, polynucleotides and polypeptides of the invention are useful as reagents for differential identification of the tissue(s) or cell type(s) present in a biological sample and for diagnosis of diseases and conditions: solid tumors similar to ovarian cancer Similarly, polypeptides and antibodies directed to these polypeptides are useful in providing immunological probes for differential identification of the tissue(s) or cell type(s). For a number of disorders of the above tissues or cells, particularly of the reproductive system. expression of this gene at significantly higher or lower levels may be routinely detected in certain tissues and cell types (e.g., ovarian and other reproductive tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 329 as residues Ser-51 to Val-56.

The tissue distribution indicates that polynucleotides and polypeptides corresponding to this gene are useful for the treatment of solid tumors of the reproductive system such as ovarian cancer.

### FEATURES OF PROTEIN ENCODED BY GENE NO: 18

This gene is expressed primarily in brain medulloblastoma. Preferred polypeptide fragments comprise the amino acid sequence: IRHEQHPNFSLEMHSKGSSLLLFLPQL ILILPVCAHLHEELNC (SEQ ID NO: 643) and SFFISEEKGHLLLQAERHPWVAGALVGVSG GLTLTTCSGPTEKPATKNYFLKRLLQEMHIRAN (SEQ ID NO: 644), as well as N-terminal and C-terminal deletions. Also preferred are polynucleotide fragments encoding these polypeptide fragments.

Therefore, polynucleotides and polypeptides of the invention are useful as reagents for differential identification of the tissue(s) or cell type(s) present in a biological sample and for diagnosis of diseases and conditions: tumors particularly of the CNS or Similarly, polypeptides and antibodies directed to these polypeptides are useful in providing immunological probes for differential identification of the tissue(s) or cell type(s). For a number of disorders of the above tissues or cells, particularly of the Central nervous system, expression of this gene at significantly higher or lower levels may be routinely detected in certain tissues and cell types (e.g., brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating medulloblastoma or similar tumors.

### FEATURES OF PROTEIN ENCODED BY GENE NO: 19

This gene is expressed primarily in adipocytes.

Therefore, polynucleotides and polypeptides of the invention are useful as reagents for differential identification of the tissue(s) or cell type(s) present in a biological sample and for diagnosis of diseases and conditions: obesity. Similarly, polypeptides and antibodies directed to these polypeptides are useful in providing immunological probes for differential identification of the tissue(s) or cell type(s). For a number of disorders of the above tissues or cells, particularly of the adipose tissues expression of this gene at significantly higher or lower levels may be routinely detected in certain tissues and cell types (e.g., adipocytes and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating obesity by regulating the function and number of adipocytes

### FEATURES OF PROTEIN ENCODED BY GENE NO: 20

This gene is expressed primarily in B cell lymphoma.

Therefore, polynucleotides and polypeptides of the invention are useful as reagents for differential identification of the tissue(s) or cell type(s) present in a biological sample and for diagnosis of diseases and conditions which include, but are not limited to, of the immune system with an emphasis on B cell lymphoma. Similarly, polypeptides and antibodies directed to these polypeptides are useful in providing immunological probes for differential identification of the tissue(s) or cell type(s). For a number of disorders of the above tissues or cells, particularly of the tumors of the immune system, expression of this gene at significantly higher or lower levels may be routinely detected in certain tissues and cell types (e.g., blood cells, and lymphoid tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of B cell derived tumors based on its expression in b cell lymphomas

### FEATURES OF PROTEIN ENCODED BY GENE NO: 21

This gene is expressed primarily in immune cells and to a lesser extent in fetal tissues

Therefore, polynucleotides and polypeptides of the invention are useful as reagents for differential identification of the tissue(s) or cell type(s) present in a biological sample and for diagnosis of diseases and conditions: inflammatory diseases Similarly, polypeptides and antibodies directed to these polypeptides are useful in providing immunological probes for differential identification of the tissue(s) or cell type(s). For a number of disorders of the above tissues or cells, particularly of the immune expression of this gene at significantly higher or lower levels may be routinely detected in certain tissues and cell types (e.g., cells of the immune system, and fetal tissues, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:333 as residues Asp-10 to Pro-19, Ser-74 to Tyr-79, Glu-95 to Lys-110.

The tissue distribution indicates that polynucleotides and polypeptides corresponding to this gene are useful for treatment of diseases involving alterations in T cell activity.

### FEATURES OF PROTEIN ENCODED BY GENE NO: 22

It has been discovered that this gene is expressed primarily in ovarian tumor.

Therefore, polynucleotides and polypeptides of the invention are useful as reagents for differential identification of the tissue(s) or cell type(s) present in a biological sample and for diagnosis of diseases and conditions: tumors particularly of the ovary. Similarly, polypeptides and antibodies directed to these polypeptides are useful in providing immunological probes for differential identification of the tissue(s) or cell type(s). For a number of disorders of the above tissues or cells, particularly of tumors of the reproductive organs, expression of this gene at significantly higher or lower levels may be routinely detected in certain tissues and cell types (e.g., ovarian and other reproductive tissue and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO. 334 as residues: Leu-22 to Gln-27.

The tissue distribution indicates that polynucleotides and polypeptides corresponding to this gene are useful for diagnosis and treatment of ovarian tumors as it has only been identified in ovarian tumors.

### FEATURES OF PROTEIN ENCODED BY GENE NO: 23

It has been discovered that this gene is expressed primarily in fetal tissues and to a lesser extent in osteoclastoma cell line

Therefore, polynucleotides and polypeptides of the invention are useful as reagents for differential identification of the tissue(s) or cell type(s) present in a biological sample and for diagnosis of diseases and conditions: osteoporosis or arthritis Similarly, polypeptides and antibodies directed to these polypeptides are useful in providing immunological probes for differential identification of the tissue(s) or cell type(s). For a number of disorders of the above tissues or cells, particularly of the skeletal expression of this gene at significantly higher or lower levels may be routinely detected in certain tissues and cell types (e.g., bone cells, and fetal tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment of conditions of abnormal bone remodeling due to enhanced activity of osteoclasts. This may be useful as a specific marker for malignancies derived from osteoclasts or their precursors.

### FEATURES OF PROTEIN ENCODED BY GENE NO: 24

The translation product of this gene shares sequence homology with a periplasmic ribonuclease which is thought to be important in degrading extracellular polynucleotides

It has been discovered that this gene is expressed primarily in serum treated smooth muscle cells

Therefore, polynucleotides and polypeptides of the invention are useful as reagents for differential identification of the tissue(s) or cell type(s) present in a biological sample and for diagnosis of diseases and conditions: vascular disease such as restenosis. Similarly, polypeptides and antibodies directed to these polypeptides are useful in providing immunological probes for differential identification of the tissue(s) or cell type(s). For a number of disorders of the above tissues or cells, particularly of the vasculature expression of this gene at significantly higher or lower levels may be routinely detected in certain tissues and cell types (e.g., smooth muscle, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 336 as residues: Gln-30 to Lys-36, and Pro-41 to Arg-48.

The tissue distribution and homology to ribonucleases indicate that polynucleotides and polypeptides corresponding to this gene are useful for treatment of pathological conditions of smooth muscle associated with bacterial or viral infiltration

### FEATURES OF PROTEIN ENCODED BY GENE NO: 25

This gene is expressed primarily in Early Stage Human Brain.

Therefore, polynucleotides and polypeptides of the invention are useful as reagents for differential identification of the tissue(s) or cell type(s) present in a biological sample and for diagnosis of diseases and conditions: human brain development and related diseases. Similarly, polypeptides and antibodies directed to these polypeptides are useful in providing immunological probes for differential identification of the tissue(s) or cell type(s). For a number of disorders of the above tissues or cells, particularly of the human brain development and related diseases, expression of this gene at significantly higher or lower levels may be routinely detected in certain tissues and cell types (e.g., brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to this gene indicate that polynucleotides and polypeptides corresponding to this gene are useful for diagnosis and treatment of diseases affecting human brain development and related diseases.

### FEATURES OF PROTEIN ENCODED BY GENE NO: 26

It has been discovered that this gene is expressed primarily in human brain tissue.

Therefore, polynucleotides and polypeptides of the invention are useful as reagents for differential identification of the tissue(s) or cell type(s) present in a biological sample and for diagnosis of diseases and conditions: human brain diseases and other diseases related to brain diseases, which may be caused by brain diseases. Similarly, polypeptides and antibodies directed to these polypeptides are useful in providing immunological probes for differential identification of the tissue(s) or cell type(s). For a number of disorders of the above tissues or cells, particularly of the human brain diseases, expression of this gene at significantly higher or lower levels may be routinely detected in certain tissues and cell types (e.g., brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to the gene indicate that polynucleotides and polypeptides corresponding to this gene are useful for diagnosis and treatment of human brain diseases and other diseases related.

### FEATURES OF PROTEIN ENCODED BY GENE NO: 27

It has been discovered that this gene is expressed primarily in Anergic T-cells.

Therefore, polynucleotides and polypeptides of the invention are useful as reagents for differential identification of the tissue(s) or cell type(s) present in a biological sample and for diagnosis of diseases and conditions: immune diseases, inflammatory diseases and diseases related to T lymph cells. Similarly, polypeptides and antibodies directed to these polypeptides are useful in providing immunological probes for differential identification of the tissue(s) or cell type(s). For a number of disorders of the above tissues or cells, particularly of the immune diseases, inflammatory diseases and diseases related to T lymph cells, expression of this gene at significantly higher or lower levels may be routinely detected in certain tissues and cell types (e.g., blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to the gene indicate that polynucleotides and polypeptides corresponding to this gene are useful for immune diseases, inflammatory diseases and diseases related to T lymph cells.

### FEATURES OF PROTEIN ENCODED BY GENE NO: 28

The translation product of this gene shares sequence homology with *Shigella flexneri* positive transcriptional regulator CriR (criR) gene which is thought to be important in regulation of gene expression.

This gene is expressed primarily in human synovial sarcoma and normal human brain tissues.

Therefore, polynucleotides and polypeptides of the invention are useful as reagents for differential identification of the tissue(s) or cell type(s) present in a biological sample and for diagnosis of diseases and conditions: human brain diseases particularly sarcomas of the synovium. Similarly, polypeptides and antibodies directed to these polypeptides are useful in providing immunological probes for differential identification of the tissue(s) or cell type(s). For a number of disorders of the above tissues or cells, particularly of the human brain and synovium and other related human brain diseases, expression of this gene at significantly higher or lower levels may be routinely detected in certain (e.g., synovial tissue, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of human synovial sarcoma and other related human brain diseases.

### FEATURES OF PROTEIN ENCODED BY GENE NO: 29

This gene is expressed in bone marrow, infant brain, fetal liver and spleen, prostate and to a lesser extent in pineal gland, adipose tissue, kidney, adrenal gland, umbilical vein endothelial cells, and T cells.

Therefore, polynucleotides and polypeptides of the invention are useful as reagents for identification of the tissue(s) or cell type(s) present in a biological sample and for diagnosis of diseases and conditions: diseases related to bone marrow or hematoplastic tissues, prostate, kidney, adrenal gland, and cardiovascular tissue or organs. Similarly, polypeptides and antibodies directed to these polypeptides are useful in providing immunological probes for identification of the tissue(s) or cell type(s). For a number of disorders of the above tissues or cells, particularly of the diseases related to hematoplastic tissues, immune system, prostate, kidney, adrenal gland, and cardiovascular tissue or organs, expression of this gene at significantly higher or lower levels may be routinely detected in certain tissues and cell types (e.g., bone marrow, hematopoietic cells, pineal gland, adipose tissue, kidney, adrenal gland, endothelial cells, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to the gene indicate that polynucleotides and polypeptides corresponding to this gene are useful for diagnosis and treatment of diseases related to hematoplastic tissues, immune system, prostate, kidney, adrenal gland, and cardiovascular tissue or organs.

### FEATURES OF PROTEIN ENCODED BY GENE NO: 30

This gene is expressed primarily in meningea and to a lesser extent in breast and adult brain.

Therefore, polynucleotides and polypeptides of the invention are useful as reagents for differential identification of the tissue(s) or cell type(s) present in a biological sample and for diagnosis of diseases and conditions: Diseases of the meningea and related brain diseases. Similarly, polypeptides and antibodies directed to these polypeptides are useful in providing immunological probes for differential identification of the tissue(s) or cell type(s). For a number of disorders of the above tissues or cells, particularly of the meningea and related brain diseases, expression of this gene at significantly higher or lower levels may be routinely detected in certain tissues and cell types (e.g., miningea, mammary tissue, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of diseases of the meningea and related brain diseases.

### FEATURES OF PROTEIN ENCODED BY GENE NO: 31

This gene is expressed in meningea, fetal spleen, osteoblast and to a lesser extent in activated T-cells, endometrial stromal cells, fetal lung, HL-60, thymus, testis and endothelial cells.

Therefore, polynucleotides and polypeptides of the invention are useful as reagents for differential identification of the tissue(s) or cell type(s) present in a biological sample and for diagnosis of diseases and conditions: meningeal disease, osteoporosis, immune diseases, and hematoplastic diseases. Similarly, polypeptides and antibodies directed to these polypeptides are useful in providing immunological probes for identification of the tissue(s) or cell type(s). For a number of disorders of the above tissues or cells, particularly of the meningeal diseases, osteoporosis, immune diseases, and hematoplastic diseases, expression of this gene at significantly higher or lower levels may be routinely detected in certain tissues and cell types (e.g., blood cells, endometrium, lung, thymus, testis, and endothelial cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to gene indicate that polynucleotides and polypeptides corresponding to this gene are useful for diagnosis and treatment of meningeal, osteoporosis, immune diseases, hematoplastic diseases, testis diseases and lung diseases.

### FEATURES OF PROTEIN ENCODED BY GENE NO: 32

This gene is expressed primarily in human thymus and to a much lesser extent in infant brain, T-cells, smooth muscle, endothelial cells, bone marrow, human ovarian tumor and keratinocytes testes, osteoclastoma, breast, and tonsils.

Therefore, polynucleotides and polypeptides of the invention are useful as reagents for differential identification of the tissue(s) or cell type(s) present in a biological sample and for diagnosis of diseases and conditions: Diseases involving the thymus, particularly thymic cancer and diseases involving T-cell maturation. Similarly, polypeptides and antibodies directed to these polypeptides are useful in providing immunological probes for differential identification of the tissue(s) or cell type(s). For a number of disorders of the above tissues or cells, particularly of the thymus, expression of this gene at significantly higher or lower levels may be routinely detected in certain tissues and cell types (e.g., thymus, brain, and other tissue of the nervous system, blood cells, bone marrow, ovaries, and testes, and other reproductive tissue, mammary tissue, tonsils, melanocytes and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to gene indicate that polynucleotides and polypeptides corresponding to this gene are useful for diagnosis and treatment of diseases of the thymus particularly thymic cancer and diseases involving T-cell maturation.

### FEATURES OF PROTEIN ENCODED BY GENE NO: 33

This gene is expressed primarily in human tonsils, and placenta, and to a lesser extent in adipocytes, melanocyte, and infant brain.

Therefore, polynucleotides and polypeptides of the invention are useful as reagents for differential identification of the tissue(s) or cell type(s) present in a biological sample and for diagnosis of diseases and conditions: inflammatory diseases, immune diseases, and obesity. Similarly, polypeptides and antibodies directed to these polypeptides are useful in providing immunological probes for differential identification of the tissue(s) or cell type(s). For a number of disorders of the above tissues or cells, particularly of the inflammatory diseases, immune diseases, and obesity, expression of this gene at significantly higher or lower levels may be routinely detected in certain tissues and cell types (e.g., tonsils, placenta, adipocytes, melanocytes, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to this gene indicate that polynucleotides and polypeptides corresponding to this gene are useful for diagnosis and treatment of diseases such as inflammation, immune diseases, and obesity.

### FEATURES OF PROTEIN ENCODED BY GENE NO: 34

This gene is expressed in activated T cells, and to a lesser extent in pituitary, testis, and breast lymph node.

Therefore, polynucleotides and polypeptides of the invention are useful as reagents for differential identification of the tissue(s) or cell type(s) present in a biological sample and for diagnosis of diseases and conditions: diseases relating to T cells. Similarly, polypeptides and antibodies directed to these polypeptides are useful in providing immunological probes for differential identification of the tissue(s) or cell type(s). For a number of disorders of the above tissues or cells, particularly of the disorders of the immune system, expression of this gene at significantly higher or lower levels may be routinely detected in certain tissues and cell types (e.g., pituitary, testes and other reproductive tissue, mammary tissue, and lymphoid tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment of immune disorders.

### FEATURES OF PROTEIN ENCODED BY GENE NO: 35

This gene is expressed primarily in infant brain.

Therefore, polynucleotides and polypeptides of the invention are useful as reagents for differential identification of the tissue(s) or cell type(s) present in a biological sample and for diagnosis of diseases and conditions: neurological disorders. Similarly, polypeptides and antibodies directed to these polypeptides are useful in providing immunological probes for differential identification of the tissue(s) or cell type(s). For a number of disorders of the above tissues or cells, particularly of the diseases relating to neurological disorders, expression of this gene at significantly higher or lower levels may be routinely detected in certain tissues and cell types (e.g., brain, and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of neurological disorders.

### FEATURES OF PROTEIN ENCODED BY GENE NO: 36

This gene is expressed primarily in infant brain.

Therefore, polynucleotides and polypeptides of the invention are useful as reagents for differential identification of the tissue(s) or cell type(s) present in a biological sample and for diagnosis of diseases and conditions: neurological disorders. Similarly, polypeptides and antibodies directed to these polypeptides are useful in providing immunological probes for differential identification of the tissue(s) or cell type(s). For a number of disorders of the above tissues or cells, particularly of the diseases relating to neurological disorders, expression of this gene at significantly higher or lower levels may be routinely detected in certain tissues and cell types (e.g., brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of neurological disorders.

### FEATURES OF PROTEIN ENCODED BY GENE NO: 37

This gene is expressed primarily in human ovary.

Therefore, polynucleotides and polypeptides of the invention are useful as reagents for differential identification of the tissue(s) or cell type(s) present in a biological sample and for diagnosis of diseases and conditions: ovarian cancer. Similarly, polypeptides and antibodies directed to these polypeptides are useful in providing immunological probes for differential identification of the tissue(s) or cell type(s). For a number of disorders of the above tissues or cells, particularly of the ovarian disorders such as those involving germ cells, ovarian follicles, stromal cells, expression of this gene at significantly higher or lower levels may be routinely detected in certain tissues and cell types (e.g., ovary and other reproductive tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of ovariopathy.

### FEATURES OF PROTEIN ENCODED BY GENE NO: 38

This gene is expressed primarily in lymph node breast cancer.

Therefore, polynucleotides and polypeptides of the invention are useful as reagents for differential identification of the tissue(s) or cell type(s) present in a biological sample and for diagnosis of diseases and conditions: breast cancer. Similarly, polypeptides and antibodies directed to these polypeptides are useful in providing immunological probes for differential identification of the tissue(s) or cell type(s). For a number of disorders of the above tissues or cells, particularly of the breast cancer, expression of this gene at significantly higher or lower levels may be routinely detected in certain tissues and cell types (e.g., mammary tissue and lymphoid tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for used as a diagnostic marker for breast cancer.

### FEATURES OF PROTEIN ENCODED BY GENE NO: 39

This gene is expressed primarily in brain and to a lesser extent in other tissues.

Therefore, polynucleotides and polypeptides of the invention are useful as reagents for differential identification of the tissue(s) or cell type(s) present in a biological sample and for diagnosis of diseases and conditions: neuronal disorders such as trauma, brain degeneration, and brain tumor. Similarly, polypeptides and antibodies directed to these polypeptides are useful in providing immunological probes for differential identification of the tissue(s) or cell type(s). For a number of disorders of the above tissues or cells, particularly of the brain, expression of this gene at significantly higher or lower levels may be routinely detected in certain tissues and cell types (e.g., brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and therapeutic treatment of neuronal disorders.

### FEATURES OF PROTEIN ENCODED BY GENE NO: 40

This gene is expressed in early stage human embryo, adrenal gland tumor, and immune tissues such as fetal liver, fetal spleen, T-cell, and myoloid progenitor cell line and to a lesser extent in ovary, colon cancer, and a few orther tissues.

Therefore, polynucleotides and polypeptides of the invention are useful as reagents for differential identification of the tissue(s) or cell type(s) present in a biological sample and for diagnosis of diseases and conditions: tumorigenesis including adrenal gland tumor, colon cancer and various other tumors, developmental and immune disorders. Similarly, polypeptides and antibodies directed to these polypeptides are useful in providing immunological probes for differential identification of the tissue(s) or cell type(s). For a number of disorders of the above tissues or cells, particularly of the cancer tissues, early stage human tissues, and immune system, expression of this gene at significantly higher or lower levels may be routinely detected in certain tissues and cell types (e.g., liver, spleen, blood cells, bone marrow, ovary and other reproductive tissue, and colon, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and therapeutic treatment of immune and developmental disorders, and tumorigenesis.

### FEATURES OF PROTEIN ENCODED BY GENE NO: 41

This gene is expressed primarily in fetal lung, endothelial cells, liver, thymus and a few other immune tissues.

Therefore, polynucleotides and polypeptides of the invention are useful as reagents for differential identification of the tissue(s) or cell type(s) present in a biological sample and for diagnosis of diseases and conditions: immune disorders such as immune deficiency and autoimmune diseases, pulmonary diseases, liver diseases, and tumor matasis. Similarly, polypeptides and antibodies directed to these polypeptides are useful in providing immunological probes for differential identification of the tissue(s) or cell type(s). For a number of disorders of the above tissues or cells, particularly of the fetal lung, liver, endothelial cells, and immune tissues, expression of this gene at significantly higher or lower levels may be routinely detected in certain tissues and cell types (e.g., lung, endothelial cells, liver, thymus, and other tissue of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis of immune disorders and pulmonary and hepatic diseases. Its promoter may also be used for immune system and lung-specific gene therapies. The expression of this gene in endothelial cells indicates that it may also involve in angiogenesis which therefore may play role in tumor matasis.

### FEATURES OF PROTEIN ENCODED BY GENE NO: 42

This gene is expressed primarily in liver, thyroid, parathyroid and to a lesser extent in fetal lung, stomach and early embryos.

Therefore, polynucleotides and polypeptides of the invention are useful as reagents for differential identification of the tissue(s) or cell type(s) present in a biological sample and for diagnosis of diseases and conditions: metabolic regulation, obesity, heptic failure, heptacellular tumors or thyroiditis and thyroid tumors. Similarly, polypeptides and antibodies directed to these polypeptides are useful in providing immunological probes for differential identification of the tissue(s) or cell type(s). For a number of disorders of the above tissues or cells, particularly of the digestive/endocrine system expression of this gene at significantly higher or lower levels may be routinely detected in certain tissues and cell types (e.g., liver, thyroid, parathyroid, lung, stomach, and embryonic tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and the extracellular locations indicates that polynucleotides and polypeptides corresponding to this gene are useful for the detection and treatment of digestive/endocrine disorders, including metabolic regulation, heptic failure, malabsortion, gastritis and neoplasms.

### FEATURES OF PROTEIN ENCODED BY GENE NO: 43

This gene is expressed primarily in Schizophrenic adult brain, pituitary, front cortex, hypothalmus and to a lesser extent in retina, adipose and stomach cancer and placenta.

Therefore, polynucleotides and polypeptides of the invention are useful as reagents for differential identification of the tissue(s) or cell type(s) present in a biological sample and for diagnosis of diseases and conditions: schizophrenia and other neurological disorders. Similarly, polypeptides and antibodies directed to these polypeptides are useful in providing immunological probes for differential identification of the tissue(s) or cell type(s). For a number of disorders of the above tissues or cells, particularly of the central nerve system, expression of this gene at significantly higher or lower levels may be routinely detected in certain tissues and cell types (e.g., retinal tissue, adipose, stomach, and placenta, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful in treatment/detection of disorders in the nerve system, including schizophrenia, neurodegeneration, and neoplasia. Additionally, a secreted protein in brain may serve as an endocrine.

### FEATURES OF PROTEIN ENCODED BY GENE NO: 44

The translation product of this gene shares sequence homology with GTP binding proteins which are thought to be important in signal transduction and protein transport.

This gene is expressed primarily in umbilical vein and microvascular endothelial cells, GM-CSF treated macrophage, anergic T cells, osteoblast, osteoclast, CD34+ cells and to a lesser extent in gall bladder.

Therefore, polynucleotides and polypeptides of the invention are useful as reagents for differential identification of the tissue(s) or cell type(s) present in a biological sample and for diagnosis of diseases and conditions: bone formation and growth, osteonecrosis, osteoporosis, angiogenesis and/or hematopoeisis. Similarly, polypeptides and antibodies directed to these polypeptides are useful in providing immunological probes for differential identification of the tissue(s) or cell type(s). For a number of disorders of the above tissues or cells, particularly of the skeletal and hematopoeisis systems, expression of this gene at significantly higher or lower levels may be routinely detected in certain tissues and cell types (e.g., endothelial cells, blood cells, bone, and gall bladder, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to GTP binding proteins indicate that polynucleotides and polypeptides corresponding to this gene are useful for treatment/detection of bone formation and growth, osteonecrosis, osteoporosis, and/or hematopoeisis because its involvement in the growth signaling or angiogenesis.

### FEATURES OF PROTEIN ENCODED BY GENE NO: 45

The translation product of this gene shares sequence homology with signal sequence receptor gamma subunit which is thought to be important in protein translocation on endoplasmic reticulum.

This gene is expressed primarily in adrenal gland, salivary gland, prostate, and to a lesser extent in endothelial cells and smooth muscle.

Therefore, polynucleotides and polypeptides of the invention are useful as reagents for differential identification of the tissue(s) or cell type(s) present in a biological sample and for diagnosis of diseases and conditions: protein secretion. Similarly, polypeptides and antibodies directed to these polypeptides are useful in providing immunological probes for differential identification of the tissue(s) or cell type(s). For a number of disorders of the above tissues or cells, particularly of the secretory organs, expression of this gene at significantly higher or lower levels may be routinely detected in certain tissues and cell types (e.g., adrenal gland, salivary gland, prostate, endothelial cells, and smooth muscle, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to SSR gamma subunit indicate that polynucleotides and polypeptides corresponding to this gene are useful for endocrine disorders, prostate cancer, xerostomia or sialorrhea.

### FEATURES OF PROTEIN ENCODED BY GENE NO: 46

This gene is expressed primarily in osteoclastoma cells and to a lesser extent in melanocyte, amygdala, brain, and stomach.

Therefore, polynucleotides and polypeptides of the invention are useful as reagents for differential identification of the tissue(s) or cell type(s) present in a biological sample and for diagnosis of diseases and conditions: ossification, osteoporosis, fracture, osteonecrosis, osteosarcoma. Similarly, polypeptides and antibodies directed to these polypeptides are useful in providing immunological probes for differential identification of the tissue(s) or cell type(s). For a number of disorders of the above tissues or cells, particularly of the skeletal systems, expression of this gene at significantly higher or lower levels may be routinely detected in certain tissues and cell types (e.g., melanocytes, amygdala, brain and other tissue of the nervous system, and stomach, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful in intervention of ossification, osteoporosis, fracture, osteonecrosis and osteosarcoma.

### FEATURES OF PROTEIN ENCODED BY GENE NO: 48

The translation product of this gene shares sequence homology with proline rich proteins which is thought to be important in protein-protein interaction.

This gene is expressed primarily in brain.

Therefore, polynucleotides and polypeptides of the invention are useful as reagents for differential identification of the tissue(s) or cell type(s) present in a biological sample and for diagnosis of diseases and conditions: neurological and psychological disorders. Similarly, polypeptides and antibodies directed to these polypeptides are useful in providing immunological probes for differential identification of the tissue(s) or cell type(s). For a number of disorders of the above tissues or cells, particularly of the central nerve system and endocrine system, expression of this gene at significantly higher or lower levels may be routinely detected in certain tissues and cell types (e.g., brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to proline-rich proteins indicate that polynucleotides and polypeptides corresponding to this gene are useful in intervention and detection of neurological diseases, including trauma, neoplasia, degenerative or metabolic conditions in the central nerve system. Additionally, the gene product may be a secreted by the brain as an endocrine.

### FEATURES OF PROTEIN ENCODED BY GENE NO: 49

The translation product of this gene shares sequence homology with the AOCB gene from Aspergillus nidulans which is important in asexual development.

This gene is expressed primarily in infant brain and to a lesser extent in the developing embryo, trachea tumors, B-cell lymphoma and synovial sarcoma.

Therefore, polynucleotides and polypeptides of the invention are useful as reagents for differential identification of the tissue(s) or cell type(s) present in a biological sample and for diagnosis of diseases and conditions: neurodegenerative diseases, leukemia and sarcoma's. Similarly, polypeptides and antibodies directed to these polypeptides are useful in providing immunological probes for differential identification of the tissue(s) or cell type(s). For a number of disorders of the above tissues or cells, particularly of the brain and immune system, expression of this gene at significantly higher or lower levels may be routinely detected in certain tissues and cell types (e.g., embryonic tissue, blood cells, trachea, and synovial tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in infant brain and sarcoma's and homology to a gene involved in a key step of eukaryotivc development (fungal spore formation) indicates that the protein product of this clone could play a role in neurological diseases such as schizophrenia, particularly in infants. The existence of the gene in a B-cell lymphoma indicates the gene may be used in the treatment and detection of leukemia.

### FEATURES OF PROTEIN ENCODED BY GENE NO: 50

This gene is expressed primarily in fetal lung.

Therefore, polynucleotides and polypeptides of the invention are useful as reagents for differential identification of the tissue(s) or cell type(s) present in a biological sample and for diagnosis of diseases and conditions: pulmonary disorders including lung cancer. Similarly, polypeptides and antibodies directed to these polypeptides are useful in providing immunological probes for differential identification of the tissue(s) or cell type(s). For a number of disorders of the above tissues or cells, particularly of the pulmonary system, expression of this gene at significantly higher or lower levels may be routinely detected in certain tissues and cell types (e.g., lung, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution of this gene only in fetal lung indicates that it plays a key role in development of the pulmonary system. This would suggest that misregulation of the expression of this protein product in the adult could lead to lymphoma or sarcoma formation, particularly in the lung. It may also be involved in predisposition to certain pulmonary defects such as pulmonary edema and embolism, bronchitis and cystic fibrosis.

### FEATURES OF PROTEIN ENCODED BY GENE NO: 51

This gene is expressed primarily in hematopoietic cell types and fetal cells and to a lesser extent in all tissue types.

Therefore, polynucleotides and polypeptides of the invention are useful as reagents for differential identification of the tissue(s) or cell type(s) present in a biological sample and for diagnosis of diseases and conditions: defects in the immune system and hematopoeisis. Similarly, polypeptides and antibodies directed to these polypeptides are useful in providing immunological probes for differential identification of the tissue(s) or cell type(s). For a number of disorders of the above tissues or cells, particularly of the immune and hematopoietic systems, expression of this gene at significantly higher or lower levels may be routinely detected in certain tissues and cell types (e.g., hematopoietic cells, and fetal tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution of this gene predominantly in hematopoeitic cells and in the developing embryo indicates that polynucleotides and polypeptides corresponding to this gene are useful for detection and treatment of lymphomas and disease states affecting the immune system or hematopoeisis disorders such as leukemia, AIDS, arthritis and asthma..

### FEATURES OF PROTEIN ENCODED BY GENE NO: 52

This gene is expressed primarily in prostate and to a lesser extent in fetal spleen, fetal liver, infant brain and T cell leukemias.

Therefore, polynucleotides and polypeptides of the invention are useful as reagents for differential identification of the tissue(s) or cell type(s) present in a biological sample and for diagnosis of diseases and conditions: prostate disorders, prostate cancer, leukemia. Similarly, polypeptides and antibodies directed to these polypeptides are useful in providing immunological probes for differential identification of the tissue(s) or cell type(s). For a number of disorders of the above tissues or cells, particularly of the immune system, and/or prostate gland expression of this gene at significantly higher or lower levels may be routinely detected in certain tissues and cell types (e.g., thymus, spleen, liver, brain and other tissue of the nervous system, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution of this gene in prostate indicates that polynucleotides and polypeptides corresponding to this gene are useful for detection or treatment of prostate disorders or prostate cancer. Its distribution in fetal liver and fetal spleen indicates it may play a role in the immune system and its misregulation could lead to immune disorders such as leukemia, arthritis and asthma.

### FEATURES OF PROTEIN ENCODED BY GENE NO: 53

The translation product of this gene shares sequence homology with dynein.

This gene is expressed primarily in brain.

Therefore, polynucleotides and polypeptides of the invention are useful as reagents for differential identification of the tissue(s) or cell type(s) present in a biological sample and for diagnosis of diseases and conditions: neuro-degenerative diseases of the brain. Similarly, polypeptides and antibodies directed to these polypeptides are useful in providing immunological probes for differential identification of the tissue(s) or cell type(s). For a number of disorders of the above tissues or cells, particularly neuro-degenerative diseases expression of this gene at significantly higher or lower levels may be routinely detected in certain tissues and cell types (e.g., brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The predominant tissue distribution in the brain and homology to dynein, a microtubule motor protein involved in the positioning of cellular organelles and molecules indicates that polynucleotides and polypeptides corresponding to this gene are useful for detection/treatment of neurodegenerative diseases, such as Alzheimers, Huntigtons, Parkinsons diseases and shizophrenia.

### FEATURES OF PROTEIN ENCODED BY GENE NO: 54

The translation product of this gene shares sequence homology with ubiquitin-conjugation protein, an enzyme which is thought to be important in the processing of the Huntingtons Disease causing gene.

This gene is expressed primarily in brain and to a lesser extent in activated macrophages.

Therefore, polynucleotides and polypeptides of the invention are useful as reagents for differential identification of the tissue(s) or cell type(s) present in a biological sample and for diagnosis of diseases and conditions: neurodegenerative disease states including Huntington's disease. Similarly, polypeptides and antibodies directed to these polypeptides are useful in providing immunological probes for differential identification of brain tissues. For a number of disorders of the above tissues or cells, particularly of the neurological systems expression of this gene at significantly higher or lower levels may be routinely detected in certain tissues and cell types (e.g., brain and other tissue of the nervous system, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The predominant tissue distribution of this gene in the brain and its homology to a Huntington interacting protein indicates that polynucleotides and polypeptides corresponding to this gene are useful for the regulation of the expression of the Huntington disease gene and other neurodegenerative diseases including spinocerebullar ataxia types I and III, dentatorubropallidoluysian and spinal bulbar muscular atrophy. In addition, the existence of elevated levels of free ubiquitin pools in Alzheimer's disease, Parkinson's disease and amylotrophic lateral sclerosis indicates that the ubiquitin pathway of protein degradation plays a role in these disease states. Thus, considering the gene described here is homologous to a ubiquitin-conjugation protein it may play a general role in neurodegenarative conditions.

### FEATURES OF PROTEIN ENCODED BY GENE NO: 56

This gene is expressed primarily in T-cells (anergic T-cells, resting T-Cells, apoptotic T-cells) and lymph node (breast), as well as brain (hypothalamus, hippocampus, pituitary, infant brain, early-stage brain).

Therefore, polynucleotides and polypeptides of the invention are useful as reagents for differential identification of the tissue(s) or cell type(s) present in a biological sample and for diagnosis of diseases and conditions: immune (e.g. immunodeficiencies, autoimmunities, inflammation, leukemias & lymphomas) and neurological (e.g. Alzheimer's disease, dementia, schizophrenia) disorders. Similarly, polypeptides and antibodies directed to these polypeptides are useful in providing immunological probes for differential identification of the tissue(s) or cell type(s). For a number of disorders of the above tissues or cells, particularly of the central nervous, hematopoietic and immune systems, expression of this gene at significantly higher or lower levels may be routinely detected in certain tissues and cell types (e.g., blood cells, lymphoid tissue, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful in the intervention or detection of pathologies associated with the hematopoietic and immune systems, such as anemias (leukemias). In addition, the expression in brain (including fetal) might suggest a role in developmental brain defects, neuro-degenerative diseases or behavioral abnomalities (e.g. schizophrenia, Alzheimer's, dementia, depression, etc.).

### FEATURES OF PROTEIN ENCODED BY GENE NO: 57

This gene is expressed primarily in lung, and to a lesser extent in a variety of other hematological cell types (e.g. Raji cells, bone marrow cell line, activated monocytes).

Therefore, polynucleotides and polypeptides of the invention are useful as reagents for differential identification of the tissue(s) or cell type(s) present in a biological sample and for diagnosis of diseases and conditions: pulmonary and/or hematological disfunction. Similarly, polypeptides and antibodies directed to these polypeptides are useful in providing immunological probes for differential identification of the tissue(s) or cell type(s). For a number of disorders of the above tissues or cells, particularly of the vasculo-pulmonary and hematopoietic systems, expression of this gene at significantly higher or lower levels may be routinely detected in certain tissues and cell types (e.g., lung and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful in the intervention and detection of pathologies associated with the vasculo-pulmonary system. In addition the expression of this gene in a variety of leukocytic cell types and a bone marrow cell line might suggest a role in hematopoietic and immune system disorders, such as leukemias & lymphomas, inflammation, immunodeficiencies and autoimmunities.

### FEATURES OF PROTEIN ENCODED BY GENE NO: 58

The translation product of this gene shares sequence homology with adenylate kinase isozyme 3 (gil163528 GTP:AMP phosphotransferase (EC 2.7.4.10) [Bos taurus]), which is thought to be important in catalyzing the phosphorylation of AMP to ADP in the presence of ATP or inorganic triphosphate.

This gene is expressed primarily in fetal liver, heart and placenta, and to a lesser extent in many other tissues.

Therefore, polynucleotides and polypeptides of the invention are useful as reagents for differential identification of the tissue(s) or cell type(s) present in a biological sample and for diagnosis of diseases and conditions: hepatic, cardiovascular or reproductive disorders. Similarly, polypeptides and antibodies directed to these polypeptides are useful in providing immunological probes for differential identification of the tissue(s) or cell type(s). For a number of disorders of the above tissues or cells, particularly of the hepatic, cardiovascular and reproductive systems, expression of this gene at significantly higher or lower levels may be routinely detected in certain tissues and cell types (e.g., liver, heart, and placenta, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of conditions related to hepatic function and pathogenesis, in particular, those dealing with liver development and the differentiation of hepatocyte progenitor cells.

### FEATURES OF PROTEIN ENCODED BY GENE NO: 59

This gene is expressed primarily in CD34 positive cells (Cord Blood).

Therefore, polynucleotides and polypeptides of the invention are useful as reagents for differential identification of the tissue(s) or cell type(s) present in a biological sample and for diagnosis of diseases and conditions: hematopoietic differentiation and immune disorders. Similarly, polypeptides and antibodies directed to these polypeptides are useful in providing immunological probes for differential identification of the tissue(s) or cell type(s). For a number of disorders of the above tissues or cells, particularly of hematopoietic and immune systems, expression of this gene at significantly higher or lower levels may be routinely detected in certain tissues and cell types (e.g., hematopoietic cells, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful in the detection and treatment of conditions associated with CD34-positive cells, and therefore as a marker for cell differentiation in hematapoiesis, as well as immunological disorders.

### FEATURES OF PROTEIN ENCODED BY GENE NO: 60

The translation product of the predicted open reading frame of this contig has sequence identity to the murine gene designated Insulin-Like Growth Factor-Binding Protein (IGFBP)-1 as described by Lee and colleagues (Hepatology 19 (3), 656-665 (1994)).

This gene is expressed exclusively in hemangiopericytoma.

Therefore, polynucleotides and polypeptides of the invention are useful as reagents for differential identification of the tissue(s) or cell type(s) present in a biological sample and for diagnosis of hemangiopericytoma and other pericyte or endothelial cell proliferative disorders. Similarly, polypeptides and antibodies directed to these polypeptides are useful in providing immunological probes for differential identification of the tissue(s) or cell type(s). For a number of disorders of the above tissues or cells, particularly of the circulatory and immune systems, expression of this gene at significantly higher or lower levels may routinely be detected in certain tissues and cell types (e.g., pericyte or endothelial cells, and liver, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

Polynucleotides and polypeptides corresponding to this gene are useful as cell growth regulators since IGFBP-1-like molecules function as modulators of insulin-like growth factor activity. In addition, since IGFBP-1 is expressed at high levels following hepatectomy and during fetal liver development, polynucleotides of the present invention may also be used for the diagnosis of developmental disorders. Further, polypeptides of the present invention may be used therapeutically to treat developmental liver disorders as well as to regulate hepatocyte and supporting cell growth following hepatectomy or to treat liver disorders.

The tissue distribution indicates that polynucleotides and polypeptides corresponding to this gene are useful for diagnosis and treatment of hemangiopericytoma and liver disorders.

### FEATURES OF PROTEIN ENCODED BY GENE NO: 61

This gene is expressed primarily in schizophrenic frontal cortex.

Therefore, polynucleotides and polypeptides of the invention are useful as reagents for differential identification of the tissue(s) or cell type(s) present in a biological sample and for diagnosis of diseases and conditions: nervous system and cognitive disorders. Similarly, polypeptides and antibodies directed to these polypeptides are useful in providing immunological probes for differential identification of the tissue(s) or cell type(s). For a number of disorders of the above tissues or cells, particularly of the frontal cortex and CNS expression of this gene at significantly higher or lower levels may be routinely detected in certain tissues and cell types (e.g., brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study, treatment and diagnosis of frontal cortex, neuro-degenerative and CNS disorders

### FEATURES OF PROTEIN ENCODED BY GENE NO: 62

This gene is expressed primarily in human adrenal gland tumor, and to a lesser extent in human kidney, medulla and adult pulmonary tissue.

Therefore, polynucleotides and polypeptides of the invention are useful as reagents for differential identification of the tissue(s) or cell type(s) present in a biological sample and for diagnosis of diseases and conditions: metabolic, endocrine disorders. Similarly, polypeptides and antibodies directed to these polypeptides are useful in providing immunological probes for differential identification of the tissue(s) or cell type(s). For a number of disorders of the above tissues or cells, particularly of the endocrine and nervous system disorders and neoplasia, expression of this gene at significantly higher or lower levels may be routinely detected in certain tissues and cell types (e.g., adrenal gland, kidney, brain and other tissue of the nervous system, pulmonary tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study, treatment and diagnosis of neurological and endocrine disorders including neoplasia.

### FEATURES OF PROTEIN ENCODED BY GENE NO: 63

This gene is expressed primarily in human adipocytes, and to a lesser extent in spleen, 12-week old human, and testes.

Therefore, polynucleotides and polypeptides of the invention are useful as reagents for differential identification of the tissue(s) or cell type(s) present in a biological sample and for diagnosis of diseases and conditions: immune, metabolic and growth disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., adipocytes, spleen, and testes and other reproductive tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study, diagnosis and treatment of immune, developmental and metabolic disorders.

### FEATURES OF PROTEIN ENCODED BY GENE NO: 64

One translated product of this clone is homologous to the mouse zinc finger protein PZF. (See Accession No. 453376; see also Gene 152 (2), 233-238 (1995).) Preferred polypeptide fragments correspond to the highly conserved domains shared between mouse and man. For example, preferred polypeptide fragments comprise the amino acid sequence: LQCEICGFTCRQKASLNWHMKKHDADSFYQFSCNICGKKFEKKDSVVAHKAKSH PEV (SEQ ID NO: 621); ITSTDILGTNPESLTQPSD (SEQ ID NO: 622); NSTSGECLLLEAEGM SKSY (SEQ ID NO: 623); CSGTERVSLMADGKIFVGSGSSGGTEGLVMNSDILGATTEVLIEDSD SAGP (SEQ ID NO: 624); IQYVRCEMEGCGTVLAHPRYLQHHIKYQHLLKKKYVCPHPSCGRLF RLQKQLLRHAKHHT (SEQ ID NO: 625); DQRDYICEYCARAFKSSHNLAVHRMIHTGEK (SEQ ID NO: 626); RSSRTSVSRHRDTENTRSSRSKTGSLQLICKSEPNTDQLDY (SEQ ID NO: 627); PFKDDPRDETYKPHLERETPKPRRKSG (SEQ ID NO: 630); QYVRCEMEGCGTVLAHPRYLQ HHIKYQHLLKKKYVCPHPSCGRLFRLQKQLLRHAKHHTD (SEQ ID NO: 629); or residues 151-182 of QRDYICEYCARAFKSSHNLAVHRMIHTGEKHY (SEQ ID NO: 628). Also preferred are polynucleotide fragments encoding these polypeptide fragments.

This gene is expressed primarily in Rhabdomyosarcoma, melanocyte and colon cancer tissue and to a lesser extent in smooth muscle, pancreatic tumor, and apoptotic T-cells.

Therefore, polynucleotides and polypeptides of the invention are useful as reagents for differential identification of the tissue(s) or cell type(s) present in a biological sample and for diagnosis of diseases and conditions which include, but are not limited to,. Similarly, polypeptides and antibodies directed to these polypeptides are useful in providing immunological probes for differential identification of the tissue(s) or cell type(s). For a number of disorders of the above tissues or cells, particularly of the immune and hemopoetic, expression of this gene at significantly higher or lower levels may be routinely detected in certain tissues and cell types (e.g., striated muscle, melanocytes, colon, smooth muscle, pancreas, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study, diagnosis and treatment of cancer and hemopoetic disorders.

### FEATURES OF PROTEIN ENCODED BY GENE NO: 65

This gene is expressed primarily in human adipose and salivary gland tissue and to a lesser extent in human bone marrow and fetal kidney.

Therefore, polynucleotides and polypeptides of the invention are useful as reagents for differential identification of the tissue(s) or cell type(s) present in a biological sample and for diagnosis of diseases and conditions: metabolic and immune disorders. Similarly, polypeptides and antibodies directed to these polypeptides are useful in providing immunological probes for differential identification of the tissue(s) or cell type(s). For a number of disorders of the above tissues or cells, particularly of the metabolic and hemopoetic systems, expression of this gene at significantly higher or lower levels may be routinely detected in certain tissues and cell types (e.g., adipose, salivary gland, bone marrow, and kidney, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study, diagnosis of metabolic and immune disorders.

### FEATURES OF PROTEIN ENCODED BY GENE NO: 66

This translated product of this gene was recently identified as oxytocinase splice variant 1. (See Accession Nos. 2209276 and d1010078.) Preferred polypeptide fragments comprise the amino acid sequence: EMFDSLSYFKGSSLLLMLKTYLSEDVFQHAVVLYLHN HSYASIQSDDLWDSFNEVTNQTLDVKRMMKTWTLQKGFPLVTVQKKGKELFIQQERFFLNMK PEIQPSDTRYM (SEQ ID NO: 631). Also preferred are polynucleotide fragments encoding this polypeptide fragment.

### FEATURES OF PROTEIN ENCODED BY GENE NO: 67

This gene is expressed primarily in hemopoetic cells, particularly apoptotic T-cells, and to lesser extent in primary dendritic cells and adipose tissue.

Therefore, polynucleotides and polypeptides of the invention are useful as reagents for differential identification of apoptotic T-cells, primary denritic cells, and adipose tissue present in a biological sample and for diagnosis of diseases and conditions: hemopoetic diseases including cancer and general immune disorders. Similarly, polypeptides and antibodies directed to these polypeptides are useful in providing immunological probes for differential identification of the tissue(s) or cell type(s). For a number of disorders of the above tissues or cells, particularly of the oral and intestinal mucosa as well as hemopoetie and immune systems, expression of this gene at significantly higher or lower levels may be routinely detected in certain tissues and cell types (e.g., hematopoietic cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment of diseases of the immune system, including cancer, hemopoetic and infectious diseases.

### FEATURES OF PROTEIN ENCODED BY GENE NO: 68

This gene is expressed primarily in kidney cortex and to a lesser extent in infant brain, heart, uterus, and blood.

Therefore, polynucleotides and polypeptides of the invention are useful as reagents for differential identification of kidney tissue present in a biological sample and for diagnosis of diseases and conditions: soft tissue cancer, inflammation, kidney fibrosis. Similarly, polypeptides and antibodies directed to these polypeptides are useful in providing immunological probes for differential identification of the tissue(s) or cell type(s). For a number of disorders of the above tissues or cells, particularly of the nervous and endocrines systems, expression of this gene at significantly higher or lower levels may be routinely detected in certain tissues and cell types (e.g., kidney, brain, and other nervous tissue, heart, uterus, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study and treatment of cancer and fibroses.

### FEATURES OF PROTEIN ENCODED BY GENE NO: 69

The translation product of this gene shares strong sequence homology with vertebrate and invertebrate protein tyrosine phosphatases.

This gene is expressed primarily in endometrial tumors, melanocytes, myeloid progenitors and to a lesser extent in infant brain, adipocytes, and several hematopoietic stem cells.

Therefore, polynucleotides and polypeptides of the invention are useful as reagents for differential identification of transformed hematopoietic and epithelial cells present in a biological sample and for diagnosis of diseases and conditions which include, but are not limited to, of skin and endometrium, leukemia. Similarly, polypeptides and antibodies directed to these polypeptides are useful in providing immunological probes for differential identification of the tissue(s) or cell type(s). For a number of disorders of the above tissues or cells, particularly of the nervous and hemopoietic systems, expression of this gene at significantly higher or lower levels may be routinely detected in certain tissues and cell types (e.g., endometrium, melanocytes, bone marrow, adipocytes, hematopoietic cells, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and sequence similarity with tyrosine phosphatases indicate that polynucleotides and polypeptides corresponding to this gene are useful for study and treatment of cancer and hematopoietic disorders.

### FEATURES OF PROTEIN ENCODED BY GENE NO: 70

This gene is expressed primarily in osteoclastoma, breast, and infant brain and to a lesser extent in various fetal and transformed bone, ovarian, and neuronal cells.

Therefore, polynucleotides and polypeptides of the invention are useful as reagents for differential identification of the tissue(s) or cell type(s) present in a biological sample and for diagnosis of diseases and conditions: degenerative conditions of the brain and skeleton. Similarly, polypeptides and antibodies directed to these polypeptides are useful in providing immunological probes for differential identification of the tissue(s) or cell type(s). For a number of disorders of the above tissues or cells, particularly of the nervous and skeletal system, expression of this gene at significantly higher or lower levels may be routinely detected in certain tissues and cell types (e.g., bone, mammary tissue, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study and treatment of degenerative, neurological and skeletal disorders.

### FEATURES OF PROTEIN ENCODED BY GENE NO: 71

This gene was originally cloned from tumor cell lines. Recently another group has also cloned this gene, calling it the human malignant melanoma metastasis-suppressor (KiSS-1) gene. (See Accession No. U43527.) Preferred polypeptide fragments comprise the amino acid sequence: LEKVASVGNSRPTGQQLESLGLLA (SEQ ID NO: 632); VHREEASCYCQAEPSGDL (SEQ ID NO: 633); RPALRQAGGGTREPRQKRWAGL (SEQ ID NO: 634); and AVNFRPQRSQSM (SEQ ID NO: 635). Any frame shifts can easily be resolved using known molecular biology techniques.

This gene is expressed primarily in many types of carcinomas and to a lesser extent in many normal organs.

Therefore, polynucleotides and polypeptides of the invention are useful as reagents for differential identification of the tissues(s) or cell type(s) present in a biological sample and for diagnosis of diseases and conditions which include, but are not limited to, cancer particularly melanomas, and other hyperproliferative disorders. Similarly, polypeptides and antibodies directed to these polypeptides are useful in providing immunological probes for differential identification of the tissue(s) or cell type(s). For a number of disorders of the above tissues or cells, particularly of transformed organ tissue, expression of this gene at significantly higher or lower levels may be routinely detected in certain tissues and cell types (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. As a tumor suppressor gene, increase amounts of the polypeptide can be used to treat patients having a particular cancer.

The tissue distribution indicates that this gene and the translated product is useful for diagnosing and study of cancer.

### FEATURES OF PROTEIN ENCODED BY GENE NO: 72

This gene is expressed primarily in striatum and to a lesser extent in adipocytes and hemangioperiocytoma.

Therefore, polynucleotides and polypeptides of the invention are useful as reagents for differential identification of striatal cells present in a biological sample and for diagnosis of diseases and conditions: neurological, fat and lysosomal storage diseases. Similarly, polypeptides and antibodies directed to these polypeptides are useful in providing immunological probes for differential identification of the tissue(s) or cell type(s). For a number of disorders of the above tissues or cells, particularly of the nervous and immune systems, expression of this gene at significantly higher or lower levels may be routinely detected in certain tissues and cell types (e.g., striatal tissue, adipocytes, and vascular tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis, study and treatment of neurodegenerative and growth disorders.

### FEATURES OF PROTEIN ENCODED BY GENE NO: 73

This gene is expressed primarily in bone marrow stromal cells and to a lesser extent in smooth muscle, testes, endothelium, and brain.

Therefore, polynucleotides and polypeptides of the invention are useful as reagents for differential identification of bone marrow present in a biological sample and for diagnosis of diseases and conditions: connective tissue and hematopoietic diseases. Similarly, polypeptides and antibodies directed to these polypeptides are useful in providing immunological probes for differential identification of the tissue(s) or cell type(s). For a number of disorders of the above tissues or cells, particularly of the skeletal and hematopoietic systems, expression of this gene at significantly higher or lower levels may be routinely detected in certain tissues and cell types (e.g., bone marrow, stromal cells, smooth muscle, testes and other reproductive tissue, endothelium, brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study, diagnosis, and treatment of connective tissue and blood diseases.

### FEATURES OF PROTEIN ENCODED BY GENE NO: 74

This gene is expressed primarily in brain, fetal liver and lung and to a lesser extent in retina, spinal chord, activated T-cells and endothelial cells.

Therefore, polynucleotides and polypeptides of the invention are useful as reagents for differential identification of brain and regenerating liver present in a biological sample and for diagnosis of diseases and conditions: CNS and spinal chord injuries, immune disorders. Similarly, polypeptides and antibodies directed to these polypeptides are useful in providing immunological probes for differential identification of the tissue(s) or cell type(s). For a number of disorders of the above tissues or cells, particularly of the nervous and immune system, expression of this gene at significantly higher or lower levels may be routinely detected in certain tissues and cell types (e.g., brain and other tissue of the nervous system, liver, pulmonary tissue, blood cells, and endothelial cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study and treatment of hematopoietic and neurological conditions.

### FEATURES OF PROTEIN ENCODED BY GENE NO: 75

The translation product of this gene shares sequence homology with GTP binding proteins (intracellular).

This gene is expressed primarily in bone marrow, brain, and melanocytes and to a lesser extent in various endocrine and hematopoietic tissues.

Therefore, polynucleotides and polypeptides of the invention are useful as reagents for differential identification of the tissue(s) or cell type(s) present in a biological sample and for diagnosis of diseases and conditions: hematopietic and nervous system conditions. Similarly, polypeptides and antibodies directed to these polypeptides are useful in providing immunological probes for differential identification of the tissue(s) or cell type(s). For a number of disorders of the above tissues or cells, particularly of the nervous and immune, expression of this gene at significantly higher or lower levels may be routinely detected in certain tissues and cell types (e.g., bone marrow, melanocytes, brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to nucleotide binding factors indicate that polynucleotides and polypeptides corresponding to this gene are useful for study, diagnosis, and treatment of brain degenerative, skin and blood diseases.

### FEATURES OF PROTEIN ENCODED BY GENE NO: 76

This gene is expressed primarily in activated T-cells and to a lesser extent in retina, brain, and fetal bone.

Therefore, polynucleotides and polypeptides of the invention are useful as reagents for differential identification of activated T-cells and developing brain present in a biological sample and for diagnosis of diseases and conditions: immune deficiencies and skeletal and neuronal growth disorders. Similarly, polypeptides and antibodies directed to these polypeptides are useful in providing immunological probes for differential identification of the tissue(s) or cell type(s). For a number of disorders of the above tissues or cells, particularly of the nervous, immune, and skeletomuscular sustems, expression of this gene at significantly higher or lower levels may be routinely detected in certain tissues and cell types (e.g., blood cells, brain and other tissue of the nervous system, retinal tissue, and bone, and cancerous and wounded tissues) or bodily fluids (e.g., serum, plasma, urine, synovial fluid or spinal fluid) Or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis, study and treatment of cancer, urogenital, and brain degenerative diseases.

### FEATURES OF PROTEIN ENCODED BY GENE NO: 77

This gene is expressed primarily in fetal liver, activated monocytes, osteoblasts and to a lesser extent in synovial, brain, and lymphoid tissues.

Therefore, polynucleotides and polypeptides of the invention are useful as reagents for differential identification of myeloid and lymphoid present in a biological sample and for diagnosis of diseases and conditions: inflammation, immune deficiencies, cancer. Similarly, polypeptides and antibodies directed to these polypeptides are useful in providing immunological probes for differential identification of the tissue(s) or cell type(s). For a number of disorders of the above tissues or cells, particularly of the immune system and skeleton, expression of this gene at significantly higher or lower levels may be routinely detected in certain tissues and cell types (e.g., liver, blood cells, bone, synovial tissue, brain and other tissue of the nervous system, and lymphoid tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study, diagnosis, and treatment of lymphoid and mesenchymal cancers and nervous system diseases.

### FEATURES OF PROTEIN ENCODED BY GENE NO: 78

The translation product of this gene shares sequence homology with polymerase polyprotein precursor which is thought to be important in DNA repair and replication

This gene is expressed primarily in infant brain and to a lesser extent in tumors and tumor cell lines

Therefore, polynucleotides and polypeptides of the invention are useful as reagents for differential identification of the tissue(s) or cell type(s) present in a biological sample and for diagnosis of diseases and conditions which include, but are not limited to, especially of the neural system and developing organs. Similarly, polypeptides and antibodies directed to these polypeptides are useful in providing immunological probes for differential identification of the tissue(s) or cell type(s). For a number of disorders of the above tissues or cells, particularly of the neural system expression of this gene at significantly higher or lower levels may be routinely detected in certain (e.g., brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to polymerase polyprotein precursor indicate that polynucleotides and polypeptides corresponding to this gene are useful for diagnosis and treatment of cancers especially of the neural system and developing organs

### FEATURES OF PROTEIN ENCODED BY GENE NO: 79

This gene is expressed primarily in muscle and endothelial cells and to a lesser extent in brain.

Therefore, polynucleotides and polypeptides of the invention are useful as reagents for differential identification of the tissue(s) or cell type(s) present in a biological sample and for diagnosis of diseases and conditions: vascular diseases. Similarly, polypeptides and antibodies directed to these polypeptides are useful in providing immunological probes for differential identification of the tissue(s) or cell type(s). For a number of disorders of the above tissues or cells, particularly of the vascular system, expression of this gene at significantly higher or lower levels may be routinely detected in certain (e.g., muscle, endothelial cells, brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of disorders of the vascular and neural system including cardiovascular and endothelial.

### FEATURES OF PROTEIN ENCODED BY GENE NO: 80

This gene is expressed primarily in placenta and to a lesser extent in fetal liver

Therefore, polynucleotides and polypeptides of the invention are useful as reagents for differential identification of the tissue(s) or cell type(s) present in a biological sample and for diagnosis of diseases and conditions: developmental disorders and disorder of the haemopoietic system, fetal liver and placenta. Similarly, polypeptides and antibodies directed to these polypeptides are useful in providing immunological probes for differential identification of the tissue(s) or cell type(s). For a number of disorders of the above tissues or cells, particularly of developmental disorders and disorder of the haemopoietic system, fetal liver and placenta, expression of this gene at significantly higher or lower levels may be routinely detected in certain tissues and cell types (e.g., placenta and liver, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of developmental disorders and disorders of the haemopoietic system, fetal liver and placenta.

### FEATURES OF PROTEIN ENCODED BY GENE NO: 81

This gene is expressed primarily in bone marrow, placenta and tissues and organs of the hematopoietic system.

Therefore, polynucleotides and polypeptides of the invention are useful as reagents for differential identification of the tissue(s) or cell type(s) present in a biological sample and for diagnosis of diseases and conditions: disorders of the bone and haemopoietic system. Similarly, polypeptides and antibodies directed to these polypeptides are useful in providing immunological probes for differential identification of the tissue(s) or cell type(s). For a number of disorders of the above tissues or cells, particularly of the immune, bone and hematopoietic system, expression of this gene at significantly higher or lower levels may be routinely detected in certain tissues and cell types (e.g., bone marrow, placenta, and hematopoietic cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of disorders of the immune, bone and hematopoietic system

### FEATURES OF PROTEIN ENCODED BY GENE NO: 82

The translation product of this gene shares sequence homology with secretory carrier membrane protein which is thought to be important in protein transport and export. Any frame shifts in coding sequence can be easily resolved using standard molecular biology techniques. Another group recently cloned this gene, calling it SCAMP. (See Accession No. 2232243.)

This gene is expressed primarily in prostate, breast and spleen, and to a lesser extent in several other tissues and organs.

Therefore, polynucleotides and polypeptides of the invention are useful as reagents for differential identification of the tissue(s) or cell type(s) present in a biological sample and for diagnosis of diseases and conditions: disorders of the breast prostate and spleen. Similarly, polypeptides and antibodies directed to these polypeptides are useful in providing immunological probes for differential identification of the tissue(s) or cell type(s). For a number of disorders of the above tissues or cells, particularly disorders of the breast prostate and spleen, expression of this gene at significantly higher or lower levels may be routinely detected in certain tissues and cell types (e.g., prostate, mammary tissue, and spleen, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to secretory carrier membrane protein indicate that polynucleotides and polypeptides corresponding to this gene are useful for diagnosis and treatment of disorders of the breast, prostate and spleen.

### FEATURES OF PROTEIN ENCODED BY GENE NO: 83

This gene is expressed primarily in developing organs and tissue like placenta and infant brain and to a lesser extent in developed organs and tissue like cerebellum and heart.

Therefore, polynucleotides and polypeptides of the invention are useful as reagents for differential identification of the tissue(s) or cell type(s) present in a biological sample and for diagnosis of diseases and conditions: neurological diseases. Similarly, polypeptides and antibodies directed to these polypeptides are useful in providing immunological probes for differential identification of the tissue(s) or cell type(s). For a number of disorders of the above tissues or cells, particularly of the neural system, expression of this gene at significantly higher or lower levels may be routinely detected in certain tissues and cell types (e.g., placenta, heart, brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of diseases of the neural system including neurological disorders and cancer.

### FEATURES OF PROTEIN ENCODED BY GENE NO: 84

The translation product of this gene shares sequence homology with ATPase 6 in Trypanosoma brucei which is thought to be important in metabolism.

This gene is expressed primarily in tumor and fetal tissues and to a lesser extent in melanocytes, kidney cortex, monocytes and ovary.

Therefore, polynucleotides and polypeptides of the invention are useful as reagents for differential identification of the tissue(s) or cell type(s) present in a biological sample and for diagnosis of diseases and conditions: metabolism disorders. Similarly, polypeptides and antibodies directed to these polypeptides are useful in providing immunological probes for differential identification of the tissue(s) or cell type(s). For a number of disorders of the above tissues or cells, particularly of the fetal systems, expression of this gene at significantly higher or lower levels may be routinely detected in certain tissues and cell types (e.g., fetal tissues, melanocytes, kidney, blood cells, ovary and other tissue of the reproductive system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to ATPase indicate that polynucleotides and polypeptides corresponding to this gene are useful for treatment and diagnosis of metabolism disorders, especially in fetal and tumor tissue growth.

### FEATURES OF PROTEIN ENCODED BY GENE NO: 85

The translation product of this gene shares sequence homology with the immunoglobulin superfamily of proteins which are known to be important in immune response and immunity.

This gene is expressed primarily in stromal cells, colon cancer, lung, amygdala, melanocyte and to a lesser extent in a variety of other tissues.

Therefore, polynucleotides and polypeptides of the invention are useful as reagents for differential identification of the tissue(s) or cell type(s) present in a biological sample and for diagnosis of diseases and conditions: defects of stromal cell development and cancer. Similarly, polypeptides and antibodies directed to these polypeptides are useful in providing immunological probes for differential identification of the tissue(s) or cell type(s). For a number of disorders of the above tissues or cells, particularly of the stromal cells, expression of this gene at significantly higher or lower levels may be routinely detected in certain tissues and cell types (e.g., stromal cells, colon, lung, amygdala, and melanocytes, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to immunoglobulin indicate that polynucleotides and polypeptides corresponding to this gene are useful for treatment and diagnosis of immune system disorders.

### FEATURES OF PROTEIN ENCODED BY GENE NO: 86

The translation product of this gene shares sequence homology with transcription iniation factor eIF-4 gamma which is thought to be important in gene transcription.

This gene is expressed primarily in tumor tissues.

Therefore, polynucleotides and polypeptides of the invention are useful as reagents for differential identification of the tissue(s) or cell type(s) present in a biological sample and for diagnosis of diseases and conditions: tumorigenesis. Similarly, polypeptides and antibodies directed to these polypeptides are useful in providing immunological probes for differential identification of the tissue(s) or cell type(s). For a number of disorders of the above tissues or cells, particularly in tumor tissues, expression of this gene at significantly higher or lower levels may be routinely detected in certain tissues and cell types (e.g., endometrium and lung, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to transcription iniation factor eIF-4 gamma indicate that polynucleotides and polypeptides corresponding to this gene are useful for gene regulation in tumorigenesis.

### FEATURES OF PROTEIN ENCODED BY GENE NO: 87

The translation product of this gene shares sequence homology at low level in prolines with secreted basic proline-rich peptide II-2 which is thought to be important in protein structure or inhibiting hydroxyapatite formation in vitro.

This gene is expressed primarily in endometrial tumor and fetal lung.

Therefore, polynucleotides and polypeptides of the invention are useful as reagents for differential identification of the tissue(s) or cell type(s) present in a biological sample and for diagnosis of diseases and conditions: endometrial tumors. Similarly, polypeptides and antibodies directed to these polypeptides are useful in providing immunological probes for differential identification of the tissue(s) or cell type(s). For a number of disorders of the above tissues or cells, particularly of the muscular/skeletal and reproductive systems, expression of this gene at significantly higher or lower levels may be routinely detected in certain tissues and cell types (e.g., endometrium, and lung, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to secreted basic proline-rich peptide II-2 indicate that polynucleotides and polypeptides corresponding to this gene are useful for inhibiting hydroxyapatite formation or establishing cell/tissue structure.

### FEATURES OF PROTEIN ENCODED BY GENE NO: 88

This gene is expressed primarily in: amniotic cells inducted with TNF in culture; and to a lesser extent in colon tissue from a patient with Crohn's Disease; parathyroid tumor, activated T-cells; cells of the human Caco-2 cell line; adenocarcinoma; colon; corpus colosum; fetal kidney; pancreas tumor; fetal brain; early stage brain, and anergic T-cells.

Therefore, polynucleotides and polypeptides of the invention are useful as reagents for differential identification of the tissue(s) or cell type(s) present in a biological sample and for diagnosis of diseases and conditions: tumors. Similarly, polypeptides and antibodies directed to these polypeptides are useful in providing immunological probes for differential identification of the tissue(s) or cell type(s). For a number of disorders of the above tissues or cells, particularly of the immune system; e.g., tumors, expression of this gene at significantly higher or lower levels may be routinely detected in certain (e.g., amniotic cells, colon, kidney, pancreas, parathyroid, brain and other tissue of the nervous system, blood cells, hematopoietic cells, liver, spleen, bone, testes and other reproductive tissue, brain and other tissue of the nervous system, and epithelial cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone is useful for modulating tumorigenesis and other immune system conditions such as disorders in immune response.

### FEATURES OF PROTEIN ENCODED BY GENE NO: 89

This gene is expressed primarily in fetal liver/spleen and hematopoietic cells and to a lesser extent in brain, osteosarcoma, and testis tumor.

Therefore, polynucleotides and polypeptides of the invention are useful as reagents for differential identification of the tissue(s) or cell type(s) present in a biological sample and for diagnosis of diseases and conditions: leukemia and hematopoietic disorders. Similarly, polypeptides and antibodies directed to these polypeptides are useful in providing immunological probes for differential identification of the tissue(s) or cell type(s). For a number of disorders of the above tissues or cells, particularly of the hematopoietic and immune systems, expression of this gene at significantly higher or lower levels may be routinely detected in certain tissues and cell types (e.g., hematopoietic cells, liver, spleen, bone, testes, and other reproductive tissue, brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of hematopoietic and immune disorders.

### FEATURES OF PROTEIN ENCODED BY GENE NO: 90

The translation product of this gene shares weak sequence homology with mouse Gcap 1 protein which is developmentally regulated in brain.

This gene is expressed primarily in infant and adult brain and fetal liver/spleen and to a lesser extent in smooth muscle, T cells, and a variety of other tissues.

Therefore, polynucleotides and polypeptides of the invention are useful as reagents for differential identification of the tissue(s) or cell type(s) present in a biological sample and for diagnosis of diseases and conditions: neurological or hematopoietic disorders. Similarly, polypeptides and antibodies directed to these polypeptides are useful in providing immunological probes for differential identification of the tissue(s) or cell type(s). For a number of disorders of the above tissues or cells, particularly of the nervous, hematopoietic, immune, and endocrine systems, expression of this gene at significantly higher or lower levels may be routinely detected in certain tissues and cell types (e.g., brain and other tissue of the nervous system, blood cells, liver, spleen ,and smooth muscle, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and its homology to Gcapl protein indicate that polynucleotides and polypeptides corresponding to this gene are useful for treatubg and diagnosis of disorders in neuronal, hematopoietic, immune, and endocrine systems.

### FEATURES OF PROTEIN ENCODED BY GENE NO: 91

This gene is expressed primarily in brain and hematopoietic cells and to a lesser extent in tumor tissues.

Therefore, polynucleotides and polypeptides of the invention are useful as reagents for differential identification of the tissue(s) or cell type(s) present in a biological sample and for diagnosis of diseases and conditions: disorder in nervous, hematopoietic, immune systems and tumorigenesis. Similarly, polypeptides and antibodies directed to these polypeptides are useful in providing immunological probes for differential identification of the tissue(s) or cell type(s). For a number of disorders of the above tissues or cells, particularly of the in nervous, hematopoietic, immune systems, expression of this gene at significantly higher or lower levels may be routinely detected in certain tissues and cell types (e.g., brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone is useful for diagnosis and treatment of disorders in the nervous, hematopoietic, and immune systems.

### FEATURES OF PROTEIN ENCODED BY GENE NO: 92

The translation product of this gene shares sequence homology with neuroendocrine-specific protein A which is thought to be important in neurologic systems.

This gene is expressed primarily in brain tissues.

Therefore, polynucleotides and polypeptides of the invention are useful as reagents for differential identification of the tissue(s) or cell type(s) present in a biological sample and for diagnosis of diseases and conditions: neural disorders and degeneration disease. Similarly, polypeptides and antibodies directed to these polypeptides are useful in providing immunological probes for differential identification of the tissue(s) or cell type(s). For a number of disorders of the above tissues or cells, particularly of the central or peripheral nervous systems, expression of this gene at significantly higher or lower levels may be routinely detected in certain tissues and cell types (e.g., hematopoietic cells, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to neuroendocrine-specific protein A indicate that polynucleotides and polypeptides corresponding to this gene are useful for treatment or diagnosis of neural disorders and degeneration disease.

### FEATURES OF PROTEIN ENCODED BY GENE NO: 93

The translation product of this gene shares sequence homology with collagen-like protein and prolin-rich protein which are thought to be important in connective tissue function and tissue structure.

This gene is expressed primarily in fetal liver/spleen and brain tissues.

Therefore, polynucleotides and polypeptides of the invention are useful as reagents for differential identification of the tissue(s) or cell type(s) present in a biological sample and for diagnosis of diseases and conditions: neuronal or hematopoietic disorders. Similarly, polypeptides and antibodies directed to these polypeptides are useful in providing immunological probes for differential identification of the tissue(s) or cell type(s). For a number of disorders of the above tissues or cells, particularly of the nervous and hematopoietic systems, expression of this gene at significantly higher or lower levels may be routinely detected in certain tissues and cell types (e.g., liver, spleen, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to collagen-like protein and proline-rich proteins indicate that polynucleotides and polypeptides corresponding to this gene are useful for supporting brain and hematopoietic tissue function and diagnosis and treatment of disorders in these functions.

### FEATURES OF PROTEIN ENCODED BY GENE NO: 94

This gene is expressed primarily in embryonic tissues and tumor tissues.

Therefore, polynucleotides and polypeptides of the invention are useful as reagents for differential identification of the tissue(s) or cell type(s) present in a biological sample and for diagnosis of diseases and conditions which include, but are not limited to,. Similarly, polypeptides and antibodies directed to these polypeptides are useful in providing immunological probes for differential identification of the tissue(s) or cell type(s). For a number of disorders of the above tissues or cells, particularly of the immune system (e.g., tumors), expression of this gene at significantly higher or lower levels may be routinely detected in certain tissues and cell types (e.g., embryonic tissue and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of cancer.

### FEATURES OF PROTEIN ENCODED BY GENE NO: 95

This gene is expressed primarily in brain tumor, placenta, and melanoma.

Therefore, polynucleotides and polypeptides of the invention are useful as reagents for differential identification of the tissue(s) or cell type(s) present in a biological sample and for diagnosis of diseases and conditions: brain tumor or melanoma. Similarly, polypeptides and antibodies directed to these polypeptides are useful in providing immunological probes for differential identification of the tissue(s) or cell type(s). For a number of disorders of the above tissues or cells, particularly of the brain or melanocytes, expression of this gene at significantly higher or lower levels may be routinely detected in certain tissues and cell types (e.g., brain and other tissue of the nervous system, placenta, and melanocytes, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the translation product of this gene is useful in the diagnosis and treatment of brain tumors and melanoma.

### FEATURES OF PROTEIN ENCODED BY GENE NO: 96

The translation product of this gene shares sequence homology with a yeast membrane protein, SUR4, which encodes for APA1 that acts on a glucose-signaling pathway that controls the expression of several genes that are transcriptionally regulated by glucose.

This gene is expressed primarily in fetal liver, and to a lesser extent in placenta and breast tissue.

Therefore, polynucleotides and polypeptides of the invention are useful as reagents for differential identification of the tissue(s) or cell type(s) present in a biological sample and for diagnosis of diseases and conditions: defects of fetal liver or defects of glucose-regulated ATPase activities in tissues. Similarly, polypeptides and antibodies directed to these polypeptides are useful in providing immunological probes for differential identification of the tissue(s) or cell type(s). For a number of disorders of the above tissues or cells, particularly of the fetal immune/hematopoietic system, expression of this gene at significantly higher or lower levels may be routinely detected in certain tissues and cell types (e.g., liver, placenta, and mammary tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to yeast SUR4 membrane protein indicate that polynucleotides and polypeptides corresponding to this gene are useful for diagnosis and treatment of defects of fetal liver or defects of glucose-regulated ATPase activities.

### FEATURES OF PROTEIN ENCODED BY GENE NO: 97

This gene is expressed primarily in fetal liver, brain, and amniotic fluid.

Therefore, polynucleotides and polypeptides of the invention are useful as reagents for differential identification of the tissue(s) or cell type(s) present in a biological sample and for diagnosis of diseases and conditions: defects of the fetal immune system and adult brain. Similarly, polypeptides and antibodies directed to these polypeptides are useful in providing immunological probes for differential identification of the tissue(s) or cell type(s). For a number of disorders of the above tissues or cells, particularly of the fetal immune system and adult brain, expression of this gene at significantly higher or lower levels may be routinely detected in certain tissues and cell types (e.g., liver, and brain and other tissue of the nervous system, and cancerous and wounded tissues) or bodily fluids (e.g., amniotic fluid, serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone is useful for detecting defects of the fetal immune and hematopoietic systems since fetal liver is the predominant organ responsible for hematopoiesis in the fetus. In addition, the gene product of this gene is thought to be useful for detecting certain neurological defects of the brain.

### FEATURES OF PROTEIN ENCODED BY GENE NO: 98

The translation product of this gene shares sequence homology with an yolk protein precursor, Vitellogenin which is thought to be important in binding lipids such as phosvitin.

This gene is expressed primarily in amniotic cells and fetal liver.

Therefore, polynucleotides and polypeptides of the invention are useful as reagents for differential identification of the tissue(s) or cell type(s) present in a biological sample and for diagnosis of diseases and conditions: defects in amniotic cells, fetal liver development and the fetal immune system. Similarly, polypeptides and antibodies directed to these polypeptides are useful in providing immunological probes for differential identification of the tissue(s) or cell type(s). For a number of disorders of the above tissues or cells, particularly of the [insert system where a related disease state is likely, e.g., immune], expression of this gene at significantly higher or lower levels may be routinely detected in certain tissues and cell types (e.g., amniotic cells, and liver, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to vitellogenin indicate that the protein product of this clone is useful for treatment and diagnosis of defects in amniotic cells, fetal liver development and the fetal immune system.

### FEATURES OF PROTEIN ENCODED BY GENE NO: 99

This gene is expressed primarily in placenta, endometrial tumor, osteosarcoma and stromal cells.

Therefore, polynucleotides and polypeptides of the invention are useful as reagents for differential identification of the tissue(s) or cell type(s) present in a biological sample and for diagnosis of diseases and conditions: tumor of the endometrium or bone, and osteosarcoma. Similarly, polypeptides and antibodies directed to these polypeptides are useful in providing immunological probes for differential identification of the tissue(s) or cell type(s). For a number of disorders of the above tissues or cells, particularly of the obstetric system (e.g. placenta, endometrium) and the bones, expression of this gene at significantly higher or lower levels may be routinely detected in certain tissues and cell types (e.g., placenta, endometrium, bone, and stromal cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of tumors and abnormalities of the endometrium, and the bones because of its abundance in the aforementioned tissues..

### FEATURES OF PROTEIN ENCODED BY GENE NO: 100

This gene is expressed primarily in hepatocellular tumor.

Therefore, polynucleotides and polypeptides of the invention are useful as reagents for differential identification of the tissue(s) or cell type(s) present in a biological sample and for diagnosis of diseases and conditions: hepatocellular tumor. Similarly, polypeptides and antibodies directed to these polypeptides are useful in providing immunological probes for differential identification of the tissue(s) or cell type(s). For a number of disorders of the above tissues or cells, particularly of the liver, expression of this gene at significantly higher or lower levels may be routinely detected in certain tissues and cell types (e.g., liver, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone is useful for diagnosis and treatment of hepatocellular cancer because of its abundant expression in this tissue.

### FEATURES OF PROTEIN ENCODED BY GENE NO: 101

This gene is expressed primarily in Corpus Colosum, fetal lung and infant brain.

Therefore, polynucleotides and polypeptides of the invention are useful as reagents for differential identification of the tissue(s) or cell type(s) present in a biological sample and for diagnosis of diseases and conditions: defects of the Corpus Colosum or defects of the fetal lung. Similarly, polypeptides and antibodies directed to these polypeptides are useful in providing immunological probes for differential identification of the tissue(s) or cell type(s). For a number of disorders of the above tissues or cells, particularly of the Corpus Colosum and brain in general, and fetal lung, expression of this gene at significantly higher or lower levels may be routinely detected in certain tissues and cell types (e.g., lung, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone is useful for diagnosis and treatment of defects of the Corpus Colosum and brain in general, and defects of fetal lung.

### FEATURES OF PROTEIN ENCODED BY GENE NO: 102

This gene is expressed primarily in T cells and stromal cells, and to a lesser extent in adrenal gland.

Therefore, polynucleotides and polypeptides of the invention are useful as reagents for differential identification of the tissue(s) or cell type(s) present in a biological sample and for diagnosis of diseases and conditions: defects of T cell immunity and stromal cell development. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., blood cells, stromal cells, and adrenal gland, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone is useful for diagnosis and treatment of defects of T cell immunity and stromal cell development because of its abundant expression in these tissues.

### FEATURES OF PROTEIN ENCODED BY GENE NO: 103

This gene is expressed primarily in infant brain and placenta.

Therefore, polynucleotides and polypeptides of the invention are useful as reagents for differential identification of the tissue(s) or cell type(s) present in a biological sample and for diagnosis of diseases and conditions: defects of the brain and nervous system. Similarly, polypeptides and antibodies directed to these polypeptides are useful in providing immunological probes for differential identification of the tissue(s) or cell type(s). For a number of disorders of the above tissues or cells, particularly of the nervous system, especially brain, expression of this gene at significantly higher or lower levels may be routinely detected in certain tissues and cell types (e.g., brain and other tissue of the nervous system, and placenta, cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone is useful for detecting defects of the brain, especially in young children.

### FEATURES OF PROTEIN ENCODED BY GENE NO: 105

This gene is expressed primarily in human osteoclastoma and to a lesser extent in human pancreas tumor.

Therefore, polynucleotides and polypeptides of the invention are useful as reagents for differential identification of the tissue(s) or cell type(s) present in a biological sample and for diagnosis of diseases and conditions which include, but are not limited to, cancer particularly osteoclastoma and pancreatic tumor. Similarly, polypeptides and antibodies directed to these polypeptides are useful in providing immunological probes for differential identification of the tissue(s) or cell type(s). For a number of disorders of the above tissues or cells, particularly in transformed tissues, expression of this gene at significantly higher or lower levels may be routinely detected in certain tissues and cell types (e.g., bone and pancreas, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone is useful for diagnosis and treatment of some types of tumors, particularly pancreatic cancer and osteoclastoma.

### FEATURES OF PROTEIN ENCODED BY GENE NO: 106

This gene is expressed primarily in fetal liver/spleen, and to a lesser extent in activated T-Cells.

Therefore, polynucleotides and polypeptides of the invention are useful as reagents for differential identification of the tissue(s) or cell type(s) present in a biological sample and for diagnosis of diseases and conditions: immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., liver, spleen, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis or treatment of immune disorders.

### FEATURES OF PROTEIN ENCODED BY GENE NO: 107

This gene is expressed primarily in human embryo and to a lesser extent in spleen and chronic lymphocytic leukemia.

Therefore, polynucleotides and polypeptides of the invention are useful as reagents for differential identification of the tissue(s) or cell type(s) present in a biological sample and for diagnosis of diseases and conditions: leukemia. Similarly, polypeptides and antibodies directed to these polypeptides are useful in providing immunological probes for differential identification of the tissue(s) or cell type(s). For a number of disorders of the above tissues or cells, particularly of the immune or hemopoietic systems, expression of this gene at significantly higher or lower levels may be routinely detected in certain tissues and cell types (e.g., embryonic tissue, spleen, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone is useful for the diagnosis and treatment of leukemia.

### FEATURES OF PROTEIN ENCODED BY GENE NO: 108

This gene is expressed primarily in placenta, and to a lesser extent in early stage human brain and in lung.

Therefore, polynucleotides and polypeptides of the invention are useful as reagents for differential identification of the tissue(s) or cell type(s) present in a biological sample and for diagnosis of diseases and conditions: fetal developmental abnormalities. Similarly, polypeptides and antibodies directed to these polypeptides are useful in providing immunological probes for differential identification of the tissue(s) or cell type(s). For a number of disorders of the above tissues or cells, particularly in fetal and amniotic tissue, expression of this gene at significantly higher or lower levels may be routinely detected in certain tissues and cell types (e.g., placenta, brain and other tissue of the nervous system, and lung, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this is useful for production of growth factor(s) associated with fetal development. Preferred polypeptides comprise the full-length polypeptide shown in the sequence listing, truncated however, at the amino terminus and beginning with QTIE.

### FEATURES OF PROTEIN ENCODED BY GENE NO: 109

This gene is expressed primarily in fetal spleen, and to a lesser extent in B-Cell lymphoma and T-Cell lymphoma.

Therefore, polynucleotides and polypeptides of the invention are useful as reagents for differential identification of the tissue(s) or cell type(s) present in a biological sample and for diagnosis of diseases and conditions: lymphoma. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., spleen and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone is useful for the treatment and diagnosis of human lymphomas.

### FEATURES OF PROTEIN ENCODED BY GENE NO: 110

The translation product of this gene shares sequence homology with sarcoma amplified sequence (SAS), a tetraspan receptor which is thought to be important in malignant fibrous histiocytoma and liposarcoma.

This gene is expressed primarily in human osteoclastoma, and to a lesser extent in pineal gland and infant brain.

Therefore, polynucleotides and polypeptides of the invention are useful as reagents for differential identification of the tissue(s) or cell type(s) present in a biological sample and for diagnosis of diseases and conditions: malignant fibrous histiocytoma and liposarcoma. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., bone, pineal gland, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to sarcoma amplified sequence (SAS) indicate that the protein product of this clone is useful for treatment of, osteosarcoma, malignant fibrous histiocytoma and liposarcoma and related cancers, particularly sarcomas.

### FEATURES OF PROTEIN ENCODED BY GENE NO: 111

The translation product of this gene shares sequence homology with 6.8K proteolipid protein, mitochondrial - bovine.

This gene is expressed primarily in Wilm's tumor and to a lesser extent in cerebellum and placenta.

Therefore, polynucleotides and polypeptides of the invention are useful as reagents for differential identification of the tissue(s) or cell type(s) present in a biological sample and for diagnosis of diseases and conditions: Wilm's tumor. Similarly, polypeptides and antibodies directed to these polypeptides are useful in providing immunological probes for differential identification of the tissue(s) or cell type(s). For a number of disorders of the above tissues or cells, particularly of the immune or renal systems, expression of this gene at significantly higher or lower levels may be routinely detected in certain tissues and cell types (e.g., brain and other tissue of the nervous system, and placenta, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to 6.8K proteolipid protein indicate that the protein product of this clone is useful for diagnostic and therapeutics associated with tumors, particularly Wilm's tumor disease.

### FEATURES OF PROTEIN ENCODED BY GENE NO: 112

This gene is expressed primarily in embryonic tissue and to a lesser extent in osteoblasts, endothelial cells, macrophages (GM-CSF treated), and bone marrow.

Therefore, polynucleotides and polypeptides of the invention are useful as reagents for differential identification of the tissue(s) or cell type(s) present in a biological sample and for diagnosis of diseases and conditions: immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., embryonic tissue, bone, endothelial cells, blood cells and bone marrow, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment or diagnosis of immune disorders. Preferred polypeptides encoded by this gene comprise the following amino acid sequence: MITDVQLAIFANMLGVSLFLLVVLYHYVAVNNPKKQE (SEQ ID NO: 636).

### FEATURES OF PROTEIN ENCODED BY GENE NO: 113

This gene is expressed primarily in hepatocellular tumor, and to a lesser extent in fetal liver/spleen.

Therefore, polynucleotides and polypeptides of the invention are useful as reagents for differential identification of the tissue(s) or cell type(s) present in a biological sample and for diagnosis of diseases and conditions: tumors, particularly hepatocellular tumors. Similarly, polypeptides and antibodies directed to these polypeptides are useful in providing immunological probes for differential identification of the tissue(s) or cell type(s). For a number of disorders of the above tissues or cells, particularly of the hepatic system, expression of this gene at significantly higher or lower levels may be routinely detected in certain tissues and cell types (e.g., liver, and spleen, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone is useful for diagnosis and treatment of tumors, particularly hepatocellular tumors.

### FEATURES OF PROTEIN ENCODED BY GENE NO: 114

The translation product of this gene exhibits a very high degree of sequence identity with the human Pig8 gene which is thought to be important in p53 mediated apoptosis. The sequence of this gene has since been published by Polyak and colleagues (Nature 389, 300-306 (1997)). In addition, the predicted translation product of this contig exhibits very high sequence homology with a murine gene denoted as EI24 which is also thought to be important in p53 mediated apoptosis.

This gene is expressed primarily in infant brain and activated T-cells and to a lesser extent in bone marrow, fetal liver, and prostate.

Therefore, polynucleotides and polypeptides of the invention are useful as reagents for differential identification of the tissue(s) or cell type(s) present in a biological sample and for diagnosis of diseases and conditions which include, but are not limited to, and tissue damage by radiation and anti-cancer drugs. Similarly, polypeptides and antibodies directed to these polypeptides are useful in providing immunological probes for differential identification of the tissue(s) or cell type(s). For a number of disorders of the above tissues or cells, particularly of the nervous and immune systems, expression of this gene at significantly higher or lower levels may be routinely detected in certain tissues and cell types (e.g., brain and other tissue of the nervous system, blood cells, bone marrow, liver, and prostate, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to human Pig8 and murine EI24 genes indicate that polynucleotides and polypeptides corresponding to this gene are useful for preventing apoptosis in patients being treated with anti-oncogenic drugs such as etoposide, hydroperoxycyclophosphamide, and X-irradiation, since this protein product is upregulated in cells undergoing such treatment where p53 was overexpressed. It may also be useful in the treatment of hematopoietic disorders and in boosting numbers of hematopoietic stem cells by interfering with the apoptosis of progenitor cells. The mature polypeptide is predicted to comprise the following amino acid sequence: EEMADSVKTFLQDLARGIKDSIWGICTISKLDARIQQKREEQRRRRASSVLAQRRAQSIERKQES EPRIVSRIFQCCAWNGGVFWFSLLLFYRVFIPVLQSVTARIIGDPSLHGDVWSWLEFFLTSIFSA LWVLPLFVLSKVVNAIWFQDIADLAFEVSGRKPHPFPSVSKIIADMLFNLLLQALFLIQGMFVSL FPIHLVGQLVSLLHMSLLYSLYCFEYRWFNKGIEMHQRLSNIERNWPYYFGFGLPLAFLTAMQ SSYIISGCLFSILFPLFIISANEAKTPGKAYLFQLRLFSLVVFLSNRLFHKTVYLQSALSSSTSAEK FPSPHPSPAKLKATAGH (SEQ ID NO: 637). Accordingly, polypeptides comprising the foregoing amino acid sequence are provided as are polynucleotides encoded such polypeptides.

### FEATURES OF PROTEIN ENCODED BY GENE NO: 115

This gene is expressed primarily in stromal cells and to a lesser extent in multiple sclerosis.

Therefore, polynucleotides and polypeptides of the invention are useful as reagents for differential identification of the tissue(s) or cell type(s) present in a biological sample and for diagnosis of diseases and conditions: affecting the nervous system. Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may be routinely detected in certain tissues and cell types (e.g., stromal cells and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of multiple sclerosis and other autoimmune diseases.

### FEATURES OF PROTEIN ENCODED BY GENE NO: 116

This gene is expressed primarily in the gall bladder

Therefore, polynucleotides and polypeptides of the invention are useful as reagents for differential identification of the tissue(s) or cell type(s) present in a biological sample and for diagnosis of diseases and conditions: gall stones or infection of the digestive system . Similarly, polypeptides and antibodies directed to these polypeptides are useful in providing immunological probes for differential identification of the tissue(s) or cell type(s). For a number of disorders of the above tissues or cells, particularly of the digestive system or renal system, expression of this gene at significantly higher or lower levels may be routinely detected in certain tissues and cell types (e.g., gall bladder and tissue of the digestive system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for possible prevention of digestive disorders where there may be a lack of digestive enzymes produced or in the detection and possible prevention of gall stones.

### FEATURES OF PROTEIN ENCODED BY GENE NO: 117

The translation product of this gene shares sequence homology with dystrophin gene which is thought to be important in building and maintenance of muscles.

This gene is expressed primarily in placenta and to a lesser extent in fetal brain and fetal liver, and spleen.

Therefore, polynucleotides and polypeptides of the invention are useful as reagents for differential identification of the tissue(s) or cell type(s) present in a biological sample and for diagnosis of diseases and conditions: muscular dystropy, Duchenne and Becker's muscular dystropies. Similarly, polypeptides and antibodies directed to these polypeptides are useful in providing immunological probes for differential identification of the tissue(s) or cell type(s). For a number of disorders of the above tissues or cells, particularly of the skeletal muscle system, expression of this gene at significantly higher or lower levels may be routinely detected in certain tissues and cell types (e.g., placenta, brain and other tissue of the nervous system, muscle, liver, and spleen, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to the dystrophin gene indicate that polynucleotides and polypeptides corresponding to this gene are useful for diseases related the degenerative myopathies that are characterized by the weakness and atrophy of muscles without neural degradation; such as Duchenne and Becker's muscular dystropies.

### FEATURES OF PROTEIN ENCODED BY GENE NO: 118

This gene is expressed primarily in olfactory tissue and to a lesser extent in cartilage.

Therefore, polynucleotides and polypeptides of the invention are useful as reagents for differential identification of the tissue(s) or cell type(s) present in a biological sample and for diagnosis of diseases and conditions: connective tissue diseases; chondrosarcoma. Similarly, polypeptides and antibodies directed to these polypeptides are useful in providing immunological probes for differential identification of the tissue(s) or cell type(s). For a number of disorders of the above tissues or cells, particularly of the connective tissue, expression of this gene at significantly higher or lower levels may be routinely detected in certain tissues and cell types (e.g., olfactory tissue and cartilage, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for tumors of connective tissues, osteoarthritis and the treatment and diagnosis of chondrosarcoma.

### FEATURES OF PROTEIN ENCODED BY GENE NO: 119

This gene is expressed primarily in Activated Neutrophils and to a lesser extent in fetal spleen, and CD34 positive cells from cord blood.

Therefore, polynucleotides and polypeptides of the invention are useful as reagents for differential identification of the tissue(s) or cell type(s) present in a biological sample and for diagnosis of diseases and conditions: allergies, defects in hematopoiesis and inflammation. Similarly, polypeptides and antibodies directed to these polypeptides are useful in providing immunological probes for differential identification of the tissue(s) or cell type(s). For a number of disorders of the above tissues or cells, particularly of the immune system and hematopoiesis system the, expression of this gene at significantly higher or lower levels may be routinely detected in certain tissues and cell types (e.g., blood cells, and spleen, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for reducing the allergic effects felt by allergy suffers by neutralizing the activity of the immune system, especially since neutrophils are abundant in persons suffering from allergies and other inflammatory conditions.

### FEATURES OF PROTEIN ENCODED BY GENE NO: 120

The translation product of this gene shares sequence homology with poly A binding protein II which is thought to be important in RNA binding for transcription of RNA to DNA

This gene is expressed primarily in colon and to a lesser extent in brain and immune system.

Therefore, polynucleotides and polypeptides of the invention are useful as reagents for differential identification of the tissue(s) or cell type(s) present in a biological sample and for diagnosis of diseases and conditions: colon cancer. Similarly, polypeptides and antibodies directed to these polypeptides are useful in providing immunological probes for differential identification of the tissue(s) or cell type(s). For a number of disorders of the above tissues or cells, particularly of the immune and digestive system, expression of this gene at significantly higher or lower levels may be routinely detected in certain tissues and cell types (e.g., colon, tissue and cells of the immune system, and brain or other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to poly A binding protein II indicate that polynucleotides and polypeptides corresponding to this gene are useful for detection and treatment of colon cancer and other disorders of the digestive system..

### FEATURES OF PROTEIN ENCODED BY GENE NO: 121

The translation product of this gene shares sequence homology with thymidine diphosphoglucose 4.6 dehydrase which is thought to be important in the metabolism of sugar.

This gene is expressed primarily in fetal liver and spleen and to a lesser extent in infant brain.

Therefore, polynucleotides and polypeptides of the invention are useful as reagents for differential identification of the tissue(s) or cell type(s) present in a biological sample and for diagnosis of diseases and conditions: diabetes. Similarly, polypeptides and antibodies directed to these polypeptides are useful in providing immunological probes for differential identification of the tissue(s) or cell type(s). For a number of disorders of the above tissues or cells, particularly of the endocrine system, expression of this gene at significantly higher or lower levels may be routinely detected in certain tissues and cell types (e.g., liver, spleen, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to thymidine diphospoglucose 4.6 dehydrase indicate that polynucleotides and polypeptides corresponding to this gene are useful for treatment of persons with diabetes since it appears that this protein is needed in the metabolism of sugar in to its more basic components.

### FEATURES OF PROTEIN ENCODED BY GENE NO: 122

The translation product of this gene shares sequence homology with ceruloplasmin which is thought to be important in the metabolism and transport of iron and copper. Ceruloplasmin also contains domains with homology to clotting factors V and VIII. Defects in the circulating levels of ceruloplasmin (aceruloplasminemia) have been associated with certain disease conditions such as Wilson disease, and the accompanying hepatolenticular degeneration.

This gene is expressed primarily in brain and retina and to a lesser extent in endothelial cells.

Therefore, polynucleotides and polypeptides of the invention are useful as reagents for differential identification of the tissue(s) or cell type(s) present in a biological sample and for diagnosis of diseases and conditions: diseases marked by defects in iron metabolism; aceruloplasminemia not characterized by defects in the known ceruloplasmin gene locus; nonclassical Wilson disease; movement disorders; and tumors derived from a brain tissue origin. Similarly, polypeptides and antibodies directed to these polypeptides are useful in providing immunological probes for differential identification of the tissue(s) or cell type(s). For a number of disorders of the above tissues or cells, particularly of the brain, retina, and nervous system, expression of this gene at significantly higher or lower levels may be routinely detected in certain tissues and cell types (e.g., brain and other tissue of the nervous system, retinal tissue, and endothelial cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to ceruloplasmin indicate that polynucleotides and polypeptides corresponding to this gene are useful for treatment of patients with aceruloplasminemia, or other defects in iron and/or copper metabolism. Mutations in this locus could also be diagnostic for patients currently experiencing or predicted to experience aceruloplasminemia.

### FEATURES OF PROTEIN ENCODED BY GENE NO: 123

This gene is expressed primarily in brain and B cell lymphoma and to a lesser extent in fetal liver and spleen.

Therefore, polynucleotides and polypeptides of the invention are useful as reagents for differential identification of the tissue(s) or cell type(s) present in a biological sample and for diagnosis of diseases and conditions: B cell lymphoma; tumors and diseases of the brain and/or spleen; hematopoietic defects. Similarly, polypeptides and antibodies directed to these polypeptides are useful in providing immunological probes for differential identification of the tissue(s) or cell type(s). For a number of disorders of the above tissues or cells, particularly of the brain and hematopoietic system, expression of this gene at significantly higher or lower levels may be routinely detected in certain tissues and cell types (e.g., brain and other tissue of the nervous system, blood cells, liver, and spleen, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment of disorders in neuronal, hematopoietic, and immune systems. It could potentially be useful for neurodegenerative disorders and neuronal and/or hematopoietic cell survival or proliferation.

### FEATURES OF PROTEIN ENCODED BY GENE NO: 124

This gene is expressed primarily in osteoclastoma, dermatofibrosarcoma, and B cell lymphoma and to a lesser extent in endothelial cells.

Therefore, polynucleotides and polypeptides of the invention are useful as reagents for differential identification of the tissue(s) or cell type(s) present in a biological sample and for diagnosis of diseases and conditions which include, but are not limited to, cancer in particular osteoclastoma, dermatofibrosarcoma, and B cell lymphoma. Similarly, polypeptides and antibodies directed to these polypeptides are useful in providing immunological probes for differential identification of the tissue(s) or cell type(s). For a number of disorders of the above tissues or cells, particularly of the bone, immune, and circulatory system, expression of this gene at significantly higher or lower levels may be routinely detected in certain tissues and cell types (e.g., bone, epidermis, blood cells, and endothelial cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of cancers and lymphoma; osteoporosis; and the control of cell proliferation and/or differentiation.

### FEATURES OF PROTEIN ENCODED BY GENE NO: 125

This gene is expressed primarily in immune tissues and hematopoietic cells, particularly in activated T cells and neutrophils, spleen, and fetal liver, and to a lesser extent in infant adrenal gland.

Therefore, polynucleotides and polypeptides of the invention are useful as reagents for differential identification of the tissue(s) or cell type(s) present in a biological sample and for diagnosis of diseases and conditions: defects in T cell activation; hematopoietic disorders; tumors of a hematopoietic and/or adrenal gland origin. Similarly, polypeptides and antibodies directed to these polypeptides are useful in providing immunological probes for differential identification of the tissue(s) or cell type(s). For a number of disorders of the above tissues or cells, particularly of the hematopoietic and/or endocrine systems, expression of this gene at significantly higher or lower levels may be routinely detected in certain tissues and cell types (e.g., cells and tissues of the immune system, hematopoietic cells, blood cells, liver, and adrenal gland, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for immune and/or hematopoietic disorders; diseases related to proliferation and/or differentiation of hematopoietic cells; defects in T cell and neutrophil activation and responsiveness; and endocrine and/or metabolic disorders, particularly of early childhood.

### FEATURES OF PROTEIN ENCODED BY GENE NO: 126

This gene is expressed primarily in placenta and endothelial cells and to a lesser extent in melanocytes and embryonic tissues.

Therefore, polynucleotides and polypeptides of the invention are useful as reagents for differential identification of the tissue(s) or cell type(s) present in a biological sample and for diagnosis of diseases and conditions: tumors of an endothelial cell origin; angiogenesis associated with tumor development and metastasis. Similarly, polypeptides and antibodies directed to these polypeptides are useful in providing immunological probes for differential identification of the tissue(s) or cell type(s). For a number of disorders of the above tissues or cells, particularly of the vascular system and developing embryo, expression of this gene at significantly higher or lower levels may be routinely detected in certain tissues and cell types (e.g., placenta, endothelial cells, melanocytes, and embryonic tissues, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment of developmental disorders; inhibition of angiogenesis; and vascular patterning.

### FEATURES OF PROTEIN ENCODED BY GENE NO: 127

This gene is expressed primarily in endothelial cells and hematopoietic tissues, including spleen, tonsils, leukocytes, and both B- and T-cell lymphomas.

Therefore, polynucleotides and polypeptides of the invention are useful as reagents for differential identification of the tissue(s) or cell type(s) present in a biological sample and for diagnosis of diseases and conditions: tumors of an endothelial cell and/or hematopoietic origin; leukemias and lymphomas. Similarly, polypeptides and antibodies directed to these polypeptides are useful in providing immunological probes for differential identification of the tissue(s) or cell type(s). For a number of disorders of the above tissues or cells, particularly of the immune and vascular systems, expression of this gene at significantly higher or lower levels may be routinely detected in certain tissues and cell types (e.g., endothelial cells, hematopoietic cells, spleen, tonsils, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the manipulation of angiogenesis; the differentiation and morphogenesis of endothelial cells; the proliferation and/or differentiation of hematopoietic cells; and the commitment of hematopoietic cells to distinct cell lineages.

### FEATURES OF PROTEIN ENCODED BY GENE NO: 128

This gene is expressed primarily in kidney medulla and to a lesser extent in spleen from chronic myelogenous leukemia patients, prostate cancer, and some other tissues.

Therefore, polynucleotides and polypeptides of the invention are useful as reagents for differential identification of the tissue(s) or cell type(s) present in a biological sample and for diagnosis of diseases and conditions: tumors of a kidney origin; chromic myelogenous leukemia; prostate cancer. Similarly, polypeptides and antibodies directed to these polypeptides are useful in providing immunological probes for differential identification of the tissue(s) or cell type(s). For a number of disorders of the above tissues or cells, particularly of the kidney and spleen, expression of this gene at significantly higher or lower levels may be routinely detected in certain tissues and cell types (e.g., kidney, spleen, and prostate, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of kidney disorders and cancer, particularly chromic myelogenous leukemia and prostate cancer. It may also be useful for the enhancement of kidney tubule regeneration in the treatment of acute renal failure.

### FEATURES OF PROTEIN ENCODED BY GENE NO: 129

This gene is expressed primarily in adult and infant brain and to a lesser extent in mesenchymal or fibroblast cells, as well as tissues with a mesenchymal origin.

Therefore, polynucleotides and polypeptides of the invention are useful as reagents for differential identification of the tissue(s) or cell type(s) present in a biological sample and for diagnosis of diseases and conditions: tumors of a brain and/or mesenchymal origin; neurodegenerative disorders; cancer; fibrosis. Similarly, polypeptides and antibodies directed to these polypeptides are useful in providing immunological probes for differential identification of the tissue(s) or cell type(s). For a number of disorders of the above tissues or cells, particularly of the brain and of mesenchymal cells and tissues, expression of this gene at significantly higher or lower levels may be routinely detected in certain tissues and cell types (e.g., brain and other tissue of the nervous system and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis of tumors of a brain and/or mesenchymal origin; neurodegenerative disorders; cancer; and fibrosis, based upon the expression of this gene within those tissues. Fibrosis is considered as mesenchymal cells and fibroblasts are the primary cellular targets involved in this pathological condition.

### FEATURES OF PROTEIN ENCODED BY GENE NO: 130

This gene is expressed primarily in hepatocellular cancer and to a lesser extent in fetal tissues as well as testes tumor.

Therefore, polynucleotides and polypeptides of the invention are useful as reagents for differential identification of the tissue(s) or cell type(s) present in a biological sample and for diagnosis of diseases and conditions: liver cancer. Similarly, polypeptides and antibodies directed to these polypeptides are useful in providing immunological probes for differential identification of the tissue(s) or cell type(s). For a number of disorders of the above tissues or cells, particularly of the digestive system, expression of this gene at significantly higher or lower levels may be routinely detected in certain tissues and cell types (e.g., liver, fetal tissue, and testes and other reproductive tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of liver cancer.

### FEATURES OF PROTEIN ENCODED BY GENE NO: 131

This gene is expressed only in infant early brain.

Therefore, polynucleotides and polypeptides of the invention are useful as reagents for differential identification of the tissue(s) or cell type(s) present in a biological sample and for diagnosis of diseases and conditions: development and diseases of the nervous system. Similarly, polypeptides and antibodies directed to these polypeptides are useful in providing immunological probes for differential identification of the tissue(s) or cell type(s). For a number of disorders of the above tissues or cells, particularly of the brain and nervous system, expression of this gene at significantly higher or lower levels may be routinely detected in certain tissues and cell types (e.g., brain and other tissue of the nervous system and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating diseases of the brain in children and in treating nervous system disorders such as Alzheimer's disease, schizophrenia, dementia, depression, etc.

### FEATURES OF PROTEIN ENCODED BY GENE NO: 132

This gene is expressed primarily in brain and to a lesser extent in glioblastoma.

Therefore, polynucleotides and polypeptides of the invention are useful as reagents for differential identification of the tissue(s) or cell type(s) present in a biological sample and for diagnosis of diseases and conditions: Alzheimer's disease, schizophrenia, depression, mania, and dementia. Similarly, polypeptides and antibodies directed to these polypeptides are useful in providing immunological probes for differential identification of the tissue(s) or cell type(s). For a number of disorders of the above tissues or cells, particularly of the brain and nervous system, expression of this gene at significantly higher or lower levels may be routinely detected in certain tissues and cell types (e.g., brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating brain disorders such as Alzheimer's disease, schizophrenia, depression, mania, and dementia.

### FEATURES OF PROTEIN ENCODED BY GENE NO: 133

The translation product of this gene shares sequence homology with ribitol dehydrogenase of bacteria which is thought to be important in metabolism of sugars.

This gene is expressed primarily in macrophage and to a lesser extent in T-cell lymphoma and lung.

Therefore, polynucleotides and polypeptides of the invention are useful as reagents for differential identification of the tissue(s) or cell type(s) present in a biological sample and for diagnosis of diseases and conditions: tissue destruction in inflammation. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., blood cells and lung, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to ribitol dehydrogenase indicate that polynucleotides and polypeptides corresponding to this gene are useful for altering macrophage metabolism in diseases such as inflammation where macrophages are causing excess tissue destruction.

### FEATURES OF PROTEIN ENCODED BY GENE NO: 134

This gene is expressed primarily in pancreatic tumor and to a lesser extent in synovial sarcoma.

Therefore, polynucleotides and polypeptides of the invention are useful as reagents for differential identification of the tissue(s) or cell type(s) present in a biological sample and for diagnosis of diseases and conditions which include, but are not limited to,. Similarly, polypeptides and antibodies directed to these polypeptides are useful in providing immunological probes for differential identification of the tissue(s) or cell type(s). For a number of disorders of the above tissues or cells, particularly of the endocrine and connective tissue systems, expression of this gene at significantly higher or lower levels may be routinely detected in certain tissues and cell types (e.g., pancreas, and synovial tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating and diagnosing various cancers.

### FEATURES OF PROTEIN ENCODED BY GENE NO: 135

This gene is expressed primarily in T cell lines such as Raji and to a lesser extent in infant brain.

Therefore, polynucleotides and polypeptides of the invention are useful as reagents for differential identification of the tissue(s) or cell type(s) present in a biological sample and for diagnosis of diseases and conditions: immune system disorders and inflammation. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., blood cells, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating and diagnosing inflammatory diseases such as rheumatoid arthritis, sepsis, inflammatory bowel disease, and psoriasis, as well as neutropenia.

### FEATURES OF PROTEIN ENCODED BY GENE NO: 136

The translation product of this gene shares high sequence homology with SAR1 subfamily of GTP-binding proteins which is thought to be important in vesicular transport in mammalian cells.

This gene is expressed primarily in serum-stimulated smooth muscle cells and to a lesser extent in a T-cell lymphoma.

Therefore, polynucleotides and polypeptides of the invention are useful as reagents for differential identification of the tissue(s) or cell type(s) present in a biological sample and for diagnosis of diseases and conditions: diseases affecting vesicular transport. Similarly, polypeptides and antibodies directed to these polypeptides are useful in providing immunological probes for differential identification of the tissue(s) or cell type(s). For a number of disorders of the above tissues or cells, particularly of the muscular system, expression of this gene at significantly higher or lower levels may be routinely detected in certain tissues and cell types (e.g., blood cells, and smooth muscle, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to GTP-binding proteins indicate that polynucleotides and polypeptides corresponding to this gene are useful for gene therapy in treating the large number of diseases involved in defective vesicular transport within cells..

### FEATURES OF PROTEIN ENCODED BY GENE NO: 137

The translation product of this gene shares sequence homology with a protein found in *C. elegans* cosmid F25B5.

This gene is expressed primarily in a fetal tissues and to a lesser extent in melanocytes.

Therefore, polynucleotides and polypeptides of the invention are useful as reagents for differential identification of the tissue(s) or cell type(s) present in a biological sample and for diagnosis of diseases and conditions: abnormal fetal development, especially of the pulmonary system. Similarly, polypeptides and antibodies directed to these polypeptides are useful in providing immunological probes for differential identification of the tissue(s) or cell type(s). For a number of disorders of the above tissues or cells, particularly of the fetal pulmonary system, expression of this gene at significantly higher or lower levels may be routinely detected in certain tissues and cell types (e.g., fetal tissue, pulmonary tissue, and melanocytes, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of diseases affecting the pulmonary system, such as emphysema.

### FEATURES OF PROTEIN ENCODED BY GENE NO: 138

This gene is expressed primarily in gall bladder and to a lesser extent in smooth muscle.

Therefore, polynucleotides and polypeptides of the invention are useful as reagents for differential identification of the tissue(s) or cell type(s) present in a biological sample and for diagnosis of diseases and conditions: digestive system disease and gall bladder problems. Similarly, polypeptides and antibodies directed to these polypeptides are useful in providing immunological probes for differential identification of the tissue(s) or cell type(s). For a number of disorders of the above tissues or cells, particularly of the digestive system, expression of this gene at significantly higher or lower levels may be routinely detected in certain tissues and cell types (e.g., gall bladder and tissue of the digestive system, and smooth muscle, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating diseases of the digestive system.

### FEATURES OF PROTEIN ENCODED BY GENE NO: 139

This gene is expressed primarily in placenta and to a lesser extent in brain.

Therefore, polynucleotides and polypeptides of the invention are useful as reagents for differential identification of the tissue(s) or cell type(s) present in a biological sample and for diagnosis of diseases and conditions: abnormal fetal development. Similarly, polypeptides and antibodies directed to these polypeptides are useful in providing immunological probes for differential identification of the tissue(s) or cell type(s). For a number of disorders of the above tissues or cells, particularly of developing tissues, expression of this gene at significantly higher or lower levels may be routinely detected in certain tissues and cell types (e.g., placenta, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating and diagnosing abnormal fetal development.

### FEATURES OF PROTEIN ENCODED BY GENE NO: 140

This gene is expressed primarily in smooth muscle and to a lesser extent in ovary, prostate cancer, and activated monocytes.

Therefore, polynucleotides and polypeptides of the invention are useful as reagents for differential identification of the tissue(s) or cell type(s) present in a biological sample and for diagnosis of diseases and conditions: hypertension and atherosclerosis. Similarly, polypeptides and antibodies directed to these polypeptides are useful in providing immunological probes for differential identification of the tissue(s) or cell type(s). For a number of disorders of the above tissues or cells, particularly of the circulatory system, expression of this gene at significantly higher or lower levels may be routinely detected in certain tissues and cell types (e.g., smooth muscle, ovary and other reproductive tissue, prostate, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating diseases of the circulatory system, such as hypertension, atherosclerosis, etc.

### FEATURES OF PROTEIN ENCODED BY GENE NO: 141

This gene is expressed primarily in fetal spleen and to a lesser extent in placenta and bone marrow.

Therefore, polynucleotides and polypeptides of the invention are useful as reagents for differential identification of the tissue(s) or cell type(s) present in a biological sample and for diagnosis of diseases and conditions: anemia and other diseases affecting blood cells. Similarly, polypeptides and antibodies directed to these polypeptides are useful in providing immunological probes for differential identification of the tissue(s) or cell type(s). For a number of disorders of the above tissues or cells, particularly of the circulatory and pulmonary systems, expression of this gene at significantly higher or lower levels may be routinely detected in certain tissues and cell types (e.g., spleen, placenta, bone marrow, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the generation of red and white blood cells and for the diagnosis of disease of these cells.

### FEATURES OF PROTEIN ENCODED BY GENE NO: 142

The predicted translation product of this contig is a human homolog of the murine tetracycline/sugar transporter molecule recently reported by Matsuo and colleagues (Biochem. Biophys. Res. Commun. 238 (1), 126-129 (1997)).

This gene is expressed primarily in synovium and to a lesser extent in endothelial cells.

Therefore, polynucleotides and polypeptides of the invention are useful as reagents for differential identification of the tissue(s) or cell type(s) present in a biological sample and for diagnosis of diseases and conditions: rheumatoid arthritis and inflammation. Similarly, polypeptides and antibodies directed to these polypeptides are useful in providing immunological probes for differential identification of the tissue(s) or cell type(s). For a number of disorders of the above tissues or cells, particularly of the immune and lymphatic systems, expression of this gene at significantly higher or lower levels may be routinely detected in certain tissues and cell types (e.g., synovial tissue, and endothelial cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of inflammatory diseases, such as rheumatoid arthritis, leukemia, neutropenia, inflammatory bowel disease, psoriasis, sepsis, and the like.

### FEATURES OF PROTEIN ENCODED BY GENE NO: 143

This gene is expressed primarily in placenta and to a lesser extent in melanocyte, fetal liver and spleen, and bone marrow.

Therefore, polynucleotides and polypeptides of the invention are useful as reagents for differential identification of the tissue(s) or cell type(s) present in a biological sample and for diagnosis of diseases and conditions: abnormal early development. Similarly, polypeptides and antibodies directed to these polypeptides are useful in providing immunological probes for differential identification of the tissue(s) or cell type(s). For a number of disorders of the above tissues or cells, lower levels may be routinely detected in certain tissues and cell types (e.g., placenta, melanocytes, liver, spleen, and bone marrow, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of abnormal early development phenomena and diseases.

### FEATURES OF PROTEIN ENCODED BY GENE NO: 144

This gene is expressed primarily in fetal liver and spleen.

Therefore, polynucleotides and polypeptides of the invention are useful as reagents for differential identification of the tissue(s) or cell type(s) present in a biological sample and for diagnosis of diseases and conditions: anemia and neutropenia. Similarly, polypeptides and antibodies directed to these polypeptides are useful in providing immunological probes for differential identification of the tissue(s) or cell type(s). For a number of disorders of the above tissues or cells, particularly of the immune and blood systems, expression of this gene at significantly higher or lower levels may be routinely detected in certain tissues and cell types (e.g., liver and spleen, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful in hematopoeisis and bone marrow regeneration as it is most abundant in fetal tissues responsible for the generation of hematopoeitic cells.

### FEATURES OF PROTEIN ENCODED BY GENE NO: 145

The translation product of this gene shares sequence homology with protein tyrosine phosphatase which is thought to be important in transducing signal to activate cells such as T cell, B cell and other cell types.

This gene is expressed primarily in T cells and tissues in early stages of development and to a lesser extent in cancers.

Therefore, polynucleotides and polypeptides of the invention are useful as reagents for differential identification of the tissue(s) or cell type(s) present in a biological sample and for diagnosis of diseases and conditions: immuno-related diseases and cancer. Similarly, polypeptides and antibodies directed to these polypeptides are useful in providing immunological probes for differential identification of the tissue(s) or cell type(s). For a number of disorders of the above tissues or cells, particularly of the immune system expression of this gene at significantly higher or lower levels may be routinely detected in certain tissues and cell types (e.g., embryonic and fetal tissue, undifferentiated cells, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to the protein tyrosine phosphatase family indicate that polynucleotides and polypeptides corresponding to this gene are useful for modulating the immune system.

### FEATURES OF PROTEIN ENCODED BY GENE NO: 146

This gene is expressed primarily in T cell and to a lesser extent in B cell, macrophages and tumor tissues.

Therefore, polynucleotides and polypeptides of the invention are useful as reagents for differential identification of the tissue(s) or cell type(s) present in a biological sample and for diagnosis of diseases and conditions: immuno-disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for regulating the immune system therefore can be used in treating diseases such as autoimmune diseases and cancers.

### FEATURES OF PROTEIN ENCODED BY GENE NO: 147

This gene is expressed primarily in placenta and to a lesser extent in endothelial cells, testis tumor, ovarian cancer, uterine cancer.

Therefore, polynucleotides and polypeptides of the invention are useful as reagents for differential identification of the tissue(s) or cell type(s) present in a biological sample and for diagnosis of diseases and conditions which include, but are not limited to cancer. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., placenta, endothelial cells, testis and ovary and other reproductive tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of cancers.

### FEATURES OF PROTEIN ENCODED BY GENE NO: 148

This sequence has significant homology to mouse torsin A. Recently, another group cloned the human Torsin A gene. (See, Accession No. 2358279; see also Nature Genet. 17, 40-48 (1997).)

This gene is expressed primarily in osteoclastoma, T-cell, and placenta and to a lesser extent in fetal lung, fetal liver, fetal brain, adult brain and tumor tissues

Therefore, polynucleotides and polypeptides of the invention are useful as reagents for differential identification of the tissue(s) or cell type(s) present in a biological sample and for diagnosis of diseases and conditions: disease conditions in hematopoiesis and cancers. Similarly, polypeptides and antibodies directed to these polypeptides are useful in providing immunological probes for differential identification of the tissue(s) or cell type(s). For a number of disorders of the above tissues or cells, particularly of the hematopoiesis system, expression of this gene at significantly higher or lower levels may be routinely detected in certain tissues and cell types (e.g., blood cells, bone, placenta, lung, liver, and brain and other tissues of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating blood related diseases such as deficiencies in red blood cell, white blood cell, platelet and other hematopoiesis cells.

### FEATURES OF PROTEIN ENCODED BY GENE NO: 149

This gene is expressed primarily in T cell, prostate and prostate cancer, endothelial cells and to a lesser extent in monocyte, dendritic cell, bone marrow, salivary gland, colon cancer, stomach cancer, pancreatic tumor, uterine cancer, fetal spleen and osteoclastoma.

Therefore, polynucleotides and polypeptides of the invention are useful as reagents for differential identification of the tissue(s) or cell type(s) present in a biological sample and for diagnosis of diseases and conditions: immuno-related diseases and cancers. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., blood cells, prostate, endothelial cells, dendritic cells, bone marrow, salivary gland, colon, stomach, pancreas, uterus, spleen and bone, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment of cancers.

### FEATURES OF PROTEIN ENCODED BY GENE NO: 150

This gene was recently cloned by another group, calling it eIF3-p66. (See Accession No. 2351378.) This gene plays a role in RNA binding and macromolecular assembly, and therefore, any mutations in this gene would likely result in a diseased phenotype. Preferred polypeptide fragments comprise the amino acid sequence: MAKFMTPVIQDNPSGWGPCAVPEQFRDMPYQPFSKGDRLGKVADWTGATYQDKRYTNKYSS QFGGGSQYAYFHEEDESSPQLVDTARTQKTAYQRNRMRFAQRNLRRDKDRRNMLQFNLQILP KSAKQKERERIRLQKKFQKQFGVRQKWDQKSQKPRDSSVEVRSDWEVKEEMDFPQLMKMRY LEVSEPQDIECCGALEYYDKAFDRITTRSEKPLRXXKRIFHTVTTTDDPVIRKLAKTQGNVFATD AILATLMSCTRSVYSWDIVVQRVGSKLFFDKRDNSDFDLLTVSETANEPPQDEGNSFNSPRNL AMEATYINHNFSQQCLRMGKERYNFPNPNPFVEDDMDKNEIASVAYRYRSGKLGDDIDLIVRC EHDGVMTGANGEVSFINIKTLNEWDSRHCNGVDWRQKLDSQRGAVIATELKNNSYKLARWTC CALLAGSEYLKLGYVSRYHVKDSSRHVILGTQQFKPNEFASQINLSVENAWGILRCVIDICMKL EEGKYLILKDPNKQVIRVYSLPDGTFSS (SEQ ID NO: 638), as well as N-terminal and C-terminal deletions of this polypeptide fragment.

This gene is expressed primarily in T cell, bone marrow, embryo and endothelial cells and to a lesser extent in testis tumor and endometrial tumor.

Therefore, polynucleotides and polypeptides of the invention are useful as reagents for differential identification of the tissue(s) or cell type(s) present in a biological sample and for diagnosis of diseases and conditions: immune diseases and tumors. Similarly, polypeptides and antibodies directed to these polypeptides are useful in providing immunological probes for differential identification of the tissue(s) or cell type(s). For a number of disorders of the above tissues or cells, particularly of the immune system and reproductive system, expression of this gene at significantly higher or lower levels may be routinely detected in certain tissues and cell types (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for immune disorders and cancers.

### FEATURES OF PROTEIN ENCODED BY GENE NO: 151

This gene is expressed primarily in testis and to a lesser extent in T cell, spinal cord, placenta, neutrophil and monocyte.

Therefore, polynucleotides and polypeptides of the invention are useful as reagents for differential identification of the tissue(s) or cell type(s) present in a biological sample and for diagnosis of diseases and conditions: male reproductive and endocrine disorders. Similarly, polypeptides and antibodies directed to these polypeptides are useful in providing immunological probes for differential identification of the tissue(s) or cell type(s). For a number of disorders of the above tissues or cells, particularly of the reproductive, immune and endocrine systems, expression of this gene at significantly higher or lower levels may be routinely detected in certain tissues and cell types (e.g., testis and other reproductive tissue, blood cells, tissue of the nervous system, and placenta, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for regulating immune and reproductive functions.

### FEATURES OF PROTEIN ENCODED BY GENE NO: 152

The translation product of this gene shares sequence homology with tyrosyl-tRNA synthetase which is thought to be important in cell growth.

This gene is expressed primarily in brain, liver, keratinocytes, tonsils, and heart.

Therefore, polynucleotides and polypeptides of the invention are useful as reagents for differential identification of the tissue(s) or cell type(s) present in a biological sample and for diagnosis of diseases and conditions which include, but are not limited to, cancer autoimmune diseases. Similarly, polypeptides and antibodies directed to these polypeptides are useful in providing immunological probes for differential identification of the tissue(s) or cell type(s). For a number of disorders of the above tissues or cells, particularly of the brain, liver, keratinocytes, tonsils, heart expression of this gene at significantly higher or lower levels may be routinely detected in certain tissues and cell types (e.g., brain and other tissues of the nervous system, liver, keratinocytes, tonsils and heart, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to tyrosyl-tRNA synthetase indicate that polynucleotides and polypeptides corresponding to this gene are useful for modulating cell growth.

### FEATURES OF PROTEIN ENCODED BY GENE NO: 153

This gene is homologous to the Drosophila transcriptional regulator dre4. (See Accession No. 2511745.) Dre4 is a gene required for steroidogenesis in Drosophila melanogaster and encodes a developmentally expressed homologue of the yeast transcriptional regulator CDC68. Preferred polypeptide fragments comprise the amino acid sequence: KKRHTDVQFYTEVGEITTDLGKHQHMHDRDDLYAEQMEREMRHKLKTAFKN FIEKVEALTKEELEFEVPFRDLGFNGAPYRSTCLLQPTSSALVNATEWPPFVVTLDEVELIHFXR VQFHLKNFDMVIVYKDYSKKVTMINAIPVASLDPIKEWLNSCDLKYTEGVQSLNWfKIMKTIVD DPEGFFEQGGWSFL (SEQ ID NO: 639), as well as N-terminal and C-terminal deletions of this fragments. Also preferred are polynucleotide fragments encoding this polypeptide fragment.

This gene is expressed primarily in fetal liver, spleen, placenta, lung, T cell, thyroid, testes.

Therefore, polynucleotides and polypeptides of the invention are useful as reagents for differential identification of the tissue(s) or cell type(s) present in a biological sample and for diagnosis of diseases and conditions: brain tumor, heart and liver diseases. Similarly, polypeptides and antibodies directed to these polypeptides are useful in providing immunological probes for differential identification of the tissue(s) or cell type(s). For a number of disorders of the above tissues or cells, particularly of the fetal liver, spleen, placenta, lung, T cell, thyroid, testes expression of this gene at significantly higher or lower levels may be routinely detected in certain tissues and cell types (e.g., liver, spleen, placenta, lung, blood cells, thyroid, and testes and other reproductive tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

### FEATURES OF PROTEIN ENCODED BY GENE NO: 154

This gene is expressed primarily in brain and to a lesser extent in fetal heart, testis, spleen, lung.

Therefore, polynucleotides and polypeptides of the invention are useful as reagents for differential identification of the tissue(s) or cell type(s) present in a biological sample and for diagnosis of diseases and conditions: heart, liver and spleen diseases, immunological diseases. Similarly, polypeptides and antibodies directed to these polypeptides are useful in providing immunological probes for differential identification of the tissue(s) or cell type(s). For a number of disorders of the above tissues or cells, particularly of the brain, fetal heart, testis, spleen, lung expression of this gene at significantly higher or lower levels may be routinely detected in certain tissues and cell types (e.g., brain and other tissue of the nervous system, heart, testes and other reproductive tissue, spleen, and lung, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

### FEATURES OF PROTEIN ENCODED BY GENE NO: 155

Activation of T cells through the T cell antigen receptor (TCR) results in the rapid tyrosine phosphorylation of a number of cellular proteins, one of the earliest being a 100 kDa protein. This gene is the human equivalent of murine valosin containing protein (VCP). VCP is a member of a family of ATP binding, homo-oligomeric proteins, and the mammalian homolog of Saccharomyces cerevisiae cdc48p, a protein essential to the completion of mitosis in yeast. Both endogenous and expressed murine VCP are tyrosine phosphorylated in response to T cell activation. Thus we have identified a novel component of the TCR mediated tyrosine kinase activation pathway that may provide a link between TCR activation and cell cycle control.

This gene is expressed primarily in brain, liver, spleen, placenta.

Therefore, polynucleotides and polypeptides of the invention are useful as reagents for differential identification of the tissue(s) or cell type(s) present in a biological sample and for diagnosis of diseases and conditions which include, but are not limited to, cancer immunological disorders. Similarly, polypeptides and antibodies directed to these polypeptides are useful in providing immunological probes for differential identification of the tissue(s) or cell type(s). For a number of disorders of the above tissues or cells, particularly of the brain, liver, spleen, placenta expression of this gene at significantly higher or lower levels may be routinely detected in certain tissues and cell types (e.g., brain and other tissue of the nervous system, liver, spleen, and placenta, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to VCR indicate that polynucleotides and polypeptides corresponding to this gene are useful for treating cancer.

### FEATURES OF PROTEIN ENCODED BY GENE NO: 156

The translation product of this gene shares sequence homology with rat growth response protein which is thought to be important in cell growth. A group recently cloned the human homolog of this gene, calling it insulin induced protein 1. (See Accession No. 2358269, see also, Genomics 43 (3), 278-284 (1997).) Preferred polypeptide fragments comprise the amino acid sequence: RSGLGLGITIAFLATLITQF LVYNGVYQYTSPDFLYIRSWLPCIFFSGGVTVGNIGRQLAMGVPEKPHSD (SEQ ID NO: 640), as well as N-terminal and C-terminal deletions of this polypeptide fragment. Also preferred are polynucleotide fragments encoding these polypeptide fragments.

This gene is expressed primarily in brain, liver, placenta, heart, spleen, lymphoma.

Therefore, polynucleotides and polypeptides of the invention are useful as reagents for differential identification of the tissue(s) or cell type(s) present in a biological sample and for diagnosis of diseases and conditions which include, but are not limited to, cancer immunological disorders. Similarly, polypeptides and antibodies directed to these polypeptides are useful in providing immunological probes for differential identification of the tissue(s) or cell type(s). For a number of disorders of the above tissues or cells, particularly of the brain, liver, placenta, heart, spleen. expression of this gene at significantly higher or lower levels may be routinely detected in certain tissues and cell types (e.g., brain and other tissue of the nervous system, liver, placenta, heart, spleen, and lymphoid tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to growth-response protein indicate that polynucleotides and polypeptides corresponding to this gene are useful for modulating cell growth.

### FEATURES OF PROTEIN ENCODED BY GENE NO: 157

This gene is expressed primarily in Glioblastoma, endometrial tumor, lymphoma and pancreas tumor.

Therefore, polynucleotides and polypeptides of the invention are useful as reagents for differential identification of the tissue(s) or cell type(s) present in a biological sample and for diagnosis of diseases and conditions: Glioblastoma, Endometrial tumor, lymphoma and pancreas tumor. Similarly, polypeptides and antibodies directed to these polypeptides are useful in providing immunological probes for differential identification of the tissue(s) or cell type(s). For a number of disorders of the above tissues or cells, particularly of the immune, expression of this gene at significantly higher or lower levels may be routinely detected in certain tissues and cell types (e.g., endometrium, lymphoid tissue, pancreas, and tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

### FEATURES OF PROTEIN ENCODED BY GENE NO: 158

The translation product of this gene shares sequence homology with IGE receptor which is thought to be important in allergy and asthma.

This gene is expressed primarily in T cell, and fetal liver.

Therefore, polynucleotides and polypeptides of the invention are useful as reagents for differential identification of the tissue(s) or cell type(s) present in a biological sample and for diagnosis of diseases and conditions: allergy and asthma and other immunological disorders. Similarly, polypeptides and antibodies directed to these polypeptides are useful in providing immunological probes for differential identification of the tissue(s) or cell type(s). For a number of disorders of the above tissues or cells, particularly of the immune, expression of this gene at significantly higher or lower levels may be routinely detected in certain tissues and cell types (e.g., blood cells, and liver, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to IgE receptor indicate that polynucleotides and polypeptides corresponding to this gene are useful for allergy and asthma.

### FEATURES OF PROTEIN ENCODED BY GENE NO: 159

The translation product of this gene shares sequence homology with immunoglobin heavy chain which is thought to be important in immune response to the antigen.

This gene is expressed primarily in activated neutrophil and to a lesser extent in activated T cell, monocyte and heart.

Therefore, polynucleotides and polypeptides of the invention are useful as reagents for differential identification of the tissue(s) or cell type(s) present in a biological sample and for diagnosis of diseases and conditions: infection , inflammation and cancer. Similarly, polypeptides and antibodies directed to these polypeptides are useful in providing immunological probes for differential identification of the tissue(s) or cell type(s). For a number of disorders of the above tissues or cells, particularly of the immune, expression of this gene at significantly higher or lower levels may be routinely detected in certain tissues and cell types (e.g., blood cells, and heart, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to immunoglobin heavy chain variable region indicate that polynucleotides and polypeptides corresponding to this gene are useful for making the ligand to block specific antigen which cause certain disease.

### FEATURES OF PROTEIN ENCODED BY GENE NO: 160

The translation product of this gene shares sequence homology with mouse X inactive specific transcript protein which is thought to be important in X chromosome inactivation.

This gene is expressed primarily in HSA172 cell and to a lesser extent in normal ovary tissue, ovarian cancer, frontal cortex and brain.

Therefore, polynucleotides and polypeptides of the invention are useful as reagents for differential identification of the tissue(s) or cell type(s) present in a biological sample and for diagnosis of diseases and conditions: ovarian tumor, schizophrenia and other neurological disorders. Similarly, polypeptides and antibodies directed to these polypeptides are useful in providing immunological probes for differential identification of the tissue(s) or cell type(s). For a number of disorders of the above tissues or cells, particularly of the immune and neural system, expression of this gene at significantly higher or lower levels may be routinely detected in certain tissues and cell types (e.g., ovary and other reproductive tissue, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to X inactive specific transcript protein indicate that polynucleotides and polypeptides corresponding to this gene are useful for diagnosis and treatment of reproductive system tumors and CNS tumors.

### FEATURES OF PROTEIN ENCODED BY GENE NO: 161

This gene is expressed primarily in adipose cell and to a lesser extent in liver and prostate.

Therefore, polynucleotides and polypeptides of the invention are useful as reagents for differential identification of the tissue(s) or cell type(s) present in a biological sample and for diagnosis of diseases and conditions: obesity and liver disorder. Similarly, polypeptides and antibodies directed to these polypeptides are useful in providing immunological probes for differential identification of the tissue(s) or cell type(s). For a number of disorders of the above tissues or cells, particularly of the adipose cell, expression of this gene at significantly higher or lower levels may be routinely detected in certain tissues and cell types (e.g., adipose cells, liver, and prostate, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment of obesity and liver disorder.

### FEATURES OF PROTEIN ENCODED BY GENE NO: 162

The translation product of this gene shares sequence homology with yeast ubiquitin activating enzyme homolog which is thought to be important in protein posttraslation processing.

This gene is expressed primarily in stromal cell and to a lesser extent in retina, H. Atrophic Endometrium, colon carcinoma and myeloid progenitor cell.

Therefore, polynucleotides and polypeptides of the invention are useful as reagents for differential identification of the tissue(s) or cell type(s) present in a biological sample and for diagnosis of diseases and conditions: defects of stromal cell development, neuronal growth disorders and tumors. Similarly, polypeptides and antibodies directed to these polypeptides are useful in providing immunological probes for differential identification of the tissue(s) or cell type(s). For a number of disorders of the above tissues or cells, particularly of the immune and neural system, expression of this gene at significantly higher or lower levels may be routinely detected in certain tissues and cell types (e.g., retinal cells, endometrium, colon, and bone marrow, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to ubiquitin-activating enzyme homolog indicate that polynucleotides and polypeptides corresponding to this gene are useful for diagnosis or treatment of some type of tumors , fucosidosis and neuronal growth disorders.

### FEATURES OF PROTEIN ENCODED BY GENE NO: 163

This gene is expressed primarily in primary breast cancer and hemangiopericytoma and to a lesser extent in adult brain and cerebellum.

Therefore, polynucleotides and polypeptides of the invention are useful as reagents for differential identification of the tissue(s) or cell type(s) present in a biological sample and for diagnosis of diseases and conditions: breast cancer, leukemia and cerebellum disorders. Similarly, polypeptides and antibodies directed to these polypeptides are useful in providing immunological probes for differential identification of the tissue(s) or cell type(s). For a number of disorders of the above tissues or cells, particularly of the immune system and neural system, expression of this gene at significantly higher or lower levels may be routinely detected in certain tissues and cell types (e.g., mammary tissue, brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis or treatment of various tumors and disease involved in neural system.

### FEATURES OF PROTEIN ENCODED BY GENE NO: 164

The translation product of this gene shares sequence homology with proline rich proteins. Recently, another group has also cloned this gene, calling it CD84 leukocyte antigen, a new member of the Ig superfamily. (See Accession No. U82988, see also, Blood 90 (6), 2398-2405 (1997).)

This gene is expressed primarily in Weizmann olfactory tissue and osteoclastoma and to a lesser extent in anergic T-cell.

Therefore, polynucleotides and polypeptides of the invention are useful as reagents for differential identification of the tissue(s) or cell type(s) present in a biological sample and for diagnosis of diseases and conditions: ostsis and immune disease. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., olfactory tissue, bone, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to the Ig superfamily indicate that the protein product of this clone is useful for treatment of osteoporosis, autoimmune disease, and other immune disorders.

### FEATURES OF PROTEIN ENCODED BY GENE NO: 165

This gene is expressed primarily in atrophic endometrium and colon cancer and to a lesser extent in some fetal tissues.

Therefore, polynucleotides and polypeptides of the invention are useful as reagents for differential identification of the tissue(s) or cell type(s) present in a biological sample and for diagnosis of diseases and conditions: tumors. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., endometrium, colon, and fetal tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of tumors, specifically endometrium and colon tumors.

### FEATURES OF PROTEIN ENCODED BY GENE NO: 166

This gene is expressed primarily in human primary breast cancer and to a lesser extent in activated monocyte. Although the predicted signal sequence is identified in Table 1, other upstream sequences are also relevant. Preferred polypeptide fragments comprise the amino acid sequence: VTQPKHLSASMGGSVEIPFSFYYPWELAXXPXVRISWRRGHFHG QSFYSTRPPSIHKDYVNRLFLNWTEGQESGFLRISNLRKEDQSVYFCRVELDTRRSG (SEQ ID NO: 641), as well as N-terminal and C-terminal deletions. Also preferred are polynucleotide fragments encoding these polypeptide fragments.

Therefore, polynucleotides and polypeptides of the invention are useful as reagents for differential identification of the tissue(s) or cell type(s) present in a biological sample and for diagnosis of diseases and conditions: breast cancer. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., mammary tissue, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis of breast cancer.

### FEATURES OF PROTEIN ENCODED BY GENE NO: 167

This gene is expressed primarily in fetal tissues and to a lesser extent in adult lung. This gene has also been mapped to chromosomal location 9q34, and thus, can be used as a marker for linkage analysis for chromosome 9.

Therefore, polynucleotides and polypeptides of the invention are useful as reagents for differential identification of the tissue(s) or cell type(s) present in a biological sample. Similarly, polypeptides and antibodies directed to these polypeptides are useful in providing immunological probes for differential identification of the tissue(s) or cell type(s). For a number of disorders of the above tissues or cells, particularly of the embryo tissues, expression of this gene at significantly higher or lower levels may be routinely detected in certain tissues and cell types (e.g., fetal tissues, and lung, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

### FEATURES OF PROTEIN ENCODED BY GENE NO: 168

The translation product of this gene shares sequence homology with Ig Heavy Chain which is thought to be important in immune response.

This gene is expressed primarily in prostate cancer tissue specifically

Therefore, polynucleotides and polypeptides of the invention are useful as reagents for differential identification of the tissue(s) or cell type(s) present in a biological sample and for diagnosis of diseases and conditions: prostate cancer. Similarly, polypeptides and antibodies directed to these polypeptides are useful in providing immunological probes for differential identification of the tissue(s) or cell type(s). For a number of disorders of the above tissues or cells, particularly of the prostate, expression of this gene at significantly higher or lower levels may be routinely detected in certain tissues and cell types (e.g., prostate, tissue and cells of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

### FEATURES OF PROTEIN ENCODED BY GENE NO: 169

The translation product of this gene shares sequence homology with cytosolic acyl coenzyme-A hydrolase, which is thought to be important in neuron-specific fatty acid metabolism. The gene represented by this contig has since been published by Hajra and colleagues (GenBank Accession No. U91316).

This gene is expressed primarily in human pituitary gland and to a lesser extent in colorectal cancer tissue. This gene has also been observed in the LNCAP cell line.

Therefore, polynucleotides and polypeptides of the invention are useful as reagents for differential identification of the tissue(s) or cell type(s) present in a biological sample and for diagnosis of diseases and conditions: hyperlipidemias of familial and/or idiopathic origins. Similarly, polypeptides and antibodies directed to these polypeptides are useful in providing immunological probes for differential identification of the tissue(s) or cell type(s). For a number of disorders of the above tissues or cells, particularly blood, expression of this gene at significantly higher or lower levels may be routinely detected in certain tissues and cell types (e.g., pituitary and colon, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to rat cytosolic acyl coenzyme-A hydrolase indicate that polynucleotides and polypeptides corresponding to this gene are useful for the detection or treatment of hyperlipidemia disease states by virtue of the ability of specific drugs to activate the enzyme.

### FEATURES OF PROTEIN ENCODED BY GENE NO: 170

The translation product of this gene shares sequence homology with a *Caenorhabditis elegans* gene which is thought to be important in organism development.

This gene is expressed primarily in human synovial sarcoma tissue, bone marrow, and to a lesser extent in human brain.

Therefore, polynucleotides and polypeptides of the invention are useful as reagents for differential identification of the tissue(s) or cell type(s) present in a biological sample and for diagnosis of diseases and conditions which include, but are not limited to, of bone, specifically synovial sarcoma. Similarly, polypeptides and antibodies directed to these polypeptides are useful in providing immunological probes for differential identification of the tissue(s) or cell type(s). For a number of disorders of the above tissues or cells, particularly of the bone, connective tissues and possibly immune system, expression of this gene at significantly higher or lower levels may be routinely detected in certain tissues and cell types (e.g., synovial tissue, bone marrow, brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to Caenorhabditis elegans indicate that polynucleotides and polypeptides corresponding to this gene are useful as a diagnostic and/or therapeutic modality directed at the detection and/or treatment of connective tissue sarcomas or other related bone diseases.

### FEATURES OF PROTEIN ENCODED BY GENE NO: 171

The translation product of this gene shares sequence homology with betal-6GlcNAc transferase which is thought to be important in the transfer and metabolism of beta 1-6, N-acetylglucosamine. This gene product has previously been shown to suppress melanoma lung metastasis in both syngeneic and nude mice, decreased invasiveness into the matrigel, and inhibition of cell attachment to collagen and laminin without affecting cell growth.

This gene is expressed primarily in human testes and prostate tissues, and to a lesser extent in kidney, medulla, and pancreas.

Therefore, polynucleotides and polypeptides of the invention are useful as reagents for differential identification of the tissue(s) or cell type(s) present in a biological sample and for diagnosis of diseases and conditions which include, but are not limited to, cancer particularly melanoma. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., testes and other reproductive tissue, prostate, kidney, pancreas, brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to beta1-6GlcNAc transferase indicate that the protein product of this clone is useful for the development of diagnostic and/or therapeutic modalities directed at the detection and/or treatment of cancer, the metastasis of malignant tissue or cells. Defects in this potentially secreted enzyme may play a role in metastasis.

### FEATURES OF PROTEIN ENCODED BY GENE NO: 172

This gene is expressed primarily in fetal spleen and liver.

Therefore, polynucleotides and polypeptides of the invention are useful as reagents for differential identification of the tissue(s) or cell type(s) present in a biological sample and for diagnosis of diseases and conditions: immune disorders, Wilm's tumor disease, hepatic disorders, and hematopoietic disorders. Similarly, polypeptides and antibodies directed to these polypeptides are useful in providing immunological probes for differential identification of the tissue(s) or cell type(s). For a number of disorders of the above tissues or cells, particularly of the hematopoiesis and immune systems, expression of this gene at significantly higher or lower levels may be routinely detected in certain tissues and cell types (e.g., spleen and liver, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and identification of fetal defects along with correcting diseases that affect hematopoiesis and the immune system.

### FEATURES OF PROTEIN ENCODED BY GENE NO: 173

The translation product of this gene shares sequence homology with ret II oncogene which is thought to be important in Hirschsprung disease and many types of cancers.

This gene is expressed in multiple tissues including the lymphatic system, brain, and thyroid.

Therefore, polynucleotides and polypeptides of the invention are useful as reagents for identification of the tissue(s) or cell type(s) present in a biological sample and for diagnosis of diseases and conditions: Hirschsprung disease and multiple cancers. Similarly, polypeptides and antibodies directed to these polypeptides are useful in providing immunological probes for identification of the tissue(s) or cell type(s). For a number of disorders of the above tissues or cells, particularly of the immune and central nervous system, expression of this gene at significantly higher or lower levels may be routinely detected in certain tissues and cell types (e.g., lymphoid tissue, thyroid, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to ret II oncogene indicate that polynucleotides and polypeptides corresponding to this gene are useful for diagnosis and treatment of various cancers. It would also be useful for the diagnosis and treatment of Hirschsprung disease. Preferred polypeptides of the invention comprise the amino acid sequence: MEAQQVNEAESAREQLQXLHDQIAGQKASKQELETELERLKQEFHYIEEDLY RTKNTLQSRIKDRDEEIQKLRNQLTNKTLSNSSQSELENRLHQLTETLIQKQTMLESLSTEKNSL VFQLERLEQQMNSASGSSSNGSSINMSGIDNGEGTRLRNVPVLFNDTETNLAGMYGKVRKAAS SIDQFSIRLGIFLRRYPIARVFVIIYMALLHLWVMIVLLTYTPEM HHDQPYGK (SEQ ID NO: 642).

### FEATURES OF PROTEIN ENCODED BY GENE NO: 174

The translation product of this gene shares sequence homology with testis enhanced gene transcript which is thought to be important in regulation of human development.

This gene is expressed primarily in infant brain and to a lesser extent in a variety of other tissues and cell types, including the prostate, testes, monocytes, macrophages, dendritic cells, keratinocytes, and adipocytes.

Therefore, polynucleotides and polypeptides of the invention are useful as reagents for differential identification of the tissue(s) or cell type(s) present in a biological sample and for diagnosis of diseases and conditions: neurological, developmental, immune and inflammation disorders. Similarly, polypeptides and antibodies directed to these polypeptides are useful in providing immunological probes for differential identification of the tissue(s) or cell type(s). For a number of disorders of the above tissues or cells, particularly of the brain and immune systems, expression of this gene at significantly higher or lower levels may be routinely detected in certain tissues and cell types (e.g., brain and other tissue of the nervous system, prostate, testes and other reproductive tissue, blood cells, keratinocytes, and adipocytes, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to testis enhanced gene transcript indicate that the protein product of this clone is useful for diagnosis and treatment of disorders involving the developing brain and the immune system.

### FEATURES OF PROTEIN ENCODED BY GENE NO: 175

This gene is expressed primarily in prostate and to a lesser extent in various other tissues, including placenta.

Therefore, polynucleotides and polypeptides of the invention are useful as reagents for differential identification of the tissue(s) or cell type(s) present in a biological sample and for diagnosis of diseases and conditions which include, but are not limited to, cancers, especially of the prostate. Similarly, polypeptides and antibodies directed to these polypeptides are useful in providing immunological probes for differential identification of the tissue(s) or cell type(s). For a number of disorders of the above tissues or cells, particularly of the prostate, expression of this gene at significantly higher or lower levels may be routinely detected in certain tissues and cell types (e.g., prostate and placenta, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone is useful for diagnosis and treatment of prostate disorders and cancer. It may also be useful for the diagnosis and treatment of endocrine disorders.

### FEATURES OF PROTEIN ENCODED BY GENE NO: 176

The translation product of this gene shares sequence homology with *Sacchromyces cerevisiae* YNT20 gene which is thought to be important in mitochondrial function.

This gene is expressed at a particularly high level in muscle tissue.

Therefore, polynucleotides and polypeptides of the invention are useful as reagents for differential identification of the tissue(s) or cell type(s) present in a biological sample and for diagnosis of diseases related to such tissues and cell types including: muscle wasting diseases. Similarly, polypeptides and antibodies directed to these polypeptides are useful in providing immunological probes for differential identification of the tissue(s) or cell type(s). For a number of disorders of the above tissues or cells, particularly of the neuromuscular system, expression of this gene at significantly higher or lower levels may be routinely detected in certain tissues and cell types (e.g., muscle and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to the YNT20 gene indicate that this protein is useful for treatment and detection of neuromuscular diseases caused by loss of mitochondrial function. For example this gene or its protein product could be used in replacement therapy for such diseases.

### FEATURES OF PROTEIN ENCODED BY GENE NO: 177

This gene is expressed primarily in the brain and to a lesser extent in kidney, placenta, smooth muscle, heart and lung.

Therefore, polynucleotides and polypeptides of the invention are useful as reagents for differential identification of the tissue(s) or cell type(s) present in a biological sample and for diagnosis of diseases and conditions: neuromuscular diseases, degenerative diseases of the central nervous system, and heart disease. Similarly, polypeptides and antibodies directed to these polypeptides are useful in providing immunological probes for differential identification of the tissue(s) or cell type(s). For a number of disorders of the above tissues or cells, particularly of the neuromuscular system, central nervous system, and heart, expression of this gene at significantly higher or lower levels may be routinely detected in certain tissues and cell types (e.g., brain and other tissue of the nervous system, kidney, placenta, muscle, heart and lung, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

This gene or its protein product could also be used for replacement therapy for the above mentioned diseases.

### FEATURES OF PROTEIN ENCODED BY GENE NO: 178

The translation product of this gene shares sequence homology with caldesmon which is thought to be important in the cellular response to changes in glucose levels.

This gene is expressed primarily in multiple tissues including brain and retina.

Therefore, polynucleotides and polypeptides of the invention are useful as reagents for identification of the tissue(s) or cell type(s) present in a biological sample and for diagnosis of diseases and conditions: central nervous system disorders and retinopathy. Similarly, polypeptides and antibodies directed to these polypeptides are useful in providing immunological probes for identification of the tissue(s) or cell type(s). For a number of disorders of the above tissues or cells, particularly of the CNS disorders and retinopathy, expression of this gene at significantly higher or lower levels may be routinely detected in certain tissues and cell types (e.g., brain and other tissue of the nervous system, and retinal tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to caldesmon indicate that polynucleotides and polypeptides corresponding to this gene are useful for treatment of retinopathies.

### FEATURES OF PROTEIN ENCODED BY GENE NO: 179

The translation product of this gene shares sequence homology with mouse fibrosin protein which is thought to be important in regulation of fibrinogenesis in certain chronic inflammatory diseases.

This gene is expressed primarily in amniotic cells and breast tissue.

Therefore, polynucleotides and polypeptides of the invention are useful as reagents for differential identification of the tissue(s) or cell type(s) present in a biological sample and for diagnosis of breast cancer and abnormal embryo development. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues and cell types (e.g., amniotic cells, and mammary tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to fibrosin indicate that the protein product of this clone is useful for treatment of breast cancer. This gene or its protein product could be used in replacement therapy for breast cancer. In addition the protein product of this gene is useful in the treatment of chronic inflammatory diseases.

### FEATURES OF PROTEIN ENCODED BY GENE NO: 180

This gene is expressed several infant tissues including brain and liver and various adult tissues including brain, lung, liver, testes, and prostate.

Therefore, polynucleotides and polypeptides of the invention are useful as reagents for differential identification of the tissue(s) or cell type(s) present in a biological sample and for diagnosis of diseases and conditions which include, but are not limited to, brain cancer, lung cancer, liver cancer and cancers of the reproductive system. Similarly, polypeptides and antibodies directed to these polypeptides are useful in providing immunological probes for differential identification of the tissue(s) or cell type(s). For a number of disorders of the above tissues or cells, particularly of the central nervous system, hepatic system, and reproductive system, expression of this gene at significantly higher or lower levels may be routinely detected in certain tissues and cell types (e.g., brain and other tissue of the nervous system, lung, liver, testes and other reproductive tissue, and prostate, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution of this gene product indicates that the protein product of this clone is involved in growth regulation and could be used as a growth factor or growth blocker in a variety of settings including treatment of cancers.

### FEATURES OF PROTEIN ENCODED BY GENE NO: 181

This gene is expressed primarily in activated monocytes and to a lesser extent in melanocytes and dendritic cells.

Therefore, polynucleotides and polypeptides of the invention are useful as reagents for differential identification of the tissue(s) or cell type(s) present in a biological sample and for diagnosis of immune system diseases and cancer. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., blood cells, melanocytes, and dendritic cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone could be involved in growth regulation and could be used as a growth factor or growth blocker in a variety of settings including treatment of cancers.

### FEATURES OF PROTEIN ENCODED BY GENE NO: 182

This gene is expressed primarily in placenta and several tumors of various tissue origin and to a lesser extent in normal tissues including liver, lung, brain, and skin,

Therefore, polynucleotides and polypeptides of the invention are useful as reagents for differential identification of the tissue(s) or cell type(s) present in a biological sample and for diagnosis of cancers of all kinds. Similarly, polypeptides and antibodies directed to these polypeptides are useful in providing immunological probes for differential identification of the tissue(s) or cell type(s). For a number of disorders of the above tissues or cells, particularly of the central nervous system, respiratory system and skin, expression of this gene at significantly higher or lower levels may be routinely detected in certain tissues and cell types (e.g., liver, lung, brain and other tissues of the nervous system, and skin, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The high expression of this gene in multiple tumors indicates that the protein product of the clone may be involved in cell growth control and therefore would be useful for treatment of certain cancers. Likewise molecules developed to block the activity of the protein product of this clone could be used to block its potential role in tumor growth promotion.

### FEATURES OF PROTEIN ENCODED BY GENE NO: 183

The translation product of this gene shares sequence homology with the mouse Ndr1 gene which is thought to be important in cancer progression.

This gene is expressed multiple cell types and tissues including brain, lung, kidney, bone marrow, liver, and spleen.

Therefore, polynucleotides and polypeptides of the invention are useful as reagents for differential identification of the tissue(s) or cell type(s) present in a biological sample and for diagnosis of all types of cancers. Similarly, polypeptides and antibodies directed to these polypeptides are useful in providing immunological probes for differential identification of the tissue(s) or cell type(s). For a number of disorders of the above tissues or cells, particularly of the nervous, immune, and endocrine systems, expression of this gene at significantly higher or lower levels may be routinely detected in certain tissues and cell types (e.g., brain and other tissue of the nervous system, lung, kidney, bone marrow, liver and spleen, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to Ndr1 gene, which is thought to be involved in cancer progression, indicate that polynucleotides and polypeptides corresponding to this gene are useful for treatment of certain cancers. Likewise molecules developed to block the activity of the protein product of this clone could be used to block its potential role in tumor growth promotion.

### FEATURES OF PROTEIN ENCODED BY GENE NO: 184

This gene is expressed primarily in early stage human brain and liver and to a lesser extent in several other fetal tissues.

Therefore, polynucleotides and polypeptides of the invention are useful as reagents for differential identification of the tissue(s) or cell type(s) present in a biological sample and for diagnosis of diseases and conditions: brain and liver cancers. Similarly, polypeptides and antibodies directed to these polypeptides are useful in providing immunological probes for differential identification of the tissue(s) or cell type(s). For a number of disorders of the above tissues or cells, particularly of the central nervous system and immune system, expression of this gene at significantly higher or lower levels may be routinely detected in certain tissues and cell types (e.g., brain and other tissue of the nervous system, liver, and fetal tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The expression of this gene in embryonic tissues indicates that the protein could be involved in growth regulation and could be used as a growth factor or growth blocker in a variety of settings including treatment of cancers.

### FEATURES OF PROTEIN ENCODED BY GENE NO: 185

This gene is expressed primarily in infant and embryonic brain.

Therefore, polynucleotides and polypeptides of the invention are useful as reagents for differential identification of the tissue(s) or cell type(s) present in a biological sample and for diagnosis of degenerative nervous system disorders and brain cancer. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues and cell types (e.g., embryonic tissue, brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The expression of this gene in embryonic tissues indicates that the protein could be involved in growth regulation and could be used as a growth factor or growth blocker in a variety of settings including treatment of cancers.

### FEATURES OF PROTEIN ENCODED BY GENE NO: 186

This gene is expressed primarily in multiple tissues including placenta, fetal lung, fetal liver, and brain.

Therefore, polynucleotides and polypeptides of the invention are useful as reagents for differential identification of the tissue(s) or cell type(s) present in a biological sample and for diagnosis of all types of cancers including liver, brain and lung. Similarly, polypeptides and antibodies directed to these polypeptides are useful in providing immunological probes for differential identification of the tissue(s) or cell type(s). For a number of disorders of the above tissues or cells, particularly of the central nervous system, pulmonary system, and hepatic system, expression of this gene at significantly higher or lower levels may be routinely detected in certain tissues and cell types (e.g., placenta, lung, liver, and brain and other tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The expression of this gene in embryonic tissues indicates that the protein could be involved in growth regulation and could be used as a growth factor or growth blocker in a variety of settings including treatment of cancers.

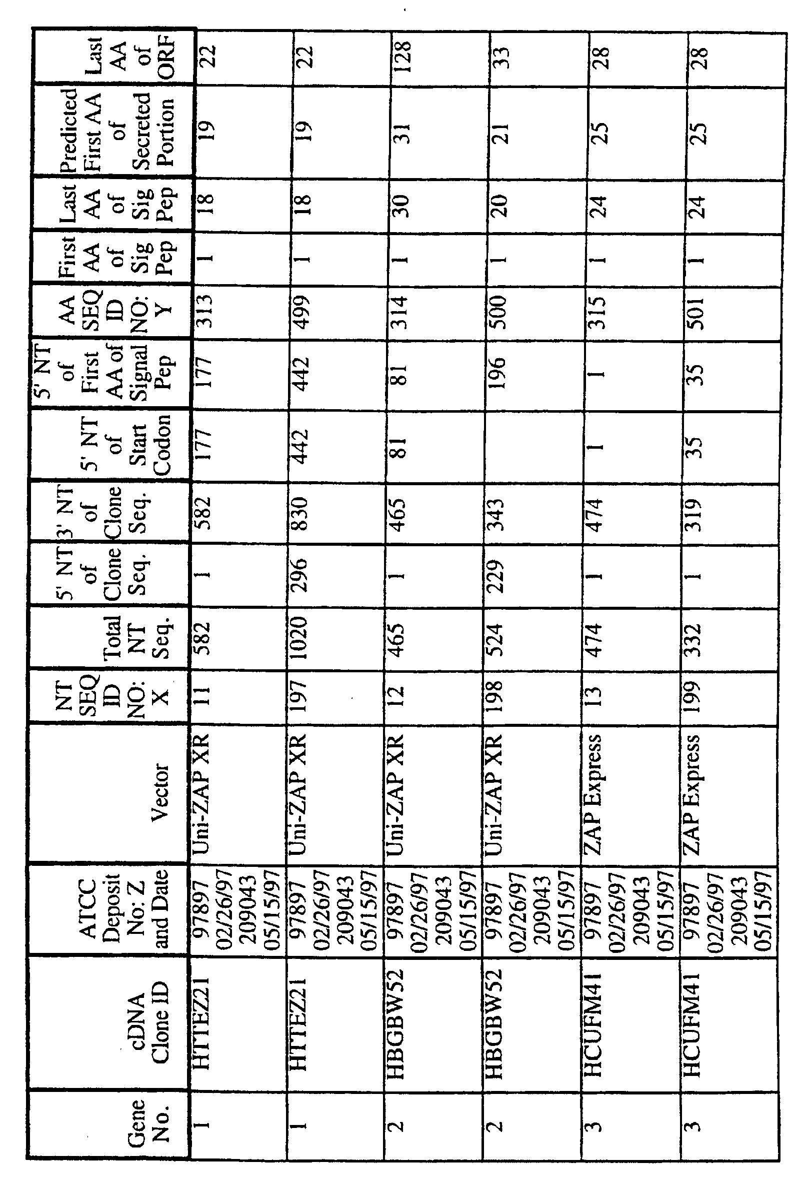

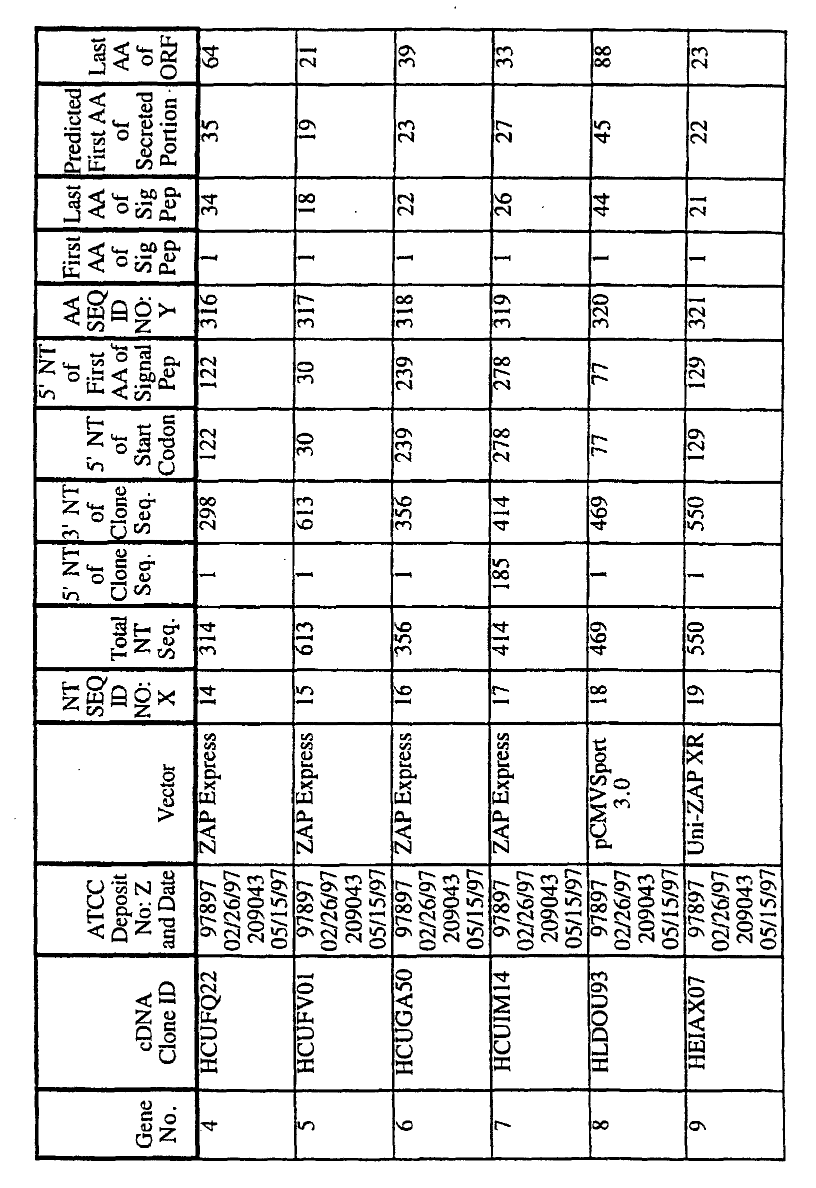

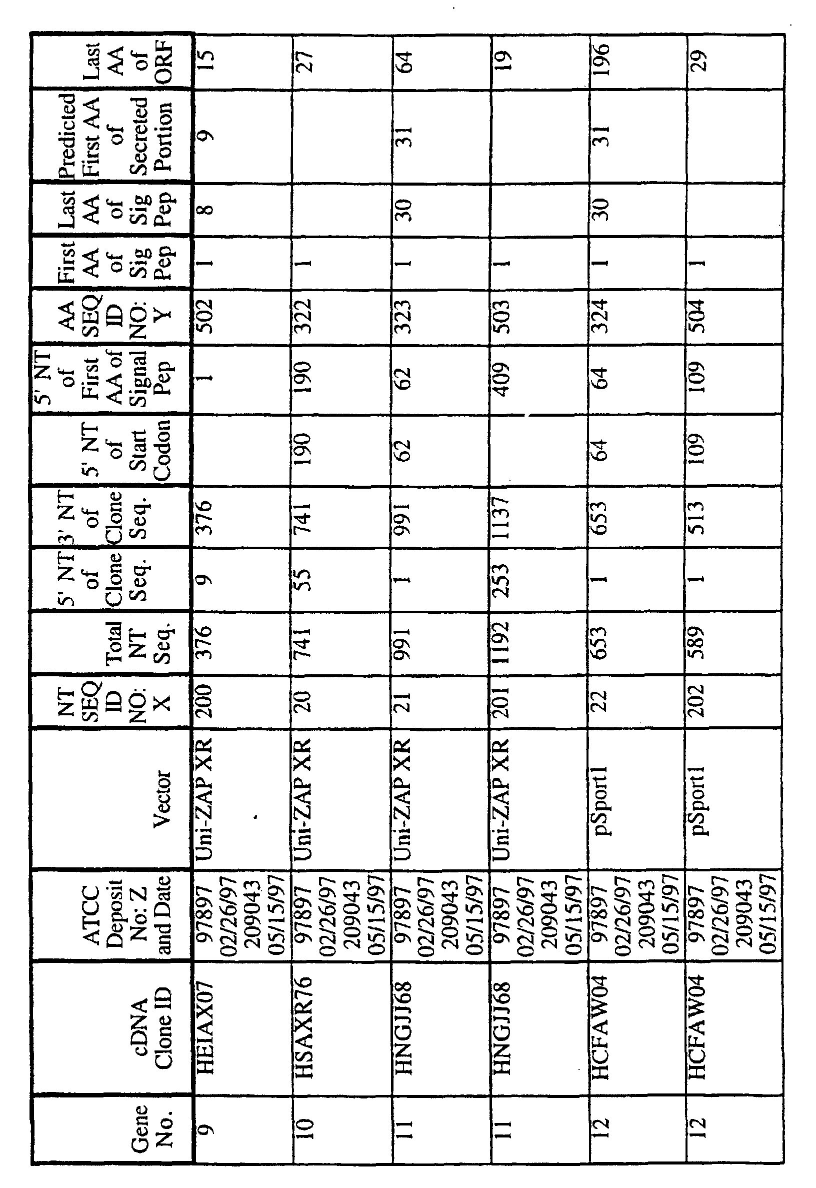

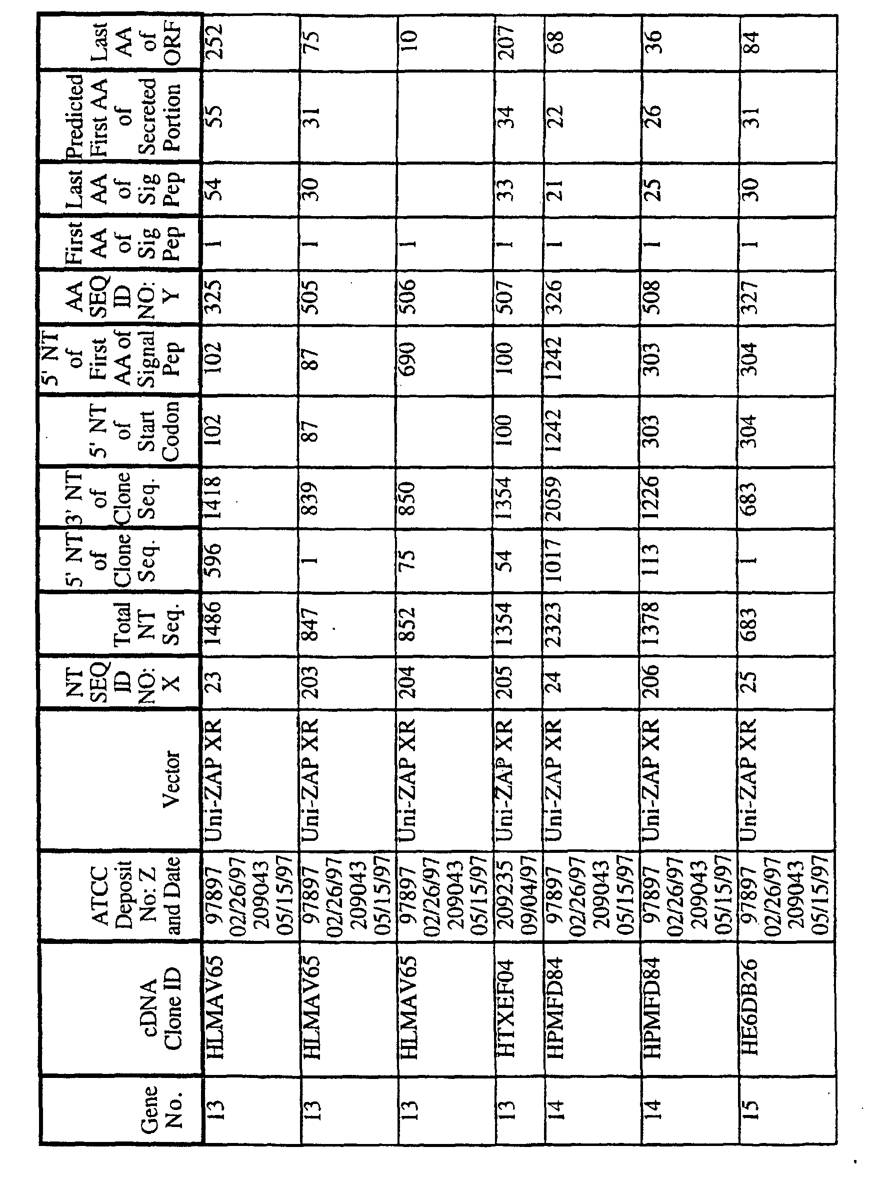

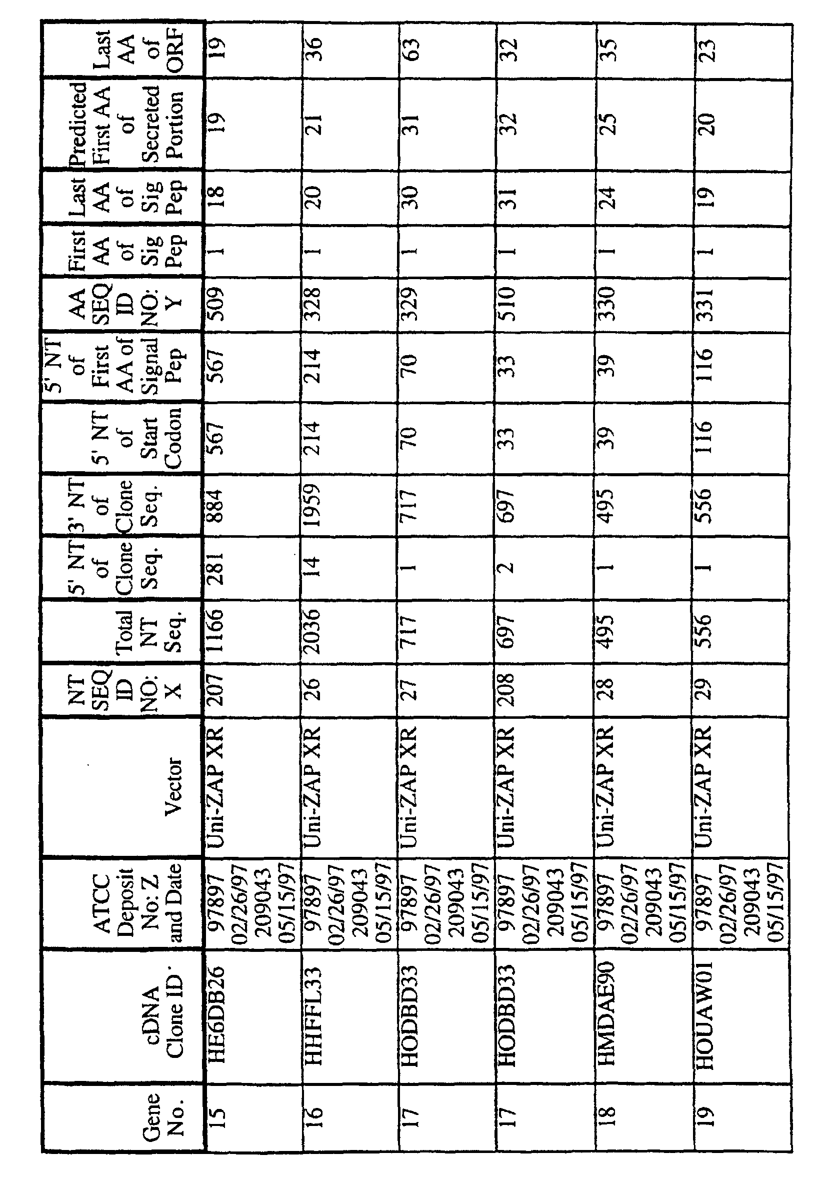

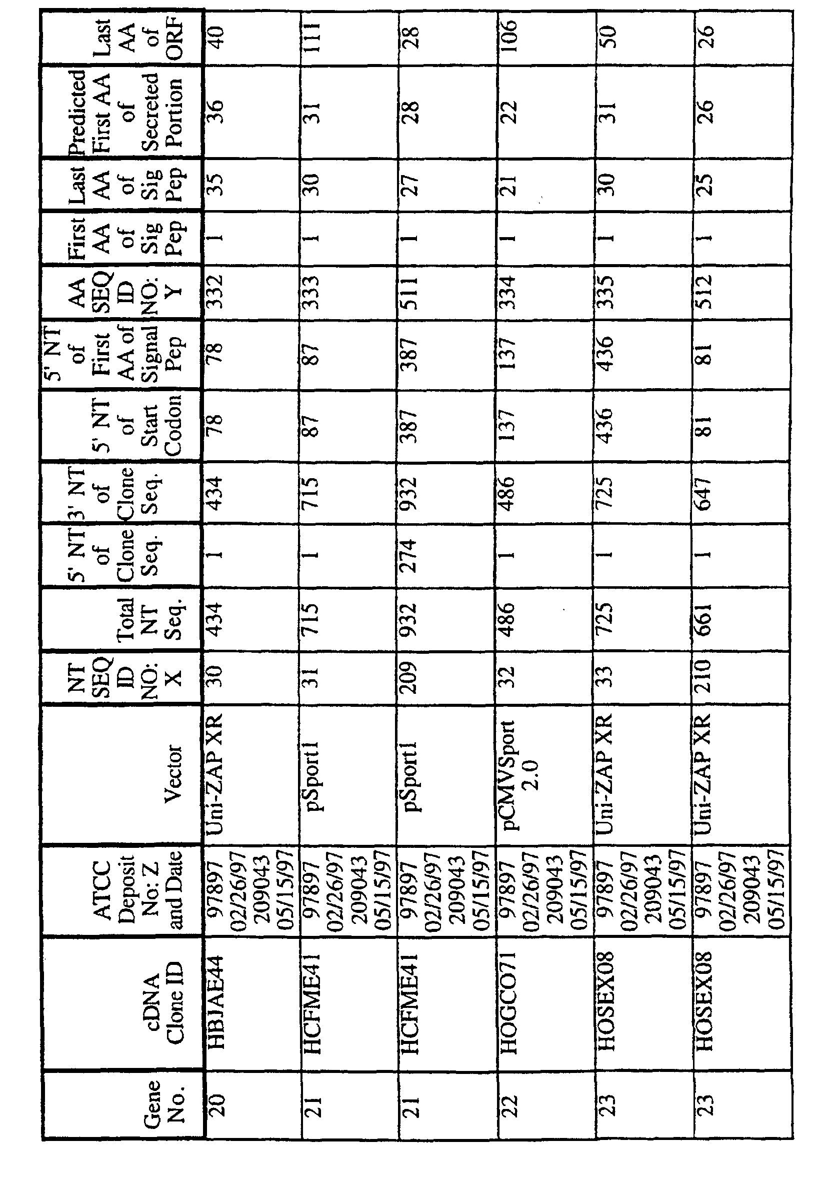

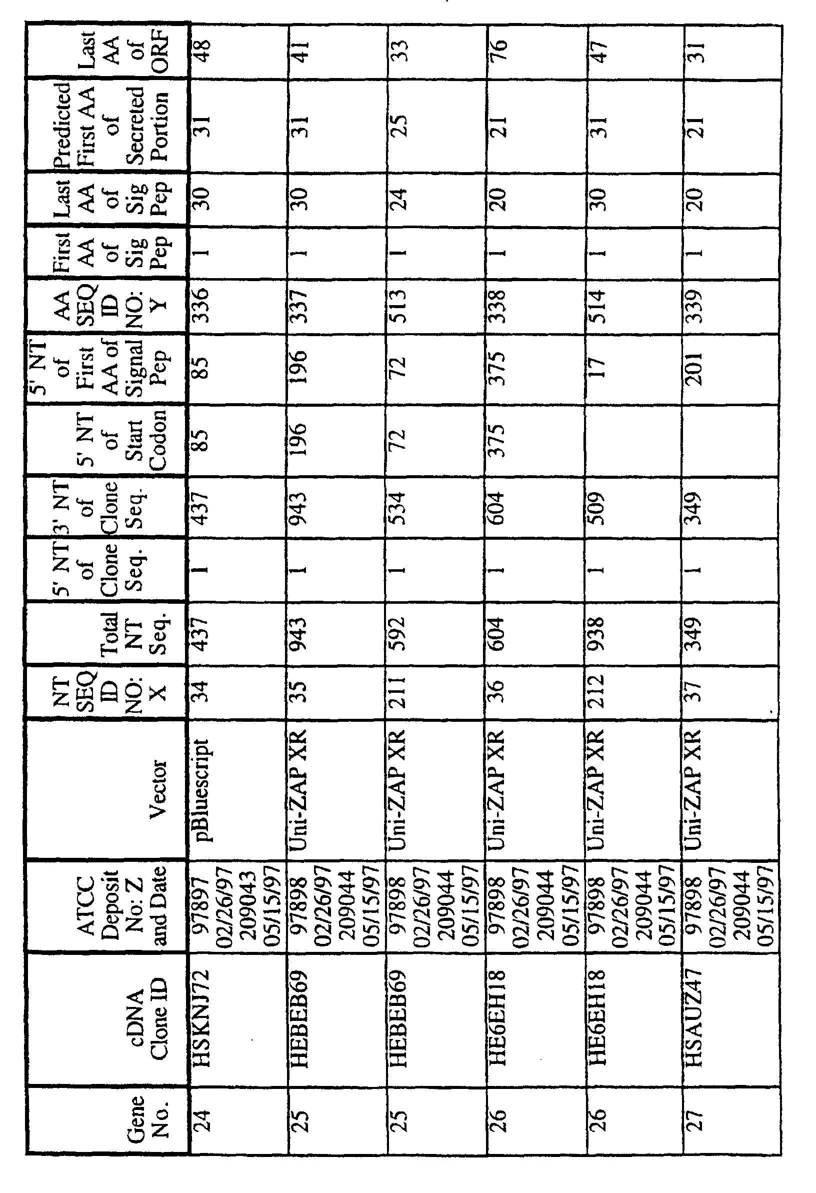

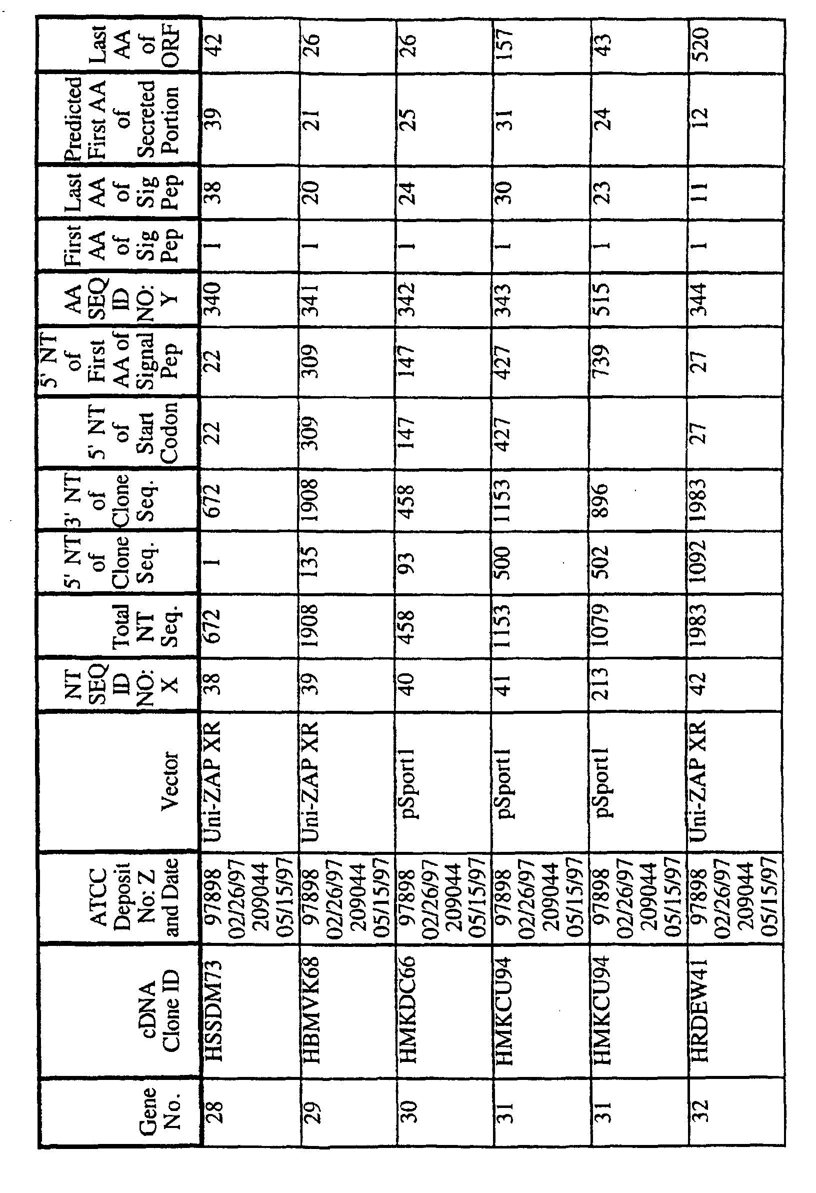

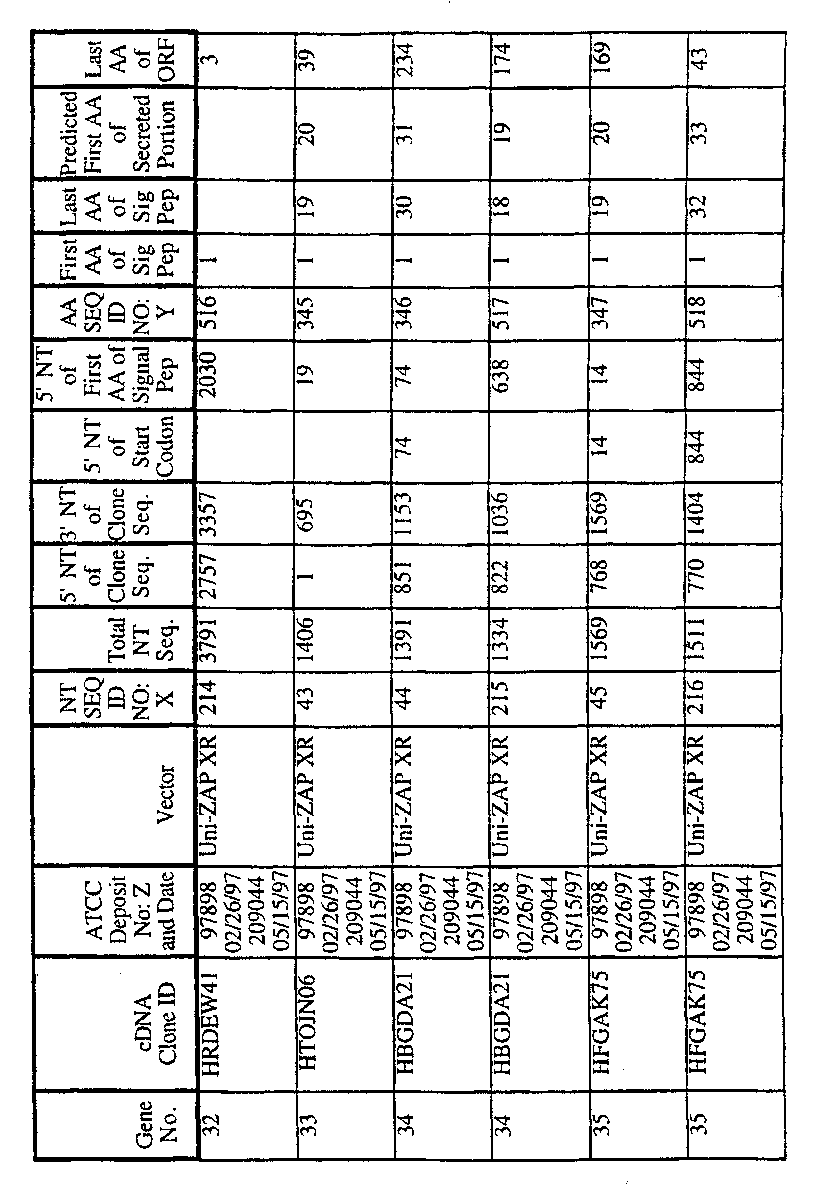

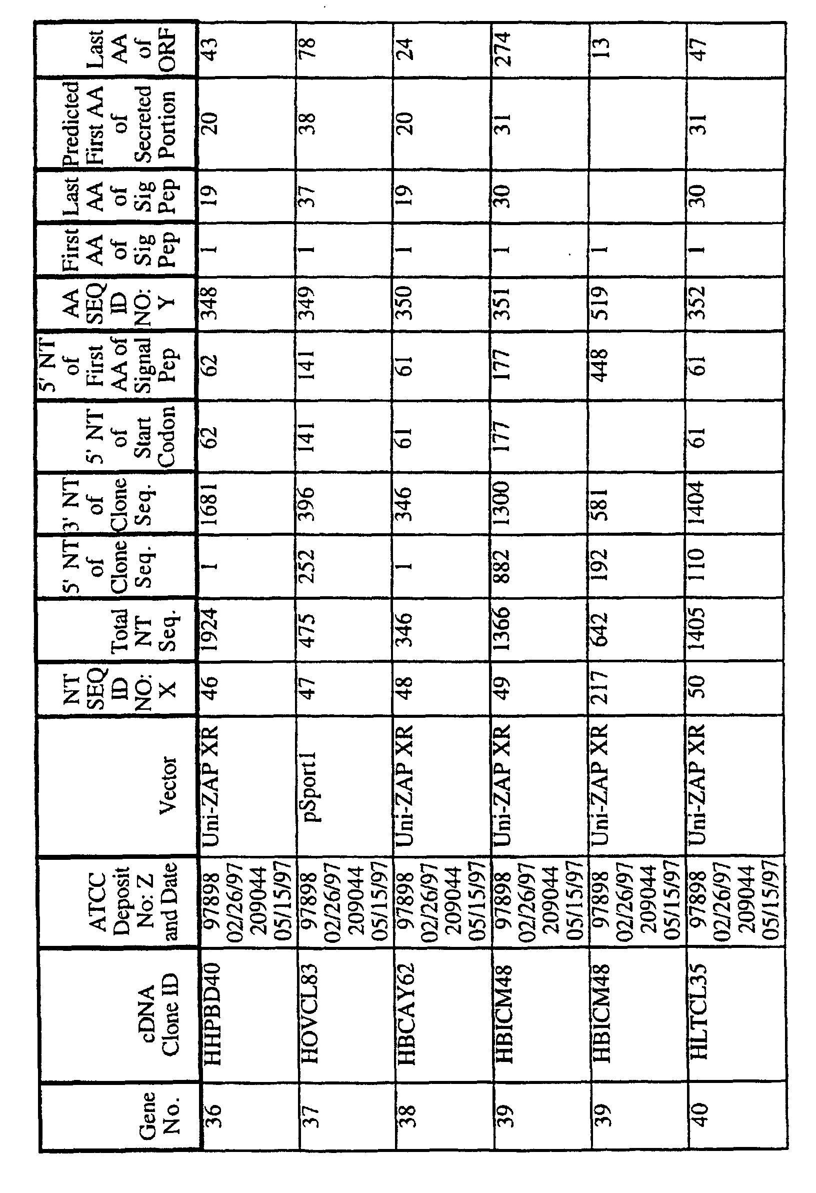

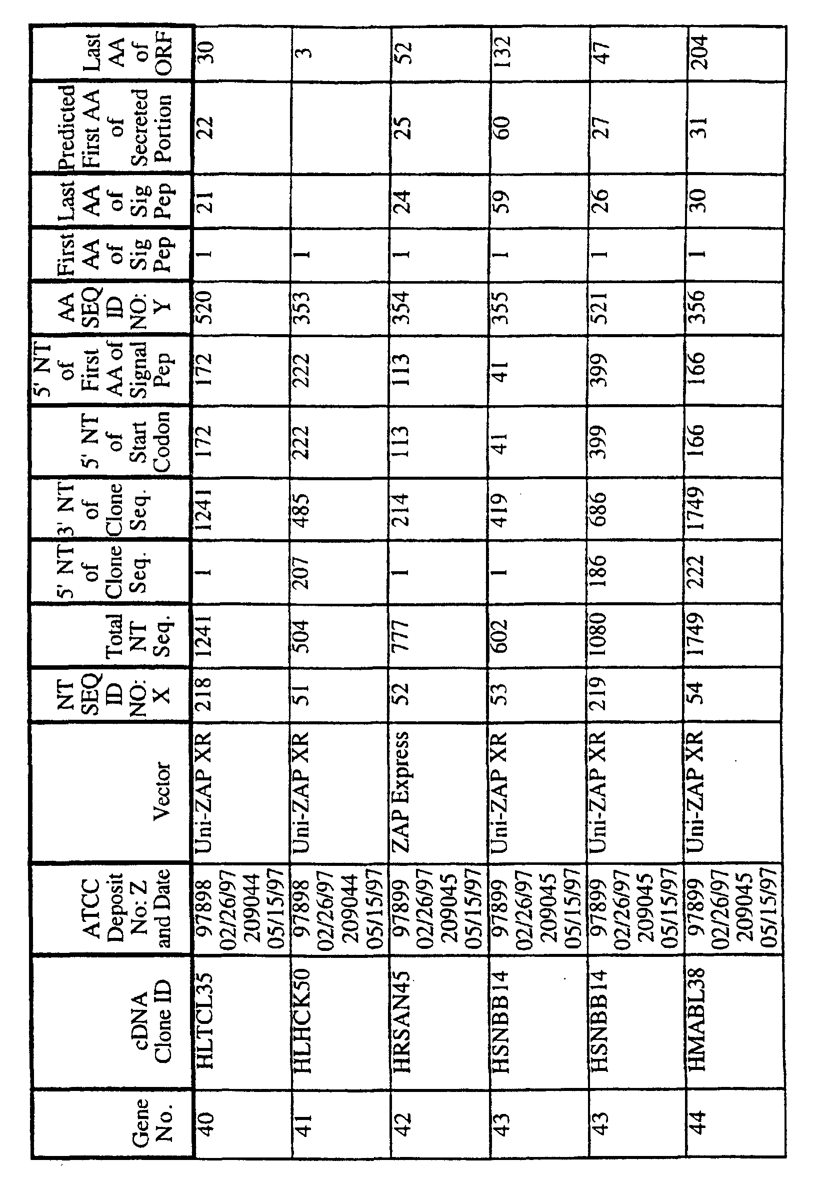

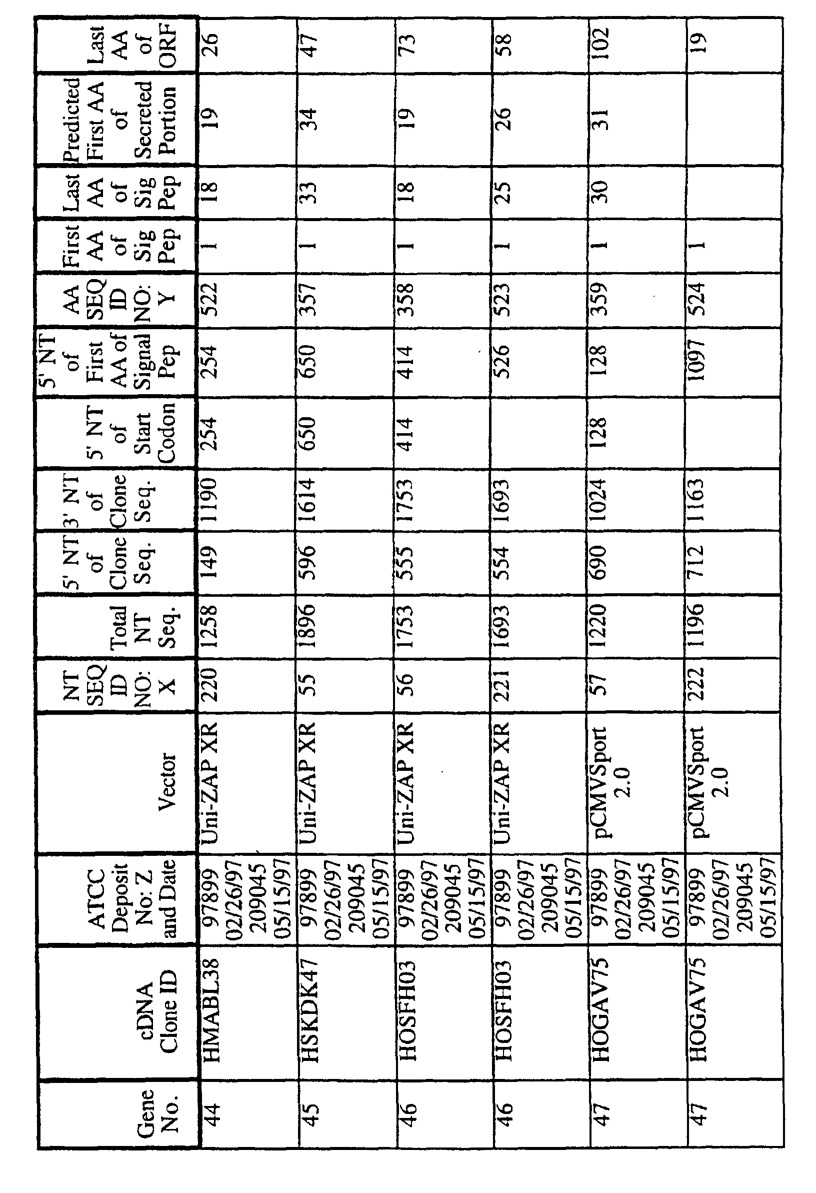

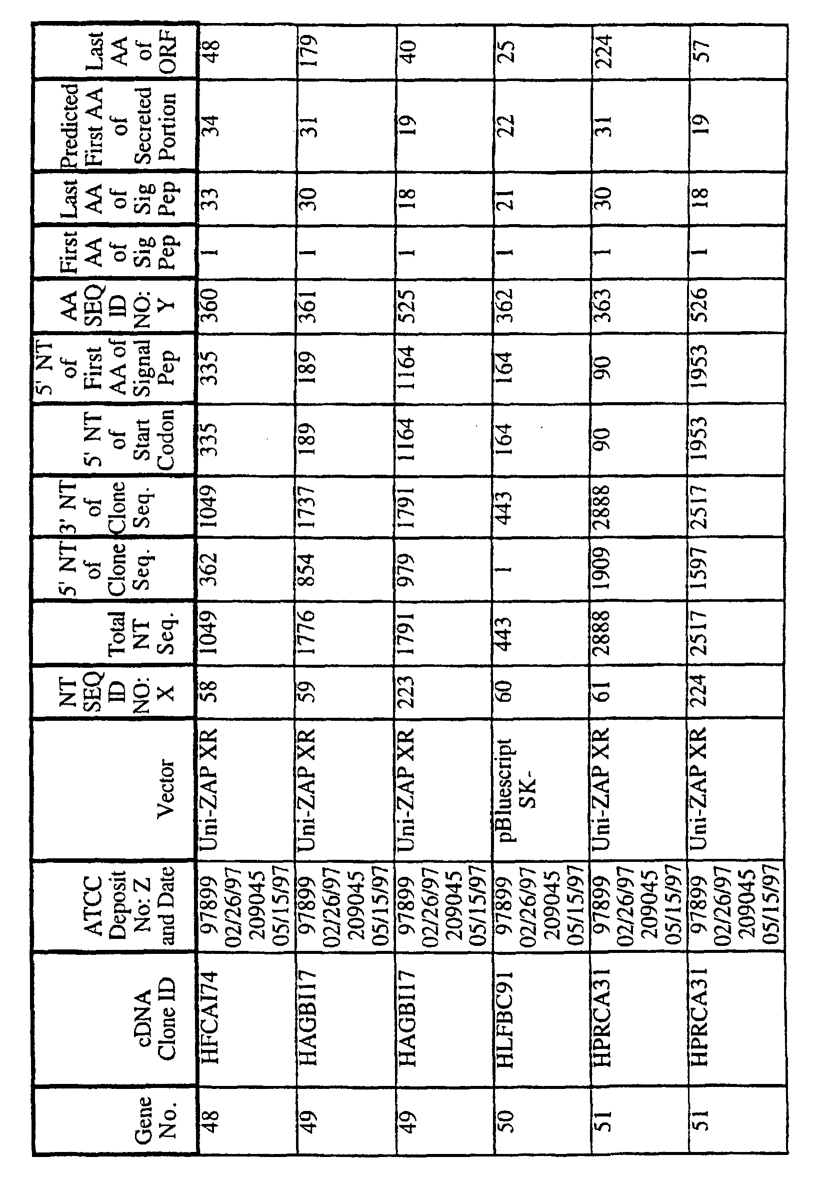

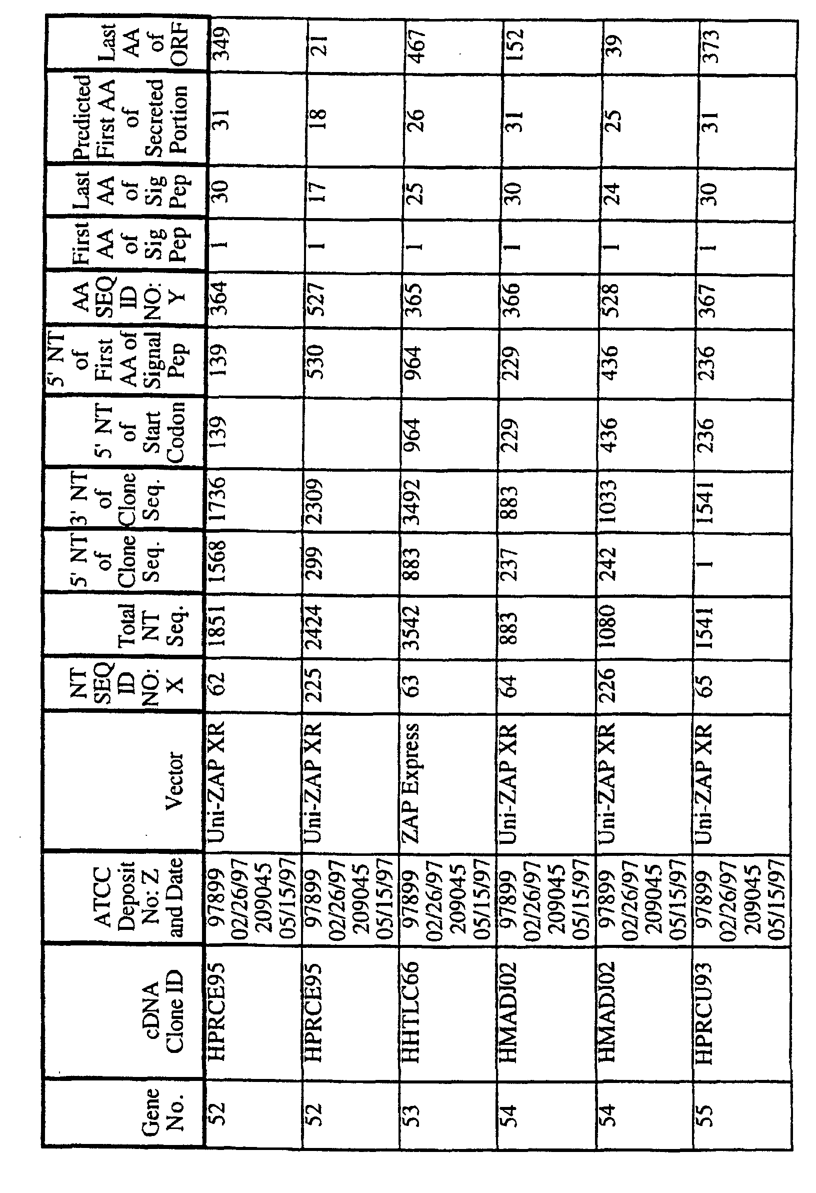

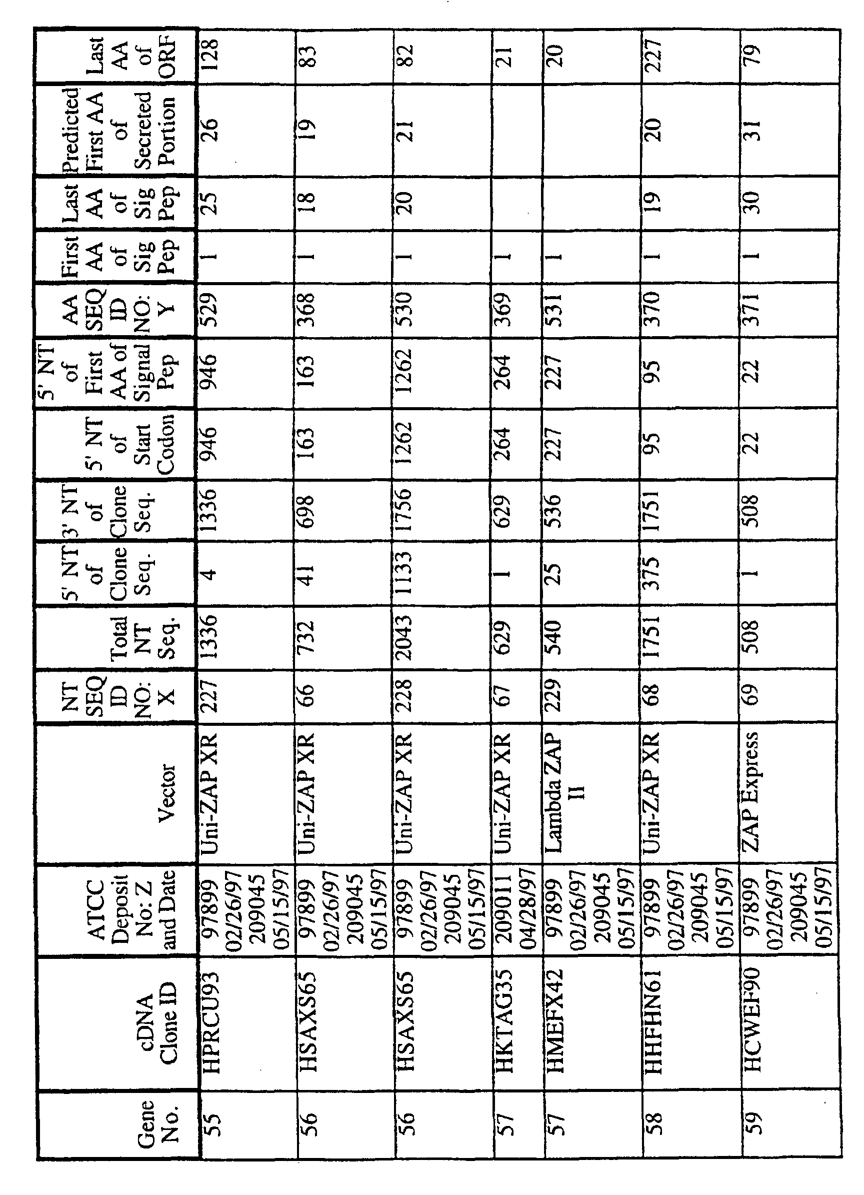

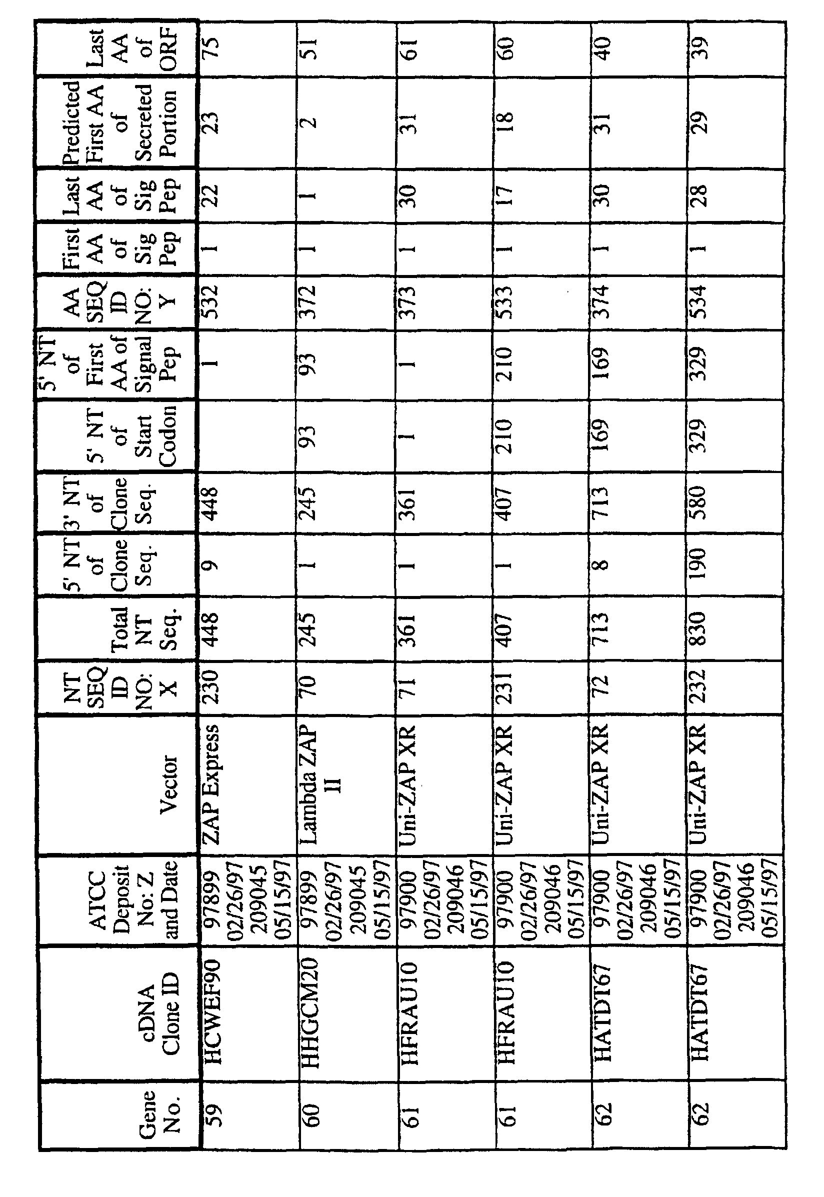

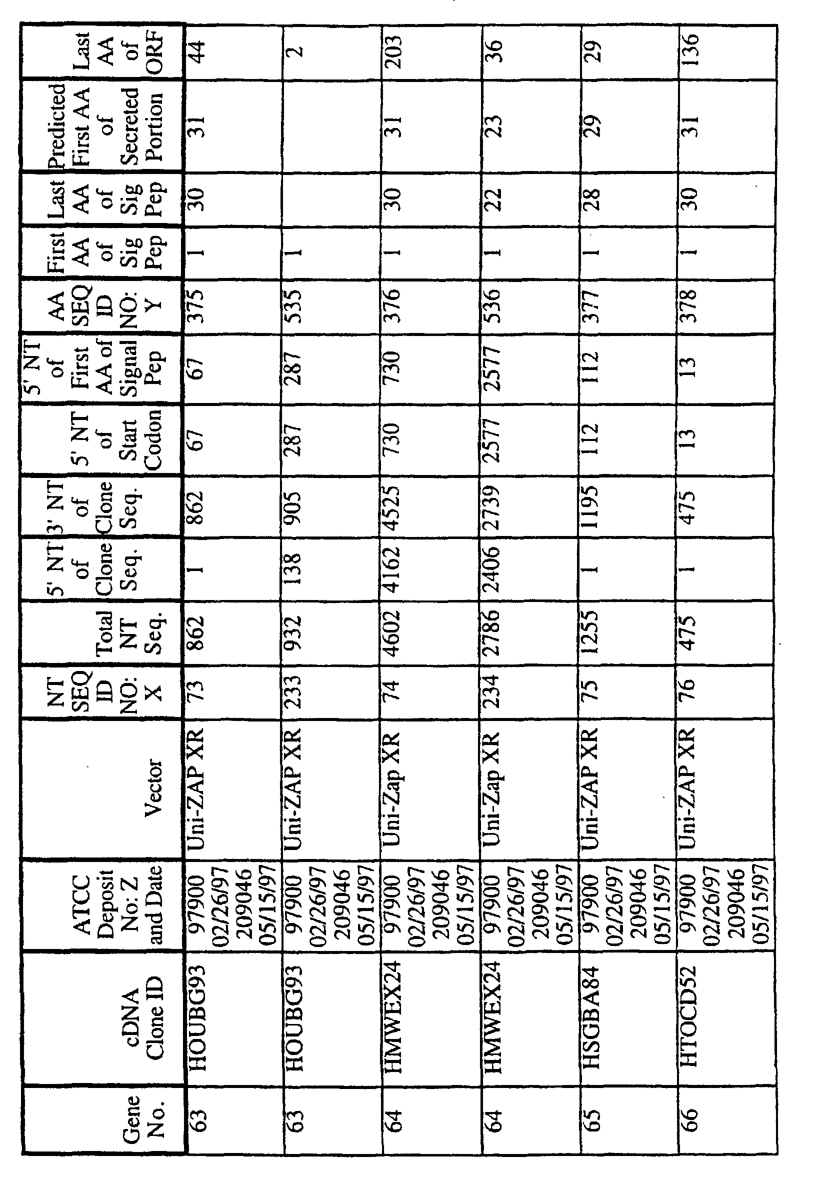

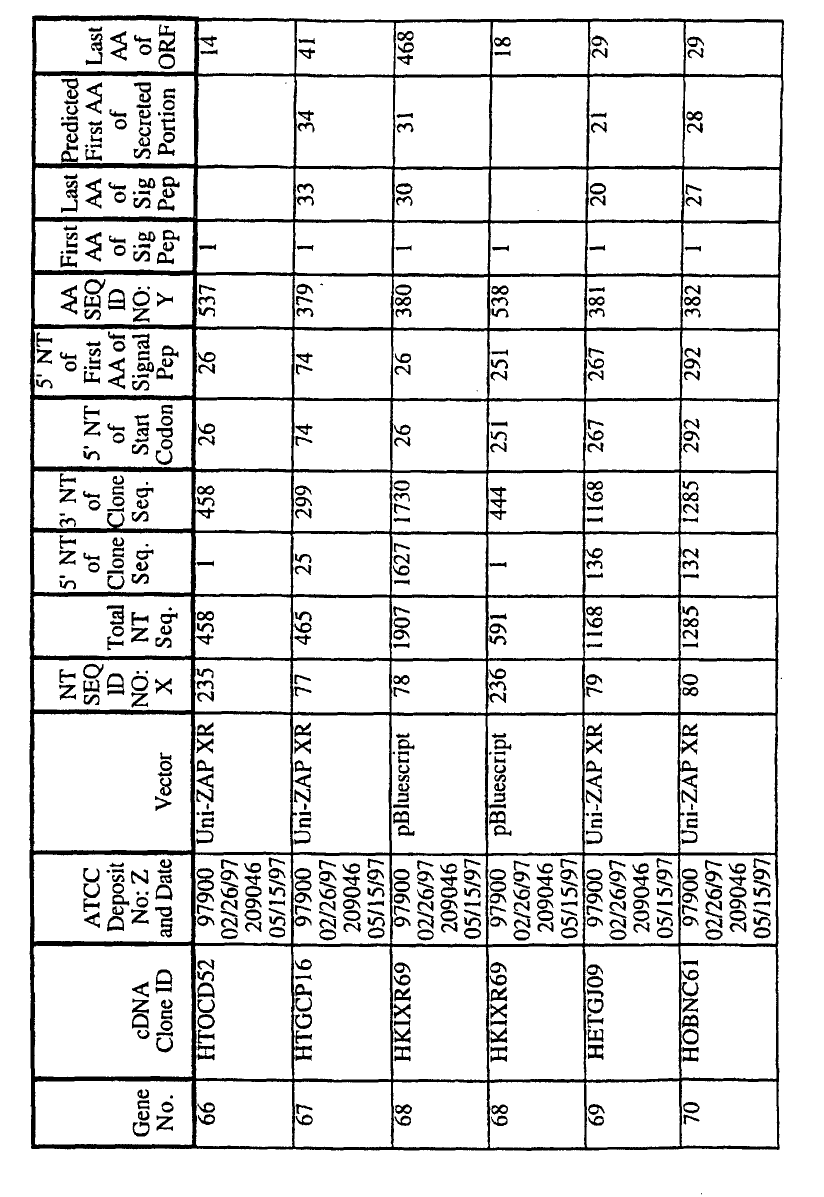

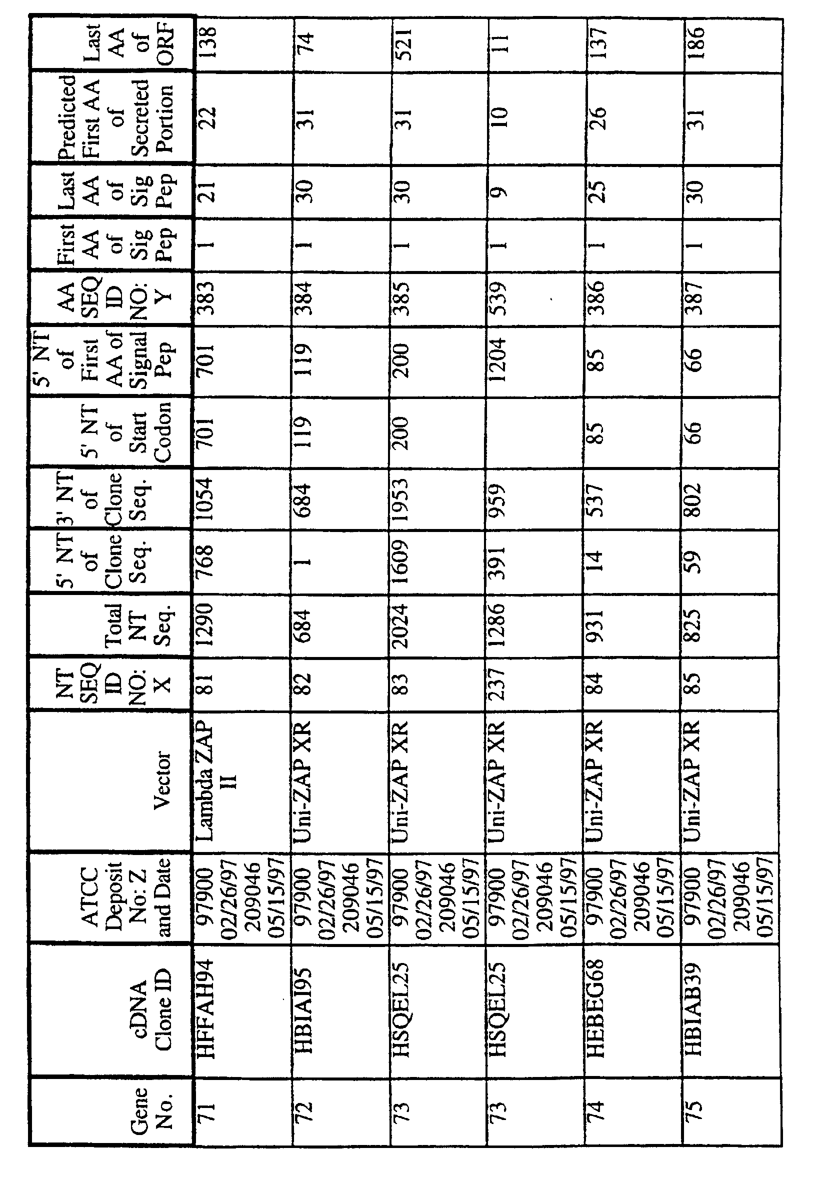

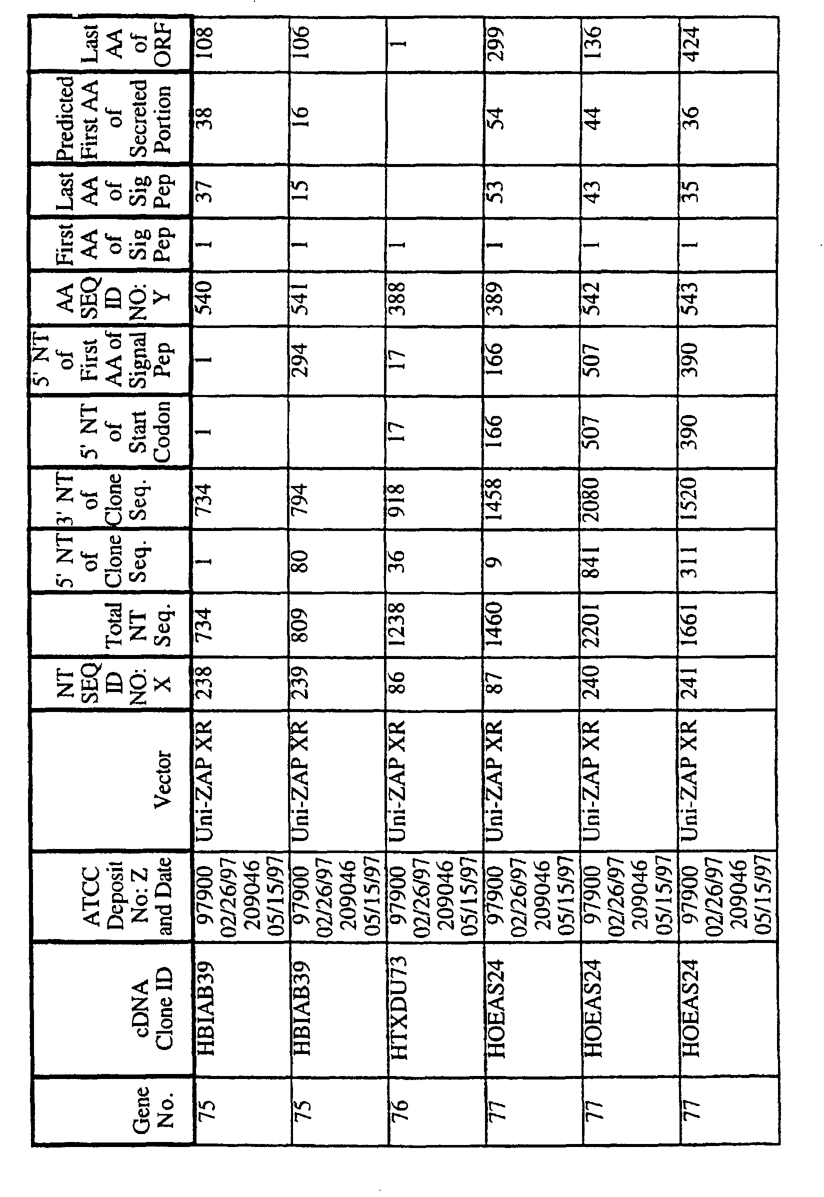

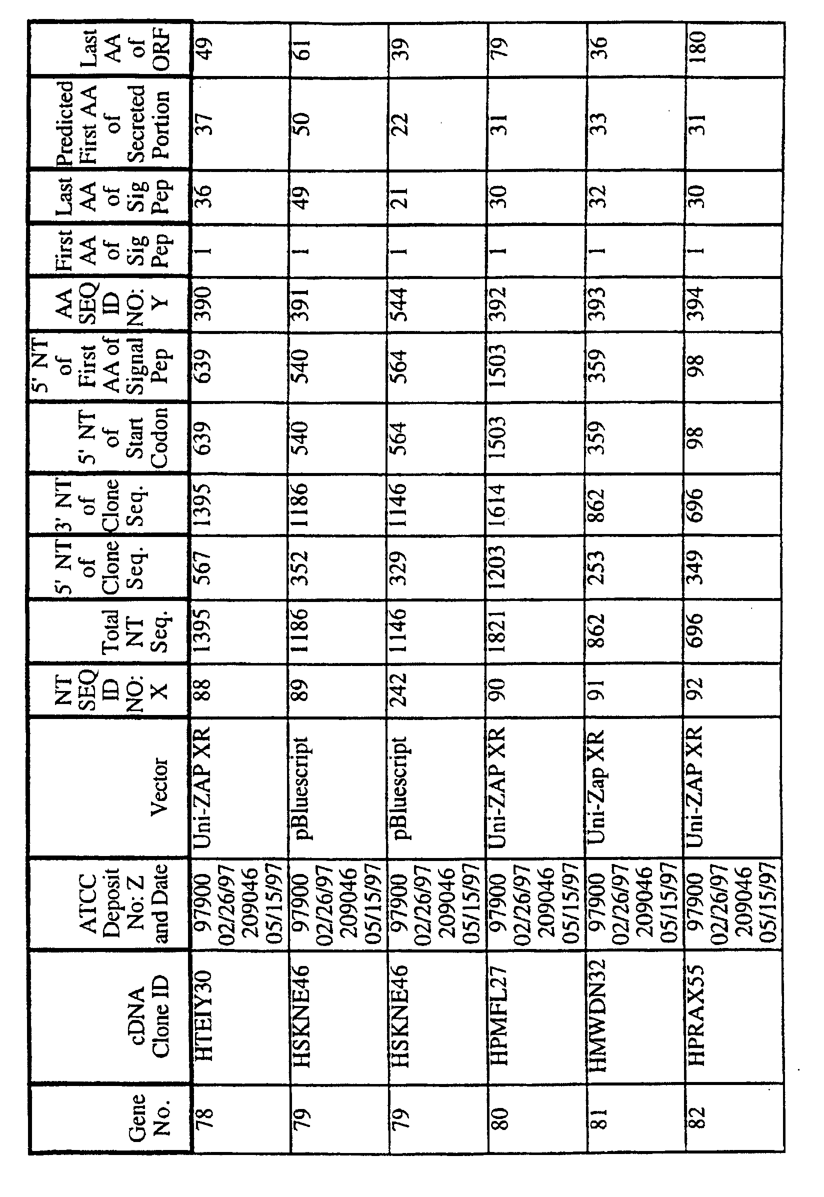

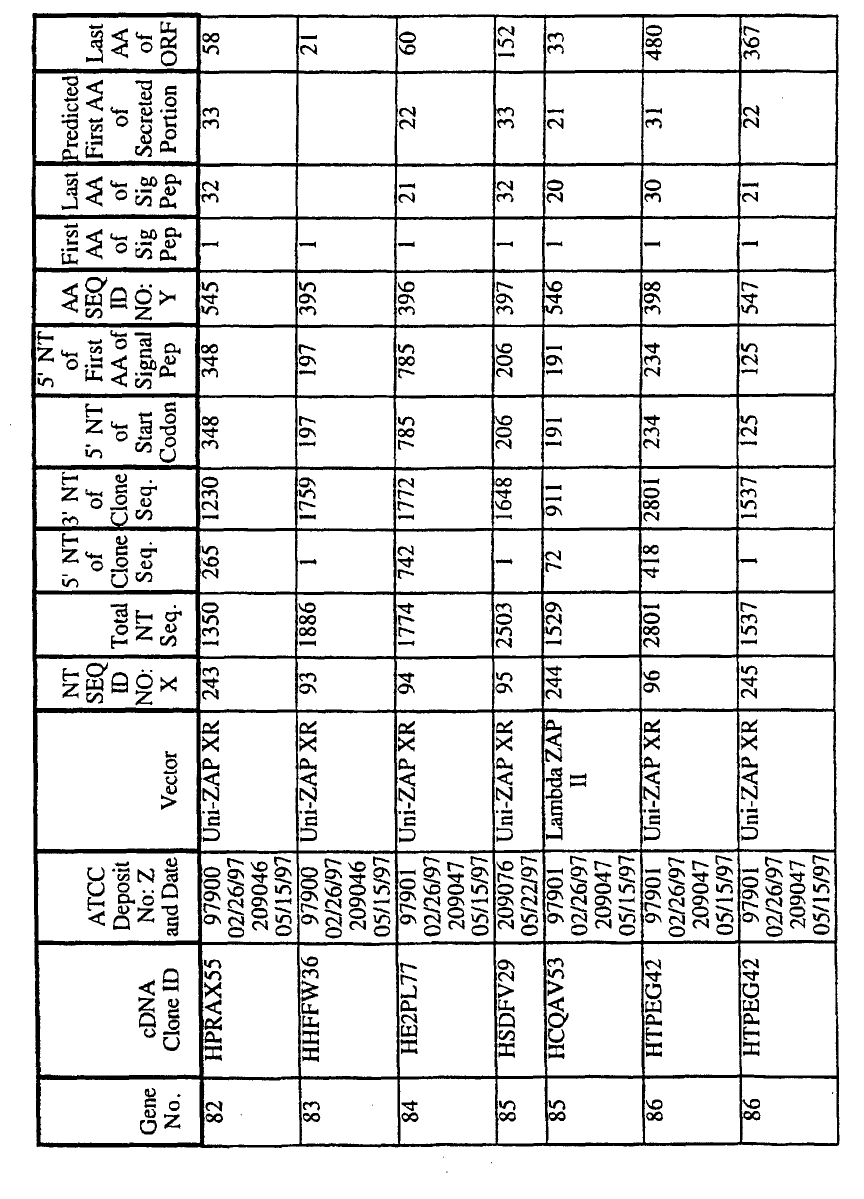

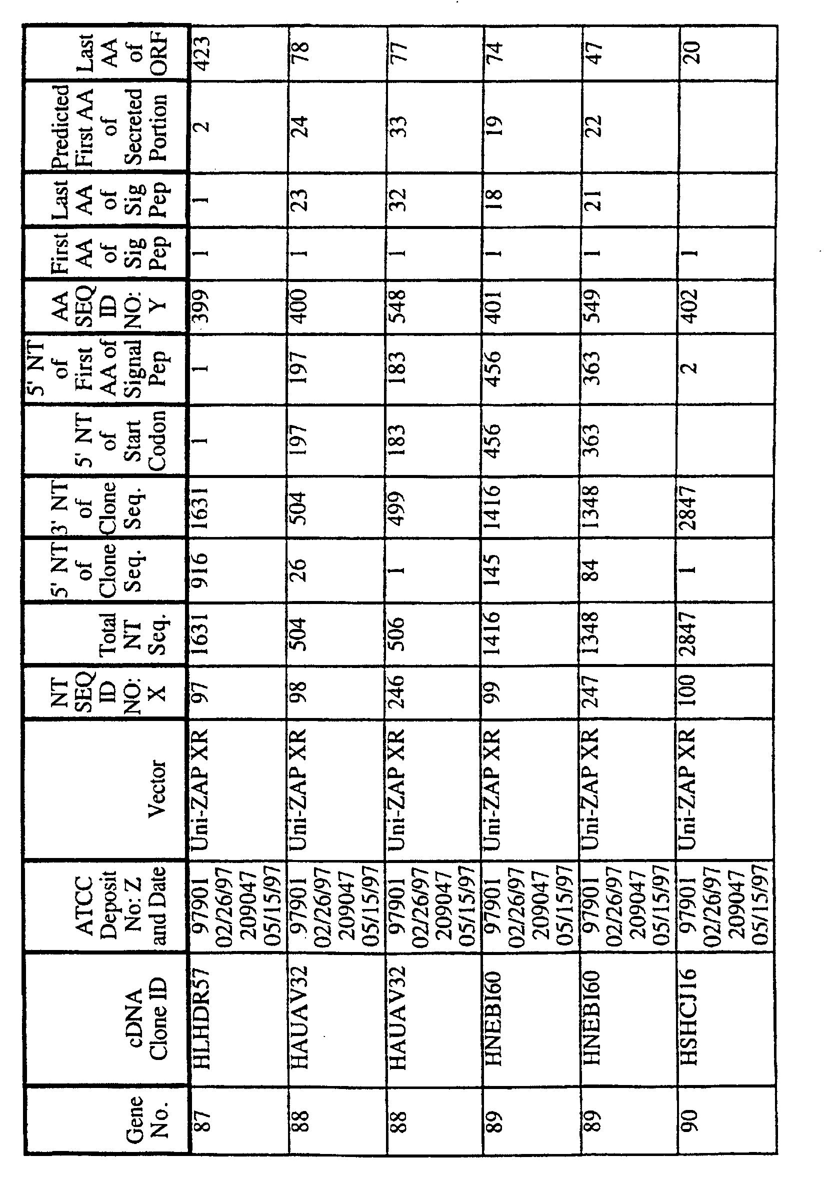

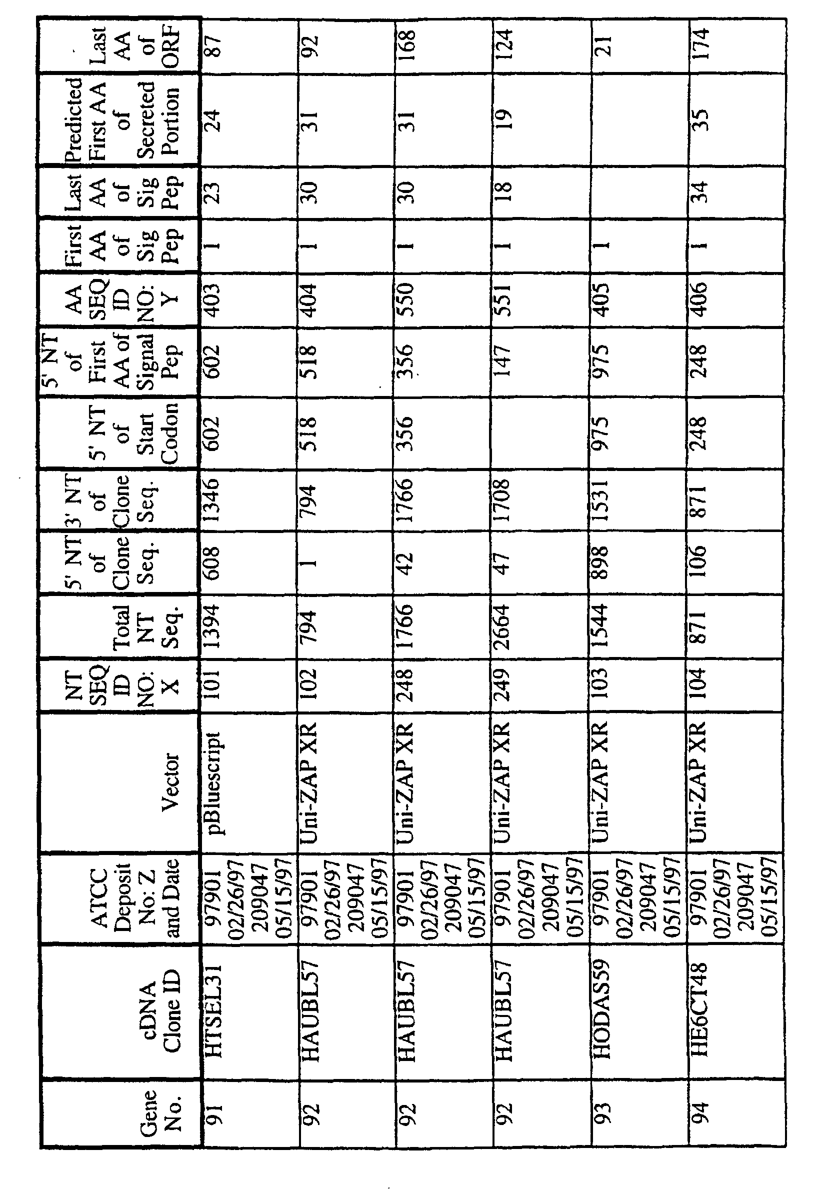

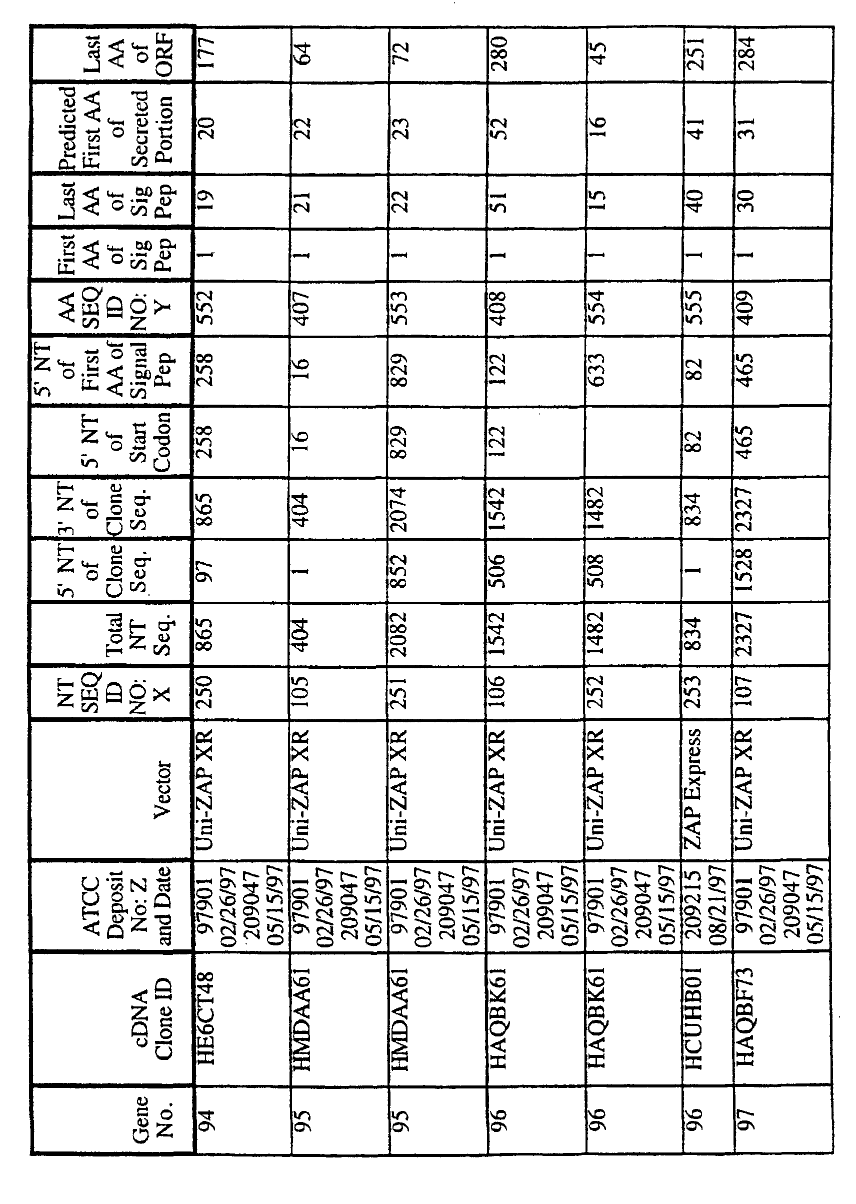

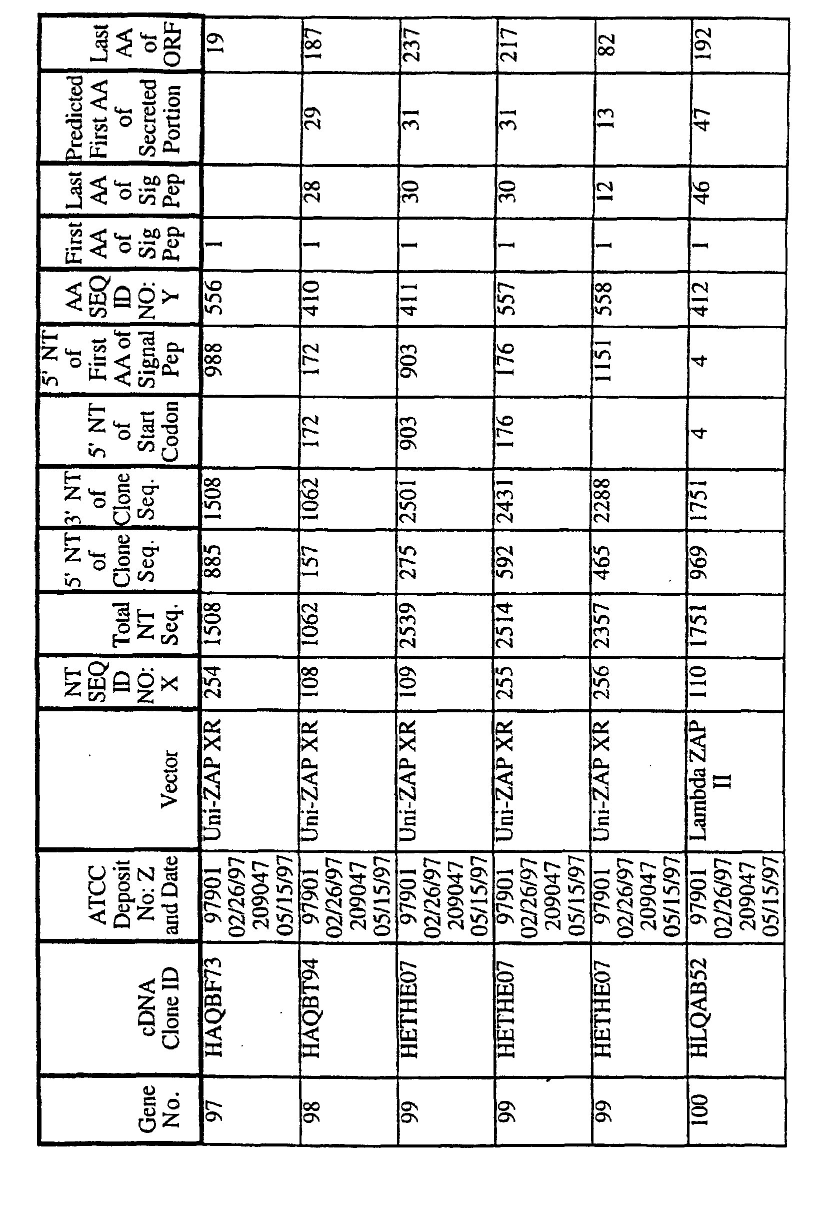

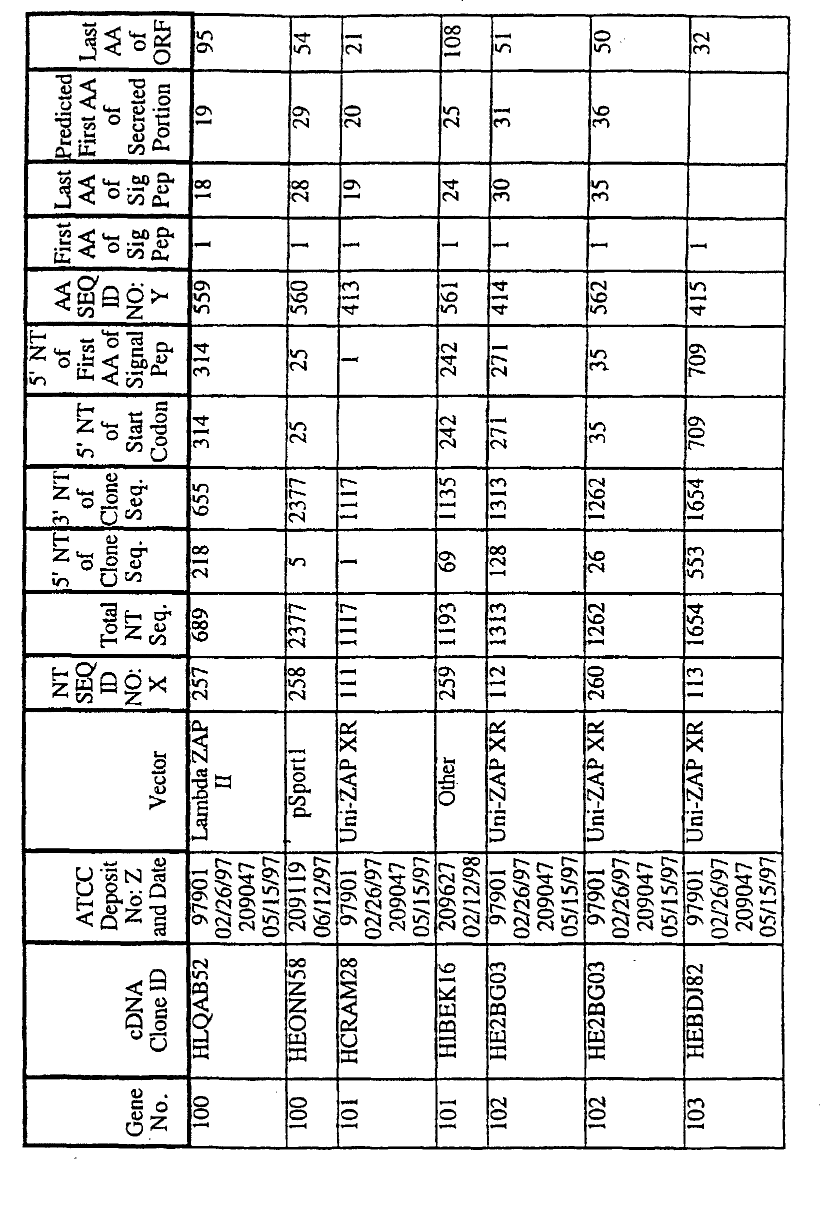

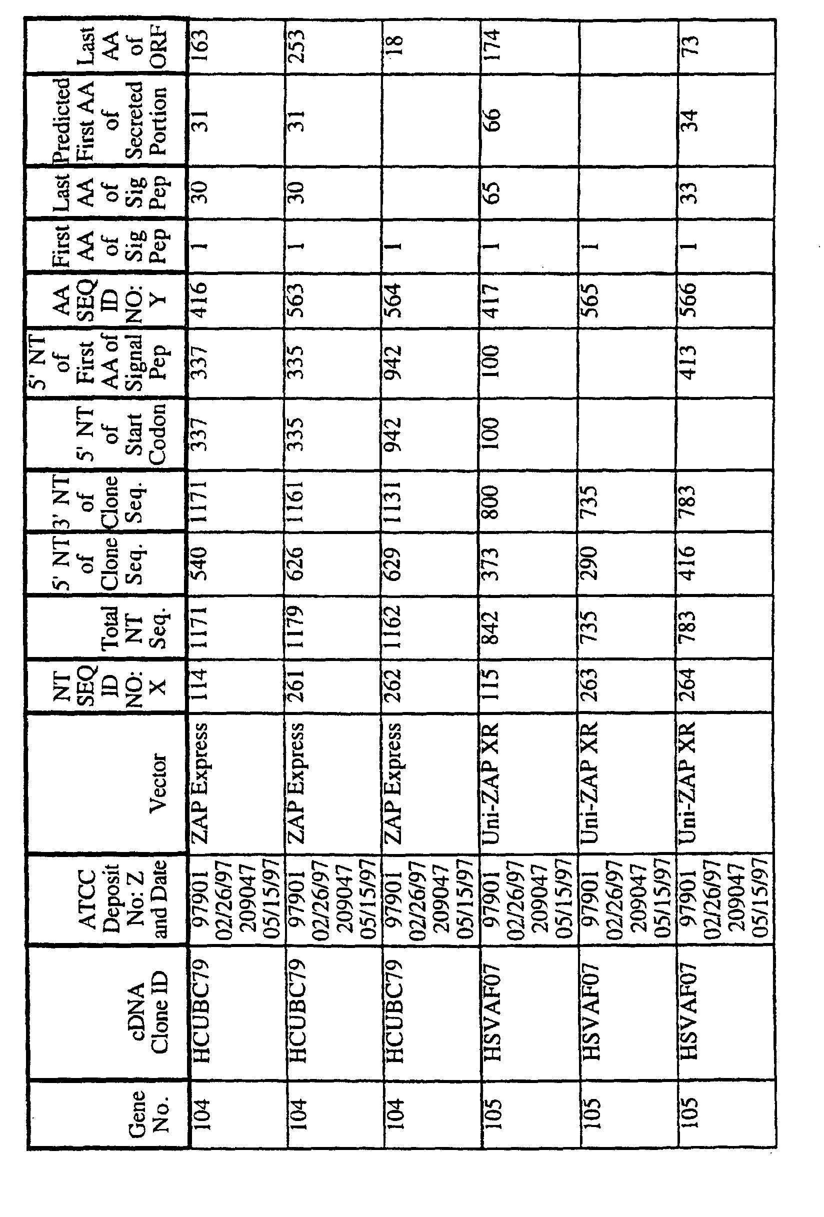

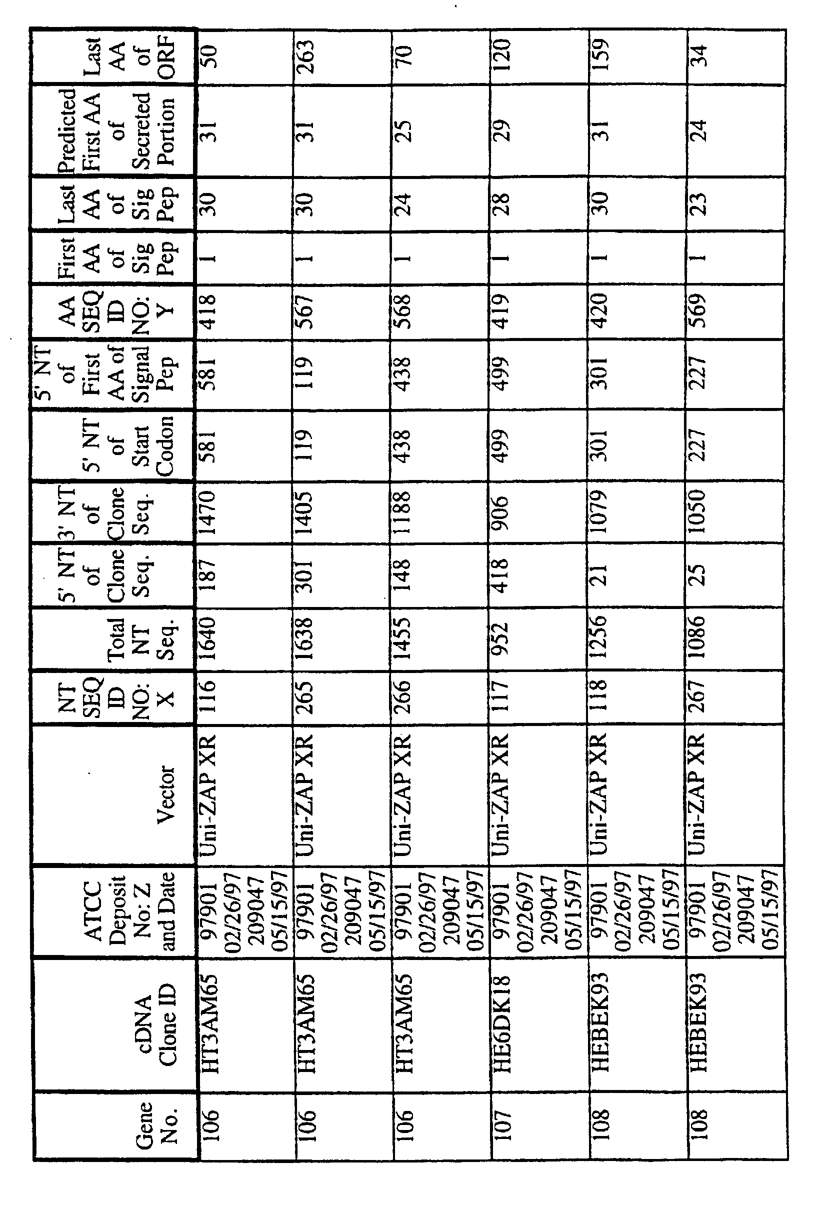

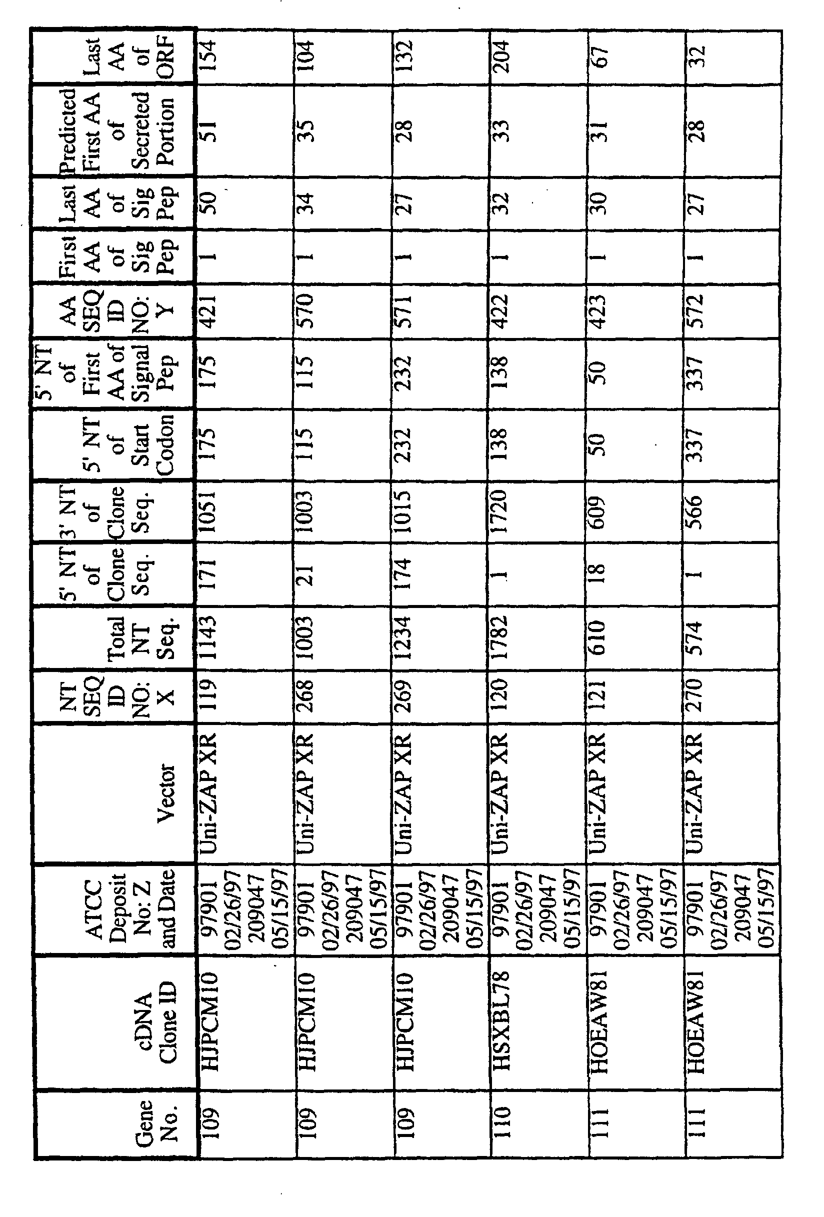

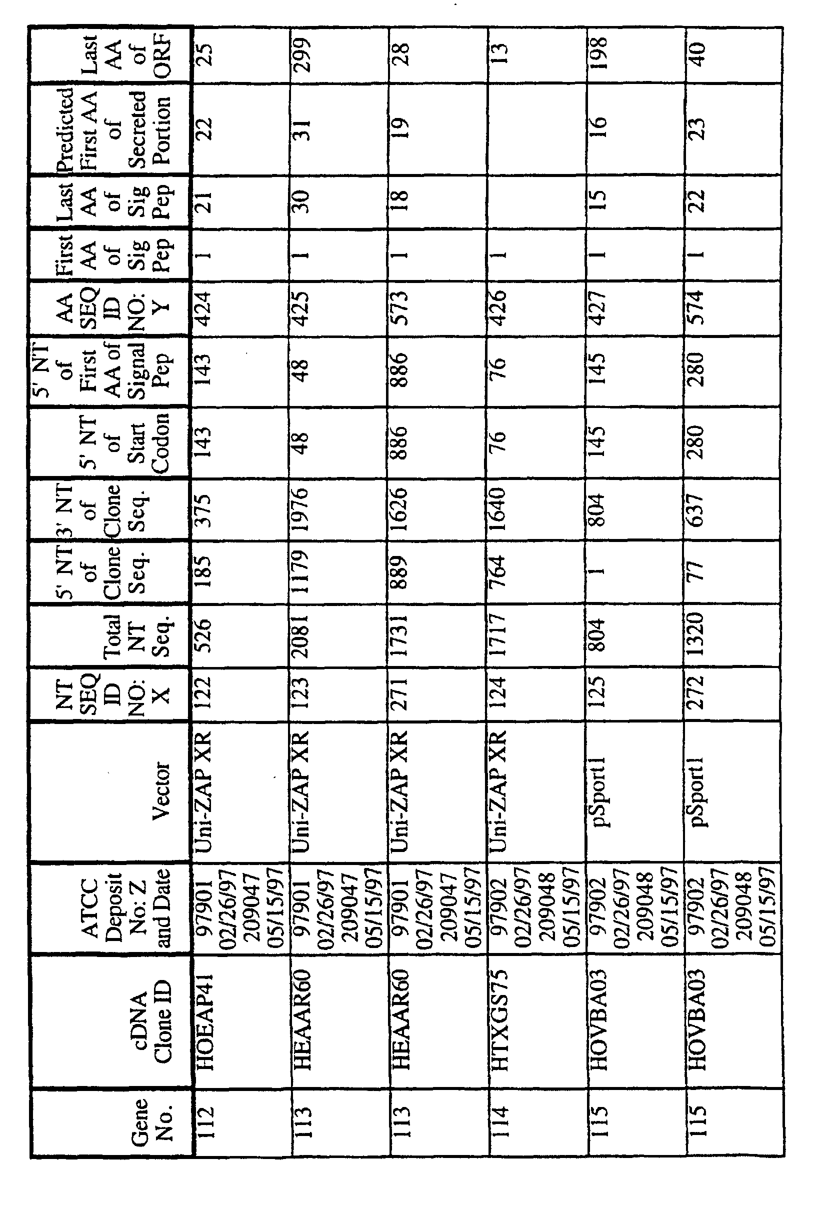

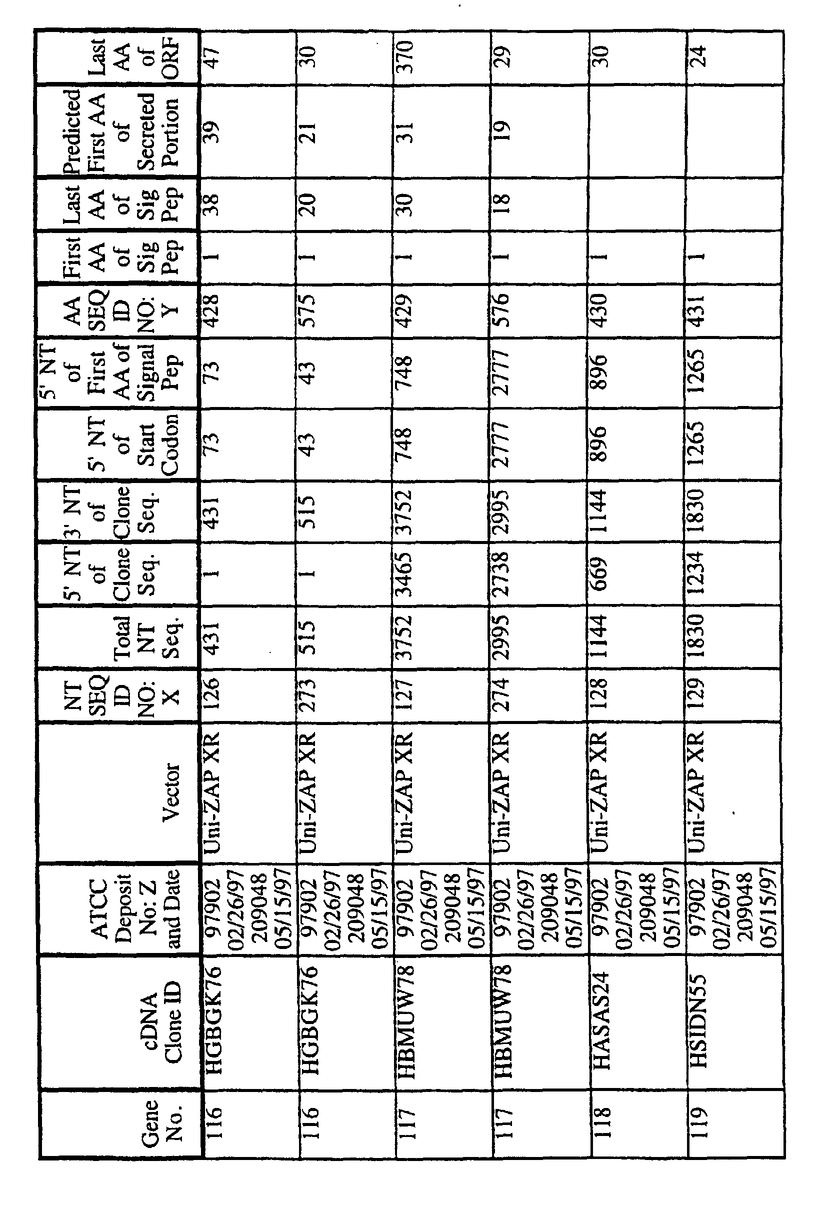

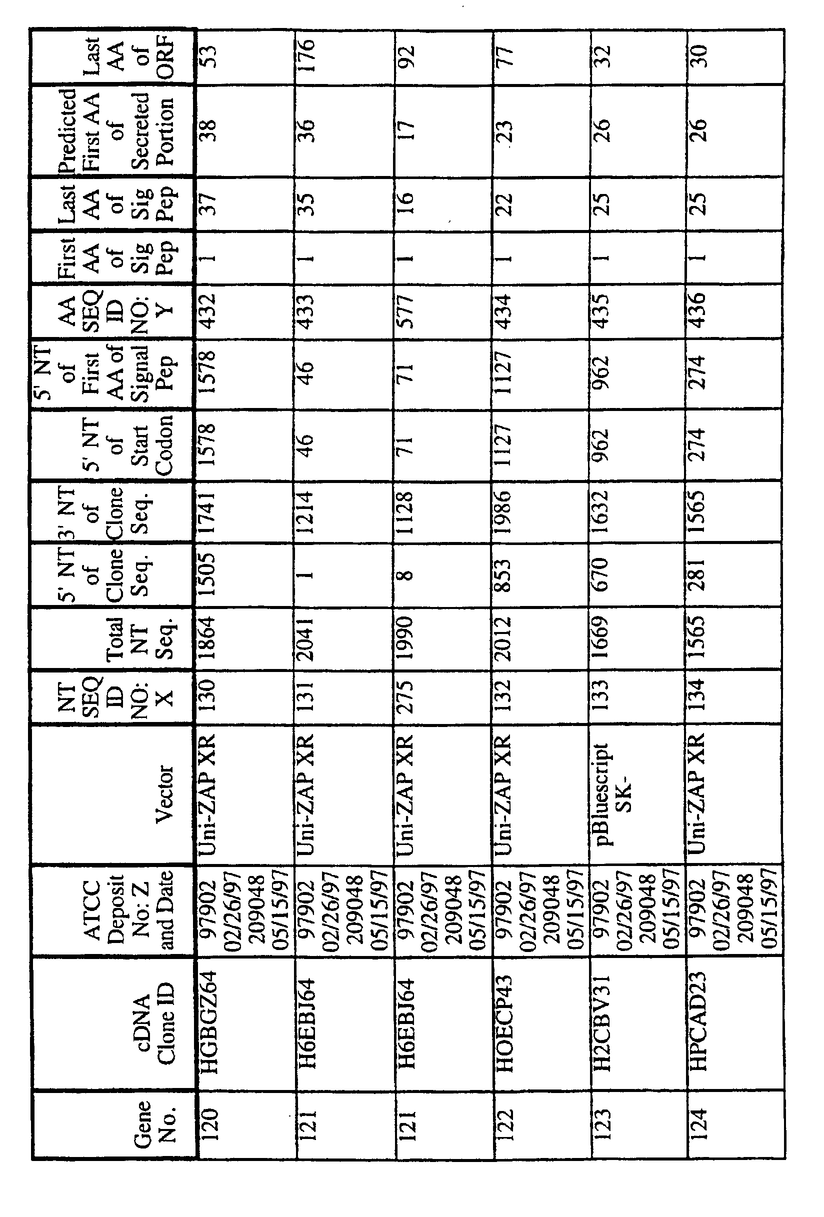

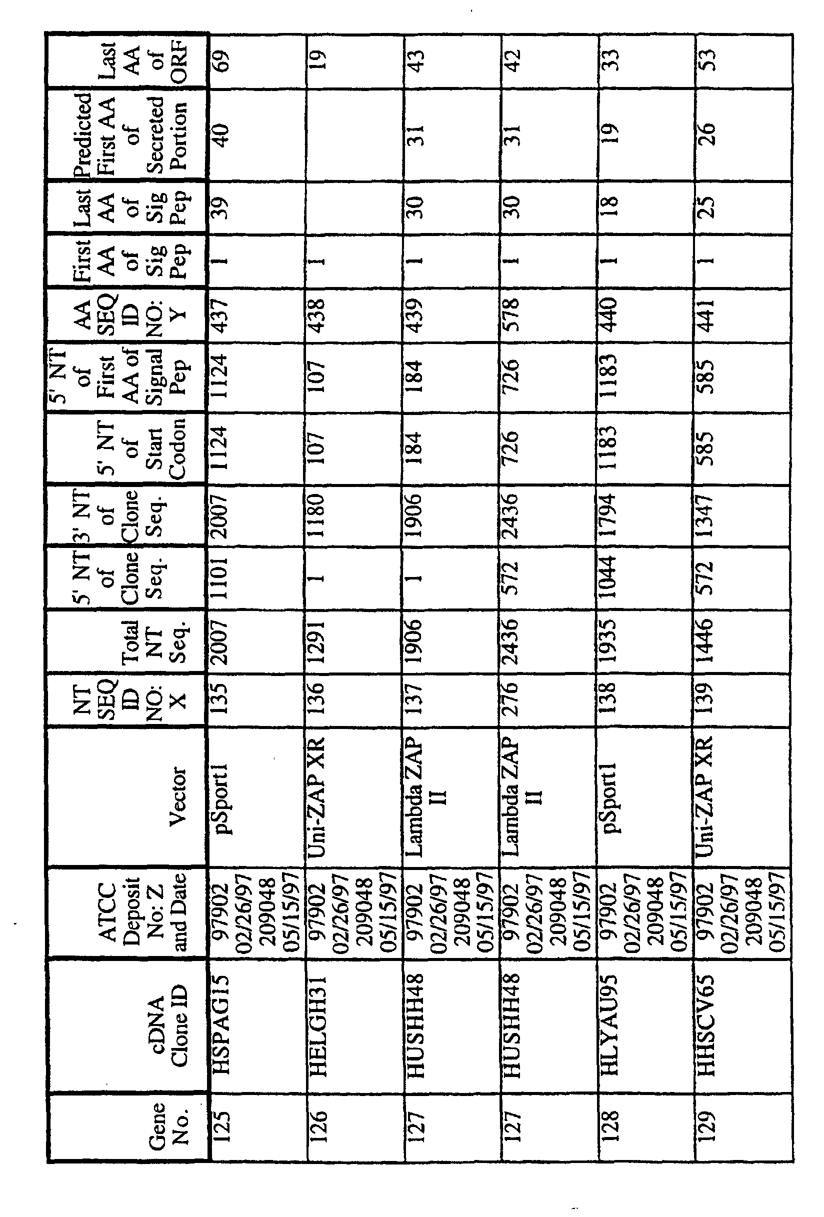

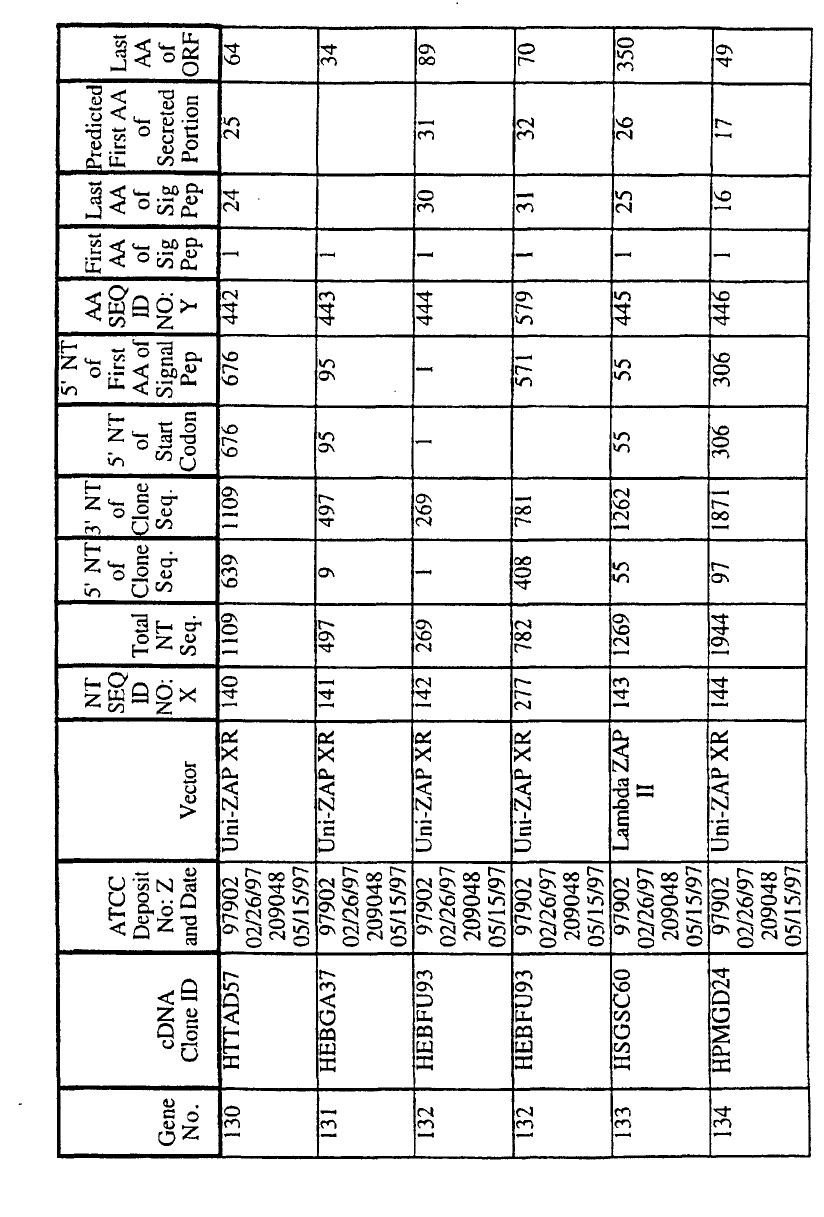

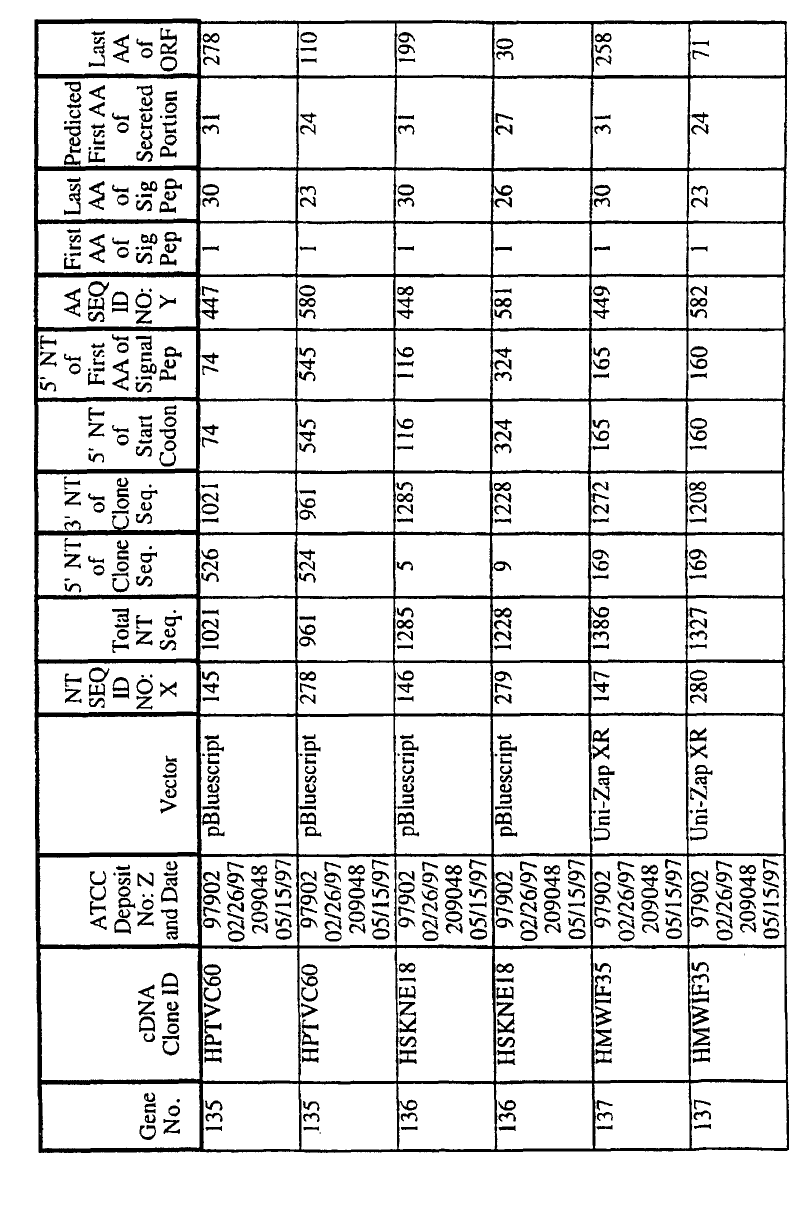

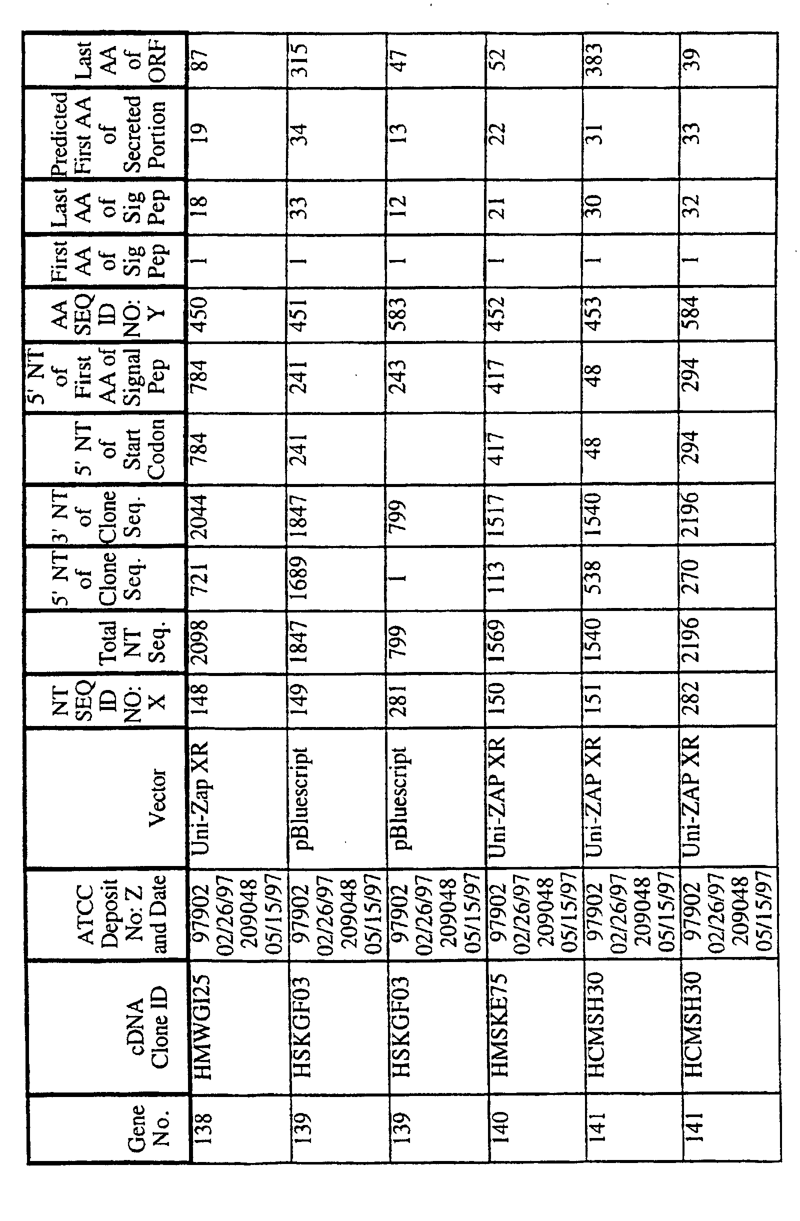

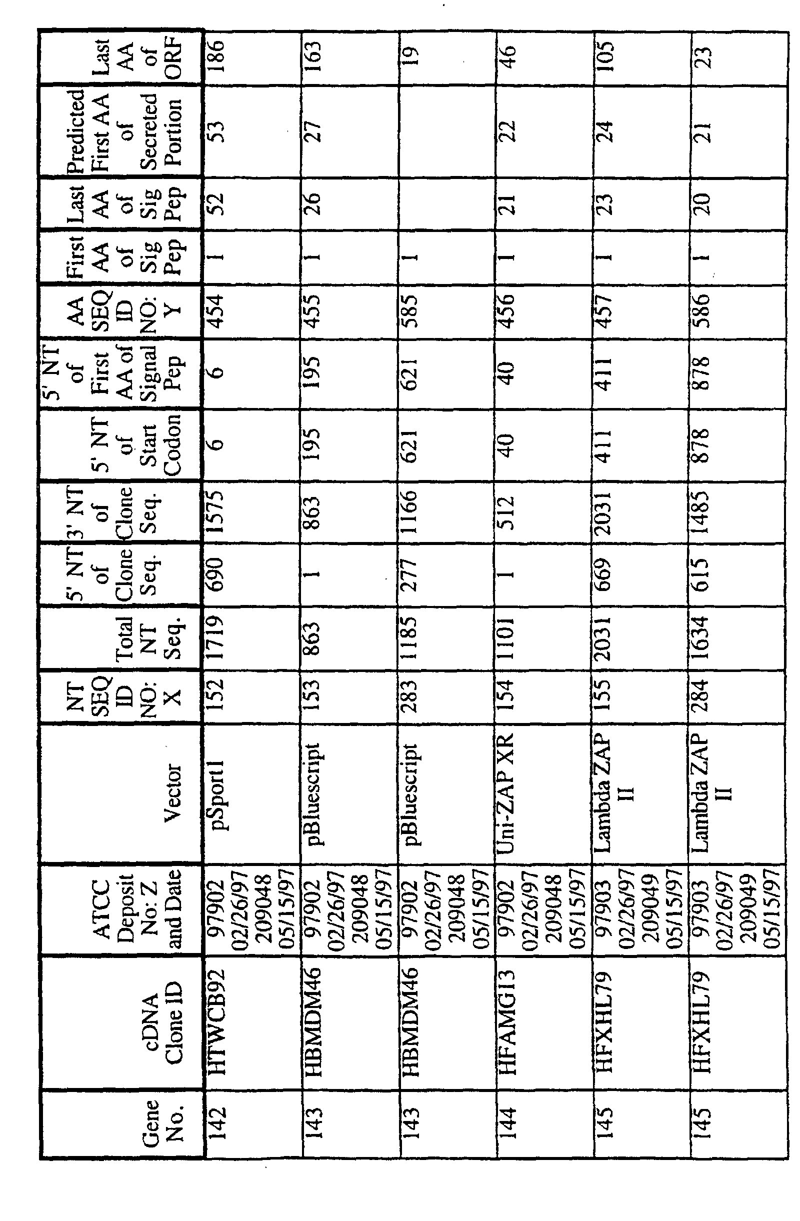

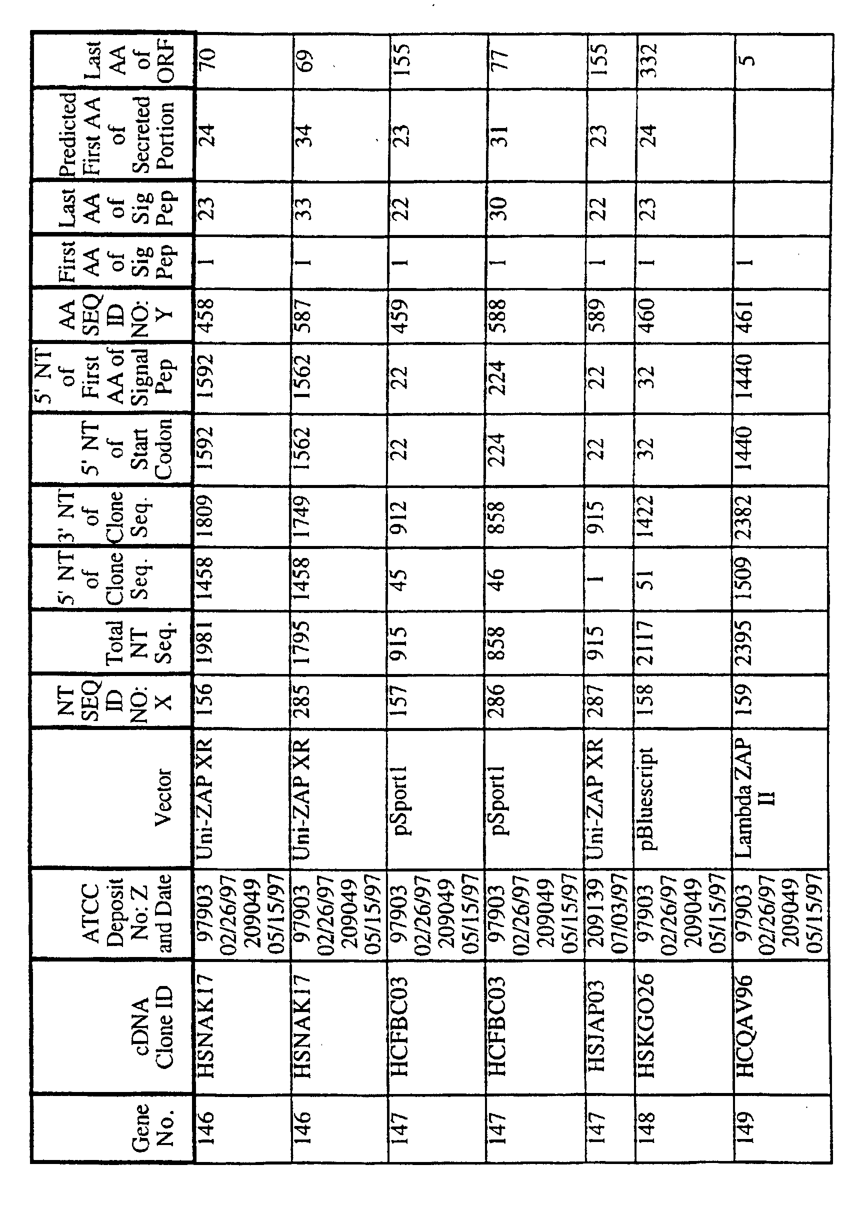

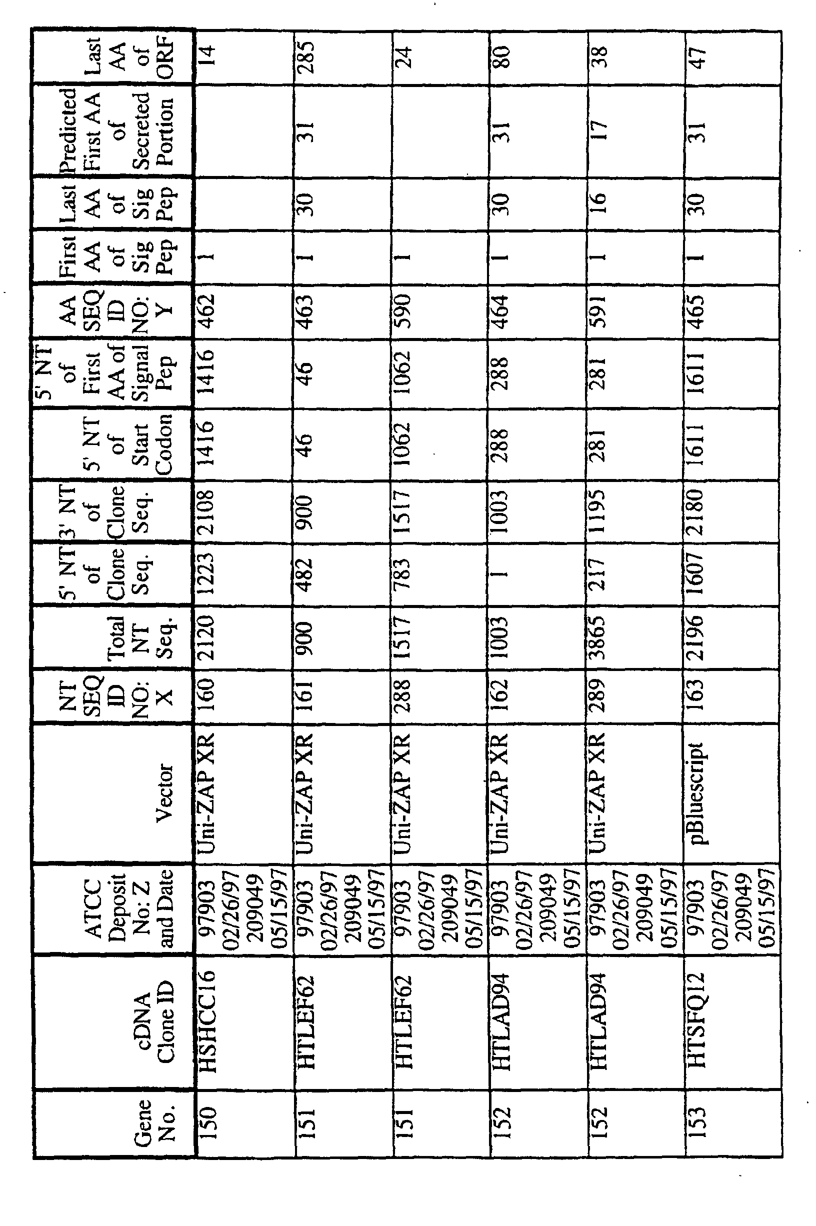

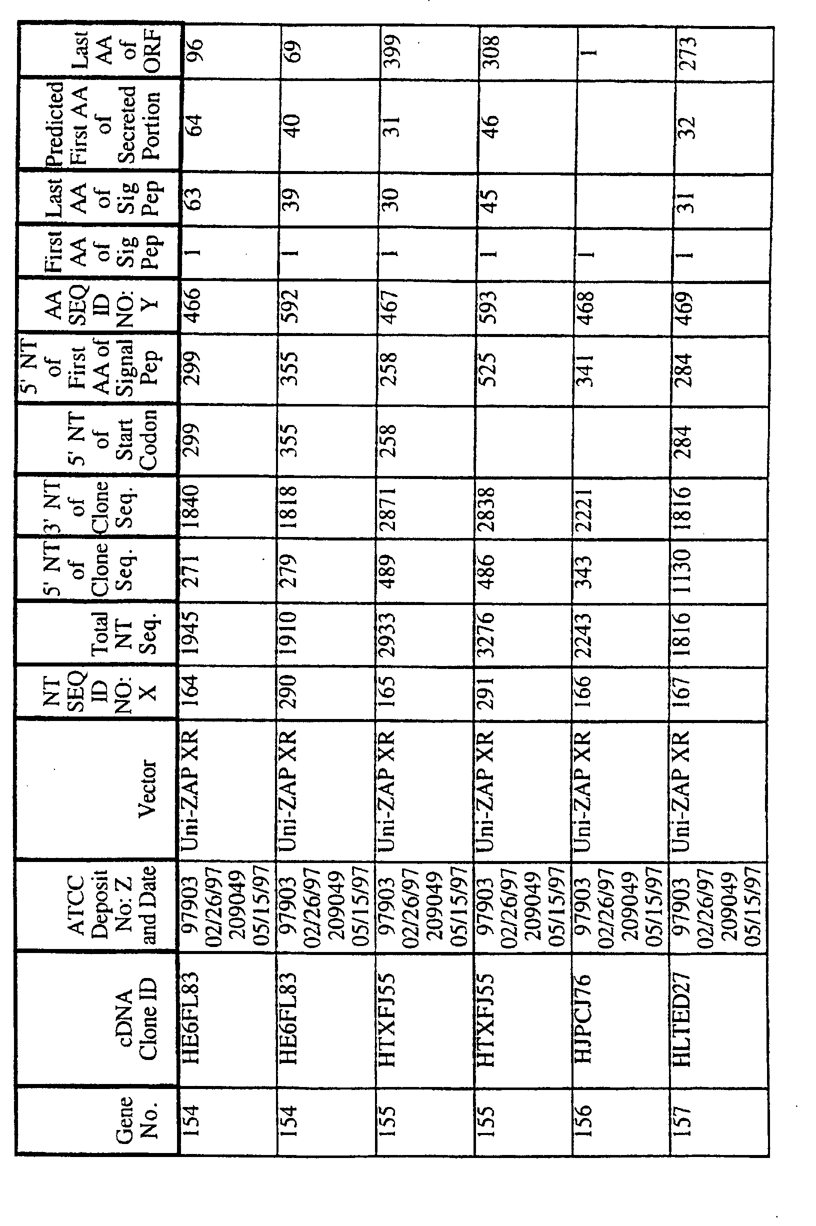

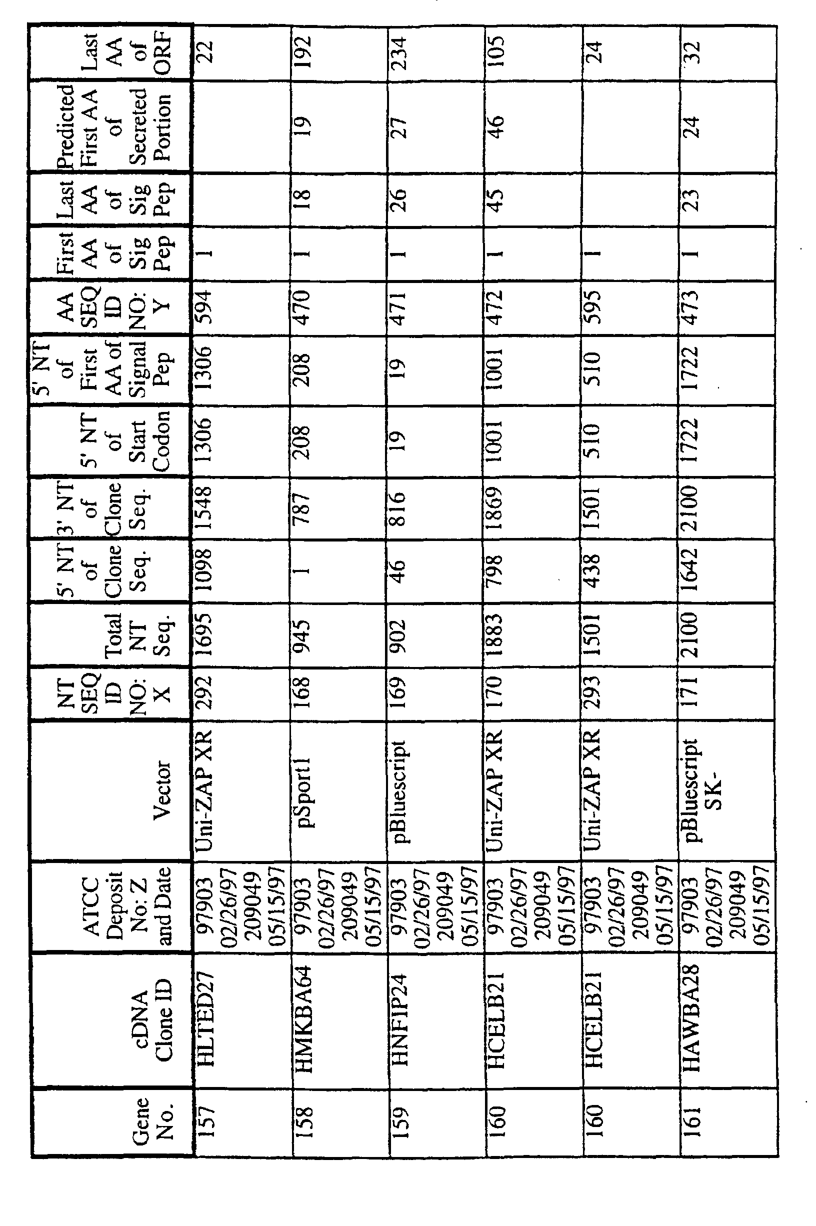

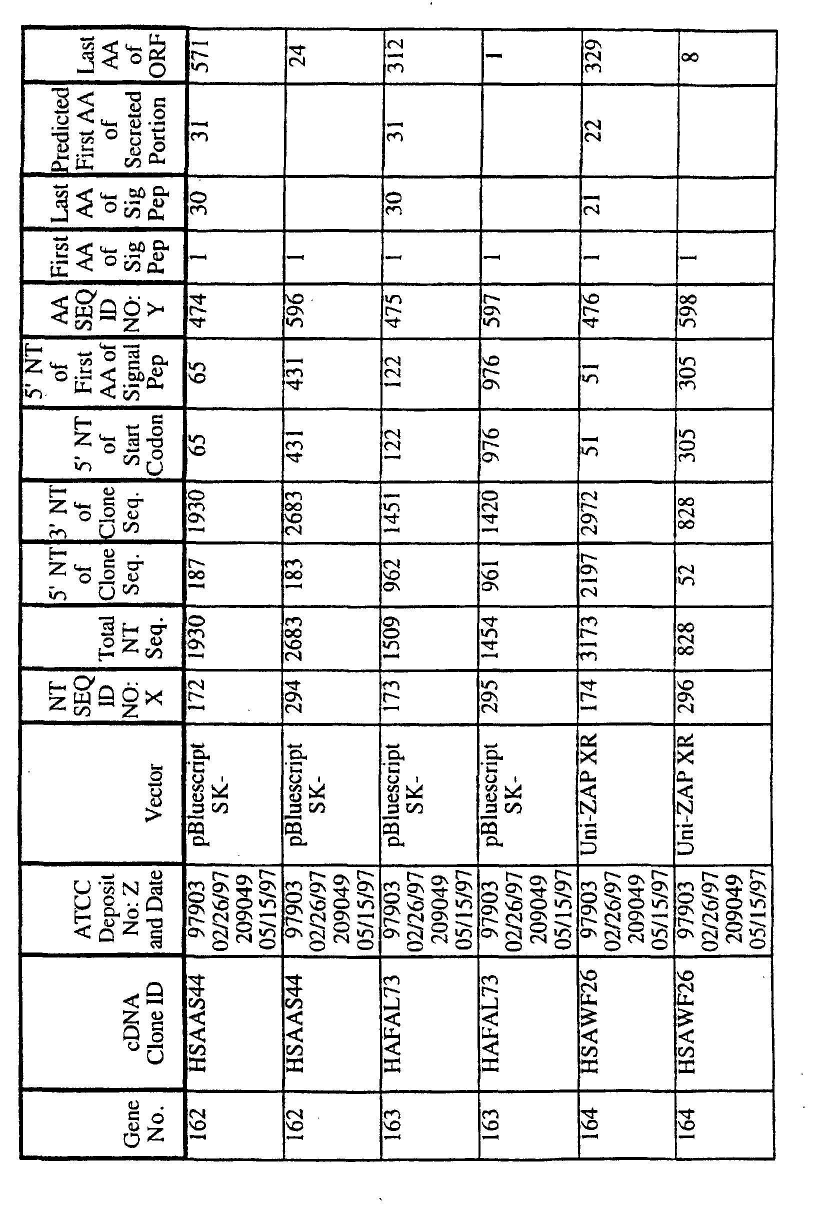

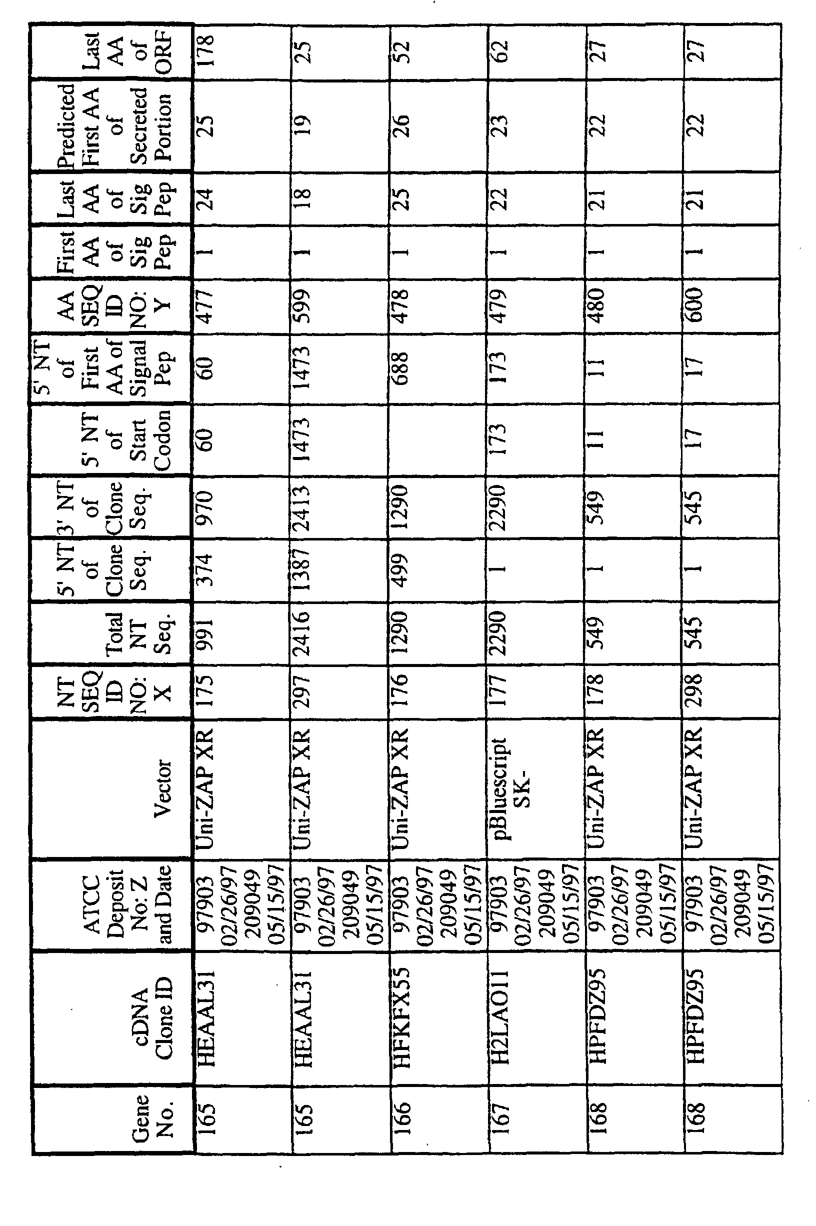

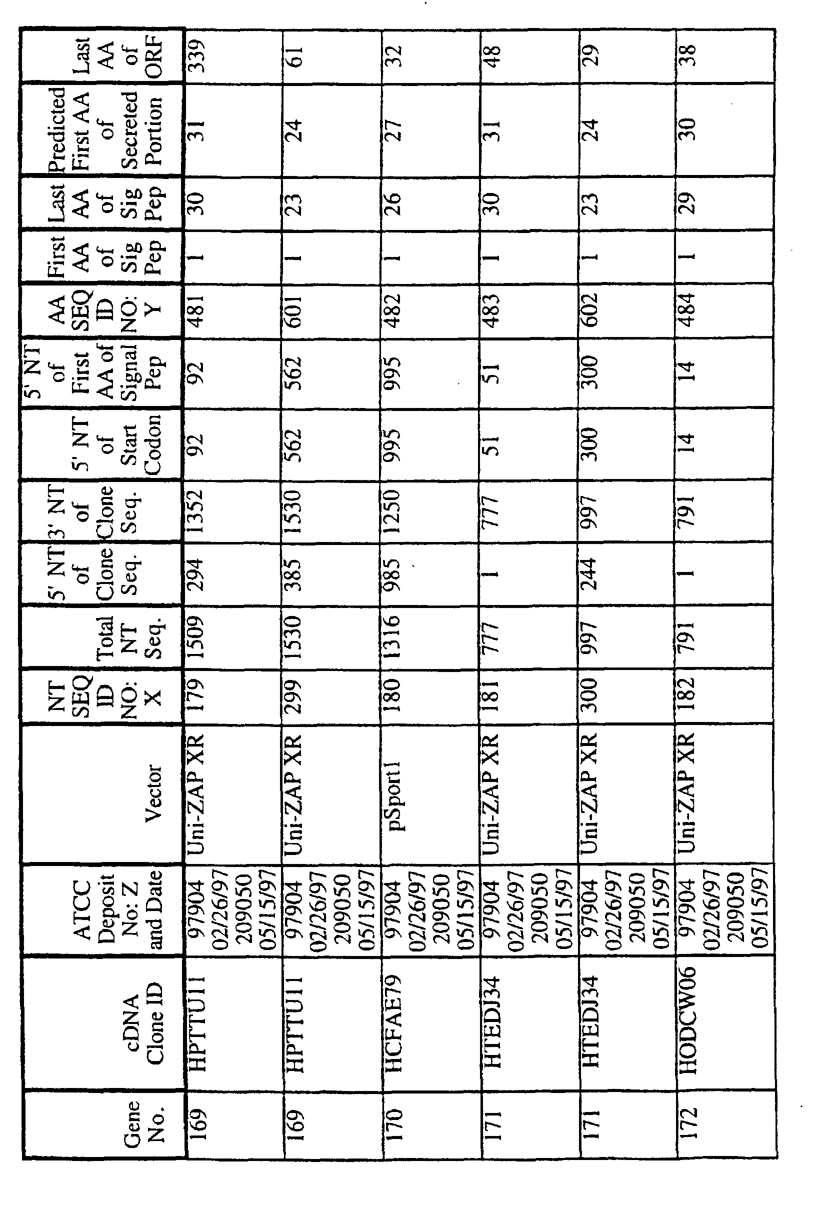

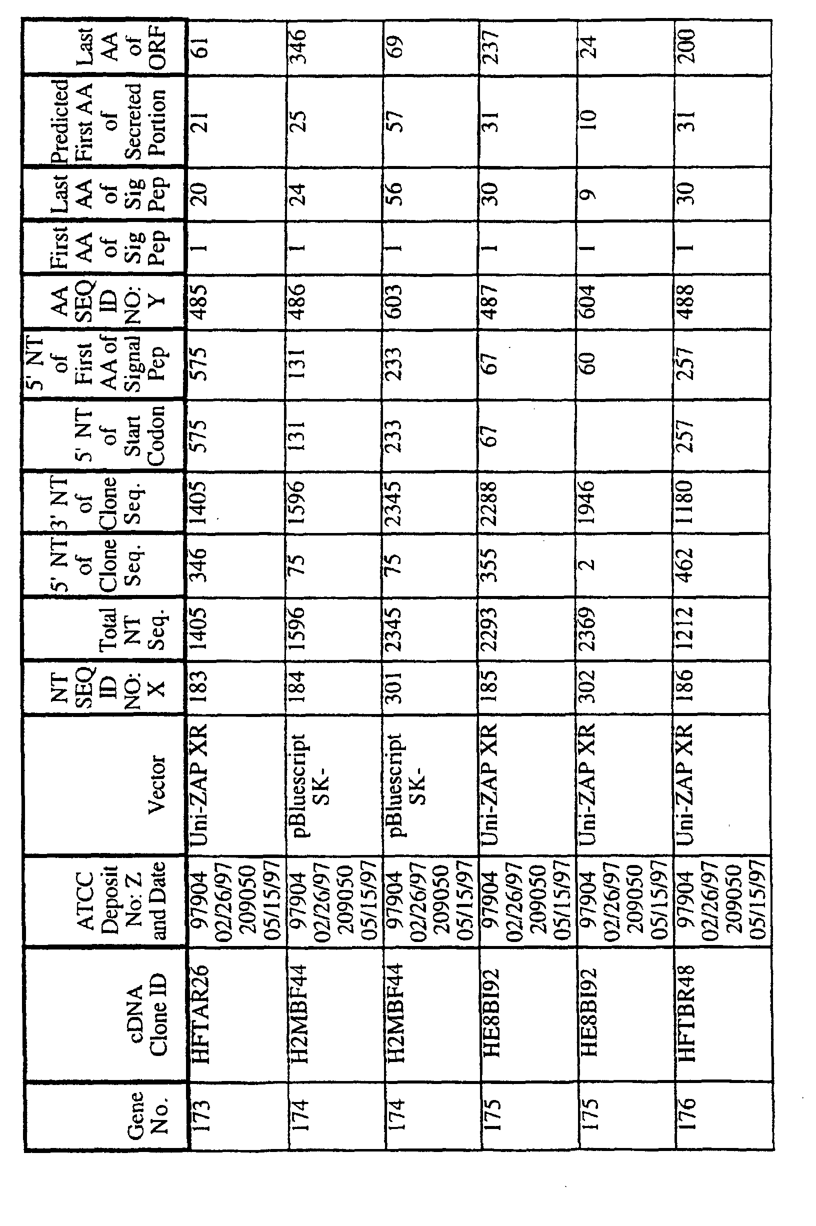

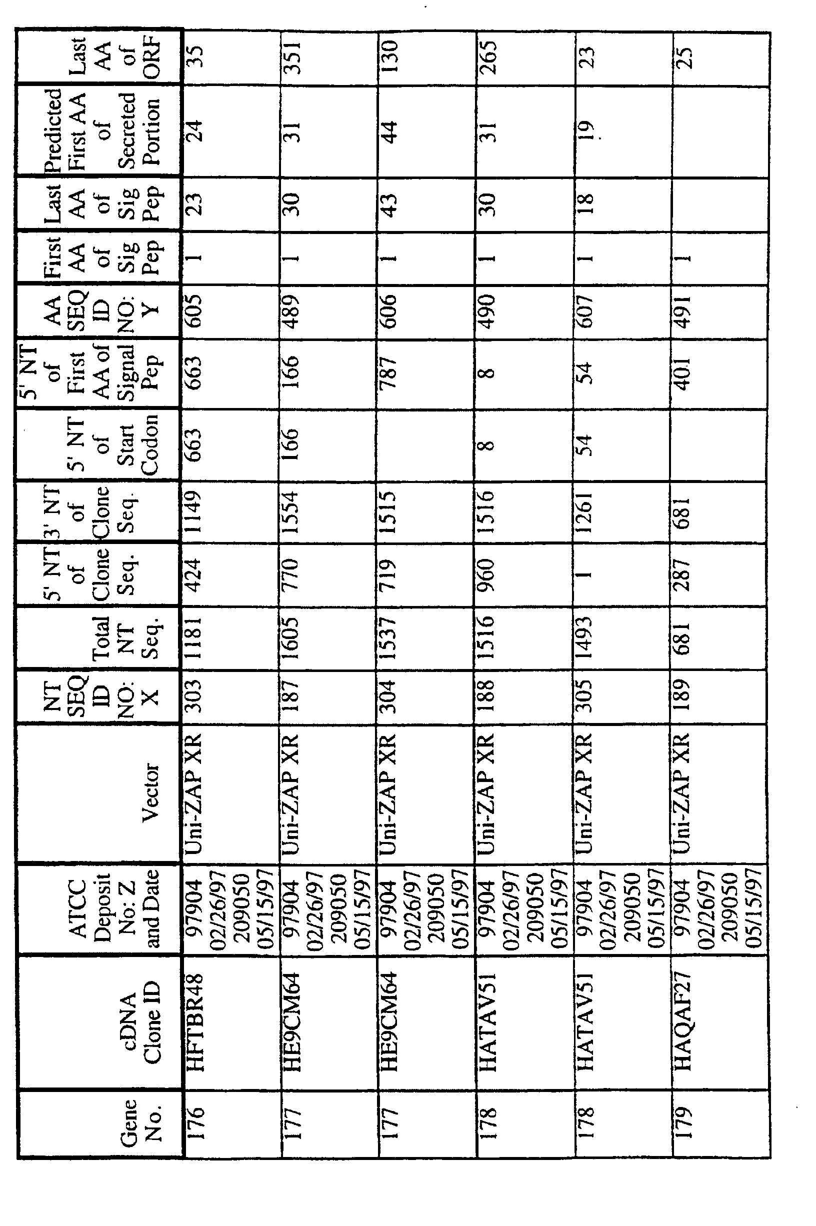

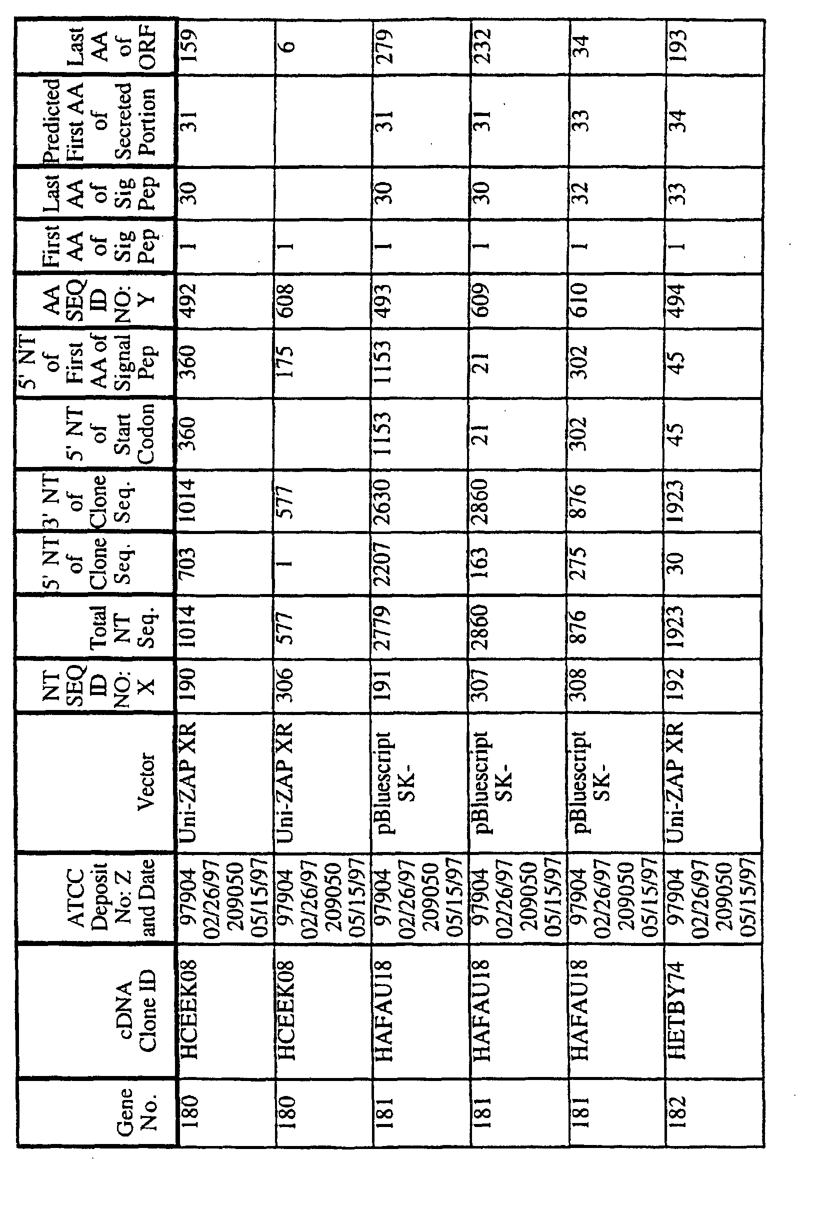

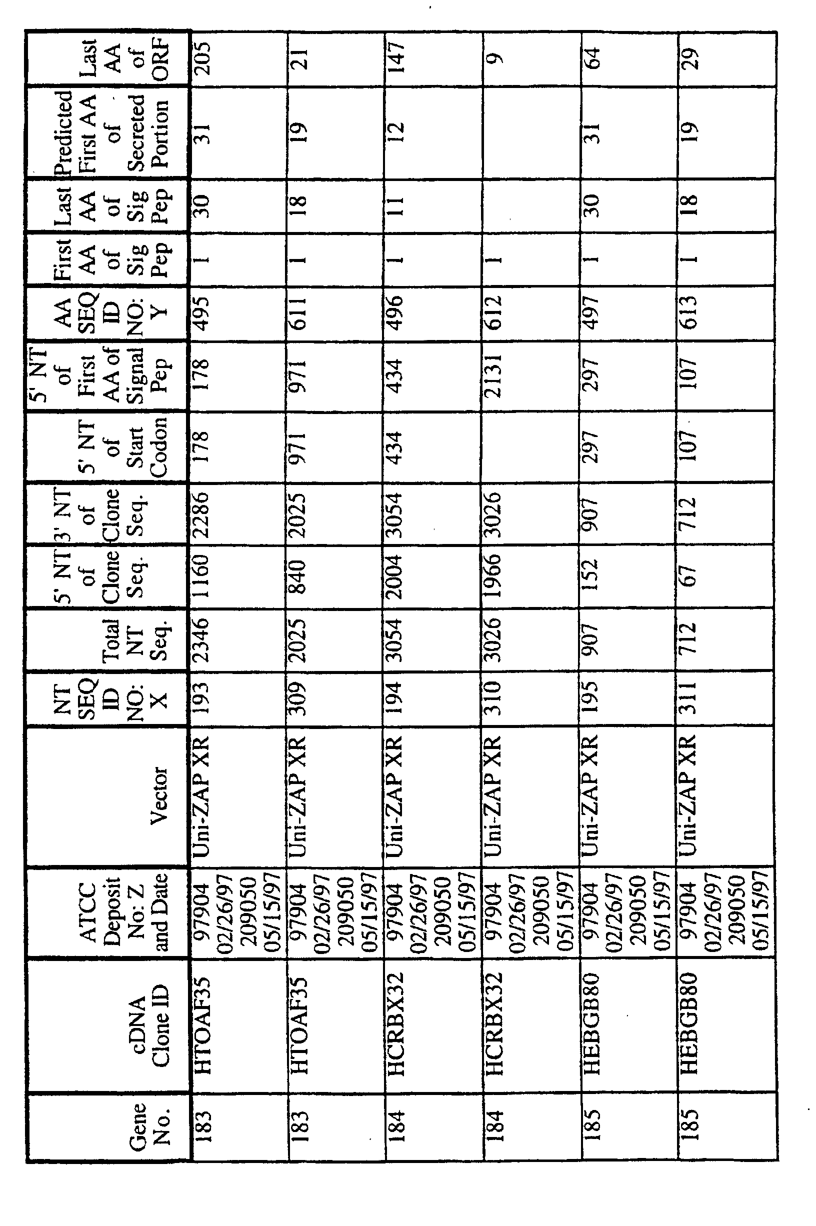

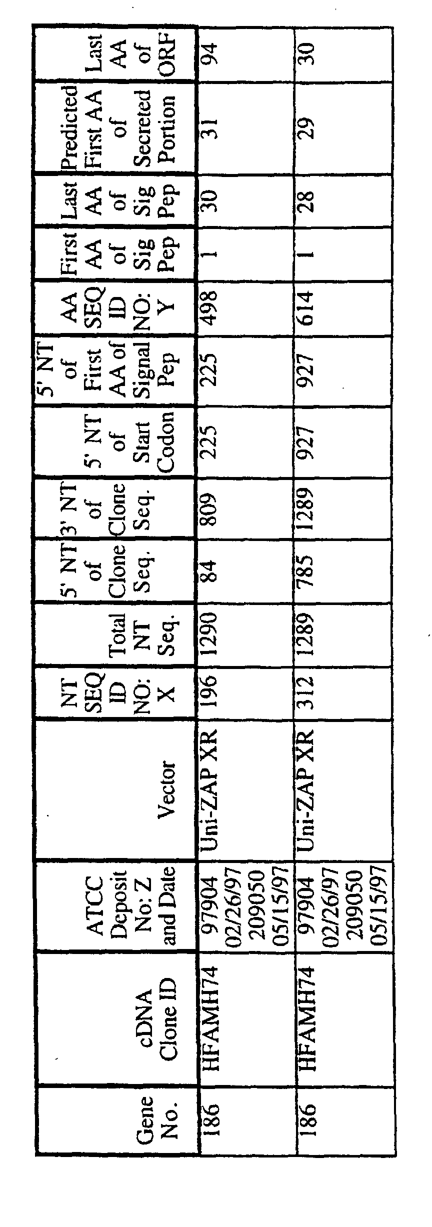

Table 1 summarizes the information corresponding to each "Gene No." described above. The nucleotide sequence identified as "NT SEQ ID NO:X" was assembled from partially homologous ("overlapping") sequences obtained from the "cDNA clone ID" identified in Table 1 and, in some cases, from additional related DNA clones. The overlapping sequences were assembled into a single contiguous sequence of high redundancy (usually three to five overlapping sequences at each nucleotide position), resulting in a final sequence identified as SEQ ID NO:X.

The cDNA Clone ID was deposited on the date and given the corresponding deposit number listed in "ATCC Deposit No:Z and Date." Some of the deposits contain multiple different clones corresponding to the same gene. "Vector" refers to the type of vector contained in the cDNA Clone ID.

"Total NT Seq." refers to the total number of nucleotides in the contig identified by "Gene No." The deposited clone may contain all or most of these sequences, reflected by the nucleotide position indicated as "5' NT of Clone Seq." and the "3' NT of Clone Seq." of SEQ ID NO:X. The nucleotide position of SEQ ID NO:X of the putative start codon (methionine) is identified as "5' NT of Start Codon." Similarly , the nucleotide position of SEQ ID NO:X of the predicted signal sequence is identified as "5' NT of First AA of Signal Pep."

The translated amino acid sequence, beginning with the methionine, is identified as "AA SEQ ID NO:Y," although other reading frames can also be easily translated using known molecular biology techniques. The polypeptides produced by these alternative open reading frames are specifically contemplated by the present invention.

The first and last amino acid position of SEQ ID NO:Y of the predicted signal peptide is identified as "First AA of Sig Pep" and "Last AA of Sig Pep." The predicted first amino acid position of SEQ ID NO:Y of the secreted portion is identified as "Predicted First AA of Secreted Portion." Finally, the amino acid position of SEQ ID NO:Y of the last amino acid in the open reading frame is identified as "Last AA of ORF."

SEQ ID NO:X and the translated SEQ ID NO:Y are sufficiently accurate and otherwise suitable for a variety of uses well known in the art and described further below. For instance, SEQ ID NO:X is useful for designing nucleic acid hybridization probes that will detect nucleic acid sequences contained in SEQ ID NO:X or the cDNA contained in the deposited clone. These probes will also hybridize to nucleic acid molecules in biological samples, thereby enabling a variety of forensic and diagnostic methods of the invention. Similarly, polypeptides identified from SEQ ID NO:Y may be used to generate antibodies which bind specifically to the secreted proteins encoded by the cDNA clones identified in Table 1.

Nevertheless, DNA sequences generated by sequencing reactions can contain sequencing errors. The errors exist as misidentified nucleotides, or as insertions or deletions of nucleotides in the generated DNA sequence. The erroneously inserted or deleted nucleotides cause frame shifts in the reading frames of the predicted amino acid sequence. In these cases, the predicted amino acid sequence diverges from the actual amino acid sequence, even though the generated DNA sequence may be greater than 99.9% identical to the actual DNA sequence (for example, one base insertion or deletion in an open reading frame of over 1000 bases).

Accordingly, for those applications requiring precision in the nucleotide sequence or the amino acid sequence, the present invention provides not only the generated nucleotide sequence identified as SEQ ID NO:X and the predicted translated amino acid sequence identified as SEQ ID NO:Y, but also a sample of plasmid DNA containing a human cDNA of the invention deposited with the ATCC, as set forth in Table 1. The nucleotide sequence of each deposited clone can readily be determined by sequencing the deposited clone in accordance with known methods. The predicted amino acid sequence can then be verified from such deposits. Moreover, the amino acid sequence of the protein encoded by a particular clone can also be directly determined by peptide sequencing or by expressing the protein in a suitable host cell containing the deposited human cDNA, collecting the protein, and determining its sequence.

The present invention also relates to the genes corresponding to SEQ ID NO:X, SEQ ID NO:Y, or the deposited clone. The corresponding gene can be isolated in accordance with known methods using the sequence information disclosed herein. Such methods include preparing probes or primers from the disclosed sequence and identifying or amplifying the corresponding gene from appropriate sources of genomic material.

Also provided in the present invention are species homologs. Species homologs may be isolated and identified by making suitable probes or primers from the sequences provided herein and screening a suitable nucleic acid source for the desired homologue.

The polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The polypeptides may be in the form of the secreted protein, including the mature form, or may be a part of a larger protein, such as a fusion protein (see below). It is often advantageous to include an additional amino acid sequence which contains secretory or leader sequences, pro-sequences, sequences which aid in purification , such as multiple histidine residues, or an additional sequence for stability during recombinant production.

The polypeptides of the present invention are preferably provided in an isolated form, and preferably are substantially purified. A recombinantly produced version of a polypeptide, including the secreted polypeptide, can be substantially purified by the one-step method described in Smith and Johnson, Gene 67:31-40 (1988). Polypeptides of the invention also can be purified from natural or recombinant sources using antibodies of the invention raised against the secreted protein in methods which are well known in the art.

### Signal Sequences

Methods for predicting whether a protein has a signal sequence, as well as the cleavage point for that sequence, are available. For instance, the method of McGeoch, Virus Res. 3:271-286 (1985), uses the information from a short N-terminal charged region and a subsequent uncharged region of the complete (uncleaved) protein. The method of von Heinje, Nucleic Acids Res. 14:4683-4690 (1986) uses the information from the residues surrounding the cleavage site, typically residues -13 to +2, where +1 indicates the amino terminus of the secreted protein. The accuracy of predicting the cleavage points of known mammalian secretory proteins for each of these methods is in the range of 75-80%. (von Heinje, supra.) However, the two methods do not always produce the same predicted cleavage point(s) for a given protein.

In the present case, the deduced amino acid sequence of the secreted polypeptide was analyzed by a computer program called SignalP (Henrik Nielsen et al., Protein Engineering 10:1-6 (1997)), which predicts the cellular location of a protein based on the amino acid sequence. As part of this computational prediction of localization, the methods of McGeoch and von Heinje are incorporated. The analysis of the amino acid sequences of the secreted proteins described herein by this program provided the results shown in Table 1.

As one of ordinary skill would appreciate, however, cleavage sites sometimes vary from organism to organism and cannot be predicted with absolute certainty. Accordingly, the present invention provides secreted polypeptides having a sequence shown in SEQ ID NO:Y which have an N-terminus beginning within 5 residues (i.e., + or - 5 residues) of the predicted cleavage point. Similarly, it is also recognized that in some cases, cleavage of the signal sequence from a secreted protein is not entirely uniform, resulting in more than one secreted species. These polypeptides, and the polynucleotides encoding such polypeptides, are contemplated by the present invention.

Moreover, the signal sequence identified by the above analysis may not necessarily predict the naturally occurring signal sequence. For example, the naturally occurring signal sequence may be further upstream from the predicted signal sequence. However, it is likely that the predicted signal sequence will be capable of directing the secreted protein to the ER. These polypeptides, and the polynucleotides encoding such polypeptides, are contemplated by the present invention.

### Polynucleotide and Polypeptide Variants

"Variant" refers to a polynucleotide or polypeptide differing from the polynucleotide or polypeptide of the present invention, but retaining essential properties thereof. Generally, variants are overall closely similar, and, in many regions, identical to the polynucleotide or polypeptide of the present invention.

"Identity" per se has an art-recognized meaning and can be calculated using published techniques. (See, e.g.: (COMPUTATIONAL MOLECULAR BIOLOGY, Lesk, A.M., ed., Oxford University Press, New York, (1988); BIOCOMPUTING: INFORMATICS AND GENOME PROJECTS, Smith, D.W., ed., Academic Press, New York, (1993); COMPUTER ANALYSIS OF SEQUENCE DATA, PART I, Griffin, A.M., and Griffin, H.G., eds., Humana Press, New Jersey, (1994); SEQUENCE ANALYSIS IN MOLECULAR BIOLOGY, von Heinje, G., Academic Press, (1987); and SEQUENCE ANALYSIS PRIMER, Gribskov, M. and Devereux, J., eds., M Stockton Press, New York, (1991).) While there exists a number of methods to measure identity between two polynucleotide or polypeptide sequences, the term "identity" is well known to skilled artisans. (Carillo, H., and Lipton, D., SIAM J Applied Math 48:1073 (1988).) Methods commonly employed to determine identity or similarity between two sequences include, but are not limited to, those disclosed in "Guide to Huge Computers," Martin J. Bishop, ed., Academic Press, San Diego, (1994), and Carillo, H., and Lipton, D., SIAM J Applied Math 48:1073 (1988). Methods for aligning polynucleotides or polypeptides are codified in computer programs, including the GCG program package (Devereux, J., et al., Nucleic Acids Research (1984) 12(1):387 (1984)), BLASTP, BLASTN, FASTA (Atschul, S.F. et al., J. Molec. Biol. 215:403 (1990), Bestfit program (Wisconsin Sequence Analysis Package, Version 8 for Unix, Genetics Computer Group, University Research Park, 575 Science Drive, Madison, WI 53711 (using the local homology algorithm of Smith and Waterman, Advances in Applied Mathematics 2:482-489 (1981).)

When using any of the sequence alignment programs to determine whether a particular sequence is, for instance, 95% identical to a reference sequence, the parameters are set so that the percentage of identity is calculated over the full length of the reference polynucleotide and that gaps in identity of up to 5% of the total number of nucleotides in the reference polynucleotide are allowed.

A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. 6:237-245 (1990).) The term "sequence" includes nucleotide and amino acid sequences. In a sequence alignment the query and subject sequences are either both nucleotide sequences or both amino acid sequences. The result of said global sequence alignment is in percent identity. Preferred parameters used in a FASTDB search of a DNA sequence to calculate percent identiy are: Matrix=Unitary, k-tuple=4, Mismatch Penalty=1, Joining Penalty=30, Randomization Group Length=0, and Cutoff Score=1, Gap Penalty=5, Gap Size Penalty 0.05, and Window Size=500 or query sequence length in nucleotide bases, whichever is shorter. Preferred parameters employed to calculate percent identity and similarity of an amino acid alignment are: Matrix=PAM 150, k-tuple=2, Mismatch Penalty=1, Joining Penalty=20, Randomization Group Length=0, Cutoff Score=1, Gap Penalty=5, Gap Size Penalty=0.05, and Window Size=500 or query sequence length in amino acid residues, whichever is shorter.

As an illustration, a polynucleotide having a nucleotide sequence of at least 95% "identity" to a sequence contained in SEQ ID NO:X or the cDNA contained in the deposited clone, means that the polynucleotide is identical to a sequence contained in SEQ ID NO:X or the cDNA except that the polynucleotide sequence may include up to five point mutations per each 100 nucleotides of the total length (not just within a given 100 nucleotide stretch). In other words, to obtain a polynucleotide having a nucleotide sequence at least 95% identical to SEQ ID NO:X or the deposited clone, up to 5% of the nucleotides in the sequence contained in SEQ ID NO:X or the cDNA can be deleted, inserted, or substituted with other nucleotides. These changes may occur anywhere throughout the polynucleotide.

Further embodiments of the present invention include polynucleotides having at least 85% identity, more preferably at least 90% identity, and most preferably at least 95%, 96%, 97%, 98% or 99% identity to a sequence contained in SEQ ID NO:X or the cDNA contained in the deposited clone. Of course, due to the degeneracy of the genetic code, one of ordinary skill in the art will immediately recognize that a large number of the polynucleotides having at least 85%, 90%, 95%, 96%, 97%, 98%, or 99% identity will encode a polypeptide identical to an amino acid sequence contained in SEQ ID NO:Y or the expressed protein produced by the deposited clone.

Similarly, by a polypeptide having an amino acid sequence having at least, for example, 95% "identity" to a reference polypeptide, is intended that the amino acid sequence of the polypeptide is identical to the reference polypeptide except that the polypeptide sequence may include up to five amino acid alterations per each 100 amino acids of the total length of the reference polypeptide. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

Further embodiments of the present invention include polypeptides having at least 80% identity, more preferably at least 85% identity, more preferably at least 90% identity, and most preferably at least 95%, 96%, 97%, 98% or 99% identity to an amino acid sequence contained in SEQ ID NO:Y or the expressed protein produced by the deposited clone. Preferably, the above polypeptides should exhibit at least one biological activity of the protein.

In a preferred embodiment, polypeptides of the present invention include polypeptides having at least 90% similarity, more preferably at least 95% similarity, and still more preferably at least 96%, 97%, 98%, or 99% similarity to an amino acid sequence contained in SEQ ID NO:Y or the expressed protein produced by the deposited clone.

The variants may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. Polynucleotide variants can be produced for a variety of reasons, e.g., to optimize codon expression for a particular host (change codons in the human mRNA to those preferred by a bacterial host such as E. coli).

Naturally occurring variants are called "allelic variants," and refer to one of several alternate forms of a gene occupying a given locus on a chromosome of an organism. (Genes II, Lewin, B., ed., John Wiley & Sons, New York (1985).) These allelic variants can vary at either the polynucleotide and/or polypeptide level. Alternatively, non-naturally occurring variants may be produced by mutagenesis techniques or by direct synthesis.

Using known methods of protein engineering and recombinant DNA technology, variants may be generated to improve or alter the characteristics of the polypeptides of the present invention. For instance, one or more amino acids can be deleted from the N-terminus or C-terminus of the secreted protein without substantial loss of biological function. The authors of Ron et al., J. Biol. Chem. 268: 2984-2988 (1993), reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein. (Dobeli et al., J. Biotechnology 7:199-216 (1988).)

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See, Abstract.) In fact, only 23 unique amino acid sequences, out of more than 3,500 nucleotide sequences examined, produced a protein that significantly differed in activity from wild-type.

Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the secreted form will likely be retained when less than the majority of the residues of the secreted form are removed from the N-terminus or C-terminus. Whether a particular polypeptide lacking N- or C-terminal residues of a protein retains such immunogenic activities can readily be determined by routine methods described herein and otherwise known in the art.

Thus, the invention further includes polypeptide variants which show substantial biological activity. Such variants include deletions, insertions, inversions, repeats, and substitutions selected according to general rules known in the art so as have little effect on activity. For example, guidance concerning how to make phenotypically silent amino acid substitutions is provided in Bowie, J. U. et al., Science 247:1306-1310 (1990), wherein the authors indicate that there are two main strategies for studying the tolerance of an amino acid sequence to change.

The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions where substitutions have been tolerated by natural selection indicates that these positions are not critical for protein function. Thus, positions tolerating amino acid substitution could be modified while still maintaining biological activity of the protein.

The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used. (Cunningham and Wells, Science 244:1081-1085 (1989).) The resulting mutant molecules can then be tested for biological activity.

As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

Besides conservative amino acid substitution, variants of the present invention include (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (for example, polyethylene glycol), or (iv) fusion of the polypeptide with additional amino acids, such as an IgG Fc fusion region peptide, or leader or secretory sequence, or a sequence facilitating purification. Such variant polypeptides are deemed to be within the scope of those skilled in the art from the teachings herein.

For example, polypeptide variants containing amino acid substitutions of charged amino acids with other charged or neutral amino acids may produce proteins with improved characteristics, such as less aggregation. Aggregation of pharmaceutical formulations both reduces activity and increases clearance due to the aggregate's immunogenic activity. (Pinckard et al., Clin. Exp. Immunol. 2:331-340 (1967); Robbins et al., Diabetes 36: 838-845 (1987); Cleland et al., Crit. Rev. Therapeutic Drug Carrier Systems 10:307-377 (1993).)

### Polynucleotide and Polypeptide Fragments

In the present invention, a "polynucleotide fragment" refers to a short polynucleotide having a nucleic acid sequence contained in the deposited clone or shown in SEQ ID NO:X. The short nucleotide fragments are preferably at least about 15 nt, and more preferably at least about 20 nt, still more preferably at least about 30 nt, and even more preferably, at least about 40 nt in length. A fragment "at least 20 nt in length," for example, is intended to include 20 or more contiguous bases from the cDNA sequence contained in the deposited clone or the nucleotide sequence shown in SEQ ID NO:X. These nucleotide fragments are useful as diagnostic probes and primers as discussed herein. Of course, larger fragments (e.g., 50, 150, 500, 600, 2000 nucleotides) are preferred.

Moreover, representative examples of polynucleotide fragments of the invention, include, for example, fragments having a sequence from about nucleotide number 1-50, 51-100, 101-150, 151-200, 201-250, 251-300, 301-350, 351-400, 401-450, 451-500, 501-550, 551-600, 651-700, and 701 to the end of SEQ ID NO:X or the cDNA contained in the deposited clone. In this context "about" includes the particularly recited ranges, larger or smaller by several (5, 4, 3, 2, or 1) nucleotides, at either terminus or at both termini. Preferably, these fragments encode a polypeptide which has biological activity.

In the present invention, a "polypeptide fragment" refers to a short amino acid sequence contained in SEQ ID NO:Y or encoded by the cDNA contained in the deposited clone. Protein fragments may be "free-standing," or comprised within a larger polypeptide of which the fragment forms a part or region, most preferably as a single continuous region. Representative examples of polypeptide fragments of the invention, include, for example, fragments from about amino acid number 1-20, 21-40, 41-60, 61-80, 81-100, 102-120, 121-140, 141-160, and 161 to the end of the coding region. Moreover, polypeptide fragments can be about 20, 30, 40, 50, 60, 70, 80, 90, 100, 110, 120, 130, 140, or 150 amino acids in length. In this context "about" includes the particularly recited ranges, larger or smaller by several (5, 4, 3, 2, or 1) amino acids, at either extreme or at both extremes.

Preferred polypeptide fragments include the secreted protein as well as the mature form. Further preferred polypeptide fragments include the secreted protein or the mature form having a continuous series of deleted residues from the amino or the carboxy terminus, or both. For example, any number of amino acids, ranging from 1-60, can be deleted from the amino terminus of either the secreted polypeptide or the mature form. Similarly, any number of amino acids, ranging from 1-30, can be deleted from the carboxy terminus of the secreted protein or mature form. Furthermore, any combination of the above amino and carboxy terminus deletions are preferred. Similarly, polynucleotide fragments encoding these polypeptide fragments are also preferred.

Also preferred are polypeptide and polynucleotide fragments characterized by structural or functional domains, such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Polypeptide fragments of SEQ ID NO:Y falling within conserved domains are specifically contemplated by the present invention. Moreover, polynucleotide fragments encoding these domains are also contemplated.

Other preferred fragments are biologically active fragments. Biologically active fragments are those exhibiting activity similar, but not necessarily identical, to an activity of the polypeptide of the present invention. The biological activity of the fragments may include an improved desired activity, or a decreased undesirable activity.

### Epitopes & Antibodies

In the present invention, "epitopes" refer to polypeptide fragments having antigenic or immunogenic activity in an animal, especially in a human. A preferred embodiment of the present invention relates to a polypeptide fragment comprising an epitope, as well as the polynucleotide encoding this fragment. A region of a protein molecule to which an antibody can bind is defined as an "antigenic epitope." In contrast, an "immunogenic epitope" is defined as a part of a protein that elicits an antibody response. (See, for instance, Geysen et al., Proc. Natl. Acad. Sci. USA 81:3998-4002 (1983).)

Fragments which function as epitopes may be produced by any conventional means. (See, e.g., Houghten, R. A., Proc. Natl. Acad. Sci. USA 82:5131-5135 (1985) further described in U.S. Patent No. 4,631,211.)

In the present invention, antigenic epitopes preferably contain a sequence of at least seven, more preferably at least nine, and most preferably between about 15 to about 30 amino acids. Antigenic epitopes are useful to raise antibodies, including monoclonal antibodies, that specifically bind the epitope. (See, for instance, Wilson et al., Cell 37:767-778 (1984); Sutcliffe, J. G. et al., Science 219:660-666 (1983).)

Similarly, immunogenic epitopes can be used to induce antibodies according to methods well known in the art. (See, for instance, Sutcliffe et al., supra; Wilson et al., supra; Chow, M. et al., Proc. Natl. Acad. Sci. USA 82:910-914; and Bittle, F. J. et al., J. Gen. Virol. 66:2347-2354 (1985).) A preferred immunogenic epitope includes the secreted protein. The immunogenic epitopes may be presented together with a carrier protein, such as an albumin, to an animal system (such as rabbit or mouse) or, if it is long enough (at least about 25 amino acids), without a carrier. However, immunogenic epitopes comprising as few as 8 to 10 amino acids have been shown to be sufficient to raise antibodies capable of binding to, at the very least, linear epitopes in a denatured polypeptide (e.g., in Western blotting.)

As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. (Wahl et al., J. Nucl. Med. 24:316-325 (1983).) Thus, these fragments are preferred, as well as the products of a FAB or other immunoglobulin expression library. Moreover, antibodies of the present invention include chimeric, single chain, and humanized antibodies.

### Fusion Proteins

Any polypeptide of the present invention can be used to generate fusion proteins. For example, the polypeptide of the present invention, when fused to a second protein, can be used as an antigenic tag. Antibodies raised against the polypeptide of the present invention can be used to indirectly detect the second protein by binding to the polypeptide. Moreover, because secreted proteins target cellular locations based on trafficking signals, the polypeptides of the present invention can be used as targeting molecules once fused to other proteins.

Examples of domains that can be fused to polypeptides of the present invention include not only heterologous signal sequences, but also other heterologous functional regions. The fusion does not necessarily need to be direct, but may occur through linker sequences.

Moreover, fusion proteins may also be engineered to improve characteristics of the polypeptide of the present invention. For instance, a region of additional amino acids, particularly charged amino acids, may be added to the N-terminus of the polypeptide to improve stability and persistence during purification from the host cell or subsequent handling and storage. Also, peptide moieties may be added to the polypeptide to facilitate purification. Such regions may be removed prior to final preparation of the polypeptide. The addition of peptide moieties to facilitate handling of polypeptides are familiar and routine techniques in the art.

Moreover, polypeptides of the present invention, including fragments, and specifically epitopes, can be combined with parts of the constant domain of immunoglobulins (IgG), resulting in chimeric polypeptides. These fusion proteins facilitate purification and show an increased half-life in vivo. One reported example describes chimeric proteins consisting of the first two domains of the human CD4-polypeptide and various domains of the constant regions of the heavy or light chains of mammalian immunoglobulins. (EP A 394,827; Traunecker et al., Nature 331:84-86 (1988).) Fusion proteins having disulfide-linked dimeric structures (due to the IgG) can also be more efficient in binding and neutralizing other molecules, than the monomeric secreted protein or protein fragment alone. (Fountoulakis et al., J. Biochem. 270:3958-3964 (1995).)

Similarly, EP-A-O 464 533 (Canadian counterpart 2045869) discloses fusion proteins comprising various portions of constant region of immunoglobulin molecules together with another human protein or part thereof. In many cases, the Fc part in a fusion protein is beneficial in therapy and diagnosis, and thus can result in, for example, improved pharmacokinetic properties. (EP-A 0232 262.) Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, D. Bennett et al., J. Molecular Recognition 8:52-58 (1995); K. Johanson et al., J. Biol. Chem. 270:9459-9471 (1995).)

Moreover, the polypeptides of the present invention can be fused to marker sequences, such as a peptide which facilitates purification of the fused polypeptide. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86:821-824 (1989), for instance, hexa-histidine provides for convenient purification of the fusion protein. Another peptide tag useful for purification, the "HA" tag, corresponds to an epitope derived from the influenza hemagglutinin protein. (Wilson et al., Cell 37:767 (1984).)

Thus, any of these above fusions can be engineered using the polynucleotides or the polypeptides of the claimed invention.

### Vectors, Host Cells, and Protein Production

The present invention also relates to vectors containing the polynucleotide of the present invention, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The polynucleotide insert should be operatively linked to an appropriate promoter, such as the phage lambda PL promoter, the E. coli lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells such as CHO, COS, 293, and Bowes melanoma cells; and plant cells. Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNH8A, pNH16a, pNH18A, pNH46A, available from Stratagene Cloning Systems, Inc.; and ptrc99a, pKK223-3, pKK233-3, pDR540, pRIT5 available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

A polypeptide of this invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention, and preferably the secreted form, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

### Uses of the Polynucleotides

Each of the polynucleotides identified herein can be used in numerous ways as reagents. The following description should be considered exemplary and utilizes known techniques.

The polynucleotides of the present invention are useful for chromosome identification. There exists an ongoing need to identify new chromosome markers, since few chromosome marking reagents, based on actual sequence data (repeat polymorphisms), are presently available. Each polynucleotide of the present invention can be used as a chromosome marker.

Briefly, sequences can be mapped to chromosomes by preparing PCR primers (preferably 15-25 bp) from the sequences shown in SEQ ID NO:X. Primers can be selected using computer analysis so that primers do not span more than one predicted exon in the genomic DNA. These primers are then used for PCR screening of somatic cell hybrids containing individual human chromosomes. Only those hybrids containing the human gene corresponding to the SEQ ID NO:X will yield an amplified fragment.

Similarly, somatic hybrids provide a rapid method of PCR mapping the polynucleotides to particular chromosomes. Three or more clones can be assigned per day using a single thermal cycler. Moreover, sublocalization of the polynucleotides can be achieved with panels of specific chromosome fragments. Other gene mapping strategies that can be used include in situ hybridization, prescreening with labeled flow-sorted chromosomes, and preselection by hybridization to construct chromosome specific-cDNA libraries.

Precise chromosomal location of the polynucleotides can also be achieved using fluorescence in situ hybridization (FISH) of a metaphase chromosomal spread. This technique uses polynucleotides as short as 500 or 600 bases; however, polynucleotides 2,000-4,000 bp are preferred. For a review of this technique, see Verma et al., "Human Chromosomes: a Manual of Basic Techniques," Pergamon Press, New York (1988).

For chromosome mapping, the polynucleotides can be used individually (to mark a single chromosome or a single site on that chromosome) or in panels (for marking multiple sites and/or multiple chromosomes). Preferred polynucleotides correspond to the noncoding regions of the cDNAs because the coding sequences are more likely conserved within gene families, thus increasing the chance of cross hybridization during chromosomal mapping.

Once a polynucleotide has been mapped to a precise chromosomal location, the physical position of the polynucleotide can be used in linkage analysis. Linkage analysis establishes coinheritance between a chromosomal location and presentation of a particular disease. (Disease mapping data are found, for example, in V. McKusick, Mendelian Inheritance in Man (available on line through Johns Hopkins University Welch Medical Library).) Assuming 1 megabase mapping resolution and one gene per 20 kb, a cDNA precisely localized to a chromosomal region associated with the disease could be one of 50-500 potential causative genes.

Thus, once coinheritance is established, differences in the polynucleotide and the corresponding gene between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the polypeptide and the corresponding gene from several normal individuals is required to distinguish the mutation from a polymorphism. If a new polymorphism is identified, this polymorphic polypeptide can be used for further linkage analysis.

Furthermore, increased or decreased expression of the gene in affected individuals as compared to unaffected individuals can be assessed using polynucleotides of the present invention. Any of these alterations (altered expression, chromosomal rearrangement, or mutation) can be used as a diagnostic or prognostic marker.

In addition to the foregoing, a polynucleotide can be used to control gene expression through triple helix formation or antisense DNA or RNA. Both methods rely on binding of the polynucleotide to DNA or RNA. For these techniques, preferred polynucleotides are usually 20 to 40 bases in length and complementary to either the region of the gene involved in transcription (triple helix - see Lee et al., Nucl. Acids Res. 6:3073 (1979); Cooney et al., Science 241:456 (1988); and Dervan et al., Science 251:1360 (1991)) or to the mRNA itself (antisense - Okano, J. Neurochem. 56:560 (1991); Oligodeoxy-nucleotides as Antisense Inhibitors of Gene Expression, CRC Press, Boca Raton, FL (1988).) Triple helix formation optimally results in a shut-off of RNA transcription from DNA, while antisense RNA hybridization blocks translation of an mRNA molecule into polypeptide. Both techniques are effective in model systems, and the information disclosed herein can be used to design antisense or triple helix polynucleotides in an effort to treat disease.

Polynucleotides of the present invention are also useful in gene therapy. One goal of gene therapy is to insert a normal gene into an organism having a defective gene, in an effort to correct the genetic defect. The polynucleotides disclosed in the present invention offer a means of targeting such genetic defects in a highly accurate manner. Another goal is to insert a new gene that was not present in the host genome, thereby producing a new trait in the host cell.

The polynucleotides are also useful for identifying individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identifying personnel. This method does not suffer from the current limitations of "Dog Tags" which can be lost, switched, or stolen, making positive identification difficult. The polynucleotides of the present invention can be used as additional DNA markers for RFLP.

The polynucleotides of the present invention can also be used as an alternative to RFLP, by determining the actual base-by-base DNA sequence of selected portions of an individual's genome. These sequences can be used to prepare PCR primers for amplifying and isolating such selected DNA, which can then be sequenced. Using this technique, individuals can be identified because each individual will have a unique set of DNA sequences. Once an unique ID database is established for an individual, positive identification of that individual, living or dead, can be made from extremely small tissue samples.

Forensic biology also benefits from using DNA-based identification techniques as disclosed herein. DNA sequences taken from very small biological samples such as tissues, e.g., hair or skin, or body fluids, e.g., blood, saliva, semen, etc., can be amplified using PCR. In one prior art technique, gene sequences amplified from polymorphic loci, such as DQa class II HLA gene, are used in forensic biology to identify individuals. (Erlich, H., PCR Technology, Freeman and Co. (1992).) Once these specific polymorphic loci are amplified, they are digested with one or more restriction enzymes, yielding an identifying set of bands on a Southern blot probed with DNA corresponding to the DQa class II HLA gene. Similarly, polynucleotides of the present invention can be used as polymorphic markers for forensic purposes.

There is also a need for reagents capable of identifying the source of a particular tissue. Such need arises, for example, in forensics when presented with tissue of unknown origin. Appropriate reagents can comprise, for example, DNA probes or primers specific to particular tissue prepared from the sequences of the present invention. Panels of such reagents can identify tissue by species and/or by organ type. In a similar fashion, these reagents can be used to screen tissue cultures for contamination.

In the very least, the polynucleotides of the present invention can be used as molecular weight markers on Southern gels, as diagnostic probes for the presence of a specific mRNA in a particular cell type, as a probe to "subtract-out" known sequences in the process of discovering novel polynucleotides, for selecting and making oligomers for attachment to a "gene chip" or other support, to raise anti-DNA antibodies using DNA immunization techniques, and as an antigen to elicit an immune response.

### Uses of the Polypeptides

Each of the polypeptides identified herein can be used in numerous ways. The following description should be considered exemplary and utilizes known techniques.

A polypeptide of the present invention can be used to assay protein levels in a biological sample using antibody-based techniques. For example, protein expression in tissues can be studied with classical immunohistological methods. (Jalkanen, M., et al., J. Cell. Biol. 101:976-985 (1985); Jalkanen, M., et al., J. Cell . Biol. 105:3087-3096 (1987).) Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (125I, 121I), carbon (14C), sulfur (35S), tritium (3H), indium (112In), and technetium (99mTc), and fluorescent labels, such as fluorescein and rhodamine, and biotin.

In addition to assaying secreted protein levels in a biological sample, proteins can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, 131I, 112In, 99mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982).)

Thus, the invention provides a diagnostic method of a disorder, which involves (a) assaying the expression of a polypeptide of the present invention in cells or body fluid of an individual; (b) comparing the level of gene expression with a standard gene expression level, whereby an increase or decrease in the assayed polypeptide gene expression level compared to the standard expression level is indicative of a disorder.

Moreover, polypeptides of the present invention can be used to treat disease. For example, patients can be administered a polypeptide of the present invention in an effort to replace absent or decreased levels of the polypeptide (e.g., insulin), to supplement absent or decreased levels of a different polypeptide (e.g., hemoglobin S for hemoglobin B), to inhibit the activity of a polypeptide (e.g., an oncogene), to activate the activity of a polypeptide (e.g., by binding to a receptor), to reduce the activity of a membrane bound receptor by competing with it for free ligand (e.g., soluble TNF receptors used in reducing inflammation), or to bring about a desired response (e.g., blood vessel growth).

Similarly, antibodies directed to a polypeptide of the present invention can also be used to treat disease. For example, administration of an antibody directed to a polypeptide of the present invention can bind and reduce overproduction of the polypeptide. Similarly, administration of an antibody can activate the polypeptide, such as by binding to a polypeptide bound to a membrane (receptor).

At the very least, the polypeptides of the present invention could be used as molecular weight markers on SDS-PAGE gels or on molecular sieve gel filtration columns using methods well known to those of skill in the art. Polypeptides can also be used to raise antibodies, which in turn are used to measure protein expression from a recombinant cell, as a way of assessing transformation of the host cell. Moreover, the polypeptides of the present invention can be used to test the following biological activities.

### Biological Activities

The polynucleotides and polypeptides of the present invention can be used in assays to test for one or more biological activities. If these polynucleotides and polypeptides do exhibit activity in a particular assay, it is likely that these molecules may be involved in the diseases associated with the biological activity. Thus, the polynucleotides and polypeptides could be used to treat the associated disease.

### Immune Activity

A polypeptide or polynucleotide of the present invention may be useful in treating deficiencies or disorders of the immune system, by activating or inhibiting the proliferation, differentiation, or mobilization (chemotaxis) of immune cells. Immune cells develop through a process called hematopoiesis, producing myeloid (platelets, red blood cells, neutrophils, and macrophages) and lymphoid (B and T lymphocytes) cells from pluripotent stem cells. The etiology of these immune deficiencies or disorders may be genetic, somatic, such as cancer or some autoimmune disorders, acquired (e.g., by chemotherapy or toxins), or infectious. Moreover, a polynucleotide or polypeptide of the present invention can be used as a marker or detector of a particular immune system disease or disorder.

A polynucleotide or polypeptide of the present invention may be useful in treating or detecting deficiencies or disorders of hematopoietic cells. A polypeptide or polynucleotide of the present invention could be used to increase differentiation and proliferation of hematopoietic cells, including the pluripotent stem cells, in an effort to treat those disorders associated with a decrease in certain (or many) types hematopoietic cells. Examples of immunologic deficiency syndromes include, but are not limited to: blood protein disorders (e.g. agammaglobulinemia, dysgammaglobulinemia), ataxia telangiectasia, common variable immunodeficiency, Digeorge Syndrome, HIV infection, HTLV-BLV infection, leukocyte adhesion deficiency syndrome, lymphopenia, phagocyte bactericidal dysfunction, severe combined immunodeficiency (SCIDs), Wiskott-Aldrich Disorder, anemia, thrombocytopenia, or hemoglobinuria.

Moreover, a polypeptide or polynucleotide of the present invention could also be used to modulate hemostatic (the stopping of bleeding) or thrombolytic activity (clot formation). For example, by increasing hemostatic or thrombolytic activity, a polynucleotide or polypeptide of the present invention could be used to treat blood coagulation disorders (e.g., afibrinogenemia, factor deficiencies), blood platelet disorders (e.g. thrombocytopenia), or wounds resulting from trauma, surgery, or other causes. Alternatively, a polynucleotide or polypeptide of the present invention that can decrease hemostatic or thrombolytic activity could be used to inhibit or dissolve clotting. These molecules could be important in the treatment of heart attacks (infarction), strokes, or scarring.

A polynucleotide or polypeptide of the present invention may also be useful in treating or detecting autoimmune disorders. Many autoimmune disorders result from inappropriate recognition of self as foreign material by immune cells. This inappropriate recognition results in an immune response leading to the destruction of the host tissue. Therefore, the administration of a polypeptide or polynucleotide of the present invention that inhibits an immune response, particularly the proliferation, differentiation, or chemotaxis of T-cells, may be an effective therapy in preventing autoimmune disorders.

Examples of autoimmune disorders that can be treated or detected by the present invention include, but are not limited to: Addison's Disease, hemolytic anemia, antiphospholipid syndrome, rheumatoid arthritis, dermatitis, allergic encephalomyelitis, glomerulonephritis, Goodpasture's Syndrome, Graves' Disease, Multiple Sclerosis, Myasthenia Gravis, Neuritis, Ophthalmia, Bullous Pemphigoid, Pemphigus, Polyendocrinopathies, Purpura, Reiter's Disease, Stiff-Man Syndrome, Autoimmune Thyroiditis, Systemic Lupus Erythematosus, Autoimmune Pulmonary Inflammation, Guillain-Barre Syndrome, insulin dependent diabetes mellitis, and autoimmune inflammatory eye disease.

Similarly, allergic reactions and conditions, such as asthma (particularly allergic asthma) or other respiratory problems, may also be treated by a polypeptide or polynucleotide of the present invention. Moreover, these molecules can be used to treat anaphylaxis, hypersensitivity to an antigenic molecule, or blood group incompatibility.

A polynucleotide or polypeptide of the present invention may also be used to treat and/or prevent organ rejection or graft-versus-host disease (GVHD). Organ rejection occurs by host immune cell destruction of the transplanted tissue through an immune response. Similarly, an immune response is also involved in GVHD, but, in this case, the foreign transplanted immune cells destroy the host tissues. The administration of a polypeptide or polynucleotide of the present invention that inhibits an immune response, particularly the proliferation, differentiation, or chemotaxis of T-cells, may be an effective therapy in preventing organ rejection or GVHD.

Similarly, a polypeptide or polynucleotide of the present invention may also be used to modulate inflammation. For example, the polypeptide or polynucleotide may inhibit the proliferation and differentiation of cells involved in an inflammatory response. These molecules can be used to treat inflammatory conditions, both chronic and acute conditions, including inflammation associated with infection (e.g., septic shock, sepsis, or systemic inflammatory response syndrome (SIRS)), ischemia-reperfusion injury, endotoxin lethality, arthritis, complement-mediated hyperacute rejection, nephritis, cytokine or chemokine induced lung injury, inflammatory bowel disease, Crohn's disease, or resulting from over production of cytokines (e.g., TNF or IL-1.)

### Hyperproliferative Disorders

A polypeptide or polynucleotide can be used to treat or detect hyperproliferative disorders, including neoplasms. A polypeptide or polynucleotide of the present invention may inhibit the proliferation of the disorder through direct or indirect interactions. Alternatively, a polypeptide or polynucleotide of the present invention may proliferate other cells which can inhibit the hyperproliferative disorder.

For example, by increasing an immune response, particularly increasing antigenic qualities of the hyperproliferative disorder or by proliferating, differentiating, or mobilizing T-cells, hyperproliferative disorders can be treated. This immune response may be increased by either enhancing an existing immune response, or by initiating a new immune response. Alternatively, decreasing an immune response may also be a method of treating hyperproliferative disorders, such as a chemotherapeutic agent.

Examples of hyperproliferative disorders that can be treated or detected by a polynucleotide or polypeptide of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital.

Similarly, other hyperproliferative disorders can also be treated or detected by a polynucleotide or polypeptide of the present invention. Examples of such hyperproliferative disorders include, but are not limited to: hypergammaglobulinemia, lymphoproliferative disorders, paraproteinemias, purpura, sarcoidosis, Sezary Syndrome, Waldenstron's Macroglobulinemia, Gaucher's Disease, histiocytosis, and any other hyperproliferative disease, besides neoplasia, located in an organ system listed above.

### Infectious Disease

A polypeptide or polynucleotide of the present invention can be used to treat or detect infectious agents. For example, by increasing the immune response, particularly increasing the proliferation and differentiation of B and/or T cells, infectious diseases may be treated. The immune response may be increased by either enhancing an existing immune response, or by initiating a new immune response. Alternatively, the polypeptide or polynucleotide of the present invention may also directly inhibit the infectious agent, without necessarily eliciting an immune response.

Viruses are one example of an infectious agent that can cause disease or symptoms that can be treated or detected by a polynucleotide or polypeptide of the present invention. Examples of viruses, include, but are not limited to the following DNA and RNA viral families: Arbovirus, Adenoviridae, Arenaviridae, Arterivirus, Birnaviridae, Bunyaviridae, Caliciviridae, Circoviridae, Coronaviridae, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza), Papovaviridae, Parvoviridae, Picornaviridae, Poxviridae (such as Smallpox or Vaccinia), Reoviridae (e.g., Rotavirus), Retroviridae (HTLV-I, HTLV-II, Lentivirus), and Togaviridae (e.g., Rubivirus). Viruses falling within these families can cause a variety of diseases or symptoms, including, but not limited to: arthritis, bronchiollitis, encephalitis, eye infections (e.g., conjunctivitis, keratitis), chronic fatigue syndrome, hepatitis (A, B, C, E, Chronic Active, Delta), meningitis, opportunistic infections (e.g., AIDS), pneumonia, Burkitt's Lymphoma, chickenpox , hemorrhagic fever, Measles, Mumps, Parainfluenza, Rabies, the common cold, Polio, leukemia, Rubella, sexually transmitted diseases, skin diseases (e.g., Kaposi's, warts), and viremia. A polypeptide or polynucleotide of the present invention can be used to treat or detect any of these symptoms or diseases.

Similarly, bacterial or fungal agents that can cause disease or symptoms and that can be treated or detected by a polynucleotide or polypeptide of the present invention include, but not limited to, the following Gram-Negative and Gram-positive bacterial families and fungi: Actinomycetales (e.g., Corynebacterium, Mycobacterium, Norcardia), Aspergillosis, Bacillaceae (e.g., Anthrax, Clostridium), Bacteroidaceae, Blastomycosis, Bordetella, Borrelia, Brucellosis, Candidiasis, Campylobacter, Coccidioidomycosis, Cryptococcosis, Dermatocycoses, Enterobacteriaceae (Klebsiella, Salmonella, Serratia, Yersinia), Erysipelothrix, Helicobacter, Legionellosis, Leptospirosis, Listeria, Mycoplasmatales, Neisseriaceae (e.g., Acinetobacter, Gonorrhea, Menigococcal), Pasteurellacea Infections (e.g., Actinobacillus, Heamophilus, Pasteurella), Pseudomonas, Rickettsiaceae, Chlamydiaceae, Syphilis, and Staphylococcal. These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis, Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections, wound infections. A polypeptide or polynucleotide of the present invention can be used to treat or detect any of these symptoms or diseases.

Moreover, parasitic agents causing disease or symptoms that can be treated or detected by a polynucleotide or polypeptide of the present invention include, but not limited to, the following families: Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, and Trichomonas. These parasites can cause a variety of diseases or symptoms, including, but not limited to: Scabies, Trombiculiasis, eye infections, intestinal disease (e.g., dysentery, giardiasis), liver disease. lung disease, opportunistic infections (e.g., AIDS related), Malaria, pregnancy complications, and toxoplasmosis. A polypeptide or polynucleotide of the present invention can be used to treat or detect any of these symptoms or diseases.

Preferably, treatment using a polypeptide or polynucleotide of the present invention could either be by administering an effective amount of a polypeptide to the patient, or by removing cells from the patient, supplying the cells with a polynucleotide of the present invention, and returning the engineered cells to the patient (ex vivo therapy). Moreover, the polypeptide or polynucleotide of the present invention can be used as an antigen in a vaccine to raise an immune response against infectious disease.

### Regeneration

A polynucleotide or polypeptide of the present invention can be used to differentiate, proliferate, and attract cells, leading to the regeneration of tissues. (See, Science 276:59-87 (1997).) The regeneration of tissues could be used to repair, replace, or protect tissue damaged by congenital defects, trauma (wounds, burns, incisions, or ulcers), age, disease (e.g. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (e.g., pancreas, liver, intestine, kidney, skin, endothelium), muscle (smooth, skeletal or cardiac), vascular (including vascular endothelium), nervous, hematopoietic, and skeletal (bone, cartilage, tendon, and ligament) tissue. Preferably, regeneration occurs without or decreased scarring. Regeneration also may include angiogenesis.

Moreover, a polynucleotide or polypeptide of the present invention may increase regeneration of tissues difficult to heal. For example, increased tendon/ligament regeneration would quicken recovery time after damage. A polynucleotide or polypeptide of the present invention could also be used prophylactically in an effort to avoid damage. Specific diseases that could be treated include of tendinitis, carpal tunnel syndrome, and other tendon or ligament defects. A further example of tissue regeneration of non-healing wounds includes pressure ulcers, ulcers associated with vascular insufficiency, surgical, and traumatic wounds.

Similarly, nerve and brain tissue could also be regenerated by using a polynucleotide or polypeptide of the present invention to proliferate and differentiate nerve cells. Diseases that could be treated using this method include central and peripheral nervous system diseases, neuropathies, or mechanical and traumatic disorders (e.g., spinal cord disorders, head trauma, cerebrovascular disease, and stoke). Specifically, diseases associated with peripheral nerve injuries, peripheral neuropathy (e.g., resulting from chemotherapy or other medical therapies), localized neuropathies, and central nervous system diseases (e.g., Alzheimer's disease, Parkinson's disease, Huntington's disease, amyotrophic lateral sclerosis, and Shy-Drager syndrome), could all be treated using the polynucleotide or polypeptide of the present invention.

### Chemotaxis

A polynucleotide or polypeptide of the present invention may have chemotaxis activity. A chemotaxic molecule attracts or mobilizes cells (e.g., monocytes, fibroblasts, neutrophils, T-cells, mast cells, eosinophils, epithelial and/or endothelial cells) to a particular site in the body, such as inflammation, infection, or site of hyperproliferation. The mobilized cells can then fight off and/or heal the particular trauma or abnormality.

A polynucleotide or polypeptide of the present invention may increase chemotaxic activity of particular cells. These chemotactic molecules can then be used to treat inflammation, infection, hyperproliferative disorders, or any immune system disorder by increasing the number of cells targeted to a particular location in the body. For example, chemotaxic molecules can be used to treat wounds and other trauma to tissues by attracting immune cells to the injured location. Chemotactic molecules of the present invention can also attract fibroblasts, which can be used to treat wounds.

It is also contemplated that a polynucleotide or polypeptide of the present invention may inhibit chemotactic activity. These molecules could also be used to treat disorders. Thus, a polynucleotide or polypeptide of the present invention could be used as an inhibitor of chemotaxis.

### Binding Activity

A polypeptide of the present invention may be used to screen for molecules that bind to the polypeptide or for molecules to which the polypeptide binds. The binding of the polypeptide and the molecule may activate (agonist), increase, inhibit (antagonist), or decrease activity of the polypeptide or the molecule bound. Examples of such molecules include antibodies, oligonucleotides, proteins (e.g., receptors),or small molecules.

Preferably, the molecule is closely related to the natural ligand of the polypeptide, e.g., a fragment of the ligand, or a natural substrate, a ligand, a structural or functional mimetic. (See, Coligan et al., Current Protocols in Immunology 1(2):Chapter 5 (1991).) Similarly, the molecule can be closely related to the natural receptor to which the polypeptide binds, or at least, a fragment of the receptor capable of being bound by the polypeptide (e.g., active site). In either case, the molecule can be rationally designed using known techniques.

Preferably, the screening for these molecules involves producing appropriate cells which express the polypeptide, either as a secreted protein or on the cell membrane. Preferred cells include cells from mammals, yeast, Drosophila, or *E. coli.* Cells expressing the polypeptide (or cell membrane containing the expressed polypeptide) are then preferably contacted with a test compound potentially containing the molecule to observe binding, stimulation, or inhibition of activity of either the polypeptide or the molecule.

The assay may simply test binding of a candidate compound to the polypeptide, wherein binding is detected by a label, or in an assay involving competition with a labeled competitor. Further, the assay may test whether the candidate compound results in a signal generated by binding to the polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing a polypeptide, measuring polypeptide/molecule activity or binding, and comparing the polypeptide/molecule activity or binding to a standard.

Preferably, an ELISA assay can measure polypeptide level or activity in a sample (e.g., biological sample) using a monoclonal or polyclonal antibody. The antibody can measure polypeptide level or activity by either binding, directly or indirectly, to the polypeptide or by competing with the polypeptide for a substrate.

All of these above assays can be used as diagnostic or prognostic markers. The molecules discovered using these assays can be used to treat disease or to bring about a particular result in a patient (e.g., blood vessel growth) by activating or inhibiting the polypeptide/molecule. Moreover, the assays can discover agents which may inhibit or enhance the production of the polypeptide from suitably manipulated cells or tissues.

Therefore, the invention includes a method of identifying compounds which bind to a polypeptide of the invention comprising the steps of: (a) incubating a candidate binding compound with a polypeptide of the invention; and (b) determining if binding has occurred. Moreover, the invention includes a method of identifying agonists/antagonists comprising the steps of: (a) incubating a candidate compound with a polypeptide of the invention, (b) assaying a biological activity , and (b) determining if a biological activity of the polypeptide has been altered.

### Other Activities

A polypeptide or polynucleotide of the present invention may also increase or decrease the differentiation or proliferation of embryonic stem cells, besides, as discussed above, hematopoietic lineage.

A polypeptide or polynucleotide of the present invention may also be used to modulate mammalian characteristics, such as body height, weight, hair color, eye color, skin, percentage of adipose tissue, pigmentation, size, and shape (e.g., cosmetic surgery). Similarly, a polypeptide or polynucleotide of the present invention may be used to modulate mammalian metabolism affecting catabolism, anabolism, processing, utilization, and storage of energy.

A polypeptide or polynucleotide of the present invention may be used to change a mammal's mental state or physical state by influencing biorhythms, caricadic rhythms, depression (including depressive disorders), tendency for violence, tolerance for pain, reproductive capabilities (preferably by Activin or Inhibin-like activity), hormonal or endocrine levels, appetite, libido, memory, stress, or other cognitive qualities.

A polypeptide or polynucleotide of the present invention may also be used as a food additive or preservative, such as to increase or decrease storage capabilities, fat content, lipid, protein, carbohydrate, vitamins, minerals, cofactors or other nutritional components.

### Other Preferred Embodiments

Other preferred embodiments of the claimed invention include an isolated nucleic acid molecule comprising a nucleotide sequence which is at least 95% identical to a sequence of at least about 50 contiguous nucleotides in the nucleotide sequence of SEQ ID NO:X wherein X is any integer as defined in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Clone Sequence and ending with the nucleotide at about the position of the 3' Nucleotide of the Clone Sequence as defined for SEQ ID NO:X in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Start Codon and ending with the nucleotide at about the position of the 3' Nucleotide of the Clone Sequence as defined for SEQ ID NO:X in Table 1.

Similarly preferred is a nucleic acid molecule wherein said sequence of contiguous nucleotides is included in the nucleotide sequence of SEQ ID NO:X in the range of positions beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

Also preferred is an isolated nucleic acid molecule comprising a nucleotide sequence which is at least 95% identical to a sequence of at least about 150 contiguous nucleotides in the nucleotide sequence of SEQ ID NO:X.

Further preferred is an isolated nucleic acid molecule comprising a nucleotide sequence which is at least 95% identical to a sequence of at least about 500 contiguous nucleotides in the nucleotide sequence of SEQ ID NO:X.

A further preferred embodiment is a nucleic acid molecule comprising a nucleotide sequence which is at least 95% identical to the nucleotide sequence of SEQ ID NO:X beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

A further preferred embodiment is an isolated nucleic acid molecule comprising a nucleotide sequence which is at least 95% identical to the complete nucleotide sequence of SEQ ID NO:X.

Also preferred is an isolated nucleic acid molecule which hybridizes under stringent hybridization conditions to a nucleic acid molecule, wherein said nucleic acid molecule which hybridizes does not hybridize under stringent hybridization conditions to a nucleic acid molecule having a nucleotide sequence consisting of only A residues or of only T residues.

Also preferred is a composition of matter comprising a DNA molecule which comprises a human cDNA clone identified by a cDNA Clone Identifier in Table 1, which DNA molecule is contained in the material deposited with the American Type Culture Collection and given the ATCC Deposit Number shown in Table 1 for said cDNA Clone Identifier.

Also preferred is an isolated nucleic acid molecule comprising a nucleotide sequence which is at least 95% identical to a sequence of at least 50 contiguous nucleotides in the nucleotide sequence of a human cDNA clone identified by a cDNA Clone Identifier in Table 1, which DNA molecule is contained in the deposit given the ATCC Deposit Number shown in Table 1.

Also preferred is an isolated nucleic acid molecule, wherein said sequence of at least 50 contiguous nucleotides is included in the nucleotide sequence of the complete open reading frame sequence encoded by said human cDNA clone.

Also preferred is an isolated nucleic acid molecule comprising a nucleotide sequence which is at least 95% identical to sequence of at least 150 contiguous nucleotides in the nucleotide sequence encoded by said human cDNA clone.

A further preferred embodiment is an isolated nucleic acid molecule comprising a nucleotide sequence which is at least 95% identical to sequence of at least 500 contiguous nucleotides in the nucleotide sequence encoded by said human cDNA clone.

A further preferred embodiment is an isolated nucleic acid molecule comprising a nucleotide sequence which is at least 95% identical to the complete nucleotide sequence encoded by said human cDNA clone.

A further preferred embodiment is a method for detecting in a biological sample a nucleic acid molecule comprising a nucleotide sequence which is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1; which method comprises a step of comparing a nucleotide sequence of at least one nucleic acid molecule in said sample with a sequence selected from said group and determining whether the sequence of said nucleic acid molecule in said sample is at least 95% identical to said selected sequence.

Also preferred is the above method wherein said step of comparing sequences comprises determining the extent of nucleic acid hybridization between nucleic acid molecules in said sample and a nucleic acid molecule comprising said sequence selected from said group. Similarly, also preferred is the above method wherein said step of comparing sequences is performed by comparing the nucleotide sequence determined from a nucleic acid molecule in said sample with said sequence selected from said group. The nucleic acid molecules can comprise DNA molecules or RNA molecules.

A further preferred embodiment is a method for identifying the species, tissue or cell type of a biological sample which method comprises a step of detecting nucleic acid molecules in said sample, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for identifying the species, tissue or cell type of a biological sample can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a method for diagnosing in a subject a pathological condition associated with abnormal structure or expression of a gene encoding a secreted protein identified in Table 1, which method comprises a step of detecting in a biological sample obtained from said subject nucleic acid molecules, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for diagnosing a pathological condition can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a composition of matter comprising isolated nucleic acid molecules wherein the nucleotide sequences of said nucleic acid molecules comprise a panel of at least two nucleotide sequences, wherein at least one sequence in said panel is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1. The nucleic acid molecules can comprise DNA molecules or RNA molecules.

Also preferred is an isolated polypeptide comprising an amino acid sequence at least 90% identical to a sequence of at least about 10 contiguous amino acids in the amino acid sequence of SEQ ID NO:Y wherein Y is any integer as defined in Table 1.

Also preferred is a polypeptide, wherein said sequence of contiguous amino acids is included in the amino acid sequence of SEQ ID NO:Y in the range of positions beginning with the residue at about the position of the First Amino Acid of the Secreted Portion and ending with the residue at about the Last Amino Acid of the Open Reading Frame as set forth for SEQ ID NO:Y in Table 1.

Also preferred is an isolated polypeptide comprising an amino acid sequence at least 95% identical to a sequence of at least about 30 contiguous amino acids in the amino acid sequence of SEQ ID NO:Y.

Further preferred is an isolated polypeptide comprising an amino acid sequence at least 95% identical to a sequence of at least about 100 contiguous amino acids in the amino acid sequence of SEQ ID NO:Y.

Further preferred is an isolated polypeptide comprising an amino acid sequence at least 95% identical to the complete amino acid sequence of SEQ ID NO:Y.

Further preferred is an isolated polypeptide comprising an amino acid sequence at least 90% identical to a sequence of at least about 10 contiguous amino acids in the complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is a polypeptide wherein said sequence of contiguous amino acids is included in the amino acid sequence of a secreted portion of the secreted protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 30 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 100 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Further preferred is an isolated antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Further preferred is a method for detecting in a biological sample a polypeptide comprising an amino acid sequence which is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1; which method comprises a step of comparing an amino acid sequence of at least one polypeptide molecule in said sample with a sequence selected from said group and determining whether the sequence of said polypeptide molecule in said sample is at least 90% identical to said sequence of at least 10 contiguous amino acids.

Also preferred is the above method wherein said step of comparing an amino acid sequence of at least one polypeptide molecule in said sample with a sequence selected from said group comprises determining the extent of specific binding of polypeptides in said sample to an antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Also preferred is the above method wherein said step of comparing sequences is performed by comparing the amino acid sequence determined from a polypeptide molecule in said sample with said sequence selected from said group.

Also preferred is a method for identifying the species, tissue or cell type of a biological sample which method comprises a step of detecting polypeptide molecules in said sample, if any, comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is the above method for identifying the species, tissue or cell type of a biological sample, which method comprises a step of detecting polypeptide molecules comprising an amino acid sequence in a panel of at least two amino acid sequences, wherein at least one sequence in said panel is at least 90% identical to a sequence of at least 10 contiguous amino acids in a sequence selected from the above group.

Also preferred is a method for diagnosing in a subject a pathological condition associated with abnormal structure or expression of a gene encoding a secreted protein identified in Table 1, which method comprises a step of detecting in a biological sample obtained from said subject polypeptide molecules comprising an amino acid sequence in a panel of at least two amino acid sequences, wherein at least one sequence in said panel is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

In any of these methods, the step of detecting said polypeptide molecules includes using an antibody.

Also preferred is an isolated nucleic acid molecule comprising a nucleotide sequence which is at least 95% identical to a nucleotide sequence encoding a polypeptide wherein said polypeptide comprises an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is an isolated nucleic acid molecule, wherein said nucleotide sequence encoding a polypeptide has been optimized for expression of said polypeptide in a prokaryotic host.

Also preferred is an isolated nucleic acid molecule, wherein said polypeptide comprises an amino acid sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Further preferred is a method of making a recombinant vector comprising inserting any of the above isolated nucleic acid molecule into a vector. Also preferred is the recombinant vector produced by this method. Also preferred is a method of making a recombinant host cell comprising introducing the vector into a host cell, as well as the recombinant host cell produced by this method.

Also preferred is a method of making an isolated polypeptide comprising culturing this recombinant host cell under conditions such that said polypeptide is expressed and recovering said polypeptide. Also preferred is this method of making an isolated polypeptide, wherein said recombinant host cell is a eukaryotic cell and said polypeptide is a secreted portion of a human secreted protein comprising an amino acid sequence selected from the group consisting of: an amino acid sequence of SEQ ID NO:Y beginning with the residue at the position of the First Amino Acid of the Secreted Portion of SEQ ID NO:Y wherein Y is an integer set forth in Table 1 and said position of the First Amino Acid of the Secreted Portion of SEQ ID NO:Y is defined in Table 1; and an amino acid sequence of a secreted portion of a protein encoded by a human cDNA clone identified by a cDNA Clone Identifier in Table 1 and contained in the deposit with the ATCC Deposit Number shown for said cDNA clone in Table 1. The isolated polypeptide produced by this method is also preferred.

Also preferred is a method of treatment of an individual in need of an increased level of a secreted protein activity, which method comprises administering to such an individual a pharmaceutical composition comprising an amount of an isolated polypeptide, polynucleotide, or antibody of the claimed invention effective to increase the level of said protein activity in said individual.

Having generally described the invention, the same will be more readily understood by reference to the following examples, which are provided by way of illustration and are not intended as limiting.

### Examples

### Example 1: Isolation of a Selected cDNA Clone From the Deposited Sample

Each cDNA clone in a cited ATCC deposit is contained in a plasmid vector. Table I identifies the vectors used to construct the cDNA library from which each clone was isolated. In many cases, the vector used to construct the library is a phage vector from which a plasmid has been excised. The table immediately below correlates the related plasmid for each phage vector used in constructing the cDNA library. For example, where a particular clone is identified in Table 1 as being isolated in the vector "Lambda Zap," the corresponding deposited clone is in "pBluescript."

| Vector Used to Construct Library | Corresponding Deposited Plasmid |
|---|---|
| Lambda Zap | pBluescript (pBS) |
| Uni-Zap XR | pBluescript (pBS) |
| Zap Express | pBK |
| lafmid BA | plafmid BA |
| pSport1 | pSport1 |
| pCMVSport 2.0 | pCMVSport 2.0 |
| pCMVSport 3.0 | pCMVSport 3.0 |
| pCR®2.1 | pCR®2.1 |

Vectors Lambda Zap (U.S. Patent Nos. 5,128,256 and 5,286,636), Uni-Zap XR (U.S. Patent Nos. 5,128, 256 and 5,286,636), Zap Express (U.S. Patent Nos. 5,128,256 and 5,286,636), pBluescript (pBS) (Short, J. M. et al., Nucleic Acids Res. 16:7583-7600 (1988); Alting-Mees, M. A. and Short, J. M., Nucleic Acids Res. 17:9494 (1989)) and pBK (Alting-Mees, M. A. et al., Strategies 5:58-61 (1992)) are commercially available from Stratagene Cloning Systems, Inc., 11011 N. Torrey Pines Road, La Jolla, CA, 92037. pBS contains an ampicillin resistance gene and pBK contains a neomycin resistance gene. Both can be transformed into E. coli strain XL-1 Blue, also available from Stratagene, pBS comes in 4 forms SK+, SK-, KS+ and KS. The S and K refers to the orientation of the polylinker to the T7 and T3 primer sequences which flank the polylinker region ("S" is for SacI and "K" is for KpnI which are the first sites on each respective end of the linker). "+" or "-" refer to the orientation of the f1 origin of replication ("ori"), such that in one orientation, single stranded rescue initiated from the f1 on generates sense strand DNA and in the other, antisense.

Vectors pSport1, pCMVSport 2.0 and pCMVSport 3.0, were obtained from Life Technologies, Inc., P. O. Box 6009, Gaithersburg, MD 20897. All Sport vectors contain an ampicillin resistance gene and may be transformed into E. coli strain DH10B, also available from Life Technologies. (See, for instance, Gruber, C. E., et al., Focus 15:59 (1993).) Vector lafmid BA (Bento Soares, Columbia University, NY) contains an ampicillin resistance gene and can be transformed into E. coli strain XL-1 Blue. Vector pCR®2.1, which is available from Invitrogen, 1600 Faraday Avenue, Carlsbad, CA 92008, contains an ampicillin resistance gene and may be transformed into E. coli strain DH10B, available from Life Technologies. (See, for instance, Clark, J. M., Nuc. Acids Res. 16:9677-9686 (1988) and Mead, D. et al., Bio/Technology 9: (1991).) Preferably, a polynucleotide of the present invention does not comprise the phage vector sequences identified for the particular clone in Table 1, as well as the corresponding plasmid vector sequences designated above.

The deposited material in the sample assigned the ATCC Deposit Number cited in Table 1 for any given cDNA clone also may contain one or more additional plasmids, each comprising a cDNA clone different from that given clone. Thus, deposits sharing the same ATCC Deposit Number contain at least a plasmid for each cDNA clone identified in Table 1. Typically, each ATCC deposit sample cited in Table 1 comprises a mixture of approximately equal amounts (by weight) of about 50 plasmid DNAs, each containing a different cDNA clone; but such a deposit sample may include plasmids for more or less than 50 cDNA clones, up to about 500 cDNA clones.

Two approaches can be used to isolate a particular clone from the deposited sample of plasmid DNAs cited for that clone in Table 1. First, a plasmid is directly isolated by screening the clones using a polynucleotide probe corresponding to SEQ ID NO:X.

Particularly, a specific polynucleotide with 30-40 nucleotides is synthesized using an Applied Biosystems DNA synthesizer according to the sequence reported. The oligonucleotide is labeled, for instance, with ³²P-γ-ATP using T4 polynucleotide kinase and purified according to routine methods. (E.g., Maniatis et al., Molecular Cloning: A Laboratory Manual, Cold Spring Harbor Press, Cold Spring, NY (1982).) The plasmid mixture is transformed into a suitable host, as indicated above (such as XL-1 Blue (Stratagene)) using techniques known to those of skill in the art, such as those provided by the vector supplier or in related publications or patents cited above. The transformants are plated on 1.5% agar plates (containing the appropriate selection agent, e.g., ampicillin) to a density of about 150 transformants (colonies) per plate. These plates are screened using Nylon membranes according to routine methods for bacterial colony screening (e.g., Sambrook et al., Molecular Cloning: A Laboratory Manual, 2nd Edit., (1989), Cold Spring Harbor Laboratory Press, pages 1.93 to 1.104), or other techniques known to those of skill in the art.

Alternatively, two primers of 17-20 nucleotides derived from both ends of the SEQ ID NO:X (i.e., within the region of SEQ ID NO:X bounded by the 5' NT and the 3' NT of the clone defined in Table 1) are synthesized and used to amplify the desired cDNA using the deposited cDNA plasmid as a template. The polymerase chain reaction is carried out under routine conditions, for instance, in 25 µl of reaction mixture with 0.5 ug of the above cDNA template. A convenient reaction mixture is 1.5-5 mM MgCl₂, 0.01% (w/v) gelatin, 20 µM each of dATP, dCTP, dGTP, dTTP, 25 pmol of each primer and 0.25 Unit of Taq polymerase. Thirty five cycles of PCR (denaturation at 94°C for 1 min; annealing at 55°C for 1 min; elongation at 72°C for 1 min) are performed with a Perkin-Elmer Cetus automated thermal cycler. The amplified product is analyzed by agarose gel electrophoresis and the DNA band with expected molecular weight is excised and purified. The PCR product is verified to be the selected sequence by subcloning and sequencing the DNA product.

Several methods are available for the identification of the 5' or 3' non-coding portions of a gene which may not be present in the deposited clone. These methods include but are not limited to, filter probing, clone enrichment using specific probes, and protocols similar or identical to 5' and 3' "RACE" protocols which are well known in the art. For instance, a method similar to 5' RACE is available for generating the missing 5' end of a desired full-length transcript. (Fromont-Racine et al., Nucleic Acids Res. 21(7):1683-1684 (1993).)

Briefly, a specific RNA oligonucleotide is ligated to the 5' ends of a population of RNA presumably containing full-length gene RNA transcripts. A primer set containing a primer specific to the ligated RNA oligonucleotide and a primer specific to a known sequence of the gene of interest is used to PCR amplify the 5' portion of the desired full-length gene. This amplified product may then be sequenced and used to generate the full length gene.

This above method starts with total RNA isolated from the desired source, although poly-A+ RNA can be used. The RNA preparation can then be treated with phosphatase if necessary to eliminate 5' phosphate groups on degraded or damaged RNA which may interfere with the later RNA ligase step. The phosphatase should then be inactivated and the RNA treated with tobacco acid pyrophosphatase in order to remove the cap structure present at the 5' ends of messenger RNAs. This reaction leaves a 5' phosphate group at the 5' end of the cap cleaved RNA which can then be ligated to an RNA oligonucleotide using T4 RNA ligase.

This modified RNA preparation is used as a template for first strand cDNA synthesis using a gene specific oligonucleotide. The first strand synthesis reaction is used as a template for PCR amplification of the desired 5' end using a primer specific to the ligated RNA oligonucleotide and a primer specific to the known sequence of the gene of interest. The resultant product is then sequenced and analyzed to confirm that the 5' end sequence belongs to the desired gene.

### Example 2: Isolation of Genomic Clones Corresponding to a Polynucleotide

A human genomic P1 library (Genomic Systems, Inc.) is screened by PCR using primers selected for the cDNA sequence corresponding to SEQ ID NO:X., according to the method described in Example 1. (See also, Sambrook.)

### Example 3: Tissue Distribution of Polypeptide

Tissue distribution of mRNA expression of polynucleotides of the present invention is determined using protocols for Northern blot analysis, described by, among others, Sambrook et al. For example, a cDNA probe produced by the method described in Example 1 is labeled with P³² using the rediprime™ DNA labeling system (Amersham Life Science), according to manufacturer's instructions. After labeling, the probe is purified using CHROMA SPIN-100™ column (Clontech Laboratories, Inc.), according to manufacturer's protocol number PT1200-1. The purified labeled probe is then used to examine various human tissues for mRNA expression.

Multiple Tissue Northern (MTN) blots containing various human tissues (H) or human immune system tissues (IM) (Clontech) are examined with the labeled probe using ExpressHyb™ hybridization solution (Clontech) according to manufacturer's protocol number PT1190-1. Following hybridization and washing, the blots are mounted and exposed to film at -70°C overnight, and the films developed according to standard procedures.

### Example 4: Chromosomal Mapping of the Polynucleotides

An oligonucleotide primer set is designed according to the sequence at the 5' end of SEQ ID NO:X. This primer preferably spans about 100 nucleotides. This primer set is then used in a polymerase chain reaction under the following set of conditions : 30 seconds, 95°C; 1 minute, 56°C; 1 minute, 70°C. This cycle is repeated 32 times followed by one 5 minute cycle at 70°C. Human, mouse, and hamster DNA is used as template in addition to a somatic cell hybrid panel containing individual chromosomes or chromosome fragments (Bios, Inc). The reactions is analyzed on either 8% polyacrylamide gels or 3.5 % agarose gels. Chromosome mapping is determined by the presence of an approximately 100 bp PCR fragment in the particular somatic cell hybrid.

### Example 5: Bacterial Expression of a Polypeptide

A polynucleotide encoding a polypeptide of the present invention is amplified using PCR oligonucleotide primers corresponding to the 5' and 3' ends of the DNA sequence, as outlined in Example 1, to synthesize insertion fragments. The primers used to amplify the cDNA insert should preferably contain restriction sites, such as BamHI and XbaI, at the 5' end of the primers in order to clone the amplified product into the expression vector. For example, BamHI and XbaI correspond to the restriction enzyme sites on the bacterial expression vector pQE-9. (Qiagen, Inc., Chatsworth, CA). This plasmid vector encodes antibiotic resistance (Amp^{r}), a bacterial origin of replication (ori), an IPTG-regulatable promoter/operator (P/O), a ribosome binding site (RBS), a 6-histidine tag (6-His), and restriction enzyme cloning sites.

The pQE-9 vector is digested with BamHI and XbaI and the amplified fragment is ligated into the pQE-9 vector maintaining the reading frame initiated at the bacterial RBS. The ligation mixture is then used to transform the E. coli strain M15/rep4 (Qiagen, Inc.) which contains multiple copies of the plasmid pREP4, which expresses the lacI repressor and also confers kanamycin resistance (Kan^{r}). Transformants are identified by their ability to grow on LB plates and ampicillin/kanamycin resistant colonies are selected. Plasmid DNA is isolated and confirmed by restriction analysis.

Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG (Isopropyl-B-D-thiogalacto pyranoside) is then added to a final concentration of 1 mM. IPTG induces by inactivating the lacI repressor, clearing the P/O leading to increased gene expression.

Cells are grown for an extra 3 to 4 hours. Cells are then harvested by centrifugation (20 mins at 6000Xg). The cell pellet is solubilized in the chaotropic agent 6 Molar Guanidine HCl by stirring for 3-4 hours at 4°C. The cell debris is removed by centrifugation, and the supernatant containing the polypeptide is loaded onto a nickel-nitrilo-tri-acetic acid ("Ni-NTA") affinity resin column (available from QIAGEN, Inc., *supra*). Proteins with a 6 x His tag bind to the Ni-NTA resin with high affinity and can be purified in a simple one-step procedure (for details see: The QIAexpressionist (1995) QIAGEN, Inc., *supra*).

Briefly, the supernatant is loaded onto the column in 6 M guanidine-HCl, pH 8, the column is first washed with 10 volumes of 6 M guanidine-HCl, pH 8, then washed with 10 volumes of 6 M guanidine-HCl pH 6, and finally the polypeptide is eluted with 6 M guanidine-HCl, pH 5.

The purified protein is then renatured by dialyzing it against phosphate-buffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol, 20 mM Tris/HCl pH 7.4, containing protease inhibitors. The renaturation should be performed over a period of 1.5 hours or more. After renaturation the proteins are eluted by the addition of 250 mM immidazole. Immidazole is removed by a final dialyzing step against PBS or 50 mM sodium acetate pH 6 buffer plus 200 mM NaCl. The purified protein is stored at 4°C or frozen at -80° C.

In addition to the above expression vector, the present invention further includes an expression vector comprising phage operator and promoter elements operatively linked to a polynucleotide of the present invention, called pHE4a. (ATCC Accession Number XXXXXX.) This vector contains: 1) a neomycinphosphotransferase gene as a selection marker, 2) an E. coli origin of replication, 3) a T5 phage promoter sequence, 4) two lac operator sequences, 5) a Shine-Delgarno sequence, and 6) the lactose operon repressor gene (lacIq). The origin of replication (oriC) is derived from pUC19 (LTI, Gaithersburg, MD). The promoter sequence and operator sequences are made synthetically.

DNA can be inserted into the pHEa by restricting the vector with NdeI and XbaI, BamHI, XhoI, or Asp718, running the restricted product on a gel, and isolating the larger fragment (the stuffer fragment should be about 310 base pairs). The DNA insert is generated according to the PCR protocol described in Example 1, using PCR primers having restriction sites for NdeI (5' primer) and XbaI, BamHI, XhoI, or Asp718 (3' primer). The PCR insert is gel purified and restricted with compatible enzymes. The insert and vector are ligated according to standard protocols.

The engineered vector could easily be substituted in the above protocol to express protein in a bacterial system.

### Example 6: Purification of a Polypeptide from an Inclusion Body

The following alternative method can be used to purify a polypeptide expressed in *E coli* when it is present in the form of inclusion bodies. Unless otherwise specified, all of the following steps are conducted at 4-10°C.

Upon completion of the production phase of the *E. coli* fermentation, the cell culture is cooled to 4-10°C and the cells harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells are then lysed by passing the solution through a microfluidizer (Microfuidics, Corp. or APV Gaulin, Inc.) twice at 4000-6000 psi. The homogenate is then mixed with NaCl solution to a final concentration of 0.5 M NaCI, followed by centrifugation at 7000 xg for 15 min. The resultant pellet is washed again using 0.5M NaCI, 100 mM Tris, 50 mM EDTA, pH 7.4.

The resulting washed inclusion bodies are solubilized with 1.5 M guanidine hydrochloride (GuHCl) for 2-4 hours. After 7000 xg centrifugation for 15 min., the pellet is discarded and the polypeptide containing supernatant is incubated at 4°C overnight to allow further GuHCl extraction.

Following high speed centrifugation (30,000 xg) to remove insoluble particles, the GuHCl solubilized protein is refolded by quickly mixing the GuHCl extract with 20 volumes of buffer containing 50 mM sodium, pH 4.5, 150 mM NaCl, 2 mM EDTA by vigorous stirring. The refolded diluted protein solution is kept at 4°C without mixing for 12 hours prior to further purification steps.

To clarify the refolded polypeptide solution, a previously prepared tangential filtration unit equipped with 0.16 µm membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM, and 1500 mM NaCl in the same buffer, in a stepwise manner. The absorbance at 280 nm of the effluent is continuously monitored. Fractions are collected and further analyzed by SDS-PAGE.

Fractions containing the polypeptide are then pooled and mixed with 4 volumes of water. The diluted sample is then loaded onto a previously prepared set of tandem columns of strong anion (Poros HQ-50, Perseptive Biosystems) and weak anion (Poros CM-20, Perseptive Biosystems) exchange resins. The columns are equilibrated with 40 mM sodium acetate, pH 6.0. Both columns are washed with 40 mM sodium acetate, pH 6.0, 200 mM NaCl. The CM-20 column is then eluted using a 10 column volume linear gradient ranging from 0.2 M NaCl, 50 mM sodium acetate, pH 6.0 to 1.0 M NaCl, 50 mM sodium acetate, pH 6.5. Fractions are collected under constant A₂₈₀ monitoring of the effluent. Fractions containing the polypeptide (determined, for instance, by 16% SDS-PAGE) are then pooled.

The resultant polypeptide should exhibit greater than 95% purity after the above refolding and purification steps. No major contaminant bands should be observed from Commassie blue stained 16% SDS-PAGE gel when 5 µg of purified protein is loaded. The purified protein can also be tested for endotoxin/LPS contamination, and typically the LPS content is less than 0.1 ng/ml according to LAL assays.

### Example 7: Cloning and Expression of a Polypeptide in a Baculovirus Expression System

In this example, the plasmid shuttle vector pA2 is used to insert a polynucleotide into a baculovirus to express a polypeptide. This expression vector contains the strong polyhedrin promoter of the *Autographa californica* nuclear polyhedrosis virus (AcMNPV) followed by convenient restriction sites such as BamHI, Xba I and Asp718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate a viable virus that express the cloned polynucleotide.

Many other baculovirus vectors can be used in place of the vector above, such as pAc373, pVL941, and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow et al., Virology 170:31-39 (1989).

Specifically, the cDNA sequence contained in the deposited clone, including the AUG initiation codon and the naturally associated leader sequence identified in Table 1, is amplified using the PCR protocol described in Example 1. If the naturally occurring signal sequence is used to produce the secreted protein, the pA2 vector does not need a second signal peptide. Alternatively, the vector can be modified (pA2 GP) to include a baculovirus leader sequence, using the standard methods described in Summers et al., "A Manual of Methods for Baculovirus Vectors and Insect Cell Culture Procedures," Texas Agricultural Experimental Station Bulletin No. 1555 (1987).

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The plasmid is digested with the corresponding restriction enzymes and optionally, can be dephosphorylated using calf intestinal phosphatase, using routine procedures known in the art. The DNA is then isolated from a 1 % agarose gel using a commercially available kit ("Geneclean" BIO 101 Inc., La Jolla, Ca.).

The fragment and the dephosphorylated plasmid are ligated together with T4 DNA ligase. *E. coli* HB101 or other suitable *E. coli* hosts such as XL-1 Blue (Stratagene Cloning Systems, La Jolla, CA) cells are transformed with the ligation mixture and spread on culture plates. Bacteria containing the plasmid are identified by digesting DNA from individual colonies and analyzing the digestion product by gel electrophoresis. The sequence of the cloned fragment is confirmed by DNA sequencing.

Five µg of a plasmid containing the polynucleotide is co-transfected with 1.0 µg of a commercially available linearized baculovirus DNA ("BaculoGold™ baculovirus DNA", Pharmingen, San Diego, CA), using the lipofection method described by Felgner et al., Proc. Natl. Acad. Sci. USA 84:7413-7417 (1987). One µg of BaculoGold™ virus DNA and 5 µg of the plasmid are mixed in a sterile well of a microtiter plate containing 50 µl of serum-free Grace's medium (Life Technologies Inc., Gaithersburg, MD). Afterwards, 10 µl Lipofectin plus 90 µl Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27° C. The transfection solution is then removed from the plate and 1 ml of Grace's insect medium supplemented with 10% fetal calf serum is added. Cultivation is then continued at 27° C for four days.

After four days the supernatant is collected and a plaque assay is performed, as described by Summers and Smith, *supra.* An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10.) After appropriate incubation, blue stained plaques are picked with the tip of a micropipettor (e.g., Eppendorf). The agar containing the recombinant viruses is then resuspended in a microcentrifuge tube containing 200 µl of Grace's medium and the suspension containing the recombinant baculovirus is used to infect Sf9 cells seeded in 35 mm dishes. Four days later the supernatants of these culture dishes are harvested and then they are stored at 4° C.

To verify the expression of the polypeptide, Sf9 cells are grown in Grace's medium supplemented with 10% heat-inactivated FBS. The cells are infected with the recombinant baculovirus containing the polynucleotide at a multiplicity of infection ("MOI") of about 2. If radiolabeled proteins are desired, 6 hours later the medium is removed and is replaced with SF900 II medium minus methionine and cysteine (available from Life Technologies Inc., Rockville, MD). After 42 hours, 5 µCi of ³⁵S-methionine and 5 µCi ³⁵S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the produced protein.

### Example 8: Expression of a Polypeptide in Mammalian Cells

The polypeptide of the present invention can be expressed in a mammalian cell. A typical mammalian expression vector contains a promoter element, which mediates the initiation of transcription of mRNA, a protein coding sequence, and signals required for the termination of transcription and polyadenylation of the transcript. Additional elements include enhancers, Kozak sequences and intervening sequences flanked by donor and acceptor sites for RNA splicing. Highly efficient transcription is achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter).

Suitable expression vectors for use in practicing the present invention include, for example, vectors such as pSVL and pMSG (Pharmacia, Uppsala, Sweden), pRSVcat (ATCC 37152), pSV2dhfr (ATCC 37146), pBC12MI (ATCC 67109), pCMVSport 2.0, and pCMVSport 3.0. Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells.

Alternatively, the polypeptide can be expressed in stable cell lines containing the polynucleotide integrated into a chromosome. The co-transfection with a selectable marker such as dhfr, gpt, neomycin, hygromycin allows the identification and isolation of the transfected cells.

The transfected gene can also be amplified to express large amounts of the encoded protein. The DHFR (dihydrofolate reductase) marker is useful in developing cell lines that carry several hundred or even several thousand copies of the gene of interest. (See, e.g., Alt, F. W., et al., J. Biol. Chem. 253:1357-1370 (1978); Hamlin, J. L. and Ma, C., Biochem. et Biophys. Acta, 1097:107-143 (1990); Page, M. J. and Sydenham, M. A., Biotechnology 9:64-68 (1991).) Another useful selection marker is the enzyme glutamine synthase (GS) (Murphy et al., Biochem J. 227:277-279 (1991); Bebbington et al., Bio/Technology 10:169-175 (1992). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

Derivatives of the plasmid pSV2-dhfr (ATCC Accession No. 37146), the expression vectors pC4 (ATCC Accession No. 209646) and pC6 (ATCC Accession No. 209647) contain the strong promoter (LTR) of the Rous Sarcoma Virus (Cullen et al., Molecular and Cellular Biology, 438-447 (March, 1985)) plus a fragment of the CMV-enhancer (Boshart et al., Cell 41:521-530 (1985).) Multiple cloning sites, e.g., with the restriction enzyme cleavage sites BamHI, XbaI and Asp718, facilitate the cloning of the gene of interest. The vectors also contain the 3' intron, the polyadenylation and termination signal of the rat preproinsulin gene, and the mouse DHFR gene under control of the SV40 early promoter.

Specifically, the plasmid pC6, for example, is digested with appropriate restriction enzymes and then dephosphorylated using calf intestinal phosphates by procedures known in the art. The vector is then isolated from a 1% agarose gel.

A polynucleotide of the present invention is amplified according to the protocol outlined in Example 1. If the naturally occurring signal sequence is used to produce the secreted protein, the vector does not need a second signal peptide. Alternatively, if the naturally occurring signal sequence is not used, the vector can be modified to include a heterologous signal sequence. (See, e.g., WO 96/34891.)

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. *E. coli* HB101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transfection. Five µg of the expression plasmid pC6 is cotransfected with 0.5 µg of the plasmid pSVneo using lipofectin (Felgner et al., *supra*). The plasmid pSV2-neo contains a dominant selectable marker, the *neo* gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 µM, 2 µM, 5 µM, 10 mM, 20 mM). The same procedure is repeated until clones are obtained which grow at a concentration of 100 - 200 µM. Expression of the desired gene product is analyzed, for instance, by SDS-PAGE and Western blot or by reversed phase HPLC analysis.

### Example 9: Protein Fusions

The polypeptides of the present invention are preferably fused to other proteins. These fusion proteins can be used for a variety of applications. For example, fusion of the present polypeptides to His-tag, HA-tag, protein A, IgG domains, and maltose binding protein facilitates purification. (See Example 5; see also EP A 394,827; Traunecker, et al., Nature 331:84-86 (1988).) Similarly, fusion to IgG-1, IgG-3, and albumin increases the halflife time in vivo. Nuclear localization signals fused to the polypeptides of the present invention can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made by modifying the following protocol, which outlines the fusion of a polypeptide to an IgG molecule, or the protocol described in Example 5.

Briefly, the human Fc portion of the IgG molecule can be PCR amplified, using primers that span the 5' and 3' ends of the sequence described below. These primers also should have convenient restriction enzyme sites that will facilitate cloning into an expression vector, preferably a mammalian expression vector.

For example, if pC4 (Accession No.209646) is used, the human Fc portion can be ligated into the BamHI cloning site. Note that the 3' BamHI site should be destroyed. Next, the vector containing the human Fc portion is re-restricted with BamHI, linearizing the vector, and a polynucleotide of the present invention, isolated by the PCR protocol described in Example 1, is ligated into this BamHI site. Note that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence. (See, e.g., WO 96/34891.)

### Human IgG Fc region:

GGGATCCGGAGCCCAAATCTTCTGACAAAACTCACACATGCCCACCGTGCC
CAGCACCTGAATTCGAGGGTGCACCGTCAGTCTTCCTCTTCCCCCCAAAACC
CAAGGACACCCTCATGATCTCCCGGACTCCTGAGGTCACATGCGTGGTGGT
GGACGTAAGCCACGAAGACCCTGAGGTCAAGTTCAACTGGTACGTGGACG
GCGTGGAGGTGCATAATGCCAAGACAAAGCCGCGGGAGGAGCAGTACAAC
AGCACGTACCGTGTGGTCAGCGTCCTCACCGTCCTGCACCAGGACTGGCTG
AATGGCAAGGAGTACAAGTGCAAGGTCTCCAACAAAGCCCTCCCAACCCCC
ATCGAGAAAACCATCTCCAAAGCCAAAGGGCAGCCCCGAGAACCACAGGT
GTACACCCTGCCCCCATCCCGGGATGAGCTGACCAAGAACCAGGTCAGCCT
GACCTGCCTGGTCAAAGGCTTCTATCCAAGCGACATCGCCGTGGAGTGGGA
GAGCAATGGGCAGCCGGAGAACAACTACAAGACCACGCCTCCCGTGCTGG
ACTCCGACGGCTCCTTCTTCCTCTACAGCAAGCTCACCGTGGACAAGAGCA
GGTGGCAGCAGGGGAACGTCTTCTCATGCTCCGTGATGCATGAGGCTCTGC
ACAACCACTACACGCAGAAGAGCCTCTCCCTGTCTCCGGGTAAATGAGTGC
GACGGCCGCGACTCTAGAGGAT (SEQ ID NO:1)

### Example 10: Production of an Antibody from a Polypeptide

The antibodies of the present invention can be prepared by a variety of methods. (See, Current Protocols, Chapter 2.) For example, cells expressing a polypeptide of the present invention is administered to an animal to induce the production of sera containing polyclonal antibodies. In a preferred method, a preparation of the secreted protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or protein binding fragments thereof). Such monoclonal antibodies can be prepared using hybridoma technology. (Köhler et al., Nature 256:495 (1975); Köhler et al., Eur. J. Immunol. 6:511 (1976); Köhler et al., Eur. J. Immunol. 6:292 (1976); Hammerling et al., in: Monoclonal Antibodies and T-Cell Hybridomas, Elsevier, N.Y., pp. 563-681 (1981).) In general, such procedures involve immunizing an animal (preferably a mouse) with polypeptide or, more preferably, with a secreted polypeptide-expressing cell. Such cells may be cultured in any suitable tissue culture medium; however, it is preferable to culture cells in Earle's modified Eagle's medium supplemented with 10% fetal bovine serum (inactivated at about 56°C), and supplemented with about 10 g/l of nonessential amino acids, about 1,000 U/ml of penicillin, and about 100 µg/ml of streptomycin.

The splenocytes of such mice are extracted and fused with a suitable myeloma cell line. Any suitable myeloma cell line may be employed in accordance with the present invention; however, it is preferable to employ the parent myeloma cell line (SP20), available from the ATCC. After fusion, the resulting hybridoma cells are selectively maintained in HAT medium, and then cloned by limiting dilution as described by Wands et al. (Gastroenterology 80:225-232 (1981).) The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the protein-specific antibody can be blocked by the polypeptide. Such antibodies comprise anti-idiotypic antibodies to the protein-specific antibody and can be used to immunize an animal to induce formation of further protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, secreted protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. (See, for review, Morrison, Science 229:1202 (1985); Oi et al., BioTechniques 4:214 (1986); Cabilly et al., U.S. Patent No. 4,816,567; Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 (1985).)

### Example 11: Production Of Secreted Protein For High-Throughput Screening Assays

The following protocol produces a supernatant containing a polypeptide to be tested. This supernatant can then be used in the Screening Assays described in Examples 13-20.

First, dilute Poly-D-Lysine (644 587 Boehringer-Mannheim) stock solution (1mg/ml in PBS) 1:20 in PBS (w/o calcium or magnesium 17-516F Biowhittaker) for a working solution of 50ug/ml. Add 200 ul of this solution to each well (24 well plates) and incubate at RT for 20 minutes. Be sure to distribute the solution over each well (note: a 12-channel pipetter may be used with tips on every other channel). Aspirate off the Poly-D-Lysine solution and rinse with 1ml PBS (Phosphate Buffered Saline). The PBS should remain in the well until just prior to plating the cells and plates may be poly-lysine coated in advance for up to two weeks.

Plate 293T cells (do not carry cells past P+20) at 2 x 10⁵ cells/well in .5ml DMEM(Dulbecco's Modified Eagle Medium)(with 4.5 G/L glucose and L-glutamine (12-604F Biowhittaker))/10% heat inactivated FBS(14-503F Biowhittaker)/1x Penstrep(17-602E Biowhittaker). Let the cells grow overnight.

The next day, mix together in a sterile solution basin: 300 ul Lipofectamine (18324-012 Gibco/BRL) and 5ml Optimem I (31985070 Gibco/BRL)/96-well plate. With a small volume multi-channel pipetter, aliquot approximately 2ug of an expression vector containing a polynucleotide insert, produced by the methods described in Examples 8 or 9, into an appropriately labeled 96-well round bottom plate. With a multi-channel pipetter, add 50ul of the Lipofectamine/Optimem I mixture to each well. Pipette up and down gently to mix. Incubate at RT 15-45 minutes. After about 20 minutes, use a multi-channel pipetter to add 150ul Optimem I to each well. As a control, one plate of vector DNA lacking an insert should be transfected with each set of transfections.

Preferably, the transfection should be performed by tag-teaming the following tasks. By tag-teaming, hands on time is cut in half, and the cells do not spend too much time on PBS. First, person A aspirates off the media from four 24-well plates of cells, and then person B rinses each well with .5-1ml PBS. Person A then aspirates off PBS rinse, and person B, using a12-channel pipetter with tips on every other channel, adds the 200ul of DNA/Lipofectamine/Optimem I complex to the odd wells first, then to the even wells, to each row on the 24-well plates. Incubate at 37°C for 6 hours.

While cells are incubating, prepare appropriate media, either 1%BSA in DMEM with 1x penstrep, or CHO-5 media (see below) with 2mm glutamine and 1x penstrep. (BSA (81-068-3 Bayer) 100gm dissolved in 1L DMEM for a 10% BSA stock solution). Filter the media and collect 50 ul for endotoxin assay in 15ml polystyrene conical.

The transfection reaction is terminated, preferably by tag-teaming, at the end of the incubation period. Person A aspirates off the transfection media, while person B adds 1.5ml appropriate media to each well. Incubate at 37°C for 45 or 72 hours depending on the media used: 1%BSA for 45 hours or CHO-5 for 72 hours.

On day four, using a 300ul multichannel pipetter, aliquot 600ul in one 1ml deep well plate and the remaining supernatant into a 2ml deep well. The supernatants from each well can then be used in the assays described in Examples 13-20.

It is specifically understood that when activity is obtained in any of the assays described below using a supernatant, the activity originates from either the polypeptide directly (e.g., as a secreted protein) or by the polypeptide inducing expression of other proteins, which are then secreted into the supernatant. Thus, the invention further provides a method of identifying the protein in the supernatant characterized by an activity in a particular assay.

| ***HGS-CHO-5 medium formulation:*** **Inorganic Salts** | |
|---|---|
| CaCl2 (anhyd) | 116.6 mg/L |
| CuSO₄-5H₂O | 0.00130 |
| Fe(NO₃)₃-9H₂O | 0.050 |
| FeSO₄-7H₂O | 0.417 |
| KCl | 311.80 |
| MgCl₂ | 28.64 |
| MgSO₄ | 48.84 |
| NaCl | 6995.50 |
| NaHCO₃ | 2400.0 |
| NaH₂PO₄-H₂O | 62.50 |
| Na₂HPO4 | 71.02 |
| ZnSO₄-7H₂O | .4320 |

| **Lipids** | |
|---|---|
| Arachidonic Acid | .002 mg/L |
| Cholesterol | 1.022 |
| DL-alpha-Tocopherol-Acetate | .070 |
| Linoleic Acid | 0.0520 |
| Linolenic Acid | 0.010 |
| Myristic Acid | 0.010 |
| Oleic Acid | 0.010 |
| Palmitric Acid | 0.010 |
| Palmitic Acid | 0.010 |
| Pluronic F-68 | 100 |
| Stearic Acid | 0.010 |
| Tween 80 | 2.20 |

| **Carbon Source** | |
|---|---|
| D-Glucose | 4551 mg/L |

| **Amino Acids** | |
|---|---|
| L- Alanine | 130.85 mg/ml |
| L-Arginine-HCL | 147.50 |
| L-Asparagine-H₂O | 7.50 |
| L-Aspartic Acid | 6.65 |
| L-Cystine-2HCL-H₂O | 29.56 |
| L-Cystine-2HCL | 31.29 |
| L-Glutamic Acid | 7.35 |
| L-Glutamine | 365.0 |
| Glycine | 18.75 |
| L-Histidine-HCL- | 52.48 |
| H₂O | |
| L-Isoleucine | 106.97 |
| L-Leucine | 111.45 |
| L-Lysine HCL | 163.75 |
| L-Methionine | 32.34 |
| L-Phenylalainine | 68.48 |
| L-Proline | 40.0 |
| L-Serine | 26.25 |
| L-Threonine | 101.05 |
| L-Tryptophan | 19.22 |
| L-Tryrosine-2Na-2H₂O | 91.79 |
| L-Valine | 99.65 |

| **Vitamins** | |
|---|---|
| Biotin | 0.0035 mg/L |
| D-Ca Pantothenate | 3.24 |
| Choline Chloride | 11.78 |
| Folic Acid | 4.65 |
| i-Inositol | 15.60 |
| Niacinamide | 3.02 |
| Pyridoxal HCL | 3.00 |
| Pyridoxine HCL | 0.031 |
| Riboflavin | 0.319 |
| Thiamine HCL | 3.17 |
| Thymidine | 0.365 |
| Vitamin B₁₂ | 0.680 |

| **Other Components** | |
|---|---|
| HEPES Buffer | 25 mM |
| Na Hypoxanthine | 2.39 mg/L |
| Lipoic Acid | 0.105 |
| Sodium Putrescine-2HCL | 0.081 |
| Sodium Pyruvate | 55.0 |
| Sodium Selenite | 0.0067 |
| Ethanolamine | 20uM |
| Ferric Citrate | 0.122 |
| Methyl-B-Cyclodextrin complexed with Linoleic Acid | 41.70 |
| Methyl-B-Cyclodextrin complexed with Oleic Acid | 33.33 |
| Methyl-B-Cyclodextrin complexed with Retinal Acetate | 10 |

### Adjust osmolarity to 327 mOsm

### Example 12: Construction of GAS Reporter Construct

One signal transduction pathway involved in the differentiation and proliferation of cells is called the Jaks-STATs pathway. Activated proteins in the Jaks-STATs pathway bind to gamma activation site "GAS" elements or interferon-sensitive responsive element ("ISRE"), located in the promoter of many genes. The binding of a protein to these elements alter the expression of the associated gene.

GAS and ISRE elements are recognized by a class of transcription factors called Signal Transducers and Activators of Transcription, or "STATs." There are six members of the STATs family. Stat1 and Stat3 are present in many cell types, as is Stat2 (as response to IFN-alpha is widespread). Stat4 is more restricted and is not in many cell types though it has been found in T helper class I, cells after treatment with IL-12. Stat5 was originally called mammary growth factor, but has been found at higher concentrations in other cells including myeloid cells. It can be activated in tissue culture cells by many cytokines.

The STATs are activated to translocate from the cytoplasm to the nucleus upon tyrosine phosphorylation by a set of kinases known as the Janus Kinase ("Jaks") family. Jaks represent a distinct family of soluble tyrosine kinases and include Tyk2, Jak1, Jak2, and Jak3. These kinases display significant sequence similarity and are generally catalytically inactive in resting cells.

The Jaks are activated by a wide range of receptors summarized in the Table below. (Adapted from review by Schidler and Darnell, Ann. Rev. Biochem. 64:621-51 (1995).) A cytokine receptor family, capable of activating Jaks, is divided into two groups: (a) Class 1 includes receptors for IL-2, IL-3, IL-4, IL-6, IL-7, IL-9, IL-11, IL-12, IL-15, Epo, PRL, GH, G-CSF, GM-CSF, LIF, CNTF, and thrombopoietin; and (b) Class 2 includes IFN-a, IFN-g, and IL-10. The Class 1 receptors share a conserved cysteine motif (a set of four conserved cysteines and one tryptophan) and a WSXWS motif (a membrane proxial region encoding Trp-Ser-Xxx-Trp-Ser (SEQ ID NO:2)).

Thus, on binding of a ligand to a receptor, Jaks are activated, which in turn activate STATs, which then translocate and bind to GAS elements. This entire process is encompassed in the Jaks-STATs signal transduction pathway.

Therefore, activation of the Jaks-STATs pathway, reflected by the binding of the GAS or the ISRE element, can be used to indicate proteins involved in the proliferation and differentiation of cells. For example, growth factors and cytokines are known to activate the Jaks-STATs pathway. (See Table below.) Thus, by using GAS elements linked to reporter molecules, activators of the Jaks-STATs pathway can be identified.

| JAKs | | | | | STATS | GAS(elements) or |
|---|---|---|---|---|---|---|
| ISRE | | | | | | |
| Ligand | tyk2 | Jak1 | Jak2 | Jak3 | | |
| IFN family | | | | | | |
| IFN-a/B | + | + | - | - | 1,2,3 | ISRE |
| IFN-g | | + | + | - | 1 | GAS |
| (IRF1>Lys6>IFP) | | | | | | |
| Il-10 | + | ? | ? | - | 1,3 | |
| | | | | | | |

| gp130 family | | | | | | |
|---|---|---|---|---|---|---|
| IL-6 (Pleiotrohic) | + | + | + | ? | 1,3 | GAS |
| (IRF1>Lys6>IFP) | | | | | | |
| Il-11(Pleiotrohic) | ? | + | ? | ? | 1,3 | |
| OnM(Pleiotrohic) | ? | + | + | ? | 1,3 | |
| LIF(Pleiotrohic) | ? | + | + | ? | 1,3 | |
| CNTF(Pleiotrohic) | -/+ | + | + | ? | 1,3 | |
| G-CSF(Pleiotrohic) | ? | + | ? | ? | 1,3 | |
| IL-12(Pleiotrohic) | + | - | + | + | 1,3 | |
| | | | | | | |

| g-C family | | | | | | |
|---|---|---|---|---|---|---|
| IL-2 (lymphocytes) | - | + | - | + | 1,3,5 | GAS |
| IL-4 (lymph/myeloid) | - | + | - | + | 6 | GAS (IRF1 = IFP |
| >>Ly6)(IgH) | | | | | | |
| IL-7 (lymphocytes) | - | + | - | + | 5 | GAS |
| IL-9 (lymphocytes) | - | + | - | + | 5 | GAS |
| IL-13 (lymphocyte) | - | + | ? | ? | 6 | GAS |
| IL-15 | ? | + | ? | + | 5 | GAS |
| | | | | | | |

| gp140 family | | | | | | |
|---|---|---|---|---|---|---|
| IL-3 (myeloid) | - | - | + | - | 5 | GAS |
| (IRF1>IFP>>Ly6) | | | | | | |
| IL-5 (myeloid) | - | - | + | - | 5 | GAS |
| GM-CSF (myeloid) | - | - | + | - | 5 | GAS |
| | | | | | | |

| Growth hormone family | | | | | | |
|---|---|---|---|---|---|---|
| GH | ? | - | + | - | 5 | |
| PRL | ? | +/- | + | - | 1,3,5 | |
| EPO | ? | - | + | - | 5 | GAS(B- |
| CAS>IRF1=IFP>>Ly6) | | | | | | |
| | | | | | | |

| Receptor Tyrosine Kinases | | | | | | |
|---|---|---|---|---|---|---|
| EGF | ? | + | + | - | 1,3 | GAS (IRF1) |
| | | | | | | |
| PDGF | ? | + | + | - | 1,3 | |
| CSF-1 | ? | + | + | - | 1,3 | GAS (not IRF1) |

To construct a synthetic GAS containing promoter element, which is used in the Biological Assays described in Examples 13-14, a PCR based strategy is employed to generate a GAS-SV40 promoter sequence. The 5' primer contains four tandem copies of the GAS binding site found in the IRF1 promoter and previously demonstrated to bind STATs upon induction with a range of cytokines (Rothman et al., Immunity 1:457-468 (1994).), although other GAS or ISRE elements can be used instead. The 5' primer also contains 18bp of sequence complementary to the SV40 early promoter sequence and is flanked with an XhoI site. The sequence of the 5' primer is:

5':GCGCCTCGAGATTTCCCCGAAATCTAGATTTCCCCGAAATGATTTCCCCG
AAATGATTTCCCCGAAATATCTGCCATCTCAATTAG:3' (SEQ ID NO:3)

The downstream primer is complementary to the SV40 promoter and is flanked with a Hind III site: 5':GCGGCAAGCTTTTTGCAAAGCCTAGGC:3' (SEQ ID NO:4)

PCR amplification is performed using the SV40 promoter template present in the B-gal:promoter plasmid obtained from Clontech. The resulting PCR fragment is digested with XhoI/Hind III and subcloned into BLSK2-. (Stratagene.) Sequencing with forward and reverse primers confirms that the insert contains the following sequence:

5':CTCGAGATTTCCCCGAAATCTAGATTTCCCCGAAATGATTTCCCCGAAATG
ATTTCCCCGAAATATCTGCCATCTCAATTAGTCAGCAACCATAGTCCCGCCC
CTAACTCCGCCCATCCCGCCCCTAACTCCGCCCAGTTCCGCCCATTCTCCGC
CCCATGGCTGACTAATTTTTTTTATTTATGCAGAGGCCGAGGCCGCCTCGGC
CTCTGAGCTATTCCAGAAGTAGTGAGGAGGCTTTTTTGGAGGCCTAGGCTTT
TGCAAAAAGCTT:3' (SEQ ID NO:5)

With this GAS promoter element linked to the SV40 promoter, a GAS:SEAP2 reporter construct is next engineered. Here, the reporter molecule is a secreted alkaline phosphatase, or "SEAP." Clearly, however, any reporter molecule can be instead of SEAP, in this or in any of the other Examples. Well known reporter molecules that can be used instead of SEAP include chloramphenicol acetyltransferase (CAT), luciferase, alkaline phosphatase, B-galactosidase, green fluorescent protein (GFP), or any protein detectable by an antibody.

The above sequence confirmed synthetic GAS-SV40 promoter element is subcloned into the pSEAP-Promoter vector obtained from Clontech using HindIII and XhoI, effectively replacing the SV40 promoter with the amplified GAS:SV40 promoter element, to create the GAS-SEAP vector. However, this vector does not contain a neomycin resistance gene, and therefore, is not preferred for mammalian expression systems.

Thus, in order to generate mammalian stable cell lines expressing the GAS-SEAP reporter, the GAS-SEAP cassette is removed from the GAS-SEAP vector using Sail and NotI, and inserted into a backbone vector containing the neomycin resistance gene, such as pGFP-1 (Clontech), using these restriction sites in the multiple cloning site, to create the GAS-SEAP/Neo vector. Once this vector is transfected into mammalian cells, this vector can then be used as a reporter molecule for GAS binding as described in Examples 13-14.

Other constructs can be made using the above description and replacing GAS with a different promoter sequence. For example, construction of reporter molecules containing NFK-B and EGR promoter sequences are described in Examples 15 and 16. However, many other promoters can be substituted using the protocols described in these Examples. For instance, SRE, IL-2, NFAT, or Osteocalcin promoters can be substituted, alone or in combination (e.g., GAS/NF-KB/EGR, GAS/NF-KB, Il-2/NFAT, or NF-KB/GAS). Similarly, other cell lines can be used to test reporter construct activity, such as HELA (epithelial), HUVEC (endothelial), Reh (B-cell), Saos-2 (osteoblast), HUVAC (aortic), or Cardiomyocyte.

### Example 13: High-Throughput Screening Assay for T-cell Activity.

The following protocol is used to assess T-cell activity by identifying factors, such as growth factors and cytokines, that may proliferate or differentiate T-cells. T-cell activity is assessed using the GAS/SEAP/Neo construct produced in Example 12. Thus, factors that increase SEAP activity indicate the ability to activate the Jaks-STATS signal transduction pathway. The T-cell used in this assay is Jurkat T-cells (ATCC Accession No. TIB-152), although Molt-3 cells (ATCC Accession No. CRL-1552) and Molt-4 cells (ATCC Accession No. CRL-1582) cells can also be used.

Jurkat T-cells are lymphoblastic CD4+ Th1 helper cells. In order to generate stable cell lines, approximately 2 million Jurkat cells are transfected with the GAS-SEAP/neo vector using DMRIE-C (Life Technologies)(transfection procedure described below). The transfected cells are seeded to a density of approximately 20,000 cells per well and transfectants resistant to 1 mg/ml genticin selected. Resistant colonies are expanded and then tested for their response to increasing concentrations of interferon gamma. The dose response of a selected clone is demonstrated.

Specifically, the following protocol will yield sufficient cells for 75 wells containing 200 ul of cells. Thus, it is either scaled up, or performed in multiple to generate sufficient cells for multiple 96 well plates. Jurkat cells are maintained in RPMI + 10% serum with 1%Pen-Strep. Combine 2.5 mls of OPTI-MEM (Life Technologies) with 10 ug of plasmid DNA in a T25 flask. Add 2.5 ml OPTI-MEM containing 50 ul of DMRIE-C and incubate at room temperature for 15-45 mins.

During the incubation period, count cell concentration, spin down the required number of cells (10⁷ per transfection), and resuspend in OPTI-MEM to a final concentration of 10⁷ cells/ml. Then add 1ml of 1 x 10⁷ cells in OPTI-MEM to T25 flask and incubate at 37°C for 6 hrs. After the incubation, add 10 ml of RPMI + 15% serum.

The Jurkat:GAS-SEAP stable reporter lines are maintained in RPMI + 10% serum, 1 mg/ml Genticin, and 1% Pen-Strep. These cells are treated with supernatants containing a polypeptide as produced by the protocol described in Example 11.

On the day of treatment with the supernatant, the cells should be washed and resuspended in fresh RPMI + 10% serum to a density of 500,000 cells per ml. The exact number of cells required will depend on the number of supernatants being screened. For one 96 well plate, approximately 10 million cells (for 10 plates, 100 million cells) are required.

Transfer the cells to a triangular reservoir boat, in order to dispense the cells into a 96 well dish, using a 12 channel pipette. Using a 12 channel pipette, transfer 200 ul of cells into each well (therefore adding 100, 000 cells per well).

After all the plates have been seeded, 50 ul of the supematants are transferred directly from the 96 well plate containing the supernatants into each well using a 12 channel pipette. In addition, a dose of exogenous interferon gamma (0.1, 1.0, 10 ng) is added to wells H9, H10, and H11 to serve as additional positive controls for the assay.

The 96 well dishes containing Jurkat cells treated with supernatants are placed in an incubator for 48 hrs (note: this time is variable between 48-72 hrs). 35 ul samples from each well are then transferred to an opaque 96 well plate using a 12 channel pipette. The opaque plates should be covered (using sellophene covers) and stored at -20°C until SEAP assays are performed according to Example 17. The plates containing the remaining treated cells are placed at 4°C and serve as a source of material for repeating the assay on a specific well if desired.

As a positive control, 100 Unit/ml interferon gamma can be used which is known to activate Jurkat T cells. Over 30 fold induction is typically observed in the positive control wells.

### Example 14: High-Throughput Screening Assay Identifying Myeloid Activity

The following protocol is used to assess myeloid activity by identifying factors, such as growth factors and cytokines, that may proliferate or differentiate myeloid cells. Myeloid cell activity is assessed using the GAS/SEAP/Neo construct produced in Example 12. Thus, factors that increase SEAP activity indicate the ability to activate the Jaks-STATS signal transduction pathway. The myeloid cell used in this assay is U937, a pre-monocyte cell line, although TF-1, HL60, or KG1 can be used.

To transiently transfect U937 cells with the GAS/SEAP/Neo construct produced in Example 12, a DEAE-Dextran method (Kharbanda et. al., 1994, Cell Growth & Differentiation, 5:259-265) is used. First, harvest 2x10e⁷ U937 cells and wash with PBS. The U937 cells are usually grown in RPMI 1640 medium containing 10% heat-inactivated fetal bovine serum (FBS) supplemented with 100 units/ml penicillin and 100 mg/ml streptomycin.

Next, suspend the cells in 1 ml of 20 mM Tris-HCl (pH 7.4) buffer containing 0.5 mg/ml DEAE-Dextran, 8 ug GAS-SEAP2 plasmid DNA, 140 mM NaCl, 5 mM KCl, 375 uM Na₂HPO₄.7H₂O, 1 mM MgCl₂, and 675 uM CaCl₂. Incubate at 37°C for 45 min.

Wash the cells with RPMI 1640 medium containing 10% FBS and then resuspend in 10 ml complete medium and incubate at 37°C for 36 hr.

The GAS-SEAP/U937 stable cells are obtained by growing the cells in 400 ug/ml G418. The G418-free medium is used for routine growth but every one to two months, the cells should be re-grown in 400 ug/ml G418 for couple of passages.

These cells are tested by harvesting 1x10⁸ cells (this is enough for ten 96-well plates assay) and wash with PBS. Suspend the cells in 200 ml above described growth medium, with a final density of 5x10⁵ cells/ml. Plate 200 ul cells per well in the 96-well plate (or 1x10⁵ cells/well).

Add 50 ul of the supernatant prepared by the protocol described in Example 11. Incubate at 37°C for 48 to 72 hr. As a positive control, 100 Unit/ml interferon gamma can be used which is known to activate U937 cells. Over 30 fold induction is typically observed in the positive control wells. SEAP assay the supernatant according to the protocol described in Example 17.

### Example 15: High-Throughput Screening Assay Identifying Neuronal Activity.

When cells undergo differentiation and proliferation, a group of genes are activated through many different signal transduction pathways. One of these genes, EGR1 (early growth response gene 1), is induced in various tissues and cell types upon activation. The promoter of EGR1 is responsible for such induction. Using the EGR1 promoter linked to reporter molecules, activation of cells can be assessed.

Particularly, the following protocol is used to assess neuronal activity in PC12 cell lines. PC12 cells (rat phenochromocytoma cells) are known to proliferate and/or differentiate by activation with a number of mitogens, such as TPA (tetradecanoyl phorbol acetate), NGF (nerve growth factor), and EGF (epidermal growth factor). The EGR1 gene expression is activated during this treatment. Thus, by stably transfecting PC12 cells with a construct containing an EGR promoter linked to SEAP reporter, activation of PC12 cells can be assessed.

The EGR/SEAP reporter construct can be assembled by the following protocol. The EGR-1 promoter sequence (-633 to +1)(Sakamoto K et al., Oncogene 6:867-871 (1991)) can be PCR amplified from human genomic DNA using the following primers:

5' GCGCTCGAGGGATGACAGCGATAGAACCCCGG -3' (SEQ ID NO:6)

5' GCGAAGCTTCGCGACTCCCCGGATCCGCCTC-3' (SEQ ID NO:7)

Using the GAS:SEAP/Neo vector produced in Example 12, EGR1 amplified product can then be inserted into this vector. Linearize the GAS:SEAP/Neo vector using restriction enzymes XhoI/HindIII, removing the GAS/SV40 stuffer. Restrict the EGR1 amplified product with these same enzymes. Ligate the vector and the EGR1 promoter.

To prepare 96 well-plates for cell culture, two mls of a coating solution (1:30 dilution of collagen type I (Upstate Biotech Inc. Cat#08-115) in 30% ethanol (filter sterilized)) is added per one 10 cm plate or 50 ml per well of the 96-well plate, and allowed to air dry for 2 hr.

PC12 cells are routinely grown in RPMI-1640 medium (Bio Whittaker) containing 10% horse serum (JRH BIOSCIENCES, Cat. # 12449-78P), 5% heat-inactivated fetal bovine serum (FBS) supplemented with 100 units/ml penicillin and 100 ug/ml streptomycin on a precoated 10 cm tissue culture dish. One to four split is done every three to four days. Cells are removed from the plates by scraping and resuspended with pipetting up and down for more than 15 times.

Transfect the EGR/SEAP/Neo construct into PC12 using the Lipofectamine protocol described in Example 11. EGR-SEAP/PC12 stable cells are obtained by growing the cells in 300 ug/ml G418. The G418-free medium is used for routine growth but every one to two months, the cells should be re-grown in 300 ug/ml G418 for couple of passages.

To assay for neuronal activity, a 10 cm plate with cells around 70 to 80% confluent is screened by removing the old medium. Wash the cells once with PBS (Phosphate buffered saline). Then starve the cells in low serum medium (RPMI-1640 containing 1% horse serum and 0.5% FBS with antibiotics) overnight.

The next morning, remove the medium and wash the cells with PBS. Scrape off the cells from the plate, suspend the cells well in 2 ml low serum medium. Count the cell number and add more low serum medium to reach final cell density as 5x10⁵ cells/ml.

Add 200 ul of the cell suspension to each well of 96-well plate (equivalent to 1x10⁵ cells/well). Add 50 ul supernatant produced by Example 11, 37°C for 48 to 72 hr. As a positive control, a growth factor known to activate PC12 cells through EGR can be used, such as 50 ng/ul of Neuronal Growth Factor (NGF). Over fifty-fold induction of SEAP is typically seen in the positive control wells. SEAP assay the supernatant according to Example 17.

### Example 16: High-Throughput Screening Assay for T-cell Activity

NF-κB (Nuclear Factor κB) is a transcription factor activated by a wide variety of agents including the inflammatory cytokines IL-1 and TNF, CD30 and CD40, lymphotoxin-alpha and lymphotoxin-beta, by exposure to LPS or thrombin, and by expression of certain viral gene products. As a transcription factor, NF-κB regulates the expression of genes involved in immune cell activation, control of apoptosis (NF-κB appears to shield cells from apoptosis), B and T-cell development, anti-viral and antimicrobial responses, and multiple stress responses.

In non-stimulated conditions, NF- κB is retained in the cytoplasm with I-κB (Inhibitor κB). However, upon stimulation, I- κB is phosphorylated and degraded, causing NF- κB to shuttle to the nucleus, thereby activating transcription of target genes. Target genes activated by NF- κB include IL-2, IL-6, GM-CSF, ICAM-1 and class 1 MHC.

Due to its central role and ability to respond to a range of stimuli, reporter constructs utilizing the NF-κB promoter element are used to screen the supernatants produced in Example 11. Activators or inhibitors of NF-κB would be useful in treating diseases. For example, inhibitors of NF-κB could be used to treat those diseases related to the acute or chronic activation of NF-κB, such as rheumatoid arthritis.

To construct a vector containing the NF-κB promoter element, a PCR based strategy is employed. The upstream primer contains four tandem copies of the NF-κB binding site (GGGGACTTTCCC) (SEQ ID NO:8), 18 bp of sequence complementary to the 5' end of the SV40 early promoter sequence, and is flanked with an XhoI site:

5':GCGGCCTCGAGGGGACTTTCCCGGGGACTTTCCGGGGACTTTCCGGGAC
TTTCCATCCTGCCATCTCAATTAG:3' (SEQ ID NO:9)

The downstream primer is complementary to the 3' end of the SV40 promoter and is flanked with a Hind III site:

5':GCGGCAAGCTTTTTGCAAAGCCTAGGC:3' (SEQ ID NO:4)

PCR amplification is performed using the SV40 promoter template present in the pB-gal:promoter plasmid obtained from Clontech. The resulting PCR fragment is digested with XhoI and Hind III and subcloned into BLSK2-. (Stratagene) Sequencing with the T7 and T3 primers confirms the insert contains the following sequence:

5':CTCGAGGGGACTTTCCCGGGGACTTTCCGGGGACTTTCCGGGACTTTCC
ATCTGCCATCTCAATTAGTCAGCAACCATAGTCCCGCCCCTAACTCCGCCCA
TCCCGCCCCTAACTCCGCCCAGTTCCGCCCATTCTCCGCCCCATGGCTGACT
AATTTTTTTTATTTATGCAGAGGCCGAGGCCGCCTCGGCCTCTGAGCTATTC
CAGAAGTAGTGAGGAGGCTTTTTTGGAGGCCTAGGCTTTTGCAAAAAGCTT:
3' (SEQ ID NO:10)

Next, replace the SV40 minimal promoter element present in the pSEAP2-promoter plasmid (Clontech) with this NF-κB/SV40 fragment using XhoI and HindIII. However, this vector does not contain a neomycin resistance gene, and therefore, is not preferred for mammalian expression systems.

In order to generate stable mammalian cell lines, the NF-κB/SV40/SEAP cassette is removed from the above NF-κB/SEAP vector using restriction enzymes Sail and NotI, and inserted into a vector containing neomycin resistance. Particularly, the NF-κB/SV40/SEAP cassette was inserted into pGFP-1 (Clontech), replacing the GFP gene, after restricting pGFP-1 with Sail and NotI.

Once NF-κB/SV40/SEAP/Neo vector is created, stable Jurkat T-cells are created and maintained according to the protocol described in Example 13. Similarly, the method for assaying supernatants with these stable Jurkat T-cells is also described in Example 13. As a positive control, exogenous TNF alpha (0.1,1, 10 ng) is added to wells H9, H10, and H11, with a 5-10 fold activation typically observed.

### Example 17: Assay for SEAP Activity

As a reporter molecule for the assays described in Examples 13-16, SEAP activity is assayed using the Tropix Phospho-light Kit (Cat. BP-400) according to the following general procedure. The Tropix Phospho-light Kit supplies the Dilution, Assay, arid Reaction Buffers used below.

Prime a dispenser with the 2.5x Dilution Buffer and dispense 15 µl of 2.5x dilution buffer into Optiplates containing 35 µl of a supernatant. Seal the plates with a plastic sealer and incubate at 65°C for 30 min. Separate the Optiplates to avoid uneven heating.

Cool the samples to room temperature for 15 minutes. Empty the dispenser and prime with the Assay Buffer. Add 50 µl Assay Buffer and incubate at room temperature 5 min. Empty the dispenser and prime with the Reaction Buffer (see the table below). Add 50 µl Reaction Buffer and incubate at room temperature for 20 minutes. Since the intensity of the chemiluminescent signal is time dependent, and it takes about 10 minutes to read 5 plates on luminometer, one should treat 5 plates at each time and start the second set 10 minutes later.

Read the relative light unit in the luminometer. Set H12 as blank, and print the results. An increase in chemiluminescence indicates reporter activity.

| **Reaction Buffer Formulation**: | | |
|---|---|---|
| # of plates | Rxn buffer diluent (ml) | CSPD (ml) |
| 10 | 60 | 3 |
| 11 | 65 | 3.25 |
| 12 | 70 | 3.5 |
| 13 | 75 | 3.75 |
| 14 | 80 | 4 |
| 15 | 85 | 4.25 |
| 16 | 90 | 4.5 |
| 17 | 95 | 4.75 |
| 18 | 100 | 5 |
| 19 | 105 | 5.25 |
| 20 | 110 | 5.5 |
| 21 | 115 | 5.75 |
| 22 | 120 | 6 |
| 23 | 125 | 6.25 |
| 24 | 130 | 6.5 |
| 25 | 135 | 6.75 |
| 26 | 140 | 7 |
| 27 | 145 | 7.25 |
| 28 | 150 | 7.5 |
| 29 | 155 | 7.75 |
| 30 | 160 | 8 |
| 31 | 165 | 8.25 |
| 32 | 170 | 8.5 |
| 33 | 175 | 8.75 |
| 34 | 180 | 9 |
| 35 | 185 | 9.25 |
| 36 | 190 | 9.5 |
| 37 | 195 | 9.75 |
| 38 | 200 | 10 |
| 39 | 205 | 10.25 |
| 40 | 210 | 10.5 |
| 41 | 215 | 10.75 |
| 42 | 220 | 11 |
| 43 | 225 | 11.25 |
| 44 | 230 | 11.5 |
| 45 | 235 | 11.75 |
| 46 | 240 | 12 |
| 47 | 245 | 12.25 |
| 48 | 250 | 12.5 |
| 49 | 255 | 12.75 |
| 50 | 260 | 13 |

### Example 18: High-Throughput Screening Assay Identifying Changes in Small Molecule Concentration and Membrane Permeability

Binding of a ligand to a receptor is known to alter intracellular levels of small molecules, such as calcium, potassium, sodium, and pH, as well as alter membrane potential. These alterations can be measured in an assay to identify supernatants which bind to receptors of a particular cell. Although the following protocol describes an assay for calcium, this protocol can easily be modified to detect changes in potassium, sodium, pH, membrane potential, or any other small molecule which is detectable by a fluorescent probe.

The following assay uses Fluorometric Imaging Plate Reader ("FLIPR") to measure changes in fluorescent molecules (Molecular Probes) that bind small molecules. Clearly, any fluorescent molecule detecting a small molecule can be used instead of the calcium fluorescent molecule, fluo-3, used here.

For adherent cells, seed the cells at 10,000 -20,000 cells/well in a Co-star black 96-well plate with clear bottom. The plate is incubated in a CO₂ incubator for 20 hours. The adherent cells are washed two times in Biotek washer with 200 ul of HBSS (Hank's Balanced Salt Solution) leaving 100 ul of buffer after the final wash.

A stock solution of 1 mg/ml fluo-3 is made in 10% pluronic acid DMSO. To load the cells with fluo-3, 50 ul of 12 ug/ml fluo-3 is added to each well. The plate is incubated at 37°C in a CO₂ incubator for 60 min. The plate is washed four times in the Biotek washer with HBSS leaving 100 ul of buffer.

For non-adherent cells, the cells are spun down from culture media. Cells are re-suspended to 2-5x10⁶ cells/ml with HBSS in a 50-ml conical tube, 4 ul of 1 mg/ml fluo-3 solution in 10% pluronic acid DMSO is added to each ml of cell suspension. The tube is then placed in a 37°C water bath for 30-60 min. The cells are washed twice with HBSS, resuspended to 1x10⁶ cells/ml, and dispensed into a microplate, 100 ul/well. The plate is centrifuged at 1000 rpm for 5 min. The plate is then washed once in Denley CellWash with 200 ul, followed by an aspiration step to 100 ul final volume.

For a non-cell based assay, each well contains a fluorescent molecule, such as fluo-3. The supernatant is added to the well, and a change in fluorescence is detected.

To measure the fluorescence of intracellular calcium, the FLIPR is set for the following parameters: (1) System gain is 300-800 mW; (2) Exposure time is 0.4 second; (3) Camera F/stop is F/2; (4) Excitation is 488 nm; (5) Emission is 530 nm; and (6) Sample addition is 50 ul. Increased emission at 530 nm indicates an extracellular signaling even which has resulted in an increase in the intracellular Ca⁺⁺ concentration.

### Example 19: High-Throughput Screening Assay Identifying Tyrosine Kinase Activity

The Protein Tyrosine Kinases (PTK) represent a diverse group of transmembrane and cytoplasmic kinases. Within the Receptor Protein Tyrosine Kinase RPTK) group are receptors for a range of mitogenic and metabolic growth factors including the PDGF, FGF, EGF, NGF, HGF and Insulin receptor subfamilies. In addition there are a large family of RPTKs for which the corresponding ligand is unknown. Ligands for RPTKs include mainly secreted small proteins, but also membrane-bound and extracellular matrix proteins.

Activation of RPTK by ligands involves ligand-mediated receptor dimerization, resulting in transphosphorylation of the receptor subunits and activation of the cytoplasmic tyrosine kinases. The cytoplasmic tyrosine kinases include receptor associated tyrosine kinases of the src-family (e.g., src, yes, lck, lyn, fyn) and non-receptor linked and cytosolic protein tyrosine kinases, such as the Jak family, members of which mediate signal transduction triggered by the cytokine superfamily of receptors (e.g., the Interleukins, Interferons, GM-CSF, and Leptin).

Because of the wide range of known factors capable of stimulating tyrosine kinase activity, the identification of novel human secreted proteins capable of activating tyrosine kinase signal transduction pathways are of interest. Therefore, the following protocol is designed to identify those novel human secreted proteins capable of activating the tyrosine kinase signal transduction pathways.

Seed target cells (e.g., primary keratinocytes) at a density of approximately 25,000 cells per well in a 96 well Loprodyne Silent Screen Plates purchased from Nalge Nunc (Naperville, IL). The plates are sterilized with two 30 minute rinses with 100% ethanol, rinsed with water and dried overnight. Some plates are coated for 2 hr with 100 ml of cell culture grade type I collagen (50 mg/ml), gelatin (2%) or polylysine (50 mg/ml), all of which can be purchased from Sigma Chemicals (St. Louis, MO) or 10% Matrigel purchased from Becton Dickinson (Bedford,MA), or calf serum, rinsed with PBS and stored at 4°C. Cell growth on these plates is assayed by seeding 5,000 cells/well in growth medium and indirect quantitation of cell number through use of alamarBlue as described by the manufacturer Alamar Biosciences, Inc. (Sacramento, CA) after 48 hr. Falcon plate covers #3071 from Becton Dickinson (Bedford,MA) are used to cover the Loprodyne Silent Screen Plates. Falcon Microtest III cell culture plates can also be used in some proliferation experiments.

To prepare extracts, A431 cells are seeded onto the nylon membranes of Loprodyne plates (20,000/200ml/well) and cultured overnight in complete medium. Cells are quiesced by incubation in serum-free basal medium for 24 hr. After 5-20 minutes treatment with EGF (60ng/ml) or 50 ul of the supernatant produced in Example 11, the medium was removed and 100 ml of extraction buffer ((20 mM HEPES pH 7.5, 0.15 M NaCl, 1% Triton X-100, 0.1% SDS, 2 mM Na3VO4, 2 mM Na4P2O7 and a cocktail of protease inhibitors (# 1836170) obtained from Boeheringer Mannheim (Indianapolis, IN) is added to each well and the plate is shaken on a rotating shaker for 5 minutes at 4°C. The plate is then placed in a vacuum transfer manifold and the extract filtered through the 0.45 mm membrane bottoms of each well using house vacuum. Extracts are collected in a 96-well catch/assay plate in the bottom of the vacuum manifold and immediately placed on ice. To obtain extracts clarified by centrifugation, the content of each well, after detergent solubilization for 5 minutes, is removed and centrifuged for 15 minutes at 4°C at 16,000 x g.

Test the filtered extracts for levels of tyrosine kinase activity. Although many methods of detecting tyrosine kinase activity are known, one method is described here.

Generally, the tyrosine kinase activity of a supernatant is evaluated by determining its ability to phosphorylate a tyrosine residue on a specific substrate (a biotinylated peptide). Biotinylated peptides that can be used for this purpose include PSK1 (corresponding to amino acids 6-20 of the cell division kinase cdc2-p34) and PSK2 (corresponding to amino acids 1-17 of gastrin). Both peptides are substrates for a range of tyrosine kinases and are available from Boehringer Mannheim.

The tyrosine kinase reaction is set up by adding the following components in order. First, add 10ul of SuM Biotinylated Peptide, then 10ul ATP/Mg₂₊ (5mM ATP/50mM MgCl₂), then 10ul of 5x Assay Buffer (40mM imidazole hydrochloride, pH7.3, 40 mM beta-glycerophosphate, 1mM EGTA, 100mM MgCl₂, 5 mM MnCl_{2,} 0.5 mg/ml BSA), then 5ul of Sodium Vanadate(1mM), and then 5ul of water. Mix the components gently and preincubate the reaction mix at 30°C for 2 min. Initial the reaction by adding 10ul of the control enzyme or the filtered supernatant.

The tyrosine kinase assay reaction is then terminated by adding 10 ul of 120mm EDTA and place the reactions on ice.

Tyrosine kinase activity is determined by transferring 50 ul aliquot of reaction mixture to a microtiter plate (MTP) module and incubating at 37°C for 20 min. This allows the streptavadin coated 96 well plate to associate with the biotinylated peptide. Wash the MTP module with 300ul/well of PBS four times. Next add 75 ul of anti-phospotyrosine antibody conjugated to horse radish peroxidase(anti-P-Tyr-POD(0.5u/ml)) to each well and incubate at 37°C for one hour. Wash the well as above.

Next add 100ul of peroxidase substrate solution (Boehringer Mannheim) and incubate at room temperature for at least 5 mins (up to 30 min). Measure the absorbance of the sample at 405 nm by using ELISA reader. The level of bound peroxidase activity is quantitated using an ELISA reader and reflects the level of tyrosine kinase activity.

### Example 20: High-Throughput Screening Assay Identifying Phosphorylation Activity

As a potential alternative and/or compliment to the assay of protein tyrosine kinase activity described in Example 19, an assay which detects activation (phosphorylation) of major intracellular signal transduction intermediates can also be used. For example, as described below one particular assay can detect tyrosine phosphorylation of the Erk-1 and Erk-2 kinases. However, phosphorylation of other molecules, such as Raf, JNK, p38 MAP, Map kinase kinase (MEK), MEK kinase, Src, Muscle specific kinase (MuSK), IRAK, Tec, and Janus, as well as any other phosphoserine, phosphotyrosine, or phosphothreonine molecule, can be detected by substituting these molecules for Erk-1 or Erk-2 in the following assay.

Specifically, assay plates are made by coating the wells of a 96-well ELISA plate with 0.1ml of protein G (1ug/ml) for 2 hr at room temp, (RT). The plates are then rinsed with PBS and blocked with 3% BSA/PBS for 1 hr at RT. The protein G plates are then treated with 2 commercial monoclonal antibodies (100ng/well) against Erk-1 and Erk-2 (1 hr at RT) (Santa Cruz Biotechnology). (To detect other molecules, this step can easily be modified by substituting a monoclonal antibody detecting any of the above described molecules.) After 3-5 rinses with PBS, the plates are stored at 4°C until use.

A431 cells are seeded at 20,000/well in a 96-well Loprodyne filterplate and cultured overnight in growth medium. The cells are then starved for 48 hr in basal medium (DMEM) and then treated with EGF (6ng/well) or 50 ul of the supernatants obtained in Example 11 for 5-20 minutes. The cells are then solubilized and extracts filtered directly into the assay plate.

After incubation with the extract for 1 hr at RT, the wells are again rinsed. As a positive control, a commercial preparation of MAP kinase (10ng/well) is used in place of A431 extract. Plates are then treated with a commercial polyclonal (rabbit) antibody (1ug/ml) which specifically recognizes the phosphorylated epitope of the Erk-1 and Erk-2 kinases (1 hr at RT). This antibody is biotinylated by standard procedures. The bound polyclonal antibody is then quantitated by successive incubations with Europium-streptavidin and Europium fluorescence enhancing reagent in the Wallac DELFIA instrument (time-resolved fluorescence). An increased fluorescent signal over background indicates a phosphorylation.

### Example 21: Method of Determining Alterations in a Gene Corresponding to a Polynucleotide

RNA isolated from entire families or individual patients presenting with a phenotype of interest (such as a disease) is be isolated. cDNA is then generated from these RNA samples using protocols known in the art. (See, Sambrook.) The cDNA is then used as a template for PCR, employing primers surrounding regions of interest in SEQ ID NO:X. Suggested PCR conditions consist of 35 cycles at 95°C for 30 seconds; 60-120 seconds at 52-58°C; and 60-120 seconds at 70°C, using buffer solutions described in Sidransky, D., et al., Science 252:706 (1991).

PCR products is then sequenced using primers labeled at their 5' end with T4 polynucleotide kinase, employing SequiTherm Polymerase. (Epicentre Technologies). The intron-exon borders of selected exons is also determined and genomic PCR products analyzed to confirm the results. PCR products harboring suspected mutations is then cloned and sequenced to validate the results of the direct sequencing.

PCR products is cloned into T-tailed vectors as described in Holton, T.A. and Graham, M.W., Nucleic Acids Research, 19:1156 (1991) and sequenced with T7 polymerase (United States Biochemical). Affected individuals is identified by mutations not present in unaffected individuals.

Genomic rearrangements are also observed as a method of determining alterations in a gene corresponding to a polynucleotide. Genomic clones isolated according to Example 2 are nick-translated with digoxigenindeoxy-uridine 5'-triphosphate (Boehringer Manheim), and FISH performed as described in Johnson, Cg. et al., Methods Cell Biol. 35:73-99 (1991). Hybridization with the labeled probe is carried out using a vast excess of human cot-1 DNA for specific hybridization to the corresponding genomic locus.

Chromosomes are counterstained with 4,6-diamino-2-phenylidole and propidium iodide, producing a combination of C- and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters. (Johnson, Cv. et al., Genet. Anal. Tech. Appl., 8:75 (1991).) Image collection, analysis and chromosomal fractional length measurements are performed using the ISee Graphical Program System. (Inovision Corporation, Durham, NC.) Chromosome alterations of the genomic region hybridized by the probe are identified as insertions, deletions, and translocations. These alterations are used as a diagnostic marker for an associated disease.

### Example 22: Method of Detecting Abnormal Levels of a Polypeptide in a Biological Sample

A polypeptide of the present invention can be detected in a biological sample, and if an increased or decreased level of the polypeptide is detected, this polypeptide is a marker for a particular phenotype. Methods of detection are numerous, and thus, it is understood that one skilled in the art can modify the following assay to fit their particular needs.

For example, antibody-sandwich ELISAs are used to detect soluble polypeptides in a sample, preferably a biological sample. Wells of a microtiter plate are coated with specific antibodies, at a final concentration of 0.2 to 10 ug/ml. The antibodies are either monoclonal or polyclonal and are produced by the method described in Example 10. The wells are blocked so that non-specific binding of the polypeptide to the well is reduced.

The coated wells are then incubated for > 2 hours at RT with a sample containing the polypeptide. Preferably, serial dilutions of the sample should be used to validate results. The plates are then washed three times with deionized or distilled water to remove unbounded polypeptide.

Next, 50 ul of specific antibody-alkaline phosphatase conjugate, at a concentration of 25-400 ng, is added and incubated for 2 hours at room temperature. The plates are again washed three times with deionized or distilled water to remove unbounded conjugate.

Add 75 ul of 4-methylumbelliferyl phosphate (MUP) or p-nitrophenyl phosphate (NPP) substrate solution to each well and incubate 1 hour at room temperature. Measure the reaction by a microtiter plate reader. Prepare a standard curve, using serial dilutions of a control sample, and plot polypeptide concentration on the X-axis (log scale) and fluorescence or absorbance of the Y-axis (linear scale). Interpolate the concentration of the polypeptide in the sample using the standard curve.

### Example 23: Formulating a Polypeptide

The secreted polypeptide composition will be formulated and dosed in a fashion consistent with good medical practice, taking into account the clinical condition of the individual patient (especially the side effects of treatment with the secreted polypeptide alone), the site of delivery, the method of administration, the scheduling of administration, and other factors known to practitioners. The "effective amount" for purposes herein is thus determined by such considerations.

As a general proposition, the total pharmaceutically effective amount of secreted polypeptide administered parenterally per dose will be in the range of about 1 µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the secreted polypeptide is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Pharmaceutical compositions containing the secreted protein of the invention are administered orally, rectally, parenterally, intracistemally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrastemal, subcutaneous and intraarticular injection and infusion.

The secreted polypeptide is also suitably administered by sustained-release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2- hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al.) or poly-D- (-)-3-hydroxybutyric acid (EP 133,988). Sustained-release compositions also include liposomally entrapped polypeptides. Liposomes containing the secreted polypeptide are prepared by methods known per se: DE 3,218,121; Epstein et al., Proc. Natl. Acad. Sci. USA 82:3688-3692 (1985); Hwang et al., Proc. Natl. Acad. Sci. USA 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal secreted polypeptide therapy.

For parenteral administration, in one embodiment, the secreted polypeptide is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the polypeptide uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The secreted polypeptide is typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of polypeptide salts.

Any polypeptide to be used for therapeutic administration can be sterile. Sterility is readily accomplished by filtration through sterile filtration membranes (e.g., 0.2 micron membranes). Therapeutic polypeptide compositions generally are placed into a container having a sterile access port, for example, an intravenous solution bag or vial having a stopper pierceable by a hypodermic injection needle.

Polypeptides ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous polypeptide solution, and the resulting mixture is lyophilized. The infusion solution is prepared by reconstituting the lyophilized polypeptide using bacteriostatic Water-for-Injection.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

### Example 24: Method of Treating Decreased Levels of the Polypeptide

It will be appreciated that conditions caused by a decrease in the standard or normal expression level of a secreted protein in an individual can be treated by administering the polypeptide of the present invention, preferably in the secreted form. Thus, the invention also provides a method of treatment of an individual in need of an increased level of the polypeptide comprising administering to such an individual a pharmaceutical composition comprising an amount of the polypeptide to increase the activity level of the polypeptide in such an individual.

For example, a patient with decreased levels of a polypeptide receives a daily dose 0.1-100 ug/kg of the polypeptide for six consecutive days. Preferably, the polypeptide is in the secreted form. The exact details of the dosing scheme, based on administration and formulation, are provided in Example 23.

### Example 25: Method of Treating Increased Levels of the Polypeptide

Antisense technology is used to inhibit production of a polypeptide of the present invention. This technology is one example of a method of decreasing levels of a polypeptide, preferably a secreted form, due to a variety of etiologies, such as cancer.

For example, a patient diagnosed with abnormally increased levels of a polypeptide is administered intravenously antisense polynucleotides at 0.5, 1.0, 1.5, 2.0 and 3.0 mg/kg day for 21 days. This treatment is repeated after a 7-day rest period if the treatment was well tolerated. The formulation of the antisense polynucleotide is provided in Example 23.

### Example 26: Method of Treatment Using Gene Therapy

One method of gene therapy transplants fibroblasts, which are capable of expressing a polypeptide, onto a patient. Generally, fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin, is added. The flasks are then incubated at 37°C for approximately one week.

At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. et al., DNA, 7:219-25 (1988)), flanked by the long terminal repeats of the Moloney murine sarcoma virus, is digested with EcoRI and HindIII and subsequently treated with calf intestinal phosphatase. The linear vector is fractionated on agarose gel and purified, using glass beads.

The cDNA encoding a polypeptide of the present invention can be amplified using PCR primers which correspond to the 5' and 3' end sequences respectively as set forth in Example 1. Preferably, the 5' primer contains an EcoRI site and the 3' primer includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is then used to transform bacteria HB 101, which are then plated onto agar containing kanamycin for the purpose of confirming that the vector has the gene of interest properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells transduced with the vector. The packaging cells now produce infectious viral particles containing the gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether protein is being produced.

The engineered fibroblasts are then transplanted onto the host, either alone or after having been grown to confluence on cytodex 3 microcarrier beads.

It will be clear that the invention may be practiced otherwise than as particularly described in the foregoing description and examples. Numerous modifications and variations of the present invention are possible in light of the above teachings and, therefore, are within the scope of the appended claims.

The entire disclosure of each document cited (including patents, patent applications, journal articles, abstracts, laboratory manuals, books, or other disclosures) in the Background of the Invention, Detailed Description, and Examples is hereby incorporated herein by reference.

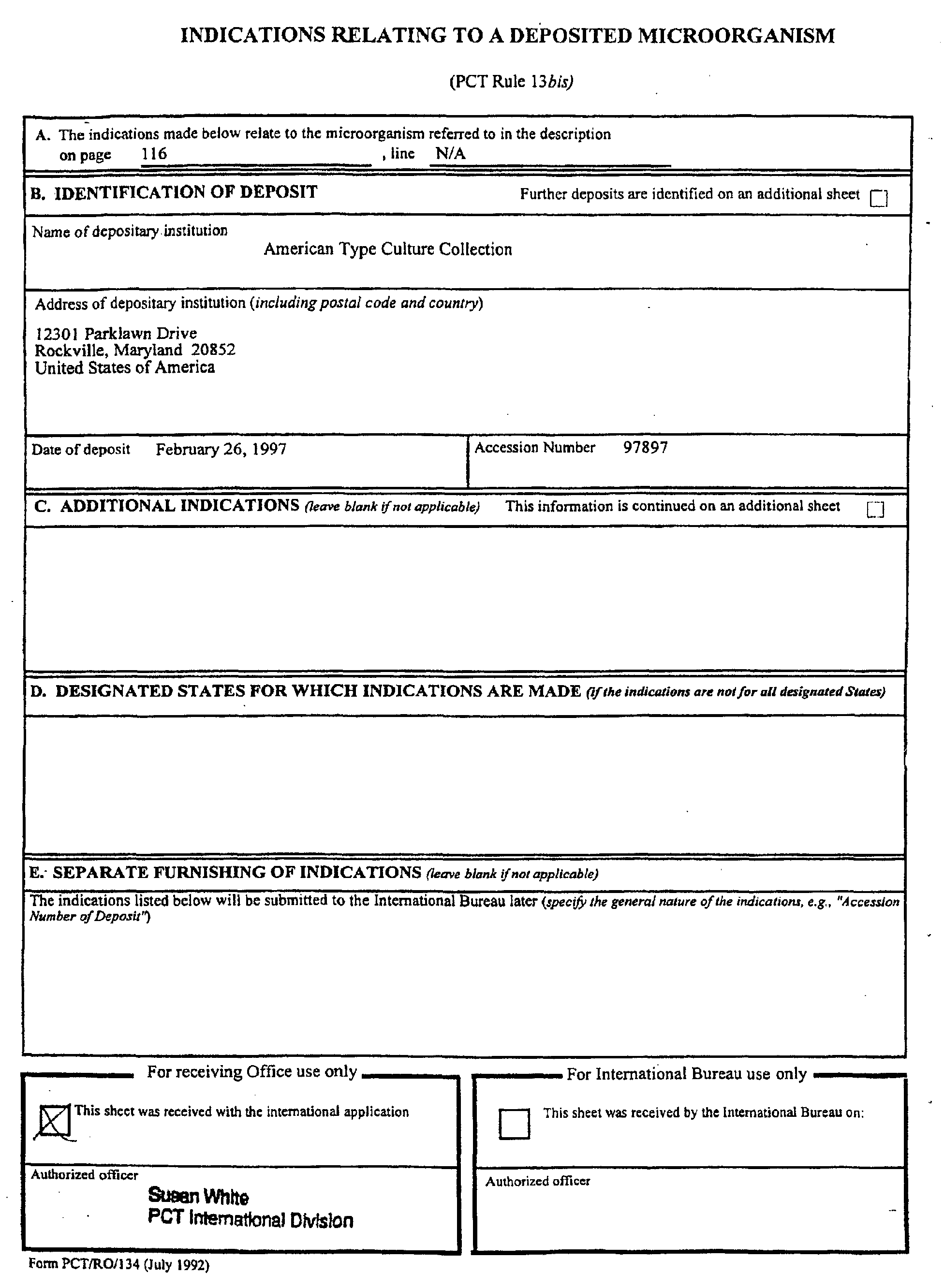

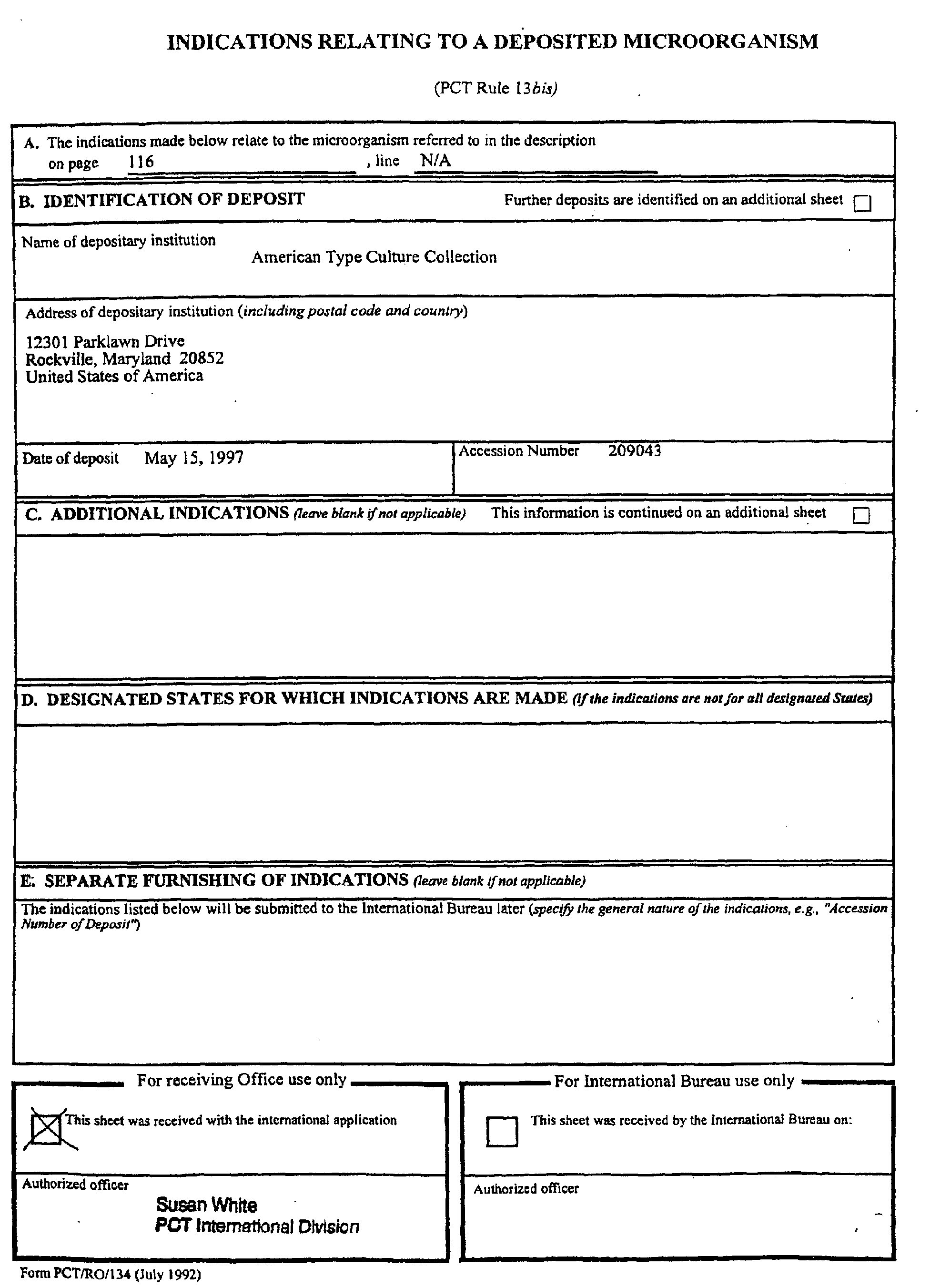

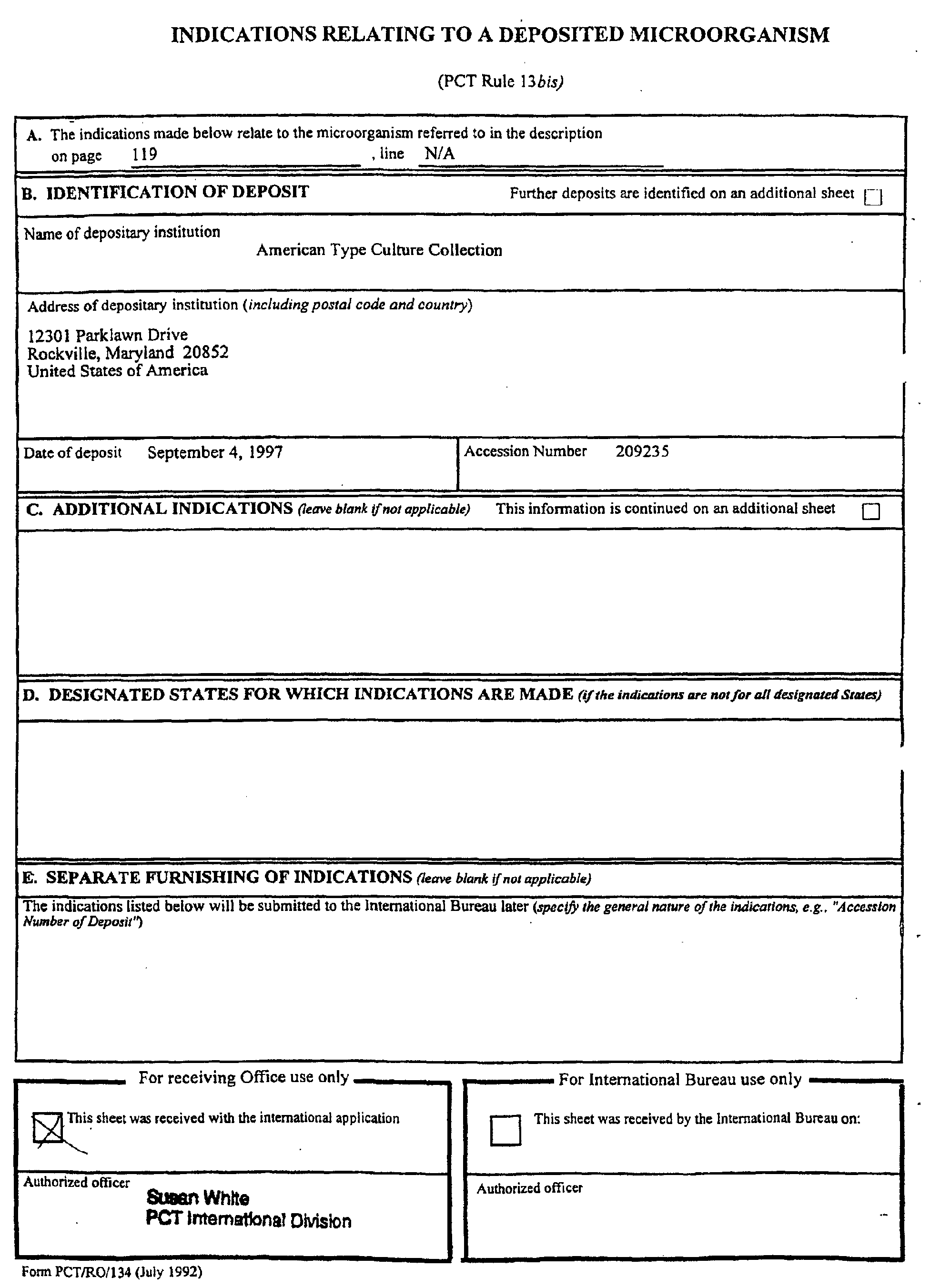

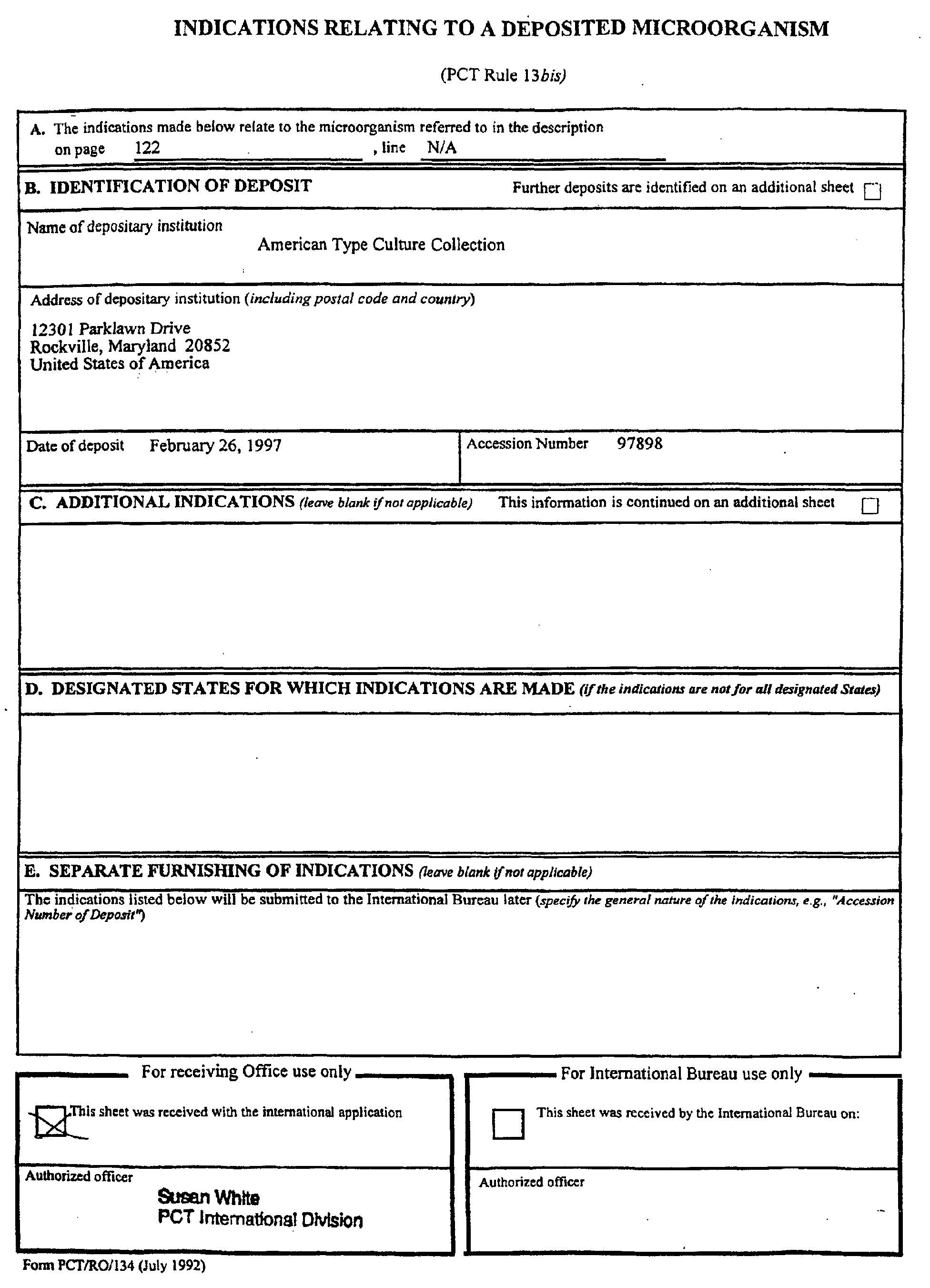

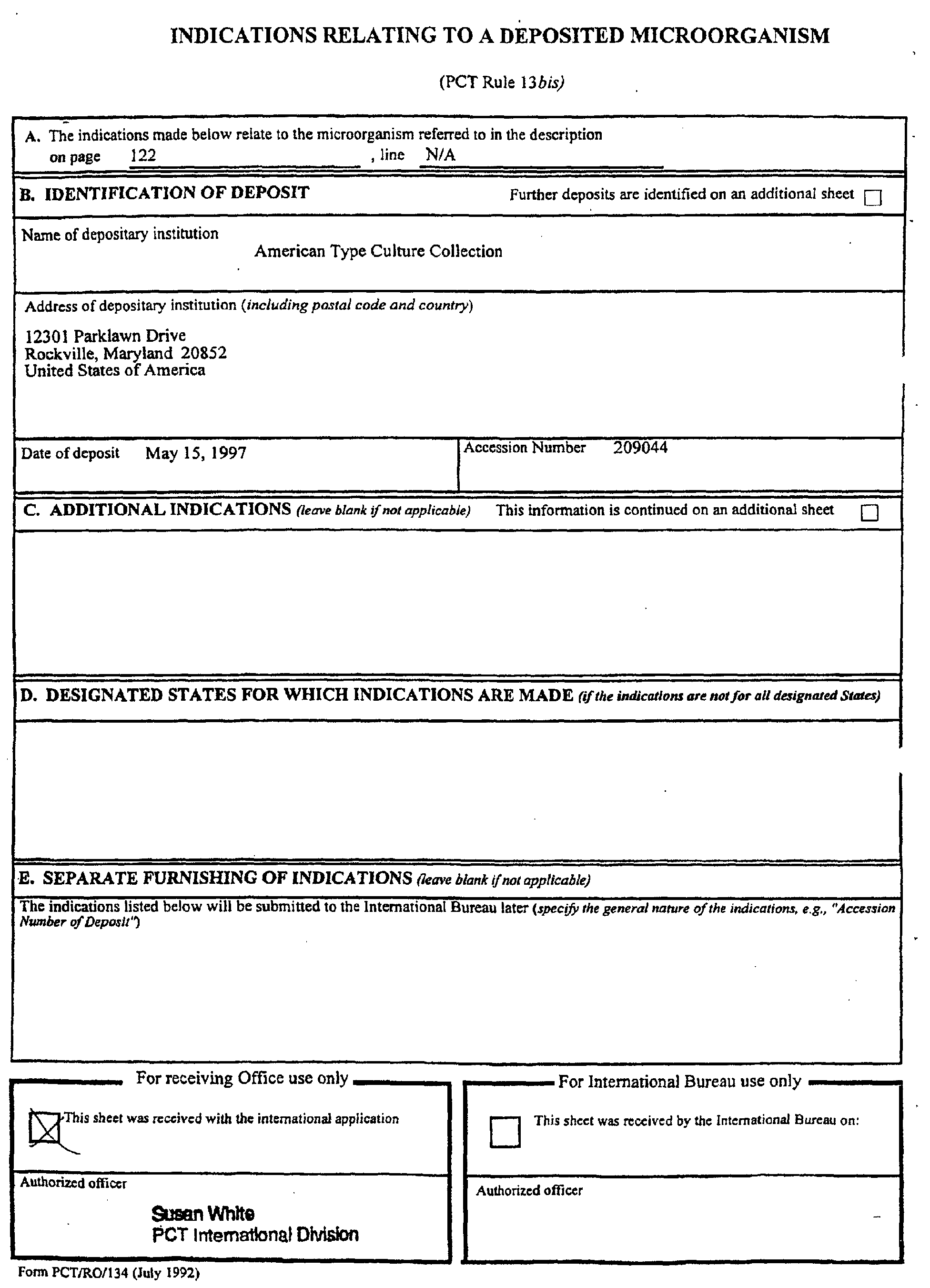

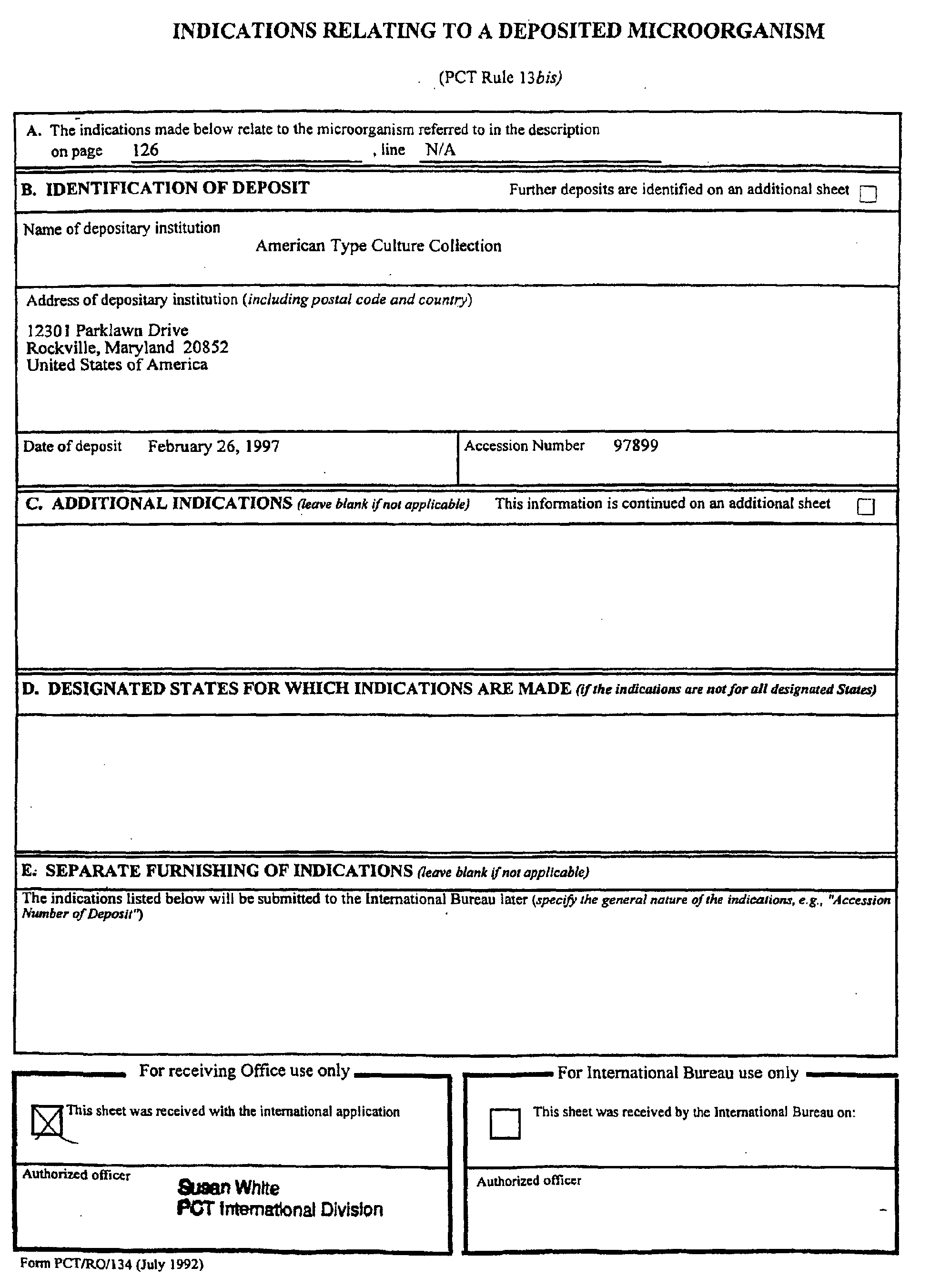

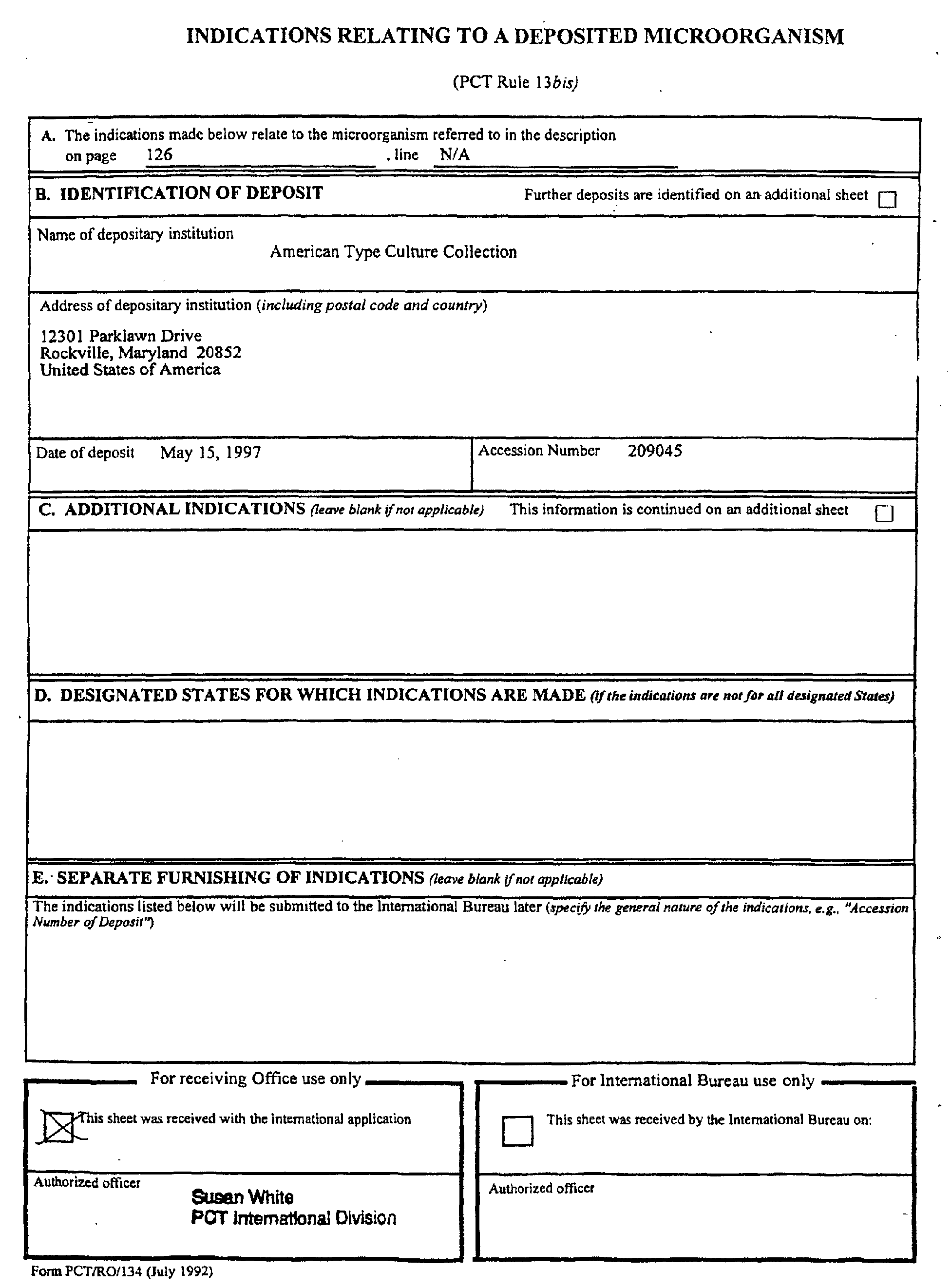

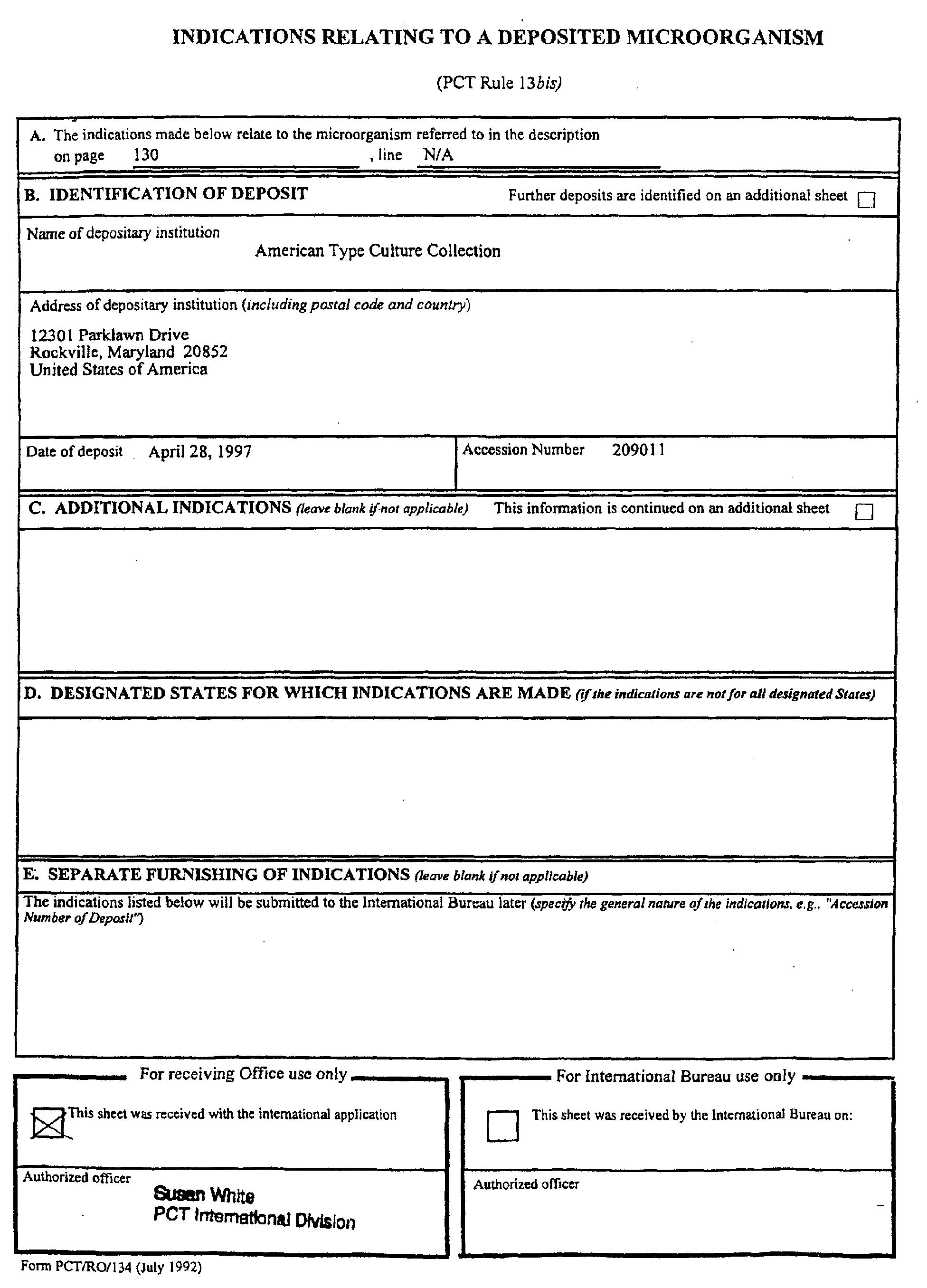

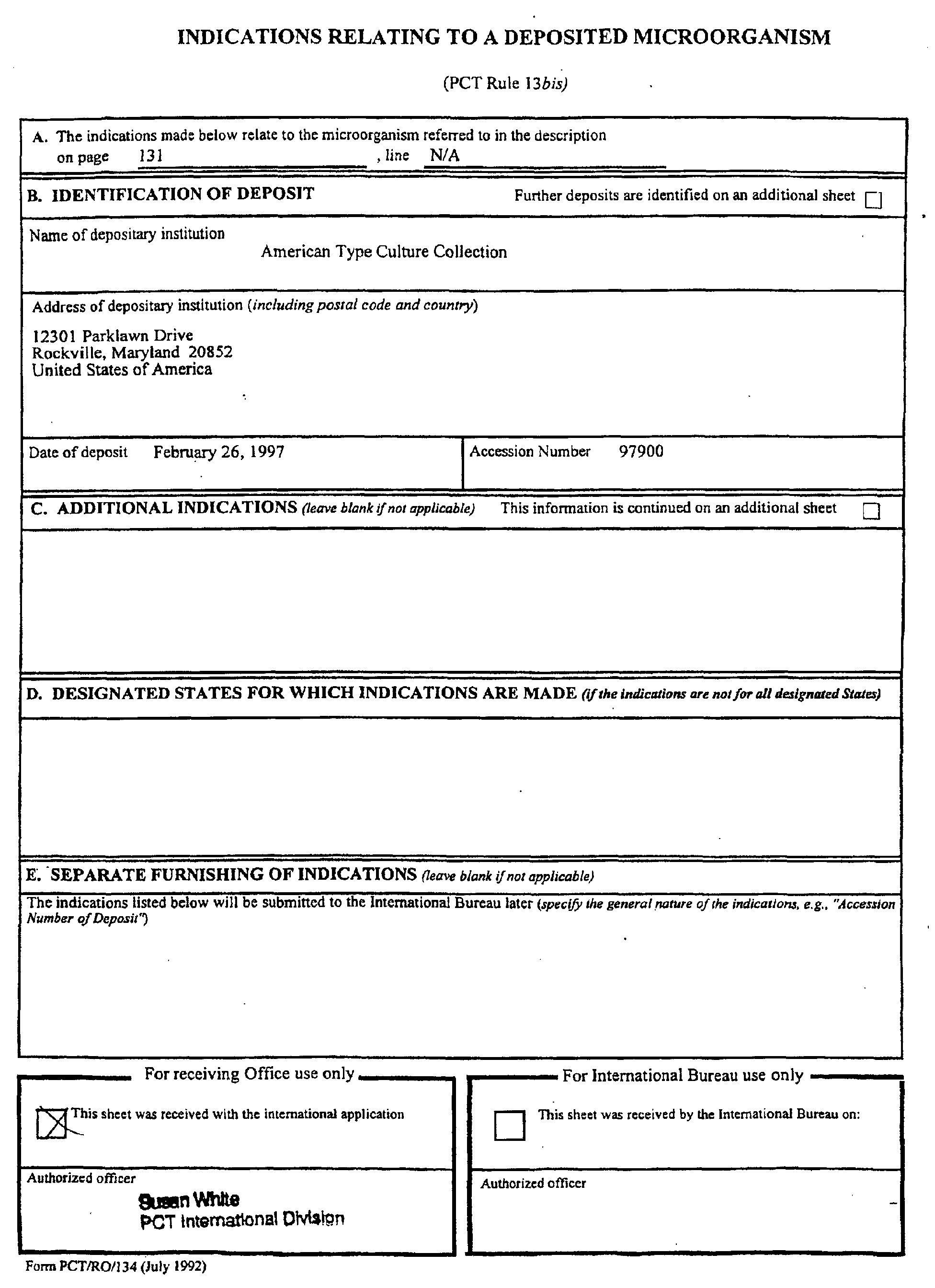

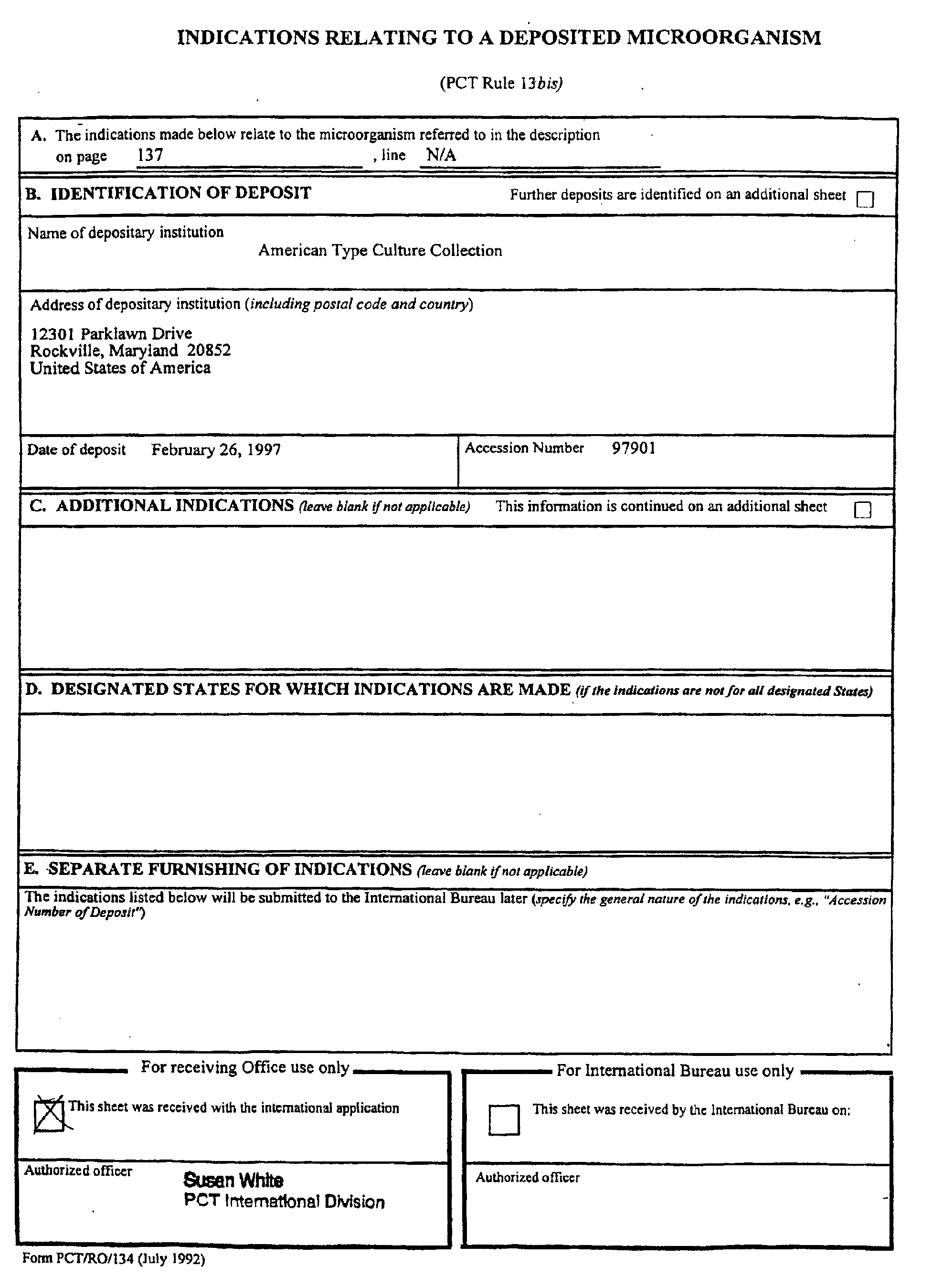

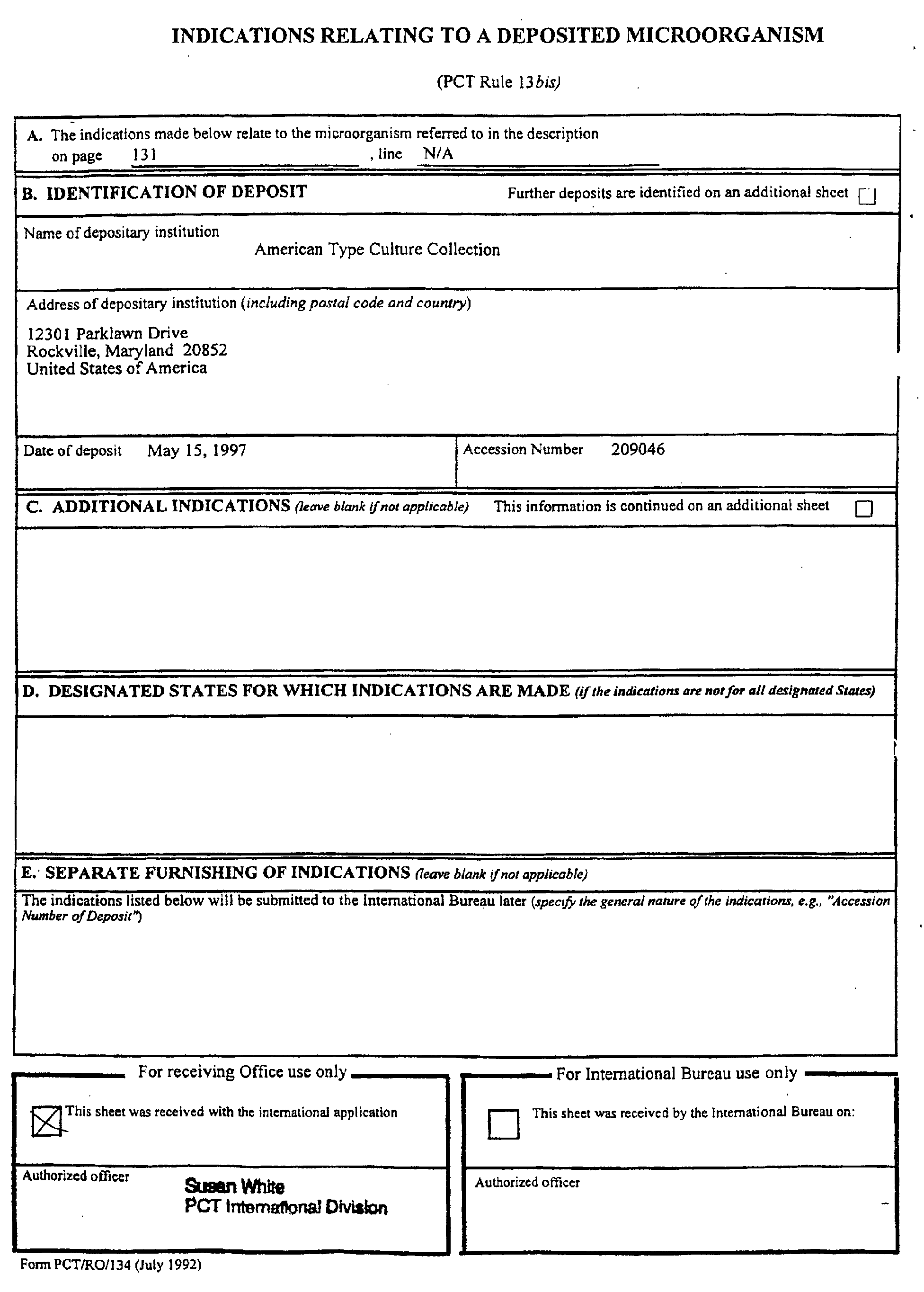

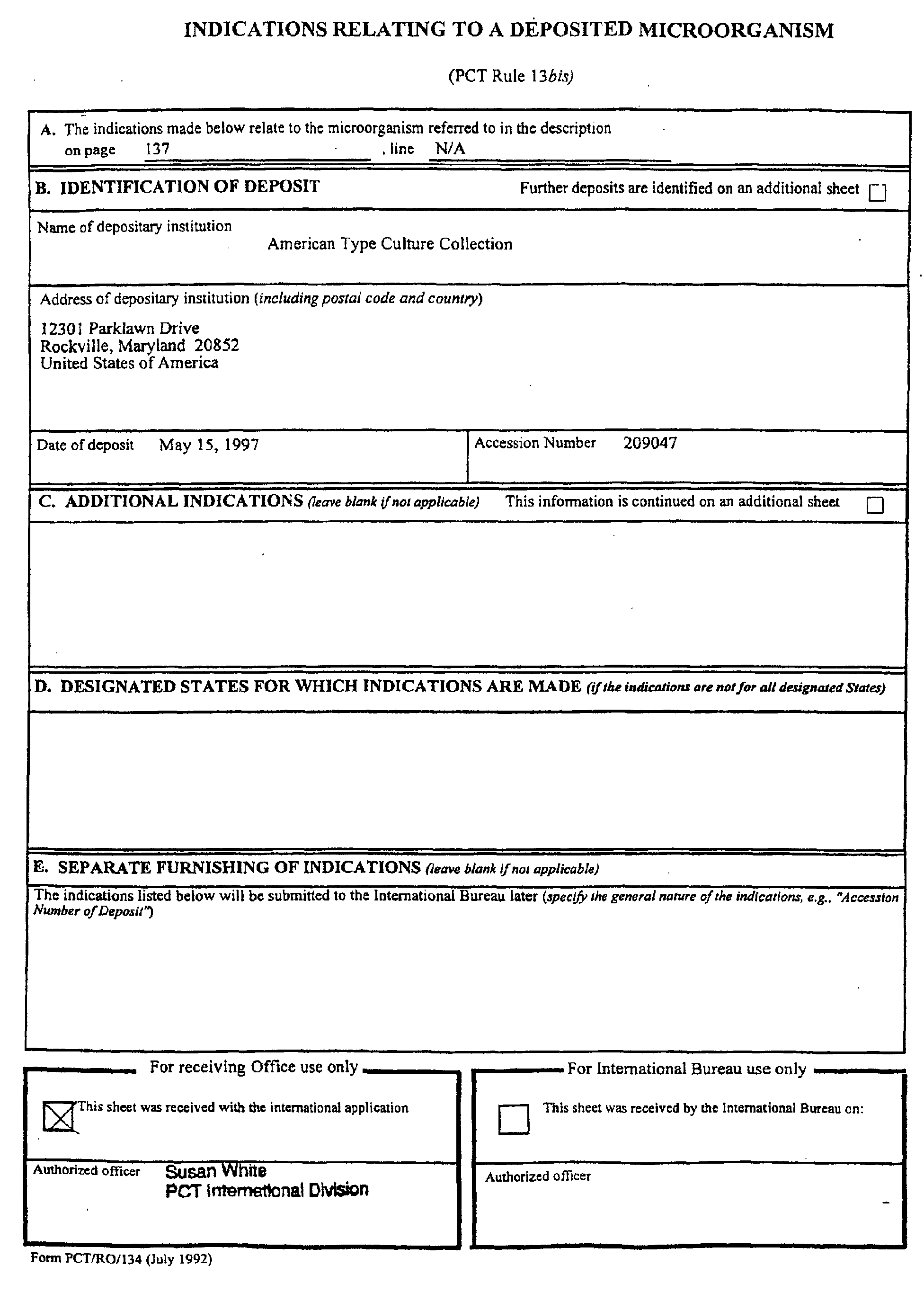

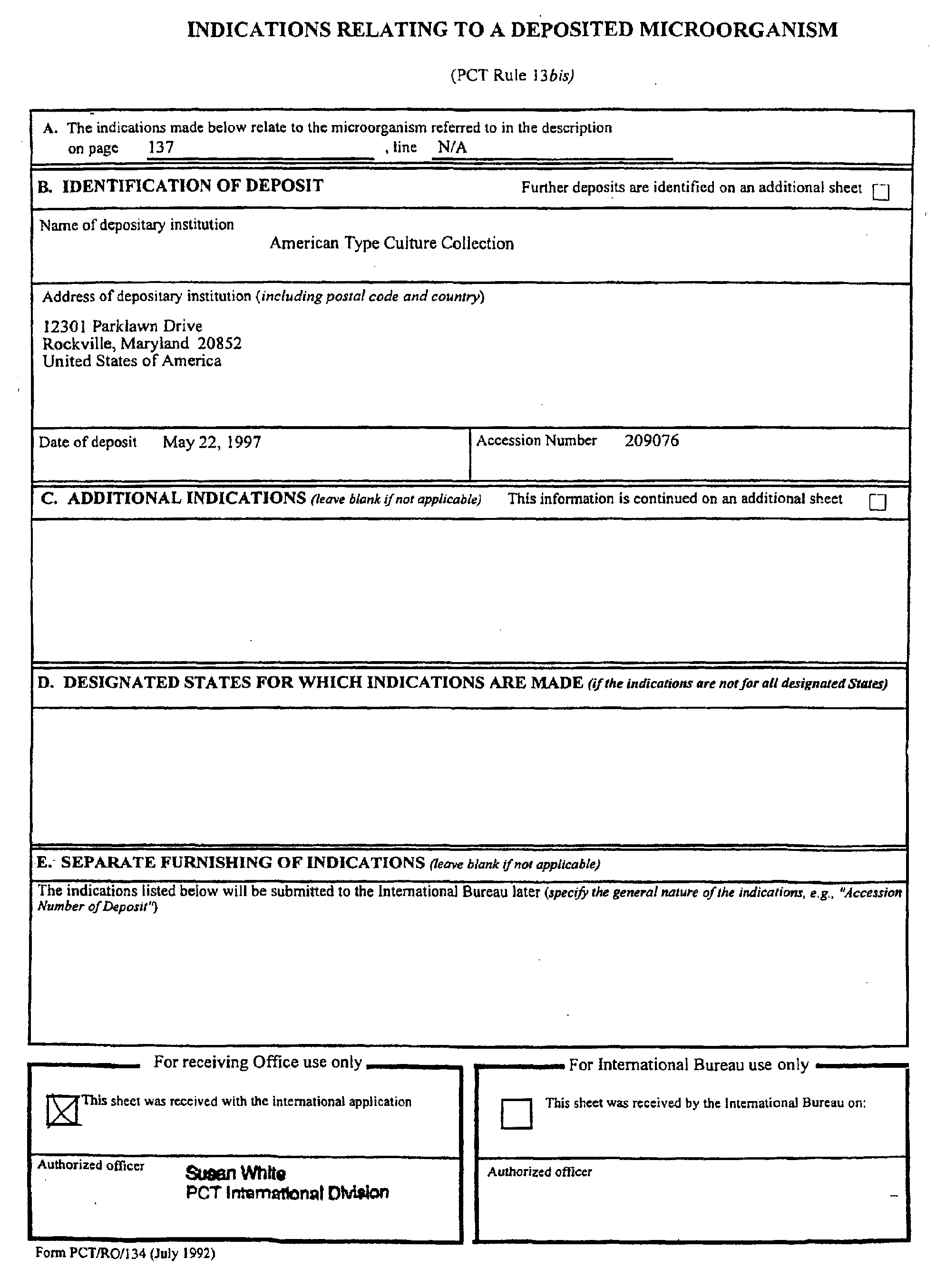

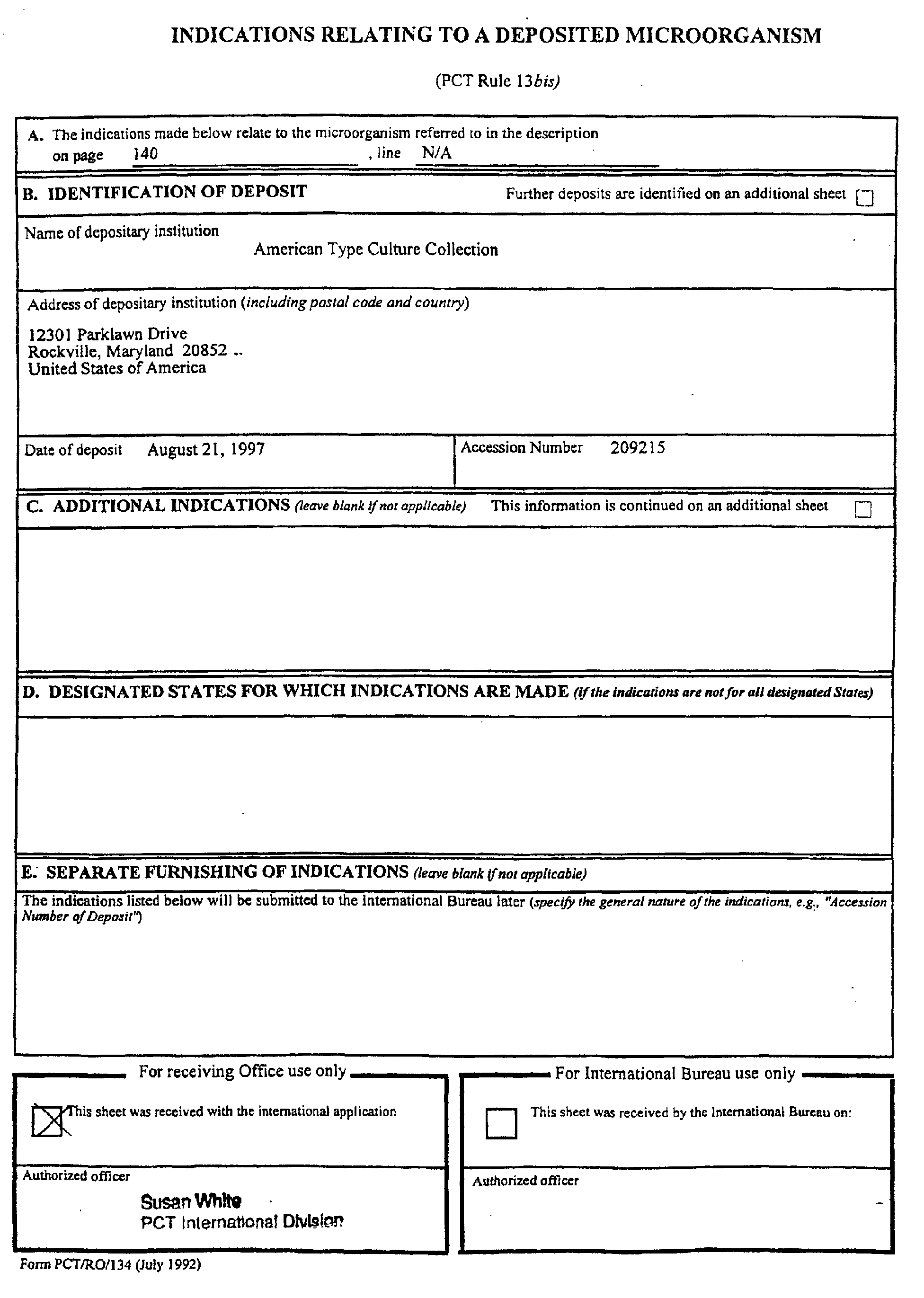

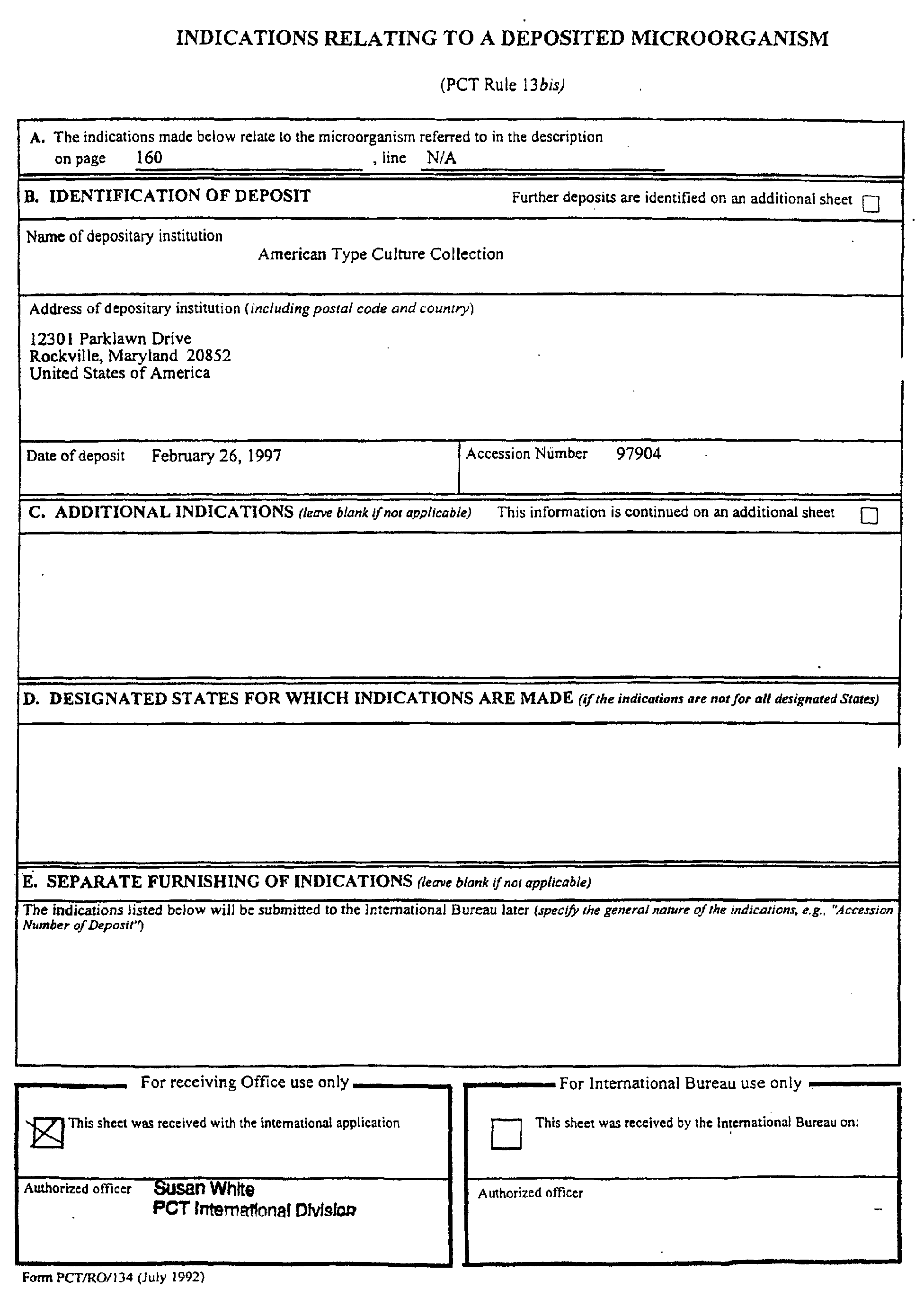

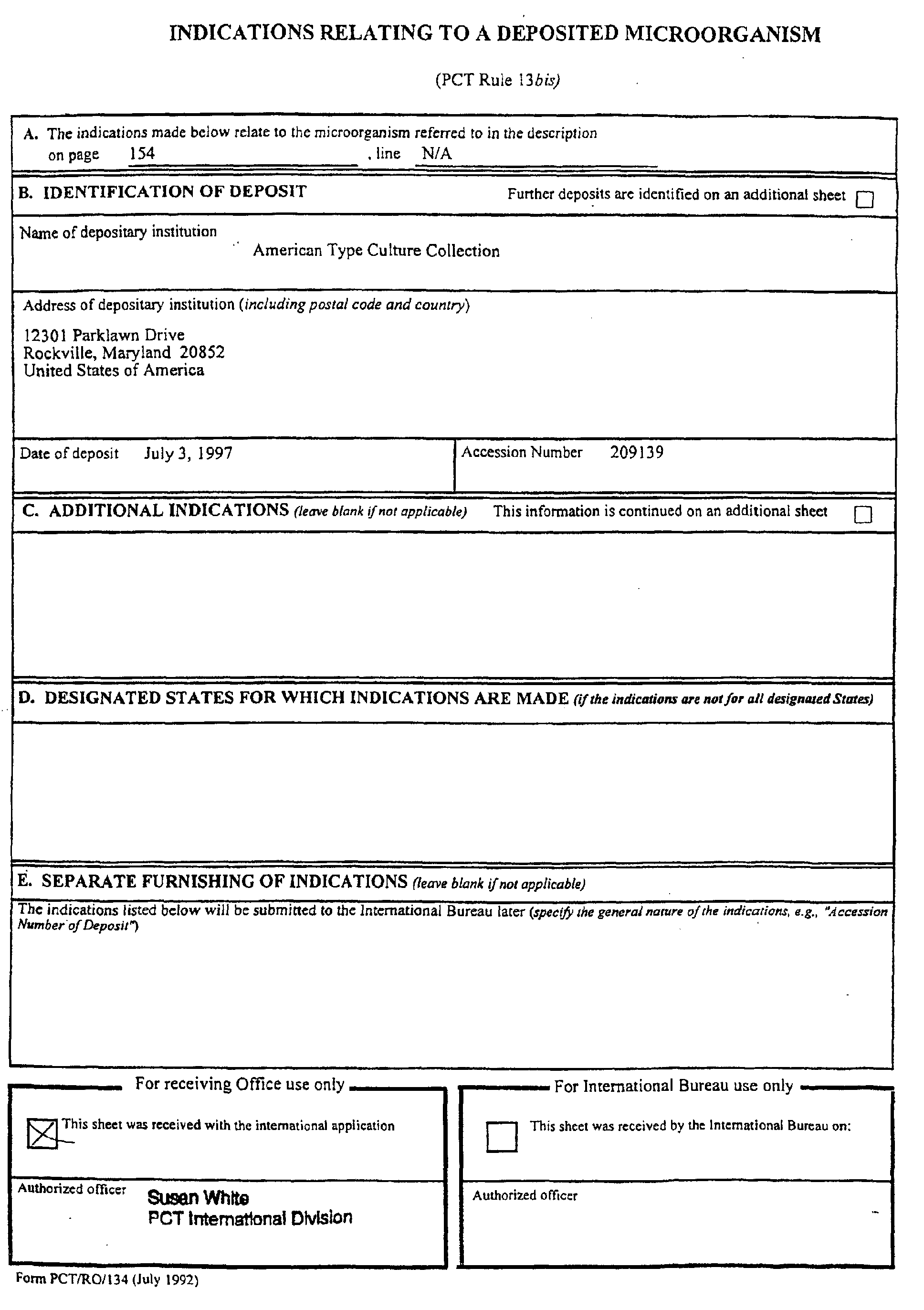

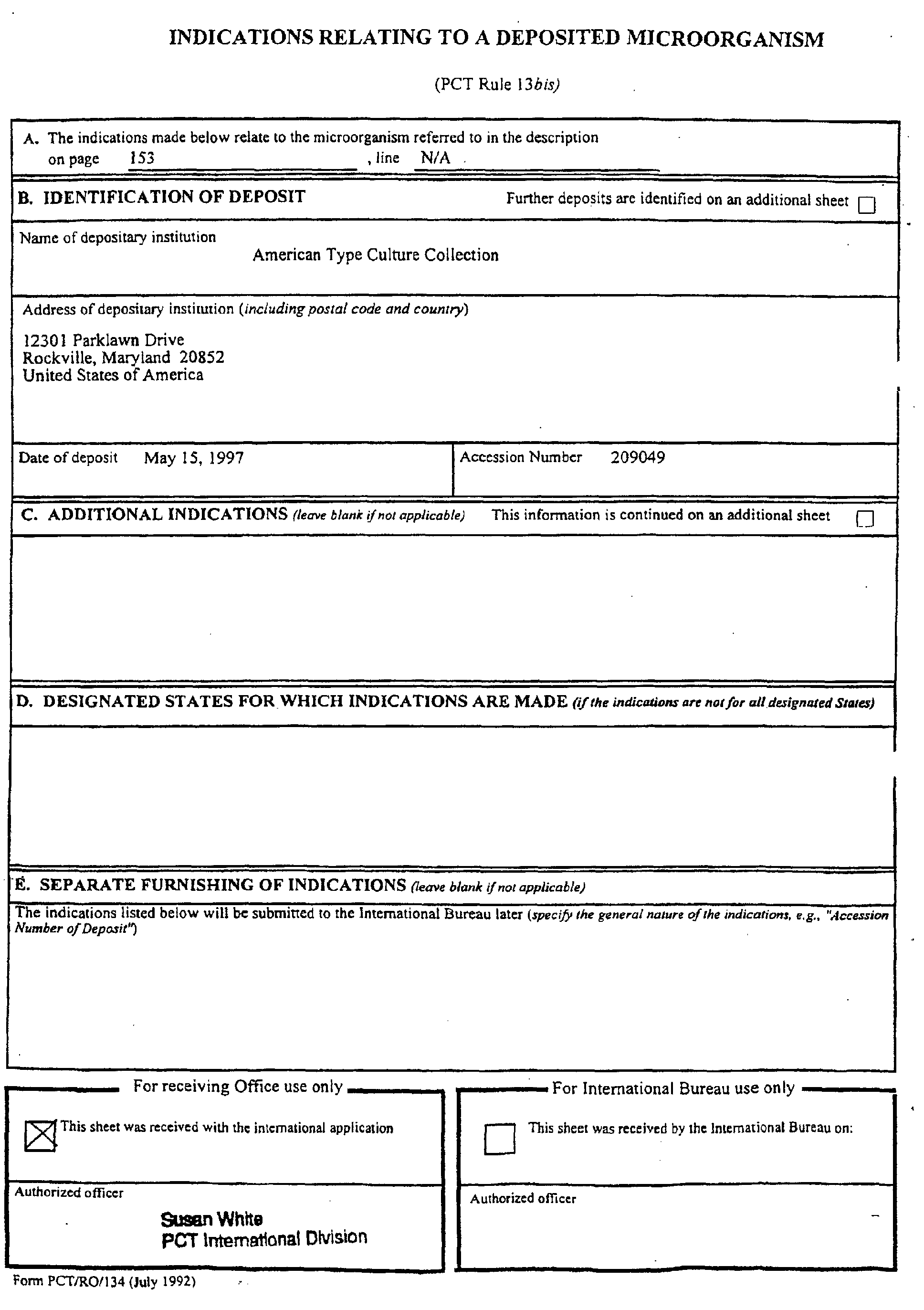

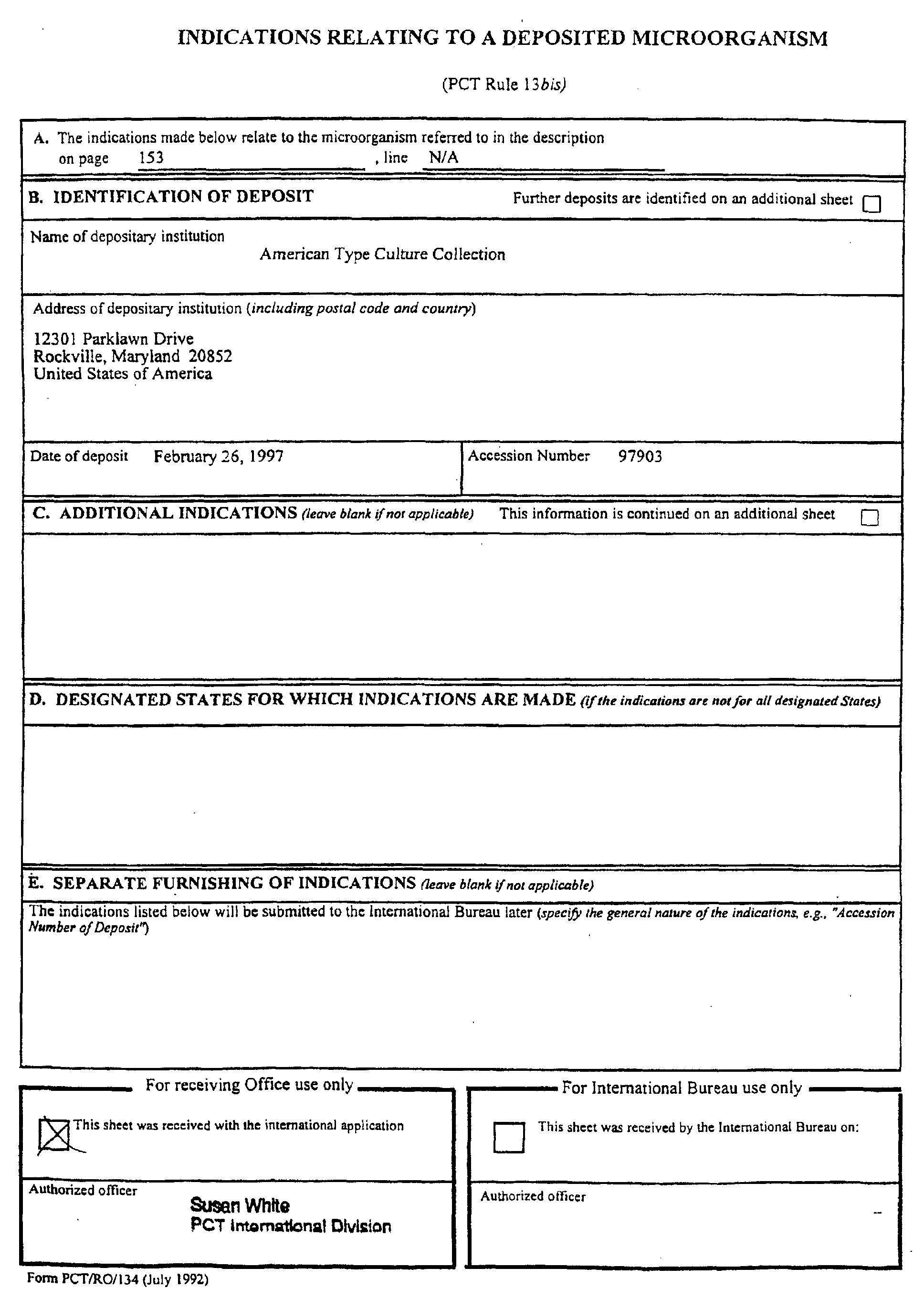

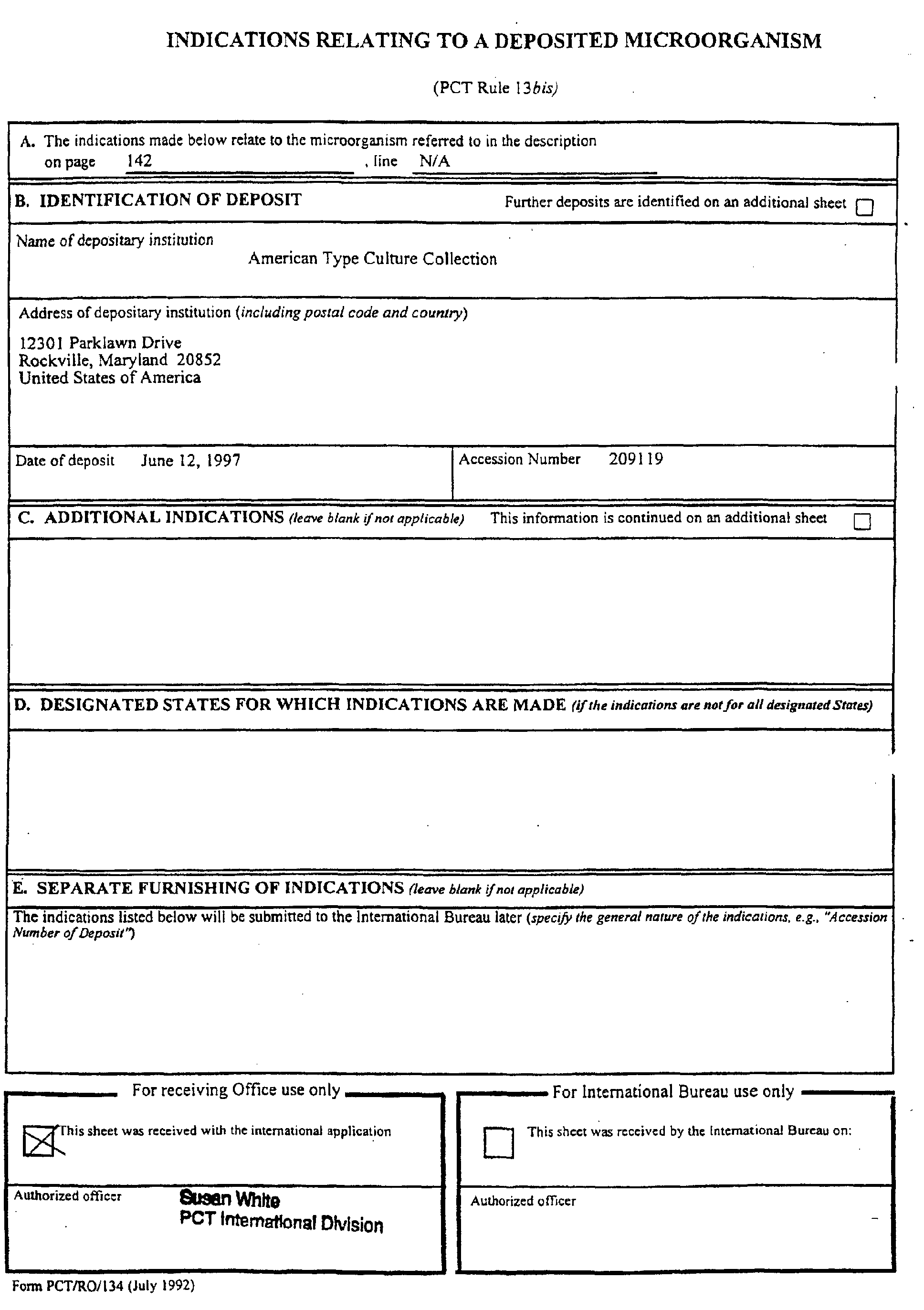

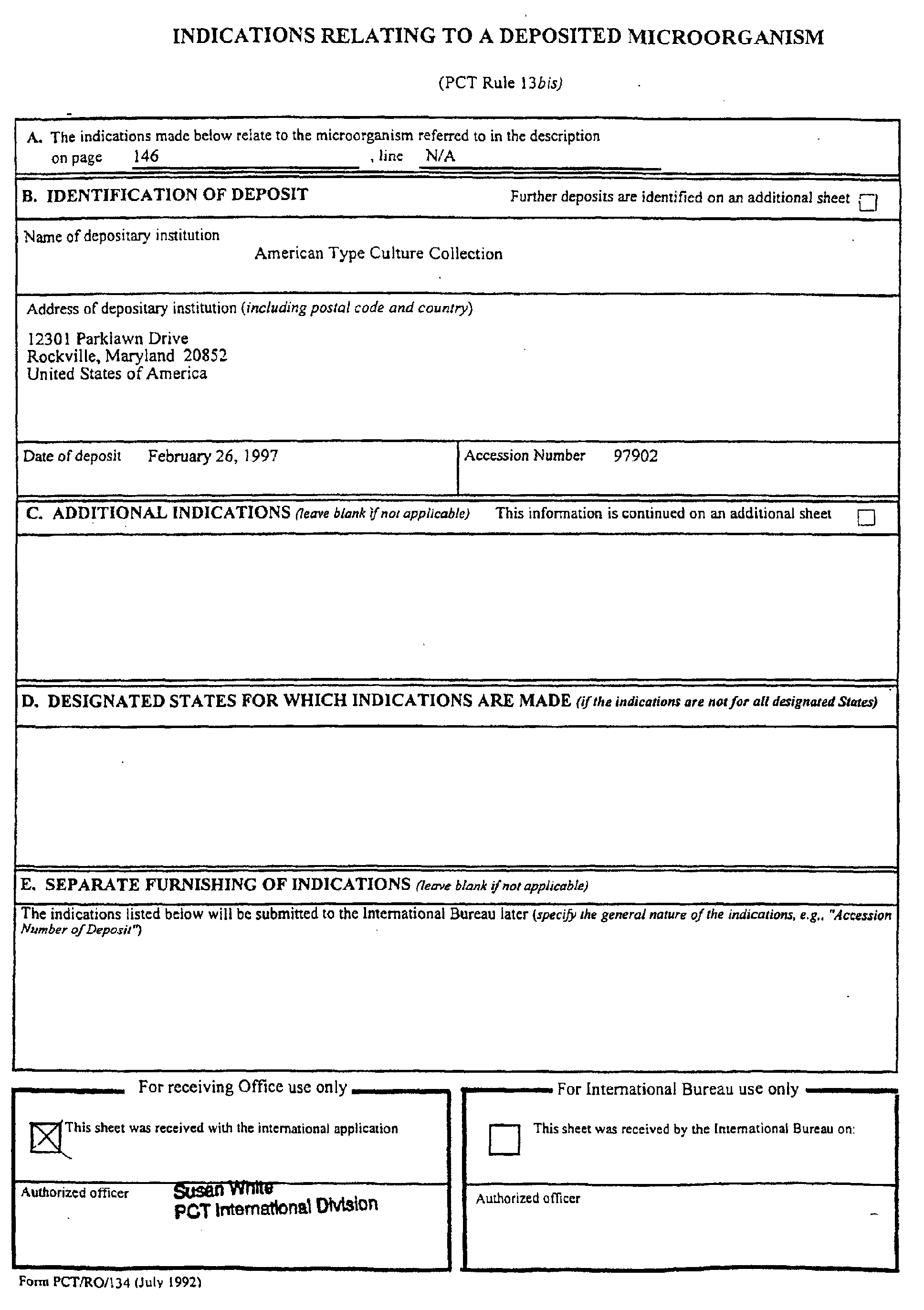

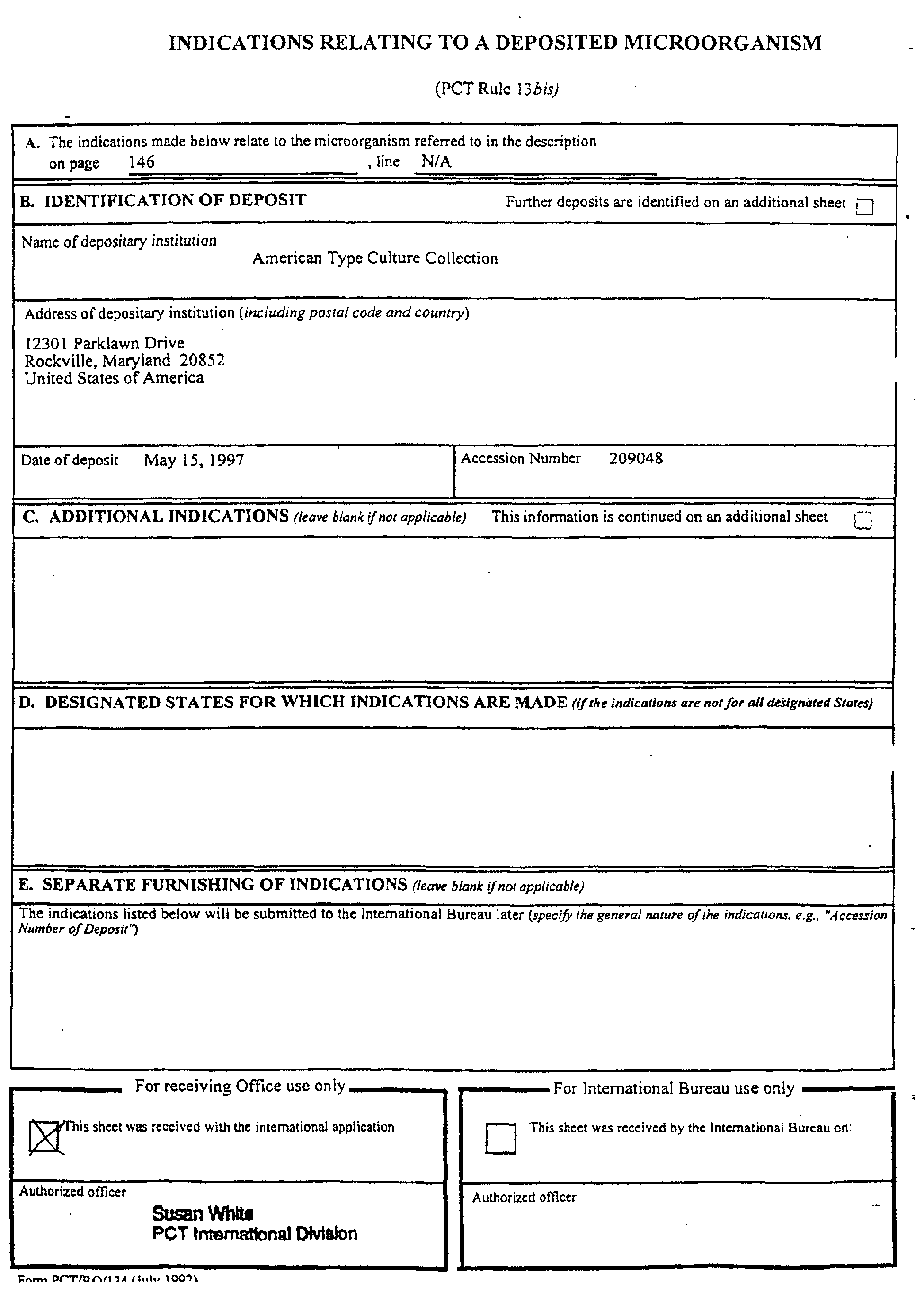

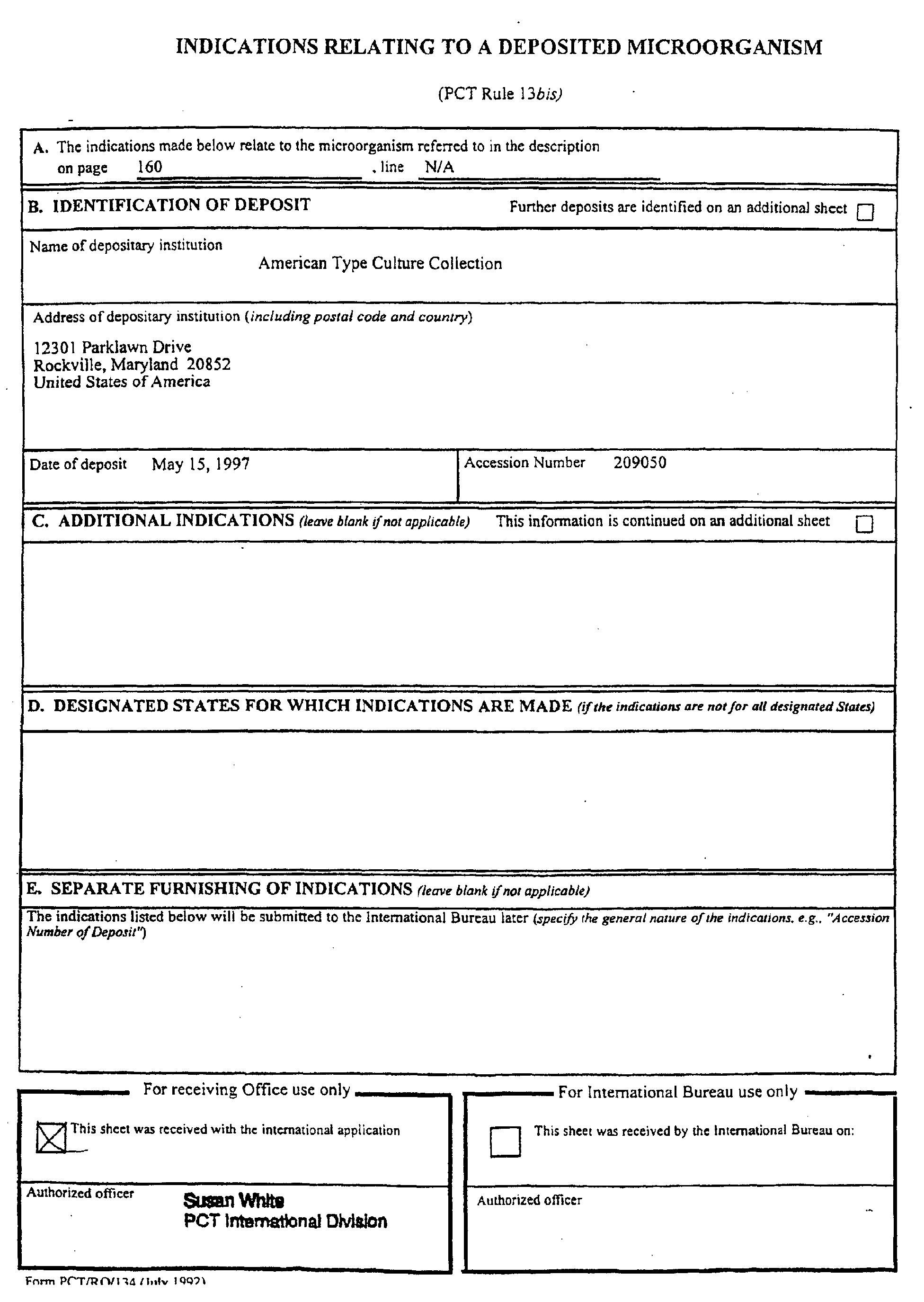

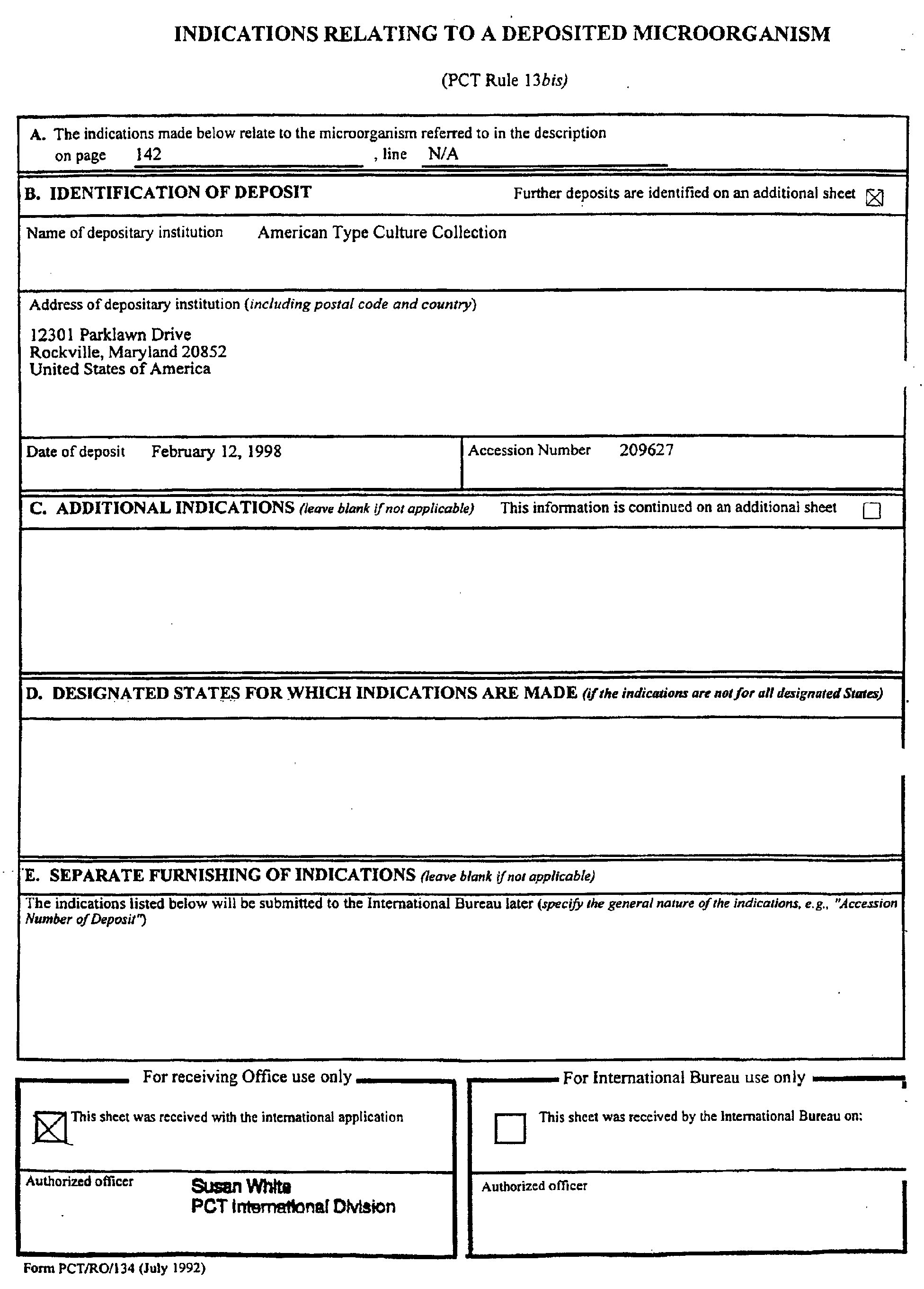

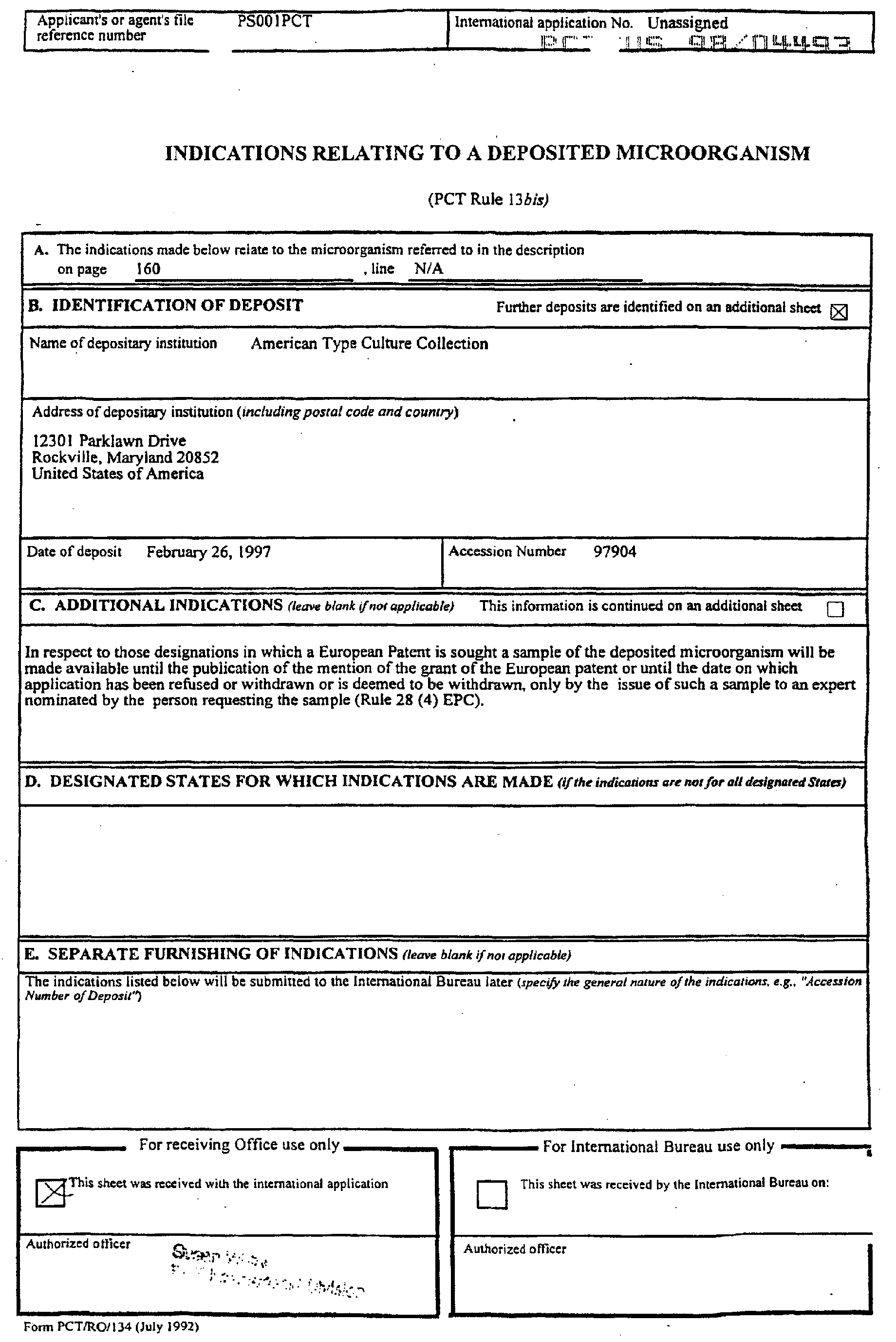

(1) GENERAL INFORMATION:

(i) APPLICANT: Human Genome Sciences, Inc. et al.

(ii) TITLE OF INVENTION: 186 Human Secreted Proteins

(iii) NUMBER OF SEQUENCES: 644

(iv) CORRESPONDENCE ADDRESS:

(A) ADDRESSEE: Human Genome Sciences, Inc.

(B) STREET: 9410 Key West Avenue

(C) CITY: Rockville

(D) STATE: Maryland

(E) COUNTRY: USA

(F) ZIP: 20850

(v) COMPUTER READABLE FORM:

(A) MEDIUM TYPE: Diskette, 3.50 inch, 1.4Mb storage

(B) COMPUTER: HP Vectra 486/33

(C) OPERATING SYSTEM: MSDOS version 6.2

(D) SOFTWARE: ASCII Text

(vi) CURRENT APPLICATION DATA:

(A) APPLICATION NUMBER:

(B) FILING DATE: March 6, 1998

(C) CLASSIFICATION:

(vii) PRIOR APPLICATION DATA:

(A) APPLICATION NUMBER:

(B) FILING DATE:

(viii) ATTORNEY/AGENT INFORMATION:

(A) NAME: A. Anders Brookes, Esq.

(B) REGISTRATION NUMBER: 36,373

(C) REFERENCE/DOCKET NUMBER: PS002.PCT

(vi) TELECOMMUNICATION INFORMATION:

(A) TELEPHONE: (301) 309-8504

(B) TELEFAX: (301) 309-8439

(2) INFORMATION FOR SEQ ID NO: 1:

(i) SEQUENCE CHARACTERISTICS:
(A) LENGTH: 733 base pairs
(B) TYPE: nucleic acid
(C) STRANDEDNESS: double
(D) TOPOLOGY: linear

(xi) SEQUENCE DESCRIPTION: SEQ ID NO: 1:

GGGATCCGGA GCCCAAATCT TCTGACAAAA CTCACACATG CCCACCGTGC CCAGCACCTG 60

AATTCGAGGG TGCACCGTCA GTCTTCCTCT TCCCCCCAAA ACCCAAGGAC ACCCTCATGA 120

TCTCCCGGAC TCCTGAGGTC ACATGCGTGG TGGTGGACGT AAGCCACGAA GACCCTGAGG 180

TCAAGTTCAA CTGGTACGTG GACGGCGTGG AGGTGCATAA TGCCAAGACA AAGCCGCGGG 240

AGGAGCAGTA CAACAGCACG TACCGTGTGG TCAGCGTCCT CACCGTCCTG CACCAGGACT 300

GGCTGAATGG CAAGGAGTAC AAGTGCAAGG TCTCCAACAA AGCCCTCCCA ACCCCCATCG 360

AGAAAACCAT CTCCAAAGCC AAAGGGCAGC CCCGAGAACC ACAGGTGTAC ACCCTGCCCC 420

CATCCCGGGA TGAGCTGACC AAGAACCAGG TCAGCCTGAC CTGCCTGGTC AAAGGCTTCT 480

ATCCAAGCGA CATCGCCGTG GAGTGGGAGA GCAATGGGCA GCCGGAGAAC AACTACAAGA 540

CCACGCCTCC CGTGCTGGAC TCCGACGGCT CCTTCTTCCT CTACAGCAAG CTCACCGTGG 600

ACAAGAGCAG GTGGCAGCAG GGGAACGTCT TCTCATGCTC CGTGATGCAT GAGGCTCTGC 660

ACAACCACTA CACGCAGAAG AGCCTCTCCC TGTCTCCGGG TAAATGAGTG CGACGGCCGC 720

GACTCTAGAG GAT 733

(2) INFORMATION FOR SEQ ID NO: 2:

(i) SEQUENCE CHARACTERISTICS:
(A) LENGTH: 5 amino acids
(B) TYPE: amino acid
(D) TOPOLOGY: linear

(xi) SEQUENCE DESCRIPTION: SEQ ID NO: 2:

Trp Ser Xaa Trp Ser
1               5

(2) INFORMATION FOR SEQ ID NO: 3:

(i) SEQUENCE CHARACTERISTICS:
(A) LENGTH: 86 base pairs
(B) TYPE: nucleic acid
(C) STRANDEDNESS: double
(D) TOPOLOGY: linear

(xi) SEQUENCE DESCRIPTION: SEQ ID NO: 3:

GCGCCTCGAG ATTTCCCCGA AATCTAGATT TCCCCGAAAT GATTTCCCCG AAATGATTTC 60

CCCGAAATAT CTGCCATCTC AATTAG 86

(2) INFORMATION FOR SEQ ID NO: 4:

(i) SEQUENCE CHARACTERISTICS:
(A) LENGTH: 27 base pairs
(B) TYPE: nucleic acid
(C) STRANDEDNESS: double
(D) TOPOLOGY: linear

(xi) SEQUENCE DESCRIPTION: SEQ ID NO: 4:

GCGGCAAGCT TTTTGCAAAG CCTAGGC 27

(2) INFORMATION FOR SEQ ID NO: 5:

(i) SEQUENCE CHARACTERISTICS:
(A) LENGTH: 271 base pairs
(B) TYPE: nucleic acid
(C) STRANDEDNESS: double
(D) TOPOLOGY: linear

(xi) SEQUENCE DESCRIPTION: SEQ ID NO: 5:

CTCGAGATTT CCCCGAAATC TAGATTTCCC CGAAATGATT TCCCCGAAAT GATTTCCCCG 60

AAATATCTGC CATCTCAATT AGTCAGCAAC CATAGTCCCG CCCCTAACTC CGCCCATCCC 120

GCCCCTAACT CCGCCCAGTT CCGCCCATTC TCCGCCCCAT GGCTGACTAA TTTTTTTTAT 180

TTATGCAGAG GCCGAGGCCG CCTCGGCCTC TGAGCTATTC CAGAAGTAGT GAGGAGGCTT 240

TTTTGGAGGC CTAGGCTTTT GCAAAAAGCT T 271

(2) INFORMATION FOR SEQ ID NO: 6:

(i) SEQUENCE CHARACTERISTICS:
(A) LENGTH: 32 base pairs
(B) TYPE: nucleic acid
(C) STRANDEDNESS: double
(D) TOPOLOGY: linear

(xi) SEQUENCE DESCRIPTION: SEQ ID NO: 6:

GCGCTCGAGG GATGACAGCG ATAGAACCCC GG 32

(2) INFORMATION FOR SEQ ID NO: 7:

(i) SEQUENCE CHARACTERISTICS:
(A) LENGTH: 31 base pairs
(B) TYPE: nucleic acid
(C) STRANDEDNESS: double
(D) TOPOLOGY: linear

(xi) SEQUENCE DESCRIPTION: SEQ ID NO: 7:

GCGAAGCTTC GCGACTCCCC GGATCCGCCT C 31

(2) INFORMATION FOR SEQ ID NO: 8:

(i) SEQUENCE CHARACTERISTICS:
(A) LENGTH: 12 base pairs
(B) TYPE: nucleic acid
(C) STRANDEDNESS: double
(D) TOPOLOGY: linear

(xi) SEQUENCE DESCRIPTION: SEQ ID NO: 8:

GGGGACTTTC CC 12

(2) INFORMATION FOR SEQ ID NO: 9:

(i) SEQUENCE CHARACTERISTICS:

(A) LENGTH: 73 base pairs
(B) TYPE: nucleic acid
(C) STRANDEDNESS: double
(D) TOPOLOGY: linear

(xi) SEQUENCE DESCRIPTION: SEQ ID NO: 9:

GCGGCCTCGA GGGGACTTTC CCGGGGACTT TCCGGGGACT TTCCGGGACT TTCCATCCTG 60

CCATCTCAAT TAG 73

(2) INFORMATION FOR SEQ ID NO: 10:

(i) SEQUENCE CHARACTERISTICS:
(A) LENGTH: 256 base pairs
(B) TYPE: nucleic acid
(C) STRANDEDNESS: double
(D) TOPOLOGY: linear

(xi) SEQUENCE DESCRIPTION: SEQ ID NO: 10:

CTCGAGGGGA CTTTCCCGGG GACTTTCCGG GGACTTTCCG GGACTTTCCA TCTGCCATCT 60

CAATTAGTCA GCAACCATAG TCCCGCCCCT AACTCCGCCC ATCCCGCCCC TAACTCCGCC 120

CAGTTCCGCC CATTCTCCGC CCCATGGCTG ACTAATTTTT TTTATTTATG CAGAGGCCGA 180

GGCCGCCTCG GCCTCTGAGC TATTCCAGAA GTAGTGAGGA GGCTTTTTTG GAGGCCTAGG 240

CTTTTGCAAA AAGCTT 256

(2) INFORMATION FOR SEQ ID NO: 11:

(i) SEQUENCE CHARACTERISTICS:
(A) LENGTH: 582 base pairs
(B) TYPE: nucleic acid
(C) STRANDEDNESS: double
(D) TOPOLOGY: linear

(xi) SEQUENCE DESCRIPTION: SEQ ID NO: 11:

GGCACGAGGT AATTTCTACC AGAAATTTCC AGAGCATTAT GTAGGTAGAA AAAAATGCAA 60

GCAAGCTGTT AAAGATCTTG GATCCCATTA TATAGTATGT ATAGCTGAAA TCTGTAATTC 120

AATCACTTTT TCTCTTTTAT CCTCTAACCA AAAAATTGTT TAATTTTGCA TCCCAAATGT 180

TTTTAATCTT TGTATATTTT TTAAAAATCC TTTTCTCCTC ATCATTGCCT TTTTTGTGGT 240

TGTAAATAGA CTTACTTGCA CTTTGAAGAT GAGTTACTCC TTGTCATCTT ACAAATATGT 300

GATATGGTAA TTTTCATAAC AGATGTCAGT TTTGAACCAA GAATTGGTGA TTTGTTTATA 360

AGAAAAAAAC TGGCTTCATT TCTGTGAAAT TGCTCTTTGA AAATTTCTTT TTACACGTGT 420

AAGCCAACTG AGATACCGTG ATGGTGTTGA TTTCTTTCAA TGATGCTTAC CATCTATTTT 480

AGCCACTGAG CCTTTTATTA TTTGTCTATT TGTAAAGTTT ATTTGTCTTA ACTCATTTAA 540

TAAATATACT GTTTATCTGT TTCTGAAAAA AAAAAAAAAA AA 582

(2) INFORMATION FOR SEQ ID NO: 12:

(i) SEQUENCE CHARACTERISTICS:
(A) LENGTH: 465 base pairs
(B) TYPE: nucleic acid
(C) STRANDEDNESS: double
(D) TOPOLOGY: linear

(xi) SEQUENCE DESCRIPTION: SEQ ID NO: 12:

GTTTGGGGGT GAGGCCGAGC TGCTGCGGGG CTTCGTCGCC GGCCAGGACA CAGCTACTCG 60

CACGGCGGCG GCGCCTGGCT ATGATGTTCC TCACCCAGGG CGGGCCTCTG CCCTCTACTC 120

GTGCCAGGCC CACTTGCCAG GCAGGAGCCC TCCCCAAGCC TTCAGGGCTG CTCGGAGTCA 180

CCTGTTGGAA TGGACTAAAA GGACCCTTGT GTGGGAACAG GTGCTCCCCA AACACCCTGC 240

TGCTGGCTGC CAGGCAGGCC CTCTGGAAGG GAAGGGGCAG GACTCATCAG GACCTCCCTG 300

GACCCCTGCA GGGCAGGCAG CTTGGGCCCG AGCCCAAGCA TTTGGCTCTG CTGCCCCCAA 360

GGGGACAGGA AGCCTCTTGG GCCTCTTCCC TTCCTGGACA AGGCCCCCTG CCTTTGCCTC 420

ACATAAACTG TACAGTATTT TCATTAAAAG CCTCTTTCAT AAAAA 465

(2) INFORMATION FOR SEQ ID NO: 13:

(i) SEQUENCE CHARACTERISTICS:
(A) LENGTH: 474 base pairs
(B) TYPE: nucleic acid
(C) STRANDEDNESS: double
(D) TOPOLOGY: linear

(xi) SEQUENCE DESCRIPTION: SEQ ID NO: 13:

ATGCAATTCC TGCTCACAGC CTTTCTGTTG GTGCCACTTC TGGCTCTTTG TGATGTCCCC 60

ATATCCCTAG GCTTCTCCCC CTCCTAGAAG GGCTTCTTGA TAGATTAGAA AATAAGAATG 120

AGTGACATTT CCTATGTGCA TATAAGAAGG AGCCACAAGA CATGTCTTTT AAATAAAAGG 180

ACAGTGTCCA TCCTTTTAGC TGCCGAATAG AACCTTGGTC TCATCCTCCT GGAGCTAGGC 240

CTTTAAAACA GCTTCTGTGT TTCTCATTTG TCTCAGTGTT TTGCCAGGGT TTTATCGGAA 300

AGATAATGTT CCGTTTAAAA TATTTCCTAA TGAGGCCGGG CGTGGTGGCT CACGCCTGTA 360

ACCCTAGCAM TTGGGGGCTG AGCGGGTGGA TCACGAGGTC AGGAGATCGA GACCATCCTG 420

GSTAACATGG TGAAACCCCG TCTCTACTAA AAATACAAAA AAAAAAAAAA AAAA 474

(2) INFORMATION FOR SEQ ID NO: 14:

(i) SEQUENCE CHARACTERISTICS:
(A) LENGTH: 314 base pairs
(B) TYPE: nucleic acid
(C) STRANDEDNESS: double
(D) TOPOLOGY: linear

(xi) SEQUENCE DESCRIPTION: SEQ ID NO: 14:

TTATGTTGGG GAGCAAGACC TGATAGCCAG CCTTTACATG GGAGTATAAT TCTGTCCTCC 60

ATCTCATAAG CCCCAGTACC TGAGCCAGAA TGATTATAAC CAACCACACT GTCTCTTTAT 120

CATGGATGGC TTTAGCAGTA GGTTATTTTC ATCATTGCCA TTTGTAGCTC TACAGTGGTT 180

TATAGTAATT TCTCATCTTT TAAGTCTCTC CCTCAGTGCC TGTTGTTATC AAACTCATTG 240

CTCTCTCANG CAGTTGAGCT CTGCATTCTC CCYTATGGGG GAGAGCTGTG TTGGAGAGAG 300

AGAATATNAC TTCC 314

(2) INFORMATION FOR SEQ ID NO: 15:

(i) SEQUENCE CHARACTERISTICS:
(A) LENGTH: 613 base pairs
(B) TYPE: nucleic acid
(C) STRANDEDNESS: double
(D) TOPOLOGY: linear

(xi) SEQUENCE DESCRIPTION: SEQ ID NO: 15:

CTCATATTGC CGTCTGGCTA AAAGTGAACA TGCCATTGAT CAATCTGCTT TTATTATATT 60

ATGTTCCTAA TGGTGGCAAG CAAGACAAGA AGTAGAAAGA AAGATGGTGT AAGCTCAAGA 120

ACCCACTAAA TCTATCCTAT GGCCTGGGTT CACCCAGCCT GCTTTGTGGA TTTTGTCTCA 180

CTATAACAGA GCTCCCAAGG AGACTGCAGA GTCAGCTCCC TTAAGCACTG TAACTAAAGC 240

CTAACTCTTC CGTTCCACCC AACAATGTYC CCAGCTCATC CTCTTTCCCR AAGTCCCCTT 300

TCTGCCCCAG ATGCGAATTG CATTTAACTA ATCCTCAAGT GAAATGTCCA CACAGRATTC 360

CATTTTAATT AGCATACCAT AGTTTTTGTG CAAATTTGCT TTCAGARGAC TCCCATTGCA 420

GCTGCTCAGA GACGCTAAWG GCAGGGCCTC TTGAWGCTTT CCCGATAGCT TTCAGCTGCA 480

ATAGCTCTTA GGCAGAATGC CATGAGCGTC CTGCCCAACT GTATTACTGG GGAACACCTG 540

ATTGGCTAGA AGTTGATCCT CCTGTAACTT TTCTGAGTTC TTTACATTTA CTCGTGAAAC 600

CCAAATATGC CAC 613

(2) INFORMATION FOR SEQ ID NO: 16:

(i) SEQUENCE CHARACTERISTICS:
(A) LENGTH: 356 base pairs
(B) TYPE: nucleic acid
(C) STRANDEDNESS: double
(D) TOPOLOGY: linear

(xi) SEQUENCE DESCRIPTION: SEQ ID NO: 16:

CCCCCCCCAT TGAACCCTGG GCTGTGAAAG TTTTTGCCTG TGTGGGTCGT TCTGTGTGGC 60

GCCTGGTGTG TGGKTCCCAA CTCCTGTTGC AAAGTGGCAG CAGCCAATCA TGAAGCGCCC 120

TTATTTTTAG TTGCAGATGA CCAGGTCTCC CCCCCACAGC CTCTGTCTGG TCCCTCATTG 180

GTGAGTGGTC TGCCTGCCCA AGGAGCCTGA TTGGTGGGAA ATGGCATCAT CTAATATGAT 240

GGGAAGGCAT TTGGTCCTGG TTATGTTTAT TACAACATCA TTGCACTCTG GGACTCCAGT 300

CCCTGAAAAC GTAATTTGTG GTGTTACCAA AGGACCACAG GGGAAAAAAA AAAAAA 356

(2) INFORMATION FOR SEQ ID NO: 17:

(i) SEQUENCE CHARACTERISTICS:
(A) LENGTH: 414 base pairs
(B) TYPE: nucleic acid
(C) STRANDEDNESS: double
(D) TOPOLOGY: linear

(xi) SEQUENCE DESCRIPTION: SEQ ID NO: 17:

GAAACTANAT CCCGGGGCTT TTAACNGGTA CTTGGGAAAT AAGTATTGGG TAATCACTAA 60

GNGGACATTG ACTGCACCAA ACCAAAGCTA TAGAAAGAAA TGATTGACTT TTTAAAATAT 120

ATTCACATTA ACTGTCCTAG GATACTTCTC TTGAGGCTTT GGAAAACTTC TTCCTTGAAA 180

TTTGCATATC CACTCCAGTT CTGTCACCAA AGATTTTAAT CTTCAGATCG CAATTTCCTC 240

TCTCCCAGAA AAAAGTACTA CAACAGGCTC AAGGGATATG CTTTGGTGGT CAAGGGATTA 300

CACTATGGTT TTCCTTCTGT TCACAATGGT ATTTACAGGA GACCTTGTCA TCAGAGGACG 360

TACTGAACTA TCTTTATGAC TTTGGATTTG ATCAGAGGTT TAAAAAAAAA AAAA 414

(2) INFORMATION FOR SEQ ID NO: 18:

(i) SEQUENCE CHARACTERISTICS:
(A) LENGTH: 469 base pairs
(B) TYPE: nucleic acid
(C) STRANDEDNESS: double
(D) TOPOLOGY: linear

(xi) SEQUENCE DESCRIPTION: SEQ ID NO: 18:

AATCACCATT GCAATACAAA TGATCTGCCT GGTGAATGYT GAGCTGTACC CCACATTCGT 60

CAGGAACYTC GGAGTGATGG TGTGTTCCTC CCTGTGTGAC ATAGGTGGGA TAATCACCCC 120

CTTCATAGTC TTCAGGCTGA GGGAGGTCTG GCAAGCCTTG CCCCTCATTT TGTTTGCGGT 180

GTTGGGCCTG CTTGCCGCGG GAGTGACGCT ACTTCTTCCA GAGACCAAGG GGGTCGCTTT 240

GCCAGAGACC ATGAAGGACG CCGAGAACCT TGGGAGAAAA GCAAAGCCCA AAGAAAACAC 300

GATTTACCTT AAGGTCCAAA CCTCAGAACC CTCGGGCACC TGAGAGAGAT GTTTTGCGGC 360

GATGTCGTGT TGGAGGGATG AAGATGGAGT TATCCTCTGC AGAAATTCCT AGACGCCTTC 420

ACTTCTCTGT ATTCTTCCTC ATACTTGCCT ACCCCCAAAT TAATATCAG 469

(2) INFORMATION FOR SEQ ID NO: 19:

(i) SEQUENCE CHARACTERISTICS:
(A) LENGTH: 550 base pairs
(B) TYPE: nucleic acid
(C) STRANDEDNESS: double
(D) TOPOLOGY: linear

(xi) SEQUENCE DESCRIPTION: SEQ ID NO: 19:

CCCCCCCCCC CCCCCACACT TTCAGGAGTC ACCCCCCAGC ATTTGGGGTT GGGTTGGCCC 60

TACTCCAGCC TGGAGCTCCC TGAGGGAGCC TGCACTCCCT GCTCCCAATC CCCGCTACTG 120

GTGCAGGGAT GCAGCCTGGA GCTGGCGTCC TTGTTCTGGG CCTGCTGCTG CCGCCACCCC 180

AGAGCCCCAG CCTGTCCTGA ATTGACATCA GTGCTTCCCT GAACTGCCTC CCCCACCCCT 240

GGGCATTATC CCAGGAAACT TTATGTTTTC TAGAAGCTAA GCAGCTGCTG GGACTCAGGG 300

ACTGGTGCAG GTAGGCTGAG TGGCAGCTCA GTCCTAGAAG GTCTCTGAAG ATCTGGACTG 360

AGGACCTTGC TACTCCCCAA GCCAGAGCCC ATCAGCCAGG CCTGCTGTGA GCCACCTGCC 420

TGTGGAGTGC TGAGCTCAAC CAAAGGCTGG CAAGCTCTGG GCCTCATTTA AGGGATTCTG 480

ATGAGCCGAT GGGCCCTGGA GGCAGCCCAT TAAAGCATCT GGCTCGTTTT TGGAAAAAAA 540

AAAAAAAAAA 550

(2) INFORMATION FOR SEQ ID NO: 20:

(i) SEQUENCE CHARACTERISTICS:
(A) LENGTH: 741 base pairs
(B) TYPE: nucleic acid
(C) STRANDEDNESS: double
(D) TOPOLOGY: linear

(xi) SEQUENCE DESCRIPTION: SEQ ID NO: 20:

TCTTGAAGAG TGTACAGTAC AGGATTATTA TAATGAAAGT TTATATCAAC AGGGTTTCGT 60

TGGCTCTGCA TATATTATAA GCAAAAGAGA TTGGTAAAGT GCCACAGTAT TCCAGATAAC 120

TTTTCAGTTG CGGCCTTTCT TCTCGTTCTT TAATTTGAAA CCTAGATACA TGCAGTAAAA 180

ACTAGGAGAA TGACTTTTAC CCTTGGGGAC AGCCAAGTTT TGTTGATAAA CCTATTTCCT 240

AGCATGCCTT CAGGAAGTTG TGCCAGACCC TAGATTGTGA AGGACCCACT GTTCTTCTGT 300

TGTACGAGCT CCCTGAACCA TTGTTCAGAG GACCAATGTC ACATCGCTTC ATGGGCATGG 360

NCCATGGGAG CATCTGGGTG ATAYCTGTCT ACAGTATTGG CTCTTCTGCG AGGCTGATAC 420

ACAAGGCCTC TCTTCCACAT GATCATTTGC AAACCTCCCC CAGCCCCTAC CATCCAATGT 480

GGAAGGAAAA CAAGAACTGC CTGAAGAAGA GTCCAAGCTA CAGATACACA GCGTGTGCAT 540

TGCGGCTGTC ACCTTCCTCC TCCCACTTCT GTATCCTCAG AGATGCTGCG TGGATGTTTC 600

CTTAACCTCA GCTGACTTCC CTGTGAATGT CTAATGCTAG TTCAGGGCCT CCAGGCATTG 660

ATTTGTACAG TGGTAACTCC CAATGAGGCT TCTGTTATCA TTTGGTGTGC TTTYTCTGTC 720

ATTAAAAGAA ATGATTTTCC C 741

(2) INFORMATION FOR SEQ ID NO: 21:

(i) SEQUENCE CHARACTERISTICS:
(A) LENGTH: 991 base pairs
(B) TYPE: nucleic acid
(C) STRANDEDNESS: double
(D) TOPOLOGY: linear

(xi) SEQUENCE DESCRIPTION: SEQ ID NO: 21:

GGCACGAGTC TCCCCTGGGG AAGTTTTTCT TTTTCAGGAG GGAGGAGGGC TTTCCCAGGT 60

AATGTGTCTA GAGTGTTGGG CAGAAAATCT GGGACCACAC CACACCAGTT CTCTCCTTAA 120

TCCACGTCAT TTGCCTTCTA TCCCAGCTAT GTTTCCAGTG TCCTCTGGGT GTTTCCAAGA 180

GCAACAAGAA ATGAATAAAT CTCTGGTGAG TTGTTTATTT GTTCTTCACT TTGTTTTACA 240

CTGTATTTTC TGAGTTTATG GGTGTCTGTG AATTAAAAAG GAAAAGTAGA AATAAGTAAA 300

ACTCAGGTTG AAGGAAATAT ACATAAATAA GATAAAGCTG ACCTGTAGAT ATAGCAGGTT 360

ATAAAGCTTA GAGTTGTCTA AGTTGAGTGC AAATTTTCCT CTGATCTTTC TGATGCCGAA 420

CAAAAAAGCA GTCATGTTTG TTATGTGATT GGAATGGAAC CCGAGAAGAG AGCATGCTGT 480

GTTCTTGTGG GACAGGAAAG CTTGCGTGCA CCAAGTCTGA ACCACCACCT TCATGGTGAC 540

ATAGATTATG TGCTGGAACA TATTTCACAC CGGCCTGGCA GTAAACACTT GTAGTGTTGT 600

GCAGTGGAAA CGGTCATCTT CCGCTAAAGC ACGGCGTGTT GTGCAGCGGA AATGGTCATC 660

TGCTGCTAAA ACACAGCTTC CATCGTAATG TATGCTCCTT ACTCAAAGAG TGTGGTCCCA 720

AACAGCCTTT GGGAGGTCCT CCTTGATTCA TGGATGAAAC CTGGAACATC TTGAGGACTG 780

AGTTAACCAT AGGTCCTTAA ATAACTCTCC ACACGTTTTT CTTAGTTTAT CTCTACATGC 840

AGGGTGTGCA GCAGCCTGTT CAAAGTCATA TTTTCTGGGA AATATTTCCA GTGTTTATTT 900

GCACTTTAGC CCACTCTGTG TAGCCTTATT TCTTCTAAAC TCACCATTAA TCTGAATAAT 960

AGTCAAATTT AGGGGGACTG TATTTGCCTT A 991

(2) INFORMATION FOR SEQ ID NO: 22:

(i) SEQUENCE CHARACTERISTICS:
(A) LENGTH: 653 base pairs
(B) TYPE: nucleic acid
(C) STRANDEDNESS: double
(D) TOPOLOGY: linear

(xi) SEQUENCE DESCRIPTION: SEQ ID NO: 22:

CCACGCGTCC GGAATTCCCC TGAGGATCTT GGGCTATCTT TGACAGGGGA TTCTTGCAAG 60

TTGATGCTTT CTACAAGTGA ATATAGTCAG TCCCCAAAGA TGGAGAGCTT GAGTTCTCAC 120

AGAATTGATG AAGATGGAGA AAACACACAG ATTGAGGATA CGGAACCCAT GTCTCCAGTT 180

CTCAATTCTA AATTTGTTCC TGCTGAAAAT GATAGTATCC TGATGAATCC AGCACAGGAT 240

GGTGAAGTAC AACTGAGTCA GAATGATGAC AAAACAAAGG GAGATGATAC AGACACCAGG 300

GATGACATTA GTATTTTAGC CACTGGTTGC AAGGGCAGAG AAGAAACGGT AGCAGAAGAA 360

GTTTGTATTG ATCTCACTTG TGATTCGGGG AGTCAGGCAG TTCCGTCACC AGCTACTCGA 420

TCTGAGGCAC TTTCTAGTGT GTTAGATCAG GAGGAAGCTA TGGAAATTAA AGAACACCAT 480

CCAGAGGAGG GGTCTTCAGG GTCTGAGGTG GAAGAAATCC CTGAGACACC TTGTGAAAGT 540

CAAGGAGAGG AACTCAAAGA AGAAAATATG GAGAGTGTTC CGTTGCACCT TTCTCTGACT 600

GAAACTCAGT CCCAAGGGTT GTGTCTTCGG AGGCATCCAA AAAAAAAAAA AAA 653

(2) INFORMATION FOR SEQ ID NO: 23:

(i) SEQUENCE CHARACTERISTICS:
(A) LENGTH: 1486 base pairs
(B) TYPE: nucleic acid
(C) STRANDEDNESS: double
(D) TOPOLOGY: linear

(xi) SEQUENCE DESCRIPTION: SEQ ID NO: 23:

GGCAGGCTGA CGACCTGCAA GCCACAGTGG CTGCCCTGTG CGTGCTGCGA GGTGGGGGAC 60

CCTGGGCAGG AAGCTGGCTG AGCCCCAAGA CCCCGGGGGC CATGGGCGGG GATCTGGTGC 120

TTGGCCTGGG GGCCTTGAGA CGCCGAAAGC GCTTGCTGGA GCAGGAGAAG TCTCTRGCCG 180

GCTGGGCACT GGTGCTGGCA SGARCTGGCA TTGGACTCAT GGTGCTGCAT GCAGAGATGC 240

TGTGGTTCGG GGGGTGCTCG GCTGTCAATG CCACTGGGCA CCTTTCAGAC ACACTTTGGC 300

TGATCCCCAT CACATTCCTG ACCATCGGCT ATGGTGACGT GGTGCCGGGC ACCATGTGGG 360

GCAAGATCGT YTGCCTGTGC ACTGGAGTCA TGGGTGTCTG CTGCACAGCC CTGCTGGTGG 420

CCGTGGTGGC CCGGAAGCTG GAGTTTAACA AGGCAGAGAA GCACGTGCAC AACTTCATGA 480

TGGATATCCA GTATACCAAA GAGATGAAGG AGTCCGCTGC CCGAGTGCTA CAAGAAGCCT 540

GGATGTTCTA CAAACATACT CGCAGGAAGG AGTCTCATGC TGCCCGCANG CATCAGCGCA 600

ANCTGCTGGC CGCCATCAAC GCGTTCCGCC AGGTGCGGCT GAAACACCGG AAGCTCCGGG 660

AACAAGTGAA CTCCATGGTG GACATCTCCA AGATGCACAT GATCCTGTAT GACCTGCAGC 720

AGAATCTGAG CAGCTCACAC CGGGCCCTGG AGAAACAGAT TGACACGCTG GCGGGGAAGC 780

TGGATGCCCT GACTGAGCTG CTTAGCACTG CCCTGGGGCC GAGGCAGCTT CCAGAACCCA 840

GCCAGCAGTC CAAGTAGCTG GACCCACGAG GAGGAACCAG GCTACTTTCC CCAGTACTGA 900

GGTGGTGGAC ATCGTCTCTG CCACTCCTGA CCCAGCCCTG AACAAAGCAC CTCAAGTGCA 960

AGGACCAAAG GGGGCCCTGG CTTGGAGTGG GTTGGCTTGC TGATGGCTGC TGGAGGGGAC 1020

GCTGGCTAAA GTGGGKAGGC CTTGGCCCAC CTGAGGCCCC AGGTGGGAAC ATGGTCACCC 1080

CCACTCTGCA TACCCTCATC AAAAACACTC TCACTATGCT GCTATGGACG ACCTCCAGCT 1140

CTCAGTTACA AGTGCAGGCG ACTGGAGGCA GGACTCCTGG GTCCCTGGGA AAGAGGGTAC 1200

TAGGGGCCCG GATCCAGGAT TCTGGGAGGC TTCAGTTACC GCTGGCCGAG CTGAAGAACT 1260

GGGTATGAGG CTGGGGCGGG GCTGGAGGTG GCGCCCCCTG GTGGGACAAC AAAGAGGACA 1320

CCATTTTTCC AGAGCTGCAG AGAGCACCTG GTGGGGAGGA AGAAGTGTAA CTCACCAGCC 1380

TCTGCTCTTA TCTTTGTAAT AAATGTTAAA GCCAGAAAAA AATAAAAAAA AAAAAAAAAA 1440

AACTCGAGGG GGGCCCRKAC CCAATCWCCC TATAGTAKAC GTANNN 1486

(2) INFORMATION FOR SEQ ID NO: 24:

(i) SEQUENCE CHARACTERISTICS:
(A) LENGTH: 2323 base pairs
(B) TYPE: nucleic acid
(C) STRANDEDNESS: double
(D) TOPOLOGY: linear

(xi) SEQUENCE DESCRIPTION: SEQ ID NO: 24:

CTTCGCCGTT TCTCCTGCCA GGGGAGGTCC CGGCTTCCCG TGGAGGCTCC GGACCAAGCC 60

CCTTCAGCTT CTCCCTCCGG ATCGATGTGC TGCCGCCGCC GCCGCCGCCG TCCCGCGTCC 120

TTCGGTCTCT GCTCCCGGGA CCCGGCTCCG CGCAGCCAGC CAGCATGTCG GGGATCAAGA 180

AGCAAAAGAC GGAGAACCAG CAGAAATCCA CCAATGTAGT CTATCAGGCC CACCATGTGA 240

GCAGGAATAA GAGAGGGCAA GTGGTTGGAA CAAGGGGTGG GTTCCGAGGA TGTACCGTGT 300

GGCTAACAGG TCTCTCTGGT GCTGGGAAAA ACAACGATAA GTTTTGCCCT GGAGGAGTAC 360

TTGTCTCCCA TGCCATCCCT GTTAATTCCT GGATGGGGAC AATGTCCGTC ATGGCCTTAA 420

CAGAATCCCC CAGATGGCTT CATGGCCCCC AAAGCATGGA AGGTCCTGAC AGATTATTAC 480

AGGTCCCTGC AGAAGAACTA AGCCTTTGGT CCAGAGTTTC TTTCTGAAGT GCTCTTTGAT 540

TACCTTTTCT ATTTTTATGA TTAGATGCTT TGTATTAAAT TGCTTCTCAA TGATGCATTT 600

TAATCTTTTA TAATGAAGTA AAAGTTGTGT CTATAATTAA AAAAATATAT ATATATATAC 660

ACACACACAT ATACATACAA AGTCAAACTG AAGACCAAAT CTTAGCAGGT AAAAGCAATA 720

TTCTTATACA TTTCATAATA AAATTAGCTC TATGTATTTT CTACTGCACC TGAGCAGGCA 780

GGTCCCAGAT TTCTTAAGGC TTTGTTTGAC CATGTGTCTA GTTACTTGCT GAAAAGTGAA 840

TATATTTTCC AGCATGTCTT GACAACCTGT ACTCTTCCAA TGTCATTTAT CAGTTGTAAA 900

ATATATCAGA TGTGTCCTCT TCTGTACAAT TGACAAAAAA AAAAATTTTT TTTTCTCACT 960

CTAAAAGAGG TGTGGCTCAC ATCAAGATTC TTCCTGATAT TTTACCTCAT GCTGTACAAA 1020

GCCTTAATGT TGTAATCATA TCTTACGTGT TGAAGACCTG ACTGGAGAAA CAAAATGTGC 1080

AATAACGTGA ATTTTATCTT AGAGATCTGT GCAGCCTATT TCTGTCACAA AAGTTATATT 1140

GTCTAATAAG AGAAGTCTTA ATGGCCTCTG TGAATAATGT AACTCCAGTT ACACGGTGAC 1200

TTTTAATAGC ATACAGTGAT TTGATGAAAG GACGTCAAAC AATGTGGCGA TGTCGTGGAA 1260

AGTTATCTTT CCCGCTCTTT GCTGTGGTCA TTGTGTCTTG CAGAAAGGAT GGCCCTGATG 1320

CAGCAGCAGC GCCAGCTGTA ATAAAAAATA ATTCACACTA TCAGACTAGC AAGGCACTAG 1380

AACTGGAAAA GACCACAGAA AACAAAGAAT CCAACCCTTT CATCTTACAG GTGAACAAAC 1440

TGTGATGATG CACATGTATG TGTTTTGTAA GCTGTGAGCA CCGTAACAAA ATGTAAATTT 1500

GCCATTATTA GGAAGTGCTG GTGGCAGTGA AGAAGCACCC AGGCCACTTG ACTCCCAGTC 1560

TGGTGCCCTG TCTACACCAG ACAACACAGG AGCTGGGTCA GATTCCCCTC AGCTGCTTAA 1620

CAAAGTTCCT CGAACAGAAA GTGCTTACAA AGCTGCCTTC TCGGATACTG AAAGGTCGAG 1680

TTTTCTGAAC TGCACTGATT TTATTGCAGT TGAAAAAAAA AAAAAGCTAT TCCAAAGATT 1740

TCAAGCTGTT CTGAGACATC TTCTGATGGC TTTACTTCCT GAGAGGCAAT GTTTTTACTT 1800

TATGCATAAT TCATTGTTGC CAAGGAATAA AGTGAAGAAA CAGCACCTTT TAATATATAG 1860

GTCTCTCTGG AAGAGACCTA AATTAGAAAG AGAAAACTGT GACAATTTTC ATATTCTCAT 1920

TCTTAAAAAA CACTAATCTT AACTAACAAA AGTTCTTTTG AGAATAAGTT ACACACAATG 1980

GCCACAGCAG TTTGTCTTTA ATAGTATAGT GCCTATACTC ATGTAATCGG TTACTCACTA 2040

CTGCCTTTAA AAAAAAAAAC CAGCATATTT ATTGAAAACA TGAGACAGGA TTATAGTGCC 2100

TTAACCGATA TATTTTGTGA CTTAAAAAAT ACATTTAAAA CTGCTCTTCT GCTCTAGTAC 2160

CATGCTTAGT GCAAATGATT ATTTCTATGT ACAACTGATG CTTGTTCTTA TTTTAATAAA 2220

TTTATCAGAG TGAAAAAAAA AAAAAAAAAA AAAAAAAAAA AAAAAAAAAA AAAAAAAAAA 2280

AAAAAAAAAA AAAAAAAAAA AAAAAAAAAA AAAAAAAAAA AAA 2323

(2) INFORMATION FOR SEQ ID NO: 25:

(i) SEQUENCE CHARACTERISTICS:
(A) LENGTH: 683 base pairs
(B) TYPE: nucleic acid
(C) STRANDEDNESS: double
(D) TOPOLOGY: linear

(xi) SEQUENCE DESCRIPTION: SEQ ID NO: 25:
   GGCACGAGCC TGTGTGGTCA TGTTCCTCGT GGTGCAGTAC CTGACATGAG CCAGCCACGC 60
   TCAGTGGCTG AACAGCATTC CCACAGCCTG CAAGTGTGTG TGTGTGTGAA AGAGAGAGGG 120
   GGGCCCAGAG CCGCCTTTTG AAATGTTTGC CTGTCTGAAC TGTGAAGACA CTTGGGAGTG 180
   ATTGTGGTCT AATTTCCAAC CTGCTCTGTT TTCTGTGACA TCTTGGAGGG GAGCTAGTGC 240
   CACACCATGC GCGGTGCTTA GAAATGAAAA AGTCCCGGGT CTGTCTCTCT CACTCTCGCT 300
   CTCATGGGGG AGGGAAAGAA TGGCTTTGGT GGCTTTGTTC ACACAGCTGA TGCGTGCTGG 360
   GAAGGTGTCC ACAGTGAGCC TGTGTGCAGG ACTGTCCACA CGGTTCACAC TTGTCACCAT 420
   CAGGCCTTTC TGGTCCTGAT AGGGTGGAGC AAAAGTGGAA AGGAAAGGAA AGAGGCTTTT 480
   CTCACAGCCA TTATATTAAA TAGTAGGTCG ATTCACATCT CGTGCTCCTG GCCACCTTCC 540
   CCTGTGCCTC AGTGACATGT AGATGACTGA CTGCCAATAC TTGTCACCAT TCCCTGGAAG 600
   CAGCTACCTA GGGGAAACAA GATGTAGTGC TATTGCCGAT AACAAGTAAG ATTTTCCACA 660
   CTAAAAAAAA AAAAAAAAAA AAA 683

(2) INFORMATION FOR SEQ ID NO: 26:

(i) SEQUENCE CHARACTERISTICS:
(A) LENGTH: 2036 base pairs
(B) TYPE: nucleic acid
(C) STRANDEDNESS: double
(D) TOPOLOGY: linear

(xi) SEQUENCE DESCRIPTION: SEQ ID NO: 26:

CTGAGAAAGG AAAGCATTCG GATCTGCTGC AAAAACACAT ATATCCATAA AGACTCATGT 60

TATTCAGAAA ACAGATTGTG AACACAATCA CATTCGCATG AATCCTTTAA AAGGAAGAAG 120

ACCTTAAAGT ATCTGCAAAT CTGAATTTCT ATTTATTCCT TCACTGAATA TAGAAACAAT 180

GGTTATCTGA TTATTAGAGA TATTATTTTG GATATGTTAC TTATTAACTT GCTATGGCTG 240

GTAACCATGA TAAAGTCTGT TATTAATAAC AACATAATTC TTTTTTTAAA GAAGAAAAGC 300

TTATTTTTCA TTGACAGTGT ATAGATTTAT CTACTTAGTT GTGTTTTGCT ATTAGTGTTT 360

TAATTTTTTT TTTAAGTTGA GTGTTTGATA AATTTTAAGA CCCTGTCCCC ACCTTGTTTT 420

GAGTCCTGTG TTGACTACAG GTATATAGCY CAWTTTAAAA ATCCTAAAGC AAAAGAATTT 480

TATTTATAAA AGAATCMAMC MGTTGCATGC ATGAGGCTGT GAAGTCAGAT ATTTAGTAAT 540

AAAAGCAGCA GTGCCTTTTT TTGTATTTAC CCATTGACCC CCACCAAATG CAACTGTTTT 600

ATATTAAGAA AATAGTAACA ATTTTAAAAT CTCAGAGTAA AATCTATTTC ACTACATGCT 660

TTTCCCCCCT TGTTCTGATT TAAGCAGTGT GTACTTGGCA TCTCTACATT GTCCTAGGGA 720

CAGTGGTGTT CTACAATATT ATCATGTATG ATGTTTTATT GGTGCTTTTT ATTCATAGTG 780

GCTTCTTACC AGAAACAGTA GGAAGAAACA CATGAACTGT GTACAAGACA TGAAACATTG 840

CTGCTGATAT GTTGTTTTTT CACATGCTTT TGAGTTTTCA CTTTTTAAAC GAGAGCCAGC 900

AAGCAAAATA GATGTGGCTG GGTCTGCCTG TCCGGGCGGC TYTTTGCACC GAGCTCTCAA 960

ATCCTGTGTA TTGAGGGTTC CTTTTTGGTA CTCAGGATTG GAGCTACAGC TGGGCCCCCC 1020

TCTCTCCCAT TCGTTTGAAG AGACACTGAG GGAAACAAGG GTTTCTTTTG AGGTGTCCTT 1080

GGCTGCCTTT TACGGGATGG GAGCCTTCTC CGGATCTTTT GTTCTTCTGC ACCTCTTGTA 1140

GCTACTGCCG GTGCAAGGTT GTAGATGTTA TTCCCCAGGA GCCTGGGCTK GGGGGCTGAG 1200

CTGGGCTGAA TGCAAAAGCA TGCAACCAGA AGGCGGGCAA GGGGAGGAAA AGCAGGCCTG 1260

GCCTCATTGG TCCCCTGGAG ATGTCTGTAG CAGTCAGCTC CAGCTTGGGC CTGGGGAAGC 1320

AGCCTGACCA AGGCGCTCAG GTGTGCCTGT TACAAGAAGA ACCTGCAGAA GGATAATTTG 1380

CACATGGAGC TGTGATAACA CTAATGTTGA TTTTTTTTTT TTTTACAAGT CATCAGRGAT 1440

GTTTGCAAAG TGAGTTTTAT TTTTTTGTAA TTCCTTTATC TTTACTTAAA GGTGAATGTG 1500

TATTCCTCTG GGAGGAATAG GAAGAAAACA GGAATGTTAA TAATGTCGAA CAGAAAACTT 1560

CCTCCCTTAT TAATATATAA TCYTCATGTA TTTATGCCNT AATGTAAGCT GACTTTTAAA 1620

AAGCTTTCTT TTGTTGCATG CCCTGTGCAG GCATCTGTAT TGTACATGCA TGCCTTTCGT 1680

CCTGTTTTCC TGTATAAAGT TAGTGAACAA AGAAATATTT TTGCCCTAGT TCATGTTGCC 1740

AAGCAATGCA TATTTTTTAA ATTTGTCATA TATGGAAAGA GCATGTTTGT TACATGTAAA 1800

AGCTTTACTG ATATACAGAT ATACTAATGT TTGAAGATGC TGTTCTTTGC AAGTGTACAG 1860

TTTTCAAATG TTGTTACCAG TGAAACACCC TTGTGGTTTA AACTTGCTAC AATGTATTTA 1920

TTATTCATTT CCTCCCATGT AACTAAGAAT CATGGCTATA TTTCATATCA ACGTTATATT 1980

GAAAGTGAAG GGAAATGATT AATACAAGGT TTTGTAACAA AAAAAAANAA ANNAAA 2036

(2) INFORMATION FOR SEQ ID NO: 27:

(i) SEQUENCE CHARACTERISTICS:
(A) LENGTH: 717 base pairs
(B) TYPE: nucleic acid
(C) STRANDEDNESS: double
(D) TOPOLOGY: linear

(xi) SEQUENCE DESCRIPTION: SEQ ID NO: 27:

GGCACGAGAT AACATAGGCA CAATAATACT GTATGTCTAC TTCTAGGATT ATAAGGAATT 60

AACATTGAGA TGACATTTCC ATTTGAGAAG AAAATAGTTG CTTTCAGTGC CTTTTATTTG 120

ATTCCTGGAG AGAGCAGACT CGCACCAACA TTCAACCCCA GCGCTGATAT GACAGTAATC 180

CTCAGAGGCA GAGCCCAGCA CAAAACAGCA ATGCTAGAAA GTTACAATTG GAAAGTTTCC 240

TGCCAGCTTC GGGAATGACA CTGCAAAGCT GATGCCAGAA ACTGCCAGAG TAATTCTCCT 300

CATTACTGCT CTACCCACCC ACTTTCAGCT CCCCAAATTA ACTAGTGCAG TTGACTAATC 360

CTCTTTACCT TTATCATTTA GGTGAGGCAT TGCACAAAAA CTCTCGACTT TGCCATATAA 420

GGGCTGTGGT TCTCTGTGGT CCTGGATAAG AGGCATCACC ATTATCTGGA AACATGCAGT 480

AAATGCAGAT TCTTCATCTT CTCCCCAGAC CTCCTGAGTT AGAAATTCAC AAGTTCTCCA 540

GGTGATCTCA TACATGCTAA AGTTTGAGAA CCATTGAGTA AAGTTAATGC ATTAAGAAGA 600

GATTAGATAG GGATGGTGGC GTATCTTCCT ACAGTTTCCC TGTTAACAAG AAAGTCAGAG 660

GTCAGTTGAT CAGACATTAG ATTATTTATT GCTAAAACTA AAAAAAATTA AAAAAAA 717

(2) INFORMATION FOR SEQ ID NO: 28:

(i) SEQUENCE CHARACTERISTICS:
(A) LENGTH: 495 base pairs
(B) TYPE: nucleic acid
(C) STRANDEDNESS: double
(D) TOPOLOGY: linear

(xi) SEQUENCE DESCRIPTION: SEQ ID NO: 28:

GAATTCGGCA CGAGCAGCAT CCTAATTTTA GTTTGGAGAT GCATTCTAAA GGATCTTCTC 60

TATTGCTTTT TCTCCCACAA TTAATCTTGA TTCTGCCTGT CTGTGCACAT TTGCATGAGG 120

AACTGAACTG TTGTTTTCAT AGGTAAATGA GAGACTGAGT TTTTTCATTT CTGAAGAGAA 180

AGGGCATTTG CTCCTACAAG CTGAAAGGCA CCCCTGGGTG GCTGGGGCCC TCGTGGGAGT 240

TTCTGGGGGA TTGACCCTTA CAACATGCAG TGGCCCTACA GAAAAACCTG CAACTAAAAA 300

TTATTTTTTA AAAAGGCTCC TCCAGGAAAT GCATATAAGG GCTAATCACC CAGTATTTTG 360

ARGCTTCGAA GARGTAATAR AMCCCTGGAG AGAGAAACTG AGACATGTAA GAGGGTGGGA 420

ATGACTCAGT GGTGGCACAC TATGGAGTCC TGCCCACAAG TAGCACACAT CAACCCACTA 480

CACAGAAATC CTAGG 495

(2) INFORMATION FOR SEQ ID NO: 29:

(i) SEQUENCE CHARACTERISTICS:
(A) LENGTH: 556 base pairs
(B) TYPE: nucleic acid
(C) STRANDEDNESS: double
(D) TOPOLOGY: linear

(xi) SEQUENCE DESCRIPTION: SEQ ID NO: 29:

AGCTTAACGT CATGATTCAT TAGGGGAATG CAAGGCAAAA CCATGATGAG AATGCCCCTA 60

GACACCTCTT AGAAGAGCTG CTAGAAAGGC AGACAGCACC AAGCGCTTAA ATGAGATGGG 120

GGCACTGGTG CTTCTTCTGT GCCTACTGGT AGGGGTGCAG CAGAGTGGTT CAGTCTGGGA 180

CAGTTAGCTG GACATCACGT GGACCCAACA CACGCATTTC CTGGGTTACT TACCAAGGAG 240

AATAGAAAGC AGGCAGATCT TTACAGCAGC TCTTACCTGW TTGCAAAACA ATGGAAATGC 300

CCACATGTCC ACAAACAAGT KTGTGGTCTG CCTGTGCCAT GAAGCACAGT GTGGCTGAGC 360

GTCAAGAGTC CCCACACTCA AAGGAGGCAG CAGATACAGG GCTGCACACT GTGTGATTCC 420

ACACATGTGA CATTCTGGAC ACGGACATGC TGGATGGCAA AACGAGCATC GGGCTGAGAG 480

GACTGCTGAG AAGGGGAACG GGGCTGCTGG GATGTGGGTT GATTGTAGCA GTAGCTCATG 540

GAGATGTGAC CTCAAA 556

(2) INFORMATION FOR SEQ ID NO: 30:

(i) SEQUENCE CHARACTERISTICS:
(A) LENGTH: 434 base pairs
(B) TYPE: nucleic acid
(C) STRANDEDNESS: double
(D) TOPOLOGY: linear

(xi) SEQUENCE DESCRIPTION: SEQ ID NO: 30:

CTAAATGGTG ACTGTGGCTT TGTCGAGACA GGCCCCAAAT GGTAGGTGTG AACACAACAT 60

GCACAGAATG AGGAGACATG CAGAGTGCTG AAATACTGTC CTGGACAGAT GTGTTACATG 120

ACTTTCTTTT CAGCTTATTT CTGTGGCCTG CCTTTGAAGA TAGAGCTTTG TTGATATTTA 180

CATTAAACCA AATTGTATAA YTATGTTCCA TTCTGACATG TTATTTAGCA AARGAAAAAR 240

GAGTAATTCT ACATCAGCAT CTTTAGTGCA TGCTAAAAGA TTAAAAATGT CTTTTGGGGA 300

ACATGTTTTG TATACATAAA TGTTTAGATA GAAATATTTA TAGAATNCTC TATGTGAGTA 360

TTNATCTCCC TATGTATATT TATATCTAGA TGTGTCAATC TTTGTATTGA TATGAAATGC 420

TATGAATAGT GAGA 434

(2) INFORMATION FOR SEQ ID NO: 31:

(i) SEQUENCE CHARACTERISTICS:
(A) LENGTH: 715 base pairs
(B) TYPE: nucleic acid
(C) STRANDEDNESS: double
(D) TOPOLOGY: linear

(xi) SEQUENCE DESCRIPTION: SEQ ID NO: 31:

CCACGCGTCC GATCTCACAG CTCCGACACT ATTGCGAGCC ATACACAACC TGGTGTCAGG 60

AAACGTACTC CCAAACTAAG CCCAAGATGC AAAGTTTGGT TCAATGGGGG TTAGACAGCT 120

ATGACTATCT CCAAAATGCA CCTCCTGGAT TTTTTCCGAG ACTTGGTGTT ATTGGTTTTG 180

CTGGCCTTAT TGGACTCCTT TTGGCTAGAG GTTCAAAAAT AAAGAAGCTA GTGTATCCGC 240

CTGGTTTCAT GGGATTAGCT GCCTCCCTCT ATTATCCACA ACAAGCCATC GTGTTTGCCC 300

AGGTCAGTGG GGAGAGATTA TATGACTGGG GTTTACGAGG ATATATAGTC ATAGAAGATT 360

TGTGGAAGGA GAACTTTCAA AAGCCAGGAA ATGTGAAGAA TTCACCTGGA ACTAAGTAGA 420

AAACTCCATG CTCTGCCATC TTAATCAGTT ATAGGTAAAC ATTGGAACTC CATAGAATAA 480

ATCAGTATTT CTACAGAAAA ATGGCATAGA AGTCAGTATT GAATGTATTA AATTGGCTTT 540

CTTCTTCAGG AAAAACTAGA CCAGACCTCT GTTATCTTCT GTGAAATCAT CCTACAAGCA 600

AACTAACCTG GAATCCCTTC ACCTAGAGAT AATGTACAAG CCTTAGAACT CCTCATTCTC 660

ATGTTGCTAT TTATGTACCT AATTAAAACC CAAGTTAAAA AAAAAAAAAA AAAAA 715

(2) INFORMATION FOR SEQ ID NO: 32:

(i) SEQUENCE CHARACTERISTICS:
(A) LENGTH: 486 base pairs
(B) TYPE: nucleic acid
(C) STRANDEDNESS: double
(D) TOPOLOGY: linear

(xi) SEQUENCE DESCRIPTION: SEQ ID NO: 32:

GAGCCAGTGC CGGCGAAAGG GGACCTTCCT CTACTTCCTG CCACAGACCC TGTCCCCACA 60

CACTTCCTGC CCCTGCTCTG CTGGGAGGCC ACTTCCTCCC CCAGTGCTGG ATTCCACCCC 120

CAGCTCACCC TCAAACATGG CCCCCTCTCT CCTCCTGCTT GCCCCTCTCT GCTCCCTGGA 180

GGCTGTTCTG TCCTCCCCTC TTGAAAAGCA ATGCCAGCTT CCTGGGATCT TCTGCCAACT 240

CCAGCTACCA TGCCCTTTGC TCCTGTCAGC TCAGCTCCTC AAGGGAATTG TCTAMCCTCG 300

GTGTCCTGCT TCCCTCCCTC AACCTCCTCA CCCTGCTCCA AGCTGGCATC TGCCCCTCCA 360

CTGCACAGAA CGGNTCCCCC ACCACCTGCC TTTACAGGGA GGAAGCAGCA ACATGGAAGA 420

ANCGAACTAT AGGGGCTACA ANGATGCTCA GCTCTGATCC CGAAGGCAAA AAGNATCTTT 480

GGGCAC 486

(2) INFORMATION FOR SEQ ID NO: 33:

(i) SEQUENCE CHARACTERISTICS:
(A) LENGTH: 725 base pairs
(B) TYPE: nucleic acid
(C) STRANDEDNESS: double
(D) TOPOLOGY: linear

(xi) SEQUENCE DESCRIPTION: SEQ ID NO: 33:

GTTCCTCTGG TAATAATTAG GTTATTCCCA GAAGCACAGT GTCATTCTTT AAATAAAAGC 60

TTTCCTGTTT AAAGCTTTTC AAAGGAGCAG ACCACCTTGA AGATTCCCCC TAGGGTTGAT 120

ATGTGTCTAA TTCATTTTAT AAAAATTATT CTTGTCTTCA TTTTAAAGCT TTGGCTATAT 180

AGTCAGAAAT GTCCTAAATA ACAAACTATT TTGTATTTAA TTTAGGGAAG ACTAAAGGGA 240

AGAAAAATGA AAACTCAGTC TTTATGTAAG CTCCAAGGAT ATTAGGGCTT AAAGGGCTTT 300

TCTAGTTTTA TGAGAATTTG TACTACTGAT TTTTATATAT TCCTGTTTTT GATGAACAGA 360

TCTCTGGGGA AATTGTTGAG TTACAATGGC ATTTCACTGT GATCCCTCTC AAGCTCAGAT 420

CAGTTCTATA ACCCAATGAC AACCTGTCTC TTTGGTTTAC TGTCCTGTGA AATGTCAGCT 480

CAAGTTTCCC AGAAGTCGTG TGTTTATGAT GAGTCAGAGT GCTTTTCCTC GGTGGGACAG 540

TTGCTGGCCC TCTTAATTTT GGTGTATGTG CTTCCAAGTA TCTAAACCTC CAGTCTGATC 600

TGTATATGCT ATCCTAACTG TTAATTGTAT TATTGATTAT GTTGATTATC TTGCTTGAAG 660

GTTCATACTT TTCAATTTGA TAGAAATAAA GTTTTTTTCT GCTTATAAAA AAAAAAAAAA 720

AAAAA 725

(2) INFORMATION FOR SEQ ID NO: 34:

(i) SEQUENCE CHARACTERISTICS:
(A) LENGTH: 437 base pairs
(B) TYPE: nucleic acid
(C) STRANDEDNESS: double
(D) TOPOLOGY: linear

(xi) SEQUENCE DESCRIPTION: SEQ ID NO: 34:

CACACAGCAT GCTGCCCTCA GACGTGTCCA TCCTGTACCA CATGAAAACG CTGCTGCTCC 60

TGCAAGATAC TGAGAGATTG AAGCATGCTC TGGAAATGTT CCCAGAACAT TGCACGATGC 120

CTCCTGCTTT TATTGGCTCT TGTCGAAATC AAATTGGAAG ATCTTCAGTC CCAGCTGCAC 180

CCAACGTGGA AAAGTATTCC AGGTCCATCC CCAAGGAACC AACACCGATG ACATGGACTC 240

AGGAATCTTA TAACCTACGT GGACTCTTTC CATCCGTACA TTGTCGTGCA CATGCCACTC 300

ATCACCTGGC GTGCCCAGAT CCTCGCARGG CAACACCCTG TGATAATTCC AGGTGATTCT 360

CTACATCTGC AGCTTGAGGT TAGCCTCATA TCACATTACA TTCTCACTAN AAACNAAAAA 420

AAAAAAAAAA AACTCNA 437

(2) INFORMATION FOR SEQ ID NO: 35:

(i) SEQUENCE CHARACTERISTICS:
(A) LENGTH: 943 base pairs
(B) TYPE: nucleic acid
(C) STRANDEDNESS: double
(D) TOPOLOGY: linear

(xi) SEQUENCE DESCRIPTION: SEQ ID NO: 35:

GGCACGAGCT GGAACAGAGA CTAAATCCCA CGAAACTGAC ATTGTTAAAC ACACTAAAAC 60

AGAAGTACTT ACCTCTTGAA GATTTAATAT ATAATGGTTG ACATGATACA TGTACATGAT 120

GAATGACCAG ATGCTTATGG TCTACATTTT CCTTTATCCT GTTAGTATTA CCTTCCTTAA 180

TCTTTGTTCA TTAACATGCT AATTCCTCTT CAGTGTTTAT TTTCTAGTGA CAGAATGCTA 240

ACATTTCTTA CACCCTGGCA GAAGGGAGAG AAATGTGTTT TGGGGTGGGT AACTAAATTT 300

TTGAGTGAAA TATCATAAGA TGANAATGGA AANAAGGAGA CACAAANAGT TATNACAAAA 360

AAACAATGGT TTTTTTAGCC ATTTGACTGG CTCTTTAAAT AGTCTACAAG ACATTCACGT 420

TTAACATCAC TTTTAGTGAA ATAAAATGTG CCATACTAGT ATGTGCTTCA AAAGGGCAAA 480

TGTGCTTTAG TGCCCTAAGG CTAAATTTTG GTCATTTGAC ATCAGAGATG TTGTAAGTAT 540

TGCACTTAAT ACGCACCTAT TTNTCAATAG TGTTATTTTT TGGNTAGCAT TTTTTTTACC 600

ACTATNTTGT TGATAGCTTT TTGTTCTNTN AGGTTGNAAN ATGACAGTGC TNATNTCAAA 660

CAGATTACCC ATNTGCAGAA CTAAGGGAAG CNATTTATGT ATGAAAGNAA TTNTTGAATT 720

NGTCATTNTC AACCNTTGNA TTAAAGCTTA GACTAAATAG TAATATATNG TGGGNAGGAT 780

TTTGGTTTTG TGATATTTNT GTGNATTAAG GNATAGATGT TAACCNTTAT TTTGTAGNAA 840

AGTGANTTGT ATGTGGTTAA TTATAAATAA AACTGGTACC AGGNAAAAAA AAAAAAAAAN 900

NAAAAAAAAA AAAAAAAAAA AAAAAAAAAA AAAAAAAAAA AAA 943

(2) INFORMATION FOR SEQ ID NO: 36:

(i) SEQUENCE CHARACTERISTICS:
(A) LENGTH: 604 base pairs
(B) TYPE: nucleic acid
(C) STRANDEDNESS: double
(D) TOPOLOGY: linear

(xi) SEQUENCE DESCRIPTION: SEQ ID NO: 36:

GGCACGAGAA ATCTTCATGC TGTAGTCACT CCAGACCATG GAGTGGCTTT CCAGCTGAAT 60

GAATCCTATG TCTCGCGTGC AGGTGGTTGG TTTTCAATGT TCTTGCTAAT TTTTTTTCTA 120

TTGGATCTTG GGAGTTTTCT TTGTTTGCTC CTGTGTTTGC CCAGCTTTAA TAAAACCAGG 180

CGCAAACAAA AACCATAGCA TTCTGAACAA TAGGGGGCCC ACATTGGACC CAGTATGTCA 240

CTTTAATGGA CTTCAAGAAA AAATCTGAAT GGGAAAAATG ACACTAGGAA TGTATACTCC 300

ACACATTTTA TGCCATATAA TGGTGTGTTT TCTTAATTTT GTTTCTTGTG GCGAAATGTG 360

GCTTTCAAAT TAAAATGACC TTTTCTTCTT TGAAACTTTT TGTTTTGACT TGTATAATTA 420

AGGGTTTGGA AAGATTCATA ATTCTGAGAG AGGTTTGCAA CCAGGAGATA CAAAGAAGTC 480

TCAGTAGTAA TCTTGTTCAT GTGCTTTTAC AGCCAGCTAC ATTTAAGGAT GTATTAGTTA 540

CAGAAATTAT ATGTCTGTGT ATGTGTCTCT ACTCAATAAA GTACATGCCT CCACAAAAAA 600

AAAA 604

(2) INFORMATION FOR SEQ ID NO: 37:

(i) SEQUENCE CHARACTERISTICS:
(A) LENGTH: 349 base pairs
(B) TYPE: nucleic acid
(C) STRANDEDNESS: double
(D) TOPOLOGY: linear

(xi) SEQUENCE DESCRIPTION: SEQ ID NO: 37:

GTGAGTGCCC GGGAGCCCCG AGGCCCTGCC CCTAAGAAGG ATATCTYTRA CCGCTCCCTT 60

GTCCACACCC TAACCCCCCA GCTGCTCAGG CAGTGGGCAC ATGGCAGGGG CCTCACTGGG 120

GGCACATAGA GCATTTGGGG GACTGCGAGT GCTCACCTTT GACTTCCTGC AGGTCGGGGG 180

AAAACCAGAT CATGATGACC AAAGTYTACA TATTCTTGAT CTTCATGGTG CTGATCCTGC 240

CCTCCCTGGG TCTCACCAGG TATATGCCAC CACYTTCTGY TCTAAATTCA GAATAAGAGT 300

CACATCAGGA GAGCACTGTC CCCAGGANAA TGCAAACGGG TTGGCAGCA 349

(2) INFORMATION FOR SEQ ID NO: 38:

(i) SEQUENCE CHARACTERISTICS:
(A) LENGTH: 672 base pairs
(B) TYPE: nucleic acid
(C) STRANDEDNESS: double
(D) TOPOLOGY: linear

(xi) SEQUENCE DESCRIPTION: SEQ ID NO: 38:

GTAGTCGTTG CGGTTGCCGG GATGGCGAAG ATCTCGCCGT TTGAAGTCGT AAAACGCACC 60

TCGGTACCGG TGCTTGTTGG TTTGGTGATT GTWATCGTTG CTACAGAGCT GATGGTGCCA 120

GGAACGGCAG CAGCGGTCAC AGGCAAGTAA ATAGTAATGC CGGAGCAAGT TTCCTCCGGC 180

TTTATCATGT CACCCACTGT GGTATATGCG TTGTGGTCTG CCAACTTTGC CGTGAACAAT 240

TTCAGCAATA ATCAGATGGC GGCTGGCGCA ATATTCAAGA TAACGCCTGG CAGTGGTGCG 300

GCTGATGGTT CAGTGCCTGC GSCACCGTTT YTGCCGTATG TTGCACACCA GGNTCTTTAA 360

ACAGTTTTCG SACCGCGTTT AGCGTCAAGG GTTCAATGCC GGTCGGTAGC TCGTCCTTAG 420

GTTCACCGCG AGCATAAGCA TTAAACATCT CATCAATTTG CTTCTGGCTG GCGCTATCAA 480

TACTTTCCAG CATATGTTTA CGCTGGCGGA AACGGGTTAG CGTTTGCCCC ARCMGWTCAT 540

AGGCAATGGG CTTAATGAGA TAATCAAATA CACCACAACG TACGGCTTCA GACACCGTTT 600

CCATATCGCT GGCTGCAGTG GTAAACACCA CGTCGCCGGG ATAATGCGCC TGCACCAGTT 660

CATGCAGTAA AT 672

(2) INFORMATION FOR SEQ ID NO: 39:

(i) SEQUENCE CHARACTERISTICS:
(A) LENGTH: 1908 base pairs
(B) TYPE: nucleic acid
(C) STRANDEDNESS: double
(D) TOPOLOGY: linear

(xi) SEQUENCE DESCRIPTION: SEQ ID NO: 39:

AGAGTTGATA TTTTTAGAAA CAGTAATTTT ACTTTTAAGG AAATTGGCTA GCTCTTTGAC 60

TNNAGAGCTG TAGGAAGCTC AACATTTCTT TGTAGAGAAC GTTGCTTTTT TTGGATTGTA 120

CAGGTATAAA AACATTGCTT TTGTTGAATT GTATAGGTGT AAAAAGGGAA TAACTGTATG 180

CAGGTTTGAA AAGGAAATGT GCTTTAGGCA TGAGTCATAA GATGCCATTG TACTTGTAGG 240

CATTTTATTT TCCTTTAGAA ATGGACATCA GCTCTTCTCT TCTGACTGGT AACACATAGC 300

CCCAAAGCAT GAGATTATTT TTCATTGGGT TTTTATTGTT GTTTAGTTTT GGTTTGTTAC 360

GCCAGCCCAG TCTGTCTGCG GAACACTGAC TCTGCTCTCT AATGAGAACA AAGTTAGAAA 420

TCTGCCGATA ACCTAAAATA ATTTAGAAAT GAATTAAAAA TGTGAAATCG GGTTAAAGTG 480

ATGATGATAA AATAGCATGC AAGAAACAAG CTCCTTCCAT CAGACTTGGC TACTGTTTTC 540

TTCTGGTACG ATTTGGTTTG GAAGAGCCTC TTGTTTCCTT CTCTTTGGGG TATGTCTTCG 600

TTTCTTAATA TGTTTGTAAC ATTATTGAGA TATAATTCAC ATACCTTACA ATTCACTTAT 660

TTTAAGGGTA CAATTTAGTG GTTTTTAGTG TATTCACAAA GTTGTGTAAC CGTGACCACA 720

GTCAATTTTA GAACATTTCG TTACCCCAAA AAGAAACCCT GTACCCTTGA GCAGTCACCT 780

CTCATTTTCT CCCAGTGCCC ACCCCATCCC CGAGCCCCKG GAACCACTAA TCTATTTCTC 840

TCTCTGTAGA TTTGCTTATT CTGGTCATTT CATATAAATG GAATTCTACA ATATTCGGTC 900

TTTTGGGACT GGCTTCCCAA ATATGATTTT CTATATGGAG TGAGAAAATT CTTCTCATCT 960

TGAGAACTCT TATTGCTGTG AAAGGGAGTG GTTGGTAAAA TCAATAGATT TCAGGCAAGA 1020

GGGCCAGATA CCTAACAGGT TTTTCTCCGT GAATCTTATG CTGAGTAGTT TTTCCTCATA 1080

ACCAAGCATT TATGATATAT TACTACTTAT AATACTGTGG CTAGTCTCTA GAATGGATGT 1140

TGAAATCTTT GCCTCCTCAG TCGGGAAGAG TCCTGCTAAA AATCAGGCTA AAAATCAGGC 1200

CAAAAATCAG GCCAAATGAC TTGGCAAATA ATTGACAAAG TGGTTTTCAC GTGTGTCTAT 1260

CTTTGCTAGC AGCTTGTATA CCTCAGGCCA GGTGAGCTCC CCAAATTTCT TTTTTCATTT 1320

ACTCCAGTGA GTTTCTGCTG TCTTTTTCAA GTATGTACCA TAGGACTTAA AGGTGATTTG 1380

GATGCGTTGT AACACTGCTA AATATGCTAA GTACAGAATT TTATCTACAG TACTGTGAGA 1440

CAGTCAATTA TTGCCTAGGG TAGTTCAAAA ATATGATGTG AGCTAGTTAA GCCTTTGCTT 1500

GACTGATTTC AGTGATATTC AGAAGTGTGT ACCAATCAAG GCTCTTTAAA ATACGGAACG 1560

ACTCACTTAA TAACCAGGGA ACCAGCCAAA TACTGTGCAG CCGCAGAATA TGCATATCAA 1620

TGAGTTGGAG GTGATTATTC TCTGTAACTC CCTAATGATT GTTTTCTAAG CATTGTGGCT 1680

TCTCAGTGGC TTGACAGCAT CTTCCTGGTT GTATGTGGCC TGTTTACATG ATGTATTGAA 1740

TAATGTTGTT TGTTGTGAGC ATCAATGCCT GTAACACCAA ACTAAACACG TGTTTTTGGG 1800

ATATGTTTCC AATCTTTAAA TGACCTTGCC CTGTCCAATA AATAAATGAT TGTCTCACCC 1860

TGTTAAAAAA AAAAAAAATT AAAAAAACTG GGNGGGGGGC CCGGTACN 1908

(2) INFORMATION FOR SEQ ID NO: 40:

(i) SEQUENCE CHARACTERISTICS:
(A) LENGTH: 458 base pairs
(B) TYPE: nucleic acid
(C) STRANDEDNESS: double
(D) TOPOLOGY: linear

(xi) SEQUENCE DESCRIPTION: SEQ ID NO: 40:

CCTCAAAAAA AAAAANGAAA GGAAAGAGGT CTCTACACAA GCCCGTGATT CTTCATGGCA 60

AGGGATAACA TCAGAAATGT TTCATTTYCK GCTATTAGTT TCCATTCCTT TCCCCATCCA 120

GGCATAAAGA GAAACAAAAG ACAATGATGG TATTCTCTGT GTCCTCAGCT TTGGCACTTT 180

TGTTGATGTT GCTAAGGAGC AGTGACCTTG CTAAAAAGAC TGAATAATCC ACCCACTGAA 240

TAGCTAACCT GGGGAGGAAA TGAAAATTTC CTTTGTGGAT CTCCCCAAAT CCATTGTTGT 300

CACCAGGCCC TCCCAGAACC TCCTCAGTTC CTTCACAGTG CAACCCTGTG TACTTGGCCC 360

GCAACCCAAT AGTATTGTGC CTCACTTCAC CTTCCATGGG CAACTGCCCT CCCTTCTGGA 420

CATAAAACCT CATATTTTAA ATNAAGTTGA AATTTGAA 458

(2) INFORMATION FOR SEQ ID NO: 41:

(i) SEQUENCE CHARACTERISTICS:
(A) LENGTH: 1153 base pairs
(B) TYPE: nucleic acid
(C) STRANDEDNESS: double
(D) TOPOLOGY: linear

(xi) SEQUENCE DESCRIPTION: SEQ ID NO: 41:

GGCACAGAGC CTCCGACCCA GGTGGTCTGG AGCCTGCCGG GAGAGTGGTG GCATCTGAGA 60

GGCTGGTCGT GGACTGTGGT TGGGGGAGGT GGGAGCTGTT TTAACCGTGT GCCCCCTCTC 120

CTGTGCCGGC GTGGGCATCC CCCGGGGCAG TGGAACCCGG GCGCTCCTCC AGCTTCCGAG 180

TCCAGCCAGC CTGGGCGCGG GGCGCGCCCC GAGACACCCG AGGAGTCCGT TCCTCCCTGG 240

TTACGTGGAC TGTGGAGCTG GTCTCTTGTG GCTCAGCGCC GTGCGGAGGT TGAAGCGTAC 300

CTGCGGAGGT CGCACCAGGG CGTGAGGAGG AGGAGGAAGG GCATGAGCCG AGCTTGAGGA 360

ATCCGTGCTC CAAACTCTAC ACTCAAGGAT GCACTGCGCA ACTCTGGTGG CGATGGGCTG 420

GGGCAGATGT CCTTGGAGTT CTACCAGAAG AAGAAGTCTC GCTGGCCATT CTCAGACGAG 480

TGCATCCCAT GGGAAGTGTG GACGGTCAAG GTGCATGTGG TAGCCCTGGC CACGGAGCAG 540

GAGCGGCAGA TCTGCCGGGA GAAGGTGGGT GAGAAACTCT GCGAGAAGAT CATCAACATC 600

GTGGAGGTGA TGAATCGGCA TGAGTACTTG CCCAAGATGC CCACACAGTC GGAGGTGGAT 660

AACGTGTTTG ACACAGGCTT GCGGGACGTG CAGCCCTACC TGTACAAGAT CTCCTTCCAG 720

ATCACTGATG CCCTGGGCAC CTCAGTCACC ACCACCATGC GCAGGCTCAT CAAAGACACC 780

CTGCCCTCTG AGCGTCGCTG GATCTCTGGG AGCTCCTTGA TGGCTCCCAG ACCTTGGCTT 840

TTGGGAATTG CACTTTTGGG CCTTTGGGCT CTGGAACCTG CTCTGGGTCA TTGGTGAGAC 900

TTGGAAGGGG CAGCCCCCGC TGGCTTCTTG GTTTTGTGGT TGCCAGCCTC AGGTCATCCT 960

TTTAATCTTT GCTGACGGTT CAGTCCTGCC TCTACTGTCT CTCCATAGCC CTGGTGGGGT 1020

CCCCCTTCTT TCTCCACTGT ACAGAAGAGC CACCACTGGG ATGGGGAATA AAGTTGAGAA 1080

CATGAGTTTG GGCTGAAAAA AAAAAAAAAA AAAAAAAAAA AAAAAAAAAA AAAAAAAAAA 1140

AAAAAAAAAA AAA 1153

(2) INFORMATION FOR SEQ ID NO: 42:

(i) SEQUENCE CHARACTERISTICS:
(A) LENGTH: 1983 base pairs
(B) TYPE: nucleic acid
(C) STRANDEDNESS: double
(D) TOPOLOGY: linear

(xi) SEQUENCE DESCRIPTION: SEQ ID NO: 42:

GGCACGAGAG GGGCCGAGCC GACAAGATGT TCTTGCTGCC TCTTCCGGCT GCGGGGCGAG 60

TAGTCGTCCG ACGTCTGGCC GTGAGACGTT TCGGGAGCCG GAGTCTCTCC ACCGCAGACA 120

TGACGAAGGG CCTTGTTTTA GGAATCTATT CCAAAGAAAA AGAAGATGAT GTGCCACAGT 180

TCACAAGTGC AGGAGAGAAT TTTGATAAAT TGTTAGCTGG AAAGCTGAGA GAGACTTTGA 240

ACATATCTGG ACCACCTCTG AAGGCAGGGA AGACTCGAAC CTTTTATGGT CTGCATCAGG 300

ACTTCCCCAG CGTGGTGCTA GTTGGCCTCG GCAAAAAGGC AGCTGGAATC GACGAACAGG 360

AAAACTGGCA TGAAGGCAAA GAAAACATCA GAGCTGCTGT TGCAGCGGGG TGCAGGCAGA 420

TTCAAGACCT GGAGCTCTCG TCTGTGGARG TGGATCCCTG TGGAGACGCT CAGGCTGCTG 480

CGGAGGGAGC GGTGCTTGGT CTCTATGAAT ACGATGACCT AAAGCAAAAA AAGAAGATGG 540

CTGTGTCGGC AAAGCTCTAT GGAAGTGGGG ATCAGGAGGC CTGGCAGAAA GGAGTCCTGT 600

TTGCTTCTGG GCAGAACTTG GCACGCCAAT TGATGGAGAC GCCAGCCAAT GAGATGACGC 660

CAACCAGATT TGCCGAAATT ATTGAGAAGA ATCTCAAAAG TGCTAGTAGT AAAACCGAGG 720

TCCATATCAG ACCCAAGTCT TGGATTGAGG AACAGGCAAT GGGATCATTC CTCAGTGTGG 780

CCAAAGGATC TGACGAGCCC CCAGTCTTCT TGGAAATTCA CTACAAAGGC AGCCCCAATG 840

CAAACGAACC ACCCCTGGTG TTTGTTGGGA AAGGAATTAC CTTTGACAGT GGTGGTATCT 900

CCATCAAGGC TTCTGCAAAT ATGGACCTCA TGAGGGCTGA CATGGGAGGA GCTGCAACTA 960

TATGCTCAGC CATCGTGTCT GCTGCAAAGC TTAATTTGCC CATTAATATT ATAGGTCTGG 1020

CCCCTCTTTG TGAAAATATG CCCAGCGGCA AGGCCAACAA GCCGGGGGAT GTTGTTAGAG 1080

CCAAAAACGG GAAGACCATC CAGGTTGATA ACACTGATGC TGAGGGGAGG CTCATACTGG 1140

CTGATGCGCT CTGTTACGCA CACACGTTTA ACCCGAAGNT CATCCTCAAT GCCGCCACCT 1200

TAACAGGTGC CATGGATGTA GCTTTGGGAT CAGGTGCCAC TGGGGTCTTT ACCAATTCAT 1260

CCTGGCTCTG GAACAAACTC TTCGAGGCCA GCATTGAAAC AGGGGACCGT GTCTGGAGGA 1320

TGCCTCTCTT CGAACATTAT ACAAGACAGG TTGTAGATTG CCAGCTTGCT GATGTTAACA 1380

ACATTGGAAA ATACAGATCT GCAGGAGCAT GTACAGCTGC AGCATTCCTG AAAGAATTCG 1440

TAACTCATCC TAAGTGGGCA CATTTAGACA TAGCAGGCGT GATGACCAAC AAAGATGAAG 1500

TTCCCTATCT ACGGAAAGGC ATGACTGGGA GGCCCACAAG GACTCTCATT GAGTTCTTAC 1560

TTCGTTTCAG TCAAGACAAT GCTTAGTTCA GATACTCAAA AATGTCTTCA CTCTGTCTTA 1620

AATTGGACAG TTGAACTTAA AAGGTTTTTG AATAAATGGA TGAAAATCTT TTAACGGAGA 1680

CAAAGGATGG TATTTAAAAA TGTAGAACAC AATGAAATTT GTATGCCTTG ATTTTTTTTT 1740

CATTTCACAC AAAGATTTAT AAAGGTAAAG TTAATATCTT ACTTGATAAG GATTTTTAAG 1800

ATACTCTATA AATGATTAAA ATTTTTAGAA CTTCCTAATC ACTTTTCAGA GTATATGTTT 1860

TTCATTGAGA AGCAAAATTG TAACTCAGAT TTGTGATGCT AGGAACATGA GCAAACTGAA 1920

AATTACTATG CACTTGTCAG AAACAATAAA TGCAACTTGT TGTGCAAAAA AAAAAAAAAA 1980

AAA 1983

(2) INFORMATION FOR SEQ ID NO: 43:

(i) SEQUENCE CHARACTERISTICS:
(A) LENGTH: 1406 base pairs
(B) TYPE: nucleic acid
(C) STRANDEDNESS: double
(D) TOPOLOGY: linear

(xi) SEQUENCE DESCRIPTION: SEQ ID NO: 43:

ATGATGATGA CTTTGAAGAC GATTTTATTC CTCTTCCTCC AGCTAAGCGC CTTGAGGTTA 60

ATAGTTGGAA AAGACTCTAT AGATATTGAC ATTTCTTCAA GGAGAAGAGA AGATCAGTCT 120

TTAAGGCTTA ATGCCTAAGC NCTTGGTCTT AACTTGACCT GGGATAACTA CTTTAAAGAA 180

ATAAAAAATT CCAGTCAATT ATTCCTCAAC TGAAAGTTTA GTGGCAGCAC TTCTATTGTC 240

CCTTCACTTA TCAGCATACT ATTGTAGAAA GTGTACAGCA TACTGACTCA ATTCTTAAGT 300

CTGATTTGTG CAAATTTTTA TCGTACTTTT TAAATAGCCT TCTTACGTGC AATTCTGAGT 360

TAGAGGTAAA GCCCTGTTGT AAAATAAAGG CTCAAGCAAA ATTGTACAGT GATAGCAACT 420

TTCCACACAG GACGTTGAAA ACAGTAATGT GGCTACACAG TTTTTTTAAC TGTAAGAGCA 480

TCAGCTGGCT CTTTAATATA TGACTAAACA ATAATTTAAA ACAAATCATA GTAGCAGCAT 540

ATTAAGGGTT TCTAGTATGC TAATATCACC AGCAATGATC TTTGGCTTTT TGATTTATTT 600

GCTAGATGTT TCCCCCTTGG AGTTTTGTCA GTTTCACACT GTTTGCTGGC CCAGGTGTAC 660

TGTTTGTGGC CTTTGTTAAT ATCGCAAACC ATTGGTTGGG AGTCAGATTG GTTTCTTAAA 720

AAAAAAAAAA AAAACGACAT ACGTGACAGC TCACTTTTCA GTTCATTATA TGTACCGAGG 780

GTAGCAGTGT GTGGGATGAG GTTCGATACA GNCGTATTTA TTGCTTGTCA TGTAAATTAA 840

AAACCTTGTA TTTAACTCTT TTCAATCCTT TTAGATAAAA TTGTTCTTTG CAAGAATGAT 900

TGGTGCTTAT TTTTTCAAAA ATTTGCTGTG AACAACGTGA TGACAACAAG CAACATTTAT 960

CTAATGAACT ACAGCTATCT TAATTTGGTT CTTCAAGTTT TCTGKTGCAC TTGTAAAATG 1020

CTACAAGGAA TATTAAAAAA ATCTATTCAC TTTAACTTAT AATAGTTTAT GAAATAAAAA 1080

CATGAGTCAC AGCTTTTGTT CTGTGGTAAC CTATAAAAAA AGTTTGTCTT TGAGATTCAA 1140

TGTAAAGAAC TGAAAACAAT GTATATGTTG TAAATATTTG TGTGTTGTGA GAAATTTTTG 1200

TCATAAGAAA TTAAAAGAAC TTACCAGGAA GGTTTTTAAG TTAGAAATAT TCCATGCCAA 1260

TAAAATAGGA AATTATAAAT ATATAGTTTT AAGCCTGCAT CAGTGGGAGT CTTGGCTATG 1320

TAGTTATGTA GTTATTATGN AACCACCAAG ATTTTTTTGG CTATTTACCG TAACCAAAGG 1380

GGCCGATTAA NTGGTTTGAA GNCTTG 1406

(2) INFORMATION FOR SEQ ID NO: 44:

(i) SEQUENCE CHARACTERISTICS:
(A) LENGTH: 1391 base pairs
(B) TYPE: nucleic acid
(C) STRANDEDNESS: double
(D) TOPOLOGY: linear

(xi) SEQUENCE DESCRIPTION: SEQ ID NO: 44:

GGGCCTGAAG GCGGCRCGCC AGTCCCGAGC AGTGCTCGCT CCTGCTCGGG GCGCTGCGGC 60

CCCGGGCGTC GCCATGACCA GTGAGCTGGA CATCTTCGTG GGGAACACGA CCCTTATCGA 120

CGAGGACGTG TATCGCCTCT GGCTCGATGG TTACTCGGTG ACCGACGCGG TGGCCCTGCG 180

GGTGCGCTCG GGAATCCTGG AGCAGACTGG CGCCACGGCA GCGGTGCTGC AGAGCGACAC 240

CATGGACCAT TACCGCACCT TCCACATGCT CGAGCGGCTG CTGCATGCGC CGCCCAAGCT 300

ACTGCACCAG CTCATCTTCC AGATTCCGCC CTCCCGGCAG GCACTACTCA TCGAGAGGTA 360

CTATGCCTTT GATGAGGCCT TTGTTCGGGA GGTGCTGGGC AAGAAGCTGT CCAAAGGCAC 420

CAAGAAAGAC CTGGATGACA TCAGCACCAA AACAGGCATC ACCCTCAAGA GCTGCCGGAG 480

ACAGTTTGAC AACTTTAAAC GGGTCTTCAA GGTGGTAGAG GAAATGCGGG GCTCCCTGGT 540

GGACAATATT CAGCAACACT TCCTCCTCTC TGACCGGTTG GCCAGGGACT ATGCAGCCAT 600

CGTCTTCTTT GCTAACAACC GCTTTGAGAC AGGGAAGAAA AAACTGCAGT ATCTGAGCTT 660

CGGTGACTTT GCCTTCTGCG CTGAGCTCAT GATCCAAAAC TGGACCCTTG GACCCGTCGA 720

CTCACAGATG GATGACATGG ACATGGACTT AGACAGGAAT TTCTCCAGGA CTTGAAGGAG 780

CTCAAGGTGC TAGTGGCTGA CAAGGACCTT CTGGACCTGC ACAAGAGCCT GGTGTGCACT 840

GCTCTCCGGG AAAGCTGGGC GTCTTCTCTG AGATGGAAGC CAACTTCAAG AACCTGTCCC 900

GGGGGCTGGT GAACGTGCCG CCAAGCTGAC CCACAATAAA GATGTCAGAG ACCTGTTTGT 960

GGACCTCGTG GAGAAGTTTG TGGAACCCTG CCGCTCCGAC CACTGGCCAC TCAGCGACGT 1020

GCGGTTCTTC CTGAATCAGT ATTCAGCGTC TGTCCAATCC CTCGATGGCT TCCGACACCA 1080

GGCCCTCTGG GACCGCTACA TGGGCACCCT CCGCGGCTGC CTCCTGCGCC TGTATCATGA 1140

CTGAGGTGCC TCCCAACGTC CGCCCACGCT GACAATAAAG TTGCTCTGAG TTTGGAGACT 1200

GGTCCTCGCT CCGGGGAGCA AGTGGGGGGC GTGCAGATGT GCCTGTGTCT GTCTCTGAGC 1260

ACCTGGTGTC CGTGTACAAG GATGGATGTG TNCNGTGGCT CCTTGGGAAC TGAGACATAT 1320

CTCAGGGAAT GGTGTCTGTG CTCAGCCCAT CCACCAGAAG AGTCTGCTCA CAAAAAAAAA 1380

AAAAAAAAAA A 1391

(2) INFORMATION FOR SEQ ID NO: 45:

(i) SEQUENCE CHARACTERISTICS:
(A) LENGTH: 1569 base pairs
(B) TYPE: nucleic acid
(C) STRANDEDNESS: double
(D) TOPOLOGY: linear

(xi) SEQUENCE DESCRIPTION: SEQ ID NO: 45:

GGCACGAGTG GAGATGGCTG CGGCCGTGGC GGGGATGCTG CGAGGGGGTC TCCTGCCCCA 60

GGCGGGCCGG CTGCCTACCC TCCAGACTGT CCGCTATGGC TCCAAGGCTG TTACCCGCCA 120

CCGTCGTGTG ATGCACTTTC AGCGGCAGAA GCTGATGGCT GTGACTGAAT ATATCCCCCC 180

GAAACCAGCC ATCCACCCAT CATGCCTGCC ATCTCCTCCC AGCCCCCCAC AGGAGGAGAT 240

AGGCCTCATC AGGCTTCTCC GCCGGGAGAT AGCAGCAGTT TTCCAGGACA ACCGAATGAT 300

AGCCGTCTGC CAGAATGTGG CTCTGAGTGC AGAGGACAAG CTTCTTATTG CGACACCAGC 360

TGCGGAAACA CAAGATCCTG ATGAAGGTCT TCCCCAACCA GGTCCTGAAA GCCCTTCCTG 420

GAGGATTCCA AGTACCAAAA TCTGCTGCCC CTTTTTGTGG GGCACAACAT GCTGCTGGTC 480

AGTGAAGAGC CCAAGGTCAA GGAGATGGTA CGGATCTTAA GGGACTGTGC CATTCCTGCC 540

GCTGCTAGGT GGCTGCATTG ATGACACCAT CCTCAGCAGG CAGGGCTTTA TCAACTACTC 600

CAAGCTCCCC AGCCTGCCCC TGGTGCAGGG GGAGCTTGTA GGAGGCCTCA CCTGCCTCAC 660

AGCCCAGACC CACTCCCTGC TCCAGCACCA GCCCCTCCAG CTGACCACCC TGTTGGACCA 720

GTACATCAGA GAGCAACGCG AGRAAGGATT CTGTCATGTC GGCCAATGGG AAGCCAGATC 780

CTGACACTGT TCCGGACTCG TAGCCAGCCT GTTTAGCCAG CCCTGCGCAT AAATACACTC 840

TGCGTTATTG GCTGTGCTCT CCTCAATGGG ACATGTGGAA GAACTTGGGG TCGGGGAGTG 900

TGTTTGTCAC TTGGTTTTCA CTAGTAATGA TATTGTCAGG TATAGGGCCA CTTGGAGATG 960

CAGAGGATTC CATTTCAGAT GTCAGTCACC GGCTTCGTCC TTAGTTTTCC CAACTTGGGA 1020

CGTGATAGGA GCAAAGTCTC TCCATTCTCC AGGTCCAAGG CAGAGATCCT GAAAAGATAG 1080

GGCTATTGTC CCCTGCCTCC TTGGTCACTG CCTCTTGCTG CACGGGCTCC TGAGCCCACC 1140

CCCTTGGGGC ACAACCTGCC ACTGCCACAG TAGCTCAACC AAGCAGTTGT GCTGAGAATG 1200

GCACCTGGTG AGAGCCTGCT GTGTGCCAGG CTTTGTGCTG AGTGCTGTTA CATGTATTAG 1260

TTCCTTTACT GCTGACCACA TTGTACCCAT TTCACAGAGA AGGAGCAGAG AAATTAAGTG 1320

GCTTGCTCAA GGTCATGCAG TTAGTAAGTG GCAGAACAGG GACTTGAACC AAGCCCTCTG 1380

CTCTGAAGAC CGCGTCCTGA ATTTCTTCAC TAGAGCTTCC TCATCAGGTT ACCCAGAAGT 1440

GGGTCCCATC CACCATCCAG GTGTGCTTGG ATGTTAGTTC TCCACCCTCG AGGTGTACGC 1500

TGTGAAAAGT TTGGGAGCAC TGCTTTATAA TAAAATGAAA TATATTCTAA AAAAAAAAAA 1560

AAAAAAAAA 1569

(2) INFORMATION FOR SEQ ID NO: 46:

(i) SEQUENCE CHARACTERISTICS:
(A) LENGTH: 1924 base pairs
(B) TYPE: nucleic acid
(C) STRANDEDNESS: double
(D) TOPOLOGY: linear

(xi) SEQUENCE DESCRIPTION: SEQ ID NO: 46:

GGGCCCCCCC WCGWKTTTTT TTTTTTTTTT TTTAATTAGG ATAATGCCTT TATTAACGAG 60

AATGAAACGT TCATTCCTCC TTCCACTCCT TCTCGTTGGT TTTCTGGACA CAGCTCACCT 120

GATCCTGCTA GAAACGTTGT CAGTCTGCTT GTGGCTTCCC TCCTTGATTG ACTCACGCTG 180

TGTGATGTCT TGAGAAGTAT CTATCCACTT CATGTGAATG AGCACTCCAA TATCAGCCAA 240

CATCAATCAT TCTTACCTAA AGAATAATAA GAAAAAGTTA ATATAAAAGA CAAGGGTATA 300

AAATAAAGGT TTGAAAATGC TAGTCAACTT CAAAATTTAA AGAGTAAAAA TCCAGAGATA 360

AAGATTGGGG GTAAGTTACA GCATAAAAAA ATAGGAAGAA ACTTCATGGT GGGGGGGAAA 420

TCTAAAATTA TTCTTACATA AAATAAGTAG ACACCTGAAT TAGAATGAAA ACTGTATTTT 480

CTTTAAAATG TAAAAGCCTG ACTCTCAGTT TCACCAGTCT GAGCACAAGT TTGACTGCAA 540

CCCAAAATAT ACTATCCCTT ATGTGAAGGT ATGTGACAAC GTTGACCTCA CCAAATGAGT 600

TTTAACATCA GCTCTTTTTT CATATGAAAG CACATACCCT GCTCCCCATT CAAGTATGTC 660

TTCCATTGTC AGGCAGGCTG ACCACCTTCA GCAGGAGTCC TCCAAGAGTG CCCAACTCCC 720

CTTCCCACAG TACACAACGC TGTAGTTGTT GTCCTGCAAT CCTTTGTATT TACCTCATTC 780

TTTCCCATCT AAGTCCTCAC TGAGTTTTAA AGTTAGGGCT GGAAAAGCTA TGCCTTACTG 840

GGACAGCAAG GAACCAATTT TTTTCTGAGG GAGAAGACAT TCACCTTCAC TATATGCCTG 900

GCAGGGCCAC AGTGCACAAA ACAAAGATCA GCCTTCATTC AAGTTCCAGG TTTTTCTTCC 960

TCCCTGAATG ATTACTGCAA AGGGTATATG AAGTAAGAGT TCCCTGTTGC ACATGTACCA 1020

TCCATAAGGG ATACTATATC GTTTTGCATT CTTCCCCCCA TTCTCCACAT TGTCCTATCT 1080

TAAGTCCAAG CCCTTTTCAC TCTCAAAAAA AAAAAAAAAA TATTTTTTTC AGCACTGGTG 1140

TTCAAAAGCA ACGTTTTTAT GGTTAATGGT TTACCAGCAA CTGTTGAGAT TTCCAGTTGA 1200

GTCTTAAAAA TTGCCAATCA TTATCTAGCA GCAATGACAG ATGATTAGGA GCAGTCAAAT 1260

CCTCTGAATT CTTTCCCTAA TAGGCAGCCA TTTGAGAACT GCACTAGCTG ACATCACTAA 1320

AACATTATCA GCTAAAGCCA AAACCAAATA AAGGCCCAGA CCAACATCCT GGCTCTCTAA 1380

AACCTGTCCA AAATCATTAA GTGAAAGGCA GTAAATGCAG GACTGTGGAT CATGTCACTG 1440

CAGCTGACAA TGATTAACAA TAGGAGACAT GCAACCCCCA TTAAGGTTAA AAGTCCAAAA 1500

CTAGTCACAC GCATCTCTTT ATTGGGGAAA AGTGAGACTA TTATGCATTC TTGGTAGGTT 1560

TGCAACCTTG CATGAAGAGC ACCCATTGCA TTTCTTTCAT CTTTCAGAAA GCACCGGTAT 1620

CTGTTCCAAG GGCCTAACAG TACGAAAATA CATTCTGGCA TCACACCTCT GAACCCAAGA 1680

CTGTTCTCAT TAAAAATAAT TTTGGTTTGT AACAAAATTA TGAAATACAA TGCAAGCACC 1740

TCGGTATAGC ATTATTACTG AAACCACTTA ATTCCCAGCT TTTTGAGTTT TTTAAAAAAA 1800

CCCACTGCAC TAAGATTCAC AATTCATTGC TACATACAAA TTAAAGCTAG TAAGAACACA 1860

CTAACGTCAC AAGTTTCTCA TTCTAAAGTG CAAAAGCCTA ATCATCTGAA AGTGAACAGG 1920

GTAA 1924

(2) INFORMATION FOR SEQ ID NO: 47:

(i) SEQUENCE CHARACTERISTICS:
(A) LENGTH: 475 base pairs
(B) TYPE: nucleic acid
(C) STRANDEDNESS: double
(D) TOPOLOGY: linear

(xi) SEQUENCE DESCRIPTION: SEQ ID NO: 47:

TGGTGTGGGG CCCAGAAAMC AAGGGACCAG TGAAAACAMC CCCAGAGACT TGTATCCGCC 60

AGGAAAGCCA TTGCCAMTYC TGAGCCCTTG AAGGGCAAGG AGGGAAACAG TGTTACCAGA 120

GCCCAGTAAG AACTGCTGTC ATGAAGGAGG GGCCACCTTG TAAGAGACAT CATTACTACC 180

AGAACTGTGG TGCCAAATTG CTGGTGTCTC TCTTTGGAGA AACCAACCAG ATACATCTGC 240

TGGAGACCCA GGTGGGCACA GAGAAGGGTG GAGAGAGAAT CTGGGAAGAG AAATGGAGAA 300

TAAGCAGCAC AGTGTTATTC ATTTCTGTAA ATTCCTATGT AGAAGGCTCA GTGTTAGAAA 360

TAAAGTTATT CTACTAGTTG CAAGTTAAGT GTTTCTGTTT GTTCTGCTTT CCTGTTAGCA 420

TAAGTAAACT CCCTTTGGAA CTACACAGGT ATGTCTCTCC TTCAACATGT GTGAA 475

(2) INFORMATION FOR SEQ ID NO: 48:

(i) SEQUENCE CHARACTERISTICS:
(A) LENGTH: 346 base pairs
(B) TYPE: nucleic acid
(C) STRANDEDNESS: double
(D) TOPOLOGY: linear

(xi) SEQUENCE DESCRIPTION: SEQ ID NO: 48:

AAGGGACAGA GACCTGGATT CAGATCTCAT TTTACAATGA AGACCCCAAT GCAGAAAGTC 60

ATGTCTGAAA TTCTGAGCTT ACTCTTCTGC CTGCTGGGAC CTGCTCTGGA TGAGAGAAGG 120

GAGGAAAAGG ACTAATCAGA GGAGCCAATG AAGTCACTCC ATGAGTTTCC TGAACCCTGC 180

CCAGCTAGAG ATTAACGTYT GACCWTCAAC GTAGGACACT GTGCAGATGG CTACTTGCTG 240

GCGCACATGA AGACCAAAGC CAGGACCAAG CCCCMASCCT GCTWAACACG GCAGARTCTT 300

GCCCAGCCMA CYTCTGTGAR AATCTGCTTC CCTCCACAGC TGACCC 346

(2) INFORMATION FOR SEQ ID NO: 49:

(i) SEQUENCE CHARACTERISTICS:
(A) LENGTH: 1366 base pairs
(B) TYPE: nucleic acid
(C) STRANDEDNESS: double
(D) TOPOLOGY: linear

(xi) SEQUENCE DESCRIPTION: SEQ ID NO: 49:

TAGGTGTCAG CCGCCACCCC CCCCCCATAT GCAGATTTAC TSGGCATGGT AGTGGCCAGC 60

TTCTAACACA GCTGGTATTT CAAGTCTCCT GGGACCTCAC TCAGGAATGA TACCCCCTCA 120

GTAGAAGCAG CAGGTGATCT TAACTCCTTT CAAAGAGCAG GCCTGTCTGG GAAGCCATGT 180

CCTCAGCAGG CACAGCAACC CCTCTGGAAA TGGATCACAA ACTCACTTCT CAGCCAGGCA 240

GGCCAAGCTT CTATTGTAAC AGTAGGCACA GTATAGTCGG ATCATCACAT CAGCTGGGTT 300

TTTGGTTTAG TCATCTAGAG TCGTCTGGAC TAAAGGTCTT TCAGGTCTCC TTGCCCTGTG 360

AGTGCGTGAA CCTCCCCACC CGAATTGCCT CAGTTGTCCT GAGCCTCATG TCTCTCCTGG 420

TGGTGGGCCA GGCCCCTGCA TGGGAAGGGA GCCTGCTGCG GGGCAGGCCA GCTGGGGGTG 480

CTCACCTATG CGCAATGANA GTTATTGAAG GACTGGTTGT TGATGTTGGT GAGCGTATCC 540

TTCATGGCCA GCGCGAAGTC GGCCAGGTCA GCCAGGTGCT GCCAGCGCTC TCTCTCGGAC 600

TTGTCTTCCT GTGCCAGGGG ACCGTGGAGA AAGTGTCAGG GGCCGCTCAC TGCAGCAGCC 660

TGCTCTGCTG CCTTCCCTGG CAGTGTTCTG GGGGTGGATT CCCTACAMCT AGATGTTCAA 720

GGCCTTACTT TTCCTCCCAC AAAGGAGTCG CAGCCACGCT AGCTCTGACT TGCCACTGTG 780

ACAAAGTTCA CGTAGCAGGT CTAGGCAAAG ACTGGGCAAT TGAGCAGAGG AGACGGACCT 840

GTGAGTCTGA CCRYGAGSCG GRCCCCTTCA CCTTGGCTGG GCTGGTCCTG GTCCTTAGGT 900

TTTGTCAGGT TGTCCTTGTT TGGATCCCTC AACTAGGTGA TAAGCACTGG AGGGGGATGA 960

CCCGCCTTGG ACGTGTTTCT TTAACCTCAT CCATATAATA GGGCCGTGGG ATGGTTGTAG 1020

AGGTAAAGCA GGATGATGGT GTTTTAAGAC CAGAGCTTGG GACCAGGGCT CCTACACCTA 1080

ATTTTCTCTC CTGGTAGCTG AACAAAGGTC TAAATTAGCT TAACAAAAGA ACAGGCTGCC 1140

GTCAGCCAGA GTTCTGAAGG CCATGCTTTC AGTTTCCCTT GTTGACAATT GCTCTCCAGT 1200

TCCTATGAAA GCACAGAGCC TTAGGGGGCC TGGCCACAGA ACACAACCAT CTTAGGCCTG 1260

AGCTGTGAAC AGCAGGGGGT TGTGTGTCTG TTCTGTTTCT CTGCTTGCCG AACTTTCTCA 1320

ATAAACCCTA TTTCTTATTT ATAAAAAAAA AAAAAAAAAA AAAAAA 1366

(2) INFORMATION FOR SEQ ID NO: 50:

(i) SEQUENCE CHARACTERISTICS:
(A) LENGTH: 1405 base pairs
(B) TYPE: nucleic acid
(C) STRANDEDNESS: double
(D) TOPOLOGY: linear

(xi) SEQUENCE DESCRIPTION: SEQ ID NO: 50:

GCAGTAATTC CTGTTAGCCA CTGCATCCAC CAAAACTAGT TTATTTTTCC CCTCAAATTC 60

ATGATTTTTA CGTCTGTTAC AAAGGGAATT TTGCTGATAG CTCTTTGGGT CCCACTGTTC 120

CATTTTATGC TAATAGATTC CATTCTAGGG CCCAGCCGTC TCTTGACTGA TGGTGTTCCC 180

TTTAACCCTT GGCATGTATA ATAGAATTTT GGTGAATGAA AGAACCCAAA TAGGCCAGAT 240

AGTCCCCCCA GGCCCTGATA TCCATAAAAG GCTTGGGAAT GCATTATGTA ATTGTCCTTA 300

GTCTTTTTGT TGTTTTAGAA AAAAAAAACA AGATGGGCTC AGATGGATGC CTACGTAAAA 360

ATGGTTCCTA GCTGTGTACT CATAACTTTT CTTTGAATTG AGTAGTGAAA GGAAGGAGGA 420

GGAAAGGAAA TTAAATGTCC TTCTAGTATT CTCTGGACTC AAGTCTGACA TATGAGATAA 480

TAACCTATAT TGAAATGCCA AGAATTGTAT CTGAAACAAG AGAACAGTTT GACACATTTA 540

TCATGCCTTC ATATTACATA TTAACTGAAA CCAATTAATA AACATATGAA ATATCCATTG 600

CACAAGGCAA AGGCACCTAA ACCTTTTGTT TCTTTTTCTA CATAGCAGAA ATTGATTTTT 660

TTTTTATTTT TTTAGGGGAA CCTATATAAT TATGACCCAG TGATGTCTTT TGGTGACTTA 720

AGCTTATGAA TTCAGGTTAC AATTGAGTTG ATTCTAGATG GTTACTACCT TGAAAAGGAT 780

GTTGGTGCCT TATGTGACAC GAGCCAGAGC CTGCTGGGGA ATAAACAAAG CAGGTTTCAT 840

GCCAACACCA ACTCGTAGCT TTAGTGGGCA GATGGGGAGT GGTTCACAGA CTTCCCAAAA 900

TGTGGGGGCT TTGGGATTTT CCACACCATC CCACGTGTGT TGTTCATTCT TCCTCTTTTC 960

ACACTCTTGG ATGGATWATT TGRAAATGGT GRAAWYMMCY YYKRAATTTG CCCAATAGCC 1020

WTGRGCCACC ATTCTTWATG ACACCATAAC CAAATAGTTC CWTAATGTTG AAATATTAGA 1080

AACCTGTTAC CAGCCYKSMA KTWACCCWWA WTTTTCCCAT GTTTGTGGAA TTGATATTGA 1140

AATAGCAGGG CTAAGGAATT ACTGGCAAGT TTTAGCCTGT GGGTAATACC TTAGGGTTAT 1200

TTAAATATTT GTAATTTTAT TTAAATGTTC ATGAATGTTT GAAAGGAACA AAATTATCAG 1260

GGATGGCTCT TTGCCATGGG TCTTATTTTC ACCCTCTTTT CTGTAAGAAA AAAGAACAAT 1320

GTCTTAATGT ATTTTTAAAG TTTTTGGTAT AGTTTCTAAT TCCAATTTTA ATAAAAGTTT 1380

TWTRTAAAAA AAAAAAAAAA AAAAA 1405

(2) INFORMATION FOR SEQ ID NO: 51:

(i) SEQUENCE CHARACTERISTICS:
(A) LENGTH: 504 base pairs
(B) TYPE: nucleic acid
(C) STRANDEDNESS: double
(D) TOPOLOGY: linear

(xi) SEQUENCE DESCRIPTION: SEQ ID NO: 51:

CGGATTTTCT AGGACCCCAA AAAAAAAAAA AGGGNAAAAA AAACCCNCAA AACCANCCAA 60

AACCCCAAAA AAAAAAAAAA TCCACAAAAA CAAAAAAACT ATAAAAAAGA AAGAATTAAA 120

AACTTTCAGA GAATTACTAT TTACTTTATT AACTTACGGA TTTATTATAT AAATATATAT 180

TCACCTAGCA ACATATCTCT GCCGTCTCTC CTGCTCTCAT AATGAAGACA TAGCCGATTC 240

TCTGCCCGGG CCCCTTGCTG ATGCTCCTCC GGGTCTGCGT CGGGCGTGGG TCTCTGGGGA 300

CCCTCCAGAG GTGGAGGTGG GCTGATGGCC TGGCTGCCTG GTGGTTGATG GTTTTGCTCC 360

CCCTACCTTT TTTTTTTGAG TTTATTCTGA TTGATTTTTT TTCTTGGTTT CTGGATAAAC 420

CACCCTCTGG GGACAGGATA ATAAAACATG TAATATTTTT AAGAAGGAAA AAAAAAAAAA 480

AAAAAACTNG GGGGGGCCC CGAA 504

(2) INFORMATION FOR SEQ ID NO: 52:

(i) SEQUENCE CHARACTERISTICS:
(A) LENGTH: 777 base pairs
(B) TYPE: nucleic acid
(C) STRANDEDNESS: double
(D) TOPOLOGY: linear

(xi) SEQUENCE DESCRIPTION: SEQ ID NO: 52:

NAAGTATCTT GGCCAGTTTA TTACAGAGGA CGATAAATGA TTCCATGTGG ATAGGGCATA 60

ACATACAGAG AATGAGACTA TGCCAGAAAT GGGAGGAGGC ATTTGAAACA ACATGAGTAT 120

CTCAGGGACA GATGGATTGA TTCTGCTATT GGTAGGCCTG GAAGCAANGG TCAGAAGTAG 180

CAAAAAATGG ATACCAAAAG CACTATTWGT CACCCAAGCT AAGTGGAATA GCTGGCCCAG 240

TAGGAGAAAT GCAGGTTTTG CTCTACACTA AGTTCTCCAA CTCTTGATAA GCCTCCAAAA 300

ACAAATGTTA GGGGAAAAAA ACGCAGCTGG TTATGAAAAG ATATATCTCA TTTCATTAAA 360

AAATCAATGT CAATGCTGTT AATAGAATCC TTTTATCTTC AGGACAGAGG CAATGCCCTA 420

AACAAACACC AGCTCAAGAG CCTCTGATGC CAACCTAGAG GGTACCCAAA CACAAACTTA 480

GCATAGAGGT AAGAATCTCT ATGTCTTTTG GTGGAGGCAA AGCCATTTGG TTGGTACTTC 540

ACAGGAACAT CTTTCTACCA AGTCTTCATC ATATGGTATG TGCCACGAGT CTCCAGTTGT 600

TTGCACCACT GTGTCATAGC TGAGAATACG CTGAAAGGTT AGTTTTGATC CTGGAAACCT 660

ATTTACAATT GCCAGCTGAT GTCCCTGCTG CCACTTAAAA AAGGCTTGGG TCTGGCATAG 720

GCAGAMAGGC CTGTGGTCCC CTCGTGCCGA TTCTNGGCTC GAGGCCAATT NCCTTAT 777

(2) INFORMATION FOR SEQ ID NO: 53:

(i) SEQUENCE CHARACTERISTICS:
(A) LENGTH: 602 base pairs
(B) TYPE: nucleic acid

(C) STRANDEDNESS: double
(D) TOPOLOGY: linear

(xi) SEQUENCE DESCRIPTION: SEQ ID NO: 53:

ATGACTACAG TGTTATACCC TCCAATCTTT GCAGGTGGGC ATGGAACACT GCTTGTATCA 60

CTCTGTGCAC GGTATAAATC CATATATCCA CAAAAACACA CATCCATCCA TCAACATATA 120

CATGGTTTGG GATGAGCAGG TCAATAGTTT TGAGAGGGAG TTTGTTCCTT TTTTTTTTCT 180

CATTATACTC TTAAATTGTT GTCAGTTATC AAACAAACAA ACAGAAAAAT TGTTTGGAAA 240

AACCTTGCAT ACGCCTTTTC TATCAAGTGC TTTAAAATAT AGACTAAATA CACACATCCT 300

GCCAGTTTTT TCTTACAGTG ACAGTATCCT TACCTGCCAT TTAATATTAG CCTCGTATTT 360

TTCTCACGTA TATTTACCTG TGACTTGTAT TTGTTATTTA AACAGGAAAA AAAACATTCA 420

AAAAAAGAAA AATTAACTGT AGCGCTTCAT TATACTATTA TATTATTATT ATTATTGTGA 480

CATTTTGGAA TACTGTGGAA GTTTTATCTC TTGCATATAC TTTATACGGA AGTATTACGC 540

CTTAAAAATA CGAAAATAAA TTTTACAAGG TTCCGGTTTT GGTGGTGGAA AGAGTAAATT 600

GA 602

(2) INFORMATION FOR SEQ ID NO: 54:

(i) SEQUENCE CHARACTERISTICS:
(A) LENGTH: 1749 base pairs
(B) TYPE: nucleic acid
(C) STRANDEDNESS: double
(D) TOPOLOGY: linear

(xi) SEQUENCE DESCRIPTION: SEQ ID NO: 54:

AGTCACTGAC TTGGAGCCGC TCGGGGGAAG TCCCGCCCAG ACAGGCGGTG GGTGGGAATG 60

CCTCACTTCA GTTTGAAGAG GGTCCGGATC CAAAGGGGTT AAAACGAGCG AACCCCGATC 120

CCCGACCACA CTTCCCGCCT CCCTAAAACG CACACCCCGC TAGCCATGGG CAGCCGCGAC 180

CACCTGTTCA AAGTGCTGGT GGTGGGGGAC GCCGCAGTGG GCAAGACGTC GCTGGTGCAG 240

GATTATTCCC AGGACAGCTT CAGCAAACAC TACAAGTCCA CGGTGGGAGT GGATTTTGCT 300

CTGAAGGTTC TCCAGTGGTC TGACTACGAG ATAGTGCGGC TTCAGCTGTG GGATATTGCA 360

GGGCAGGAGC GCTTCACCTC TATGACACGA TTGTATTATC GGGATGCCTC TGCCTGTGTT 420

ATTATGTTTG ACGTTACCAA TGCCACTACC TTCAGCAACA GCCAGAGGTG GAAACAGGAC 480

CTAGACAGCA AGCTCACACT ACCCAATGGA GAGCCGGTGC CCTGCCTGCT CTTGGCCAAC 540

AAGTGTGATC TGTCCCCTTG GGCAGTGAGC CGGGACCAGA TTGACCGGTT CAGTAAAGAG 600

AACGGTTTCA CAGGTTGGAC AGAAACATCA GTCAAGGAGA ACAAAAATAT TAATGAGGCT 660

ATGAGAGTCC TCATTGAAAA GATGATGAGA AATTCCACAG AAGATATCAT GTCTTTGTCC 720

ACCCAAGGGG ACTACATCAA TCTACAAACC AAGTCCTCCA GCTGGTCCTG CTGCTAGTAG 780

TGTTTGGCTT ATTTTCCATC CCAGTTCTGG GAGGTCTTTT AAGTCTCTTC CCTTTGGTTG 840

CCCACCTGAC CATTTTATTA AGTACATTTG AATTGTCTCC TGACTACTGT CCAGTAAGGA 900

GGGCCCATTG TCACTTAGAA AAGACACCTG GAACCCATGT GCATTTCTGC ATCTCCTGGA 960

TTAGCCTTTC ACATGTTGCT GRCTCACATT AGTGCCAGTT AGTGCCTTCG GTGTAAGATC 1020

TTCTCATCAG CCCTCAATTT GTGATCCGGA ATTTTGTGAG AAGGATTAGA AATCAGCACC 1080

TGCGTTTTAG AGATCATAAT TCTCACCTAC TTCTGAGCTT ATTTTTCCAT TTGATATTCA 1140

TTGATATCAT GACTTCCAAT TGAGAGGAAA ATGAGATCAA ATGTCATTTC CCAAATTTCT 1200

TGTAGGCCGT TGTTTCAGAT TCTTTCTGTC TTGGAATGTA AACATCTGAT TCTGGAATGC 1260

AGAAGGAGGG GTCTGGGCAT CTGTGGATTT TTGGCTACTA GAAGTGTCCC AGAAGTCACT 1320

GTATTTTTGA AACTTCTAAC GTCATAATTA AGTTTCTCTT GTCTTGGCAT CAAGAATAGT 1380

CAAGTTTTTT GGCCGGGCAT GGTGGCTCAT GCCKGTAATC CCAGCACTTG GGGAGGCCAA 1440

GGCAGGCGGA TCACATGAGG CCAGGAATTC GAGACCAACC TGGTCAGCAT GGCAAAACCC 1500

CGTCTCTACT AAAAGTACAA AAATTAGCCA GGCGTGATGG CACGTGTCTG TAATCCCAGC 1560

TACTCTGGAG ACTGAGGTGG GAGAATCGCT TGAGACTGGG AGGCAGAGGT TGCAGTGAAC 1620

CGAGATCATG CCACCGCACT TCAGCCTGGG TGACAGAGAA GGACTCCGTC TCAAAAAAAA 1680

AAAAAAAAAA AAAACTCGAG GGGGGGCCCG GTACCCAAAT CGCCSTGATA GTGATCGTAW 1740

ACAATCNAA 1749

(2) INFORMATION FOR SEQ ID NO: 55:

(i) SEQUENCE CHARACTERISTICS:
(A) LENGTH: 1896 base pairs
(B) TYPE: nucleic acid
(C) STRANDEDNESS: double
(D) TOPOLOGY: linear

(xi) SEQUENCE DESCRIPTION: SEQ ID NO: 55:

AAAGAGATGG GCTCTTTATT TTCTCGAAAA ACCAATTTGG AGTTACTCAT TTTTCCATAA 60

CATTAAATTT CTTACAGTGA ACTACATATT GTCCATAAGT GCTTCATCAG GACTCATCGC 120

CCTCCTGTCT ACTGGCTCCA AATAGACCAT GTCAGCTTCA CCCCCTGGCT TTGTGTCTAT 180

GGGTGGCCTG TGGTATATGG AAAAGTAGCA GGGTGGTCAG GGTGGGAGAC ACAAGATGTT 240

TTTATAGTCT AGAGCCTTTA AAAAACCCAG CAGAATGTAA TTCAGTATTT GTTTATTGGC 300

TGTTTTTTGA CAGATTGTTG AAATTAAATG AATTGAAAGG GAAACTCAGA GTACTAGGAC 360

GTTTATTAAA AGGAAAAAAA TGTCTTGCAA TGTGCTGTAA TCACAAGAGG AGAAAATAAC 420

TTGTTTCCTT GATCTGTCAG AGGTCACAGT AACCTGGGCC GAGCTGTTAT TATTTATTAT 480

ATAATAGTAG TAGGAAGTTA ATAACTGGTT CTCTGTGTTC CAAGCACAAT ATTACAACTT 540

CTTTTGAACC GTAAATATCA GAATGAATCC TCTTCCCAGG GGATTGAACA GAAGCTTAAT 600

GTTTACAAGT GTTTGAATTT GTGATCTGAA ATAACACAAA ATTAAAAACA TGATTTCTCT 660

AATTTTCCAA CTAGAGGAAG AGAAACTTGT GGAAAAGTTC TTTTTTTTTC TTTTTTTTTT 720

CTTAAAGAAG GGCAGCCAAG GTAGTAACCT AAAAATAGTG CCCAGGCATA TGAGAGTTGT 780

CCTACGAGGT TAAAGAACAC ACTGTTCCAC TGTATGGCTT TGGCCCTGAG TGGCCAGGGA 840

GGTCAACTTG ACCCTGCCAT GTTGGTTTGA CTTACTAAGA CACAGGAATC ATTGTTTTCC 900

TTGACCAGGG TCTCACACCC TGGAGGAATG TTAAGTAAGA GAAAGAACCT CTTTCCTGAA 960

TATTGACATG TAAAAGACCA AAGTAATTTT TCTGAACTTC TGCAATTCTG AGAACTCTCC 1020

AAGGAATTTA CAGTGATTTT AGTGCTTGTC AGCATTTTTC CATGAGGACT TTCATACATT 1080

TGACTCTTTA GTTCACAGGT TCCCATTGAT TGTGAGCAAG ATATTTATCT CTTTAGCCCT 1140

TGGGGATCCA GCTGAGAGCA ATCTCTTGCA TTTTTTTACC CGTGTATGTA CAGATATCAT 1200

TTCTTGTGTA TGCCATGACT TGAAAAAGTT TGGGAAGCTC TTTAGCAATA TCAGCTAAAA 1260

GGATATGAAA TCACAGGTGA TAGCAGTTGT CATTCAGTAA TTTCCTACAA GCAGCACCCC 1320

AAAGGAAATA TAGTCCTAAT CTTTACTATC CACTTCTAAA TTTAATGTGA ATTTCATACA 1380

TGTTATTAGT TGTTTTCTTT ATAATTTTAT AAAAATTATT CATCGGGAGT TTAACTTCCA 1440

CTTCCATGCT ATCGGATGTG TTGGGCTCCA TGCAAGAACT TGGAAGAAAA ACAGGCAGGA 1500

ATGCATTTGC ATAATGACCC AGATCATCAT TTTCTGCAAC TGAGAATTAT ATTTCATCAT 1560

TGCTTCTAGA AGTCTGCAAT TCTTTACTTT TCTTTGGTGC ATTATTATCT AGGTGCCATC 1620

ACTGGATAAT GTGGAGTGAC TAGAGAAGTC AYATATCACT GTAAGGTACA GTTAGGGGTA 1680

ACACTTTAGA GGTTTATTAT TTTTAAAAAA CTTTTCTTGA ACTCCTGGGC CAACATGGGT 1740

GAAACCCCGT CTTCTTACTT AAAAATACCC AAAATTAGGC CAGGGGCGTG GATGGGTGGG 1800

GTGCCTGTTA ATCTTCAGCT ACTTNGGGGA GGGCTTGAAG CCAGGGAGGA ACTGCCCTGG 1860

ANCCCCGGGG NGGGCCAGNA GGTTTGCCAG TTGAGT 1896

(2) INFORMATION FOR SEQ ID NO: 56:

(i) SEQUENCE CHARACTERISTICS:
(A) LENGTH: 1753 base pairs
(B) TYPE: nucleic acid
(C) STRANDEDNESS: double
(D) TOPOLOGY: linear

(xi) SEQUENCE DESCRIPTION: SEQ ID NO: 56:

TCTTTTTAAA ATAGACATTT GTGGGGCTCA CACAATATAT GAAATAGTAC CCTCTAAAAA 60

AGAGAAAAAA AAAATCAGGC GGTCAAACTT AGAGCAACAT TGTCTTATTA AAGCATAGTT 120

TATTTCACTA GAAAAAATTT AATATCAAGG ACTATTACAT ACTTCATTAC TAGGAAGTTC 180

TTTTTAAAAT GACACTTAAA ACAATCACTG AAAACTTGAT CCACATCACA CCCTGTTTAT 240

TTTCCTTAAA CATCTTGGAA GCCTAAGCTT CTGAGAATCA TGTGGCAAGT GTGATGGGCA 300

GTAAAATACC AGAGAAGATG TTTAGTAGCA ATTAAAGGCT GTTTGCACCT TTAAGGACCA 360

GCTGGGCTGT AGTGATTCCT GGGGCCAGAG TGGCATTATG TTTTTACAAA ATAATGACAT 420

ATGTCACATG TTTGCATGTT TGTTTGCTTG TTGAATTTTT GAACAGCCAG TTGACCAATC 480

ATAGAAAGTA TTACTTTCTT TCATATGGTT TTTGGTTCAC TGGCTTAAGA GGTTTCTCAG 540

AATATCTATG GCCACAGCAG CATACCAGTT TCCATCCTAA TAGGAATGAA ATTAATTTTG 600

TATCTACTGA TAACAGAATC TGGGTCACAT GAAAAAAAAT CATTTTATCC GTCTTTTAAG 660

TATATGTTTA AAATAATAAT TTATGTGTCT GCATATTGCA GAACAGCTCT GAGAGCAACA 720

GTTTCCCATT AACTCTTTCT GACCAATAGT GCTGGCACCG TTGCTTCCTC TTTGGGAAGA 780

GGAAAGGGTG TGTGAACATG GCTAACAATC TTCAAATACC CAAATTGTGA TAGCATAAAT 840

AAAGTATTTA TTTTATGCCT CAGTATATTA TTATTTAATT TTTTAGGTAA TGCCTATCTC 900

TTGGTCTATT AAGGAAAGAA GCAATCAGTA GAGAATTCAG GATAGTTTTG TTTAAATTCT 960

TGCAGATTAC ATGTTTTTAC AGTGGCCTGC TATTGAGGAA AGGTATTCTT CYATACAACT 1020

TGTTTTAACC TTTGAGAACA TTGACAGAAA TTATGCAATG GTTTGTTGAG ATACGGACTT 1080

GATGGTGCTG TTTAATCAGT TTGCTTCCAA AGTGGCCTAC TCAAGAGGCC CTAAGACTGG 1140

TAGAAATTAA AAGGATTTCA AAAACTTTCT ATTCCTTTCT TAAACCTACC AGCAAACTAG 1200

GATTGTGATA GCAATGAATG GTATGATGAA GAAAGTTTGA CCAAATTTGT TTTTTTGTTG 1260

TTGTTGTTGT TTTGAATTTG AAATCATTCT TATTCCCTTT AAGAATGTTT ATGTATGAGT 1320

GTGAAGATGC TAGCGAACCT ATGCTCAGAT ATTCATCGTA AGTCTCCCTT CACCTGTTAC 1380

AGAGTTTCAG ATCGGTCACT GATAGTATGT ATTTCTTTAG TAAGAATGTG TTAAAATTAC 1440

AATGATCTTT TAAAAAGATG ATGCAGTTCT GTATTTATTG TGCTGTGTCT GGTCCTAAGT 1500

GGAGCCAATT AAACAAGTTT CATATGTATT TTTCCAGTGT TGAATCTCAC ACACTGTACT 1560

TTGAAAATTT CCTTCCATCC TGAATAACGA ATAGAAGAGG CCATATATAT TGCCTCCTTA 1620

TCCTTGAGAT TTCACTACCT TTATGTTAAA AGTTGTGTAT AATTGTTAAA ATCTGTGAAA 1680

GAATAAAAAG TGGATTTAAA TTAAAAAAAA AAAAAAAAAA AAAAAAAAAA AAAAAAAAAA 1740

AAAAAAAAGG GGG 1753

(2) INFORMATION FOR SEQ ID NO: 57:

(i) SEQUENCE CHARACTERISTICS:
(A) LENGTH: 1220 base pairs
(B) TYPE: nucleic acid
(C) STRANDEDNESS: double
(D) TOPOLOGY: linear

(xi) SEQUENCE DESCRIPTION: SEQ ID NO: 57:

GCGGAAGTTA CTGCAGCCGC GGTGTTGTGC TGTGGGGAAG GGAGAAGGAT TTGTAAACCC 60

CGGAGCGAGG TTCTGCTTAC CCGAGGCCGC TGCTGTGCGG AGACCCCCGG GTGAAGCCAC 120

CGTCATCATG TCTGACCAGG AGGCAAAACC TTCAACTGAG GACTTGGGGG ATAAGAAGGA 180

AGGTGAATAT ATTAAACTCA AAGTCATTGG ACAGGATAGC AGTGAGATTC ACTTCAAAGT 240

GAAAATGACA ACACATCTCA AGAAACTCAA AGAATCATAC TGTCAAAGAC AGGGTGTTCC 300

AATGAATTCA CTCAGGTTTC TCTTTGAGGG TCAGAGAATT GCTGATAATC ATACTCCAAA 360

AGAACTGGGA ATGGAGGAAG AAGATGTGAT TGAAGTTTAT CAGGAACAAA CGGGGGGTCA 420

TTCAACAGTT TAGATATTCT TTTTATTTTT TTTCTTTTCC CTCAATCCTT TTTTATTTTT 480

AAAAATAGTT CTTTTGTAAT GTGGTGTTCA AAACGGAATT GAAAACTGGC ACCCCATCTC 540

TTTGAAACAT CTGGTAATTT GAATTCTAGT GCTCATTATT CATTATTGTT TGTTTTCATT 600

GTGCTGATTT TTGGTGATCA AGCCTCAGTC CCCTTCATAT TACCCTCTCC TTTTTAAAAA 660

TTACGTGTGC ACAGAGAGGT CACCTTTTTC AGGACATTGC ATTTTCAGGC TTGTGGTGAT 720

AAATAAGATC GACCAATGCA AGTGTTCATA ATGACTTTCC AATTGGCCCT GATGTTCTAG 780

CATGTGATTA CTTCACTCCT GGACTGTGAC TTTCAGTGGG AGATGGAAGT TTTTCAGAGA 840

ACTGAACTGT GGAAAAATGA CCTTTCCTTA ACTTGAAGCT ACTTTTAAAA TTTGAGGGTC 900

TGGACCAAAA GAAGAGGAAT ATCAGGTTGA AGTCAAGATG ACAGATAAGG TGAGAGTAAT 960

GACTAACTCC AAAGATGGCT TCACTGAAGA AAAGGCATTT TAAGATTTTT TAAAAATCTT 1020

GTCAGAAGAT CCCAGAAAAG TTCTAATTTT CATTAGCAAT TAATAAAGCT ATACATGCAG 1080

AAATGAATAC AACAGAACAC TGCTCTTTTT GATTTTATTT GTACTTTTTG GCCTGGGATA 1140

TGGGTTTTAA ATGGACATTG TCTGTACCAG CTTCATTAAA ATAAACAATA TTTGTAAAAA 1200

TCAWAAAAAA AAAAAAAAAA 1220

(2) INFORMATION FOR SEQ ID NO: 58:

(i) SEQUENCE CHARACTERISTICS:
(A) LENGTH: 1049 base pairs
(B) TYPE: nucleic acid
(C) STRANDEDNESS: double
(D) TOPOLOGY: linear

(xi) SEQUENCE DESCRIPTION: SEQ ID NO: 58:

TCGCGCCTGC AGACACAGCA TCTACTCAGC GTGGGTCACC TCTGTGAACA TCACTGACTG 60

CAAGCCTCCC TCAATTTCTG GTGCAGCCCA TCAGGGACCC ACAGCGCCTG GGAGGATGGT 120

GCGGATCTTG GCCAATGGGG AAATCGTGCA GGACGACGAC CCCCGAGTGA GGACCACTAC 180

CCAGCCACCA AGAGGTAGCA TTCCTCGACA GAGCTTCTTC AATAGGGGCC ATGGTGCTCC 240

CCCAGGGGGT CCTGGCCCCC GCCAGCAGCA GGCAGGTGCC AGGCTGGGTG CTGCTCAGTC 300

CCCCTTCAAT GACCTCAACC GGCAGCTGGT GAACATGGGC TTTCCGCAGT GGCATCTCGG 360

CAACCATGCT GTGGAGCCGG TGACCTCCAT CCTGCTCCTC TTCCTGCTCA TGATGCTTGG 420

TGTTCGTGGC CTCCTCCTGG TTGGCCTTGT CTACCTGGTG TCCCACCTGA GTCAGCGGTG 480

ACCTCTGAGG GCTGATAGGG GTGGGTTTGT TGAGAGGGAC TTGCTGGGCC TTGGTGTGAG 540

AGCAGGCATA TTTGGAGGGG ATCTGGTGGT GCCTTGAAGG TATGATCAGA GAGGGGACCA 600

CAGGTGTGTG TTTCCCCTTT GTGTTAAGCG TGAGGCAGAG GGAGACGTTA GTCCCAGCAT 660

TTCCCAAAGT GTGGGTGGGT CCGTTGGTTC CCGAGATACT TTTAGGTGGT ATGGGGCCTG 720

CATTAAGTGG CACAAAATCA GAGCAAGAAA GCGATGCCCT TCCCAATTCT CTCAATCCTT 780

TTATGCCGAG AAGATCTCAG CTGGATGCCA ACATGTTCCG ATGCCTGTGG AAGACATGCC 840

GACGTCTCCT CTGCCTAGGG AGCAGGACTT GGGCTTAGGG CAGGTGGAAA AAATTCCAGA 900

CTTTTTTAGC ACTGTTTTTG TTTTAATGGT ATATTTTTAT TGGCTACTTT ATTGTTTAGG 960

ACAAGTGGTA GTGGCATTCT ATTTATTGTG ACCTTTTCAA TAAATAGATT TAAGTAAAAA 1020

AAAAAAAAAA AAAACTCGAG GGGGGGCCC 1049

(2) INFORMATION FOR SEQ ID NO: 59:

(i) SEQUENCE CHARACTERISTICS:

(A) LENGTH: 1776 base pairs
(B) TYPE: nucleic acid
(C) STRANDEDNESS: double
(D) TOPOLOGY: linear

(xi) SEQUENCE DESCRIPTION: SEQ ID NO: 59:

AAAGAGGATG TGMAGCTAGA GGTCCCCGAT GGCTGGTCGG ATGGGAAGCA CAAGGCTGAG 60

GGACTGGATT GTAAAGGCAC TAAGTCGTTC TGCGGTGAGA ATCAGACATG GGGGACCTCT 120

AGCTTCACAT CCTCTTTCCT TGCAGSTCTG GACATCCTGA GCCCAAGTCC CCCACACTCA 180

GTGCAGTGAT GAGTGCGGAA GTGAAGGTGA CAGGGCAGAA CCAGGAGCAA TTTCTGCTCC 240

TAGCCAAGTC GGCCAAGGGG GCAGCGCTGG CCACACTCAT CCATCAGGTG CTGGAGGCCC 300

CTGGTGTCTA CGTGTTTGGA GAACTGCTGG ACATGCCCAA TGTTAGAGAG CTGGCTGAGA 360

GTGACTTTGC CTCTACCTTC CGGCTGCTCA CAGTGTTTGC TTATGGGACA TACGCTGACT 420

ACTTAGCTGA AGCCCGGAAT CTTCCTCCAC TAACAGAGGC TCAGAAGAAT AAGCTTCGAC 480

ACCTCTCAGT TGTCACCCTG GCTGCTAAAG TAAAGTGTAT CCCATATGCA GTGTTGCTGG 540

AGGCTCTTGC CCTGCGTAAT GTGCGGCAGC TGGAAGACCT TGTGATTGAG GCTGTGTATG 600

CTGACGTGCT TCGTGGCTCC CTGGACCAGC GCAACCAGCG GCTCGAGGTT GACTACAGCA 660

TCGGGCGGGA CATCCAGCGC CAGGACCTCA GTGCCATTGC CCGAACCCTK AANAAAAACC 720

ATTAAAGTTA CGACGGCAGC AGCAGCCGCA GCCACATCTC AGGACCCTGA GCAACACCTG 780

ACTGAGCTGA GGGAACCAGC TCCTGGCACC AACCAGCGCC ASCCAGCAAG AAAGCCTCAA 840

AGGGCAAGGG GCTCCGAGGG ANCGCCAAGA TTTGGTCCAA GTCGAATTGA AAGRACTGTC 900

GTTTCCTCCC TGGGGATGTG GGGTCCCAGC TGCCTGCCTG CCTCTTAGGA GTCCTCAGAG 960

AGCCTTCTGT GCCCCTGGCC AGCTGATAAT CCTAGGTTCA TGACCCTTCA CCTCCCCTAA 1020

CCCCAAACAT AGATCACACC TTCTCTAGGG AGGAGKCAAA TGTAGGTCAT GTTTTTGTTG 1080

GTACTTTCTG TTTTTTGTGA CTTCATGTGT TCCATTGCTC CCCGCTGCCA TGCTCTCTCC 1140

CTTGTTTCCT TAAGAGCTCA GCATCTGTCC CTGTTCATTA CATGTCATTG AGTAGGTGGG 1200

TAGCCCTGAT GGGGGTCGCT CTGTCTGGAG CATAACCCAC AGGCGTTTTT TCTGCCACCC 1260

CATCCCTGCA TGCCTGATCC CCAGTTCCTA TACCCTACCC CTGACCTATT GAGCAGCCTC 1320

TGAAGAGCCA TAGGGCCCCC ACCTTTACTC ACACCCTGAG AATTCTGGGA GCCAGTCTGC 1380

CATGCCAGGA GTCACTGGAC ATGTTCATCC TAGAATCCTG TCACACTACA GTCATTTCTT 1440

TTCCTCTCTC TGGCCCTTGG GTCCTGGGAA TGCTGCTGCT TCAACCCCAG AGCCTAAGAA 1500

TGGCAGCCGT TTCTTAACAT GTTGAGAGAT GATTCTTTCT TGGCCCTGGC CATCTCGGGA 1560

AGCTTGATGG CAATCCTGGA AGGGTTTAAT CTCCTTTTGT GAGTTTGGTG GGGAAGGGAA 1620

GGGTATATAG ATTGTATTAA AAAAAAAAAG GTATATATGC ATATATCTAT ATATAATATG 1680

ACGCAGAAAT AAATCTATGA GAAATCTATC TACAAAMWAA AAAAAAAAAA AAAAAAAAAA 1740

AGGAATTCGA TNTCAAGCTT ATCGATACCG TCNACC 1776

(2) INFORMATION FOR SEQ ID NO: 60:

(i) SEQUENCE CHARACTERISTICS:
(A) LENGTH: 443 base pairs
(B) TYPE: nucleic acid
(C) STRANDEDNESS: double
(D) TOPOLOGY: linear

(xi) SEQUENCE DESCRIPTION: SEQ ID NO: 60:

ACAGATAAAT AAATAAATAA TAAATTAAAT TAAATAAAAA ATCTGAGCTA ATCTGAATAA 60

ATTGAGAGAT TTCACATGAA AGCCAGGATT TCTGGCTTCC CAGGAACAGT CAGAAGAGCT 120

AGCTAGCAAC ACTGGTCTGC TTGGCTACCT TCTTTGGAAC AACATGAAAT CTAGCTCCCT 180

TTTTTTTTTT TTTTTGGCCC ACTTCATCCA TTCACATGAC CTGCCTGGCC TCTGCAGGTA 240

AGTGAGTATG CAACAAAAAT GTAGCACAGG TTTTGTCGCT GAACTACGTG GTTTCAGGTC 300

CAGCTCTGCC ACTTGCTAGC ATGACCTCGT GCCGAATTCC NGCACGAAGT TTTTTTTTTT 360

TTTTTCAGTG CTCCAGTCCC CCTATTGGAG AATCCTGCCC CCCCCTGGGA CAGAATGTTC 420

ACCCTGGCCC CGCGANTCCC TGA 443

(2) INFORMATION FOR SEQ ID NO: 61:

(i) SEQUENCE CHARACTERISTICS:
(A) LENGTH: 2888 base pairs
(B) TYPE: nucleic acid
(C) STRANDEDNESS: double
(D) TOPOLOGY: linear

(xi) SEQUENCE DESCRIPTION: SEQ ID NO: 61:
   TTAATGTTGT CAATAACCAC CAGGCCAAAC AGAATTTATA TGACCTGGAT GAAGATGATG 60
   ATGGTATAGC TTCCGTTCCT ACTAAACAGA TGAAGTTTGC AGCCTCAGGC GNCTTTCTCC 120
   ACCACATGGC TGGGCTAAGC AGTTCCAAGC TTTCCATGTC CAAGGCCCTC CCTCTCACCA 180
   AAGTGGTTCA GAATGATGCA TACACAGCTC CTGCTCTCCC TTCCTCTATT CGAACAAAAG 240
   CCTTGACCAA CATGTCCCGG ACACTGGTGA ACAAGGAAGA ACCCCCCAAA GAGCTGCCAG 300
   CTGCTGAGCC TGTTCTCAGC CCATTGGAAG GCACCAAGAT GACTGTGAAT AATCTGCACC 360
   CTCGAGTCAC TGAGGAGGAC ATTGTTGAGC TTTTCTGTGT GTGTGGGGCC CTCAAGCGAG 420
   CTCGACTGGT CCATCCTGGG GTAGCGGAGG TGGTGTTTGT GAAAAAGGAC GATGCCATCA 480
   CCGCATATAA GAAGTACAAC AACCGGTGTC TGGACGGGCA GCCGATGAAG TGCAACCTTC 540
   ACATGAATGG GAATGTTATC ACCTCAGACC AGCCCATCCT GCTGCGGCTG AGTGACAGCC 600
   CATCAATGAA AAAGGAGAGC GAGCTGCCTC GCAGGGTGAA CTCTGCCTCC TCCTCCAACC 660
   CCCCTGCYGA AGTGGACCCT GACACCATCC TGAAGGCACT CTTCAAGTCC TCAGGGGCCT 720
   CTKTGACCAC GCAGCCCACA GAATTCAAAA TCAAGCTTTG AGCAGGGGAG TGAGGCAGCC 780
   AGAAGTGGGG GCAGAGGAGG GTGGCTCTGT TTCCCCAAGG CAAAGCTTAT GACCAATGGG 840
   CCATCGGACT GGAGACCCCT GATTGTGGGA AGGGTTGCCA GGGATAAAGA GCTTCCTCAC 900
   TGGATGGGAC CCGCCTTTCT GTGTTGTGTT CTGCCCTGTG CTCTTCTCTC TACGTTAACG 960
   TTTCCTGTAG TATGTTTCTT CATCTCATCG CCAAGGTAGG CTTGTGTTTT TCAGTGTGTG 1020
   CCTCCCCGAG CCTCAGCCCC AAGCTGATTT CTTATCTGGA AATGGTACAC TGAATTCTCT 1080
   GGGTGGCTTT CTTGTGGCCC CATGGGATGC AGCGTGGGGG CTGTCTGAAG GACCCTGCTT 1140
   TTTCCAGGGG CCGAGGGGCT GCCTTTCCTT TGTGTGTATT AAGCTTTTCA AACAATGGAG 1200
   GGGATGGAGA GCCCTGGTGT CCTGACGGGA GCCAGGTCGG CCTGAGAGCT GTGCCGCTCC 1260
   TCTGTCTTGT CAGTGGAGGT GCCTGGGTGG GGAGCAGGTC TCAGGCCTCT TGTCCTCTCC 1320
   CCAGTGGCTC CAGGCCTCAC TAGTGGCAAG GGCAGGATGA GGCTGCACCG CTGGGAAGAG 1380
   TCTATCTAAG YTCTTGGCTT GGAGTCCCGT GTCGTCTCCR CCCAGAGGAA GTTCTCCAGA 1440
   GTTCACCTTT CCCTTTTCCT TGAGTTGTGC TGAATGCCCC ACCCCAGCTC TCTTTCCCTT 1500
   CTGGGTGTCT TTGCTGGGAG GGGGCTGTGT TGTGAGCCCT CCCGGTTCTC ACCTCGCCTG 1560
   GCACTTAACC ACACCCTGGT TTTGTGTAGC CGCCAGCTCT CTTCTGGTTG GGCCTTTGAA 1620
   AGGCTCAGCC TCCCATTGTG CAGTGCTTGG GTTTGGAGCT TATTTGAATG GAAGAGGTCA 1680
   GTTTGTTCCT GGCTCTCCAT TTCTGGCCTC AGTTGTCTAC AGGACAGTGG TCAGGGATGC 1740
   CTGGAGGCAT ATATCCAGCT GCCACCAAGG GGCACTGTTT GTTCCCACTT ATGTGAGTGA 1800
   CCCCATCCAT CCATGACCAG AGGATTATTT TCCTGCCTTG GCAGAGGAGG AGGAGTCAAG 1860
   GGAGCAGGGC AGCTCTACCA GGCAAGGTGT TTCCCCAGCA TAGGCGCAGA CAGTTGGGAC 1920
   GAAACTTCAG AGCCCAGGCA GTCCCTGAAT GACCAGGCCA GTGTTGTCAC TGAGTGGTCC 1980
   CCTGCTGGTT GGGAGTGAAG AGAATCCAGG CTGGCAGAGC TGGAGCCAGT TGGGGAGCAC 2040
   GGTTCTGGGA GCTCTGCAAA ATCAGTAGCA AGTGCTGGAA AAGGCACATG CCGAAGATAC 2100
   TCAAGAGCTC CCAAGATTTG CTTGAGGCTA GCCCAGTGAA RAAAACCAGA GACTCATGTT 2160
   TCCAGGGGTC AGTCTGTCAG GCAGGAAGGA CCCAGGATTT GAACCCAGCT TCAGTGTGCA 2220
   GGCTCTGAGG CTGCCCAGGA CGGGAAAGTC CAAGGAAGGG GCCTGGTGGT GCTCCACTTG 2280
   CAGTTCTTTA AAGAATGCTG CTTTTTATTC TCCTAACCCT TTCAAGTGGG TGCAGACTTC 2340
   TCGTTAGCAG CTGGAAGACA TTCCTCCCAC ACTTTTCCCT TCCTGGCCCA AGAGAGCATC 2400
   CAGAAGGCAG TAGGACCTGG TTTTTCAGGT ACTGGAGCCC GGGGGCTCAC TGCTTGCACT 2460
   GTGCTTAGGG TAGGGATGGT AAATATCCTC CCTGCATGGC TTTATCCTCC CTCTCATCCC 2520
   AAAGCAGGTA TCTTCTGGTT GTCACAGAGT TTCATTGAGT CCAGCTGCAG CCACGTGGCC 2580
   ATCTGGAGCT GGTGCTATAG GTGACCATCT GGTACATTGA GGGGACCTGT TTGCCTCCTC 2640
   CACTCTATAA GCAGTCATCT TGGGAGACCG GGAGGAGAAG GTGGTGGGCT AGTCCTGTGT 2700
   CCTCCTCCAC TTCCCATGCC TCTATGTTAC CCATCTGTGT CTCCTGTGCA GAAGGAGAGG 2760
   AAGGGGCATT AAGAGATGAA GGGTGATTAT GTATTACTTA TCCATTTCTG AATAAACATT 2820
   TGTTATTCCT AAAAAAAAAA AAAAAAAACT CGAGGGGGGG CCCGGWACCC AWATCGCCSK 2880
   AAAGTGAG 2888

(2) INFORMATION FOR SEQ ID NO: 62:

(i) SEQUENCE CHARACTERISTICS:
(A) LENGTH: 1851 base pairs
(B) TYPE: nucleic acid
(C) STRANDEDNESS: double
(D) TOPOLOGY: linear

(xi) SEQUENCE DESCRIPTION: SEQ ID NO: 62:

CACTAGTATA ATTTATAATT ATAACCTATT CTGATTTCTT TTCAAATATT AGGTGTCCTA 60

GTTGCCTATG AAGGTTTGCC ACTTCATCTT GCACTGTTCC CCAAACTTTG GACTGAGCTA 120

TGCCAGACTC AGTCTGCTAT GTCAAAAAAC TGCATCAAGC TTTTGTGTGA AGATCCTGTT 180

TTCGCAGAAT ATATTAAATG TATCCTAATG GATGAAAGAA CTTTTTTAAA CAACAACATT 240

GTCTACACGT TCATGACACA TTTCCTTCTA AAGGTTCAAA GTCAAGTGTT TTCTGAAGCA 300

AACTGTGCCA ATTTGATCAG CACTCTTATT ACAAACTTGA TAAGCCAGTA TCAGAACCTA 360

CAGTCTGATT TCTCCAACCG AGTTGAAATT TCCAAAGCAA GTGCTTCTTT AAATGGGGAC 420

CTGAGGGCAC TCGCTTTGCT CCTGTCAGTA CACACTCCCA AACAGTTAAA CCCAGCTCTA 480

ATTCCAACTC TGCAAGAGCT TTTAAGCAAA TGCAGGACTT GTCTGCAACA GAGAAACTCA 540

CTCCAAGAGC AAGAAGCCAA AGAAAGAAAA ACTAAAGATG ATGAAGGAGC AACTCCCATT 600

AAAAGGCGGC GTGTTAGCAG TGATGAGGAG CACACTGTAG ACAGCTGCAT CAGTGACATG 660

AAAACAGAAA CCAGGGAGGT CCTGACCCCA ACGAGCACTT CTGACAATGA GACCAGAGAC 720

TCCTCAATTA TTGATCCAGG AACTGAGCAA GATCTTCCTT CCCCTGAAAA TAGTTCTGTT 780

AAAGAATACC GAATGGAAGT TCCATCTTCG TTTTCAGAAG ACATGTCAAA TATCAGGTCA 840

CAGCATGCAG AAGAACAGTC CAACAATGGT AGATATGACG ATTGTAAAGA ATTTAAAGAC 900

CTCCACTGTT CCAAGGATTC TACCCTAGCC GAGGAAGAAT CTGAGTTCCC TTCTACTTCT 960

ATCTCTGCAG TTCTGTCTGA CTTAGCTGAC TTGAGAAGCT GTGATGGCCA AGCTTTGCCC 1020

TCCCAGGACC CTGAGGTTGC TTTATCTCTC AGTTGTGGCC ATTCCAGAGG ACTCTTTAGT 1080

CATATGCAGC AACATGACAT TTTAGATACC CTGTGTAGGA CCATTGAATC TACAATCCAT 1140

GTCGTCACAA GGATATCTGG CAAAGGAAAC CAAGCTGCTT CTTGACATTA GGTGTAGCAT 1200

GTCTACTTTT AAGTCCCTCA CCCCCAACCC CCATGCTGTT TGTATAAGTT TTGCTTATTT 1260

GTTTTTGTGC TTCAGTTTGT CCAGTGCTCT CTGCTTGAAT GGCAAGATAG ATTTATAGGC 1320

TTAATTCTTG GTCAGGCAGA ACTCCAGATG AAAAAAACTT GCATCTTCAG TATACTTCCT 1380

AAAGGGCAAT CAGATAATGG ATATGTTTTA TGTAATTAAG AGTTCACTTT AGTGGCTTTC 1440

ATTTAATATG GCTGTCTGGG AAGAACAGGG TTGCCTAGCC CTGTACAATG TAATTTAAAC 1500

TTACAGCATT TTTACTGTGT ATGATATGGT GTCCTCTGTG CCAGTTTTGT ACCTTATAGA 1560

GGCAGATTGC CTCCGATCGC TGTGGTTCTT ATTATCAAAA TTAAGTTTAC TTGTATACGG 1620

AACAACCACA AGAAATTTGA TTCTGTAAAG AATCCTCTTT AGCTGTGGCC TGGCAGTATA 1680

TAAATGGTGC TTTATTTAAC AGAATACCTG TGGAGGAAAT AAAGCACACT TGATGTAAAA 1740

ATAATTGTTT TATTTTTATT GACATGACTG ATTGATTGCT ATTCTGTGCA CTTAATTAAA 1800

CTGATTGTGA TGACTTWWAA AAAAAAAAAA AAAAAAAAAA AAAAAAAAAA A 1851

(2) INFORMATION FOR SEQ ID NO: 63:

(i) SEQUENCE CHARACTERISTICS:
(A) LENGTH: 3542 base pairs
(B) TYPE: nucleic acid
(C) STRANDEDNESS: double
(D) TOPOLOGY: linear

(xi) SEQUENCE DESCRIPTION: SEQ ID NO: 63:

TCCAATGCTG ATGAGCGTCT TCGCTGGCAG GCCAGCTCCT TGCCTGCTGA TGACCTTTGC 60

ACAGAAAATG CCATCATGCT GAAACGATTC AATAGGTATC CGCTGATCAT TGACCCCTCT 120

GGACAGGCCA CAGAATTCAT TATGAATGAA TATAAGGWTC GTAAGATCAC ACGGACCAGC 180

TTCCTGGATG ACGCCTTCAG AAAGAACTTA GAGAGTGCAC TGAGATTCGG TAACCCCCTT 240

CTGGTCCAGG ATGTGGAAAG CTACGATCCA GTTTTGAACC CGGTGCTGAA CCGTGAAGTG 300

CGGCGAACAG GGGGGAGAGT GCTGATCACT CTCGGGGACC AGGACATAGA CCTGTCGCCA 360

TCGTTTGTCA TCTTCCTGTC CACCCGGGAT CCAACTGTCG AGTTCCCACC AGATCTCTGT 420

TCCCGGGTTA CTTTTGTAAA CTTCACAGTT ACCCGTAGCA GTTTACAAAG CCAGTGTCTA 480

AATGAAGTAC TTAAAGCAGA AAGACCTGAT GTGGACGAGA AACGATCTGA TCTTCTTAAA 540

CTTCAAGGGG AATTTCAGCT CCGTTTGCGT CAGCTGGAAA AATCTCTACT ACAAGCTCTG 600

AACGAGGTGA AAGGGCGCAT TTTGGATGAC GACACGATCA TAACCACTCT GGAGAACCTG 660

AAGAGAGAGG CTGCAGAGGT CACCAGGAAA GTTGAGGAGA CGGACATTGT CATGCAGGAG 720

GTGGAGACCG TGTCCCAGCA GTACCTCCCG CTCTCCACCG CCTGCAGCAG CATCTACTTC 780

ACCATGGAGT CCCTCAAGCA GATACACTTC TTGTACCAGT ACTCCCTCCA GTTTTTCCTG 840

GACATTTATC ACAACGTCCT ATACGAGAAC CCGAACCTGA AGGGTGTCAC CGACCACACA 900

CAGCGCCTGT CCATTATAAC AAAGGACCTC TTCCAGGTGG CGTTTAACCG AGTGGCTCGA 960

GGCATGCTGC ATCAGGACCA CATTACCTTT GCCATGCTGC TGGCAAGAAT CAAACTGAAG 1020

GGCACCGTGG GGGAGCCCAC CTACGATGCA GAATTCCAGC ACTTCTTGAG AGGAAATGAG 1080

ATTGTCCTGA GTGCTGGCTC CACCCCCAGG ATCCAGGGCC TGACTGTGGA GCAGGCGGAG 1140

GCGGTGGTGA GGCTGAGCTG CCTTCCCGCG TTTAAGGACT TGATTGCAAA GGTTCAGGCA 1200

GACGAGCAAT TTGGCATCTG GCTGGACAGC AGCTCCCCGG AGCAGACTGT GCCCTACCTC 1260

TGGAGTGAAG AAACACCTGC AACACCCATT GGCCAGGCCA TCCACCGCCT GCTCCTGATC 1320

CAGGCTTTCC GGCCCGATCG CCTGTTGGCC ATGGCCCACA TGTTTGTTTC AACAAACCTT 1380

GGGGAGTCTT TCATGTCCAT CATGGAGCAG CCGCTCGACC TGACCCACAT TGTGGSCACA 1440

GAGGTGAAGC CCAACACTCC TGTCTTAATG TGCTCTGTGC CTGGTTATGA TGCCAGTGGA 1500

CATGTCGAGG ACCTTGCAGC CGAGCAGAAC ACGCAGATCA CTTCAATTGC AATCGGCTCT 1560

GCAGAAGGCT TTAACCAAGC AGATAAGGCA ATAAACACCG CTGTAAAGTC GGGCAGGTGG 1620

GTGATGCTGA AGAATGTGCA TCTGGCCCCA GGGTGGCTGA TGCAGCTGGA GAAGAAGTTG 1680

CATTCCCTGC AGCCGCATGC CTGCTTCCGA CTCTTCCTCA CCATGGAGAT CAACCCCAAG 1740

GTGCCTGTGA ATCTGCTCCG TGCGGGCCGC ATCTTTGTGT TCGAGCCACC GCCAGGGKTG 1800

AAGGCCAACA TGCTGAGGAC GTTCAGCAGC ATTCCCGTCT CACGGATATG CAAGTCTCCC 1860

AACGAGCGTG CCCGCTTGTA CTTCCTGCTG GCCTGGTTTC ATGCGATCAT CCAAGAACGC 1920

TTACGATACG CACCACTGGG GTGGTCAAAG AAGTATGAAT TTGGAGAGTC TGACCTGCGG 1980

TCANYTTGCG ATACGGTGGA CACGTGGCTG GATGACACGG CCAAGGGCAG GCAGAACATC 2040

TCACCGGATA AGATCCCGTG GTCTGCACTA AAGACCTTAA TGGCCCAGTC CATTTATGGC 2100

GGGCGCGTGG ACAACGAGTT TGACCAGCGT CTGCTCAACA CCTTCCTGGA GCGCCTGTTC 2160

ACAACCAGGA GTTTCGACAG TGAGTTTAAG CTGGCATGCA AGGTCGACGG ACATAAAGAC 2220

ATTCAAATGC CAGATGGCAT GCAGGCGAGA GGAGTTTGTG CAGTGGGTGG AGTTGCTCCC 2280

CGACACCCAG ACGCCCTCCT GGCTGGGCCT GCCCAACAAC GCCGAGAGAG TCCTCCTTAC 2340

CACACAGGGT GTGGACATGA TCAGTAAAAT GCTGAAGATG CAGATGTTGG AGGATGAGGA 2400

CGACCTGGCC TACGCAGAGA CTGAGAAGAA GACGAGGACA GACTCCACGT CCGACGGGCG 2460

CCCTGCCTGG ATGCGGACAC TGCACACCAC CGCGTCCAAC TGGCTGCACC TCATCCCCCA 2520

GACGCTGAGC CACCTCAAGC GCACCGTGGA GAATATCAAG GATCCTTTGT TCAGGTTCTT 2580

TGAGAGAGAA GTGAAGATGG GCGCAAAGCT GCTTCAGGAC GTTCGCCAGG ACCTTGCAGA 2640

TGTCGTCCAG GTGTGCGAAG GAAAGAAGAA GCAGACCAAC TACTTGCGCA CGCTGATCAA 2700

CGAGCTAGTG AAAGGGATCT TGCCTCGGAG CTGGTCCCAC TACACGGTGC CTGCCGGCAT 2760

GACCGTCATC CAGTGGGTGT CCGACTTCAG CGAGAGGATC AAACAGCTGC AGAACATCTC 2820

ACTGGCAGCT GCATCTGGTG GCGCCAAGGA GCTAAAGAAC ATCCACGTGT GCCTGGGTGG 2880

CCTGTTCGTG CCTGAGGCGT ACATCACTGC CACCAGGCAG TATGTGGCCC AGGCCAACAG 2940

CTGGTCCCTG GAGGAGCTCT GCCTGGAAGT CAACGTCACC ACCTCACAGG GCGCCACCCT 3000

TGACGCTTGC AGCTTCGGAG TCACGGGTTT GAAACTTCAA GGGGCCACGT GCAACAACAA 3060

CAAGCTGTCA CTGTCCAATG CCATCTCAAC CGCCCTTCCC CTGACGCAGC TGCGCTGGGT 3120

CAAGCAGACA AACACCGAGA AGAAGGCCAG TGTGGTAACC TTACCTGTCT ACCTGAACTT 3180

CACCCGTGCA GACCTCATCT TCACCGTGGA CTTCGAAATT GCTACAAAGG AGGATCCTCG 3240

CAGCTTCTAC GAGCGGGGTG TCGCAGTCTT GTGCACAGAG TAAACTTTTC TAGCTGCCCC 3300

TTTCTGTAAT AGTGAAAGTT GGTATTTAAC ATTTATTCAT TTTTAAAATA TTTGGAAGGT 3360

CTGAGCTTGT GAAAAGAAAG TGGTTGGTCT GAGGTTGGAG GAAGCTGAAT GGAATCTGAC 3420

GGTTGGGAGT GGTGGAAATT GGAAGGATAC CAGGAGGTAT TTGGGAAGGC CAATGGCGTG 3480

GCTCCTTTGA GGAAATAAAA CACTAAGCAT GAAAAAAAAA AAAAAACTTA CAANCCNCAA 3540

GG 3542

(2) INFORMATION FOR SEQ ID NO: 64:

(i) SEQUENCE CHARACTERISTICS:
(A) LENGTH: 883 base pairs
(B) TYPE: nucleic acid
(C) STRANDEDNESS: double
(D) TOPOLOGY: linear

(xi) SEQUENCE DESCRIPTION: SEQ ID NO: 64:

AGGTGATTTT AATGATAGGT GTCATATATA GGACGGATAA TCTGTTTACA TTCTGTTCTT 60

CTCGATGCAC TCACAAGCGG GTAACTAGGT GACAAGAAAA CAAAGATCTT ATTCAAAAGA 120

GGTCTTACAG CAACCCAACG TCTCATCTTC CCATAGTAAA GATGACGGCG CCTTGAGGTA 180

AGCTACAGGC AACACCACTT CCGCGTTTCT CTTGCGCCCT GGTCCAAGAT GGCGGATGAA 240

GCCACGCGAC GTGTTGTGTC TGAGATCCCG GTGCTGAAGA CTAACGCCGG ACCCCGAGAT 300

CGTGAGTTGT GGGTGCAGCG ACTGAAGGAG GAATATCAGT CCCTTATCCG GTATGTGGAG 360

AACAACAAGA ATGCTGACAA CGATTGGTTC CGACTGGAGT CCAACAAGGA AGGAACTCGG 420

TGGTTTGGAA AATGCTGGTA TATCCATGAC CTCCTGAAAT ATGAGTTTGA CATCGAGTTT 480

GACATTCCTA TCACATATCC TACTACTGCC CCAGAAATTG CAGTTCCTGA GCTGGATGGA 540

AAGACAGCAA AGATGTACAG GGGTGGCAAA ATATGCCTGA CGGATCATTT CAAACCTTTG 600

TGGGGCCAGG AATGTGCCCA AATTTGGACT AGCTCATCTC ATGGCTCTGG GGCTGGGTCC 660

ATGGSTGGCA GTGGAAATCC CTGATCTGAT TCAGAAGGGC GTCATCCAAC ACAAAGAGAA 720

ATGCAACCAA TGAAGAATCA AGCCACTGAG GCAGGGCAGA GGGACCTTTG ATAGGCTACG 780

ATACTAWTTT CCTGTGCATC ACACTTAACT CATCTAACTG TTCCCCGGAC ANCCTCCACT 840

CTAGTTGTTA CTAAGTANTG CAGTAGCATT NTGGGGAAGA ACA 883

(2) INFORMATION FOR SEQ ID NO: 65:

(i) SEQUENCE CHARACTERISTICS:
(A) LENGTH: 1541 base pairs
(B) TYPE: nucleic acid
(C) STRANDEDNESS: double
(D) TOPOLOGY: linear

(xi) SEQUENCE DESCRIPTION: SEQ ID NO: 65:

GGCACGAGGT GGCCTCTACC CTGGGCTCAT CTGGCTACAC AGGGACTCTA AACGCTTCCA 60

GATTCCCTGG AAACATGCCA CCCGGCATAG CCCTCAACAA GAAGAGGAAA ATACCATTTT 120

TAAGGCCTGG GCTGTAGAGA CAGGGAAGTA CCAGGAAGGG GTGGATGACC CTGACCCAGC 180

TAAATGGAAG GCCCAGCTGC GCTGTGCTCT CAATAAGAGC AGAGAATTCA ACCTGATGTA 240

TGATGGCACC AAGGAGGTGC CCATGAACCC AGTGAAGATA TATCAAGTGT GTGACATCCC 300

TCAGCCCCAG GGCTCGATCA TTAACCCAGG ATCCACAGGG TCTGCTCCCT GGGATGAGAA 360

GGATAATGAT GTGGATGAAG AAGATGAGGA AGATGAGCTG GATCAGTCGC AGCACCATGT 420

TCCCATCCAG GACACCTTCC CCTTCCTGAA CATCAATGGT TCTCCCATGG CGCCAGCCAG 480

TGTGGGCAAT TGCAGTGTGG GCAACTGCAG CCCGGAGGCA GTGTGGCCCA AAACTGAACC 540

CCTGGAGATG GAAGTACCCC AGGCACCTAT ACAGCCCTTC TATAGCTCTC CAGAACTGTG 600

GATCAGCTCT CTCCCAATGA CTGACCTGGA CATCAAGTTT CAGTACCGTG GGAAGGAGTA 660

CGGGCAGACC ATGACCGTGA GCAACCCTCA GGGCTGCCGA CTCTTCTATG GGGACCTGGG 720

TCCCATGCCT GACCAGGAGG AGCTCTTTGG TCCCGTCAGN CTGGAGCAGG TCAAATTCCC 780

AGGTCCTGAG CATATTACCA ATGAGAAGCA GAAGCTGTTC ACTAGCAAGC TGCTGGACGT 840

CATGGACAGA GGACTGATCC TGGAGGTCAG CGGTCATGCC ATTTATGCCA TCAGGCTGTG 900

CCAGTGCAAG GTGTACTGGT CTGGGCCATG TGCCCCATCA CTTGTTGCTC CCAACCTGAT 960

TGAGAGACAA AAGAAGGTCA AGCTATTTTG TCTGGAAACA TTCCTTAGCG ATCTCATTGC 1020

CCACCAGAAA GGACAGATAG AGAAGCAGCC ACCGTTTGAG ATCTACTTAT GCTTTGGGGA 1080

AGAATGGCCA GATGGGAAAC CATTGGAAAG GAAACTCATC TTGGTTCAGG TCATTCCAGT 1140

AGTGGCTCGG ATGATCTACG AGATGTTTTC TGGTGATTTC ACACGATCCT TTGATAGTGG 1200

CAGTGTCCGC CTGCAGATCT CAACCCCAGA CATCAAGGAT AACATCGTTG CTCAGCTGAA 1260

GCAGCTGTAC CGCATCCTTC AAACCCAGGA GAGCTGGCAG CCCATGCAGC CCACCCCCAG 1320

CATGCAACTG CCCCCTGCCC TGCCTCCCCA GTAATTGTGA ATGCCATCTT CTTCCTTCTC 1380

TTTTTTATAA TATTGTACAT ATGGATTTTT TTATTGTTTA GATTTAACCA GCTTTTAAAT 1440

CTCTGTTTTC TGTGACAGTG TTAGAAGTTT GTGATTCTCC AAATATGCCT AGATTTAAAG 1500

CTGATTTAAT TTATGGAAAA AAAAAAAAAA AAAAAAAAAA A 1541

(2) INFORMATION FOR SEQ ID NO: 66:

(i) SEQUENCE CHARACTERISTICS:
(A) LENGTH: 732 base pairs
(B) TYPE: nucleic acid
(C) STRANDEDNESS: double
(D) TOPOLOGY: linear

(xi) SEQUENCE DESCRIPTION: SEQ ID NO: 66:

AGAAAATGAA TGTTAGAAGG TGCCTGCCGA GGCGGGACAG AGTGTTTGCT CGCGCTGGAG 60

AAGGCTCTGC TCAGCCCTGA GAGTCCCTTC CTGCCCCACC GATACTGGCA CTTTAAAAAG 120

GAAGCTGACC GCACAGTGTC CAGACGAATT GGCCCCCAGA AGATGGGGAG TTCTGTCCTG 180

CCCTTCTGTG TCTGCGTGAC CTCACCCAGC CTAGGAGGGA GGTGCATTCA GGGTAGATTT 240

GCCTCTCATT CAAAGTTCTG GGGCTTTGGG CGGAAAACAG CCAGCTTTGG CGCTGTTGGG 300

GAGACTCCTC CAGACCAGGA ACCCCAGAAG GAGACAGAGC CTGCCACATC CTCCCACGCC 360

AGGCCCTGGG CCAGGGTGAT TGGACTGAGA ATTTGGCCAC AACCAAATTG ATGCTGGCTG 420

GAACCAGAGG CCAGAAAGCC TGGCCTTGTC CCCATGTGGG AGCCCTGTCC TCAGCCCTCT 480

TGTCCCCTTG AGCTCAGTGA ATTCCCACCA GGTGCCCACA GCTCCTGGAC TTCAAATTCT 540

ATATATTGAG AGAGTTGGAG AGTATATCAG AGATATTTTT GGAAAGGAGT TGGTCTATGC 600

AATGTCAGTT TGGAATCTTC TTGAAAGTTT AATGTTTTTA TTAGGAGATT TAAAGAAAAT 660

AAAGGTCTAC AATATCAAAA AAAAAAAAAA AAAAAAAAAA AAAAAAAAAA AAAAAAAAAA 720

AAAAAAAAAA AA 732

(2) INFORMATION FOR SEQ ID NO: 67:

(i) SEQUENCE CHARACTERISTICS:
(A) LENGTH: 629 base pairs
(B) TYPE: nucleic acid
(C) STRANDEDNESS: double
(D) TOPOLOGY: linear

(xi) SEQUENCE DESCRIPTION: SEQ ID NO: 67:

TTAAGGAATT CGGCMCGATC CCGGCAAGTA ACATGACTAA AAAGAAGCGG GAGAATCTGG 60

GCGTCGCTCT AGAGATCGAT GGGCTAGAGG AGAAGCTGTC CCAGTGTCGG AGAGACCTGG 120

AGGCCGTGAA CTCCAGACTC CACAGCCGGG AGCTGAGCCC AGAGGCCAGG AGGTCCCTGG 180

AGAAGGAGAA AAACAGCCTA ATGAACAAAG CCTCCAACTA CGAGAAGGAA CTGAAGTTTC 240

TTCGGCAAGA GAACCGGAAG AACATGCTGC TCTCTGTGGC CATCTTTATC CTCCTGACGC 300

TCGTCTATGC CTACTGGACC ATGTGAGCCT GGCACTTCCC CACAACCAGC ACAGGCTTCC 360

ACTTGGCCCC TTGGTCAGGA TCAAGCAGGC ACTTCAAGCC TCAATAGGAC CAAGGTGCTG 420

GGGTGTTCCC CTCCCAACCT AGTGTTCAAG CATGGCTTCC TGGCGGCCCA GGCCTTGCCT 480

CCCTGGCCTG CTGGGGGGTT CCGGGTCTCC AGAAGGACAT GGTGCTGGTC CCTCCCTTAG 540

CCCAAGGGAG AGGCAATAAA GAACACAAAG CTGAAAAAAA AAAAAAAAAA AACTCGTAGG 600

GGGGGCCCGT ACCCAATCGC CCTNTCGTG 629

(2) INFORMATION FOR SEQ ID NO: 68:

(i) SEQUENCE CHARACTERISTICS:
(A) LENGTH: 1751 base pairs
(B) TYPE: nucleic acid
(C) STRANDEDNESS: double
(D) TOPOLOGY: linear

(xi) SEQUENCE DESCRIPTION: SEQ ID NO: 68:

CTGCTAGCCG GCCGGCGCAG GCTGCCGAGC GGGTGAGCGC GCAGGCCAGG CCAAAGCCCT 60

GGTACCCGCG CGGTGCGGGC CTCAGTCTGC GGCCATGGGG GCGTCCGCGC GGCTGCTGCG 120

AGCGGTGATC ATGGGGGCCC CGGGCTCGGG CAAGGGCACC GTGTCGTCGC GCATCACTAC 180

ACACTTCGAG CTGAAGCACC TCTCCAGCGG GGACCTGCTC CGGGACAACA TGCTGCGGGG 240

CACAGAAATT GGCGTGTTAG CCAAGGCTTT CATTGACCAA GGGAAACTCA TCCCAGATGA 300

TGTCATGACT CGGCTGGCCC TTCATGAGCT GAAAAATCTC ACCCAGTATA GCTGGCTGTT 360

GGATGGTTTT CCAAGGACAC TTCCACAGGC AGAAGCCCTA GATAGAGCTT ATCAGATCGA 420

CACAGTGATT AACCTGAATG TGCCCTTTGA GGTCATTAAA CAACGCCTTA CTGCTCGCTG 480

GATTCATCCC GCCAGTGGCC GAGTCTATAA CATTGAATTC AACCCTCCCA AAACTGTGGG 540

CATTGATGAC CTGACTGGGG AGCCTCTCAT TCAGCGTGAG GATGATAAAC CAGAGACGGT 600

TATCAAGAGA CTAAAGGCTT ATGAAGACCA AACAAAGCCA GTCCTGGAAT ATTACCAGAA 660

AAAAGGGGTG CTGGAAACAT TCTCCGGAAC AGAAACCAAC AAGATTTGGC CCTATGTATA 720

TGCTTTCCTA CAAACTAAAG TTCCACAAAG AAGCCAGAAA GCTTCAGTTA CTCCATGAGG 780

AGAAATGTGT GTAACTATTA ATAGTAAGAT GGGCAAACCT CCTAGTCCTT GCATTTAGAA 840

GCTGCTTTTC CTAAGACTTC TAGTATGTAT GAATTCTTTG AAAATTATAT TACTTTTATT 900

TCTACTGATT TTATTTTGGA TACTAAGGAT GTGCCAAATG ATTCGGATAC TAAGATGCAT 960

CGTTTGAAAT CATCTAGTGT GTTGTATGCA GTTATCCTCA AAAACATCAG CGATGTCTGA 1020

ACCTTTAAAA CATCTGTTAG AGCAAAATTA AAAGAGCATT TGGTAGTAAT CTAACTTTTT 1080

GTTCAGTTAA TAAGTGGTTG ATAAAGTTTC CATATTTTTC TGGAAAAGTT AAAAAAAGTT 1140

ACATGTCATT TGGAGAAAAT ACGTAATCAG AAATTTGTGC ATAGATTGAT GCCAAAAAAG 1200

ACATTTCCAG CATTGTGGAA CATGGTGAGA CACTATATAA AATTCCAGAA AGAAAGCAAC 1260

TGGATTTACA GATTTATTGT GAGACACAAA TTCACTGCTG CCTTTACACT AAGAAATGTA 1320

TATGTTAACC ATATATGCTG TATTTATTTT GTCGTTAAGC ATACTTTCAG TTTACTCAGA 1380

ATTTTCAATT TGCTATAAAG ATGTATCAAT TAGCATATAG AAAAATATTA CTTTAAGATG 1440

ACTTGTTTCC TTTGAAAATA CCTGTGTACT GAGGGTTATG ATTTGTGTCA AAAATTGACA 1500

TAAGTGCTTT TACAAGCACC AAAGTTGAAT GAATTTTCAA CAAAATGTAA TTAAAGTCTA 1560

TGTTTTCAGT TATGACTCAG GTTAAGAAAT GTGTTTTAGG ATCTACTTGC TGGTTTTTCT 1620

TTTTGATCCA AATGTGTGAT CTGCCCTGAT AAATAACAAG TTATNGTACC ATCTCCCCCG 1680

CCAATAAAAA AAAAAAAAAA AAAAAAAAAC TCGAGGGGGG GCCCGGTACC CAATTCTCCG 1740

NAATAGGNAG T 1751

(2) INFORMATION FOR SEQ ID NO: 69:

(i) SEQUENCE CHARACTERISTICS:
(A) LENGTH: 508 base pairs
(B) TYPE: nucleic acid
(C) STRANDEDNESS: double
(D) TOPOLOGY: linear

(xi) SEQUENCE DESCRIPTION: SEQ ID NO: 69:

GGCACGAGAT TATGTATTAA AATGTTTTTG AATTGTGAAA TATTAGAATA TTGTTACTAT 60

TTGACCCAAC TCAAAATCTC CATGGGAAAA TACCTGTCGA TACCCACAGT ATTGTTGAAA 120

ATAATCAGAT GCAGTATCAC AGCTGTGTCA GACTCTAGTA CCAGTTGGGC AATCAAGGCA 180

CAGCTAAAAA TTGAAAACAA AGATCTGGAC AACAAAACAG CCAAAGGTGG GGGTCAAGAA 240

GCTCTGACGT GTACCTAGCT GTAGAATGCT ATGCACACGT GCCAGGTGTA GTGTGCATAT 300

CCAGGAAAAA CTGCAGAGAG CCCCAGTCTT CACCTCTGGT TGACCATGAG CTCTGTGTAA 360

GCAGGAAGTG AAGGCTAAGG CAGATTTAAG CTCTGAAAGC ATTCCACAAC ATACACACAA 420

ATCGTGCAAA GCATTAAGGA AATCTTGTTA CTGCTAAGTG TTGCTGACCC AGGAACAACT 480

CCTACTCAGC TGGACTTAAA AATAAAAA 508

(2) INFORMATION FOR SEQ ID NO: 70:

(i) SEQUENCE CHARACTERISTICS:
(A) LENGTH: 245 base pairs
(B) TYPE: nucleic acid
(C) STRANDEDNESS: double
(D) TOPOLOGY: linear

(xi) SEQUENCE DESCRIPTION: SEQ ID NO: 70:

TACATAGAGC AAAGAGAAAT TTCCAGAATT TCTARAATTC TGGAAAGAGA ATTTTCCTGA 60

GATTGCAGAT TTGCTTGTGT CCTCAGGTGA TGATGAGGGC TGTTTTCCCC TGTTGTCCTT 120

TCCTCACACT CATGCTTCCT CTCCTAGAGT GTCTGGTTGG CATGATCATG TGCTACCTAG 180

GCATTTCTTT CACTGATACA AGGAAAACTG CAGGGTTAAA AAAAAAAAAA AAAAAAAAAA 240

NCNCG 245

(2) INFORMATION FOR SEQ ID NO: 71:

(i) SEQUENCE CHARACTERISTICS:
(A) LENGTH: 361 base pairs
(B) TYPE: nucleic acid
(C) STRANDEDNESS: double
(D) TOPOLOGY: linear

(xi) SEQUENCE DESCRIPTION: SEQ ID NO: 71:

ATGTTCCTCA TGAGGATGCA CTTGTGCTTC TGCAAGTATT GCTGCAGCTT CATAGTGACT 60

CCCACCAGCA CCAGCAATAC AGCTAGCTAC CTGTGGCCTT GGATCTCAGC CAGCATGGCT 120

GGGAGAGGGA GCAGCTGGGC ATGTACCCTA AATGCTGTTA CCAGGGAAGG ACTCCCAGAG 180

TGAAGACAAG TAGGGACTTC CTGCAGAGGT GGTACATGTG CTCTCTGTAT CCATACTTTT 240

TTTTTTTTTT TTTTGAGATA GAGTTTCACC CTTGTTGCCC TGGCTGGAGT GCAATGGTGC 300

GATCTCAGCT CACTGCAACC TCTCTGCCTC CCGGGTTCAA GTGATTCTCC TGCCTCAGCC 360

T 361

(2) INFORMATION FOR SEQ ID NO: 72:

(i) SEQUENCE CHARACTERISTICS:
(A) LENGTH: 713 base pairs
(B) TYPE: nucleic acid
(C) STRANDEDNESS: double
(D) TOPOLOGY: linear

(xi) SEQUENCE DESCRIPTION: SEQ ID NO: 72:

AGGATCACAC AATAGAGAAC ACTGTAGTAA CATTTCGGTC TGCTCACAAG ACCCAGAACA 60

TTGATCAGTT TTTGTTGTTG GTTTATTATT TTTCTGTTAA AAAATTGTGA AAAGTTTGTT 120

TTAGCTAGAT GATATTTTAA TAGCTGCGAG TGCTTTGGAA CTATAAAGAT GTCACTACTT 180

AACACACATA CCTTATGTTT TGTTTTGTTT TGTTTTACAC TCAGTATAAA TCAGGAGAAG 240

TTAGCCAACC ATCTAGCATT TAGAATCCTC TTTTTTATTG TCTTCTAAGG ATATGGATGT 300

TCCCATAACA GCAACAAAAC AGCAACAAAA ACATTTCATA AATATCACTT GATAGACTGT 360

AAGCACCTGC TTAACTTTGT GTCCCAAATA TTTAGTGTGT ATATATATAT ATATATATAC 420

ACACACACAC ACATATATAT TCAACAAATA AAGCAAAATA TAACATGCAT TTCACATTTT 480

GTCTTTCCCT GTTACGATTT TAATAGCAGA ACTGTATGAC AAGTTTAGGT GATCCTAGCA 540

TATGTTAAAT TCAAATTAAT GTAAAACAGA TTAACAACAA CAAAGAAACT GTCTATTTGA 600

GTGAAGTCAT GCTTTCTATT ATAATAACTT GGCTTCGGTT ATCCATCAAA TGCACACTTA 660

TACTGTTATC TGATTGTTTA TAATAAAGAA TACTGTACTT ATAAAAAAAA AAA 713

(2) INFORMATION FOR SEQ ID NO: 73:

(i) SEQUENCE CHARACTERISTICS:
(A) LENGTH: 862 base pairs
(B) TYPE: nucleic acid
(C) STRANDEDNESS: double
(D) TOPOLOGY: linear

(xi) SEQUENCE DESCRIPTION: SEQ ID NO: 73:

GAAAGTCAGA GCTGTCCAAT CCCTCAGCAC CTTTTAGATT TGCTCCAAAT TAGAAACGTG 60

GGGACTATGT GTTCTGGGCA ATCACAGGTC TGGAAAATGG CTCTGCAGGC TCTTGATAGT 120

GAGACAGTGG TCATCTTACC AGACATGCAT CTGATTTTAA GCCTCAGGCT AATCCACAAT 180

GCTCGGCCAT GCCTATGATT AACAAACAAA AGCAAAATCT GCTTTTATAG TTTAGGAAAC 240

CTGGATAGAA CAGTATTTTT CAGCATTCTT GGATAAAGCA GTTCTGCATT TTTAAATTGG 300

GACTGCAGAA GTGACTGTCT ATAGTTGTGA AATACAAAAA ATGGTATGTT TGATCAGAAA 360

AGGAAGCCCG TGCCTGGCAC TTGGAAAGAT ACTGAGCATC ATAACCCTAA TGAGAAAATG 420

TAGGCTCTGT GAATGTTAAC TACAAATCAG GTTAGGAAAG CATATGACAC CCTTTGTCAA 480

ACTAAGCTTC ACTAGGAGGA CCTGTGCTCA TAGAAGAATA TGCTTTAAAA GTATCAATTT 540

TCCACAGTCG ATGATGGAGA AAAGTTCATT TGCACCAGAA TGCTGATAGT CACAATACAC 600

AGCCTGACAT ATATAACAAT ACAGTTTTCT GTAAACAGAA GTTCTTCCTC TTCCAATTCA 660

GGAGTCAGTC AGAGCATAAA TATTGCATGT TTCACTTTAG AAACTGATTC ATTTTAGAAA 720

GCAGATCTGG ATTATTTTGC AGGGTAGAAA TGAAGGCTAT TTCTGGCATT CTTGCTCAAA 780

AAGTCAATAT ATGTACATTA AGTATAAAAA AGGGTCTCTT TCACCTCTTT TGTTTCGTAG 840

CATTGGCTAC ATAACTCGTG CC 862

(2) INFORMATION FOR SEQ ID NO: 74:

(i) SEQUENCE CHARACTERISTICS:
(A) LENGTH: 4602 base pairs
(B) TYPE: nucleic acid
(C) STRANDEDNESS: double

(D) TOPOLOGY: linear

(xi) SEQUENCE DESCRIPTION: SEQ ID NO: 74:

GCGAGGGGGC GKGGGGAGCA GCGCCGARGC CGCCGCCTCC GCCTCCGCCG CCTAGGACTA 60

GGGGGTGGGG GACGGACAAG CCCCGATGCC GGGGGAKACG GAAGAGCCGA GACCCCCGGA 120

GCAGCAGGAC CAGGAAGGGG GAGAGGCGGC CAAGGCGGCT CCGGAGGACC CGCAACAACG 180

GCCCCCTGAG GCGGTCGCGG CGGCGCCTGC AGGGACCACT AGCAGCCGCG TGCTGAGGGG 240

AGGTCGGGAC CGAGGCCGGG CCGCTGCGRC CGCCGCGCMG CAGCTGTGTC CCGCCGGAGA 300

AGGCCGAGTA TCCCCGCCGG CGAGGAGCAG CCCCAGCGCC AGGCCTCCCG ACGTCCCCGG 360

GCAGCAGCCC AGGCCGCGAA GTCCCCGTCT CCAGTTCAGG GCAAGAAGAG TCCGCGACTC 420

CTATGCATAG AAAAAGTAAC AACTGATAAA GATCCCAAGG AAGAAAAAGA GGAAGAAGAC 480

GATTCTGCCC TCCCTCAGGA AGTTTCCATT GCTGCATCTA GACCTAGCCG GGGCTGGCGT 540

AGTAGTAGGA CATCTGTTTC TCGCCATCGT GATACAGAGA ACACCCGAAG CTCTCGGTCC 600

AAGACCGGTT CATTGCAGCT CATTTGCAAG TCAGAACCAA ATACAGACCA ACTTGATTAT 660

GATGTTGGAG AAGAGCATCA GTCTCCAGGT GGCATTAGTA GTGAAGAGGA AGAGGAGGAG 720

GAAGAAGAGA TGTTAATCAG TGAAGAGGAG ATACCATTCA AAGATGATCC AAGAGATGAG 780

ACCTACAAAC CCCACTTAGA AAGGGAAACC CCAAAGCCAC GGAGAAAATC AGGGAAGGTA 840

AAAGAAGAGA AGGAGAAGAA GGAAATTAAA GTGGAAGTAG AGGTGGAGGT GAAAGAAGAG 900

GAGAATGAAA TTAGAGAGGA TGAGGAACCT CCAAGGAAGA GAGGAAGAAG ACGAAAAGAT 960

GACAAAAGTC CACGTTTACC CAAAAGGAGA AAAAAGCCTC CAATCCAGTA TGTCCGTTGT 1020

GAGATGGAAG GATGTGGAAC TGTCCTTGCC CATCCTCGCT ATTTGCAGCA CCACATTAAA 1080

TACCAGCATT TGCTGAAGAA GAAATATGTA TGTCCCCATC CCTCCTGTGG ACGACTCTTC 1140

AGGCTTCAGA AGCAACTTCT GCGACATGCC AAACATCATA CAGATCAAAG GGATTATATC 1200

TGTGAATATT GTGCTCGGGC CTTCAAGAGT TCCCACAATC TGGCAGTGCA CCGGATGATT 1260

CACACTGGCG AGAAGCATTA CAATGTGAGA TCTGTGGATT TACTTGTCGA CAAAAGGCAT 1320

CTCTTAATTG GCACATGAAG AAACATGATG CAGACTCCTT CTACCAGTTT TCTTGCAATA 1380

TCTGTGGCAA AAAATTTGAG AAGAAGGACA GCGTAGTGGC ACACAAGGCA AAAAGCCACC 1440

CTGAGGTGCT GATTGCAGAA GCTCTGGCTG CCAATGCAGG CGCCCTCATC ACCAGCACAG 1500

ATATCTTGGG CACTAACCCA GAGTCCCTGA CGCAGCCTTC AGATGGTCAG GGTCTTCCTC 1560

TTCTTCCTGA GCCCTTGGGA AACTCAACCT CTGGAGAGTG CCTACTGTTA GAAGCTGAAG 1620

GGATGTCAAA GTCATACTGC AGTGGGACGG AACGGGTGAG CCTGATGGCT GATGGGAAGA 1680

TCTTTGTGGG AAGCGGCAGC AGTGGAGGCA CTGAAGGGCT GGTTATGAAC TCAGATATAC 1740

TCGGTGCTAC CACAGAGGTT CTGATTGAAG ATTCAGACTC TGCCGGACCT TAGTGGACAG 1800

GAAGACTTGG GGCATGGGAC AGCTCAGACT TTGTATTTAA AAGTTAAAAA GGACAAAAAA 1860

AAAATCTAAA GCATTTAAAA TCTAGTGAAA TAACTGAAGG GCCTGCTCTT TCCATTGTGG 1920

ATCACAGCAC ACACATACAT ACACCCTCCA CCTCCCCATC CCCTGTTCTC CCTCTGTTGC 1980

TCCCCTTATA AAATTGATGT TGTCTTTACC AGAAAGGTAG ACAAAAAAGA AGCAGCAGCA 2040

GCTCTTAAAG TGAGGGTTAT TCTCATACTC GGTTCCAGCC ATCAGCAGAC TTCCTGCTCA 2100

TCGGCAGATC CCCCTTTCCA ACCTGTAACT CTGATGTGCT CTGGATCAGC TTTTAACTTT 2160

TAATCATATA TTACTGTCTT CTAAATCCCT TCTCCTCCTC TACTGCTGCC CTATGGTTCT 2220

GGCTCCTACC CCCTGCGGCA CACTTATCTT CAAATACCAT AGAATTCTAA TCTCTGAAAT 2280

CATAGCTCTC CAGTGGCTTT TAAAGAAAGC TGGTCCTCAG CACTAACAAA ATCACTACAA 2340

TAGCCTAGTG CTTTTTTGGA AGCCTTTTTA GGGAAGAATG TTAGGTTCAT GGTAACTAGT 2400

ATGCTCTTTG AGATTTTTAC AGTGTTGAAA CTTAAGAATT TTGAGAGGGT GAGGAGGGTT 2460

GTTCAGAATC TAAATTACAG ATAGATGATT GTTTCTTGTG AATTTGTTTC TTTTCCTTTT 2520

TTTTTGTCCC TACCATTTCC TTACATTTCC CTTGGGGCCC ATCTCTGGCT CCTTGCTTTT 2580

TGTTTCTTGC TTTGCTTTAT CAGTTCATTC CAGCTCCCTG TTAGTGAAGG ACACTGCTGT 2640

TAGTGAAGGA ACAAAGTCTA TGAGTCCTAA AATTTTAAGT CAAAGAAAAC TGCTCTGTTT 2700

CCCCTTTAGT AACACTTCTG AAGAGGAAAA ACTTCAATAG CCAAAGTTAA TAATCCTATA 2760

TAATAATTGC TTTGGCTTTC ACCTAAAATT CTGGGCATCA CAATTTCCTT GGGATAGAGG 2820

TTGTGTTGGG GAATAGATTG CTTATTGCTG TTCACTGGAG AGAAAAGGTA GTGTTTTTGT 2880

ACAAGGTCAT ACCGCCAGAA GCCCCAAATC CTATTTTGGC TCATCTTCAG GTAAAGAGTA 2940

ATTCCTATCC TGTGTGCCTC AGAAGCTAGA ATCGAAGGCT TACCCTATTC ATTGTTTATT 3000

GTCAGAAATG CATGATGGCT CTTGGAAAGA ATGACGTTTT GCTGGAAAAA AAAAAAARAA 3060

CMGTTTGTGT TTCACAAACA TGGCTTATCA ATTTTTTCAA AGAATTCTTT TTTCCCAAAA 3120

AGAGGAGTAA CAAAATGTCA TTTCTGAAAG AGGCTTACTT TATACCAACT AGTGTCAGCA 3180

TTTGGGATGC CAGGGAACAG AGAGTGAGAC ACCTACAATC ACCAGTCTCA AATGCGCTAT 3240

TGTTTCTTTT CAGAGTGTTG CAGATTTGCC ATTTCTCCAT AATATGGGGA TAGAAAATGG 3300

AATAAAGATA GAAGGGATGT AGAATATGCT TTCCTGCCAA CATGGTTTGG AGTCGACTTT 3360

GGTATATTGA CTAGATTTGA AAATACAAGA TTGATTAGAT GAATCTACAA AAAAGTTGTC 3420

CTCCTCTCAG GTCCCTTTTA CACTTTTTGA CTAACTAGCA TCTATATTCC ACACTTAGCT 3480

TTTTTGTCAC ACTTATCCTT TGTCTCCGTA AATTTCATTT GCAGTGGTTA GTCATCAGAT 3540

ATTTTAGCCA CCTACACAAA AGCAAACTGC ATTTTTAAAA ATCTTTCTGA GATGGGAGAA 3600

AATGTATTCT CCTTTCCTAT ACCGCTCTCC CAACAAAAAA ACAACTAGTT AGTTCTACTA 3660

ATTAGAAACT TGCTGTACTT TTTCTTTTCT TTTAGGGGTC AAGGACCCTC TTTATAGCTA 3720

CCATTTGCCT ACAATAAATT ATTGCAGCAG TTTGCAATAC TAAAATATTT TTTATAGACT 3780

TTATATTTTT CCTTTTGATA AAGGGATGCT GCATAGTAGA GTTGGTGTAA TTAAACTATC 3840

TCAGCCGTTT CCCTGCTTTC CCTTCTGCTC CATATGCCTC ATTGTCCTTC CAGGGAGCTC 3900

TTTTAATCTT AAAGTTCTAC ATTTCATGCT CTTAGTCAAA TTCTGTTACC TTTTTAATAA 3960

CTCTTCCCAC TGCATATTTC CATCTTGAAT TGGTGGTTCT AAATTCTGAA ACTGTAGTTG 4020

AGATACAGCT ATTTAATATT TCTGGGAGAT GTGCATCCCT CTTCTTTGTG GTTGCCCAAG 4080

GTTGTTTTGC GTAACTGAGA CTCCTTGATA TGCTTCAGAG AATTTAGGCA AACACTGGCC 4140

ATGGCCGTGG GAGTACTGGG AGTAAAATAA AAATATCGAG GTATAGACTA GCATCCACAT 4200

AGAGCACTTG AACCTCCTTT GTACCTGTTT GGGGAAAAAG TATAATGAGT GTACTACCAA 4260

TCTAACTAAG ATTATTATAG TCTGGTTGTT TGAAATACCA TTTTTTTCTC CTTTTGTGTT 4320

TTTCCCACTT TCCAATGTAC TCAAGAAAAT TGAACAAATG TAATGGATCA ATTTAAAATA 4380

TTTTATTTCT TAAAAGCCTT TTTTGCCTGT TGTAATGTGC AGGACCCTTC TCCTTTCATG 4440

GGAGAGACAG GTAGTTACCT GAATATAGGT TGAAAAGGTT ATGTAAAAAG AAATTATAAT 4500

AAAAGGGATA CTTTGCTTTT CAAATCTTTG TTTTCTCTTA TTCTAGGTAA GGCATATTAA 4560

AAATAAATAT GTAAAGAAGA AAAATAAAAG TTGTCTTCAT GG 4602

(2) INFORMATION FOR SEQ ID NO: 75:

(i) SEQUENCE CHARACTERISTICS:
(A) LENGTH: 1255 base pairs
(B) TYPE: nucleic acid
(C) STRANDEDNESS: double
(D) TOPOLOGY: linear

(xi) SEQUENCE DESCRIPTION: SEQ ID NO: 75:

CGCGCCCCGG GCCGGCGGGT TTCTCTAACA AATAAACAGA ACCCGCACTG CCCAGGCGAG 60

CGTTGCCACT TTCAAAGTGG TCCCCTGGGG GAGCTCAGCC TCATCCTGAT GATGCTGCCA 120

AGGCGCACTT TTTATTTTTA TTTTATTTTT ATTTTTTTTT TAGCATCCTT TTGGGGCTTC 180

ACTCTCAGAG CCAGTTTTTA AGGGACACCA GAGCCGCAGC CTGCTCTGAT TCTATGGCTT 240

GGTTGTTACT ATAAGAGTAA TTGCCTAACT TGATTTTTCA TCTCTTTAAC CAAACTTGTG 300

GCCAAAAGAT ATTTGACCGT TTCCAAAATT CAGATTCTGC CTCTGCGGAT AAATATTTGC 360

CACGAATGAG TAACTCCTGT CACCACTCTG AAGGTCCAGA CAGAAGGTTT TGACACATTC 420

TTAGCACTGA ACTCCTCTGT GATCTAGGAT GATCTGTTCC CCCTCTGGAT GAACATCCTC 480

TGATGATCAA GGCTCCCAGC AGGCTACTTT GAAGGGAACA ATCAGATGCA AAAGCTCTTG 540

GGTGTTTATT TAAAATACTA GTGTCACTTT CTGAGTACCC GCCGCTTCAC AGGCTGAGTC 600

CAGGCCTGTG TGCTTTGTAG AGCCAGCTGC TTGCTCACAG CCACATTTCC ATTTGCATCA 660

TTACTGCCTT CACCTGCATA GTCACTCTTT TGATGCTGGG GAACCAAAAT GGTGATGATA 720

TATAGACTTT ATGTATAGCC ACAGTTCATC CCCAACCCTA GTCTTCGAAA TGTTAATATT 780

TGATAAATCT AGAAAATGCA TTCATACAAT TACAGAATTC AAATATTGCA AAAGGATGTG 840

TGTCTTTCTC CCCGAGCTCC CCTGTTCCCC TTCATTGAAA ACCACCACGG TGCCATCTCT 900

TGTGTATGCA GGGCTATGCA CCTGCAGGCA CGTGTGTATG CACTCCCCGC TTGTGTTTAC 960

ACAAGCTGTG GGGTGTTACG CATGCCTGCT TTTTTCACTT AATAATACAG CTTGGAGAGA 1020

TTTTTGTATC ACATTATAAA TCCCACTCGC TCTTTTTGAT GGCCACATAA TAACTACTGC 1080

ATAATATGGA TACGCCTTAT TTGATTTAAC TAGTTCCCTA ATGATGGACT TTTAAGTTGT 1140

TTCCTTTTTT TTTCTTTTTT GCTACTGCAA ACGATGCTAT AATAAATGTC CTTATCAAAA 1200

AAAAAAAAAA AAAAAAAAAA AAAAAANCCC NGGGGGGGGG CCCCGGGAAC NCAAT 1255

(2) INFORMATION FOR SEQ ID NO: 76:

(i) SEQUENCE CHARACTERISTICS:
(A) LENGTH: 475 base pairs
(B) TYPE: nucleic acid
(C) STRANDEDNESS: double
(D) TOPOLOGY: linear

(xi) SEQUENCE DESCRIPTION: SEQ ID NO: 76:

GGCACGAGAG AAATGTTTGA TTCTCTTTCC TATTTTAAGG GATCTTCTCT CTTGTTGATG 60

TTGAAAACTT ACCTTAGTGA AGATGTGTTT CAACATGCTG TTGTCCTTTA CCTGCATAAT 120

CACAGCTATG CATCTATTCA AAGTGATGAT CTGTGGGATA GTTTTAATGA GGTCACAAAC 180

CAAACACTAG ATGTAAAGAG AATGATGAAA ACCTGGACCC TGCAGAAAGG ATTTCCTTTA 240

GTGACTGTTC AAAAGAAAGG AAAGGAACTT TTTATACAAC AAGAGAGATT CTTTTTAAAT 300

ATGAAGCCTG AAATTCAGCC TTCAGATACA AGGTACATGC CCTCTTTCTT TTCATGCCAT 360

CTCTTTTGCA CTCTCAGGTG GAAATATTTT GAAGTGTTTT ATAATCATAA GTTCTTGTGA 420

AACCTAACAA GATTATCCCT TCCTAAGAAT ACTTAACCTT CCTACCAAAT TAAAA 475

(2) INFORMATION FOR SEQ ID NO: 77:

(i) SEQUENCE CHARACTERISTICS:
(A) LENGTH: 465 base pairs
(B) TYPE: nucleic acid
(C) STRANDEDNESS: double
(D) TOPOLOGY: linear

(xi) SEQUENCE DESCRIPTION: SEQ ID NO: 77:

TTCTCTCTGC TCTTCGACTG CACCGCACTC GCGCGTGACC CTGACTCCCC CTAGTCAGCT 60

CAGCGGTGCT GCCATGGCGT GGCGGCGGCG CGAACCRGCG TCGGGGCTCG CGGCGTGTTG 120

GCTCTGGCGT TGCTCGCCCT GGCCCTGTGC GTGCCCGGGG CCCGGGGCCG GGCTCTCGAG 180

TGGTTCTCGG CCGTGGTAAA CATCGAGTAC GTGGACCCGC AGACCAACCT GACGGTGTGG 240

AGCGTCTCGG AGAGTGGCCG CTTCGGCGAC AGCTCGCCCA AGGAGGGCGC GCATGGCCTG 300

GTGGGCGTCC CGTGGGCGCC CGGCGGAGAM CTCGARGGCT KCGCGCCCGA CACGCGCTTC 360

TTCGTGCCCG AGCCCGGCGG CCGAGGGGCC GCGCCCTGGG TCGCCCTGGT GGTCGTGGGG 420

GCTGCACCTT TCAAGGACAA AGTGCTGGTG GCGGCGCNGA ANGAA 465

(2) INFORMATION FOR SEQ ID NO: 78:

(i) SEQUENCE CHARACTERISTICS:
(A) LENGTH: 1907 base pairs
(B) TYPE: nucleic acid
(C) STRANDEDNESS: double
(D) TOPOLOGY: linear

(xi) SEQUENCE DESCRIPTION: SEQ ID NO: 78:

ACATGCAGCC CAACTACAGA TTCTTATGGA ATTCCTCAAG GTTGCAAGAA GAAATAAGAG 60

AGAGCAACTG GAACAGATCC AGAAGGAGCT AAGTGTTTTG GAAGAGGATA TTAAGAGAGT 120

GGAAGAAATG AGTGGCTTAT ACTCTCCTGT CAGTGAGGAT AGCACAGTGC CTCAATTTGA 180

AGCTCCTTCT CCATCACACA GTAGTATTAT TGATTCCACA GAATACAGCC AACCTCCAGG 240

TTTCAGTGGC AGTTCTCAGA CAAAGAAACA GCCTTGGTAT AATAGCACGT TAGCATCAAG 300

ACGAAAACGA CTTACTGCTC ATTTTGAAGA CTTGGAGCAG TGTTACTTTT CTACAAGGAT 360

GTCTCGTATC TCAGATGACA GTCGAACTGC AAGCCAGTTG GATGAATTTC AGGAATGCTT 420

GTCCAAGTTT ACTCGATATA [?][?]TCAGTACG ACCTTTAGCC ACATTGTCAT ATGCTAGTGA 480

TCTCTATAAT GGTTCCAGTA TAGTCTCTAG TATTGAATTT GACCGGGATT GTGACTATTT 540

TGCGATTGCT GGAGTTACAA AGAAGATTAA AGTCTATGAA TATGACACTG TCATCCAGGA 600

TGCAGTGGAT ATTCATTACC CTGAGAATGA AATGACCTGC AATTCGAAAA TCAGCTGTAT 660

CAGTTGGAGT AGTTACCATA AGAACCTGTT AGCTAGCAGT GATTATGAAG GCACTGTTAT 720

TTTATGGGAT GGATTCACAG GACAGAGGTC AAAGGTCTAT CAGGAGCATG AGAAGAGGTG 780

TTGGAGTGTT GACTTTAATT TGATGGATCC TAAACTCTTG GCTTCAGGTT CTGATGATGC 840

AAAAGTGAAG CTGTGGTCTA CCAATCTAGA CAACTCAGTG GCAAGCATTG AGGCAAAGGC 900

TAATGTGTGC TGTGTTAAAT TCAGCCCCTC TTCCAGATAC CATTTGGCTT TCGGCTGTGC 960

AGATCACTGT GTCCACTACT ATGATCTTCG TAACACTAAA CAGCCAATCA TGGTATTCAA 1020

AGGACACCGT AAAGCAGTCT CTTATGCAAA GTTTGTGAGT GGTGAGGAAA TTGTCTCTGC 1080

CTCAACAGAC AGTCAGCTAA AACTGTGGAA TGTAGGGAAA CCATACTGCC TACGTTCCTT 1140

CAAGGGTCAT ATCAATGAAA AAAACTTTGT AGGCCTGGCT TCCAATGGAG ATTATATAGC 1200

TTGTGGAAGT GAAAATAACT CTCTCTACCT GTACTATAAA GGACTTTCTA AGACTTTGCT 1260

AACTTTTAAG TTTGATACAG TCAAAAGTGT TCTCGACAAA GACCGAAAAG AAGATGATAC 1320

AAATGAATTT GTTAGTGCTG TGTGCTGGAG GGCACTACCA GATGGGGAGT CCAATGTGCT 1380

GATTGCTGCT AACAGTCAGG GTACAATTAA GGTGCTAGAA TTGGTATGAA GGGTTAACTC 1440

AAGTCAAATT GTACTTGATC CTGCTGAAAT ACATCTGCAG CTGACAATGA GAGAAGAAAC 1500

AGAAAATGTC ATGTGATGTC TCTCCCCAAA GTCATCATGG GTTTTGGATT TGTTTTGAAT 1560

ATTTTTTTCT TTTTTTCTTT TCCCTCCTTT ATGACCTTTG GGACATTGGG AATACCCAGC 1620

CAACTCTCCA CCATCAATGT AACTCCATGG ACATTGCTGC TCTTGGTGGT GTTATCTAAT 1680

TTTTGTGATA GGGAAACAAA TTCTTTTGAA TAAAAATAAA TAACAAAACA ATAAAAGTTT 1740

ATTGAGCCAC AGTTGAGCTT GGAAAGTTTT TGTCAAATGC NGCAAGAGAT AACTCTTTTT 1800

ANGAAGTAGC ATATGTGAAC TATAATGTAA CAGTGAATAA TTTGTAAAGT TCGTATTTCC 1860

CAACCTCTTT GGGAATTACA CATATCAATA TAAACAAAAT ATAAAGT 1907

(2) INFORMATION FOR SEQ ID NO: 79:

(i) SEQUENCE CHARACTERISTICS:
(A) LENGTH: 1168 base pairs
(B) TYPE: nucleic acid
(C) STRANDEDNESS: double
(D) TOPOLOGY: linear

(xi) SEQUENCE DESCRIPTION: SEQ ID NO: 79:

GCTGGGGTGT CCCCKCSGCC ACCATCGTCA TCGCTTACTT GATGAAGCAC ACTCGGATGA 60

CCCATGACTG ATGCTTATAA ATTTGTCAAA GGCAAACGAC CAATTATCTC CCCAAACCTT 120

AACTTCATGG GGCAGTTGCT AGAGTTCGAG GAAGACCTAA ACAACGGTGT GACACCGAGA 180

ATCCTTACAC CAAAGCTGAT GGGCGTGGAG ACGGTTGTGT GACAATGGTC TGGATGGAAA 240

GGATTGCTGC TCTCCATTAG GAGACAATGA GGAAGGAGGA TGGATTCTGG TTTTTTTTCT 300

TTCTTTTTTT TTTTGTAGTT GGGAGTAAGT TTGTGAATGG AAACAAACTT GTTTAAACAC 360

TTTATTTTTA ACAAGTGTAA GAAGACTATA ACTTTTGATG CCATTGAGAT TCACCTCCCA 420

CAAACTGACA AATTAAGGAG GTTAAAGAAG TAATTTTTTT AAGCCAACAA TAAAAATATA 480

ATACAACTTG TTTCTCCCCC TTTTCCTTTT AAGCTATTTG TAGAGTTTAT GACTAAATAG 540

TCTGTGCAGG TTCATAGACC GAAGATACTA CACACTTTAA ACCAATTAAA AAGAACCAAA 600

AGTAAATAGA AAAGACATTG AATCACCAAG GCCTGGGATC AACCTGGGCT GTCCACACAG 660

AAAACAAAAA CCCAACCAAA CCAAGCCCTG TTGTGCTCAC TGGTGCAAAG AGAAGATCAG 720

GGCAGCTTAA GTGGTCTAAG RATCCTTCAG GCATTCTTTA AGGAGAAAAA GGATACCTTT 780

GATTTTGTGT GTTTCATGCT CTGGATTTTT TTTTTTTTTC CTTCTCTGGG TTTAAGAGAT 840

TTTTTTTGAA ATAGTGAGGA ACTGACCATT ATATGCCTTC ACTGGCTTCT TGTGCAATAA 900

TATGATGTTT TAAGTGTGCA AACAAGTTAG AGCTGGCAGC TGAATGATAG ACAAATAGTG 960

CAAATTTGCC AGCTTGGAGA TAGAAAGGAA TTCAACAATA TATCAAATAC TTTCCTTCCC 1020

ACCTTTTTCC TTTTTTTTTT TTTTTTCTGA TTTGATTCTG GTTACAGTGC CATAAACCTT 1080

GTTACATATG TATATCAGAA TGTAAGAAAA AAAAATTTAT TTAAAAATAT TTTTCGCAAA 1140

AAAAAAANNA AAAAACTCGA GGGGGGCC 1168

(2) INFORMATION FOR SEQ ID NO: 80:

(i) SEQUENCE CHARACTERISTICS:
(A) LENGTH: 1285 base pairs
(B) TYPE: nucleic acid
(C) STRANDEDNESS: double
(D) TOPOLOGY: linear

(xi) SEQUENCE DESCRIPTION: SEQ ID NO: 80:

AGAAAATCAC ATCCTAACAA AGAAGTCTGT CTAAGACAGT ACATCTCCTG TTGAACTTGC 60

ATCTTTCCAC AGGACTTTCT GTTTTTAGGG ATGAGACTAT TCTCTGCTTC ATCAAGGAAA 120

GAGAAATGTT CAGGGTTGTA GGGATGGCAC ACTTATTAGT TCTGCCTGTC TGAAAGGTTC 180

CTGCAGGACA GTTTGGTCAG AGCTGCAATT CTTAGTCCAT GGTCTAATGC TTGAGTATCT 240

CTTCTTTCCC TTTCCTGTCT CAGGAATCAG CTGAGAATTC ATTCGATTGT CATGCCTCTA 300

GCCCCTTACT GTGATTTGTT GGTTGCACTT TCATTTGCTT TAGTTCTAGA ATCACCTGTT 360

GACTCCTCAG ACTTCACCTA ACTTTGGAAA CTCTCTTTTG GAGGCTTCTC ATTTCCCCCT 420

AATTCTGTGC TGCCTGAGCC CTAGAATTTT CCCACCAACG AATTATTCCA GGTAGATCCT 480

AAGTTGCTGG ATCTAGTTGA TATTTAAACA ATATCTAGTT GATATTTCTC ATTCAGTTGG 540

ATCCAGAAAC CAGTATCTCT NAAAAACAAC CTCTCATACC TTGTGGACCT AATTTTGTGT 600

GCGTGTGTGT GTGCGCGCAT ATGTATATAG ACAGGCACAT CTTTTTTACT TTTGTAAAAG 660

CTTATGCCTC TTTGGTATCT ATATCTGTGA AAGTTTTAAT GATCTGCCAT AATGTCTTGG 720

GGACCTTTGT CTTCTGTGTA AATGGTACTA GAGAAAACAC CTATATTATG AGTCAATCTA 780

GTTGGTTTTA TTCGACATGA AGGAAATTTC CAGATAACAA CACTAACAAA CTCTCCCTTG 840

ACTAGGGGGA CAAAGAAAAG CAAAACTGAC CATAAAAAAC AATTACCTGG TGAGAAGTTG 900

CATAAACAGA ATTAGGTAGT ATATTGAAGA CAGCATCATT AAACAGTTAT GTTGTTCTCC 960

TTGCAAAAAA CATGTACTGA CTTCCCGTTG AGTAATGCCA AGTTGTTTTT TTTATTATAA 1020

AACTTGCCCT TCATTACATG TTTCAAAGTG GTGTGGTGGG CCAAAATATT GAAATGATGG 1080

AACTGACTGA TAAAGCTGTA CAAATAAGCA GTGTGCCTAA CAAGCAACAC AGTAATGTTG 1140

ACATGCTTAA TTCACAAATG CTAATTTCAT TATAAATTGT TTTGCTAAAA TACACTTTGA 1200

AACTATTTTT CTGTATTCCA AGAGCTGAGA TCTTAGATTT TATGTAGTAT TAAGTGAAAA 1260

AATACGAAAA TAATAAACAT TGAAG 1285

(2) INFORMATION FOR SEQ ID NO: 81:

(i) SEQUENCE CHARACTERISTICS:
(A) LENGTH: 1290 base pairs
(B) TYPE: nucleic acid
(C) STRANDEDNESS: double
(D) TOPOLOGY: linear

(xi) SEQUENCE DESCRIPTION: SEQ ID NO: 81:

TCTCCAGCCC CAATTTCTAC GCGCACCGGA AGACGGAGGT CCTCTTTCCT TGCCTAACGC 60

AGCCATGGCT CGTGGTCCCA AGAAGCATCT GAAGCGGGTG GCAGCTCCAA AGCATTGGAT 120

GCTGGATAAA TTGACCGGTG TGTTTGCTCC TCGTCCATCC ACCGGTCCCC ACAAGTTGAG 180

AGAGTGTCTC CCCCTCATCA TTTTCCTGAG GAACAGACTT AAGTATGCCC TGACAGGAGA 240

TGAAGTAAAG AAGATTTGCA TGCAGCGGTT CATTAAAATC GATGGCAAGG TCCGAACTGA 300

TATAACCTAC CCTGCTGGAT TCATGGATGT CATCAGCATT GACAAGACGG GAGAGAATTT 360

CCGTCTGATC TATGACACCA AGGGTCGCTT TGCTGTACAT CGTATTACAC CTGAGGAGGC 420

CAAGTACAAG TTGTGCAAAG TGAGAAAGAT CTTTGTGGGC ACAAAAGGAA TCCCTCATCT 480

GGTGACTCAT GATGCCCGCA CCATCCGCTA CCCCGATCCC CTCATCAAGG TGAATGATAC 540

CATTCAGATT GATTTAGAGA CTGGCAAGAT TACTGATTTC ATCAAGTTCC ATTCACCCAG 600

CCAGGTGGTC TCGTCACCTC AGAGGCTCCG CAGACTCCTG CCCAGGCCAG GACTGAGGCA 660

AGCCTCAAGG CACTTCTAGG ACCTGCCTCT TCTCACCAAG ATGAACTCAC TGGTTTCTTG 720

GCAGCTACTG CTTTTCCTCT GTGCCACCCA CTTTGGGGAG CCATTAGAAA AGGTGGCCTC 780

TGTGGGGAAT TCTAGACCCA CAGGCCAGCA GCTAGAATCC CTGGGCCTCC TGGCCCCSGG 840

GGAGCAGAGC CTGCCGTGCA CCGAGAGGAA GCCAGCTGCT ACTGCCAGGC TGAGCCGTCG 900

GGGGACCTCG CTGTCCCCGC CCCCCGAGAG CTCCGGGAGC CCCCAGCAGC CGGGCCTGTC 960

CGCCCCCCAC AGCCGCCAGA TCCCCGCACC CCAGGGCGCG GTGCTGGTGC AGCGGGAGAA 1020

GGACCTGCCG AACTACAACT GGAACTCCTT CGGCCTGCGC TTCGGCAAGC GGGAGGCGGC 1080

ACCAGGGAAC CACGGCAGAA GCGCTGGGCG GGGCTGAGGG CGCAGGTGCG GGGCAGTGAA 1140

CTTCAGACCC CAAAGGAGTC AGAGCATGCG GGGCGGGGGC GGGGGGCGGG GACGTAGGGC 1200

TAAGGGAGGG GGCGCTGGAG CTTCCAACCC GAGGCAATAA AAGAAATGTT GCGTAACTCA 1260

AAAAAAAAAA AAAAAAAANC TCGGGGGGGG 1290

(2) INFORMATION FOR SEQ ID NO: 82:

(i) SEQUENCE CHARACTERISTICS:
(A) LENGTH: 684 base pairs
(B) TYPE: nucleic acid
(C) STRANDEDNESS: double
(D) TOPOLOGY: linear

(xi) SEQUENCE DESCRIPTION: SEQ ID NO: 82:

TTTATTGTAT TCTGTAACTA TAGAACTTCT ATTTWATTCT TTTTTGGACT TGCTAAGTTG 60

TCTTTWATGG TTTTWAGTTC CATGCTGAAG TTTTCAGTAT TGACTTATCC CCTTGAACAT 120

GAGTTGTTTT ATAGACTCTR ATGATTCAAA AATCTTACAT CTTTTGGTAG TCTCTTTCAT 180

TTGTYCACTG TTTCTGTTGA TTCTWACTCA TGGTATTTTA ATTCTTCGTT WTTTTTTTTC 240

TGTTWAGAWA CATTCTTTGA AAAATAATTT GGAGGAATAT TTGATTCTTA TGAACAAGGC 300

ATTACTCACC AGAGAAGATT TTTTTGTTYT ACCARGTGCC TARGAATGCT AACAGTCTGG 360

GAMCACATAG AMCACCAGGT GATGAGACAA TCCTGGGART CCTGTTTTAC TTTGGSCCAT 420

CTTTTCTCCC AACCCTGTGG GAATARTCAT YCATATCCTA RCTGCAGGCT ARAAGGTGGT 480

TTATCAGAGC CCAACTTCGA GGGCTCTGGG CTTTAGCTAC TGTCACCCCA TCATAACTGA 540

GCTTCATGGA TTGATTCTCT TTTTATCTTT CAGATTTTCT TTTAAAAATC TTTGTTTTTT 600

TTTTTCTTCC GAAAGATTCC CCCAACATTA CCATTCCCCA CCTTCCGTTG AATTTTTTTG 660

GCTCTCATTT TGAATTTTTC AAGA 684

(2) INFORMATION FOR SEQ ID NO: 83:

(i) SEQUENCE CHARACTERISTICS:
(A) LENGTH: 2024 base pairs
(B) TYPE: nucleic acid
(C) STRANDEDNESS: double
(D) TOPOLOGY: linear

(xi) SEQUENCE DESCRIPTION: SEQ ID NO: 83:

CTGCAGGAAT TCGGCACAGC TGCGCTGGAG GCTTCATCTT TGCCGCCGCT GCCGTCGCCT 60

TCCTGGGATT GGAGTCTCGA GCTTTCTTCG TTCGTTCGYC GGCGGGTTCG CGCCCTTCTC 120

GCGCCTCGGG GCTGCGAGGC TGGGGAAGGG GTTGGAGGGG GCTGTTGATC GCCGCGTTTA 180

AGTTGCGCTC GGGGCGGCCA TGTCGGCCGG CGAGGTCGAG CGCCTAGTGT CGGAGCTGAG 240

CGGCGGGACC GGAGGGGATG AGGAGGAAGA GTGGCTCTAT GGCGATGAAA ATGAAGTTGA 300

AAGGCCAGAA GAAGAAAATG CCAGTGCTAA TCCTCCATCT GGAATTGAAG ATGAAACTGC 360

TGAAAATGGT GTACCAAAAC CGAAAGTGAC TGAGACCGAA GATGATAGTG ATAGTGACAG 420

CGATGATGAT GAAGATGATG TTCATGTCAC TATAGGAGAC ATTAAAACGG GAGCACCACA 480

GTATGGGAGT TATGGTACAG CACCTGTAAA TCTTAACATC AAGACAGGGG GAAGAGTTTA 540

TGGAACTACA GGGACAAAAG TCAAAGGAGT AGACCTTGAT GCACCTGGAA GCATTAATGG 600

AGTTCCACTC TTAGAGGTAG ATTTGGATTC TTTTGAAGAT AAACCATGGC GTAAACCTGG 660

TGCTGATCTT TCTGATTATT TTAATTATGG GTTTAATGAA GATACCTGGA AAGCTTACTG 720

TGAAAAACAA AAGAGGATAC GAATGGGACT TGAAGTTATA CCAGTAACCT CTACTACAAA 780

TAAAATTACG GTACAGCAGG GAAGAACTGG AAACTCAGAG AAAGAAACTG CCCTTCCATC 840

TACAAAAGCT GAGTTTACTT CTCCTCCTTC TTTGTTCAAG ACTGGGCTTC CACCGAGCAG 900

GAGATTACCT GGGGCAATTG ATGTTATCGG TCAGACTATA ACTATCAGCC GAGTAGAAGG 960

CAGGCGACGG GCAAATGAGA ACAGCAACAT ACAGGTCCTT TCTGAAAGAT CTGCTACTGA 1020

AGTAGACAAC AATTTTAGCA AACCACCTCC GTTTTTCCCT CCAGGAGCTC CTCCCACTCA 1080

CCTTCCACCT CCTCCATTTC TTCCACCTCC TCCGACTGTC AGCACTGCTC CACCTCTGAT 1140

TCCACCACCG GGTTTTCCTC CTCCACCAGG CGCTCCACCT CCATCTCTTA TACCAACAAT 1200

AGAAAGTGGA CATTCCTCTG GTTATGATAG TCGTTCTGCA CGTGCATTTC CATATGGCAA 1260

TGTTGCCTTT CCCCATCTTC CTGGTTCTGC TCCTTCGTGG CCTAGTCTTG TGGACACCAG 1320

CAAGCAGTGG GACTATTATG CCAGAAGAGA GAAAGACCGA GATAGAGAGA GAGACAGAGA 1380

CAGAGAGCGA GACCGTGATC GGGACAGAGA AAGAGAACGC ACCAGAGAGA GAGAGAGGGA 1440

GCGTGATCAC AGTCCTACAC CAAGTGTTTT CAACAGCGAT GAAGAACGAT ACAGATACAG 1500

GGAATATGCA GAAAGAGGTT ATGAGCGTCA CAGAGCAAGT CGAGAAAAAG AAGAACGACA 1560

TAGAGAAAGA CGACACAGGG AGAAAGAGGA AACCAGACAT AAGTCTTCTC GAAGTAATAG 1620

TAGACGTCGC CATGAAAGTG AAGAAGGAGA TAGTCACAGG AGACACAAAC ACAAAAAATC 1680

TAAAAGAAGC AAAGAAGGAA AAGAAGCGGG CAGTGAGCCT GCCCCTGAAC AGGAGAGCAC 1740

CGAAGCTACA CCTGCAGAAT AGGCATGGTT TTGGCCTTTT GTGTATATTA GTACCAGAAG 1800

TAGATACTAT AAATCTTGTT ATTTTTCTGG ATAATGTTTA AGAAATTTAC CTTAAATCTT 1860

GTTCTGTTTG TTAGTATGAA AAGTTAACTT TTTTTCCAAA ATAAAAGAGT GAATTTTTCA 1920

TGTTAAGTTA AAAATCTTTG TCTTGTACTA TTTCAAAAAT AAAAAGACAG CAATGACTTT 1980

ATATCCAAAA AAAAAAAAAA AAAAAAAAAA AAAAAAGGGC GGCC 2024

(2) INFORMATION FOR SEQ ID NO: 84:

(i) SEQUENCE CHARACTERISTICS:
(A) LENGTH: 931 base pairs
(B) TYPE: nucleic acid
(C) STRANDEDNESS: double
(D) TOPOLOGY: linear

(xi) SEQUENCE DESCRIPTION: SEQ ID NO: 84:

CGCGCCMATA GCCGGACGGG GATCTGAGCT GGCAGGATGA ATGTGGGGGT GGCACACAGC 60

GAAGTAAACC CCAACACCCG AGTGATGAAT AGCCGAGGCA TCTGGCTGGC CTACATCATC 120

TTGGTAGGAT TGCTGCATAT GGTTCTACTC AGCATCCCCT TCTTCAGCAT TCCTGTTGTC 180

TGGACCCTGA CCAACGTCAT CCATAACCTG GCTACGTATG TCTTCCTTCA TACGGTGAAA 240

GGGACACCCT TTGAGACTCC TGACCAAGGA AAGGCTCGGC TACTGACACA CTGGGAGCAA 300

ATGGACTATG GGCTCCAGTT TACCTCTTCC CGCAAGTTCC TCAGCATCTC TCCTATTGTG 360

CTCTATCTCC TGGCCAGCTT CTATACCAAG TATGATGCTG CGCACTTCCT CATCAACACA 420

GCCTCATTGC TAAGTGTACT GCTGCCGAAG TTGCCCCAGT TCCATGGGGT TCGTGTCTTT 480

GGCATCAACA AATACTGAGG GATGGGTTTT GGGACAGCTC CATGGGCATG GGGAAGGCAC 540

TGAAACAGAG GACTATAAAA CATCCTTCTC TTATTCTCCA TACTGTCTTC TACACCTTTA 600

AAGCCTGAGA ACTATACAAC CTTTCCCAGA CTCCCAAGAA GAGAAGAGAT TGGCAAATGG 660

GGCTCCTGGG CCCAGTCCTG CTAGTGGCAA GTTTCTTTGA ATCAGGAAGG CAGGTGAGGT 720

AAGGGCCAAA TCACTCTCCT CCATAGCAGG AAGCCATTTG GGCAGCTCCT TTGGTGATTA 780

CATCTTTCCA TATCTTTTAC ACTTACCACC TTCCAGCTCT GTTTTGCTGT GTATTTTTCT 840

TACAATAATT TTTTTCAGCT ATAGCTGCAG TTTAATCAGG ATGGGTAGAG AGCTGTCCTC 900

ATAAGGCTGG GGGTGGGAAG ATGGAATACT G 931

(2) INFORMATION FOR SEQ ID NO: 85:

(i) SEQUENCE CHARACTERISTICS:
(A) LENGTH: 825 base pairs
(B) TYPE: nucleic acid
(C) STRANDEDNESS: double
(D) TOPOLOGY: linear

(xi) SEQUENCE DESCRIPTION: SEQ ID NO: 85:

CGGGGCCGGC GGGGTCTTCA GGGTACCGGG CTGGTTACAG CAGCTCTACC CCTCACGACG 60

CAAACATGGC AGCGCAGAAG GACCAGCAGA AAGATGCCGA GGCGGAAGGG CTGAGCGGCA 120

CGACCCTGCT GCCGAAGCTG ATTCCCTCCG GTGCAGGCCG GGAGTGGCTG GAGCGGCGCC 180

GCGCGACCAT CCGGCCCTGG AGCACCTTCG TGGACCAGCA GCGCTTCTCA CGGCCCCGCA 240

ACCTGGGAGA GCTGTGCCAG CGCCTCGTAC GCAACGTGGA GTACTACCAG AGCAACTATG 300

TGTTCGTGTT CCTGGGCCTC ATCCTGTACT GTGTGGTGAC GTCCCCTATG TTGCTGGTGG 360

CTCTGGCTGT CTTTTTCGGC GCCTGTTACA TTCTCTATCT GCGCACCTTG GAGTCCAAGC 420

TTGTGCTCTT TGGCCGAGAG GTGAGCCCAG CGCATCAGTA TGCTCTGGCT GGAGGCATCT 480

CCTTCCCCTT CTTCTGGCTG GCTGGTGCGG GCTCGGCCGT CTTCTGGGTG CTGGGAGCCA 540

CCCTGGTGGT CATCGGCTCC CACGCTGCCT TCCACCAGAT TGAGGCTGTG GACGGGGAGG 600

AGCTGCAGAT GGAACCCGTG TGAGGTGTCT TCTGGGACCT GCCGGCCTCC CGGGCCAGCT 660

GCCCCACCCC TGCCCATGCC TGTCCTGCAC GGCTCTGCTG CTCGGGCCCA CAGCGCCGTC 720

CCATCACAAG CCCGGGGAGG GATCCCGCCT TTGAAAATAA AGCTGTTATG GGTGTCATTC 780

AGGAAAAAAA AAAAAAAAGG GGGGCCCCTC TAGGGGTCAA AGTTA 825

(2) INFORMATION FOR SEQ ID NO: 86:

(i) SEQUENCE CHARACTERISTICS:
(A) LENGTH: 1238 base pairs
(B) TYPE: nucleic acid
(C) STRANDEDNESS: double
(D) TOPOLOGY: linear

(xi) SEQUENCE DESCRIPTION: SEQ ID NO: 86:

CATGTAAAAG GATGAAATGT GACTTCTGGT GTTTTTTTAT TTCTATGGAG GGACTTTCTG 60

GGGACGGTTT CTGGCTCTCA GGCTCTGAGA AGCTGCAGTT TATGAGTGGC TCTGTGTGTG 120

CTGCCACCTA CTGGAGAAGC CATAAGCTGC AGCTTTAGGA AAAGGGAACC CGGGGCAGAG 180

TGTGGGGAAG TGGGATGGCA GCATGGCAGG GCTTTGGAAA ATGAGAGGTG AGAGTKTKTC 240

CAGGAAGGGT GTAAGGAGAG GATGGATCCT GATACATGGA TTCAGGATCA TTAGGGTCCT 300

GTCTGGGACA CTGGCCTTCC TGCTTACCTG CTCTTTCCTT CCTCCTTGGT CGGAGGAGGG 360

GCTGGCTCAC TGCTCTGGCT TCATTTTCCA GAGCTGCCTG CTGCAGTCAC ACTTAGGTCA 420

TCTTCTCTCA CTTTTCTCCT TTTGCCGATT AGTGGACGTG ACAGAGATGT GAATGGGGCA 480

GGGATGTCCT TTGATGGCAT CAAGACTTTA GCTTCTGGTG CGCTGTGTCC CAGCTCTGAT 540

TTCAGTTGCA GCCGTGATGG AMAGTTNGCA TGGAAGCTGA GACTCTCACT GACAGTGAAA 600

CCCTCAAATG AACACAATCC CTGCTTTCCT GCCAAGGATC CTTGTAGGGT NCCCCCAGCT 660

TCCCCACTTT TTTTCTGTGT CCTGACAAAG AAACACAGAG TAACTTGATT GCCCTGTGAC 720

CTGGCCAGTT GCATTTCCCC TGCAGGCTTG AGCCCAAGCC AGAGCCTTGA AAAGGTATTC 780

AGGTTGTTGC CCAAAACACT GAAAAAAACT GCCCTGGCCC TGAACCAAAT ACCTTGAACC 840

CTCGTAAACT CCATACCCTG ACCCCCTTGT TTTGGATATA CCCAGGTAGA ACAACTCTCT 900

CTCACTGTCT GTTGTGAGGA TACGCTGTAG CCCACTCATT AAGTACATTC TCCTAATAAA 960

TGCTTTGGAC TGATCACCCT GCCAGTCTTT TGTCTTGGGC AATCTATACT TTTNCTCAGA 1020

GGTTCCCAAG GCCTACTGAA GGGACTTAAC ATACTCTTAA TGGCTTTCCT CTCTCTTGTT 1080

TTACCTTATG CCCTCACTTC CTGAGTTAAC CTCCCAAATA CAGGATTCAC CTGTACCCAA 1140

GCCCTTAGCT TCAAGAATAC AGGATCACCT GTACCCAAGC CCTTAGCTCA AGCTCTGCTT 1200

TGGAAGAACC CAAACTAAGA CAGTGCTCCT GGTGCCCT 1238

(2) INFORMATION FOR SEQ ID NO: 87:

(i) SEQUENCE CHARACTERISTICS:

(A) LENGTH: 1460 base pairs
(B) TYPE: nucleic acid
(C) STRANDEDNESS: double
(D) TOPOLOGY: linear

(xi) SEQUENCE DESCRIPTION: SEQ ID NO: 87:

ATTGCCTTCT GGTCCCTGGT GACACTGGGG TCATCCTTCA TCCCCGGAGA GCATTTCTGG 60

CTGCTCCTCC TGACCCGGGG CCTGGTGGGG GTCGGGGAGG CCAGTTATTC CACCATCGCG 120

CCCACTCTCA TTGCCGACCT CTTTGTGGCC GACCAGCGCG ACCGGATGCT CAGCATCTTC 180

TACTTTGCCA TTCCGGTGGG CAGTGGTCTG GGCTACATTG CAGGCTCCAA AGTGAAGGAT 240

ATGGCTGGAG ACTGGCACTG GGCTCTGAGG GTGACACCGG GTCTAGGAGT GGTGGCCGTT 300

CTGCTGCTGT TCCTGGTAGT GCGGGAGCCG CCAAGGGGAG CCGTGGAGCG CCACTCAGAT 360

TTGCCACCCC TGAACCCCAC CTCGTGGTGG GCAGATCTGA GGGCTCTGGC AAGAAATCCT 420

AGTTTCGTCC TGTCTTCCCT GGGCTTCACT GCTGTGGCCT TTGTCACGGG CTCCCTGGCT 480

CTGTGGGCTC CGGCATTCCT GCTGCGTTCC CGCGTGGTCC TTGGGGAGAC CCCACCCTGC 540

CTTCCCGGAG ACTCCTGCTC TTCCTCTGAC AGTCTCATCT TTGGACTCAT CACCTGCCTG 600

ACCGGAGTCC TGGGTGTGGG CCTGGGTGTG GAGATCAGCC GCCGGCTCCG CCACTCCAAC 660

CCCCGGGCTG ATCCCCTGGT CTGTGCCACT GGCCTCCTGG GCTCTGCACC CTTCCTCTTC 720

CTGTCCCTTG CCTGCGCCCG TGGTAGCATC GTGGCCACTT ATATTTTCAT CTTCATTGGA 780

GAGACCCTCC TGTCCATGAA CTGGGCCATC GTGGCCGACA TTCTGCTGTA CGTGGTGATC 840

CCTACCCGAC GCTCCACCGC CGAGGCCTTC CAGATCGTGC TGTCCCACCT GCTGGGTGAT 900

GCTGGGAGCC CCTACCTCAT TGGCCTGATC TCTGACCGCC TGCGCCGGAA CTGGCCCCCC 960

TCCTTCTTGT CCGAGTTCCG GGCTCTGCAG TTCTCGCTCA TGCTCTGCGC GTTTGTTGGG 1020

GCACTGGGCG GCGCACTTCC TGGGCACCGC CATCTTCATT GAGGCCGACC GCCGGCGGGC 1080

ACAGCTGCAC GTGCAGGGCC TGCTGCACGA AGCAGGGTCC ACAGACGACC GGATTGTGGT 1140

GCCCCAGCGG GGCCGCTCCA CCCGCGTGCC CGTGGCCAGT GTGCTCATCT GGAGAGGCTG 1200

CCGCTCACCT ACCTGCACAT CTGCCACAGC TGGCCCTGGG CCCACCCCAC GAAGGGCCTG 1260

GGCCTAAACC CCTTGGCCTG GCCCAGCTTC CAGAGGGACC CTGGGCCGTG TGCCAGCTCC 1320

CAGACACTAC ATGGGTAGCT CAGGGGAGGA GGTGGGGGTC CAGGAGGGGG ATCCCTCTCC 1380

AACAGGGGCA GCCCCAAGGG CTCGGTGCTA TTTGTAACGG GATTAAAATT TGTAGCCAGA 1440

AAAAAAAAAA AAAAAAAAAA 1460

(2) INFORMATION FOR SEQ ID NO: 88:

(i) SEQUENCE CHARACTERISTICS:
(A) LENGTH: 1395 base pairs
(B) TYPE: nucleic acid
(C) STRANDEDNESS: double
(D) TOPOLOGY: linear

(xi) SEQUENCE DESCRIPTION: SEQ ID NO: 88:

CAGGTGCAAA GTGGGAAGTG TGAGTCCTCA GTCTTGGGCT ATTCGGCCAC GTGCCTGCCG 60

GACATGGGAC GCTGGAGGGT CAGCAGCGTG GAGTCCTGGC CTTTTGCGTC CACGGGTGGG 120

AAATTGGCCA TTGCCACGGC GGGAACTGGG ACTCAGGCTG CCCCCCGGCC GTTTCTCATC 180

CGTCCACCGG AYTCGTGGGC GCTCGCACTG GCGCTGATGT AGTTTCCTGA CCTCTGACCC 240

GTATTGTCTC CAGATTAAAG GTACGACATT TGGAGGCCCC AGCGAGAAAC GTCACCGGGA 300

GAAACGTCAC CGGGCGAGAG CGGKCCCGCT GTGTGCTCCC CCGGAAGGAC AGCCAGCTTG 360

TAGGGGGGAG TGCCACCTGA AAAAAAAATT TCCAGGTCCC CAAAGGGTGA CCGTCTTCCG 420

GAGACAGCGG ATCGACTACC ATGTGGGTGC CCACAAAAAT TYCACCTYTG AGTCCTCAAC 480

TGCTGACCCC GGGGTCAGTT CCAGAGAGAA GGACTCCCTC CTGCTTGGAA GAGACCTCAC 540

ACCGTCATCA CGATGCCAAC GGCTCTGAAG GTGGATGGCA TTCCTGCGTG GATTCATCAC 600

TCCCGCATCA AAAAGGCCAA CRGAGCCCAA CTAGAAACAT GGGTCCCCAG GGCTGGGTCA 660

GGCCCCTTAA AACTGCACCT AAGTTGGGTG AAGCCATTAG ATTAATTCTT TTTCTTAATT 720

TTGTAAAACA ATGCATAGCT TCTGTCAACT TATGTATCTT AAGACTCAAT ATAACCCCCT 780

TGTTATAACT GAGGGAATCA ATGATTTGAT TCCCCAAAAA CACAAGTGGG GAATGTAGTG 840

TCCAACCTGG TTTTTACTAA CCCTGTTTTT AGACTYTCCC TTTCCTTTAA TCACTCAGCC 900

TTGTTTCCAC CTGAATTGAC TCTCCCTTAG CTAAGAGCGC CAGATGGACT CCATCTTGGC 960

TCTTTCNACT GGCAGCCGCT TCCTYCAAGG ACTTAACTTG TGCAAGCTGA CTCCCAGCAC 1020

ATCCAAGAAT GCAATTAACT GATAAGATAC TGTGGCAAGC TATATCCGCA GTTCCCAGGA 1080

ATTCGTCCAA TTGATTACAC CCMAAAGCCC CGCGTCTATC ACCTTGTAAT AATCTTAAAG 1140

CCCCTGCACC TGGAACTATT AACGTTCCTG TAACCATTTA TCCTTTTAAC TTTTTTGCCT 1200

ACTTTATTTC TGTAAAATTG TTTTAACTAG ACCCCCCCTC TCCTTTCTAA ACCAAAGTAT 1260

AAAAGCAAAT CTAGCCCCTT CTTCAGGCCG AGAGAATTTC GAGCGTTAGC CGTCTCTTGG 1320

CCACCAGCTA AATAAACGGA TTCTTCATGT GTAAAAAAAA AAAAAAAAAA CTCGGAGGGG 1380

GGGCCCGGTA CCCAA 1395

(2) INFORMATION FOR SEQ ID NO: 89:

(i) SEQUENCE CHARACTERISTICS:
(A) LENGTH: 1186 base pairs
(B) TYPE: nucleic acid
(C) STRANDEDNESS: double
(D) TOPOLOGY: linear

(xi) SEQUENCE DESCRIPTION: SEQ ID NO: 89:

GGCACGAGCC GGCAAGCCGA GCTAGGGTGA AAACTGGGGG CGCACCAGGA TGTNNGACAG 60

AAAAGCAGAA GATGAGACTC TGTTCATTCA CTTTTCCTAG GCCCATCCTG TGGTCATCTT 120

TCCCCCTCCC ATCATACCTC CTCCTTCCTG GAGCCTCTGC CGGCTTGGCT GTAATGGTGG 180

CACTTACCTG GATATTTCAG TGGGAGGATG AAAGGCGAGA CTCACCCTAC GCGGTGGGAC 240

AGATGGGGAG AGGAAAAAGG CAGAGATGGC CAGGAGAGGG GTGCAGGACA AACCAGAGAG 300

GTTGGGTCAG GGGAAAAGGG TGGGGAGAAA GAGGGGTGCA GGCCCTGCAG GCCGGTTAGC 360

CAGCAGCTGC GGCCTCCCCG GGCCCTTGGC ATCCAACTTC GCAGACAGGG TACCAGCCTC 420

CTGGTGTGTA TCATAGGATT TGTTCACATA GTGTTATGCA TGATCTTCGT AAGGTTAAGA 480

AGCCGTGGTG GTGCACCATG ACATCCAACC CGTATATATA AAGATAAATA TATATATATA 540

TGTATGTAAA TTATGGCACG AGAAATTATA GCACTGAGGG CCCTGCTGCC CTGCTGGACC 600

AAGCAAAACT AAGCCTTTTG GTTTGGGTAT TATGTTTCGT TTTGTTATTT GTTTGTTTTT 660

GTGGCTTGTC TTATGTCGTG ATAGCACAAG TGCCAGTCGG ATTGCTCTGT ATTACAGAAT 720

AGTGTTTTTA ATTCATCAAT GTTCTAGTTA ATGTCTACCT CAGCACCTCC TCTTAGCCTA 780

ATTTTAGGAG GTTGCCCAAT TTTGTTTCTT CAATTTTACT GGTTACTTTT TTGTACAAAT 840

CAATCTCTTT CTCTCTTTCT CTCCTCCCCA CCTCTCACCC TTGCCCTCTC CATCTCCCTC 900

TCCCGCCCTC CCCTCCTCCC TCTGGCTCCC CGTCTCATTT CTGTCCACTC CATTCTCTCT 960

CCCTCTCTCC TGCCTCCTGC TGCCCCCTCC CCAGCCCACT TCCCCGAGTT GTGCTTGCCG 1020

CTCCTTATCT GTTCTAGTTC CGAAGCAGTT TCACTCGAAG TTGTGCAGTC CTGGTTGCAG 1080

CTTTCCGCAT CTGCCTTCGT TTCGTGTAGA TTGACGCGTT TCTTTGTAAT TTCAGTGTTT 1140

CTGACAAGAT TTAAAAAAAA AAAAAGGAAA AAAAAAAAAA AAAAAA 1186

(2) INFORMATION FOR SEQ ID NO: 90:

(i) SEQUENCE CHARACTERISTICS:
(A) LENGTH: 1821 base pairs
(B) TYPE: nucleic acid
(C) STRANDEDNESS: double

(D) TOPOLOGY: linear

(xi) SEQUENCE DESCRIPTION: SEQ ID NO: 90:

AAAACATGCT TTCAGGGCGT CCCCTATGTA TTCGGGGGGC CCACGGACAC TCAGGCTGGA 60

KATCCGTCCT CACTGCGCTC AAGATGGCCT CAGCAGACAC CAGTTACCCA GCTGAAAGTC 120

ACAATCCCTC CCAGAAGTCT CCCAACACTA GTGCTGACCA GAGGTGGGGC TCTCAGGCTA 180

GGAGTTTCAC ACACAATGAC AGGCTGCTGG GGGACATTGC AGGACCCCTT TTCCTYTCCT 240

CTCCATGCTA GAAGCCAGCC CTAGGMAGCT GCAGTTACTC CCTGTGACTC AGCAGCAGGC 300

TGATTCAACA CAGCTGCCCA CACAAAGCCA GTGGTAATAC ATCTGTTTAC CTTTCCCTAT 360

CACCCAGACA CAAGCCCCTT TCCCAGGTCA AACCACAGGC CGATGCATCT CCAGTTTGAC 420

AGTCAAATCA CTACTTCCAT TGCTACTTTA GATCAGCCAA AGTGGTGACT GCTGCAGTGT 480

GTGGCTATCC CTACAAGGCC CACCCAAGGG ATGCCCAAAG CCCAACCTTC TCCAGGGCTG 540

CAGCAGNAGC AACCCCACCA GCCTAAGTCC AGCAGAGGAC CTCCCACCCA ATGTCTTGTT 600

CTAATTAGAA GGGGAAGTTA GCCACAGAAA ATCAACTTAT CTATAATTAC AAAATTCTCT 660

TGACTCACCT TAAAGTTCCT ATTGACATCT ACTGCTTTTA AACCTATTTG AAAACTCTGA 720

TACTAAAACA AATGACACTC TAAGAAAGTT TGGGAGCCCC ATGCTGAGAA CCATTTCTGT 780

GCAGTGAGGA TGTTTCCAGA AGCTACTTAC CTACATGTGA ATGTGCCATT TTCTTTCCTT 840

TTGTAGAGAA AATCCCCTTT ACTTTTTGGA ACAGTAATGG CAGCTTCTAG TACAGCCATT 900

ACAGTTTCAT ATGAGAAAAA TTAAGAATAA CTATAAAATT GTTAAAATAT CCAATAATGG 960

ATAATGATGG CCAGAAGATT TAACATACAA AGTAATTCTC AATGTAAAGC TATTCAGCTC 1020

TTCCAGGTTG AATGCCCTGT AACCCACCCT GACCTTCCAC ATCATCTTCA AAAAGCAGTT 1080

TCTCTGTTCC CCATGATTCT CCTATAAGGT AACTCTTTAG TCCTCCATTT AGCACATTTT 1140

AAATCCTCCA AAGAATAAGT ATCATGTGAT TATTTTAGCT TTACAAAAAA AAAGTTGAAT 1200

GGCGTTTTAT TTTCATGGCC TATAAGCAGG TACCTTAGTA GGGCAGATAT AGGAAAAACA 1260

AATTAGAGCA AAACAAATCC TCTACAAATC CAAGGCAGGA AAAGTGGTGG CAGAGTGACT 1320

CATTCTCCTG TCCCTCCCAT CAGGTCAAAT CAGGAGGCTG CAGTGAATGC CTGTTCTTTG 1380

AATGTGTAGC AGTTGTTCCT GTAACTCTTT AAAACTTGGC TATAGGCTGT TTAGCACAGT 1440

ACAGATTAAA GATACAGTTA CGTAAACAGC AAAGTAATTT TATAGTGCTT CATCCATTTA 1500

TCATGCTTTG GTTTGCTAAT TTTTTCACAT ACCTTTTTCT ATCACAGTCT GTTGCTTTTG 1560

TACACATTTC TCATATTGGG GTTCGACAGG TAAACACAAA CTGCTATTTC AGTAGAAAAA 1620

GTTATTGTTA TGGAATATTA AACCCAATAA ATTGTATAAA GGGTAAAAAA AAAAAAAAAA 1680

AAAAAAAAAA AAAAAAAAAA AAAAAAATTC CTGCGGGCCG CANGCTTTTT CCCTTTGGGT 1740

GAGGGGTTAT TTTNGGCTTG GGCACTGGGC CCTTCGTTTT TACAACGTCG TGANGGGGGG 1800

AACCCGGGGG GGGTTTCCCC C 1821

(2) INFORMATION FOR SEQ ID NO: 91:

(i) SEQUENCE CHARACTERISTICS:
(A) LENGTH: 862 base pairs
(B) TYPE: nucleic acid
(C) STRANDEDNESS: double
(D) TOPOLOGY: linear

(xi) SEQUENCE DESCRIPTION: SEQ ID NO: 91:

TGCCCTTTTT CCCACCGATT CGGGGCNTGG TGAAGGTGGG AGATGTGAAC TCCAATTAAG 60

GGACTGGAGA GAGGTGAAGA ATTTTGCAGG TGGGAGATTT GGATTTGAAT GTGGACTTGT 120

AAATGACTTG ACCTTGCCAT CTGTGTTCAA GGTCACGGTT TGCTGTGGGG TTCCTGGGAG 180

AGCTTACTCA CCCCGGAGTC TTTTCTTTCT CTTGCTCCAA GAAGAGCCCT GTTGGTGCTT 240

TACCACCGCT TGGAGTCTCC CGAGGACACA AACAGGCAGA GAGGGACGTG TAGGGAGAGT 300

TCTTTCCTGT TTTCTGTGCT TTCCTTTTTA CAGGACTCCC GGAAGGCCAC TCATGGCCAT 360

GCCAGGAGCT TTCTCAGAAA CAGTCATAAA CGATCTCTTG AGTCTCTTTC TTGTCCTCCC 420

AGCTGAGCTT TCTTATTCCA CCCTTTCTGG TGTCTATAGG AATGCATGAG AAGACCCTGG 480

GACGTTTTTC TGCTCTCTTC TGGCCCTCCA TGGAGCCATG GGCCTCGGCC TCGGCGGCTC 540

CTCACCCTCA CAATTTATTT CCTCCTCCCG TGCCAGCCCT TCTTTTGTGT CTGAAACCGG 600

TTTTAAAATG TGACTCTCCC AGAGAAGAAG CCGCTGGCTG TATGAAACTT GACGGCGCTT 660

TTGTAAGGTG CCACCCCCAA ACTTTAAGGT AGCTAAACCA ATTTTTAAAA GATTCAATGG 720

CTTGTTCATC CTCCAGATGT AGCTATTGAT GTACACTTCG CAACGGAGTG TCTGAAATTG 780

TGGTGGTCCT GATTTATAGG ATTTCATAAT TAAAATGTCT GCTGAATAAA AAAAAAAAAA 840

AAAAACTCGA GGGGGGCCCG GT 862

(2) INFORMATION FOR SEQ ID NO: 92:

(i) SEQUENCE CHARACTERISTICS:
(A) LENGTH: 696 base pairs
(B) TYPE: nucleic acid
(C) STRANDEDNESS: double
(D) TOPOLOGY: linear

(xi) SEQUENCE DESCRIPTION: SEQ ID NO: 92:

CTGAGGCGAG TGAAGTGGAC TCTGAGGGCT ACCGCTACCG CCACTGCTGC GGCAGGGGCG 60

TGGAGGGCAG AGGGCCGCGG AGGCCGCAGT TGCAAACATG GCTCAGAGCA GAGACGGCGG 120

AAACCCGTTC GCCGAGCCCA GCGAGCTTGA CAACCCCTTT CAGGACCCAG CTGTGATCCA 180

GCACCGACCC AGCCGGCAGT ATGCCACGCT TGACGTCTAC AACCCTTTTG AGACCCGGGA 240

GCCACCACCA GCCTATGAGC CTCCAGCCCC TGCCCCATTG CCTCCACCCT CAGCTCCCTC 300

CTTGCAGCCC TCGAGAAAGC TCAGCCCCAC AGAACCTAAG AACTATGGCT CATACAGCAC 360

TCAGGCCTCA GCTGCAGCAG CCACAGCTGA GCTGCTGAAG AAACAGGAGG AGCTCAACCG 420

GAAGGCAGAG GAGTTGGACC GAAGGAGCGA GAGCTGCAGC ATGCTGCCCT GGGRGGCACA 480

GCTACTCGAC AGAACAATTG GCCCCCTCTA CCTTCTTTTT GTCCAGTTCA GCCCTGCTTT 540

TTCCAGGACA TCTCCATGGA GATCCCCCAA GAATTTCAGA AGACTGTATC CACCATGTAC 600

TACCTCTGGA TGTGCAGCAC GSTGGNTCTT CTCCTGAAYT TCMTCGSCTG CCTGGCCAGT 660

TCTGTGTGGA AACCAACAAT GGCGAGGCTT TGGGTT 696

(2) INFORMATION FOR SEQ ID NO: 93:

(i) SEQUENCE CHARACTERISTICS:
(A) LENGTH: 1886 base pairs
(B) TYPE: nucleic acid
(C) STRANDEDNESS: double
(D) TOPOLOGY: linear

(xi) SEQUENCE DESCRIPTION: SEQ ID NO: 93:

CAGGCCACTG ACGCTTCTTT GCGAGGGATG CAGGAGGTCC TACAGAGAAA GGCGCTTCTT 60

GCATKTCAGA GGGCCCACAG CCTGTCACCC ACAGATCACC AAGCAGCTTT CTACCTGGCT 120

CTGCAGCTTG CCATCTCCAG ACAGATCCCA GAGGCTCTGG GGTATGTCCG CCAAGCTCTT 180

CAGCTTCAAG GTGACGATGC CAACTCCCTG CACCTCCTTG CCCTCCTGCT GTCAGCACAG 240

AAGCATTACC ATGACGCTCT GAACATCATC GACATGGCCC TGAGTGAATA CCCAGAAAAT 300

TTCATACTAC TGTTTTCCAA AGTGAAGTTG CAGTCACTCT GCCGAGGCCC GGACGARGCA 360

CTGCTGACTT GTAAGCACAT GCTGCAGATA TGGAAATCCT GCTACAACCT CACCAACCCC 420

AGTGATTCTG GACGTGGGAG CAGCCTCTTA GATAGAACCA TTGCTGACAG ACGACAGCTT 480

AATACAATTA CTTTGCCAGA CTTCAGCGAT CCCGAGACAG GCTCCGTCCA TGCCACATCG 540

GTAGCAGCCT CAAGAGTGGA GCAGGCACTG TCGGAAGTGG CTTCGTCTCT GCAGAGCATG 600

CCCCTAAGCA GGGCCCGCTG CACCCCTGGA TGACGCTGGC ACAGATCTGG CTCCATGCAG 660

CTGAAGTCTA TATCGGCATC GGGAAGCCTG CAGAAGCCAC AGCCTGTACC CAAGAAGCTG 720

CCAACCTCTT CCCAATGTCC CACAATGTCC TCTACATGCG CGGCCAGATT GCTGAGCTCC 780

GGGGAAGCAT GGACGAGGCG CGGCGGTGGT ATGAAGAGGC CTTAGCCANT CAGCCCCACC 840

CACGTGAAGA GCATGCAGCG ACTTGGCCCT GATCCTTCAC CAGYTAGGCC GYTACAGTYT 900

GGCGGAGAAG ATCCTCCGGG ACGCGGTGCA GGTGAACTCG ACAGCCCACG AGGTCTGGAA 960

CGGGCTGGGC GAGGTCCTCC AAGCTCAGGG CAACGATGCG GCGGCTACGG AGTGCTTCCT 1020

GACAGCCTTG GAGCTGGAGG CCAGCAGCCC CGCCGTGCCC TTCACCATCA TCCCCCGCGT 1080

GCTCTGAGCA GGCGCCTGCC AGCCTCACCT GCCGCTCAGC CTNCAGAGGC CCTGCCGGGC 1140

ACCAGGGCTT GTGCCATCGC CCCAAGGGGA TGAATCTGCC GCACTGAGGC CAGGGACGAG 1200

TGTTCAGTGG GCCACAGTGA ACCAACCAAA CCAACCCCGA ATCATCGCTC TCGCCATGTG 1260

CGTTTCTCTT GTTTTTTTTG CCAGCCCAAT GGTAGTTTCT GAACCTATTG ACATTGTTCA 1320

AAATGGATCA TGTGCCATAT TTTGTTAGTT GACATCTGAG TTTTCAGTAA AATGATTATG 1380

GAATTAATCA GCAAATGTAG AAGAATATAT TCAAAGTTAA AATTCAGTGG CAGCACAGAT 1440

TATTTTTATC AGAGCTGTAA AGAAAACAAC TGTCCTTTTC TCCCCACCAC CCCTCCTGCC 1500

CCACTTTGGC CCAGAAACCA AATGTGAACT TCCTGTCTCC CACCTCAGCA CTAGTCCATG 1560

CCAGGACACC AGCTGACAAT TTCTTGGTTT TACTGTCAAT AATTGTACCA TGTGATCAAT 1620

TACTGTCCTC ACTTAGAACA AAGCCTGAGT CCGAGAATAT TTATATTTTA CCAATATATG 1680

CCTGTTACAA GAGAAGGAAA TATGAGTTAT TTAAGTTTAA CTTTTTTATG TGAATTCAGA 1740

GTTTATTTAT CGAGGGAAAT ATGTACAAAG AAGCTTCAAA TGGAATATTT ACCGACATTC 1800

CTTATACATG ACAGACACTT GGCTACATGG GAAGATGATG TTAATAATAA AATGATTTTT 1860

AAATGGAAAA AAAAAAAAAA AAAAAN 1886

(2) INFORMATION FOR SEQ ID NO: 94:

(i) SEQUENCE CHARACTERISTICS:
(A) LENGTH: 1774 base pairs
(B) TYPE: nucleic acid
(C) STRANDEDNESS: double
(D) TOPOLOGY: linear

(xi) SEQUENCE DESCRIPTION: SEQ ID NO: 94:

CTCAGCTACC GTATACAGTA GGACATAACC CCATTTCACA TGCACTACAC TGAGACTTGC 60

CTCCTCTCCC CCCACATTGA AGATGTTCTT TTTTCATAAC TATATACTAT TCCATTGCAT 120

GAATATTCTG TAATTTATTT AATCCCCTAT GGATTGATAA TTAGGTTCAT TATAGATAGA 180

AGTGTAATTA ACATTCCTGT ACATGTATTT TGCTACTTGT GTGGGTATTT CTGTAGGATG 240

AATAACTAGA AATTTATTGG ATCAGGTTTC ACATTTGCAG TTTTGAAAAC TACTACCAAA 300

AAGATTTCAC CAATTTACAA CTCCATCATT AGTAAGAATG CCTGTTTGCC TATAGTCTGC 360

CAACCCTGAA TCCTTAAAAA TTTTTGCCAA TCTGGTAGGC AAAATTTCTT TCTTTTCTTT 420

GAATATTAAT GAGGAGGAAC ATCTTTTCAT GTTTCTTGGC CATTTGCATT TCCTATTATG 480

AATTGCTTTT GCCCATTTTC CTTTTTTTAA TTATGAAAGT CTAATGACTA CCTTCTCATT 540

GTATAAAAAA CACAGTTCTT TGAATAGAGA GACCCTTTTC TCCAATGCTA CCAATCACAT 600

TCCACTTACC ACAGTTTAAC ATACATCCTC TAGTCACCTT TCCGTACGAA TATACATACA 660

CATAAAAACA CTTTTTACAT AAATAGGATC TCATATTCTG TAGCTTTTTA AAATTTTGGT 720

CTCAAAAAAA GATAACAGGT CTTTAAATTT CTTTAATGGT TGAATATGAT TAAATACTAT 780

GAAAATGCCA TTATTTATTC CCTTAATTTT TTTCCTCTCG CTATTACATT GCCAAAGTAA 840

ACATCCTATT CAGATGTCTT TGTGCATGTG TGTGAATATT TCTTTAGTCT GGAGTCCAGT 900

AAGGTGGATT TTTGGATCAA AGGGTTTGTT CTCTGTCCAC CTTCAGTCTT CCCAAAGGCC 960

TTCATAACTG TATTTTCACC AAGTGTATGG AGAATGTTCA TTTCCCCATA TAACCATACC 1020

TACACTTGAT AGTTTTTATC TGTTGGGCGA AAAAGAACCT TTTCTTATTT TGCATTTCCC 1080

TGATTATAAA AAAAAATGGT GAGATTGGGG TTATTTTCAT GTTTATTGGC CATTTATAGT 1140

TTACTGTGGA TTGTTTGTAT CCCTTACCTG CTTTCTATTG GGTTATGTGT GGATATATTG 1200

TTTTTATTTG TTCAGCATCT CCTTCCCCAT CTTCTGGTAA CACAACCTTT ATTTATTTGT 1260

GGGGAACCTA TTCCCTGTGG CTTAGGTGAG CATGTGACCA GGCCTGGCCT CCTGAGTCCC 1320

ACAGCTTCCT AGCCACAGTG ATAAAAGAAT GGGTATATAA CTTAAGCCAG GCTAAGGAAA 1380

GCCCTTAACA GAACTTCTGC TGGAACTACT GGAAAGAAGG CTTTATGGAG ATCCCAGGAA 1440

CCAAGGACCA TGTAAGCCTG AATTTGTGCC ATGTGGAGAG AGTCTGTCTG AGGAGAAACT 1500

CGGATGCTAG CAGAAATGGA AAGAGAACTA AGTTCTGATG TCATTTTTCT GGAGGCCCTA 1560

GATCCAGCTG TGCCTAAAGC CTGCCCTACT CCGGACTTTA AAGTTTTGTG AGCCAATAAA 1620

GTCCCTTTCT TGTTTAAGAT AATTGAATTG AGTTTCTGTT CTGATTAATA TAGGTTATTT 1680

GTATTTTCTT ATTGATTTGT AGAAAACCTT TGTAATTTTA AATTCTAGAC TTTATGCACT 1740

ATATAAGTTA ATAAAATTAG CATGGCCTTC CATG 1774

(2) INFORMATION FOR SEQ ID NO: 95:

(i) SEQUENCE CHARACTERISTICS:
(A) LENGTH: 2503 base pairs
(B) TYPE: nucleic acid
(C) STRANDEDNESS: double
(D) TOPOLOGY: linear

(xi) SEQUENCE DESCRIPTION: SEQ ID NO: 95:

GGCACGAGCG AAGGCAAGGG GGCACCAGCT CAGGACTGCA TCTGCCTGCC ATTTCCCTTC 60

CACTCCTCCT TTCTGGAGTC TGACATTAGA AAGCCAGCGA GAAGGAAGAT TCAAACAACC 120

AACCCTGATT TCCTGCTTCT CCTTTTCATG AGTGTTCCTG TGGTCTCTGC ACCTCCTTTC 180

TGTCCCCCGG CAGAGGGCAG TAGAGATGGC CGGCCCAAGG CCTCRGTGGC GCGACCAGCT 240

GCTGTTCATG AGCATCATAG TCCTCGTGAT TGTGGTCATC TGCCTGATGT TATACGCTCT 300

TCTCTGGGAG GCTGGCAACC TCACTGACCT GCCCAACCTG AGAATCGGCT TCTATAACTT 360

CTGCCTGTGG AATGAGGACA CCAGCACCCT ACAGTGTCAC CAGTTCCCTG AGCTGGAAGC 420

CCTGGGGGTG CCTCGGGTTG GCCTGGGCCT GGCCAGGCTT GGCGTGTACG GGTCCCTGGT 480

CCTCACCCTC TTTGCCCCCC AGCCTCTCCT CCTAGCCCAG TGCAACAKTG ATGAGAGAGC 540

GTGGCGSCTG GCAGTGGGCT TCCTGGCTGT KTCCTCTGTG CTGCTGGCAG GCGGCCTGGG 600

CCTCTTCCTC TCCTATGTGT GGAATGGGTC ARGCTCTCCC TCCCGGGGCC TGGGTTTCTA 660

GCTCTGGGCA GCGCCCAGSC CTTACTCATC CTCTTGCTTA TAGCCATGGC TGTGTTCCCT 720

CTGAGGGCTG AGAGGGCTGA GAGCAAGCTT GAGAGCTGCT AAAGGCTTAC GTGATTGCAA 780

GGGTTCAGTT CCAACCATGG TCAGAGGTGG CACATCTGCT CAGCCATCTC ATTTTACAGC 840

TAACGCTGAT CTCCAGCTCC AGCGATGGAA CCCACTACAG AGGAGGTGGG GCCCCTGTGT 900

CAAAGAGGCC GAGGGGCAGC AAGGGCAGMC AGGGCACCTG TGACTTCTTA GTACAAGATT 960

GTCTGTCCTT CAGGACTTCC AAGGCTCCCA AAGACTCCCT AAACCATGCA GCTCATTGTC 1020

ACACCAATTC CTGCTTTAAT TAATGGATCT GAGCAAATCT TCCTCTAGCT TCAGGAGGGT 1080

GGGGAGGGAG TGATTGCTGT CATGGGGCCA GACTTCCAGG CTGATTTGCC AAATGCCAAA 1140

ATGAAACCTA GCAAAGAACT TACGGCAACA AACGAGGACA TTAAAAGAGC GAGCACCTCA 1200

GTGTCTCTGG GGACATGGTT AAGGAGCTTC CACTCAGCCC ACCATAGTGA GTGGGCCGCC 1260

ATAAGCCATC ACTGGAACTC CAACCCCAGA GGTCCAGGAG TGATCTCTGA GTGACTCAAC 1320

AAAGACAGGA CACATGGGGT ACAAAGACAA GGCTTGACTG CTTCAAAGCT TCCCTGGACC 1380

TGAAGCCAGA CAGGGCAGAG GCGTCCGCTG ACAAATCACT CCCATGATGA GACCCTGGAG 1440

GACTCCAAAT CCTCGCTGTG AACAGGACTG GACGGTTGCG CACAAACAAA CGCTGCCACC 1500

CTCCACTTCC CAACCCAGAA CTTGGAAAGA CATTAGCACA ACTTACGCAT TGGGGAATTG 1560

TGTGTATTTT CTAGCACTTG TGTATTGGAA AACCTGTATG GCAGTGATTT ATTCATATAT 1620

TCCTGTCCAA AGCCACACTG AAAACAGAGG CAGAGACATG TACTCTGGTG TGATCTCTTG 1680

TCCTCAGTGT CTCTTCTGGG CTCCTGTCCC TCTTGCTTTA TAGCTAGCTG CCCGGGGACC 1740

AAGGTACAGG TGAAAGCAAG GTAGCAGCTT GCGGGAGGAG GCCTGTCTGG CTTACCAGTC 1800

TATACACTGT GGCCTCAACC TCCCAGACAG GGCAGAGAAC TGTGGGCAGC TCGTTTGCTT 1860

TCTAGGCTGG CTGGAGAGGT GGGAGCTCAT TGATAGACTC ATGATGGAAA CTATTTTTGA 1920

AACAGGCTTC CTCCTTCAGG AGAGATCATG CGGACTAAAC TGTAGCAATT CCAGTGCACC 1980

TGGCAGTGAT CCTTTTCTTT GCAAAGTACT GTCTCTTTGG TTCCAGTAAG TTGGACCACC 2040

ACATGACATY ATTTTCCCTG GAACCTGGTC ACTGACTAAC ACAGACAATT GGGACTCCAG 2100

AGCCTCAAGA GCCAGGAGAG GGCACAGTAC ATACAGAGGG AGTCAAATGG GATCTCATTT 2160

TGAGTCCTGC CTTCCGCACA CTCAGAACGG CANCCCCAAG GCCCGGAGTG TCCAGGGCTT 2220

CTGGCCTGAG GTGAATCTGC CAGGCCCAAG AAGGCACAAA GGTAGGAGCA CAGAGAGCCC 2280

CATTCCCACA GGCGGKCGGC CCAGCAGCAC CAGTGGAAGC TCAGCTGTCC TCCAGCTGCT 2340

CTCGGCAGAC AGTTCAGTGC ACAGTTTATG CCCTAGCTGA AAAAGATCTC CCGGACGTAT 2400

TTCAGCACAT CCTCTTCCTC CTCCTCCTCA GGGCTCCTGC TACAGGCAGA GCTGGAACCC 2460

CCCGGCCTCT GGGAAGGGCT GAGGCCTGGA GYCAGTGCCT GTC 2503

(2) INFORMATION FOR SEQ ID NO: 96:

(i) SEQUENCE CHARACTERISTICS:
(A) LENGTH: 2801 base pairs
(B) TYPE: nucleic acid
(C) STRANDEDNESS: double
(D) TOPOLOGY: linear

(xi) SEQUENCE DESCRIPTION: SEQ ID NO: 96:

CTGGAAAGCC GAGGGTAGCC GAGCGGGGCG GGCGCTCTGG AGCGGCGGGT GCTCGGGCTG 60

CCGTCCGCTC CGCCAGAAGC ACCGAGCAGC CGAGCCGGGG CCCGCCGCCC TCCTCCTCCA 120

TGAGGCCCGA GTGAGGCGCG GCGGCTATAG CCGACCCGCG GCGCCTTCCC CCCGCGTCCT 180

ATCGCGAGCG CACGACMAGC GGCCCCTGGA GGAGGAGGCG GAGGAGGAGG AGCATGTCGG 240

ACGGTTTCGA TCGGGCCCCA GGTGCTGGTC GGGGCCGGAR CCGGGGCCTG GGCCGCGGAG 300

GGGGCGGGCC TRAGGGCGGC GGTTTYCCGA AMGGARCGGR GCCTGCTGAG CGGRCGCGGC 360

ACCAGCCGCC GCAACCCAAA GCCCCGGGCT TYCTGCARCC AMCGCCGCTG CGCCARCCCA 420

GGACGACCCC GCCGCCAGGG GCCCAGTGCG AGGTCCCCGC CAGCCCCCAG CGGCCTTCCC 480

GGCCCGGGGC GCTCCCAGAG CAAACGAGGC CCCTGAGAGC TCCACCTAGT TCACAGGATA 540

AAATCCCACA GCAGAACTCG GAGTCAGCAA TGGCTAAGCC CCAGGTGGTT GTAGCTCCTG 600

TATTAATGTC TAAGCTGTCT GTGAATGCCC CTGAATTTTA CCCTTCAGGT TATTCTTCCA 660

GTTACACAGA ATCCTATGAG GATGGTTGTG AGGATTATCC TACTCTATCA GAATATGTTC 720

AGGATTTTTT GAATCATCTT ACAGAGCAGC CTGGCAGTTT TGAAACTGAA ATTGAACAGT 780

TTGCAGAGAC CCTGAATGGT TGTGTTACAA CAGATGATGC TTTGCAAGAA CTTGTGGAAC 840

TCATCTATCA ACAGGCCACA TCTATCCCAA ATTTCTCTTA TATGGGAGCT CGCCTGTGTA 900

ATTACCTGTC CCATCATCTG ACAATTAGCC CACAGAGTGG CAACTTCCGC CAATTGCTAC 960

TTCAAAGATG TCGGACTGAA TATGAAGTTA AAGATCAAGC TGCAAAAGGG GATGAAGTTA 1020

CTCGAAAACG ATTTCATGCA TTTGTACTCT TTCTGGGAGA ACTTTATCTT AACCTGGAGA 1080

TCAAGGGAAC AAATGGACAG GTTACAAGAG CAGATATTCT TCAGGTTGGT CTTCGAGAAT 1140

TGCTGAATGC CCTGTTTTCT AATCCTATGG ATGACAATTT AATTTGTGCA GTAAAATTGT 1200

TAAAGTTGAC AGGATCAGTT TTGGAAGATG CTTGGAAGGA AAAAGGAAAG ATGGATATGG 1260

AAGAAATTAT TCAGAGAATT GAAAACGTTG TCCTAGATGC AAACTGCAGT AGAGATGTAA 1320

AACAGATGCT CTTGAAGCTT GTAGAACTCC GGTCAAGTAA CTGGGGCAGA GTCCATGCAA 1380

CTTCAACATA TAGAGAAGCA ACACCAGAAA ATGATCCTAA CTACTTTATG AATGAACCAA 1440

CATTTTATAC ATCTGATGGT GTTCCTTTCA CTGCAGCTGA TCCAGATTAC CAAGAGAAAT 1500

ACCAAGAATT ACTTGAAAGA GAGGACTTTT TTCCAGATTA TGAAGAAAAT GGAACAGATT 1560

TATCCGGGGC TGGTGATCCA TACTTGGATG ATATTGATGA TGAGATGGAC CCAGAGATAG 1620

AAGAAGCTTA TGAAAAGTTT TGTTTGGAAT CAGAGCGTAA GCGAAAACAG TAAAGTTAAA 1680

TTTCAGCATA TCAGTTTTAT AAAGCAGTTT AGGTATGGTG ATTTAGCAGA ACACAAGAGA 1740

GCAAGAAAAT GTGTCACATC TATACCAAAT TRAGGATGTT GAGTTATGTT ACTAATGTAT 1800

GCAACTTTAA TTTTGTTTAA CACTATCTGC CAAAATAAAC TTTATTCCCT ATAACTTAAA 1860

ATGTGTATAT ATATATAATA GTTTATTATG TACAGTTAAT TCTACTGTTT TGGCTGCAAT 1920

AAAATCGATT TTGAAATAAA TGAAATGTTG AAAATTTTGC TAGTTGGTTA GATGCTTATC 1980

CTTTAAATTC TACTTTTCTT GAGGGGAAAA AGTCTTCGTC TGGAAATACA TATTACTGCA 2040

AAAATGTAGC ATCCTTTTTT AGGTAGGAGT ATTATAGCTT YCATTTTAGT TKGACATTTA 2100

GTGTCCCAAT GAATTGAATT TCAAATATGA ATCATAATCT TGAAAATCTT TAGCACTAAA 2160

GTCTTGGGAA TATATCAACA ACTGATTTAC ATATGCAGAT GCTATTTGNA TACCAAGGGC 2220

TTTTTAAATG TCATGGGGGG GAAAAACCCA ACTTGGTGGA ACTCCCAGCT AAACAACCAA 2280

GACTTCACTG GAAGATTTAT TCCAATTCTA GGAATTGTTC TTTTTTATTT TTATTTTTTC 2340

AACTGRCTAA CTTCATTACC TTAAAGCCTA GAACATTATT CTGCTTTATT TATATGGCTT 2400

TCTCACTTTT ATTTTGTAGC AKGGGTTGCA TCGACTTTTT TACTAGAGAA TTTTACTAGA 2460

TATTTGTCAT TCAAGTTTTC ATCTGCTTTA TAATTGATAC ACCTTGAGGG TCACTTTTCT 2520

AATACTTTTA CTATAATGTG GTACCACCTC AGCCCTAATA AATAATATTT TTACCTAATG 2580

TCAAATCTTT TTCCAGCTAA CTAAAAACTG TGTACAAAAG GATTGCTTGT AAATATGCAT 2640

GTAAATAGTT CTGTTAATAA CCCACTGTTT TACATTTGGT ACATCTGTGT CTGCTAATAC 2700

AGTTAGCTTT CTCACTTTTC TGCTTGTTTG TTCAGTCTGA ATTAAAATTA GACTTTGAAA 2760

ATAAAGCTTA AAAAAAAAAA AAAAAAAAAA AAAAACTCGA G 2801

(2) INFORMATION FOR SEQ ID NO: 97:

(i) SEQUENCE CHARACTERISTICS:
(A) LENGTH: 1631 base pairs
(B) TYPE: nucleic acid
(C) STRANDEDNESS: double
(D) TOPOLOGY: linear

(xi) SEQUENCE DESCRIPTION: SEQ ID NO: 97:

ATGGAGCCAA AGACAATCAC TGATGCTTTG GCTTCTAGTA TAATTAAGAG TGTGCTGCCT 60

AATTTTCTTC CATACAATGT CATGCTCTAC AGTGATGCTC CAGTGAGTGA ACTGTCCCTC 120

GAGCTGCTTC TGCTTCAGGT TGTCTTGCCA GCATTACTCG AACAGGGACA CACGAGGCAG 180

TGGCTGAAGG GGCTGGTGCG AGCGTGGACT GTGACCGCCG GATACTTGCT GGATCTTCAT 240

TCTTATTTAT TGGGAGACCA GGAAGAAAAT GAAAACAGTG CAAATCAACA AGTTAACAAT 300

AATCAGCATG CTCGAAATAA CAACGCTATT CCTGTGGTGG GAGAAGGCCT TCATGCAGCC 360

CACCAAGCCA TACTCCAGCA GGGAGGGCCT GTTGGYTTTC AGCYTTACCG CCGACCTTTA 420

AATTTTCCAC TCAGGATATT TCTGTTGATT GTCTTCATGT GTATAACATT ACTGATTGCC 480

AGCCTCATCT GCCTTACTTT ACCAGTATTT GCTGGCCGTT GGTTAATGTC GTTTTGGACG 540

GGGACTGCCA AAATCCATGA GCTCTACACA GCTGCTTGTG GTCTCTATGT TTGCTGGCTA 600

ACCATAAGGG CTGTGACGGT GATGGTGGCA TGGATGCCTC AGGGACGCAG AGTGATCTTC 660

CAGAAGGTTA AAGAGTGGTC TCTCATGATC ATGAAGACTT TGATAGTTGC GGTGCTGTTG 720

GCTGGAGTTG TCCCTCTCCT TCTGGGGCTC CTGTTTGAGC TGGTCATTGT GGCTCCCCTG 780

AGGGTTCCCT TGGATCAGAC TCCTCTTTTT TATCCATGGC AGGACTGGGC ACTTGGAGTC 840

CTGCATGCCA AAATCATTGC AGCTATAACA TTGATGGGTC CTCAGTGGTG GTTGAAAACT 900

GTAATTGAAC AGGTTTACGC AAATGGCATC CGGAACATTG ACCTTCACTA TATTGTTCGT 960

AAACTGGCAG CTCCCGTGAT CTCTGTGCTG TTGCTTTCCC TGTGTGTACC TTATGTCATA 1020

GCTTCTGGTG TTGTTCCTTT ACTAGGTGTT ACTGCGGAAA TGCAAAACTT AGTCCATCGG 1080

CGGATTTATC CATTTTTACT GATGGTCGTG GTATTGATGG CAATTTTGTC CTTCCAAGTC 1140

CGCCAGTTTA AGCGCCTTTA TGAACATATT AAAAATGACA AGTACCTTGT GGGTCAACGA 1200

CTCGTGAACT ACGAACGGAA ATCTGGCAAA CAAGGCTCAT CTCCACCACC TCCACAGTCA 1260

TCCCAAGAAT AAAGTAGTTG TCTCAACAAC TTGACCTTCC CCTTTACATG TCCTTTTTTG 1320

TGGACTTCTC TCTTTGGAGA TTTTTCCCAG TGATCTCTCA GCGTTGTTTT TAAGTTAAAT 1380

GTATTTGACT TGTGTTCTCA GCATTCAGAG AGCAGCGGTG TAAGATTCTG CTGTTCTCCC 1440

TGGATCTTCT GACATTACTG CTGTCTGAGA TTTGTATATG TGTAAATACA AGTTCCTTGA 1500

TACCCTAAAA CCTTGGATTA AACAGAATGT GCATTGTACA TCTTTAAACA AAATGTATAT 1560

TAATTTATTA AATCTAGTTG TCACTTTAAA AAAAAAAAAA AAAAAACTCG AGGGGGGCCC 1620

GGTACCCAAA T 1631

(2) INFORMATION FOR SEQ ID NO: 98:

(i) SEQUENCE CHARACTERISTICS:
(A) LENGTH: 504 base pairs
(B) TYPE: nucleic acid
(C) STRANDEDNESS: double
(D) TOPOLOGY: linear

(xi) SEQUENCE DESCRIPTION: SEQ ID NO: 98:

CCGAGCTGGG CGAGAAGTAG GGGAGGGCAC GAGCCGCCGC GGTGGCGGTT GCTATCGCTT 60

CGCAGAACCT ACTCAGGCAG CCAGCTGAGA AGAGTTGAGG GAAAGTGCTG CTGCTGGGTC 120

TGCAGACGCG ATGGATAACG TGCAGCCGAA AATAAAACAT CGCCCCTTCT GCTTCAGTGT 180

GAAAGGCCAC GTGAAGATGC TGCGGCTGGA TATTATCAAC TCACTGGTAA CAACAGTATT 240

CATGCTCATC GTATCTGTGT TGGCACTGAT ACCAGAAACC ACAACATTGA CAGTTGGTGG 300

AGGGGTGTTT GCACTTGTGA CAGCAGTATG CTGTCTTGCC GACGGGGCCC TTATTTACCG 360

GAAGCTTCTG TTCAATCCCA GCGGTCCTTA CCAGAAAAAG CCTGTGCATG AAAAAAAAGA 420

AGTTTTGTAA TTTTATATTA CTTTTTAGTT TGATACTAAG TATTAAACAT ATTTCTGTAT 480

TCTTCCAAAA AAAAAAAAAA AAAA 504

(2) INFORMATION FOR SEQ ID NO: 99:

(i) SEQUENCE CHARACTERISTICS:
(A) LENGTH: 1416 base pairs
(B) TYPE: nucleic acid
(C) STRANDEDNESS: double
(D) TOPOLOGY: linear

(xi) SEQUENCE DESCRIPTION: SEQ ID NO: 99:

GGCACGAGGG AGGGAGCCCT CTCCGTTGGG TGACTCTTGT GTGCCCTTTA GACAGGCTGG 60

CCTGCCGGTT CCACAGGGTA CAGTTAGGAC TTGAGTCTTT CTTTTTCTGT TTTGAGTTGG 120

TGAGTGAGTG ATAGGGTAAC ATGGGCCTTC AGGATGACCC CTTGGAACTG TGCCGAGTTC 180

CTTAAATCTC AGCTGGGATC CTGGACCTGG GAGGCCCCTG TGAGGGCCAG CTCTGGAAAA 240

ACCTGGGAGT TGATGCCGGA GCTGTGGAAG AACTCTGCTC GAGGGCAGGG TGCCCTGGAA 300

CACTGGTAGT TCTGGGGCTG GGAGGGAGAG GGGCTCCGGC TTTCTCTGAA ATGAACACTG 360

CTCTTCAGCA GTTCAAGTAC TTGTTCTCAA AACATTTTCT AATTGATTGG TAGGTTTTCA 420

TAAGCATTGT TTCTTTAAGG CATGGAAAGG GAAGAATGCT CAAGCAAGTC ATGTTTGTTT 480

TCAGTGGGAT GGGCCCGCGT TCTCACTGCT GGGGGCTTCC CCTTCATGTG GCACCTTTGT 540

GCAGGGGCCA CCAGGCAGAC TCTTCCCACC TTCTCCCACT GAAGCACCAA GGGGCTTGGA 600

ACCGTAATTT GGCTAATCAG AGGCATTTTT TTTGTCCTAG TATCTTTCAC ACTTGTCCAA 660

CCGTCTTATT TTTTTAAAAG TTCTGTTGCT TGTATTAACA CGAAACTAGA GAGAAATAGT 720

TTCTGAAGCC AGTTTATTGT GAAGATCCCC AAGGGGAGGT TCGGTAGAGA AAAATAGTAA 780

GCTGGTTTAG AAACTGACGA GGGCAAACAG CCAGGACGCA TTGGAGAGGA ATTTGCCAAA 840

GATCTACCCT GAGATAACGC CTGTCCAGTG TCTTCACCAC GTGAATAACC AGCGCTCCAA 900

AGTGTTTTTC TGCTTTGAAA AAAAAAATTC CACAAGCTTT TAAAGGTGCA TTTAAGAATC 960

CATGTGACTT TAGAATGGAA CTGCCGGCCC TGGCAACTGT CACGTGTGCT AGAAGGTTCG 1020

ATGCCTCTGG AATGCATGTG ATACTCATCT CCATTTTGTT TCCTTGATTG CATTTTTGTT 1080

CTTTTAGCAG ATCTGTCCCT GTGGGTGGTG TCTAAGAAGT CGGACACCTT GGTTTTTGTG 1140

TTAGATTGAG CTGGGCAGCT GCAATCAGCT TCTTTATATG CAAATTAGGC ACGACCCATC 1200

TGTGGTTCCT GGTTGGTGGC TAATGAAGTG AGGGGAGGGA GGGATGTCAC CCCAAAAGTA 1260

GGCCCTCCCA TTGGCTTTGG CCAGGCCAGA CACTTCACAT CGTTTACATG GTTCTGTGTA 1320

ATTTTAAAGT TTATGTGTAT AAAGCGAAGC TGTTTCTGTG AAACTGTATA TTTTGTAAAT 1380

AAATATATTG CTACTTGAAA AAAAAAAAAA AAAAAA 1416

(2) INFORMATION FOR SEQ ID NO: 100:

(i) SEQUENCE CHARACTERISTICS:
(A) LENGTH: 2847 base pairs
(B) TYPE: nucleic acid
(C) STRANDEDNESS: double
(D) TOPOLOGY: linear

(xi) SEQUENCE DESCRIPTION: SEQ ID NO: 100:

GGCTAGGACA ATTTTGGTGC TTTACCTATC TCTGCAAAGA CTGGAGAATT TGGCATACCA 60

TTAATTACAA CCACCAATCA TATCCAACAA AAGTACCCTA AAAGAAGGAC CAGTGGCCAC 120

TCTCGAAAAA ATTTAAGTAT CAGAAGATTA AAAAGATTTT AGGATTTGGA AGCTTGTATT 180

GTCTTTCCCC AATAATCATT GTTTGATCTC CAAATAGTAG CCTTATATTA GCAATRGACA 240

GATCATTGGT TCTCCATATC TGATCATATG TTACTACTTT GGAATCAGTA TTTGGGCAAA 300

TTCAAGCATT TATGCAGTGG ATATAAATGG AAATATAAAA ATATTTGCCA ACCTGTCTCA 360

GTAACTTATC ATATCTCTGT GNATCCTCAA GGAAAGCACT TTTGCTTTTA CTTAGAAAGC 420

GTTTCAGATT TGCTTTATAG ACTCCTGCTG TCTTCAGTAC CTGATAAAAC TTTAACCAGG 480

GAAGCATTAA ACACAGTGCA GCAGCTTTTG CCCAGGCTTC TAAGTTCCTG CCGGCAGCAT 540

TTATCAATGT AAGAACTAGG ATGCTTCCTG CAGTGGCACT ACCTTCCCCT AGAGCTGGAG 600

CATGCTGCTT GGCCTTAAGC CCCAGCATGA TGAGGCTTCC CTCCTGCCAG GTCAGTAAAA 660

GTTAGAGAGC TCAGAATTGG GTCTTGCCTG GGTGCAGGTG GCAGGGTTTG CTGAAACCCC 720

TAAAGAGAAG TCACCAAGGG AGGCAGGTAA TGAATGTTTC CAGAATCAGT CKGATACTCA 780

TAGCAATTTC TGGCTATCTT TCAAATGTTG AATTTCTGGA TGCTGAGAGG GACTTTGATT 840

TGATATCATT AAATCCAGGA CAGTCCCAAG AAGTGCTTGG AGTCTCGGCT CTGACAGCCC 900

AAGAAGGGAA ATAACTTGTA TTAAGGAACA ACTATGAGCC AGGCCCTGAG CTGTCTCTTA 960

GATAATAAAA CAGATGGGGA GTGGAAGAGT CATTTGCTTC AAGTTATACA GCTAGGAAAT 1020

ACTCAAGCCA AATCTTGAAC GCAGCTCCCC CTAATTCTGT GGACAGGCAC TTTGTACCAC 1080

ACACCATGGT CCACCTAAAA ACAGAAGGAT AAAAAGACTT CAGGTTTTCC CACTGTGTGC 1140

TGACCATCCC AATTTATGAA TCTTCTTCAA AATGACATTT CACAGTTATA GTTAGGGCTC 1200

AGAAATGGCA TTGAGGTAGC CTTATTTCTC CCCTTTAGCA GATGCTTTAA GTACACATTG 1260

CTGACTTGAG CCCACCCCCA GGAGTTAGGA GAACATTTCC TTTTTCATGC CATCTTCCAT 1320

AAATAAGGTG TTTCTTGGCC TTCAAAGATA TAGAACTTTG CAGCAGTAGT AAAAGTGAAG 1380

GGTGTTCTGC TCTCTACTCA ACTTTATTTG AAAATGTCTG CAGCTTCACT CCTGTAGAAA 1440

AGGAAATCTT CATATTTTAG TAAACTTAGC CGCCAGTGTA CTCTGTGAGG ATGTGGCAAT 1500

TCAAAGTCCA GTGAATCTGG CTCTCTTACT GATTCCTGGT TTTAGTGTGT GTGTCGGGGG 1560

AGTGTGTACC TATATATAAA GGACAAGTGT GATATGTGTG TATATGTATA TACATACATA 1620

CATGTCCACA CACACACACA CAATATTTGA GAGCTAAGGA AAACTCAAAG CAGCCCCTTC 1680

ATTATCTTGC GTACTACTTC AAAGATTTCT GTCAGCCCTA ATTACAAGTG TCACCATATA 1740

GTTGGGGCTT AGGTACTTGC TTACAGGAAG AGCAATTCCC TAGCAAAGGT CATTAGCTCC 1800

TAAGGCACTG AGTCAAAGTG ACAGCCCTGA AGGAAATTGC ACTCCAGCCC TCCTCCAGGA 1860

TGTCTAATAA GATGGGAAAC TTGGATGCCC AGCCATTTTG GTGACCTGAG AGTCTAACTA 1920

CTCCAGTTAG ACCTAAGGGC ACAAATGCAG AATTCATGAC CTTGTAGTTG TGGCAGGGTC 1980

TAGGAAGTCC TCTCTCCCCA AGTAGAAAAT ATTCTCTTGC CATTCCTGAA ATTCCACATT 2040

CATATAATGG CTGTGCAATA CATGCTTCTC AATAAGAAAA TTAACTGCAT GTTTACTGTG 2100

TGCTGATCAC ATCAGATTTT TATGTTTAAA AAAATCTCAT TATGGNTTGA GTCCAGCCCA 2160

GCTCTAAGAG AAAAAGAAGG CCCATATGGG AGACTTCAGT CTCATTATTA TTGCCTTTAT 2220

CCAGCAGTGC TTATRAAGCC CCCTACCCTG TCCCATTCCA GAAACCATAA GACTCAGGCA 2280

GTTCTTGATT CTGGAGGCCT GCCTGGTAAG ATAAGATAGT ATAATTTGGA ACTGAGAACA 2340

TACCAGAAAC AGCAGAACGA GGGCCAGAGC AGAAAAATGA AAATAAGTGG AGACACTTAT 2400

GGATACATTG GTGCAAAAAA AGCCACGGGS CCCATACTGG GCTTGATATG ACTTTGAGGG 2460

GACAGCAGAT TAATACTTAA TGAGGGTTAA ACCTGACCAG TCTTTCTACA GTGACAGGCC 2520

ACACTGCATG AATGGGGAGA ACCAATGAAT CCATTGTCCT CTGCCTATTT TCCTGTGCAC 2580

AGTCACATTC CCTCCTTAGG AATCTTCCCC TTCCACCCTT TACATTAAAC AAGGGAACAC 2640

TGAATCTTTC AAGGGAATTA CACGTTTGGG TTAATGTTTC AGTATATCAT TTTCATACTG 2700

TAAATTATTT TGTAAGAGAG ATTTACTGCT ATCCCAGGAT GTTCGGACTT GGTGCCCCTG 2760

TGCATTTGGA AATCAATAAA CTATTACTGG AAATGCCAAA AAAAAAAAAA AAAAAAAAAN 2820

NAAAAAACTC GAGGGGGGCC CGTACCC 2847

(2) INFORMATION FOR SEQ ID NO: 101:

(i) SEQUENCE CHARACTERISTICS:
(A) LENGTH: 1394 base pairs
(B) TYPE: nucleic acid
(C) STRANDEDNESS: double
(D) TOPOLOGY: linear

(xi) SEQUENCE DESCRIPTION: SEQ ID NO: 101:

GAGATTGGTG GAGGAGAGTA AATAATCTAG AGGCAAGAGT TCAGTGAGGG CCAAGGGGGA 60

CCCCCAGAAA AAGGTATGGA GCTAACTCAT CTCTTTTACA AGGGGTGGCC ATGACTTACT 120

GTTGCAAAGT ACTCAGTGTA TATTTAATGT TGATTGTTGA ATTTTAGTTA CGAGAGGGAA 180

GAACAATTTT ACTTCTGTCC TTATTTCACT TGCTGAAAAG CTGTGGGACA AAATGTATGG 240

AATAGACAAG GCCACTTTCT TTGTGATTTC TGCTTTTCAT GCATATTATT TTATTTACCC 300

ATAATTTCCA AGAGGTTTGG CGTTCCGCTC TCCTGCTTTT TTCTTTCATC CACCCCTTTC 360

CTTTTTTTGG AAGGGGGTTA TATATGAGAG TTCATTGAAG AAGTCCAGTG AGGCTGAAGT 420

AAAGGGGCAA GATAGGGCAG TTAACTAAAG AGCACTTTAT TTCTTTGAAG CCTTTCTAAG 480

AAAGAAATGG GGGTGCGAGT GGCTTGAATC TCCCATGATG TTGGAGGGCA CTTAGTGGGG 540

TTGAAGTATG ACATAATATT TCCCATTGGG GAAAGGAGAA TTTCTCTTAG AGGGTGGCAA 600

AATGCCTTTG CCCAGTGTCC CTATTTTAGG CATCTTTTCC TTCCTTATTC CTTCCAGTCA 660

GGGTGTGTCC TATACAAAAC TTCCCATCAG TTCTCCTCAA TATTCCCCAT TTGTAAATGA 720

TCACTTCTCT TTTCTAAACC CTTTTCCTGT TCAGATCCAT ACAGGATTTG CAAGGGTAGG 780

ATCATACATG CAAATGCCCC TTGTTCATCT GTGTCTTCTG CAAACTAGTC TCATGAAGAA 840

TTCTGGCGTG CAGCAGGGTA GCTGAAGTTT GGGTCTGGGA CTGGAGATTG GCCATTAGGC 900

NTCNCTGAGA TTCCAGCTCC CTTCCACCAA GCCCAGTCTT GCTACGTGGC ACAGGGCAAA 960

CCTGACTCCC TTTGGGCCTC AGTTTCCCCT CCCCTTCATG AAATGAAAAG AATACTACTT 1020

TTTCTTGTTG GTCTAGCATT GCTGGACACA AAGTGTAGTC ATTATTGTTG TATTGGGTGA 1080

TGTGTGCAAA ACTGCAGAAG CTCACTGCCT ATAAGAGGAA ATAAGAGAGA AAGTGGAGGA 1140

GAGGGACAAA AGGAGTAATT ATTTGGTATA GATCCACCCA TCCCAACCTT TCTCTCCTCA 1200

GTCCCTGCTC CTCATGTTTC TGGTTTGGTG AGTCCTTTGT GCCACCACCC ATAATGCTTT 1260

GCATTGCTGC ATCCTGGGAA GGGGGTATAT GGTCTCACAA GTTGTTGTCA TTGTTTTTTT 1320

GCATGCTTTC TTAATAAAAA AAAAAAAAAA ATGTTTANAG TTTTATCTTA AAAAAAAAAA 1380

AAAAAAAAAA ACCC 1394

(2) INFORMATION FOR SEQ ID NO: 102:

(i) SEQUENCE CHARACTERISTICS:
(A) LENGTH: 794 base pairs
(B) TYPE: nucleic acid
(C) STRANDEDNESS: double

(D) TOPOLOGY: linear

(xi) SEQUENCE DESCRIPTION: SEQ ID NO: 102:

GGMRCGAGGC GGAGTAAAGG GACTTGAGCG AGCCAGTTGC CGGATTATTC TATTTCCCCT 60

CCCTCTCTCC CGCCCCGTAT CTCTTTTCAC CCTTCTCCCA CCCTCGCTCG CGTACCATGG 120

CGGAGCGTCG GCGGCCACTC AGTCCCATTC CATCTCCTCG TCGTCCTTCG GAGCCGAGCC 180

GTCCGCGCCC GGCGGCGGCG GGAGCCCAGG AGCCTGCCCC GCCCTGGGGA CGAAGAGCTG 240

CAGCTCCTCC TGTGCGGTGC ACGATCTGAT TTTCTGGAGA GATGTGAAGA AGACTGGGTT 300

TGTCTTTGGA CACGCTGATC ATGCTGCTTT CCCTGGCAGC TTTCAGTGTC ATCARTGTGG 360

GTTTCTTAMC TCATCCTGGC TCTTCTCTCT GTCACCATCA RCTTCAGGAT CTACAAGTCC 420

GTCATCCAAG CTGTWCAGAA RTCAGAARAA GGCCATCCAW TCCAAAGCCT ACCTGGACGT 480

AGACATTACT CTGTCCTCAG AAGCTTTCCA TAATTACATG AATGCTGCCA TGGTGCACAT 540

CAACAGGGCC CTGAAACTCA TTATTCGTCT CTTTCTGGTA GAAGATCTGG TTGACTCCTT 600

GAAGCTGGCT GTCTTCATGT GGCTGATGAC CTATGTTGGT GCTGTTTTTA ACGGAATCAC 660

CCTTCTAATT CTTGCTGAAC TGCTCATTTT CAGTGTCCCG ATTGTCTATG AGAAGTACAA 720

GACCCAGATT GATCACTATG TTGGCATCGC CCGAGATCAG ACCAAGTCAA TTGTTGAAAA 780

GATCCCAAGC AAAA 794

(2) INFORMATION FOR SEQ ID NO: 103:

(i) SEQUENCE CHARACTERISTICS:
(A) LENGTH: 1544 base pairs
(B) TYPE: nucleic acid
(C) STRANDEDNESS: double
(D) TOPOLOGY: linear

(xi) SEQUENCE DESCRIPTION: SEQ ID NO: 103:

TTTGCTTGCT AGTCTGAACC AAAGAGTTGT TTGGGCATTT GCTGTGTTGG CCATTTCTGG 60

AGCAAGAGGG TCTTCTTCCT CCTTCCCCCA GCCAGCCAGC TGTCCTGGGG CCAGGCTTTC 120

CTGGGTGGAA AGAAGTATAC CTTTCCCTGG GGCCCTAGGA TAGCAAAGTG AGCCATAGTG 180

GGCCAGGCTG CCCTCCATGC TGGGCCCCAG CCCAGGTCTG CACTCGCCTG GATCACCTTC 240

TTTGAGCCTT AGCCATCTCC TGTCAGGTAG GAATGAACTT GCCAGCCTTC AGGYTCGTTC 300

AGCTATGACC ATCTGTGCGG TCAGGGTACA CTCAGCTCTC CTCCCCAACT CCAGCAGCCT 360

TTAAGAAGTG TCCCTTTGGC GCCCCCTGGA GGCAGAGCAC TGAGCTGGAC CCTGGGTAGA 420

CTCCCACAGG GAGGACGGAG CTGGCCTCAG GAGTGGGACA CCCAGACTTG GCAGGGCCTT 480

CAAGAGGCCT GTGTGGGGGC CCCAGGAATC CTTAGCTGAA GCGGGGAGAC TCACTCTCCA 540

TCTCAGGAAA TTCTAGCCCT TGCCCTCAGG GAGCCACGGT TGAGGGTGAG GCCCAACACC 600

TGCCTTAGGG CCCTGGGTGG GCAAGTCTGG GCCCTGGGGT AGGGAGGGAG ACTCAGGCCC 660

ACACTTGGGT ATTTTCTAAT TTCAGACAAA CACACACTCA GCGCGCACTC ACTGATTCCT 720

ACACATTGCC AAGATTTCAC ACATGTGACC AGGGGCCACC AAAGTCCCTG TGACCTTTGT 780

GACTAGGATC CTAATTTCTC TATTTTCTCC TGGGTGCCTG GGTCTGTGTC ACCTGGGGCA 840

GTGTGGATAA TGTTTAGTTC TGTGACACTG TTTTTTGGGG GTGGCACCTG GTTCTCCGAT 900

GCCTGGGCTG GTGTCAGGCC CAGGACTGTA GTGCTGGGAG CAGTAAAGCT CAGCTCTGTG 960

TAATGAGTGA TGCTATGGCT TGCTCGTGTC TTATGATCCA ATCCTTTTCT ACATCAGCCC 1020

TTGTTTTGTT TTATGGCTAG TCTTATCTGG CCTGGTTATT TCCTTGCGGG GAGGAGAGGG 1080

TTTGCTAATC TGCTCCCAGC CCAACCTATT ACCACCCCAC CTCGCTGGGA CCTACTGCTC 1140

GGGAGGCAGC AGACAGGGAG CCACCAGCAG TGGCTTCCTG GCCCTGTGCT GGGGGTGGGG 1200

GGAAGCTGGG GGCACATGTG GCCCTTGCCT TCTGAGCAGC TCCCAGTGCC AGGGCTTTGA 1260

GACTTTCCCA CATGATAAAA GAAAAGGGAG GTACAGAAGT TCCAATTCCC TTTTTATTTT 1320

GCTGGTTGGT ATCTGTAAAT GTTTAATAAA TATCTGAGCA TGTATCTATC AACGCCAAGA 1380

ATTTCAAAGT CTCCTTCAAC AATATGAGGC TTTTAGGATG TTTATATTCC TTCATCCCTC 1440

TTGTTTCCCA GGTTTTGCAG GGAAAAAAAG TCTGGAATTA TAGATACAGC TTATTATTAA 1500

ATTTGTTCTT GCATAAAAAA AAAAAAAAAA AACNCNNGGG GGGG 1544

(2) INFORMATION FOR SEQ ID NO: 104:

(i) SEQUENCE CHARACTERISTICS:
(A) LENGTH: 871 base pairs
(B) TYPE: nucleic acid
(C) STRANDEDNESS: double
(D) TOPOLOGY: linear

(xi) SEQUENCE DESCRIPTION: SEQ ID NO: 104:

ACCCACGCGT CCGNCTTGTC CACCCGGGGG CGTGGGAGTG AGGTACCAGA TTCAGCCCAT 60

TTGGCCCCGA CGCCTCTGTT CTCGGAATCC GGGTGCTGCG GATTGAGGTC CCGGTTCCTA 120

AGGTGGGTCG CTGTCCACCC GGGGGCGTGG GAGTGAGGTA CCAGATTCAG CCCATTTGGC 180

CCCGACGCCT CTGTTCTCGG AATCCGGGTG CTGCGGATTG AGGTCCCGGT TCCTAACGGA 240

CTGCAAGATG GAGGAAGGCG GGAACCTAGG AGGCCTGATT AAGATGGTCC ATCTACTGGT 300

CTTGTCAGGT GCCTGGGGCA TGCAAATGTG GGTGACCTTC GTCTCAGGCT TTCCTGCTTT 360

TCCGAAGCCT TCCCCGACAT ACCTTCGGAC TAGTGCAGAG CAAACTCTTC CCCTTCTACT 420

TCCACATCTC CATGGGCTGT GCCTTCATCA ACCTCTGCAT CTTGGCTTCA CAGCATGCTT 480

GGGCTCAGCT CACATTCTGG GAGGCCAGCC AGCTTTACCT GCTGTTCCTG AGCCTTACGC 540

TGGCCACTGT CAACGCCCGC TGGCTGGAAC CCCGCACCAC AGCTGCCATG TGGGCCCTGC 600

AAACCGTGGG AGAAGGAGCG AGGCCTGGGT GGGGAGGTAC CAGGCAGCCA ACAGGTTCCC 660

GATCCTTAAC GCCAGNTGCG AGAGAAGGAC CCCAAGTACA GTGCTCTCCG CCAGAATTTC 720

TTCCGCTACC ATGGGCTGTC CTCTCTTTGC AATCTGGGCT GCGTCCTGAG CAATGGGCTC 780

TGTCTCGCTG GCCTTGCCCT GGAAATAAGG AGCCTCTAGC ATGGGCCCTG CATGCTAATA 840

AATGCTTCTT CAGAAAAAAA AAAAAAAAAA A 871

(2) INFORMATION FOR SEQ ID NO: 105:

(i) SEQUENCE CHARACTERISTICS:
(A) LENGTH: 404 base pairs
(B) TYPE: nucleic acid
(C) STRANDEDNESS: double
(D) TOPOLOGY: linear

(xi) SEQUENCE DESCRIPTION: SEQ ID NO: 105:

GGCACGAGTT ATAGCATGGC ATTCATACTT TTGTTTTATT GCCTCATGAC TTTTTTGAGT 60

TTAGAACAAA ACAGTGCAAC CGTAGAGCCT TCTTCCCATG AAATTTTGCA TCTGCTCCAA 120

AACTGCTTTG AGTTACTCAG AACTTCAACC TCCCAATGCA CTGAAGGCAT TCCTTGTCAA 180

AGATACCAGA ATGGGTTACA CATTTAACCT GGCAAACATT GAAGAACTCT TAATGTTTTC 240

TTTTTAATAA GAATGACGCC CCACTTTGGG GACTAAAATT GTGCTATTGC CGAGAAGCAG 300

TCTAAAATTT ATTTTTTTAA AAAGAGAAAC TGCCCCATTA TTTTGGTGGG GTTGGTTTTT 360

AATTTNTAAT NTGAAAAATT TTTTTGGGGT TTTTGGGGCC ATGG 404

(2) INFORMATION FOR SEQ ID NO: 106:

(i) SEQUENCE CHARACTERISTICS:
(A) LENGTH: 1542 base pairs
(B) TYPE: nucleic acid
(C) STRANDEDNESS: double
(D) TOPOLOGY: linear

(xi) SEQUENCE DESCRIPTION: SEQ ID NO: 106:

GTCAGACAGG TGGAGCCGCC GGGGCAGGAG TCTCAAAGAG CCAGGCTCCA GGAGAGGAAG 60

GGCTCTRCGA GAGGAGAGAG GAGAGCGCTG GAGAGGAGAG GCTGGAGAGT CCTTAGCCAG 120

GATGGAGGCT GTTGTGAACT TGTACCAAGA GGTGATGAAG CACGCAGATC CCCGGATCCA 180

GGGCTACCCT CTGATGGGGT CCCCCTTGCT AATGACCTCC ATTCTCCTGA CCTACGTGTA 240

CTTCGTTCTC TCACTTGGGC CTCGCATCAT GGCTAATCGG AAGCCCTTCC AGCTCCGTGG 300

CTTCATGATT GTCTACAACT TCTCACTGGT GGCACTCTCC CTCTACATTG TCTATGAGTT 360

CCTGATGTCG GGCTGGCTGA GCACCTATAC CTGGCGCTGT GACCCTGTGG ACTATTCCAA 420

CAGCCCTGAG GCACTTAGGA TGGTTCGGGT GGCCTGGCTC TTCCTCTTCT CCAAGTTCAT 480

TGAGCTGATG GACACAGTGA TCTTTATTCT CCGAAAGAAA GACGGGCAGG TGACCTTCCT 540

ACATGTCTTC CATCACTCTG TGCTTCCCTG GAGCTGGTGG TGGGGGGTAA AGATTGCCCC 600

GGGAGGAATG GGCTCTTTCC ATGCCATGAT AAACTCTTCC GTGCATGTCA TAATGTACCT 660

GTACTACGGA TTATCTGCCT TTGGCCCTGT GGCACAACCC TACCTTTGGT GGAAAAAGCA 720

CATGACAGCC ATTCAGCTGA TCCAGTTTGT CCTGGTCTCA CTGCACATCT CCCAGTACTA 780

CTTTATGTCC AGCTGTAACT ACCAGTACCC AGTCATTATT CACCTCATCT GGATGTATGG 840

CACCATCTTC TTCATGCTGT TCTCCAACTT CTGGTATCAC TCTTATACCA AGGGCAAGCG 900

GCTGCCCCGT GCACTTCAGC AAAATGGAGC TCCAGGTATT GCCAAGGTCA AGGCCAACTG 960

AGAAGCATGG CCTAGATAGG CGCCCACCTA AGTGCCTCAG GACTGCACCT TAGGGCAGTG 1020

TCCGTCAGTG CCCTCTCCAC CTACACCTGT GACCAAGGCT TATGTGGTCA GGACTGAGCA 1080

GGGGACTGGC CCTCCCCTCC CCACAGCTGC TCTACAGGGA CCACGGCTTT GGTTCCTCAC 1140

CCACTTCCCC CGGGCAGCTC CAGGGATGTG GCCTCATTGC TGTCTGCCAC TCCAGAGCTG 1200

GGGGCTAAAA GGGCTGTACA GTTATTTCCC CCTCCCTGCC TTAAAACTTG GGAGAGGAGC 1260

ACTCAGGGCT GGCCCCACAA AGGGTCTCGT GGCCTTTTTC CTCACACAGA AGAGGTCAGC 1320

AATAATGTCA CTGTGGACCC AGTCTCACTC CTCCACCCCA CACACTGAAG CAGTAGCTTC 1380

TGGGCCAAAG GTCAGGGTGG GCGGGGGCCT GGGAATACAG CCTGTGGAGG CTGCTTACTC 1440

AACTTGTGTC TTAATTAAAA GTGACAGAGG AAACCANAAA AAAAAAAAAA AAAAACTCGA 1500

GGGGGGCCCG TACCCAAATC GCCGGTATGA TCGTAAACAA TC 1542

(2) INFORMATION FOR SEQ ID NO: 107:

(i) SEQUENCE CHARACTERISTICS:
(A) LENGTH: 2327 base pairs
(B) TYPE: nucleic acid

(C) STRANDEDNESS: double
(D) TOPOLOGY: linear

(xi) SEQUENCE DESCRIPTION: SEQ ID NO: 107:

GGTAGCTCAN TGCAGTGAAA TAGTCTTACT GGAAACAAAG CCCTTTATCA AGAATAATTA 60

ACTCTTCCCT TTTCTTTTTG GAGAGGTGCT TTGTTTCTGA TCGGACCATT TCACTGCAGC 120

AAGCAACACA GTATTCTRAG CAGAAGATCG GGACTTGAGG CCATGTTGCG GAGGGCCAGT 180

RACATTATCT GGACTCTGGA GTGTGAGGAA TATGGACTCC ACTCTTCACT ATATTCACAR 240

CGATTCAGAC TTGAGCAACA ATAGCAGTTT TAGCCCTGAT GAGGAAAGGA GAACTAAAGT 300

ACAAGATGTT GTACCTCAGG CGTTGTTAGA TCAGTATTTA TCTATGACTG ACCCTTCTCG 360

TGCACAGACG GTTGACACTG AAATTGCTAA GCACTGTGCA TATAGCCTCC CTGGTGTGGC 420

CTTGACACTC GGAAGACAGA ATTGGCACTG CCTGAGAGAG ACGTATGRGA CTYTGGCCTC 480

AGACATGCAG TGGAAAGTTC GACGGAACTC TAGCATTCTC CATCCACGRG CTTGCAGTTA 540

TTCTTGGAGA TCAATTGACA GCTGCAGATC TGGTTCCAAT TTTTAATGGA TTTTTAAAAG 600

ACCTCGATGA AGTCAGGATA GGTGTTCTTA AACACTTGCA TGATTTTCTG AAGCTTCTTC 660

ATATTGACAA AAGAAGAGAA TATCTTTATC AACTTCAGGA GTTTTTGGTG ACAGATAATA 720

GTAGAAATTG GCGGTTTCGA GCTGAACTGG CTGAACAGCT GATTTTACTT CTAGAGTTAT 780

ATAGTCCCAG AGATGTTTAT GACTATTTAC GTCCCATTGC TCTGAATCTG TGTGCAGACA 840

AAGTTTCTTC TGTTCGTTGG ATTTCCTACA AGTTGGTCAG CGAGATGGTG AAGAAGCTGC 900

ACGCGGCAAC ACCACCAACG TTCGGAGTGG ACCTCATCAA TGAGCTTGTG GAGAACTTTG 960

GCAGATGTCC CAAGTGGTCT GGTCGGCAAG CCTTTGTCTT TGTCTGCCAG ACTGTCATTG 1020

AGGATGACTG CCTTCCCATG GACCAGTTTG CTGTGCATCT CATGCCGCAT CTGCTAACCT 1080

TAGCAAATGA CAGGGTTCCT AACGTGCGAG TGCTGCTTGC AAAGACATTA AGACAAACTC 1140

TACTAGAAAA AGACTATTTC TTGGCCTCTG CCAGCTGCCA CCAGGAGGCT GTGGAGCAGA 1200

CCATCATGGC TCTTCAGATG GACCGTGACA GCGATGTCAA GTATTTTGCA AGCATCCACC 1260

CTGCCAGTAC CAAAATCTCC GAAGATGCCA TGAGCACAGC GTCCTCAACC TACTAGAAGG 1320

CTTGAATCTC GGTGTCTTTC CTGCTTCCAT GAGAGCCGAG GTTCAGTGGG CATTCGCCAC 1380

GCATGTGACC TGGGATAGCT TTCGGGGGAG GAGAGACCTT CCTCTCCTGC GGACTTCATT 1440

GCAGGTGCAA GTTGCCTACA CCCAATACCA GGGATTTCAA GAGTCAAGAG AAAGTACAGT 1500

AAACACTATT ATCTTATCTT GACTTTAAKG KKWAWKMMWW KCTCAGMSRA TTATAMTTSW 1560

CWMMRARGSM WYMAAWSCTK SWGCTCYWCC KSRSTGRMKG MMRCTCTAGA AYTRGYRGAK 1620

CMYYYKSGCT KMWGGAAKKS GGCASGAGCC AGAGACCTGC ATTGCTTTCT CCTGGTTTTA 1680

TTTAACAATC GACAAATGAA ATTCTTACAG CCTGAAGGCA GACGTGTGCC CAGATGTGAA 1740

AGAGACCTTC AGTATCAGCC CTAACTCTTC TCTCCCAGGA AGGACTTGCT GGGCTCTGTG 1800

GCCAGCTGTC CAGCCCAGCC CTGTGTGTGA ATCGTTTGTG ACGTGTGCAA ATGGGAAAGG 1860

AGGGGTTTTT ACATCTCCTA AAGGACCTGA TGCCAACACA AGTAGGATTG ACTTAAACTC 1920

TTAAGCGCAG CATATTGCTG TACACATTTA CAGAATGGTT GCTGAGTGTC TGTGTCTGAT 1980

TTTTTCATGC TGGTCATGAC CTGAAGGAAA TTTATTAGAC GTATAATGTA TGTCTGGTGT 2040

TTTTAACTTG ATCATGATCA GCTCTGAGGT GCAACTTCTT CACATACTGT ACATACCTGT 2100

GACCACTCTT GGGAGTGCTG CAGTCTTTAA TCATGCTGTT TAAACTGTTG TGGCACAAGT 2160

TCTCTTGTCC AAATAAAATT TATTAATAAG ATCTATAGAG AGAGATATAT ACACTTTTGA 2220

TTGTTTTCTA GATGTCTACC AATAAATGCA ATTTGTGACC TGTAAAAAAA AAAWAAAAAA 2280

ACTCGAGGGG GGCCCGGTAC CCAAATCGCC GATATGATCT AANCATC 2327

(2) INFORMATION FOR SEQ ID NO: 108:

(i) SEQUENCE CHARACTERISTICS:
(A) LENGTH: 1062 base pairs
(B) TYPE: nucleic acid
(C) STRANDEDNESS: double
(D) TOPOLOGY: linear

(xi) SEQUENCE DESCRIPTION: SEQ ID NO: 108:

GGCCGCCGAG GCGCAACAGC CGTTCTGTCA GCTCTGGGTC CAACCGGACT AGCGAANATC 60

TTCCTCATCC TCATCATCGT CTTCCTCATC CCGATCTCGG TCCAGGTCCC TCTCCCCCCC 120

ACACAAGAGG TGGCGAAGGT CCAGCTGTAG TTCCTCTGGA CGTTCTCGAA GATGCTCTTC 180

CTCTTCTTCG TCATCATCTT CCTCTTCGTC TTCCTCATCC TCATCATCCA GTTCTCGAAG 240

CCGCTCACGA ATCCCCATCC CCCCGCCGGA GRAAGTGACA GGAGGCGGCG GTACAGCTCT 300

TATCGTTCAC ATGACCATTA CCAAAGGCAA AGAGTGCTAC AAAAGGAGCG TGCAATAGAA 360

GAAAGAAGGG TGGTCTTCAT TGGAAAGATA CCTGGCCGCA TGACTCGATC AGAGCTGAAA 420

CAGAGGTTCT CCGTTTTTGG AGAGATTGAG GAGTGCACCA TCCACTTCCG TGTCCAAGGG 480

GACAACTACG GCTTCGTCAC TTATCGCTAT GCTGAGGAGG CATTTGCAGC CATTGAGAGT 540

GGCCACAAGC TGCGGCAGGC AGATGAGCAG CCCTTTGATC TCTGCTTTGG GGGCCGAAGG 600

SWGTNCTGCA AGAGGAGCTA TTCTGATCTT GACTCCAACC GGGAAGACTT TGACCCAGCA 660

CCTGTAAAGA GCAAATTTGA TTCTCTTGAC TTTGACACAT TGTTGAAACA GGCCCAGAAG 720

AACCTCAGGA GGTAACCTTG GGCCCTTCCC TGCTATCCTT TTTCTCCTTT GGAGGTGCCC 780

AACCTCCTCC ACCCCCTTCC CCTACTCTAG GGGAGAGAGC TGCTAGTGAG ATGACTGTTT 840

TATAAAGAAA TGGAAAAAAG TGAAATAAAA AATATGTTGA ATCAGATTTT TTAAAAGGGG 900

TATTTGTTTT TTTATAACAG GTATTGAAAC AAGTTAACTT GCATTCCTAT GTAAGATAGG 960

AGGGGCTGAG GGGATCCCCA GTGTTTGGAA CATAAGTCAC TATGCAGACT AATAAACATC 1020

AACTAGAGAG NAAAAAAAAA AAAAAAAAAA ATTTAAAAAA CT 1062

(2) INFORMATION FOR SEQ ID NO: 109:

(i) SEQUENCE CHARACTERISTICS:
(A) LENGTH: 2539 base pairs
(B) TYPE: nucleic acid
(C) STRANDEDNESS: double
(D) TOPOLOGY: linear

(xi) SEQUENCE DESCRIPTION: SEQ ID NO: 109:

GAGAGACTCA CACTTCTTTT CCATTATCAC TGACGATGTA GTGGACATAG CAGGGGAAGA 60

GCACCTACCT GTGTTGGTGA GGTTTGTTGA TGAATCTCAT AACCTAAGAG AGGAATTTAT 120

AGGCTTCCTG CCTTATGAAG CCGATGCAGA AATTTTGGCT GTGAAATTTC ACACTATGAT 180

AACTGAGAAG TGGGGATTAA ATATGGAGTA TTGTCGTGGC CAGGCTTACA TTGWCTCTAG 240

TGGATTTTCT TCCAAAATGA AAGTTGTTGC TTCTAGACTT TYAAGMKMRA TWKCCCCMAK 300

YWAWCKGAAC AMAMKCTGSW CYTCCWSYGC SKTRRMKRYC GYKSTATRRC WARWKSAKYM 360

CCYGKKMTGS RRGTAWYTSK TGCAYKAGGG AACAATTGAG GAAGTTTGTT CTTTTTTCCA 420

TCGATCACCA CAACTGCTTT TAGAACTTGA CAACGTAATT TCTGTTCTTT TTCAGAACAG 480

TAAAGAAAGG GGTAAAGAAC TGAAGGAAAT CTGCCATTCT CAGTGGACAG GCAGGCATGA 540

TGCTTTTGAA ATTTTAGTGG AACTCCTGCA AGCACTTGTT TTATGTTTAG ATGGTATAAA 600

TAGTGACACA AATATTAGAT GGAATAACTA TATAGCTGGC CGAGCATTTG TACTCTGAGT 660

GCAGTGTCAG ATTTTGATTT CATTGTTACT ATTGTTGTTC TTAAAAATGT CCTATCTTTT 720

ACAAGAGCCT TTGGGAAAAA CYYCMAGGGG CAAACCTCTG ATGTCTTCTT TGCKKMMSRT 780

ARMTTTTGAY ATRMARYACT RMMTKSAYTY AAYGRWGTGA CWSGAWAATA TTRAASTYTA 840

TACAATKAAT YWTRRYTTSM KRMAGMYAAT CCGAAAYTGT GGMAAMYAAA CTTGATATTC 900

AAATGAAACT CCCTGGGAAA TTCCGCAGAG CTCACCAGGG TAACTTGGAA TCTCAGCTAA 960

CCTCTGAGAG TTACTATAAA GAAACCCTAA GTGTCCCAAC AGTGGAGCAC ATTATTCAGG 1020

AACTTAAAGA TATATTCTCA GAACAGCACC TCAAAGCTCT TAAATGCTTA TCTCTGGTAC 1080

CCTCAGTCAT GGGACAACTC AAATTCAATA CGTCGGAGGA ACACCATGCT GACATGTATA 1140

GAAGTGACTT ACCCAATCCT GACACGCTGT CAGCTGAGCT TCATTGTTGG AGAATCAAAT 1200

GGAAACACAG GGGGAAAGAT ATAGAGCTTC CGTCCACCAT CTATGAAGCC CTCCACCTGC 1260

CTGACATCAA GTTTTTTCCT AATGTGTATG CATTGCTGAA GGTCCTGTGT ATTCTTCCTG 1320

TGATGAAGGT TGAGAATGAG CGGTATGAAA ATGGACGAAA GCGTCTTAAA GCATATTTGA 1380

GGAACACTTT GACAGACCAA AGGTCAAGTA ACTTGGCTTT GCTTAACATA AATTTTGATA 1440

TAAAACACGA CCTGGATTTA ATGGTGGACA CATATATTAA ACTCTATACR AKTAMGTCAG 1500

MGCTYYCTAC AKAYRAYTCM SWAWMTGTGG AAARYWSSTA MGMSWGCWKK TAMMRRTMCG 1560

GMWWTYYYMK RKTYGAYMYW YGCGWMCGAG AAAAAGCCGT AAGGTGTATG TAGACCACTT 1620

AATCACTAAA TATCTTTGCC TATAGGACTC CATTGAATAC ATTAGCCATT GATAATCTAC 1680

CTGTTTAAAT GGCCCCTGTT TGAACTCTCA AGCTTTGAAG ACCTACCTGT TCTTCCAGAA 1740

GAGAACGTTG AAAGTGCCAT GTTTCCTTTT GCGTGATCTC TGTTGATGGC ACTCTGGAAT 1800

TGTTTCCAGT TTAAKTCATT TTAGACATAG CATTTATTAT CACTGTGGAT CTCTACTTGT 1860

TGGGTGTTAT GAATTCTTTG AAGAATATAT TTTGAAGAGG TGTGGGAGGA AGGAATACAT 1920

TTTATAAAAT GTTGTAGTGA AGCCCACAAT TGACCTTKGA CTAATAGGAG TTTTAAGTAT 1980

GTTAAAAATC TATACTGGAC AGTTACAAGA AATTACCGGA GAAAAGCTTG TGAGCTCACC 2040

AAACAAGGAT TTCAGTGTAG ATTTTGTCTT TCTTGAACTT AAAGAAACAA ATGACAAAGT 2100

TTGAATGGAA AAGCCTGCTG TTGTTCCACA TCTCGTTGCT GTTTACATTC CTTTGTGGAG 2160

CCTACATCTT CCTAAGCTTT TTAGCAGGTA TATGTTGAAC ACTTCTGTTT CATGGTTGAG 2220

ACAGAATCAG AGGCCATGGA TACTGACAAC TGATTTGTCT GTTTTTTTTC TCTGTCTTTT 2280

TCCATGACTC TTATATACTG CCTCATCTTG ATTTATAAGC AAAACCTGGA AAACCTACAA 2340

AATAAGTGTT GTGGTTTATC TAGAAAAATA TGGAAAATAT TGCTGTTATT TTTGGTGAAG 2400

AAAATCAATT TTGTATAGTT TATTTCAATC TAAATAAAAT GTGAATTTTG TTWWATTAAA 2460

AATTWGGSAC AAABTBGHGG GGGDTCCAAA CHTWVTCGHG KAAMTTCTCT WAARMATYTK 2520

ATAAACMSCT TCACAATTC 2539

(2) INFORMATION FOR SEQ ID NO: 110:

(i) SEQUENCE CHARACTERISTICS:
(A) LENGTH: 1751 base pairs
(B) TYPE: nucleic acid
(C) STRANDEDNESS: double

(D) TOPOLOGY: linear

(xi) SEQUENCE DESCRIPTION: SEQ ID NO: 110:

AGCATGAAGC CGATGGCCGT GGTGGCCAGT ACCGTCCTGG GCCTGGTGCA AAACATGCGT 60

GCGTTTGGCG GGATCCTGGT GGTGGTCTAC TACGTATTTG CCATCATTGG GATCAACTTG 120

TTTAGAGGCG TCATTGTGGC TCTTCCTGGA AACAGCAGCC TGGCCCCTGC CAATGGCTCG 180

GCGCCCTGTG GGAGCTTCGA GCAGCTGGAG TACTGGGCCA ACAACTTCGA TGACTTTGCG 240

GCTGCCCTGG TCACTCTGTG GAACTTGATG GTGGTGAACA ACTGGCAGGT GTTTCTGGAT 300

GCATATCGGC GCTACTCAGG CCCGTGGTCC AAGATCTATT TTGTATTGTG GTGGCTGGTG 360

TCGTCTGTCA TCTGGGTCAA CCTGTTTCTG GCCCTGATTC TGGAGAACTT CCTTCACAAG 420

TGGGACCCCC GCAGCCACCT GCAGCCCCTT GCTGGGACCC CAGAGGCCAC CTACCAGATG 480

ACTGTGGAGC TCCTGTTCAG GGATATTCTG GAGGAGCCCG GGGAGGATGA GCTCACAGAG 540

AGGCTGAGCC AGCACCCGCA CCTGTGGCTG TGCAGGTGAC GTCCGGGCTG CCATCCCAGC 600

AGGGGCGGCA GGAGAGAGAG GCTGGCCTAA CACAGGTGCC CATCATGGAA GAGGCGGCCA 660

TGCTGTGGCC AGCCAGGCAG GAAGAGACCT TTCCTCTGAC GGACCACTAA GCTGGGGACA 720

GGAACCAAGT CCTTTGCGTG TGGCCCAACA ACCATCTACA GAACAGCTGC TGGTGCTTCA 780

GGGAGGCGCC GTGCCCTCCG CTTTCTTTTA TAGCTGCTTC AGTGAGAATT CCCTCGTCGA 840

CTCCACAGGG ACCTTTCAGA CAAAAATGCA AGAAGCAGCG GCCTCCCCTG TCCCCTGCAG 900

CTTCGGTGGT GCCTTTGCTG CCGGCAGCCC TTGGGGACCA CAGGCCTGAC CAGGGCCTGC 960

ACAGGTTAAC CGTGAGTCTG TCTCATCTAT TCACAGCTGG GAATGATACT AATACCTCCG 1020

ATTTTAGCCC AGCACCACAG GGTACGTTCC AGTTTTTCTC TCTTTCCATA GCTGTAAGGC 1080

CCTTTCTGGG AATGGTTCTC ATTCTCCTTA ATCTATTATT GGGTCAGTTT TCCTGCATGT 1140

CCCCAGCCTC CCATCACTGC CACCCACTCC CCACAGAGAT GCCCTGCTCA TCCGACTGGG 1200

GCTTTGACTC CCACACTGTG TACCCCTCTT GTGTGGACGC CCTGCTGCCA AAACCTTCAG 1260

CAAACAGCTT TCCAAATGGA AGTTGTCACT GTCAGGCCTT TACAATCAGC AACAGCAAAA 1320

TCTACATGCT GCTGAGGGTC CTGCCTCATT AAGATGCAAT AAATATGTAA GTACATAAAA 1380

ACAGCAATAG AAGAAACGTA ATGCTTTATT CTCAAATATG ATGTCTACAT AGAAAAGCCA 1440

AAATTATTAA GAATAGTAAG AATTCACCCA GCACTTTGGG AGGCCGAGGC GGGTGGATCA 1500

TGAGGTCAGG AGATCGAGAC CATCCTGGCT AACAGGGTGA AACCCCGTCT CTACTAAAAA 1560

TACAAAAAAT TGGCCGGGCG CAGTGGCGGG CGCCTGTGGT CCCAGCTACT GGGGAGGCTG 1620

AGGCAGGAGA ATGGCGTGAA CCCGGGAAGC GGAGCTTGCA GTGAGCCGAG ATTGCGCCAC 1680

TGCAGTCCGC AGTCCAGCCT GGGCGACAGA GCGAGACTCC GTCTCAAAAA AAAAAAAAAA 1740

AAAAAAAAAA A 1751

(2) INFORMATION FOR SEQ ID NO: 111:

(i) SEQUENCE CHARACTERISTICS:
(A) LENGTH: 1117 base pairs
(B) TYPE: nucleic acid
(C) STRANDEDNESS: double
(D) TOPOLOGY: linear

(xi) SEQUENCE DESCRIPTION: SEQ ID NO: 111:

AATGTTGTGG TGGTAGCATT TGGGTTAATT CTRATTATAG AGTCTCTTGG AGAGCAATGT 60

CCATAAACTA ATCCCAAACA ACATTGTCTT TTTRATGTTG TAGTGAACAG CAGAGAATTT 120

CAAAGGACCT TGCTAATATC TGTAAGACGG CAGCTACAGC AGGCATCATT GGCTGGGTGT 180

ATGGGGGAAT ACCAGCTTTT ATTCATGCTA AACAACAATA CATTGAGCAG AGCCAGGCAG 240

AAATTTATCA TAACCGGTTT GATGCTGTGC AATCTGCACA TCGTGCTGCC ACACGAGGCT 300

TCATTCGTTA TGGCTGGCGC TGGGGTTGGA GAACTGCAGT GTTTGTGACT ATATTCAACA 360

CAGTGAACAC TAGTCTGAAT GTATACCGAA ATAAAGATGC CTTAAGCCAT TTTGTAATTG 420

CAGGAGCTGT CACGGGAAGT CTTTTTAGGA TAAACGTAGG CCTGCGTGGC CTGGTGGCTG 480

GTGGCATAAT TGGAGCCTTG CTGGGCACTC CTGTAGGAGG CCTGCTGATG GCATTTCAGA 540

AGTACTCTGG TGAGACTGTT CAGGAAAGAA AACAGAAGGA TCGAAAGGCA CTCCATGAGC 600

TAAAACTGGA AGAGTGGAAA GGCAGACTAC AAGTTACTGA GCACCTCCCT GAGAAAATTG 660

AAAGTAGTTT ACAGGAAGAT GAACCTGAGA ATGATGCTAA GAAAATTGAA GCACTGCTAA 720

ACCTTCCTAG AAACCCTTCA GTAATAGATA AACAAGACAA GGACTGAAAG TGCTCTGAAC 780

TTGAAACTCA CTGGAGAGCT GAAGGGAGCT GCCATGTCCG ATGAATGCCA ACAGACAGGC 840

CACTCTTTGG TCAGCCTGCT GACAAATTTA AGTGCTGGTA CCTGTGGTGG CAGTGGCTTG 900

CTCTTGTCTT TTTCTTTTCT TTTTAACTAA GAATGGGGCT GTTGTACTCT CACTTTACTT 960

ATCCTTAAAT TTAAATACAT ACTTATGTTT GTATTAATCT ATCAATATAT GCATACATGA 1020

ATATATCCAC CCACCTAGAT TTTAAGCAGT AAATAAAACA TTTCGCAAAA GATTAAAGTT 1080

GAATTTTACA GTTAAAAAAA AAAAAAAAAA AAAAAAA 1117

(2) INFORMATION FOR SEQ ID NO: 112:

(i) SEQUENCE CHARACTERISTICS:
(A) LENGTH: 1313 base pairs
(B) TYPE: nucleic acid
(C) STRANDEDNESS: double
(D) TOPOLOGY: linear

(xi) SEQUENCE DESCRIPTION: SEQ ID NO: 112:

GGCAGAGGTT TTCTTATATT TTAAGTAAAT TTAAAGTGGC TATCAGAATA TTTATTCTTG 60

TTTGAGACTA CCAACATAAC TACGTGTTGA AGGTGCTTCA CAGAGAATAT ATTGCCTTTA 120

ATGTGAAATA ATTTTCACCA ATGTTGCTAA CTTTAATAAA GTATAAAATT TGTAGAATAT 180

TCAGTTAAGT AGTTGGTAAC CCTTTTCTAT TTTAGTAAAA CTTAATGCAT GTTTACTTTT 240

TTTTGAAAGA TGCAGACAAT CTCTTTGAAC ATGAATTGGG GGCTCTCAAT ATGGCTGCAT 300

TACTACGAAA AGAAGAAAGA GCAAGTCTTC TTAGTAATCT TGGCCCATGT TGTAAGGCGT 360

TGTGCTTCAG ACGGGATTCT GCAATTCGAA AGCAGCTTGT TAAAAATGAG AAGGGCACCA 420

TAAAACAAGC TTACACGAGT GCTCCAATGG TAGACAATGA ATTACTTCGA TTGAGTCTTC 480

GGTTATTTAA GCGGAAGACT ACTTGCCATG CTCCAGGACA TGAAAAGACT GAAGATAATA 540

AACTTTCACA GTCCAGTATC CAACAGGAAC TGTGTGTGTC TTAAGACCGA AGTTACAATA 600

TGGTATTTTT GGTACTGTCT TCCTTCAGCA GTGCATATTC TTTTGCAAAG TTCTTTGGTT 660

TGACAAGCAT TAGTGACAAA GGCAGAAAAG ATTTATCAGC CATGCTAAAA GAGTGAAGAA 720

TTTTGATCTT TAGAGACACT AGTTTTGGCC AACTTAAGAT TTTACGTTAA TTTTTACATA 780

GTATTTGACA CTCATGCAAA ATAATGTGAA AACATCTAGA TTTAGTAGTT TATTCTGCGC 840

CTTTTGTTAA AACTGAAGAT TTTGGAAAAT GGTTGTCACT GCTCTTCCAG CCTATGAATA 900

TTTTTGTGAA ATGGAACCAT GGATTTATGT CTGGATCATC CATACAGAAC CAACAATTTT 960

ATTCAAAAAC AATGTGTTCA TCAAAGTAAT TGCTCACATT GTGCAGTACT ATGTTGTACA 1020

GACCACGTGA AAGGGAATGC TGGTCTAGCT GGCGTGGTAT GTTTATAGGC GAATTTCAGC 1080

AGAAGGAAGC CAAAATAGTT TTTTCCTTTT GAAAGTTTTT TAAAAATTAT TTCATGGGTC 1140

TTTTTTTTAA TTAATATGTG TGCATTGTTA CAATGTATGT TGGGATGTCT TTTGACCCTA 1200

AATGCTTTTT TTGTTATCAG AGATTGTGTA CTATTTTTAT TTTTAATAAA TGTATCTTCC 1260

CTTTTMAAAA AAAAAAAAAA AAAAAAAAAA AAAAAAAAAA AAAAAAAAAA AAA 1313

(2) INFORMATION FOR SEQ ID NO: 113:

(i) SEQUENCE CHARACTERISTICS:
(A) LENGTH: 1654 base pairs
(B) TYPE: nucleic acid

(C) STRANDEDNESS: double
(D) TOPOLOGY: linear

(xi) SEQUENCE DESCRIPTION: SEQ ID NO: 113:

ACAGGGACAG AATACTTTCT TTCCTTCCTT CAAGTACAAG AAGGCTTTCT CTACCATTTG 60

CGTCTACACT TTATTTTAAA AGCTATCCTT TTCTAGTAGT ATTTTATCAT GGCAATGGCA 120

TGATGACAAC AACAGTCTTT CATTACAGAC TGAAGGGAAG CATGTCCTTA CTTAAAATAG 180

TTCTGCTACT TTCCCTCCTA TTATAAGGAA ATTTTACAGA TTCTAAAAAT ACCTTAATTT 240

TTCTTTGATT TTTATTTTAC CAAGTCACAA ATGTCTTTTT GATGTTTTGA GAATTGTTCT 300

CATAGAATCA CAAATACTGA CATTTCATTA GATGATTATT TTCCTAGAAT CCCCAAAGAG 360

CAGTGGCAGT CCATGGCTTG GTTGAAGCTA GAAATTTTCC TGCCCCTGGT GACCTGGTAA 420

GCCTCCTGCT CGGAACCGTG TGAGTGGGTG AGGAAGATGA GAGATGGTCA GATGGAAGAG 480

AGRAATACAT GAACTGCTCT GGCCTCTCTG GTTCTGTTCT TGGCCCAGAG TTTTTGAAAA 540

GCAGCGGANA TNGACTGACT TCACATGCTC AGCTTTCTCA GCCTTTTGTT TATTTTGTTG 600

TCCTTAGATT TCCCTGTTGT AAAAGGGGCA AGAAAAGTAA CTCATCATCT CTAACACACC 660

ATGGCAGCTT AGCCAGGTAG TCTTAGTGGT GGTGTTTAGG CATAAGATAT GCTGATCATC 720

AGTCTCAGGC CACAGTTTCC TTCACTAATC GTCCAGCTTG AGTGTTCTGT TCTCTTCCTG 780

CCCATTTCCT TGAACCTCCT GCTCTAGCCT TGGCGGAGGG AGAGTGCTAT TTGCTTTTGT 840

TCTCCCTCTG TCTTAGGAAA AGCCATCTTT AATATAGTTC TTCACCACTG TTGGGGTTGT 900

TTTGTGATTT TTTTTTTCTT CCGAAGAACT CCTGGTTGTT ATTGGATTTT GTATTTTAAT 960

ACAAATTATT GAATTTTATA AGCTTGTACA CAATATTTAA TTAGTGTGAA AGGAAACAAA 1020

GAATGCAGGA AAAATAATTT AATATCAACC TCAGTTGACA AGGTGCTCAG ATTATTCAAT 1080

TCGGGATCCT CCTTTTGTTA GGTTTTTGAG ACAACCCTAG ACCTAAACTG TGTCACAGAC 1140

TTCTGAATGT TTAGGCAGTG CTAGTAATTT CCTCGTAATG ATTCTGTTAT TACTTTCCTA 1200

TTCTTTATTC CTCTTTCTTC TGAAGATTAA TGAAGTTGAA AATTGAGGTG GATAAATACA 1260

AAAAGGTAGT GTGATAGTAT AAGTATCTAA GTGCAGATGA AAGTGTGTTA TATACATCCA 1320

TTCAAAATTA TGCAAGTTAG TAATTACTCA GGGTTAACTA AATTACTTTA ATATGCTGTT 1380

GAAYCTACTC TGTTCCTTGG CTAGAAAAAA TTATAAACAG GACTTTGTAG TTTGGGAAGC 1440

CAAATTGATA ATATTCTATG TTCTAAAAGT TGGGCTATAC ATAAATTATT AAGAAATATG 1500

GATTTTTATT CCCAGGATAT GGTGTTCATT TTATGATATT ACGCAGGATG ATGTATTGAG 1560

TAAAATCAGT TTTGTAAATA TGTAAATATG TCATAAATAA ACAATGCTTT GACTTATTTC 1620

CAAAAAAAAA AAAAAATAAA NTTCGAGGGG GGGC 1654

(2) INFORMATION FOR SEQ ID NO: 114:

(i) SEQUENCE CHARACTERISTICS:
(A) LENGTH: 1171 base pairs
(B) TYPE: nucleic acid
(C) STRANDEDNESS: double
(D) TOPOLOGY: linear

(xi) SEQUENCE DESCRIPTION: SEQ ID NO: 114:

GGCAAACTTT CCCCCAANGC TTCGAAACTT GCAAGCCGAA ACCTTGAATC GTTAAAAGTT 60

GGGTTGCGNC GGCGCCCTGG CCCGAAGAAG CGCAATTGGC GTTCCGCGAA CGTTGGCCCT 120

CAACGGCTCG GCAGCCAGCC ATGTCCTGCA CCCAGGACAG CGGCCCTGGG CTACAAGGAC 180

CTGGMCCTCA TCTTCCTGCG CCGACCTGCG CGGGGTAAGG GGWAGTTTCA GACTGTGAAG 240

GACGTCGTGC TGGACTGCCT GTTGGACTTC TTACCCGAGG GGGTGAACAA AGAGAAGATC 300

ACACCACTCA CGCTCAAGGA AGCTTATGTG CAGAAAATGG TTAAAGTGTG CAATGACTCT 360

GACCGATGGA GTCTTATATC CCTGTCAAAC AACAGTGGCA AAAATGTGGA ACTGAAATTT 420

GTGGATTCCC TCCGGAGGCA GTTTGAATTC AGTGTAGATT CTTTTCAAAT CAAATTAGAC 480

TCTCTTCTGC TCTTTTATGA ATGTTCAGAG AACCCAATGA CTGAGACATT TCACCCCACA 540

ATAATCGGGG AGAGCGTCTA TGGCGATTTC CAGGAAGCCT TTGATCACCT TTGTAACAAG 600

ATCATTGCCA CCAGGAACCC AGAGGAAATC CGAGGGGGAG GCCTGCTTAA GTACTGCAAC 660

CTCTTGGTGA GGGGCTTTAG GCCCGCCTCT GATGAAATCA AGACCCTTCA AAGGTATATG 720

TGTTCCAGGT TTTTCATCGA CTTCTCAGAC ATTGGAGAGC AGCAGAGAAA ACTGGAGTCC 780

TATTTGCAGA ACCACTTTGT GGGAATTGGA AGACCGCAAG TATGAGTATC TCATGACCCT 840

TCATGGAGTG GTAAATGAGA GCACAGTGTG CCTGATGGGA CATGAAAGAA GACAGACTTT 900

AAACCTTATC ACCATGCTGG CTATCCGGGT GTTAGCTGAC CAAAATGTCA TTCCTAATGT 960

GGCTAATGTC ACTTGCTATT ACCAGCCAGC CCCCTATGTA GCAGATGCCA ACTTTAGCAA 1020

TTACTACATT GCACAGGTTC AGCCAGTATT CACGTGCCAG CAACAGACCT ACTCCACTTG 1080

GCTACCCTGC AATTAAGAAT CATTTAAAAA TGTCCTGTGG GGAAGCCATT TCAGACAAGA 1140

CAGGAGAGAA AAAAAAAAAA AAAAAAAAAA A 1171

(2) INFORMATION FOR SEQ ID NO: 115:

(i) SEQUENCE CHARACTERISTICS:

(A) LENGTH: 842 base pairs
(B) TYPE: nucleic acid
(C) STRANDEDNESS: double
(D) TOPOLOGY: linear

(xi) SEQUENCE DESCRIPTION: SEQ ID NO: 115:

GGTCTGCGCC GGAAGTGCAT GAGCTGCCGA TGTGGTGCTT AGTGATTGCG GTTTCGGTCG 60

CTCTCCCGTG TTTCCCGGGC TGGGTATTTG CCTCGCACCA TGGCGCCCAA GGGCAAAGTG 120

GGCACGAGAG GGAAGAAGCA GATATTTGAA GAGAACAGAG AGACTCTGAA GTTCTACCTG 180

CGGATCATAC TGGGGGCCAA TGCCATTTAC TGCCTTGTGA CGTTGGTCTT CTTTTACTCA 240

TCTGCCTCAT TTTGGGCCTG GTTGGCCCTG GGCTTTAGTC TGGCAGTGTA TGGGGCCAGC 300

TACCACTCTA TGAGCTCGAT GGCACGAGCA GCGTTCTCTG AGGATGGGGC CCTGATGGAT 360

GGTGGCATGG ACCTCAACAT GGAGCAGGGC ATGGCAGAGC ACCTTAAGGA TGTGATCCTA 420

CTGACAGCCA TCGTGCAGGT GCTCAGCTGC TTCTCTCTCT ATGTCTGGTC CTTCTGGCTT 480

CTGGCTCCAG GCCGGGCCCT TTACCTCCTG TGGGTGAATG TGCTGGGCCC CTGGTTCACT 540

GCAGACAGTG GCACCCCAGC ACCAGAGCAC AATGAGAAAC GGCAGCGCCG ACAGGAGCGG 600

CGGCAGATGA AGCGGTTATA GCCATTGACA TTGTGGCCAC AGGCCACTGG CCCTGGGTGG 660

CTCTGTCAGG GTGCACAGCC CCTCATGCCT GGAGCAATGA GGGTCTAGTC CAGGGGCCAA 720

AAGCAGTCTG AGGTATTGGG TATACTTATA CTCTATAGGG TCGTTGAATA AATGGCTTAG 780

AATGTGAAAA AAAAAAAAAA AAAAAACTCG AGGGGGGCCC GGTACCCAAT TTCNCCTANA 840

AT 842

(2) INFORMATION FOR SEQ ID NO: 116:

(i) SEQUENCE CHARACTERISTICS:
(A) LENGTH: 1640 base pairs
(B) TYPE: nucleic acid
(C) STRANDEDNESS: double
(D) TOPOLOGY: linear

(xi) SEQUENCE DESCRIPTION: SEQ ID NO: 116:

GGCACGAGGC GGCGGCAGCG GTGGCGGCGG CGCCCCCCGG CGGGAGCCGT TCCCTTTCCC 60

GTCGGGGAGC GCGGGGYCGG GGCCCAGGGG ACCCCGGGCC ACGGAGAGCG GGAAGAGGAT 120

GGATTGCCCG GCCCTCCCCC CCGGATGGAA GAAGGAGGAA GTGATCCGAA AATCTGGGCT 180

AAGTGCTGGC AAGAGCGATG TCTACTACTT CAGTCCAAGT GGTAAGAAGT TCAGAAGCAA 240

GCCTCAGTTG GCAAGGTACC TGGGAAATAC TGTTGATCTC AGCAGTTTTG ACTTCAGAAC 300

TGGAAAGATG ATGCCTAGTA AATTACAGAA GAACAAACAG AGACTGCGAA ACGATCCTCT 360

CAATCAAAAT AAGGGTAAAC CAGACTTGAA ATACAACATT GCCAATTAGA CAAACAGCAT 420

CAATTTTCAA ACAACCGGTA ACCCAAAGTC ACAAATCATC CTAGTAATAA AGTGAAATCA 480

GACCCACAAC GAATGAATGA ACAGCCACGT CAGCTTTTCT GGGAGAAGAG GCTACAAGGA 540

CTTTAGTGCA TCAGATGTAA CAGAACAAAT TATAAAAACC ATGGAACTAC CCAAAGGTCT 600

TCAAGGAGTT GGTCCAGTAG CAATGATGAG ACCCTTTTAT CTGCTGTTGC CAGTGCTTTG 660

CACACAAGCT CTGCGCCAAT CACAGGGCAA GTCTCCGCTG CTGTGGAAAA GAACCTGCTG 720

TTTGGCTTAA CACATCTCAA CCCCTCTGCA AAGCTTTTAT TGTCACAGAT GAAGACTCAG 780

GAAACAGAAG AGCGAGTACA GCAAGTACGC AAGAAATTGG AAGAAGCACT GATGGCAGAC 840

ATCTTGTCGC GAGCTGCTGA TACAGAAGAG ATGGATATTG AAATGGACAG TGGAGATGAA 900

GCCTAAGAAT ATGATCAGGT AACTTTCGAC CGACTTTCCC CAAGAGAAAA TTCCTAGGAA 960

ATTGAACAAA AATGTTTCCA CTGGCTTTTG CCTGTAAGAA AAAAAATGTA CCCGAGCACA 1020

TAGAGCTTTT TAATAGCACT AACCAATGCC TTTTTAGATG TATTTTTGAT GTATATATCT 1080

ATTATTCAAA AAATCATGTT TATTTTGAGT CCTAGGACTT AAAATTAGTC TTTTGTAATA 1140

TCAAGCAGGA CCCTAAGATG AAGCTGAGCT TTTGATGCCA GGTGCAATCT ACTGGAAATG 1200

TAGCACTTAC GTAAAACATT TGTTTCCCCC ACAGTTTTAA TAAGAACAGA TCAGGAATTC 1260

TAAATAAATT TCCCAGTTAA AGATTATTGT GACTTCACTG TATATAAACA TATTTTTATA 1320

CTTTATTGAA AGGGGACACC TGTACATTCT TCCATCGTCA CTGTAAAGAC AAATAAATGA 1380

TTATATTCCA CAGAAAAAAA AAAAAAAAAW MWSTYGARRR GSRGCMCRSW AYMMARWWCC 1440

CCWMRTWRGS MKTCSTMTKA YTTACATTCA ACTCTGATCC CGGGGCCTTA GGTTTGACAT 1500

GGGAGGTGGG AGGAAGATAG CGCATATATT TGCAGTATGA ACTATTGCCT CTGGGACGTT 1560

GTGAGGAATT GTGCTTTCAC CAGAATTTCT AAGGATTTCT GGCTTAAATA TCACCTAGCC 1620

TGTGGTAATT TTTTTTCCCT 1640

(2) INFORMATION FOR SEQ ID NO: 117:

(i) SEQUENCE CHARACTERISTICS:
(A) LENGTH: 952 base pairs
(B) TYPE: nucleic acid
(C) STRANDEDNESS: double
(D) TOPOLOGY: linear

(xi) SEQUENCE DESCRIPTION: SEQ ID NO: 117:

TGAATTTAGN AAACACTTTG GAAAACTCAT AACCTCATCA GAAACTGCCT TTAGCCACAC 60

TCCTGACCTT CTAGATGAGT AACAAAAAAA TGAAATAAGT TCTTGGAAAT TAAGCCATTT 120

ATTTTAATTT GCTATTTTTT TCAATGTTCT AGGTATCTTT AAATTTGTTA TTGTGGAATC 180

ATTTTCCTGC CAGATACCTT TATCAAAATT ATTGGCCTCA TGAGAGCTGA AGTAAGTCAG 240

CTTTTTGGTG AACTTTAGTG GACTTCTGTG AGATTGTAGT TGTACTTTGT ATCTCTAAAT 300

CTAAAGATAG TTTTTTAAAA CTCCCAAAGA AAATCTGCTC TCCTTTCTGA TCTAAAAACT 360

CATCTTTGGG GTAAAGAGTT AAGTGTCCAA AGGTTGTCAC AGTTCATGAG GTCAGAGGGA 420

GCTAGCCTGG CACCTGGACT CTGCCCATCC ACAGCTGACA GATTCCAACA GAAGTGTATT 480

TAAATTCTCC AGTAGACAAT GCTGGGTAAG GGAGGGGGTA GGGCTGGGTT ATTAAGATAC 540

AGGCTGCTGT ATTTTACATT GGTTGTGGGG GAAGGGGAGC CTGGAGAAAA CAAAGTCACT 600

ATTCCCTTTT TTGAAACAGG AAAAAAAATT ATTTTTTGTT CAGTAAAAAT GGTAGAGAAT 660

TCCAATGTCC CTAGCCACAA GGGACCAGTT CCACTGAGAA GTGAACAGTG GGAACTCAAA 720

ATTTCAGAAA CATTGGGGGA AGGGAAAATT GGCTTTCTCT TAATTGGCAG ATGTTCCAGT 780

GGGGSGGGGG GGCTCTGTTT TTGTTGGGAT GTGTTATGTT GTATGTACGC ATATATGGAC 840

CGGAGTCTGC TGAGTTTATA AGGTTCCAAA AATATGGTAA AATCTTGGTT TTTGTTAATT 900

TATCTCAATA AAAGCCCACT GGRACTCCAA AAAAAAAAAA AAAAAAAAGA NN 952

(2) INFORMATION FOR SEQ ID NO: 118:

(i) SEQUENCE CHARACTERISTICS:
(A) LENGTH: 1256 base pairs
(B) TYPE: nucleic acid
(C) STRANDEDNESS: double
(D) TOPOLOGY: linear

(xi) SEQUENCE DESCRIPTION: SEQ ID NO: 118:

GACGTCATAG GTAAACAGGC TCTGTATCCG TGGCAGCGGC CGTGGCAGGC TGGCTGGGTA 60

CCGGCTGTCG CTGACCCAGG AGAAGCTGCC TGTCTACATC AGCCTGGGCT GCAGCGCGCT 120

GCCGCCGCGG GGCCGGCAGC TGAACTATGT GCTCTTCAGG GCGGGCACCG TGTTGCATTC 180

ATCTTTGTAC CCCCAGCATC TAGCAGTGTT GGCATGTAGT AGGCACTCAA GAAATGTGTG 240

TTGAATGAAC GATGCCTGTG ACAAGCAAGC GGACTTTATT CTTTCCTGAC CCTTGCTCCT 300

ATGACACACC TCCTCCTGAC TGCCACTGTC ACTCCTTCAG AGCAGAACTC CTCTAGGGAA 360

CCTGGATGGG AAACAGCCAT GGCCAAGGAC ATCCTGGGTG AAGCAGGGCT ACACTTTGAT 420

GAACTGAACA AGCTGAGGGT GTTGGACCCA GAGGTTACCC AGCAGACCAT AGAGCTGAAG 480

GAAGAGTGCA AAGACTTTGT GGACAAAATT GGCCAGTTTC AGAAAATAGT TGGTGGTTTA 540

ATTGAGCTTG TTGATCAACT TGCAAAAGAA GCAGAAAATG AAAAGATGAA GGCCATCGGT 600

GCTCGGAACT TGCTCAAATC TATAGCAAAG CAGAGAGAAG CTCAACAGCA GCAACTTCAA 660

GCCCTAATAG CAGAAAAGAA AATGCAGCTA GAAAGGTATC GGGTTGAATA TGAAGCTTTG 720

TGTAAAGTAG AAGCAGAACA AAATGAATTT ATTGACCAAT TTATTTTTCA GAAATGAACT 780

GAAAATTTCG CTTTTATAGT AGGAAGGCAA AACAAAAAAA AGCCTCTCAA AACCAAAAAA 840

ACCTCTGTAG CATTCCAGCG GCTTGACCAA TGACCTATGT CACAAGAGGT GGCGTGTAAG 900

GAATGCAGCC CCCTGAAGAC AGCACTACAA GTCTGGGGGA GCCAGTTTTA ACATCAGTGC 960

ACAGCTGCTG CTGGTGGCCC TGCAGTGTAC GTTCTCACCT CTTATGCTTA GTTGGAACTA 1020

AGCAGTTTGT AAACTTTCAT CCTTTTTTTT GTAAATTCAC AAAGCTTTGG AAGGAGAAGC 1080

AATAAATTTT TGTTTTCAAA TGGCTTGATG TACCTTTTTT CCTGTTGCTC TTGAAATATG 1140

TTTAACTCCT CATGAGAGAA CCCTGGATTC TCTATCCCCT AGTCCACAAA ACAAACCAGG 1200

CAGTGGTCAG CAGCTACCTT TNATTTGGAT CACACACGTG AGTCAGACAG TACCAC 1256

(2) INFORMATION FOR SEQ ID NO: 119:

(i) SEQUENCE CHARACTERISTICS:
(A) LENGTH: 1143 base pairs
(B) TYPE: nucleic acid
(C) STRANDEDNESS: double
(D) TOPOLOGY: linear

(xi) SEQUENCE DESCRIPTION: SEQ ID NO: 119:

GGCCGTAGCA GCCGGGCTGG TCCTGCTGCG AGCCGGCGGC CCGGAGTGGG GCGGCGGCAT 60

GTACCTTCCA CATTGAGTAT TCAGAAAGAA GTGATCTGAA CTCTGACCAT TCTTTATGGA 120

TACATTAAGT CAAATATAAG AGTCTGACTA CTTGACACAC TGGCTCGAGC AAACATGAAC 180

GTTGGAGTTG CCCACAGTGA AGTGAATCCA AATACCCGTG TCATGAACAG CCGGGGTATG 240

TGGCTGACAT ATGCATTGGG AGTTGGCTTG CTTCATATTG TCTTACTCAG CATTCCCTTC 300

TTCAGTGTTC CTGTTGCTTG GACTTTAACA AATATTATAC ATAATCTGGG GATGTACGTA 360

TTTTTGCATG CAGTGAAAGG AACACCTTTC GAAACTCCTG ACCAGGGTAA AGCAAGGCTC 420

CTAACTCATT GGGAACAACT GGACTATGGA GTACAGTTTA CATCTTCACG GAAGTTTTTC 480

ACAATTTCTC CAATAATTCT ATATTTTCTG GCAAGTTTCT ATACGAAGTA TGATCCAACT 540

CACTTCATCC TAAACACAGC TTCTCTCCTG AGTGTACTAA TTCCCAAAAT GCCACAACTA 600

CATGGTGTTC GGATCTTTGG AATTAATAAG TATTGAAATG TTTTGAAACT GAAAAAAAAT 660

TTTACAGCTA CTGAATTTCT TATAAGGAAG GAGTGGTTAG TAAACTGCAC TGTTTCTSTG 720

ATAATGTGAA ATGAGAAGTA TTTACATTGG AGGGCCAATG GCTGGTCCTT CAAGTGCTGT 780

TTTGAAGTGC AGATTTCCAT TAAATGATGC CTCTGTTTAA TACACCTGGT ACATTTCTGA 840

AGAGGGGCTT TATAAGCAGG CTGGGCAGGC CCAGCTTATA AGTTAAAGGG CATCACAGTG 900

AGGGTGTAGT AGATAAATTC AAGGAAATAA GAGATTTGTA AGAAACTAGG ACCAGCTTAA 960

CTTATAATGA ATGGGCATTG TGTTAAGAAA AGAACATTTC CAGTCATTCA GCTGTGGTTA 1020

TTTAAAGCAG ACTTACATGT AAACCGGAAT CCTCTCTATA CAAGTTTATT AAAGATTATT 1080

TTTATTACCG TAAAAAAAAA AAAAAAAAAA AAAAAAAAAA AAAAAAAAAA AAAAAAAANA 1140

GAN 1143

(2) INFORMATION FOR SEQ ID NO: 120:

(i) SEQUENCE CHARACTERISTICS:
(A) LENGTH: 1782 base pairs
(B) TYPE: nucleic acid
(C) STRANDEDNESS: double
(D) TOPOLOGY: linear

(xi) SEQUENCE DESCRIPTION: SEQ ID NO: 120:

CAGGCCCCGG CCCCCCACCC ACGTCTGCGT TGCTGCCCCG CCTGGGCCRG GCCCCAAAGG 60

CAAGGACAAA GCAGCTGTCA GGGAACCTCC GCCGGAGTCG AATTTACGTG CAGCTGCCGG 120

CAACCACAGG TTCCAAGATG GTTTGCGGGG GCTTCGCGTG TTCCAAGAAC TGCCTGTGCG 180

CCCTCAACCT GCTTTACACC TTGGTTAGTC TGCTGCTAAT TGGAATTGCT GCGTGGGGCA 240

TTGGCTTCGG GCTGATTTCC AGTCTCCGAG TGGTCGGCGT GGTCATTGCA GTGGGCATCT 300

TCTTGTTCCT GATTGCTTTA GTGGGTCTGA TTGGAGCTGT AAAACATCAT CAGGTGTTGC 360

TATTYTTTTA TATGATTATT CTGTTACTTG TATTTATTGT TCAGTTTTCT GTATCTTGCG 420

CTTGTTTAGC CCTGAACCAG GAGCAACAGG GTCAGCTTCT GGAGGTTGGT TGGAACAATA 480

CGGCAAGTGC TCGAAATGAC ATCCAGAGAA ATCTAAACTG CTGTGGGTTC CGAAGTGTTA 540

ACCCAAATGA CACCTGTCTG GCTAGCTGTG TTAAAAGTGA CCACTCGTGC TCGCCATGTG 600

CTCCAATCAT AGGAGAATAT GCTGGAGAGG TTTTGAGATT TGTTGGTGGC ATTGGCCTGT 660

TCTTCAGTTT TACAGAGATC CTGGGTGTTT GGCTGACCTA CAGATACAGG AACCAGAAAG 720

ACCCCCGCGC RAATCCTAGT GCATTCCTTT GATGAGAAAA CAAGGAAGAT TTCCTTTCGT 780

ATTATGATCT TGTTCACTTT CTGTAATTTT CTGTTAAGCT CCATTTGCCA GTTTAAGGAA 840

GGAAACACTA TCTGGAAAAG TACCTTATTG ATAGTGGAAT TATATATTTT TACTCTATGT 900

TTCTCTACAT GTTTTTTTCT TTCCGTTGCT GAAAAATATT TGAAACTTGT GGTCTCTGAA 960

GCTCGGTGGC ACCTGGGAAT TTACTGTATT CATTGTCGGG CACTGTCCAC TGTGGCCTTT 1020

CTTAGCATTT TTACCTGCAG AAAAACTTTG TATGGTACCA CTGTGTTGGT TATATGGTGA 1080

ATCTGAACGT ACATCTCACT GGTATAATTA TATGTAGCAC TGTGCTGTGT AGATAGTTCC 1140

TACTGGAAAA AGAGTGGRAA TTTATTAAAA TCAGAAAGTA TGAGATCCTG TTATGTTAAG 1200

GGAAATCCAA ATTCCCAATT TTTTTTGGTC TTTTTAGGAA AGATGTGTTG TGGTAAAAAG 1260

TGTTAGTATA AAAATGATAA TTWACTKGTA GTCTTTTATG ATWACACCAA TGTATTCTAG 1320

AAATAGTTAT GYCYTAGGAA ATTGTGGTTT AATTTTTGAC TTTTACAGGT AAGTGCAAAG 1380

GAGAAGTGGT TTCATGAAAT GTTCTAATGT ATAATAACAT TTACCTTCAG CCTCCATCAG 1440

AATGGAACGA GTTTTGAGTA ATCAGGAAGT ATATCTATAT GATCTTGATA TTGTTTTATA 1500

ATAATTTGAA GTCTAAAAGA CTGCATTTTT AAACAAGTTA GTATTAATGC GTTGGCCCAC 1560

GTAGCAAAAA GATATTTGAT TATCTTAAAA ATTGTTAAAT ACCGTTTTCA TGAAAGTTCT 1620

CAGTATTGTA ACAGCAACTT GTYAAACCTA AGCATATTTG AATATGATCT CCCATAATTT 1680

GAAATTGAAA TCGTATTGTG TGGCTCTGTA TATTCTGTTA AAAAATTAAA GGACAGAAAC 1740

CTTTCTTTGT GTATGCATGT TTGAATTAAA AGAAAGTAAT GG 1782

(2) INFORMATION FOR SEQ ID NO: 121:

(i) SEQUENCE CHARACTERISTICS:
(A) LENGTH: 610 base pairs
(B) TYPE: nucleic acid
(C) STRANDEDNESS: double
(D) TOPOLOGY: linear

(xi) SEQUENCE DESCRIPTION: SEQ ID NO: 121:

GTTGGCTGCA GATTTGTGGT GCGTTCTGAG CCGTCTGTCC TGCGCCAAGA TGCTTCAAAG 60

TATTATTAAA AACATATGGA TCCCCATGAA GCCCTACTAC ACCAAAGTTT ACCAGGAGAT 120

TTGGATAGGA ATGGGGCTGA TGGGCTTCAT CGTTTATAAA ATCCGGGCTG CTGATAAAAG 180

AAGTAAGGCT TTGAAAGCTT CAGCGCCTGC TCCTGGTCAT CACAACCAGA TTTACTTGGA 240

GTACATGTGA AAGAAAACGT CAGTCTGCCT GTAAATTTCA GCAAGCCGTG TTAGATGGGG 300

AGCGTGGAAC GTCACTGTAC ACTTGTATAA GTACCGTTTA CTTCATGGCA TGAATAAATG 360

GATCTGTGAG ATGCACTGCT ACCTGGTACT GCTTTCAGTG TGTTCCCCCT CAGCCCTCCG 420

GCGTGTCAGG CATACTCTGA GTAGATAATT TGTCATGCAG CGCATGCAAT CAGAATCTCA 480

CTGAGCCACC CATCATTGTG AAATAATTAC CTCAGTTGTA CAGGACTTGG TGATCAGGAT 540

CCAGGCACTC ACTTGTATTC TACTGCTCAA TAAACGTTTA TTAAACTTGA AAAAAAAAAA 600

AAAAAAAAAA 610

(2) INFORMATION FOR SEQ ID NO: 122:

(i) SEQUENCE CHARACTERISTICS:
(A) LENGTH: 526 base pairs
(B) TYPE: nucleic acid
(C) STRANDEDNESS: double
(D) TOPOLOGY: linear

(xi) SEQUENCE DESCRIPTION: SEQ ID NO: 122:

GGTACGCCTG CAGGTACCGG TCCGGAATTC CGGGTCGCCC ACGCGTCNGG CCACGCGTCC 60

ACCCACGCGT CCGSCCACGC GTCGGAGCCG AGCCGGACTG GTCAGGATGA TCACGGACGT 120

GCAGCTCGCC ATCTTCGCCA ACATGCTGGG CGTGTCGCTC TTCTTGCTTG TCGTTCTCTA 180

TCACTACGTG GCCGTCAACA ATCCCAAGAA GCAGGAATGA AAGTGGCGCT TTCTCCGCCC 240

CAGGGTTCCA GGACATAGTC TGAGGCAAGA TGGAGGGTAT GAGGGGCCTT CACACTTCAC 300

TTCATCCCTT CTACCCATCA CAACATACAA AGCAACTACA CCTGGATTTT TCCAAACAAC 360

TTTTATTTCC TCAGAGTCTT CCTTAATCCT ATGGAACAAG AAGCTGCCAC TGAATAGGGC 420

CCAGTATAGG GGCTTGCTTT TCTACTCCCT CCCCCCAATA TAAAAATATA GACTTTTTAA 480

AAAAAAAAAA AAAAANTTCG NGGGGGGSCC GGTACCCATC CCCCTA 526

(2) INFORMATION FOR SEQ ID NO: 123:

(i) SEQUENCE CHARACTERISTICS:
(A) LENGTH: 2081 base pairs
(B) TYPE: nucleic acid
(C) STRANDEDNESS: double
(D) TOPOLOGY: linear

(xi) SEQUENCE DESCRIPTION: SEQ ID NO: 123:

TGTACCGGTC CGGAAATTCC CGGGTCGACC CACGTCGTCS GGGGAACATG GCGGCTKCGG 60

AGCCGGCGGT CCTTGCGCTC CCCAACAGCG GCGCCGGGGG CGCGGGGGCG CCGTCGGGCA 120

CAGTCCCGGT GCTCTTCTGT TTCTCAGTCT TCGCGCGACC CTCGTCGGTG CCACACGGGG 180

CGGGCTACGA GCTGCTCATC CAGAAGTTCC TCAGCCTGTA CGGCGACCAG ATCGACATGC 240

ACCGCAAATT CGTGGTGCAG CTGTTCGCCG AGGAGTGGGG CCAGTACGTG GACTTGCCCA 300

AGGGCTTCGC GGTRAGCGAG CGCTGCAAGG TGCGCCTCGT GCCGYTGCAG ATCCAGCTCA 360

CTACCCTGGG AAATCTTACA CCTTCAAGCA CTGTGTTTTT CTGCTGTGAT ATGCAGGAAA 420

GGTTCAGACC AGCCATCAAG TATTTTGGGG ATATTATTAG CGTGGGACAG AGATTGTTGC 480

AAGGGGCCCG GATTTTAGGA ATTCCTGTTA TTGTAACAGA ACAATACCCT AAAGGTCTTG 540

GGAGCACGGT TCAAGAAATT GATTTAACAG GTGTAAAACT GGTACTTCCA AAGACCAAGT 600

TTTCAATGGT ATTACCAGAA GTAGAAGCGG CATTAGCAGA GATTCCCGGA GTCAGGAGTG 660

TTGTATTATT TGGAGTAGAA ACTCATGTGT GCATCCAACA AACTGCCCTG GAGCTAGTTG 720

GCCGAGGAGT CGAGGTTCAC ATTGTTGCTG ATGCCACCTC ATCAAGAAGC ATGATGGACA 780

GGATGTTTGC CCTCGAGCGT CTCGCTCRAR CCGGGATCAT AGTGACCACG AGTGAGGCTG 840

TTCTGCTTCA GCTGGTAGCT GATAAGGACC ATCCAAAATT CAAGGAAATT CAGAATCTAA 900

TTAAGGCGAG TGCTCCAGAG TCGGGTCTGC TTTCCAAAGT ATAGGACATT TGAAGAACTG 960

GTATGCTACT CACTGGTGAA GGACAGTCAG GTGAAGGACT GTAAGCCCAC ACAAGCTCTT 1020

CTTATCTCTA CTAGAATTAA AATGTTAAGT CAAAAACGGC TCCTTTTTTG CGCCTCCTAG 1080

TGAAACTTAA CCAGCTAGAC CATTTGAGTA CCAGCATTTA GTTACAAACG TCAAAGGCTT 1140

CCGGTGCTGC TTACCTTCCT TTTTTGTTAA TGTGCTTTTA TTTATTAAAA AAAATTACAA 1200

TGAAGATGCC TGTTTTGTCT CTACTGTGTA CTCTGATCGT ATCTTTCCAA AGTGCAGACT 1260

CTTGTGAAGT TTTCTTAAAT TGTTCACTTT AAAGAAAATG ACGTACCAAC AATGATTTGG 1320

CTTTTATATT ACTGTAAGAT GTTATAATGT TAATGTGGAT GTAGTGCTTT TACTTTACAG 1380

ATTGATTGGA ATAAGATTAT TGCATATGAA TTTACCCACA GGACTCTGAA TCATGTTACC 1440

CACTCCCCTC ACAATGTTGT CCACTTAGTG AGTTGCATTG ATCTATCCGT ACCAAATGAT 1500

GTTGAATAAT TACATATCTT TCTTGACTAT ACTGATTTCT TATTTTGGTC ACTATTACTA 1560

AATCTCTGTT AATATTCTCT CTTTTAACTG AAAAGGGATG GGATAGAAGG GTTTGCAATG 1620

CCATATTATT GGTGGAGGGC TGTTTTAACA TCTTTGAAGT ATGGCTTGCT GAATATCTTT 1680

ACCAACATCT TGAATATATA TTCTAGTGTC CACAAGATTT AGCAAAAAGA TAAAGCTTGG 1740

GTGGAATATC ATTTTAAAAT GTTCATGTTC TGTTCTATAT TTTCTTCACC TACTCTCCAA 1800

ATATTGTAAT GCAAAAAGTC TCAGTAATGA TTTGGTAGTA TTAATTTTGT GGTCATTGTT 1860

TCTCTTCGAT AAATTTATTT TCATTAAATA CTTRTTAGAG GGTTTTGAAA TGTTTTTCAA 1920

ATATGTGAAA TGTGAAACTG CTGTCTTTTA TATTAAAGTA ATTAAAGAAA ATGTATTGTG 1980

ATTGAAATTA TTTTGNCCTC CACAAGATGG CTCTATGAGT ATTCTTCCAG GGATTCTAAT 2040

ATTTATTTAA GGTNATAAAA TCTTGACATT TATAATCTTT C 2081

(2) INFORMATION FOR SEQ ID NO: 124:

(i) SEQUENCE CHARACTERISTICS:
(A) LENGTH: 1717 base pairs
(B) TYPE: nucleic acid
(C) STRANDEDNESS: double
(D) TOPOLOGY: linear

(xi) SEQUENCE DESCRIPTION: SEQ ID NO: 124:

CCCCGGCGGA GCTGGACCCG CGGTGGGCTA GGGGCAGGGC CGGAGCCGCG GCGGCGGAGC 60

TGTGGATCCT TCATGATGAG AGATTTGGGG ACACTTCTCT CTCCTGTGTG TAGTTGATAG 120

TTTGGTGGTG AAGAGATGGC TGACAGTGTC AAAACCTTTC TCCAGGACCT TGCCAGAGGA 180

ATCAAAGACT CCATCTGGGG TATTTGTACC ATCTCAAAGC TAGATGCTCG AATCCAGCAA 240

AAGAGAGAGG AGCAGCGTCG AAGAAGGGCA AGTAGTGTCT TGGCACAGAG AAGAGCCCAG 300

AGTATAGAGC GGAAGCAAGA GAGTGAGCCA CGTATTGTTA GTAGAATTTT CCAGTGTTGT 360

GCTTGGAATG GTGGAGTGTT CTGGTTCAGT CTCCTCTTGT TTTATCGAGT ATTTATTCCT 420

GTGCTTCAGT CGGTAACAGC CCGAATTATC GGTGACCCAT CACTACATGG AGATGTTTGG 480

TCGTGGCTGG AATTCTTCCT CACGTCAATT TTCAGTGCTC TTTGGGTGCT CCCCTTGTTT 540

GTGCTTAGCA AAGTGGTGAA TGCCATTTGG TTTCAGGATA TAGCTGACCT GGCATTTGAG 600

GTATCAGGGA GGAAGCCTCA CCCATTCCCT AGTGTCAGCA AAATAATTGC TGACATGCTC 660

TTCAACCTTT TGCTGCAGGC TCTTTTCCTC ATTCAGGGAA TGTTTGTGAG TCTCTTTCCC 720

ATCCATCTTG TCGGTCAGCT GGTTAGTCTC CTGCATATGT CCCTTCTCTA CTCACTGTAC 780

TGCTTTGAAT ATCGTTGGTT CAATAAAGGA ATTGAAATGC ACCAGCGGTT GTCTAACATA 840

GAAAGGAATT GGCCTTACTA CTTTGGGTTT GGTTTGCCCT TGGCTTTTCT CACAGCAATG 900

CAGTCCTCAT ATATTATCAG TGGCTGCCTT TTCTCTATCC TCTTTCCTTT ATTCATTATC 960

AGCGCCAATG AAGCAAAGAC CCCTGGCAAA GCRTATCTCT TCCAGTTGCG CCTCTTCTCC 1020

TTGGTGGTCT TCTTAAGCAA CAGACTCTTC CACAAGACAG TCTACCTGCA GTCGGCCCTG 1080

AGCAGCTCTA CTTCTGCAGA GAAGTTCCCT TCACCGCATC CGTCGCCTGC CAAACTGAAG 1140

GCTACTGCAG GTCACTGAGT TGCCTGCCAT CCAAAGGGGA TGGGCGGGAT TGGAAGAAGC 1200

TGTGGCAGCT CTTTTCCCTG TTCACCTCCC GCCTGCCAGG GAAGGCAGGA CCCGCTCTGC 1260

CAAGGGCCCT CTGCGTATTC CCTTCTCTCT GAGGAATTGA AATTTTTGTC TCTGGTGCAC 1320

GTAAGGCAGA ATGTTCCCTG ACACCAGTGT GTGGATTTTT AACATCACCG TGAGTCTGAA 1380

AGGACCACAG GTTTTTCTGC AGCTATTTTC TAGCATTTGC CAGTCCCTGT GCCTGGACTG 1440

ATTGGAACAC TTTGTTTTTC TCCCTGTGCC ATTTACCCTT CCACCTTTCC ATCCTGCCTT 1500

CTACCACCCT TGGATGAATG GATTTTGTAA TTCTAGCTGT TGTATTTTGT GAATTTGTTA 1560

ATTTTGTTGT TTTTCTGTGA AACACATACA TTGGATATGG GAGGTAAAGG AGTGTCCCAG 1620

TTGCTCCTGG TCACTCCCTT TATAGCCATT ACTGTCTTGT TTCTTGTAAC TCAGGTTAGG 1680

TTTTGGTCTC TCTTGCTCCA CTGCAAAAAA AAAAAAA 1717

(2) INFORMATION FOR SEQ ID NO: 125:

(i) SEQUENCE CHARACTERISTICS:
(A) LENGTH: 804 base pairs
(B) TYPE: nucleic acid
(C) STRANDEDNESS: double
(D) TOPOLOGY: linear

(xi) SEQUENCE DESCRIPTION: SEQ ID NO: 125:

CCACGCGTCC GGTCACTATG TAGTGGAGGG GCAGACACCC TCCCGCAAAT TCTGGAAGGT 60

TCTTAGTCTC GACTAGGGCA GTAGCCCCAG GACTCCTAGT CGCCGGCTTC AGGTCACTGC 120

CGGCTGAACG GAGCTGCCGT CGCCATGTTT GGCTGCTTGG TGGCGGGGAG GCTGGTGCAA 180

ACAGCTGCAC AGCAAGTGGC AGAGGATAAA TTTGTTTTTG ACTTACCTGA TTATGAAAGT 240

ATCAACCATG TTGTGGTTTT TATGCTGGGA ACAATCCCAT TTCCTGAGGG AATGGGAGGA 300

TCTGTCTACT TTTCTTATCC TGATTCAAAT GGAATGCCAG TATGGCAACT CCTAGGATTT 360

GTCACGAATG GGAAGCCAAG TGCCATCTTC AAAATTTCAG GTCTTAAATC TGGAGAAGGA 420

AGCCAACATC CTTTTGGAGC CATGAATATT GTCCGAACTC CATCTGTTGC TCAGATTGGA 480

ATTTCAGTGG AATTATTAGA CAGTATGGCT CAGCAGACTC CTGTAGGTAA TGCTGCTGTA 540

TCCTCAGTTG ACTCATTCAC TCAGTTCACA CAAAAGATGT TGGACAATTT CTACAATTTT 600

GCTTCATCAT TTGCTGTCTC TCAGGCCCAG ATGACACCAA GCCCATCTGA AATGTTCATT 660

CCGGCAAATG TGGTTCTGAA ATGGTATGAA AACTTTCAAA GACGACTAGC ACAGAACCCT 720

NTNTTTTGGN AAACATAATT TGAATAAAAT AATTTTTAAT GGATTNTGNA AAAAAAAAAA 780

AAAAAAAAAA AAAAAAAAAA AAAA 804

(2) INFORMATION FOR SEQ ID NO: 126:

(i) SEQUENCE CHARACTERISTICS:
(A) LENGTH: 431 base pairs

(B) TYPE: nucleic acid
(C) STRANDEDNESS: double
(D) TOPOLOGY: linear

(xi) SEQUENCE DESCRIPTION: SEQ ID NO: 126:

GGCACAGCCC AGGGCCTTGA AGCCAGCTGG CCCTGGAGAG GGGCTGCTGT GCCAGCTTGG 60

GGAGGGTCTG GGATGGGGCT GCCCCTGATG GCCCTGATGT GGAGTACCTT GCCAGCATCT 120

GCTGGGGTGA ACTTTATTTT AGCCCTTCCC TTGTTGCTCT TATGGAAGAA CAGAGGAGGG 180

GTGGGCAGGT CAGTGATGTC AGCAGTGGAG TGATTCCCAG CACAGCGGCT TCTGGGAAGA 240

GGGCATGGAG GCATTTCTTT CAGGGAAATG GTCCATNATT TCAGCCAGAA GGCATTGCAT 300

TAAGTTAAGT CCNGGACTTT TGTGGCCCAG CTCTGTGTTA TTAAGGGCCC TTGGCGAAGA 360

CTTCAAGGAG GGGGCAAAAN GACCTTTAAG TTTTTAGGTT TAACACAGGG AACCCNCAAA 420

GGGTTATTTT G 431

(2) INFORMATION FOR SEQ ID NO: 127:

(i) SEQUENCE CHARACTERISTICS:
(A) LENGTH: 3752 base pairs
(B) TYPE: nucleic acid
(C) STRANDEDNESS: double
(D) TOPOLOGY: linear

(xi) SEQUENCE DESCRIPTION: SEQ ID NO: 127:

NGGCACGAGG AGAGTCACCT GGACTCAGAA CTAGAGATAT CCAATGACCC AGACAAAATT 60

AAACTTCAGC TTTCTAAGCA TAAGGAGTTT CAGAAGACTC TTGGTGGCAA GCAGCCTGTG 120

TATGATACCA CAATTAGAAC TGGCAGAGCA CTGAAAGAAA AGACTTTGCT TCCCGAAGAT 180

ASTCAGAAAC TTGACAATTT CCTAGGAGAA GTCAGAGACA AATGGGATAC TGTTTGTGGC 240

AAGTCTGTGG AGCGGCAGCA CAAGTTGGAG GAAGCCCTGC TCTTTTCGGG TCAGTTCATG 300

GATGCTTTGC AGGCATTGGT TGACTGGTTA TACAAGGTGG AGCCACAGCT GGCTGAGGAC 360

CAGCCCGTGC ACGGGGGACC TTGACCTCGT CATGAACCTC ATGGATGCAC ACAAGGTTTT 420

CCAGAAGGAA CTGGNGAAAG CGAACAGGAA CCGTTCAGGT CCTGAAGCGG TCAGGCCGAG 480

AGCTGATTGA GAATAGTCGA GATGACACCA CTTGGGTAAA AGGACAGCTC CAGGAACTGA 540

GCACTCGCTG GGACACTGTC TGTAAACTCT CTGTTTCCAA ACAAAGCCGG CTTGAGCAGG 600

CCTTAAAACA AGCGGAAGTG TTTCGAGACA CAGTCCACAT GCTGTTGGAG TGGCTTTCTG 660

AAGCAGAGCA AACGCTTCGC TTTCGGGGAG CACTTCCTGG ATGACACAGA GGCCCTGCAG 720

TCTCTCATTG ACACCCATAA GGAATTCATG AAGAAAGTAG AAGAAAAGCG AGTGGACGTT 780

AACTCAGCAG TAGCCATGGG AGAAGTCATC CTGGCTGTCT GCCACCCCGA TTGCATCACA 840

ACCATCAAAC ACTGGATCAC CATCATCCGA GCTCGCTTCG AGGAGGTCCT GACATGGGCT 900

AAGCAGCACC AGCAGCGTCT TGAAACGGCC TTGTCAGAAC TGGTGGCTAA TGCTGAGCTC 960

CTGGAAGAAC TTCTGGCATG GATCCAGTGG GCTGAGACCA CCCTCATTCA GCGGGATCAG 1020

GAGCCAATCC CGCAGAACAT TGACCGAGTT AAAGCCCTTA TCGCTGAGCA TCAGACATTT 1080

ATGGAGGAGA TGACTCGCAA ACAGCCTGAC GTGGACCGGG TCACCAAGAC ATACAAAAGG 1140

AAAAACATAG AGCCTACTCA CGCGCCTTTC ATAGAGAAAT CCCGCAGCGG AGGCAGGAAA 1200

TCCCTAAGTC AGCCAACCCC TCCTCCCATG CCAATCCTTT CACAGTCTGA AGCAAAAAAC 1260

CCACGGATCA ACCAGCTTTC TGCCCGCTGG CAGCAGGTGT GGCTGTTAGC ACTGGAGCGG 1320

CAAAGGAAAC TGAATGATGC CTTGGATCGG CTGGAGGAGT TGAAAGAATT TGCCAACTTT 1380

GACTTTGATG TCTGGAGGAA AAAGTATATG CGTTGGATGA ATCACAAAAA GTCTCGAGTG 1440

ATGGATTTCT TCCGGCGCAT TGATAAGGAC CAGGATGGGA AGATAACACG TCAGGAGTTT 1500

ATCGATGGCA TTTTAGCATC CAAGTTCCCC ACCACCAAGT TAGAGATGAC TGCTGTGGCT 1560

GACATTTTCG ACCGAGATGG GGATGGTTAC ATTGATTATT ATGAATTTGT GGCTGCTCTT 1620

CATCCCAACA AGGATGCGTA TCGACCAACA ACCGATGCAG ATAAAATCGA AGATGAGGTT 1680

ACAAGACAAG TGGCTCAGTG CAAATGTGCA AAAAGGTTTC AGGTGGAGCA GATCGGAGAG 1740

AATAAATACC GGTTCTTCCT CGGCAATCAG TTTGGGGATT CTCAGCAGTT GCGGCTGGTC 1800

CGTATTCTGC GCAACCGTGA TGGTTCGCGT TGGTGGAGGA TGGATGGCCT TGGATGAATT 1860

TTTAGTGAAA AATGATCCCT GCCGAGCACG AGGTAGAACT AACATTGAAC TTAGAGAGAA 1920

ATTCATCCTA CCAGAGGGAG CATCCCAGGG AATGACCCCC TTCCGCTCAC GGGGTCGAAG 1980

GTCCAAACCA TCTTCCCGGG CAGCTTCCCC TACTCGTTCC AGCTCCAGTG CTAGTCAGAG 2040

TAACCACAGC TGTACATCCA TGCCATCTTC TCCAGCCACC CCAGCCAGTG GAACCAAGGT 2100

TATCCCATCA TCAGGTAGCA AGTTGAAACG ACCAACACCA ACTTTTCATT CTAGTCGGAC 2160

ATCCCTTGCT GGTGATACCA GCAATNAGTT CTTCCCCGGC CTCCACAGGT GCCAAAACTA 2220

ATCGGGCAGA CCCTAAAAAG TCTGCCAGTC GCCCTGGGAG TCGGGCTGGG AGTCGAGCCG 2280

GGAGTCGAGC CAGCAGCCGG CGAGGAAGTG ACGCTTCTGA CTTTGACCTC TTAGAGACGC 2340

ATTGCTTGTT CCGACACTTC AGAAAGCAGC GCTGCAGGGG GCCAAGGCAA CTCCAGGAGA 2400

GGGCTAAACA AACCTTCCAA AATCCCAACC ATGTCTAAGA AGACCACCAC TGCCTCCCCC 2460

AGGACTCCAG GTCCCAAGCG ATAACACTGT CTAAGCACCC CCAAGCCACT ATCCACTTTG 2520

AATCCTGCTC CATACATTGG GTGTATATTT ATTCTGAACG GGAGAAGTTA TATTGTTAAA 2580

AGTGTAAAAG AATAATTGTG TTATGAAGCT GCCTTATTTT TTTTCTTTTT GTAAGTTACT 2640

ATTTTCATGT GAATATTTAT GTAGATAAAA TTTGCCTCCT GGTAACCCTG TAATGGATGG 2700

GGCCCAGAAA TGAAATATTT GAGAAAAACA AGTGAAAAGG TCAAGATACA AATGTGTATT 2760

AAAAAAAAAA AAGCCTATTA ATAGGGTTTC TGCGCGGTGC AGGGTTGTAA ACCTGCTTTA 2820

TCTTTTAGGA TTATTCCTAA ATGCATCTTC TTTATAAACT TGACTTGCTA TCTCAGCAAG 2880

ATAAATTATA TTAAAAAAAT AAGAATCCTG CAGTGTTTAA GGAACTCTTT TTTTGTAAAT 2940

CACGGACACC TCAATTAGCA AGAACTGAGG GGAGGGCTTT TTCCATTGTT TAATGTTTTG 3000

TGATTTTTAG CTAAAGAGAG GGAACCTCAT CTAAGTAACA TTTGCACATG ATACAGCAAA 3060

AGGAGTTCAT TGCAATACTG TCTTTGGATA TTGTTTCAGT ACTGGGTGTT TAAAGGACAA 3120

ATAGCTGCTA GAATTCAGGG GTAAATGTAA GTGTTCAGAA AACGTCAGAA CATTTGGGGT 3180

TTTAAACTGA TTTGTTGCTC CCTATCCAGC CTAGACACCA GTAACTCTTG TGTTCACCAG 3240

GACCCAGACC CTTGGCAAGG GATAGGCTCG TTGGTGACAT TGTGAATTTC AGATTTGTTT 3300

TATCCACTTT TTTTGCTATT TATTTAAATG GTCGATCAAC TTCCCACAAA CTGAGGAATG 3360

AATTCCACGA GCCTGTTCTG AAAATGTGGA CGTAAGACAA ACACGTGCTC GTCCTTTAAT 3420

GGAGTTCACC AGCACACTTG TTAACCAGTC CTGTTTGCTT TCGTCTTTTT TTGTGCGTAA 3480

TAAAGTCAAC TGACCAAGTG ACCATGAAAA GGGGCTGTCT GGGGCTCCTG TTTTTTAGCT 3540

GCTGTTCTTC AGCTCCGACC ATGTTGCTGT GTGATTATCT CAATTGGTTT TAATTGAGGC 3600

AGAAACTGAA GCTCTACCAA TGAACTGTTT AGAAACAAGA CACACTTTTG TATTAAAATT 3660

GCTTGCAGTA ACAAAAAAAA AAAAAAAAAA AAAAAAAAAA AAACTCGAGG GGGGCCCGGT 3720

ACCCAATTCG CCGTATATGA TCGTAAACAA TC 3752

(2) INFORMATION FOR SEQ ID NO: 128:

(i) SEQUENCE CHARACTERISTICS:
(A) LENGTH: 1144 base pairs
(B) TYPE: nucleic acid
(C) STRANDEDNESS: double
(D) TOPOLOGY: linear

(xi) SEQUENCE DESCRIPTION: SEQ ID NO: 128:

TGACCCTCTG CCTGCCGGGC TCAGTGCTGG ACGCTTTCTG TTTTGTCGCA GTCGGTCCTC 60

GGTAACACCA GCGGCCTGTG GTCCACCACT CCATTCAGCA GCTCCATTTG GTCCAGCAAC 120

CTTAGCAGCG CCTTCCCTTC ACCACTCCAG CAAACACGCT GGCAAGCATC GGCCTCATGG 180

GCACAGAAAA CTCCCCTGCT CCTCACGCTC CCTCCACCTC CAGTCCAGCT GACGACTTGG 240

GACAGACCTA CAACCCGTGG CGGATATGGA GCCCCACGAT TGGAAGAAGA AGCTCGGACC 300

CTTGGTCTAA TTCGCACTTT CCTCACGAGA ATTAAATTAA GCAAAAAACA AACAAACATA 360

GTGGGCCCTC GTCTAGATCA TGATGTGCCA GTTTCTGAGA CATCTTTTTA AGGCTCTTAC 420

TGCAGCTCCC CTCCCCACCC TCCTCTTCTT TGCAAAACAG ACCCAAGCAG GGCAGGCTCA 480

GACCACTCGC TTCTTTCAGA TCTTTCTTGC AATTATGATA ACATGAGATT TGCTGTTGTG 540

CTTTTAGAGA AAAGTCTGGA CTCAGCCACA AACTCTAATA AGACCTGTAC ATCTGAGAAC 600

CTTTCCCGTT ACTGCGTTTT CACCACCTGT CTTCCCCATG CTTTATTTAT CTGTATGAAC 660

ACAGATTTGA CATTACAGCT AAGGAAATAA TTTGAGTTGA TTCAGAAATC CTGGCATGTG 720

ACAATTTTGT TAAATTACCA AGTTTGGTTT TTAATAATTT CTCAATATTA TGCGCCAAGA 780

TCTAATTTTA AAACTGTATG AGGACTTTGT GCTGAAAATA GAGTATTTTT TTAAAGTAAG 840

GCTGTCTTGG TTTAAAAGCA GATTACAGAA ATGTAAGTCA ACTTAAGAAC RGTGAATGAA 900

TGTAAAAACA TTCAGTYGAG ACCATATGCA TTTTCTGTGC TGTTTGTACT TGAGGTATGT 960

AACATTTGTA TACCTGAACT TATTTTAAAG ATGAACTGAA ATGCACATAG CCAAGTCTTG 1020

AGATACAAGA TTGAATGTGT ATTTCTTAAA AATACAACTT TGTGTTGTAC TTTGAAATAA 1080

ATGATGCTTT TTTCAAAAAA AAAAAAAAAA AAAAAAAAAC TCGAGGGGGG GCCCGGTACC 1140

CAAT 1144

(2) INFORMATION FOR SEQ ID NO: 129:

(i) SEQUENCE CHARACTERISTICS:
(A) LENGTH: 1830 base pairs
(B) TYPE: nucleic acid
(C) STRANDEDNESS: double
(D) TOPOLOGY: linear

(xi) SEQUENCE DESCRIPTION: SEQ ID NO: 129:

GCATGCAGAG GAGCACCCTG AGCGTGTYCC TGGAGCAGGC GGCCATSTTG GCACGGAGCC 60

ACGGGTTGCT GCCCAAGTGC ATCATGCAGG CCACGGACAT CATGCGGAAC AGGGCCCAAG 120

GGTGGAGATT CTGGCCAAAA ACCTGCGAGT CAAGGACCAG ATGCCCCAGG GTGCTCCGCG 180

CCTCTACCGC CTCTGCCAGC CGCCGGTGGA TGGGGACCTC TGAACACCCA AATGCCCCAC 240

GCTGGGCCGC GGCCTCTGGA GCTGGGATTT GGGAGGACAC AGCAGGCAGC GCTGGCCTTC 300

TCCAGGGATG GCCCAANGCT TCCGCARCCG CCCGTTCCGG GACCTGCCCA GCGTCCTCCC 360

TGCCTCCTTC CGGGACAAGC CTGGCCACCC TCGCTGTGAT GACGAGCTGG CTGATTGGCC 420

CTGGGCCGGC CCATTCTTCA CACGCCTGCC AGAAGCTGGA GGGGTGCTGG AGACCCATAG 480

AGCTGATGGG AGCAGCTGGT GCCTGGCCTT CGGCTCCTGC GTCCCCAGAA CCCAAGGGAA 540

CGTCATGGAG GCCACATGGG GCCACCCGGC TCCCTCGGGA TGGCTCCGCT GCACTTTTGA 600

AACCCCGGTT TCCTTCAACG TCCACATTCC AGGTGACCAC ACGTGTCTCC TCCTCCTCAT 660

CTTAGCTTCC AGGTTCACCC TAACCCTGTA CTAACCTGCT TGGTGGACTT GGAAAAGACT 720

TGGCTCTGTC GGGAAAGGAG AGACGGGGCC TCCATCACGC CTGTTACCAG AGGATCCCCG 780

AGAGCCACAC CAGCTCTGGA CATCACCGCC CCTGGAACTG GGGCCACCAG CCCTGGGCAC 840

GAGATTTGCT CTGACTTTAT TTATATGGCA TGAAATCTCT GGTTTATTTT GGGATTTTTT 900

GTTGTTGGTG TTGTCAAAGT TTGTTTTTTC TAAAGTTGTG TGATTATATA TTTGACATTT 960

TACATTTCAA AGAAAGGTAT GTTGTCTAAC AGGGGACCAA CAGAAGGTAG TATTGACAAC 1020

TGTTCCTGCT TCTACTAAAA AAAAAAGAGC ACAAAAGAAA AACTAAATTA TTGAAAAATT 1080

AAAAAATGTC ATTGTTTCCT GTTTGTTAAT ATTAGGGTTG TAAGGTGTCG TTTTGAGGTA 1140

TCGACTGTGA TTCCTTCCCC CACCCTCCAT TCTCCAGCGG TTGGCCGGTG TTAGAACTCG 1200

CTCTCTTTGA GTGACTGGCT ACAAGGGCCT GAGAGGTGGC CAGCCAGGGT TGGAGCTGGA 1260

GGGGATGGAG CCCCACCTGA GGTGCCGTGT CACACGGGTT AGAGGGTCAC TGGGAAACAC 1320

CGGGCGGTGG CTTCTGTGAT TTATTTTCTT GATGGTAACT TCTCAGAGCA GGGCRATTGG 1380

GACATCACCA GCCAGAGCAC AGGAAGCCAC CCTGCCTGCT GGGGAGGAGG GACCCACACA 1440

AGCCCCCTCG GCAGTTTGTC CCCCCAGCTT CGGTATGCCT TCAGGGAAAG GTCACAGCTG 1500

GGGAGGAAGC GGGGGGACGC CTGTCACCCC TGGCAGGTGG TGAGTTCAGG TGGGGGCTCC 1560

CTGCTKCCCC CAGGCCTGGG AGCTTGAAGC CCTCCCGGCA TCTGGCATCC GAGCCTCCCG 1620

CCCTCCAGGG TGCGCTTCCC TCTCTTGCCG CAGCATACAC GAGGGCAGGC AGTGGCCTTG 1680

TCACTGTATC TTGCATCAGA GACAAAGGAG GACCCGCTTT AGCCCTGCTG CGGGAAATGG 1740

GGGATGGCCC AGGGCCAGCG CATTGTGCAC TGGTTTACTT TAAAATGTAC AGATTCTTCT 1800

CGTTAAATTC TTGATAGATT TTTTATTATT 1830

(2) INFORMATION FOR SEQ ID NO: 130:

(i) SEQUENCE CHARACTERISTICS:
(A) LENGTH: 1864 base pairs
(B) TYPE: nucleic acid
(C) STRANDEDNESS: double
(D) TOPOLOGY: linear

(xi) SEQUENCE DESCRIPTION: SEQ ID NO: 130:

GGCCGCCCGG ATGGCGACCC CAGCCTCGGC CCCAGACACA CGGGCTCTGG TGGCAGACTT 60

TGTAGGTTAT AAGCTGAGGC AGAAGGGTTA TGTCTGTGGA GCTGGCCCCG GGGAGGGCCC 120

AGCAGCTGAC CCGCTGCACC AAGCCATGCG GGCAGCKGGA GATGAGTTCG AGACCCGCTT 180

CCGGCGCACC TTCTCTGATC TGGCGGCTCA GCTGCATGTG ACCCCAGGCT CAGCCCAACA 240

ACGCTTCACC CAGGTCTCCG ATGAACTTTT TCAAGGGGGC CCCAACTGGG GCCGCCTTGT 300

AGCCTTCTTT GTCTTTGGGG CTGCACTGTG TGCTGAGAGT GTCAACAAGG AGATGGAACC 360

ACTGGTGGGA CAAGTGCAGG AGTGGATGGT GGCCTACCTG GAGACGCGGC TGGCTGACTG 420

GATCCACAGC AGTGGGGGCT GGTTATCCCA GATCACTGAA GCTGAGATGG CTGATGAAGT 480

AATTTGCAGT GAAATTTTAA GCGACTGTGA CTCTGCTGCA AGTTCCCCAG ATCTTGAGGA 540

GCTGGAAGCT ATCAAAGCTC GAGTCAGGGA GATGGAGGAA GAAGCTGAGA AGCTAAAGGA 600

GCTACAGAAC GAGGTAGAGA AGCAGATGAA TATGAGTCCA CCTCCAGGCA ATGCTGGCCC 660

GGTGATCATG TCCATTGAGG AGAAGATGGA GGCTGATGCC CGTTCCATCT ATGTTGGCAA 720

TGTGGACTAT GGTGCAACAG CAGAAGAGCT GGAAGCTCAC TTTCATGGCT GTGGTTCAGT 780

CAACCGTGTT ACCATACTGT GTGACAAATT TAGTGGCCAT CCCAAAGGGT TTGCGTATAT 840

AGAGTTCTCA GACAAAGAGT CAGTGAGGAC TTCCTTGGCC TTAGATGAGT CCCTATTTAG 900

AGGAAGGCAA ATCAAGGTGA TCCCAAAACG AACCAACAGA CCAGGCATCA GCACAACAGA 960

CCGGGGTTTT CCACGAGCCC GCTACCGCGC CCGGACCACC AACTACAACA GCTCCCGCTC 1020

TCGATTCTAC AGTGGTTTTA ACAGCAGGCC CCGGGGTCGC GTCTACAGGG GCCGGGCTAG 1080

AGCGACATCA TGGTATTCCC CTTACTAAAA AAAGTGTGTA TTAGGAGGAG AGAGAGGAAA 1140

AAAAGAGGAA AGAAGGAAAA AAAAAAGAAT TAAAAAAAAA AAAAAAAAAA ACAGAAGWTG 1200

MCCTTGATGG AAAAAAAATA TTTTTTAAAA AAAAGATATA CTGTGGAAGG GGGGAGAATC 1260

CCATAACTAA CTGCTGAGGA GGGACCTGCT TTGGGGAGTA GGGGAAGGCC CAGGGARTGG 1320

GGCAGGGGGC TGCTTATTCA CTCTGGGGAT TCGCCATGGA CACGTCTCAA CTGCGCAACT 1380

GCTTGCCCAT GTTTCCCTGC CCCACCCCAC CCCTCTTCTC CGGCTCCCTG CCCCTCCAGA 1440

TTGCCTGGTG ATCTATTTTG TTTCCTTTTG TGTTTCTTTT TCTGTTTTGA GTGTCTTTCT 1500

TTGCAGGTTT CTGTAGCCGG AAGATCTCCG TTCCGCTCCC AGCGGCTCCA GTGTAAATTC 1560

CCCTTCCCCC TGGGGAAATG CACTACCTTG TTTTGGGGGG TTTAGGGGTG TTTTTGTTTT 1620

TCAGTTGTTT TGTTTTTTTG TTTTTTTNTT TTTCCTTTGC CTTTTTTCCC TTTTATTTGG 1680

AGGGAATGGG AGGAAGTGGG AACAGGGAGG TGGGAGGTGG ATTTTGTTTA TTTTTTTAGC 1740

TCATTTCCAG GGGTGGGAAT TTTTTTTTAA TATGTGTCAT GAATAAAGTT GTTTTTGAAA 1800

AKAAAAAAAA AAAAAAAAAA AAAAAAAAAA AAAAAAAAAA AAAAAAAAAA AAAAAAAAAA 1860

AAAA 1864

(2) INFORMATION FOR SEQ ID NO: 131:

(i) SEQUENCE CHARACTERISTICS:
(A) LENGTH: 2041 base pairs
(B) TYPE: nucleic acid
(C) STRANDEDNESS: double
(D) TOPOLOGY: linear

(xi) SEQUENCE DESCRIPTION: SEQ ID NO: 131:

GGCACGAGCG CGCGGCAGGG CCCTGGACCC GCGCGGCTCC CGGGGATGGT GAGCAAGGCG 60

CTGCTGCGCC TCGTGTCTGC CGTCAACCGC AGGAGGATGA AGCTGCTGCT GGGCATCGCC 120

TTGCTGGCCT ACGTCGCCTC TGTTTGGGGC AACTTCGTTA ATATGAGGTC TATCCAGGAA 180

AATGGTGAAC TAAAAATTGA AAGCAAGATT GAAGAGATGG TTGAACCACT AAGAGAGAAA 240

ATCAGAGATT TAGAAAAAAG CTTTACCCAG AAATACCCAC CAGTAAAGTT TTTATCAGAA 300

AAGGATCGGA AAAGAATTTT GATAACAGGA GGCGCAGGGT TCGTGGGCTC CCATCTAACT 360

GACAAACTCA TGATGGACGG CCACGAGGTG ACCGTGGTGG ACAATTTCTT CACGGGCAGG 420

AAGAGAAACG TGGAGCACTG GATCGGACAT GAGAACTTCG AGTTGATTAA CCACGACGTG 480

TGGAGCCCCT CTACATCGAG GTTGACCAGA TATACCATCT GGCATCTCCA GCCTCCCCTC 540

CAAACTACAT GTATAATCCT ATCAAGACAT TAAAGACCAA TACGATTGGG ACATTAAACA 600

TGTTGGGGCT GGCAAAACGA GTCGGTGCCC GTCTGCTCCT GGCCTCCACA TCGGAGGTGT 660

ATGGAGATCC TGAAGTCCAC CCTCAAAGTG AGGATTACTG GGGCCACGTG AATCCAATAG 720

GACCTCGGGC CTGCTACGAT GAAGGCAAAC GTGTTGCAGA GACCATGTGC TATGCCTACA 780

TGAAGCAGGA AGGCGTGGAA GTGCGAGTGG CCAGAATCTT CAACACCTTT GGGCCACGCA 840

TGCACATGAA CGATGGGCGA GTAGTCAGCA ACTTCATCCT GCAGGCGCTC CAGGGGGAGC 900

CACTCACGGT ATACGGATCC GGGTCTCAGA CAAGGGCGTT CCAGTACGTC AGCGATCTAG 960

TGAATGGCCT CGTGGCTCTC ATGAACAGCA ACGTCAGCAG CCCGGTCAAC CTGGGGAACC 1020

CAGAAGAACA CACAATCCTA GAATTTGCTC AGTTAATTAA AAACCTTGTT GGTAGCGGAA 1080

GTGAAATTCA GTTTCTCTCC GAAGCCCAGG ATGACCCACA GAAAAGAAAA CCAGACATCA 1140

AAAAAGCAAA GCTGATGCTG GGGTGGGAGC CCGTGGTCCC GCTGGAGGAA GGTTTAAACA 1200

AAGCAATTCA CTACTTCCGT AAAGAACTCG AGTACCAGGC AAATAATCAG TACATCCCCA 1260

AACCAAAGCC TGCCAGAATA AAGAAAGGAC GGACTCGCCA CAGCTGAACT CCTCACTTTT 1320

AGGACACAAG ACTACCATTG TACACTTGAT GGGATGTATT TTTGGCTTTT TTTTGTTGTC 1380

GTTTAAAGAA AGACTTTAAC AGGTGTCATG AAGAACAAAC TGGAATTTCA TTCTGAAGCT 1440

TGCTTTAATG AAATGGATGT GCCTAAAAGC TCCCCTCAAA AAACTGCAGA TTTTGCCTTG 1500

CACTTTTTGA ATCTCTCTTT TTATGTAAAA TAGCGTAGAT GCATCTCTGC GTATTTTCAA 1560

GTTTTTTTAT CTTGCTGTGA GAGCATATGT TGTGACTGTC GTTGACAGTT TTATTTACTG 1620

GTTTCTTTGT GAAGCTGAAA AGGAACATTA AGCGGGACAA AAAATGCCGA TTTTATTTAT 1680

AAAAGTGGGT ACTTAATAAA TGAGTCGTTA TACTATGCAT AAAGAAAAAT CCTAGCAGTA 1740

TTGTCAGGTG GTGGTGCGCC GGCATTGATT TTAGGGCAGA TAAAAGAATT CTGTGTGAGA 1800

GCTTTATGTT TCTCTTTTAA TTCAGAGTTT TTCCAAGGTC TACTTTTGAG TTGCAAACTT 1860

GACTTTGAAA TATTCCTGTT GGTCATGATC AAGGATATTT GAAATCACTA CTGTGTTTTG 1920

CTGCGTATCT GGGGCGGGGG CAGGTTGGGG GGCACAAAGT TAACATATTC TTGGTTAACC 1980

ATGGTTAAAT ATGCTATTTT AATAAAATAT TGAAACTCAC CAAAAAAAAA AAAAAAAAAA 2040

A 2041

(2) INFORMATION FOR SEQ ID NO: 132:

(i) SEQUENCE CHARACTERISTICS:
(A) LENGTH: 2012 base pairs
(B) TYPE: nucleic acid
(C) STRANDEDNESS: double
(D) TOPOLOGY: linear

(xi) SEQUENCE DESCRIPTION: SEQ ID NO: 132:

TACCAAGCTG CAAGAATCTA CTATATCATG GCAGAAGAAG TAGAGTGGGA CTATTGCCCT 60

GACCGGAGCT GGGAACGGGA ATGGCACAAC CAGTCTGAGA AGGACAGTTA TGGTTACATT 120

TTCCTGAGCA ACAAGGATGG GCTCCTGGGT TCCAGATACA AGAAAGCTGT ATTCAGGGAA 180

TACACTGATG GTACATTCAG GNTCCCTCGG CCAAGGACTG GACCAGAAGA ACACTTGGGA 240

ATCTTGGGTC CACTTATCAA AGGTGAAGTT GGTGATATCC TGACTGTGGT ATTCAAGAAT 300

AATGCCAGCC GCCCCTACTC TGTGCATGCT CATGGAGTGC TAGAATCTAC TACTGTCTGG 360

CCACTGGCTG CTGAGCCTGG TGAGGTGGTC ACTTATCAGT GGAACATCCC AGAGAGGTCT 420

GGCCCTGGGC CAATGACTCT GCTTGTGTTT CCTGGATCTA TTATTCTGCA GTGGATCCCA 480

TCAAGGACAT GTATAGTGGC CTGGTGGGGC CCTTGGCTAT CTGCCAAAAG GGCATCCTGG 540

NAGCCCCATG GAGGACGGAN TGACATGGAT CGGGAATTTG CATTGTTGTT CTTGATTTTT 600

GATGAAAATA AGTCTTGGTA TTTGGAGGAA AATGTGGCAA CCCATGGGTC CCAGGATCCA 660

GGCAGTATTA ACCTACAGGA TGAAACTTTC TTGGAGAGCA ATAAAATGCA TGCAATCAAT 720

GGGAAACTCT ATGCCAACCT TAGGGGTCTT ACCATGTACC AAGGAGAACG AGTGGCCTGG 780

TACATGCTGG CCATGGGCCA AGATGTGGAT CTACACACCA TCCACTTTCA TGCAGAGAGC 840

TTCCTCTATC GGAATGGCGA GAACTACCGG GCAGATGTGG TGGATCTGTT CCCAGGGACT 900

TTTGAGGTTG TGGAGATGGT GGCCAGCAAC CCTGGGACAT GGCTGATGCA CTGCCATGTG 960

ACTGACCATG TCCATGCTGG CATGGAGACC CTCTTCACTG TTTTTTCTCG AACAGAACAC 1020

TTAAGCCCTC TCACCGTCAT CACCAAAGAG ACTGAAAAAG CAGTGCCCCC CAGAGACATT 1080

GAAGAAGGCA ATGTGAAGAT GCTGGGCATG CAGATCCCCA TAAAGAATGT TGAGATGCTG 1140

GCCTCTGTTT TGGTTGCCAT TAGTGTCACC CTTCTGCTCG TTGTTCTGGC TCTTGGTGGA 1200

GTGGTTTGGT ACCAACATCG ACAGAGAAAG CTACGACGCA ATAGGAGGTC CATCCTGGAT 1260

GACAGCTTCA AGCTTCTGTC TTTCAAACAG TAACATCTGG AGCCTGGAGA TATCCTCAGG 1320

AAGCACATCT GTAGTGCACT CCCAGCAGGC CATGGACTAG TCACTAACCC CACACTCAAA 1380

GGGGCATGGG TGGTGGAGAA GCAGAAGGAG CAATCAAGCT TATCTGGATA TTTCTTTCTT 1440

TATTTATTTT ACATGGAAAT AATATGATTT CACTTTTTCT TTAGTTTCTT TGCTCTACGT 1500

GGGCACCTGG CACTAAGGGA GTACCTTATT ATCCTACATC GCAAATTTCA ACAGCTACAT 1560

TATATTTCCT TCTGACACTT GGAAGGTATT GAAATTTCTA GAAATGTATC CTTCTCACAA 1620

AGTAGAGACC AAGAGAAAAA CTCATTGATT GGGTTTCTAC TTCTTTCAAG GACTCAGGAA 1680

ATTTCACTTT GAACTGAGGC CAAGTGAGCT GTTAAGATAA CCCACACTTA AACTAAAGGC 1740

TAAGAATATA GGCTTGATGG GAAATTGAAG GTAGGCTGAG TATTGGGAAT CCAAATTGAA 1800

TTTTGATTCT CCTTGGCAGT GAACTACTTT GAAGAAGTGG TCAATGGGTT GTTGCTGCCA 1860

TGAGCATGTA CAACCTCTGG AGCTAGAAGC TCCTCAGGAA AGCCAGTTCT CCAAGTTCTT 1920

AACCTGTGGC ACTGAAAGGA ATGTTGAGTT ACCTCTTCAT GTTTTAGACA GCAAACCCTA 1980

TCCATTAAAG TACTTGTTAG AACACTGAAA AA 2012

(2) INFORMATION FOR SEQ ID NO: 133:

(i) SEQUENCE CHARACTERISTICS:
(A) LENGTH: 1669 base pairs
(B) TYPE: nucleic acid
(C) STRANDEDNESS: double
(D) TOPOLOGY: linear

(xi) SEQUENCE DESCRIPTION: SEQ ID NO: 133:

GAGCAGTATT TTAACCAACT TGTATTACAG ATGTTACAGT TCATGTTAGG AAGTCAGAAA 60

AGACTTTGTT TGTCTTTGTT CTGCTGATGT GAGTCATGTT TTGTGGGGTC TTCCATGGCA 120

CATTTACCTG TTGCTCCGTC CAGATGTTGA GGGCCAGTCT AGGCTGACAC ATCCTACCCG 180

AGGACAAGCC TGTTCTCCAT TTCTTCACTC TCCCCTCCCC ATATAGCAAC TCTCCCAGGT 240

TTAGATTACC GTTTTCGACG ACAGATTAAC CAAAAATGCC CCACACAGGT TTTATTACTG 300

TTATATACTA TACTTTTAAC AGTACAGACC CTAAATTTTA TTATTTGTTG CTCCCCCAAT 360

CTGATACCAA ATGTTTAAAG TTGTTTGAAA TCCAAACATG GTAGTGTTCA TGGGTAAATA 420

TTTTCTAGGC TATGTAAGAG TTAGCAGCCC ATAGCATAGA AGTAATCAAG TAGCATCTGA 480

GACTGTTGGA GGCACTAGGG CCTCTCTGGG CCTAACAGCC TCACTTCCCC AGCCTCACCT 540

TGCTGTCCTC TGACACTGCC ATCAGGGCTG TTAGTGGCAC CTGTATGAGG CCAAGTGTGC 600

GTCCAGGGGA ACAGCACAGG TTAATGCGTC TCCCTAGAAC TCATGAAGTC AGTTTAATTC 660

ATGCATGAAC ATGAGTTCAT TTTATGTTTT ATATAGCTTT CTTAGACATA CCAAACCATC 720

ATTCATAAAT CAGATAAATT ATTCAGTTTT TGTGTTTAGA AAGCTAAGTA TGTGTAGCTG 780

GAAACAAAAA TGAGCGTGTT TTCTCTCCTG TTAATCTAGA GTGTGCAGTT ACACATGTGT 840

GGATAATTTC ATGTTCCAGG GGCGCTTGGC ATCTCCCATG GACTGATTCC CAGGAAGAAA 900

AGCCCAAAGG GAAACCCACG ATTCCTTTCG AGTAGATGTG GGAAAGAGCC CATTGGAGGA 960

TATGAGGTCC TGTGAAATTC AGTTGTGTGT GTGGCTCCTT GTTAGCAGTC ATGTTGACAT 1020

GGTGTTAGGA GGCTCCCCAT CCACCCTTTA CATGATGTAG GGACCAGTGT CTTGTGAGAT 1080

TAACCTTGGG ACACAGTGGG TTAGCCTGGA GAAAATGAGA GGCCCTGCCT GGACCCAGGG 1140

AGAGGAGCCA GTGACACAGG CAGAGCGGTG CAGCCCTCCT TCCCTTCCAT TTGGAGGAGG 1200

TGGTGCCAGG AGCCTGCCCG CTTACCTCTG CTGAAGCATA AGTGGACTTT GCTTTTGGGG 1260

CTTATCTCTG ATACATGCTG GAGCCCTGCC TCTCCACTGC TAGATGGAAC CTGGAATCTC 1320

TCATCTACCT CTTAGTCTGT CAGTTTCTAC GTGTGAGAAG CAAGCTTGTG GGCCAGTGTC 1380

CTTGTACATG CTGTAGCACT TAAAAAATAA TTCCAGGGTT CCCTGGAAAA CCAGTCCCAG 1440

GGTTCCTATG ATCTGTAGTT TCTACCTGGA TTATAACTGG TTTTGGGTAC CTGAATTTTG 1500

ATTGGTTAGC CTTAATTATA GTCTGGCGTG ATCATGTAGA ATCTTTTCTG GTGAAGAGAT 1560

CATAAAGTTC TATCAAGGAG TTCTATCAAG GCATCCATGT CAGTGGTGCT ATGCTGGTTA 1620

CAACTTGAGA TTTTTGAAAT AAAAAATTTG TCATAAAAAA AAAAAAAAA 1669

(2) INFORMATION FOR SEQ ID NO: 134:

(i) SEQUENCE CHARACTERISTICS:
(A) LENGTH: 1565 base pairs
(B) TYPE: nucleic acid
(C) STRANDEDNESS: double
(D) TOPOLOGY: linear

(xi) SEQUENCE DESCRIPTION: SEQ ID NO: 134:

CACTTTTGCT ATATAACCTA AGTGATAACC CTCTTTTAGT TACCTGCCAA ACTCTGGNCT 60

TGGTTTATAT TGCAGTTAAC ACAGTTACAA AGCTGTAATG GTGTCTTTTT TTCCTTTGTA 120

ACGGAATGTG TAAATCAAAG TATATACATT GTGTGGTGTT CCTGTTTCTG GAGTTTCATG 180

AGGATTTACA CATGGCATTC AGTGTTCTGT ATAGATCTGC CTACCTTTGT GAATTCATCT 240

GTTAACCCCT CTTCCTTTGA GAGAGCACCG GCGATGGTGG TTAACTCCTT GTGTTTTCTC 300

TCTCTCCTAC TGGTTATTCT TGAATTAAGC ACAGACTCGT CAGCTCGGTT GCTTTATCAT 360

GAATAATGTG TGTGACCTTG CAGTTCTTCC ACAGTTCAGC AAACAAGTGC TAGCTTCACT 420

GACCAAAAAT TAAGGAAGGA AAACACAGTT TTTAAAACGA TCCATCTTTT AACAGCCGAA 480

ACCGATGTGT CTATGGTGCT GCACCTTGCT GTTGTACTTC TGAAATCAGA CGTGTGTGAA 540

CGATCATTTC TGACTTAACC GTGAGATGCT CACGAGTACC CTTCCTGTTG TTTTGTTAGC 600

ATTGAAATCG AGACTATTTA TTTGGAATAT ATACAACAGT GTTTTTCCAC TGTATTTCAT 660

TTGCAAAAGT TGAGAACTGC TTTCTCTACC TTTTGCAAAA TAATTGATAT TCCATATTGG 720

ATTCTCAAAG ACTTCGATAT GGTGAACCTA TTAAACCTAG AAATTGTATT CATCCTTTCA 780

TGACTGTGGC CTGAGTTCCC CAGCCCCTCT CCTCCTTTTT TTTAGATGAG ATTTAGCACA 840

CTCTCAGTTA TTTAAACATG CAACATTTCT TGAGTATGTA TGTTGAGGCC ATCTGAGCTC 900

ATAGCTGATT CAGTAACCAG TTTCATGCTG TGTCATTCAC ACTCACTACT TAATACTGCC 960

ATGGTGAAAA TGTGGAGGAA AAATGTATCC ATGTGTGTCT GGGAAGCATA TACACTTGTA 1020

CATTTTTTAA TACTCTGATT CTGTAACATT TCTGAGTTTT GTTTTGTTTT ACAGNAAAAA 1080

AAAAAAAAGT GATAAAGCAA TCAGAAGACC AAGAGGTTTA CTATTGATGC TTAGGGTCGT 1140

CTGACCTTGG CTGGCCAATA GACCTACACG GCCAAATTAA TTTACGAGAG TAATAATTTT 1200

TCAAAAGCCA ATTTTTTTTC TGTATTTTCT GTATGAAACT GCCAATATCA TGAATAGAAA 1260

GGGAGAACCA TAAAGGAGAA AGAACGTGAT GTTCTGTTAT GTTCATGTAA ACCTAAAGAA 1320

ACAGTGTGGA GGCAGGCGCG ATCAGCCGAA CTCTAGGGAC TTGGTGTTGC TTGGAAGGCA 1380

TCCATACCTG CATTTTGCAT TCTTCGTATG TAATCATATT GCCAAAGACA AACTATTTCA 1440

TCATTTATTG TAAATAACAC TTTTCCCCAG ACCTACCATA AAGTTTCTGT GATGTATTGT 1500

CTTCCAGTTG CAATAAAAAT TACTGAGTTG CATCAATTGA AGAAAAAAAA AAAAAAAAAA 1560

CTCGA 1565

(2) INFORMATION FOR SEQ ID NO: 135:

(i) SEQUENCE CHARACTERISTICS:
(A) LENGTH: 2007 base pairs
(B) TYPE: nucleic acid
(C) STRANDEDNESS: double
(D) TOPOLOGY: linear

(xi) SEQUENCE DESCRIPTION: SEQ ID NO: 135:

TCTAAAAGCC CCCTTATACC CCACTTTGTG CAGCAAAGAT CCCCGTGCAG GTCACAGCCT 60

GATTTGTGGC CAGGCTGGAC AAATTCCTGA GGCACAACTT GGCTTCAGTT CAGATTTCAA 120

GCTGTGTTGG TGTTGGGACC AGCAGAAGGC AAACGTCCAG CCAACACACA GGACTGTAAG 180

AGGACTCTGA GCTACGTGCC CTGTGAAGAC CCCCAGGCTT TGTCATAGGA GGTCGTTCAG 240

CTTCCCCAAA GTCAGAGGTG ATTTGATTTG GGGAAGACTG AATATTCACA CCTAAGTCGT 300

GAGCATATCC TGAGTTTTAC TTCCTTATGG CTTGCCCTCC AAGTTCTCTC TCTCATACAC 360

ACACACACCC TTGCTCCAGA ATCACCAGAC ACCTCCATGG CTCCAGCTAT GGGAACAGCT 420

GCATTGGGGC TGCCTTTCTG TTTGGCTTAG GAACTTCTGT GCTTCTTGTG GCTCCACTCG 480

CGAGGCAGCT CGGAGGTGTG GACTCCGATT GGGCTGCAGG CAGCTCTGGG ACGGCACAGG 540

GCGGGCGCTC TGATCAGCTC GTGTAAAACA CACCGTCTTC TTGGCCTCCT GGCAGTTCTT 600

TCTGCGAATA GTCCTCTCCC TGGCCAGTTG AATGGGGGAA GCTGCTGGCA CAGGAAGGAG 660

AGGCGATCCC GGCTGAGGCT TAGGAAATTG CTGGAGCCGG CTCCAAGCAG ATAATTCACT 720

GGGGAGGTTT TCAGAGTCAA ACATCATTCT GCCTGTKTTG GGGGCCAGGT GTGTCACACA 780

AGCATCTCAA AGTCAAAAGC CATCTGGGGC TGCTGCTTCT CTTTCTCAGG CTCTGGGGAA 840

AGGAATCTCC CTCTCCTCTC ACTTGATTCC AAGTGTGGTT GAATTGTCTG GAGCACTGGG 900

ACTTTTTTTC TCTTTTCCTT GATGGACCAA CAGTGCAAAT GCAATCTCGC CATTTAACTT 960

TCAGGTCGAT TTCCTTTCCT GATCAGACAT CTTTGTGCCC CCTTTAGGAA GGAAAAGAAT 1020

ACACCTACGA TGTGCCAGGC ACTGTGTTAG GCGCTTTTAT ATAGATCCTC GTTAGGATGA 1080

GACTAAGGGA TGAGGACATC TCTTTATAAA AGGCCCCTAA GTAATGGATA AACAGAAACA 1140

CTTAGAGGTG AGAAGGTCTG TCTTCAAGAT CCAAGGTAAG ATTGCCTTCA GTCTGATGTT 1200

TGTTCTCAAG GACTTATCCC CTACAATATT CTCCCACTCC ATACTTCTCC TTCTACCCCA 1260

CCATGTGCTC CCGTGCACTC CTCAGATGGT CAGAGGGGTA ACCCAAGTCC TTAGAGAATT 1320

TGGGGACCAA TAGAATATGT GATGTGTGAA TTTTCTTTAA AAAACTTAAG GAGTCTTTGC 1380

TACCTTCTGC TTGTTGAGTT GTTTTGGCAT TCATATTAAA AGCCAGCATC TCACTATTTA 1440

TTGACAGGTT GGGCTGTGTG TGTGCGCATG TGTGTATACA TTTCCAGGCG TGCCTGTGTC 1500

CTGTAGCTTT TTAAAAGGAA ACCCAGTCAT CCCACTATGA ATCTGGCATC TTCTTATGCT 1560

TCTAGTGTTT TGGCCATACA TCAACCAAGG GGTTTAATTT ATCCAATGCT TGACGACATG 1620

TTCAGGAGGG GCTGGATCAA ATTTTGAGAG GGTTATGGGA AAGGGAGGGG GAGAAGAAAT 1680

TGACATTTAT TTTATTATTT ATTTTAAATG TTTACATCTT CTTTATGTTG TATCAAGCCT 1740

GAATAGAAAC TGATAGCATT AAAATACTCC GTTCCTCTCT CTCTTCTCGC TTCCTTTTTT 1800

TTTTTTTTTA AATTTAGGAT AACACATTTT TGTTTCTAAA GTGATTTGTG ATTTGTGCTG 1860

TATAAACTGT ATAAAAGGTT CTGTTTTTAA AGGTGGATTT TCATTCCTCT GGGGACAGTG 1920

GTCGCCAAGA CATCTACATT GTAAGAGAAC ACAGTGGAAG ATCCTGTCCT GATTCTCAAA 1980

AATTATTTTC TCTGTATGAT TAAAAGT 2007

(2) INFORMATION FOR SEQ ID NO: 136:

(i) SEQUENCE CHARACTERISTICS:
(A) LENGTH: 1291 base pairs
(B) TYPE: nucleic acid
(C) STRANDEDNESS: double
(D) TOPOLOGY: linear

(xi) SEQUENCE DESCRIPTION: SEQ ID NO: 136:

CTTTTAACCC TCCCCCTTCA CACACATACA TATCAGGTTG TTTTCTAGTT AAAAACCCAA 60

GTAGCTCAGA TTCTACTTTA ATGTCAGTGC AGATTTGCAT TGAATCATGC CATTATGTTT 120

TTTCTCATTT TTATGCTGTT GGGTCTTAGT TTTTAAATTG ATATAAAGAA CTCAGCAATG 180

GTTTTATTTT CTACTCATAC TTAGGGTTTA GGAAACACTA CCACTAGTTA TCATTTAATC 240

AACTTCAATG GTCTACTGAA ACAAAAATGG TAACTTTTCA TTAGTGGATT ATTTAGAGTT 300

ATAGTAGTTG TTTCCAGAAA ACACTTCCTC ACAATTGTAC TTCCCAATCA AATCATGTGA 360

TCATACAGTT ATTCCCATGA AAGGCAGAAT GTTTGTTTCA AAATTAATCT AGTTTTCTGT 420

ACATTTAAAT TTGAGAAGGT GACAACTGGC TCTTTTCCAG TCTTCCTTCA TGTCAGTTTT 480

CTGATAGACC ACTATTGGCA AACAGTATCT GTCAACTACC AAATGTGTAA AATTTTCTGT 540

ATTTCACTTT GTCTTATTTG TAAATAGTGA ACTAAAACTT TTGGCAGATC AGCAACATTT 600

GCTGAGCCTG TTTTTTAAGC TAATGTGTAT TCTTACTAAT GTTCCTATCA AGAATGGATT 660

TGTAATATAT GCTGTCTATT TCTAATGTTC ACATTCATAT TTTGAGGTTC TATCTTATTT 720

TAATAGAGAA CAGACTTCTC AAAAAATCTT CAGAAGCAGC TTATTATTGA AATATCGAAA 780

TATTGAAATA AACCCGGTGG GTTAGATTAC TCATCTGTCC ACCAAGTGGG ACATTTGCAT 840

GGACTGGGGG CTTAAAGGAC TTAGAAGAGA CCTGTAAGTA AATCCTGAAA ATGAGCCAAT 900

CCCCACTTGA ATGGTTACTG GAGTAAACCC ACCTTTACCA CCCCAATTAC AGCACCCGAG 960

GCCGATAAAC CAACTTGGCT CTGGTTCATT TTTCTTTTCT TCATTTGTGA TGCTCAGATT 1020

CAAAATGTGT GTTCTACACT GTTACAGGCT TCTCTTTTGT TTGATTAAAG ATTTTAGTCC 1080

TACTTTTGTA TGGACACATT AGAATATTCA GAGACCAAAA TAGAAGAATT TGCTGTTAGA 1140

TATTTTTCAG AAGTCAGCAG ATTTGTGGCA AATCATTTAT TTGCCTTTTT AAAAATTCAT 1200

TTAAGCAGTT CAGAGAGTAG ACTACTCAGA AAATTATTTC ACGTAATTGT CTAAGAGGTC 1260

AATATTTTTT AATGCATATT GAATCAAATA A 1291

(2) INFORMATION FOR SEQ ID NO: 137:

(i) SEQUENCE CHARACTERISTICS:
(A) LENGTH: 1906 base pairs
(B) TYPE: nucleic acid
(C) STRANDEDNESS: double
(D) TOPOLOGY: linear

(xi) SEQUENCE DESCRIPTION: SEQ ID NO: 137:

GGCACGAGGA CCTACTTTTG TAACAGACCA TGGTTGTGTC CAAGGTAAAA CCACAGTGAT 60

ATTTTTGGAT GCTTTGTCTG CAATCTTGAC TTGTTTTTGC AGTATCATTA TTCAGACTTC 120

AAATTGTGAA TCTTTTAAAC ATCTTGATAA TTTGTTGTTG AGAGCTGTTC ATTCTAAAAT 180

GTAATGAAAT TCAGTCTAGT TCTGCTGATA AAGATCATCA GTTTTGAAAG GTTACTGATT 240

TTCCTCTTCC CTCTTAGTTT TTTACCCAAT ATATGGAGAA GAGTAATGGT CAATCTTAAC 300

ATTTTGTTTT AATTGTTTAA TAAAGCTGCT GGGCAGTGGT GCAGCATTCC TACCTAGTGT 360

CATAAAAGCA AAATACTTAC ATAGCTTTCT TAAAATATAG GAATGACATT ACATTTTTAG 420

GAGAAAGTAA GTTGCTTTGC ACCGCCTACT TAATTCCTTT CCATATATTG TGATACAAAC 480

TTTTGAATAT GGAATCTTAC TATTTGAATA GAAATGTGTA TGTATAATAT ACATACATAC 540

ATAAGCATAT ATGTGTGTGT GTGTGTGTAT ATATATATAT ATGCATGCTG TGAAACTTGA 600

CTACACAACA TAAATCACTT TTTAAATTCC AGGAACGGGT AGTCTGACAC GGTGATTATC 660

CTTTTGAGGC TGAATCCGTT ATTAACTTGT TATTTAGGTT TTACTCCCAG TAGCAAGGGA 720

TTCTAAGTTA GTTGCACTTA CATGATTATT GTGATTTAAA ACTAAGAATA AAGGCTGCAT 780

TTTCAAAGAT AAATTGGAAT TGCTGTTGGT GAAATAACAA CCAAAATACT GAATCTGATG 840

TACATACAGG TTTCTACAGG AAGAGATGGT ATAATTTACA ATTTGGAGAT TTAATAACCA 900

GGGCTACCCA GAAAAAGTGA CTTGATAACA TGGTACCAAT AAGTAAGGGA TGCTCTCTCG 960

GTTTGCTTTT GCCACTTTCA AGATTTTAAC TTCTCAGGTT ATTAATCAAA ATTATTGTAT 1020

AAGTTAGCCA ATAGAATTTT TAGGTTAAAA CAACAGATGG GGGGTTTGTG GAGTGTTTAA 1080

TGTCATGGGC ATTTTTAGTA GCATAGACCC TTTGTTCTGC ATTTGAATGT TTCGTATATT 1140

TTTGTTTCAC AGTTAATCTT CCCTCCCCAA GTTTGCTATT CAAATCAACT GCCTGAATGA 1200

CATTTCTAGT AGTCTGATGT ATTTTTCTGA GGAATAGTTT GTGATTCCAA TGCAGGTGTC 1260

TTCATTACCA TTACCTCTAC ACTGCAGAAG AAGCAAAACT CCTTTATTAG AATTACTGCA 1320

CATGTGTATG GGGAAAATAG TTCTGAAAGG CTAGAATGAT ACAAGTGAGC AAAAGTTGGT 1380

CAGCTTGGCT ATGGAGTGGT GGCAATAATC TCTAAACATT CCAAAAGACC ATGAGCTGAA 1440

CCTAAACTCC CTTGGGAATC TGGAACAAAG GAATATGAAA ATTGCCATTT GAAAACTGAC 1500

CAGCTAATCT GGACCTCAGA GATAGATCAG CCAGTGGCCC AAAGCCATTT CAAGTACAGA 1560

AATTATAGAG ACTACAGCTA AATAAATTTG AACATTAAAT ATAATTTTAC CACTTTTTGT 1620

CTTTATAAGC ATATTTGTAA ACTCAGAACT GAGCAGAAGT GACTTTACTT TCTCAAGTTT 1680

GATACTGAGT TGACTGTTCC CTTATCCCTC ACCCTTCCCC TTCCCTTTCC TAAGGCAATA 1740

GTGCACAACT TAGGTTATTT TTGCTTCCGA ATTTGAATGA AAAACTTAAT GCCATGGATT 1800

TTTTTCTTTT GCAAGACACC TGTTTATCAT CTTGTTTAAA TGTAAATGTC CCCTTATGCT 1860

TTTGAAATAA ATTTCCTTTT GTAAAAAAAA AAAAAAAAAA AAAAAA 1906

(2) INFORMATION FOR SEQ ID NO: 138:

(i) SEQUENCE CHARACTERISTICS:
(A) LENGTH: 1935 base pairs
(B) TYPE: nucleic acid
(C) STRANDEDNESS: double
(D) TOPOLOGY: linear

(xi) SEQUENCE DESCRIPTION: SEQ ID NO: 138:

TCTGAACTAA TGCTAACAGA TCCCCCTGAG GGATTCTTGA TGGGCTGAGC AGCTGGCTGG 60

AGCTAGTACT GACTGACATT CATTGTGATG AGGGCAGCTT TCTGGTACAG GATTCTAAGC 120

TCTATGTTTT ATATACATTT TCATCTGTAC TTGCACCTCA CTTTACACAA GAGGAAACTA 180

TGCAAAGTTA GCTGGATCGC TCAAGGTCAC TTAGGTAAGT TGGCAAGTCC ATGCTTCCCA 240

CTCAGCTCCT CAGGTCAGCA AGTCTACTTC TCTGCCTATT TTGTATACTC TCTTTAATAT 300

GTGCCTAGCT TTGGAAAGTC TAGAATGGGT CCCTGGTGCY TTTTTACTTT GAAGAAATCA 360

GTTTCTGCCT CTTTTTGGAA AAGAAAACAA AGTGCAATTG TTTTTTACTG GAAAGTTACC 420

CAATAGCATG AGGTGAACAG GACGTAGTTN AGGCCTTCCT GTAAACAGAA AATCATATCA 480

AAACACTATC TTCCCATCTG TTTCTCAATG CCTGCTACTT CTTGTAGATA TTTCATTTCA 540

GGAGAGCAGC AGTTAAACCC GTGGATTTTG TAGTTAGGAA CCTGGGKTCA AACCCTCTTC 600

CACTAATTGG CTATGTCTCT GGACAAGTTT TTTTTTTTTT TTTTTTTTAA ACCCTTTCTG 660

AACTTTCACT TTCTATGTCT ACCTCAAAGA ATTGTTGTGA GGCTTGAGAT AATGCATTTG 720

TAAAGGGTCT GCCAGATAGG AAGATGCTAG TTATGGATTT ACAAGGTTGT TAAGGCTGTA 780

AGAGTCTAAA ACCTACAGTG AATCACAATG CATTTACCCC CACTGACTTG GACATAAGTG 840

AAAACTAGCC AGAAGTCTCT TTTTCAAATT ACTTACAGGT TATTCAATAT AAAATTTTTG 900

TAATGGATAA TCTTATTTAT CTAAACTAAA GCTTCCTGTT TATACACACT CCTGTTATTC 960

TGGGATAAGA TAAATGACCA CAGTACCTTA ATTTCTAGGT GGGTGCCTGT GATGGTTCAT 1020

TGTAGGTAAG GACATTTTCT YTTTTTCAGC AGCTGTGTAG GTCCAGAGCC TCTGGGAGAG 1080

GAGGGGGGTA GCATGCACCC AGCAGGGGAC TGAACTGGGA AACTCAAGGT TCTTTTTACT 1140

GTGGGGTAGT GAGCTGCCTT TCTGTGATCG GTTTCCCTAG GGATGTTGCT GTTCCCCTCC 1200

TTGCTATTCG CAGCTACATA CAACGTGGCC AACCCCAGTA GGCTGATCCT ATATATGATC 1260

AGTGCTGGTG CTGACTCTCA ATAGCCCCAC CCAAGCTGGC TATAGGTTTA CAGATACATT 1320

AATTAGGCAA CCTAAAATAT TGATGCTGGT GTTGGTGTGA CATAATGCTA TGGCCAGAAC 1380

TGAAACTTAG AGTTATAATT CATGTATTAG GGTTCTCCAG AGGGACAGAA TTAGTAGGAT 1440

ATATGTATAT ATGAAAGGGA GGTTATTAGG GAGAACTGGC TCCCACAGTT AGAAGGCGAA 1500

GTCGCACAAT AGGCCGTCTG CAAGCTGGGT TAGAGAGAAG CCAGTAGTGG CTCAGCCTGA 1560

GTTCAAAAAC CTCAAAACTG GGGAAGCTGA CAGTGCAGCC AGCCTTCAGT CTGTGGCCAA 1620

AGGCCAAGAG CCCCTGGCAA CCAACCCACT GGTGCAAGTC CTAGATTCCA AAGGCTGAAG 1680

AACCTGGAGT CTGATGTCCA AGAGCAGGAA GAGTGGAAGA AAGCCAGAAG ACTCAGCAAA 1740

CAAGGTAGAC AGTGTCTACC ACCAYAGTGG CCATACCAAA GAGGCTACCG ATTCCTTCCT 1800

GCTACCTGGA TCCCTGAAGT TGCCCTGGTC TCTGCACCTT CTAAACCTAG TTCTTAAGAG 1860

CTTTCCATTA CATGAGCTGT CTCAAAGCCC TCCAATWAAT TCTCAGTGTA AGYTTCAAAA 1920

AAAAAAAAAA AAAAA 1935

(2) INFORMATION FOR SEQ ID NO: 139:

(i) SEQUENCE CHARACTERISTICS:
(A) LENGTH: 1446 base pairs
(B) TYPE: nucleic acid
(C) STRANDEDNESS: double
(D) TOPOLOGY: linear

(xi) SEQUENCE DESCRIPTION: SEQ ID NO: 139:

NGCCCCCTTG GCACAAGTCA GATGAAGCAC GTTCTGCCGG GGAGGCCCTC AMCTTCCAGA 60

GAGGACAGAC ACAGATTTCC TGCTGGGGGA GGGAGGAGTC CACGCATCCT GATGCTGCCT 120

GGAAGCTTAT TTTCCCGTGG CCAGGATGCA TTTCTCTGAG TGGAAACAGG TTCTTGCATG 180

TGGATGTGTG TTTCCCCAGG CAGACGGCCC CTCTYTTCCC AGCACTTCCC TGCCTCCCCC 240

AGGCCTCAGG CCAGCACCCA GTTCCTCCTC ACATGGCAGG TGAGCACAGA CTTCTAGTTG 300

GCAGGAGCTG AGGAGGGTGA ACAAACCCCG AGGGAGGCCC GGCCCTTGCT CCCGAGTTGG 360

GGGGAGGGGG TGTGGCAACG TGCCCCCCGC AGAGGCCACG CATGTTTGAC CAAAGCCCTC 420

ATTGTGGTCC GAGGACAGCC TTTTCCCCAG GCCTCARAGC ATTGCTCATC CGTGCCAAAC 480

TGGGTAGGTG GATTTGAGCG GAAAGACTCC CAAAATGTGC CAAGAATTTC CCRGTCCCAG 540

GCAGGGCAGG GGAAACTAAG GGCAAGCAGG ATACAGGGCG AGGGATGTGG CAGGTGAGGG 600

GGCTCCCGCC TGTGCCCCTT CTCCTCACCA TGTCTCCCCC ACCCTGCCTC AGTTCTCCGT 660

TCCCCTTCAT CTCCGTCCCC CTCTTTGAAG CTGTCCCCAT CTCAGTGTCA GACCAGCCTT 720

CTCCTCAKCT GACCACCCTC CTCTGACCSA CGCCCCCTCC TTGTCTGAAA AAAGGAGCCT 780

TGAATGGTGG AGGGAGGCAG TGGGGAGAAA GGTCTCACCG GACAGGTTGG GAGAATGAGG 840

TCAGCGGTGC TGGGGAACAG ATGGAGGGGG CAGTGGGGAC AGGGCTTGGG CAGACACCAG 900

CAGGAATAAT TTGAAATGTG TGAGGTGACT CCCCGGAGGC CTTGGGCTTG GGCATTTGGG 960

AAAAGAATGA TGTCTGGAAG GGCTTAAGGG ACACAGTGGA CGAGGGGAGA GTCCTCATCT 1020

GCTGGCATTT TGTGGGGTGT TAGTGCCAAA CTTGAATAGG GGCTGGGGTG CTGTCTTCCA 1080

CTGACACCCA AATCCAGAAT CCCTGGTCTT GAGTCCCCAG AACTTTGCCT CTTGACTGTC 1140

CCTTCTCTTC CTACCTCCAT CCATGGAAAA TTAGTTATTT TCTGATCCTT TCCCCTGCCT 1200

GGTCTAGCTC CTCTCCAAAC AGCCATGCCC TCCAAATGCT AGAGACCTGG GCCCTGAACC 1260

CTGTAGACAG ATGCCCTCAG AATTGGGGCA TGGGAGGGGG GSTGGGGGAC CCCATGATTC 1320

AGCCACGGAC TCCAATGCCC AGCTCCTCTC CCCAAAACAA TCCCGACAAT CCCTTATCCC 1380

TACCCCAACC CTTTGCGGCT CTGTACACAT TTTTAAACCT GGCAAAAGAT GAAGAGAATA 1440

TTGTAA                                                              1446

(2) INFORMATION FOR SEQ ID NO: 140:

(i) SEQUENCE CHARACTERISTICS:
(A) LENGTH: 1109 base pairs
(B) TYPE: nucleic acid
(C) STRANDEDNESS: double
(D) TOPOLOGY: linear

(xi) SEQUENCE DESCRIPTION: SEQ ID NO: 140:

TTTTTTTTTT TTTGATATGA AATTGTCTTT CTCCATTGCA GAAATAAGCT AGGGAAACAC      60

TAACCCAAAA ACTTTCTGTA GAGCTGTTCC TTTGGAGGCA GCATCACTTA TTGGCAGTAA     120

AGACTCAGTA TAAAAGCACC AGCATCCCTA CTTGGGTGAT GGGGATTAAT TTTATAGCAT     180

TCCATTTTCC TAGTGCCACA TGTGAAATTG GATTTTGATG ATCTTAATCT ATATTCTACC     240

CTTATAATAA AAGATCAAAA GATATATCTC CTATGAACAG ATTGGAGATA GGAGATGAAA     300

AGTTGGGAGG ATGTCTTTAT TCTAATGTGA GGGTAGGGAA AATGTGGATA ACATTACTGG     360

GGTGARGGAG GCATTGTTCT TTAGTTGGAG TTCTCATTTT TATTCTCCAG TACTGACTTG     420

TGGGGAAAGC ATACTTTTTC ACTGCCAGGT ACTGAATGCA GAGGCTCAGT GAAGTATATA     480

TGTGGGAAGT GCATGCATTT CGTTTATTAG CAAACATAGC TGGATTAAGA CAAAGTTGTT     540

GGTTTGGAAA GGGGTTAAAG CCTTAAGTGA ACAAATCTAG CTAACAGTGA ATGAACTAGG     600

TAATATAACT TGCATATTTT TAATTTCCTT TGGTTAAAGG TCCCCCATAC TTCTCTGTTC     660

GGAGACATGA GAAGTATGAT TACTTCAGTG TTAGTTTTCT TAATTTTTTT TTTCCCCTAT     720

TTGTCCCTTG TCACTTTGTT GCAAGCTAGA AATCTGTGGG TTATACATAG GGCAGCTCTT     780

TGTGAAAGTG GTTTATTCCA CTGGAGAAAG GGGATTGAAA ATCAGTTAGA ACCAATGTAT     840

TTCTTGCCCC ACGGAACACT ATTCCTATAA GATAGCTGAA AGAAGCTGCT GTGAGGAGCT     900

CAGCTCCAAA CACAGGATCA GCACCTTGTA TAGGAATTCC CATGAATTAT GACTTCTCAT     960

TCTGTTTTAT CAGAGTGCAT ATATGTCCTA CTTCAGGAAA AGTAAAACAG TCATTTACGA    1020

AAGAAAGTCA ATCTGTATCC TAAGCATTTT AATAAAAAGT TAAAACAAAA AATTAAAAGG    1080

GACACTCGAG GGGGGGCCCG AAACCCAAT                                     1109

(2) INFORMATION FOR SEQ ID NO: 141:

(i) SEQUENCE CHARACTERISTICS:

(A) LENGTH: 497 base pairs
(B) TYPE: nucleic acid
(C) STRANDEDNESS: double
(D) TOPOLOGY: linear

(xi) SEQUENCE DESCRIPTION: SEQ ID NO: 141:

TAGGACTAAC TTAAATTCTT TTATTCATCT TTTATTTATT AAAAAATTTT ATTTCTTTGA 60

ATTTTCCTGT AATTTCCTTA RGCTCTTCTA TAAAATGTTA TATTCATGTG AACCATACCT 120

CATTATCCTT AACATTTACT CTCAAAAAGC TTTTTATTTT TATTTTTTTG AAGGTAGTTT 180

TTCTGTGTGT ACTCTGTAAC ATGATTTTGC TTTCAAATCA TTGTTGTGCC CCCATACAAA 240

ATGCCTTTTA TTTTTGAGGA TCGTGGACTT TTTAGTATGG CATGAGTGTG CTAAAAGCCA 300

GATATCTTTC CACATTCACT GGTGGCTTTG ACACCTAGTT TTTAATCTCC CATCCTTACT 360

TTAAACCCTG ACAGTGCAGT CCTCAGTCAG GGCCAGGACC GGGCTGAGGC CCTTTGTGGA 420

GATGCTGCAC CACCAGCAGA AGGCTGAGAC CTGGTTACCT GTACCTGTTC ACTTGTAATA 480

AAAAGAATTA TCTAAAA 497

(2) INFORMATION FOR SEQ ID NO: 142:

(i) SEQUENCE CHARACTERISTICS:
(A) LENGTH: 269 base pairs
(B) TYPE: nucleic acid
(C) STRANDEDNESS: double
(D) TOPOLOGY: linear

(xi) SEQUENCE DESCRIPTION: SEQ ID NO: 142:

ATGAGGCAGA GGCAAGCTGC CTGCCAACCC CCTCCCTCAA GGAATGGCCT TGCCCAGGAA 60

TGCCCACCAC ACATACCCTC TTCTTTTTTT CTAGTCAAAC TCTTGTTTAT TCCTTGGCTT 120

GCCTCCCTCC TTTCCTCCCC TCTCAACCTT TTACTTCTGG TTTCTATTTC ATGGGATTTG 180

GGGTTGAAGT TAAACTTACA ACAGTGCCGC CAACACCAAG TCTTGCAGGA AAAAAATACA 240

AAGAAATTTA ACAAAAAAAA AAAAAAAAA 269

(2) INFORMATION FOR SEQ ID NO: 143:

(i) SEQUENCE CHARACTERISTICS:
(A) LENGTH: 1269 base pairs
(B) TYPE: nucleic acid
(C) STRANDEDNESS: double
(D) TOPOLOGY: linear

(xi) SEQUENCE DESCRIPTION: SEQ ID NO: 143:

TTGATTGACT ATGGTCTCTC CGGCTACCAG GAAGAGTCTG CCGAAGTGAA GGCCATGGAC 60

TTCATCACCT CCACAGCCAT CCTGCCCCTG CTGTTCGGCT GCCTGGGCGT CTTCGGCCTC 120

TTCCGGCTGC TGCAGTGGGT GCGCGGGAAG GCCTACCTGC GGAATGCTGT GGTGGTGATC 180

ACAGGCGCCA CCTCAGGGCT GGGCAAAGAA TGTGCAAAAG TCTTCTATGC TGCGGGTGCT 240

AAACTGGTGC TCTGTGGCCG GAATGGTGGG GCCCTAGAAG AGCTCATCAG AGAACTCACC 300

GCTTCTCATG CCACCAAGGT GCAGACACAC AAGCCTTACT TGGTGACCTT CGACCTCACA 360

GACTCTGGGG CCATAGTTGC AGCAGCAGCT GAGATCCTGC AGTGCTTTGG CTATGTCGAC 420

ATACTTGTCA ACAATGCTGG GATCAGCTAC CGTGGTACCA TCATGGACAC CACAGTGGAT 480

GTGGACAAGA GGGTCATGGA GACAAACTAC TTTGGCCCAG TTGCTCTAAC GAAAGCACTC 540

CTGCCCTCCA TGATCAAGAG GAGGCAAGGC CACATTGTCG CCATCAGCAG CATCCAGGGC 600

AAGATGAGCA TTCCTTTTCG ATCAGCATAT GCAGCCTCCA AGCACGCAAC CCAGGCTTTC 660

TTTGACTGTC TGCGTGCCGA GATGGAACAG TATGAAATTG AGGTGACCGT CATCAGCCCC 720

GGCTACATCC ACACCAACCT CTCTGTAAAT GCCATCACCG CGGATGGATC TAGGTATGGA 780

GTTATGGACA CCACCACAGC CCAGGGCCGA AGCCCTGTGG AGGTGGCCCA GGATGTTCTT 840

GCTGCTGTGG GGAAGAAGAA GAAAGATGTG ATCCTGGCTG ACTTACTGCC TTCCTTGGCT 900

GTTTATCTTC GAACTCTGGC TCCTGGGCTC TTCTTCAGCC TCATGCCTCC AGGGCCAGAA 960

AAGAGCGGAA ATCCAAGAAC TCCTAGTACT CTGACCAGCC AGGGCCAGGG CAGAGAAGCA 1020

GCACTCTTAG GCTTGCTTAC TCTACAAGGG ACAGTTGCAT TTGTTGAGAC TTTAATGGAG 1080

ATTTGTCTCA CAAGTGGGAA AGACTGAAGA AACACATCTC GTGCAGATCT GCTGGCAGAG 1140

GACAATCAAA AACGACAACA AGCTTCTTCC CAGGGTGAGG GGAAACACTT AAGGAATAAA 1200

TATGGAGCTG GGGTTTAACA CTAAAAACTA GAAATAAACA TCTCAAACAG TAAAAAAAAA 1260

AAAAAAAAC 1269

(2) INFORMATION FOR SEQ ID NO: 144:

(i) SEQUENCE CHARACTERISTICS:
(A) LENGTH: 1944 base pairs
(B) TYPE: nucleic acid
(C) STRANDEDNESS: double
(D) TOPOLOGY: linear

(xi) SEQUENCE DESCRIPTION: SEQ ID NO: 144:

AAAAGGCAAA CTATAGGATA ACACAGAGCC CTTTTTGAAA ATAAATTGGC ATTGGAGTGT 60

TTTACCCTCT AGCTGTTTTA CTTAGAATGT AACATATGCT GCCTACCCAC CTCAAAATGT 120
CTGTACTGCA AGAGGGCCCT GGGCCTCTGC TTTCCATATT CACGTTTGGC CAGAGTTGTA 180
GTCCCAAAGA AGAGCATGGG TGGCAGATGG TAGGGAATTG AACTGGCCTG TGCAATGGGC 240
ATGGAGCACA AGGGGTCACA GCATGCCTCC TGCCTTACCG TGGCAGTACG GAGACAGTCC 300
AGAACATGGT CTTCTTGCCA CGGGGTGTTG TTGTCTCTGG TGGTGCTGCA TGTCTGTGGC 360
TCACCTTTAT TCTTGAAACT GAGGTTTACC TGGATCTGGC TACTGAGGCT AGAGCCCACA 420
GCAGAATGGG GTTGGGCCTG TGGCCCCCAA ACTAGGGGGT GTGGGTTCAT CACAGTGTTG 480
CCTTTTGTCT CCTAAAGATA GGGATCTACT TTTGAAGGGA ATTGTTCCTC CCAAATAAAT 540
TTGCTTTACC TTGGTCCTTT CTTTTGTGCC AGTATTCAAG TGGTATAGCT CTGAGCAGGG 600
TCACATTTGG CCAAACCTGA CACTGTCTTG CTGCATTCTC CTTTGGCAAA CATCAGGGTC 660
AGAATTCAGG ATAGCCCTTC CTAGGGCACT GGACTTTCTG GCATGGGGGC TGTGTTTGCA 720
CAAGTTATTT TCATGTTACC TGGAGAGTGT CCAGAGGCTG CTCTGAGGCT GAGGTGTGTT 780
CCCCCTTGCC TGGTTCCAGC TGTCAGAGGG ATACCATCCT AGGGTCTGGG AATCCAAGGC 840
CACGAGACTC CTTGGTTTGT GGTCCGAGAT CCTGTACTAA GGAGGGTCTG GCCAGAGGAA 900
CAGACCAGCT TTTGCACAAT GAAGCGCAAG GGAACAAGTG GTTTGCCTGG TGTCCTACCT 960
GTCCTGAACC TGGTCCTGTG GGCCATTGAA AAGTTAGATC TGTGATCTCT GGGGTTTTTG 1020
TGGCTTTGTT CAATGCTTCC ACTCTAGGGC AGGCAGAGCA GTCTATACTC TCCCAAGCCT 1080
GCTTGACCTC CAAGTAGAGC TGATACAGAG ATCTGTGAAT ATTGTGATAG AAATTCTTTG 1140
GTATTCATAC ATTTCAGCTG CAAGTCAGCA ATTTCCCAGG TACCATGTAA GCTATAAAAC 1200
AGTCATTCTT AAAGACAGAG GATAGCTGTG ACTCATGGGA TCATGAGGTC CATGGCTGGT 1260
TGCAGGTTCC CTTTTTCCTT CCTCAGGTTT TGTCTCTTCC TGTGTTGTCC CCAGCAAGGG 1320
AGAGACTGTG GGGTGGATTG GGAGAACAGA TTAGGAGTAT AGCAAATGAA CCCAGAATGG 1380
AACAGTGGGG AGCTAACTGT GAATGAGGAG AGTACCTGCT GCAGGACCTG GAGGTCAGGT 1440
GTGAATGCTG TATTGGCACA GGGAATAAAT ATCCTGGCGT CTGGAGCCTT CACCTCTCCG 1500
TCAAGTCCTT CCTGTGATAC TGCCATGGCA CAGGATCTGA GTTGCAGCTC TGCACCCTAA 1560
ATCACACCCT GGGCATTGTC TGGGCTGCAG GGCTGCCAGG TTCTGTACTT GTGTCCAGCT 1620
GTGGCCCTGG ATGCTGGAGC TGGAGGGTTT TCTGTGCTCA GACTGTAGCC TGTAGCTCTT 1680
GGCCTGTGTA GAGCCCCCTC CTGTGCCCTC AGTGGCTGTC GTTTGTTAAC ATCATCAGGA 1740
AGATGGGAAA GGTCAGGCAG AATTTTTCTG CCCTACAAAG GGTGGAAGAG AAAGGACACA 1800
GTATTTTCAT GAATTTACCA TATATCTTTG TTTTTCTTCA ACGAAAAAGT TAATTGAGGC 1860

AATGTCATCT GCTCAAAGTT GAGTGGTTTA TTCACAATAA ACTGTAAGTT TCTGATTATA 1920

AAAAAAAAAA AAAAAAAAAA AAAG 1944

(2) INFORMATION FOR SEQ ID NO: 145:

(i) SEQUENCE CHARACTERISTICS:
(A) LENGTH: 1021 base pairs
(B) TYPE: nucleic acid
(C) STRANDEDNESS: double
(D) TOPOLOGY: linear

(xi) SEQUENCE DESCRIPTION: SEQ ID NO: 145:

TCGACCCACG CGTCCGGGGT GCGCAACGGG GAGTTCCGGC TGGAGACCCG TGCTCTGGGC 60

CGGCGCCTTC ACCATGGCCT CGGCAGAGCT GGACTACACC ATCGAGATCC CGGATCAGCC 120

CTGCTGGAGC CAGAAGAACA GCCCCAGCCC AGGTGGGAAG GAGGCAGAAA CTCGGCAGCC 180

TGTGGTGATT CTYTTGGGCT GGGGTGGCTG CAAGGACAAG AACCTTGCCA AGTACAGTGC 240

CATCTACCAC AAAAGGGGCT GCATCGTAAT CCGATACACA GCCCCGTGGC ACATGGTCTT 300

CTTCTCCGAG TCACTGGGTA TCCCTTCACT TCGTGTTTTG GCCCAGAAGC TGCTCGAGCT 360

GCTCTTTGAT TATGAGATTG AGAAGGAGCC CCTGCTCTTC CATGTCTTCA GCAACGGTGG 420

CGTCATGCTG TACCGCTACG TGCTGGAGCT CCTGCAGACC CGTCGCTTCT GCCGCCTGCG 480

TGTGGTGGGC ACCATCTTTG ACAGCGCTCC TGGTGACAGC AACCTGGTAG GGGCTCTGCG 540

GGCCCTGGCA GCCATCCTGG AGCGCCGGGC CGCCATGCTG CGCCTGTTGC TGCTGGTGGC 600

CTTTGCCCTG GTGGTCGTCC TGTTCCACGT CCTGCTTGCT CCCATCACAG CCNTCTTCCA 660

CACCCACTTC TATGACAGGC TACAGGACGC GGGCTCTCGC TGGCCCGAGC TCTACCTCTA 720

CTCGAGGGCT GACGAAGTAG TCCTGGCCAG AGACATAGAA CGCATGGTGG AGGCACGCCT 780

GGCACGCCGG GTCCTGGCGC GTTCTGTGGA TTTCGTGTCA TCTGCACACG TCAGCCACCT 840

CCGTGACTAC CCTACTTACT ACACAAGCCT CTGTGTCGAC TTCATGCGCA ACTGCGTCCG 900

CTGCTGAGGC CATTGCTCCA TCTCACCTCT GCTCCAGAAA TAAATGCCTG ACACCTCCCC 960

ACAAAAAAAA AAAAAAAAAA ACTCGAGGGG GGGCCCGGTA CCCAATTCGC CCTATAAAGG 1020

T 1021

(2) INFORMATION FOR SEQ ID NO: 146:

(i) SEQUENCE CHARACTERISTICS:
(A) LENGTH: 1285 base pairs

(B) TYPE: nucleic acid
(C) STRANDEDNESS: double
(D) TOPOLOGY: linear

(xi) SEQUENCE DESCRIPTION: SEQ ID NO: 146:

GGCACGAGGA GGGCCACGGC AGCCATCGCG CTTTGCAGTT CGGTCTCCTG GTGTACGGCC 60

AACGCCAAGT AGGGGATTGC GTTCCCTCCA GTCGCAGACC CTATCAGATT TGGATATGTC 120

CTTCATATTT GATTGGATTT ACAGTGGTTT CAGCAGTGTG CTACAGTTTT TAGGATTATA 180

TAAGAAAACT GGTAAACTGG TATTTCTTGG ATTGGATAAT GCAGGAAAAA CAACATTGCT 240

ACACATGCTA AAAGATGACA GACTTGGACA ACATGTCCCA ACATTACATC CCACTTCCGA 300

AGAACTGACC ATTGCTGGCA TGACGTTTAC AACTTTTGAT CTGGGTGGAC ATGTTCAAGC 360

TCGAAGAGTG TGGAAAAACT ACCTTCCTGC TATCAATGGC ATTGTATTTC TGGTGGATTG 420

TGCAGACCAC GAAAGGCTGT TAGAGTCAAA AGAAGAACTT GATTCACTAA TGACAGATGA 480

AACCATTGCT AATGTGCCTA TACTGATTCT TGGGAATAAG ATCGACAGAC CTGAAGCCAT 540

CAGTGAAGAG AGGTTGCGAG AGATGTTTGG TTTATATGGT CAGACAACAG GAAAGGGGAG 600

TATATCTCTG AAAGAACTGA ATGCCCGACC CTTAGAAGTT TTCATGTGTA GTGTGCTCAA 660

AAGACAAGGT TACGGAGAAG GCTTCCGCTG GATGGCACAG TACATTGATT AACACAAACT 720

CACATTGGTT CCAGGTCTCA ACGTTCAGGC TTACTCAGAG ATTTGATTGC TCAACATGCA 780

TAACTTGAAT TCAATAGACT TTTGCTGGTT ATAAAACAGA TGTTTTTTAG ATTATTAATA 840

TTAAATCAAC TTAATTTGAA TGAGAATTGA AAACTGATTC AAGTAAGTTT GAGTATCACA 900

ATGTTAGCTT TCTAATTCCA TAAAAGTACT TGGTTTTTAC AGTTTATAAT CTGACATCAC 960

CCCAGCGCCA TTTGTAAAGA GCAACTTTCC AGCAGTACAT TTGAAGCACT TTTTAACAAC 1020

ATGAAACTAT AAACCATATT TAAAAGCTCA TCATGTTAAA TTTTTTATGT ACTTTTCTGG 1080

AACTAGTTTT TAAATTTTAG ATTATATGTC CACCTATCKT AAGTGTACAG TTAATAATTA 1140

GCTTATTCAA TGATTGCATG ATGCCTTACA GTTTTCAATA ACTTTTTTTC TTATGCAAAC 1200

GTCATGCAAT AAAACAAACT CTAATGTTTG GCAAAAAAAA AAAAAAAAAA NTCGAGGGGG 1260

GGCCCGTACC CAATTCGCCC TAAAG 1285

(2) INFORMATION FOR SEQ ID NO: 147:

(i) SEQUENCE CHARACTERISTICS:
(A) LENGTH: 1386 base pairs
(B) TYPE: nucleic acid
(C) STRANDEDNESS: double
(D) TOPOLOGY: linear

(xi) SEQUENCE DESCRIPTION: SEQ ID NO: 147:

GGCACGAGGT GGCGCAGGGG TCAGTGGTTC TCTCGGGTCT CGGGACAGGT GAGCACCCTG 60

ATGAAGGCCA CGGTCCTGAT GCGGCACCTG GGCGGGTGCA GGAGATCGTG GGCGCCCTCC 120

GCAAGGGCGS CGGAGACCGG TTACAGGTGA TTTCTGATTT TRACATGACC TTGAGCAGGT 180

TTGCATATAA TGGAAAGCGA TGCCCTTCTT CTTACAATAT TCTGGATAAT AGCAAGATCA 240

TCAGTGAGGA GTGTCGGAAA GAGCTCACAG CGCTCCTTCA CCACTATTAC CCAATTGAGA 300

TCGACCCACA CCGGACCGTC AAGGAGAAGC TACCTCATAT GGTGGAATGG TGGACCAAAG 360

CGCACAATCT CCTATGTCAG CAGAAGATTC AGAAGTTTCA GATAGCCCAG GTGGTTAGAG 420

AGTCCAATGC AATGCTCAGG GAGGGATATA AGACCTTCTT CAACACACTC TACCATAACA 480

ACATTCCCCT TTTCATCTTT TCTGCGGGCA TTGGTGATAT CCTGGAAGAA ATTATCCGAC 540

AGATGAAAGT GTTCCACCCC AACATCCACA TCGTGTCTAA CTACATGGAT TTTAATGAAG 600

ATGGTTTTCT CCAGGGATTT AAGGGCCAGC TGATACACAC ATACAACAAG AACAGCTCTG 660

TGTGTGAGAA CTSTGGTTAC TTCCAGCAAC TTGAGGGCAA AACCAATGTC ATCCTGCTGG 720

GAGACTCTAT CGGGGACCTC ACCATGGCCG ATGGGGTTCC TGGTGTGCAG AACATTCTCA 780

AAATTGGCTT CCTGAATGAC AAGGTGGAGG AGCGGCGGGA NCGCTACATG GACTCCTATG 840

ACATCGTGCT GGAGAAGGAC GAGACTCTGG ATGTGGTCAA CGGGCTACTG CAGCACATCC 900

TGTGCCAGGG GGTCCAGCTG GAGATGCAAG GCCCCTGAAG GCGCAGGCTN CCAGNCCGCC 960

TGCAGGCCGT GGTGAGGAGG GGCGCCTCCC CAGAGTCTGC TCCCCCGTGA ACACAGAGCA 1020

GANGCCAGGG TGGCCAGCAG TGGCTGGGTC CTTCCGCGCC CCTCCGTCCT CCTTTCCCTG 1080

AGCACCTTCA TCACCAGAGG CTTGAAGGAA CCCCGCCATG TGGCAGGGCA CAGGCACTGT 1140

TCCTGGTGAA CCTTGGACCA CAGCATGTCA GTGCTCTAGG GATTGTCTAC TCCAGGGATT 1200

TTCTTCAAAA TTTTTAAACA TGGGAAGTTC AAACAAATAT AATGTGTGAA ACAGATCAAA 1260

ATTTTTAAAA TGAAAAAAAA GCTGCTCTGA TTCAGGGGAT GTGGGTCGGG GTAGAACCTG 1320

GACCTCTTGG CCTGGGGGCA CATGGGATGC TTCTAGGAAC ACAGTTTGAG AACCACCAAA 1380

AAAAAA 1386

(2) INFORMATION FOR SEQ ID NO: 148:

(i) SEQUENCE CHARACTERISTICS:
(A) LENGTH: 2098 base pairs
(B) TYPE: nucleic acid
(C) STRANDEDNESS: double

(D) TOPOLOGY: linear

(xi) SEQUENCE DESCRIPTION: SEQ ID NO: 148:

AGCCCTTCTC CCCGCGCTTG GGACTCTGAC ATCTTAAGGC TGCACGGTCG TGTCCTTGTC 60

TGGGTGAGGC CATGTCTGTG ATCCAAGGTT CCTGGAACTG ACACAGGAAG GGGCTGTGAA 120

CCCTAAGTGG GTGTMATCTC CTCCRACCGA GGCTTCTMAC CCTGGAGATG GCAGTTACTC 180

CTGGCCATGG TTGCTGAGCA TGGGCAGACC AGTGGAGGCC ACCCTACTGT GTTATCTGCG 240

CCTTCRATGA AGTGAGACCC TTGGGGAGAA CGGGCTGTGG ATGAAGGAGT GGACTGCAGC 300

CTTGGCCTAG CCACTGGGCT GGGATCTTCT GGGTCATGTG ACTGTGTATC CAGGAGCAGA 360

AACTTGTATT CTCAGGATTC AGGATCTACC CAGCACCAAA GATGTATTTT CAGGAGAACA 420

GACCTAGAAA TGGGCCTGTC TGGCATTTCA GAGTCAGGCA AAGCAGGCAG GGCCAGGGAG 480

CTTCTGTGGG TCTACACAAG AAGGTTCCTG TGAGGGCTAT CAGTTGTTGC CTTCTAGCTT 540

GCTGGTAACT TTGGCGCCTC CGCCAAGCCC TGCCAGACTC CCCTGGCTGT GATGGCATTC 600

TGTGCCATCC TGCCTTGTCC CCAGCCTCTG CAGGATGCCC TCCCTACCCA MCTYTYCCTG 660

GGCCTTCCCT GTCCACTGGG CTGGATTCAT GTTCAAACCA CTGGACTGGC AGGGCAACGA 720

CTTCTTCCCA CCTCAAGATG AGGTCCTCGC CCCCTTGTCT TGGCATAAAA ACACCTTTAA 780

AGCATGAGCC ATGTGCTTCT TTGCCCTTCT CTGTCCTGTT CCAATCTTCT GCCTCCCAGT 840

CACTCCCTGG GGACTATGGG ATCACTGTCC CCCCACCTGT GTGGCCACAC CATGTGTCCT 900

GTCAATCCAG AACTGCCTCT GAGCTCCAGG CTGACCACAG ATCAGCCACA GCCTGATGCC 960

TGCAGCCCCA CTTTGCTCAC CCTTCCCCTC CCCTCCTCCT TCCTTCCACA CAGCAAGCCT 1020

ACCTTTYTCC ATCCATGCTC ACCATAGCCC CCTTCCTTGT GACCTGGACC CTCCATTGTA 1080

CCTGGCTGAG ACTGTCAGCC TCCTGGAGGA GTGGGGTCCA CCTTCTTCTT GCCCTATGCA 1140

GTGCAAGCTT CACTTCTCAC CCAGCAAGGT TGACTCATCT GCCTCCATGT CTCTGGGGCT 1200

TTGCTGTTGC CCTGAAACCT AGCTGGGCTG GTCTTGCTCC CAGCTTGCTT CCCCCTCCTC 1260

GGATGTCCCT TTGCAGGCCC CTGTCGTTCC TCCGGCACCA GTGTCCTTGG CTGCCATGGC 1320

AAGCTCATCA GGGGCTTGTA CCCTGGTCAC CAAGCATGGT AGCAGCTGCC TGCATTGTAT 1380

CTCCATCTGG TCACTGCAGG TGCCAACCCT TCATCCCCCA TGTTTTCCTG GGCCATGGAG 1440

GGCTGACCTC CGTTTCTGGG GAATGTGGCT GAGCTGTGGT AACCAGCTAC ACCCCAGGTG 1500

CTCTTTCCAT GGTGGTGCCT GCTCATCTTG CTGATGCAAA CTAGGAAGTT AGGCTGCATC 1560

TCGGAGTGGC TTTCGCTGGA GAGGTGCTTT GCTGTCTCTC AGACTCAGTC ACTGTGTTCC 1620

CTCCCCGCCT CTCTTATCTC CATGGCTGTT TGCAGCTCTC CCAGGTACTT TGGGGTCTGA 1680

GCTGGAATTC CTTTGTGGTT TGCTCTTCTG CTTCTCACTC TTGTATTAAG AAGGATTCCA 1740

CAAAGGGAGA GTGGCATCCC TGCTGCTGCT GTGCCAGACC AGAGTTTCCT GAGGGGCCCT 1800

GACCCTAACC CTCCAGCTCA GCCCTGTACA CCTGACCCTG TAAATGAGTG GGGTTTGCTG 1860

ACTGTAATCC CTGACACCAG TAAAACCAAA AGGACTCTTG GGGGCTCAGT GTGAGAGCCA 1920

GGGTTACCTA CTCTGCCAAG TGAGGACAAA CTGCTAGGCT GTATCCCATA ATTTCAGGAT 1980

GAGAAACATT AACAATAAAA ATTTGTAGTA AACATAACCT CATGANGACT AAAAAAAAAA 2040

AAAAACTYGG GGGGGGGCCC GTAACCCATT GGGCCCTTNG GGGGGGNGTT TTAAAATT 2098

(2) INFORMATION FOR SEQ ID NO: 149:

(i) SEQUENCE CHARACTERISTICS:
(A) LENGTH: 1847 base pairs
(B) TYPE: nucleic acid
(C) STRANDEDNESS: double
(D) TOPOLOGY: linear

(xi) SEQUENCE DESCRIPTION: SEQ ID NO: 149:

TCGACCCACG CGTCCGAACT GAGGCGGCGG CGGGAGCCGG TTGGKGTCTG GTCTTCGCGT 60

CGGCCCCGCG GACCAGACGC TGCCCCCGGC GCGGGGAGAA GATGGTGCCK AGCGGCCTCG 120

GGCCCGCCAC GCGCCGCCAC GAGTGAGCCC AGCGCGACCG CGGGCGTCCG CCGAGCAGCT 180

GGCCCGGCTG GGCCCGGGGC GCGCANTGCC CGCCGGGGCG GGGTGGAGCT GATCAGAATA 240

ATGTTCAGCA TCAACCCCCT GGAGAACCTG AAGGTGTACA TCAGCAGTCG GCCTCCCCTG 300

GTGGTCTTCA TGATCAGCGT AANGCCCATG GCCATAGCTT TCCTGACCCT GGGCTACTTC 360

TTCAAAATCA AGGAGATTAA ATCCCCAGAA ATGGCAGAGG ATTGGAATAC TTTTCTGCTA 420

CGGTTCAATG ATTTGGACTT GTGTGTATCA GAGAATGAAA CCCTCAAGCA TCTCACAAAC 480

GACACCACAA CTCCGGAAAG TACAATGACC AGCGGGCAGG CCCGAGCTTC CACCCAGTCC 540

CCCCAGGCCC TGGAGGACTC GGGCCCGGTG AATATCTCAG TCTCAATCAC CCTAACCCTG 600

GACCCACTGA AACCCTTCGG AGGGTATTCC CGCAACGTCA CCCATCTGTA CTCAACCATC 660

TTAGGGCATC AGATTGGACT TTCAGGCAGG GAAGCCCACG AGGAGATAAA CATCACCTTC 720

ACCCTGCCTA CAGCGTGGAG CTCAGATGAC TGCGCCCTCC ACGGTCACTG TGAGCAGGTG 780

GTATTCACAG CCTGCATGAC CCTCACGGCC AGCCCTGGGG TGTTCCCCGT CACTGTACAG 840

CCACCGCACT GTGTTCCTGA CACGTACAGC AACGCCACGC TCTGGTACAA GATCTTCACA 900

ACTGCCAGAG ATGCCAACAC AAAATACGCC CAAGATTACA ATCCTTTCTG GTGTTATAAG 960

GGGGCCATTG GAAAAGTCTA TCATGCTTTA AATCCCAAGC TTACAGTGAT TGTTCCAGAT 1020

GATGACCGTT CATTAATAAA TTTGCATCTC ATGCACACCA GTTACTTCCT CTTTGTGATG 1080

GTGATAACAA TGTTTTGCTA TGCTGTTATC AAGGGCAGAC CTAGCAAATT GCGTCAGAGC 1140

AATCCTGAAT TTTGTCCCGA GAAGGTGGCT TTGGCTGAAG CCTAATTCCA CAGCTCCTTG 1200

TTTTTTGAGA GAGACTGAGA GAACCATAAT CCTTGCCTGC TGAACCCAGC CTGGGCCTGG 1260

ATGCTCTGTG AATACATTAT CTTGCGATGT TGGGTTATTC CAGCCAAAGA CATTTCAAGT 1320

GCCTGTAACT GATTTGTACA TATTTATAAA AATCTATTCA GAAATTGGTC CAATAATGCA 1380

CGTGCTTTGC CCTGGGTACA GCCAGAGCCC TTCAACCCCA CCTTGGACTT GAGGACCTAC 1440

CTGATGGGAC GTTTCCACGT GTCTCTAGAG AAGGATTCCT GGATCTAGCT GGTCACGACG 1500

ATGTTTTCAC CAAGGTCACA GGAGCATTGC GTCGCTGATG GGGTTGAAGT TTGGTTTGGT 1560

TCTTGTTTCA GCCCAATATG TAGAGAACAT TTGAAACAGT CTGCACCTTT GATACGGTAT 1620

TGCATTTCCA AAGCCACCAA TCCATTTTGT GGATTTTATG TGTCTGTGGC TTAATAATCA 1680

TAGTAACAAC AATAATACCT TTTTCTCCAT TTTGCTTGCA GGAAACATAC CTTAAGTTTT 1740

TTTTGTTTTG TTTTTGTTTT TTTGTTTTTT GTTTTCCTTT ATGAAGAAAA AATAAAATAG 1800

TCACATTTTA ATACTACCAA AAAATGGACA AAAAAAGTCG AGGGGGG 1847

(2) INFORMATION FOR SEQ ID NO: 150:

(i) SEQUENCE CHARACTERISTICS:
(A) LENGTH: 1569 base pairs
(B) TYPE: nucleic acid
(C) STRANDEDNESS: double
(D) TOPOLOGY: linear

(xi) SEQUENCE DESCRIPTION: SEQ ID NO: 150:

GACGCTGACG AGAGAAGGCC TCTTCCTTGA GGGTTGGTGC TGTGTTGCAG TGACCGTGGC 60

GGATTACGCC AACTCGGATC CGGCGGTCGT GAGGTCTGGA CGAGTCAAGA AAGCCGTAGC 120

CAACGCTGTT CAGCAGGAAG TAAAATCTCT TTGTGGCTTG GAAGCCTCTC AGGTTCCTGC 180

AGAGGAAGCT CTTTCTGGGG CTGGTGAGCC CTGTGACATC ATCGACAGCA GTGATGAGAT 240

GGATGCCCAG GAGGAAAGCA TCCATGAGAG AACTGTCTCC AGAAAAAAGA AAAGCAAGAG 300

ACACAAAGAA GAACTGGACG GGGCTGGAGG AGAAGAGTAT CCCATGGATA TTTGGCTATT 360

GCTGGCCTCC TATATCCGTC CTGAGGACAT TGTGAATTTT TCCCTGATTT GTAAGAATGC 420

CTGGACTGTC ACTTGCACTG CTGCCTTTTG GACCAGGTTG TACCGAAGCA CTACACGCTG 480

GATGCTTCCC TGCCTTTGCG TCTGCGACCA GAGTCAATGG AGAAGCTGCG CTGTCTCCGG 540

GCTTGTGTGA TCCGATCTCT GTACCATATG TATGAGCCAT TTGCTGCTCG AATCTCCAAG 600

AATCCAGCCA TTCCAGAAAG CACCCCCAGC ACATTAAAGA ATTCCAAATG CTTACTTTTC 660

TGGTGCAGAA AGATTGTTGG GAACAGACAG GAACCAATGT GGGAATTCAA CTTCAAGTTC 720

AAAAAACAGT CCCCTAGGTT AAAGAGCAAG TGTACAGGAG GATTGCAGCC TCCCGTTCAG 780

TACGAAGATG TTCATACCAA TCCAGACCAG GACTGCTGCC TACTGCAGGT CACCACCCTC 840

AATTTCATCT TTATTCCGAT TGTCATGGGA ATGATATTTA CTCTGTTTAC TATCAATGTG 900

AGCACGGACA TGCGGCATCA TCGAGTGAGA CTGGTGTTCC AAGATTCCCC TGTCCATGGT 960

GGTCGGAAAC TGCGCAGTGA ACAGGGTGTG CAAGTCATCC TGGACCCAGT GCACAGCGTT 1020

CGGCTCTTTG ACTGGTGGCA TCCTCAGTAC CCATTCTCCC TGAGAGCGTA GTTACTGCTT 1080

CCCATCCCTT GGGGGCAGCC TCGAGTGTAG TCCATTAGTA ATCAGATTCC AGTTTGGACA 1140

GGGTGGCTGG ATTGTATATC TCGTTAGTAA TGTACATGCT CTTCAGGTTC TAGGGCTCCT 1200

GTTAGGGGAG GGAGAAATGT TGAATCAAGA GGGAAAACAA CTACTATGAT TTATAAACAT 1260

ATTTTAATGT AAAAATTTGC ATTTAAAAGG AGTGGCCCTG TTTTCTGTGT TAAAACCCCA 1320

TTTGGTGCTA TTGAGTTTGT TCTTTATTCT TTTATCCCAG TGAAAATTGT TGATCTTGCT 1380

GTAGGGAAAA ATTAAACTCT TTGAATCTCC AAACAAGGAA GTTTCAGCAT TCCCTTATGG 1440

ATCAGAGGAA CCTTAGAGGC CTGAAATTGT TGCTTCCAGT TTAGCTGCCC CTCAAATTCA 1500

AGTGAATATT TTCCCTTCTC CCTTTACCCT TCTCCAGAAA TAAAGCAGGT GACAGGGTTT 1560

CAGAATCTT 1569

(2) INFORMATION FOR SEQ ID NO: 151:

(i) SEQUENCE CHARACTERISTICS:
(A) LENGTH: 1540 base pairs
(B) TYPE: nucleic acid
(C) STRANDEDNESS: double
(D) TOPOLOGY: linear

(xi) SEQUENCE DESCRIPTION: SEQ ID NO: 151:

CCCACGCGTC CGGAAGGATT GACCAGTTAA CCAACATCTT AGCCCCCATG GCTGTTGGCC 60

AGATTATGAC ATTTGGCTCC CCAGTCATCG GCTGTGGCTT TATTTCGGGA TGGAACTTGG 120

TATCCATGTG CGTGGAGTAC GTCCTGCTCT GGAAGGTTTA CCAGAAAACC CCAGCTCTAG 180

CTGTGAAAGC TGGTCTTAAA GAAGAGGAAA CTGAATTGAA ACAGCTGAAT TTACACAAAG 240

ATACTGAGCC AAAACCCCTG GAGGGAACTC ATCTAATGGG TGTGAAAGAC TCTAACATCC 300

ATGAGCTTGA ACATGAGCAA GAGCCTACTT GTGCCTCCCA GATGGCTGAG CCCTTCCGTA 360

CCTTCCGAGA TGGATGGGTC TCCTACTACA ACCAGCCTGT GTTTCTGGCT GGCATGGGTC 420

TTGCTTTCCT TTATATGACT GTCCTGGGCT TTGACTGCAT CACCACAGGG TACGCCTACA 480

CTCAGGGACT GAGTGGGTTC CATCCTCAGT ATTTTGATGG GAGCATCAGC TATAACTGGA 540

ATAATGGGAA CTGTAGCTTT TACTTGGCTA CGTCGAAAAT GTGGTTTGGT TCGGCAGGTC 600

TGATCTCAGG ATTGGCACAG CTTTCCTGTT TGATCTTGTG TGTGATCTCT GTATTCATGC 660

CTGGAAGCCC CCTGGACTTG TCCGTTTCTC CTTTTGAAGA TATCCGATCA AGGTTCATTC 720

AAGGAGAGTC AATTACACCT ACCAAGATAC CTGAAATTAC AACTGAAATA TACATGTCTA 780

ATGGGTCTAA TTCTGCTAAT ATTGTCCCGG AGACAAGTCC TGAATCTGTG CCCATAATCT 840

CTGTCAGTCT GCTGTTTGCA GGCGTCATTG CTGCTAGAAT CGGTCTTTGG TCCTTTGATT 900

TAACTGTGAC ACAGTTGCTG CAAGAAAATG TAATTGAATC TGAAAGAGGC ATTATAAATG 960

GTGTACAGAA CTCCATGAAC TATCTTCTTG ATCTTCTGCA TTTCATCATG GTCATCCTGG 1020

CTCCAAATCC TGAAGCTTTT GGCTTGCTCG TATTGATTTC AGTCTCCTTT GTGGCAATGG 1080

GCCACATTAT GTATTTCCGA TTTGCCCAAA ATACTCTGGG AAACAAGCTC TTTGCTTGCG 1140

GTCCTGATGC AAAAGAAGTT AGGAAGGAAA ATCAAGCAAA TACATCTGTT GTTTGAGACA 1200

GTTTAACTGT TGCTATCCTG TTACTAGATT ATATAGAGCA CATGTGCTTA TTTTGTACTG 1260

CAGAATTCCA ATAAATGGCT GGGTGTTTTG CTCTGTTTTT ACCACAGCTG TGCCTTGAGA 1320

ACTAAAAGCT GTTTAGGAAA CCTAAGTCAG CAGAAATTAA CTGGATTAAT TTCCCTTATG 1380

TTGAGGGCCA TGGRAAAAAA ATTGGGAAAA GGAAAAACTC AGTTTTAAAT ACGGGAGACT 1440

ATAATGGATA ACACTGRATT CCCCTATTTC TCATGAGTAG ATACAATCTT ACGTAAAAGA 1500

GTGGTTAGTC ACGTGAATTC AGTTATCATT TGACAGATTC 1540

(2) INFORMATION FOR SEQ ID NO: 152:

(i) SEQUENCE CHARACTERISTICS:
(A) LENGTH: 1719 base pairs
(B) TYPE: nucleic acid
(C) STRANDEDNESS: double
(D) TOPOLOGY: linear

(xi) SEQUENCE DESCRIPTION: SEQ ID NO: 152:

TACTTATGAG GTCAATTGGA AATAAGAACA CCATTTTACT GGGTCTAGGA TTTCAAATAT 60

TACAGTTGGC ATGGTATGGC TTTGGTTCAG AACCTTGGAT GATGTGGGCT GCTGGGGCAG 120

TAGCAGCCAT GTCTAGCATC ACCTTTCCTG CTGTCAGTGC ACTTGTTTCA CGAACTGCTG 180

ATGCTGATCA ACAGGGTGTC GTTCAAGGAA TGATAACAGG AATTCGAGGA TTATGCAATG 240

GTCTGGGACC GGCCCTCTAT GGATTCATTT TCTACATATT CCATGTGGAA CTTAAAGAAC 300

TGCCAATAAC AGGAACAGAC TTGGGAACAA ACACAAGCCC TCAGCACCAC TTTGAACAGA 360

ATTCCATCAT CCCTGGCCCT CCCTTCCTAT TTGGAGCCTG TTCAGTACTG CTGGCTCTGC 420

TTGTTGCCTT GTTTATTCCG GAACATACCA ATTTAAGCTT AAGGTCCAGC AGTTGGAGAA 480

AGCACTGTGG CAGTCACAGC CATCCTCATA ATACACAAGC GCCAGGAGAG GCCAAAGAAC 540

CTTTACTCCA GGACACAAAT GTGTGACGAC TGAAATCAGG AAGATTTTTC TATCAGCACC 600

CAGGTCTTAG TTTTCACCTC TAGTTCTGGA TGTACATTCC ATTTCCATCC ACAGTGTACT 660

TTAAGATTGT CTTAAGAAAT GTATCTGCAT GAACTCCGTG GGAACTAAAG GAAGTGGGAA 720

CTTAGAACCA GACAGTTTTC CAAAGATGTT ACAATTTCTT TTGAAAAACC TTTTGTTTAT 780

TAGCACCAAT TTCTYGCCAC TAAGCTATTT GTTTTATTAT ACATCCTTTA ATTAAAAACT 840

ATATATGTAA CTTCTTAGAT ATTAGCAAAT GTCTCTGCTA CCATTTCCTT AAGGTGTTGA 900

GCTTTAACTC TATGCTGACT CAGTGAGACA CAGTAGGTAG TATGGTTGTG GACCTATTTG 960

TTTTAACATT GTAAAATTTT GAGTCAGATT TTAATATTGT AAAATCTTGG GTCAAATAAT 1020

TCAAAGCCTT AATGCAGATG CACTAAAACA AAGAAATGGT AAATGAATTG TTTGCATTTA 1080

AAAAAAAAAA CTCTTAAGAA AACTGTACTA AATCTGAATC ATGTTTTGAG CTTGTTTGCA 1140

GTACTTTTAA ACATTATTCA CTACTGTTTT TGAAGTGAGA AAGTATCAGC CATTTAGCAT 1200

TTAAGTTGGG GTATTTAGAG CCTGTAATCT AAATGCTGGC TCAAATTTAT TCCCCAGCTA 1260

CTTCTTATAC CACTATTCTT TTAATGTTTG CATAATCATA AGCACCTCAA CACTTGAATA 1320

CATAATCTAA AAATTATATA GTAAAGCTGG TAGCCTTGAA AATGTCAGTG TGATATCTAT 1380

TATGTAGATA AATATATATA GTGGCCTTTC AGGACTGTCA CAGTAACACT TTATTTACAG 1440

AGCTAATGTT TGTCCTAAAT TTTCAGGACC CTAGAGGAGA GCTTTATACA ATTACCGATG 1500

TGAATTTCTC TAAAGTGTAT ATTTTTGTGT CCAGTTATAT TATTTAAAAA AGTGTTACTT 1560

TGTAAAAATT GTATATAAAG AACTGTATAG TTTACACTGT TTTCATCTTG TGTGTGGTTA 1620

TTGCTTAATG CTTTTTAAAC TTGGAACACT CACTATGGTT AAATAAGGTC TTAAAAGAAA 1680

TGTAAATATT YTGTTAATAA AGTTAAATAT TTTAATGAT 1719

(2) INFORMATION FOR SEQ ID NO: 153:

(i) SEQUENCE CHARACTERISTICS:
(A) LENGTH: 863 base pairs
(B) TYPE: nucleic acid

(C) STRANDEDNESS: double
(D) TOPOLOGY: linear

(xi) SEQUENCE DESCRIPTION: SEQ ID NO: 153:

GGCACGAGGG AAGCCGGGAC GATGTCCGCA TGACAACCGA CGTTGGAGTT TGGAGGTGCT 60

TGCCTTAGAG CAAGGGAAAC AGCTCTCATT CAAAGGAACT AGAAGCCTCT CCCTCAGTGG 120

TAGGGAGACA GCCAGGAGCG GTTTTCTGGG AACTGTGGGA TGTGCCCTTG GGGGCCCGAG 180

AAAACAGAAG GAAGATGCTC CAGACCAGTA ACTACAGCCT GGTGCTCTCT CTGCAGTTCC 240

TGCTGCTGTC CTATGACCTC TTTGTCAATT CCTTCTCAGA ACTGCTCCAA AAGACTCCTG 300

TCATCCAGCT TGTGCTCTTC ATCATCCAGG ATATTGCAGT CCTCTTCAAC ATCATCATCA 360

TTTTCCTCAT GTTCTTCAAC ACCTTCGTCT TCCAGGCTGG CCTGGTCAAC CTCCTATTCC 420

ATAAGTTCAA AGGGACCATC ATCCTGACAG CTGTGTACTT TGCCCTCAGC ATCTCCCTTC 480

ATGTCTGGGT CATGAACTTA CGCTGGAAAA ACTCCAACAG CTTCATATGG ACAGATGGAC 540

TTCAAATGCT GTTTGTATTC CAGAGACTAG CAGCAGTGTT GTACTGCTAC TTCTATAAAC 600

GGACAGCCGT AAGACTAGGC GATCCTCACT TCTACCAGGA CTCTTTGTGG CTGCGCAAGG 660

AGTTCATGCA AGTTCGAAGG TGACCTCTTG TCACACTGAT GGATACTTTT CCTTCCTGGA 720

TAGRAGGCCA CATTTGCTGC TTTGCAGGGG AGAGTTGGGC CCTATGCATG GGGCAAAACA 780

GGTGGGATTT TCCAAGGGAA GGGTTCAGAA TTAGGCNTGT TGTTTCAGCC ATTTCCAAGG 840

AAGGGGAAGG GTTTCCCTNC CCT 863

(2) INFORMATION FOR SEQ ID NO: 154:

(i) SEQUENCE CHARACTERISTICS:
(A) LENGTH: 1101 base pairs
(B) TYPE: nucleic acid
(C) STRANDEDNESS: double
(D) TOPOLOGY: linear

(xi) SEQUENCE DESCRIPTION: SEQ ID NO: 154:

AACAGCAAAA AAGAATGATT TCTTCTGAAA TTGTGGAACA TGAGGATTCA AGTTTTTATT 60

TTGTTACTAG GTGCTGGAGG AACATCCCAG TTCACAAAGC CCCCATCTCT TCCTCTGGAG 120

CCAGAGCCTG CGGTGGAATC AAGTCCAACT GAAACATCAG AACAAATAAG AGAGAAATAA 180

GAATAGAATG AATGACCCCA AAATARGGTT TTCTTGGGCG AGGATGTGCT GGATTAGGAA 240

AGGTGACATG ACACAGGCAG AGCAGAGTGG CACCCACCAC AGAATACAGT GTGTGTTATT 300

ACGAGGAGCC AGCAGTTGAG CCTAAGGTCC TTCTACCTAC CTGGTATTGG CATTTGAGGT 360

CGGAAACCCT CTACTGCCCC ATAAGCCAGG AAAAGTGAAA AGAGAACACA GTTCCTTTAA 420

GAACTGGCAG CAAGGCTTGA GGCCTTATGT ATGTAGCTGA GTCAGCAAGG TACATGATGC 480

TGTCTGCTTT CAAAAGGACT TTTCTCTCCT AGCTGACTGA CTCCTTCCTT AGTTCAAGGA 540

ACAGCTGAGA CAGACCTCTG CTGAGTAGCT CTGTGATGAC AAAGCCTTGG TTTAACTGAG 600

GTGATCCTCA GGTTGTGAGG TTTATTAGTC CCCAAGGCAA ACACAAATAT TAGATTAATA 660

ATCCAACTTT AATAGTATAC ATTTAAAAGA AAAAAAACAA AAGCCCTGGA AGNTTGAGGC 720

CAAGCCTGCT GAGTATTGCA GCTGCATTTG CCCAAAGGGA ATCCAGAACA AGTCCCTCCC 780

TGTATTTTGT TCTTGAGAGG GGTCAGTCTA GAAGCTAGAT CCTATCAGGA TGAGGAGCAG 840

CAGCCCAGGG CTTGTCTGGA TCAGCACCAA CGATTTTAAA GAAAAAAGGA AGAGTTTCTT 900

AGATGAGTAA TTGTTATTGA AGATAGTCAG TGATAACCAC TGACCAGATG CTATCAATAC 960

ACTATGTGTC CTTTTTAGAA TAAAGATTAC ATATCATCAT TCCTTTGGGG AAAATTGTTA 1020

TTCAGGTATA AAAACAAGAG ATTATAATAA AAAANTAAAA GAACCCTAAA AAAAAAAAAC 1080

CTCGTGCCGA ATTCCCTGCA G 1101

(2) INFORMATION FOR SEQ ID NO: 155:

(i) SEQUENCE CHARACTERISTICS:
(A) LENGTH: 2031 base pairs
(B) TYPE: nucleic acid
(C) STRANDEDNESS: double
(D) TOPOLOGY: linear

(xi) SEQUENCE DESCRIPTION: SEQ ID NO: 155:

CAATTAACCC GTTTGAGGCC TAGGTTGTTT GGCAAGCCCC NGGCCTAAAG TTTTAATTCG 60

GCAGAGCCAA GGGCCTGAAA GGAAGGGAAA GGGGAGGGTA GCGGGAGGGT AGCAGGTGAG 120

TTCCTAGGGC TGGAAGGTTT AGCAGCAGCC TGGTGCAGTG CCCTGTCATC AAGACAAACC 180

CACGGTCCTC CTGGGTGCCT ACCAAGCTTG GTTTGTACAA AAGCAAGGTG GGAGTCTATT 240

TTTGTACATG AGATACATCA CACTTACCTG TGGGCCAGTA TTGTGAAGTG AGTCTGAGTT 300

GTTTACACTG ATGCCTTCCC TGCCCACCAC AAATTGTGTA CATAGTCTTC AGAATGATAC 360

CACCCCTTTC CCCAGCTCCC AACCAAGAGC TGGTTCTAGG CCTGTGTTAT ATGTCATATT 420

TAGCGTTTTT ATATATGACC TTTGATTTCT GTTGTTTGTA TTTTAGCACA GTGTATGCAC 480

CTTCATTTAA ATACATCTGT GTGCATACAG ATACGCATAT ATGTGTGTGC GTATGCATAT 540

ATCTCTCATC TGTAGTTTCC AAGAGTTCAG CTGAAGCAGA TGGAGTCCTG CAGCCCAGGA 600

GACACCCTGC ATCCCTGCTA ATAGTGTTTG CCACAAGTAT TAGTGAGTCT TCCTTATTAA 660

TATTTTCATT TCAGAAGACT GAAGCAAAGC TGATAGTGTT TGCTGTTTCT TTGGCAGCTA 720

AGTGAGGGTC TTGGGATGAC TTGCTGTGTT CCTCAAGCTG CACTTTGGGG CCATCTCTGC 780

AGTATTAAGC CCCCTTTTTG CTTGGTGGTA CTCTGTCTGT GCCTGTGTGT GTGTGTGATA 840

GTCACTCTTG CATGGCTTCC ATGTCTGGTT TGTGGCATTT GGGGATAAGT GCTGAACCAG 900

AGCATTTGCA GTTTGTTTGA GGCCTCGTTG CCAATGATAG ATCACTCCTG TTGACCTGGT 960

ATGTCTGCTT GCTTGCTGCT TTTCCTTGCT TTCTCTTGGA AGAGGAAAGG ACTCTGGTCA 1020

GGCCCAGGCT GAGTGAGATG AGCTGCAGCT GGCTCATGGC CTTCTTAGAG CAGAGAGAGG 1080

AGTATGTCAT TTTACTAAGT TCCTAAACAA ACATTTATGC AGGCAACACT CCTTGCAGAT 1140

CCAGAAACTG AGGCACAATA GGGTTATGAC TTGCTCAAGA ATATGTAGCT GCTAGGGGGT 1200

AAATCAAGGC ATCACAATTT CTGTTCAGCG GGCAGGAATA GGCTGTGAAT TGCTAGCACT 1260

TTTTTTTTAA GCAATTACTT TTTGACTTGT TCCTCTGAAA GTGCAAGAGG CGTACACCTT 1320

TCCCAAATGT AGACTAGAAT CTGCAGGATG CCACCCACTG TATAGTTCTG CTTTCCCAGA 1380

GAGGAAGAAC TTTTAGAAAC CAAATGATCT TAATTGTTAT TGCCCACCCC TGGCTTTTCC 1440

GGGTAGAAAA TTCACAGTAG GAATGATTGT TAAGAGAGAG TGCTTGGAAC CATGGGTTAA 1500

CAGGAAAGGC TACCTAACTT CACATATCTG CAACCAGAGC AGCCACCAAG CATTACTTAG 1560

CAGCAGGAAA ATGATTGTAT TTGAGTTCCT GTGTGTCCAA AACTGAGGCA CCATGTTCTT 1620

TGAAAACATG CCACCTCAAG GCTGGGCGCG GTGGCTCACA CCTGTTAATC CCAGCACTTT 1680

GGGAGGCCGA GGCGGGCGGA TCACCGGAGT CGGGGAGTTT GAGACCAGCC TGGACCAACA 1740

TGGGAGAAAC CCCATCTCTA CCTAAAAATA CAAAATTAGC CGGGCGTGGT GGCATGCGCC 1800

TATAATCTCA GCTACTTGGG AGGGYTGAGG CAGGRGAATT GCTTGAACCC RGGANGGCGG 1860

AGGTTTGCGG TTGAGTTGAG GATCGTGCCA TTGCACTTCC GGGCCTTGGG GCAACAACAG 1920

CAAAAAYTCC GTCTTCAAMW MRTGCCGAAT TCGATATCAA GCTTATCGAT ACCGTCGACC 1980

TCGAGGGGGG GCCCGGTACC CAATTCGCCC TATAGNGATC GTATTACAAT C 2031

(2) INFORMATION FOR SEQ ID NO: 156:

(i) SEQUENCE CHARACTERISTICS:
(A) LENGTH: 1981 base pairs
(B) TYPE: nucleic acid
(C) STRANDEDNESS: double
(D) TOPOLOGY: linear

(xi) SEQUENCE DESCRIPTION: SEQ ID NO: 156:

CCTGCACCCT GAGCCCTTCA CCCCTCCGAG TTCCCCCCAG GTTGGCTTCC TTCGATTCCT 60
TTTCTTGGTA TCAACGTTTG ATTGGAAGAA CAACCCCCTC TTTGTCAACC TCAATAATGA 120
GCTCACTGTG GAGGAGCAGC TCGGGCACAG CTCMCCGTYA TGGTCATTGT TACCCCCCAA 180
GACCGCAAAA ACTCTGTGTG GACACAGGAT GGACCCTCAG CCCAGATCCT GCAGCAGCTT 240
GTGGTCCTGG CAGCTGAAGC CCTGCCCATG TTAGAGAAGC AGCTCATGGA TCCCCGGGGA 300
CCTGGGGACA TCAGGACAGT GTTCCGGCCG CCCTTGGACA TTTACGACGT GCTGATTCGC 360
CTGTYTCCTC GCCATATCCC GCGGCACCGC AGGCTTGTGG ACTCGCCAGY TGCCTCCTTC 420
TGCCGGGGCC TGCTCAGCCA GCCGGGGCCC TCATCCCTGA TGCCCGTGCT GGGTNATGAT 480
CCTNCTCAGC TCTATCTGAC GCAGCTCAGG GAGGCCTTTG GGGATCTGGC CCTTTTCTTC 540
TATGACCAGC ATGGTGGAGA GGTGATTGGT GTCCTCTGGA AGCCCACCAG CTTCCAGCCG 600
CAGCCCTTCA AGGCCTCCAG CACAAAGGGG CGCATGGTGA TGTCTCGAGG TGGGGAGCTA 660
GTAATGGTGC CCAATGTTGA AGCAATCCTG GAGGACTTTG CTGTGCTGGG TGAAGGCCTG 720
GTGCAGACTG TGGAGGCCCG AAGTGAGAGG TGGACTGTGT GATCCCAGCT CTGGAGCAAG 780
CTGTAGACGG ACAGCAGGAC ATTGGACCTC TAGAGCAAGA TGTCAGTAGG ATGACCTCCA 840
CCCTCCTTGG ACATGAATCC TCCATGGAGG GCCTGCTGGC TGAACATGCT GAATCATCTC 900
CAACAAAACC CAGCCCCAAC TTTCTCTCTG ATGCTCCAGC ATTGGGGCAG GGGCATGGTG 960
GCCCATGTAG TCTCCTGGGC CTCACCATCC CAGAAGAGGA GTGGGAGCCA GCTCAGAGAA 1020
GGAACTGAAC CCAGGAGATC CATCCACCTA TTAGCCCTGG GCCTGGACCT CCCTGCGATT 1080
TCCCACTCCT TTCTTAGTCT TCTTCCAGAA ACAGAGAAGG GGATGTGTGC CTGGGAGAGG 1140
CTCTGTCTCC TTCCTGCTGC CAGGACCTGT GCCTAGACTT AGCATGCCCT TCACTGCAGT 1200
GTCAGGCCTT TAGATGGGAC CCAGCGAAAA TGTGGCCCTT CTGAGTCACA TCACCGACAC 1260
TGAGCAGTGG AAAGGGGCTA TATGTGTATG AATAGACCAC ATTGAAGGAG CACAATGCCC 1320
TCCTGTGTTG ATGCCACTTC CCAGGGTGGA GACAGTGGAA AAGAACCGAG GACAGGAAAG 1380
GATTGGGTAG GTGAAGGGGT CAGGGGACTG GTAGTCACCC AATCTTGGAG AGGTGCAAAA 1440
AGCACTGGGG GCTACCCGTT AGCTGCATCT GCCCTGGCTG TTTGCCCGTT CATGTCACAA 1500
ACTGCCACTA CTATGTACCT GCAGTGGGGT TGCAGAGATG GGGGAGACTC AAGTCTTACT 1560
CCCCAGGAGC TCCCAGGGCC CAAGGAGGAG AATGCTGCCT CCTTTCAGTC TGGTCTACAC 1620
CCACTTTCTG GTAGCCTCTC TGCTTCCTGT AATTCTGGCT GTTTTTCCAG ACTCAGCTCA 1680
AATAGTGCCC CTCCTTAAGC CCATCCCTCG CCCCCAGCCT GAGGTGATCT TTCCCTCCTC 1740
TGAACTATTA GAGCAGTTAC TGTCTGTTCA GTTCGTTTGG CAGGCACACA CAGTGGCATA 1800

AATTCTATTG TTTTGAACTC TGATTTAAAA TTAAATTGCA GCTGGGCGTG GTGGCTCATG 1860

CTTGTAATCC CAACACTTAG GGAGTMAGGR GAATCACTTG ASCYCAGGAG TYCTAGACCA 1920

ATCTGGGCAA MAGAGAGACC CCATCTCTTT TAAATAAAAA GTTAAATTGC TTAAAAAAAA 1980

A 1981

(2) INFORMATION FOR SEQ ID NO: 157:

(i) SEQUENCE CHARACTERISTICS:
(A) LENGTH: 915 base pairs
(B) TYPE: nucleic acid
(C) STRANDEDNESS: double
(D) TOPOLOGY: linear

(xi) SEQUENCE DESCRIPTION: SEQ ID NO: 157:

GAATTCGGCA CGAGCGCGGC CATGGCGCTC CTGCTTTCGG TGCTGCGTGT ACTGCTGGGC 60

GGCTTCTTCG CGCTCGTGGG GTTGGCCAAG CTCTCGGAGG AGATCTCGGC TCCAGTTTCG 120

GAGCGGATGA ATGCCCTGTT CGTGCAGTTT GCTGAGGTGT TCCCGCTGAA GGTATTTGGC 180

TACCAGCCAG ATCCCCTGAA CTACCAAATA GCTGTGGGCT TTCTGGAACT GCTGGCTGGG 240

TTGCTGCTGG TCATGGGCCC ACCGATGCTG CAAGAGATCA GTAACTTGTT CTTGATTCTG 300

CTCATGATGG GGGCTATCTT CACCTTGGCA GCTCTGAAAG AGTCACTAAG CACCTGTATC 360

CCAGCCATTG TCTGCCTGGG GTTCCTGCTG CTGCTGAATG TCGGCCAGCT CTTAGCCCAG 420

ACTAAGAAGG TGGTCAGACC CACTAGGAAG AAGACTCTAA GTACATTCAA GGAATCCTGG 480

AAGTAGAGCA TCTCTGTCTC TTTATGCCAT GCAGCTGTCA CAGCAGGAAC ATGGTAGAAC 540

ACAGAGTCTA TCATCTTGTT ACCAGTATAA TATCCAGGGT CAGCCAGTGT TGAAAGAGAC 600

ATTTTGTCTA CCTGGCACTG CTTTCTCTTT TTAGCTTTAC TACTCTTTTG TGAGGAGTAC 660

ATGTTATGCA TATTAACATT CCTCATGTCA TATGAAAATA CAAAATAAGC AGAAAAGAAA 720

TTTAAATCAA CCAAAATTCT GATGCCCCAA ATAACCACTT TTAATGCCTT GGTGTAAGTA 780

TACCTCTGAA CTTTTTTCTG TGCCTTTAAA CAGATATATA TTTTTTTTWA ATGAAAATAA 840

AACCATATAT CCTATTTTAT TTCCTCCTTT TAAAACCTTA TAAACTATAA MAAAAAAAAA 900

AAAAAAAAAA CTCGA 915

(2) INFORMATION FOR SEQ ID NO: 158:

(i) SEQUENCE CHARACTERISTICS:
(A) LENGTH: 2117 base pairs

(B) TYPE: nucleic acid
(C) STRANDEDNESS: double
(D) TOPOLOGY: linear

(xi) SEQUENCE DESCRIPTION: SEQ ID NO: 158:

AGAGCGAAGC GAGGGTGGCG CGGGTCCGGG CATGAAGCTG GGCCGGGCCG TGCTGGGCCT 60

GCTGCTGCTG GCGCCGTCCG TGGTGCAGGC GGTGGAGCCC ATCAGCCTGG GACTGGCCCT 120

GGCCGGCGTC CTCACCGGCT ACATCTACCC GCGTCTCTAC TGCCTCTTCG CCGAGTGCTG 180

CGGGCAGAAG CGGAGCCTTA GCCGGGAGGC ACTGCAGAAG GATCTGGACG ACAACCTCTT 240

TGGACAGCAT CTTGCAAAGA AAATCATCTT AAATGCCGTG TTTGGTTTCA TAAACAACCC 300

AAAGCCCAAG AAACCTCTCA CGCTCTCCCT GCACGGGTGG ACAGGCACCG GCAAAAATTT 360

CGTCAGCAAG ATCATCGCAG AGAATATTTA CGAGGGTGGT CTGAACAGTG ACTATGTCCA 420

CCTGTTTGTG GCCACATTGC ACTTTCCACA TGCTTCAAAC ATCACCTTGT ACAAGGATCA 480

GTTACAGTTG TGGATTCGAG GCAACGTGAG TGCCTGTGCG AGGTCCATCT TCATATTTGA 540

TGAAATGGAT AAGATGCATG CAGGCCTCAT AGATGCCATC AAGCCTTTCC TCGACTATTA 600

TGACCTGGTG GATGGGGTCT CCTACCAGAA AGCCATGTTC ATATTTCTCA GCAATGCTGG 660

AGCAGAAAGG ATCACAGATG TGGCTTTGGA TTTCTGGAGG AGTGGAAAGC AGAGGGAAGA 720

CATCAAGCTC AAAGACATTG AACACGCGTT GTCTGTGTCG GTTTTCAATA ACAAGAACAG 780

TGGCTTCTGG CACAGCAGCT TAATTGACCG GAACCTCATT GATTATTTTG TTCCCTTCCT 840

CCCCCTGGAA TACAAACACC TAAAAATGTG TATCCGAGTG GAAATGCAGT CCCGAGGCTA 900

TGAAATTGAT GAAGACATTG TAAGCAGAGT GGCTGAGGAG ATGACATTTT TCCCCAAAGA 960

GGAGAGAGTT TTCTCAGATA AAGGCTGCAA AACGGTGTTC ACCAAGTTAG ATTATTACTA 1020

CGATGATTGA CAGTCATGAT TGGCAGCCGG AGTCACTGCC TGGAGTTGGA AAAGAAACAA 1080

CACTCAGTCC TTCCACACTT CCACCCCCAG CTCCTTTCCC TGGAAGAGGA ATCCAGTGAA 1140

TGTTCCTGTT TGATGTGACA GGAATTCTCC CTGGCATTGT TTCCACCCCC TGGTGCCTGC 1200

AGGCCACCCA GGGACCACGG GCGAGGACGT GAAGCCTCCC GAACACGCAC AGAAGGAAGG 1260

AGCCAGCTCC CAGCCCACTC ATCGCAGGGC TCATGATTTT TTACAAATTA TGTTTTAATT 1320

CCAAGTGTTT CTGTTTCAAG GAAGGATGAA TAAGTTTTAT TGAAAATGTG GTAACTTTAT 1380

TTAAAATGAT TTTTAACATT ATGAGAGACT GCTCAGATTC TAAGTTGTTG GCCTTGTGTG 1440

TGTGTTTTTT TTTAAGTTCT CATCATTATT ACATAGACTG TGATGTATCT TTACTGGAAA 1500

TGAGCCCAAG CACACATGCA TGGCATTTGT TCCACAGGAG GGCATCCCTG GGGATGTGGC 1560

TGGAGCATGA GCCAGCTCTG TCCCAGGATG GTCCCAGCGG ATGCTGCCAG GGGCAKTGAA 1620

GTGTTTAGGT GAAGGACAAG TAGGTAAGAG GACGCCTTCA GGCACCACAG ATAAGCCTGA 1680

AACAGCCTCT CCAAGGGTTT TCACCTTAGC AACAATGGGA GCTGTGGGAG TGATTTTGGC 1740

CACACTGTCA ACATTTGTTA GAACCAGTCT TTTGAAAGAA AAGTATTTCC AACTTGTCAC 1800

TTGCCAGTCA CTCCGTTTTG CAAAAGGTGG CCCTTCACTG TCCATTCCAA ATAGCCCACA 1860

CGTGCTCTCT GCTGGATTCT AAATTATGTG AATTTTGCCA TATTAAATCT TCCTCATTTA 1920

TACTATTATT TGTTACGTTC AATCAGAATC CCCGAAACCT CCTATAAAGC TTAGCTGCCC 1980

CTTCTGAGGA TGCTGAGAAC GGTGTCTTTC TTTATAAATG CAAATGGCTA CCGTTTTACA 2040

ATAAAATTTT GCATGTGCAA AAAAAAAAAA ANAAAAAAAA AAAATCCCGG GGGGGGGCCG 2100

GTAACCAATT TGNCCCC 2117

(2) INFORMATION FOR SEQ ID NO: 159:

(i) SEQUENCE CHARACTERISTICS:
(A) LENGTH: 2395 base pairs
(B) TYPE: nucleic acid
(C) STRANDEDNESS: double
(D) TOPOLOGY: linear

(xi) SEQUENCE DESCRIPTION: SEQ ID NO: 159:

TGTTCCTTAA TCCCTTTTCT AAAAAGGGGG GAAAATCCGG ATGGATTTTA GGGATTGGTC 60

TGGTGTCAGC TGTGTTTTAT TGCACACCTA AATCCTGATT ATAGGCTTTT CATTTCTCCG 120

CAAAGCCTTT ATTTTGGCAG TTAAGCCAAA TGTGTTTTCC AGAAAGTTAG TTATTTTCTC 180

CTCTTTCTTT CCTTTCTTTC CTCCCTTTTT CCCGTCTGAC CCCAAACGTT ATTGTCCAAA 240

CATGACTGGA CAGCAGCTTT TGTTTCTTGA CCCTGTAATA TGACAGTCTG CTAATATTGA 300

CAGAAGGTGC AGTTTTTGGG TTATAGTCGT GATTTTCGCT AATCAATCAT ATTAGCAGGA 360

AAAAAAAKGA CTTGTTTCTG TTGTACTTGA GTCTTAAGAA AAAGTGGCCC ATAGTTTAGT 420

GGACAATTTC CAAAGGCTTT AGTACCACCT GTATTTCAAA ATGGGGGACC CAAACTCCCG 480

GAAGAAACAA GCTCTGAACA GACTACGTGC TCAGCTTAGA AAGAAAAAAG AATCTCTAGC 540

TGACCAGTTT GACTTCAAGA TGTATATTGC CTTTGTATTC AAGGAGAAGA AGAAAAAGTC 600

AGCACTTTTT GAAGTGTCTG AGGTTATACC AGTCATGACA AATAATTATG AAGAAAATAT 660

CCTGAAAGGT GTGCGAGATT CCAGCTATTC CTTGGAAAGT TCCCTAGAGC TTTTACAGAA 720

GGATGTGGTA CAGCTCCATG CTCCTCGATA TCAGTCTATG AGAAGGGATG TAATTGGCTG 780

TACTCAGGAG ATGGATTTCA TTCTTTGGCC TCGGAATGAT ATTGAAAAAA TCGTCTGTCT 840

CCTGTTTTCT AGGTGGAAAG AATCTGATGA GCCTTTTAGG CCTGTTCAGG CAAATTTGAG 900

TTTCATCATG GTGACTATGA AAAACAGTTT CTGCATGTAC TGAGCCGCAA GGACAAGACT 960

GGAATCGTTG TCAACAATCC TAACCAGTCA GTGTTTCTCT TCATTGACAG ACAGCACTTG 1020

CAGACTCCAA AAAACAAAGC TACAATCTTC AAGTTATGCA GCATCTGCCT CTACCTGCCA 1080

CAGGAACAGC TCACCCACTG GGGCAGTTGG CACCATAGAG GRTCACCTCC GTCCTTATAT 1140

GCCAGAGTAG AGTACTGACC AGCAAAATGG AGAAGATCAG AGAATGCAGC AGCAGTTTTT 1200

TTTCTTGTTT TCTTACCACT TTATTCTTTC AGAGTTTAAA GAAAATGGAC TCATGCACAG 1260

AACACTATGC ATTTTGAAAC TTGTTCATCC TGGATTTTTT TAAATCATTT TTATCTCAGA 1320

ACTTAAACAA AAATTAGATG TCGTGCACGG ACTGTGTGAA AGAAGATGCT TTGCATATTT 1380

GCTGCACTGC ATCAGTATCT TACTAAAAAT GTGAAATGAA AGGACTATTG TACACTGAAA 1440

TGCTTAAATG TATCTGAAAG CACAAGGTGA TACTCATTTT TATGGTCTTC CCATTTGTGC 1500

TGGTTTTTGC CTCTTTGACA TCTGTCATCA GTATTTAGAG GGTGAGAAGT GAATGTAACA 1560

GGTATAAATA ACATTTTTAA AAACAATAAC TTTGCTATAA TCACAGTTGT TCCAGAGCAC 1620

TGTCAGATAC ATTCTAATGA CCAGAACTGG TTTAAAAAAA GAAAATACAA CCATGGGAAA 1680

GAAATCTTAA ATGAAAAACG CATCTCATTG TAGGCATTTT TGCCTCATAT TTTACTGGGC 1740

CATGTTTGTT TCCTGGTACT CATGTATTTT TTTTTTCCAG ATCTCTTTCC CCAAGTTGCT 1800

ATTGTAAGAG TATTCTGCTG CGTGTGGATG CAGTTATACA CATTAAAGCA GATCTGGAGT 1860

CTGAAGTAGC TATAAAGCAG CTATAAAACA GAAATACATG CATAGCTGCA GAAACCATGA 1920

TAGGTAGAGG ACTTTTCTTT TGGTTTTGTT TTGTTTTGTT TTGTTTTGTT TTTGGTTTTA 1980

CAGAGAAGAG ATTTTTATTA CAAAGAAAAA AATTCCAGTG AATTGTGCAG AAATGCTGGT 2040

TTTTACACCA TCCTAAAGAA AAACTTTACA AGGGTGTTTT GGAGTAGAAA AAAGGTTATA 2100

AAGTTGGAAT CTTAAATTGT AAAATTAACC ATTGAGTGTC AAAGTTCTAA AAGCAGAACT 2160

CATTTTGTGC AATGAACATA AGGAAAGACT ACTGTATAGG TTTTTTTTTT TTCTCCTTTT 2220

AAATGAAGAA AAGCTTTGCT TAAGGGTTGC ATACTTTTAT TGGAGTAAAT CTGAATGATC 2280

CTACTCCTTT GGAGTAAAAC TAGTGCTTAC CAGTTTCCAA TTGTATTTAG CTTCTGGTTG 2340

GAATTTGAAA AAAAAAGAAA AAAAGAAAAA GAAAACCTAA ATAAAATAGG TGAAA 2395

(2) INFORMATION FOR SEQ ID NO: 160:

(i) SEQUENCE CHARACTERISTICS:
(A) LENGTH: 2120 base pairs
(B) TYPE: nucleic acid
(C) STRANDEDNESS: double

(D) TOPOLOGY: linear

(xi) SEQUENCE DESCRIPTION: SEQ ID NO: 160:

CCCCGGATAC CGCCTGACGT AGTGCCAATC ACACCTCTCG CGTCTCGGCG CCTCGGAGGC 60

TAATGAGGAC GCCTGGCGAA ACGCAGTAAC GGATTTCCGG GTGGACCTTC GCTTTACGGC 120

TCGTGAGTTC TTCCGCCCAA CCCAGAGGAA GCGGGAGAGC AGTTTACGAC AGCGCCGGTC 180

GTGTTTACGG CGGCGCCCGC TGCGCGCGCA TGTTTCCTCT TTTCCTGGTT TCTCAAGAGT 240

GCTGCTGCTA ACGCGGTCCC CGGCACGCAC CATCTGTTGC CATCCCGGCC GGCCGAGGCA 300

TTGCAGATTT TGGAAGATGG CAAAGTTCAT GACACCCGTG ATCCAGGACA ACCCCTCAGG 360

CTGGGGTCCC TGTGCGGTTC CCGAGCAGTT TCGGGATATG CCCTACCAGC CGTTCAGCAA 420

AGGAGATCGG CTAGGAAAGG TTGCAGACTG GACAGGAGCC ACATACCAAG ATAAGAGGTA 480

CACAAATAAG TACTCCTCTC AGTTTGGTGG TGGAAGTCAA TATGCTTATT TCCATGAGGA 540

GGATGAAAGT AGCTTCCAGC TGGTGGATAC AGCGCGCACA CAGAAGACGG CCTACCAGCG 600

GAATCGAATG AGATTTGCCC AGAGGAACCT CCGCAGAGAC AAAGATCGTC GGAACATGTT 660

GCAGTTCAAC CTGCAGATCC TGCCTAAGAG TGCCAAACAG AAAGAGAGAG AACGCATTCG 720

ACTGCAGAAA AAGTTCCAGA AACAATTTGG GGTTAGGCAG AAATGGGATC AGAAATCACA 780

GAAACCCCGA GACTCTTCAG TTGAAGTTCG TAGTGATTGG GAAGTGAAAG AGGAAATGGA 840

TTTTCCTCAG TTGATGAAGA TGCGCTACTT GGAAGTATCA GAGCCACAGG ACATTGAGTG 900

TTGTGGGGCC CTAGAATACT ACGACAAAGC CTTTGACCGC ATCACCACGA GGAGTGAGAA 960

GCCACTGCGG ASATNCAAGC GCATCTTCCA CACTGTCACC ACCACAGACG ACCCTGTCAT 1020

CCGCAAGCTG GCAAAAACTC AGGGGAATGT GTTTGCCACT GATGCCATCC TGGCCACGCT 1080

GATGAGCTGT ACCCGCTCAG TGTATTCCTG GGATATTGTC GTCCAGAGAG TTGGGTCCAA 1140

ACTCTTCTTT GACAAGAGAG ACAACTCTGA CTTTGACCTC CTGACAGTGA GTGAGACTGC 1200

CAATGAGCCC CCTCAAGATG AAGGTAATTC CTTCAATTCA CCCCGCAACC TGGCCATGGA 1260

GGCAACCTAC ATCAACCACA ATTTCTCCCA GCAGTGCTTG AGAATGGGGA AGGAAAGATA 1320

CAACTTCCCC AACCCAAACC CGTTTGTGGA GGACGACATG GATAAGAATG AAATCGCCTC 1380

TGTTGCGTAC CGTTACCGCA GTGGNAAGCT TGGAGATGAT ATTGACCTTA TTGTCCGTTG 1440

TGAGCACGAT GGCGTCATGA CTGGAGCCAA CGGGGAAGTG TCCTTCATCA ACATCAAGAC 1500

ACTCAATGAG TGGGATTCCA GGCACTGTAA TGGCGTTGAC TGGCGTCAGA AGCTGGACTC 1560

TCAGCGAGGG GCTGTCATTG CCACGGAGCT GAAGAACAAC AGCTACAAGT TGGCCCGGTG 1620

GACCTGCTGT GCTTTGCTGG CTGGATCTGA GTACCTCAAG CTTGGTTATG TGTCTCGGTA 1680

CCACGTGAAA GACTCCTCAC GCCACGTCAT CCTAGGCACC CAGCAGTTCA AGCCTAATGA 1740

GTTTGCCAGC CAGATCAACC TGAGCGTGGA GAATGCCTGG GGCATTTTAC GCTGCGTCAT 1800

TGACATCTGC ATGAAGCTGG AGGAGGGCAA ATACCTCATC CTCAAGGACC CCAACAAGCA 1860

GGTCATCCGT GTCTACAGCC TCCCTGATGG CACCTTCAGC TCTGATGAAG ATGAGGAGGA 1920

AGAGGAGGAG GAAGAAGAGG AAGAAGAAGA GGAAGAAACT TAAACCAGTG ATGTGGAGCT 1980

GGAGTTTGTC CTTCCACCGA GACTACGAGG GCCTTTGATG CTTAGTGGAA TGTGTGTCTA 2040

ACTTGCTCTC TGACATTTAG CAGATGAAAT AAAATATATA TCTGTTTAGT CTTAAAAAAA 2100

AAAAAAAAAA AAAAAAAAAN 2120

(2) INFORMATION FOR SEQ ID NO: 161:

(i) SEQUENCE CHARACTERISTICS:
(A) LENGTH: 900 base pairs
(B) TYPE: nucleic acid
(C) STRANDEDNESS: double
(D) TOPOLOGY: linear

(xi) SEQUENCE DESCRIPTION: SEQ ID NO: 161:

GGAAGCTGAA GTCCTTCCAG ACCAGGGACA ACCAGGGCAT TCTCTATGAA GCTGCACCCA 60

CCTCCACCCT CACCTGTRAC TCAGGACCAC AGAAGCAAAA GTTCTCACTC AAACTGGATG 120

CCAAGGATGG GCGCTTGTTC AATGAGCAGA ACTTCTTCCA GCGGGCCGCC AAGCCTCTGC 180

AAGTCAACAA GTGGAAGAAG CTGTACTCGA CCCCACTGCT GGCCATCCCT ACCTGCATGG 240

GTTTCGGTGT TCACCAGGAC AAATACAGGT TCTTGGTGTT ACCCAGCCTG GGGAGGAGCC 300

TTCAGTCGGC CCTGGATGTC AGCCCAAAGC ATGTGCTGTG CAGAGAGGTC TGTGCTGCAG 360

GTGGCCTGCC GGCTGCTGGA TGCCCTGGAG TTCCTCCATG AGAATGAGTA TGTTCATGGA 420

AATGTGACAG CTGAAAATAT CTTTGTGGAT CCAGAGGACC AGAGTCAGGT GACTTTGGCA 480

GGCTATGGCT TCGCNTTCCG CTATTGCCCA AGTGGCAAAC ACGTGGCCTA CGTGGAAGGC 540

AGCAGGAGCC CTCACGAGGG GGACCTTGAG TTCATTAGCA TGGACCTGCA CAAGGGATGC 600

GGGCCCTCCC GCCGCRGCGA CCTCCAGAGC CTGGGCTACT GCATGCTGAA GTGGCTCTAC 660

GGGTTTCTGC CATGGACAAA TTGCCTTCCC AAMAMTGAGG ACATCATGAA GCAAAAACAG 720

AAGTTTGTTG ATAAGCCGGG GCCCTTCGTG GGACCCTGCG GTCACTGGAT CAGGCCCTCA 780

GAGACCCTGC AGAAGTACCT GAAGGTGGTG ATGGCCCTCA CGTATGAGGA GAAGCCGCCC 840

TACGCCATGC TGAGGAACAA CCTAGAAGCT TTGCTGCAGG ATCTGCGTGT GTCTCCATAT 900

(2) INFORMATION FOR SEQ ID NO: 162:

(i) SEQUENCE CHARACTERISTICS:
(A) LENGTH: 1003 base pairs
(B) TYPE: nucleic acid
(C) STRANDEDNESS: double
(D) TOPOLOGY: linear

(xi) SEQUENCE DESCRIPTION: SEQ ID NO: 162:

GGCACGAGAT GAGGGGCACC CAGTGCTTCT AGGGCAGGCT GGGTGGTGGT CCCCTAGGTA 60

TCAGCCTCTC TTACTGTACT CTCCGGGAAT GTTAACCTTT CTATTTTCAG CCTGTGCCAC 120

CTGTCTAGGC AAGCTGGCTT CCCCATTGGC CCCTGTGGGT CCACAGCAGC GTGGCTGCCC 180

CCCAGGGCCA CCGCTTCTTT CTTGATCCTC TTTCCTTAAC AGTGACTTGG GCTTGAGTCT 240

GGCAAGGAAC CTTGCTTTTA GCTTCACCAC CAAGGAGAGA GGTTGACATG ACCTCCCCGC 300

CCCCTCACCA AGGCTGGGAA CAGAGGGGAT GTGGTGAGAG CCAGGTTCCT CTGGCCCTCT 360

CCAGGGTGTT TTCCACTAGT CACTACTGTC TTCTCCTTGT AGCTAATCAA TCAATATTCT 420

TCCCTTGCCT GTGGGCAGTG GAGAGGCTGC TGGGTGTACG CTGCACCTGC CCACTGAGTT 480

GGGGAAAGAG GATAATCAGT GAGCACTGTT CTGCTCAGAG CTCCTGATCT ACCCCACCCC 540

CTAGGATCCA GGACTGGGTC AAAGCTGCAT GAAACCAGGC CCTGGCAGCA AACCTGGGAA 600

TGGCTGGAGG TGGGAGAGAA CCTGAACTTC TCTTTCCCTC TCCCTCCTCC AACATTACTG 660

GAACTCTATC CTGTTAGGAT CTTCTGAGCT TGTTTCCCTG CTGGGTGGGA CAGAGGACAA 720

AGGAGAAGGG AGGGTCTAGA AGAGGCAGCC CTTCTTTGTC CTCTGGGGTA AATGAGCTTG 780

ACCTAGAGTA AATGGAGAGA CCAAAAGCCT CTGATTTTTA ATTTCCATAA AATGTTAGAA 840

GTATATATAT ACATATATAT ATTTCTTTAA ATTTTTGAGT CTTTGATATG TCTAAAAATC 900

CATTCCCTCT GCCCTGAAGC CTGAGTGAGA CACATGAAGA AAACTGTGTT TCATTTAAAG 960

ATGTTAATTA AATGATTGAA ACTTGAAAAA AAAAAAAAAA AAA 1003

(2) INFORMATION FOR SEQ ID NO: 163:

(i) SEQUENCE CHARACTERISTICS:
(A) LENGTH: 2196 base pairs
(B) TYPE: nucleic acid
(C) STRANDEDNESS: double
(D) TOPOLOGY: linear

(xi) SEQUENCE DESCRIPTION: SEQ ID NO: 163:

AAGAAGCGGC ACACGGATGT GCAGTTCTAC ACAGAAGTGG GAGAGATAAC CACGGACTTG 60

GGGAAACATC AGCATATGCA TGACCGAGAT GACCTCTATG CTGAGCAGAT GGAACGAGAA 120

ATGAGGCACA AACTGAAAAC AGCCTTTAAA AATTTCATTG AGAAAGTAGA GGCTCTAACT 180

AAGGAGGAAC TGGAATTTGA AGTGCCTTTT AGGGACTTGG GATTTAACGG AGCTCCCTAT 240

AGGAGTACCT GCCTCCTTCA GCCCACTAGT AGTGCGCTGG TAAATGCTAC GGAATGGCCA 300

CCTTTTGTGG TGACATTGGA TGAGGTAGAG CTGATCCACT TTRAGCGGGT CCAGTTTCAC 360

CTGAAGAACT TTGATATGGT AATCGTCTAC AAGGACTACA GCAAGAAAGT GACCATGATC 420

AACGCCATTC CTGTAGCCTC TCTTGACCCC ATCAAGGAAT GGTTGAATTC CTGCGACCTG 480

AAATACACAG AAGGAGTACA GTCCCTCAAC TGGACTAAAA TCATGAAGAC CATTGTTGAT 540

GACCCTGAGG GCTTCTTCGA ACAAGGTGGC TGGTCTTTCC TGGAGCCTGA GGGTGAGGGG 600

AGTGATGCTG AAGAAGGGGA TTCAGAGTCT GAAATTGAAG ATGAGACTTT TAATCCTTCA 660

GAAGATGACT ATGAAGAGGA AGAGGAGGAC AGTGATGAAG ATTATTCATC AGAAGCAGAA 720

GAGTCAGACT ATTCTAAGGA GTCATTGGGT AGTGAAGAAG AGAGTGGAAA GGATTGGGAT 780

GAACTGGAGG AAGAAGCCCG AAAAGCGGAC CGAGAAAGTC GTTACGAGGA AGAAGAAGAA 840

CAAAGTCGAA GTATGAGCCG GAAGAGGAAG GCATCTGTGC ACAGTTCGGG CCGTGGCTCT 900

AACCGTGGTT CCAGACACAG CTCTGCACCC CCCAAGAAAA AGAGGAAGTA ACTTCTGAAC 960

TTTGGCCCTG AGCTCCATTC TTCCTCCAGC CAACCCCTGA AAATTTTACA TGACATAGAA 1020

ACTGTATTTT TCCTTTCGTT TTCATTTGAA GTTTTGCCAT TTGTGTTTAT GGGTTTAGGG 1080

GGCCATTTGT GTGGACCAAT CTACTCGGGG AATTCCAGGC CCACCAGGAC ACGTGCCAAT 1140

GGCCCCATTC AGATGGCAAG GGAGGAGGTG TTCTTGAAGA CAGGAGGAGG CTCCCGCTGT 1200

TAATAAATAT TGTTTCATTC TTCTCTCTTC CTGTCACCTT CTGCCAAGAC ATTGATGGCT 1260

TCTGACATCT TATTTGGTGT CTCAAAGCTG TATTTCCAAG ACAGTGGTAC AAGGTGACCC 1320

TTAATTACCC GTATCATGGT TCTTGACCAG CACATTCAAT CCTCCAACCT ACCCTACTGC 1380

CATGACCTTC CGCACATCTC TAAGTTTTAT CTTTGCAATA CTCAAGGTTC TCGGAAATTT 1440

GCTAATGGTT GTGATAAACC ATACAGCTTG AGCCAGTGAG GCAGATTGGG CTGGTGCCTT 1500

CGTCTGAGTT TTCCTGCTTT CCTGCCTCGT GCAGATTCTG AGGTATATCT GCTGCCTTGG 1560

AAGACATAAG AAGCAGTGAT ACTCCCTGGC TCGGTTATTT TCTCCATACA ATGCACACAT 1620

GGTACAATGA TAGAAGGCAA AATTGCCACT GTCTTCTTTT TTTTCTCATA TATCTAAGGA 1680

AGATATATCA GGTTGTGCCT CATGTACCGC TTCTAGTGAA ATGTAGAGGA AGGCTCAAAG 1740

GAGTCAACAT TTAGATCTGG AAGGGACAAG TCATGCCTTG GGCCTAGAAT ACCCTGATGA 1800

GAAAAGAGAA GAGGAAGGGA GGCCATATCT ACAACANCAN CCTCTCGGCA CTGCTGCTCC 1860

TTATTTTAAC TTTGTCTTGC ATTGTCCTGT ATTTATCACA GTTTCTGTTG AACAGCTTTT 1920

CAAGTATTTG GGGAGTTTAT CTTGCCATCC TCCCCTTCTG GTTCTCTGCA CCCACCTGTC 1980

CCACTGCAGT TCCTTCCGTG CTCTGTGACT TTAAGAGAAG AAGGGGGGAG GGGTCCCGGA 2040

TTTTATGTTT GTTTGTTTTT TCTCCTTAGC AGTAGGACTT GATATTTTCA ATTTTGGAAG 2100

AACTAAAAGA TGAATAAACT GGGTTTTTTT TGTTGTTTGT TTTTGTAAAA AAAAAAAAAA 2160

AAAAAAAAAA AAAAAAAAAA AAAAAAAAAA AAAAAA 2196

(2) INFORMATION FOR SEQ ID NO: 164:

(i) SEQUENCE CHARACTERISTICS:
(A) LENGTH: 1945 base pairs
(B) TYPE: nucleic acid
(C) STRANDEDNESS: double
(D) TOPOLOGY: linear

(xi) SEQUENCE DESCRIPTION: SEQ ID NO: 164:

GCACAGAGTC GGGCGGACGG ACAGGGAGAG GAGGAGAGGG GGTCTGCGCG CGGCCGCTAC 60

CCAGAAGCCA GCGGACGGCA GCACGGAGTG GGCTGTCCCC GAGCCCAGCC CCGAGCGAGC 120

CCCCCCCCCG CCCCCGMAGG ACGCGCCTYC CAGCCAGCCC GACTYCTAGG AGGAGGGGAG 180

GCGGGAAAGC AGCTCAAGCC TCACCCACCG CCCTGCCCCC AGCCCCGCCA CTCCCAGGCT 240

CCTCGGGACT CGGCGGGTCC TCCTGGGAGT CTCGGAGGGG ACCGGCTGTG CAGACGCCAT 300

GGAGTTGGTG CTGGTCTTCC TCTGCAGCCT GCTGGCCCCC ATGGTCCTGG CCAGTGCAGC 360

TGAAAAGGAG AAGGAAATGG ACCCTTTTCA TTATGATTAC CAGACCCTGA GGATTGGGGG 420

ACTGGTGTTC GCTGTGGTCC TCTTCTCGGT TGGGATCCTC CTTATCCTAA GTCGCAGGTG 480

CAAGTGCAGT TTCAATCAGA AGCCCCGGGC CCCAGGAGAT GAGGAAGCCC AGGTGGAGAA 540

CCTCATCACC GCCAATGCAA CAGAGCCCCA GAAAGCAGAG AACTGAAGTG CAGCCATCAG 600

GTGGAAGCCT CTGGAACCTG AGGCGGCTGC TTGAACCTTT GGATGCAAAT GTCGATGCTT 660

AAGAAAACCG GCCACTTCAG CAACAGCCCT TTCCCCAGGA GAAGCCAAGA ACTTGTGTGT 720

CCCCCACCCT ATCCCCTCTA ACACCATTCC TCCACCTGAT GATGCAACTA ACACTTGCCT 780

CCCCACTGCA GCCTGCGGTC CTGCCCACCT CCCGTGATGT GTGTGTGTGT GTGTGTGTGT 840

GTGACTGTGT GTGTTTGCTA ACTGTGGTCT TTGTGGCTAC TTGTTTGTGG ATGGTATTGT 900

GTTTGTTAGT GAACTGTGGA CTCGCTTTCC CAGGCAGGGG CTGAGCCACA TGGCCATCTG 960

CTCCTCCCTG CCCCCGTGGC CCTCCATCAC CTTCTGCTCC TAGGAGGCTG CTTGTTGCCC 1020

GAGACCAGCC CCCTCCCCTG ATTTAGGGAT GCGTAGGGTA AGAGCACGGG CAGTGGTCTT 1080

CAGTCGTCTT GGGACCTGGG AAGGTTTGCA GCACTTTGTC ATCATTCTTC ATGGACTCCT 1140

TTCACTCCTT TAACAAAAAC CTTGCTTCCT TATCCCACCT GATCCCAGTC TGAAGGTCTC 1200

TTAGCAACTG GAGATACAAA GCAAGGAGCT GGTGAGCCCA GCGTTGACGT CAGGCAGGCT 1260

ATGCCCTTCC GTGGTTAATT TCTTCCCAGG GGCTTCCACG AGGAGTCCCC ATCTGCCCCG 1320

CCCCTTCACA GAGCGCCCGG GGATTCCAGG CCCAGGGCTT CTACTCTGCC CCTGGGGAAT 1380

GTGTCCCCTG CATATCTTCT CAGCAATAAC TCCATGGGCT CTGGGACCCT ACCCCTTCCA 1440

ACCTTCCCTG CTTCTGAGAC TTCAATCTAC AGCCCAGCTC ATCCAGATGC AGACTACAGT 1500

CCCTGCAATT GGGTCTCTGG CAGGCAATAG TTGAAGGACT CCTGTTCCGT TGGGGCCAGC 1560

ACACCGGGAT GGATGGAGGG AGAGCAGAGG CCTTTGCTTC TCTGCCTACG TCCCCTTAGA 1620

TGGGCAGCAG AGGCAACTCC CGCATCCTTT GCTCTGCCTG TCRGTGGTCA GAGCGGTGAG 1680

CGAGGTGGGT TGGAGACTCA GCAGGCTCCG TGCAGCCCTT GGGAACAGTG AGAGGTTGAA 1740

GGTCATAACG AGAGTGGGAA CTCAACCCAG ATCCCGCCCC TCCTGTCCTC TGTGTTCCCG 1800

CGGAAACCAA CCAAACCGTG CGCTGTGACC CATTGCTGTT CTCTGTATCG TGATCTATCC 1860

TCAACAACAA CAGAAAAAAG GAATAAAATA TCCTTTGTTT CCTAGTGAAA AAAAAAAAAA 1920

AAAAAAAAAA AAAAAAAAAA CTCGA 1945

(2) INFORMATION FOR SEQ ID NO: 165:

(i) SEQUENCE CHARACTERISTICS:
(A) LENGTH: 2933 base pairs
(B) TYPE: nucleic acid
(C) STRANDEDNESS: double
(D) TOPOLOGY: linear

(xi) SEQUENCE DESCRIPTION: SEQ ID NO: 165:

GGGTCGACCC ACGCGTCCGG CAGCCGTCGT TTGAGTCGTT GCTGCCGCTG CCCCCTCCCG 60

GATCAGGAGC CAGTGTATAC CGCCCGCCCA CCGCCTTGGT GCCGCTAGAG GAAACGAGAA 120

GGAGGCCGCC TGCGGTTTGT CGCCGCAGCT CGCCCMCYGY CYGGRAGAGC CGAGCCCCGG 180

CCCAGTCGGT CGCYTGCCAC CSCTCGTAGC CGTTACCCGC GGGCCGCCAC AGCCGCCGGC 240

CGGGAGAGGC GCGCGCCATG GCYTCTGGAG CCGATTCAAA AGGTGATGAC CTATCAACAG 300

CCATTCTCAA ACAGAAGAAC CGTCCCAATC GGTTAATTGT TGATGAAGCC ATCAATGAGG 360

ACAACAGTGT GGTGTCCTTG TCCCAGCCCA AGATGGATGA ATTGCAGTTG TTCCGAGGTG 420

ACACAGTGTT GCTGAAAGGA AAGAAGAGAC GAGAAGCTGT TTGCATCGTC CTTTCTGATG 480

ATACTTGTTC TGATGAGAAG ATTCGGATGA ATAGAGTTGT TCGGAATAAC CTTCGTGTAC 540

GCCTAGGGGA TGTCATCAGC ATCCAGCCAT GCCCTGATGT GAAGTACGGC AAACGTATCC 600

ATGTGCTGCC CATTGATGAC ACAGTGGAAG GCATTACTGG TAATCTCTTC GAGGTATACC 660

TTAAGCCGTA CTTCCTGGAA GCGTATCGAC CCATCCGGAA AGGAGACATT TTTCTTGTCC 720

GTGGTGGGAT GCGTGCTGTG GAGTTCAAAG TGGTGGAAAC AGATCCTAGC CCTTATTGCA 780

TTGTTGCTCC AGACACAGTG ATCCACTGCG AAGGGGAGCC TATCAAACGA GAGGATGAGG 840

AAGAGTCCTT GAATGAAGTA GGGTATGATG ACATTGGTGG CTGCAGGAAG CAGCTAGCTC 900

AGATAAAGGA GATGGTGGAA CTGCCCCTGA GACATCCTGC CCTCTTTAAG GCAATTGGTG 960

TGAAGCCTCC TAGAGGAATC CTGCTTTACG GACCTCCTGG AACAGGAAAG ACCCTGATTG 1020

CTCGAGCTGT AGCAAATGAG ACTGGAGCCT TCTTCTTCTT GATCAATGGT CCTGAGATCA 1080

TGAGCAAATT GGCTGGTGAG TCTGAGAGCA ACCTTCGTAA AGCCTTTGAG GAGGCTGAGA 1140

AGAATGCTCC TGCCATCATC TTCATTGATG AGCTAGATGC CATCGCTCCC AAAAGAGAGA 1200

AAACTCATGG CGAGGTGGAG CGGCGCATTG TATCACAGTT GTTGACCCTC ATGGATGGCC 1260

TAAAGCAGAG GGCACATGTG ATTGTTATGG CAGCAACCAA CAGACCCAAC AGCATTGACC 1320

CAGCTCTACG GCGATTTGGT CGCTTTGACA GGGAGGTAGA TATTGGAATT CCTGATGCTA 1380

CAGGACGCTT AGAGATTCTT CAGATCCATA CCAAGAACAT GAAGCTGGCA GATGATGTGG 1440

ACCTGGAACA GTAGCCAATG AGACTCACGG GCATGTGGGT GCTGACTTAG CAGCCCTGTG 1500

CTCAGAGGCT GCTCTGCAAG CCATCCGCAA GAAGATGGAT CTCATTGACC TAGAGGATGA 1560

GACCATTGAT GCCGAGGTCA TGAACTCTCT AGCAGTTACT ATGGATGACT TCCGGTGGGC 1620

CTTGAGCCAG AGTAACCCAT CAGCACTGCG GGAAACCGTG GTAGAGGTGC CACAGGTAAC 1680

CTGGGAAGAC ATCGGGGGCC TAGAGGATGT CAAACGTGAG CTACAGGAGC TGGTCCAGTA 1740

TCCTGTGGAG CACCCAGACA AATTCCTGAA GTTTGGCATG ACACCTTCCA AGGGAGTTCT 1800

GTTCTATGGA CCTCCTGGCT GTGGGAAAAC TTTGTTGGCC AAAGCCATTG CTAATGAATG 1860

CCAGGCCAAC TTCATCTCCA TCAAGGGTCC TGAGCTGCTC ACCATGTGGT TTGGGGAGTC 1920

TGAGGCCAAT GTCAGAGAAA TCTTTGACAA GGCCCGCCAA GCTGCCCCCT GTGTGCTATT 1980

CTTTGATGAG CTGGATTCGA TTGCCAAGGC TCGTGGAGGT AACATTGGAG ATGGTGGTGG 2040

GGCTGCTGAC CGAGTCATCA ACCAGATCCT GACAGAAATG GATGGCATGT CCACAAAAAA 2100

AAATGTGTTC ATCATTGGCG CTACCAACCG GCCTGACATC ATTGATCCTG CCATCCTCAG 2160

ACCTGGCCGT CTTGATCAGC TCATCTACAT CCCACTTCCT GATGAGAAGT CCCGTGTTGC 2220

CATCCTCAAG GCTAACCTGC GCAAGTCCCC AGTTGCCAAG GATGTGGACT TGGAGTTCCT 2280

GGCTAAAATG ACTAATGGCT TCTCTGGAGC TGACCTGACA GAGATTTGCC AGCGTGCTTG 2340

CAAGCTGGCC ATCCGTGAAT CCATCGAGAG TGAGATTAGG CGAGAACGAG AGAGGCAGAC 2400

AAACCCATCA GCCATGGAGG TAGAAGAGGA TGATCCAGTG CCTGAGATCC GTCGAGATCA 2460

CTTTGAAGAA GCCATGCGCT TTGCGCGCCG TTCTGTCAGT GACAATGACA TTCGGAAGTA 2520

TGAGATGTTT GCCCAGACCC TTCAGCAGAG TCGGGGCTTT GGCAGCTTCA GATTCCCTTC 2580

AGGGAACCAG GGTGGAGCTG GCCCCAGTCA GGGCAGTGGA GGCGGCACAG GTGGCAGTGT 2640

ATACACAGAA GACAATGATG ATGACCTGTA TGGCTAAGTG GTGGTGGCCA GCGTGCAGTG 2700

AGCTGGCCTG CCTGGACCTT GTTCCCTGGG GGTGGGGGCG CTTGCCCAGG AGAGGGACCA 2760

GGGGTGCGCC CACAGCCTGC TCCATTCTCC AGTCTGAACA GTTCAGCTAC AGTCTGACTC 2820

TGGACAGGGG GTTTCTGTTG CAAAAATACA AAACAAAAGC GATAAAATAA AAGCGATTTT 2880

CATTTGGTAA AAAAAAAAAA AAAAAAAAAT CCGGGGGGGG GCCCGAACCA TTT 2933

(2) INFORMATION FOR SEQ ID NO: 166:

(i) SEQUENCE CHARACTERISTICS:
(A) LENGTH: 2243 base pairs
(B) TYPE: nucleic acid
(C) STRANDEDNESS: double
(D) TOPOLOGY: linear

(xi) SEQUENCE DESCRIPTION: SEQ ID NO: 166:

TCGGAGAGCC GGCGGGCGNG CGCCTCTCGG CCAGGAAGCG CCTCTTGGAC GCGTGTNACC 60

GATGCCCAGA AGTGGCCTTG GGCTGGGGAT CACCATAGCT TTTCTAGCTA CGCTGATCAC 120

GCAGTTTCTC GTGTATAATG GTGTCTATCA GTATACATCC CCAGATTTCC TCTATATTCG 180

TTCTTGGCTC CCTTGTATAT TTTTCTCAGG AGGCGTCACG GTGGGGAACA TAGGACGACA 240

GTTAGCTATG GGTGTTCCTG AAAAGCCCCA TAGTGATTGA GTCTTCAAAA CCACCGATTC 300

TGAGAGCAAG GAAGATTTTG GAAGAAAATC TGACTGTGGA TTATGACAAA GATTATCTTT 360

TTTCTTAAGT AATCTATTTA GATCGGGCTG ACTGTACAAA TGACTCCTGG AAAAAACTCT 420

TCACCTAGTC TAGAATAGGG AGGTGGAGAA TGATGACTTA CCCTGAAGTC TTCCCTTGAC 480

TGCCCGCACT GGCGCCTGTC TGTGCCCTGG AGCATTCTGC CCAGGCTACG TGGGTTCAGG 540

CAGGTGGCAG CTTCCCAAGT ATTCGATTTC ATTCATGTGA TTAAAACAAG TTGCCATATT 600

TCAAAGCCTT GAACTAAGAC TCAATTACCA ACCCGCAGTT TTGTGTCAGT GCCCAAAGGA 660

GGTAGGTTGA TGGTGCTTAA CAAACATGAA GTATGGTGTA ATAGGAATAA TATTTATCCA 720

AAAGATTTTT AAAAATAGGG CTGTGTTTAA AAAAAAAAAC AAAACARGAA AAGCAGCAGT 780

GATTATAGAG AGGTCACACT CTAAGTGGGG TCGCGGCGTG GCCACGCTTC ACGGTCACGC 840

TCGTCCGTCC TGCAGTGGCG TGTTTACATG GTCACACGTG TGTGTATCAC CAGTGGGTCA 900

ACTGCTTGTC ATTCCTCCCG TGGCAGTTTG TGTAGACAAT CTTACTGAGC AAAAGGCAAT 960

GAAAAGTCTT GGTTCCCACA CTGCGATATA TTGGAATTTT CACCTCAGTT TATGAAGTTT 1020

ATTTCGAAAT CCATAGTCAT CTAAGAATGA ATACCTGTCT GCCATGTATT TCAATCTTAG 1080

TGAGCCAAAA TTGTTTGTTT GTTACTACAG AATAGAGATG ACTGTTTTTT GCCACAGCCC 1140

TATGGRATTT GCAATCTGTG ATTGCCTTGT AAAAAGGAGA GTGCATATGG CACTGCATTA 1200

AACGTGTGGT GTTTCTAGTC AATGATATTG GTGAGCACAA TGTATTCATT TAATGGCATA 1260

GACCATACCA GACCTAATTT GCAAGTATTG GGTCTTAAAC TTCAAGTGCA ATGTATATGA 1320

AAACCAATCT GAGCCTTGTA TCTCTTAAAT ATTTATTTTT TTTAACGTGT GAGATGTTCG 1380

AGAGAAGGTT CTCCATTCAT TTCAGTGCTG CCTGGAGGAA ACTCGGCAAT GATTTCTTTC 1440

AGTTGTGAAG TTCCTTTCGT GTTACACCCT CCACTGAACC CTCAACCTTC GAAATACTCC 1500

AGTTTTGTGG GTTTGGTCAT TTTTACTTAT AAATTTACCT TTTTGTATTT TGCAATTTAC 1560

ATGTGTTTGG TTTGTTTTAA ATTCTGTGAA AGTGGCTTGA TTAAAAGACT CCTTTTAAAT 1620

GGAAGCCACC AGTCAGCAGA ATGGAAGCTT AGAGGAACTT GCCTGTGAGC GCTGGTCTTT 1680

GTGTTTGGTT TTGTGATGTA ACGATCTTTG CTGGGGTTTT TTGCTTTGTT TTGAGGGAAA 1740

TGTCTTGGAG TAAATTTTAA GTTCCTGGAG TTAATTTGTT TTACAGGAAT TTTGTTTTTT 1800

AAAAAAATAG GATCATTCTG AACTTTGGAA TGACCCCCTT ATATATTTTC TGAAAATGAA 1860

AACAGTTACA TGAAAAAAAT TTCCAATGAA GATGTCAGCA TTTTATGAAA AACCAGAAGT 1920

TATTAGATGA AAGCAGCGAG TGAATCTTTA AAACAGACTT GATCACGCAC ACACAATAAG 1980

TCTTTCTCTC CGAAACCGGA AGTAAATCTA TATCTGTTAG AAATAATGTA GCCAAAAGAA 2040

TGTAAATTTG AGGATTTTTT TGCCAATAGT TTATAGAAAA TATATGAACC AAAGTGATTT 2100

GAGTTTGTAA AAATGTAAAA TAGTATGAAC AAAATTTGCA CTCTACCAGA TTTGAACATC 2160

TAGTGAGGTT CACATTCATA CTAAGTTTTC AACATTGTGT TCTTTTTGCA TTCATTTTTT 2220

ACTTTTATTA AAGGTTCAAA ACC 2243

(2) INFORMATION FOR SEQ ID NO: 167:

(i) SEQUENCE CHARACTERISTICS:
(A) LENGTH: 1816 base pairs
(B) TYPE: nucleic acid

(C) STRANDEDNESS: double
(D) TOPOLOGY: linear

(xi) SEQUENCE DESCRIPTION: SEQ ID NO: 167:

GGTGGGNAGC TTTNAATTTC CCCTTACWGG GGCGCTNTAA GGGGAAACCT TCCCGGAATT 60

TTCGGGTCGA CCCACGCGTC CGGCCAGCCT AGGAGAAGAA GTTCGTAGTC CCAGAGGTGA 120

GGCAGGAGGC GGCAGTTTCT GGCGGGTGAG GGCGGAGCTG AAGTGACAGC GGAGGCGGAA 180

GCAACGGTCG GTGGGGCGGA GAAGGGGGCT GGCCCCAGGA GGAGGAGGAA ACCCTTCCGA 240

GAAAACAGCA ACAAGCTGAG CTGCTGTGAC AGAGGGGAAC AAGATGGCGG CGCCGAAGGG 300

GAGCCTCTGG GTGAGGACCC AACTGGGGCT CCCGCCGCTG CTGCTGCTGA CCATGGCCTT 360

GGCCGGAGGT TCGGGGACCG CTTCGGCTGA AGCATTTGAC TCGGTCTTGG GTGATACGGC 420

GTCTTGCCAC CGGGCCTGTC AGTTGACCTA CCCCTTGCAC ACCTACCCTA AGGAAGAAGA 480

GTTGTACGCA TGTCAGAGAG GTTGCAGGCT GTTTTCAATT TGTCAGTTTG TGGATGATGG 540

AATTGACTTA AATCGAACTA AATTGGAATG TGAATCTGCA TGTACAGAAG CATATTCCCA 600

ATCTGATGAG CAATATGCTT GCCATCTTGG KTGCCAGAAT CAGCTGCCAT TCGCTGAACT 660

GAGACAAGAA CAACTTATGT CCCTGATGCC AAAAATGCAC CTACTCTTTC CTCTAACTCT 720

GGTGAGGTCA TTCTGGAGTG ACATGATGGA CTCCGCACAG AGCTTCATAA CCTCTTCATG 780

GACTTTTTAT CTTCAAGCCG ATGACGGAAA AATAGTTATA TTCCRGTCTA AGCCCAGRAA 840

TCCCAGGTAC GCACCACATT TGGAGCCAGG AGCCCTACCA AATTTGRGRG RAWCMTCTCT 900

AAGCAAAATG TCCNTCAKMT CGSMAATGAG AAATTCACAA GCGCACAGGA ATTTTCTTGA 960

AGATGGAGAA AGTGATGGCT TTTTAAGATG CCTCTCTCTT AACTCTGGGT GGATTTTAAC 1020

TACAACTCTT GTCCTCTCGG TGATGGTATT GCTTTGGATT TGTTGTGCAA CTTGTTGCTA 1080

CACGCTGTTG GACGCAGTAT AGTTTCCCTC TGAGAAGCTG AGTATCTATG GTGACTTGGA 1140

GTTTATGAAT GAACAAAAGC TAAACAGATA TCCAGCTTCT TCTCTTGTGG TTGTTAGATC 1200

TAAAACTGAA GATCATGAAG AAGCAGGGCC TCTACCTACA AAAGTGAATC TTGCTCATTC 1260

TGAAATTTAA GCATTTTTCT TTTAAAAGAC AAGTGTAATA GACATCTAAA ATTCCACTCC 1320

TCATAGAGCT TTTAAAATGG TTTCATTGGA TATAGGCCTT AAGAAATCAC TATAAAATGC 1380

AAATAAAGTT ACTCAAATCT GTGAAAAAAA AAAAAAAAAA AAAAAAAAAC TCGAGGGGGG 1440

GCCCGTTACC AAKTCGCCCT ATWGTGADTB GTATTMTTAT TTTACTAATA TCTGTAGCTA 1500

TTTTGTTTTT KGCTTKGGTT ATKGTTTTTY TCCCTTYTCT WAGCTATRAG CTGATCATKG 1560

CYSCTTCTCA CCTCCTGCCA TGATACTGTC AGTTACCTTA GTTAACAAGC TGAATATTTA 1620

GTAGAAATGA TGCTTCTGCT CAGGAATGGC CCACAAATCT GTAATTTGAA ATTTAGCAGG 1680

AAATGACCTT TAATGACACT ACATTTTCAG GAACTGAAAT CATTAAAATT TTATTTGAAT 1740

AATTATGTGC TGAAAAAAAA AAAAAAAAAA AMWMRARASK RRWWACTCGA GGGGGGGCCC 1800

GGTACCCNAT TCGCCG 1816

(2) INFORMATION FOR SEQ ID NO: 168:

(i) SEQUENCE CHARACTERISTICS:
(A) LENGTH: 945 base pairs
(B) TYPE: nucleic acid
(C) STRANDEDNESS: double
(D) TOPOLOGY: linear

(xi) SEQUENCE DESCRIPTION: SEQ ID NO: 168:

AGAAACCGTT GATGGGACTG AGAAACCAGA GTTAAAACCT CTTTGGAGCT TCTGAGGACT 60

CAGCTGGAAC CAACGGGCAC AGTTGGCAAC ACCATCAACT TCTCCCAAGC AGAGAAACCC 120

GAACCCACCA ACCAGGGGCA GGATAGCCTG AAGAAACATC TACACGCAGA AATCAAAGTT 180

ATTGGGACTA TCCAGATCTT GTGTGGCATG ATGGTATTGA GCTTGGGGAT CATTTTGGCA 240

TCTGCTTCCT TCTCTCCAAA TTTTACCCAA GTGACTTCTA CACTGTTGAA CTCTGCTTAC 300

CCATTCATAG GACCCTTTTT TTTTATCATC TCTGGCTCTC TATCAATCGC CACAGAGAAA 360

AGGTTRACCA AGCTTTTGGT GCATAGCAGC CTGGTTGGAA GCATTCTGAG TGCTCTGTCT 420

GCCCTGGTGG GTTTCATTAT CCTGTCTGTC AAACAGGCCA CCTTAAATCC TGCCTCACTG 480

CAGTGTGAGT TGGACAAAAA TAATATACCA ACAAGAAGTT ATGTTTCTTA CTTTTATCAT 540

GATTCACTTT ATACCACGGA CTGCTATACA GCCAAAGCCA GTCTGGCTGG AWCTCTCTCT 600

CTGATGCTGA TTTGCACTCT GCTGGAATTC TGCCTAGCTG TGCTCACTGC TGTGCTGCGG 660

TGGAAACAGG CTTACTCTGA CTTCCCTGGG AGTGTACTTT TCCTGCCTCA CAGTTACATT 720

GGTAATTCTG GCATGTCCTC AAAAATGACT CATGACTGTG GATATGAAGA ACTATTGACT 780

TCTTAAGAAA AAAGGGAGAA ATATTAATCA GAAAGTTGAT TCTTATGATA ATATGGAAAA 840

GTTAACCATT ATAGAAAAGC AAAGCTTGAG TTTCCTAAAT GTAAGCTTTT AAAGTAATGA 900

ACATTAAAAA AAACCATTAT TTCACTGTCA TTTAAAGATA ATGTG 945

(2) INFORMATION FOR SEQ ID NO: 169:

(i) SEQUENCE CHARACTERISTICS:
(A) LENGTH: 902 base pairs
(B) TYPE: nucleic acid

(C) STRANDEDNESS: double
(D) TOPOLOGY: linear

(xi) SEQUENCE DESCRIPTION: SEQ ID NO: 169:

GGCAGAGCCA CAGGAAGGAT GAGGAAGACC AGGCTCTGGG GGCTGCTGTG GATGCTCTTT 60

GTCTCAGAAC TCCGAGCTGC AACTAAATTA ACTGAGGAAA AGTATGAACT GAAAGAGGGG 120

CAGACCCTGG ATGTGAAATG TGACTACACG CTAGAGAAGT TTGCCAGCAG CCAGAAAGCT 180

TGGCAGATAA TAAGGGACGG AGAGATGCCC AAGACCCTGG CATGCACAGA GAGGCCTTCA 240

AAGAATTCCC ATCCAGTCCA AGTGGGGAGG ATCATACTAG AAGACTACCA TGATCATGGT 300

TTACTGCGCG TCCGAATGGT CAACCTTCAA GTGGAAGATT CTGGACTGTA TCAGTGTGTG 360

ATCTACCAGC CTCCCAAGGA GCCTCACATG CTGTTCGATC GCATCCGCTT GGTGGTGACC 420

AAGGGTTTTT CAGGGACCCC TGGCTCCAAT GAGAATTCTA CCCAGAATGT GTATAAGATT 480

CCTCCTACCA CCACTAAGGC CTTGTGCCCA CTCTATACCA GCCCCAGAAC TGTGACCCAA 540

GCTCCACCCA AGTCAACTGC CGATGTCTCC ACTCCTGACT CTGAAATCAA CCTTACAAAT 600

GTGACAGATA TCATCAGGGT TCCGGTGTTC AACATTGTCA TTCTCCTGGC TGGTGGATTC 660

CTGAGTAAGA GCCTGGTCTT CTCTGTCCTG TTTGCTGTCA CGCTGAGGTC ATTTGTACCC 720

TAGGCCCACG AACCCACGAG AATGTCCTCT GACTTCCAGC CACATCCATC TGGCAGTTGT 780

GCCAAGGGAG GAGGGAGGAG GTAAAAGGCA GGGAGTTAAT AACATGAATT AAATCTGTAA 840

TCACCRGCTA AAAAAAAAAA AAAAAAAACN CGANCCTNGG TTTTCAGCTC CATCAGCTCC 900

TT 902

(2) INFORMATION FOR SEQ ID NO: 170:

(i) SEQUENCE CHARACTERISTICS:
(A) LENGTH: 1883 base pairs
(B) TYPE: nucleic acid
(C) STRANDEDNESS: double
(D) TOPOLOGY: linear

(xi) SEQUENCE DESCRIPTION: SEQ ID NO: 170:

AGAAAACAAC TGAAAAACCA CATTTTTCTA CATACAGCTG GGGAGGTAGC TGAGAACTTG 60

GCACTGCGCA CACATACTAG GTTGAAAGAG AGTTGAGGAA ACCAGAAGGC CAAGTGGATC 120

TGCTGGCAAA CCCTGAACCT GTCTCCTGCG CTTGCTCTAC AGTTCTGAAG TTGAAAATCC 180

TTTTCATGCC TAGCATCTGC TTGAGTTATA AACCCCAAGG CAGCCATGTC ATAGACTAGT 240

GTTTACTCTT GTTTTGACTT TGTTTTAATG CTTCCTAAGA CCCAAGTGCC TCCTGCTGTT 300

TCCTCCTTTG TGGTAGCCTC TGGCCATCTG GGACCTCAAT CCCCAGCTTT CCCACTTTCA 360

GCAGTCCTTT GCTCTCTTTG CTTCTACCTC AAATAGCCCC AGGAGTGGGC TTTAGTCTCC 420

AATATGGAGC ATYTCAAGCT TCTCCTGGGG GATGGGGATT GGGATGGGCA GAATCTGTTT 480

TGGWTCTCCG GGTTATTTCC AGTGGGTGTA AAAGCAGAGC TGGGCCTTTC CCTCTCTTAT 540

CCCTGAGGGT GGGTAAGAAG GACTGTATCT ACACCTGTTC TTCCCTACCT TCTCTTTTGT 600

TAGGGAGGCC TCATTCTAAG TTCCTCAAGA GAGTCCTTGG CTTAAAGCTG TAGCAAGGGT 660

GTGCTAGGTG GGGGATTTGG AGCAAAACCG TCGAGTAGGC ATGATACTGG TATGGAGTGG 720

GCCTGCAAAA TCAGACAGAA ATGGCTTGAG AAGCCGCAGG GGAGCATGCC TGTCTCTCAG 780

TGATAGAGTA TGGGAGGGAC CTCCCTAGCT TGGAAAATGA GAATTGAAGG GGTTATGAAC 840

AAATAGGATG CCTAGTTGAG GATGTTCCCA AAGTTTTGTC CAATCTTATC ATTAGTAGAT 900

TTTATAAGCC ACAGAGACAA ACCAGAAACG GAATAATGTT ACTTTGGATG CTTTATTTTT 960

TTGTTCTAGG TGTGGCTTTG TACATGCAGA AGAATGCTAT ATGCTGCACA TTTTGCCTTT 1020

AAAGTCTTAC GACTTTCCCC ATTTTAGTCT AATGGGAAGA TACAGATGTG CAAGTCTGCT 1080

TTTTTGTTTT TTGTTATTAT TTTTTTTTTT TTGCTCTGTG TTATGGACAT TTTCAGACAT 1140

GCACAGAAGT GGAGAGGATG GTCCTTGGAC CCCATGTGTC CATCACCTAG CTGCATCACT 1200

TATCAGCTAT GGTCAACCTG GTTTCATCTG TATCTCTCTC TTTTCACCTG TATTGTTTAT 1260

TGAAAATCCA AGACACTATG CCAATGCAAC CGTGACTACT TTGGGAGATT GGTAGTCTCT 1320

TTTGATGGTG ATAGTGATGG GGTGCACTAT CATAATCACA TCAGGTCTGC TTTTTGCTTT 1380

TAATGTTAAC TAATGAAGTT CCAGAGATGG GCCTTAGAAA TGTGTTTTAA GAATTAACAA 1440

GGAGTCTCAA AAAGAAATGA GAGGGATGCT TCCTTTCCCC TTGCATCTAC AAAACAAGAG 1500

AGAGACTGTT CTGTTGTAAA ACTCTTTCAA AAATTCTGAT ATGGTAAGGT ACTTGAGACC 1560

CTTCACCAGA ATGTCAATCT TTTTTTCTGT GTAACATGGA AACTTGTGTG ACCATTAGCA 1620

TTGTTATCAG CTTGTACTGG TCTCATAACT CTGGTTTTGG AAGAATAATT TGGAAATTGT 1680

TGCTGTGTTC TGTGAAAATA ACCTCCCCAA AATAATTAGT AACTGGTTGT TCTACTTGGT 1740

AATTTGACAC CCTGTTAATA ACGCAATTAT TTCTGTGTTC TTAAACAGTA TAAATAGTTG 1800

TAAGTTTGCA TGCATGATGG AAAAATAAAA ACCTGTATCT CTGTTAAAAA AAAAAAAAAA 1860

AAAAAAAAAA AAAAAAAAAA AAA 1883

(2) INFORMATION FOR SEQ ID NO: 171:

(i) SEQUENCE CHARACTERISTICS:

(A) LENGTH: 2100 base pairs
(B) TYPE: nucleic acid
(C) STRANDEDNESS: double
(D) TOPOLOGY: linear

(xi) SEQUENCE DESCRIPTION: SEQ ID NO: 171:

TACTTTTAGA TTTACTGCCT TCAAAAAGTG CCTATTCTGA GCAACATAAA CGTTATTCCT 60

TACATATGTA TGTACACACG GTACCCAGAG TCGTACTGTG GCAGCCTTCA AAAACATACC 120

ATCAGAAAGA GTAGGTGCTG AGATAAGGNA ACTTTGCCAA ATGNAAGAAA GTCACTCACT 180

TCCAATATCC CCTCTTCAAG CGGCTACCGT GRAASGGGCT GCAAACACAT TCCCTGAGCA 240

TCCCTTGCTG ATACAGCTTC TTTATATTTA TATCCTACTG GATGGTAGCA TATTGCTAAG 300

GTTTCCTGTA CTCTGCTTCA AGGGAATGTA AGYTTTATGG CATTGAAACA TTTAGGAAAA 360

AAAAAGATGT TTAAGAGAAT TAATAGAGCC GTAGTCTGTA TTAGGATGTG TGTCATATGT 420

GTGTTCTATA AACTAAGCAT CGGTGGGTTT AGAGTGTTAA AGTGTCAGCA CATTCCTTCT 480

CCTTTTGTCT CTCAGGCTAA CATGAGAGAA AATAGAAAAG TCTTGGCTGT GGGGATTGGA 540

AGCTCAGGGG GCCAAATGTC CTTGCCAGAT CCTTAGAGCA TTACTTTGAC TCCTAAAAAT 600

AGTAGTGTAT GTTATTTGAT GGCTTTTGTT TCCATAGTTC CATCACTGAC AAAACTGTCA 660

ATACTGTTGA TGGAGCAGCA GCATAGCCTA GAGTGATGCA TTCTTACCCA GAGGTGGCAA 720

TAGGAGAGGG TCCATGTAAA TAGGACGAGG TAGACAGTGC ATGATTGTAG GAGAAGGGTT 780

GAAGGGAGGA CATGATTCCA AAAAAGATCG TTCTCAATGT GTCGTCTGAC TCAACCAGCT 840

GGCAGATTAC ACTTGCCAAG TCGTTCCCTT TCCTTCTAAG TCAGTTGGCT CCATATTCAC 900

TTGAATATGC CTCTGTTTGG GCAAAGCAAG ATACCTCCAC TTAACCTTTA TCCAAGGAAG 960

CTCTTGGTGT CCTCTTGGTC ATAAAGTTGT CTCCTACCTA ACCCAGTTTT ACCAAATGGA 1020

AGTAAAAGGG GACAAACTAT GGAAGATGGA CTCCATGCCA TTGCAGTCAG CCACCATTCT 1080

CTTTTCCATA TAAGGAGCCC CATTACATAA GCTACGGGTG AGGTTGGAAC AGCTATGTTT 1140

CATAATTTCA AGAGTGTGAC CACCCTGCTC TAGTCATCAT CATTGGATGA ATCCAGTTGA 1200

CTCTTTGGCA AAAGGGTGAT ACTTTTCACT AAAAATGCCT ACTCTTCCTG TTGATGTTCC 1260

TTTTCTGTTT TTACCTTGTC CAATTTCCAC ACTAGTCATT TTTTTTATTT TTTAGAGGAT 1320

CAGATTTTAG CGCTGGAAAA TGAGTTCAAA AATTTCAGTG TAATGTCATA AGGATGTTGG 1380

GATACAGAGA TTTTTTTTTT CCTTGGAAAC AAATGGACTG GGAAGAAACA CAGCATGGCT 1440

TTGCTCTGAG TTTCAATCTG ATGATTATGA CCATGGAAGA TAGTCTTATG TAAAGGTTAA 1500

ATGGTGTTTA CAAGTGGATA GATAAGGCGG AGATGGTGAG AAGCCGGGTT TTCTCTATGC 1560

TAAATGTGTC TACTAAGAGC AGCACTTCCT ACTAGCTAAG CACAATCATA GCCCCACCGT 1620

GATGAGCTGC TAGTCTGAAT AACATTCCCT GACTTAGGGA AAGGCACACA AAAACATATA 1680

AAGAATATGT CTATTTTCAT ATGTGTGATA CTGACAGAGC CATGGTATTC CTAAAATATA 1740

GGTTTCTCTT TTTTCTTGTA TTCTTAGCAA ATTGCATTTA TTCACTACAT TACAAACCAT 1800

CACTGATGTA TCCAAAATAG CACACATAGT TCAGTATGAA AATAAGAGAA TAAAATCTGT 1860

TATAAGCAAG TGATTTAGGT ATTTTCTTTT GTGTTTATGC ATTATCTGAC TATATTAAAA 1920

CCTGTTTTTC TATTTACCTT CTATCAGTTT TCTCTACCAA TTATGTTTTT TCAATGCTCT 1980

ATAAGAATGA ATATGGAAAT TATATTTCTT TTTTCTGTAA AAGAGTTGCA ACTACTTTAT 2040

TATATTTAGA AATCCAATAA ACTTCTTATT ACATTTAAAA AAAAAAAAAA AAAACTCGAA 2100

(2) INFORMATION FOR SEQ ID NO: 172:

(i) SEQUENCE CHARACTERISTICS:
(A) LENGTH: 1930 base pairs
(B) TYPE: nucleic acid
(C) STRANDEDNESS: double
(D) TOPOLOGY: linear

(xi) SEQUENCE DESCRIPTION: SEQ ID NO: 172:

CCTTTGANTG TGGTCCCGGG TGCNGATTGG CAGCGCCTCC GCCGCGGCTC GTGGTTGTCC 60

CGCCATGGCA CTGTCGCGGG GGCTGCCCCG GGAGCTGGCT GAGGCGGTGG CCGGGGGCCG 120

GGTGCTGGTG GTGGGGGCGG GCGGCATCGG CTGCGAGCTC CTCAAGAATC TCGTGCTCAC 180

CGGTTTCTCC CACATCGACC TGATTGATCT GGATACTATT GATGTAAGCA ACCTCAACAG 240

ACAGTTTTTG TTTCAAAAGA AACATGTTGG AAGATCAAAG GCACAGGTTG CCAAGGAAAG 300

TGTACTGCAG TTTTACCCGA AAGCTAATAT CGTTGCCTAC CATGACAGCA TCATGAACCC 360

TGACTATAAT GTGGAATTTT TCCGACAGTT TATACTGGTT ATGAATGCTT TAGATAACAG 420

AGCTGCCCGA AACCATGTTA ATAGAATGTG CCTGGCAGCT GATGTTCCTC TTATTGAAAG 480

TGGAACAGCT GGGTATCTTG GACAAGTAAC TACTATCAAA AAGGGTGTGA CCGAGTGTTA 540

TGAGTGTCAT CCTAAGCCGA CCCAGAGAAC CTTTCCTGGC TGTACAATTC GTAACACACC 600

TTCAGAACCT ATACATTGCA TCGTTTGGGC AAAGTACTTG TTCAACCAGT TGTTTGGGGA 660

AGAAGATGCT GATCAAGAAG TATCTCCTGA CAGAGCTGAC CCTGAAGCTG CCTGGGAACC 720

AACGGAAGCC GAAGCCAGAG CTAGAGCATC TAATGAAGAT GGTGACATTA AACGTATTTC 780

TACTAAGGAA TGGGCTAAAT CAACTGGATA TGATCCAGTT AAACTTTTTA CCAAGCTTTT 840

TAAAGATGAC ATCAGGTATC TGTTGACAAT GGACAAACTA TGGCGGAAAA GGAAACCTCC 900

AGTTCCGTTG GACTGGGCTG AAGTACAAAG TCAAGGAGAA GAAACGAATG CATCAGATCA 960

ACAGAATGAA CCCCAGTTAG GCCTGAAAGA CCAGCAGGTT CTAGATGTAA AGAGCTATGC 1020

ACGTCTTTTT TCAAAGAGCA TCGAGACTTT GAGAGTTCAT TTAGCAGAAA AGGGGGATGG 1080

AGCTGAGCTC ATATGGGATA AGGATGACCC ATCTGCAATG GATTTTGTCA CCTCTGCTGC 1140

AAACCTCAGG ATGCATATTT TCAGTATGAA TATGAAGAGT AGATTTGATA TCAAATCAAT 1200

GGCAGGGAAC ATTATTCCTG CTATTGCTAC TACTAATGCA GTAATTGCTG GGTTGATAGT 1260

ATTGGAAGGA TTGAAGATTT TATCAGGAAA AATAGACCAG TGCAGAACAA TTTTTTTGAA 1320

TAAACAACCA AACCCAAGAA AGAAGCTTCT TGTGCCTTGT GCACTGGATC CTCCCAACCC 1380

CAATTGTTAT GTATGTGCCA GCAAGCCAGA GGTGACTGTG CGGCTGAATG TCCATAAAGT 1440

GACTGTTCTC ACCTTACAAG ACAAGATAGT GAAAGAAAAA TTTGCTATGG TAGCACCAGA 1500

TGTCCAAATT GAAGATGGGA AAGGAACAAT CCTAATATCT TCCGAAGAGG GAGAGACGGA 1560

AGCTAATAAT CACAAGAAGT TGTCAGAATT TGGAATTAGA AATGGCAGCC GGCTTCAAGC 1620

AGATGACTTC CTCCAGGACT ATACTTTATT GATCAACATC CTTCATAGTG AAGACCTAGG 1680

AAAGGACGTT GAATTTGAAG TTGTTGGTGA TGCCCCGGAA AAAGTGGGGS CCAAACAAGC 1740

TGAAGATGCT GCCAAAAGCA TAACCAATGG GCAGTGATGA TGGGAGCTTC AGCCCTCCAC 1800

CTYCACAGCT TCAAGGAGGC AAGATGGACG TYTCYCATAG TTGATYCGGR TGAAGAAGRT 1860

TCTCCAATAA TTGCCCGACG TTCATTGAAG GAAGGAGGAG GAGGCCCGCC AAGAGGGGAA 1920

TTTAGGNTTG 1930

(2) INFORMATION FOR SEQ ID NO: 173:

(i) SEQUENCE CHARACTERISTICS:
(A) LENGTH: 1509 base pairs
(B) TYPE: nucleic acid
(C) STRANDEDNESS: double
(D) TOPOLOGY: linear

(xi) SEQUENCE DESCRIPTION: SEQ ID NO: 173:

GGCCCTGGCC TCTGGGCTGA GGCTTGCTAG GGACTCGGGG TGGCTCTAAG GGGCAGGGAT 60

AGGGCTGGGG AGCGCCGGCC TGTGGCCCTG ACCAGCCCCT TCTCGTGCRG GTTCCACCCC 120

GATGCAGGTG GTCACGTGCT TGACGCGGGA CAGCTACCTG ACGCACTGCT TCCTCCAGCA 180

CCTCATGGTC GTGCTGTCCT CTCTGGAACG CACGCCCTCG CCGGAGCCTG TTGACAAGGA 240

CTTCTACTCC GAGTTTGGGA ACAAGACCAC AGGGAAGATG GAGAACTACG AGCTGATCCA 300

CTCTAGTCGC GTCAAGTTTA CCTACCCCAG TGAGGAGGAG ATTGGGGACC TGACGTTCAC 360

TGTGGCCCAA AAGATGGCTG AGCCAGAGAA GGCCCCAGCC CTCAGCATCC TGCTGTACGT 420

GCAGGCCTTC CAGGTGGGCA TGCCACCCCC TGGGTGCTGC AGGGGCCCCC TGCGCCCCAA 480

GACACTCCTG CTCACCAGCT CCGAGATCTT CCTCCTGGAT GAGGACTGTG TCCACTACCC 540

ACTGCCCGAG TTTGCCAAAG AGCCGCCGCA GAGAGACAGG TACCGGCTGG ACGATGGCCG 600

CCGCGTCCGG GACCTGGACC GAGTGCTCAT GGGCTACCAG ACCTACCCGC AGCCCTCACC 660

CTCGTCTTCG ATGACGTGCA AGGTCATGAC CTCATGGGCA GTGTCACCCT GGACCACTTT 720

GGGGAGGTGC CAGGTGGCCC GGCTAGAGCC AGCCAGGGCC GTGAAGTCCA GTGGCAGGTG 780

TTTGTCCCCA GTGCTGAGAG CAGAGAGAAG CTCATCTCGC TGTTGGCTCG CCAGTGGGAG 840

GCCCTGTGTG GCCGTGAGCT GCCTGTCGAG CTCACCGGCT AGCCCAGGCC ACAGCCAGCC 900

TGTCGTGTCC AGCCTGACGC CTACTGGGGC AGGGCAGCAG GCTTTTGTGT TCTCTAAAAA 960

TGTTTTATCC TCCCTTTGGT ACCTTAATTT GACTGTCCTC GCAGAGAATG TGAACATGTG 1020

TGTGTGTTGT GTTAATTCTT TCTCATGTTG GGAGTGAGAA TGCCGGGCCC CTCAGGGCTG 1080

TCGGTGTGCT GTCAGCCTCC CACAGGTGGT ACAGCCGTGC ACACCAGTGT CGTGTCTGCT 1140

GTTGTGGGAC CGTTGTTAAC ACGTGACACT GTGGGTCTGA CTTTCTCTTC TACACGTCCT 1200

TTCCTGAAGT GTCGAGTCCA GTCCTTTGTT GCTGTTGCTG TTGCTGTTGC TGTTGCTGTT 1260

GGCATCTTGC TGCTAATCCT GAGGCTGGTA GCAGAATGCA CATTGGAAGC TCCCACCCCA 1320

TATTGTTCTT CAAAGTGGAG GTCTCCCCTG ATCCAGACAA GTGGGAGAGC CCGTGGGGGC 1380

AGGGGACCTG GAGCTGCCAG CACCAAGCGT GATTCCTGCT GCCTGTATTC TCTATTCCAA 1440

TAAAGCAGAG TTTGACACCG TCAAAAAAAA AAAAAAAAAA AAAAAAAAAA ATTNCTGCGG 1500

CCTCAAGGG 1509

(2) INFORMATION FOR SEQ ID NO: 174:

(i) SEQUENCE CHARACTERISTICS:
(A) LENGTH: 3173 base pairs
(B) TYPE: nucleic acid
(C) STRANDEDNESS: double
(D) TOPOLOGY: linear

(xi) SEQUENCE DESCRIPTION: SEQ ID NO: 174:

TCGACCCCAS GCGTCCGTGC TTTTCCACAG AAGGTTAGAC CCTGAAAGAG ATGGCTCAGC 60

ACCACCTATG GATCTTGCTC CTTTGCCTGC AAACCTGGCC GGAAGCAGCT GGAAAAGACT 120

CAGAAATCTT CACAGTGAAT GGGATTCTGG GAGAGTCAGT CACTTTCCCT GTAAATATCC 180

AAGAACCACG GCAAGTTAAA ATCATTGCTT GGACTTCTAA AACATCTGTT GCTTATGTAA 240

CACCAGGAGA CTCAGAAACA GCACCCGTAG TTACTGTGAC CCACAGAAAT TATTATGAAC 300

GGATACATGC CTTAGGTCCG AACTACAATC TGGTCATTAG CGATCTGAGG ATGGAAGACG 360

CAGGAGACTA CAAAGCAGAC ATAAATACAC AGGCTGATCC CTACACCACC ACCAAGCGCT 420

ACAACCTGCA AATCTATCGT CGGCTTGGGA AACCAAAAAT TACACAGAGT TTAATGGCAT 480

CTGTGAACAG CACCTGTAAT GTCACACTGA CATGCTCTGT AGAGAAAGAA GAAAAGAATG 540

TGACATACAA TTGGAGTCCC CTGGGAGAAG AGGGTAATGT CCTTCAAATC TTCCAGACTC 600

CTGAGGACCA AGAGCTGACT TACACGTGTA CAGCCCAGAA CCCTGTCAGC AACAATTCTG 660

ACTCCATCTC TGCCCGGCAG CTCTGTGCAG ACATCGCAAT GGGCTTCCGT ACTCACCACA 720

CCGGGTTGCT GAGCGTGCTG GCTATGTTCT TTCTGCTTGT TCTCATTCTG TCTTCAGTGT 780

TTTTGTTCCG TTTGTTCAAG AGAAGACAAG ATGCTGCCTC AAAGAAAACC ATATACACAT 840

ATATCATGGC TTCAAGGAAC ACCCAGCCAG CAGAGTCCAG AATCTATGAT GAAATCCTGC 900

AGTCCAAGGT GCTTCCCTCC AAGGAAGAGC CAGTGAACAC AGTTTATTCC GAAGTGCAGT 960

TTGCTGATAA GATGGGGAAA GCCAGCACAC AGGACAGTAA ACCTCCTGGG ACTTCAAGCT 1020

ATGAAATTGT GATCTAGGCT GCTGGGCTGA ATTCTCCCTC TGGAAACTGA GTTACAACCA 1080

CCAATACTGG CAGGTTCCCT GGATCCAGAT CTTCTCTGCC CAACTCTTAC TGGGAGATTG 1140

CAAACTGCCA CATCTCAGCC TGTAAGCAAA GCAGGAAACC TTCTGCTGGG CATAGCTTGT 1200

GCCTAAATGG ACAAATGGAT GCATACCCTT CCTGAAATGA CTCCCTTCTG AATGAATGAC 1260

AAAGCAGGTT ACCTAGTATA GTTTTCCCAA ACTTCTTCCC ATCATAGCAC ATGTAGAAAA 1320

TAATATTTTT ATGGCACACT GGGATAAACA AGCAAGATTG CTCACTTCTG GAAGCTGCAT 1380

ATGACTAGAG GCCTCTTGTG ACTGGAGGTA ACAACCCTGC CCAGTAACTG TGGGAGAAGG 1440

GGATCAATAT TTTGCACACC TGTAATAGGC CATGGCACAC CAGCCAAGAT GCTCTGCTCA 1500

CAGTCAGTAT GTGTGAAGAT CCCTGGTGCG TGGCCTTCAC CACGCATCTT GAGCAAATTA 1560

GGAAAATGTA CCCTTCGCTT GAGGCAGATG CAGCCCTTCC CCCGAGTGCA TGGCTTGGAG 1620

AGCAGAATGT GGGCTGCATA TAAGCACACT CATCCCTTTG TCTGGGAATC TTTGTGCAGG 1680

GCATAACAGG CTTAGTAAGT CCAAACACAG ATGACAGTGC TGTGTGGGTC TCTGTCAGAG 1740

TTGTGGCTCT CAGCCATGTA GACACACTCT CCAAATGGAG TGTTGGAAAA TGTTCTTTCT 1800

GCAGGGTCTA GAGACTGCTG GGACACTTTT CTTGGAGTGC TACTTCAGAA GCCTTATAGG 1860

ATTTTCTTTC TGGCCAAGAT TTCCTTCTGT ATCACTCCAA GCAGCCTCAG CAGAAGAAGC 1920

AGCCATGCCC AGTATTCCCA CTCTCCAAAA GGAACTGACC AGCTTATATT TCTCACACTT 1980

CTGGGGAACT GGGTATAATC CAACCATCAA AATAGAAGAC CTTGCAAGAA GCAGAGTCAT 2040

TCTCCAGAAG GAACTTGGGA GATGATGGTG CAGATGATGA AACTGGGTTC ATCCCAGTTC 2100

CAAAGACTCA GAGAACTAGA GTTTAAGCTG AGGCAGAGTG CCGCCACCCT GGCATGCCCC 2160

ACAAACAGAT CACCAGCCAG CTTACACAGG CATTAACTCT CCTCAATGAG GAAGAATCAT 2220

TCACAACTGA GCAAGACATT CATATGATCA TTTAAGGAAG TGTTTCCCTT ATGTGTTAGC 2280

AAGTATAATC GGCTAACTCC TAAATCCCAA TGAATAGTCC TAGGCTGGAC AGCAATGGGC 2340

TGCAATTAGG CAGATAAAGA CATCAGTCCC AGTAAATGAA TCCATAGACT CATCTAGCAC 2400

CAACTACCAT TAGCACTATG TTAGGAGCTG CAAGGCCCCA AAGTAGAAGA TGTGCATAAT 2460

GTCTGCTCTT GTGTAGCTCA GGAGACAATT CCAGCACAGA CACTACAGTT AACGCTGAAC 2520

TGCAGCTGCA AGTAATAGCA TGAACAGTCA GAAAAATACC TTATGAGGGG GCAGGGCTGA 2580

AGCTGGGCCT TGAAGGATGG ATGAAATTTG GATAGAGAAT GAGGAAGACA GAGGGCCTCC 2640

AAGTGAGAGA AGCATGAAAA ATGAGCAGGG GCCTGGATCA GTGGGGTGTA TTCAGAGCAC 2700

CTCTCCAGAT GCACCATGCA TGCTCACAGT CCCTTGCCTA TGTGTGGCAG AGTGTCCCAG 2760

CCAGATGTGT GCCCCCACCC CATGTCCATT TACATGTCCT TCAATGCCCA CCTCAAAAGG 2820

TACCTCTTCT GTAAAGCTTT CCCTGGTATC AGGAATCAAA ATTAATCAGG GATCTTTTCA 2880

CACTGCTGTT TTTTCCTCTT TGGTCCTTCT ATCACTAAAA CTCATCTCAT TCAGCCTTAC 2940

AGCATAACTA ATTATTTGTT TTCCTCACTA CATTGTACAT GTGGGAATTA CAGATAAACG 3000

GAAGCCKGCT GGGGTGGTGG CTCACGCCTG TAATCCCAAC ACTTTGGGAG GCCAAGGCAG 3060

GCGGATCACC TGAGGTCAGG ARTTCGAGAT TARTCTGGCC AACATGGTGA AACCCCATNT 3120

NTACTAAAAA TACGAAATTA GCCAGGTGTG GTGGCACACA TCTGTAGTCC CAG 3173

(2) INFORMATION FOR SEQ ID NO: 175:

(i) SEQUENCE CHARACTERISTICS:
(A) LENGTH: 991 base pairs
(B) TYPE: nucleic acid
(C) STRANDEDNESS: double
(D) TOPOLOGY: linear

(xi) SEQUENCE DESCRIPTION: SEQ ID NO: 175:

AAATTCGGCA CAGCTGAGAG GAGACACAAG GAGCAGCCCG CAAGCACCAA GTGAGAGGCA 60

TGAAGTTACA GTGTGTTTCC CTTTGGCTCC TGGGTACAAT ACTGATATTG TGCTCAGTAG 120

ACAACCACGG TCTCAGGAGA TGTCTGATTT CCACAGACAT GCACCATATA GAAGAGAGTT 180

TCCAAGAAAT CAAAAGAGCC ATCCAAGCTA AGGACACCTT CCCAAATGTC ACTATCCTGT 240

CCACATTGGA GACTCTGCAG ATCATTAAGC CCTTAGATGT GTGCTGCGTG ACCAAGAACC 300

TCCTGGCGTT CTACGTGGAC AGGGTGTTCA AGGATCATCA GGAGCCAAAC CCCAAAATCT 360

TGAGAAAAAT CAGCAGCATT GCCAACTCTT TCCTCTACAT GCAGAAAACT CTGCGGCAAT 420

GTCAGGAACA GAGGCAGTGT CACTGCAGGC AGGAAGCCAC CAATGCCACC AGAGTCATCC 480

ATGACAACTA TGATCAGCTG GAGGTCCACG CTGCTGCCAT TAAATCCCTG GGAGAGCTCG 540

ACGTCTTTCT AGCCTGGATT AATAAGAATC ATGAAGTAAT GTCCTCAGCT TGATGACAAG 600

GAACCTGTAT AGTGATCCAG GGATGAACAC CCCCTGTGCG GTTTACTGTG GGAGACAGCC 660

CACCTTGAAG GGGAAGGAGA TGGGGAAGGC CCCTTGCAGC TGAAAGTCCC ACTGGCTGGC 720

CTCAGGCTGT CTTATTCCGC TTGAAAATAG CCAAAAAGTC TACTGTGGTA TTTGTAATAA 780

ACTCTATCTG CTGAAAGGGC CTGCAGGCCA TCCTGGGAGT AAAGGGCTGC CTTCCCATCT 840

AATTTATTGT GAAGTCATAT AGTCCATGTC TGTGATGTGA GCCAAGTGAT ATCCTGTAGT 900

ACACATTGTA CTGAGTGGTT TTTCTGAATA AATTCCATAT TTTACCTAAA AAAAAAAAAA 960

AAAAACTCGA GGGGGGGCCC GTACCCAATT T 991

(2) INFORMATION FOR SEQ ID NO: 176:

(i) SEQUENCE CHARACTERISTICS:
(A) LENGTH: 1290 base pairs
(B) TYPE: nucleic acid
(C) STRANDEDNESS: double
(D) TOPOLOGY: linear

(xi) SEQUENCE DESCRIPTION: SEQ ID NO: 176:

ACAGCCCTCT TCGGAGCCTG AGCCCGGCTC TCCTCACTCA CCTCAACCCC CAGGCGGCCC 60

CTCCACAGGG CCCCTCTCCT GCCTGGACGG CTCTGCTGGT CTCCCCGTCC CCTGGAGAAG 120

AACAAGGCCA TGGGTCGGCC CCTGCTGCTG CCCCTRCTGC YCCTGCTGCW GCCGCCAGCA 180

TTTCTGCAGC CTRGTGGCTC CACAGGATCT GGTCCAAGCT ACCTTTATGG GGTCACTCAA 240

CCAAAACACC TCTCAGCCTC CATGGGTGGC TCTGTGGAAA TCCCCTTCTC CTTCTATTAC 300

CCCTGGGAGT TAGCCAYAGY TCCCRACGTG AGAATATCCT GGAGACGGGG CCACTTCCAC 360

GGGCAGTCCT TCTACAGCAC AAGGCCGCCT TCCATTCACA AGGATTATGT GAACCGGCTC 420

TTTCTGAACT GGACAGAGGG TCAGGAGAGC GGCTTCCTCA GGATCTCAAA CCTGCGGAAG 480

GAGGACCAGT CTGTGTATTT CTGCCGAGTC GAGCTGGACA CCCGGAGATC AGGGAGGCAG 540

CAGTTGCAGT CCATCAAGGG GACCAAACTC ACCATCACCC AGGCTGTCAC AACCACCACC 600

ACCTGGAGGC CCAGCAGCAC AACCACCATA GCCGGCCTCA GGGTCACAGA AAGCAAAGGG 660

CACTCAGAAT CATGGCACCT AAGTCTGGAC ACTGCCATCA GGGTTGCATT GGCTGTCGCT 720

GTGCTCAAAA CTGTCATTTT GGGACTGCTG TGCCTCCTCC TCTGTGGTGG AGGAGAAGGA 780

AAGGTAGCAG GGCGCCAAGC AGTGACTTCT GACCAACAGA GTGTGGGGAG AAGGGATGTG 840

TATTAGCCCC GGAGGACGTG ATGTGAGACC CGCTTGTGAG TCCTCCACAC TCGTTCCCCA 900

TTGGCAAGAT ACATGGAGAG CACCCTGAGG ACCTTTAAAA GGCAAAGCCG CAAGGCAGAA 960

GGAGGCTGGG TCCCTGAATC ACCGACTGGA GGAGAGTTAC CTACAAGAGC CTTCATCCAG 1020

GAGCATCCAC ACTGCAATGA TATAGGAATG AGGTCTGAAC TCCACTGAAT TAAACCACTG 1080

GCATTTGGGG GCTGTTYATT ATAGCAGTGC AAAGAGTTCC TTTATCCTCC CCAAGGATGG 1140

AAAATACAAT TTATTTTGCT TACCATACAC CCCTTTTCTC CTCGTCCACA TTTTCCAATC 1200

TGTATGGTGG CTGTCTTCTA TGGCAGAAGG TTTTGGGGAA TAAATAGCGT GANATGNTNC 1260

TGACTNAAAA AAAAAAAAAA AAAAACTCGA 1290

(2) INFORMATION FOR SEQ ID NO: 177:

(i) SEQUENCE CHARACTERISTICS:
(A) LENGTH: 2290 base pairs
(B) TYPE: nucleic acid
(C) STRANDEDNESS: double
(D) TOPOLOGY: linear

(xi) SEQUENCE DESCRIPTION: SEQ ID NO: 177:

TGGGGCCCCT TTTGGATGCT CTGGGTGTTT TTGCCAAGAG TTACAGGATG TCAAGTGTGG 60

GGAGCTCAGC ACCCTTGCTG TGGACCAGTG AAGGCTGTTC CAGACCAGGT GCTTCCAGAC 120

ATTTCCAGGC TCCAGGAGAG AGGCTGGGAG CCCCCACAGA AAGCACAGGA AAATGCAAAA 180

AAAAAACAGT CTTTTTTTTT TTTTTGCTTT TTATTATGAA AACAAAACAA ATGCCCCAGG 240

AGAAGGGTCC ATGATTACCA GAAACATCAA AGAGTACTTT CTACCATTTT TATTCTGTTG 300

TGTTGAGGCC AGCATTGCAA TAAACAAGCT AAACTACTTA CATTGGACTC ATTTTCAGTA 360

ACTGACATTT ACAGGAATAT ACTAGAAACG GCACTAAAAA GTTTAAGAAA AGTTACGGTA 420

AACTTGCATG CACATCATAC AGAAAAGTAA CATTTTAAAT ATAAAAAAGA AAAACTTCCT 480

GGAAGCATTA TGCCAGTATT AAGGAACAGT GCTACTCTGG ATGTGACAAA TTCTGTATGT 540

GGGTGTTACT CTTTCCCAAA AGACTGTCAG AGGCGTGAGT GCTGCAAAAG AACAACAACA 600

AAAACAAACA CACAAAAAAA TGTGTCTTAC AGTTTGTAAG CAAGATGACA CTGCCCAACA 660

CAAAGAGGGG TCTGGAGTTC AGTTCACGCC CGAAGCCTGC CCCCTCGGCC TCCAGGGGTC 720

ATTCAGAGTG TTCTCAAATC CAATTCCGAC ACACGACTTG TCACTACTCC TCTCCCCTTG 780

AAAAAAGCAT GTTAGAAGCT GCCCTACAGG TCTCAGCAGT GGGACAATCT AATTGAATCA 840

CCGCAGCCTT CTAATACAGA AGAAACGGAC GTGACTGTCA CCCTCAGCCC GCCAGCAAGG 900

GCGCTGAGGA AGTCATTAAT CCTTCGAAAC TCTGAAAAGA AACCAGTGTT GAAGTCTGGA 960

CAGAAAGCCT TAAAAAAGTG ACAGCACCAA TGCAGCTGCT CAGTGTACCC NCCGTGGGCT 1020

GTCAGGGTCA GTGGCTTCTT TCTAGATGAA AGGAGCAGAG GCGAGCCGAC GCCACCGTCA 1080

CAGAGAACCA GCCGAGAAGG AAAGGCCCCA CGATGCTCCC TGTGCGCTGC CCCCACAGCC 1140

GGCCGCTCCC CCGACGGCTC ACACAGGCAG CACCTCACTG CCCTGTGGCT GGAGGGGCAT 1200

TGCAAGGAGC GCCCCCCAGC CCCAGGCACC CCCGGCTTAG GGTGTACGTA TCACCCAGCC 1260

CTGTGCTGGC AGCACGTTAC CAACCAGCCT GCGTGAAGAC CTGTCAACTG TCGTGTGTGA 1320

ATTCCTTAAA TTCGGTTTAA ATAGTCCATT AAAGATCTGT TTAGAAAATA CCTTTGAAAA 1380

CGAGGGTAAC TTTAAAAAAT GGAAACTTTC AAATCCATTT ATATTTTTAT TATAAACAAA 1440

ACTTAATTAA AAGTTTAACA AACTGGCTGA AAACTCACCA AGTGTCAGAC TCACCAGCAA 1500

TTTAAAAAAT GATAATTTAC CAGCATCTCC TCATCAGAGT TCCCTCTCCA GTAAGGGTAT 1560

ACCTACATCT GTAAGGGTCA GTGGACTCTG AATCAATTTT ATGGTTGTTT TAAAATCACC 1620

GTGTATTAGG ATACTAATGA TAGTCCCTAT ATCCATCCAG AAATGCTGGC AGAAAGCACT 1680

GGCCACCATA CAGGACAGAC CACACCACAG CTCCATACCC AGCGTCTGCC TGGAGGCTCC 1740

CCCACGCTGA GGTCCGGGAG AATGCCTGGT TTCAGTCATT TCCGGACTAA CTGTGACAAC 1800

GCGTGAGCAG GGAGCACCGT GCGAGTCTCC GGGAGGGAAT CCTCCTGGGG CCCAGAGACT 1860

CCTCCACCCC TGGGGAGGGC AGACAGGCTC GGGARGGCCT GGCCAGGCCA CTGGAGGCTG 1920

GCAGGGAGCA GGCATGTCCA CCCGCAAGCC TGGGAGGCTA ACTCTGGCAT TCCTGGCCGG 1980

AGCCGCCATG CTCATTGGTG GGCCAGTTTG GGACATCCCC GTACTCAAAG ACCATATGGC 2040

AGCCTCTGGG AAAACAAAAC CAAAACATCA CCTTCTATTA AACTCTGTAT ATTATTATTT 2100

TTTACAATAG AAAGTTAAAA ATCAAGACTT AGATTTACTA TACATTTTTT CTCTCAGATT 2160

ACAAAGTTTA TATTATATAA CTGGGGTTCC CTAAATTGAT TTCTTTTAAA ACAGTCTTAA 2220

AGAGACCAGA AGTGAATACA AAAGAACTAA ACAAAATAAA AAATTAGAAT GTGCTGTAGC 2280

TGAAAGCTGT 2290

(2) INFORMATION FOR SEQ ID NO: 178:

(i) SEQUENCE CHARACTERISTICS:
(A) LENGTH: 549 base pairs
(B) TYPE: nucleic acid
(C) STRANDEDNESS: double
(D) TOPOLOGY: linear

(xi) SEQUENCE DESCRIPTION: SEQ ID NO: 178:

GGCACGAGCC ATGCCTGGCC TCTCCTTGAT TCTTACAGTC ACTTTGTTGG CTGTTTCTGA 60

CTCAGCAGCT ACCTGCATTG TGGCCAAAGG ATGACCTATT CCTTCTCAGG AGGGCAAAAA 120

TGTGGAATAG TGTCTGTCCA TGCCTCTCCT CATGGGCTAC CACCTCTGCC ACCGTGGTTA 180

ATCAGTAACA ACCAGGAGAG AAGCTGCTGG AACTGACCTC TGGGAACTCC CTGGGATGGT 240

TTGGTGCAGG AATGTAGTAG GCATACACGT GGTTGCGTGG ATCTGGGCCC TCCTGATGTG 300

AGTAGAGAGG TAAAAGGCCA CCATCTCCTT GACCTCTGGG GAACTCATCC ACAAAGAAGA 360

TGTTTCCAAG ATGCTTCTGA AGATTGCCTA AAAATAGCCG GTTTCCACCC CCGTGAATGC 420

ATCCATTCTA GAATGCTCCT TCACCAGGAC CAGAGAACTG ATTTACAGAA GTGACATGAA 480

AACATTCCAT CCCAGAATTT GCAGTAGCTC AAATTAAGTT TCTAGCTATT AAAAAGAAAA 540

AAAAAAAAA 549

(2) INFORMATION FOR SEQ ID NO: 179:

(i) SEQUENCE CHARACTERISTICS:
(A) LENGTH: 1509 base pairs
(B) TYPE: nucleic acid
(C) STRANDEDNESS: double
(D) TOPOLOGY: linear

(xi) SEQUENCE DESCRIPTION: SEQ ID NO: 179:

GGCACGAGGG CTCATTCATT CCGCGCCGGG CCTGCCAGAC ACCTGCGCCC TTCTGCAGCC 60

GCCCGCCGCA TCCGCCGCCG CAGCCCCCAG CATGTCGGGC CCAGACGTCG AGACGCCGTC 120

CGCCATCCAG ATCTGCCGGA TCATGCGGCC AGATGATGCC AACGTGGCCG GCAATGTCCA 180

CGGGGGGACC ATCCTGAAGA TGATCGAGGA GGCAGGCGCC ATCATCAGCA CCCGGCATTG 240

CAACAGCCAG AACGGGGAGC GCTGTGTGGC CGCCCTGGCT CGTGTCGAGC GCACCGACTT 300

CCTGTCTCCC ATGTGCATCG GTGAGGTGGC GCATGTCAGC GCGGAGATCA CCTACACCTC 360

CAAGCACTCT GTGGAGGTGC AGGTCAACGT GATGTCCGAA AACATCCTCA CAGGTGCCAA 420

AAAGCTGACC AATAAGGCCA CCCTGTGGTA TGTGCCCCTG TCGCTGAAGA ATGTGGACAA 480

GGTCCTCGAG GTGCCTCCTG TTGTGTATTC CCGGCANGAG CAGGAGGAGG AGGGCCGGAA 540

GCGGTATGAA GCCCAGAAGC TGGAGCGCAT GGAGACCAAG TGGAGGAACG GGGACATCGT 600

CCAGCCAGTC CTCAACCCAG AGCCGAACAC TGTCAGCTAC AGCCAGTCCA GCTTGATCCA 660

CCTGGTGGGG CCTTCAGACT GCACCCTGCA CGGCTTTGTG CACGGAGGTG TGACCATGAA 720

GCTCATGGAT GAGGTCGCCG GGATCGTGGC TGCACGCCAC TGCAAGACCA ACATCGTCAC 780

AGCTTCCGTG GACGCCATTA ATTTTCATGA CAAGATCAGA AAAGGCTGCG TCATCACCAT 840

CTCGGGACGC ATGACCTTCA CGAGCAATAA GTCCATGGAG ATCGAGGTGT TGGTGGACGC 900

CGACCCTGTT GTGGACAGCT CTCAGAAGCG CTACCGGGCC GCCAGTGCCT TCTTCACCTA 960

CGTGTCGCTG AGCCAGGAAG GCAGGTCGCT GCCTGTGCCC CAGCTGGTGC CCGAGACCGA 1020

GGACGAGAAG AAGCGCTTTG AGGAAGGCAA AGGGCGGTAC CTGCAGATGA AGGCGAAGCR 1080

ACAGGGCCAC GCGGASCYTC AGCCCTAGAC TCCCTCCTCC TGCCACTGGT GCCTCGAGTA 1140

GCCATGGCAA CGGGCCCAGT GTCCAGTCAC TTAGAAGTTC CCCCCTTGGC CAAAAACCCA 1200

ATTCACATTG AGAGCTGGTG TTGTCTGAAG TTTTCGTATC ACAGTGTTAA CCTGTACTCT 1260

CTCCTGCAAA CCTACACACC AAAGCTTTAT TTATATCATT CCAGTATCAA TGCTACACAG 1320

TGTTGTCCCG AGCGCCGGGA GGCGTTGGGC AGAAACCCTC GGGAATGCTT CCGAGCACGC 1380

TGTAGGGTAT GGGAAGAACC CAGCACCACT AATAAAGCTG CTGCTTGGCT GGAAAAAAAA 1440

AAAAAAAAAA AAAAAAAAAA AAAAAAAAAA AAAAAAAAAA AAAAAAAAAA AAAAAAAAAA 1500

AGAAAAAAN 1509

(2) INFORMATION FOR SEQ ID NO: 180:

(i) SEQUENCE CHARACTERISTICS:
(A) LENGTH: 1316 base pairs
(B) TYPE: nucleic acid
(C) STRANDEDNESS: double
(D) TOPOLOGY: linear

(xi) SEQUENCE DESCRIPTION: SEQ ID NO: 180:

AGCTGTATCA TAGGAAAGAT GGCCACACCG GCGGTACCAG TAAGTGCTCC TCCGGCCACG 60

CCAACCCCAG TCCCGGCGGC GGCCCCAGCC TCAGTTCCAG CGCCAACGCC AGCACCGGCT 120

GCGGCTCCGG TTCCCGCTGC GGCTCCAGCC TGCATCCTCA GACCCTGCGG CAGCAGCGGC 180

TGCAACTGCG GCTCCTGGCC AGACCCCGGC CTCAGCGCAA NTCCAGCGCA GACCCCAGCG 240

CCCGCTCTGC CTGGTCCTGC TCTTCCAGGG CCCTTCCCCG GCGGCCGCGT GGTCAGGCTG 300

CACCCAGTCA TTTTGGCCTC CATTGTGGAC AGCTACGAGA GACGCAACGA GGGTGCTGCC 360

CGAGTTATCG GGACCCTGTT GGGAACTGTC GACAAACACT CAGTGGAGGT CACCAATTGC 420

TTTTCAGTGC CGCACAATGA GTCAGAAGAT GAAGTGGCTG TTGACATGGA ATTTGCTAAG 480

AATATGTATG AACTGCATAA AAAAGTTTCT CCAAATGAGC TCATCCTGGG CTGGTACGCT 540

ACGGGCCATG ACATCACAGA GCACTCTGTG CTGNATCCAT GAGTACTACA GCCGAGAGGC 600

CCCCAACCCC ATCCACCTCA CTGTGGACAC AAGTCTCCAG AACGGCCGCA TGAGCATCAA 660

AGCCTACGTC AGCACTTTAA TGGGAGTCCC TGGGAGGACC ATGGGAGTGA TGTTCACGCC 720

TCTGACAGTG AAATACGCGT ACTACGACAC TGAACGCATC GGAGTTGACC TGATCATGAA 780

GACCTGCTTT AGCCCCAACA GAGTGATTGG ACTCTCAAGT GACTTGCAGC AAGTAGGAGG 840

GGCATCAGCT CGCATCCAGG ATGCCCTGAG TACAGTGTTG CAATATGCAG AGGATGTACT 900

GTCTGGAAAG GTGTCAGCTG ACAATACTGT GGGCCGCTTC CTGATGAGCC TGGTTAACCA 960

AGTACCGAAA ATAGTTCCCG ATGACTTTGA GACCATGCTC AACAGCAACA TCAATGACCT 1020

TTTGATGGTG ACCTACCTGG CCAACCTCAC ACAGTCACAG ATTGCACTCA ATGAAAAACT 1080

TGTAAACCTG TGAATGGACC CCAAGCAGTA CACTTGCTGG TCTAGGTATT AACCCCAGGA 1140

CTCAGAAGTG AAGGAGAAAT GGGTTTTTTG TGGTCTTGAG TCACACTGAG ATAGTCAGTT 1200

GTGTGTGACT CTAATAAACG GAGCCTACCT TTTGTAAATT AAAAAAAAAA AAAAAAACCN 1260

SGRGGGGGGG CCCGGTCCCA TTSSCCCTTT NGTAATTCGT NTTACAATCC CCNGGC 1316

(2) INFORMATION FOR SEQ ID NO: 181:

(i) SEQUENCE CHARACTERISTICS:
(A) LENGTH: 777 base pairs
(B) TYPE: nucleic acid
(C) STRANDEDNESS: double
(D) TOPOLOGY: linear

(xi) SEQUENCE DESCRIPTION: SEQ ID NO: 181:

GGCATGWKCA GACATGACTT CTATTGCCAG GCTGGTCAAG TGGCAGGGTC ATGAGGGAGA 60

CATCGATAAG GGTGCTCCTT ATGCTCCCTG CTCTGGAATC CACCAGCGGG CTATCTGCGT 120

TTATGGGGCT GGGGACTAGA ATTGGATGCT TCAAAACCAT CACCTGTTGG CCAACAAGTT 180

TGACCCAAAG GTAGATGATA ATGCTCTTCA GTGCTTAGAA GAATACCTAC GTTATAAGGG 240

CCATTCTATT GGGACCTGAA CTTTGAAGAC CACAMTATTG AAGAGGCGTT GCTTACCYGT 300

TGGGGGCCAA GAGGCATGTT ACCAAACATG GYYCARGAAM YTTGGYKGGG AMCARKKKKG 360

GKKGGGARRM CMRGGGYTTG SCAAWTTCSK KGGCMWCCYT TTAGGGTAAR RRGGGCKGTW 420

ATTAGATTGT GGGTAAAGTA GGATCTTTTG CCCTTGCAAA TTTGCTGCCT GGGTGAATGY 480

TGCTTGTTCC TTCTCMACCC CTAACCCTAG TAGTTCCTCC ACTAACTTTC TCACTAAGTG 540

AGAATGAGAA CTGCTGTGAT AGGGAGAGTG AAGGAGGGAT ATGTGGTAGA GCACTTGATT 600

TCAGTTGAAT GCCTGCTGGT AGCTTTTCCA TTCTGTGGAG CTGCCGTTCC TAATAATTCC 660

AGGTTTGGTA GCGTGGAGGA GAACTTTGAT GGAAAGAGAA CCTTCCCTTC TGTACTGTTA 720

ACTTAAAAAT AAATAGCTCC TGATTCAAAG TAAAAAAAAA AAAAAAAAAA AAAAAAA 777

(2) INFORMATION FOR SEQ ID NO: 182:

(i) SEQUENCE CHARACTERISTICS:
(A) LENGTH: 791 base pairs
(B) TYPE: nucleic acid
(C) STRANDEDNESS: double
(D) TOPOLOGY: linear

(xi) SEQUENCE DESCRIPTION: SEQ ID NO: 182:

GGCACAGATA ACTATGTACA TGTATTCCTT AAATGTTTTT TTAAGTTTTA TATTCTTGGC 60

ACTGGTCTTC AAATGTGTAC ATGTGTGCCA GGGAGCAAAT GCCTTCTTGT TTCTGAAATT 120

GGTCTTTTAG ACTGTTCTTT TTTCCCATCT TCTCACCTCC TGCCCCTCCT TCAGGGTACT 180

TCCGTGGCCA GAACCCCTCC AGGTCAGAGG CAGAAGAGAA GCCTCATGGG TCACAGCAGC 240

AGATGTGGGC TGGAGATCTA TTCATTTGGT TTTGGCTTGA ATTTTCTGRA TGGTTTACTT 300

GATCYTGGGA AAGANATATC TTGCCAGGAA AAATGATAGN CCTTGACAAT GTTGAATGAT 360

CCTGCACCAC CTTGAAAGAC ATTTCTAATA TGGTTTGTCA GGCAAAGTGG TTAGTAGTCA 420

TTTGTGGCCT GAGGTAGAAG TCCTCAGAAA TCAGCAGACT TCACTGATAA AATGCTGACT 480

TGCCCCTGGA CTGGGCTCTG TGAGAGTGGC CTTCTGCACT GTGCACAGTA GGTGTGAACA 540

CACCACACCT ACAGGGACCA CGTGGTGGGC TGTGGACTAG CGGCCAAGCT CCCTGCAGGC 600

CCACTAATAG AATTCAGCTT TTAGCATGGG CTGTTTCATA CTGTTCTGAT GAAACTGATT 660

TGGTTTCTTT CCTCCATACC CCTTCTGCAT TTCAGTGTTT TTGTTTAGTT TTCCTGGTTT 720

TTAATTATAA CTACAAAATA AAATCTTTAG GCTATTCACC TTAGCTTAGT AAAAAAAAAA 780

AAAAAAAACT C 791

(2) INFORMATION FOR SEQ ID NO: 183:

(i) SEQUENCE CHARACTERISTICS:
(A) LENGTH: 1405 base pairs
(B) TYPE: nucleic acid
(C) STRANDEDNESS: double
(D) TOPOLOGY: linear

(xi) SEQUENCE DESCRIPTION: SEQ ID NO: 183:

AAATTGATTA ACAGCTTGAA AGAAGGCTCT GGTTTTGAAG GCCTAGATAG CAGCACTGCC 60

AGTAGCATGG AGCTGGAAGA ACTTCGGCAT GAGAAAGAGA TGCAGAGGGA GGAAATACAG 120

AAGCTGATGG GCCAGATACA TCAGCTCAGA TCCGAATTAC AGGATATGGA GGCACAGCAA 180

GTTAATGAAG CAGAATCAGC AAGAGAACAG TTACAGGWTC TGCATGACCA AATAGCTGGG 240

CAGAAAGCAT CCAAACAAGA ACTAGAGACA GAACTGGAGC GACTGAAGCA GGAGTTCCAC 300

TATATAGAAG AAGATCTTTA TCGAACAAAG AACACATTGC AAAGCAGAAT TAAAGATCGA 360

GACGAAGAAA TTCAAAAACT CAGGAATCAG CTTACCAATA AAACTTTAAG CAATAGCAGT 420

CAGTCTGAGT TAGAAAATCG ACTCCATCAG CTAACAGAGA CTCTCATCCA GAAACAGACC 480

ATGCTGGAGA GTCTCAGCAC AGAAAAGAAC TCCCTGGTCT TTCAACTGGA GCGCCTCGAA 540

CAGCAGATGA ACTCCGCCTC TGGAAGTAGT AGTAATGGGT CTTCGATTAA TATGTCTGGA 600

ATTGACAATG GTGAAGGCAC TCGTCTGCGA AATGTTCCTG TTCTTTTTAA TGACACAGAA 660

ACTAATCTGG CAGGAATGTA CGGAAAAGTT CGCAAAGCTG CTAGTTCAAT TGATCAGTTT 720

AGTATTCGCC TGGGAATTTT TCTCCGAAGA TACCCCATAG CGCGAGTTTT TGTAATTATA 780

TATATGGCTT TGCTTCACCT CTGGGTCATG ATTGTTCTGT TGACTTACAC ACCAGAAATG 840

CACCACGACC AACCATATGG CAAATGAACC AAGCCCAGTT GTTGCAGTGA TTGGTTGTCT 900

TTTTCTAGAC TTGGGATCTG CAAGAAGGCC AATTGCCTAA AATTTCTGAG AACAGTGCAC 960

AAGATTATTT TATCACTACA AGCTTTTAAC TTTTTAAGTT ATTGTACAAG TATTCTACCT 1020

AAATCTTCCA ATTTCCTTTA AATGGTAAGA GTTTCTAAAA CAGACAATAA TTTAACAAGC 1080

TCAGCTCTGC TTTATCTGAG TTTAGTGGTC CTAATATATA TGTAGAGAAA GATGGTGGGG 1140

TTGTTCACCT CTGTACAGAC CATCTGTATG TTAGGTGACA TTGATTATGG GTTATAATCA 1200

GGGAAACTAA TTGTATTTAG TGACAAAAAT AAAAAGTTTT TTTTTTATAA TTCAGTCTGC 1260

TTTTGGATTT TCATATATTT AACTTTGCAA AAAGATTTAC TTTGTACATG TTACAGGCTT 1320

GATTGGTGTA AATCTTTTTA TAAATACATA AATAAAAGNA AAATATGCAT TTTTCTTTTC 1380

TAAAAAAAAA AAAAAAAAAA CTCGA 1405

(2) INFORMATION FOR SEQ ID NO: 184:

(i) SEQUENCE CHARACTERISTICS:
(A) LENGTH: 1596 base pairs
(B) TYPE: nucleic acid
(C) STRANDEDNESS: double

(D) TOPOLOGY: linear

(xi) SEQUENCE DESCRIPTION: SEQ ID NO: 184:

GTCATGCAGT GCGCCGGAGA ACTGTGCTCT TTGAGGCCGA CGCTAGGGGC CCGGAAGGGA 60

AACTGCGAGG CGAAGGTGAC CGGGGACCGA GCATTTCAGA TCTGCTCGGT AGACCTGGTG 120

CACCACCACC ATGTTGGCTG CAAGGCTGGT GTGTCTCCGG ACACTACCTT CTAGGGTTTT 180

CCACCCAGCT TTCACCAAGG CCTCCCCTGT TGTGAAGAAT TCCATCACGA AGAATCAATG 240

GCTGTTAACA CCTAGCAGGG AATATGCCAC CAAAACAAGA ATTGGGATCC GGCGTGGGAG 300

AACTGGCCAA GAACTCAAAG AGGCAGCATT GGAACCATCG ATGGAAAAAA TATTTAAAAT 360

TGATCAGATG GGAAGATGGT TTGTTGCTGG AGGGGCTGCT GTTGGTCTTG GAGCATTGTG 420

CTACTATGGC TTGGGACTGT CTAATGAGAT TGGAGCTATT GAAAAGGCTG TAATTTGGCC 480

TCAGTATGTC AAGGATAGAA TTCATTCCAC CTATATGTAC TTAGCAGGGA GTATTGGTTT 540

AACAGCTTTG TCTGCCATAG CAATCAGCAG AACGCCTGTT CTCATGAACT TCATGATGAG 600

AGGCTCTTGG GTGACAATTG GTGTGACCTT TGCAGCCATG GTTGGAGCTG GAATGCTGGT 660

ACGATCAATA CCATATGACC AGAGCCCAGG CCCAAAGCAT CTTGCTTGGT TGCTACATTC 720

TGGTGTGATG GGTGCAGTGG TGGCTCCTCT GACAATATTA GGGGGTCCTC TTCTCATCAG 780

AGCTGCATGG TACACAGCTG GCATTGTGGG AGGCCTCTCC ACTGTGGCCA TGTGTGCGCC 840

CAGTGAAAAG TTTCTGAACA TGGGTGCACC CCTGGGAGTG GGCCTGGGTC TCGTCTTTGT 900

GTCCTCATTG GGATCTATGT TTCTTCCACC TACCACCGTG GCTGGTGCCA CTCTTTACTC 960

AGTGGCAATG TACGGTGGAT TAGTTCTTTT CAGCATGTTC CTTCTGTATG ATACCCAGAA 1020

AGTAATCAAG CGTGCAGAAG TATCACCAAT GTATGGAGTT CAAAAATATG ATCCCATTAA 1080

CTCGATGCTG AGTATCTACA TGGATACATT AAATATATTT ATGCGAGTTG CAACTATGCT 1140

GGCAACTGGA GGCAACAGAA AGAAATGAAG TGACTCAGCT TCTGGCTTCT CTGCTACATC 1200

AAATATCTTG TTTAATGGGG CAGATATGCA TTAAATAGTT TGTACAAGCA GCTTTCGTTG 1260

AAGTTTAGAA GATAAGAAAC ATGTCATCAT ATTTAAATGT TCCGGTAATG TGATGCCTCA 1320

GGTCTGCCTT TTTTTCTGGA GAATAAATGC AGTAATCCTC TCCCAAATAA GCACACACAT 1380

TTTCAATTCT CATGTTTGAG TGATTTTAAA ATGTTTTGGT GAATGTGAAA ACTAAAGTTT 1440

GTGTCATGAG AATGTAAGTC TTTTTTCTAC TTTAAAATTT AGTAGGTTCA CTGAGTAACT 1500

AAAATTTAGC AAACCTGTGT TTGCATATTT TTTKGGAGTG CAGMMTAWTG TAATTARAGC 1560

ATTCCAGTAA NAGTGTNTTT AAAGTTGNTC TATATN 1596

(2) INFORMATION FOR SEQ ID NO: 185:

(i) SEQUENCE CHARACTERISTICS:
(A) LENGTH: 2293 base pairs
(B) TYPE: nucleic acid
(C) STRANDEDNESS: double
(D) TOPOLOGY: linear

(xi) SEQUENCE DESCRIPTION: SEQ ID NO: 185:

GCGCAGAGCC CGYACGAGCA GGACGACGAC GACAAGGGCG ACTCCAAGGA AACGCGGCTG 60

ACCCTGATGG AGGAAGTGCT CCTGCTGGGC CTCAAGGACC GCGARGGTTA CACATCATTT 120

TGGAATGACT GTATATCATC TGGATTACGT GGCTGTATGT TAATTGAATT AGCATTGAGA 180

GGAAGGTTAC AACTAGAGGC TTGTGGAATG AGACGTAAAA GTCTATTAAC AAGAAAGGTA 240

ATCTGTAAGT CAGATGCTCC AACAGGGGAT GTTCTTCTTG ATGAAGCTCT GAAGCATGTT 300

AAGGAAACTC AGCCTCCAGA AACGGTCCAG AACTGGATTG AATTACTTAG TGGTGAGACA 360

TGGAATCCAT TAAAATTGCA TTATCAGTTA AGAAATGTAC GGGAACGATT AGCTAAAAAC 420

CTGGTGGAAA AGGGTGTATT GACAACAGAG AAACAGAACT TCCTACTTTT TGACATGACA 480

ACACATCCCC TCACCAATAA CAACATTAAG CAGCGCCTCA TCAAGAAAGT ACAGGAAGCC 540

GTTCTTGACA AATGGGTGAA TGACCCTCAC CGCATGGACA GGCGCTTGCT GGCCCTCATT 600

TACCTGGCTC ATGCCTCGGA CGTCCTGGAG AATGCTTTTG CTCCTCTTCT GGACGAGCAG 660

TATGATTTGG CTACCAAGAG AGTGCGGCAG CTTCTCGACT TAGACCCTGA AGTGGAATGT 720

CTGAAGGCCA ACACCAATGA GGTTCTGTGG GCGGTGGTGG CGGCGTTCAC CAAGTAACTC 780

TGCTCGGGGT GAACCATTCT CCTTTCTCTC AAGTAAACCA GTAGTTTTTC TTCTGTTGAC 840

TTCTGGTTTT CTGTAATTTG TACTTTCCCA CACTATAATT GGCTTCTGTT TTACAAAATG 900

GTGGGTGGCT TTTTCTTTTT TGTACGTGTA CAGGATTCTG CTGGTACGAG AGGCCTTCCT 960

CTTTCTGTTT TTAAAAAAAG TTTTACTGCC ATATTGGCAT TCCATTCCCT GTTGCCATCC 1020

TCACTGTTAC CTGTTTTGGG TTTCTGGTCT ACTTTGACTT TCAAAGTACC TCCAGCCTCC 1080

TCATACGCAC AGCTTTTGGA TGACCTCAGC TTGAGTTTCT CCATATGTGC ATGTACATCT 1140

AGCATTCTGC CTACAGTTCA GACAGAAGTC ACAAAAAGGC CTTCAACTCA CCAAAGGTAA 1200

ATATCTGTAT CTATTAGGAC ATTTTTTACA TAGACTTCAG TTGAGATGTA TACTTAGCAA 1260

AATTATTTTT AAATTGAAAC AGCACAGTAA ATACTTAATA TAAAATGTCC CTTGGATTTT 1320

GCTTCCCATG TAAATCTATT GTATTATTAC ACTTGTTATA ATTTTAACTA TAAAGGTCCA 1380

ATTGTTTCAC AGAGCCAGTT TGGGATGGGC TGCATTCCAT TTATGCTGTA TATAGTTTGA 1440

ATTATATATA AATTACCCCT TCTTCTGGCC ACCCCTGCTC CCATCTTAGT ATTTTGCAAG 1500

ATCTAATCAG TTGTACACCT GGTGCCCCTC GCTTGCTTCA ATCATGGTTA TTTGATGGCA 1560

AAATCGACCT CTTGTCGCTG AAGGAGAGAG AAAAGATGTG TGTCTGATTG GTCCTGGGAT 1620

TTTTTGAGCT GTGCCATTTA TGGTACTCTT TGCCTATGCA TCCCCTTTTT AGATTTTTTT 1680

TAAATTTTAT CTTACTGTTT TTATAATTTC TATTGGGAAG AGGCTTGTGA CCAGTACCAA 1740

TCTTGAGTTT CTTTTTCTGT CCACAAGTAA ATTAATATCT GCTCTGAAAT GTCATTTATC 1800

TACTCACACA TTCTTGGGGA AAAAAATCAA ATGTCAGTCC TAGCAGATGT TGCATGTAAA 1860

TTGGTAGCAA GTAATGATTA CAACCCAGAG GATTAAGAAT TTTGTAACAG AAAGCTCTAT 1920

GTTTTAATTT TTTATATACA ATTAGGATAA TTAGCATTGT CAGACTATAA ACCTTTGCTT 1980

TTTAAAGTTT ATTTTTACTA TTTCTTTATC ACTTTATTGT ATCATCACCA TTGGTTTCAT 2040

AATGTAAATA CTATATGTTG AACAAATTAA ATGTCAAAAT TTTTTATTAC CATAGTCCAT 2100

GTTAATAGTG GGGCTTTCAG GTGTTTAGAG ATTTTTTTTG TTGTTGTTAA CATTCATTGC 2160

AAAAGTACTA GATGGTGTAT AACTCTAGAG TTGAATTTTA AGGGATTCCC TAATATGTAT 2220

ACTATCTTTT TATCTGAAGT AATAAATAAA CAATGATCTT GAAAGTGCCY RAAAMAAAAA 2280

AAAAAAAAAA AAA 2293

(2) INFORMATION FOR SEQ ID NO: 186:

(i) SEQUENCE CHARACTERISTICS:
(A) LENGTH: 1212 base pairs
(B) TYPE: nucleic acid
(C) STRANDEDNESS: double
(D) TOPOLOGY: linear

(xi) SEQUENCE DESCRIPTION: SEQ ID NO: 186:

GGCACGAGGC GAGCCGGCGC ACCGTACGCT GGGACGTGTG GTTTCAGCTC GTGCGCCTCC 60

CCGTGGGTTT GCGACGTTTA GCGACTATTG CGCCTGCGCC ACGCCGGCTG CGAGACTGGG 120

GCCGTGGCTG CTGGTCCCGG GTGATGCTAG GCGGCTCCCT GGGCTCCAGG CTGTTGCGGG 180

GTGTAGGTGG GAGTCACGGA CGGTTCGGGG CCCGAGGTGT CCGCGAAGGT GGCGCACATG 240

GGCGGCAGGG GAGAGCATGG CTCAGCGGAT GGTCTGGGTG GACCTGGAGA TGACAGGATT 300

GGACATTGAG AAGGACCAGA TTATTGAGAT GGCCTGTCTG ATAACTGACT CTGATCTCAA 360

CATTTTGGCT GAAGGTCCTA ACCTGATTAT AAAACAACCA GATGAGTTGC TGGACAGCAT 420

GTCAGATTGG TGTAAGGAGC ATCACGGGAA GTCTGGCCTT ACCAAGGCAG TGAAGGAGAG 480

TACAATTACA TTGCAGCAGG CAGAGTATGA ATTTCTGTCC TTTGTACGAC AGCAGACTCC 540

TCCAGGGCTC TGTCCACTTG CAGGAAATTC AGTTCATGAA GATAAGAAGT TTCTTGACAA 600

ATACATGCCC CAGTTCATGA AACATCTTCA TTATAGAATA ATTGATGTGA GCACTGTTAA 660

AGAACTGTGC AGACGCTGGT ATCCAGAAGA ATATGAATTT GCACCAAAGA AGGCTGCTTC 720

TCATAGGGCA CTTGATGACA TTAGTGAAAG CATCAAAGAG CTTCAGTTTT ACCGAAATAA 780

CATCTTCAAG AAAAAAATAG ATGAAAAGAA GAGGAAAATT ATAGAAAATG GGGAAAATGA 840

GAAGACCGTG AGTTGATGCC AGTTATCATG CTGCCACTAC ATCGTTATCT GGAGGCAACT 900

TCTGGTGGTT TTTTTTTCTC ACGCTGATGG CTTGGCAGAG CACCTTCGGT TAACTTGCAT 960

CTCCAGATTG ATTACTCAAG CAGACAGCAC ACGAAATACT ATTTTTCTCC TAATATGCTG 1020

TTTCCATTAT GACACAGCAG CTCCTTTGTA AGTACCAGGT CATGTCCATC CCTTGGTACA 1080

TATATGCATT TGCTTTTAAA CCATTTCTTT TGTTTAAATA AATAAATAAG TAAATAAAGC 1140

TAGTTCTATT GAAATGCAAA AAAAAAAAAA AAAAAAAAAA AAAAAAAAAA AAAAAAAAAA 1200

AAAAAAAAAA AN 1212

(2) INFORMATION FOR SEQ ID NO: 187:

(i) SEQUENCE CHARACTERISTICS:
(A) LENGTH: 1605 base pairs
(B) TYPE: nucleic acid
(C) STRANDEDNESS: double
(D) TOPOLOGY: linear

(xi) SEQUENCE DESCRIPTION: SEQ ID NO: 187:

GCTTCCGGAA GTTGCTTTTG TCCAAACATC CGGGCTTCTC CTTTTTGTGT TCCGGCCGAT 60

CCCACCTCTC CTCGACCCTG GACGTCTACC TTCCGGAGGC CCACATCTTG CCCACTCCGC 120

GCGCGGGGCT AGCGCGGGTT TCAGCGACGG GAGCCCTCAA GGGACATGGC AACTACAGCG 180

GCGCCGGCGG GCGGCGCCCG AAATGGAGCT GGCCCGGAAT GGGGAGGGTT CGAAGAAAAC 240

ATCCAGGGCG GAGGCTCAGC TGTGATTGAC ATGGAGAACA TGGATGATAC CTCAGGCTCT 300

AGCTTCGAGG ATATGGGTGA GCTGCATCAG CGCCTGCGCG AGGAAGAAGT AGACGCTGAT 360

GCAGCTGATG CAGCTGCTGC TGAAGAGGAG GATGGAGAGT TCCTGGGCAT GAAGGGCTTT 420

AAGGGACAGC TGAGCCGGCA GGTGGCAGAT CAGATGTGGC AGGCTGGGAA AAGACAAGCC 480

TCCAGGGCCT TCAGCTTGTA CGCCAACATC GACATCCTCA GACCCTACTT TGATGTGGAG 540

CCTGCTCAGG TGCGAACAGG GCTCCTGGAG TCCATGATCC CTATCAAGAT GGTCAACTTC 600

CCCCAGAAAA TTGCAGGTGA ACTCTATGGA CCTCTCATGC TGGTCTTCAC TCTGGTTGCT 660

ATCCTACTCC ATGGGATGAA GACGTCTGAC ACTATTATCC GGGAGGGCAC CCTGATGGGC 720

ACAGCCATTG GCACCTGCTT CGGCTACTGG CTGGGAGTCT CATCCTTCAT TTACTTCCTT 780

GCCTACCTGT GCAACGCCCA GATCACCATG CTGCAGATGT TGGCACTGCT GGGCTATGGC 840

CTCTTTGGGC ATTGCATTGT CCTGTTCATC ACCTATAATA TCCACCTCCA CGCCCTCTTC 900

TACCTCTTCT GGCTGTTGGT GGGTGGACTG TCCACACTGC GCATGGTAGC AGTGTTGGTG 960

TCTCGGACCG TGGGCCCCAC ACAGCGGCTG CTCCTCTGTG GCACCCTGGC TGCCCTACAC 1020

ATGCTCTTCC TGCTCTATCT GCATTTTGCC TACCACAAAG TGGTAGAGGG GATCCTGGAC 1080

ACACTGGAGG GCCCCAACAT CCCGCCCATC CAGAGGGTCC CCAGAGACAT CCCTGCCATG 1140

CTCCCTGCTG CTCGGCTTCC CACCACCGTC CTCAACGCCA CAGCCAAAGC TGTTGCGGTG 1200

ACCCTGCAGT CACACTGACC CCACCTGAAA TTCTTGGCCA GTCCTCTTTC CCGCAGCTGC 1260

AGAGAGGAGG AAGACTATTA AAGGACAGTC CTGATGACAT GTTTCGTAGA TGGGGTTTGC 1320

AGCTGCCACT GAGCTGTAGC TGCGTAAGTA CCTCCTTGAT GCNTGTCGGC ACTTCTGAAA 1380

GGCACAAGGC CAAGAACTCC TGGCCAGGAC TGCAAGGCTC TGCAGCCAAT GCAGAAAATG 1440

GGTCAGCTCC TTTGAGAACC CCTCCCCACC TACCCCTTCC TTCCTCTTTA TCTCTCCCAC 1500

ATTGTCTTGC TAAATATAGA CTTGGTAATT AAAATGTTGA TTGAAGTCTG GAAAAAAAAA 1560

AAAAAAAAAA AAAAAAAAAA AAAAAAAAAA AAAAAAAAAC TCGAG 1605

(2) INFORMATION FOR SEQ ID NO: 188:

(i) SEQUENCE CHARACTERISTICS:
(A) LENGTH: 1516 base pairs
(B) TYPE: nucleic acid
(C) STRANDEDNESS: double
(D) TOPOLOGY: linear

(xi) SEQUENCE DESCRIPTION: SEQ ID NO: 188:

ATTCGGCATG AGGGGGTCAC GTGGTGGCTG GGCCGGGGAA ATGGCGGCTT CAGGAGAGAG 60

CGGGACTTCA GGCGGCGGAG GCAGCACCGA GGAAGCATTT ATGACCTTCT ACAGTGAGGT 120

GAAACAAATA GAGAAGAGAG ACTCGGTTCT AACTTCGAAA AATCAGATTG AAAGACTGAC 180

CCGTCCTGGT TCCTCTTACT TCAATTTGAA CCCATTTGAG GTTCTTCAGA TAGATCCTGA 240

AGTTACAGAT GAAGAAATAA AAAAGAGGTT TCGGCAGTTA TCCATCTTGG TGCATCCTGA 300

CAAAAATCAA GATGATGCTG ACAGAGCACA AAAGGCTTTT GAAGCTGTGG ACAAAGCTTA 360

CAAGTTGCTA CTGGATCAGG AGCAAAAGAA GAGGGCCCTG GATGTAATTC AGGCAGGAAA 420

AGAATACGTG GAACACACTG TGAAAGAGCG AAAAAAACAA TTAAAGAAGG AAGGAAAACC 480

TACAATTGTA GAGGAGGATG ATCCTGAGCT GTTCAAACAA GCTGTATATA AACAGACAAT 540

GAAACTCTTT GCAGAGCTGG AAATTAAAAG GAAAGAGAGA GAAGCCAAAG AGATGCATGA 600

AAGGAAACGA CAAAGGGAAG AAGAGATTGA AGCTCAAGAA AAAGCCAAAC GGGAAAGAGA 660

GTGGCAGAAA AACTTTGAGG AAAGTCGAGA TGGTCGTGTG GACAGCTGGC GAAACTTCCA 720

AGCCAATACG AAGGGGAAGA AAGAGAAGAA AAATCGGACC TTCCTGAGAC CACCGAAAGT 780

AAAAATGGAG CAACGTGAGT GACCGCCCAA GGTCACAGGC ACAGAACCTT TCCCCTGCTA 840

TCTCCCTTCC TGCTTCGAAG GACTCATTCT TTCCTCCCAC TTCCACCCCA ACATAGAGTA 900

GTATTTGCTT TTTAGTCCAT TTTGTTTTCA ATACGATTTA ATATCGATCA GAGTAATTCT 960

TTTGTACATT GAAATGAGGG GCTTGGTTTA AAAAAAGACC TTTCCCTCTC CCTGCCCCTA 1020

GAACAACCAG TATTAGAAGG TGCCACCATT GGTGCTGCCT TCTCTTCCCA CAGCCTGTAA 1080

CTCAGTGTTT TGTACTTCAC TGAATTGTGA TGGTTAGAAA CTTCGTGGAT AGTTTGTGGA 1140

AATCATCCAA TTAAACATAC TGCTTAAAAC AGTGTTGCTG TGACTTCAGA GACAAGCCTG 1200

GAAGGGGCAC CTTAGGAAGC CCCTTCGCTT CAGTTGCTCG CTTCTGGGTG TGCTCCCTTC 1260

GAAGGCCCAG ATAAGACAGG GAACACTTGT GAGCACACAG AGCAGCATCT GATGCCCTGT 1320

GGTGTTTGGC ATGTGCCCCC TGTCTACTGA CCAATCAGTG TGGCATGAGG CCCACGCCAC 1380

CCAAACCTTT CACTTTCCAA AGAGCTAGCC GTCCTCCACC CAGTACCATG TCCTAGCCTG 1440

TCTGCATTTG TTAGTGGTAA TATTCTTTAT GTATAATAAA TTTTTATACC CAAAAAAAAA 1500

AAAAAAAAAA ACTCGA 1516

(2) INFORMATION FOR SEQ ID NO: 189:

(i) SEQUENCE CHARACTERISTICS:
(A) LENGTH: 681 base pairs
(B) TYPE: nucleic acid
(C) STRANDEDNESS: double
(D) TOPOLOGY: linear

(xi) SEQUENCE DESCRIPTION: SEQ ID NO: 189:

GCTCCCATGT TGCTGGCTGT CCGTACATCA CCCTGTCCCC TGCAGGAGGG GGCTACAGGC 60

CATCTCCCTC CTGTAGGCCT CTGACTCCCC TCCACTTTTG GGCCCTCAGC TTATCTCGGG 120

CAGGGGACCA TTGCAGCATC CTCCCCTCCT CNGGACTCAA GGTGCTGAGG TATAAGCCCT 180

GGGCCCCAGA TCCCTGRTKA CACCTTCCTG GAGAAGACTC TCAAAAGTGA CTGTATATTT 240

GAGTTCACCA GCAATAACTC CCCACACTCG AAGCAGGTCC AAACCCMAGG ATCCCAGGGT 300

CCTTGGGCTC TGTGGCACTG TCTTCCCAAG ATCCTTCCTG TTGCACAATG GGAAACCTAA 360

GAGGAAAAAG ACAGGGGCCT GCTTGCCCAG CCATGCGAGG GATTCCATGC CCACCTGCCC 420

TCTGYCTGCC TCGCTGGAAT GTGGGCCCCT GCTCCCCGTC AGGTTGTGCT GTCTCTGACC 480

TATGTTTACA TCCCCGAGGG GTTTCTGCCT CCTCCCCACC CAGGTCAGGG TGTGGTCCAG 540

CAGCTTGCTG TGGGGTGCTG ACATGTGTCA CCACTGCCCC CCTTGCCCCC GGGGGGGTCA 600

TGGTCTCCTC CTGGATGCTG CTCCTTGAAT YTTTTTTYTT GAWAAACCYT TTAMAATTAA 660

AAAAAAAAAA AAAAAACTCG A 681

(2) INFORMATION FOR SEQ ID NO: 190:

(i) SEQUENCE CHARACTERISTICS:
(A) LENGTH: 1014 base pairs
(B) TYPE: nucleic acid
(C) STRANDEDNESS: double
(D) TOPOLOGY: linear

(xi) SEQUENCE DESCRIPTION: SEQ ID NO: 190:

GCCTCAAGCC ACGCATATGA TAATTTTCTG GAACATTCAA ATTCAGTGTT TCTACAGCCA 60

GTTAGTCTAC AAACCATTGC AGCAGCACCA TCAAACCAGA GTCTGCCACT TTTTGTCATC 120

GCTGGATGAT TGCTGGGCAA AGGTGGCCTT TTAGAGCTCT TAAAAGCCCA CAAAAAGGCT 180

ATTCGTAGAG CCACAGTCAA CACATTTGGT TATATTGCAA AGGCCATTGG CCTCATGATG 240

TATTGGCTAC ACTTCTGAAC AACCTCAAAG TTCAAGAAAG GCAGAACAGA GTTTGTACCA 300

CTGTAGCAAT AGCTATTGTT GCAGAAACAT GTTCACCCTT TACAGTACTC CCTGCCTTAA 360

TGAATGAATA CAGAGTTCCT GAACTGAATG TTCAAAATGG AGTGTTAAAA TCGCTTTCCT 420

TCTTGTTTGA ATATATTGGT GAAATGGGAA AAGACTACAT TTATGCCGTA ACACCGTTAC 480

TTGAAGATGC TTTAATGGAT AGAGACCTTG TACACAGACA GACGGCTAGT GCAGTGGTAC 540

AGCACATGTC ACTTGGGGTT TATGGATTTG GTTGTGAAGA TTCGCTGAAT CACTTGTTGA 600

ACTATGTATG GCCCAATGTR TTTGAGACAT CTCCTCATGT AATTCAGGCA GTTATGGGAG 660

CCCTAGAGGG CCTGAGAGTT GCTATTGGAC CATGTAGAAT GTTGCAATAT TGTTTACAGG 720

GTCTGTTTCA CCCAGCCCGG AAAGTCAGAG ATGTATATTG GAAAATTTAC AACTCCATCT 780

ACATTGGTTC CCAGGACGCT CTCATAGCAC ATTACCCAAG AATCTACCAA CGATGATAAG 840

RACACCTATA TTCGTTATGA ACTTGACTAT ATCTTATAAT TTTATTGTTW ATTTKGTGKT 900

TAATGCACAS TACTTCACAC CTTAAACTTG CTTTGATTTG GTGATGTAAA CTTTTAAACA 960

TTGCAGATCA GTGTAGGACT GGTCCATAGG GGAAGAGCTA GGAANTCCAT AGGC 1014

(2) INFORMATION FOR SEQ ID NO: 191:

(i) SEQUENCE CHARACTERISTICS:
(A) LENGTH: 2779 base pairs
(B) TYPE: nucleic acid
(C) STRANDEDNESS: double
(D) TOPOLOGY: linear

(xi) SEQUENCE DESCRIPTION: SEQ ID NO: 191:

TCGCAGCAGG GTGTGTCCAG ATGGTCAGTC TCTGGTGGCT AGCCTGTCCT GACAGGGGAG 60

AGTTAAGCTC CCGYTCTCCA CCGTGCCGGC TGGCCAGGTG GGCTGAGGGT GACCGAGAGA 120

CCAGAACCTG CTTGCTGGAG CTTAGTGCTC AGAGCTGGGG AGGGAGGTTC CGCCGCTCCT 180

CTGCTGTCAG CGCCGGCAGC CCCTCCCGGC TTCACTTCCT CCCGCAGCCC CTGCTACTGA 240

GAAGCTCCGG GATCCCAGCA GCCGCCACGC CCTGGCCTCA GCCTGCGGGG CTCCAGTCAG 300

GCCAACACCG ACGCGCANTG GGAGGAAGAC AGGACCCTTG ACATCTCCAT CTGCACAGAG 360

GTCCTGGCTG GACCGAGCAG CCTCCTCCTC CTAGGATGAC CTCACCCTCC AGCTCTCCAG 420

TTTTCAGGTT GGAGACATTA GATGGAGGCC AAGAAGATGG CTCTGAGGCG GACAGAGGAA 480

AGCTGGATTT TGGGAGCGGG CTGCCTCCCA TGGAGTCACA GTTCCAGGGC GAGGACCGGA 540

AATTCGCCCC TCAGATAAGA GTCAACCTCA ACTACCGAAA GGGAACAGGT GCCAGTCAGC 600

CGGATCCAAA CCGATTTGAC CGAGATCGGC TCTTCAATGC GGTCTCCCGG GGTGTCCCCG 660

AGGATCTGGC TGGACTTCCA GAGTACCTGA GCAAGACCAG CAAGTACCTC ACCGACTCGG 720

AATACACAGA GGGCTCCACA GGTAAGACGT GCCTGATGAA GGCTGTGCTG AACCTTAAGG 780

ACGGGGTCAA TGCCTGCATT CTGCCACTGC TGCAGATCGA CCGGGACTCT GGCAATCCTC 840

AGCCCCTGGT AAATGCCCAG TGCACAGATG ACTATTACCG AGGCCACAGC GCTCTGCACA 900

TCGCCATTGA GAAGAGGAGW CTGCAGTGTG TGAAGCTCCT GGTGGAGAAT GGGGCCAATG 960

TGCATGCCCG GGTCTGCGGC GCTTCTTCCA GAAGGGCCAA GGGACTTGCT TTTATTTCGG 1020

TGAGCTACCC CTCTYTTTGG CCGCTTGCAC CAAGCAGTGG GATGTGGTAA GCTACCTCCT 1080

GGAGAACCCA CACCAGCCCG CCAGCCTGCA GGCACTGACT CCCAGGGCAA CACAGTCCTG 1140

CATGCCCTAG TGATGATCTC GGACAACTCA GCTGAGAACA TTGCACTGGT GACCAGCATG 1200

TATGATGGGC TCCTCCAAGC TGGGGCCCGC CTCTGCCCTA CCGTGCAGCT TGAGGACATC 1260

CGCAACCTGC AGGATCTCAC GCCTCTGAAG CTGGCCGCCA AGGAGGGCAA GATCGAGATT 1320

TTCAGGCACA TCCTGCAGCG GGAGTTTTCA GGACTGAGCC ACCTTTCCCG AAAGTTCACC 1380

GAGTGGTGCT ATGGGCCTGT CCGGGTGTCG CTGTATGACC TGGCTTCTGT GGACAGCTGT 1440

GAGGAGAACT CAGTGCTGGA GATCATTGCC TTTCATTGCA AGAGCCCGCA CCGACACCGA 1500

ATGGTCGTTT TGGAGCCCCT GAACAAACTG CTGCAGGCGA AATGGGATCT GCTCATCCCC 1560

AAGTTCTTCT TAAACTTCCT GTGTAATCTG ATCTACATGT TCATCTTCAC CGCTGTTGCC 1620

TACCATCAGC CTACCCTGAA GAAGCAGGCC GCCCCTCACC TGAAAGCGGA GGTTGGAAAC 1680

TCCATGCTGC TGACGGGCCA CATCCTTATC CTGCTAGGGG GGATCTACCT CCTCGTGGGC 1740

CAGCTGTGGT ACTTCTGGCG GCGCCACGTG TTCATCTGGA TCTCGTTCAT AGACAGCTAC 1800

TTTGAAATCC TCTTCCTGTT CCARGCCCTG CTCACAGTGG TGTCCCARGT GCTGTGTTTC 1860

CTGGSCATCG AGTGGTACCT GCCCCTGCTT GTGTCTGCGC TGGTGCTGGG CTGGCTGAAC 1920

CTGCTTTACT ATACACGTGG CTTCCAGCAC ACAGGCATCT ACAGTGTCAT GATCCAGAAG 1980

CCCTGGTGAG CCTGAGCCAG GANNTTGGCG CCCCGAAGCT CCTACAGGCC CCAATGCCAC 2040

AGAGTCAGTG CAGCCCATGG AGGGACAGGA KGACGAKGGC AACGGGGCCC AGTACAGGGG 2100

TATCCTGGAA GCCTCCTTGG AGCTCTTCAA ATTCACCATC GGCATGGGCG AGCTGGCCTT 2160

CCAGGARCAG CTGCACTTCC GCGGCATGGT GCTGCTGCTG CTGCTGGSCT ACGTGCTGCT 2220

CACCTACATC CTGCTGCTCA ACATGCTCAT CGCCCTCATG AGCGAGACCG TCAACAGTGT 2280

CGCCACTGAC AGCTGGAGCA TCTGGAAGCT GCAGAAAGCC ATCTCTGTCC TGGAGATGGA 2340

GAATGGCTAT TGGTGGTGCA GGAAGAAGCA GCGGGCAGGT GTGATGCTGA CCGTTGGCAC 2400

TAAGCCAGAT GGCAGCCCSG ATGAGCGCTG GTGCTTCAGG GTGGAGGAGG TGAACTGGGC 2460

TTCATGGGAG CAGACGCTGC CTACGCTGTG TGAGGACCCG TCAGGGGCAG GTGTCCCTCG 2520

AACTCTCGAG AACCCTGTCC TGGCTTCCCC TCCCAAGGAG GATGAGGATG GTGCCTCTGA 2580

GGAAAACTAT GTGCCCGTCC AGCTCCTCCA GTCCAACTGA TGGCCCAGAT GCAGCAGGAG 2640

GCCAGAGGAC AGAGCAGAGG ATCTTTCCAA CCACATCTGC TGGCTCTGGG GTCCCAGTGA 2700

ATTCTGGTGG CAAATATATA TTTTCACTAA CTCAAAAAAA AAAAAAAAAA AAAAAAAAAA 2760

AAAAAAAAAA AAAAAAGGC 2779

(2) INFORMATION FOR SEQ ID NO: 192:

(i) SEQUENCE CHARACTERISTICS:
(A) LENGTH: 1923 base pairs
(B) TYPE: nucleic acid
(C) STRANDEDNESS: double
(D) TOPOLOGY: linear

(xi) SEQUENCE DESCRIPTION: SEQ ID NO: 192:

ACCCGCTCCG CTCCGCTCCG CTCGGCCCCG CGCCGCCCGT CAACATGATC CGCTGCGGCC 60

TGGCCTGCGA GCGCTGCCGC TGGATCCTGC CCCTGCTCCT ACTCAGCGCC ATCGCCTTCG 120

ACATCATCGC GCTGGCCGGC CGCGGCTGGT TGCAGTCTAG CGACCACGGC CAGACGTCCT 180

CGCTGTGGTG GAAATGCTCC CAAGAGGGCG GCGGCAGCGG GTCCTACGAG GAGGGCTGTC 240

AGAGCCTCAT GGAGTACGCG TGGGGTAGAG CAGCGGCTGC CATGCTCTTC TGTGGCTTCA 300

TCATCCTGGT GATCTGTTTC ATCCTCTCCT TCTTCGCCCT CTGTGGACCC CAGATGCTTG 360

TCTTCCTGAG AGTGATTGGA GGTCTCCTTG CCTTGGCTGC TGTGTTCCAG ATCATCTCCC 420

TGGTAATTTA CCCCGTGAAG TACACCCAGA CCTTCACCCT TCATGCCAAC CSTGCTGTCA 480

CTTACATCTA TAACTGGGCC TACGGCTTTG GGTGGGCAGC CACGATTATC CTGATYGGCT 540

GTGCCTTCTT CTTCTGCTGC CTCCCCAACT ACGAAGATGA CCTTCTGGGC AATGCCAAGC 600

CCAGGTACTT CTACACATCT GCCTAACTTG GGAATGAATG TGGGAGAAAA TCGCTGCTGC 660

TGAGATGGAC TCCAGAAGAA GAAACTGTTT CTCCAGGCGA CTTTGAACCC ATTTTTTGGC 720

AGTGTTCATA TTATTAAACT AGTCAAAAAT GCTAAAATAA TTTGGGAGAA AATATTTTTT 780

AAGTAGTGTT ATAGTTTCAT GTTTATCTTT TATTATGTTT TGTGAAGTTG TGTCTTTTCA 840

CTAATTACCT ATACTATGCC AATATTTCCT TATATCTATC CATAACATTT ATACTACATT 900

TGTAAGAGAA TATGCACGTG AAACTTAACA CTTTATAAGG TAAAAATGAG GTTTCCAAGA 960

TTTAATAATC TGATCAAGTT CTTGTTATTT CCAAATAGAA TGGACTCGGT CTGTTAAGGG 1020

CTAAGGAGAA GAGGAAGATA AGGTTAAAAG TTGTTAATGA CCAAACATTC TAAAAGAAAT 1080

GCAAAAAAAA AGTTTATTTT CAAGCCTTCG AACTATTTAA GGAAAGCAAA ATCATTTCCT 1140

AAATGCATAT CATTTGTGAG AATTTCTCAT TAATATCCTG AATCATTCAT TTCAGCTAAG 1200

GCTTCATGTT GACTCGATAT GTCATCTAGG AAAGTACTAT TTCATGGTCC AAACCTGTTG 1260

CCATAGTTGG TAAGGCTTTC CTTTAAGTGT GAAATATTTA GATGAAATTT TCTCTTTTAA 1320

AGTTCTTTAT AGGGTTAGGG TGTGGGAAAA TGCTATATTA ATAAATCTGT AGTGTTTTGT 1380

GTTTATATGT TCAGAACCAG AGTAGACTGG ATTGAAAGAT GGACTGGGTC TAATTTATCA 1440

TGACTGATAG ATCTGGTTAA GTTGTGTAGT AAAGCATTAG GAGGGTCATT CTTGTCACAA 1500

AAGTGCCACT AAAACAGCCT CAGGAGAATA AATGACTTGC TTTTCTAAAT CTCAGGTTTA 1560

TCTGGGCTCT ATCATATAGA CAGGCTTCTG ATAGTTTGCA ACTGTAAGCA GAAACCTACA 1620

TATAGTTAAA ATCCTGGTCT TTCTTGGTAA ACAGATTTTA AATGTCTGAT ATAAAACATG 1680

CCACAGGAGA ATTCGGGGAT TTGAGTTTCT CTGAATAGCA TATATATGAT GCATCGGATA 1740

GGTCATTATG ATTTTTTACC ATTTCGACTT ACATAATGAA AACCAATTCA TTTTAAATAT 1800

CAGATTATTA TTTTGTAAGT TGTGGAAAAA GCTAATTGTA GTTTTCATTA TGAAGTTTTC 1860

CCAATAAACC AGGTATTCTA AAAAAAAAAA AAAAAAACTN GAGGGGGGGC CCGGTACCCA 1920

ATT 1923

(2) INFORMATION FOR SEQ ID NO: 193:

(i) SEQUENCE CHARACTERISTICS:
(A) LENGTH: 2346 base pairs
(B) TYPE: nucleic acid
(C) STRANDEDNESS: double
(D) TOPOLOGY: linear

(xi) SEQUENCE DESCRIPTION: SEQ ID NO: 193:

AGGCTCAGGG GGACACTCTC AAAATTACAC AGCTTTTAAC AGGTGGCAGA ATTGGGGTTC 60

AGACCCAGAT CTGGGTTCAA GTCACTCATG GTGTGATTGC GGCATTCCTT CCCGCATCTG 120

GGCCTTGCCA TCTCTCTCTC CGAGTGGACA TGGAGAGGAC GGGGGCCCAG CAGCTGGATG 180

GCTGCAGGGG ATCAAGTCTT CTCTGGGGCT GGGCACGTAN AAGAGCATGT GGCTGGTGGA 240

CGGCATGCCT GGCTCCTCAC CTGGCAGTCT GCCTGCCCTG CTAACCGGCT GTCTCTTGTT 300

CCCCTAGTGC CCTCGGCTAG CATGACCCGC CTGATGCGWT SCCGCACAGC CTCTGGTTCC 360

AGCGTCATTC TCTGGATGGC ACCCGCAGCC GCTCCCACAC CAGCGAGGGC ACCCGAAGCC 420

GCTCCCACAC CAGCGAGGGC ACCCGCAGCC GCTCGCACAC CAGCGAGGGG GCCCACCTGG 480

ACATCACCCC CAACTCGGGT GCTGCTGGGA ACAGNGCCGG GCCCAAGTCC ATGGAGGTCT 540

CCTGCTAGGC GGCCTGCCCA GCTGCCGCCC CCGGACTCTG ATCTCTGTAG TGGCCCCCTC 600

CTCCCCGGCC CCTTTTCGCC CCCTGCCTGC CATACTGCGC CTAACTCGGT ATTAATCCAA 660

AGCTTATTTT GTAAGAGTGA GCTCTGGTGG AGACAAATGA GGTCTATTAC GTGGGTGCCC 720

TCTCCAAAGG CGGGGTGGCG GTGGACCAAA GGAAGGAAGC AAGCATCTCC GCATCGCATC 780

CTCTTCCATT AACCAGTGGC CGGTTGCCAC TCTCCTCCCC TCCCTCAGAG ACACCAAACT 840

GCCAAAAACA AGACGCGTAC AGCACACACT TCACAAAGCC AAGCCTAGGC CGCCCTGAGC 900

ATCCTGGTTC AAACGGGTGC CTGGTCAGAA GGCCAGCCGC CCACTTCCCG TTTCCTCTTT 960

AACTGAGGAG AAGCTGATCC AGTTTCCGGA AACAAAATCC TTTTCTCATT TGGGGAGGGG 1020

GGTAATAGTG ACATGCAGGC ACCTCTTTTA AACAGGCAAA ACAGGAAGGG GGAAAAGGTG 1080

GGATTCATGT CGAGGCTAGA GGCATTTGGA ACAACAAATC TACGTAGTTA ACTTGAAGAA 1140

ACCGATTTTT AAAGTTGGTG CATCTAGAAA GCTTTGAATG CAGAAGCAAA CAAGCTTGAT 1200

TTTTCTAGCA TCCTCTTAAT GTGCAGCAAA AGCAGGCRAC AAAATCTCCT GGCTTTACAG 1260

ACAAAAATAT TTCAGCAAAC GTTGGGCATC ATGGTTTTTG AAGGCTTTAG TTCTGCTTTC 1320

TGCCTCTCCT CCACAGCCCC AACCTCCCAC CCCTGATACA TGAGCCAGTG ATTATTCTTG 1380

TTCAGGGAGA AGATCATTTA GATTTGTTTT GCATTCCTTA GAATGGAGGG CAACATTCCA 1440

CAGCTGCCCT GGCTGTGATG AGTGTCCTTG CAGGGGCCGG AGTAGGAGCA CTGGGGTGGG 1500

GGCGGAATTG GGGTTACTCG ATGTAAGGGA TTCCTTGTTG TTGTGTTGAG ATCCAGTGCA 1560

GTTGTGATTT CTGTGGATCC CAGCTTGGTT CCAGGAATTT TGTGTGATTG GCTTAAATCC 1620

AGTTTTCAAT CTTCGACAGC TGGGCTGGAA CGTGAACTCA GTAGCTGAAC CTGTCTGACC 1680

CGGTCACGTT CTTGGATCCT CAGAACTCTT TGCTCTTGTC GGGGTGGGGG TGGGAACTCA 1740

CGTGGGGAGC GGTGGCTGAG AAAATGTAAG GATTCTGGAA TACATATTCC ATGGGACTTT 1800

CCTTCCCTCT CCTGCTTCCT CTTTTCCTGC TCCCTAACCT TTCGCCGAAT GGGGCAGCAC 1860

CACTGACGTT TCTGGGCGGC CAGTGCGGCT GCCAGGTTCC TGTACTACTG CCTTGTACTT 1920

TTCATTTTGG CTCACCGTGG ATTTTCTCAT AGGAAGTTTG GTCAGAGTGA ATTGAATATT 1980

GTAAGTCAGC CACTGGGACC CGAGGATTTC TGGGACCCCG CAGTTGGGAG GAGGAAGTAG 2040

TCCAGCCTTC CAGGTGGCGT GAGAGGCAAT GACTCGTTAC CTGCCGCCCA TCACCTTGGA 2100

GGCCTTCCCT GGCCTTGAGT AGAAAAGTCG GGGATCGGGG CAAGAGAGGC TGAGTACGGA 2160

TGGGAAACTA TTGTGCACAA GTCTTTCCAG AGGAGTTTCT TAATGAGATA TTTGTATTTA 2220

TTTCCAGACC AATAAATTTG TAACTTTGCA AAAAAAAAAA AAAAAAAAAA AAAAAAAAAA 2280

AAAAAAAAAA AAAAAAAACT CGAGGGGGGC CCGTACCCAA TTCGCCGTAT ATGATCGTAA 2340

ACAATC 2346

(2) INFORMATION FOR SEQ ID NO: 194:

(i) SEQUENCE CHARACTERISTICS:
(A) LENGTH: 3054 base pairs
(B) TYPE: nucleic acid
(C) STRANDEDNESS: double
(D) TOPOLOGY: linear

(xi) SEQUENCE DESCRIPTION: SEQ ID NO: 194:

TATCTGAACC ACCCTTTATT CTACATATGA TAGGCAGCAC TGAAATATCC TAACCCCCTA 60

AGCTCMAGGT GCCCTGTGGN ACGAGCAACT GGACTATAGC AGGGCTGGGC TCTGTCTTCC 120

TGGTCATAGG CTCACTCTTT CCCCCAAATC TTCCTCTGGA GCTTTGCAGC CAAGGTGCTA 180

AAAGGAATAG GTAGGAGACC TCTTCTATCT AATCCTTAAA AGCATAATGT TGAACATTCA 240

TTCAACAGCT GATGCCCTAT AACCCCTGCC TGGATTTCTT CCTATTAGGC TATAAGAAGT 300

AGCAAGATCT TTACATAATT CAGAGTGGTT TCATTGCCTT CCTACCCTCT CTAATGGCCC 360

CTCCATTTAT TTGACTAAAG CATCACACAG TGGCACTAGC ATTATACCAA GAGTATGAGA 420

AATACAGTGC TTTATGGCTC TAACATTACT GCCTTCAGTA TCAAGGCTGC CTGGAGAAAG 480

GATGGCAGCC TCAGGGCTTC CTTATGTCCT CCACCACAAG AGCTCCTTGA TGAAGGTCAT 540

CTTTTTCCCC TATCCTGTTC TTCCCCTCCC CGCTCCTAAT GGTACGTGGG TACCCAGGCT 600

GGTTCTTGGG CTAGGTAGTG GGGACCAAGT TCATTACCTC CCTATCAGTT CTAGCATAGT 660

AAACTACGGT ACCAGTGTTA GTGGGAAGAG CTGGGTTTTC CTAGTATACC CACTGCATCC 720

TACTCCTACC TGGTCAACCC GCTGCTTCCA GGTATGGGAC CTGCTAAGTG TGGAATTACC 780

TGATAAGGGA GAGGGAAATA CAAGGAGGGC CTCTGGTGTT CCTGGCCTCA GCCAGCTGCC 840

CACAAGCCAT AAACCAATAA AACAAGAATA CTGAGTCAGT TTTTTATCTG GGTTCTCTTC 900

ATTCCCACTG CACTTGGTGC TGCTTTGGCT GACTGGGAAC ACCCCATAAC TACAGAGTCT 960

GACAGGAAGA CTGGAGACTG*TCCACTTCTA GCTCGGAACT TACTGTGTAA ATAAACTTTC 1020

AGAACTGCTA CCATGAAGTG AAAATGCCAC ATTTTGCTTT ATAATTTCTA CCCATGTTGG 1080

GAAAAACTGG CTTTTTCCCA GCCCTTTCCA GGGCATAAAA CTCAACCCCT TCGATAGCAA 1140

GTCCCATCAG CCTATTATTT TTTTAAAGAA AACTTGCACT TGTTTTTCTT TTTACAGTTA 1200

CTTCCTTCCT GCCCCAAAAT TATAAACTCT AAGTGTAAAA AAAAGTCTTA ACAACAGCTT 1260

CTTGCTTGTA AAAATATGTA TTATACATCT GTATTTTTAA ATTCTGCTCC TGAAAAATGA 1320

CTGTCCCATT CTCCACTCAC TGCATTTGGG GCCTTTCCCA TTGGTCTGCA TGTCTTTTAT 1380

CATTGCAGGC CAGTGGACAG AGGGAGAAGG GAGAACAGGG GTCGCCAACA CTTGTGTTGC 1440

TTTCTGACTG ATCCTGAACA AGAAAGAGTA ACACTGAGGC GCTCGCTCCC ATGCACAACT 1500

CTCCAAAACA CTTATCCTCC TGCAAGAGTG GGCTTTCCAG GGTCTTTACT GGGAAGCAGT 1560

TAAGCCCCCT CCTCACCCCT TCCTTTTTTC TTTCTTTACT CCTTTGGCTT CAAAGGATTT 1620

TGGAAAAGAA ACAATATGCT TTACACTCAT TTTCAATTTC TAAATTTGCA GGGGATACTG 1680

AAAAATACGG CAGGTGGCCT AAGGCTGCTG TAAAGTTGAG GGGAGAGGAA ATCTTAAGAT 1740

TACAAGATAA AAAACGAATC CCCTAAACAA AAAGAACAAT AGAACTGGTC TTCCATTTTG 1800

CCACCTTTCC TGTTCATGAC AGCTACTAAC CTGGAGACAG TAACATTTCA TTAACCAAAG 1860

AAAGTGGGTC ACCTGACCTC TGAAGAGCTG AGTACTCAGG CCACTCCAAT CACCCTACAA 1920

GATGCCAAGG AGGTCCCAGG AAGTCCAGCT CCTTAAACTG ACGCTAGNCA ATAAACCTGG 1980

GCAAGTGAGG CAAGAGAAAT GAGGAAGAAT CCATCTGTGA GGTGACAGGC AAGGATGAAA 2040

GACAAAGAAG GAAAAGAGTA TCAAAGGCAG AAAGGAGATC ATTTAGTTGG GTCTGAAAGG 2100

AAAAGTCTTT GCTATCCGAC ATGTACTGCT AGTACCTGTA AGCATTTTAG GTCCCAGAAT 2160

GGAAAAAAAA ATCAGCTATT GGTAATATAA TAATGTCCTT TCCCTGGAGT CAGTTTTTTT 2220

AAAAAGTTAA CTCTTAGTTT TTACTTGTTT AATTCTAAAA GAGAAGGGAG CTGAGGCCAT 2280

TCCCTGTAGG AGTAAAGATA AAAGGATAGG AAAAGATTCA AAGCTCTAAT AGAGTCACAG 2340

CTTTCCCAGG TATAAAACCT AAAATTAAGA AGTACAATAA GCAGAGGTGG AAAATGATCT 2400

AGTTCCTGAT AGCTACCCAC AGAGCAAGTG ATTTATAAAT TTGAAATCCA AACTACTTTC 2460

TTAATATCAC TTTGGTCTCC ATTTTTCCCA GGACAGGAAA TATGTCCCCC CCTAACTTTC 2520

TTGCTTCAAA AATTAAAATC CAGCATCCCA AGATCATTCT ACAAGTAATT TTGCACAGAC 2580

ATCTCCTCAC CCCAGTGCCT GTCTGGAGCT CACCCAAGGT CACCAAACAA CTTGGTTGTG 2640

AACCNAACTG CCTTAACCTT CTGGGGGAGG GGGATTAGCT AGACTAGGAG ACCAGAAGTG 2700

AATGGGAAAG GGTGAGGACT TCACAATGTT GGCCTGTCAG AGCTTGATTA GAAGCCAAGA 2760

CAGTGGCAGC AAAGGAAGAC TTGGCCCAGG AAAAACCTGT GGGTTGTGCT AATTTCTGTC 2820

CAGAAAATAG GGTGGACAGA AGCTTGTGGG GTGCATGGAG GAATTGGGAC CTGGTTATGT 2880

TGTTATTCTC GGACTGTGAA TTTTGGTGAT GTAAAACAGA ATATTCTGTA AACCTAATGT 2940

CTGTATAAAT AATGAGCGTT AACACAGTAA AATATTCAAT AAGAAGTCAA AAAAAAAAAA 3000

AAAAAACTCG AGGGGGGGCC CGGTACCCAA TTTNCCAAAT AGAGATNGTA TTAC 3054

(2) INFORMATION FOR SEQ ID NO: 195:

(i) SEQUENCE CHARACTERISTICS:
(A) LENGTH: 907 base pairs
(B) TYPE: nucleic acid
(C) STRANDEDNESS: double
(D) TOPOLOGY: linear

(xi) SEQUENCE DESCRIPTION: SEQ ID NO: 195:

GGCAGAGCTC GTGGCCGNAA CTTTTTCTGC TCCTGGCTGC CACCTACTGG CTGGCCGCGG 60

CCCTGGCCTG GGCCTGCACC AGCCTGCGNG CGGGCTCCCA CAGCAGCCCC CTTCCAAGCA 120

GCGTCCCCAC ACCGCGCACC TTCTGCGGGA ACGTGCTCGC CGTGCCGGGG ACCATATGGA 180

CGGAAGGCTT TGTGCTCACC TACAAGCTGG GTGAGCAGGG TGCCAGCAGC CTGTTGATCC 240

TCTTGGCTCC TGCTGGAGCA CGAGCGGCGT TTCTGCTCCC GAGTTGGGAC TGTGGAATGG 300

TGTGGGTGCT GTGGTCTGCT CCATCGCTGG CTCCTCCCTG GGTGGGACCT TGCTGGCCAA 360

GCACTGGAAA CTGCTGCCTC TGTGAGGTCG GTGCTGCGCT TCCGCCTCGG GGGCCTAGCC 420

TGTCAGACTG CCTTGGTCTT CCACCTTGGA CACCCTGGGG GCCAGCATGG ACGCTGGCAC 480

AATCTTGAGA GGGTCAGCCT TGCTGAGCCT ATGTCTGCAG CACTTCTTGG GARGCCTGGT 540

CACCACAGTC ACCTTCACTG GGAATGATGC GCTGCAGCCA GCTGGCCCCC AGGGCCTTGC 600

AGGCCACACA CTACAGCCTT CTGGCCACGC TGGAGCTGCT GGGGAAGCTG CTGCTGGGCA 660

CTYTGGSCGG AGGGCCTGGC TGATGGGTTG GGGCCACATC CCTGCTTCTT GCTCCTGCTC 720

ATCCTCTCTG CCTTTCCCGT TCTGTACCTG GACCTAGCAC CCAGCACCTT TCTCTGAGCT 780

GAGTGGCTGG AGTGGTCAAT AAAGCCACAT GTGCCTGTGG CCCAAAAAAA AAAAAAAAAA 840

AAAAAAAAAA AAAAAAACTG GAGGGGGGGC CCGGTACCCA AATCGCCGGA TATGATCGTA 900

AACAATC 907

(2) INFORMATION FOR SEQ ID NO: 196:

(i) SEQUENCE CHARACTERISTICS:
(A) LENGTH: 1290 base pairs
(B) TYPE: nucleic acid
(C) STRANDEDNESS: double
(D) TOPOLOGY: linear

(xi) SEQUENCE DESCRIPTION: SEQ ID NO: 196:

GGCACGAGGA GGGACAGGGA GTGGGCAAGG GGAAGAAGCA GCTTATTTGA CTAACCAGCC 60

CCTCTGTGGT CCACCAGCGT CTTGGCTTGG TGGGAGGGCT CTCAATCAGC AGGGCCCCAG 120

KAGGGCAAGA AGAAGTGGGG CAAAGCCTGG CGCTCGGCCG CGGTCGCGGC AGCTTTGCMA 180

TCTGGAGCCA CGCCTCCTCC AGGCCATGCT CCTTGAACTT GGAAATGTCA ACCGGAGCCC 240

TTAACACCAG CCCTCCAGCA TCTAATAGAC TTGAATCTAC TCTAAACGAA TATTTAATCC 300

AACCTCAACT ACATTGTAGC TCAGTCCAAC GACTAACCCT GAAATGGGGG TGTTCCAGCC 360

TTCAGCGAGA TGGCCAAGCG GTCCCCTGGG GGCTGTGGCA GCGGGCTTAT CCTTCTCTGT 420

TGCCAACCTT GCCGTCCGAC CTCCTCCGCC CCCATGCGGT GACCCCGTCC GTGTCTGTGT 480

CTGTCCATAC GTGTGAGTCC AGCTAAAAAG ACAAAACAGA ACCCGTGGGC CCAGCTCGGA 540

AGGTGCGTGG AGAAGGCTCC GACGTCTCCG AAGTGCAGCC CTTGGGATGG CATTCCGTTG 600

TGTGCCTTAT TCCTGGAGAA TCTGTATACG GCTCGCCTAT AAGAAATATA GCCTCTTCAT 660

GCTGTATTAA AAGGACTTTT AAAAGCAAAA AAAAAAAAAA AAAAACTCGA GGGGGGGCCC 720

GGTACCCAAT TCGCCCAATA GTGAGTCGTA TTACAATTCA CTGGGCCGTC STTTTAACAA 780

CGTCGTGAAC TGGGAAAACC CTGGCGTTTA CCCAACTTAA TCGCCTTGCA GCACATCCCC 840

CTTTCGCCAG CTGGCGTTAA TAGCGAAAAA NGCCCGCACC CGAATCGCCC TTCCCAACAG 900

TTTGCGCAGC CCTGAATGGC GAAATGGCAA ATTGTAAGCG TTTAATATTT TKKTTAAAAT 960

TCCNCGTTWA AWTTTTTGTT TAAATCARCT CAATTTTTTT AACCCAATAA GSCCGAAATC 1020

CGGCAAATCC CCYTTATTAA TTCCAAAAAA ATAAACCSAA AAWGGGTTTG AATTTTTTKT 1080

TTCCCCAYTT TTGGAAACAA AWTYCCCCCT TTTTAAAAAA GTTGGAACCC CCAMCCYTCC 1140

AAAGGGGAAA AAACSYTTTT YTGGGGGGNA ANGGGGCCCC CNTACTTTNA ACAYCCCCCC 1200

CCAAWCAATT TTTTTGGGGG GTCCCNAAAG GTCCCCCTAA AANCTTTTTT CGGAACCCNA 1260

AGGGGANCCC CCCATTTAAA ATTTTNGGTN 1290

(2) INFORMATION FOR SEQ ID NO: 197:

(i) SEQUENCE CHARACTERISTICS:
(A) LENGTH: 1020 base pairs
(B) TYPE: nucleic acid
(C) STRANDEDNESS: double
(D) TOPOLOGY: linear

(xi) SEQUENCE DESCRIPTION: SEQ ID NO: 197:

GGTGTGCCTG GATGGTCGTG TAGGTGAGTT TTACCAAGGA TTATGGTAAC AAATGAGTGA 60

GACCTCTATG GAGAAAATAT TGAAGNNCAT TAAAGAAGAC CTCATANTAG GAGAGAATGT 120

SCTTTGGAGG ATTTGTATTG AGCTTTTACA GTATTCATTT TTCAACTCAA GGCAATGGCT 180

TTCTACACCA ACTCTAATCC ATAAACGGGT CTTATGACAT CTATGAAGTA GTAGCAAGAC 240

ATGCTTAGTG TGTATTTCTC TCTTTGAGAC ACTGTAATTT CTACCAGAAA TTTCCAGAGC 300

ATTATGTAGG TAGAAAAAAA TGCAAGCAAG CTGTTAAAGA TCTTGGATCC CATTATATAG 360

TATGTATAGC TGAAATCTGT AATTCAATCA CTTTTTCTCT TTTATCCTCT AACCAAAAAA 420

TTGTTTAATT TTGCATCCCA AATGTTTTTA ATCTTTGTAT ATTTTTTAAA AAYCCTTTTC 480

TCCTCATCAT TGCCTTTTTT GTGGTTGTAA ATAGACTTAC TTGCACTTTG AAGATGAGTT 540

ACTCCTTGTC ATCTTACAAA TATGTGATAT GGTAATTTTC ATAACAGATG TCAGTTTTGA 600

ACCAAGAATT GGTGATTTGT TTATAAGAAA AAAACTGGCT TCATTTCTGT GAAATTGCTC 660

TTTGAAAATT TCTTTTTACA CGTGTAAGCC AACTGAGATA CCGTGATGGT GTTGATTTCT 720

TTCAATGATG CTTACCATCT ATTTTAGCCA CTGAGCCTTT TATTATTTGT CTATTTGTAA 780

AGTTTATTTG TCTTAACTCA TTTAATAAAT ATACTGTTTA TCTGTTTCTG AATGGGGACT 840

GAACTTTTTG GATATTGATA TTGATTTGAA AATATTTTGG AATTTTTTCT ACTTGAAATT 900

TTAGAAATCT AATKGAAAAT TCTATAATGT ACTGAAAGTA WGGTTGTGTA CAGTGAKCAC 960

TCTCTAATAA TATGATGNCT TGCCCTAAAN GAGGNGGGAC ATGTCCCACT TTCCACCACG 1020

(2) INFORMATION FOR SEQ ID NO: 198:

(i) SEQUENCE CHARACTERISTICS:
(A) LENGTH: 524 base pairs
(B) TYPE: nucleic acid
(C) STRANDEDNESS: double
(D) TOPOLOGY: linear

(xi) SEQUENCE DESCRIPTION: SEQ ID NO: 198:

AATTCCCGAA GCTGAGGGTT GTGTGCCNTC GGGCGAGCCA AGTCTTTTGA CCGGACCCTT 60

CCCGGCGCAG AAGANCTGAA GTTGATTTGA GAGCCTGTKT TTGGGGTTRA GCCGAGCTGC 120

TGCGGGCTTY GTCGCCGGCC AGGACACAAG YTACTTGCAA CGGGGCGGCG CCTGGCTTAT 180

GATGTTCCTC AACCCAGGGG CGGCCTCTGC CCTCTACTCG TGCCAGGCCC ACTTGCCAGG 240

CAGGAGCCCT CCCCAAGCCT TCAGGGCTGC TCGGAGTCAC CTGTTGGAAT GGACTAAAAG 300

GACCCTTGTG TGGGAACAGG TGCTCCAAAC ACCCTGCTGC TGGCTGCCAG GCAGGCCCTC 360

TGGAAGGGAA GGGGCAGGAC TCATCAGGAC CTCCCTGGAC CCTGCAGGGC AGGCAGTTGG 420

CCCGAGCCCA AGCATTTGGC TCTGCTTGCC CCAAGGGGAC AGGAAGCCTC TTGGGCCTCT 480

TCCCTTCCTG GACAAGGCCC CCTGCCTTTG CCTCACATAA ACTG 524

(2) INFORMATION FOR SEQ ID NO: 199:

(i) SEQUENCE CHARACTERISTICS:
(A) LENGTH: 332 base pairs
(B) TYPE: nucleic acid
(C) STRANDEDNESS: double
(D) TOPOLOGY: linear

(xi) SEQUENCE DESCRIPTION: SEQ ID NO: 199:

GTGATACAAG GAAGGGTGAT CATCATCTGT CACCATGCAA TTCCTGCTCA CAGCCTTTCT 60

GTTGGTGCCA CTTCTGGCTC TTTGTGATGT CCCCATATCC CTAGGCTTCT CCCCCTCCTA 120

GAAGGGCTTC TTGATAGATT AGAAAATAAG AATGAGTGAC ATTTCCTATG TGCATATAAG 180

AAGGAGCCAC AAGACATGTC TTTTAAATAA AAGGACAGTG TCCATCCTTT TAGCTGCCGA 240

ATAGAACCTT GGTCTCATCC TCCTGGAGCT AGGSCTTAAA ACAGCTTCTG TGTTTCTSAT 300

TKGTCTCART GTTTTGCCAA GGTTTTATTC GG 332

(2) INFORMATION FOR SEQ ID NO: 200:

(i) SEQUENCE CHARACTERISTICS:
(A) LENGTH: 376 base pairs
(B) TYPE: nucleic acid
(C) STRANDEDNESS: double
(D) TOPOLOGY: linear

(xi) SEQUENCE DESCRIPTION: SEQ ID NO: 200:

CCAGGGAAGC CCCARGCCTG TCCTGAATTG ACATCAGTGC TTCCCTGAAC TGCCTCCCCC 60

ACCCCTGGGC ATTATCCCAG GAAACTTATG TTTTCTAGAA GCTAAGCAGC TGCTGGGACT 120

CAGGGACTGG TGCAGGTAGG CTGAGTGGCA GCTCAGTCCT AGAAGGTCTC TGAAGATCTG 180

GACTGAGGAC CYTGCTACTC CCCAAGCCAG AGCCCATCAG CCAGGCCTGC TGTGAGCCAC 240

CTGCCTGTGG AGTGCTGAGC TCAACCAAAG GCTGGCAAGC TCTGGGCCTC ATTTAAGGGA 300

TTCTGATGAG CCGATGGGCC CTGGAGGCAG CCCATTAAAG CATCTGGCTC GTTTTTGGAA 360

AAAAAAAAAA AAAAAG 376

(2) INFORMATION FOR SEQ ID NO: 201:

(i) SEQUENCE CHARACTERISTICS:
(A) LENGTH: 1192 base pairs
(B) TYPE: nucleic acid
(C) STRANDEDNESS: double
(D) TOPOLOGY: linear

(xi) SEQUENCE DESCRIPTION: SEQ ID NO: 201:

CCCAGTATAT TTCTATAACA TTTATTTTAG TGAACTTATA ATGTTTCTTT GTATTAAATT 60

ATTAGATTAT ATCTTTAGAT AATATTGTTA CTNAATTAGT AGGTAATATA TATTTTATTC 120

AAAAATAAAT TGTGCATCTA ATGTCTACCA ATTAATGTAC TTGTAGATGT ATCTTATCTT 180

AACTTGAGTC TTTGCTGCCC CTAATGAGGT GTGAAGGACT CTTCTCCCCT GGGGAAGTTT 240

TTCTTTTTCA GGAGGGAGGA GGGCTTTCCC AGGTAATGTG TCTAGAGTGT TGGGCAGAAR 300

AATCTGGGAC CACACCACAC CAGTTCTCTC CTTAATCCAC GTCATTTGCC TTCTATCCCA 360

GCTATGTTTC CAGTGTCCTC TGGGTGTTTC CAAGAGCAAC AAGAAAYGAA TAAATCTCTG 420

KTGAGTTGTT TATTTGTTCT TCACTTTGTT TTACACTGTA WTTTCTGAGT TTATGGGTGT 480

CTGTGAATTA AAAAGGAAAA GTRGAAATAA GTAAAACTCA GGTTGAAGGA AATATACATA 540

AATAAGATAA AGCTGACCTG TAGATATARR CAGGTTATAA RAGCTTAGAG TTGTCTAAGT 600

TGRGTGCAAA KTTTCCTCTG ATCTTTCTGA TGCCGARACA AAAAAGGCAG TCATGTTTGT 660

WATGTGATTG GAATGGAACC CGARAAGAGA GCAYGCTGTG TTCTTGGGGA CAGGAAAGCT 720

TGYGTGCACC AAGTCTKAAC CACCACCTTC ATGGGACATA GRTTATGTGC TGGAACATAT 780

TTCACACCGG CCTGGCAGTA AACACTTGTA GTGTTGTGCA GTGGAAACGG TCATCTTCCG 840

CTAAAGCACG GCGTGTTGTG CAGCGGAAAT GGTCATCTGC TGCTAAAACA CAGCTTCCAT 900

CGTAATGTAT GCTCCTTACT CAAAGAGTGT GGTCCCAAAC AGCCTTTGGG AGGTCCTCCT 960

TGATTCATGG ATGAAACCTG GAACATCTTG AGGACTGAGT TAACCATAGG TCCTTAAATA 1020

ACTCTCCACA CGTTTTTCTT AGTTTATCTC TACATGCAGG GTGTGCAGCA GCCTGTTCAA 1080

AGTCATATTT TCTGGGAAAT ATTTCCAGTG TTTATTTGCA CTTTAGCCCA CTCTGTGTAG 1140

CCTTATTTCT TCTAAACTCA CCATTAATCT GAATAATAGT CAAATTTAGG GG 1192

(2) INFORMATION FOR SEQ ID NO: 202:

(i) SEQUENCE CHARACTERISTICS:
(A) LENGTH: 589 base pairs
(B) TYPE: nucleic acid
(C) STRANDEDNESS: double
(D) TOPOLOGY: linear

(xi) SEQUENCE DESCRIPTION: SEQ ID NO: 202:

ATCTTGGGCT ATCTTTGACA GGGGATTCTT GCAAGTTGAT GCTTTCTACA AGTGAATATA 60

GTCAGTCCCC AAAGATGGAG AGCTTGAGTT CTCACAGAAT TGATGAAGAT GGAGAAAACA 120

CACAGATTGA GGATACGGAA CCCATGTCTC CAGTTCTCAA TTCTAAATTT GTTCCTGCTG 180

AAAATGATAG TATCCTGATG AATCCAGCAC AGGATGGTGA AGTACAACTG AGTCAGAATG 240

ATGACAAAAC AAAGGGAGAT GATACAGACA CCMGGGATGA CATTAGTATT TTAGCCACTG 300

GTTGCAAGGG CAGAGAAGAA ACGGTAGCAG AAGATGTTTG TATTGATCTC ACTTGTGATT 360

CGGGGAGTCA GGCAGTTCCG TCACCAGCTA CTCGATCTGA GGCACTTTCT AGTGTGTTAG 420

ATCAGGAGGA AGCTATGGAA ATTAAAGAAC ACCATCCAGA GGAGGGGTCT TCAGGGTCTG 480

AGGTGGAAGA AATCCCTGAG ACACCTTGTG AAAGTCAAGG AGAGGAACTC AAAGAAGAAA 540

ATATGGAGAG TGTTCCGTTG CACCTTTCTC TGACTGAAAC TCAGTCCCA 589

(2) INFORMATION FOR SEQ ID NO: 203:

(i) SEQUENCE CHARACTERISTICS:
(A) LENGTH: 847 base pairs
(B) TYPE: nucleic acid

(C) STRANDEDNESS: double
(D) TOPOLOGY: linear

(xi) SEQUENCE DESCRIPTION: SEQ ID NO: 203:

GGCACGAGCG CAAGCTGCTG GCCGCCATCA ACGCGTTCCG CCAGGTGCGG CTGAAACACC 60

GGAAGCTCCG GGAACAAGTG AACTCCATGG TGGACATCTC CAAGATGCAC ATGATCCTGT 120

ATGACCTGCA GCAGAATCTG AGCAGCTCAC ACCGGGCCCT GGAGAAACAG ATTGACACGC 180

TGGCGGGGAA GCTGGATGCC CTGACTGAGC TGCTTAGCAC TGCCCTGGGG CCGAGCAGCT 240

TCCAGAACCC AGCCAGCAGT CCAAGTAGCT GGACCCACGA GGAGGAACCA GGCTACTTTC 300

CCCAGTACTG AGTGGTGGAC ATCGTCTCTG CCACTCCTGA CCAGCCTGAA CAAAGCACCT 360

CAAGTGCAAG GACCAAAGGG GGCCTGGCTT GGATGGGTTG GCTTGCTGAT GGCTGCTGGA 420

GGGGACGCTG GCTAAAGTGG GGAGGCCTTG GCCCACCTGA GGCCCCAGGT GGGAACATGG 480

TCACCCCCAC TCTGCATACC CTCATCAAAA ACACTCTCAC TATGCTGCTA TGGACGACCT 540

CCAGCTCTCA GTTACAAGTG CAGGCGACTG GAGGCAGGAC TCTTGGGTCC CTGGGAAAGA 600

GGGTACTAGG GGCCCGGATC CAGGATTCTG GGAGGCTTCA GTTACCGCTG GCCGAGCTGA 660

AGAACTGGGT ATGAGGCTGG GGCGGGGCTG GAGGTGGCGC CCCCTGGTGG GACAACAAAG 720

AGGACACCAT TTTTCCAGAG CTGCAGAGAG CACCTGGTGG GGAGGAAGAA GTGTAACTCA 780

CCAGCCTCTG CTCTTATCTT TGTAATAAAT GTTAAAGCCA GAAAAAAAAA AAAAAAAAAA 840

AAAAAAA 847

(2) INFORMATION FOR SEQ ID NO: 204:

(i) SEQUENCE CHARACTERISTICS:
(A) LENGTH: 852 base pairs
(B) TYPE: nucleic acid
(C) STRANDEDNESS: double
(D) TOPOLOGY: linear

(xi) SEQUENCE DESCRIPTION: SEQ ID NO: 204:

ACAAACATAC TCGCAGGAAG GAGTCTCATG CTGCCCGCAG CATCAGCGCA ACNCNTGGCC 60

GCCATCAACG CGTTCCGCCA GGTGCGGCTG AAACACCGGA AGCTCCGGGA ACAAGTGAAC 120

TCCATGGTGG ACATCTCCAA GATGCACATG ATCCTGTATG ACCTGCAGCA GAATCTGAGC 180

AGCTCACACC GGGCCCTGGA GAAACAGATT GACACGCTGG CGGGGAAGCT GGATGCCCTG 240

ACTGAGCTGC TTAGCACTGC CCTGGGGCCG AGGCAGCTTC CAGAACCCAG CCAGCAGTCC 300

AAGTAGCTGG ACCCACGNAG GAGGAACCAG GCTACTTTCC CCAGTACTGA GGTGGTGGAC 360

ATNCGTCTCT TGCCACTCCN TGNACCCAGC CCTGAACAAA GCACCTCAAG TGCAAGGACC 420

AAAGGGGGCC CTGGCTTGGA GTGGGTTGGC TTGCTGATGG CTGCTGGAGG GGACGCTGGC 480

TAAAGTGGGK AGGCCTTGGC CCACCTGAGG CCCCAGGTGG GAACATGGTC ACCCCCACTC 540

TGCATACCCT CATCAAAAAC ACTCTCACTA TGCTGCTATG GACGACCTCC AGCTCTCAGT 600

TACAAGTGCA GGCGACTGGA GGCAGGACTC CTGGGTCCCT GGGAAAGAGG GTACTAGGGG 660

CCCGGATCCA GGATTCTGGG AGGCTTCAGT TACCGCTGGC CGAGCTGAAG AACTGGGTAT 720

GAGGCTGGGG CGGGGCYGGA GGTGGCGCCC CCTGGTGGGA CAACAAAGAG GACACCATTT 780

TTCCAGAGCT GCAGAGAGCA CCTGGTGGGG AGGAAGAAGT GTAACTCACC AGCCTCTGCT 840

CTTATCTTTG TA 852

(2) INFORMATION FOR SEQ ID NO: 205:

(i) SEQUENCE CHARACTERISTICS:
(A) LENGTH: 1354 base pairs
(B) TYPE: nucleic acid
(C) STRANDEDNESS: double
(D) TOPOLOGY: linear

(xi) SEQUENCE DESCRIPTION: SEQ ID NO: 205:

GATTCGGCAC GAGGCTTGCT GGAGCAGGAG AAGTCTCTRG CCGGCTGGGC ACTGGTGCTG 60

GCASGARCTG GCATTGGACT CATGGTGCTG CATGCAGAGA TGCTGTGGTT CGGGGGGTGC 120

TCGGCTGTCA ATGCCACTGG GCACCTTTCA GACACACTTT GGCTGATCCC CATCACATTC 180

CTGACCATCG GCTATGGTGA CGTGGTGCCG GGCACCATGT GGGGCAAGAT CGTYTGCCTG 240

TGCACTGGAG TCATGGGTGT CTGCTGCACA GCCCTGCTGG TGGCCGTGGT GGCCCGGAAG 300

CTGGAGTTTA ACAAGGCAGA GAAGCACGTG CACAACTTCA TGATGGATAT CCAGTATACC 360

AAAGAGATGA AGGAGTCCGC TGCCCGAGTG CTACAAGAAG CCTGGATGTT CTACAAACAT 420

ACTCGCAGGA AGGAGTCTCA TGCTGCCCGC AGGCATCAGC GCAANCTGCT GGCCGCCATC 480

AACGCGTTCC GCCAGGTGCG GCTGAAACAC CGGAAGCTCC GGGAACAAGT GAACTCCATG 540

GTGGACATCT CCAAGATGCA CATGATCCTG TATGACCTGC AGCAGAATCT GAGCAGCTCA 600

CACCGGGCCC TGGAGAAACA GATTGACACG CTGGCGGGGA AGCTGGATGC CCTGACTGAG 660

CTGCTTAGCA CTGCCCTGGG GCCGAGGCAG CTTCCAGAAC CCAGCCAGCA GTCCAAGTAG 720

CTGGACCCAC GAGGAGGAAC CAGGCTACTT TCCCCAGTAC TGAGGTGGTG GACATCGTCT 780

CTGCCACTCC TGANCCCAGC CCTGAACAAA GCACCTCAAG TGCAAGGACC AAAGGGGGCC 840

CTGGCTTGGA GTGGGTTGGC TTGCTGATGG CTGCTGGAGG GGACGCTGGC TAAAGTGGGK 900

AGGCCTTGGC CCACCTGAGG CCCCAGGTGG GAACATGGTC ACCCCCACTC TGCATACCCT 960

CATCAAAAAC ACTCTCACTA TGCTGCTATG GACGACCTCC AGCTCTCAGT TACAAGTGCA 1020

GGCGACTGGA GGCAGGACTC YTGGGTCCCT GGGAAAGAGG GYACTAGGGG CCCGGATCCA 1080

GGATTCTGGG AGGCTTCAGT TACCGCTGGC CGAGCTGAAG AACTGGGTAT GAGGCTGGGG 1140

CGGGGCTGGA GGTGGCGCCC CCTGGTGGGA CAACAAAGAG GACACCATTT TTCCAGAGCT 1200

GCAGAGAGCA CCTGGTGGGG AGGAAGAAGT GTAACTCACC AGCCTCTGCT CTTATCTTTG 1260

TAATAAATGT TAAAGCCAGA AAAAAATAAA AAAAAAAAAA AAAAAACTCG AGGGGGGCCC 1320

AGACCCAATC TCCCTATAGT AAGNCGCCNN ANAN 1354

(2) INFORMATION FOR SEQ ID NO: 206:

(i) SEQUENCE CHARACTERISTICS:
(A) LENGTH: 1378 base pairs
(B) TYPE: nucleic acid
(C) STRANDEDNESS: double
(D) TOPOLOGY: linear

(xi) SEQUENCE DESCRIPTION: SEQ ID NO: 206:

TCCCCAGGTG CACAGCCAGG GCCCTCCTGT CTGCAGGAGA ATTCACAGCT GGTGTGGGAC 60

TCAGCCCCTA GNCCATTCAA AGCCTTAATG TTGTAATCAT ATCTTACGTG TTGAAGACCT 120

GACTGGAGAA ACAAAATGTG CAATAACGYG AATTTTATCT TAGAGATCTG TGCAGCCTAT 180

TTCTGTCACA AAAGTTATAT TGTCTAATAA GAGAAGTCTT AATGGCCTCT GTGAATAATG 240

TAACTCCAGT TACACGGTGA CTTTTAATAG CATACAGTGA TTTGATGAAA GGACGTCAAA 300

CAATGTGGCG ATGTCGTGGA AAGTTATCTT TCCCGCTCTT TGCTGTGGTC ATTGTGTCTT 360

GCAGAAAGGA TGGCCCTGAT GCAGCAGCAG CGCCAGCTGT ANATAAAAAA TAATTCACAC 420

TATCAGACTA GCAAGGCACT AGAACTGGAA AAGACCACAG AAAACAAAGA ATCCAACCCT 480

TTCATCTTAC AGGTGAACAA ACTGTGATGA TGCACATGTA TGTGTTTTGT AAGCTGTGAG 540

CACCGTAACA AAATGTAAAT TTGCCATTAT TAGGAAGTGC TGGTGGCAGT GAAGAAGCAC 600

CCAGGCCACT TGACTCCCAG TCTGGTGCCC TGTCTACACC AGACAACACA GGAGCTGGGT 660

CAGATTCCCC TCAGCTGCTT AACAAAGTTC CTCGAACAGA AAGTGCTTAC AAAGCTGCCT 720

TCTCGGATAC TGAAAGGTCG AGTTTTCTGA ACTGCACTGA TTTTATTGCA GTTGAAAAAA 780

AAAAAAAGCT ATTCCAAAGA TTTCAAGCTG TTCTGAGACA TCTTCTGATG GCTTTACTTC 840

CTGAGAGGCA ATGTTTTTAC TTTATGCATA ATTCATTGTT GCCAAGGAAT AAAGTGAAGA 900

AACAGCACCT TTTAATATAT AGGTCTCTCT GGAAGAGACC TAAATTAGAA AGAGAAAACT 960

GTGACAATTT TCATATTCTC ATTCTTAAAA AACACTAATC TTAACTAACA AAAGTTCTTT 1020

TGAGAATAAG TTACACACAA TGGCCACAGC AGTTTGTCTT TAATAGTATA GTGCCTATAC 1080

TCATGTAATC GGTTACTCAC TACTGCCTTT AAAAAAAAAA ACCAGCATAT TTATTGAAAA 1140

CATGAGACAG GATTATAGTG CCTTAACCGA TATATTTTGT GACTTAAAAA ATACATTTAA 1200

AACTGCTCTT CTGCTCTAGT ACCATGCTTA GTGCAAATGA TTATTTCTAT GTACAACTGA 1260

TGCTTGTTCT TATTTTAATA AATTTATCAG AGTGAAAAAA AAAAAAAAAA AAAAAAAAAA 1320

AAAAAAAAAA AAAAAAAAAA AAAAAAAAAA AAAAAAAAAA AAAAAAAGAA NAAAANAA 1378

(2) INFORMATION FOR SEQ ID NO: 207:

(i) SEQUENCE CHARACTERISTICS:
(A) LENGTH: 1166 base pairs
(B) TYPE: nucleic acid
(C) STRANDEDNESS: double
(D) TOPOLOGY: linear

(xi) SEQUENCE DESCRIPTION: SEQ ID NO: 207:

AANCCACTGC ANTTTAAACC CCCTCCCCTC CAAGAAAGTT CACAACCGGC CATGGATGAC 60

CCTCATTTTA GATGGGCCNC AATATTTAAG ATGGACTGRG GMCCCCARAG ACTGACCCTT 120

GAAAGGGGGA CTCAGAAGAA AGATCCTTGA CATTGCCMAA CATGCTGGGC TTGTCCAACA 180

CAGTGATGCG GCTCATCGAG AARCGGGCTT TCCMAGGACA AGTACTTTAT GATAGGTGGG 240

ATGCTGCTGA CCTGTGTGGT CATGTTCCTC GTGGTGCAGT ACCTGACATG AGCCAGCCAC 300

GCTCAGTGGC TGAACAGCAT TCCCACAGCC TGCAAGTGTG TGTGTGTGTG AAAGAGAGAG 360

GGGGCCCAGA GGCCGCCTTT TGAAATGTTT GCCTGTCTGA ACTGTGAAGA CACTTGGGAG 420

TGATTGTGGT CTAATTTCCA ACCTGCTCTG TTTTCTGTGA CATCTTGGAG GGGGAGCTAG 480

TGCCAMCACC ATGCGCGGTG CTTAGGAAAT GAAAGAAGTC CCGGGTCTGT CTCTCTCACT 540

CTCGCTCTCA MTGGGGGAGG GAAAGAATGG CTTTGGTGGC TTTGTTCACA CAGCTGATGC 600

GTGSCCTGGG AAGGTGTCCA CAGTGAGCCC TGTGTGCAGG ACTGTCCACN ACGGTTCACA 660

CCTTGTCACC ATCAGGCCTT TCTGGCTCCT GATAGGGTGG AGCAAAAGTG GAAAGGAAAG 720

GAAAGAGGCY TTTTCTCACA GCCATTATAT TAAATAGTAG GTCGATTCAC ATCYTCGTGC 780

TCCTGGCCAC CCTCCCCTGT GCCTCAGTGA CATGTAGATG ACTGACTGCC AATACTTGTC 840

ACCATTCCCT GGAAGCAGCT ACCTAGGGGA AACAAGATGT AGTGCTATTG CCGATAACAA 900

GTAAGATTTT CCACACTACA GCTGGGTGTT TCTCTTTTCT AAAGTGAGGC CAGTGTTATT 960

TCCCGGGAGT GTTCAGTCTT GACCCTAGTC ACTGATTTTT TCTAGTTGTT AATAGAGTGG 1020

TTGGGCTTTT AAGGTTCAGA GACTGTGGGC TTGGGCACCT GCGCCCAGGG STTTTGTGGG 1080

GGCCTTTGCC CCTTAGRAAA GTAGCTTTTA GGGGCAAAGA TTTGTTGATT TTCCCCATTA 1140

CAGTCTTCAG CTCNAGGGTT TTAAAA 1166

(2) INFORMATION FOR SEQ ID NO: 208:

(i) SEQUENCE CHARACTERISTICS:
(A) LENGTH: 697 base pairs
(B) TYPE: nucleic acid
(C) STRANDEDNESS: double
(D) TOPOLOGY: linear

(xi) SEQUENCE DESCRIPTION: SEQ ID NO: 208:

TACTTCTAGG ATTATAAGGA ATTAACATTG AGATGACATT TCCATTTGAG AAGGAAAATA 60

GTTGCTTTCA GTGCCTTTTA TTTGATTCCT GGAGAGAGCA GACTCGCACS AACATTCAAC 120

CCCAGCGCTG ATATGACAGT AATCCTCAGA GGCAGAGCCC AGCACAAAAC AGCAATGCTA 180

GAAAGTTACA ATTGGAAAGT TTCCTGCCAG CTTCGGGAAT GACACTGCAA AGCTGATGCC 240

AGAAACTGCC AGRGTAATTC TCCTCATTAC TGCTCTACCC ACCCACTTTC AGCTCCCCAA 300

ATTAACTAGT GCAGTTGACT AATTCTCTTT ACCTTTATCA TTTARGGTGA RGCATTGCAC 360

AAAAACTCTC GACTTTGCCA TATAAGGGCT GTGGTTCTCT GTGGTCCCCT GGATAAGAGG 420

CATCACCATT ATCTGGAAAC ATGCAGTAAA TGCAGATTNT TCATCTTCTC CCCAGACCTC 480

CTGAGTTAGA AATTCACAAG TTCTCCAGGT GATCTCATAC ATGCTAAAGT TTGAGAACCA 540

TTGAGTAAAG TTAATGCATT AAGAAGAGAT TAGATAGGGA TGGTGGCGTA TCTTCCTACA 600

GTTTCCCTGT TAACAAGAAA GTCAGAGGTC AGTTGATCAG ACATTAGATT ATTTATTGCT 660

AAAACTAAAA AAAATTAAAA AAAACTGGAG GGGGGCC 697

(2) INFORMATION FOR SEQ ID NO: 209:

(i) SEQUENCE CHARACTERISTICS:
(A) LENGTH: 932 base pairs
(B) TYPE: nucleic acid
(C) STRANDEDNESS: double
(D) TOPOLOGY: linear

(xi) SEQUENCE DESCRIPTION: SEQ ID NO: 209:

CGTGAGTCAC CTCTCTATAG TGGGCGTGGC CGAGGCCGGG GTGACCCTGC CGAAGCCTCC 60

GCTGCCAGAA ACCATGTTCA AGGTAATTAA AAGGTCCGTG GGGCCAGCCA GCCTGAGCTT 120

GCTCACCTTC AAAGTCTATG CAGCACCAAA AAAGGACTCA CCTCCCAAAA ATTCCGTGAA 180

GGTTGATGAG CTTTCACTCT ACTCAGTTCC TGAGGGTCAA TCGAAGTATG TGGAGGAGGC 240

AAGGAGCCAG CTTGAAGAAA GCATCTCACA GCTCCGACAC TATTGCGAGC CATACACAAC 300

CTGGTGTCAG GAAACGTACT CCCAAACTAA GCCCAAGATG CAAAGTTTGG TTCAATGGGG 360

GTTAGACAGC TATGACTATC TCCAAAATGC ACCTCCTGGA TTTTTTCCGA GACTTGGTGT 420

TATTGGTTTT GCTGGCCTTA TTGGACTCCT TTTGGCTAGA GGTTCAAAAA TAAAGAAGCT 480

AGTGTATCCG CCTGGTTTCA TGGGATTAGC TGCCTCCCTC TATTATCCAC AACAAGCCAT 540

CGTGTTTGCC CAGGTCAGTG GGGAGAGATT ATATGACTGG GGTTTACGAG GATATATAGT 600

CATAGAAGAT TTGTGGAAGG AGAACTTTCA AAAGCCAGGA AATGTGAAGA ATTCACCTGG 660

AACTAAGTAG AAAACTYCAT GYTCTGCCAT CTTAATCAGT TATRGGTAAA CATTGGAAAC 720

TCCATAGAAT AAATCAGTAT TTCTACAGAA AAATGGCATA GAAGTCAGTA TTGAATGTAT 780

TAAATTGGCT TTCTTCTTCA GGAAAAACTA GACCAGACCT CTGTTATCTT CTGTGAAATC 840

ATCCTACAAG CAAACTAACC TGGAATCCCT TCACCTAGAG ATAATGTACA AGCCTTAGAA 900

CTCCTCATTC TCATGTTGCT ATTTATGTAC CT 932

(2) INFORMATION FOR SEQ ID NO: 210:

(i) SEQUENCE CHARACTERISTICS:
(A) LENGTH: 661 base pairs
(B) TYPE: nucleic acid
(C) STRANDEDNESS: double
(D) TOPOLOGY: linear

(xi) SEQUENCE DESCRIPTION: SEQ ID NO: 210:

GTCATTCTTT AAATAAAAGC TTTCCTGTTT AAAGCTTTTC AAAGGAGCAG ACCACCTTGA 60

AGATTCCCCC TAGGGTTGAT ATGTGTCTAA TTCATTTTAT AAAAATTATT CTTGTCTTCA 120

TTTTAAAGCT TTGGCTATAT AGTCAGAAAT GTCCTAAATA ACAAACTATT TTGTATTTAA 180

TTTAGGGAAG ACTAAAGGGA AGAAAAATGA AAACTCAGTC TTTATGTAAG CTCCAAGGAT 240

ATTAGGGCTT AAAGGGCTTT TCTAGTTTTA TGAGAATTTG TACTACTGAT TTTTATATAT 300

TCCTGTTTTT GAGATGAACA GATCTCTGGG GAAATTGTTG AGTTACAATG GCATTTCACT 360

GTGATCCCTC TCAAGCTCAG ATCAGTTCTA TAACCCAATG ACAACCTGTC TCTTTGGTTT 420

ACTGTCCTGT GAAATGTCAG CTCAAGTTTC CCAGAAGTCG TGTGTTTATG ATGAGTCAGA 480

GTGCTTTTCC TCGGTGGGAC AGTTGCTGGC CCTCTTAATT TTGGTGTATG TGCTTCCAAG 540

TATCTAAACC TCCAGTCTGA TCTGTATATG CTATCCTAAC TGTTAATTGT ATTATTGATT 600

ATGTTGATTA TCTTGCTTGA AGGTTCATAC TTTTCAATTT GATAGAAATA AAGTTTTTTT 660

C 661

(2) INFORMATION FOR SEQ ID NO: 211:

(i) SEQUENCE CHARACTERISTICS:
(A) LENGTH: 592 base pairs
(B) TYPE: nucleic acid
(C) STRANDEDNESS: double
(D) TOPOLOGY: linear

(xi) SEQUENCE DESCRIPTION: SEQ ID NO: 211:

GAAACTGACA TTGTTAAACA CACTAAAACA GAAGTACTTA CCTCTTGAAG ATTTAATATA 60

TAATGGTTGA CATGATACAT GTACATGAAT GGAATGACCA GATGCTTATG GTCTACATTT 120

TCCTTTATCC TGTTAGTATT ACCTTCCTTA ATCTTTGTTC CTTAACATGC TAAATTCCTC 180

TTCAGTGTTT ATTTTCTAGT GACAGAATGC TAACATTTCT TACACCCTGG CAGAAGGGAG 240

AGAAATGTGT TTTGGGGTGG GTAACTAAAT TTTTGAGTGA AATATCATAA GATGAGAATG 300

GAAAGAGGGA GACACAAAGA GTTATAACAA AAAAACAATG GTTTTTTTAG CCATTTGACT 360

GGCTCTTTAA ATAGTCTACA AGACATTCAC GTTNAACATC ACTTTTAGTG AAATAAAATG 420

TGCCATACTA GTATGTGCTT CAAAAGGGCA AATGTGCTTT AGTGCCCTAA GGCTAAATTT 480

TGGTCATTTG ACATCAGAGA TGTTGTAAGT ATTGCACTTA ATACGCACCT ATTTCTCAAT 540

AGTGNTATTT TTTTGGCTAG CATTTNCTTT ACCACTAACC TTGTTGGATA GC 592

(2) INFORMATION FOR SEQ ID NO: 212:

(i) SEQUENCE CHARACTERISTICS:
(A) LENGTH: 938 base pairs
(B) TYPE: nucleic acid
(C) STRANDEDNESS: double
(D) TOPOLOGY: linear

(xi) SEQUENCE DESCRIPTION: SEQ ID NO: 212:

TGGAGTGGCT TTCCAGCTGA ATGAATCCTA TGTCTCGCGT GCAGGTGGTT GGTTTTCAAT 60

GTTCTTSCTA ATTTTTTTCC TATTGGCTCT TGGGAGTTTN CTTTGTTTGC TCCTGTGTTT 120

GCCCAGCTTT AATAAAACCA GGCGCAAACA AAAACCATAG CATTCTGAAA CAATAGGGGG 180

CCCACATTGG ACCCAGTATG TCACTTTAAT GGACTTCAAG AAAAAATCTG AATGGGAAAA 240

TGACACTAGG AATGTATACT CCACACATTT TATGCCATAT AATGGTGTGT TTTCTTAATT 300

TTGTTTCTTG TGGCGAAATG TGGCTTTCAA ATTAAAATGM CCTTTTCTTC TTKGAAACTT 360

TTTGTTTKGA CTKGTATAAT TAAGGGTTTG GAAAGATTCA TAATTMTGAG AGAGGTTTGC 420

AACCAGGAGA TACAAAGAAG TCTCAGTAGT AATCTTGTTC ATGTGCTTTT ACAGCCAGCT 480

ACATTTAAGR ATGTATTAGT TACAGAAATT ATATGTCTGT GTATGTGTCT CTACTCAATA 540

AAGTACATGC CTCCACATAA TGCGGTGCTG TCCATCTCGG CAAATACTGG CCAAGTCCCT 600

TTATGACAGG CACACAGAAA CCATAGCATG GTCTGGCTTT CAGAAAATGC CTCTCATCTT 660

TCCTGGAACC TTATTTTGCT AAATGTCTGT TTTCTTGTGA TTTGTTGTAC CTCACAGCAC 720

CATTGTGACC ATGGTGATGC CTCATTTGCA TGATATGTAC CTTGTGTTTA ATGTGAAATA 780

CATTTTCATT GAAGAGTCTG ATGACTTGCT AGCGTTTTAT TTTTTCTGTA AGCTCAATGT 840

GCTGAAACCA AACCAGGCTT TTAAAAACCT GTGTAGAAGA AAACCAAAAA ATCCTGTGTG 900

GGTGTCCTTT CCCTGTCAAA CTCATTAAAA ATTCCTTT 938

(2) INFORMATION FOR SEQ ID NO: 213:

(i) SEQUENCE CHARACTERISTICS:
(A) LENGTH: 1079 base pairs
(B) TYPE: nucleic acid
(C) STRANDEDNESS: double
(D) TOPOLOGY: linear

(xi) SEQUENCE DESCRIPTION: SEQ ID NO: 213:

AGCCTGCCGG GAGAGTGGTG GCATCTRARA GGCTGGTCGT GGACTGTGGT TGGGGGAGGT 60

GGGAGCTGTT TTAACCGTGT GCCCCCTCTC CTGTGCCKGC GTGGGCATCC CCCGGGGCAG 120

TGGAACGCGG GCGCTCCTCC AGCTTCCGAG TCCAGCCAGC CTGGGCGCGG GGCGCGCCCC 180

CGAGACACCC GAGGAGTCCG TTCCTCCCTG GTTACGTGGA CTGTGGAGCT GGTCTCTTGT 240

GGCTCAGCGC CGTGCGGAGG TTGAAGCGTA CCTGCGGAGG TCGCACCAGG GGCGTGAGGA 300

GGAGGAGGAA GGGCATGAGC CGAGCTTGAG GAATCCGTGY TCCAAACTCT ACACTCAAGG 360

RTGCMCTGCG CAACTCTGGT GGCGATGGGC TGGGGCAGAT GTCCTTGGAG TTCTACCAGA 420

AGAAGAAGTC TCGCTGGCCA TTCTCAGACG AGTGCATCCC ATGGGAAGTG TGGACGGTCA 480

AGGTGCATGT GGTAGCCCTG GCCACGGAGC AGGAGCGGCA GATCTGCCGG GAGAAGGTGG 540

GTGAGAAACT CTGCGAGAAG ATCATCAACA TCGTGGAGGT GATGAATCGG CATGAGTACT 600

TGCCCAAGAT GCCCACACAG TCGGAGGTGG ATAACGTGTT TGACACAGGC TTGCGGGACG 660

TGCAGCCCTA CCTGTACAAG ATCTCCTTCC AGATCACTGA TGCCCTGGGC ACCTCAGTCA 720

CCACCACCAT GCGCAGGCTC ATCAAAGACA CCCTTGCCCT CTGAGCGTCG CTGGATCTCT 780

GGGAGCTCCT TGATGGCTCC CAGACCTTGG CTTTTGGGAA TTGCACTTTT GGGCCTTTGG 840

GCTCTGGAAC CTGCTCTGGG TCATTGGTGA GACTTGGAAG GGGCAGCCCC CGCTGGCTTC 900

TTGGTTTTGT GGTTGCCAGC CTCAGGTCAT CCTTTTAATC TTTGCTGACG GTTCAGTCCT 960

GCCTCTACTG TCTCTCCATA GCCCTGGTGG GGTCCCCCTT CTTTCTCCAC TGTACAGAAG 1020

AGCCACCACT GGGATGGGGA ATAAAGTTGA GAACATGAGT TTGGGCTGAA AAAAAAAAA 1079

(2) INFORMATION FOR SEQ ID NO: 214:

(i) SEQUENCE CHARACTERISTICS:
(A) LENGTH: 3791 base pairs
(B) TYPE: nucleic acid
(C) STRANDEDNESS: double
(D) TOPOLOGY: linear

(xi) SEQUENCE DESCRIPTION: SEQ ID NO: 214:

TGAAGCAGGC GCTCTTGGCT CGGCGCGGCC CGCTGCAATC CGTGGAGGAA CGCGCCGCCG 60

AGCCACCATC ATGCCTGGGC ACTTACAGGA AGGCTTCGGC TGCGTGGTCA CCAACCGATT 120

CGACCAGTTA TTTGACGACG AATCGGACCC CTTCGAGGTG CTGAAGGCAG CAGAGAACAA 180

GAAAAAAGAA GCCGGCGGGG GCGGCGTTGG GGGCCCTGGG GCCAAGAGCG CATCAGGGCC 240

GCGGCCCAGA CCAACTCCAA CGCGGCAGGC AAACAGCTGC GCAAGGAGTC CCAGAAAGAC 300

CGCAAGAACC CGCTGCCCCC CAGCGTTGGC GTGGTTGACA AGAAAGAGGA GACGCAGCCG 360

CCCGTGGCGC TTTAAGAAAG AAGGAATAAG ACGAGTTGGA AGAAGACCTG ATCAACAACT 420

TCAGGGTGAA GGGAAAATAA TTGATAGAAG ACCAGAAAGG CGACCACCTC GTGAACGAAG 480

ATTCGAAAAG CCACTTGAAG AAAAGGGTGA AGGAGGCGAA TTTTCAGTTG ATAGACCGAT 540

TATTGACCGA CCTATTCGAG GTCGTGGTGG TCTTGGAAGA GGTCGAGGGG GCCGTGGACG 600

TGGAATGGGC CGAGGAGATG GATTTGATTC TCGTGGCAAA CGTGAATTTG ATAGGCATAG 660

TGGAAGTGAT AGATCTTCTT TTTCACATTA CAGTGGCCTG AAGCACGAGG ACAAACGTGG 720

AGGTAGCGGA TCTCACAACT GGGGAACTGT CAAAGACGAA TTAACTGACT TGGATCAATC 780

AAATGTGACT GAGGAAACAC CTGAAGGTGA AGAACATCAT CCAGTGGCAG ACACTGAAAA 840

TAAGGAGAAT GAAGTTGAAG AGGTAAAAGA GGAGGGTCCA AAAGAGATGA CTTTGGATGA 900

GTGGAAGGCT ATTCAAAATA AGGACCGGGC AAAAGTAGAA TTTAATATCC GAAAACCAAA 960

TGAAGGTGCT GATGGGCAGT GGAAGAAGGG ATTTGTTCTT CATAAATCAA AGAGTGAAGA 1020

GGCTCATGCT GAAGATTCGG TTATGGACCA TCATTTCCGG AAGCCAGCAA ATGATATAAC 1080

GTCTCAGCTG GAGATCAATT TTGGAGACCT TGGCCGCCCA GGACGTGGCG GCAGGGGAGG 1140

ACGAGGTGGA CGTGGGCGTG GTGGGCGCCC AAACCGTGGC AGCAGGACCG ACAAGTCAAG 1200

TGCTTCTGCT CCTGATGTGG ATGACCCAGA GGCATTCCCA GCTCTGGCTT AACTGGATGC 1260

CATAAGACAA CCCTGGTTCC TTTGTGAACC CTTCTGTTCA AAGCTTTTGC ATGCTTAAGG 1320

ATTCCAAACG ACTAAGAAAT TAAAAAAAAA AAGACTGTCA TTCATACCAT TCACACCTAA 1380

AGACTGAATT TTATCTGTTT TAAAAATGAA CTTCTCCCGC TACACAGAAG TAACAAATAT 1440

GGTAGTCAGT TTTGTATTTA GAAATGTATT GGTAGCAGGG ATGTTTTCAT AATTTTCAGA 1500

GATTATGCAT TCTTCATGAA TACTTTTGTA TTGCTGCTTG CAAATATGCA TTTCCAAACT 1560

TGAAATATAG GTGTGAACAG TGTGTACCAG TTTAAAGCTT TCACTTCATT TGTGTTTTTT 1620

AATTAAGGAT TTAGAAGTTC CCCCAATTAC AAACTGGTTT TAAATATTGG ACATACTGGT 1680

TTTAATACCT GCTTTGCATA TTCACACATG GTCAACTGGG ACATGTTAAA CTTTGATTTG 1740

TCAAATTTTA TGCTGTGTGG AATACTAACT ATATGTATTT TAACTTAGTT TTAATATTTT 1800

CATTTTTGGG GAAAAATCTT TTTTCACTTC TCATGATAGC TGTTATATAT ATATGCTAAA 1860

TCTTTATATA CAGAAATATC AGTACTTGAA CAAATTCAAA GCACATTTGG TTTATTAACC 1920

CTTGCTCCTT GCATGGCTCA TTAGGTTCAA ATTATAACTG ATTTACATTT TCAGCTATAT 1980

TTACTTTTTA AATGCTTGAG TTTCCCATTT TAAAATCTAA ACTAGACATC TTAATTGGTG 2040

AAAGTTGTTT AAACTACTTA TTGTTGGTAG GCACATCGTG TCAAGTGAAG TAGTTTTATA 2100

GGTATGGGTT TTTTCTCCCC CTTCACCAGG GTGGGTGGAA TAAGTTGATT TGGCCAATGT 2160

GTAATATTTA AACTGTTCTG TAAAATAAGT GTCTGGCCAT TTGGTATGAT TTCTGTGTGT 2220

GAAAGGTCCC AAAATCAAAA TGGTACATCC ATAATCAGCC ACCATTTAAC CCTTCCTTGT 2280

TCTAAAACAA AAACCAAAGG GCGCTGGTTG GTAGGGTGAG GTGGGGGAGT ATTTTAATTT 2340

TTGGAATTTG GGAAGCAGAC AGCTTTACTT TGTAAGGTTG GAACAGCAGC ACTATACATG 2400

AAATATAAAC CAAAAACCTT TACTGTTTCT AAATTTCCTA GATTGCTATT ATTTGGTTGT 2460

AAGTTGAGTA TTCCACAGAA AGTGGTAATT ATCTCTTCTC TCTTCCTCCA TTAGAAAATT 2520

AGGTAAATAA TGGATTCCTA TAATGGGAGC ATCACCACTT ATTAAAACAC ACATAGAATG 2580

ATGAATTAAA AAAGTTTTCT AGGATTGTCT TTTATTCTGC CACATTTATT GATAAACAGT 2640

GAAGGAATTT TTAAAAAATT TTTAAGAATT GTTTGTCACG TCATTTTTAG AAATGTTCTA 2700

CCTGTATATG GTAATGTCCA GTTTTAAAAA TATTGGACAT CTTCAATCTT AAACATTTCT 2760

ATTTAGCTGA TTGGTTCTCA CATATACTTC TAAAAGAAAC TTTTATGTTA TAAGAGTTAC 2820

TTTTTGGATA AGATTTATTA ATCTCAGTTA CCTACTATTC TGACATTTTA GGAAGGAGGT 2880

AATTGTTTTT AATGATGGAT AAACTTGTGC TGGTGTTTTG GATCTTATGA TGCTGAGCAT 2940

GTTCTGCACT GGTGCTAATG TCTAATATAA TTTTATATTT ACACACATAC GTGCTACCCA 3000

GAGATTAATT TAGTCCATAT GAACTATTGA CCCATTGTTC ATTGAGACAG CAACATACGC 3060

ACTCCTAAAT CAGTGTGTTT AGACTTTTCA AGTATCTAAC TCATTTCCAA ACATGTACCA 3120

TGTTTTATAA ACCTCTTGAT TTCCAGCAAC ATACTATAGA AAACACCTGC TACTCAAAAC 3180

ACAACTTCTC AGTGTCATCC ATTGCTGTCG TGAGAGACAA CATAGCAATA TCTGGTATGT 3240

TGCAAGCTTT CAAGATAGCC TGAACTTAAA AAGTTGGTGC ATTAGTTGTA TCTGATGGAT 3300

ATAAATTTGC CTCCTAGTTC ACTTTGTGTC AAGAGCTAAA ACTGTGAACC TAACTTTCTC 3360

TTATTGGTGG GTAATAACTG AAAATAAAGA TTTATTTTCA TGCTCACTTC TTAAAAGTCA 3420

TAAAAACAAT CAAATAGGRT CATGTTTATT GTCATGTGTT TCCTGGKTTC TGACCTGTGT 3480

GCACACCCCT GTGTGTTTAT AATTTTTAAA TTGAATTTTA TATGGGGTTT TTATTTGCTA 3540

AAAACCAGGC TGTTGAATCA CATTTGGGAA GGGTACTTAT CTTAATGACT AATGACTTAA 3600

TTGGGAAAGT TGAATTCTTG TAAAATACAA AATCCAAGGA CTTCTTGGGA TTTAATCTAA 3660

TTGTCACTTC NTTAGGCAGA TNCACTTTTT TGGATAATGG AAAGTTAAGC ATACCGAATG 3720

CTACTTTTGG TTGACAAACG GGCCTAATAG TCCGGGGGGA AATCCCTAAC NGGTAAGGNT 3780

CCCAAGTATG G 3791

(2) INFORMATION FOR SEQ ID NO: 215:

(i) SEQUENCE CHARACTERISTICS:
(A) LENGTH: 1334 base pairs
(B) TYPE: nucleic acid
(C) STRANDEDNESS: double
(D) TOPOLOGY: linear

(xi) SEQUENCE DESCRIPTION: SEQ ID NO: 215:

CAGTGCTCGC TCCTGCTCGG GGCGCTGCGG CCCCGGGCGT CGCCATGACC AGTGAGCTGG 60

ACATCTTCGT GGGGAACAGA CCCTTATCGA CGAGGACGTG TATCGCCTCT GGCTCGATGG 120

TTACTCGGTG ACCGACGCGG TGGCCCTGCG GGTGCGCTCG GGAATCCTGG AGCAGACTGG 180

CGCCACGGCA GCGGTGCTNC AGAGCGACAC CATGGACCAT TACCGCACCT TCCACATGCT 240

CGAGCGGCTG CTGCATGCGC CGCCCAAGCT ACTGCACCAG YTCATCTTCC AGATTCCGCC 300

CTCCCGGCAG GCACTACTCA TCGAGAGGTA CTATGCCTTT RATGAGGCCT TTGTTCGGGA 360

GGTGCTGGGC AAGAAGCTGT CCAAAGGCAC CAAGAAAGAC CTGGATGACA TCAGCACCAA 420

AACAGGCATC ACCCTCAAGA GCTGCCGGAG ACAGTTTGAC AACTTTAAAC GGGTCTTCAA 480

GGTGGTAGAG GAAATGCGGG GCTCCCTGGT GGACAATATT CAGCAACACT TCCTCCTCTC 540

TGACCGGTTG GCCAGGGACT ATGCAGCCAT CGTCTTCTTT GCTAACAACC GCTTTGAGAC 600

AGGGAAGAAA AAACTGCAGT ATCTGAGCTT CGGTGACTTT GCCTTCTGCG CTGAGCTCAT 660

GATCCAAAAC TGGACCCTTG GAGCCGTCGA CTCACAGATG GATGACATGG ACATGGACTT 720

AGACAAGGAA TTTCTCCAGG ACTTGAAGGA GCTCAAGGTG CTAGTGGCTG ACAAGGACCT 780

TCTGGACCTG CACAAGAGCC TGGTGTGCAC TGCTCTCCGG GGAAAGCTGG GCGTCTTCTC 840

TGAGATGGAA GCCAACTTCA AGAACCTGTC CCGGGGGCTG GTGAACGTGG CCGCCAAGCT 900

GACCCACAAT AAAGATGTCA GAGACCTGTT TGTGGACCTC GTGGAGAAGT TTGTGGAACC 960

CTGCCGCTCC GACCACTGGC CACTCAGCGA CGTGCGGTTC TTCCTGAATC AGTATTCAGC 1020

GTCTGTCCAC TCCCTCGATG GCTTCCGACA CCAGGCCTCT GGGACCGCTA CATGGGCACC 1080

CTCCGCGGCT GCCTCCTGCG CCTGTATCAT GACTGAGGTG CCTCCCAACG CTCCGCCCAC 1140

GCTGACAATA AAGTTGCTCT GAGTTTGGAG ACTGGTCCTC GCTCCGGGGA GCAAGTGGGG 1200

GGCGTGCAGA TGTGCCTGTG TCTGTCTCTG AGCACCTGGT GTCCGTGTAC AAGGATGGAT 1260

GTGTNCNGTG GCTCCTTGGG AACTGAGACA TATCTCAGGG AATGGTGTCT GTGCTCAGCC 1320

CATCCACCAG AAGA 1334

(2) INFORMATION FOR SEQ ID NO: 216:

(i) SEQUENCE CHARACTERISTICS:
(A) LENGTH: 1511 base pairs
(B) TYPE: nucleic acid
(C) STRANDEDNESS: double
(D) TOPOLOGY: linear

(xi) SEQUENCE DESCRIPTION: SEQ ID NO: 216:

GTGGCGGGGA TGCTGCGAGG GGGTCTCCTG CCCCAGGCGG GCCGGCTGCC TACCCTCCAG 60

ACTGTCCGCT ATGGCTCCAA GGCTGTTACC CGCCACCGTC GTGTGATGCA CTTTCAGCGG 120

CAGAAGCTGA TGGCTGTGAC TGAATATATC CCCCCGAAAC CAGCCATCCA CCCATCATGC 180

CTGCCATCTC CTCCCAGCCC CCCACAGGAG GAGATAGGCC TCATCAGGCT TCTCCGCCGG 240

GAGATAGCAG CAGTTTTCCA GGACAACCGA ATGATAGCCG TCTGCCAGAA TGTGGCTCTG 300

AGTGCAGAGG ACAAGCTTCT TATGCGACAC CAGCTGCGGA AACACAAGAT CCTGATGAAG 360

RTCTTCCCCA ACCAGGTCCT GAAGCCCTTC CTGGAGGATT CCAAGTACCA AAATCTGCTG 420

CCCCTTTTTG TGGGGCACAA CATGCTGCTG GTCAGTGAAG AGCCCAAGGT CAAGGAGATG 480

GTACGGATCT TAAGGACTGT GCCATTCCTG CCGCTGCTAG GTGGCTGCAT TGATGACACC 540

ATCCTCAGCA GGCAGGGCTT TATCAACTAC TCCAAGCTCC CCAGCCTGCC CCTGGTGCAG 600

GGGGAGCTTG TAGGAGGCCT CACCTGCCTC ACAGCCCAGA CCCACTCCCT GCTCCAGCAC 660

CAGCCCCTCC AGCTGACCAC CCTGTTGGAC CAGTACATCA GAGAGCAACG CGAGAAGGAT 720

TCTGTCATGT CGGCCAATGG GAAGCCAGAT CCTGACACTG TTCCGGACTC GTAGCCAGCC 780

TGTTTAGCCA GCCCTGCGCA TAAATACACT CTGCGTTATT GGCTGTGCTC TCCTCAATGG 840

GACATGTGGA AGAACTTGGG GTCGGGGAGT GTGTTTGTCA CTTGGTTTTC ACTAGTAATG 900

ATATTGTCAG GTATAGGGCC ACTTGGAGAT GCAGAGGATT CCATTTCAGA TGTCAGTCAC 960

CGGCTTCGTC CTTAGTTTTC CCAACTTGGG ACGTGATAGG AGCAAAGTCT CTCCATTCTC 1020

CAGGTCCAAG GCAGAGATCC TGAAAAGATA GGGCTATTGT CCCCTGCCTC CTTGGTCACT 1080

GCCTCTTGCT GCACGGGCTC CTGAGCCACC CCCTTGGGGC ACAACCTGCC ACTGCCACAG 1140

TAGCTCAACC AAGCAGTTGT GCTGAGAATG GCACCTGGTG AGAGCCTGCT GTGTGCCAGG 1200

CTTTGTGCTG AGTGCTGTAC ATGTATTAGT TCCTTTACTG CTGACCACAT TGTACCCATT 1260

TCACAGAGAA GGAGCAGAGA AATTAAGTGG CTTGCTCAAG GTCATGCAGT TAGTAAGTGG 1320

CAGAACAGGG ACTTGAACCA AGCCCTCTGC TCTGAAGACC GCGTCCTGAA TTTCTTCACT 1380

AGAGCTTCCT CATCAGGTTA CCCAGAAGTG GGTCCCATCC ACCATCCAGG TGTGCTTGGA 1440

TGTTAGTTCT CCACCCTCGA GGTGTACGCT GTGAAAAGTT TGGGAGCACT GCTTTATAAT 1500

AAAATGAAAT A 1511

(2) INFORMATION FOR SEQ ID NO: 217:

(i) SEQUENCE CHARACTERISTICS:
(A) LENGTH: 642 base pairs
(B) TYPE: nucleic acid
(C) STRANDEDNESS: double
(D) TOPOLOGY: linear

(xi) SEQUENCE DESCRIPTION: SEQ ID NO: 217:

AGGCCTTACT TTTCCTCCCA CAAAGGAGTC GCAGCCACGC TAGCTCTGAC TTGCCACTGT 60

GACAAAGTTC ACGTAGCAGG TCTAGGCAAA GACTGGGCAA TTGAGCAGAG GAGACGGACC 120

TGTGAGTCTG ACCRYGAGSC GGRCCCCTTC ACCTTGGCTG GGCTGGTCCT GGTCCTTAGG 180

TTTTGTCAGG TTGTCCTTGT TTGGATCCCT CAACTAGGTG ATAAGCACTG GAGGGGGATG 240

ACCCGCCTTG GACGTGTTTC TTTAACCTCA TCCATATAAT AGGGCCGTGG GATGGTTGTA 300

GAGGTAAAGC AGGATGATGG TGTTTTAAGA CCAGAGCTTG GGACCAGGGC TCCTACACCT 360

AATTTTCTCT CCTGGTAGCT GAACAAAGGT CTAAATTAGC TTAACAAAAG AACAGGCTGC 420

CGTCAGCCAG AGTTCTGAAG GCCATGCTTT CAGTTTCCCT TGTTGACAAT TGCTCTCCAG 480

TTCCTATGAA AGCACAGAGC CTTAGGGGGC CTGGCCACAG AACACAACCA TCTTAGGCCT 540

GAGCTGTGAA CAGCAGGGGG TTGTGTGTCT GTTCTGTTTC TCTGCTTGCC GAACTTTCTC 600

AATAAACCCT ATTTCTTATT TTATATTTAC GTNGGTGCTG GG 642

(2) INFORMATION FOR SEQ ID NO: 218:

(i) SEQUENCE CHARACTERISTICS:
(A) LENGTH: 1241 base pairs
(B) TYPE: nucleic acid
(C) STRANDEDNESS: double
(D) TOPOLOGY: linear

(xi) SEQUENCE DESCRIPTION: SEQ ID NO: 218:

GGTCCCACTG TTCCATTTTA TGCTAATAGA TTCCATTCTA GGGCCCAGCC GTCTCTTGAC 60

TGATGGTGTT CCCTTTAACC CTTGGCATGT ATAATAGAAT TTTGGTGAAT GAAAGAACCC 120

AAATAGGCCA GATAGTCCCC CCAGGCCCTG ATATCCATAA AAGGCTTGGG AATGCATTAT 180

GTAATTGTCC TTAGTCTTTT TGTTGTTTTA GAAAAAAAAA ACAAGATGGG CTCAGATGGA 240

TGCCTACGTA AAAATGGTTC CTAGCTGTGT ACTCATAACT TTTCTTTGAA TTGAGTAGTG 300

AAAGGAAGGA GGAGGAAAGG AAATTAAATG TCCTTCTAGT ATTCTCTGGA CTCAAGTCTG 360

ACATATGRGA TAATAACCTA TATTGAAATG CCAAGAATTG TATCTGAAAC AAGRGAACAG 420

TTTGACACAT TTATCATGCC TTCATATTAC ATATTAACTG AAACCAATTA ATAAACATAT 480

GAAATATCCA TTGCACAAGG CAAAGGCACC TAAACCTTTT GTTTCTTTTT CTACATAGCA 540

GAAATTGATT TTTTTTTTAT TTTTTTAGGG GAACCTATAT AATTATGACC CAGTGATGTC 600

TTTTGGTGAC TTAAGCTTAT GAATTCAGGT TACAATTGAG TTGATTCTAG ATGGTTACTA 660

CCTTGAAAAG GATGTTGGTG CCTTATGTGA CACGAGCCAG AGCCTGCTGG GAATAAACAA 720

AGCAGATTCA TGCCAACACC AACTCGTAGC TTTAGTGGCA GATGGGAGTG GTCACAGACT 780

CCCAAAATGT GGGGCTTTGG ATTTCCACAC CATCCCACGT GTGTGTCATC TTCCTCTTTC 840

ACACTCTTGA TGATAATTTG AAAATGRTGA AATCACCTCT GAATTTGCCT ATAGCATGAG 900

CACATTCTTA TGACAACATA ACAAATAGTT CATAATGTGA ATATTAGAAA CTGTTACAGC 960

CTGCAGTTAC CATAATTTTC CATGTTTGTG GAATTGATAT TGAAATAGCA GGGCTAAGGA 1020

ATTACTGGCA AGTTTTAGCC TGTGGGTAAT ACCTTAGGGT TATTTAAATA TTTGTAATTT 1080

TATTTAAATG TTCATGAATG TTTGAAAGGA ACAAAATTAT CAGGGATGGC TCTTTGCCAT 1140

GGGTCTTATT TTCACCCTCT TTTCTGTAAG AAAAAAGAAC AATGTCTTAA TGTATTTTTA 1200

AAGTTTTTGG TATAGTTTCT AATTCCAATT TTAATAAAAG T 1241

(2) INFORMATION FOR SEQ ID NO: 219:

(i) SEQUENCE CHARACTERISTICS:
(A) LENGTH: 1080 base pairs
(B) TYPE: nucleic acid
(C) STRANDEDNESS: double
(D) TOPOLOGY: linear

(xi) SEQUENCE DESCRIPTION: SEQ ID NO: 219:

TGTTTATGTG ACCTAAAACA TACACACATG CACACACACA TACATATCCA TTCATTCATT 60

CATTCAAGTG GTGTTTCCAG TGTCTGTGTG TCACTGTTTA TGCAGTTTCC ATTTCCCAGT 120

GAATTATGAG TGGAGGGCAA CTTTTCTAAC CAGATTGTCT TTTCAGAACA AAGACCKGGG 180

RATTGAGGAA GAGTTTGGAA AGAGGGAGAG GCAAGGAAAG AGAGCTTTAA ATTGAAAGGT 240

TAATTTCCTA AGAGGAACCT GGGCTGAATG ACTACAGTGT TATACCCTCC AATCTTTGCA 300

GGTGGGCATG GAACACTGCT TGTATCACTC TGTGCACGGT ATAAATCCAT ATATCCACAA 360

AAACACACAT CCATCCATCA ACATATACAT GGTTTGGGAT GAGCAGGTCA ATAGTTTTGA 420

GAGGGAGTTT GTTCCTTTTT TTTTCTCATT ATACTCTTAA ATTGTTGTCA GTTATCAAAC 480

AAACAAACAG AAAAATTGTT TGGGAAAAAC CTTGCATACG CCTTTTCTAT CMAGTGCTTT 540

AAAATATAGA CTAAATACAC ACATCCTGCC AGTTTTTTCT TACAGTGACA GTATCCTTAC 600

CTGCCATTTA ATATTAGCCT CGTATTTTTC TCACGTATAT TTACCTGTGA CTTGTATTTG 660

TTATTTAAAC AGGAAAAAAA ACATTCAAAA AAAGAAAAAT TAACTGTAGC GCTTCATTAT 720

ACTATTATAT TATTATTATT ATTGTGACAT TTTGGAATAC TGTGAAGTTT TATCTCTTGC 780

ATATACTTTA TACGGAAGTA TTACGCCTTA AAAATACGAA AATAAATTTT ACAAGGTTTC 840

TGTTTTGTGT GGAAGAGTAA TTGATGTTGC TAAGAATGAT GTTTGTTTTT TTGGGGTTTT 900

TGTTGTTTTT TTTTTAAATG TTACCAGCAC TTTTTTTGTA AGTTTCACTT TCCGAGGTAT 960

TGTACAAGTT CACACTGTTT GTGAAGTTTG AATATGAAGG AATAATTAAA AAAAAAAAAA 1020

AAACCNCGGG GGGGGCCCGG TCCCATTGGN CCCAAGGGGG CGGTTACGGG GTCACGGCCG 1080

(2) INFORMATION FOR SEQ ID NO: 220:

(i) SEQUENCE CHARACTERISTICS:
(A) LENGTH: 1258 base pairs
(B) TYPE: nucleic acid
(C) STRANDEDNESS: double
(D) TOPOLOGY: linear

(xi) SEQUENCE DESCRIPTION: SEQ ID NO: 220:

TGAATTGAGG GCTTAAAGAT AAACATATGG GRTTGGAGTT GTGTGTCCAT AGGGTTTCAC 60

TGCCTATTTG ATTTGAGTTT ATCCCTATTA ATTTTTTACA GTGAAATTTT ATTAAAGTAT 120

AATGTACATA TATTTTCAGT GGATTTTGCT CTGAAGGTTC TCCAGTGGTC TGACTACGAG 180

ATAGTGCGGC TTCAGCTGTG GGATATTGCA GGGCAGGAGC GCTTCACCTC TATGACACGA 240

TTGTATTATC GGGATGCCTC TGCCTGTGTT ATTATGTTTG ACGTTACCAA TGCCACTACC 300

TTCAGCAACA GCCAGAGGTG GAAACAGGAC CTAGACAGCA AGCTCACACT ACCCAATGGA 360

GAGCCGGTGC CCTGCCTGCT CTTGGCCAAC AAGTGTGATC TGTCCCCTTG GGCAGTGAGC 420

CGGGASCAGA TTGACCGGTT CAGTAAAGAG AACGGTTTCA CAGGTTGGAC AGAAACATCA 480

GTCAAGGAGA ACAAAAATAT TAATGAGGCT ATGAGAGTCC TCATTGAAAA GATGATGAGA 540

AATTCCACAG AAGATATCAT GTCTTTGTCC ACCCAAGGGG ACTACATCAA TCTACAAACC 600

AAGTCCTCCA GCTGGTCCTG CTGCTAGTAG TGTTTGGYTT ATTTTCCATC CCAGTTCTGG 660

GAGGTCTTTT AAGTCTCTTC CCTTTGGTTG CCCACCTGAC MATTTTATTA AGTACATTTG 720

AATTGTCTCC TGACTACTGT CCAGTAAGGA GGCCCATTGT CACTTAGAAA AGACACCTGG 780

AACCCAKGTG CATTTCTGCA TCTCCTGGAT TAGCCTTTSA CATGTTGCTG RCTCACATTA 840

GTGCCAGTTA GTGCCTTCGG TGTAAGATCT TCTCATCAGC CCTCAATTTG TGATCCGGAA 900

TTTTGTGAGA AGGATKAGAA ATCAGCACCT GCGTTTTAGA GATCATAATT CTCACCTACT 960

TCTGAGCTTA TTTTTCCATT TGATATTCAT TGATATCATG ACTTCCAATT GAGAGGAAAA 1020

TGAGATCAAA TGTCATTTCC CAAATTTCTT GTAGGCCGTT GTTTCAGATT CTTTCTGTCT 1080

TGGAATGTAA ACATCTGATT CTGGAATGCA GAAGGAGGGG TCTGGGCATC TGTGGATTTT 1140

TGGCTACTAG AAGTGTCCCA GAAGTCACTG TATTTTTGAA ACTTCTAACG TCATAATTAA 1200

GTTTCTCTTG TCTTGGGCAT CAAGANTAGT TCCAATTTTT TGGGCCGGGG CAGGGTGG 1258

(2) INFORMATION FOR SEQ ID NO: 221:

(i) SEQUENCE CHARACTERISTICS:

(A) LENGTH: 1693 base pairs
(B) TYPE: nucleic acid
(C) STRANDEDNESS: double
(D) TOPOLOGY: linear

(xi) SEQUENCE DESCRIPTION: SEQ ID NO: 221:

CACAATATAT GAAATAGTAC CCTCTAAAAA AGAGAAAAAA AAAATCAGGC GGTCAAACTT 60

AGAGCAACAT TGTCTTATTA AAGCATAGTT TATTTCACTA GAAAAAATTT AATATCAAGG 120

ACTATTACAT ACTTCATTAC TAGGAAGTTC TTTTTAAAAT GACACTTAAA ACAATCACTG 180

AAAACTTGAT CCACATCACA CCCTGTTTAT TTTCCTTAAA CATCTTGGAA GCCTAAGCTT 240

CTGAGAATCA TGTGGCAAGT GTGATGGGCA GTAAAATACC AGAGAAGATG TTTAGTAGCA 300

ATTAAAGGCT GTTTGCACCT TTAAGGACCA GCTGGGCTGT AGTGATTCCT GGGGCCAGAG 360

TGGCATTATG TTTTTACAAA ATAATGACAT ATGTCACATG TTTGCATGTT TGTTTGCTTG 420

TTGAATTTTT GAACAGCCAG TTGACCAATC ATAGAAAGTA TTACTTTCTT TCATATGGTT 480

TTTGGTTCAC TGGCTTAAGA GGTTTCTCAG AATATCTATG GCCACAGCAG CATACCAGTT 540

TCCATCCTAA TAGGAATGAA ATTAATTTTG TATCTACTGA TAACAGAATC TGGGTCACAT 600

GAAAAAAAAT CATTTTATCC GTCTTTTAAG TATATGTTTA AAATAATAAT TTATGTGTCT 660

GCATATTGCA GAACAGCTCT GAGAGCAACA GTTTCCCATT AACTCTTTCT GACCAATAGT 720

GCTGGCACCG TTGCTTCCTC TTTGGGAAGA GGAAAGGGTG TGTGAACATG GCTAACAATC 780

TTCAAATACC CAAATTGTGA TAGCATAAAT AAAGTATTTA TTTTATGCCT CAGTATATTA 840

TTATTTAATT TTTTAGGTAA TGCCTATCTC TTGGTCTATT AAGGAAAGAA GCAATCAGTA 900

GAGAATTCAG GATAGTTTTG TTTAAATTCT TGCAGATTAC ATGTTTTTAC AGTGGCCTGC 960

TATTGAGGAA AGGTATTCTT CYATACAACT TGTTTTAACC TTTGAGAACA TTGACAGAAA 1020

TTATGCAATG GTTTGTTGAG ATACGGACTT GATGGTGCTG TTTAATCAGT TTGCTTCCAA 1080

AGTGGCCTAC TCAAGAGGCC CTAAGACTGG TAGAAATTAA AAGGATTTCA AAAACTTTCT 1140

ATTCCTTTCT TAAACCTACC AGCAAACTAG GATTGTGATA GCAATGAATG GTATGATGAA 1200

GAAAGTTTGA CCAAATTTGT TTTTTTGTTG TTGTTGTTGT TTTGAATTTG AAATCATTCT 1260

TATTCCCTTT AAGAATGTTT ATGTATGAGT GTGAAGATGC TAGCGAACCT ATGCTCAGAT 1320

ATTCATCGTA AGTCTCCCTT CACCTGTTAC AGAGTTTCAG ATCGGTCACT GATAGTATGT 1380

ATTTCTTTAG TAAGAATGTG TTAAAATTAC AATGATCTTT TAAAAAGATG ATGCAGTTCT 1440

GTATTTATTG TGCTGTGTCT GGTCCTAAGT GGAGCCAATT AAACAAGTTT CATATGTATT 1500

TTTCCAGTGT TGAATCTCAC ACACTGTACT TTGAAAATTT CCTTCCATCC TGAATAACGA 1560

ATAGAAGAGG CCATATATAT TGCCTCCTTA TCCTTGAGAT TTCACTACCT TTATGTTAAA 1620

AGTTGTGTAT AATTGTTAAA ATCTGTGAAA GAATAAAAAG TGGATTTAAA TTAAAAAAAA 1680

AAAAAAAAAA AAA 1693

(2) INFORMATION FOR SEQ ID NO: 222:

(i) SEQUENCE CHARACTERISTICS:
(A) LENGTH: 1196 base pairs
(B) TYPE: nucleic acid
(C) STRANDEDNESS: double
(D) TOPOLOGY: linear

(xi) SEQUENCE DESCRIPTION: SEQ ID NO: 222:

ACGCGTGGGT CGACCCACGC GTCCGCGACN TGGCGTGGTG GGGAAGGGAG AAGGATTTGT 60

AAACCCCGGA GCGAGGTTCT GCTTACCCGA GGCCGCTGCT GTGCGGAGAC CCCCGGGTGA 120

AGCCACCGTC ATCATGTCTG ACCAGGAGGC AAAACCTTCA ACTGAGGACT TGGGGGATAA 180

GAAGGAAGGT GAATATATTA AACTCAAAGT CATTGGACAG GATAGCAGTG AGATTCACTT 240

CAAAGTGAAA ATGACAACAC ATCTCAAGAA ACTCAAAGAA TCATACTGTC AAAGACAGGG 300

TGTTCCAATG AATTCACTCA GGTTTCTCTT TGAGGGTCAG AGAATTGCTG ATAATCATAC 360

TCCAAAAGAA CTGGGAATGG AGGAAGAAGA TGTGATTGAA GTTTATCAGG AACAAACGGG 420

GGGTCATTCA ACAGTTTAGA TATTCTTTTT ATTTTTTTTC TTTTCCCTCA ATCCTTTTTT 480

ATTTTTAAAA ATAGTTCTTT TGTAATGTGG TGTTCAAAAC GGAATTGAAA ACTGGCACCC 540

CATCTCTTTG AAACATCTGG TAATTTGAAT TCTAGTGCTC ATTATTCATT ATTGTTTGTT 600

TTCATTGTGC TGATTTTTGG TGATCAAGCC TCAGTCCCCT TCATATTACC CTCTCCTTTT 660

TAAAAATTAC GTGTGCACAG AGAGGTCACC TTTTTCAGGA CATTGCATTT TCAGGCTTGT 720

GGTGATAAAT AAGATCGACC AATGCAAGTG TTCATAATGA CTTTCCAATT GGCCCTGATG 780

TTCTAGCATG TGATTACTTC ACTCCTGGAC TGTGACTTTC AGTGGGAGAT GGAAGTTTTT 840

CAGAGAACTG AACTGTGGAA AAATGACCTT TCCTTAACTT GAAGCTACTT TTAAAATTTG 900

AGGGTCTGGA CCAAAAGAAG AGGAATATCA GGTTGAAGTC AAGATGACAG ATAAGGTGAG 960

AGTAATGACT AACTCCAAAG ATGGCTTCAC TGAAGAAAAG GCATTTTAAG ATTTTTTAAA 1020

AATCTTGTCA GAAGATCCCA GAAAAGTTCT AATTTTCATT AGCAATTAAT AAAGCTATAC 1080

ATGCAGAAAT GAATACAACA GAACACTGCT CTTTTTGATT TTATTTGTAC TTTTTGGCCT 1140

GGGATATGGG TTTTAAATGG ACATTGTCTG TACCAGCTTC ATTAAAATAA ACAATA 1196

(2) INFORMATION FOR SEQ ID NO: 223:

(i) SEQUENCE CHARACTERISTICS:
(A) LENGTH: 1791 base pairs
(B) TYPE: nucleic acid
(C) STRANDEDNESS: double
(D) TOPOLOGY: linear

(xi) SEQUENCE DESCRIPTION: SEQ ID NO: 223:

TCAGGGAGGT GGCAGGAAAG GCTTGGAACA GCTGCCGGAG TGACGGAGCG GCGGCCCCGC 60

CCGGTTGCGC TGGAGGTCGA AGCTTCCAGG TAGCGGCCCG CAGAGCCTGA CCCAGGCTCT 120

GGACATCCTG AGCCCAAGTC CCCCACACTC AGTGCAGTGA TGAGTGCGGA AGTGAAGGTG 180

ACAGGGCAGA ACCAGGAGCA ATTTCTGCTC CTAGCCAAGT CGGCCAAGGG GGCAGCGCTG 240

GCCACACTCA TCCATCAGGT GCTGGAGGCC CCTGGTGTCT ACGTGTTTGG AGAACTGCTG 300

GACATGCCCA ATGTTAGAGA GCTGGCTGAG AGTGACTTTG CCTCTACCTT CCGGCTGCTC 360

ACAGTGTTTG CTTATGGGAC ATACGCTGAC TACTTAGCTG AAGCCCGGAA TCTTCCTCCA 420

CTAACAGAGG CTCAGAAGAA TAAGCTTCGA CACCTCTCAG TTGTCACCCT GGCTGCTAAA 480

GTAAAGTGTA TCCCATATGC AGTGTTGCTG GAGGTCTTGC CCTGCGTAAT GTGCGGCAGC 540

TGGAAGACCT TGTGATTGAG GCTGTGTATG CTGACGTGCT TCGTGGCTCC CTGGACCAGC 600

GCAACCAGCG GCTCGAGGTT GACTACAGCA TCGGGCGGGA CATCCAGCGC CAGGACCTCA 660

GTGCCATTGC CCGAACCCTG CAGGAATGGT GTGTGGGCTG TRAGGTCGTG CTGTCAGGCA 720

TTGAGGAGCA GGTGAGCCGT GCCAACCAAC ACAAGGAGCA GCAGCTGGGC CTGAAGCAGC 780

AGATTGAGAG TGAGGTTGCC AACCTTAAAA AAACCATTAA AGTTACGACG GCAGCAGCAG 840

CCGCAGCCAC ATCTCAGGAC CCTGAGCAAC ACCTGACTGA GCTGAGGGAA CCAGCTCCTG 900

GCACCAACCA GCGCCASCCA GCAAGAAAGC CTCAAAGGGC AAGGGGCTCC GAGGGAGCGC 960

CAAGATTTGG TCCAAGTCGA ATTGAAAGRA CTGTCGTTTC CTCCCTGGGG ATGTGGGGTC 1020

CCAGCTGCCT GCCTGCCTCT TAGGAGTCCT CAGAGAGCCT TCTGTGCCCC TGGCCAGCTG 1080

ATAATCCTAG GTTCATGACC CTTCACCTCC CCTAACCCCA AACATAGATC ACACCTTCTC 1140

TAGGGAGGAG KCAAATGTAG GTCATGTTTT TGTTGGTACT TTCTGTTTTT TGTGACTTCA 1200

TGTGTTCCAT TGCTCCCCGC TGCCATGCTC TCTCCCTTGT TTCCTTAAGA GCTCAGCATC 1260

TGTCCCTGTT CATTACATGT CATTGAGTAG GTGGGTAGCC CTGATGGGGG TCGCTCTGTC 1320

TGGAGCATAA CCCACAGGCG TTTTTTCTGC CACCCCATCC CTGCATGCCT GATCCCCAGT 1380

TCCTATACCC TACCCCTGAC CTATTGAGCA GCCTCTGAAG AGCCATAGGG CCCCCACCTT 1440

TACTCACACC CTGAGAATTC TGGGAGCCAG TCTGCCATGC CAGGAGTCAC TGGACATGTT 1500

CATCCTAGAA TCCTGTCACA CTACAGTCAT TTCTTTTCCT CTCTCTGGCC CTTGGGTCCT 1560

GGGAATGCTG CTGCTTCAAC CCCAGAGCCT AAGAATGGCA GCCGTTTCTT AACATGTTGA 1620

GAGATGATTC TTTCTTGGCC CTGGCCATCT CGGGAAGCTT GATGGCAATC CTGGAAGGGT 1680

TTAATCTCCT TTTGTGAGTT TGGTGGGGAA GGGAAGGGTA TATAGATTGT ATTAAAAAAA 1740

AAAAGGTATA TATGCATATA TCTATATATA ATATGACGCA GAAATAAATC T 1791

(2) INFORMATION FOR SEQ ID NO: 224:

(i) SEQUENCE CHARACTERISTICS:
(A) LENGTH: 2517 base pairs
(B) TYPE: nucleic acid
(C) STRANDEDNESS: double
(D) TOPOLOGY: linear

(xi) SEQUENCE DESCRIPTION: SEQ ID NO: 224:

ACACTAGTGG ATCCAAAGAA TTCGGCACAG CGGCACAGCA TTGTTGAGCT TTTCTGTGTG 60

TGTGGGGCCC TCAAGCGAGC TCGACTGGTC CATCCTGGGG TAGCGASGTG GTGTTTGTGA 120

AAAAGGACGA TGCCATCACC GCATAYAAGA AGTACAACAA CCGGTGTCTG GACGGGCAGC 180

CGATGAAGTG CAACCTTCAC ATGAATGGGA ATGTTATCAC CTCAGACCAG CCCATCCTGC 240

TGCGGCTGAG TGACAGCCCA TCAATGAAAA AGGAGAGCGA GCTGCCTCGC AGGGTGAACT 300

CTGCCTCCTC CTCCAACCCC CCTGCCGAAG TGGACCCTGA CACCATCCTG AAGGCACTCT 360

TCAAGTCCTC AGGGGCCTCT KTGACCACGC AGCCCACAGA WTTCAAAATC AAGCTTTGAG 420

CAGGGGAGTR AGGCAGCCAG AAGTGGGGGC AGAGGAGGGT GGCTCTGTTT CCCCAAGGCA 480

AAGCTTATGA CCAATGGGCC ATCGGACTGG AGACCCCTGA TTGTGGGAAG GGTTGCCAGG 540

GATAAAGAGC TTCCTCACTG GATGGGACCC GCCTTTCTGT GTTGTGTTCT GCCCTGTGCT 600

CTTCTCTCTA CGTTAACGTT TCCTGTAGTA TGTTTCTTCA TCTCATCGCC AAGGTAGGCT 660

TGTGTTTTTM AGTGTGTGCC TCCCCGAGCC TCAGCCCCAA GCTGATTTCT TATCTGGAAA 720

TGGTACACTG AATTCTCTGG GTGGCTTTCT TGTGGCCCCA TGGGATGCAG CGTGGGGGCT 780

GTCTGAAGGA CCCTGCTTTT TCCAGGGGCC GAGGGGCTGC CTTTCCTTTG TGTGTATTAA 840

GCTTTTCAAA CAATGGAGGG GATGGAGAGC CCTGGTGTCC TGACGGGAGC CAGGTCGGCC 900

TGAGAGCTGT GCCGCTCCTC TGTCTTGTCA GTGGAGGTGC CTGGGTGGGG AGCAGGTCTC 960

AGGCCTCTTG TCCTCTCCCC AGTGGCTCCA GGCCTCACTA GTGGCAAGGG CAGGATGAGG 1020

CTGCACCGCT GGGAAGAGTC TATCTAAGCT CTTGGCTTGG AGTCCCGTGT CGTCTCCRCC 1080

CAGAGGAAGT TCTCCAGAGT TCACCTTTCC CTTTTCCTTG AGTTGTGCTG AATGCCCCAC 1140

CCCAGCTCTC TTTCCCTTCT GGGTGTCTTT GCTGGGAGGG GGCTGTGTTG TGAGCCCTCC 1200

CGGTTCTCAC CTCGCCTGGC ACTTAACCAC ACCCTGGTTT TGTGTAGCCG CCAGCTCTCT 1260

TCTGGTTGGG CCTTTGAAAG GCTCAGCCTC CCATTGTGCA GTGCTTGGGT TTGGAGCTTA 1320

TTTGAATGGA AGAGGTCAGT TTGTTCCTGG CTCTCCATTT CTGGCCTCAG TTGTCTACAG 1380

GACAGTGGTC AGGGATGCCT GGAGGCATAT ATCCAGCTGC CACCAAGGGG CACTGTTTGT 1440

TCCCACTTAT GTGAGTGACC CCATCCATCC ATGACCAGAG GATTATTTTC CTGCCTTGGC 1500

AGAGGAGGAG GAGTCAAGGG AGCAGGGCAG CTCTACCAGG CAAGGTGTTT CCCCAGCATA 1560

GGCGCAGACA GTTGGGACGA AACTTCAGAG CCCAGGCAGT CCCTGAATGA CCAGGCCAGT 1620

GTTGTCACTG AGTGGTCCCC TGCTGGTTGG GAGTGAAGAG AATCCAGGCT GGCAGAGCTG 1680

GAGCCAGTTG GGGAGCACGG TTCTGGGAGC TCTGCAAAAT CAGTAGCAAG TGCTGGAAAA 1740

GGCACATGCC GAAGATACTC AAGAGCTCCC AAGATTTGCT TGAGGCTAGC CCAGTGAAAA 1800

AAACCAGAGA CTCATGTTTC CAGGGGTCAG TCTGTCAGGC AGGAAGGACC CAGGATTTGA 1860

ACCCAGCTTC AGTGTGCAGG CTCTGAGGCT GCCCAGGACG GGAAAGTCCA AGGAAGGGGC 1920

CTGGTGGTGC TCCACTTGCA GTTCTTTAAA GAATGCTGCT TTTTATTCTC CTAACCCTTT 1980

CAAGTGGGTG CAGACTTCTC GTTAGCAGCT GGAAGACATT CCTCCCACAC TTTTCCCTTC 2040

CTGGCCCAAG AGAGCATCCA GAAGGCAGTA GGACCTGGTT TTTCAGGTAC TGGGAGCCGG 2100

GGGCTCACTG CTTGCACTGT GCTTAGGGTA GGGATGGTAA ATATCCTCCC TGCATGGCTT 2160

TATCCTCCCT CTCATCCCAA AGCAGGTATC TTCTGGTTGT CACAGAGTTT CATTGAGTCC 2220

AGCTGCAGCC ACGTGGCCAT CTGGAGCTGG TGCTATAGGT GACCATCTGG TACATTGAGG 2280

GGACCTGTTT GCCTCCTCCA CTCTATAAGC AGTCATCTTG GGAGACCGGG AGGAGAAGGT 2340

GGTGGGCTAG TCCTGTGTCC TCCTCCACTT CCCATGCCTC TATGTTACCC ATCTGTGTCT 2400

CCTGTGCAGA AGGAGAGGAA GGGGCATTAA GAGATGAAGG GTGATTATGT ATTACTTATC 2460

CATTTCTGAA TAAACATTTG TTATTCCTAA AAAAAAAAAA AAAAAACTCG AGGGGGG 2517

(2) INFORMATION FOR SEQ ID NO: 225:

(i) SEQUENCE CHARACTERISTICS:
(A) LENGTH: 2424 base pairs
(B) TYPE: nucleic acid
(C) STRANDEDNESS: double
(D) TOPOLOGY: linear

(xi) SEQUENCE DESCRIPTION: SEQ ID NO: 225:

TTGTANCTAA TCGAGGATTG ATTCTAATGA CAGAGTCTTT CAACACTTTG CACATGATGT 60

ATCACGAAGC TACAGCTTGC CATGTGACTG GAGATTTAGT AGAACTTCTG TCAATATTTC 120

TTTCGGTTTT GAAGTCTACA CGCCCTTATC TTCAGAGAAA AGATGTGAAA CAAGCATTAA 180

TCCAGTGGCA GGAGCGAATT GAATTTGCCC ATAAACTGTT AACTCTTCTT AATTCCTATA 240

GTCCTCCAGA ACTTAGAAAT GCCTGTATAG ATGTCCTCAA GGAACTTGTA CTTTTGAGTC 300

CCCATGATTT TYTTCATACT CTGGTTCCCT TTCTACAACA CAACCATTGT ACTTACCATC 360

ACAGTAATAT ACCAATGTCT CTTGGACCTT ATTTCCCTTG TCRAGAAAAT ATCAAGCTAA 420

TAGGAGGGAA AAGCAATATT CGGCCTCCGC GCCCTGAACT CAATATGTGC CTCTTGCCCA 480

CAATGGTGGA AACCAGTAAG GGCAAAGATG ACGTTTATGA TCGTATGCTG CTAGACTACT 540

TCTTTTCTTA TCATCAGTTC ATCCATCTAT TATGCCGAGT TGCAATCAAC TGTGAAAAAT 600

TTACTGAAAC ATTAGTTAAG CTGAGTGTCC TAGTTGCCTA TGAAGGTTTG CCACTTCATC 660

TTGCACTGTT CCCCAAACTT TGGACTGAGC TATGCCAGAC TCAGTCTGCT ATGTCAAAAA 720

ACTGCATCAA GCTTTTGTGT GAAGATCCTG TTTTCGCAGA ATATATTAAA TGTATCCTAA 780

TGGATGAAAG AACTTTTTTA AACAACAACA TTGTCTACAC GTTCATGACA CATTTCCTTC 840

TAAAGGTTCA AAGTCAAGTG TTTTCTGAAG CAAACTGTGC CAATTTGATC AGCACTCTTA 900

TTACAAACTT GATAAGCCAG TATCAGAACC TACAGTCTGA TTTCTCCAAC CGAGTTGAAA 960

TTTCCAAAGC AAGTGCTTCT TTAAATGGGG ACCTGAGGGC ACTCGCTTTG CTCCTGTCAG 1020

TACACACTCC CAAACAGTTA AACCCAGCTC TAATTCCAAC TCTGCAAGAG CTTTTAAGCA 1080

AATGCAGGAC TTGTCTGCAA CAGAGAAACT CACTCCAAGA GCAAGAAGCC AAAGAAAGAA 1140

AAACTAAAGA TGATGAAGGA GCAACTCCCA TTAAAAGGCG GCGTGTTAGC AGTGATGAGG 1200

AGCACACTGT AGACAGCTGC ATCAGTGACA TGAAAACAGA AACCAGGGAG GTCCTGACCC 1260

CAACGAGCAC TTCTGACAAT GAGACCAGAG ACTCCTCAAT TATTGATCCA GGAACTGAGC 1320

AAGATCTTCC TTCCCCTGAA AATAGTTCTG TTAAAGAATA CCGAATGGAA GTTCCATCTT 1380

CGTTTTCAGA AGACATGTCA AATATCAGGT CACAGCATGC AGAAGAACAG TCCAACAATG 1440

GTAGATATGA CGATTGTAAA GAATTTAAAG ACCTCCACTG TTCCAAGGAT TCTACCCTAG 1500

CCGAGGAAGA ATCTGAGTTC CCTTCTACTT CTATCTCTGC AGTTCTGTCT GACTTAGCTG 1560

ACTTGAGAAG CTGTGATGGC CAAGCTTTGC CCTCCCAGGA CCCTGAGGTT GCTTTATCTC 1620

TCAGTTGTGG CCATTCCAGA GGACTCTTTA GTCATATGCA GCAACATGAC ATTTTAGATA 1680

CCCTGTGTAG GACCATTGAA TCTACAATCC ATGTCGTCAC AAGGGATATC TGGCAAAGGA 1740

AACCAAGCTG CTTCTTGACA TTAGGTGTAG CATGTCTACT TTTAAGTCCC TCACCCCCAA 1800

CCCCCATGCT GTTTGTATAA GTTTTGCTTA TTTGTTTTTG TGCTTCAGTT TGTCCAGTGC 1860

TCTCTGCTTG AATGGCAAGA TAGATTTATA GGCTTAATTC TTGGTCAGGC AGAACTCCAG 1920

ATGAAAAAAA CTTGCATCTT CAGTATACTT CCTAAAGGGC AATCAGATAA TGGATATGTT 1980

TTATGTAATT AAGAGTTCAC TTTAGTGGCT TTCATTTAAT ATGGCTGTCT GGGAAGAACA 2040

GGGTTGCCTA GCCCTGTACA ATGTAATTTA AACTTACAGC ATTTTTACTG TGTATGATAT 2100

GGTGTCCTCT GTGCCAGTTT TGTACCTTAT AGAGGCAGAT TGCCTCCGAT CGCTGTGGTT 2160

CTTATTATCA AAATTAAGTT TACTTGTATA CGGAACAACC ACAAGAAATT TGATTCTGTA 2220

AAGAATCCTC TTTAGCTGTG GCCTGGCAGT ATATAAATGG TGCTTTATTT AACAGAATAC 2280

CTGTGGAGGA AATAAAGCAC ACTTGATGTA AAAATAATTG TTTTATTTTT ATTGACATGA 2340

CTGATTGATT GCTATTCTGT GCACTNAATT AAACTGATTG TGATGACTTA AAAAAAAAAA 2400

AAAAAAAAAA AAAAAAAAAA AAAA 2424

(2) INFORMATION FOR SEQ ID NO: 226:

(i) SEQUENCE CHARACTERISTICS:
(A) LENGTH: 1080 base pairs
(B) TYPE: nucleic acid
(C) STRANDEDNESS: double
(D) TOPOLOGY: linear

(xi) SEQUENCE DESCRIPTION: SEQ ID NO: 226:

ATATAGGACG GATAATCTGT TTACATTCTG TTCTTCTCGA TGCACTCACA AGCGGGTAAC 60

TAGGTGACAA GAAAACAAAG ATCTTATTCA AAAGAGGTCT TACAGCAACC CAACGTCTCA 120

TCTTCCCATA GTAAAGATGA CGGCGCCTTG AGGTAAGCTA CAGGCAACAC CACTTCCGCG 180

TTTCTCTTGC GCCCTGGTCC AAGATGGCGG ATGAAGCCAC GCGACGTGTT GTGTCTGAGA 240

TCCCGGTGCT GAAGACTAAC GCCGGACCCC GAGATCGTGA GTTGTGGGTG CAGCGACTGA 300

AGGAGGAATA TCAGTCCCTT ATCCGGTATG TGGAGAACAA CAAGAATGCT GACAACGATT 360

GGTTCCGACT GGAGTCCAAC AAGGAAGGAA CTCGGTGGTT TGGAAAATGC TGGTATATCC 420

ATGACCTCCT GAAATATGAG TTTGACATCG AGTTTGACAT TCCTATCACA TATCCTACTA 480

CTGCCCCAGA AATTGCAGTT CCTGAGCTGG ATGGAAAGAC AGCAAAGATG TACAGGGGTG 540

GCAAAATATG CCTGACGGAT CATTTCAAAC CTTTGTGGGC CAGGAATGTG CCCAAATTTG 600

GACTAGCTCA TCTCATGGCT CTGGGGCTGG GTCCATGGCT GGCAGTGGAA ATCCCTGATC 660

TGATTCAGAA GGGCGTCATC CAACACAAAG AGAAATGCAA CCAATGAAGA ATCAAGCCAC 720

TGAGGCAGGG CAGAGGGACC TTTGATAGGC TACGATACTA TTTTCCTGTG CATCACACTT 780

AACTCATCTA ACTGCTTCCC CGGACACCCT CCACCTCTAG TTGTTACTAA GTAGCTGCAG 840

TAGGCATTGC TGGGGAAGAA ACAAACACAC ACCAAACAGT ACTGCTACTT AGTTTCTAAG 900

GCTGCACAGG GAAGGGAAAG ACTGGGCTTT GGACAATCTA GAGGTAATTT ATATCCGCCC 960

CCAGGTGGAG CAACATGCGA TTCTGGAGGC ACGGGGGTAA CTGAAAGTGA GTACATATAG 1020

TCTTTCTGGT TTCTGGAGAT AACCCATCAA TAAAAGCTGC TTCCTCTGGG TAAAAAAAAG 1080

(2) INFORMATION FOR SEQ ID NO: 227:

(i) SEQUENCE CHARACTERISTICS:
(A) LENGTH: 1336 base pairs
(B) TYPE: nucleic acid
(C) STRANDEDNESS: double
(D) TOPOLOGY: linear

(xi) SEQUENCE DESCRIPTION: SEQ ID NO: 227:

TTGCATTCAC AATTACTGGG AGGCAGGCAG GGGCAGTTGC ATGCTGGGGG TGGCTGCATG 60

GSCTGCCASC TCTCCTGGGT TTGAAGGATG CGGTACASCT GCTTCAGCTG AGCAACGATG 120

TTATCCTTGA TGTCTGGGGT TGAGATCTGC AGGCGGACAC TGCCACTATC AAAGGATCGT 180

GTGAAATCAC CAGAAAACAT CTCGTAGATC ATCCGAGCCA CTACTGGAAT GACCTGAACC 240

AAGATGAGTT TCCTTTCCAA TGGTTTCCCA TCTGGCCATT CTTCCCCAAA GCATAAGTAG 300

ATCTCAAACG GTGGCTGCTT CTCTATCTGT CCTTTCTGGT GGGCAATGAG ATCGCTAAGG 360

AATGTTTCCA GACAAAATAG CTTGACCTTC TTTTGTCTCT CAATCAGGTT GGGAGCAACA 420

AGTGATGGGG CACATGGCCC AGACCAGTAC ACCTTGCACT GGCACAGYCT GATGGCATAA 480

ATGGCATGAC CGCTGACCTC CAGGATCAGT CCTCTGTCCA TGACGTCCAG CAGCTTGCTA 540

GTGAACAGCT TCTGCTTCTC ATTGGTAATA TGCTCAGGAC CTGGGAATTT GACCTGCTCC 600

AGNCTGACGG GACCAAAGAG CTCCTCCTGG TCAGGCATGG GACCCAGGTC CCCATAGAAG 660

AGTCGGCAGC CCTGAGGGTT GCTCACGGTC ATGGTCCTGC CCGTACTCCT TCCCACGGTA 720

CTGAAACTTG ATGTCCAGGT CAGTCATTGG GAGAGAGCTG ATCCACAGTT CTGGAGAGCT 780

ATAGAAGGRC TGTATAGGTG CCTGGGGWAC TTCCATCTCC AGGGGTTCAG TTTTGGGCCA 840

CACTGCCTCC GGSCTGCAGT TGCCCACACT GCAATTGCCC ACACTGGCTG GCGCCATGGG 900

AGAACCATTG ATGTTCAGGA AGGGGAAGGT GTCCTGGATG GGAACATGGT GCTGCGACTG 960

ATCCAGCTCA TCTTCCTCAT CTTCTTCATC CACATCATTA TCCTTCTCAT CCCAGGGAGC 1020

AGACCCTGTG GATCCTGGGT TAATGATCGA SCCCTGGGGC TGAGGGATGT CACACACTTG 1080

ATATATCTTC ACTGGGTTCA TGGGCACCTC CCTTGGTGCC ATCCATACAT CCAGGTTGAA 1140

TTCTCTGCTC TTATTGAGAG CACAGCGCAG CTGGGCCTTC CATTTAGCTG GGTCAGGGTC 1200

ATCCACCCCT TCCTGGTACT TCCCTGTCTC TACAGCCCAG GCCTTAAAAA TGGTATTTTC 1260

CTCTTCTTGT TGAGGGCTAT GCCGGGTGGC ATGTTTCCAG GGAATCTGGA AGCGTTTAGA 1320

GTCCCTGTGT AGCCAG 1336

(2) INFORMATION FOR SEQ ID NO: 228:

(i) SEQUENCE CHARACTERISTICS:
(A) LENGTH: 2043 base pairs
(B) TYPE: nucleic acid
(C) STRANDEDNESS: double
(D) TOPOLOGY: linear

(xi) SEQUENCE DESCRIPTION: SEQ ID NO: 228:

TCAGCTGGTC CCTTCCTTGT GTCCTGGGGG ACCTGCTGGC GGCCTCTTCC TGGGAGCCAT 60

GACCTCAGAC CCCACCCACA CTCCAGATCG AGACCCCTGC CTCCCCCCGG CAAATGTCCT 120

CCCGCTGCCT TGCAGCCTGC ACTTTGCACA TGCTCACCCC CAGCACAGTC CCACTGGCCC 180

CTCAMCTCCC CTTCCCTGAG CTCCTTCCCA AGGACTCCTG GTCACTGCCT GCTGTGCAKT 240

CAGAGGCCCA GGGTCCAGCA GCCCGGSGGG AACGGGTGCT GCCTSTTCCT CCAGTTAGCT 300

CCAGYTCAGG TCTGAGACCC GTGYTGAGTA AAGGTCTGAG CAMCGACCGT GCCCTCTGCC 360

CAGGGCTGGG TCCTGAGCAG CTGGTTTTCC TGCAGGAAGG TTGGAGCAAG CAAAGTCCTT 420

CTCTGCCCTC AGGGTCAGCT GCCCAGACTG GGGCGGATGC AGAGAGGCAG GTGGGCTGTG 480

GCTGGACTGG TCCGGAGCTG GCTTCCTTAC CAGAAAAGCC TCAGCCTTCC TCTGGAAGCA 540

TCCCCCGTTC TGGGCAAGGG GGAAGGGCTC CTTTAAGGGG TGTGCTTTCC CAGTGGGGAG 600

CAGTCTGGCC CTGCCCCCTA CTAAAGCCTC TGCTCTCAGC ACTTTCCCCC AAGTCCTTGT 660

AACTTGCTTG AAGGTGGGTT CTGGCTGCCA GCCAGTCCCT GGACAAACTC TCCTGCCCCT 720

TTTAAATTTC ACTCATTTTG TATAAACCCA GCAGGCTGGT GTTTACTTAG CCCTGTAGCT 780

TTTTTCATTT TTTCTTTCCG TCTTTCTTCT TGAGTTCACG GTTCAATATT GCCTCCTCGC 840

CCTGGTGAGG GGAGGTGCTG CTTTTCTGCC CCACCTGCCG GCTGGTTCCA GCAGCGCTGG 900

NGCCCAGCTG GGGGGCCGGG ATGGGGGCTT CTCTCTCTGG GAGGGGTGCA GGTGCCCTCC 960

CCAGGCTGGG AGGGTTCCTT CCCTAGCTCC CCATCTGCCC CCGCTGGTGA GAGTTGGGCT 1020

TCTTGGTCTT GGAACTCCCT GGCATTGGGA ACAGAGCATT TCCAGCATTT GTTGTTGTTG 1080

TTTTACTCAC CTAACCCTTA GAAAATGAAT GTTAGAAGGT GCCTGCCGAG GCGGGACAGA 1140

GTGTTTGCTC GCGCTGGAGA AGGCTCTGCT CAGCCCTGAG AGTCCCTTCC TGCCCCACCG 1200

ATACTGGCAC TTTAAAAAGG AAGCTGACCG CACAGTGTCC AGACGAATTG GCCCCCAGAA 1260

GATGGGGAGT TCTGTCCTGC CCTTCTGTGT CTGCGTGACC TCACCCAGCC TAGGAGGGAG 1320

GTGCATTCAG GGTAGATTTG CCTCTCATTC AAAGTTCTGG GGCTTTGGGY GGAAAACAGC 1380

CAGCTTTGGC GCTGTTGGGG AGACTCCTCC AGACCAGGAA CCCCAGAAGG AGACAGAGCC 1440

TGCCACATCC TCCCACGCCA GGCCCTGGGC CAGGGTGATT GGACTGAGAA TTTGGCCACA 1500

ACCAAATTGA TGCTGGCTGG AACCAGAGGC CAGAAAGCCT GGCCTTGTCC CCATGTGGGA 1560

GCCCTGTCCT CAGCCCTCTT GTCCCCTTGA GCTCAGTGAA TTCCCACCAG GTGCCCACAG 1620

CTCCTGGACT TCAAATTCTA TATATTGAGA GAGTTGGAGA GTATATCAGA GATATTTTTG 1680

GAAAGGAGTT GGTCTATGCA ATGTCAGTTT GGAATCTTCT TGAAAGTTTA ATGTTTTTAT 1740

TAGGAGATTT AAAGAAAATA AAGGTCTACA ATATCTTTAG GTTTTTTTTT TTTCCTGTTT 1800

ACCGCACAAA CTGACCACAT GGCATGTCTA TCAGGATGGA GGGTGTCCAT GTTCTCCTCT 1860

GTCTTTAGGG AGGTGATAAG GAGATGGSCG RAGGGGTGTT TTTTTCTTTG ACTCCCCTCC 1920

TTTCTAACAG AATGTTGCCA CCACTGCTTG AGTGGGCTGT GTTTGTTCCT CTGTCCCAGC 1980

TTCTGTTGTA GAAAATAACA TTGTTAGGGG AACTCAGGCT AGTGTCAGCG TCTTGGTTTG 2040

GGG 2043

(2) INFORMATION FOR SEQ ID NO: 229:

(i) SEQUENCE CHARACTERISTICS:
(A) LENGTH: 540 base pairs
(B) TYPE: nucleic acid
(C) STRANDEDNESS: double
(D) TOPOLOGY: linear

(xi) SEQUENCE DESCRIPTION: SEQ ID NO: 229:

TAAAAAGAAG CGGGAGAATC TGGGCGTCGC TCTAGAGATC GATGGGCTAG AGGAGAAGCT 60

GTCCCAGTGT CGGAGAGACC TGGAGGCCGT GAACTCCAGA CTCCACAGCC GGGAGCTGAG 120

CCCAGAGGCC AGGAGGTCCC TGGAGAAGGA GAAAAACAGC CTAATGAACA AAGCCTCCAA 180

CTACGAGAAG GAACTGAAGT TTCTTCGGCA AGAGAACCGG AAGAACATGC TGCTCTCTGT 240

GGCCATCTTT ATCCTCCTGA CGCTCGTCTA TGCCTACTGG ACCATGTGAG CCTGGCACTT 300

CCCCACAACC AGCACAGGCT TCCACTTGGC CCCTTGGTCA GGATCAAGCA GGCACTTCAA 360

GCCTCAATAG GACCAAGGTG CTGGGGTGTT CCCCTCCCAA CCTAGTGTTC AAGCATGGCT 420

TCCTGGCGGC CCAGGCCTTG CCTCCCTGGC CTGCTGGGGG GTTCCGGGTC TCCAGAAGGA 480

CATGGTGCTG GTCCCTCCCT TAGCCCAAGG GAGAGGCAWT AAAGACACAA AGCTGGAAAT 540

(2) INFORMATION FOR SEQ ID NO: 230:

(i) SEQUENCE CHARACTERISTICS:
(A) LENGTH: 448 base pairs
(B) TYPE: nucleic acid
(C) STRANDEDNESS: double
(D) TOPOLOGY: linear

(xi) SEQUENCE DESCRIPTION: SEQ ID NO: 230:

AATTGTGAAA TATTAGAATA TTGTTACTAT TTGACCCAAC TCAAAATCTC CATGGGAAAA 60

TACCTGTCGA TACCCACAGT ATTGTTGAAA ATAATCAGAT GCAGTATCAC AGCTGTGTCA 120

GACTCTAGTA CCAGTTGGGC AATCAAGGCA CAGCTAAAAA TTGAAAACAA AGATCTGGAC 180

AACAAAACAG CCAAAGGTGG GGGTCAAGAA GCTCTGACGT GTACCTAGCT GTAGAATGCT 240

ATGCACACGT GCCAGGTGTA GTGTGCATAT CCAGGAAAAA CTGCAGAGAG CCCCAGTCTT 300

CAMCTCTGGT TGACCATGAG CTCTGTGTAA GCAGGAAGTG AAGGCTAAGG CAGATTTAAG 360

CTCTGAAAGC ATTCCACAAC ATACACACAA ATCGTGCAAA GCATTAAGGA AATCTTGTTA 420

CTGCTAAGTG TTGCTGACCC AGGAACAA 448

(2) INFORMATION FOR SEQ ID NO: 231:

(i) SEQUENCE CHARACTERISTICS:
(A) LENGTH: 407 base pairs
(B) TYPE: nucleic acid
(C) STRANDEDNESS: double
(D) TOPOLOGY: linear

(xi) SEQUENCE DESCRIPTION: SEQ ID NO: 231:

GTATGCTGCC CCAAACCAAT ATGTGTGGCT GCCTTTWACC TGACTTCTCC AACATGTAGC 60

CCCAAGAGGA GGCCTCTAGA CTRAGGGAGG GGCTGGTGAC CCAGGTGTGG TGGGGCTGCA 120

TGARACTACC AGAGAGACAG ACATTCTGGA ACTCACCCTG GGGGATCCAG TGGATCTGCC 180

TATGGTCTGG TCCACCCCAG ACCTGTGAGA TGTTCCTCAT GAGGATGCAC TTGTGCTTCT 240

GCAAGTATTG CTGCAGCTTC ATAGTGACTC CCACCAGCAC CAGCAATACA GYTAGCTACC 300

TGTGGCCTTG GATCTCAGCC AGCATGGCTG GGAGAGGGAG CARCTGGGCA TGTACCCTAA 360

ATGCTGTTAC CAGGGAAGGA CTCCCAGAGT GAAGACAAGT AGGGACT 407

(2) INFORMATION FOR SEQ ID NO: 232:

(i) SEQUENCE CHARACTERISTICS:
(A) LENGTH: 830 base pairs
(B) TYPE: nucleic acid
(C) STRANDEDNESS: double
(D) TOPOLOGY: linear

(xi) SEQUENCE DESCRIPTION: SEQ ID NO: 232:

GTATTTGATT TCAGGCTGCT AAATGGGCTC ATTTAGCATT CATTCCTTGA TGTAGACATT 60

AAAAAAAAAA CTGAATAGCA TTCTTTCCAG GNTAACTAAT AAAGCAGACA TGCTAAGCCT 120

ATAAATACAT CAGCACTGCA GCACACGTTT AAGGTTGCCA CGGACAAGGA TCACACAATA 180

GAGAACACTG TAGTTCGGTC TGCTCACAAG ACCCAGAACA TTGATCAGTT TTTGTTGTTG 240

GTTTATTATT TTTCTGTTAA AAAATTGTGA AAAGTTTGTT TTAGCTAGAT GATATTTTAA 300

TAGCTGCGAG TGCTTTGGAA CTATAAAGAT GTCACTACTT AACACACATA CCTTATGTTT 360

TGTTTTGTTT TGTTTTACAC TCAGTATAAA TCAGGAGAAG TTAGCCAACC ATCTAGCATT 420

TAGAATCCTC TTTTTTATTG TCTTCTAAGG ATATGGATGT TCCCATAACA GCAACAAAAC 480

AGCAACAAAA ACATTTCATA AATATCACTT GATAGACTGT AAGCACCTGC TTAACTTTGT 540

GTNCCAAATA TTTAGTGTGT ATATATATAT ATATATACAC ACACACACAC ATATATATTC 600

AACAAATAAA GCAAAATATA ACATGCATTT CACATTTTGT CTTTCCCTGT TACGATTTTA 660

ATAGCAGAAC TGTATGACAA GTTTAGGTGA TCCTAGCATA TGTTAAATTC AAATTAATGT 720

AAAACAGATT AACAACAACA AAGAAACTGT CTATTTGAGT GAAGTCATGC TTTCTATTAT 780

AATAACTTGG CTTCGGTTAT CCATCAAATG CACACTTATA CTGTTATCTG 830

(2) INFORMATION FOR SEQ ID NO: 233:

(i) SEQUENCE CHARACTERISTICS:
(A) LENGTH: 932 base pairs
(B) TYPE: nucleic acid
(C) STRANDEDNESS: double
(D) TOPOLOGY: linear

(xi) SEQUENCE DESCRIPTION: SEQ ID NO: 233:

CCAGAAGAAA GACCAATCTA GAATATGGAA CTCTAATCAC TTCTAGTATT TCAACTTCCT 60

AGCAGAAATG AACTTGGCCC TAGACCTAGG GGATAAGCAA TGTTCTTTAT GTAGCCAATG 120

CTACGGAAAC AAAAGAGGTG AAAGAGACCC TTTTTTTATA CTTAATGTAC ATATATTGAC 180

TTTTTGAGCA AGAATGCCAG AAATAGCCTT CATTTCTACC CTGCAAAATA ATCCAGATCT 240

GCTTTCTAAA ATGRANTCAG TTTCTAAAGT GAAACATGCA ATATTTATGC TCTGACTGAC 300

TCCTGAATTG GARGAGGAAG RACTTCTGTT TACAGAAAAC YGTATTGTTA TATATGTCAG 360

GCTGTGTATT GTGACTATCA GCATTCTGGT GCAAATGAAC TTTTCTCCAT CATCGACTGT 420

GGAAAATTGA TACTTTTAAA GCATATTCTT CTATGAGCAC AGGTCCTCCT AGTGAAACTT 480

AATTTGACAA AGGGTGTCAT ATGCTTTCCT AACCTGAWTT GTATTAACAT TCACAGAGCC 540

TACATTTTCT CATTAGGGTT RTGATGCTCA GTATCTTTCC AAGTGCCAGG CAGRGCTTNC 600

CTTTTCTGAT CAAACATACC ATTTTTTGTA TTTCACAACT ATAGACAGTC ACTTCTGCAG 660

TCCCAATTTA AAAATGCAGA ACTGCTTTAT CCAAGAATGC TGAAAAATAC TGTTCTATCC 720

AGGTTTCCTA AACTATAAAA GCAGATTTTG CTTTTGTTTG TTAATCATAG GCATGGCCGA 780

GCATTGTGGA TTAGCCTGAG GCTTAAAATC AGATGCATGT CTGGTAAGAT GACCACTGTC 840

TCACTATCAA GAGCCTGCAG AGCCATTTTC CAGACCTGTG ATTGCCCAGA ACACATAGTC 900

CCCACGTTTC TAATTTGGAG CAAATCTAAA AG 932

(2) INFORMATION FOR SEQ ID NO: 234:

(i) SEQUENCE CHARACTERISTICS:
(A) LENGTH: 2786 base pairs
(B) TYPE: nucleic acid
(C) STRANDEDNESS: double
(D) TOPOLOGY: linear

(xi) SEQUENCE DESCRIPTION: SEQ ID NO: 234:

TTAGCAGGGT GAGCTGTTAA AACAGCACAC ATCTCTCATC CCCTCTTCCT TTATTCCCCC 60

CTGGGTTTCA GAAAGGAAGG ATATATGGGG ACCACCTCCC CCTTCTTTGA TCCCAGCATC 120

TCAGTCCCCC TCCCAACCCT CCATATGGCT CTCAATGGTG CTCACTTGCT TGGAAGCAGG 180

CTCCCAATAG GGAGGGGSCT GCCCTCTACA GTCTCTTTGA CTGTAAGACA GGGCTCTGTA 240

TCAGTGAGAC GATGAGAAAA GTCCCAGGCT AATGGCAGAA ATTTGCACTT TGAACATGTG 300

TGTTTTTGTG TTGTGGAACC TGAGATTCCT TATTTATTAA CAGGAAGTCT GATTTTTTTT 360

TTTTGGAGTC TTTGTTGCTA TATTTTGTGG GGCTGGGAGA GAGAGATTAG ATTATTTTGA 420

CATGGGATCC CTTCCATAAC AGGTACTTTG AAGGCAAGAC ATAGGGTTGA AGAAGCACAA 480

CCAGCCTCTG AAATCATAGC TCTCCAGTGG CTTTTAAAGA AAGCTGGTCC TCAGCACTAA 540

CAAAATCACT ACAATAGCCT AGTGCTTTTT TGGAAGCCTT TTTAGGGAAG AATGTTAGGT 600

TCATGGTAAC TAGTATGCTC TTTGAGATTT TTACAGTGTT GAAACTTAAG AATTTTGAGA 660

GGGTGAGGAG GGTTGTTCAG AATCTAAATT ACAGATAGAT GATTGTTTCT TGTGAATTTG 720

TTTCTTTTCC TTTTTTTTTG TCCCTACCAT TTCCTTACAT TTCCCTTGGG GCCCATCTCT 780

GGCTCCTTGC TTTTTGTTTC TTGCTTTGCT TTATCAGTTC ATTCCAGCTC CCTGTTAGTG 840

AAGGACACTG CTGTTAGTGA AGGAACAAAG TCTATGAGTC CTAAAATTTT AAGTCAAAGA 900

AAACTGCTCT GTTTCCCCTT TAGTAACACT TCTGAAGAGG AAAAACTTCA ATAGCCAAAG 960

TTAATAATCC TATATAATAA TTGCTTTGGC TTTCACCTAA AATTCTGGGC ATCACAATTT 1020

CCTTGGGATA GAGGTTGTGT TGGGGAATAG ATTGCTTATT GCTGTTCACT GGAGAGAAAA 1080

GGTAGTGTTT TTGTACAAGG TCATACCGCC AGAAGCCCCA AATCCTATTT TGGCTCATCT 1140

TCAGGTAAAG AGTAATTCCT ATCCTGTGTG CCTCAGAAGC TAGAATCGAA GGCTTACCCT 1200

ATTCATTGTT TATTGTCAGA AATGCATGAT GGCTCTTGGA AAGAATGACG TTTTGCTGGA 1260

AAAAAAAAAA AGAACAGTTT GTGTTTCACA AACATGGCTT ATCAATTTTT TCAAAGAATT 1320

CTTTTTTCCC AAAAAGAGGA GTAACAAAAT GTCATTTCTG AAAGAGGCTT ACTTTATACC 1380

AACTAGTGTC AGCATTTGGG ATGCCAGGGA ACAGAGAGTG AGACACCTAC AATCACCAGT 1440

CTCAAATGCG CTATTGTTTC TTTTCAGAGT GTTGCAGATT TGCCATTTCT CCATAATATG 1500

GGGATAGAAA ATGGAATAAA GATAGAAGGG ATGTAGAATA TGCTTTCCTG CCAACATGGT 1560

TTGGAGTCGA CTTTGGTATA TTGACTAGAT TTGAAAATAC AAGATTGATT AGATGAATCT 1620

ACAAAAAAGT TGTCCTCCTC TCAGGTCCCT TTTACACTTT TTGACTAACT AGCATCTATA 1680

TTCCACACTT AGCTTTTTTG TCACACTTAT CCTTTGTCTC CGTAAATTTC ATTTGCAGTG 1740

GTTAGTCATC AGATATTTTA GCCACCTACA CAAAAGCAAA CTGCATTTTT AAAAATCTTT 1800

CTGAGATGGG AGAAAATGTA TTCTCCTTTC CTATACCGCT CTCCCAACAA AAAAACAACT 1860

AGTTAGTTCT ACTAATTAGA AACTTGCTGT ACTTTTTCTT TTCTTTTAGG GGTCAAGGAC 1920

CCTCTTTATA GCTACCATTT GCCTACAATA AATTATTGCA GCAGTTTGCA ATACTAAAAT 1980

ATTTTTTATA GACTTTATAT TTTTCCTTTT GATAAAGGGA TGCTGCATAG TAGAGTTGGT 2040

GTAATTAAAC TATCTCAGCC GTTTCCCTGC TTTCCCTTCT GCTCCATATG CCTCATTGTC 2100

CTTCCAGGGA GCTCTTTTAA TCTTAAAGTT CTACATTTCA TGCTCTTAGT CAAATTCTGT 2160

TACCTTTTTA ATAACTCTTC CCACTGCATA TTTCCATCTT GAATTGGTGG TTCTAAATTC 2220

TGAAACTGTA GTTGAGATAC AGCTATTTAA TATTTCTGGG AGATGTGCAT CCCTCTTCTT 2280

KGTGGTTGCC CAAGGTTGTT TTGCGTAACT GAGACTCCTT GATATGCTTC AGAGAATTTA 2340

GGCAAACACT GGCCATGGCC GTGGGAGTAC TGGGAGTAAA ATAAAAATAT CGAGGTATAG 2400

ACTAGCATCC ACATAGAGCA CTTGAACCTC CTTTGTACCT GTTTGGGGAA AAAGTATAAT 2460

GAGTGTACTA CCAATCTAAC TAAGATTATT ATAGTCTGGT TGTTTGAAAT ACCATTTTTT 2520

TCTCCTTTTG TGTTTTTCCC ACTTTCCAAT GTACTCAAGA AAATTGAACA AATGTAATGG 2580

ATCAATTTAA AATATTTTAT TTCTTAAAAG CCTTTTTTGC CTGTTGTAAT GTGCAGGACC 2640

CTTCTCCTTT CATGGGAGAG ACAGGTAGTT ACCTGAATAT AGGTTGAAAA GGTTATGTAA 2700

AAAGAAATTA TAATAAAAGG GATACTTTGC TTTTCAAATC TTTGTTTTCT CTTATTCTAG 2760

GTAAGGCATA TTAAAAATAA ATATGT 2786

(2) INFORMATION FOR SEQ ID NO: 235:

(i) SEQUENCE CHARACTERISTICS:
(A) LENGTH: 458 base pairs
(B) TYPE: nucleic acid
(C) STRANDEDNESS: double
(D) TOPOLOGY: linear

(xi) SEQUENCE DESCRIPTION: SEQ ID NO: 235:

GGGTGCAGGA ATTCGGCACG AGAGAATGTT TGATTTTCTT TCCTATTTTA AGGATCTTCT 60

CTCTTGTTGA TGTTGAAAAC TTACCTTAGT GAAGATGTGT TTCAACATGC TGTTGTCCTT 120

TACCTGCATA ATCACAGCTA TGCATCTATT CAAAGTGATG ATCTGTGGGA TAGTTTTAAT 180

GAGGTCACAA ACCAAACACT AGATGTAAAG AGAATGATGA AAACCTGGAC CCTGCAGAAA 240

GGATTTCCTT TAGTGACTGT TCAAAAGAAA GGAAAGGAAC TTTTTATACA ACAAGAGAGA 300

TTCTTTTTAA ATATGAAGCC TGAAATTCAG CCTTCAGATA CAAGGTACAT GCCCTCTTTC 360

TTTTCATGCC ATCTCTTTTG CACTCTCAGG TGGAAATATT TTTAAGTGTT TTATAATCAT 420

AAGTTCTTGT GAAACCTAAC AAGATTATCC CTTCCTAA 458

(2) INFORMATION FOR SEQ ID NO: 236:

(i) SEQUENCE CHARACTERISTICS:
(A) LENGTH: 591 base pairs
(B) TYPE: nucleic acid
(C) STRANDEDNESS: double
(D) TOPOLOGY: linear

(xi) SEQUENCE DESCRIPTION: SEQ ID NO: 236:

AGGATGAAGA GGAAATTATC TCTTGGATTG CTCTCCAGGA AATCCTTCTC TATACTTTAA 60

AAGCTCTTGT TCTTTTCTAG GARTCCAATG TGCTGATTGC TGCTAACAGT CAGGGTACAA 120

TTAAGGTGCT AGAATTGGTA TGAAGGGTTA ACTCAAGTCA AATTGTACTT GATCCTGCTG 180

AAATACATCT GCAGCTGACA ATGAGAGARG AAACAGAAAA TGTCATGTGA TGTCTCTCCC 240

CAAAGTCATC ATGGGTTTTG GATTTGTTTT GAATATTTTT TCTTTTTTTC TTKTCCCTCC 300

TTTATGAGCC TTTGGGACAT TGGGAATACC CAGCCAACTC TCCACCATCA ATGTAACTCC 360

ATGGACATTG CTGCTCTTGG TGGTGTTATC TAATTTTTGT GATAGGGAAA CAAATTCTTT 420

TGAATAAAAA TAAATAACWA AACAATAAAA GTTTATTGAG CCACAGTTGA GCTTGGAAAG 480

TTTTTGTCAA ATGCNGCAAG AGATAACTCT TTTTANGAAG TAGCATATGT GAACTATAAT 540

GTAACAGTGA ATAATTTGTA AAGTTCGTAT TTCCCAACCT CTTTGGGAAT T 591

(2) INFORMATION FOR SEQ ID NO: 237:

(i) SEQUENCE CHARACTERISTICS:
(A) LENGTH: 1286 base pairs
(B) TYPE: nucleic acid
(C) STRANDEDNESS: double
(D) TOPOLOGY: linear

(xi) SEQUENCE DESCRIPTION: SEQ ID NO: 237:

TCTTTTTAAG GTACAGCAGG GAAGAACTGG AAACTCAGAG AAAGAAACTG CCCTTCCATC 60

TACAAAAGCT GAGTTTACTT CTCCTCCTTC TTTGTTCAAG ACTGGGCTTC CACCGAGCAG 120

GAGATTACCT GGGGCAATTG ATGTTATCGG TCAGACTATA ACTATCAGCC GAGTAGAAGG 180

CAGGCGACGG GCAAATGAGA ACAGCAACAT ACAGGTCCTT TCTGAAAGAT CTGCTACTGA 240

AGTAGACAAC AATTTTAGCA AACCACCTCC GTTTTTCCCT CCAGGAGCTC CTCCCACTCA 300

CCTTCCACCT CCTCCATTTC TTCCACCTCC TCCGACTGTC AGCACTGCTC CACCTCTGAT 360

TCCACCACCG GGTTTTCCTC CTCCACCAGG CGCTCCACCT CCATCTCTTA TACCAACAAT 420

AGAAAGTGGA CATTCCTCTG GTTATGATAG TSGTTCTGCA CGTGCATTTC CATATGGCAA 480

TGCGATGAAG AACGATACAG ATACAGGGAA TATGCAGAAA GAGGTTATGA GCGTCACAGA 540

GCAAGTCGAG AAAANGAAGA ACGACATAGA GAAAGACGAC ACAGGGAGAA AGAGGAAACC 600

AGACATAAGT CTTCTCGAAG TAATAGTAGA CGTCGCCATG AAAGTGAAGA AGGAGATAGT 660

CACAGGAGAC ACAAACACAA AAAATCTAAA AGAAGCAAAG AAGGAAAAGA AGCGGGCAGT 720

GAGCCTGCCC CTGAACAGGA GAGCACCGAA GCTACACCTG CAGAATAGGC ATGGTTTTGG 780

CCTTTTGTGT ATATTAGTAC CAGAAGTAGA TACTATAAAT CTTGTTATTT TTCTGGATAA 840

TGTTTAAGAA ATTTACCTTA AATCTTGTTC TGTTTGTTAG TATGAAAAGT TAACTTTTTT 900

TCCAAAATAA AAGAGTGAAT TTTTCATGTT AAGTTAAAAA TCTTTGTCTT GTACTATTTC 960

AAAAATAAAA AGACAGCAAT GACTTTATAT CCAAGAAAGG AATGTGAATG AGTCACTTAA 1020

CAGGGAATCT AAAGAGCTGT GTTAGCTGTG TACATACACA GATTATCTGA GAAAAGGTCA 1080

AGGGTTCCAC TTGGGCCACA GTTTTTTTGT TAATCAAACA CCACTCTCTT AAGRGGCTGC 1140

ATCACAAARG GCAACCAARG GGCCCCTCTT ARGGCTTTGA GGATTAAAAC TAGTCTTTAT 1200

CCATTACTGC TGTGGACACT CTTGGCTTRG TATWTTTAGG GGGGNTCCTT ACCTTTTTTT 1260

GGTTTTCCNC ACCTTTTTGG TTGGGC 1286

(2) INFORMATION FOR SEQ ID NO: 238:

(i) SEQUENCE CHARACTERISTICS:
(A) LENGTH: 734 base pairs
(B) TYPE: nucleic acid
(C) STRANDEDNESS: double
(D) TOPOLOGY: linear

(xi) SEQUENCE DESCRIPTION: SEQ ID NO: 238:

ATGGCAGCGC AGAAGGACCA GCAGAAAGAT GCCGAGGCGG AAGGGCTGAG CGGCACGACC 60

CTGCTGCCGA AGCTGATTCC CTCCGGTGCA GGCCGGGAGT GGCTGGAGCG GCGCCGCGCG 120

ACCATCCGGC CCTGGAGCAC CTTCGTGGAC CAGCAGCGCT TCTCACGGCC CCGCAACCTG 180

GGAGAGCTGT GCCAGCGCCT CGTACGCAAC GTGGAGTACT ACCAGAGCAA CTATGTGTTC 240

GTGTTCCTGG GCCTCATCCT GTACTGTGTG GTGACGTCCC CTATGTTGCT GGTGGCTCTG 300

GCTGTCTTTT TCGGCGCCTG TTAACATTCT CTATCTGCGC ACCTTGGAGT CCAAGCTTGT 360

CCTCTTTGGC CGAAAGGTGA GCCCAGCGCA TCATATGCTC TGGCTGGAGG CATCTCCTTC 420

CCCTTCTTCT GGCTGGCTGG TGCGGGCTCG GCCGTCTTCT GGGTGCTGGG AGCCACCCTG 480

GTGGTCATCG GCTCCCACGC TGCCTTCCAC CAGATTGAGG CTGTGGACGG GGAGGAGCTG 540

CAGATGGAAC CCGTGTGAGG TGTCTTCTGG GACCTGCCGG CCTCCCGGGC CAGCTGCCCC 600

ACCCCTGCCC ATGCCTGTCC TGCACGGTCT GCTGCTCGGG CCCACAGCGC CGTCCCATCA 660

CAAGCCCGGG GAGGGATCCC GCCTTTGAAA ATAAAGCTGT TATGGGTGTC ATTCAAAAAA 720

AAAAAAAAAA AAAA 734

(2) INFORMATION FOR SEQ ID NO: 239:

(i) SEQUENCE CHARACTERISTICS:
(A) LENGTH: 809 base pairs
(B) TYPE: nucleic acid

(C) STRANDEDNESS: double
(D) TOPOLOGY: linear

(xi) SEQUENCE DESCRIPTION: SEQ ID NO: 239:

CGGGGTCTTC AGGGTACCGG GCTGGTTACA GCAGCTCTAC CCCTCACGAC GCARACATGG 60

CAGCGCAGAA GGACCAGCAG AAAGATGCCG AGGCGGAAGG GCTGAGCGGC ACGACCCTGC 120

TGCCGAAGCT GATTCCCTCC GGTGCAGGCC GGGAGTGGCT GGAGCGGCGC CGCGCGACCA 180

TCCGGCCCTG GAGCACCTTC GTGGACCAGC AGCGCTTCTC ACGGCCCCGC AACCTGGGAG 240

AGCTGTGCCA GCGCCTCGTA CGCAACGTGG AGTACTACCA GAGCAACTAT GTGTTCGTGT 300

TCCTGGGCCT CATCCTGTAC TGTGTGGTGA CGTCCCCTAT GTTGCTGGTG GCTCTGGCTG 360

TCTTTTTCGG CGCCTGTTAC ATTCTCTATC TGCGCACCTT GGAGTCCAAG CTTGTGCTCT 420

TTGGCCGAGA GGTGAGCCCA GCGCATCAGT ATGCTCTGGC TGGAGGCATC TCCTTCCCCT 480

TCTTCTGGCT GGCTGGTGCG GGCTCGGCCG TCTTCTGGGT GCTGGGAGCC ACCCTGGTGG 540

TCATCGGCTC CCACGCTGCC TTCCACCAGA TTGAGGCTGT GGACGGGGAG GAGCTGCAGA 600

TGGAACCCGT GTGAGGTGTC TTCTGGGACC TGCCGGCCTC CCGGGCCAGC TGCCCCACCC 660

CTGCCCATGC CTGTCCTGCA CGGCTCTGCT GCTCGGGCCC ACAGCGCCGT CCCATCACAA 720

GCCCGGGGAG GGATCCCGCC TTTGAAAATA AAGCTGTTAT GGGTGTCATT CAGGAAAAAA 780

AAAAAAAAAA AAAAAAAAAA AAAAAAAAA 809

(2) INFORMATION FOR SEQ ID NO: 240:

(i) SEQUENCE CHARACTERISTICS:
(A) LENGTH: 2201 base pairs
(B) TYPE: nucleic acid
(C) STRANDEDNESS: double
(D) TOPOLOGY: linear

(xi) SEQUENCE DESCRIPTION: SEQ ID NO: 240:

TCGACCCACG CGTCCGGCAA CATGGCGGCT GCCGTGGTGC AGCGCCCGGG CTGAGCGACA 60

GCAAGTGCAG CGGGCTCCTA CCCCGGGTGA GGGGTGGCCT CCGCGTGGGA TCGTGCCCTC 120

TTCAGCCCGC TCCTGTCCCC GACATCACGT GTATTCCGCA CGTCCCCTCC GCGCTGTGTG 180

TCTACTGAGA CGGGGAGGCG TGACAGGGCC CGGGTCCCTT CTCAGTGGTG CTCTGTGCTT 240

CAGGGCAAGC TCCCCGTCTC CGGGCGCACT TCCCTCGCCT GTGTTCGGTC CATCCTCCTT 300

TCTCCAGCCT CCTCCCCTCG CAGGCGGATG AMCCGGACGA CGGGCCAGTG CCTGGCACCC 360

CGGGGTTGCC ARGGTCCAMG GGGAACCCGA AGTCCGAGGA GCCCGARGTC CCGAACCAGG 420

ARGGGCTGCA GCGCATCAMC GGCCTGTCTC CCGGCCGTTC GGCTCTCATA GTGGCGGTGC 480

TGTGCTACAT CAATCTCCTG AACTACATGG ACCGCTTCAC CGTGGCTGGC GTCCTTCCCG 540

ACATCGAGCA GTTCTTCAAC ATCGGGGACA GTAGCTCTGG GCTCATCCAG ACCGTGTTCA 600

TCTCCAGTTA CATGGTGTTG GCACCTGTGT TTGGCTACCT GGGTGACAGG TACAATCGGA 660

AGTATCTCAT GTGCGGGGGC ATTGCCTTCT GGTCCCTGGT GACACTGGGG TCATCCTTCA 720

TCCCCGGAGA GCATTTCTGG CTGCTCCTCC TGACCCGGGG CCTGGTGGGG GTCGGGGAGG 780

CCAGTTATTC CACCATCGCG CCCACTCTCA TTGCCGACCT CTTTGTGGCC GACCAGCGGA 840

CCGGATGCTC AGCATCTTCT ACTTTGCCAT TCCGGTGGGC AGTGGTCTGG GCTACATTGC 900

AGGCTCCAAA GTGAAGGATA TGGCTGGAGA CTGGCACTGG GCTCTGAGGG TGACACCGGG 960

TCTAGGAGTG GTGGCCGTTC TGCTGCTGTT CCTGGTAGTG CGGGAGCCGC CAAGGGGAGC 1020

CGTGGAGCGC CACTCAGATT TGCCACCCCT GAACCCCACC TCGTGGTGGG CAGATCTGAG 1080

GGCTCTGGCA AGAAATCCTA GTTTCGTCCT GTCTTCCCTG GGCTTCACTG CTGTGGCCTT 1140

TGTCACGGGC TCCCTGGCTC TGTGGGCTCC GGCATTCCTG CTGCGTTCCC GCGTGGTCCT 1200

TGGGGAGACC CCACCCTGCC TTCCCGGAGA CTCCTGCTCT TCCTCTGACA GTCTCATCTT 1260

TGGACTCATC ACCTGCCTGA CCGGAGTCCT GGGTGTGGGC CTGGGTGTGG AGATCAGCCG 1320

CCGGCTCCGC CACTCCAACC CCCGGGCTGA TCCCCTGGTC TGTGCCACTG GCCTCCTGGG 1380

CTCTGCACCC TTCCTCTTCC TGTCCCTTGC CTGCGCCCGT GGTAGCATCG TGGCCACTTA 1440

TATTTTMATC TTCATTGGAG AGACCCTCCT GTCCATGAAC TGGGCCATCG TGGCCGACAT 1500

TCTGCTGTAC GTGGTGATCC CTACCCGACG CTCCACCGCC GAGGCCTTCC AGATCGTGCT 1560

GTCCCACCTG CTGGGTGATG CTGGGAGCCC CTACCTCATT GGCCTGATCT CTGACCGCCT 1620

GCGCCGGAAC TGGCCCCCCT CCTTCTTGTC CGAGTTCCGG GCTCTGCAGT TCTCGCTCAT 1680

GCTCTGCGCG TTTGTTGGGG CACTGGGCGG CGCACTTTCC TGGGCACCGC CATCTTCATT 1740

GAGGCCGACC GCCGGCGGGC ACAGCTGCAC GTGCAGGGCC TGCTGCACGA AGCAGGGTCC 1800

ACAGACGACC GGATTGTGGT GCCCCAGCGG GGCCGCTCCA CCCGCGTGCC CGTGGCCAGT 1860

GTGCTCATCT GAGARGCTGC CGCTCACCTA CCTGCACATC TGCCACAGCT GGCCCTGGGC 1920

CCACCCCACG AAGGGCCTGG GCCTAACCCC TTGGCCTGGC CCAGCTTCCA GAGGGACCCT 1980

GGGCCGTGTG CCAGCTCCCA GACACTACMT GGGTAGCTCA GGGGAGGAGG TGGGGGTCCA 2040

GGAGGGGGAT CCCTCTCCAC AGGGGCAGCC CCAAGGGCTC GGTGCTATTT GTAACGGAAT 2100

AAAATTTGTA GCCAGACCCC AGGTGCCTGC TCTCGTCTTT CTCTGGGTGG CCTCTGATCT 2160

TGCACCCCGT CTTCACCCCA GGGCTCCTGA AGACTGTGGG T 2201

(2) INFORMATION FOR SEQ ID NO: 241:

(i) SEQUENCE CHARACTERISTICS:
(A) LENGTH: 1661 base pairs
(B) TYPE: nucleic acid
(C) STRANDEDNESS: double
(D) TOPOLOGY: linear

(xi) SEQUENCE DESCRIPTION: SEQ ID NO: 241:

GTCCTTCCCG ACATCGAGCA GTTCTTCAAC ATCGGGGACA GTAGCTCTGG GCTCATCCAG 60

ACCGTGTTCA TCTCCAGTTA CATGGTGTTG GCACCTGTGT TTGGCTACCT GGGTGACAGG 120

TACAATCGGA AGTATCTCAT GTGCGGGGGC ATTGCCTTCT GGTCCCTGGT GACACTGGGG 180

TCATSCTTCA TCCCCGGAGA GCATTTCTGG CTGCTCCTCC TGACCCGGGG CCTGGTGGGG 240

GTCGGGGAGG CCAGTTATTC CACCATCGCG CCCACTCTCA TTGCCGACCT CTTTGTGGCC 300

GACCAGCGGA SCGGATGCTC AGCATCTTCT ACTTTGCCAT TCCGGTGGGC AGTGGTCTGG 360

GCTACATTGC AGGCTCCAAA GTGAAGGATA TGGCTGGAGA CTGGCACTGG GCTCTGAGGG 420

TGACACCGGG TCTAGGAGTG GTGGCCGTTC TGCTGCTGTT CCTGGTAGTG CGGGAGCCGC 480

CAAGGGGAGC CGTGGAGCGC CACTCAGATT TGCCACCCCT GAACCCCACC TCGTGGTGGG 540

CAGATYTGAG GGCTCTGGCA AGAAATCCTA GTTTCGTCCT GTCTTCCCTG GGCTTCACTG 600

CTGTGGCCTT TGTCACGGGC TCCCTGGCTC TGTGGGCTCC GGCATTCCTG CTGCGTTCCC 660

GCGTGGTCCT TGGGGAGACC CCACCCTGCC TTCCCGGAGA CTCCTGCTCT TCCTCTGACA 720

GTCTCATCTT TGGACTCATC ACCTGCCTGA CCGGAGTCCT GGGTGTGGGC CTGGGTGTGG 780

AGATCAGCCG CCGGYTCCGC CACTCCAACC CCCGGGCTGA TCCCCTGGTC TGTGCCACTG 840

GCCTCCTGGG CTCTGCACCC TTCCTCTTCC TGTCCCTTGC CTGCGCCCGT GGTAGCATCG 900

TGGCCACTTA TATTTTCATC TTCATTGGAG AGACCCTCCT GTCCATGAAC TGGGCCATCG 960

TGGCCGACAT TCTGCTGTAC GTGGTGATCC CTACCCGACG CTCCACCGCC GAGGCCTTCC 1020

AGATCGTGCT GTCCCACCTG CTGGGTGATG CTGGGAGCCC CTACCTCATT GGCCTGATCT 1080

CTGACCGCCT GCGCCGGAAC TGGCCCCCCT CCTTCTTGTC CGAGTTCCGG GCTCTGCAGT 1140

TCTCGCTCAT GCTCTGCGCG TTTGTTGGGG CACTGGGCGG CGCACTTTCC TGGGCACCGN 1200

CATCTTCATT GAGGCCGACC GCCGGCGGGC ACAGCTGCAC GTGCAGGGCC TGCTGCACGA 1260

AGCAGGGTCC ACAGACGACC GGATTGTGGT GCCCCAGCGG GGCCGCTCCA CCCGCGTGCC 1320

CGTGGCCAGT GTGCTCATCT GAGAGGCTGC CGCTCACCTA CCTGCACATC TGCCACAGCT 1380

KGCCCTGGGC CCACCCCACG AAGGGCCTGG GCCTAACCCC TTGGCCTGGC CCAGCTTCCA 1440

GAGGGACCCT GGGCCGTGTG CCAGCTCCCA GACACTACMT GGGTAGCTCA GGGGAGGAGG 1500

TGGGGGTCCA GGAGGGGGAT CCCTCTCCAC AGGGGNCACC CCAAGGGCTC GGTGCTATTT 1560

GTAACGGAAT AAAATTTGTA GCCAGACCCC AGGTGCCTGC TCTCGTCTTT CTCTGGGTGG 1620

CCTCTGATCT TGCACCCCGT CTTCACCCCA GGGCTCCTGA A 1661

(2) INFORMATION FOR SEQ ID NO: 242:

(i) SEQUENCE CHARACTERISTICS:
(A) LENGTH: 1146 base pairs
(B) TYPE: nucleic acid
(C) STRANDEDNESS: double
(D) TOPOLOGY: linear

(xi) SEQUENCE DESCRIPTION: SEQ ID NO: 242:

NGACAGAAAA GCAGAAGATG AGACTCTGTT CATTCACTTT TCCTAGGCCC ATCCTGTGGT 60

CATCTTTCCC CCTCCCATCA TACCTCCTCC TTCCTGGAGC CTCTGCCGGC TTGGCTGTAA 120

TGGTGGCACT TACCTGGATA TTTCAGTGGG AGGATGAAAG GCGAGACTCA CCCTACGCGG 180

TGGGACAGAT GGGGAGAGGA AAAAGGCAGA GATNGCCAGG AGAGGGGTGC AGGACAAACC 240

AGAGAGGTTG GGTCAGGGGA AAAGTGTNGG GAGAAAGTGG GGTGCAGGCC CTGCAGGCCG 300

GTTTAGCCAG CAGCTGCGGC CTCCCCGGGC CCTTGGCATC CAACTTCGCA GACAGGGTAC 360

CAGCCTCCTG GTGTGTATCA TAGGATTTGT TCACATAGTG TTATGCATGA TCTTCGTAAG 420

GTTAAGAAGC CGTGGTGGTG CACCATGACA TCCAACCCGT ATATATAAAG ATAAATATAT 480

ATATATATGT ATGTAAATTA TAGCACTGAG GGCCCTGCTG CCCTGCTGGA CCAAGCAAAA 540

CTAAGCCTTT TGGTTTGGGT ATTATGTTTC GTTTTGTTAT TTGTTTGTTT TTGTGGCTTG 600

TCTTATGTCG TGATAGCACA AGTGCCAGTC GGATTGCTCT GTATTACAGA ATAGTGTTTT 660

TAATTCATCA ATGTTCTAGT TAATGTCTAC CTCAGCACCT CCTCTTAGCC TAATTTTAGG 720

AGGTTGCCCA ATTTTGTTTC TTCAATTTTA CTGGTTACTT TTTTGTACAA ATCAATCTCT 780

TTCTCTCTTT CTCTCCTCCC CACCTCTCAC CCTTGCCCTC TCCATCTCCC TCTCCCGCCC 840

TCCCCTCCTC CCTCTGGCTC CCCGTCTCAT TTCTGTCCAC TCCATTCTCT CTCCCTCTCT 900

CCTGCCTCCT GCTGCCCCCT CCCCAGCCCA CTTSCCCGAG TTGTGCTTGC CGCTCCTTAT 960

CTGTTCTAGT TCCGAAGCAG TTTCACTCGA AGTTGTGCAG TCCTGGTTGC AGCTTTCCGC 1020

ATCTGCCTTC GTTTCGTGTA GATTGACGCG TTTCTTTGTA ATTTCAGTGT TTCTGACAAG 1080

ATTTAAAAAA AAAAAAAGGA AAAAAAAAAA AAAAAAAAAC TCGAGGGGGG GCCCGGTACC 1140

CAATTG                                                              1146.

(2) INFORMATION FOR SEQ ID NO: 243:

(i) SEQUENCE CHARACTERISTICS:
(A) LENGTH: 1350 base pairs
(B) TYPE: nucleic acid
(C) STRANDEDNESS: double
(D) TOPOLOGY: linear

(xi) SEQUENCE DESCRIPTION: SEQ ID NO: 243:

AACCCACGGC TGCTGCGGCA GGGCGTGGAG GGCAGAGGGC CGCGGAGGCG CAGTTGCAAA      60

CATGGCTCAG AGCAGAGACG GCGGAAACCC GTTCGCCGAG CCCAGCGAGC TTGACAACCC     120

CTTTCAGCCA CCACCAGCCT ATGAGCCTCC AGCCCCTGCC CCATTGCCTC CACCCTCAGC     180

TCCCTCCTTG CAGCCCTCGA GAAAGCTCAG CCCCACAGAA CCTAAGAACT ATGGCTCATA     240

CAGCACTCAG GCCTCAGCTG CAGCAGCCAC AGCTGAGCTG CTGAAGAAAC AGGAGGAGCT     300

CAACCGGAAG GCAGAGGAGT TGGACCGAAG GAGNCGAGAG CTGCAGCATG CTGCCCTGGG     360

RGGCACAGCT ACTCGACAGA ACAATTGGCC CCCTCTACCT TCTTTTTGTC CAGTTCAGCC     420

CTGCTTTTTC CAGGACATCT CCATGGAGAT CCCCCAAGAA TTTCAGAAGA CTGTATCCAC     480

CATGTACTAC CTCTGGATGT GCAGCACGST GGCTCTTCTC CTGAACTTCC TCGCCTGCCT     540

GGCCAGCTTC TGTGTGGAAA CCAACAATGG CGCAGGCTTT GGGCTTTCTA TCCTCTGGGT     600

CCTCCTTTTC ACTCCCTGCT CCTTTGTCTG CTGGTACCGC CCCATGTATA AGGCTTTCCG     660

GAGTGACAGT TCATTCAATT TCTTCGTTTT CTTCTTCATT TTCTTCGTCC AGGATGTGCT     720

CTTTGTCCTC CAGGCCATTG GTATCCCAGG TTGGGGATTC AGTGGCTGGA TCTCTGCTCT     780

GGTGGTGCCG AAGGCAACAC AGCAGTATCC GTGCTCATGC TGCTGGTCGC CCTGCTCTTC     840

ACTGGCATTG CTGTGCTAGG AATTGTCATG CTGAAACGGA TCCACTCCTT ATACCGCCGC     900

ACAGGTGCCA GCTTTCAGAA GGCCCAGCAA GAATTTGCTG CTGGTGTCTT CTCCAACCCT     960

GCGGTGCGAA CCGCARCTTG CCAATGCAGC CGCTGGGGCT GCTGAAAATG CCTTCCGGGC    1020

CCCGTGACCC CTGACTGGGA TGCCCTGGCC CTGCTACTTG AGGGAGCTGA CTTAGCTCCC    1080

GTCCCTAAGG TCTCTGGGAC TTGGAGAGAC ATCACTAACT GATGGCTCCT CCGTAGTGCT    1140

CCCAATCCTA TGGCCATGAC TGCTGAACCT GACAGGCGTG TGGGGAGTTC ACTGTGACCT    1200

AGTCCCCCCA TCAGGCCACA CTGCTGCCAC CTCTCACACG CCCCAACCCA GCTTCCCTCT    1260

GCTGTGCCAC GGCTGTTGCT TCGGTTATTT AAATAAAAAG AAAGTGGAAC TGGAAAAAAA    1320

AAAAAAAAAA AAAAAAAAAG GGGGGNCCNC                                     1350

(2) INFORMATION FOR SEQ ID NO: 244:

(i) SEQUENCE CHARACTERISTICS:
(A) LENGTH: 1529 base pairs
(B) TYPE: nucleic acid
(C) STRANDEDNESS: double
(D) TOPOLOGY: linear

(xi) SEQUENCE DESCRIPTION: SEQ ID NO: 244:

TCCCAGAGGC CGGGGGGTTC CAGCTCTGCC TGTAGCAGAG CCCTGAGGAG GAGGAGGAAG 60

AGGATGTGCT GAAATACGTC CGGGAGATCT TTTTCAGCTA GGGCATAAAC TGTGCACTGA 120

ACTGTCTGCC GAGAGCAGCT GGAGGACAGC TGAGCTTCCA CTGGTGCTGC TGGGCCGMCC 180

GCCTGTGGGA ATGGGGCTCT CTGTGCTCCT ACCTTTGTGC CTTCTTGGGC CTGGCAGATT 240

CACCTCAGGC CAGAAGCCCC TGGACACTCC GGGCCTTGGG GTGCCGTTCT GAGTGTGCGG 300

AAGGCAGGAC TCAAAATGAG ATCCCATTTG ACTCCCTCTG TATGTACTGT GCCCTCTCCT 360

GGCTCTTGAG GCTCTGGAGT CCCAATTGTC TGTGTTAGTC AGTGACCAGG TTCCAGGGAA 420

AATRATGTCA TGTGGTGGTC CAACTTACTG GAACCAAAGA GACAGTACTT TGCAAAGAAA 480

AGGATCACTG CCAGGTGCAC TGGAATTGCT ACAGTTTAGT CCGCATGATC TCTCCTGAAG 540

GAGGAAGCCT GTTTCAAAAA TAGTTTCCAT CATGAGTCTA TCAATGAGCT CCCACCTCTC 600

CAGCCAGCCT AGAAAGCAAA CGAGCTGCCC ACAGTTCTCT GCCCTGTCTG GGAGGTTGAG 660

GCCACAGTGT ATAGACTGGT AAGCCAGACA GGCCTCCTCC CGCAAGCTGC TACCTTGCTT 720

TCACCTGTAC CTTGGTCCCC GGGCAGCTAG CTATAAAGCA AGAGGGACAG GAGCCCAGAA 780

GAGACACTGA GGACAAGAGA TCACACCAGA GTACATGTCT CTGCCTCTGT TTTCAGTGTG 840

GCTTTGGACA GGAATATATG AATAAATCAC TGCCATACAG GTTTTCCAAT ACACAAGTGC 900

TAGAAAATAC ACACAATTCC CCAATGCGTA AGTTGTGCTA ATGTCTTTCC AAGTTCTGGG 960

TTGGGAAGTG GAGGGTGGCA GCGTTTGTTT GTGCGCAACC GTCCAGTCCT GTTCACAGCG 1020

AGGATTTGGA GTCCTCCAGG GTCTCATCAT GGGAGTGATT TGTCAGCGGA CGCCTCTGCC 1080

CTGTCTGGCT TCAGGTCCAG GGAAGCTTTG AAGCAGTCAA GCCTTGTCTT TGTACCCCAT 1140

GTGTCCTGTC TTTGTTGAGT CACTCAGAGA TCACTCCTGG ACCTCTGGGG TTGGAGTTCC 1200

AGTGATGGCT TATGGCGGCC CACTCACTAT GGTGGGCTGA GTGGAAGCTC CTTAACCATG 1260

TCCCCAGAGA CACTGAGGTG CTCGCTCTTT TAATGTCCTC GTTTGTTGCC GTAAGTTCTT 1320

TGCTAGGTTT CATTTTGGCA TTTGGCAAAT CAGCCTGGAA GTCTGGCCCC ATGACAGCAA 1380

TCACTCCCTC CCCACCCTCC TGAAGCTAGA GGAAGATTTG CTCAGATCCA TTAATTAAAG 1440

CAGGAATTGG TGTGACAATG AGCTGCATGG TTTAGGGAGT CTTTGGGAGC CTTGGAAGTC 1500

CTGAAGGACA AACAATCTTG TACTAAGAA 1529

(2) INFORMATION FOR SEQ ID NO: 245:

(i) SEQUENCE CHARACTERISTICS:
(A) LENGTH: 1537 base pairs
(B) TYPE: nucleic acid
(C) STRANDEDNESS: double
(D) TOPOLOGY: linear

(xi) SEQUENCE DESCRIPTION: SEQ ID NO: 245:

GTGCGAGGTC CCCGCCAGCC CCCAGCGGCC TTCCCGGCCC GGGGCGCTCC CAGAGCAAAC 60

GAGGCCCCTG AGAGCTCCAC CTAGTTCACA GGATAAAATC CCACAGCAGA ACTCGGAGTC 120

AGCAATGGCT AAGCCCCAGG TGGTTGTAGC TCCTGTATTA ATGTCTAAGC TGTCTGTGAA 180

TGCCCCTGAA TTTTACCCTT CAGGTTATTC TTCCAGTTAC ACAGAATCCT ATGAGGATGG 240

TTGTGAGGAT TATCCTACTC TATCAGAATA TGTTCAGGAT TTTTTGAATC ATCTTACAGA 300

GCAGCCTGGC AGTTTTGAAA CTGAAATTGA ACAGTTTGCA GAGACCCTGA ATGGTTGTGT 360

TACAACAGAT GATGCTTTGC AAGAACTTGT GGAACTCATC TATCAACAGG CCACATCTAT 420

CCCAAATTTC TCTTATATGG GAGCTCGCCT GTGTAATTAC CTGTCCCATC ATCTGACAAT 480

TAGCCCACAG AGTGGCAACT TCCGCCAATT GCTACTTCAA AGATGTCGGA CTGAATATGA 540

AGTTAAAGAT CAAGCTGCAA AAGGGGATGA AGTTACTCGA AAACGATTTC ATGCATTTGT 600

ACTCTTTCTG GGAGAACTTT ATCTTAACCT GGAGATCAAG GGAACAAATG GACAGGTTAC 660

AAGAGCAGAT ATTCTTCAGG TTGGTCTTCG AGAATTGCTG AATGCCCTGT TTTCTAATCC 720

TATGGATGAC AATTTAATTT GTGCAGTAAA ATTGTTAAAG TTGACAGGAT CAGTTTTGGA 780

AGATGCTTGG AAGGAAAAAG GAAAGATGGA TATGGAAGAA ATTATTCAGA GAATTGAAAA 840

CGTTGTCCTA GATGCAAACT GCAGTAGAGA TGTAAAACAG ATGCTCTTGA AGCTTGTAGA 900

ACTCCGGTCA AGTAACTGGG GCAGAGTCCA TGCAACTTCA ACATATAGAG AAGCAACACC 960

AGAAAATGAT CCTAACTACT TTATGAATGA ACCAACATTT TATACATCTG ATGGTGTTCC 1020

TTTCACTGCA GCTGATCCAG ATTACCAAGA GAAATACCAA GAATTACTTG AAAGAGAGGA 1080

CTTTTTTCCA GATTATGAAG AAAATGGAAC AGATTTATCC GGGGCTGGTG ATCCATACTT 1140

GGATGATATT GATGATGAGA TGGACCCAGA GATAGAAGAA GCTTATGAAA AGTTTTGTTT 1200

GGAATCAGAG CGTAAGCGAA AACAGTAAAG TTAAATTTCA GCATATCAGT TTTATAAAGC 1260

AGTTTAGGTA TGGTGATTTA GCAGAACACA AGAGAGCAAG AAAATGTGTC ACATCTATAC 1320

CAAATTRAGG ATGTTGAGTT ATGTTACTAA TGTATGCAAC TTTAATTTTG TTTAACACTA 1380

TCTGCCAAAA TAAACTTTAT TCCCTATAAC TTAAAATGTG TATATATATA TAATAGTTTA 1440

TTATGTACAG TTAATTCTAC TGTTTTGGCT GCAATAAAAT CGATTTTGAA ATAAAWRAAA 1500

AAAAAAAAAA AAGGGNGGCC GCTCTAGAGG ANCCAAG 1537

(2) INFORMATION FOR SEQ ID NO: 246:

(i) SEQUENCE CHARACTERISTICS:
(A) LENGTH: 506 base pairs
(B) TYPE: nucleic acid
(C) STRANDEDNESS: double
(D) TOPOLOGY: linear

(xi) SEQUENCE DESCRIPTION: SEQ ID NO: 246:

TGCAGGATTT GGCCAGGACC CSCCGCGGTG GCGGTTGCTA TCGCTTCGCA GAACCTACTC 60

AGGCAGCCAG CTGAGAAGAG TTGAGGGAAA GTGCTGCTGC TGGGTCTGCA GACGCGATGG 120

ATAACGTGCA GCCGAAAATA AAACATCGCC CCTTCTGCTT CAGTGTGAAA GGCCACGTGA 180

AGATGCTGCG GCTGGATATT ATCAACTCAC TGGTAACAAC AGTATTCATG CTCATCGTAT 240

CTGTGTTGGC ACTGATACCA GAAACCACAA CATTGACAGT TGGTGGAGGG GTGTTTGCAC 300

TTGTGACAGC AGTATGCTGT CTTGCCGACG GGGCCCTTAT TTACCGGAAG CTTCTGTTCA 360

ATCCCAGCGG TCCTTACCAG AAAAAGCCTG TGCATGAAAA AAAAGAAGTT TTGTAATTTT 420

ATATTACTTT TTAGTTTGAT ACTAAGTATT AAACATATTT CTGKATTATT CCAAAAAAAA 480

AAAAAAAAAA AAAAAAAATT TGGTGG 506

(2) INFORMATION FOR SEQ ID NO: 247:

(i) SEQUENCE CHARACTERISTICS:
(A) LENGTH: 1348 base pairs
(B) TYPE: nucleic acid
(C) STRANDEDNESS: double
(D) TOPOLOGY: linear

(xi) SEQUENCE DESCRIPTION: SEQ ID NO: 247:

GTCTTTCTTT TNCTGTTTTG AGTTGGTGAG TGAGTGAATA GGGTAACATG GGCCTTCAGG 60

ATGACCCCTT GGAACTGTGC CGAGTTCCTT AAATCTCAGC TGGGATCCTG GACCTGGGAG 120

GCCCCTGTGA GGGCCAGCTC TGGAAAAACC TGGGAGTTGA TGCCGGAGGY TGGGAAGAAC 180

TCTGCTCGAG GGCAGGGTGC CCTGGAACAC TGGTAGTTCT GGGGCTGGGA GGGAGAGGGG 240

CTCCGGCTTT CTCTGAAATG AACACTGCTC TTCAGCAGTT CAAGTACTTG TTCTCAAAAC 300

ATTTTCTAAT TGATTGGTAG GTTTTCATAA GCATTGTTTC TTTAAGGCAT GGAAAGGGAA 360

GAATGCTCAA GCAAGTCATG TTTGTTTTCA GTGGGATGGG CCCGCGTTCT CACTGCTGGG 420

GGCTTCCCCT TGCATGTGGC ACCTTTGTGC AGGGCCACCA GGCAGACTCT TCCCACCTTC 480

TCCCACTGAA GCACCAAGGG GCTTGAACCG TAATTTGGCT AATCAGAGGC ATTTTTTTTG 540

TCCTAGTATC TTTCACACTT GTCCAACCGT CTTATTTTTT TAAAAGTTCT GTTGCTTGTA 600

TTAACACGAA ACTAGAGAGA AATAGTTTCT GAAGCCAGTT TATTGTGAAG ATCCCCAAGG 660

GGAGGTTCGG TAGAGAAAAA TAGTAAGCTG GTTTAGAAAC TGACGAGGGC AAACAGCCAG 720

GACGCATTGG AGAGGAATTT GCCAAAGATC TACCCTGAGA TAACGCCTGT CCAGTGTCTT 780

CACCACGTGA ATAACCAGCG CTCCAAAGTG TTTTTCTGCT TTGAAAAAAA AAATTCCACA 840

AGCTTTTAAA GGTGCATTTA AGAATCCATG TGACTTTAGA ATGGAACTGC CGGCCCTGGC 900

AACTGTCACG TGTGCTAGAA GGTTCGATGC CTCTGGAATG CATGTGATAC TCATCTCCAT 960

TTTGTTTCCT TGATTGCATT TTTGTTCTTT TAGCAGATCT GTCCCTGTGG GTGGTGTCTA 1020

AGAAGTCGGA CACCTTGGTT TTTGTGTTAG ATTGAGCTGG GCAGCTGCAA TCAGCTTCTT 1080

TATATGCAAA TTAGGCACGA CCCATCTGTG GTTCCCTGGT TGGTGGCTAA TGAAGTGAGG 1140

GGAGGGAGGG ATGTCACCCC AAAAGTAGGC CCTCCCATTG GCTTTGGCCA GGCCAGACAC 1200

TTCACATCGT TTACATGGTT CTGTGTAATT TTAAAGTTTA TGTGTATAAA GCGAAGCTGT 1260

TTCTGTGAAA CTGTATATTT TGTAAATAAA TATATTGCTA CTTTGAGAWR AAAAAAAAAA 1320

AAAAACTCGA GGGGGGCCCG GTACCCAA 1348

(2) INFORMATION FOR SEQ ID NO: 248:

(i) SEQUENCE CHARACTERISTICS:
(A) LENGTH: 1766 base pairs
(B) TYPE: nucleic acid
(C) STRANDEDNESS: double
(D) TOPOLOGY: linear

(xi) SEQUENCE DESCRIPTION: SEQ ID NO: 248:

GTGCCGAATC GGCAGAGCGG CACGAGCGGC CACGAGAGCA GGCGGAGTAA AGGGACTTGA 60

GCGAGCCAGT TGCCGGATTA TTCTATTTCC CCTCCCTCTC TCCCGCCCCG TATCTCTTTT 120

CACCCTTCTC CCACCCTCGC TCGCGTASCA TGGCGGAGCG TCGGCGGCCA CTCAGTCCCA 180

TTCCATCTCC TCGTCGTCCT TCGGAGCCGA GCCGTCCGCG CCCGGCGGCG GCGGGAGCCC 240

AGGAGCCTGC CCCGCCCTGG GGACGAAGAG CTGCAGCTCC TCCTGTGCGG TGCACGATCT 300

GATTTTCTGG AGAGATGTGA AGAAGACTGG GTTTGTCTTT GGCACCACGC TGATCATGCT 360

GCTTTCCCTG GCAGCTTTCA GTGTCATCAG TGTGGTTTCT TACCTCATCC TGGCTCTTCT 420

CTCTGTCACC ATCAGCTTCA GGATCTACAA GTCCGTCATC CAAGCTGTAC AGAAGTCAGA 480

AGAAGGCCAT CCATTCAAAG CCTACCTGGA CGTAGACATT ACTCTGTCCT CAGAAGCTTT 540

CCATAATTAC ATGAATGCTG CCATGGTGCA CATCAACAGG GCCCTGAAAC TCATTATTCG 600

TCTCTTTCTG GTAGAAGATC TGGTTGACTC CTTGAAGCTG GCTGTCTTCA TGTGGCTGAT 660

GACCTATGTT GGTGCTGTTT TTAACGGAAT CACCCTTCTA ATTCTTGCTG AACTGCTCAT 720

TTTCAGTGTC CCGATTGTCT ATGAGAAGTA CAAGACCCAG ATTGATCACT ATGTTGGCAT 780

CGCCCGAGAT CAGACCAAGT CAATTGTTGA AAAGATCCAA GCAAAACTCC CTGGAATCGC 840

CAAAAAAAAG GCAGAATAAG TACATGGAAA CCAGAAATGC AACAGTTACT AAAACACCAT 900

TTAATAGTTA TAACGTCGTT ACTTGTACTA TGAAGGAAAA TACTCAGTGT CAGCTTGAGC 960

CTGCATTCCA AGCTTTTTTT TTAATTTGGT GTTTTCTCCC ATCCTTTCCC TTTAACCCTC 1020

AGTATCAAGC ACAAAAATTG ATGGACTGAT AAAAGAACTA TCTTAGAACT CAGAAGAAGA 1080

AAGAATCAAA TTCATAGGAT AAGTCAATAC CTTAATGGTG GTAGAGCCTT TACCTGTAGC 1140

TTGAAAGGGG AAAGATTGGA GGTAAGAGAG AAAATGAAAG AACACCTCTG GGTCCTTCTG 1200

TCCAGTTTTC AGCACTAGTC TTACTCAGCT ATCCATTATA GTTTTGCCCT TAAGAAGTCA 1260

TGATTAACTT ATGAAAAAAT TATTTGGGGA CAGGAGTGTG ATACCTTCCT TGGTTTTTTT 1320

TTGCAGCCCT CAAATCCTAT CTTCCTGCCC CACAATGTGA GCAGCTACCC CTGATACTCC 1380

TTTTCTTTAA TGATTTAACT ATCAACTTGA TAAATAACTT ATAGGTGATA GTGATAATTC 1440

CTGATTCCAA GAATGCCATC TGATAAAAAA GAATAGAAAT GGAAAGTGGG ACTGAGAGGG 1500

AGTCAGCAGG CATGCTGCGG TGGCGGTCAC TCCCTCTGCC ACTATCCCCA GGGAAGGAAA 1560

RGCTCCGCCA TTTGGGAAAG TGGTTTCTAC GTCACTGGAC ACCGGTTCTG AGCATTAGTT 1620

TGAGAACTCG TTCCCGAATG TGCTTTCCTC CCTCTCCCCT GCCCACCTCA AGTTTAATAA 1680

ATAAGGTTGT ACTTTTCTTA CTATAAAATA AAAAAAAAAA AACTCGAGGG GGGCCCGGTA 1740

CCCAAATCGC CGGATATGAT CGTAAA 1766

(2) INFORMATION FOR SEQ ID NO: 249:

(i) SEQUENCE CHARACTERISTICS:

(A) LENGTH: 2664 base pairs
(B) TYPE: nucleic acid
(C) STRANDEDNESS: double
(D) TOPOLOGY: linear

(xi) SEQUENCE DESCRIPTION: SEQ ID NO: 249:

AGTGTCCTCG GAGCAGGCGG AGTAAAGGGA CTTGAGCGAG CCAGTTGCCG GATTATTCTA 60

TTTCCCCTCC CTCTCTCCCG CCCCGTATCT CTTTTCACCC TTCTCCCACC CTCGCTCGCG 120

TASCATGGCG GAGCGTCGGC GGCCACTCAG TCCCATTCCA TCTCCTCGTC GTCCTTCGGA 180

GCCGAGCCGT CCGCGCCCGG CGGCGGCGGG AGCCCAGGAG CCTGCCCCGC CCTGGGGACG 240

AAGAGCTGCA GCTCCTCCTG TGCGGTGCAC GATCTGATTT TCTGGAGAGA TGTGAAGAAG 300

ACTGGGTTTG TCTTTGGCAC CACGCTGATC ATGCTGCTTT CCCTGGCAGC TTTCAGTGTC 360

ATCAGTGTGG TTTCTTACCT CATCCTGGCT CTTCTCTCTG TCACCATCAG CTTCAGGATC 420

TACAAGTCCG TCATCCAAGC TGTACAGAAG TCAGAAGAAG GCCATCCATT CAAAGCCTAC 480

CTGGACGTAG ACATTACTCT GTCCTCAGAA GCTTTCCATA ATTACATGAA TGCTGCCATG 540

GTGCACATCA ACAGGGCCCT GAAACTCATT ATTCGTCTCT TTCTGGTAGA AGATCTGGTT 600

GACTCCTTGA AGCTGGCTGT CTTCATGTGG CTGATGACCT ATGTTGGTGC TGTTTTTAAC 660

GGAATCACCC TTCTAATTCT TGCTGAACTG CTCATTTTCA GTGTCCCGAT TGTCTATGAG 720

AAGTACAAGA CCCAGATTGA TCACTATGTT GGCATCGCCC GAGATCAGAC CAAGTCAATT 780

GTTGAAAAGA TCCAAGCAAA ACTCCCTGGA ATCGCCAAAA AAAAGGCAGA ATAAGTACAT 840

GGAAACCAGA AATGCAACAG TTACTAAAAC ACCATTTAAT AGTTATAACG TCGTTACTTG 900

TACTATGAAG GAAAATACTC AGTGTCAGCT TGAGCCTGCA TTCCAAGCTT TTTTTTTAAT 960

TTGGTGTTTT CTCCCATCCT TTCCCTTTAA CCCTCAGTAT CAAGCACAAA AATTGATGGA 1020

CTGATAAAAG AACTATCTTA GAACTCAGAA GAAGAAAGAA TCAAATTCAT AGGATAAGTC 1080

AATACCTTAA TGGTGGTAGA GCCTTTACCT GTAGCTTGAA AGGGGAAAGA TTGGAGGTAA 1140

GAGAGAAAAT GAAAGAACAC CTCTGGGTCC TTCTGTCCAG TTTTCAGCAC TAGTCTTACT 1200

CAGCTATCCA TTATAGTTTT GCCCTTAAGA AGTCATGATT AACTTATGAA AAAATTATTT 1260

GGGGACAGGA GTGTGATACC TTCCTTGGTT TTTTTTTGCA GCCCTCAAAT CCTATCTTCC 1320

TGCCCCACAA TGTGAGCAGC TACCCCTGAT ACTCCTTTTC TTTAATGATT TAACTATCAA 1380

CTTGATAAAT AACTTATAGG TGATAGTGAT AATTCCTGAT TCCAAGAATG CCATCTGATA 1440

AAAAAGAATA GAAATGGAAA GTGGGACTGA GAGGGAGTCA GCAGGCATGC TGCGGTGGCG 1500

GTCACTCCCT CTGCCACTAT CCCCAGGGAA GGAAARGCTC CGCCATTTGG GAAAGTGGTT 1560

TCTACGTCAC TGGACACCGG TTCTGAGCAT TAGTTTGAGA ACTCGTTCCC GAATGTGCTT 1620

TCCTCCCTCT CCCCTGCCCA CCTCAAGTTT AATAAATAAG GTTGTACTTT TCTTACTATA 1680

AAATAAATGT CTGTAACTGC TGTGCACTGC TGTAAACTTG TTAGAGAAAA AAATAACCTG 1740

CATGTGGGCT CCTCAGTTAT TGAGTTTTTG TGATCCTATC TCAGTCTGGG GGGGAACATT 1800

CTCAAGAGGT GAAATACAGA AAGCCTTTTT TTCTTGATCT TTTCCCGAGA TTCAAATCTC 1860

CGATTCCCAT TTGGGGGCAA GTTTTTTTCT TCACCTTCAA TATGAGAATT CAGCGAACTT 1920

GAAAGAAAAA TCATCTGTGA GTTCCTTCAG GTTCTCACTC ATAGTCATGA TCCTTCAGAG 1980

GGAATATGCA CTGGCGAGTT TAAAGTAAGG GCTATGATAT TTGATGGTCC CAAAGTACGG 2040

CAGCTGCAAA AAGTAGTGGA AGGAAATTGT CTACGTGTCT TGGAAAAATT AGTTAGGAAT 2100

TTGGATGGGT AAAAGGTACC CTTGCCTTAC TCCATCTTAT TTTCTTAGCC CCCTTTGAGT 2160

GTTTTAACTG GTTTCATGTC CTAGTAGGAA GTGCATTCTC CATCCTCATC CTCTGCCCTC 2220

CCAGGAAGTC AGTGATTGTC TTTTTGGGCT TCCCCTCCAA AGGACCTTCT GCAGTGGAAG 2280

TGCCACATCC AGTTCTTTTC TTTTGTTGCT GCTGTGTTTA GATAATTGAA GAGATCTTTG 2340

TGCCACACAG GATTTTTTTT TTTTTTAAGA AAAACCTATA GATGAAAAAT TACTAATGAA 2400

ACTGTGTGTA CGTGTCTGTG CGTGCAACAT AAAAATACAG TAGCACCTAA GGAGCTTGAA 2460

TCTTGGTTCC TGTAAAATTT CAAATTGATG TGGTATTAAT AAAAAAAAAA AAAACAMAAA 2520

AAAAAAAAAA AAAAGGGCGG CCGCTCTAGA GGATCCAAGC TTACGTACGC GTGCATGCGA 2580

CGTCCATAGC TCTTTCTATA GGGGTCCCCC AAATTCCATT CANCGGGCCG TCGGTTTTAN 2640

AAAGGTCGTG ANTGGGGGAA ANCC 2664

(2) INFORMATION FOR SEQ ID NO: 250:

(i) SEQUENCE CHARACTERISTICS:
(A) LENGTH: 865 base pairs
(B) TYPE: nucleic acid
(C) STRANDEDNESS: double
(D) TOPOLOGY: linear

(xi) SEQUENCE DESCRIPTION: SEQ ID NO: 250:

CGTGGGAGTG AGGTACCAGA TTCAGCCCAT TTGGCCCCGA CGCCTCTKTT CTCGGAATCC 60

GGGTGCTGCG GATTGAGGTC CCGGTTCCTA ACGGTGGGAT CGGTGTCCTC GGGATGAGAT 120

TTGGCGTTTC CTCGGGGCTT TGGTGGGATC GGTGTCCTCA GGATGAGATT TAGGGTTTCC 180

TCGGGGCTTT CGGGATCTTC ACCTAATATC CGGACTGCAA GATGGAGGAA GGCGGGAACC 240

TAGGAGGCCT GATTAARATG GTCCATCTAC TGGTCTTGTC AGGTGCCTGG GGCATGCAAA 300

TGTGGGTGAC CTTCGTCTCA GGCTTCCTGC TTTTCCGAAG CCTTCCCCGA CATACCTTCG 360

GACTAGTGCA GAGCAAACTC TTCCCCTTCT ACTTCCACAT CTCCATGGGC TGTGCCTTCA 420

TCAACCTCTG CATCTTGGCT TCACAGCATG CTTGGGCTCA GCTCACATTC TGGGAGGCCA 480

GCCAGCTTTA CCTGCTGTTC CTGAGCCTTA CGCTGGCCAC TGTCAACGCC CGCTGGCTGG 540

AACCCCGCAC CACAGCTGCC ATGTGGGCCC TGCAAACCGT GGAGAAGGAG CGAGGCCTGG 600

GTGGGGAGGT ACCAGGCAGC CACCAGGGTC CCGATCCCTA CCGCCAGCTG CGAGAGAAGG 660

ACCCCAAGTA CAGTGCTCTC CGCCAGAATT TCTTCCGCTA CCATGGGCTG TCCTCTCTTT 720

GCAATCTGGG CTGCGTCCTG AGCAATGGGC TCTGTCTCGC TGGCCTTGCC CTGGAAATAA 780

GGAGCCTCTA GCATGGGCCC TGCATGCTAA TAAATGCTTC TTCAGAAAAA AAAAAAAAAA 840

AAACTCGAGG GGGGCCCGGT ACCCA 865

(2) INFORMATION FOR SEQ ID NO: 251:

(i) SEQUENCE CHARACTERISTICS:
(A) LENGTH: 2082 base pairs
(B) TYPE: nucleic acid
(C) STRANDEDNESS: double
(D) TOPOLOGY: linear

(xi) SEQUENCE DESCRIPTION: SEQ ID NO: 251:

TGGGGGGGGN AATGGGTGTC TGGCTCANGG ATTGCCNAAT CTGGAAATTC TCCATAACTT 60

GCTAGCTTGT TTTTTTTTTT TTTTTTTACA CCCCCCCGCC CCACCCCCGG ACTTGCACAA 120

TGTTCAATGA TCTCAGCAGA GTTCTTCATG TGAAACGTTG ATCACCTTTG AAGCCTGCAT 180

CATTCACATA TTTTTTCTTC TTCTTCCCCT TCAGTTCATG AACTGGTGTT CATTTTCTGT 240

GTGTGTGTGT GTTTTATTTT GTTTGGATTT TTTTTTTTAA TTTTACTTTT AGAGCTTGCT 300

GTGTTGCCCA CCTTTTTTCC AACCTCCACC CTCACTCCTT CTCAACCCAT CTCTTCCGAG 360

ATGAAAGAAA AAAAAAAGCA AAGTTTTTTT TTCTTCTCCT GAGTTCTTCA TGTGAGATTG 420

AGCTTGCAAA GGAAAAAAAA ATGTGAAATG TTATAGACTT GCAGCGTGCC GAGTTCCATC 480

GGGTTTTTTT TTTAGCATTG TTATGCTAAA ATAGAGAAAA AAATGCTCAT GAACCTTCCA 540

CAATCAAGCC TGCATCAACC TTCTGGGTGT GACTTGTGAG TTTTGGCCTT GTGATGCCAA 600

ATCTGAGAGT TTAGTCTGCC ATTAAAAAAA CTCATTCTCA TCTCATGCAT TATTATGCTT 660

GCTACTTTGT CTTAGCAACA ATGAACTATA ACTGTTTCAA AGACTTTATG GAAAAGAGAC 720

ATTATATTAA TAAAAAAAAA AAGCCTGCAT GCTGGACATG TATGGTATAA TTATTTTTTC 780

CTTTTTTTTT CCTTTTGGCT TGGAAATGGA CGTTCGAAGA CTTATAGCAT GGCATTCATA 840

CTTTTGTTTT ATTGCCTCAT GACTTTTTTG AGTTTAGAAC AAAACAGTGC AACCGTAGAG 900

CCTTCTTCCC ATGAAATTTT GCATCTGCTC CAAAACTGCT TTGAGTTACT CAGAACTTCA 960

ACCTCCCAAT GCACTGAAGG CATTCCTTGT GCAAAGATAC CAGAATGGGT TACACATTTA 1020

ACCTGGCAAA CATTGAAGAA CTCTTRATGT TTTCTTTTTA ATAAGAATGA CGCCCCACTT 1080

TGGGGACTAA AATTGTGCTA TTGCCGAGAA GCAGTCTAAA ATTTATTTTT TAAAAAGAGA 1140

AACTGCCCCA TTATTTTTGG TTTGTTTTAT TTTTATTTTA TATTTTTTGG CTTTTGGTCA 1200

TTGTCAAATG TGGAATGCTC TGGGTTTCTA GTATATAATT TAATTCTAGT TTTTATAATC 1260

TGTTAGCCCA GTTAAAATGT ATGCTACAGA TAAAGGAATG TTATAGATAA ATTTGAAAGA 1320

GTTAGGTCTG TTTAGCTGTA GATTTTTTAA ACGATTGATG CACTAAATTG TTTACTATTG 1380

TGATGTTAAG GGGGGTAGAG TTTGCAAGGG GACTGTTTAA AAAAAGTAGC TTATACAGCA 1440

TGTGCTTGCA ACTTAAATAT AAGTTGGGTA TGTGTAGTCT TTGCTATACC ACTGACTGTA 1500

TTGAAAACCA AAGTATTAAG AGGGGAAACG CCCCTGTTTA TATCTGTAGG GGTATTTTAC 1560

ATTCAAAAAT GTATGTTTTT TTTTCTTTTC AAAATTAAAG TATTTGGGAC TGAATTGCAC 1620

TAAGATATAA CCTGCAAGCA TATAATACAA AAAAAAATTG CAAAACTGTT TAGAACGCTA 1680

ATAAAATTTA TGCAGTTATA AAAATGGCAT TACTGCACAG TTTTAAGATG ATGCAGATTT 1740

TTTTACAGTT GTATTGTGGT GCAGAACTGG ATTTTCTGTA ACTTAAAAAA AAATCCACAG 1800

TTTTAAAGGC AATAATCAGT AAATGTTATT TTCAGGGACT GACATCCTGT CTTTAAAAAG 1860

AAATGAAAAG TAAATCTTAC CACAATAAAT ATAAAAAAAT CTTGTCAGTT ACTTTTCTTT 1920

TACATATTTT GCTGTGCAAA ATTGTTTTAT ATCTTGAGTT ACTAACTAAC CACGCGTGTT 1980

GTTCCTATGT GCTTTTCTTT CATTTTCAAT TCTGGTTATA TCAAGAAAAG AATAATCTAC 2040

AATAATAAAC GGCATTTTTT TTTGAAAAAA AAAAAAAAAA AA 2082

(2) INFORMATION FOR SEQ ID NO: 252:

(i) SEQUENCE CHARACTERISTICS:
(A) LENGTH: 1482 base pairs
(B) TYPE: nucleic acid
(C) STRANDEDNESS: double
(D) TOPOLOGY: linear

(xi) SEQUENCE DESCRIPTION: SEQ ID NO: 252:

CAGGCAGGCT GGCCCCGGGG ACTTCTCTCT GGCCCTGCTC CCTCCGAGCG CTCCGCCGTT 60

GCCCGCCTGG CCCCTACGGA GTCCTTAGCC AGGATGGAGG CTGTTGTGAA CTTGTACCAA 120

GAGGTGATGA AGCACGCAGA TCCCCGGATC CAGGGCTACC CTCTGATGGG GTCCCCCTTG 180

CTAATGACCT CCATTCTCCT GACCTACGTG TACTTCGTTC TCTCACTTGG GCCTCGCATC 240

ATGGCTAATC GGAAGCCCTT CCAGCTCCGT GGCTTCATGA TTGTCTACAA CTTCTCACTG 300

GTGGCACTCT CCCTCTACAT TGTCTATGAG TTCCTGATGT CGGGCTGGCT GAGCACCTAT 360

ACCTGGCGCT GTGACCCTGT GGACTATTCC AACAGCCCTG AGGCACTTAG GATGGTTCGG 420

GTGGCCTGGC TCTTCCTCTT CTCCAAGTTC ATTGAGCTGA TGGACACAGT GATCTTTATT 480

CTCCGAAAGA AAGACGGGCA GGTGACCTTC CTACATGTCT TCCATCACTC TGTGCTTCCC 540

TGGAGCTGGT GGTGGGGGGT AAAGATTGCC CCGGGAGGAA TGGGCTCTTT CCATGCCATG 600

ATAAACTCTT CCGTGCATGT CATAATGTAC CTGTACTACG GATTATCTGC CTTTGGCCCT 660

GTGGCACAAC CCTACCTTTG GTGGAAAAAG CACATGACAG CCATTCAGCT GATCCAGTTT 720

GTCCTGGTCT CACTGCACAT CTCCCAGTAC TACTTTATGT CCAGCTGTAA CTACCAGTAC 780

CCAGTCATTA TTCACCTCAT CTGGATGTAT GGCACCATCT TCTTCATGCT GTTCTCCAAC 840

TTCTGGTATC ACTCTTATAC CAAGGGCAAG CGGCTGCCCC GTGCACTTCA GCAAAATGGA 900

GCTCCAGGTA TTGCCAAGGT CAAGGCCAAC TGAGAAGCAT GGCCTAGATA GGCGCCCACC 960

TAAGTGCCTC AGGACTGCAC CTTAGGGCAG TGTCCGTCAG TGCCCTCTCC ACCTACACCT 1020

GTGACCAAGG CTTATGTGGT CAGGACTGAG CAGGGGACTG GCCCTCCCCT CCCCACAGCT 1080

GCTCTACAGG GACCACGGCT TTGGTTCCTC ACCCACTTCC CCCGGGCAGC TCCAGGGATG 1140

TGGCCTCATT GCTGTCTGCC ACTCCAGAGC TGGGGGCTAA AAGGGCTGTA CAGTTATTTC 1200

CCCCTCCCTG CCTTAAAACT TGGGAGAGGA GCACTCAGGG CTGGCCCCAC AAAGGGTCTC 1260

GTGGCCTTTT TCCTCACACA GAAGAGGTCA GCAATAATGT CACTGTGGAC CCAGTCTCAC 1320

TCCTCCACCC CACACACTGA AGCAGTAGCT TCTGGGCCAA AGGTCAGGGT GGGCGGGGGC 1380

CTGGGAATAC AGCCTGTGGA GGCTGCTTAC TCAACTTGTG TCTTAATTAA AAGTGACAGA 1440

GGAAACCAAA AAAAAAAAAA AAAAACTCGA GGGGGGCCCG TA 1482

(2) INFORMATION FOR SEQ ID NO: 253:

(i) SEQUENCE CHARACTERISTICS:
(A) LENGTH: 834 base pairs
(B) TYPE: nucleic acid
(C) STRANDEDNESS: double
(D) TOPOLOGY: linear

(xi) SEQUENCE DESCRIPTION: SEQ ID NO: 253:

GGCACGAGCG CCGTTGCCCG CCTGGCCCCT ACGGAGTCCT TAGCCAGGAT GGAGGCTGTT 60

GTGAACTTGT ACCAAGAGGT GATGAAGCAC GCAGATCCCC GGATCCAGGG CTACCCTCTG 120

ATGGGGTCCC CCTTGCTAAT GACCTCCATT CTCCTGACCT ACGTGTACTT CGTTCTCTCA 180

CTTGGGCCTC GCATCATGGC TAATCGGAAG CCCTTCCAGC TCCGTGGCTT CATGATTGTC 240

TACAACTTCT CACTGGTGGC ACTCTCCCTC TACATTGTCT ATGAGTTCCT GATGTCGGGC 300

TGGCTGAGCA CCTATACCTG GCGCTGTGAC CCTCAGGACT GCACCTTAGG GCAGTGTCCG 360

TCAGTGCCCT CTCCAMCTAC ACCTGTGACC AAGGCTTATG TGGTCAGGAC TGAGCAGGGG 420

ACTGGCCCTC CCCTCCCCAC AGCTGCTCTA CAGGGACCAC GGCTTTGGTT CCTCACCCAC 480

TTCCCCCGGG CAGCTCCAGG GATGTGGCCT CATTGCTGTC TGCCACTCCA GAGCTGGGGG 540

CTAAAAGGGC TGTACAGTTA TTTCCCCCTC CCTGCCTTAA AACTTGGGAG AGGAGCACTC 600

AGGGCTGGCC CCACAAAGGG TCTCGTGGCC TTTTTCCTCA CACAGAAGAG GTCAGCAATA 660

ATGTCACTGT GGACCCAGTC TCACTCCTCC ACCCCACACA CTGAAGCAGT AGCTTCTGGG 720

CCAAAGGTCA GGGTGGGCGG GGGCCTGGGA ATACAGCCTG TGGAGGCTGC TTACTCAACT 780

TGTGTCTTAA TTAAAAGTGA CAGAGGAAAC CACGAAAAAA AAAAAAAAAA AAAA 834

(2) INFORMATION FOR SEQ ID NO: 254:

(i) SEQUENCE CHARACTERISTICS:
(A) LENGTH: 1508 base pairs
(B) TYPE: nucleic acid
(C) STRANDEDNESS: double
(D) TOPOLOGY: linear

(xi) SEQUENCE DESCRIPTION: SEQ ID NO: 254:

TTGAACTTTT AAAATTTTAG ATCAGCAAAC TCTAAGATCC TAGAATGGAA GCTGTTCCTC 60

ATTTCTCCAT GCTCACCCTC CCAGGTCAGC GAGATGGTGA AGAAGCTGCA CGCGGCAACA 120

CCACCAACGT TCGGAGTGGA CCTCATCAAT GAGCTTGTGG AGAACTTTGG CAGATGTCCC 180

AAGTGGTCTG GTCGGCAAGC CTTTGTCTTT GTCTGCCAGA CTGTCATTGA GGATGACTGC 240

CTTCCCATGG ACCAGTTTGC TGTGCATCTC ATGCCGCATC TGCTAACCTT AGCAAATGAC 300

AGGGTTCCTA ACGTGCGAGT GCTGCTTGCA AAGACATTAA GACAAACTCT ACTAGAAAAA 360

GACTATTTCT TGGCCTCTGC CAGCTGCCAC CAGGAGGCTG TGGAGCAGAC CATCATGGCT 420

CTTCAGATGG ACCGTGACAG CGATGTCAAG TATTTTGCAA GCATCCACCC TGCCAGTACC 480

AAAATCTCCG AAGATGCCAT GAGCACAGCG TCCTCAACCT ACTAGAAGGC TTGAATCTCG 540

GTGTCTTTCC TGCTTCCATG AGAGCCGAGG TTCAGTGGGC ATTCGCCACG CATGTGACCT 600

GGGATAGCTT TCGGGGGAGG AGAGACCTTC CTCTCCTGCG GACTTCATTG CAGGTGCAAG 660

TTGCCTACAC CCAATACCAG GGATTTCAAG AGTCAAGAGA AAGTACAGTA AACACTATTA 720

TCTTATCTTG ACTTTAAGGG GAAATAATTT CTCAGAGGAT TATAATTGTC ACCGAAGCCT 780

TAAATCCTTC TGTCTTCCTG ACTGAATGAA ACTTGAATTG GCAGAGCATT TTCCTTATGG 840

AAGGGATGAG ATTCCCAGAG ACCTGCATTG CTTTCTCCTG GTTTTATTTA ACAATCGACA 900

AATGAAATTC TTACAGCCTG AAGGCAGACG TGTGCCCAGA TGTGAAAGAG ACCTTCAGTA 960

TCAGCCCTAA CTCTTCTCTC CCAGGAAGGA CTTGCTGGGC TCTGTGGCCA GCTGTCCAGC 1020

CCAGCCCTGT GTGTGAATCG TTTGTGACGT GTGCAAATGG GAAAGGAGGG GTTTTTACAT 1080

CTCCTAAAGG ACCTGATGCC AACACAAGTA GGATTGACTT AAACTCTTAA GCGCAGCATA 1140

TTGCTGTACA CATTTACAGA ATGGTTGCTG AGTGTCTGTG TCTGATTTTT TCATGCTGGT 1200

CATGACCTGA AGGAAATTTA TTAGACGTAT AATGTATGTC TGGTGTTTTT AACTTGATCA 1260

TGATCAGCTC TGAGGTGCAA CTTCTTCACA TACTGTACAT ACCTGTGACC ACTCTTGGGA 1320

GTGCTGCAGT CTTTAATCAT GCTGTTTAAA CTGTTGTGGC ACAAGTTCTC TTGTCCAAAT 1380

AAAATTTATT AATAAGATCT ATAGAGAGAG ATATATACAC TTTTGATTGT TTTCTAGATG 1440

TCTACCAATA AATGCAATTT GTGACCTGTA TTAAAAAAAA NTAAAAAAAC TCGAGGGGGG 1500

CCCGGTAC 1508

(2) INFORMATION FOR SEQ ID NO: 255:

(i) SEQUENCE CHARACTERISTICS:
(A) LENGTH: 2514 base pairs
(B) TYPE: nucleic acid
(C) STRANDEDNESS: double
(D) TOPOLOGY: linear

(xi) SEQUENCE DESCRIPTION: SEQ ID NO: 255:

GAGAGACTCA CACTTCTTTT CCATTATCAC TGACGATGTA GTGGACATAG CAGGGGAAGA 60

GCACCTACCT GTGTTGGTGA GGTTTGTTGA TGAATCTCAT AACCTAAGAG AGGAATTTAT 120

AGGCTTCCTG CCTTATGAAG CCGATGCAGA AATTTTGGCT GTGAAATTTC ACACTATGAT 180

AACTGAGAAG TGGGGATTAA ATATGGAGTA TTGTCGTGGC CAGGCTTACA TTGWCTCTAG 240

TGGATTTTCT TCCAAAATGA AAGTTGTTGC TTCTAGACTT TTAGAGAAAT ATCCCCAAGC 300

TATCTACACA CTCTGCTCTT CCTGTGCCTT AAATATGTGG TTGGCAAAAT CAGTACCTGT 360

TATGGGAGTA TCTGTTGCAT TAGGAACAAT TGAGGAAGTT TGTTCTTTTT TCCATCGATC 420

ACCACAACTG CTTTTAGAAC TTGACAACGT AATTTCTGTT CTTTTTCAGA ACAGTAAAGA 480

AAGGGGTAAA GAACTGAAGG AAATCTGCCA TTCTCAGTGG ACAGGCAGGC ATGATGCTTT 540

TGAAATTTTA GTGGAACTCC TGCAAGCACT TGTTTTATGT TTAGATGGTA TAAATAGTGA 600

CACAAATATT AGATGGAATA ACTATATAGC TGGCCGAGCA TTTGTACTCT GCAGTGCAGT 660

GTCAGATTTT GATTTCATTG TTACTATTGT TGTTCTTAAA AATGTCCTAT CTTTTACAAG 720

AGCCTTTGGG AAAAACCTCC AGGGGCAAAC CTCTGATGTC TTCTTTGCGG CCGGTAGCTT 780

GACTGCAGTA CTGCATTCAC TCAACGAAGT GATTGGAAAA TATTGAAGTT TATCATGAAT 840

TTTGGTTTGA GGAAGCCACA AATTTGGCAA CCAAACTTGA TATTCAAATG AAACTCCCTG 900

GGAAATTCCG CAGAGCTCAC CAGGGTAACT TGGAATCTCA GCTAACCTCT GAGAGTTACT 960

ATAAAGAAAC CCTAAGTGTC CCAACAGTGG AGCACATTAT TCAGGAACTT AAAGATATAT 1020

TCTCAGAACA GCACCTCAAA GCTCTTAAAT GCTTATCTCT GGTACCCTCA GTCATGGGAC 1080

AACTCAAATT CAATACGTCG GAGGAACACC ATGCTGACAT GTATAGAAGT GACTTACCCA 1140

ATCCTGACAC GCTGTCAGCT GAGCTTCATT GTTGGAGAAT CAAATGGAAA CACAGGGGGA 1200

AAGATATAGA GCTTCCGTCC ACCATCTATG AAGCCCTCCA CCTGCCTGAC ATCAAGTTTT 1260

TTCCTAATGT GTATGCATTG CTGAAGGTCC TGTGTATTCT TCCTGTGATG AAGGTTGAGA 1320

ATGAGCGGTA TGAAAATGGA CGAAAGCGTC TTAAAGCATA TTTGAGGAAC ACTTTGACAG 1380

ACCAAAGGTC AAGTAACTTG GCTTTGCTTA ACATAAATTT TGATATAAAA CACGACCTGG 1440

ATTTAATGGT GGACACATAT ATTAAACTCT ATACAAGTAA GTCAGAGCTT CCTACAGATA 1500

ATTCCGAAAC TGTGGAAAAT ACCTAAGAGA CTTTTAAAAA TAGGCTTTCT TATATTTGAT 1560

ATTTGGAAGA AAAAGCCGTA AGTGTATGTA GACCACTTAA TCACTAAATA TCTTTGCCTA 1620

TAGGACTCCA TTGAATACAT TAGCCATTGA TAATCTACCT GTTTAAATGG CCCCTGTTTG 1680

AACTCTCAAG CTTTGAAGAC CTACCTGTTC TTCCAGAAGA GAACGTTGAA AGTGCCATGT 1740

TTCCTTTTGC GTGATCTCTG TTGATGGCAC TCTGGAATTG TTTCAGTTAA GTCATTTTAG 1800

ACATAGCATT TATTATCACT GTGGATCTCT ACTTGTTGGG TGTTATGAAT TCTTTGAAGA 1860

AATATATTTT GAAGAGGTGT GGGAGGAAGG AATACATTTT ATAAAATGTT GTAGTGAAGC 1920

CCACAATTGA CCTTTGACTA ATAGGAGTTT TAAGTATGTT AAAAATCTAT ACTGGACAGT 1980

TACAAGAAAT TACCGGAGAA AAGCTTGTGA GCTCACCAAA CAAGGATTTC AGTGTAGATT 2040

TTGTCTTTCT TGAACTTAAA GAAACAAATG ACAAAGTTTG AATGGAAAAG CCTGCTGTTG 2100

TTCCACATCT CGTTGCTGTT TACATTCCTT TGTGGAGCCT ACATCTTCCT AAGCTTTTTA 2160

GCAGGTATAT GTTGAACACT TCTGTTTCAT GGTTGAGACA GAATCAGAGG CCATGGATAC 2220

TGACAACTGA TTTGTCTGTT TTTTTTCTCT GTCTTTTTCC ATGACTCTTA TATACTGCCT 2280

CATCTTGATT TATAAGCAAA ACCTGGAAAA CCTACAAAAT AAGTGTTGTG GTTTATCTAG 2340

AAAAATATGG AAAATATTGC TGTTATTTTT GGTGAAGAAA ATCAATTTTG TATAGTTTAT 2400

TTCAATCTAA ATAAAATGTG AATTTTGTTT AAAGCTTAGG CACATTATTT TTTGTGGGGT 2460

CAAAACATTC TTGTGTAAAT TCTCTTAAAC ATTTGATAAA CAGCTTCACA ATTC 2514

(2) INFORMATION FOR SEQ ID NO: 256:

(i) SEQUENCE CHARACTERISTICS:
(A) LENGTH: 2357 base pairs
(B) TYPE: nucleic acid
(C) STRANDEDNESS: double
(D) TOPOLOGY: linear

(xi) SEQUENCE DESCRIPTION: SEQ ID NO: 256:

CTGCCTTATG AAGCCGATGC AGAAATTTTG GCTGTGAAAT TTCACACTAT GATAACTGAG 60

AAGTGGGGAT TAAATATGGA GTATTGTCGT GGCCAGGCTT ACATTGTCTC TAGTGGATTT 120

TCTTCCAAAA TGAAAGTTGT TGCTTCTAGA CTTTTAGAGA AATATCCCCA AGCTATCTAC 180

ACACTCTGCT CTTCCTGTGC CTTAAATATG TGGTTGGCAA AATCAGTACC TGTTATGGGA 240

GTATCTGTTG CATTAGGAAC AATTGAGGAA GTTTGTTCTT TTTTCCATCG ATCACCACAA 300

CTGCTTTTAG AACTTGACAA CGTAATTYCT GTTCTTTTTC AGAACAGTAA AGAAAGGGGT 360

AAAGAACTGA AGGAAATCTG CCATTCTCAG TGGACAGGCA GGCATGATGC TTTTGAAATT 420

TTAGTGGAAC TCCTGCAAGC ACTTGTTTTA TGTTTAGATG GTATAAATAG TGACACAAAT 480

ATTAGATGGA ATAACTATAT AGCTGGCCGA GCATTTGTAC TCTGCAGTGC AGTGTCAGAT 540

TTTGATTTCA TTGTTACTAT TGTTGTTCTT AAAAATGTCC TATCTTTTAC AAGAGCCTTT 600

GGGAAAAACC TCCAGGGGCA AACCTCTGAT GTCTTCTTTG CGGCCGGTAG CTTGACTGCA 660

GTACTGCATT CACTCAACGA AGTGANTGGA AAATATTGAA GTTTATCATG AATTTTGGTT 720

TGAGGAAGCC ACAAATTTGG CAACCAAACT TGATATTCAA ATGAAACTCC CTGGGAAATT 780

CCGCAGAGCT CACCAGGGTA ACTTGGAATC TCAGCTAACC TCTGAGAGTT ACTATAAAGA 840

AACCCTAAGT GTCCCAACAG TGGAGCACAT TATTCAGGAA CTTAAAGATA TATTCTCAGA 900

ACAGCACCTC AAAGCTCTTA AATGCTTATC TCTGGTACCC TCAGTCATGG GACAACTCAA 960

ATTCAATACG TCGGAGGAAC ACCATGCTGA CATGTATAGA AGTGACTTAC CCAATCCTGA 1020

CACGCTGTCA GCTGAGCTTC ATTGTTGGAG AATCAAATGG AAACACAGGG GGAAAGATAT 1080

AGAGCTTCCG TCCACCATCT ATGAAGCCCT CCACCTGCCT GACATCAAGT TTTTTCCTAA 1140

TGTGTATGCA TTGCTGAAGG TCCTGTGTAT TCTTCCTGTG ATGAAGGTTG AGAATGAGCG 1200

GTATGAAAAT GGACGAAAGC GTCTTAAAGC ATATTTGAGG AACACTTTGA CAGACCAAAG 1260

GTCAAGTAAC TTGGCTTTGC TTAACATAAA TTTTGATATA AAACACGACC TGGATTTAAT 1320

GGTGGACACA TATATTAAAC TCTATACAAG TAAGTCAGAG CTTCCTACAG ATAATTCCGA 1380

AACTGTGGAA AATACCTAAG AGACTTTTAA AAATAGGCTT TCTTATATTT GATATTTGGA 1440

AGAAAAAGCC GTAAGTGTAT GTAGACCACT TAATCACTAA ATATCTTTGC CTATAGGACT 1500

CCATTGAATA CATTAGCCAT TGATAATCTA CCTGTTTAAA TGGCCCCTGT TTGAACTCTC 1560

AAGCTTTGAA GACCTACCTG TTCTTCCAGA AGAGAACGTT GAAAGTGCCA TGTTTCCTTT 1620

TGCGTGATCT CTGTTGATGG CACTCTGGAA TTGTTTCAGT TAAGTCATTT TAGACATAGC 1680

ATTTATTATC ACTGTGGATC TCTACTTGTT GGGTGTTATG AATTCTTTGA AGAAATATAT 1740

TTTGAAGAGG TGTGGGAGGA AGGAATACAT TTTATAAAAT GTTGTAGTGA AGCCCACAAT 1800

TGACCTTTGA CTAATAGGAG TTTTAAGTAT GTTAAAAATC TATACTGGAC AGTTACAAGA 1860

AATTACCGGA GAAAAGCTTG TGAGCTCACC AAACAAGGAT TTCAGTGTAG ATTTTGTCTT 1920

TCTTGAACTT AAAGAAACAA ATGACAAAGT TTGAATGGAA AAGCCTGCTG TTGTTCCACA 1980

TCTCGTTGCT GTTTACATTC CTTTGTGGAG CCTACATCTT CCTAAGCTTT TTAGCAGGTA 2040

TATGTTGAAC ACTTCTGTTT CATGGTTGAG ACAGAATCAG AGGCCATGGA TACTGACAAC 2100

TGATTTGTCT GTTTTTTTTC TCTGTCTTTT TCCATGACTC TTATATACTG CCTCATCTTG 2160

ATTTATAAGC AAAACCTGGA AAACCTACAA AATAAGTGTT GTGGTTTATC TAGAAAAATA 2220

TGGAAAATAT TGCTGTTATT TTTGGTGAAG AAAATCAATT TTGTATAGTT TATTTCAATC 2280

TAAATAAAAT GTGAATTTTG TTTAAAGCTT AGGCACATTA TTTTTTGTGG GGTCAAAACA 2340

TTCTTGTGTA AATTCTC 2357

(2) INFORMATION FOR SEQ ID NO: 257:

(i) SEQUENCE CHARACTERISTICS:
(A) LENGTH: 689 base pairs
(B) TYPE: nucleic acid
(C) STRANDEDNESS: double
(D) TOPOLOGY: linear

(xi) SEQUENCE DESCRIPTION: SEQ ID NO: 257:

ACTTTCTGGT GCAAAAAGAT GTTCAAGCCT TATTTTATAC TTGCCTGCCC CTTTCTCTTT 60

CATTTATTGG AGTGAGCTGC AGCTCTAAGA AGACCTGTTC TTTTGAATGG AGAGTAGCAT 120

CAGGAACCAG GATGTGGGTG CGAGGCGTGC TCCTGGCTGT TGCAGATTGC TGCACCCGGG 180

AGCTCTTAGT GGACAGAGCT AGAGGATATG TGCACGTACT TCCATCTCTC TCTCTGTCTC 240

CGATTTTAGC CCAGCACCAC AGGGTACGTT CCAGTTTTTC TCTCTTTCCA TAGCTGTAAG 300

GCCCTTTCTG GGAATGGTTC TCATTCTCCT TAATCTATTA TTGGGTCAGT TTTCCTGCAT 360

GTCCCCAGCC TCCCATCACT GCCACCCACT CCCCACAGAG ATGCCCTGCT CATCCGACTG 420

GGGCTTTGAC TCCCACACTG TGTACCCCTC TTGTGTGGAC GCCCTGCTGC CAAAACCTTC 480

AGCAAACAGC TTTCCAAATG GAAGTTGTCA CTGTCARGGS CTTTACAATC AGCAACAGCA 540

AAATCTACAT GCTGCTGAGG GTCCTGCCTC ATTAAGATGC AATAAATATG TAAGTACATA 600

AAAACAGCAA TAGAAGAAAC GTAATGCTTT ATTCTCAAAT ATGNATGTCT ACATAGAAAA 660

GCCAAAATTA TTAAGAATAG TAAGGAATT 689

(2) INFORMATION FOR SEQ ID NO: 258:

(i) SEQUENCE CHARACTERISTICS:
(A) LENGTH: 2377 base pairs
(B) TYPE: nucleic acid
(C) STRANDEDNESS: double
(D) TOPOLOGY: linear

(xi) SEQUENCE DESCRIPTION: SEQ ID NO: 258:

TCGACCCACG CGTCCGCCGA TGTGATGATT CCTGCGTATT CCAAGAACCG GGCCTATGCC 60

ATCTTCTTCA TAGTCTTCAC TGTGATAGGG GACGCCCCCG GCGCTGTGCT ATCCTGTGCC 120

GGCCACCCTT GCGTTGGTTT TGCTGCTGTA CTGGTGGCGC CCCTGACCGT GGCTGTCTCC 180

TCTTGAAGGA AGCCTGTTTC TGATGAACCT GCTGACAGCC ATCATCTACA GTCAGTTCCG 240

GGGCTACCTG ATGAAATCTC TCCAGACCTC GCTGTTTCGG AGGCGGGTGG GAACCCGGCT 300

GCCTTTGAAG TCCTATCCTC CATGGTGGGG GAGGGAGGAG CCTTCCCTCA GGCAGTTGGG 360

GTGAAGCCCC AGAACTTGCT GCAGGTGCTT CAGAAGGTCC AGCTGGACAG CTCCCACAGA 420

CAGGCCATGA TGGAGAAGGT GCGTTCCTAT GGCAGTGTTC TGCTCTCAGC TGAGGAGTTT 480

CAGAAGCTCT TCAACGAGCT TGACAGAAGT GTGGTTAAAG AGCACCCGCC GAGGCCCGAG 540

TACCAGTCTC CGTTTCTGCA GAGCGNCCCA GTTCCTCTTC GGCCACTNAC TACTTTGACT 600

ACCTGGGGAA CCTCATCGCC CTGGCAAACC TGGTGTCCAT TTGCGTGTTC CTGGTGCTGG 660

ATGCAGATGT TGCTGCCTGC TGAGCGTGAT GACTTCATCC TGGGGGGTCT CAACTGCGTC 720

TTCATTGTGT ACTACCTGTT GGAGATGCTG GCTCAAGGTC TTTTGCCCTG GGGCCTGCGA 780

RGGTACYKKT CCTAACCCCA RCAAMGTGTT TTGAACGGGC TCCTCAMCGT TTGTCCTGGC 840

TGGWWKKGSM GATCTCAACT CTGGCTGTGT ACCGATTGCC ACACCCAGGC TGGAGGCCGG 900

ANATGGTGGG CCTGCTGTCG CTGTGGGACA TGACCCGCAT ACTGAACATG CTCATCGTGT 960

TCCGCTTCCT GCGTATCATC CCCAGCATGA AGCCGATGGC CGTGGTGGCC AGTACCGTCC 1020

TGGGCCTGGT GCAAAACATG CGTGCGTTTG GCGGGATCCT GGTGGTGGTC TACTACGTAT 1080

TTGCCATCAT TGGGATCAAC TTGTTTAGAG GCGTCATTGT GGCTCTTCCT GGAAACAGCA 1140

GCCTGGCCCC TGCCAATAGG TCGGCGCCCT GTGGGAGCTT CGAGCAGCTG GAGTACTGGG 1200

CCAACAACTT CGATGACTTT GCGGCTGCCC TGGTCACTCT GTGGAACTTG ATGGTGGTGA 1260

ACAACTGGCA GGTGTTTCTG GATGCATATC GGCGCTACTA AGGCCCGTGG TCCAAGATCT 1320

ATTTTGTATT GTGGTGGCTG GTGTCGTCTG TCATCTGGGT CAACCTGTTT CTGGCCCTGA 1380

TTCTGGAGAA CTTCCTTCAC AAGTGGGACC CCCGCAGCCA CCTGCAGCCC CTTGCTGGGA 1440

CCCCAGAGGC CACCTACCAG ATGACTGTGG AGCTCCTGTT CAGGGATATT CTGGAGGAGC 1500

CCGGGGAGGA TGAGCTCACA GAGAGGCTGA GCCAGCACCC GCACCTGTGG CTGTGCAGGT 1560

GACGTCCGGG TCTGCCATCC CAGCAGGGGC GGCAGGAGAG AGAGGCTGGC ATAACACAGG 1620

TGCCCATCAT GGAAGAGGCG GCCATGCTGT GGCCAGCCAG GCAGGAAGAG ACCTTTCCTC 1680

TGACGGACCA CTAAGCTGGG GACAGGAACC AAGTCCTTTG CGTGTGGCCC AACAACCATT 1740

TACAGAACAG CTGCTGGTGC TTCAGGGAGG CGCCGTGCCC TCCGCTTTCT TTTATAGCTG 1800

CTTCAGTGAG AATTCCCTTG TCGACTCCAC AGGGACCTTT CAGACAAAAA TGCAAGAAGC 1860

AGCGGCCTCC CCTGTCCCCT GCAGCTTCCG TGGTGCCTTT GCTGCCGGCA GCCCTTGGGG 1920

ACCACAGGCC TGACCAGGGC CTGCACAGGT TAACCGTCAG ACTTCCGGGG CATTCAGCTG 1980

GGAATGATAC TAATACCTCC GATTTTAGCC CAGCACCACA GGGTACGTTC CAGTTTTTAT 2040

TTCTTTCCAT AGCTGTAAGG CCCTTTCTGG GAATGGTTAT CATTCTCCTT AATCTATTAT 2100

TGGGTCAGTT TTCCTGCATG TCCCCAGCCT CCCATCACTG CCACCCACTC CCCACAGAGA 2160

TGCCCTGCTC ATCCGACTGG GGCTTTGACT CCCACACTGT GTACCCCTCT TGTGTGGACG 2220

CCCTGCTGCC AAAACCTTCA GCAAACAGCT TTCCAAATGG AAGTTGTCAC TGTCAGGGCC 2280

TTTACAATCA GCAACAGCAA AATCTACATG CTGCTGAGGG TCCTGCCTCA TTAAGATGCA 2340

ATAAATATGT AAGTACATAA AAAAAAAAAA AAAAAAA 2377

(2) INFORMATION FOR SEQ ID NO: 259:

(i) SEQUENCE CHARACTERISTICS:
(A) LENGTH: 1193 base pairs
(B) TYPE: nucleic acid

(C) STRANDEDNESS: double
(D) TOPOLOGY: linear

(xi) SEQUENCE DESCRIPTION: SEQ ID NO: 259:

TCTGNTCGCC GTCGCCCCGC CCCTGGCCTT TGCCCGGTCG GGCGGGACTT CCTGTGTCGT 60

ATTTCCAAGG ACTCCAAAGC GAGGCCGGGG ACTGAAGGTG TGGGTGTCGA GCCCTCTGGC 120

AGAGGGTTAA CCTGGGTCAA ATGCACGGAT TCTCACCTCG TACAGTTACG CTCTCCCGCG 180

GCACGTCCGC GAGGMYTTGA AGTCCTGAGC GCTCAAGTTT GTCCGTAGTC GAGAGAAGGC 240

CATGGAGGTG CCGCCACCGG CACCGCGGAG CTTTCTCTGT AGAGCATTGT GCCTATTTCC 300

CCGAGTCTTT GCTGCCGAAG CTGTGACTGC CGATTCGGAA GTCCTTGAGG AGCGTCAGAA 360

GCGGCTTCCC TACGTCCCAG AGCCCTATTA CCCGGAATCT GGATGGGACC GCCTCCGGGA 420

GCTGTTTGGC AAAGACACAG TGAACACTAG TCTGAATGTA TACCGAAATA AAGATGCCTT 480

AAGCCATTTT GTAATTGCAG GAGCTGTCAC GGGAAGTCTT TTTAGGATAA ACGTAGGCCT 540

GCGTGGCTGG TGGCTGGTGG CATAATTGGA GCCTTGCTGG GCACTCCTGT AGGAGGCCTG 600

CTGATGGCAT TTCAGAAGTA CTCTGGTGAG ACTGTTCAGG AAAGAAAACA GAAGGATCGA 660

AAGGCACTCC ATGAGCTAAA ACTGGAAGAG TGGAAAGGCA GACTACAAGT TACTGAGCAC 720

CTCCCTGAGA AAATTGAAAG TAGTTTACAG GAAGATGAAC CTGAGAATGA TGCTAAGAAA 780

ATTGAAGCAC TGCTAAACCT TCCTAGAAAC CCTTCAGTAA TAGATAAACA AGACAAGGAC 840

TGAAAGTGCT CTGAACTTGA AACTCACTGG AGAGCTGAAG GGAGCTGCCA TGTCCGATGA 900

ATGCCAACAG ACAGGCCACT CTTTGGTCAG CCTGCTGACA AATTTAAGTG CTGGTACCTG 960

TGGTGGCAGT GGCTTGCTCT TGTCTTTTTC TTTTCTTTTT AACTAAGAAT GGGGCTGTTG 1020

TACTCTCACT TTACTTATCC TTAAATTTAA ATACATACTT ATGTTTGTAT TAATCTATCA 1080

ATATATGCAT ACATGAATAT ATCCACCCAC CTAGATTTTA AGCAGTAAAT AAAACATTTC 1140

GCAAAAGATT AAAGTTGAAT TTTACAGTTA AAAAAAAAAA AAAAAAAAAA AAA 1193

(2) INFORMATION FOR SEQ ID NO: 260:

(i) SEQUENCE CHARACTERISTICS:
(A) LENGTH: 1262 base pairs
(B) TYPE: nucleic acid
(C) STRANDEDNESS: double
(D) TOPOLOGY: linear

(xi) SEQUENCE DESCRIPTION: SEQ ID NO: 260:

GAAAAACCCA AAGATGCAGA CAATCTCTTT GAACATGAAT TGGGGGCTCT CAATATGGCT 60

GCATTACTAC GAAAAGAAGA AAGAGCAAGT CTTCTTAGTA ATCTTGGCCC ATGTTGTAAG 120

GCGTTGTGCT TCAGACGGGA TTCTGCAATT CGAAAGCAGC TTGTTAAAAA TGAGAAGGGC 180

ACCATAAAAC AAGCTTACAC GAGTSCTCCA ATGGTAGACA ATGAATTACT TCGATTGAGT 240

CTTCGGTTAT TTAAGCGGAA GACTACTTGC CATGCTCCAG GACATGAAAA GACTGAAGAT 300

AATAAACTTT CACAGTCCAG TATCCAACAG GAACTGTGTG TGTCTTAAGA CCGAAGTTCA 360

ATATGGTATT TTTGGTACTG TCTTCCTTCA GCAGTGCATA TTCTTTTGCA AAGTTCTTTG 420

GTTTGACAAG CATTAGTGAC AAAGGCAGAA AAGATTTATC AGCCATGCTA AAAGAGTGAA 480

GAATTTTGAT CTTTAGAGAC ACTAGTTTTG GCCAACTTAA GATTTTACGT TAATTTTTAC 540

ATAGTATTTG ACACTCATGC AAAATAATGT GAAAACATCT AGATTTAGTA GTTTATTCTG 600

CGCCTTTTGT TAAAACTGAA GATTTTGGAA AATGGTTGTC ACTGCTCTTC CAGCCTATGA 660

ATATTTTTGT GAAATGGAAC CATGGATTTA TGTCTGGATC ATCCATACAG AACCAACAAT 720

TTTATTCAAA AACAATGTGT TCATCAAAGT AATTGCTCAC ATTGTGCAGT ACTATGTTGT 780

ACAGACCACG TGAAAGGGAA TGCTGGTCTA GCTGGCGTGG TATGTTTATA GGCGAATTTC 840

AGCAGAAGGA AGCCAAAATA GTTTTTTCCT TTTGAAAGTT TTTTAAAAAT TATTTCATGG 900

GTCTTTTTTT TAATTAATAT GTGTGCATTG TTACAATGTA TGTTGGATGT CTTTTGACCC 960

TAAATGCTTT TTTTGTTATC AGAGATTGTG TACTATTTTT ATTTTTAATA AATGTATCTT 1020

CCCTTTCCTT GTTTTAGATT TACTTTGCTC TTCGTTAATC TTATTCCTGA TGATCTAGAA 1080

CATTAGTCAT CAACATTACA TGTTTCATGC TTCAGATATT TTACTGCTTG TGTCCTTATT 1140

GTTGGACAGC TTTAAACAGA GTTGATGGTA CTTCAAATAT AGCTCATTGA TACTTAAGGG 1200

CANCTTCCTT GGGATGTGGG CTTTTTGGAA GGAAAAAAAT TNCCCCAAAG GCAAATCCCA 1260

GT 1262

(2) INFORMATION FOR SEQ ID NO: 261:

(i) SEQUENCE CHARACTERISTICS:
(A) LENGTH: 1179 base pairs
(B) TYPE: nucleic acid
(C) STRANDEDNESS: double
(D) TOPOLOGY: linear

(xi) SEQUENCE DESCRIPTION: SEQ ID NO: 261:

GGCAAACTTT CCCCCAANGC TTCGAAACTT GCAAGCCGAA ACCTTGAATC GTTAAAAGTT 60

GGGTTGCGNC GGCGCCCTGG CCCGAAGAAG CGCAATTGGC GTTCCGCGAA CGTTGGCCCT 120

CAACGGCTCG GCAGCCAGCC ATGTCCTGCA CCCAGGACAG CGGCCCTGGG CTACAAGGAC 180

CTGGACCTCA TCTTCCTGCG CCGACCTGCG CGGGGAAGGG GAGTTTCAGA CTGTGAAGGA 240

CGTCGTGCTG GACTGCCTGT TGGACTTCTT ACCCGAGGGG GTGAACAAAG AGAAGATCAC 300

ACCACTCACG CTCAAGGAAG CTTATGTGCA GAAAATGGTT AAAGTGTGCA ATGACTCTGA 360

CCGATGGAGT CTTATATCCC TGTCAAACAA CAGTGGCAAA AATGTGGAAC TGAAATTTGT 420

GGATTCCCTC CGGAGGCAGT TTGAATTCAG TGTAGATTCT TTTCAAATCA AATTAGACTC 480

TCTTCTGCTC TTTTATGAAT GTTCAGAGAA CCCAATGACT GAGACATTTC ACCCCACAAT 540

AATCGGGGAG AGCGTCTATG GCGATTTCCA GGAAGCCTTT GATCACCTTT GTAACAAGAT 600

CATTGCCACC AGGAACCCAG AGGAAATCCG AGGGGGAGGC CTGCTTAAGT ACTGCAACCT 660

CTTGGTGAGG GGCTTTAGGC CCGCCTCTGA TGAAATCAAG ACCCTTCAAA GGTATATGTG 720

TTCCAGGTTT TTCATCGACT TCTCAGACAT TGGAGAGCAG CAGAGAAAAC TGGAGTCCTA 780

TTTGCAGAAC CACTTTGTGG GATTGGAAGA CCGCAAGTAT GAGTATCTCA TGACCCTTCA 840

TGGAGTGGTA AATGAGAGCA CAGTGTGCCT GATGGGACAT GAAAGAAGAC AGACTTTAAA 900

CCTTATCACC ATGCTGGCTA TCCGGGTGTT AGCTGACCAA AATGTCATTC CTAATGTGGC 960

TAATGTCACT TGCTATTACC AGCCAGCCCC CTATGTAGCA GATGCCAACT TTAGCAATTA 1020

CTACATTGCA CAGGTTCAGC CAGTATTCAC GTGCCAGCAA CAGACCTACT CCACTTGGCT 1080

ACCCTGCAAT TAAGAATCAT TTAAAAATGT CCTGTGGGGA AGCCATTTCA GACAAGACAG 1140

GAGAGAAAAA AAAAAAAAAA AAAAAAAAAA AAAAAGAGC 1179

(2) INFORMATION FOR SEQ ID NO: 262:

(i) SEQUENCE CHARACTERISTICS:
(A) LENGTH: 1162 base pairs
(B) TYPE: nucleic acid
(C) STRANDEDNESS: double
(D) TOPOLOGY: linear

(xi) SEQUENCE DESCRIPTION: SEQ ID NO: 262:

GGCAAACTTT CCCCCAANGC TTCGAAACTT GCAAGCCGAA ACCTTGAATC GTTAAAAGTT 60

GGGTTGCGNC GGCGCCCTGG CCCGAAGAAG CGCAATTGGC GTTCCGCGAA CGTTGGCCCT 120

CAACGGCTCG GCAGCCAGCC ATGTCCTGCA CCCAGGACAG CGGCCCTGGG CTACAAGGAC 180

CTGGACCTCA TCTTCCTGCG CCGACCTGCG CGGGGAAGGG GAGTTTCAGA CTGTGAAGGA 240

CGTCGTGCTG GACTGCCTGT TGGACTTCTT ACCCGAGGGG GTGAACAAAG AGAAGATCAC 300

ACCACTCACG CTCAAGGAAG CTTATGTGCA GAAAATGGTT AAAGTGTGCA ATGACTCTGA 360

CCGATGGAGT CTTATATCCC TGTCAAACAA CAGTGGCAAA AATGTGGAAC TGAAATTTGT 420

GGATTCCCTC CGGAGGCAGT TTGAATTCAG TGTAGATTCT TTTCAAATCA AATTAGACTC 480

TCTTCTGCTC TTTTATGAAT GTTCAGAGAA CCCAATGACT GAGACATTTC ACCCCACAAT 540

AATCGGGGAG AGCGTCTATG GCGATTTCCA GGAAGCCTTT GATCACCTTT GTAACAAGAT 600

CATTGCCACC AGGAACCCAG AGGAAATCCG AGGGGGAGGC CTGCTTAAGT ACTGCAACCT 660

CTTGGTGAGG GGCTTTAGGC CCGCCTCTGA TGAAATCAAG ACCCTTCAAA GGTATATGTG 720

TTCCAGGTTT TTCATCGACT TCTCAGACAT TGGAGAGCAG CAGAGAAAAC TGGAGTCCTA 780

TTTGCAGAAC CACTTTGTGG GATTGGAAGA CCGCAAGTAT GAGTATCTCA TGACCCTTCA 840

TGGAGTGGTA AATGAGAGCA CAGTGTGCCT GATGGGACAT GAAAGAAGAC AGACTTTAAA 900

CCTTATCACC ATGCTGGCTA TCCGGGTGTT AGCTGACCAA AATGTCATTC CTAATGTGGC 960

TAATGTCACT TGCTATTACC AGCCAGCCCC CTATGTAGCA GATGCCAACT TTAGCAATTA 1020

CTACATTGCA CAGGTTCAGC CAGTATTCAC GTGCCAGCAA CAGACCTACT CCACTTGGCT 1080

ACCCTGCAAT TAAGAATCAT TTAAAAATGT CCTGTGGGGA AGCCATTTCA GACAAGACAG 1140

GAGAGAAAAA NAANGAAAAG AG 1162

(2) INFORMATION FOR SEQ ID NO: 263:

(i) SEQUENCE CHARACTERISTICS:
(A) LENGTH: 735 base pairs
(B) TYPE: nucleic acid
(C) STRANDEDNESS: double
(D) TOPOLOGY: linear

(xi) SEQUENCE DESCRIPTION: SEQ ID NO: 263:

CGGGCTGGGT ATTTGCCTCG CACCATGGCG CCCAAGGGCA AAGTGGGCAC GAGAGGGAAG 60

AAGCAGATAT TTGAAGAGAA CAGAGAGACT CTGAAGTTCT ACCTGCGGAT CATACTGGGG 120

GCCAATGCCA TTTACTGCCT TGTGACGTTG GTCTTCTTTT ACTCATCTGC CTCATTTTGG 180

GCCTGGTTGG CCTTGGGCTT TAGTCTGGCA GTGTATGGGG CCAGCTACCA CTCTATGAGC 240

TCGATGGCAC GAGCAGCGTT CTTCTGAGGA TGGGGCCCTG ATGGATGGTG GCACGAGCTC 300

AACATGGAGC AGGGCATGGC AGAGCACCTT AAGGATGTGA TCCTACTGAC AGCCATCGTG 360

CAGGTGCTCA GCTGCTTCTC TCTCTATGTC TGGTCCTTCT GGCTTCTGGC TCCAGGCCGG 420

GCCCTTTACC TCCTGTGGGT GAATGTGCTG GGCCCCTGGT TCACTGCAGA CAGTGGCACC 480

CCAGCACCAG AGCACAATGA GAAACGGCAG CGCCGACAGG AGCGGCGGCA GATGAAGCGG 540

TTATAGCCAT TGACATTGTG GCCACAGGCC ACTGGCCCTG GGTGGCTCTG TCAGGGTGCA 600

CAGCCCCTCA TGCCTGGAGC AATGAGGGTC TAGTCCAGGG GCCAAAAGCA GTCTGAGGTA 660

TTGGGTATAC TTATACTCTA TAGGGTCGTT GAATAAATGG CTTAGAATGT GAAAAAAAAA 720

AAAAAAAAAA ATTTT 735

(2) INFORMATION FOR SEQ ID NO: 264:

(i) SEQUENCE CHARACTERISTICS:
(A) LENGTH: 783 base pairs
(B) TYPE: nucleic acid
(C) STRANDEDNESS: double
(D) TOPOLOGY: linear

(xi) SEQUENCE DESCRIPTION: SEQ ID NO: 264:

AAGTGCATGA GCTGCCGATG TGGTGCTTAG TGATTGCGGT TTCGGTCGCT CTCCCGTGTT 60

TCCCGGGCTG GGTATTTGCC TCGCACCATG GCGCCCAAGG GCAAAGTGGG CACGAGAGGG 120

AAGAAGCAGA TATTTGAAGA GAACAGAGAG ACTCTGAAGT TCTACCTGCG GATCATACTG 180

GGGGCCAATG CCATTTACTG CCTTGTGACG TTGGTCTTCT TTTACTCATC TGCCTCATTT 240

TGGGCCTGGT TGGCCTGGGC TTTAGTCTGG CAGTGTATGG GGCCAGCTAC CACTCTATGA 300

GCTCGATGGC ACGAGCAGCG TTCTCTGAGG ATGGGGCCCT GATGGATGGT GGCATGGACC 360

TCAACATGGA GCAGGGCATG GCAGAGTGAG TGTCCCCCAC CGCCAGCCCA GGCACCTTAA 420

GGATGTGATC CTACTGACAG CCATCGTGCA GGTGCTCAGC TGCTTCTCTC TCTATGTCTG 480

GTCCTTCTGG CTTCTGGCTC CAGGCCGGGC CCTTTACCTC CTGTGGGTGA ATGTGCTGGG 540

CCCCTGGTTC ACTGCAGACA GTGGCACCCC AGCACCAGAG CACAATGAGA AACGGCAGCG 600

CCGACAGGAG CGGCGGCAGA TGAAGCGGTT ATAGCCATTG ACGATTTKGC SACNRGCCAC 660

TGGCCCTGGG TGGCTCTGTC AGGGTGCACA GCCCCTCATG CCTGGAGCAA TGAGGGTCTA 720

GTCCAGGGGC CAAAAGCAGT CTGAGGTATT GGGTATACTT ATACTCTATA GGGTCGTTGA 780

ATA 783

(2) INFORMATION FOR SEQ ID NO: 265:

(i) SEQUENCE CHARACTERISTICS:
(A) LENGTH: 1638 base pairs
(B) TYPE: nucleic acid
(C) STRANDEDNESS: double
(D) TOPOLOGY: linear

(xi) SEQUENCE DESCRIPTION: SEQ ID NO: 265:

GGCACGAGGC GGCGGCAGCG GTGGCGGCGG CGCCCCCCGG CGGGAGCCGT NCCCTTTCCC 60

GTCGGGGAGC GCGGGGYCGG GGYCCAGGGG ANCCCGGGMC ACGGAGAGCG GGAAGAGGAT 120

GGATTGCCCG GCCCTCCCCC CCGGATGGAA GAAGGAGGAA GTGATCCGAA AATCTGGGCT 180

AAGTGCTGGC AAGAGCGATG TCTACTACTT CAGTCCAAGT GGTAAGAAGT TCAGAAGCAA 240

GCCTCAGTTG GCAAGGTACC TGGGAAATAC TGTTGATCTC AGCAGTTTTG ACTTCAGAAC 300

TGGAAAGATG ATGCCTAGTA AATTACAGAA GAACAAACAG AGACTGCGAA ACGATCCTCT 360

CAATCAAAAT AAGGGTAAAC CAGACTTGAA TACAACATTG CCAATTAGAC AAACAGCATC 420

AATTTTCAAA CAACCGGTAA CCAAAGTCAC AAATCATCCT AGTAATAAAG TGAAATCAGA 480

CCCACAACGA ATGAATGAAC AGCCACGTCA GCTTTTCTGG GAGAAGAGGC TACAAGGACT 540

TAGTGCATCA GATGTAACAG AACAAATTAT AAAAACCATG GAACTACCCA AAGGTCTTCA 600

AGGAGTTGGT CCAGGTAGCA ATGATGAGAC CCTTTTATCT GCTGTTGCCA GTGCTTTGCA 660

CACAAGCTCT GCGCCAATCA CAGGGCAAGT CTCCGCTGCT GTGGAAAAGA ACCCTGCTGT 720

TTGGCTTAAC ACATCTCAAC CCCTCTGCAA AGCTTTTATT GTCACAGATG AAGACATCAG 780

GAAACAGGAA GAGCGAGTAC AGCAAGTACG CAAGAAATTG GAAGAAGCAC TGATGGCAGA 840

CATCTTGTCG CGAGCTGCTG ATACAGAAGA GATGGATATT GAAATGGACA GTGGAGATGA 900

AGCCTAAGAA TATGATCAGG TAACTTTCGA CCGACTTTCC CCAAGAGAAA ATTCCTAGAA 960

ATTGAACAAA AATGTTTCCA CTGGCTTTTG CCTGTAAGAA AAAAAATGTA CCCGAGCACA 1020

TAGAGCTTTT TAATAGCACT AACCAATGCC TTTTTAGATG TATTTTTGAT GTATATATCT 1080

ATTATTCAAA AAATCATGTT TATTTTGAGT CCTAGGACTT AAAATTAGTC TTTTGTAATA 1140

TCAAGCAGGA CCCTAAGATG AAGCTGAGCT TTTGATGCCA GGTGCAATCT ACTGGAAATG 1200

TAGCACTTAC GTAAAACATT TGTTTCCCCC ACAGTTTTAA TAAGAACAGA TCAGGAATTC 1260

TAAATAAATT TCCCAGTTAA AGATTATTGT GACTTCACTG TATATAAACA TATTTTTATA 1320

CTTTATTGAA AGGGGACACC TGTACATTCT TCCATCRTCA CTGTAAAGAC AAATAAATGA 1380

TTATATTCAC AGACTGATTG GAATTCTTTC TGTTGAAAAG CACACACAAT AAAGAACCCC 1440

TCGTTAGCCT TCCTCTGATT TACATTCAAC TCTGATCCCG GGGCCTTAGG TTTGACATGG 1500

GAGGTGGGAG GAAGATAGCG CATATATTTG CAGTATGAAC TATTGCCTCT GGGACGTTGT 1560

GAGGAATTGT GCTTTCACCA GAATTTCTAA GGATTTCTGG CTTAAATATC ACCTAGCCTG 1620

TGGTAATTTT TTTTCCCT 1638

(2) INFORMATION FOR SEQ ID NO: 266:

(i) SEQUENCE CHARACTERISTICS:
(A) LENGTH: 1455 base pairs
(B) TYPE: nucleic acid
(C) STRANDEDNESS: double
(D) TOPOLOGY: linear

(xi) SEQUENCE DESCRIPTION: SEQ ID NO: 266:

CGTGCGTACT GCCATGCAGG TACCGGGTCC GGAATTCCCA GGGTCGACCC ACGCGTCCGC 60

TCAGTTGGCA AGGTACCTGG GAAATACTGT TGATCTCAGC AGTTTTGACT TCAGAACTGG 120

AAAGATGATG CCTAGTAAAT TACAGAAGAA CAAACAGAGA CTGCGAAACG ATCCTCTCAA 180

TCAAAATAAG GGTAAACCAG ACTTGAATAC AACATTGCCA ATTAGACAAA CAGCATCAAT 240

TTTCAAACAA CCGGTAACCA AAGTCACAAA TCATCCTAGT AATAAAGTGA AATCAGACCC 300

ACAACGAATG AATGAACAGC CACGTCAGCT TTTCTGGGAG AAGAGGCTAC AAGGACTTAG 360

TGCATCAGAT GTAACAGAAC AAATTATAAA AACCATGGAA CTACCCAAAG GTCTTCAAGG 420

AGTTGGTCCA GGTAGCAATG ATGAGACCCT TTTATCTGCT GTTGCCAGTG CTTTGCACAC 480

AAGCTCTGCG CCAATCACAG GGCAAGTCTC CGCTGCTGTG GAAAAGAACC CTGCTGTTTG 540

GCTTAACACA TCTCAACCCC TCTGCAAAGC TTTTATTGTC ACAGATGAAG ACATCAGGAA 600

ACAGGAAGAG CGAGTACAGC AAGTACGCAA GAAATTGGAA GAAGCACTGA TGGCAGACAT 660

CTTGTCGCGA GCTGCTGATA CAGAAGAGAT GGATATTGAA ATGGACAGTG GAGATGAAGC 720

CTAAGAATAT GATCAGGTAA CTTTCGACCG ACTTTCCCCA AGAGAAAATT CCTAGAAATT 780

GAACAAAAAT GTTTCCACTG GCTTTTGCCT GTAAGAAAAA AAATGTACCC GAGCACATAG 840

AGCTTTTTAA TAGCACTAAC CAATGCCTTT TTAGATGTAT TTTTGATGTA TATATCTATT 900

ATTCAAAAAA TCATGTTTAT TTTGAGTCCT AGGACTTAAA ATTAGTCTTT TGTAATATCA 960

AGCAGGACCC TAAGATGAAG CTGAGCTTTT GATGCCAGGT GCAATCTACT GGAAATGTAG 1020

CACTTACGTA AAACATTTGT TTCCCCCACA GTTTTAATAA GAACAGATCA GGAATTCTAA 1080

ATAAATTTCC CAGTTAAAGA TTATTGTGAC TTCACTGTAT ATAAACATAT TTTTATACTT 1140

TATTGAAAGG GGACACCTGT ACATTCTTCC ATCRTCACTG TAAAGACAAA TAAATGATTA 1200

TATTCACAGA CTGATTGGAA TTCTTTCTGT TGAAAAGCAC ACACAATAAA GAACCCCTCG 1260

TTAGCCTTCC TCTGATTTAC ATTCAACTCT GATCCCGGGG CCTTAGGTTT GACATGGGAG 1320

GTGGGAGGAA GATAGCGCAT ATATTTGCAG TATGAACTAT TGCCTCTGGG ACGTTGTGAG 1380

GAATTGTGCT TTCACCAGAA TTTCTAAGGA TTTCTGGCTT AAATATCACC TAGCCTGTGG 1440

TAATTTTTTT TCCCT 1455

(2) INFORMATION FOR SEQ ID NO: 267:

(i) SEQUENCE CHARACTERISTICS:
(A) LENGTH: 1086 base pairs
(B) TYPE: nucleic acid
(C) STRANDEDNESS: double
(D) TOPOLOGY: linear

(xi) SEQUENCE DESCRIPTION: SEQ ID NO: 267:

CGCCTGCAGT ACCGGTCCGG AATTCCCGGG TCGACCCACG CGTCGCTGAC CCAGGAGAAG 60

CTGCCTGTCT ACATCAGCCT GGGCTGCAGC GCGCTGCCGC CGCGGGGCCG GCAGCTGAAC 120

TATGTGCTCT TCAGGGCGGG CACCGTGTTG CATTCATCTT TGTACCCCCA GCATCTAGCA 180

GTGTTGGCAT GTAGTAGGCA CTCAAGAAAT GTGTGTTGAA TGAACGATGC CTGTGACAAG 240

CAAGCGGACT TTATTCTTTC CTGACCCTTG CTCCTATGAC ACACCTCCTC CTGACTGCCA 300

CTGTCACTCC TTCAGAGCAG AACTCCTCTA GGGAACCTGG ATGGGAAACA GCCATGGCCA 360

AGGACATCCT GGGTGAAGCA GGGCTACACT TTGATGAACT GAACAAGCTG AGGGTGTTGG 420

ACCCAGAGGT TACCCAGCAG ACCATAGAGC TGAAGGAAGA GTGCAAAGAC TTTGTGGACA 480

AAATTGGCCA GTTTCAGAAA ATAGTTGGTG GTTTAATTGA GCTTGTTGAT CAACTTGCAA 540

AAGAAGCAGA AAATGAAAAG ATGAAGGCCA TCGGTGCTCG GAACTTGCTC AAATCTATAG 600

CAAAGCAGAG AGAAGCTCAA CAGCAGCAAC TTCAAGCCCT AATAGCAGAA AAGAAAATGC 660

AGCTAGAAAG GTATCGGGTT GAATATGAAG CTTTGTGTAA AGTAGAAGCA GAACAAAATG 720

AATTTATTGA CCAATTTATT TTTCAGAAAT GAACTGAAAA TTTCGCTTTT ATAGTAGGAA 780

GGCAAAACAA AAAAAAGCCT CTCAAAACCA AAAAAACCTC TGTAGCATTC CAGCGGCTTG 840

ACCAATGACC TATGTCACAA GAGGTGGCGT GTAAGGAATG CAGCCCCCTG AAGACAGCAC 900

TACAAGTCTG GGGGAGCCAG TTTTAACATC AGTGCACAGC TGCTGCTGGT GGCCCTGCAG 960

TGTACGTTCT CACCTCTTAT GCTTAGTTGG AACTAAGCAG TTTGTAAACT TTCATCCTTT 1020

TTTTTGTAAA TTCACAAAGC TTTGGAAGGA GARGCAATAA ATTTTTGKTT TCNAAATGGC 1080

TTGATG 1086

(2) INFORMATION FOR SEQ ID NO: 268:

(i) SEQUENCE CHARACTERISTICS:
(A) LENGTH: 1003 base pairs
(B) TYPE: nucleic acid
(C) STRANDEDNESS: double

(D) TOPOLOGY: linear

(xi) SEQUENCE DESCRIPTION: SEQ ID NO: 268:

GGCACGGGAG CAGCCGGGCT GGTCCTGCTG CGAGCCGGCG GCCCGGAGTG GGGCGGCGGA 60

GCAAACATGA ACGTTGGAGT TGCCCACAGT GAAGTGAATC CAAATACCCG TGTCATGAAC 120

AGCCGGGGTA TGTGGCTGAC ATATGCATTG GGAGTTGGCT TGCTTCATAT TGTCTTACTC 180

AGCATTCCCT TCTTCAGTGT TCCTGTTGCT TGGACTTTAA CAAATATTAT ACATAATCTG 240

GGGATGTACG TATTTTTGCA TGCAGTGAAA GGAACACCTT TCGAAACTCC TGACCAGGGT 300

AAAAGCAAGG CTCCTAACTC ATTGGGAACA ACTGGACTAT GGAGTACAGT TTACATCTTC 360

ACGGAAGTTT TTCACAATTT CTCCAATAAT TCTATATTTT CTGGCAAGTT TCTATACGAA 420

GTATGATCCA ACTCACTTCA TCCTAAACAC AGCTTCTCTC CTGAGTGTAC TAATTCCCAA 480

AATGCCACAA CTACATGGTG TTCGGATCTT TGGAATTAAT AAGTATTGAA ATGTTTTGAA 540

ACTGAAAAAA AATTTTACAG CTACTGAATT TCTTATAAGG AAGGAGTGGT TAGTAAACTG 600

CACTGTTTCT CTGATAATGT GAAATGAGAA GTATTTACAT TGGAGGGCCA ATGGCTGGTC 660

CTTCAAGTGC TGTTTTGAAG TGCAGATTTC CATTAAATGA TGCCTCTGTT TAATACACCT 720

GGTACATTTC TGAAGAGGGG CTTTATAAGC AGGCTGGGCA GGCCCAGCTT ATAAGTTAAA 780

GGGCATCACA GTGAGGGTGT AGTAGATAAA TTCAAGGAAA TAAGAGATTT GTAAGAAACT 840

AGGACCAGCT TAACTTATAA TGAATGGGCA TTGTGTTAAG AAAAGAACAT TTCCAGTCAT 900

TCAGCTGTGG TTATTTAAAG CAGACTTACA TGTAAACCGG AATCCTCTCT ATACAAGTTT 960

ATTAAAGATT ATTTTTATTA CCGTAAAAAA AAAAAAAAAA AAA 1003

(2) INFORMATION FOR SEQ ID NO: 269:

(i) SEQUENCE CHARACTERISTICS:
(A) LENGTH: 1234 base pairs
(B) TYPE: nucleic acid
(C) STRANDEDNESS: double
(D) TOPOLOGY: linear

(xi) SEQUENCE DESCRIPTION: SEQ ID NO: 269:

ATCAGCATCT ACAAGTAGCA TATTTTGGAT GGTGTTTGTG TGCTACTTCA AAGTAACTAG 60

GAAAAAATAA TCCTCGCAAC ACAGGTACCT TGTCATGTCA GAATTGGGGG TGTTAGGTTG 120

CCAGTTGTAT CAGTGTTGAT TCATTTCATT ACTTCCTACA GAGCAAACAT GAACGTTGGA 180

GTTGCCCACA GTGAAGTGAA TCCAAATACC CGTGTCATGA ACAGCCGGGG TATGTGGCTG 240

ACATATGCAT TGGGAGTTGG CTTGCTTCAT ATTGTCTTAC TCAGCATTCC CTTCTTCAGT 300

GTTCCTGTTG CTTGGACTTT AACAAATATT ATACATAATC TGGGGATGTA CGTATTTTTG 360

CATGCAGTGA AAGGAACACC TTTCGAAACT CCTGACCAGG GTAAAGCAAG GCTCCTAACT 420

CATTGGGAAC AACTGGACTA TGGAGTACAG TTTACATCTT CACGGAAGTT TTTCACAATT 480

TCTCCAATAA TTCTATATTT TCTGGCAAGT TTCTATACGA AGTATGATCC AACTCACTTC 540

ATCCTAAACA CAGCTTCTCT CCTGAGTGTA CTAATTCCCA AAATGCCACA ACTACATGGT 600

GTTCGGATCT TTGGAATTAA TAAGTATTGA AATGTTTTGA AACTGAAAAA AAATTTTACA 660

GCTACTGAAT TTCTTATAAG GAAGGAGTGG TTAGTAAACT GCACTGTTTC TSTGATAATG 720

TGAAATGAGA AGTATTTACA TTGGAGGGCC AATGGCTGGT CCTTCAAGTG CTGTTTTGAA 780

GTGCAGATTT CCATTAAATG ATGCCTCTGT TTAATACACC TGGTACATTT CTGAAGAGGG 840

GCTTTATAAG CARGCTGGGC AGGCCCAGCT TATAAGTTAA AGGGCATCAC AGTGAGGGTG 900

TAGTAGATAA ATTCAAGGAA ATAAGAGATT TGTAAGAAAC TAGGACCAGC TTAACTTATA 960

ATGAATGGGC ATTGTGTTAA GAAAAGAACA TTTCCAGTCA TTCAGCTGTG GTTATTTAAA 1020

GCAGACTTAC ATGTAAACCG GAATCCTCTC TATACAAGTT TATTAAAGAT TATTTTTATT 1080

ACCRTACATA TTTCKCTTGT TTTATGTAAG YGGATGTATA TCCTCTTGTT TTATACAAGC 1140

CAGTTCCCAC TTATGAGGGT ACTTTTTTGG TTTTGCTGGG CTTAATATTG TGTATTGGTC 1200

AATGAGGCCA TTTTTACANT TATTAACGTT ACAG 1234

(2) INFORMATION FOR SEQ ID NO: 270:

(i) SEQUENCE CHARACTERISTICS:
(A) LENGTH: 574 base pairs
(B) TYPE: nucleic acid
(C) STRANDEDNESS: double
(D) TOPOLOGY: linear

(xi) SEQUENCE DESCRIPTION: SEQ ID NO: 270:

NGAGGTGCGT TCTGAGCCGT CTGTCCTGCG CCAAGATGCT TCAAAGTATT ATTAAAAACA 60

TATGGATCCC CATGAAGCCC TACTACACCA AAGTTTACCA GGAGATTTGG ATAGGAATGG 120

GGCTGATGGG CTTCATCGTT TATAAAATCC GGGCTGCTGA TAAAAGAAGT AAGGCTTTGA 180

AAGCTTCAGC GCCTGCTCCT GGTCATCACT AACCAGATTT ACTTGGAGTA CATGTGAAAG 240

AAAACGTCAG TCTGCCTGTA AATTTCAGCA AGCCGTGTTA GATGGGGAGC GTGGAACGTC 300

ACTGTACACT TGTATAAGTA CCGTTTACTT CATGGCATGA ATAAATGGAT CTGTGAGATG 360

CACTGCTACC TGGTACTGCT TTCAGTGTGT TCCCCCTCAG CCCTCCGGCG TGTCAGGCAT 420

ACTCTGAGTA GATAATTTGT CATGCAGCGC ATGCAATCAG AATCTCACTG AGCCACCCAT 480

CATTGTGAAA TAATTACCTC AGTTGTACAG GACTTGGTGA TCAGGATCCA GGCACTCACT 540

TGTATTCTAC TGCTCAATAA ACGTTTATTA AACT 574

(2) INFORMATION FOR SEQ ID NO: 271:

(i) SEQUENCE CHARACTERISTICS:
(A) LENGTH: 1731 base pairs
(B) TYPE: nucleic acid
(C) STRANDEDNESS: double
(D) TOPOLOGY: linear

(xi) SEQUENCE DESCRIPTION: SEQ ID NO: 271:

GCTGCAAGGT GCGCCTCGTG CCGCTGCAGA TCCAGCTCAC TACCCTGGGA AATCTTACAC 60

CTTCAAGCAC TGTGTTTTTC TGCTGTGATA TGCAGGAAAG GTTCAGACCA GCCATCAAGT 120

ATTTTGGGGA TATTATTAGC GTGGGACAGA GATTGTTGCA AGGGGCCCGG ATTTTAGGAA 180

TTCCTGTTAT TGTAACAGAA CAATACCCTA AAGGTCTTGG GAGCACGGTT CAAGAAATTG 240

ATTTAACAGG TGTAAAACTG GTACTTCCAA AGACCAAGTT TTCAATGGTA TTACCAGAAG 300

TAGAAGCGGC ATTAGCAGAG ATTCCCGGAG TCAGGAGTGT TGTATTATTT GGAGTAGAAA 360

CTCATGTGTG CATCCAACAA ACTGCCCTGG AGCTAGTTGG CCGAGGAGTC GAGGTTCACA 420

TTGTTGCTGA TGCCACCTCA TCAAGAAGCA TGATGGACAG GATGTTTGCC CTCGAGCGTC 480

TCGCTCRARC CNGGGATCAT AGTGACCACG AGTGNAGGCT GTTCTGCTTC AGCTGGTAGC 540

TGATAAGGAC CATCCAAAAT TCAAGGAAAT TCAGAATCTA ATTAAGGCGA GTGCTCCAGA 600

GTCGGGTCTG CTTTCCAAAG TATAGGACAT TTGAAGAACT GGTATGCTAC TCACTGGTGA 660

AGGACAGTCA GGTGAAGGAC TGTAAGCCCA CACAAGCTCT TCTTATCTCT ACTAGAATTA 720

AAATGTTAAG TCAAAAACGG CTCCTTTTTT GCGCCTCCTA GTGAACTTAA CCAGCTAGAC 780

CATTTGAGTA CCAGCATTTA GTTACAAACG TCAAAGGCTT CCGGTGCTGC TTACCTTCCT 840

TTTTTGTTAA TGTGCTTTTA TTTATTAAAA AAAATTACAA TGAAGATGCC TGTTTTGTCT 900

CTACTGTGTA CTCTGATCGT ATCTTTCCAA AGTGCAGACT CTTGTGAAGT TTTCTTAAAT 960

TGTTCACTTT AAAGAAAATG ACGTACCAAC AATGATTTGG CTTTTATATT ACTGTAAGAT 1020

GTTATAATGT TAATGTGGAT GTAGTGCTTT TACTTTACAG ATTGATTGGA ATAAGATTAT 1080

TGCATATGAA TTTACCCACA GGACTCTGAA TCATGTTACC CACTCCCCTC ACAATGTTGT 1140

CCACTTAGTG AGTTGCATTG ATCTATCCGT ACCAAATGAT GTTGAATAAT TACATATCTT 1200

TCTKGACTAT ACTGATTTCT TATTTTGGTC ACTATTACTA AATCTCTGTT AATATTCTCT 1260

CTTTTAACTG AAAAGGGATG GGATAGAAGG GTTTGCAATG CCATATTATT GGTGGAGGGC 1320

TGTTTTAACA TCTTTGAAGT ATGGCTTGCT GAATATCTTT ACCAACATCT TGAATATATA 1380

TTCTAGTGTC CACAAGATTT AGCAAAAAGA TAAAGCTTGG GTGGAATATC ATTTTAAAAT 1440

GTTCATGTTC TGTTCTATAT TTTCTTCACC TACTCTCCAA ATATTGTAAT GCAAAAAGTC 1500

TCAGTAATGA TTTGGTAGTA TTAATTTTGT GGTCATTGTT TCTCTTCGAT AAATTTATTT 1560

TCATTAAATA CTTRTTAGAG GGTTTTGAAA TGTTTTTCAA ATATGTGAAA TGTGAAACTG 1620

CTGTCTTTTA TATTAAAGTA ATTAAAGAAA ATGTATTGTG ATTGAAATTA TTTTGNCCTC 1680

CACAAGATGG CTCTATGAGT ATTCTTCCAG GGATTCTAAT ATTTATTTAA G 1731

(2) INFORMATION FOR SEQ ID NO: 272:

(i) SEQUENCE CHARACTERISTICS:
(A) LENGTH: 1320 base pairs
(B) TYPE: nucleic acid
(C) STRANDEDNESS: double
(D) TOPOLOGY: linear

(xi) SEQUENCE DESCRIPTION: SEQ ID NO: 272:

CTGCTTAGGA AGAGAAGGTC AGAGTTCGCG GGGGCAGAGG CATTCTTGCC GCTGGCCCAG 60

TCACTATGTA GTGGAGGGGC AGACACCCTC CCGCAAATTC TGGAAGGTTC TTAGTCTCGA 120

CTAGGGCAGT AGCCCAGGAC TCCTAGTCGC CGGCTTCAGG TCACTGCCGG CTGAACGGAG 180

CTGCCGTCGC CATGTTTGGC TGCTTGGTGG CGGGGAGGCT GGTGCAAACA GCTGCACAGC 240

AAGTGGCAGA GGATAAATTT GTTTTTGACT TACCTGATTA TGAAAGTATC AACCATGTTG 300

TGGTTTTTAT GCTGGGAACA ATCCCATTTC CTGAGGGAAT GGGAGGATCT GTCTACTTTT 360

CTTATCCTGA TTCAAATGGA ATGCCAGTAT GGMAACTCCT AGGATTTGTC ACGAATGGGA 420

AGCCAAGTGC CATCTTCAAA ATTTCAGGTC TTAAATCTGG AGAAGGAAGC CAACATCCTT 480

TTGGAGCCAT GAATATTGTC CGAACTCCAT CTGTTGCTCA GATTGGAATT TCAGTGGAAT 540

TATTAGACAG TATGGCTCAG CAGACTCCTG TAGGTAATGC TGCTGTATCC TCAGTTGACT 600

CATTCACTCA GTTCACACAA AAGATGTTGG ACAATTTCTA CAATTTTGCT TCATCATTTG 660

CTGTCTCTCA GGCCCAGATG ACACCAAGCC CATCTGAAAT GTTCATTCCG GCAAATGTGG 720

TTCTGCAAAT GGTATGAGGC ATNTTCTGTC TCCAATATTA AGGCTTTTTA TAACTGAATA 780

TCTATTTTGT CTATGAATAT ATTCCTTTTT TGACATTTAA ACATATTCTT TTATTGTGAA 840

CATCAGCACT GCATGCCATT AAAGTATGTA CTATAGAGAT CTGATGAGAA ACAGTTCTTA 900

CCCTAAATAT TTTGTTATAT TGTCGCCATT ATGAATTTAT AAAGACAGGA AAATATAGTT 960

GCCTATGTTT TAGGGACCAC TATTAAAGCT TATAAATATT TGTGTATTTT CATTTAGAAG 1020

TACCATCTAT GAGAGTAGTT TATACTGCAC TGTGTACATG AATGGCTAAT GAATCTATTT 1080

TCCAACTTTC CCGTGTTTTA TAGATATTTC TTTTCACTTT GAGTATCCTA GAGATGGGAG 1140

GATGCCTAGG AAGAGTTTGT TGAGAAGTGG TACCATGGTG TAGCATGGGA GAGCATTGGG 1200

AATGCACTAG GTTTGAATTT GGCATAATGG TAGCTATGTG ACCCTGAGCA AATTTCTCTC 1260

ATCTGCTCAT CTGANGAATG AGGAAATAGG AGTGAATTTG ATNTTTCCTA GGTCCNTCTA 1320

(2) INFORMATION FOR SEQ ID NO: 273:

(i) SEQUENCE CHARACTERISTICS:
(A) LENGTH: 515 base pairs
(B) TYPE: nucleic acid
(C) STRANDEDNESS: double
(D) TOPOLOGY: linear

(xi) SEQUENCE DESCRIPTION: SEQ ID NO: 273:

CCCTGGAGAG GGGCTGCTGT GCCAGCTTGG GGAGGGTCTG GGATGGGGCT GCCCCTGATG 60

GCCCTGATGT GGAGTACCTT GCCAGCATCT GCTGGGGTGA ACTTTATTTT AGCCCTTCCC 120

TTGTTGYTCT TATGAAGAAC AGAGGAGGGG TGGGCAGGTC AGTGATGTCA GCAGTGAGTA 180

TTCCCAGCAC AGCGGCTCTG GAAGAGGCAT GAGGCATTTC TTTCAGGAAA TGRTCATTAT 240

TCAGCCAGAA GGCATTCATT AAGTAAGTCC TGACTTTGTG CCCAGCTCTG TGTTATAGGC 300

CCTTGGCGAG ACTCAGGAGG GGCARAGGAC GCTAGKTTKT AGWTAACACG GAACCTCARA 360

GGWTATATGG TCCAAGAAGA CCCGGGGGCG GTGAAAACCC TGTGGACTAA TGCTCACGGG 420

AGCCCGAGGT CACACTTTGA CTTTGCTACC ATGGGCTGTG TCTANGNACG TATATATGCT 480

GCGTAATTAT TACAGAGGCA GTCCATGTGC ATTGT 515

(2) INFORMATION FOR SEQ ID NO: 274:

(i) SEQUENCE CHARACTERISTICS:
(A) LENGTH: 2995 base pairs
(B) TYPE: nucleic acid
(C) STRANDEDNESS: double
(D) TOPOLOGY: linear

(xi) SEQUENCE DESCRIPTION: SEQ ID NO: 274:

TGACACCCAT AAGGAATTCA TGAAGAAAGT AGAAGAAAAG CGAGTGGACG TTAACTCAGC 60

AGTAGCCATG GGAGAAGTCA TCCTGGCTGT CTGCCACCCC GATTGCATCA CAACCATCAA 120

ACACTGGATC ACCATCATCC GAGCTCGCTT CGAGGAGGTC CTGACATGGG CTAAGCAGCA 180

CCAGCAGCGT CTTGAAACGG CCTTGTCAGA ACTGGTGGCT AATGCTGAGC TCCTGGAAGA 240

ACTTCTGGCA TGGATCCAGT GGGCTGAGAC CACCCTCATT CAGCGGGATC AGGAGCCAAT 300

CCCGCAGAAC ATTGACCGAG TTAAAGCCCT TATCGCTGAG CATCAGACAT TTATGGAGGA 360

GATGACTCGC AAACAGCCTG ACGTGGACCG GGTCACCAAG ACATACAAAA GGAAAAACAT 420

AGAGCCTACT CACGCGCCTT TCATAGAGAA ATCCCGCAGC GGAGGCAGGA AATCCCTAAG 480

TCAGCCAACC CCTCCTCCCA TGCCAATCCT TTCACAGTCT GAAGCAAAAA ACCCACGGAT 540

CAACCAGCTT TCTGCCCGCT GGCAGCAGGT GTGGCTGTTA GCACTGGAGC GGCAAAGGAA 600

ACTGAATGAT GCCTTGGATC GGCTGGAGGA GTTGAAAGAA TTTGCCAACT TTGACTTTGA 660

TGTCTGGAGG AAAAAGTATA TGCGTTGGAT GAATCACAAA AAGTCTCGAG TGATGGATTT 720

CTTCCGGCGC ATTGATAAGG ACCAGGATGG GAAGATAACA CGTCAGGAGT TTATCGATGG 780

CATTTTAGCA TCCAAGTTCC CCACCACCAA GTTAGAGATG ACTGCTGTGG CTGACATTTT 840

CGACCGAGAT GGGGATGGTT ACATTGATTA TTATGAATTT GTGGCTGCTC TTCATCCCAA 900

CAAGGATGCG TATCGACCAA CAACCGATGC AGATAAAATC GAAGATGAGG TTACAAGACA 960

AGTGGCTCAG TGCAAATGTG CAAAAAGGTT TCAGGTGGAG CAGATCGGAG AGAATAAATA 1020

CCGGTTCTTC CTCGGCAATC AGTTTGGGGA TTCTCAGCAG TTGCGGCTGG TCCGTATTCT 1080

GCGCAACCGT GATGGTTCGC GTTGGTGGAG GATGGATGGC CTTGGATGAA TTTTTAGTGA 1140

AAAATGATCC CTGCCGAGCA CGAGGTAGAA CTAACATTGA ACTTAGAGAG AAATTCATCC 1200

TACCAGAGGG AGCATCCCAG GGAATGACCC CCTTCCGCTC ACGGGGTCGA AGGTCCAAAC 1260

CATCTTCCCG GGCAGCTTCC CCTACTCGTT CCAGCTCCAG TGCTAGTCAG AGTAACCACA 1320

GCTGTACATC CATGCCATCT TCTCCAGCCA CCCCAGCCAG TGGAACCAAG GTTATCCCAT 1380

CATCAGGTAG CAAGTTGAAA CGACCAACAC CAACTTTTCA TTCTAGTCGG ACATCCCTTG 1440

CTGGTGATAC CAGCAATTAG TTCTTCCCCG GCCTCCACAG GTGCCAAAAC TAATCGGGCA 1500

GACCCTAAAA AGTCTGCCAG TCGCCCTGGG AGTCGGGCTG GGAGTCGAGC CGGGAGTCGA 1560

GCCAGCAGCC GGCGAGGAAG TGACGCTTCT GACTTTGACC TCTTAGAGAC GCATTGCTTG 1620

TTCCGACACT TCAGAAAGCA GCGCTGCAGG GGGCCAAGGC AACTCCAGGA GAGGGCTAAA 1680

CAAACCTTCC AAAATCCCAA CCATGTCTAA GAAGACCACC ACTGCCTCCC CCAGGACTCC 1740

AGGTCCCAAG CGATAACACT GTCTAAGCAC CCCCAAGCCA CTATCCACTT TGAATCCTGC 1800

TCCATACATT GGGTGTATAT TTATTCTGAA CGGGAGAAGT TATATTGTTA AAAGTGTAAA 1860

AGAATAATTG TGTTATGAAG CTGCCTTATT TTTTTTCTTT TTGTAAGTTA CTATTTTCAT 1920

GTGAATATTT ATGTAGATAA AATTTGCCTC CTGGTAACCC TGTAATGGAT GGGGCCCAGA 1980

AATGAAATAT TTGAGAAAAA CAAGTGAAAA GGTCAAGATA CAAATGTGTA TTAAAAAAAA 2040

AAAAGCCTAT TAATAGGGTT TCTGCGCGGT GCAGGGTTGT AAACCTGCTT TATCTTTTAG 2100

GATTATTCCT AAATGCATCT TCTTTATAAA CTTGACTTGC TATCTCAGCA AGATAAATTA 2160

TATTAAAAAA ATAAGAATCC TGCAGTGTTT AAGGAACTCT TTTTTTGTAA ATCACGGACA 2220

CCTCAATTAG CAAGAACTGA GGGGAGGGCT TTTTCCATTG TTTAATGTTT TGTGATTTTT 2280

AGCTAAAGAG AGGGAACCTC ATCTAAGTAA CATTTGCACA TGGATACAGC AAAAGGAGTT 2340

CATTGCAATA CTGTCTTTGG ATATTGTTTC AGTACTGGGT GTTTAAAGGA CAAATAGCTG 2400

CTAGAATTCA GGGGTAAATG TAAGTGTTCA GAAAACGTCA GAACATTTGG GGTTTTAAAC 2460

TGATTTGTTG CTCCCTATCC AGCCTAGACA CCAGTAACTC TTGTGTTCAC CAGGACCCAG 2520

ACCCTTGGCA AGGGATAGGC TCGTTGGTGA CATTGTGAAT TTCAGATTTG TTTTATCCAC 2580

TTTTTTTGCT ATTTATTTAA ATGGTCGATC AACTTCCCAC AAACTGAGGA ATGAATTCCA 2640

CGAGCCTGTT CTGAAAATGT GGACGTAAGA CAAACACGTG CTCGTCCTTT AATGGAGTTC 2700

ACCAGCACAC TTGTTAACCA GTCCTGTTTG CTTTCGTCTT TTTTTGTGCG TAATAAAGTC 2760

AACTGACCAA GTGACCATGA AAAGGGGCTG TCTGGGGCTC CTGTTTTTTA GCTGCTGTTC 2820

TTCAGCTCCG ACCATGTTGC TGTGTGATTA TCTCAATTGG TTTTAATTGA GGCAGAAACT 2880

GAAGCTCTAC CAATGAACTG TTTAGAAACA AGACACACTT TTGTATTAAA ATTGCTTGCA 2940

GTAACAAAAA AAAAAAAAAA AAAAAAAAAA AAAAAACTCG AGGGGGGCCC GGTAC 2995

(2) INFORMATION FOR SEQ ID NO: 275:

(i) SEQUENCE CHARACTERISTICS:
(A) LENGTH: 1990 base pairs
(B) TYPE: nucleic acid
(C) STRANDEDNESS: double
(D) TOPOLOGY: linear

(xi) SEQUENCE DESCRIPTION: SEQ ID NO: 275:

GGGACCCGCG CGSCTCCCGG GGATGGTGAG CAAGGCGCTG CTGCNWCGTG TCTGCCGTCA 60

ACCGCAGAGG ATGAAGCTGC TGCTGGGCAT CGCCTTGCTG GCCTACGTCG CCTCTGTTTG 120

GGGCAACTTC GTTAATATGA GGTCTATCCA GGAAAATGGT GAACTAAAAA TTGAAAGCAA 180

GATTGAAGAG ATGGTTGAAC CACTAAGAGA GAAAATCAGA GATTTAGAAA AAAGCTTTAC 240

CCAGAAATAC CCACCAGTAA AGTTTTTATC AGAAAAGGAT CGGAAAAGAA TTTTGAWTAA 300

CAGGAGGCGC AGKGTTCGTG GGCTCCCATC TKAACTGACA AACTCATGAT GGACGGCCAC 360
GAGGTGACCG TGGTGGACAA TTTCTTCACG GGCAGGAAGA GAAACGTGGA GCACTGGATC 420
GGACATGAGA ACTTCGAGTT GATTAACCAC GACGTGTGGG AGCCCCTCTA CATCGAGGTT 480
GACCAGATAT ACCATCTGGC ATCTCCAGCC TCCCCTCCAA ACTACATGTA TAATCCTATC 540
AAGACATTAA AGACCAATAC GATTGGGACA TTAAACATGT TGGGGCTGGC AAAACGAGTC 600
GGTGCCCGTC TGCTCCTGGC CTCCACATCG GAGGTGTATG GAGATCCTGA AGTCCACCCT 660
CAAAGTGAGG ATTACTGGGG CCACGTGAAT CCAATAGGAC CTCGGGCCTG CTACGATGAA 720
GGCAAACGTG TTGCAGAGAC CATGTGCTAT GCCTACATGA AGCAGGAAGG CGTGGAAGTG 780
CGAGTGGCCA GAATCTTCAA CACCTTTGGG CCACGCATGC ACATGAACGA TGGGCGAGTA 840
GTCAGCAACT TCATCCTGCA GGCGCTCCAG GGGGAGCCAC TCACGGTATA CGGATCCGGG 900
TCTCAGACAA GGGCGTTCCA GTACGTCAGC GATCTAGTGA ATGGCCTCGT GGCTCTCATG 960
AACAGCAACG TCAGCAGCCC GGTCAACCTG GGGAACCCAG AAGAACACAC AATCCTAGAA 1020
TTTGCTCAGT TAATTAAAAA CCTTGTTGGT AGCGGAAGTG AAATTCAGTT TCTCTCCGAA 1080
GCCCAGGATG ACCCACAGAA AAGAAAACCA GACATCAAAA AAGCAAAGCT GATGCTGGGG 1140
TGGGAGCCCG TGGTCCCGCT GGAGGAAGGT TTAAACAAAG CAATTCACTA CTTCCGTAAA 1200
GAACTCGAGT ACCAGGCAAA TAATCAGTAC ATCCCCAAAC CAAAGCCTGC CAGAATAAAG 1260
AAAGGACGGA CTCGCCACAG CTGAACTCCT CACTTTTAGG ACACAAGACT ACCATTGTAC 1320
ACTTGATGGG ATGTATTTTT GGCTTTTTTT TGTTGTCGTT TAAAGAAAGA CTTTAACAGG 1380
TGTCATGAAG AACAAACTGG AATTTCATTC TGAAGCTTGC TTTAATGAAA TGGATGTGCC 1440
TAAAAGCTCC CCTCAAAAAA CTGCAGATTT TGCCTTGCAC TTTTTGAATC TCTCTTTTTA 1500
TGTAAAATAG CGTAGATGCA TCTCTGCGTA TTTTCAAGTT TTTTTATCTT GCTGTGAGAG 1560
CATATGTTGT GACTGTCGTT GACAGTTTTA TTTACTGGTT TCTTTGTGAA GCTGAAAAGG 1620
AACATTAAGC GGGACAAAAA ATGCCGATTT TATTTATAAA AGTGGGTACT TAATAAATGA 1680
GTCGTTATAC TATGCATAAA GAAAAAYCCT AGCAGTATTG TCAGGTGGTG GTGCGCCGGC 1740
ATTGATTTTA GGGCAGATAA AAGAATTCTG TGTGAGAGCT TTATGTTTCT CTTTTAATTC 1800
AGAGTTTTTC CAAGGTCTAC TTTTGAGTTG CAAACTTGAC TTTGAAATAT TCCTGTTGGT 1860
CATGATCAAG GATATTTGAA ATCACTACTG TGTTTTGCTG CGTATCTGGG GCGGGGGCAG 1920
GTTGGGGGGC ACAAAGTTAA CATATTCTTG GTTAACCATG GTTAAATATG CTATTTTAAT 1980
AAAATATTGA 1990

(2) INFORMATION FOR SEQ ID NO: 276:

(i) SEQUENCE CHARACTERISTICS:
(A) LENGTH: 2436 base pairs
(B) TYPE: nucleic acid
(C) STRANDEDNESS: double
(D) TOPOLOGY: linear

(xi) SEQUENCE DESCRIPTION: SEQ ID NO: 276:

AACTTCGCTT AGCTCTCCAG GGTNAAACGG GTGAGNCCTT AAAAACAGAA GAGAACAAGA 60

TTTAAAGTCC GTTGCATTGA AAATAACAAA CAATATCAAT GTTTTAATCA AGGATCTCTT 120

CCACATTCCT CCTTCTTATA AGAGCACAGT AACACTATCC TGGAAACCTG TACAAAAGGT 180

TGAGATTGGG CAAAAGAGAG CCAGTGAAGA TACAACTTCA GGTTCACCAC CCAAGAAATC 240

TTCAGCAGGA CCAAAAAGAG ATGCCAGGCA GATTTATAAC CCTCCCAGTG GGAAATATAG 300

CAGCAATTTG GGCAACTTTA ATTATGAGCA GAGAGGAGCC TTCAGGGGAA GTAGAGGTGG 360

CCGAGGTTGG GGCACACGAG GAAATCGTAG TCGGGGAAGA CTCTACTGAA TAAGACATCA 420

GCATTCTTCA GCATTGTCAT GAGCTTAATA TACTTAAATT CTACTACTCA TTGGATTGCC 480

GGGGATGTCC CTTTAAACAG ACTGCTGCCT TCAGCTAAAA ACTTAATGTT CTTTATACCT 540

TTGTATGTAT GACCTACTTT TGTAACAGAC CATGGTTGTG TCCAAGGTAA AACCACAGTG 600

ATATTTTTGG ATGCTTTGTC TGCAATCTTG ACTTGTTTTT GCAGTATCAT TATTCAGACT 660

TCAAATTGTG AATCTTTTAA ACATCTTGAT AATTTGTTGT TGAGAGCTGT TCATTCTAAA 720

ATGTAATGAA ATTCAGTCTA GTTCTGCTGA TAAAGATCAT CAGTTTTGAA AGGTTACTGA 780

TTTTCCTCTT CCCTCTTAGT TTTTTACCCA ATATATGGAG AAGAGTAATG GTCAATCTTA 840

ACATTTTGTT TTAATTGTTT AATAAAGCTG CTGGGCAGTG GTGCAGCATT CCTACCTAGT 900

GTCATAAAAG CAAAATACTT ACATAGCTTT CTTAAAATAT AGGAATGACA TTACATTTTT 960

AGGAGAAAGT AAGTTGCTTT GCACCGCCTA CTTAATTCTT TTCCATATAT TGTGATACAA 1020

ACTTTTGAAT ATGGAATCTT ACTATTTGAA TAGAAATGTG TATGTATAAT ATACATACAT 1080

ACATAAGCAT ATATGTGTGT GTGTGTGTGT ATATATATAT ATATGCATGC TGTGAAACTT 1140

GACTACACAA CATAAATCAC TTTTTAAATT CCAGGAACGG GTAGTCTGAC ACGGTGATTA 1200

TCCTTTTGAG GCTGAATCCG TTATTAACTT GTTATTTAGG TTTTTACTCC CAGTAGCAAG 1260

GGATTCTAAG TTAGTTGCAC TTACATGATT ATTGTTATTT AAAACTAAGA ATAAAGGCTG 1320

CATTTTCAAA GATAAATTGG AATTGCTGTT GGTGAAATAA CAACCAAAAT ACTGAATCTG 1380

ATGTACATAC AGGTTTCTAC AGGAAGAGAT GGTATAATTT ACAATTTGGA GATTTAATAA 1440

CCAGGGCTAC CCAGAAAAAG TGACTTGATA ACATGGTACC AATAAGTAAG GGATGCTCTC 1500

TCGGTTTGCT TTTGCCACTT TCAAGATTTT AACTTCTCAG GTTATTAATC AAAATTATTG 1560

TATAAGTTAG CCAATAGAAT TTTTAGGTTA AAACAACAGA TGGGGGGTTT GTGGAGTGTT 1620

TAATGTCATG GGCATTTTTA GTAGCATAGA CCCTTTGTTC TGCATTTGAA TGTTTCGTAT 1680

ATTTTTGTTT CACAGTTAAT CTTCCCTCCC CAAGTTTGCT ATTCAAATCA ACTGCCTGAA 1740

TGACATTTCT AGTAGTCTGA TGTATTTTTC TGAGGAATAG TTTGTGATTC CAATGCAGGT 1800

GTCTTCATTA CCATTACCTC TACACTGCAG AAGAAGCAAA ACTCCTTTAT TAGAATTACT 1860

GCACATGTGT ATGGGGAAAA TAGTTCTGAA AGGCTAGAAT GATACAAGTG AGCAAAAGTT 1920

GGTCAGCTTG GCTATGGAGT GGTGGCAATA ATCTCTAAAC ATTCCAAAAG ACCATGAGCT 1980

GAACCTAAAC TCCCTTGGAA TCTGAACAAA GGAATATAAA ATTGCCATTT GAAAACTGAC 2040

CAGCTAATCT GGACCTCAGA GATAGATCAG CCAGTGGCCC AAAGCCATTT CAAGTACAGA 2100

AATTATAGAG ACTACAGCTA AATAAATTTG AACATTAAAT ATAATTTTAC CACTTTTTGT 2160

CTTTATAAGC ATATTTGTAA ACTCAGAACT GAGCAGAAGT GACTTTACTT TCTCAAGTTT 2220

GATACTGAGT TGACTGTTCC CTTATCCCTC ACCCTTCCCC TTCCCTTTCC TAAGGCAATA 2280

GTGCACAACT TAGGTTATTT TTGCTTCCGA ATTTGAATGA AAAACTTAAT GCCATGGATT 2340

TTTTTCTTTT GCAAGACACC TGTTTATCAT CTTGTTTAAA TGTAAATGTC CCCTTATGCT 2400

TTTGAAATAA ATTTCCTTTT GTAATTTTAA AAAAAA 2436

(2) INFORMATION FOR SEQ ID NO: 277:

(i) SEQUENCE CHARACTERISTICS:
(A) LENGTH: 782 base pairs
(B) TYPE: nucleic acid
(C) STRANDEDNESS: double
(D) TOPOLOGY: linear

(xi) SEQUENCE DESCRIPTION: SEQ ID NO: 277:

GCCACTGACT TCTCCCACCC TTCTGTCTCC CCCATAATAG TTTATTTGGT TGGTCTGGAC 60

TCACTTGTGG CCTTTRATTA AATTCCTAAG GGGCCTGAAG AAGACATTTC TACTGCAGAG 120

GGTTAGAGGC ACTTGAGCAA GGCCCCCACA TCCCAACTCT GGGAGTTGTG GTGGGAGGAG 180

GCACTTCTGG GGGATAGGAC CAGACAAGAT AACAGGAGCT CACATGGNAA GCAGAAGCTG 240

TGACAAGTTT AGTAGTCCCA AAATGGGTTA TATCCCTTCC CCCTTTACAT CAGAATCTTG 300

TGAAATGGGA AAACAACAGA AGGAGGGGAT CAAAGATAGC TGATCTCACA TGCTTCCCAG 360

GCAGGGCARA GGTGGGAGTC AAACCCGGGT GACAGGTGGG TGGAGAGCCC TGTTTGAGGT 420

TGTGGCTGAT CCCTCTCTGG TATTAGTTTT TCCCCTGGGA GCAGGAAGCC CTAGGAAGAG 480

GGGACTGCAG GGTCCCCRGG GGATCTTTCC TCCCTCCCCT GCATGAGGCA GAGGCAAGCT 540

GCCTGCCAAC CCCCTCCCTC AAGGAATGGC CTTGCCCAGG AATGCCCACC ACACATACCC 600

TCTTCTTTTT TTCTAGTCAA ACTCTTGTTT ATTCCTTGGC TTGCCTCCCT CCTTCCTCCC 660

CTCTCAACCT TTACTTCTGA TTTCTATTTC ATGGAATTTG GGATTGAAGT TAAACTACAA 720

CAGTGCCGCC AACACCAAGT CTTGCAGGAA AAAAATACAA AGAAATTTAA CAAAAAAAAA 780

AA 782

(2) INFORMATION FOR SEQ ID NO: 278:

(i) SEQUENCE CHARACTERISTICS:
(A) LENGTH: 961 base pairs
(B) TYPE: nucleic acid
(C) STRANDEDNESS: double
(D) TOPOLOGY: linear

(xi) SEQUENCE DESCRIPTION: SEQ ID NO: 278:

GAGTTCCGGC TGGAGACCCG TGCTCTGGGC CGGCGCCTTC ACCATGGCCT CGGCAGAGCT 60

GGACTACACC ATCGAGATCC CGGATCAGCC CTGCTGGAGC CAGAAGAACA GCCCCAGCCC 120

AGGTGGGAAG GAGGCAGAAA CTCGGCAGCC TGTGGTGATT CTYTTGGGCT GGGGTGGCTG 180

CAAGGACAAG AACCTTGCCA AGTACAGTGC CATCTACCAC AAAAGGGGCT GCATCGTAAT 240

CCGATACACA GCCCCGTGGC ACATGGTCTT CTTCTCCGAG TCACTGGGTA TCCCTTCACT 300

TCGTGTTTTG GCCCAGAAGC TGCTCGAGCT GCTCTTTGAT TATGAGATTG AGAAGGAGCC 360

CCTGCTCTTC CATGTCTTCA GCAACGGTGG CGTCATGCTG TACCGCTACG TGCTGGAGCT 420

CCTGCAGACC CGTCGCTTCT GCCGCCTGCG TGTGGTGGGC ACCATCTTTG ACAGCGCTCC 480

TGGTGACAGC AACCTGGTAG GGGCTCTGCG GGCCCTGGCA GCCATCCTGG AGCGCCGGGC 540

CGCCATGCTG CGCCTGTTGC TGCTGGTGGC CTTTGCCCTG GTGGTCGTCC TGTTCCACGT 600

CCTGCTTGCT CCCATCACAG CCCTCTTCCA CACCCACTTC TATGACAGGC TACAGGACGC 660

GGGCTCTCGC TGGCCCGAGC TCTACCTCTA YTCGAGGGCT GACGAAGTAG TCCTGGCCAG 720

AGACATAGAA CGCATGGTGG AGGCACGCCT GGCACGCCGG GTCCTGGCGC GTTCTGTGGA 780

TTTCGTGTCA TCTGCACACG TCAGCCACCT CCGTGACTAC CCTACTTACT ACACAAGCCT 840

CTGTGTCGAC TTCATGCGCA ACTGCGTCCG CTGCTGAGGC CATTGCTCCA TCTCAMCTCT 900

GCTCCAGAAA TAAATGCCTG ACAMCTCCCC ACAAAAAAAA AAAAAAAAAA ACTCGAGGGG 960

G                                                                 961

(2) INFORMATION FOR SEQ ID NO: 279:

(i) SEQUENCE CHARACTERISTICS:
(A) LENGTH: 1228 base pairs
(B) TYPE: nucleic acid
(C) STRANDEDNESS: double
(D) TOPOLOGY: linear

(xi) SEQUENCE DESCRIPTION: SEQ ID NO: 279:

CGCGCTTTGC AGTTCGGTCT CCTGGTGTAC GGCCAACGCC AAGTAGGGGA TTGCGTTCCC 60

TCCAGTCGCA GCCCTATCAG ATTTGGATAT GTCCTTCATA TTTGATTGGA TTTACAGTGG 120

TTTCAGCAGT GTGCTACAGT TTTTAGGATT ATATAAGAAA ACTGGTAAAC TGGTATTTCT 180

TGGATTGGAT AATGCAGGAA AAACAACATT GCTACACATG CTAAAAGATG ACAGACTTGG 240

ACAACATGTC CCAACATTAC ATCCCACTTC CGAAGAACTG ACCATTGCTG GCATGACGTT 300

TACAACTTTT GATCTGGGTG GACATGTTCA AGCTCGAAGA GTGTGGAAAA ACTACCTTCC 360

TGCTATCAAT GGCATTGTAT TTCTGGTGGA TTGTGCAGAC CACGAAAGGC TGTTAGAGTC 420

AAAAGAAGAA CTTGATTCAC TAATGACAGA TGAAACCATT GCTAATGTGC CTATACTGAT 480

TCTTGGGAAT AAGATCGACA GACCTGAAGC CATCAGTGAA GAGAGGTTGC GAGAGATGTT 540

TGGTTTATAT GGTCAGACAA CAGGAAAGGG GAGTATATCT CTGAAAGAAC TGAATGCCCG 600

ACCCTTAGAA GTTTTCATGT GTAGTGTGCT CAAAAGACAA GGTTACGGAG AAGGCTTCCG 660

CTGGATGGCA CAGTACATTG ATTAACACAA ACTCACATTG GTTCCAGGTC TCAACGTTCA 720

GGCTTACTCA GAGATTTGAT TGCTCAACAT GCATAACTTG AATTCAATAG ACTTTTGCTG 780

GTTATAAAAC AGATGTTTTT TAGATTATTA ATATTAAATC AACTTAATTT GAATGAGAAT 840

TGAAAACTGA TTCAAGTAAG TTTGAGTATC ACAATGTTAG CTTTCTAATT CCATAAAAGT 900

ACTTGGTTTT TACAGTTTAT AATCTGACAT CACCCCAGCG CCATTTGTAA AGAGCAACTT 960

TCCAGCAGTA CATTTGAAGC ACTTTTTAAC AACATGAAAC TATAAACCAT ATTTAAAAGC 1020

TCATCATGTT AAATTTTTTA TGTACTTTTC TGGAACTAGT TTTTAAATTT TAGATTATAT 1080

GTCCACCTAT CKTAAGTGTA CAGTTAATAA TTAGCTTATT CAATGATTGC ATGATGCCTT 1140

ACAGTTTTCA ATAACTTTTT TTCTTATGCA AACGTCATGC AATAAAACAA ACTCTAATGT 1200

TTGGCAAAAA AAAAAAAAAA AAANTCGA 1228

(2) INFORMATION FOR SEQ ID NO: 280:

(i) SEQUENCE CHARACTERISTICS:
(A) LENGTH: 1327 base pairs
(B) TYPE: nucleic acid
(C) STRANDEDNESS: double
(D) TOPOLOGY: linear

(xi) SEQUENCE DESCRIPTION: SEQ ID NO: 280:

TCTCGGGTCT CGGGACAGGT GAGCACCCTG ATGAAGGCCA CGGTCCTGAT GCGGCACCTG 60

GGCGGGTGCA GGAGATCGTG GGCGCCCTCC GCAAGGGCGS CGGAGACCGG TTACAGGTGA 120

TTTCTGATTT TRACATGACC TTGAGCAGGT TTGCATATAA TGGAAAGCGA TGCCCTTCTT 180

CTTACAATAT TCTGGATAAT AGCAAGATCA TCAGTGAGGA GTGTCGGAAA GAGCTCACAG 240

CGCTCCTTCA CCACTATTAC CCAATTGAGA TCGACCCACA CCGGACCGTC AAGGAGAAGC 300

TACCTCATAT GGTGGAATGG TGGACCAAAG CGCACAATCT CCTATGTCAG CAGAAGATTC 360

AGAAGTTTCA GATAGCCCAG GTGGTTAGAG AGTCCAATGC AATGCTCAGG GAGGGATATA 420

AGACCTTCTT CAACACACTC TACCATAACA ACATTCCCCT TTTCATCTTT TCTGCGGGCA 480

TTGGTGATAT CCTGGAAGAA ATTATCCGAC AGATGAAAGT GTTCCACCCC AACATCCACA 540

TCGTGTCTAA CTACATGGAT TTTAATGAAG ATGGTTTTCT CCAGGGATTT AAGGGCCAGC 600

TGATACACAC ATACAACAAG AACAGCTCTG TGTGTGAGAA CTGTGGTTAC TTCCAGCAAC 660

TTGAGGGCAA AACCAATGTC ATCCTGCTGG GAGACTCTAT CGGGGACCTC ACCATGGCCG 720

ATGGGGTTCC TGGTGTGCAG AACATTCTCA AAATTGGCTT CCTGAATGAC AAGGTGGAGG 780

AGCGGCGGGA NCGCTAACAT GGACTCCTAT GACATCGTGC TGGAGAAGGA CGAGACTCTG 840

GATGTGGTCA ACGGGCTACT GCAGCACATC CTGTGCCNAG GGGGTCCAGC TGGAGATGCA 900

AGGCCCCTGA AGGCGCAGGC TCCNAAGKCC SCTGCAGGCC GTGGTGAGGA GGGGCGCCTC 960

CCCAGAGTCT GCTCCCCCGT GAACACAGAG CAGAGCCAGG GTGGCCAGCA GTGGCTGGGT 1020

CCTTCCGCGC CCCTCCGTCC TCCTTTCCCT GAGCACCTTC ATCACCAGAG GCTTGAAGGA 1080

ACCCCGCCAT GTGGCAGGGC ACAGGCACTG TTCCTGGTGA ACCTTGGACC ACAGCATGTC 1140

AGTGCTCTAG GGATTGTCTA CTCCAGGGAT TTTCTTCAAA ATTTTTAAAC ATGGGAAGTT 1200

CAAACAAATA TAATGTGTGA AACAGATCAA AATTTTTAAA ATGAAAAAAA AGCTGCTCTG 1260

ATTCAGGGGA TGTGGGTCGG GGTAGAACCT GGACCTCTTG GCCTGGGGGC ACATGGGATG 1320

CTTCTAG 1327

(2) INFORMATION FOR SEQ ID NO: 281:

(i) SEQUENCE CHARACTERISTICS:
(A) LENGTH: 799 base pairs
(B) TYPE: nucleic acid
(C) STRANDEDNESS: double
(D) TOPOLOGY: linear

(xi) SEQUENCE DESCRIPTION: SEQ ID NO: 281:

TCACCCTGCC TACAGCGTGG AGCTCAGATG ACTGCGCCCT CCACGGTCAC TGTGAGCAGG 60

TGGTATTCAC AGCCTGCATG ACCCTCACGG CCAGCCCTGG GGTGTTCCCC GTCACTGTGT 120

GGCTTTGGCT GAAGCCTAAT TCCACAGCTC CTTGTTTTTT GAGAGAGACT GAGAGAACCA 180

TAATCCTTGC CTGCTGAACC CAGCCTGGGC CTGGATGCTC TGTGAATACA TTATCTTGCG 240

ATGTTGGGTT ATTCCAGCCA AAGACATTTC AAGTGCCTGT AACTGATTTG TACATATTTA 300

TAAAAATCTA TTCAGAAATT GGTCCAATAA TGCACGTGCT TTGCCCTGGG TACAGCCAGA 360

GCCCTTCAAC CCCACCTTGG ACTTGAGGAC CTACCTGATG GGACGTTTCC ACGTGTCTCT 420

AGAGAAGGAT TCCTGGATCT AGCTGGTCAC GACGATGTTT TCACCAAGGT CACAGGAGCA 480

TTGCGTCGCT GATGGGGTTG AAGTTTGGTT TGGTTCTTGT TTCAGCCCAA TATGTAGAGA 540

ACATTTGAAA CAGTCTGCAC CTTTGATACG GTATTGCATT TCCAAAGCCA CCAATCCATT 600

TTGTGGATTT TATGTGTCTG TGGCTTAATA ATCATAGTAA CAACAATAAT ACCTTTTTCT 660

CCATTTTGCT TGCAGGAAAC ATACCTTAAG TTTTTTTTGT TTTGTTTTTG TTTTTTTGTT 720

TTTTGTTTTC CTTTATGAAG AAAAAATAAA ATAGTCACAT TTTTAATACY AAAAAATGGA 780

CAAAAAAAGT CGAGGGGGG 799

(2) INFORMATION FOR SEQ ID NO: 282:

(i) SEQUENCE CHARACTERISTICS:
(A) LENGTH: 2196 base pairs
(B) TYPE: nucleic acid
(C) STRANDEDNESS: double
(D) TOPOLOGY: linear

(xi) SEQUENCE DESCRIPTION: SEQ ID NO: 282:

AAAGACTCTA ACATCCATGA GCTTGAACAT GAGCAAGAGC CTACTTGTGC CKSCCAGATG 60

GCTGAGCCCT TCCGTACCTT CCGAGATGGA TGGGTCTCCT ACTACAACCA GCCTGTGTTT 120

CTGGCTGGCA TGGGTCTTGC TTTCCTTTAT ATGACTGTCC TGGGCTTTGA CTGCATCACC 180

ACAGGGTACG CCTACACTCA GGGACTGAGT GGTTCCATCC TCAGTATTTT GATGGGAGCA 240

TCAGCTATAA CTGGAATAAT GGGAACTGTA GCTTTTACTT GGCTACGTCG AAAATGTGGT 300

TTGGTTCGGA CAGGTCTGAT CTCAGGATTG GCACAGCTTT CCTGTTTGAT CTTGTGTGTG 360

ATCTCTGTAT TCATGCCTGG AAGCCCCCTG GACTTGTCCG TTTCTCCTTT TGAAGATATC 420

CGATCAAGGT TCATTCAAGG AGAGTCAATT ACACCTACCA AGATACCTGA AATTACAACT 480

GAAATATACA TGTCTAATGG GTCTAATTCT GCTAATATTG TCCCGGAGAC AAGTCCTGAA 540

TCTGTGCCCA TAATCTCTGT CAGTCTGCTG TTTGCAGGCG TCATTGCTGC TAGAATCGGT 600

CTTTGGTCCT TTGATTTAAC TGTGACACAG TTGCTGCAAG AAAATGTAAT TGAATCTGAA 660

AGAGGCATTA TAAATGGTGT ACAGAACTCC ATGAACTATC TTCTTGATCT TCTGCATTTC 720

ATCATGGTCA TCCTGGCTCC AAATCCTGAA GCTTTTGGCT TGCTCGTATT GATTTCAGTC 780

TCCTTTGTGG CAATGGGCCA CATTATGTAT TTCCGATTTG CCCAAAATAC TCTGGGAAAC 840

AAGCTCTTTG CTTGCGGTCC TGATGCAAAA GAAGTTAGGA AGGAAAATCA AGCAAATACA 900

TCTGTTGTTT GAGACAGTTT AACTGTTGCT ATCCTGTTAC TAGATTATAT AGAGCACATG 960

TGCTTATTTT GTACTGCAGA ATTCCAATAA ATGGCTGGGT GTTTTGCTCT GTTTTTACCA 1020

CAGCTGTGCC TTGAGAACTA AAAGCTGTTT AGGAAACCTA AGTCAGCAGA AATTAACTGA 1080

TTAATTTCCC TTATGTTGAG GCATGGAAAA AAAATTGGAA AAGAAAAACT CAGTTTAAAT 1140

ACGGAGACTA TAATGATAAC ACTGAATTCC CCTATTTCTC ATGAGTAGAT ACAATCTTAC 1200

GTAAAAGAGT GGTTAGTCAC GTGAATTCAG TTATCATTTG ACAGATTCTT ATCTGTACTA 1260

GAATTCAGAT ATGTCAGTTT TCTGCAAAAC TCACTCTTGT TCAAGACTAG CTAATTTATT 1320

TTTTTGCATC TTAGTTATTT TTAAAAACAA ATTCTTCAAG TATGAAGACT AAATTTTGAT 1380

AACTAATATT ATCCTTATTG ATCCTATTGA TCTTAAGGTA TTTACATGTA TGTGGAAAAA 1440

CAAAACACTT AACTAGAATT CTCTAATAAG GTTTATGGTT TAGCTTAAAG AGCACCTTTG 1500

TATTTTTATT ATCAGATGGG GCAACATATT GTATGAAGCA TATGTAGCAC TTCACAGCAT 1560

GGTTATCATG TAAGCTGCAG GTAGAAGCAA AGCTGTAAAG TAGATTTATC ACACAATGAC 1620

TGCATACAGA CTTCAAATAT GTCAATAGTT TGGTCATAGA ACCTAGAAGC CAAAAGCCAC 1680

ACAGAAGGGC AAGAATCCCA ATTTAACTCA TGTTATCATC ATTAGTGATC TGTGTTGTAG 1740

AACATGAGGG TGTAAGCCTT CAGCCTGGCA AGTTACATGT AGAAAGCCCA CACTTGTGAA 1800

GGTTTTGTTT TACAAATCAC TTGATTTAAC ACACTCAGGT AGAATATTTT TATTTTTACT 1860

GTTTTATACC CAGAAGTTAT TTCTACATTG TTCTACAGCA AGAATATTCA TAAAAGTATC 1920

CCTTTCAAAT GCCTTTGAGA AGAATAGAAG AAAAAAAGTT TGTATATATT TTAAAAAATT 1980

GTTTTAAAAG TCAGTTTGCA ACATGTCTGT ACCAAGATGG TACTTTGCCT TAACCGTTTA 2040

TATGCACTTT CATGGAGACT GCAATACGTT GCTATGAGCA CTTTCTTTAT CCTTGGAGTT 2100

TAATCCTTTG CTTCATCTTT CTACAGTATG ACATAATGAT TTGCTATGTT GTAAAATCTT 2160

TGTAAAAAAT TTCTATATAA AATATTTGAA ACTTAA 2196

(2) INFORMATION FOR SEQ ID NO: 283:

(i) SEQUENCE CHARACTERISTICS:
(A) LENGTH: 1185 base pairs
(B) TYPE: nucleic acid
(C) STRANDEDNESS: double
(D) TOPOLOGY: linear

(xi) SEQUENCE DESCRIPTION: SEQ ID NO: 283:

GCAGTTAAGG CTTCTGATAA GGAAAGAGAG TCTGAACAGA GCACACACAT CTGGAGCTCC 60

AGGAGTGGGG GATGCAGCAT CAGATTCCAT CTTGAATTTC TGCTAAAATA CTTTGTACTC 120

ATAATGGATC TCAACAAAGA TCTGTATTTC ATCTGTGGCT CCATCTTCCC TCTGGGTCAA 180

GTAGATGTTA AGCTGGACCT TGGCACGCCT CTTAACATGA AGAGATCTAG CTAGACAGAC 240

AGACTCCCCC ATTTATGGGA ACAAGAATTC AATTTATTCT CTATTTATAA AACATTTTTT 300

TAAAGTGCCT TGGGTATAAA AATCTAAATG TCTGCGGTGT GATCAGTCAG GAGCACGTAA 360

CTATCACTCT TCGCATCCTT TGGTCACTGG GAGATCCTTT GGGGGCTGGG AGGTCCTTCT 420

GTCCCAGGCT AAAGGAAAAG CTTCACAAGG GTAAGAGCCA CAGAACCCTC GGCAAGAAAG 480

GCCGGTCAGG GAGAATGAAT GGTACAGAGA GGAAAGGAAG GAAAGGGGGT GGAACAGAGG 540

TAGAAGGCAA GGAAGGGATG CCGCACTGGA GACCGATGGG GACACTCTAA TTGTGCAAGA 600

GGGAGGATCT TCCTTCTTGA ATGCTGAACA CAGCTAGTCT GAACCTTCCT TGGAAAGTCC 660

AGCTGTTTGC CCATGCATAG GGCCAACTCT CCCTGCAAAG CAGCAAATGT GGCTTCTATC 720

AGGAAGGAAA AGTATCCATC AGTGTGACAA GAGGTCACCT TCGAACTTGC ATGAACTCCT 780

TGCGCAGCCA CAAAGAGTCC TGGTAGAAGT GAGGATCGCC TAGTCTTACG GCTGTCCGTT 840

TATAGAAGTA GCAGTACAAC ACTGCTGCTA GTCTCTGGAA TACAAACAGC ATTTGAAGTC 900

CATCTGTCCA TATGAAGCTG TTGGAGTTTT TCCAGCGTAA GTTCATGACC CAGACATGAA 960

GGGAGATGCT GAGGGCAAAG TACACAGCTG TCAGGATGAT GGTCCCTTTG AACTTATGGA 1020

ATAGGAGGTT GACCAGGCCA GCCTGGAAGA CGAAGGTGTT GAAGAACATG AGGAAAATGA 1080

TGATGATGTT GAAGAGGACT GCAATATCCT GGATGCACTG AGGGAGAGGY TTCTAGTTCC 1140

TTTGAATGAG AGCTGTTTCC CTTGCTCTAA GGCAAGCACC TCCAA 1185

(2) INFORMATION FOR SEQ ID NO: 284:

(i) SEQUENCE CHARACTERISTICS:
(A) LENGTH: 1634 base pairs
(B) TYPE: nucleic acid
(C) STRANDEDNESS: double
(D) TOPOLOGY: linear

(xi) SEQUENCE DESCRIPTION: SEQ ID NO: 284:

AGGGAAAGGG GAGGGTAGCG GGAGGGTAGC AGGTGAGTTC CTAGGGCTGG AAGGTTTAGC 60

AGCAGCCTGG TGCAGTGCCC TGTCATCAAG ACAAACCCAC GGTCCTMCTG GGTGCCTACC 120

AAGCTTGGTT TGTACAAAAG CAAGGTGGGA GTCTATTTTT GTACATGAGA TACATCACAC 180

TTACCTGTGG GCCAGTATTG TGAAGTGAGT CTGAGTTGTT TACACTGATG CCTTCCCTGC 240

CCACCACAAA TTGTGTACAT AGTCTTCAGA TGATACCACC CCTTTCCCCA GCTCCCAACC 300

AAGAGCTGGT TCTAGGCCTG TGTTATATGT CATATTTAGC STTTTTATAT ATGACCTTTG 360

ATTTCTGTTG TTTGTATTTT AGCACAGTGT ATGCACCTTC ATTTAAATAC ATCTGTGTGC 420

ATACAGATAC GCATATATGT GTGTGCGTAT GCATATATCT CTCATCTGTA GTTTCCAAGA 480

GTTCAGCTGA AGCAGATGGA GTCCTGCAGC CCAGGAGACA CCCTGCATCC CTGCTAATAG 540

TGTTTGCCAC AAGTATTAGT GAGTCTTCCT TATTAATATT TTCATTTCAG AAGACTGAAG 600

CAAAGCTGAT AGTGTTTGCT GTTTCTTTGG CAGCTAAGTG AGGGTCTTGG GATGACTTGC 660

TGTGTTCCTC AAGCTGCACT TTGGGGCCAT CTCTGCAGTA TTAGCCCCCT TTTTGCTTGG 720

TGGTACTCTG TCTGTGCCTG TGTGTGTGTG TGATAGTCAC TCTTGCATGG CTTCCATGTC 780

TGGTTTGTGG CATTTGGGGA TAAGGTGCTG AAGCCAGAGC ATTTGCAGTT TGTTTGAGGC 840

CTCGTTGCCA ATGATAGATC ACTCCTGTTG ACCTGGTATG TCTGCTTGCT TGCTGCTTTT 900

CCTTGCTTTC TCTTGGAAGA GGAAAGGACT CTGGTCAGGC CCAGGCTGAG TGAGATGAGC 960

TGCAGCTGGC TCATGGCCTT CTTAGAGCAG AGAGAGGAGT ATGTCATTTT ACTAAGTTCC 1020

TAAACAAACA TTTATGCAGG CAACACTCCT TGCAGATCCA GAAACTGAGG CACAATAGGG 1080

TTATGACTTG CTCAAGAATA TGTAGCTGCT AGGGGGTAAA TCAAGGCATC ACAATTTCTG 1140

TTCAGCGGGC AGGAATAGGC TGTGAATTGC TAGCACTTTT TTTTTTTAAG CAATTACTTT 1200

TTGACTTGTT CCTCTGAAAG TGCAAGAGGC GTACACCTTT CCCAAATGTA GACTAGAATC 1260

TGCAGGATGC CACCCACTGT ATAGTTCTGC TTTCCCAGAG AGGAAGAACT TTTAGAAACC 1320

AAATGATCTT AATTGTTATT GCCCACCCCT GGCTTTTCCG GGTAGAAAAT TCACAGTAGG 1380

AATGATTGTT AAGAGAGAGT GCTTGGAACC ATGGGTTAAC AGGAAAGGCT ACCTAACTTC 1440

ACATATCTGC AACCAGAGCA GCCACCAAGC ATTACTTAGC AGCAGGAAAA TGATTGTATT 1500

TGAGTTCCTG TGTGTCCAAA ACTGAGGCAC CATGTTCTTT GAAAACATGC CACCTCAAGG 1560

CTGGGCGCGG TGGCTCACAC CTGTAATCCC AGCAYTTTGG GGAGGCCSAG GGCGGGGCGG 1620

KTTCACCGGG GGTC 1634

(2) INFORMATION FOR SEQ ID NO: 285:

(i) SEQUENCE CHARACTERISTICS:
(A) LENGTH: 1795 base pairs
(B) TYPE: nucleic acid
(C) STRANDEDNESS: double
(D) TOPOLOGY: linear

(xi) SEQUENCE DESCRIPTION: SEQ ID NO: 285:

TTCCCCCCAG GTTGGCTTCC TTCGATTCCT TTTCTTGGTA TCAACGTTTG ATTGGAAGAA 60

CAACCCCCTC TTTGTCAACC TCAATAATGA GCTCACTGTG GAGGAGCAGC TCGGGCACAG 120

CTCMCCGTYA TGGTCATTGT TACCCCCCAA GACCGCAAAA ACTCTGTGTG GACACAGGAT 180

GGACCCTCAG CCCAGATCCT GCAGCAGCTT GTGGTCCTGG CAGCTGAAGC CCTGCCCATG 240

TTAGAGAAGC AGCTCATGGA TCCCCGGGGA CCTGGGGACA TCAGGACAGT GTTCCGGCCG 300

CCCTTGGACA TTTACGACGT GCTGATTCGC CTGTYTCCTC GCCATATCCC GCGGCACCGC 360

AGGCTTGTGG ACTCGCCAGY TGCCTCCTTC TGCCGGGGCC TGCTCAGCCA GCCGGGGCCC 420

TCATCCCTGA TGCCCGTGCT GGGTNATGAT CCTNCTCAGC TCTATCTGAC GCAGCTCAGG 480

GAGGCCTTTG GGGATCTGGC CCTTTTCTTC TATGACCAGC ATGGTGGAGA GGTGATTGGT 540

GTCCTCTGGA AGCCCACCAG CTTCCAGCCG CAGCCCTTCA AGGCCTCCAG CACAAAGGGG 600

CGCATGGTGA TGTCTCGAGG TGGGGAGCTA GTAATGGTGC CCAATGTTGA AGCAATCCTG 660

GAGGACTTTG CTGTGCTGGG TGAAGGCCTG GTGCAGACTG TGGAGGCCCG AAGTGAGAGG 720

TGGACTGTGT GATCCCAGCT CTGGAGCAAG CTGTAGACGG ACAGCAGGAC ATTGGACCTC 780

TAGAGCAAGA TGTCAGTAGG ATGACCTCCA CCCTCCTTGG ACATGAATCC TCCATGGAGG 840

GCCTGCTGGC TGAACATGCT GAATCATCTC CAACAAAACC CAGCCCCAAC TTTCTCTCTG 900

ATGCTCCAGC ATTGGGGCAG GGGCATGGTG GCCCATGTAG TCTCCTGGGC CTCACCATCC 960

CAGAAGAGGA GTGGGAGCCA GCTCAGAGAA GGAACTGAAC CCAGGAGATC CATCCACCTA 1020

TTAGCCCTGG GCCTGGACCT CCCTGCGATT TCCCACTCCT TTCTTAGTCT TCTTCCAGAA 1080

ACAGAGAAGG GGATGTGTGC CTGGGAGAGG CTCTGTCTCC TTCCTGCTGC CAGGACCTGT 1140

GCCTAGACTT AGCATGCCCT TCACTGCAGT GTCAGGCCTT TAGATGGGAC CCAGCGAAAA 1200

TGTGGCCCTT CTGAGTCACA TCACCGACAC TGAGCAGTGG AAAGGGGCTA TATGTGTATG 1260

AATAGACCAC ATTGAAGGAG CACAATGCCC TCCTGTGTTG ATGCCACTTC CCAGGGTGGA 1320

GACAGTGGAA AAGAACCGAG GACAGGAAAG GATTGGGTAG GTGAAGGGGT CAGGGGACTG 1380

GTAGTCACCC AATCTTGGAG AGGTGCAAAA AGCACTGGGG GCTACCCGTT AGCTGCATCT 1440

GCCCTGGCTG TTTGCCCGTT CATGTCACAA ACTGCCACTA CTATGTACCT GCAGTGGGGT 1500

TGCAGAGATG GGGGAGACTC AAGTCTTACT CCCCAGGAGC TCCCAGGGCC CAAGGAGGAG 1560

AATGCTGCCT CCTTTCAGTC TGGTCTACAC CCACTTTCTG GTAGCCTCTC TGCTTCCTGT 1620

AATTCTGGCT GTTTTTCCAG ACTCAGCTCA AATAGTGCCC CTCCTTAAGC CCATCCCTCG 1680

CCCCCAGCCT GAGGTGATCT TTCCCTCCTC TGAACTATTA GAGCAGTTAC TGTCTGTTCA 1740

GTTCGTTTGG CAGGCACACA CAGTGGCATA AATTCTATTG TTTTGAACTC TGATT 1795

(2) INFORMATION FOR SEQ ID NO: 286:

(i) SEQUENCE CHARACTERISTICS:
(A) LENGTH: 858 base pairs
(B) TYPE: nucleic acid
(C) STRANDEDNESS: double
(D) TOPOLOGY: linear

(xi) SEQUENCE DESCRIPTION: SEQ ID NO: 286:

TCTGCTTTCG GTGCTGCGTG TACTGCTGGG CGGCTTCTTC GCGCTCGTGG GGTTGGCCAA 60

GCTCTCGGAG GAGATCTCGG CTCCAGTTTC GGAGCGGATG AATGCCCTGT TCGTGCAGTT 120

TGCTGAGGTG TTCCCGCTGA AGGTATTTGG CTACCAGCCA GATCCCCTGA ACTACCAAAT 180

AGCTGTGGGC TTTCTGGAAC TGCTGGCTGG GTTGCTGCTG GTCATGGGCC CACCGATGCT 240

GCAAGAGATC AGTAACTTGT TCTTGATTCT GCTCATGATG GGGGCTATCT TCACCTTGGC 300

AGCTCTGAAA GAGTCACTAA GCACCTGTAT CCCAGCCATT GTCTGCCTGG GGTTCCTGCT 360

GCTGCTGAAT GTCGGCCAGC TCTTAGCCCA GACTAAGAAG GTGGTCAGAC CCACTAGGAA 420

GAAGACTCTA AGTACATTCA AGGAATCCTG GAAGTAGAGC ATCTCTGTCT CTTTATGCCA 480

TGCAGCTGTC ACAGCAGGAA CATGGTAGAA CACAGAGTCT ATCATCTTGT TACCAGTATA 540

ATATCCAGGG TCAGCCAGTG TTGAAAGAGA CATTTTGTCT ACCTGGCACT GCTTTCTCTT 600

TTTAGCTTTA CTACTCTTTT GTGAGGAGTA CATGTTATGC ATATTAACAT TCCTCATGTC 660

ATATGAAAAT ACAAAATAAG CAGAAAAGAA ATTTAAATCA ACCAAAATTC TGATGCCCCA 720

AATAACCACT TTTAATGCCT TGGTGTAAGT ATACCTCTGA ACTTTTTTCT GTGCCTTTAA 780

ACAGATATAT ATTTTTTTTT AATGAAAATA AAACCATATA TCCTATTTTA TTTCCTCCTT 840

TTAAAACCTT ATAAACTA 858

(2) INFORMATION FOR SEQ ID NO: 287:

(i) SEQUENCE CHARACTERISTICS:
(A) LENGTH: 915 base pairs
(B) TYPE: nucleic acid
(C) STRANDEDNESS: double
(D) TOPOLOGY: linear

(xi) SEQUENCE DESCRIPTION: SEQ ID NO: 287:

GAATTCGGCA CGAGCGCGGC CATGGCGCTC CTGCTTTCGG TGCTGCGTGT ACTGCTGGGC 60

GGCTTCTTCG CGCTCGTGGG GTTGGCCAAG CTCTCGGAGG AGATCTCGGC TCCAGTTTCG 120

GAGCGGATGA ATGCCCTGTT CGTGCAGTTT GCTGAGGTGT TCCCGCTGAA GGTATTTGGC 180

TACCAGCCAG ATCCCCTGAA CTACCAAATA GCTGTGGGCT TTCTGGAACT GCTGGCTGGG 240

TTGCTGCTGG TCATGGGCCC ACCGATGCTG CAAGAGATCA GTAACTTGTT CTTGATTCTG 300

CTCATGATGG GGGCTATCTT CACCTTGGCA GCTCTGAAAG AGTCACTAAG CACCTGTATC 360

CCAGCCATTG TCTGCCTGGG GTTCCTGCTG CTGCTGAATG TCGGCCAGCT CTTAGCCCAG 420

ACTAAGAAGG TGGTCAGACC CACTAGGAAG AAGACTCTAA GTACATTCAA GGAATCCTGG 480

AAGTAGAGCA TCTCTGTCTC TTTATGCCAT GCAGCTGTCA CAGCAGGAAC ATGGTAGAAC 540

ACAGAGTCTA TCATCTTGTT ACCAGTATAA TATCCAGGGT CAGCCAGTGT TGAAAGAGAC 600

ATTTTGTCTA CCTGGCACTG CTTTCTCTTT TTAGCTTTAC TACTCTTTTG TGAGGAGTAC 660

ATGTTATGCA TATTAACATT CCTCATGTCA TATGAAAATA CAAAATAAGC AGAAAAGAAA 720

TTTAAATCAA CCAAAATTCT GATGCCCCAA ATAACCACTT TTAATGCCTT GGTGTAAGTA 780

TACCTCTGAA CTTTTTTCTG TGCCTTTAAA CAGATATATA TTTTTTTTWA ATGAAAATAA 840

AACCATATAT CCTATTTTAT TTCCTCCTTT TAAAACCTTA TAAACTATAA MAAAAAAAAA 900

AAAAAAAAAA CTCGA 915

(2) INFORMATION FOR SEQ ID NO: 288:

(i) SEQUENCE CHARACTERISTICS:
(A) LENGTH: 1517 base pairs
(B) TYPE: nucleic acid
(C) STRANDEDNESS: double
(D) TOPOLOGY: linear

(xi) SEQUENCE DESCRIPTION: SEQ ID NO: 288:

CCTTGTGGCA ACTAGTGGGT CCCCCGGGCT GCAGNAATTC GGGCAGTGGT TCTGNGTCTG 60

AAGATACTCT GAGTTCCTCT GAGAGATCCA AAGGCTCCGG GAGCAGACCC CCAACCCCCA 120

AAAGCAGCCC TCAGAAGACC AGGAAGAGCC CTCAGGTGAC CAGGGGTAGC CCTCAGAAGA 180

CCAGCTGTAG CCCTCAGAAG ACCAGGCAGA GCCCTCAGAC GCTGAAGCGG AGCCGAGTGA 240

CCACCTCACT TGAAGCTTTG CCCACAGGAC AGTGCTGACA GACAAGAGTG GGCGACAGTG 300

GAAGCTGAAG TCCTTCCAGA CCAGGGACAA CCAGGGCATT CTCTATGAAG CTGCACCCAC 360

CTCCACCCTC ACCTGTGACT CAGGACCACA GAAGCAAAAG TTCTCACTCA AACTGGATGC 420

CAAGGATGGG CGCTTGTTCA ATGAGCAGAA CTTCTTCCAG CGGGCCGCCA AGCCTCTGCA 480

AGTCAACAAG TGGAAGAAGC TGTACTCGAC CCCACTGCTG GCCATCCCTA CCTGCATGGG 540

TTTCGGTGTT CACCAGGACA AATACAGGTT CTTGGTGTTA CCCAGCCTGG GGAGGAGCCT 600

TCAGTCGGCC CTGGATGTCA GCCCAAAGCA TGTGCTGTGC AGAGAGGTCT GTGCTGCAGG 660

TGGCCTGCCG GCTGCTGGAT GCCCTGGAGT TCCTCCATGA GAATGAGTAT GTTCATGGAA 720

ATGTGACAGC TGAAAATATC TTTGTGGATC CAGAGGACCA GAGTCAGGTG ACTTTGGCAG 780

GCTATGGCTT CGCNTTCCGC TATTGCCCAA GTGGCAAACA CGTGGCCTAC GTGGAAGGCA 840

GCAGGAGCCY TCACGAGGGG GACCTTGAGT TTCATTAGCA TGGACCTGCA CAAGGGATGC 900

GGGCCCTCCC GCCGCRGYGA CCTCCAGAGC CTGGGYTAMT GCATGCTGAA GTGGYTCTAM 960

GGGTTTCTGC CATGGACAAA TTGCCTTCCA AMAMTGAGGA CATCATGAAG CAAAAACAGA 1020

AGTTGCCTTG GGATTCATTT TAATGTAAGC TKGACTTTGT CATGCCAGAA ACAAGGCTCG 1080

GTCACCGTCA GCAGTTTGCA GTTTTCCACC TCCWCCCAGT TCCTCCGTGT GGTTGACCCA 1140

GATATCTCCG TTATGCAGCC GCCTCCGGGG GACCACCTCC CTCCCTTTGA GTCAGCCACA 1200

GACAGCCTAC TTGACGGCCC CGCTGGCCCC CACATTCCAC TGAACTGTGC GGATGCCACA 1260

GTGACCCCCT CTCAGGCACA GCATGACCTC CTGAAGTCGA GCCTGCTTGC TTTGAACCTA 1320

CCAGTTAAAA TCTCCTCAAA ATGTTTGGAT ACCGCCCATT GGCCCCTCAC AGCCACGAGC 1380

TCCCTGACCA GTGTGCGTGT GTGTGTGTGT GTGTGTCTGT GTGTGTGCTT GGGACGGGTG 1440

GGGAGGTCAC CTTTGGGTGT GCGGTGTGCC CCCAGGACCT GTAAGTAATA AAATCTTTAT 1500

TTCCAAAAAA AAAAAAA 1517

(2) INFORMATION FOR SEQ ID NO: 289:

(i) SEQUENCE CHARACTERISTICS:
(A) LENGTH: 3865 base pairs
(B) TYPE: nucleic acid

(C) STRANDEDNESS: double
(D) TOPOLOGY: linear

(xi) SEQUENCE DESCRIPTION: SEQ ID NO: 289:

TGGAGGGGGG GAGCTTCCTT GAGCAGTGGG CCCAGGCCTG GCCCTCCACA CTTCATTCTC 60

TGACCTTTCT CTCTCCTCAT TTCGGTGCAT GTCCTTTCTG CAGCTGCCTT TCAGCACAGG 120

TGGTTCCACT GGGGGCAGCT AACGCTGAGT GACAAGGATG GGAAGCCACA GGTGCATTTT 180

ACTCAAGTCT TCTCTAGTCA ATGAGGGGCA CCCAGTGCTT CTAGGGCAGG CTGGGTGGTG 240

GTCCCCTAGG TATCAGCCTC TCTTACTGTA CTCTCCGGGA ATGTTAACCT TTCTATTTTC 300

AGCCTGTGCC ACCTGTCTAG GCAAGCTGGC TTCCCCATTG GCCCCTGTGG GTCCACAGCA 360

GCGTGGCTSC CCCCCAGGGC CACCGCTTCT TTCTTGATCC TCTTTCCTTA ACAGTGACTT 420

GGGCTTGAGT CTGGCAAGGA ACCTTGCTTT TAGCTTCACC ACCAAGGAGA GAGGTTGACA 480

TGACCTCCCC GCCCCCTCAC CAAGGCTGGG AACAGAGGGG ATGTGGTGAG AGCCAGGTTC 540

CTCTGGCCCT CTCCAGGGTG TTTTCCACTA GTCACTACTG TCTTCTCCTT GTAGCTAATC 600

AATCAATATT CTTCCCTTGC CTGTGGGCAG TNGGAGAGTG CTGCTGGGTG TACGCTGCAC 660

CTGCCCACTG AGTTGGGGAA AGAGGATAAT CAGTGAGCAC TGTTCTGCTC AGAGCTCCTG 720

ATCTACCCCA CCCCCTAGGA TCCAGGACTG GGTCAAAGCT GCATGAAACC AGGCCCTGGC 780

AGCAACCCTG GGAATGGCTG GAGGTGGGAG AGAACCTGAC TTCTCTTTCC CTCTCCCTCC 840

TCCAACATTA CTGGAACTCT ATCCTGTTAG GATCTTCTGA GCTTGTTTCC CTGCTGGGTG 900

GGACAGAGGA CAAAGGAGAA GGGAGGGTCT AGAAGAGGCA GCCCTTCTTT GTCCTCTGGG 960

GTAAATGAGC TTGACCTAGA GTAAATGGAG AGACCAAAAG CCTCTGATTT TTAATTTCCA 1020

TAAAATGTTA GAAGTATATA TATACATATA TATATTTCTT TAAATTTTTG AGTCTTTGAT 1080

ATGTCTAAAA ATCCATTCCC TCTGCCCTGA AGCCTGAGTG AGACACATGA AGAAAACTGT 1140

GTTTCATTTA AAGATGTTAA TTAAATGATT GAAACTTGGC TGTGGCTACT GCTTCTTAAT 1200

GTTGGGGGGA CAGGGCAGTG GTCTGGGCCC ACATTTAGAA GGGAAAATGT TTTGCCTGCT 1260

GCACACATTG GACCCAAGTA TGGGCCTCTT CTGCCTAGTA CTGCCAAAGG GACTGTTAAG 1320

GTGTCTTGTC CATCTTCTAC CCCCCACCCC CCATTACGGG TAAAGGRAAC CCCAGACTAG 1380

GTGAGGGGCC AGCAGCTGCC TCACATTGTG TTCTCTCCTG AGATGGTCCA GCTCACATCC 1440

AGACACCTTG TTCAGACATT TTATTTGAAT TTATGACAGT GATGGGGATT TGACTGAGAT 1500

GCCTTATGGA GAAGTACCCC ACCCTCTATG AAGACAGAAT CACTCTCTGC CATTCATTCT 1560

GCCTGATGCT AACAACACGC AGCTGATTTA GGGAGTGTCC CAGCCTAGCT GGATCAAGGG 1620

AAATTCCAGG AGCCCTGGGG CAGGCCCTGG NCCCCAGTGC CAAGCCTCAG AGTAAGCAGA 1680

CATTGGGAAA GTTGCCAACC ACTTGGTAGA CCACTAGGTT CTCTGTTTTC CCTTCCCTTT 1740

CCTTTTCAAA TCCCACAGTT TCCTGTTGGG GAGAAGCTGT AATTAGCCTA GTCCAGGTAC 1800

CAGATCCCAG CTAGGGGCGC AGCTGNCTTG GATAACTCCA AGAAAACCTG GGCACCAGTA 1860

TTTTTCCAAT TATAAGGACT GTGGCATAAA TTTTTAAATG AGTTATATTG AAACCAGATT 1920

TCTCCAGCTG CCAAGGGAAG AAGGTAGGGC TGGACTCCCT GCTGTGGCCC AGCCCTTGTT 1980

AGGGGTTGGT CTCTCACTGC AGCCAGACAG GATGATCCTG GGTTCTGGGG AGGGTAAGCT 2040

GCCCCTTGCC GAGTTCTGCA CCGAATAAAG AGTCCAAACC CGCTGCTTCC GTGTCCTGAG 2100

AGATGGGTAA ATGGGTGATG GATGGAGCAG ACTGAAGAGA CAGCAGATGA CTCAGTGGTG 2160

GAAGAAGGGG GGAAGATGCT GGGCTGGCTA GCTAATGTTC CCCCCTTTCA GCGATTTACA 2220

GGAAATGGAG CCCAGCTTGG TCATGAAGTT GGTTTGCTTC CACTGTGCGA TGCACTCCTC 2280

AGAAATTTTG AAGTCAGCCT GCAACTTCTC GAAGACTTTC TTCTTGGGCT TGAGCTCCTC 2340

ATCTGGTTGG CCCTTTTCAT AGCCCTTCAC AAACACGTGC TCACCAGGAG CAGAGCCTGC 2400

CGGAGGGTCC AGAGGTTCAA CTGGCGGTTT ATCCCTTCTA TAGAAGCACA CAGAAGCATG 2460

CCTTGGGACT CGACTCCTCT CATCTTCTGG GGTTTCAGGT TGCACAGCAC CACTACCAGC 2520

CTGTCCTGCA GTTCCTCCTT GGGCACGAAC TGTACCAGGC CGCTCACCAC AGTCCGTGGT 2580

TCAGCTTCCC CCACGTCAAT CTTCTCTACA TACAGGCTGT CTGCATCTGG GTGCTTCTCC 2640

ACAGTGATGA TTTTCCCCAC ACGGATATCC AGCCGGGATG GGATGACCTC CTCTGGTTCT 2700

GAATTCTTGG CAGGCCTTTG GCCATTGGCT TCTGCTTTGA GGGATCTGGG TAGGCAGCGC 2760

TGGCCAGTTT TTTCAGGGCA GGGGTATTAA ACTTTTCCCG GATTGGATCC AGCAACTTGT 2820

TCAGTGCGAC TTCAACAGAA TTCTTCAGGT CTCCAGGATG TACAACCTCA GCAGCAAAGT 2880

CCTTTTCCAG GTCCACGTAA GCTGTGTAGG TTTTGTTTCC ACCCCATTTC TCATCTCGTA 2940

GGATCACAAA CTCGGACTTA AGGGGAAAAA GGACATGCTT GATGAAGGAC AGAACCCCAT 3000

TGTTCTCCAC ATTTCCTGGC TCACAGAAGG CCTTCTTCAG TTTTTTCTTC ACATCCTCCT 3060

TCCGATCAAG GAGATCAATC TTGGACTCCT CTTCTGAAGA GCTCATTTTG CTGCCTGTTA 3120

ATCCTGGAAC CATAGGATTC ATCAGATGGA CCCGTTTTGA ATAGCCAAGT GCAGGGAGGT 3180

ACTTCTCTGC AAAGGTGAAA ATCTTTCTCT GATCAATGCC TCCAAATTGG GCATCTACTT 3240

TTAAATACTC TTCATCCAAA GCCTGCAGTC CGGGGTATAA GAGGCCACTC AGCAAAGGGT 3300

GCTCCACCTG CTTTACCACC TCAGCTCCAG CCTTCTTGGA ATCGTGCTGT GTGACCACGG 3360

AGGAGAGTCT GTACACATCT AGTGTGTACT CTTTGCTGAG CTGGTAATCA GTGCCTTTGA 3420

TGAACTTGAG CTTCTCCAAG GGCACACCAA TGCTCTCCAG CATTGCTTTG ATCACATTCT 3480

CATAGTAACT GACTCGGAGT TCTAGAAGTT CCCATGGGGC TTTCATGTTA TCCAGGTATG 3540

CGTGGAGGTC CGCAAACAGA ATTGTTACCT CACACCCTGC CTTTAAGAAG TCTGCAATCT 3600

TTGACATGGG CACAAAGTAA GCCACATGTG GTTTGCCCGT GGTTGCCGTT CCCCAGTAAA 3660

TTTTAAGTTC CCGCTCCTTC AGTATCTCCT TCAGCTTCTC TTCCCCCAGA ACCTCCTGCA 3720

GGTTCCGGGT GATAAGGTGC AGTTTCTCTT CAGGGCTGGG AGCGTCCCCC ATGGTCCGCT 3780

ACCCCTGCTT CCCCCGCTCA GCCCGGCACC AGAGCCCCTT CCTGGGTCAC CGTCGCCGCC 3840

GCGTGCCGGG AACTGTCACG CGAGT 3865

(2) INFORMATION FOR SEQ ID NO: 290:

(i) SEQUENCE CHARACTERISTICS:
(A) LENGTH: 1910 base pairs
(B) TYPE: nucleic acid
(C) STRANDEDNESS: double
(D) TOPOLOGY: linear

(xi) SEQUENCE DESCRIPTION: SEQ ID NO: 290:

AGGGAGAGGA GGAGAGGGGG TCTGCGCGCG GCCGCTACCC AGAAGCCAGC GGACGGCAGC 60

ACGGAGTGGG CTGTCCCCGA GCCCAGCCCC GAGCGAGCCC CCCCCCCGCC CCCGMAGGAC 120

GCGCCTYCCA GCCAGCCCGA CTCCTAGGAG GAGGGGAGGC GGGAAAGCAG CTCAAGCCTC 180

ACCCACCGCC CTGCCCCCAG CCCCGCCACT CCCAGGCTCC TCGGGACTCG GCGGGTCCTC 240

CTGGGAGTCT CGGAGGGGAC CGNCTGTGCA GACGCCATGG AGTTGGTGCT GGTCTTCCTC 300

TGCAGCCTGC TGGCCCCCAT GGTCCTGGCC AGTGCAGCTG AAAAGGAGAA GGAAATGGAC 360

CCTTTTCATT ATGATTACCA GACCCTGAGG ATTGGGGGAC TGGTGTTCGC TGTGGTCCTC 420

TTCTCGGTTG GGATCCTCCT TATCCTAAGT CGCAGGTGCA AGTGCAGTTT CAATCAGAAG 480

CCCCGGGCCC CAGGAGATGA GGAAGCCCAG GTGGAGAACC TCATCACCGC CAATGCAACA 540

GAGCCCCAGA AAGCAGAGAA CTGAAGTGCA GCCATCAGGT GGAAGCCTCT GGAACCTGAG 600

GCGGCTGCTT GAACCTTTGG ATGCAAATGT CGATGCTTAA GAAAACCGGC CACTTCAGCA 660

ACAGCCCTTT CCCCAGGAGA AGCCAAGAAC TTGTGTGTCC CCCACCCTAT CCCCTCTAAC 720

ACCATTCCTC CACCTGATGA TGCAACTAAC ACTTGCCTCC CCACTGCAGC CTGCGGTCCT 780

GCCCACCTCC CGTGATGTGT GTGTGTGTGT GTGTGTGTGT GACTGTGTGT GTTTGCTAAC 840

TGTGGTCTTT GTGGCTACTT GTTTGTGGAT GGTATTGTGT TTGTTAGTGA ACTGTGGACT 900

CGCTTTCCCA GGCAGGGGCT GAGCCACATG GCCATCTGCT CCTCCCTGCC CCCGTGGCCC 960

TCCATCACCT TCTGCTCCTA GGAGGCTGCT TGTTGCCCGA GACCAGCCCC CTCCCCTGAT 1020

TTAGGGATGC GTAGGGTAAG AGCACGGGCA GTGGTCTTCA GTCGTCTTGG GACCTGGGAA 1080

GGTTTGCAGC ACTTTGTCAT CATTCTTCAT GGACTCCTTT CACTCCTTTA ACAAAAACCT 1140

TGCTTCCTTA TCCCACCTGA TCCCAGTCTG AAGGTCTCTT AGCAACTGGA GATACAAAGC 1200

AAGGAGCTGG TGAGCCCAGC GTTGACGTCA GGCAGGCTAT GCCCTTCCGT GGTTAATTTC 1260

TTCCCAGGGG CTTCCACGAG GAGTCCCCAT CTGCCCCGCC CCTTCACAGA GCGCCCGGGG 1320

ATTCCAGGCC CAGGGCTTCT ACTCTGCCCC TGGGGAATGT GTCCCCTGCA TATCTTCTCA 1380

GCAATAACTC CATGGGCTCT GGGACCCTAC CCCTTCCAAC CTTCCCTGCT TCTGAGACTT 1440

CAATCTACAG CCCAGCTCAT CCAGATGCAG ACTACAGTCC CTGCAATTGG GTCTCTGGCA 1500

GGCAATAGTT GAAGGACTCC TGTTCCGTTG GGGCCAGCAC ACCGGGATGG ATGGAGGGAG 1560

AGCAGAGGCC TTTGCTTCTC TGCCTACGTC CCCTTAGATG GGCAGCAGAG GCAACTCCCG 1620

CATCCTTTGC TCTGCCTGTC GGTGGTCAGA GCGGTGAGCG AGGTGGGTTG GAGACTCAGC 1680

AGGCTCCGTG CAGCCCTTGG GAACAGTGAG AGGTTGAAGG TCATAACGAG AGTGGGAACT 1740

CAACCCAGAT CCCGCCCCTC CTGTCCTCTG TGTTCCCGCG GAAACCAACC AAACCGTGCG 1800

CTGTGACCCA TTGCTGTTCT CTGTATCGTG ATCTATCCTC AACAACAACA GAAAAAAGGA 1860

ATAAAATATC CTTTGTTTCM TAAAAAAAAA AAAAAAAAAA AGGGGGGGGG 1910

(2) INFORMATION FOR SEQ ID NO: 291:

(i) SEQUENCE CHARACTERISTICS:
(A) LENGTH: 3276 base pairs
(B) TYPE: nucleic acid
(C) STRANDEDNESS: double
(D) TOPOLOGY: linear

(xi) SEQUENCE DESCRIPTION: SEQ ID NO: 291:

GCGACCGTCG TTTGAGTCGT CGCTGCCGCT GCCGCTGCCA CTGCCACTGC CACCTCGCGG 60

ATCAGGAGCC AGCGTTGTTC GCCCGACGCC TCGCTGCCGG TGGGAGGAAG CGAGAGGGAA 120

GCCGCTTGCG GGTTTGTCGC CGCTGCTCGC CCACCGCCTG GAAGAGCCGA GCCCCGGCCC 180

AGTCGGTCGC TTGCCACCGC TCGTAGCCGT TACCCGCGGG CCGCCACAGC CGCCGGCCGG 240

GAGAGGCGCG CGCCATGGCT TCTGGAGCCG ATTCAAAAGG TGATGACCTA TCAACAGCCA 300

TTCTCAAACA GAAGAACCGT CCCAATCGGT TAATTGTTGA TGAAGCCATC AATGAGGACA 360

ACAGTGTGGT GTCCTTGTCC CAGCCCAAGA TGGATGAATT GCAGTTGTTC CGAGGTGACA 420

CAGTGTTGCT GAAAGGAAAG AAGAGACGAG AAGCTGTTTG CATCGTCCTT TCTGATGATA 480

CTTGTTCTGA TGAGAAGATT CGGATGAATA GAGTTGTTCG GAATAACCTT CGTGTACGCC 540
TAGGGGATGT CATCAGCATC CAGCCATGCC CTGATGTGAA GTACGGCAAA CGTATCCATG 600
TGCTGCCCAT TGATGACACA GTGGAAGGCA TTACTGGTAA TCTCTTCGAG GTATACCTTA 660
AGCCGTACTT CCTGGAAGCG TATCGACCCA TCCGGAAAGG AGACATTTTT CTTGTCCGTG 720
GTGGGATGCG TGCTGTGGAG TTCAAAGTGG TGGAAACAGA TCCTAGCCCT TATTGCATTG 780
TTGCTCCAGA CACAGTGATC CACTGCGAAG GGGAGCCTAT CAAACGAGAG GATGAGGAAG 840
AGTCCTTGAA TGAAGTAGGG TATGATGACA TTGGTGGCTG CAGGAAGCAG CTAGCTCAGA 900
TAAAGGAGAT GGTGGAACTG CCCCTGAGAC ATCCTGCCCT CTTTAAGGCA ATTGGTGTGA 960
AGCCTCCTAG AGGAATCCTG CTTTACGGAC CTCCTGGAAC AGGAAAGACC CTGATTGCTC 1020
GAGCTGTAGC AAATGAGACT GGAGCCTTCT TCTTCTTGAT CAATGGTCCT GAGATCATGA 1080
GCAAATTGGC TGGTGAGTCT GAGAGCAACC TTCGTAAAGC CTTTGAGGAG GCTGAGAAGA 1140
ATGCTCCTGC CATCATCTTC ATTGATGAGC TAGATGCCAT CGCTCCCAAA AGAGAGAAAA 1200
CTCATGGCGA GGTGGAGCGG CGCATTGTAT CACAGTTGTT GACCCTCATG GATGGCCTAA 1260
AGCAGAGGGC ACATGTGATT GTTATGGCAG CAACCAACAG ACCCAACAGC ATTGACCCAG 1320
CTCTACGGCG ATTTGGTCGC TTTGACAGGG AGGTAGATAT TGGAATTCCT GATGCTACAG 1380
GACGCTTAGA GATTCTTCAG ATCCATACCA AGAACATGAA GCTGGCAGAT GATGTGGACC 1440
TGGAACAGTA GCCAATGAGA CTCACGGGCA TGTGGGTGCT GACTTAGCAG CCCTGTGCTC 1500
AGAGGCTGCT CTGCAAGCCA TCCGCAAGAA GATGGATCTC ATTGACCTAG AGGATGAGAC 1560
CATTGATGCC GAGGTCATGA ACTCTCTAGC AGTTACTATG GATGACTTCC GGTGGGCCTT 1620
GAGCCAGAGT AACCCATCAG CACTGCGGGA AACCGTGGTA GAGGTGCCAC AGGTAACCTG 1680
GGAAGACATC GGGGGCCTAG AGGATGTCAA ACGTGAGCTA CAGGAGCTGG TCCAGTATCC 1740
TGTGGAGCAC CCAGACAAAT TCCTGAAGTT TGGCATGACA CCTTCCAAGG GAGTTCTGTT 1800
CTATGGACCT CCTGGCTGTG GGAAAACTTT GTTGGCCAAA GCCATTGCTA ATGAATGCCA 1860
GGCCAACTTC ATCTCCATCA AGGGTCCTGA GCTGCTCACC ATGTGGTTTG GGGAGTCTGA 1920
GGCCAATGTC AGAGAAATCT TTGACAAGGC CCGCCAAGCT GCCCCCTGTG TGCTATTCTT 1980
TGATGAGCTG GATTCGATTG CCAAGGCTCG TGGAGGTAAC ATTGGAGATG GTGGTGGGGC 2040
TGCTGACCGA GTCATCAACC AGATCCTGAC AGAAATGGAT GGCATGTCCA CAAAAAAAAA 2100
TGTGTTCATC ATTGGCGCTA CCAACCGGCC TGACATCATT GATCCTGCCA TCCTCAGACC 2160
TGGCCGTCTT GATCAGCTCA TCTACATCCC ACTTCCTGAT GAGAAGTCCC GTGTTGCCAT 2220
CCTCAAGGCT AACCTGCGCA AGTCCCCAGT TGCCAAGGAT GTGGACTTGG AGTTCCTGGC 2280

TAAAATGACT AATGGCTTCT CTGGAGCTGA CCTGACAGAG ATTTGCCAGC GTGCTTGCAA 2340

GCTGGCCATC CGTGAATCCA TCGAGAGTGA GATTAGGCGA GAACGAGAGA GGCAGACAAA 2400

CCCATCAGCC ATGGAGGTAG AAGAGGATGA TCCAGTGCCT GAGATCCGTC GAGATCACTT 2460

TGAAGAAGCC ATGCGCTTTG CGCGCCGTTC TGTCAGTGAC AATGACATTC GGAAGTATGA 2520

GATGTTTGCC CAGACCCTTC AGCAGAGTCG GGGCTTTGGC AGCTTCAGAT TCCCTTCAGG 2580

GAACCAGGGT GGAGCTGGCC CCAGTCAGGG CAGTGGAGGC GGCACAGGTG GCAGTGTATA 2640

CACAGAAGAC AATGATGATG ACCTGTATGG CTAAGTGGTG GTGGCCAGCG TGCAGTGAGC 2700

TGGCCTGCCT GGACCTTGTT CCCTGGGGGT GGGGGCGCTT GCCCAGGAGA GGGACCAGGG 2760

GTGCGCCCAC AGCCTGCTCC ATTCTCCAGT CTGAACAGTT CAGCTACAGT CTGACTCTGG 2820

ACAGGGGGTT TCTGTTGCAA AAATACAAAA CAAAAGCGAT AAAATAAAAG CGATTTTCAT 2880

TTGGTAGGCG GAGAGTGAAT TACCAACAGG GAATTGGGCC TTGGGCTATG CCATTTCTGT 2940

TGTAGTTTGG GGCAGTGCAG GGGACCTGTG TGGGGTGTGA ACCAAGGCAC TACTGCCACC 3000

TGCCACAGTA AAGCATCTGC ACTTGACTCA ATGCTGCCCG AGCCCTCCCT TCCCCCTATC 3060

CAACCTGGGT AGGTGGGTAG GGGCCACAGT TGCTGGATGT TTATATAGAG AGTAGGTTGA 3120

TTTATTTTAC ATGCTTTTGA GTTAATGTTG GAAAACTAAT CACAAGCAGT TTCTAAACCA 3180

AAAAATGACA TGTTGTAAAA GGACAATAAA CGTTGGGTCN AAATGGGWRA AAAAAAAAAA 3240

AAAAAAGGGG GGCCCCTCTA AAGNNCCANN CTTCGT 3276

(2) INFORMATION FOR SEQ ID NO: 292:

(i) SEQUENCE CHARACTERISTICS:
(A) LENGTH: 1695 base pairs
(B) TYPE: nucleic acid
(C) STRANDEDNESS: double
(D) TOPOLOGY: linear

(xi) SEQUENCE DESCRIPTION: SEQ ID NO: 292:

TTGCAATGGT TGAATTCCCC TCCTCACGCC AGCCTAGGAG AAGAAGTTCG TAGTCCCAGA 60

GGTGAGGCAG GAGGCGGCAG TTTCTGGCGG GTGAGGGCGG AGCTGAAGTG ACAGCGGAGG 120

CGGAAGCAAC GGTCGGTGGG GCGGAGAAGG GGGCTGGCCC CAGGAGGAGG AGGAAACCCT 180

TCCGAGAAAA CAGCAACAAG CTGAGCTGCT GTGACAGAGG GGAACAAGAT GGCGGCGCCG 240

AAGGGAGCCT CTGGGTGAGG ACCCAACTGG GGCTCCCGCC GCTGCTGCTG CTGACCATGG 300

CCTTGGCCGG AGGTTCGGGG ACCGCTTCGG CTGAAGCATT TGACTCGGTC TTGGGTGATA 360

CGGCGTCTTG CCACCGGGCC TGTCAGTTGA CCTACCCCTT GCACACCTAC CCTAAGGAAG 420

AGGAGTTGTA CGCATGTCAG AGAGGTTGCA GGCTGTTTTC AATTTGTCAG TTTGTGGATG 480

ATGGAATTGA CTTAAATCGA ACTAAATTGG AATGTGAATC TGCATGTACA GAAGCATATT 540

CCCAATCTGA TGAGCAATAT GCTTGCCATC TTGGTTGCCA GAATCAGCTG CCATTCGCTG 600

AACTGAGACA AGAACAACTT ATGTCCCTGA TGCCAAAAAT GCACCTACTC TTTCCTCTAA 660

CTCTGGTGAG GTCATTCTGG AGTGACATGA TGGACTCCGC ACAGAGCTTC ATAACCTCTT 720

CATGGACTTT TTATCTTCAA GCCGATGACG GAAAAATAGT TATATTCCAG TCTAAGCCAG 780

AAATCCAGTA CGCACCACAT TTGGAGCAGG AGCCTACAAA TTTGAGAGAA TCATCTCTAA 840

GCAAAATGTC CTATCTGCAA ATGAGAAATT CACAAGCGCA CAGGAATTTT CTTGAAGATG 900

GAGAAAGTGA TGGCTTTTTA AGATGCCTCT CTCTTAACTC TGGGTGGATT TTAACTACAA 960

CTCTTGTCCT CTCGGTGATG GTATTGCTTT GGATTTGTTG TGCAACTGTT GCTACAGCTG 1020

TGGAGCAGTA TGTTCCCTCT GAGAAGCTGA GTATCTATGG TGACTTGGAG TTTATGAATG 1080

AACAAAAGCT AAACAGATAT CCAGCTTCTT CTCTTGTGGT TGTTAGATCT AAAACTGAAG 1140

ATCATGAAGA AGCAGGGCCT CTACCTACAA AAGTGAATCT TGCTCATTCT GAAATTTAAG 1200

CATTTTTCTT TTAAAAGACA AGTGTAATAG ACATCTAAAA TTCCACTCCT CATAGAGCTT 1260

TTAAAATGGT TTCATTGGAT ATAGGCCTTA AGAAATCACT ATAAAATGCA AATAAAGTTA 1320

CTCAAATCTG TGAAGACTGT ATTTGCTATA ACTTTATTGG TATTGTTTTT GTAGTAATTT 1380

AAGAGGTGGA TGTTTGGGAT TGTATTATTA TTTTACTAAT ATCTGTAGCT ATTTTGTTTT 1440

TTGCTTTGGT TATTGTTTTT TTCCCTTTTC TTAGCTATGA GCTGATCATT GCTCCTTCTC 1500

ACCTCCTGCC ATGATACTGT CAGTTACCTT AGTTAACAAG CTGAATATTT AGTAGAAATG 1560

ATGCTTCTGC TCAGGAATGG CCCACAAATC TGTAATTTGA AATTTAGCAG GAAATGACCT 1620

TTAATGACAC TACATTTTCA GGAACTGAAA TCATTAAAAT TTTATTTGAA TAATTAAAAA 1680

AAAAAAAAAA AANCT 1695

(2) INFORMATION FOR SEQ ID NO: 293:

(i) SEQUENCE CHARACTERISTICS:
(A) LENGTH: 1501 base pairs
(B) TYPE: nucleic acid
(C) STRANDEDNESS: double
(D) TOPOLOGY: linear

(xi) SEQUENCE DESCRIPTION: SEQ ID NO: 293:

CACTTTCAGC AGTCCTTTGC TCTCTTTGCT TCTACCTCAA ATAGCCCCAG GAGTGGGCTT 60

TAGTCTCCAA TATGGAGCAT CTCAAGCTTC TCCTGGGGGA TGGGGATTGG GATGGGCAGA 120

ATCTGTTTTG GWTCTCCGGG TTATTTCCAG TGGGTGTAAA AGCAGAGCTG GGCCTTTCCC 180

TCTCTTATCC CTGAGGGTGG GTAAGAAGGA CTGTATCTAC ACCTGTTCTT CCCTACCTTC 240

TCTTTTGTTA GGGAGGCCTC ATTCTAAGTT CCTCAAGAGA GTCCTTGGCT TAAAGCTGTA 300

GCAAGGGTGT GCTAGGTGGG GGATTTGGAG CAAAACCGTC GAGTAGGCAT GATACTGGTA 360

TGGAGTGGGC CTGCAAAATC AGACAGAAAT GGCTTGAGAA GCCGCAGGGG AGCATGCCTG 420

TCTCTCAGTG ATAGAGTATG GGAGGGACCT CCCTAGCTTG GAAAATGAGA ATTGAAGGGG 480

TTATGAACAA ATAGGATGCC TAGTTGAGGA TGTTCCCAAA GTTTTGTCCA ATCTTATCAT 540

TAGTAGATTT TATAAGCCAC AGAGACAAAC CAGAAACGGA ATAATGTTAC TTTGGATGCT 600

TTATTTTTTT GTTCTAGGTG TGGCTTTGTA CATGCAGAAG AATGCTATAT GCTGCACATT 660

TTGCCTTTAA AGTCTTACGA CTTTCCCCAT TTTAGTCTAA TGGGAAGATA CAGATGTGCA 720

AGTCTGCTTT TTTGTTTTTT GTTATTATTT TTTTTTTTTT GCTCTGTGTT ATGGACATTT 780

TCAGACATGC ACAGAAGTGG AGAGGATGGT CCTTGGACCC MATGTGTCCA TCACCTAGCT 840

GCATCACTTA TCAGCTATGG TCAACCTGGT TTCATCTGTA TCTCTCTCTT TTCACCTGTA 900

TTGTTTATTG AAAATCCAAG ACACTATGCC AATGCAACCG TGACTACTTT GGGAGATTGG 960

TAGTCTCTTT TGATGGTGAT AGTGATGGGG TGCACTATCA TAATCACATC AGGTCTGCTT 1020

TTTGCTTTTA ATGTTAACTA ATGAAGTTCC AGAGATGGGC CTTAGAAATG TGTTTTAAGA 1080

ATTAACAAGG AGTCTCAAAA AGAAATGAGA GGGATGCTTC CTTTNCCCTT GCATCTACAA 1140

AACMAGAGAG AGACTGTTCT GTTGTAAAAC TCTTTCAAAA ATTCTGATAT GGTAAGGTAC 1200

TTGAGACCCT TCACCAGAAT GTCAATCTTT TTTTCTGTGT AACATGGAAA CTTGTGTGAC 1260

CATTAGCATT GTTATCAGCT TGTACTGGTC TCATAACTCT GGTTTTGGAA GAATAATTTG 1320

GAAATTGTTG CTGTGTTCTG TGAAAATAAC CTCCCCAAAA TAATTAGTAA CTGGTTGTTC 1380

TACTTGGTAA TTTGACACCC TGTTAATAAC GCAATTATTT CTGTGTTCTT AAACAGTATA 1440

AATAGTTGTA AGTTTGCATG CATGATGGAA AAATAAAAAC CTGTATCTCT GTTAAAAAAA 1500

A 1501

(2) INFORMATION FOR SEQ ID NO: 294:

(i) SEQUENCE CHARACTERISTICS:
(A) LENGTH: 2683 base pairs
(B) TYPE: nucleic acid
(C) STRANDEDNESS: double

(D) TOPOLOGY: linear

(xi) SEQUENCE DESCRIPTION: SEQ ID NO: 294:

TGANTGTGGT CCCGGGTGCN GATTGGCAGN GCCTCCGCCG CGGCTCGTGG TTGTCCCGCC 60

ATGGCACTGT CGCGGGGGCT GCCCCGGGAG CTGGCTGAGG CGGTGGCCGG GGGCCGGGTR 120

CTGGTGGTGG GGGCGGGCGG CATCGGCTGC GAGCTCCTCA AGAATCTCGT GCTCACCGGT 180

TTCTCCCACA TCGACCTGAT TGATCTGGAT ACTATTGATG TAAGCAACCT CAACAGACAG 240

TTTTTGTTTC AAAAGAAACA TGTTGGAAGA TCAAAGGCAC AGGTTGCCAA GGAAAGTGTA 300

CTGCAGTTTT ACCCGAAAGC TAATATCGTT GCCTACCATG ACAGCATCAT GAACCCTGAC 360

TATAATGTGG AATTTTTCCG ACAGTTTATA CTGGTTATGA ATGCTTTAGA TAACAGAGCT 420

GCCCGAAACC ATGTTAATAG AATGTGCCTG GCAGCTGATG TTCCTCTTAT TGAAAGTGGA 480

ACAGCTGGGT ATCTTGGACA AGTAACTACT ATCAAAAAGG GTGTGACCGA GTGTTATGAG 540

TGTCATCCTA AGCCGACCCA GAGAACCTTT CCTGGCTGTA CAATTCGTAA CACACCTTCA 600

GAACCTATAC ATTGCATCGT TTGGGCAAAG TACTTGTTCA ACCAGTTGTT TGGGGAAGAA 660

GATGCTGATC AAGAAGTATC TCCTGACAGA GCTGACCCTG AAGCTGCCTG GGAACCAACG 720

GAAGCCGAAG CCAGAGCTAG AGCATCTAAT GAAGATGGTG ACATTAAACG TATTTCTACT 780

AAGGAATGGG CTAAATCAAC TGGATATGAT CCAGTTNAAA CTTTTTACCA AGCTTTTTAA 840

AGATGACATC AGGTATCTGT TGACAATGGA CAAACTATGG CGGAAAAGGA AACCTCCAKT 900

TCCGTTGGAC TGGGCTGAAG TACAAAGTCA AGGAGAAGAA ACGAATGCAT CAGATCAACA 960

GAATGAACCC CAGTTAGGCC TGAAAGACCA GCAGGTTCTA GATGTAAAGA GCTATGCACG 1020

TCTTTTTTCA AAGAGCATCG AGACTTTGAG AGTTCATTTA GCAGAAAAGG GGGATGGAGC 1080

TGAGCTCATA TGGGATAAGG ATGACCCATC TGCAATGGAT TTTGTCACCT CTGCTGCAAA 1140

CCTCAGGATG CATATTTTCA GTATGAATAT GAAGAGTAGA TTTGATATCA AATCAATGGC 1200

AGGGAACATT ATTCCTGCTA TTGCTACTAC TAATGCAGTA ATTGCTGGGT TGATAGTATT 1260

GGAAGGATTG AAGATTTTAT CAGGAAAAAT AGACCAGTGC AGAACAATTT TTTTGAATAA 1320

ACAACCAAAC CCAAGAAAGA AGCTTCTTGT GCCTTGTGCA CTGGATCCTC CCAACCCCAA 1380

TTGTTATGTA TGTGCCAGCA AGCCAGAGGT GACTGTGCGG CTGAATGTCC ATAAAGTGAC 1440

TGTTCTCACC TTACAAGACA AGATAGTGAA AGAAAAATTT GCTATGGTAG CACCAGATGT 1500

CCAAATTGAA GATGGGAAAG GAACAATCCT AATATCTTCC GAAGAGGGAG AGACGGAAGC 1560

TAATAATCAC AAGAAGTTGT CAGAATTTGG AATTAGAAAT GGCAGCCGGC TTCAAGCAGA 1620

TGACTTCCTC CAGGACTATA CTTTATTGAT CAACATCCTT CATAGTGAAG ACCTAGGAAA 1680

GGACGTTGAA TTTGAAGTTG TTGGTGATGC CCCGGAAAAA GTGGGGSCCA AACAAGCTGA 1740

AGATGCTGCC AAAAGCATAA CCAATGGGCA GTGATGATGG AGCTCAGCCC TCCACCTCCA 1800

CAGCTCAAGA GCAAGATGAC GTTCTCATAG TTGATTCGGA TGAAGAAGAT TCTTCAAATA 1860

ATGCCGACGT CATGAAGAAG AGAGAAGCCG CAAGAGGAAA TTAGATGAGA AAGAGAATCT 1920

CAGTGCAAAG AGGTCACGTA TAGAACAGAA GGAAGAGCTT GATGATGTCA TAGCATTAGA 1980

TTGAACAGAA ATGCCTCTAA ACAGAACCCT CTTACTATTT AGTTTATCTG GGCAGAACCA 2040

GATTGTTATG TCCTTTGTTC CAAAGGGAAA AAATTGACAG CAGTGACTTG AAAATGATTC 2100

TGCTCCCTTT GAAAGCATTC ATTTTGCTAG AACTGTTAGA CACATTGCAG TATGCTGTAT 2160

TGAAAGTAGG AATATAGTTT TAAAAACCCT TTGAACAAAG TGTGTGCATA ACCAGTCATG 2220

AGATAAAACA ACACAATGCA TGTTGCCTTT TTAATGTAAA TACCCTTAGG TATCATTAAT 2280

AGTTTCAAAA TATTGTGGTT TAGTAAAGTT GATACCTGGT TATAAATATT ATGCCTTTAT 2340

TTTTGGCTAG AAGAAGAATT ATTTTTAGCC TAGATCTAAC CATTTTCATA CTCTTAACTG 2400

ATTGAAACAG ATTCAAAGAA GTATCGAGTG CTATGCATTG AAACTTGTTT TTAAATGTTA 2460

GATGGCACTA TGTATATTAA TGTAAAACAA TGTTAATTTA CTCAAGTTTT CAGTTTGTAC 2520

CGCCTGGTAT GTCTGTGTAA GAAGCCAATT TTTGTGTATT GTTACAGTTT CAGGTTATTT 2580

ATATTCGATG TTTTGTAAAA CTCAAATAAC GACTATACTT ATGGACCAAA TAAATGGCAY 2640

TGCATTCTKG TKAAAAAAAN NACAGAAAAA AAAAAAAACA AGA 2683

(2) INFORMATION FOR SEQ ID NO: 295:

(i) SEQUENCE CHARACTERISTICS:
(A) LENGTH: 1454 base pairs
(B) TYPE: nucleic acid
(C) STRANDEDNESS: double
(D) TOPOLOGY: linear

(xi) SEQUENCE DESCRIPTION: SEQ ID NO: 295:

GGACTCGGGG TGGCTCTAAG GGGCAGGGAT AGGGCTGGGG AGCGCCGGCC TGTGGCCCTG 60

ACCAGCCCCT TCTCGTGCAG GTTCCACCCC GATGCAGGTG GTCACGTGCT TGACGCGGGA 120

CAGCTACCTG ACGCACTGCT TCCTCCAGCA CCTCATGGTC GTGCTGTCCT CTCTGGAACG 180

CACGCCCTCG CCGGAGCCTG TTGACAAGGA CTTCTACTCC GAGTTTGGGA ACAAGACCAC 240

AGGGAAGATG GAGAACTACG AGCTGATCCA CTCTAGTCGC GTCAAGTTTA CCTACCCCAG 300

TGAGGAGGAG ATTGGGGACC TGACGTTCAC TGTGGCCCAA AAGATGGCTG AGCCAGAGAA 360

GGCCCCAGCC CTCAGCATCC TGCTGTACGT GCAGGCCTTC CAGGTGGGCA TGCCACCCCC 420

TGGGTGCTGC AGGGGCCCCC TGCGCCCCAA GACACTCCTG CTCACCAGCT CCGAGATCTT 480

CCTCCTGGAT GAGGACTGTG TCCACTACCC ACTGCCCGAG TTTGCCAAAG AGCCGCCGCA 540

GAGAGACAGG TACCGGCTGG ACGATGGCCG CCGCGTCCGG GACCTGGACC GAGTGCTCAT 600

GGGCTACCAG ACCTACCCGC AGCCCTCACC CTCGTYTTCG ATGACGTGCA AGGTCATGAC 660

CTCATGGGCA GTGTCACCCT GGACCACTTT GGGGAGGTGC CAGGTGGCCC GGCTAGAGCC 720

AGCCAGGGCC GTGAAGTCCA GTGGCAGGTG TTTGTCCCCA GTGCTGAGAG CAGAGAGAAG 780

CTCATCTCGC TGTTGGCTCG CCAGTGGGAG GCCCTGTGTG GCCTGAGCTG CCTGTCGAGC 840

TCACCGGCTA GCCCAGGCCA CAGCCAGCCT GTCGTGTCCA GCCTGACGCC TACTGGGGCA 900

GGGCAGCAGG CTTTTGTGTT CTCTAAAAAT GTTTTATCCT CCCTTTGGTA CCTTAATTTG 960

ACTGTCCTCG CAGAAATGTG AACATGTGTG TGTGTTGTGT TAATTCTTTC TCATGTTGGG 1020

AGTGAGAATG CCGGGCCCCT CAGGGCTGTT CGGTGTGCTG TCAGCCTCCC ACAGGTGGTA 1080

CAGCCGTGCA CACCAGTGTC GTGTCTGCTG TTGTGGGACC GTTGTTAACA CGTGACACTG 1140

TGGGTCTGAC TTTYTCTTCT ACACGTCCTT TCCTGAAGTG TCGAGTCCAG TCCTTTGTTG 1200

CTGTTGCTGT TGCTGTTGCT GTTGCTGTTG GCATCTTGCT GCTAATCCTG AGGCTGGTAG 1260

CAGAATGCAC ATTGGAAGCT CCCACCCCAT ATTGTTCTTC AAAGTGGAGG TCTCCCCTGA 1320

TCCAGACAAG TGGGAGAGCC CGTGGGGGCA GGGGACCTGG AGCTGCCAGC ACCAAGCGTG 1380

ATTCCTGCTG CCTGTATTCT CTATTCCAAT AAAGCAGAGT TTGACACCGW MAAAAAAAAA 1440

AAAAAAAAAA AACN 1454

(2) INFORMATION FOR SEQ ID NO: 296:

(i) SEQUENCE CHARACTERISTICS:
(A) LENGTH: 828 base pairs
(B) TYPE: nucleic acid
(C) STRANDEDNESS: double
(D) TOPOLOGY: linear

(xi) SEQUENCE DESCRIPTION: SEQ ID NO: 296:

ACCCTGGCAT GCCCCACAAA CAGATCACCA GCCAGCTTAC ACAGGCATTA ACTCTCCTCA 60

ATGAGGAAGA ATCATTCACA ACTGAGCAAG ACATTCATAT GATCATTTAA GGAAGTGTTT 120

CCCTTATGTG TTAGCAAGTA TAATCGGCTA ACTCCTAAAT CCCAATGAAT AGTCCTAGGC 180

TGGACAGCAA TGGGCTGCAA TTAGGCAGAT AAAGACATCA GTCCCAGTAA ATGAATCCAT 240

AGACTCATCT AGCACCAACT ACCATTAGCA CTATGTTAGG AGCTGCAAGG CCCCAAAGTA 300

GAAGATGTGC ATAATGTCTG CTCTTGTGTA GCTCAGGAGA CAATTCCAGC ACAGACACTA 360

CAGTTAACGC TGAACTGCAG CTGCAAGTAA TAGCAWGAAC AGTCAGAAAA ATACCTTATG 420

AGGGGGCAGG GCTGAAGCTG GGCCTTGAAG GATGGATGAA ATTTGGATAG AGAATGAGGA 480

AGACAGAGGG NCTCCAAGTG AGAGAAGCAT GAAAAATGAG CARGGGCCTG GATCAGTGGG 540

GTGTATTCAG AGCACCTYTC CAGATGCACC ATGCATGCTC ACAGTCCCTT GCCTATGTGT 600

GGCAGAGTGT CCCAGCCAGA TGTGTGCCCC CACCCCATGT CCATTTACAT GTCCTTCAAT 660

GCCCACCTCA AAAGGYACYT CTTCTGTAAA GCTTTCCCTK GGTATCAGGA ATCAAAATTA 720

ATCAGGGATC TTTTCACACT GCTGTTTTTT CCTCTTTGGT CCTTCTATCA CTAAAACTCA 780

TCTCATTCAG CCTTACAGCA TAACTAATTA TTTGTTTTCC TCACTACA 828

(2) INFORMATION FOR SEQ ID NO: 297:

(i) SEQUENCE CHARACTERISTICS:
(A) LENGTH: 2416 base pairs
(B) TYPE: nucleic acid
(C) STRANDEDNESS: double
(D) TOPOLOGY: linear

(xi) SEQUENCE DESCRIPTION: SEQ ID NO: 297:

TCAATTTCCA TTAACTCAGA TCAGCCATTG TGATTCACCA TTTGTCAGGC TCTCAGGTTT 60

AACAAAACCT ACTATCACCA TCATCCTTCA ACAGCCACAG TCTGAATTGA GCCAACATTT 120

TTTTTTCTTT GAGAAAGAAG TGGACTGGGG CACAACTTTT AGTCTGAGGG GAGCTAGTGG 180

AAATCTAGAC AATAGAAGTC ATCGATAGCA GCTTTTCCTC AAATGTGTGA CTCCTCAGGG 240

GCTAAACTGC TCTTAGCTTA GAATTATGCT TTACTAGAGA TCTAGCAGAT AAGTGGGTTA 300

ATCACTACCA TCCTGTAACT AGTTATATAG CTTCCAGACA TGAGGGAGAC ATCAAACAGG 360

GATGGAAGCA ACCCCAAGGA TATGCAAGAA GGGCATGATG AACCCCCTTC CCTCTGGCAG 420

GAGAACAAGG CCAACCAAGG GACAGACTGG AAAGCACTTA GATGTTTAAG GAGGAGAAAG 480

GGGAAGCTTT GACCAGTCCT TGCCTTTTGC CAAGTTCAGC CAGTTCTCCG CTGCTTGCAA 540

CCTCTAGCGC AGTAACATTT GCAGAATTGC AGATTTTCCC CCAGATACTA GGAGGAAAGG 600

GACTTTGGGG GGTGGGGAAG GGGTCGTGGT GTTTTAAAAG CATAAGTTAC CTGTTTGCAC 660

TGTTTTAAGA TAGGAAAAAA AAATAGTGGG CAAGGTGAAC ATCAGACGTA AATTTGTGTG 720

TTTTTATTTT GTCATGCTCT TGAAAATGTT TGACCATTTG TAGTATACAC AGTGAAACTT 780

GATTCTCTGT TGCATAAAAC ACTATATTTT TTTGGAAATG TTACTGTCCA AAAGCCTCTT 840

CCCTCCCTTT CCTTTTCCTA TGTACTTCCT TCATACTTGC TTTACTGATC AGCCAGGCAA 900

TAGCCATCCA AGAGCTAGAG CATGAAACAG GGCCCTTTCC AAGTAGGCTC TGGGTGTCCT 960

AAGCCAGCGT GTGCCCTCTG GTTTAGTGAG TGTAATAGAG TCCCTGGCAC CTTTCTTTGC 1020

AAATGAGGCT AACAGACCAG ACTGCAGCAA GTTATCAGAT TCCTCAATCA GATGCACTAG 1080

GAGTGAGGAG CCCAGGGATG GAGGGGGTTC CTGAAGTATT GCAGTTGGCT GTAGTAGCTG 1140

AGTTCTTTTC CATGTTACCG AAACTGTAGC CAGTTACAGT TTACTCAGGA AAACGGTAGA 1200

TCAATTCAGC CATGGTAGTG CTGGTTGGCA GGGATTGGTA ACGGAGAGAA CTGCTCATCA 1260

GCCAAAACTC AAGCCTTGCC TTTTAGGAGG CCACCAGCAG AGGGACTTGG TCCTCCTTGT 1320

CTGGTACTTG TGTACATGCC GGTGACCTGA GGACTCCACT CACACTGGCG AGCAAAAAGG 1380

GAGCAGTGAT TCTCTTTTCT CTCCCCACCC CCTGCCCTTT GTTACCAACA CCAGTTTCCC 1440

AGGGGGTACA TGAGTTTCTG AATTTTTAAA AAATGTTTTT GGTTTGGTTT TTCTGGGGAC 1500

TGATAAGTGC TTTAAGCAAT GTCCATACCC CGTCAAGACT CCCAGCTTAG TCATTTTCTT 1560

GTATTTTTCT GTTCACAGTA TTTGTGTGTG TGCTTGTTTT GGCAGCTCAT TTTGGCTGTA 1620

TTATATATTG AGTGATGAAT TGATCCTCTT TTTTCCCTAA GGGATATGAA TTGTTTTTCT 1680

TGTGTTATAT TCTGCTTGTG AATAGCTGGA GCAAACCTGG GGCTGACACG CGTAAGSTAG 1740

GGCTGCAAAR CGAGAAGAGA GCCGGTGGAG TGTACTTGTC CCTGACAGGC TGACCTACCT 1800

GAGTCTCTGA GCTTTTCAGT CCAAATCTTT GCAAGGCTCA AAATGCCACA GAACCTCTCC 1860

TCTTCTCCCC ACTCCCCATG GCAGGGACCG GACCATCCCT ACATGCAACA TGCTGTTCCT 1920

CCAGCCCCTC CCATTGCCAT GGCAAAACAG GTACCTTTGG GGCATGGGGG CATTACATGG 1980

GATGCTTGTG TAATCGACCA CCTAGCCTTC TCTCTCCCCT CCCGTCCTCC CCCAGAATCA 2040

CTTCCTAGGA CACCCGAGCT GCTTGCCCAG GGTCCTGTTT CCCTGCTAAC TCCAGAGAAG 2100

CATCCCAGGG CTTTGTGACA GTCTCTAATT CCCTTCCCTT CTCGTTAAGA ATCATATTGT 2160

ATAGTAGCTT TCAGACCATA CAGTATTCAT TGGGTTACTC CTATTATTAT CAAGTAGCTG 2220

GAATTGTGAA GGTCGGAGTA GTTAGATCTT TAGCTTTTAT TCCTTATTTT TTTGTATTAC 2280

TCTCCATGTG TATAAATTAT TGATCATGTT GCTGGCTTTT ATAAACTCTA AGCGAAGGAG 2340

GAGCACTGCC TCAGCCTTTG CACATGGTAA TGAAGCACTG TTTTTAAATA AAAGRGRGAA 2400

MCMCCAAAAA AAAAAA 2416

(2) INFORMATION FOR SEQ ID NO: 298:

(i) SEQUENCE CHARACTERISTICS:
(A) LENGTH: 545 base pairs

(B) TYPE: nucleic acid
(C) STRANDEDNESS: double
(D) TOPOLOGY: linear

(xi) SEQUENCE DESCRIPTION: SEQ ID NO: 298:

GAATTCGGCA CGAGCCATGC YTGGCCTCTC CTTGATTCTT ACAGTCACTT TGTTGGCTGT 60

TTCTGACTCA GCAGCTACCT GCATTGTGGC CAAAGGATGA CCTATTCCTT CTCAGGAGGG 120

CAAAAATGTG GAATAGTGTC TGTCCATGCC TCTCCTCATG GGCTACCACC TCTGCCACCG 180

TGGTTAATCA GTAACAACCA GGAGAGAAGC TGCTGGAACT GACCTCTGGG AACTCCCTGG 240

ATGGTTTGGT GCAGGAATGT AGTAGGCATA CACGTGGTTG CGTGGATCTG GGCCCTCCTG 300

ATGTGAGTAG AGAGGTAAAA GGSCACCATC TCCTTGACCT YTGGGGAACT CATCCACAAA 360

GAAGATGTTT CCAAGATGCT TCTGAAGATT GSCTAAAAAT AGCCGGTTTC CACCCCCGTG 420

AATGCATCCA TTCTAGAATG CTCCTTCACC AGGACCAGAG AACTGATTTA CAGAAGTGAC 480

ATGAAAACAT TCCATCCCAG AATTTGCANT ACCTCAAATT NAATTTCTAC CTATTAAAAA 540

NAAAA 545

(2) INFORMATION FOR SEQ ID NO: 299:

(i) SEQUENCE CHARACTERISTICS:
(A) LENGTH: 1530 base pairs
(B) TYPE: nucleic acid
(C) STRANDEDNESS: double
(D) TOPOLOGY: linear

(xi) SEQUENCE DESCRIPTION: SEQ ID NO: 299:

GGCTCTGCTG GGCATCATAC TTGTCACTGG GTAAACAGTT TGCCCACTTA CCGCAGATGA 60

AGCTGCTTGC CAGGGCTCTC CGGCTCTGTG AGTTTGGGAG GCAGGCATCT TCCAGGAGGC 120

TGGTGGCTGG CCAGGGATGT GTGGGGCCCC GGCGAGGGTG CTGCGCTCCC GTCCAGGTGG 180

TTGGGCCCAG GGCTGATCTC CCACCCTGTG GAGCCTGCAT TACTGGAAGG ATCATGCGGC 240

CAGATGATGC CAACGTGGCC GGCAATGTCC ACGGGGGGAC CATCCTGAAG ATGATCGAGG 300

AGGCAGGCGC CATCATCAGC ACCCGGCATT GCAACAGCCA GAACGGGGAG CGCTGTGTGG 360

CCGCCCTGGC TCGTGTCGAG CGCACCGACT TCCTGTCTCC CATGTGCATC GGTGAGGTGG 420

CGCATGTCAG CGCGGAGATC ACCTACACCT CCAAGCACTC TGTGGAGGTG CAGGTCAACG 480

TGATGTCCGA AAACATCCTC ACAGGTGCCA AAAAGCTGAC CAATAAGGCC ACCCTGTGGT 540

ATGTGCCCCT GTCGCTGAAG AATGTGGACA AGGTCCTCGA GGTGCCTCCT GTTGTGTATT 600

CCCGGCANGA GCAGGAGGAG GAGGGCCGGA AGCGGTATGA AGCCCAGAAG CTGGAGCGCA 660

TGGAGACCAA GTGGAGGAAC GGGGACATCG TCCAGCCAGT CCTCAACCCA GAGCCGAACA 720

CTGTCAGCTA CAGCCAGTCC AGCTTGATCC ACCTGGTGGG GCCTTCAGAC TGCACCCTGC 780

ACGGCTTTGT GCACGGAGGT GTGACCATGA AGCTCATGGA TGAGGTCGCC GGGATCGTGG 840

CTGCACGCCA CTGCAAGACC AACATCGTCA CAGCTTCCGT GGACGCCATT AATTTTCATG 900

ACAAGATCAG AAAAGGCTGC GTCATCACCA TCTCGGGACG CATGACCTTC ACGAGCAATA 960

AGTCCATGGA GATCGAGGTG TTGGTGGACG CCGACCCTGT TGTGGACAGC TCTCAGAAGC 1020

GCTACCGGGC CGCCAGTGCC TTCTTCACCT ACGTGTCGCT GAGCCAGGAA GGCAGGTCGC 1080

TGCCTGTGCC CCAGCTGGTG CCCGAGACCG AGGACGAGAA GAAGCGCTTT GAGGAAGGCA 1140

AAGGGCGGTA CCTGCAGATG AAGGCGAAGC GACAGGGCCA CGCGGAGCCT CAGCCCTAGA 1200

CTCCCTCCTC CTGCCACTGG TGCCTCGAGT AGCCATGGCA ACGGGCCCAG TGTCCAGTCA 1260

CTTAGAAGTT CCCCCCTTGG CCAAAAACCC AATTCACATT GAGAGCTGGT GTTGTCTGAA 1320

GTTTTCGTAT CACAGTGTTA ACCTGTACTC TCTCCTGCAA ACCTACACAC CAAAGCTTTA 1380

TTTATATCAT TCCAGTATCA ATGCTACACA GTGTTGTCCC GAGCGCCGGG AGGCGTTGGG 1440

CAGAAACCCT CGGGAATGCT TCCGAGCACG CTGTAGGGTA TGGGAAGAAC CCAGCACCAC 1500

TMATAAAGCT GNTGCTTGGC TGGGGAAGNA 1530

(2) INFORMATION FOR SEQ ID NO: 300:

(i) SEQUENCE CHARACTERISTICS:
(A) LENGTH: 997 base pairs
(B) TYPE: nucleic acid
(C) STRANDEDNESS: double
(D) TOPOLOGY: linear

(xi) SEQUENCE DESCRIPTION: SEQ ID NO: 300:

AGGTAGTGAG AGACACATTA CACCTAACCA ACAAGAAGAA GGATCCTCCC CCTTATAATT 60

TAACTATGTT TACAGGGAAT GCGTACATTG TGGCTTCCCG AGNATTTCGT CCAACATGTT 120

TTGAAGAACC CTAAATCCCA ACAACTGATT GAATGGGTAA AAGACACTTA TAGCCCAGAT 180

GAACACCTCT GGGCCACCCT TCAGCGTGCA CGGTGGATGC CTGGCTCTGT TCCCAACCAC 240

CCCAAGTACG ACATCTTCAG ACATGACTTC TATTGCCAGG CTGGTCAAGT GGCAGGGTCA 300

TGAGGGAGAC ATCGATAAGG GTGCTCCTTA TGCTCCCTGC TCTGGAATCC ACCAGCGGGC 360

TATCTGCGTT TATGGGGCTG GGGACTTGAA TTGGATGCTT CAAAACCATC ACCTGTTGGC 420

CAACAAGTTT GACCCAAAGG TAGATGATAA TGCTCTTCAG TGCTTAGAAG AATACCTACG 480

TTATAAGGCC ATCTATGGGA CTGAACTTTG AGACACACTA TGAGAGCGTT GCTACCTGTG 540

GGGCAAGAGC ATGTACAAAC ATGCTCAGAA CTTGCTGGGA CAGTGTGGGT GGGAGACCAG 600

GGCTTTGCAA TTCGTGGCAT CCTTTAGGAT AAGAGGGCTG MTATTAGATT GTGGGTAAGT 660

AGATCTTTTG CCTTGCAAAT TGCTGCCTGG GTGRATGCTG CTTGTTCTCT CACCCCTAAC 720

CCTAGTAGTT CCTCCACTAA CTTTCTCACT AAGTGAGAAT GAGAACTGCT GTGATAGGGA 780

GAGTGAAGGA GGGATATGTG GTAGAGCACT TGATTTCAGT TGAATGCCTG CTGGTAGCTT 840

TTCCATTCTG TGGAGCTGCC GTTCCTAATA ATTCCAGGTT TGGTAGCGTG GAGGAGAACT 900

TTGATGGAAA GAGAACCTTC CCTTCTGTAC TGTTAACTTA AAAATAAATA GCTCCTGATT 960

CAAAGTAAGG AAAAARAAAA AAAGAAAAAA AACTCGA 997

(2) INFORMATION FOR SEQ ID NO: 301:

(i) SEQUENCE CHARACTERISTICS:
(A) LENGTH: 2345 base pairs
(B) TYPE: nucleic acid
(C) STRANDEDNESS: double
(D) TOPOLOGY: linear

(xi) SEQUENCE DESCRIPTION: SEQ ID NO: 301:

TTGAGGCCGA CGCTAGGGGC CCGGAAGRAA ACTGCGAGGC GAAGGTGACC GGGGACCGAG 60

CATTTCAGAT CTGCTCGGTA GACCTGGTGC ACCACCACCA TGTTGGCTGC AAGGCTGGTG 120

TGTCTCCGGA CACTACCTTC TAGGGTTTTC CACCCAGCTT TCACCAAGGC CTCCCCTGTT 180

GTGAAGAATT CCATCACGAA GAATCAATGG CTGTTAACAC CTAGCAGGGA ATATGCCACC 240

AAAACAAGAA TTGGGATCCG GCGTGGGAGA ACTGGCCAAG AACTCAAAGA GGCAGCATTG 300

GAACCATCGA TGGAAAAAAT ATTTAAAATT GATCAGATGG GAAGATGGTT TGTTGCTGGA 360

GGGGCTGCTG TTGGTCTTGG AGCATTGTGC TACTATGGCT TGGGACTGTC TAATGAGATT 420

GGAGCTATTG AAAAGGCTGT AATTTGGCCT CAGTATGTCA AGGATAGAAT TCATTCCACC 480

TATATGTACT TAGCAGGGAG TATTGGTTTA ACAGCTTTGT CTGCCATAGC AATCAGCAGA 540

ACGCCTGTTC TCATGAACTT CATGATGAGA GGCTCTTGGG TGACAATTGG TGTGACCTTT 600

GCAGCCATGG TTGGAGCTGG AATGCTGGTA CGATCAATAC CATATGACCA GAGCCCAGGC 660

CCAAAGCATC TTGCTTGGTT GCTACATTCT GGTGTGATGG GTGCAGTGGT GGCTCCTCTG 720

ACAATATTAG GGGGTCCTCT TCTCATCAGA GCTGCATGGT ACACAGCTGG CATTGTGGGA 780

GGCCTCTCCA CTGTGGCCAT GTGTGCGCCC AGTGAAAAGT TTCTGAACAT GGGTGCACCC 840

CTGGGAGTGG GCCTGGGTCT CGTCTTTGTG TCCTCATTGG GATCTATGTT TCTTCCACCT 900

ACCACCGTGG CTGGTGCCAC TCTTTACTCA GTGGCAATGT ACGGTGGATT AGTTCTTTTC 960

AGCATGTTCC TTCTGTATGA TACCCAGAAA GTAATCAAGC GTGCAGAAGT ATCACCAATG 1020

TATGGAGTTC AAAAATATGA TCCCATTAAC TCGATGCTGA GTATCTACAT GGATACATTA 1080

AATATATTTA TGCGAGTTGC AACTATGCTG GCAACTGGAG GCAACAGAAA GAAATGAAGT 1140

GACTCAGCTT CTGGCTTCTC TGCTACATCA AATATCTTGT TTAATGGGGC AGATATGCAT 1200

TAAATAGTTT GTACAAGCAG CTTTCGTTGA AGTTTAGAAG ATAAGAAACA TGTCATCATA 1260

TTTAAATGTT CCGGTAATGT GATGCCTCAG GTCTGCCTTT TTTTCTGGAG AATAAATGCA 1320

GTAATCCTCT CCCAAATAAG CACACACATT TTCAATTCTC ATGTTTGAGT GATTTTAAAA 1380

TGTTTTGGTG AATGTGAAAA CTAAAGTTTG TGTCATGAGA ATGTAAGTCT TTTTTCTACT 1440

TTAAAATTTA GTAGGTTCAC TGAGTAACTA AAATTTAGCA AACCTGTGTT TGCATATTTT 1500

TTTGGAGTGC AGAATATTGT AATTAATGTC ATAAGTGATT TGGAGCTTTG GTAAAGGGAC 1560

CAGAGAGAAG GAGTCACCTG CAGTCTTTTG TTTTTTTAAA TACTTAGAAC TTAGCACTTG 1620

TGTTATTGAT TAGTGAGGAG CCAGTAAGAA ACATCTGGGT ATTTGGAAAC AAGTGGTCAT 1680

TGTTACATTC ATCTGCTGAA CTTAACAAAA CTGTTCATCC TGAAACAGGC ACAGGTGATG 1740

CATTCTCCTG CTGTTGCTTC TCAGTGCTCT CTTTCCAATA TAGATGTGGT CATGTTTGAC 1800

TTGTACAGAA TGTTAATCAT ACAGAGAATC CTTGATGGAA TTATATATGT GTGTTTTACT 1860

TTTGAATGTT ACAAAAGGAA ATAACTTTAA AACTATTCTC AAGAGAAAAT ATTCAAAGCA 1920

TGAAATATGT TGCTTTTTCC AGAATACAAA CAGTATACTC ATGATTGCTA AGTGTTTTTT 1980

TATTTTTGCA TATTTATTGA ACTGTCTAAT TGAATACAGC TTGCTCTTGT CACCTCTTCA 2040

AGCTTTCAAG CCTTTATAGA AAAGCTTCTT TGTGGCTTAC ACTGGAAATT ATGAAAGCAG 2100

TTTTTCTCCT AAGACTTTTG GTTTCTCGCA TTGCCTCTCA GACTAAGCAC TAAAAAGCAA 2160

AGCAAAACAG AACTAGTNCT GTCTTAATGA AATATATCAA CCCAAAAGTG TAATGAGGAA 2220

AATGCTTCAT TAGTTTCCCC TAGCAGACTT TTACTTCTCT TACACTGCTA CACCATTACT 2280

TTCTTGAGAC ATTTGTAAGT CCTTTGATAC AGAAGAGTTA TATTTAGGAG GNCTTTAATG 2340

AAGGG 2345

(2) INFORMATION FOR SEQ ID NO: 302:

(i) SEQUENCE CHARACTERISTICS:
(A) LENGTH: 2369 base pairs
(B) TYPE: nucleic acid
(C) STRANDEDNESS: double

(D) TOPOLOGY: linear

(xi) SEQUENCE DESCRIPTION: SEQ ID NO: 302:

TTTTTTTTTT TTTTTTTTTT TTTTTNCAAG ATCATTGTTT ATTTATTACT TCAGATAAAA 60

AGATAGTATA CATATTAGGG AATCCCTTAA AATTCAACTC TAGAGTTATA CACCATCTAG 120

TACTTTTGCA ATGAATGTTA ACAACAACAA AAAAAATCTC TAAACACCTG AAAGCCCCAC 180

TATTAACATG GACTATGGTA ATAAAAAATT TTGACATTTA ATTTGTTCAA CATATAGTAT 240

TTACATTATG AAACCAATGG TGATGATACA ATAAAGTGAT AAAGAAATAG TAAAAATAAA 300

CTTTAAAAAG CAAAGGTTTA TAGTCTGACA ATGCTAATTA TCCTAATTGT ATATAAAAAA 360

TTAAAACATA GAGCTTTCTG TTACAAAATT CTTAATCCTC TGGGTTGTAA TCATTACTTG 420

CTACCAATTT ACATGCAACA TCTGCTAGGA CTGACATTTG ATTTTTTTCC CCAAGAATGT 480

GTGAGTAGAT AAATGACATT TCAGAGCAGA TATTAATTTA CTTGTGGACA GAAAAAGAAA 540

CTCAAGATTG GTACTGGTCA CAAGCCTCTT CCCAATAGAA ATTATAAAAA CAGTAAGATA 600

AAATTTAAAA AAAATCTAAA AAGGGGATGC ATAGGCAAAG AGTACCATAA ATGGCACAGC 660

TCAAAAAATC CCAGGACCAA TCAGACACAC ATCTTTTCTC TCTCCTTCAG CGACAAGAGG 720

TCGATTTTGC CATCAAATAA CCATGATTGA AGCAAGCGAG GGGCACCAGG TGTACAACTG 780

ATTAGATCTT GCAAAATACT AAGATGGGAG CAGGGGTGGC CAGAAGAAGG GGTAATTTAT 840

ATATAATTCA AACTATATAC AGCATAAATG GAATGCAGCC CATCCCAAAC TGGCTCTGTG 900

AAACAATTGG ACCTTTATAG TTAAAATTAT AACAAGTGTA ATAATACAAT AGATTTACAT 960

GGGAAGCAAA ATCCAAGGGA CATTTTATAT TAAGTATTTA CTGTGCTGTT TCAATTTAAA 1020

AATAATTTTG CTAAGTATAC ATCTCAACTG AAGTCTATGT AAAAAATGTC CTAATAGATA 1080

CAGATATTTA CCTTTGGTGA GTTGAAGGCC TTTTTGTGAC TTCTGTCTGA ACTGTAGGCA 1140

GAATGCTAGA TGTACATGCA CATATGGAGA AACTCAAGCT GAGGTCATCC AAAAGCTGTG 1200

CGTATGAGGA GGCTGGAGGT ACTTTGAAAG TCAAAGTAGA CCAGAAACCC AAAACAGGTA 1260

ACAGTGAGGA TGGCAACAGG GAATGGAATG CCAATATGGC AGTAAAACTT TTTTTAAAAA 1320

CAGAAAGAGG AAGGCCTCTC GTACCAGCAG AATCCTGTAC ACGTACAAAA AAGAAAAAGC 1380

CACCCACCAT TTTGTAAAAC AGAAGCCAAT TATAGTGTGG GAAAGTACAA ATTACAGAAA 1440

ACCAGAAGTC AACAGAAGAA AAACTACTGG TTTACTTGAG AGAAAGGAGA ATGGTTCACC 1500

CCGAGCAGAG TTACTTGGTG AACGCCGCCA CCACCGCCCA CAGAACCTCA TTGGTGTTGG 1560

CCTTCAGACA TTCCACTTCA GGGTCTAAGT CGAGAARNTG CCGCACTCTC TTGGTAGCCA 1620

AATCATACTG CTCGTCCAGA AGAGGAGCAA AAGCATTCTC CAGGACGTCC GAGGCATGAG 1680

CCAGGTAAAT GAGGGCCAGC AAGCGCCTGT CCATGCGGTG AGGGTCATTC ACCCATTTGT 1740

CAAGAACGGC TTCCTGTACT TTCTTGATGA GGCGCTGCTT AATGTTGTTA TTGGTGAGGG 1800

GATGTGTTGT CATGTCAAAA AGTAGGAAGT TCTGTTTCTC TGTTGTCAAT ACACCCTTTT 1860

CCACCAGGTT TTTAGCTAAT CGTTCCCGTA CATTTCTTAA CTGATAATGC AATTTTAATG 1920

GATTCCATGT CTCACCACTA AGTAATTCAA TCCAGTTCTG GACCGTTTCT GGAGGCTGAG 1980

TTTCCTTAAC ATGCTTCAGA GCTTCATCAA GAAGAACATC CCCTGTTGGA GCATCTGACT 2040

TACAGATTAC CTTTCTTGTT AATAGACTTT TACGTCTCAT TCCACAAGCC TCTAGTTGTA 2100

ACCTTCCTCT CAATGCTAAT TCAATTAACA TACAGCCACG TAATCCAGAT GATATACAGT 2160

CATTCCAAAA TGATGTGTAA ACCTTCGCGG TCCTTGAGGC CCAGCAGGAG CACTTCCTCC 2220

ATCAGGGTCA GCCGCGTTTC CTTGGAGTCG CCCTTGTCGT CGTCGTCCTG CTCGTCGCGG 2280

CGGCTCTGCG CGTCGTCCTC GCTGCTAGCC GCGCCGCCGC CCGCCGCCCG CTCCTTGTCG 2340

GCGGCGTTGC GGGAGGCCTC GGTGCGCCG 2369

(2) INFORMATION FOR SEQ ID NO: 303:

(i) SEQUENCE CHARACTERISTICS:
(A) LENGTH: 1181 base pairs
(B) TYPE: nucleic acid
(C) STRANDEDNESS: double
(D) TOPOLOGY: linear

(xi) SEQUENCE DESCRIPTION: SEQ ID NO: 303:

GGGACGTGTG GTTTCAGCTC GTGCGCCTCC CCGTGGGTTT GCGACGTTTA GCGACTATTG 60

CGCCTGCGCC ACGCCGGCTG CGAGACTGGG GCCGTGGYTG CTGGTCCCGG GTGATGCTAG 120

GCGGCTCCCT GGGCTCCAGG CTGTTGCGGG GTGTAGGTGG GAGTCACGGA CGGTTCGGGG 180

CCCGAGGTGT CCGCGAAGGT GGCGCACATG GGCGGCAGGG GAGAGCATGG CTCAGCGGAT 240

GGTCTGGGTG GACCTGGAGA TGACAGGATT GGACATTGAG AAGGACCAGA TTATTGAGAT 300

GGCCTGTCTG ATAACTGACT CTGATCTCAA CATTTTGGCT GAAGGTCCTA ACCTGATTAT 360

AAAACAACCA GATGAGTTGC TGGACAGCAT GTCAGATTGG TGTAAGGAGC ATCACGGGAA 420

GTCTGGCCTT ACCAAGGCAG TGAAGGAGAG TACAATTACA TTGCAGCAGG CAGAGTATGA 480

ATTTCTGTCC TTTGTACGAC AGCAGACTCC TCCAGGGCTC TGTCCACTTG CAGGAAATTC 540

AGTTCATGAA GATAAGAAGT TTCTTGACAA ATACATGCCC CAGTTCATGA AACATCTTCA 600

TTATAGAATA ATTGATGTGA GCACTGTTAA AGAACTGTGC AGACGCTGGT ATCCAGAAGA 660

ATATGAATTT GCACCAAAGA AGGCTGCTTC TCATAGGGCA CTTGATGACA TTAGTGAAAG 720

CATCAAAGAG CTTCAGTTTT ACCGAAATAA CATCTTCAAG AAAAAAATAG ATGAAAAGAA 780

GAGGAAAATT ATAGAAAATG GGGAAAATGA GAAGACCGTG AGTTGATGCC AGTTATCATG 840

CTGCCACTAC ATCGTTATCT GGAGGCAACT TCTGGTGGTT TTTTTTTCTC ACGCTGATGG 900

CTTGGCAGAG CMCTTCGGTT AACTTGCATC TCCAGATTGA TTACTCAAGC AGACAGCACA 960

CGAAATACTA TTTTTCTCCT AATATGCTGT TTCCATTATG ACACAGCAGC TCCTTTGTAA 1020

GTACCAGGTC ATGTCCATCC CTTGGTACAT ATATGCATTT GCTTTTAAAC CATTTCTTTT 1080

GTTTAAATAA ATAAATAAGT AAATAAAGCT AGTTCTATTG AAATGCAAAA AAAAAAAAAA 1140

AAAAAAAAAA AAAAAAAAAA AAAAAAAAAA AAAAAAAAAA N 1181

(2) INFORMATION FOR SEQ ID NO: 304:

(i) SEQUENCE CHARACTERISTICS:
(A) LENGTH: 1537 base pairs
(B) TYPE: nucleic acid
(C) STRANDEDNESS: double
(D) TOPOLOGY: linear

(xi) SEQUENCE DESCRIPTION: SEQ ID NO: 304:

CTTTTTGTGT TCCGGCCGAT CCCACCTCTC CTCGACCCTG GACGTCTACC TTCCGGAGGC 60

CCACATCTTG CCCACTCCGC GCGCGGGGCT AGCGCGGGTT TCAGCGACGG GAGCCCTCAA 120

GGGACATGGC AACTACAGCG GCGCCGGCGG GCGGCGCCCG AANATGGAGC TGGCCCGGAA 180

TGGGGAGGGT TCGAAGAAAA CATCCAGGGC GGAGGCTCAG CTGTGATTGA CATGGAGAAC 240

ATGGATGATA CCTCAGGCTC TAGCTTCGAG GATATGGGTG AGCTGCATCA GCGCCTGCGC 300

GAGGAAGAAG TAGACGCTGA TGCAGCTGAT GCAGCTGCTG CTGAAGAGGA GGATGGAGAG 360

TTCCTGGGCA TGAAGGGCTT TAAGGGACAG CTGAGCCGGC AGGTGGCAGA TCAGATGTGG 420

CAGGCTGGGA AAAGACAAGC CTCCAGGGCC TTCAGCTTGT ACGCCAACAT CGACATCCTC 480

AGACCCTACT TTGATGTGGA GCCTGCTCAG GTGCGAACAG GGCTCCTGGA GTCCATGATC 540

CCTATCAAGA TGGTCAACTT CCCCCAGAAA ATTGCAGGTG AACTCTATGG ACCTCTCATG 600

CTGGTCTTCA CTCTGGTTGC TATCCTACTC CATGGGATGA AGACGTCTGA CACTATTATC 660

CGGGAGGGCA CCCTGATGGG CACAGCCATT GGCACCTGCT TCGGCTACTG GCTGGGAGTC 720

TCATCCTTCA TTTACTTCCT TGCCTACCTG TGCAACGCCC AGATCACCAT GCTGCAGATG 780

TTGGCACTGC TGGGCTATGG CCTCTTTGGG CATTGCATTG TCCTGTTCAT CACCTATAAT 840

ATCCACCTCC ACGCCCTCTT CTACCTCTTC TGGCTGTTGG TGGGTGGACT GTCCACACTG 900

CGCATGGTAG CAGTGTTGGT GTCTCGGACC GTGGGCCCCA CACAGCGGCT GCTCCTCTGT 960

GGCACCCTGG CTGCCCTACA CATGCTCTTC CTGCTCTATC TGCATTTTGC CTACCACAAA 1020

GTGNTAGAGG GGATCCTGGA CACACTGGAG GGCCCCAACA TCCCGCCCAT CCAGAGGGTC 1080

CCCAGAGACA TCCCTGCCAT GCTCCCTGCT GCTCGGCTTC CCACCACCGT CCTCAACGCC 1140

ACAGCCAAAG CTGTTGCGGT GACCCTGCAG TCACACTGAC CCCACCTGAA ATTCTTGGCC 1200

AGTCCTCTTT CCCGCAGCTG CAGAGAGGAG GAAGACTATT AAAGGACAGT CCTGATGACA 1260

TGTTTCGTAG ATGGGGTTTG CAGCTGCCAC TGAGCTGTAG CTGCGTAAGT ACCTCCTTGN 1320

AGCTGTCGGC ACTTCTGAAA GCACAAGGCC AAGAACTCCT GGCCAGGACT GCAAGGCTCT 1380

GCAGCCAATG CAGAAAATGG GTCAGCTCCT TTGAGAACCC CTCCCCACCT ACCCCTTCCT 1440

TCCTCTTTAT CTCTCCCACA TTGTCTTGCT AAATATAGAC TTGGTAATTA AAAAAAAAAA 1500

AAAAAAAAAA AAAAAAAAAA AAAAAAGGGG GGNCCCC 1537

(2) INFORMATION FOR SEQ ID NO: 305:

(i) SEQUENCE CHARACTERISTICS:
(A) LENGTH: 1493 base pairs
(B) TYPE: nucleic acid
(C) STRANDEDNESS: double
(D) TOPOLOGY: linear

(xi) SEQUENCE DESCRIPTION: SEQ ID NO: 305:

TGCATGCCAA AACCAATGCC TGCCAAACAA AATCTTAGAC ATCCCAATAT AATATGTTAG 60

TTATATTTCT ATTCACATCA TTATTGAAAA TACCCAGCTC AGTGCCTGGC TTAATAAATG 120

TTTAATTCCC TTACCTACTC TTGCTCTATT TTTTTATTTG AAATGGAGAT GAGCAAAATA 180

ACACATTCAT GGCTGAAGCA ATTTTTTGGA CATTTCTTGT TACCAAAAGA TCTATAATCA 240

GGATGATCCT GAGCTGTTCA AACAAGCTGT ATATAAACAG ACAATGAAAC TCTTTGCAGA 300

GCTGGAAATT AAAAGGAAAG AGAGAGAAGC CAAAGAGATG CATGAAAGGA AACGACAAAG 360

GGAAGAAGAG ATTGAAGCTC AAGAAAAAGC CAAACGGGAA AGAGAGTGGC AGAAAAACTT 420

TGAGGAAAGT CGAGATGGTC GTGTGGACAG CTGGCGAAAC TTCCAAGCCA ATACGAAGGG 480

GAAGAAAGAG AAGAAAAATC GGACCTTCCT GAGACCACCG AAAGTAAAAA TGGAGCAACG 540

TGAGTGACCG CCCAAGGTCA CAGGCACAGA ACCTTTCCCC TGCTATCTCC CTTCCTGCTT 600

CGAAGGACTC ATTCTTTCCT CCCACTTCCA CCCCAACATA GAGTAGTATT TGCTTTTTAG 660

TCCATTTTGT TTTCAATACG ATTTAATATC GATCAGAGTA ATTCTTTTGT ACATTGAAAT 720

GAGGGGCTTG GTTTAAAAAA AGACCTTTCC CTCTCCCTGC CCCTAGAACA ACCAGTATTA 780

GAAGGTGCCA CCATTGGTGC TGCCTTCTCT TCCCACAGCC TGTAACTCAG TGTTTTGTAC 840

TTCACTGAAT TGTGATGGTT AGAAACTTCG TGGATAGTTT GTGGAAATCA TCCAATTAAA 900

CATACTGCTT AAAACAGTGT TGCTGTGACT TCAGAGACAA GCCTGGAAGG GGCACCTTAG 960

GAAGCCCCTT CGCTTCAGTT GCTCGCTTCT GGGTGTGCTC CCTTCGAAGG CCCAGATAAG 1020

ACAGGGAACA CTTGTGAGCA CACAGAGCAG CATCTGATGC CCTGTGGTGT TTGGCATGTG 1080

CCCCCTGTCT ACTGACCAAT CAGTGTGGCA TGAGGCCCAC GCCACCCAAA CCTTTCACTT 1140

TCCAAAGAGC TAGCCGTCCT CCACCCAGTA CCATGTCCTA GCCTGTCTGC ATTTGTTAGT 1200

GGTAATATTC TTTATGTATA ATAAATTTTT ATACCCAAGC CATTGATGTA CTTTTCCTTG 1260

TACTCTCCCT TGTGGGTCCC TTGTCTGGCT TGGCTGAACC CCAAAATGCT TTGGGGTTGG 1320

ACAGACCTGG CTGAACCTTA GTTTCTTCAT CTATGAAATG GGAATATGAA TTACTGCAGC 1380

AGCTTTTAGG GCAGATTTGC CATGGCATAT ACAAGGTAAC TACCATAGTG CTCCTTGGGT 1440

ATTGCCAATA TCCTATTATT TCTGTGTAAA ATGAAGATAC TGATTGTTTT GAG 1493

(2) INFORMATION FOR SEQ ID NO: 306:

(i) SEQUENCE CHARACTERISTICS:
(A) LENGTH: 577 base pairs
(B) TYPE: nucleic acid
(C) STRANDEDNESS: double
(D) TOPOLOGY: linear

(xi) SEQUENCE DESCRIPTION: SEQ ID NO: 306:

AATTCGGCAG AGGNATTATA TACACTATAC TGGCATTTAC TGTTTCACCC AGCCCGGAAA 60

GTCAGAGATG TATATTGGAA AATTTACAAC TCCATCTACA TTGGTTCCCA GGACGCTCTC 120

ATAGCACATT ACCCAAGAAT CTACAACGAT GATAAGAACA CCTATATTCG TTATGAACTT 180

GACTATATCT TATAATTTTA TTGTTTATTT TGTGTTTAAT GCACAGCTAC TTCACACCTT 240

AAACTTGCTT TGATTTGGTG ATGTAAACTT TTAAACATTG CAGATCAGTG TAGAACTGGT 300

CATAGAGGAA GAGCTAGAAA TCCAGTAGCA TGATTTTTAA ATAACCTGTC TTTGTTTTTG 360

ATGTTAAACA GTAAATGCCA GTAGTGACCA AGAACACAGT GATTATATAC ACTATACTGG 420

AGGGATTTCA TTTTTAATTC ATCTTTATGA AGATTTAGAA CTCATTCCTT GTGTTTAAAG 480

GGAATGTTTA ATTGAGAAAT AAACATTTGT GWACAAAATG YTAAAAAAAA AAAAAAAAAA 540

AAAAAAAAAA AAAAAAAAAA AAAAAAAAAA AACTCGA 577

(2) INFORMATION FOR SEQ ID NO: 307:

(i) SEQUENCE CHARACTERISTICS:
(A) LENGTH: 2860 base pairs
(B) TYPE: nucleic acid
(C) STRANDEDNESS: double
(D) TOPOLOGY: linear

(xi) SEQUENCE DESCRIPTION: SEQ ID NO: 307:

GTGTNGACCG CTCTCNCAAT ATGGCTCCCC CGGGCTGGCA GRWRKTCRGT CWCKRGTGGC 60

TAGCCTGTCC TGACAGGGGA GAGTTAAGCT CCCGTTCTCC ACCGTGCCGG CTGGCCAGGT 120

GGGCTGAGGG TGACCGAGAG ACCAGAACCT GCTTGCTGGA GCTTAGTGCT CAGAGCTGGG 180

GAGGGAGGTT CCGCCGCTCC TCTGCTGTCA GCGCCGGCAG CCCCTCCCGG CTTCACTTCC 240

TCCCGCAGCC CCTGCTACTG AGAAGCTCCG GGATCCCAGC AGCCGCCACG CCCTGGCCTC 300

AGCCTGCGGG GCTTCCAGTC AGGCCAACAC CGACGCGCAC TGGGGAGGAA GACAGGACCC 360

TTGACATCTC CATCTGCACA GAGGTCCTGG CTGGAACCGA GCAGCCTCCT CCTCCTAGGA 420

TGACCTCACC CTCCAGCTCT CCAGTTTTCA GGTTGGAGAC ATTAGATGGA GGCCAAGAAG 480

ATGGCTCTGA GGCGGACAGA GGAAAGCTGG ATTTTGGGAG CGGGCTGCCT CCCATGGAGT 540

CACAGTTCCA GGGCGAGGAC CGGAAATTCG CCCCTTCAGA TAAGAGTCAA CCTCCAACTA 600

CCGAAAGGGA ACAGGTGCCA GTCAGCCGGA TCCAAACCGA TTTGACCGAG ATCGGCTCTT 660

CAATGCGGTC TCCCGGGGTG TCCCCGAGGA TCTGGCTGGA CTTCCAGAGT ACCTGAGCAA 720

GACCAGCAAG TACCTCACCG ACTTCGGAAA TACACAGAGG GCTCCACAGG TAAGACGGCC 780

TGATGAAGGC TGTGCTGAAA CCTTAAGGAC GGGGTCAATG CCTGCATTCT GCCACTGCTG 840

CAGATCGACC GGGACTCTGG CAATCCTCAG CCCCTGGTAA ATGCCCAGTG CACAGATGAC 900

TATTACCGAG GCCACAGCGC TCTGCACATC GCCATTGAGA AAGAGGAGTC TGCAGTGTGT 960

GAAGCTCCTG GTGGAGAATG GGGCCAATGT GCATGCCCGG GTCTGCGGCG ACTTCTTCCA 1020

GAAGGGCCAA GGGACTTGCT TTTATTTCGG TGAGCTACCC CTCTCTTTGG CCGCTTGCAC 1080

CAAGCAGTGG GATGTGGTAA GCTACCTCCT GGAGAACCCA CACCAGCCCG CCAGCCTGCA 1140

GGCCACTGAC TCCCAGGGCA ACACAGTCCT GCATGCCCTA GTGGATGATC TCGGACAACT 1200

CAGCTGAGAA CATTGCACTG GTGACCAGCA TGTATGATGG GCTCCTCCAA GCTKGGGSCC 1260

SCCYTCTGCC CTACCGTGCA GCTTGAGGAC ATCCGCAACC TGCAGGATCT CACGCCTCTG 1320

AAGCTGGCCG CCAAGGAGGG CAAGATCGAG ATTTTCAGGC ACATCCTGCA GCGGGAGTTT 1380

TCAGGACTGA GCCACCTTTC CCGAAAGTTC ACCGAGTGGT GCTATGGGCC TGTCCGGGTG 1440

TCGCTGTATG ACCTGGCTTC TGTGGACAGC TGTGAGGAGA ACTCAGTGCT GGAGATCATT 1500

GCCTTTCATT GCAAGAGCCC GCACCGACAC CGAATGGTCG TTTTGGAGCC CCTGAACAAA 1560

CTGCTGCAGG CGAAATGGGA TCTGCTCATC CCCAAGTTCT TCTTAAACTT CCTGTGTAAT 1620

CTGATCTACA TGTTCATCTT CACCGCTGTT GCCTACCATC AGCCTACCCT GAAGAAGCAG 1680

GCCGCCCCTC ACCTGAAAGC GGAGGTTGGA AACTCCATGC TGCTGACGGG CCACATCCTT 1740

ATCCTGCTAG GGGGGATCTA CCTCCTCGTG GGCCAGCTGG TGGTACTTCT GGCGGCGCCA 1800

CGTGTTCATC TGGATCTCGT TCATAGACAG CTACTTTGGA AATCCTCTTC CTGTTCCAGG 1860

CCCTGCTTCA CAGTGGTGTC CCAGGTGCTG TGTTTCCTGG GCCATCGAGT GGTACCTGCC 1920

CCTGCTTGTG TCTGCGCTGG TGGCTGGGCT GGCTGAACCT GCTTTACTAA TACACGTGGC 1980

GTTCCAGCAC ACAGGCAGTC TACAGTTTCA TGWTCCCTGA AGCCCTGGTG AGCCTGAGCC 2040

AGGAGGCTTG GCGCCCCGAA GCTCCTACAG GCCCCAATGC CACAGAGTCA GTGCAGCCCA 2100

TGGAGGGACA GGAGGACGAG GGCAACGGGG CCCAGTACAG GGGTATCCTG GAAGCCTCCT 2160

TGGAGCTCTT CAAATTCACC ATCGGCATGG GCGAGCTGGC CTTCCAGGAG CAGCTGCACT 2220

TCCGCGGCAT GGTGCTGCTG CTGCTGCTGG CCTACGTGCT GCTCACCTAC ATCCTGCTGC 2280

TCAACATGCT CATCGCCCTC ATGAAGCGAA CGTCACAGTG TCGCCACTGA CAGCTGGAGC 2340

ATCTGGAAGC TGCAGAAAGC CATCTCTGTC CTGGAGATGG AGAATGGCTA TTGGTGGTGC 2400

AGGAAAAAGC AGCGGGCAGG TGTGATGCTG ACCGTTGGCA CTAAGCCCAG ATGGCAGCCC 2460

CGATGAGCGC TGGTGCTTCA GGGTGGAGGA GGTGAACTGG GCTTCATGGG GAGCAGACGC 2520

TGCCTACGCT GTGTGAGGAC CCGTCAGGGG CAGGTGTCCC TCGAACTCTC GAGAACCCTG 2580

TCCTGGCTTC CCCTCCCAAG GAGGATGAGG ATGGTGCCTC TGAGGAAAAC TATGTGCCCG 2640

TCCAGCTCCT CCAGTCCAAC TGATGGCCCA GATGCAGCAG GAGGCCAGAG GACAGAGCAG 2700

AGGATCTTTC CAACCACATC TGCTGGCTCT GGGGTCCCAG TGAATTCTGG TGGCAAATAT 2760

ATATTTTCAC TAACTCAAAA AAAAAAAAAA AAAAAAAAAA AAAAVGAGGG GGGGCCCGKT 2820

ASCCAAWTTC GCCCTATAAG TGAGTGCCWA TTACGATAAA 2860

(2) INFORMATION FOR SEQ ID NO: 308:

(i) SEQUENCE CHARACTERISTICS:
(A) LENGTH: 876 base pairs
(B) TYPE: nucleic acid
(C) STRANDEDNESS: double
(D) TOPOLOGY: linear

(xi) SEQUENCE DESCRIPTION: SEQ ID NO: 308:

CTGCTTGTGT CTGCGCTGGT GCTGGGCTGG CTGAACCTGC TTTACTATAC ACGTGGCTTC 60

CAGCACACAG GCATCTACAG TGTCATGATC CAGAAGCCCT GGTGAGCCTG AGCCAGGANN 120

TTGGCGCCCC GAAGCTCCTA CAGGCCCCAA TGCCACAGAG TCAGTGCAGC CCATGGAGGG 180

ACAGGAGGAC GAGGGCAACG GGGCCCAGTA CAGGGGTATC CTGGAAGCCT CCTTGGAGCT 240

CTTCAAATTC ACCATCGGCA TGGGCGAGCT GGCCTTCCAG GAGCAGCTGC ACTTCCGCGG 300

CATGGTGCTG CTGCTGCTGC TGGCCTACGT GCTGCTCACC TACATCCTGC TGCTCAACAT 360

GCTCATCGCC CTCATGNAGC GAGACCGWCA ACAGTGTCGC CACTGACAGC TGGAGCATCT 420

GGAAGCTGCA GAAAGCCATC TCTGTCCTGG AGATGGAGAA TGGCTATTGG TGGTGCAGGA 480

AGAAGCAGCG GGCAGGTGTG ATGCTGACCG TTGGCACTAA GCCAGATGGC AGCCCCGATG 540

AGCGCTGGTG CTTCAGGGTG GAGGAGGTGA ACTGGGCTTC ATGGGAGCAG ACGCTGCCTA 600

CGCTGTGTGA GGACCCGTCA GGGGCAGGTG TCCCTCGAAC TCTCGAGAAC CCTGTCCTGG 660

CTTCCCCTCC CAAGGAGGAT GAGGATGGTG CCTCTGAGGA AAACTATGTG CCCGTCCAGC 720

TCCTCCAGTC CAACTGATGG CCCAGATGCA GCAGGAGGCC AGAGGACAGA GCAGAGGATC 780

TTTCCAACCA CATCTGCTGG CTCTGGGGTC CCAGTGAATT CTGGTGGCAA ATATATATTT 840

TCACTAAMWM AAAAAAAAAA AAAAAAAAAA ACTCGA 876

(2) INFORMATION FOR SEQ ID NO: 309:

(i) SEQUENCE CHARACTERISTICS:
(A) LENGTH: 2025 base pairs
(B) TYPE: nucleic acid
(C) STRANDEDNESS: double
(D) TOPOLOGY: linear

(xi) SEQUENCE DESCRIPTION: SEQ ID NO: 309:

CATGACCCGC CTGATGCGAT CCCGCACAGC CTCTGGTTCC AGCGTCACTT CTCTGGATGG 60

CACCCGCAGC CGCTCCCACA CCAGCGAGGG CACCCGAAGC CGCTCCCACA CCAGCGAGGG 120

CACCCGCAGC CGCTCGCACA CCAGCGAGGG GGCCCACCTG GACATCACCC CCAACTCGGG 180

TGCTGCTGGG AACASGCCGG GCCCAAGTCC ATGGAGGTCT CCTGCTAGGC GGCCTGCCCA 240

GCTGCCGCCC CCGGACTCTG ATCTCTGTAG TGGCCCCCTC CTCCCCGGCC CCTTTTCGCC 300

CCCTGCCTGC CATACTGCGC CTAACTCGGT ATTAATCCAA AGCTTATTTT GTAAGAGTGA 360

GCTCTGGTGG AGACAAATGA GGTCTATTAC GTGGGTGCCC TCTCCAAAGG CGGGGTGGCG 420

GTGGACCAAA GGAAGGAAGC AAGCATCTCC GCATCGCATC CTCTTCCATT AACCAGTGGC 480

CGGTTGCCAC TCTCCTCCCC TCCCTCAGAG ACACCAAACT GCCAAAAACA AGACGCGTAC 540

AGCACACACT TCACAAAGCC AAGCCTAGGC CGCCCTGAGC ATCCTGGTTC AAACGGGTGC 600

CTGGTCAGAA GGCCAGCCGC CCACTTCCCG TTTCCTCTTT AACTGAGGAG AAGCTGATCC 660

AGTTTCCGGA AACAAAATCC TTTTCTCATT TGGGGAGGGG GGTAATAGTG ACATGCAGGC 720

ACCTCTTTTA AACAGGCAAA ACAGGAAGGG GGAAAAGGTG GGATTCATGT CGAGGCTAGA 780

GGCATTTGGA ACAACAAATC TACGTAGTTA ACTTGAAGAA ACCGATTTTT AAAGTTGGTG 840

CATCTAGAAA GCTTTGAATG CAGAAGCAAA CAAGCTTGAT TTTTCTAGCA TCCTCTTAAT 900

GTGCAGCAAA AGCAGGCRAC AAAATCTCCT GGCTTTACAG ACAAAAATAT TTCAGCAAAC 960

GTTGGGCATC ATGGTTTTTG AAGGCTTTAG TTCTGCTTTC TGCCTCTCCT CCACAGCCCC 1020

AACCTCCCAC CCCTGATACA TGAGCCAGTG ATTATTCTTG TTCAGGGAGA AGATCATTTA 1080

GATTTGTTTT GCATTCCTTA GAATGGAGGG CAACATTCCA CAGCTGCCCT GGCTGTGATG 1140

AGTGTCCTTG CAGGGGCCGG AGTAGGAGCA CTGGGGTGGG GGCGGAATTG GGGTTACTCG 1200

ATGTAAGGGA TTCCTTGTTG TTGTGTTGAG ATCCAGTGCA GTTGTGATTT CTGTGGATCC 1260

CAGCTTGGTT CCAGGAATTT TGTGTGATTG GCTTAAATCC AGTTTTCAAT CTTCGACAGC 1320

TGGGCTGGAA CGTGAACTCA GTAGCTGAAC CTGTCTGACC CGGTCACGTT CTTGGATCCT 1380

CAGAACTCTT TGCTCTTGTC GGGGTGGGGG TGGGAACTCA CGTGGGGAGC GGTGGCTGAG 1440

AAAATGTAAG GATTCTGGAA TACATATTCC ATGGGACTTT CCTTCCCTCT CCTGCTTCCT 1500

CTTTTCCTGC TCCCTAACCT TTCGCCGAAT GGGGCAGCAC CACTGACGTT TCTGGGCGGC 1560

CAGTGCGGCT GCCAGGTTCC TGTACTACTG CCTTGTACTT TTCATTTTGG CTCACCGTGG 1620

ATTTTCTCAT AGGAAGTTTG GTCAGAGTGA ATTGAATATT GTAAGTCAGC CACTGGGACC 1680

CGAGGATTTC TGGGACCCCG CAGTTGGGAG GAGGAAGTAG TCCAGCCTTC CAGGTGGCGT 1740

GAGAGGCAAT GACTCGTTAC CTGCCGCCCA TCACCTTGGA GGCCTTCCCT GGCCTTGAGT 1800

AGAAAAGTCG GGGATCGGGG CAAGAGAGGC TGAGTACGGA TGGGAAACTA TTGTGCACAA 1860

GTCTTTCCAG AGGAGTTTCT TAATGAGATA TTTGTATTTA TTTCCAGACC AATAAATTTG 1920

TAACTTTGCA AAAAAAAAAA AAAAAAAAAA AAAAAAAAAA AAAAAAAAAA AAAAAAACTC 1980

GAGGGGGGCC CGTACCCAAT TCGCCGTATA TGATCGTAAA CAATC 2025

(2) INFORMATION FOR SEQ ID NO: 310:

(i) SEQUENCE CHARACTERISTICS:
(A) LENGTH: 3026 base pairs
(B) TYPE: nucleic acid
(C) STRANDEDNESS: double

(D) TOPOLOGY: linear

(xi) SEQUENCE DESCRIPTION: SEQ ID NO: 310:

TAGGCAGCAC TGAAATATCC TAACCCCCTA AGCTCCAGGT GCCCTGTGGN ACGAGCAACT 60

GGACTATAGC AGGGCTGGGC TCTGTCTTCC TGGTCATAGG CTCACTCTTT CCCCCAAATC 120

TTCCTCTGGA GCTTTGCAGC CAAGGTGCTA AAAGGAATAG GTAGGAGACC TCTTCTATCT 180

AATCCTTAAA AGCATAATGT TGAACATTCA TTCAACAGCT GATGCCCTAT AACCCCTGCC 240

TGGATTTCTT CCTATTAGGC TATAAGAAGT AGCAAGATCT TTACATAATT CAGAGTGGTT 300

TCATTGCCTT CCTACCCTCT CTAATGGCCC CTCCATTTAT TTGACTAAAG CATCACACAG 360

TGGCACTAGC ATTATACCAA GAGTATGAGA AATACAGTGC TTTATGGCTC TAACATTACT 420

GCCTTCAGTA TCAAGGCTGC CTGGAGAAAG GATGGCAGCC TCAGGGCTTC CTTATGTCCT 480

CCACCACAAG AGCTCCTTGA TGAAGGTCAT CTTTTTCCCC TATCCTGTTC TTCCCCTCCC 540

CGCTCCTAAT GGTACGTGGG TACCCAGGCT GGTTCTTGGG CTAGGTAGTG GGGACCAAGT 600

TCATTACCTC CCTATCAGTT CTAGCATAGT AAACTACGGT ACCAGTGTTA GTGGGAAGAG 660

CTGGGTTTTC CTAGTATACC CACTGCATCC TACTCCTACC TGGTCAACCC GCTGCTTCCA 720

GGTATGGGAC CTGCTAAGTG TGGAATTACC TGATAAGGGA GAGGGAAATA CAAGGAGGGC 780

CTCTGGTGTT CCTGGCCTCA GCCAGCTGCC CACAAGCCAT AAACCAATAA AACAAGAATA 840

CTGAGTCAGT TTTTTATCTG GGTTCTCTTC ATTCCCACTG CACTTGGTGC TGCTTTGGCT 900

GACTGGGAAC ACCCCATAAC TACAGAGTCT GACAGGAAGA CTGGAGACTG TCCACTTCTA 960

GCTCGGAACT TACTGTGTAA ATAAACTTTC AGAACTGCTA CCATGAAGTG AAAATGCCAC 1020

ATTTTGCTTT ATAATTTCTA CCCATGTTGG GAAAAACTGG CTTTTTCCCA GCCCTTTCCA 1080

GGGCATAAAA CTCAACCCCT TCGATAGCAA GTCCCATCAG CCTATTATTT TTTTAAAGAA 1140

AACTTGCACT TGTTTTTCTT TTTACAGTTA CTTCCTTCCT GCCCCAAAAT TATAAACTCT 1200

AAGTGTAAAA AAAAGTCTTA ACAACAGCTT CTTGCTTGTA AAAATATGTA TTATACATCT 1260

GTATTTTTAA ATTCTGCTCC TGAAAAATGA CTGTCCCATT CTCCACTCAC TGCATTTGGG 1320

GCCTTTCCCA TTGGTCTGCA TGTCTTTTAT CATTGCAGGC CAGTGGACAG AGGGAGAAGG 1380

GAGAACAGGG GTCGCCAACA CTTGTGTTGC TTTCTGACTG ATCCTGAACA AGAAAGAGTA 1440

ACACTGAGGC GCTCGCTCCC ATGCACAACT CTCCAAAACA CTTATCCTCC TGCAAGAGTG 1500

GGCTTTCCAG GGTCTTTACT GGGAAGCAGT TAAGCCCCCT CCTCACCCCT TCCTTTTTTC 1560

TTTCTTTACT CCTTTGGCTT CAAAGGATTT TGGAAAAGAA ACAATATGCT TTACACTCAT 1620

TTTCAATTTC TAAATTTGCA GGGGATACTG AAAAATACGG CAGGTGGCCT AAGGCTGCTG 1680

TAAAGTTGAG GGGAGAGGAA ATCTTAAGAT TACAAGATAA AAAACGAATC CCCTAAACAA 1740

AAAGAACAAT AGAACTGGTC TTCCATTTTG CCACCTTTCC TGTTCATGAC AGCTACTAAC 1800

CTGGAGACAG TAACATTTCA TTAACCAAAG AAAGTGGGTC ACCTGACCTC TGAAGAGCTG 1860

AGTACTCAGG CCACTCCAAT CACCCTACAA GATGCCAAGG AGGTCCCAGG AAGTCCAGCT 1920

CCTTAAACTG ACGCTAGNMA ATAAACCTGG GCAAGTGAGG CAAGAGAAAT GAGGAAGAAT 1980

CCATCTGTGA GGTGAYAGGC AAGGATGAAA GACAAAGAAG GAAAAGAGTA TCAAAGGCAG 2040

AAAGGAGATC ATTTAGTTGG GTCTGAAAGG AAAAGTCTTT GCTATCCGAC ATGTACTGCT 2100

AGTACCTGTA AGCATTTTAG GTCCCAGAAT GGAAAAAAAA ATCAGCTATT GGTAATATAA 2160

TAATGTCCTT TCCCTGGAGT CAGTTTTTTT AAAAAGTTAA CTCTTAGTTT TTACTTGTTT 2220

AATTCTAAAA GAGAAGGGAG CTGAGGCCAT TCCCTGTAGG AGTAAAGATA AAAGGATAGG 2280

AAAAGATTCA AAGCTCTAAT AGAGTCACAG CTTTCCCAGG TATAAAACCT AAAATTAAGA 2340

AGTACAATAA GCAGAGGTGG AAAATGATCT AGTTCCTGAT AGCTACCCAC AGAGCAAGTG 2400

ATTTATAAAT TTGAAATCCA AACTACTTTC TTAATATCAC TTTGGTCTCC ATTTTTCCCA 2460

GGACAGGAAA TATGTCCCCC CCTAACTTTC TTGCTTCAAA AATTAAAATC CAGCATCCCA 2520

AGATCATTCT ACAAGTAATT TTGCACAGAC ATCTCCTCAC CCCAGTGCCT GTCTGGAGCT 2580

CACCCAAGGT CANCCAAACA ACTTGGTTGT GAACCCAACT GCCTTAACCT TCTGGGGGAG 2640

GGGGATTAGC TAGACTAGGA GACCCAGAAG TGAATGGGAA AGGGTGAGGA CTTCACAATG 2700

TTGGCCTGTC AGAGCTTGAT TAGAAGCCAA GACAGTGGCA GCAAAGGAAG ACTTGGCCCA 2760

GGAAAAACCT GTGGGTTGTG CTAATTTCTG TCCAGAAAAT AGGGTGGACA GAAGCTTGTG 2820

GGGTGCATGG AGGAATTGGG ACCTGGTTAT GTTGTTATTC TCGGACTGTG AATTTTGGTG 2880

ATGTAAAACA GAATATTCTG TAAACCTAAT GTCTGTATAA ATAATGAGCG TTAACACAGT 2940

AAAATATTCA ATAAGAAGTC AAAAAAAAAA AAAAAAAACT CGAGGGGGGG CCCGGTACCC 3000

AATTTNCCAA ATAGAGATNG TATTAC 3026

(2) INFORMATION FOR SEQ ID NO: 311:

(i) SEQUENCE CHARACTERISTICS:
(A) LENGTH: 712 base pairs
(B) TYPE: nucleic acid
(C) STRANDEDNESS: double
(D) TOPOLOGY: linear

(xi) SEQUENCE DESCRIPTION: SEQ ID NO: 311:

GCAGGCTTTG TGCTCACCTA CAAGCTGGGT GAGCAGGGTG CCAGCAGCCT GTTTCCTCTT 60

CTCCTGCTGG ACCACGGCGT TTCTGCTCCC GAGTTGGGAC TGTGGAATGG TGTGGGTGCT 120

GTGGTCTGCT CCATCGCTGG CTCCTCCCTG GGTGGGACCT TGCTGGCCAA GCACTGGAAA 180

CTGCTGCCTC TGTTGARGTC GGTGCTGCGC TTCCGCCTCG GGGGCCTAGC CTGTCAGACT 240

GCCTTGGTCT TCCACCTGGA CACCCTGGGG GCCAGCATGG ACGCTGGCAC AATCTTGAGA 300

GGGTCAGCCT TGCTGAGCCT ATGTCTGCAG CACTTCTTGG GAGGCCTGGT CACCACAGTC 360

ACCTTCACTG GGATGATGCG CTGCAGCCAG CTGGCCCCCA GGGCCTGCAG GCCACACACT 420

ACAGCCTTCT GGCCACGCTG GAGCTGCTGG GGAAGCTGCT GCTGGGCACT CTGCGGAGGC 480

CTGGCTGATG GGTTGGGGCC ACATCCCTGC TTCTTGCTCC TGCTCATCCT CTCTGCCTTT 540

CCCGTTCTGT ACCTGGACCT AGCACCCAGC ACCTTTCTCT GAGCTGAGTG GCTGGAGTGG 600

TCAATAAAGC CACATGTGCC TGTGGCCCAA AAAAAAAAAA AAAAAAAAAA AAAAAAAAAA 660

AACTGGAGGG GGGGCCCGGT ACCCAAATCG CCGGATATGA TCGTAAACAA TC 712

(2) INFORMATION FOR SEQ ID NO: 312:

(i) SEQUENCE CHARACTERISTICS:
(A) LENGTH: 1289 base pairs
(B) TYPE: nucleic acid
(C) STRANDEDNESS: double
(D) TOPOLOGY: linear

(xi) SEQUENCE DESCRIPTION: SEQ ID NO: 312:

CAAAATTTCA GAACTTTCAG GAGGGCAAGA GAATATCAAA CAAAGATTTC TGGAAGTATT 60

TTGCCAACCT TCTGGTTGAG CTGCAAGAAA ATATTTATGG TGAGAACTTT TCTGTTTCCC 120

GTTATTGGGT TTTTGGTTGG TTTTTGTTTG TTTTTTACTA TGCTTTGGTC TGTAAAAATA 180

TGCAACTGAA CTACATTCAG AAGGAAATAT TGTCTACATA GAATATTATA TGAAGTTGGT 240

ACATAATTCT GATGAGGAAA AAAAATCTTT GCAATTCTTT AAGCCATATT GTTGTTTTTC 300

TGTGTTGTTT TCCCTGGATG AAAATATCAG TATTAAGTAG ACAGCATATT ATTCAAGTGT 360

TTAGACTTAT TAATATGTTC TTGTCCTGTA TTTATACATA TGTGTATTTT GGAAAGTATT 420

GCCTTTTTTA AGGGAAGCTA TAATTCGATA CATAGTGAAA AAGGGAATGG TGACCCCTTT 480

GTGCCTCTTC CACTGAGGAT AACAAACAGC ATTGTAATCC ATTCTCTTGC ACCTTCTTCT 540

TCTTATCTTG TTATTACGGT TTTATTAATT TTGTAGAGGG ACAGGGAGTG GGCAAGGGGA 600

AGAAGCAGCT TATTTGACTA ACCAGCCCCT CTGTGGTCCA CCAGCGTCTT GGCTTGGTGG 660

GAGGGCTCTC AATCAGCAGG GCCCCAGGAG GGAAGAAGAA GTGGGGCAAA GCCTGGCCTC 720

GCCGCTCGGG AGCTTTGCCA TCTGAGCCAC GCCTCCTCCA GGCCATGCTC CTTGAACTTG 780

GAAATGTCAA CCGGAGCCCT TACACCAGCC CTCCAGCATC TAATAGACTT GAATCTACTC 840

TAAACGAATA TTTAATCCAA CCTCACTACA TTGTAGCTCA GTCCAACGAC TAACCCTGAA 900

ATGGGGGTGT TCCAGCCTTC AGCGAGATGG CCAAGCGGTC CCCTGGGGGC TGTGGCAGCG 960

GGCTTATCCT TCTCTGTTGC CAACCTTGCC GTCCGACCTC CTCCGCCCCC ATGCGGTGAC 1020

CCCGTCCGTG TCTGTGTCTG TCCATACGTG TGAGTCCAGC TAAAAAGACA AAACAGAACC 1080

CGTGGGCCCA GCTCGGAAGG TGCGTGGAGA AGGCTCCGAC GTCTCCGAAG TGCAGCCCTT 1140

GGGATGGCAT TCCGTTGTGT GCCTTATTCC TGGAGAATCT GTATACGGCT CGCCTATAGA 1200

AATATAGCCT CTTCATGCTG TATTAAAAGG ACTTTTAAAA GCAAAAAAAA AAAAAAAAAA 1260

CTTGAGGGGG GGNCCGGTAC CCAATTNTC 1289

(2) INFORMATION FOR SEQ ID NO: 313:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 313:

Met Phe Leu Ile Phe Val Tyr Phe Leu Lys Ile Leu Phe Ser Ser Ser
1               5                   10                  15

Leu Pro Phe Leu Trp Leu
            20

(2) INFORMATION FOR SEQ ID NO: 314:

(i) SEQUENCE CHARACTERISTICS:
(A) LENGTH: 128 amino acids
(B) TYPE: amino acid
(D) TOPOLOGY: linear
(xi) SEQUENCE DESCRIPTION: SEQ ID NO: 314:

(2) INFORMATION FOR SEQ ID NO: 315:

        (i) SEQUENCE CHARACTERISTICS:
                (A) LENGTH: 28 amino acids
                (B) TYPE: amino acid
                (D) TOPOLOGY: linear
        (xi) SEQUENCE DESCRIPTION: SEQ ID NO: 315:

Met Gln Phe Leu Leu Thr Ala Phe Leu Leu Val Pro Leu Leu Ala Leu
  1               5                   10                  15

Cys Asp Val Pro Ile Ser Leu Gly Phe Ser Pro Ser
            20                  25

(2) INFORMATION FOR SEQ ID NO: 316:

        (i) SEQUENCE CHARACTERISTICS:
                (A) LENGTH: 64 amino acids
                (B) TYPE: amino acid
                (D) TOPOLOGY: linear
        (xi) SEQUENCE DESCRIPTION: SEQ ID NO: 316:

Met Asp Gly Phe Ser Ser Arg Leu Phe Ser Ser Leu Pro Phe Val Ala
  1               5                   10                  15

Leu Gln Trp Phe Ile Val Ile Ser His Leu Leu Ser Leu Ser Leu Ser
            20                  25                  30

Ala Cys Cys Tyr Gln Thr His Cys Ser Leu Xaa Gln Leu Ser Ser Ala
        35                  40                  45

Phe Ser Xaa Met Gly Glu Ser Cys Val Gly Glu Arg Glu Tyr Xaa Phe
    50                  55                  60

(2) INFORMATION FOR SEQ ID NO: 317:

        (i) SEQUENCE CHARACTERISTICS:

(A) LENGTH: 21 amino acids
(B) TYPE: amino acid
(D) TOPOLOGY: linear
(xi) SEQUENCE DESCRIPTION: SEQ ID NO: 317:

Met Pro Leu Ile Asn Leu Leu Leu Leu Tyr Tyr Val Pro Asn Gly Gly
1               5                   10                  15

Lys Gln Asp Lys Lys
            20

(2) INFORMATION FOR SEQ ID NO: 318:

(i) SEQUENCE CHARACTERISTICS:
(A) LENGTH: 39 amino acids
(B) TYPE: amino acid
(D) TOPOLOGY: linear
(xi) SEQUENCE DESCRIPTION: SEQ ID NO: 318:

(2) INFORMATION FOR SEQ ID NO: 319:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 319:

Met Leu Trp Trp Ser Arg Asp Tyr Thr Met Val Phe Leu Leu Phe Thr
1               5                   10                  15

Met Val Phe Thr Gly Asp Leu Val Ile Arg Gly Arg Thr Glu Leu Ser
            20                  25                  30

Leu

(2) INFORMATION FOR SEQ ID NO: 320:

(i) SEQUENCE CHARACTERISTICS:
(A) LENGTH: 88 amino acids
(B) TYPE: amino acid
(D) TOPOLOGY: linear
(xi) SEQUENCE DESCRIPTION: SEQ ID NO: 320:

Met Val Cys Ser Ser Leu Cys Asp Ile Gly Gly Ile Ile Thr Pro Phe

1               5                   10                  15

Ile Val Phe Arg Leu Arg Glu Val Trp Gln Ala Leu Pro Leu Ile Leu
            20                  25                  30

Phe Ala Val Leu Gly Leu Leu Ala Ala Gly Val Thr Leu Leu Leu Pro
        35                  40                  45

Glu Thr Lys Gly Val Ala Leu Pro Glu Thr Met Lys Asp Ala Glu Asn
    50                  55                  60

Leu Gly Arg Lys Ala Lys Pro Lys Glu Asn Thr Ile Tyr Leu Lys Val
65                  70                  75                  80

Gln Thr Ser Glu Pro Ser Gly Thr
                85

(2) INFORMATION FOR SEQ ID NO: 321:

(i) SEQUENCE CHARACTERISTICS:
(A) LENGTH: 23 amino acids
(B) TYPE: amino acid
(D) TOPOLOGY: linear
(xi) SEQUENCE DESCRIPTION: SEQ ID NO: 321:

Met Gln Pro Gly Ala Gly Val Leu Val Leu Gly Leu Leu Leu Pro Pro
1               5                   10                  15

Pro Gln Ser Pro Ser Leu Ser
            20

(2) INFORMATION FOR SEQ ID NO: 322:

(i) SEQUENCE CHARACTERISTICS:
(A) LENGTH: 27 amino acids
(B) TYPE: amino acid
(D) TOPOLOGY: linear
(xi) SEQUENCE DESCRIPTION: SEQ ID NO: 322:

Met Thr Phe Thr Leu Gly Asp Ser Gln Val Leu Leu Ile Asn Leu Phe
1               5                   10                  15

Pro Ser Met Pro Ser Gly Ser Cys Ala Arg Pro
            20                  25

(2) INFORMATION FOR SEQ ID NO: 323:

(i) SEQUENCE CHARACTERISTICS:
(A) LENGTH: 64 amino acids
(B) TYPE: amino acid
(D) TOPOLOGY: linear
(xi) SEQUENCE DESCRIPTION: SEQ ID NO: 323:

Met Cys Leu Glu Cys Trp Ala Glu Asn Leu Gly Pro His His Thr Ser

1               5                   10                  15

Ser Leu Leu Asn Pro Arg His Leu Pro Ser Ile Pro Ala Met Phe Pro
            20                  25                  30

Val Ser Ser Gly Cys Phe Gln Glu Gln Gln Glu Met Asn Lys Ser Leu
        35                  40                  45

Val Ser Cys Leu Phe Val Leu His Phe Val Leu His Cys Ile Phe Xaa
    50                  55                  60

(2) INFORMATION FOR SEQ ID NO: 324:

(i) SEQUENCE CHARACTERISTICS:
(A) LENGTH: 196 amino acids
(B) TYPE: amino acid
(D) TOPOLOGY: linear
(xi) SEQUENCE DESCRIPTION: SEQ ID NO: 324:

Met Leu Ser Thr Ser Glu Tyr Ser Gln Ser Pro Lys Met Glu Ser Leu
1               5                   10                  15

Ser Ser His Arg Ile Asp Glu Asp Gly Glu Asn Thr Gln Ile Glu Asp
            20                  25                  30

Thr Glu Pro Met Ser Pro Val Leu Asn Ser Lys Phe Val Pro Ala Glu
        35                  40                  45

Asn Asp Ser Ile Leu Met Asn Pro Ala Gln Asp Gly Glu Val Gln Leu
    50                  55                  60

Ser Gln Asn Asp Asp Lys Thr Lys Gly Asp Asp Thr Asp Thr Arg Asp
65                  70                  75                  80

Asp Ile Ser Ile Leu Ala Thr Gly Cys Lys Gly Arg Glu Glu Thr Val
                85                  90                  95

Ala Glu Glu Val Cys Ile Asp Leu Thr Cys Asp Ser Gly Ser Gln Ala
            100                 105                 110

Val Pro Ser Pro Ala Thr Arg Ser Glu Ala Leu Ser Ser Val Leu Asp
        115                 120                 125

Gln Glu Glu Ala Met Glu Ile Lys Glu His His Pro Glu Glu Gly Ser
    130                 135                 140

Ser Gly Ser Glu Val Glu Glu Ile Pro Glu Thr Pro Cys Glu Ser Gln
145                 150                 155                 160

Gly Glu Glu Leu Lys Glu Glu Asn Met Glu Ser Val Pro Leu His Leu
                165                 170                 175

Ser Leu Thr Glu Thr Gln Ser Gln Gly Leu Cys Leu Arg Arg His Pro
            180                 185                 190

Lys Lys Lys Lys
        195

(2) INFORMATION FOR SEQ ID NO: 325:

        (i) SEQUENCE CHARACTERISTICS:
                (A) LENGTH: 252 amino acids
                (B) TYPE: amino acid
                (D) TOPOLOGY: linear
        (xi) SEQUENCE DESCRIPTION: SEQ ID NO: 325:

Met Gly Gly Asp Leu Val Leu Gly Leu Gly Ala Leu Arg Arg Arg Lys
  1               5                  10                  15

Arg Leu Leu Glu Gln Glu Lys Ser Leu Ala Gly Trp Ala Leu Val Leu
             20                  25                  30

Ala Xaa Xaa Gly Ile Gly Leu Met Val Leu His Ala Glu Met Leu Trp
         35                  40                  45

Phe Gly Gly Cys Ser Ala Val Asn Ala Thr Gly His Leu Ser Asp Thr
     50                  55                  60

Leu Trp Leu Ile Pro Ile Thr Phe Leu Thr Ile Gly Tyr Gly Asp Val
 65                  70                  75                  80

Val Pro Gly Thr Met Trp Gly Lys Ile Val Cys Leu Cys Thr Gly Val
                 85                  90                  95

Met Gly Val Cys Cys Thr Ala Leu Leu Val Ala Val Val Ala Arg Lys
            100                 105                 110

Leu Glu Phe Asn Lys Ala Glu Lys His Val His Asn Phe Met Met Asp
        115                 120                 125

Ile Gln Tyr Thr Lys Glu Met Lys Glu Ser Ala Ala Arg Val Leu Gln
    130                 135                 140

Glu Ala Trp Met Phe Tyr Lys His Thr Arg Arg Lys Glu Ser His Ala
145                 150                 155                 160

Ala Arg Xaa His Gln Arg Xaa Leu Leu Ala Ala Ile Asn Ala Phe Arg
                165                 170                 175

Gln Val Arg Leu Lys His Arg Lys Leu Arg Glu Gln Val Asn Ser Met
            180                 185                 190

Val Asp Ile Ser Lys Met His Met Ile Leu Tyr Asp Leu Gln Gln Asn
        195                 200                 205

Leu Ser Ser Ser His Arg Ala Leu Glu Lys Gln Ile Asp Thr Leu Ala
    210                 215                 220

Gly Lys Leu Asp Ala Leu Thr Glu Leu Leu Ser Thr Ala Leu Gly Pro
225                 230                 235                 240

Arg Gln Leu Pro Glu Pro Ser Gln Gln Ser Lys Xaa
                245                 250

(2) INFORMATION FOR SEQ ID NO: 326:

(i) SEQUENCE CHARACTERISTICS:
(A) LENGTH: 68 amino acids
(B) TYPE: amino acid
(D) TOPOLOGY: linear
(xi) SEQUENCE DESCRIPTION: SEQ ID NO: 326:

Met Trp Arg Cys Arg Gly Lys Leu Ser Phe Pro Leu Phe Ala Val Val
1               5                   10                  15

Ile Val Ser Cys Arg Lys Asp Gly Pro Asp Ala Ala Ala Ala Pro Ala
            20                  25                  30

Val Ile Lys Asn Asn Ser His Tyr Gln Thr Ser Lys Ala Leu Glu Leu
        35                  40                  45

Glu Lys Thr Thr Glu Asn Lys Glu Ser Asn Pro Phe Ile Leu Gln Val
    50                  55                  60

Asn Lys Leu Xaa
65

(2) INFORMATION FOR SEQ ID NO: 327:

(i) SEQUENCE CHARACTERISTICS:
(A) LENGTH: 84 amino acids
(B) TYPE: amino acid
(D) TOPOLOGY: linear
(xi) SEQUENCE DESCRIPTION: SEQ ID NO: 327:

Met Gly Glu Gly Lys Asn Gly Phe Gly Gly Phe Val His Thr Ala Asp
1               5                   10                  15

Ala Cys Trp Glu Gly Val His Ser Glu Pro Val Cys Arg Thr Val His
            20                  25                  30

Thr Val His Thr Cys His His Gln Ala Phe Leu Val Leu Ile Gly Trp
        35                  40                  45

Ser Lys Ser Gly Lys Glu Arg Lys Glu Ala Phe Leu Thr Ala Ile Ile
    50                  55                  60

Leu Asn Ser Arg Ser Ile His Ile Ser Cys Ser Trp Pro Pro Ser Pro
65                  70                  75                  80

Val Pro Gln Xaa

(2) INFORMATION FOR SEQ ID NO: 328:

(i) SEQUENCE CHARACTERISTICS:
(A) LENGTH: 36 amino acids

(B) TYPE: amino acid
(D) TOPOLOGY: linear
(xi) SEQUENCE DESCRIPTION: SEQ ID NO: 328:

Met Leu Leu Ile Asn Leu Leu Trp Leu Val Thr Met Ile Lys Ser Val
1               5                   10                  15

Ile Asn Asn Asn Ile Ile Leu Phe Leu Lys Lys Lys Ser Leu Phe Phe
            20                  25                  30

Ile Asp Ser Val
        35

(2) INFORMATION FOR SEQ ID NO: 329:

(i) SEQUENCE CHARACTERISTICS:
(A) LENGTH: 63 amino acids
(B) TYPE: amino acid
(D) TOPOLOGY: linear
(xi) SEQUENCE DESCRIPTION: SEQ ID NO: 329:

Met Thr Phe Pro Phe Glu Lys Lys Ile Val Ala Phe Ser Ala Phe Tyr
1               5                   10                  15

Leu Ile Pro Gly Glu Ser Arg Leu Ala Pro Thr Phe Asn Pro Ser Ala
            20                  25                  30

Asp Met Thr Val Ile Leu Arg Gly Arg Ala Gln His Lys Thr Ala Met
        35                  40                  45

Leu Glu Ser Tyr Asn Trp Lys Val Ser Cys Gln Leu Arg Glu Xaa
    50                  55                  60

(2) INFORMATION FOR SEQ ID NO: 330:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 330:

Met His Ser Lys Gly Ser Ser Leu Leu Leu Phe Leu Pro Gln Leu Ile
1               5                   10                  15

Leu Ile Leu Pro Val Cys Ala His Leu His Glu Glu Leu Asn Cys Cys
            20                  25                  30

Phe His Arg
        35

(2) INFORMATION FOR SEQ ID NO: 331:

(i) SEQUENCE CHARACTERISTICS:
(A) LENGTH: 23 amino acids

(B) TYPE: amino acid
(D) TOPOLOGY: linear
(xi) SEQUENCE DESCRIPTION: SEQ ID NO: 331:

Met Gly Ala Leu Val Leu Leu Leu Cys Leu Leu Val Gly Val Gln Gln
1               5                   10                  15

Ser Gly Ser Val Trp Asp Ser
            20

(2) INFORMATION FOR SEQ ID NO: 332:

(i) SEQUENCE CHARACTERISTICS:
(A) LENGTH: 40 amino acids
(B) TYPE: amino acid
(D) TOPOLOGY: linear
(xi) SEQUENCE DESCRIPTION: SEQ ID NO: 332:

(2) INFORMATION FOR SEQ ID NO: 333:

(i) SEQUENCE CHARACTERISTICS:
(A) LENGTH: 111 amino acids
(B) TYPE: amino acid
(D) TOPOLOGY: linear
(xi) SEQUENCE DESCRIPTION: SEQ ID NO: 333:

Met Gln Ser Leu Val Gln Trp Gly Leu Asp Ser Tyr Asp Tyr Leu Gln
1               5                   10                  15

Asn Ala Pro Pro Gly Phe Phe Pro Arg Leu Gly Val Ile Gly Phe Ala
            20                  25                  30

Gly Leu Ile Gly Leu Leu Leu Ala Arg Gly Ser Lys Ile Lys Lys Leu
        35                  40                  45

Val Tyr Pro Pro Gly Phe Met Gly Leu Ala Ala Ser Leu Tyr Tyr Pro
    50                  55                  60

Gln Gln Ala Ile Val Phe Ala Gln Val Ser Gly Glu Arg Leu Tyr Asp
65                  70                  75                  80

Trp Gly Leu Arg Gly Tyr Ile Val Ile Glu Asp Leu Trp Lys Glu Asn
                85                  90                  95

Phe Gln Lys Pro Gly Asn Val Lys Asn Ser Pro Gly Thr Lys Xaa
            100                 105                 110

(2) INFORMATION FOR SEQ ID NO: 334:

(i) SEQUENCE CHARACTERISTICS:
(A) LENGTH: 106 amino acids
(B) TYPE: amino acid
(D) TOPOLOGY: linear
(xi) SEQUENCE DESCRIPTION: SEQ ID NO: 334:

Met Ala Pro Ser Leu Leu Leu Leu Ala Pro Leu Cys Ser Leu Glu Ala
1               5                   10                  15

Val Leu Ser Ser Pro Leu Glu Lys Gln Cys Gln Leu Pro Gly Ile Phe
            20                  25                  30

Cys Gln Leu Gln Leu Pro Cys Pro Leu Leu Leu Ser Ala Gln Leu Leu
        35                  40                  45

Lys Gly Ile Val Xaa Pro Arg Cys Pro Ala Ser Leu Pro Gln Pro Pro
    50                  55                  60

His Pro Ala Pro Ser Trp His Leu Pro Leu His Cys Thr Glu Arg Xaa
65                  70                  75                  80

Pro His His Leu Pro Leu Gln Gly Gly Ser Ser Asn Met Glu Glu Xaa
                85                  90                  95

Asn Tyr Arg Gly Tyr Xaa Asp Ala Gln Leu
            100                 105

(2) INFORMATION FOR SEQ ID NO: 335:

(i) SEQUENCE CHARACTERISTICS:
(A) LENGTH: 50 amino acids
(B) TYPE: amino acid
(D) TOPOLOGY: linear
(xi) SEQUENCE DESCRIPTION: SEQ ID NO: 335:

Met Thr Thr Cys Leu Phe Gly Leu Leu Ser Cys Glu Met Ser Ala Gln
1               5                   10                  15

Val Ser Gln Lys Ser Cys Val Tyr Asp Glu Ser Glu Cys Phe Ser Ser
            20                  25                  30

Val Gly Gln Leu Leu Ala Leu Leu Ile Leu Val Tyr Val Leu Pro Ser
        35                  40                  45

Ile Xaa
    50

(2) INFORMATION FOR SEQ ID NO: 336:

(i) SEQUENCE CHARACTERISTICS:
(A) LENGTH: 48 amino acids
(B) TYPE: amino acid

(D) TOPOLOGY: linear
(xi) SEQUENCE DESCRIPTION: SEQ ID NO: 336:

Met Leu Trp Lys Cys Ser Gln Asn Ile Ala Arg Cys Leu Leu Leu Leu
1               5                   10                  15

Leu Ala Leu Val Glu Ile Lys Leu Glu Asp Leu Gln Ser Gln Leu His
            20                  25                  30

Pro Thr Trp Lys Ser Ile Pro Gly Pro Ser Pro Arg Asn Gln His Arg
        35                  40                  45

(2) INFORMATION FOR SEQ ID NO: 337:

(i) SEQUENCE CHARACTERISTICS:
(A) LENGTH: 41 amino acids
(B) TYPE: amino acid
(D) TOPOLOGY: linear
(xi) SEQUENCE DESCRIPTION: SEQ ID NO: 337:

Met Leu Ile Pro Leu Gln Cys Leu Phe Ser Ser Asp Arg Met Leu Thr
1               5                   10                  15

Phe Leu Thr Pro Trp Gln Lys Gly Glu Lys Cys Val Leu Gly Trp Val
            20                  25                  30

Thr Lys Phe Leu Ser Glu Ile Ser Xaa
        35                  40

(2) INFORMATION FOR SEQ ID NO: 338:

(i) SEQUENCE CHARACTERISTICS:
(A) LENGTH: 76 amino acids
(B) TYPE: amino acid
(D) TOPOLOGY: linear
(xi) SEQUENCE DESCRIPTION: SEQ ID NO: 338:

(2) INFORMATION FOR SEQ ID NO: 339:

(i) SEQUENCE CHARACTERISTICS:
(A) LENGTH: 31 amino acids
(B) TYPE: amino acid
(D) TOPOLOGY: linear
(xi) SEQUENCE DESCRIPTION: SEQ ID NO: 339:

Lys Val Tyr Ile Phe Leu Ile Phe Met Val Leu Ile Leu Pro Ser Leu
1               5                   10                  15

Gly Leu Thr Arg Tyr Met Pro Pro Xaa Ser Xaa Leu Asn Ser Glu
            20                  25                  30

(2) INFORMATION FOR SEQ ID NO: 340:

(i) SEQUENCE CHARACTERISTICS:
(A) LENGTH: 42 amino acids
(B) TYPE: amino acid
(D) TOPOLOGY: linear
(xi) SEQUENCE DESCRIPTION: SEQ ID NO: 340:

Met Ala Lys Ile Ser Pro Phe Glu Val Val Lys Arg Thr Ser Val Pro
1               5                   10                  15

Val Leu Val Gly Leu Val Ile Val Ile Val Ala Thr Glu Leu Met Val
            20                  25                  30

Pro Gly Thr Ala Ala Ala Val Thr Gly Lys
        35                  40

(2) INFORMATION FOR SEQ ID NO: 341:

(i) SEQUENCE CHARACTERISTICS:
(A) LENGTH: 26 amino acids
(B) TYPE: amino acid
(D) TOPOLOGY: linear
(xi) SEQUENCE DESCRIPTION: SEQ ID NO: 341:

Met Arg Leu Phe Phe Ile Gly Phe Leu Leu Leu Phe Ser Phe Gly Leu
1               5                   10                  15

Leu Arg Gln Pro Ser Leu Ser Ala Glu His
            20                  25

(2) INFORMATION FOR SEQ ID NO: 342:

(i) SEQUENCE CHARACTERISTICS:
(A) LENGTH: 26 amino acids
(B) TYPE: amino acid
(D) TOPOLOGY: linear
(xi) SEQUENCE DESCRIPTION: SEQ ID NO: 342:

Met Val Phe Ser Val Ser Ser Ala Leu Ala Leu Leu Leu Met Leu Leu
1               5                   10                  15

Arg Ser Ser Asp Leu Ala Lys Lys Thr Glu
            20                  25

(2) INFORMATION FOR SEQ ID NO: 343:

(i) SEQUENCE CHARACTERISTICS:
(A) LENGTH: 157 amino acids
(B) TYPE: amino acid
(D) TOPOLOGY: linear
(xi) SEQUENCE DESCRIPTION: SEQ ID NO: 343:

Met Ser Leu Glu Phe Tyr Gln Lys Lys Lys Ser Arg Trp Pro Phe Ser
1               5                   10                  15

Asp Glu Cys Ile Pro Trp Glu Val Trp Thr Val Lys Val His Val Val
            20                  25                  30

Ala Leu Ala Thr Glu Gln Glu Arg Gln Ile Cys Arg Glu Lys Val Gly
        35                  40                  45

Glu Lys Leu Cys Glu Lys Ile Ile Asn Ile Val Glu Val Met Asn Arg
    50                  55                  60

His Glu Tyr Leu Pro Lys Met Pro Thr Gln Ser Glu Val Asp Asn Val
65                  70                  75                  80

Phe Asp Thr Gly Leu Arg Asp Val Gln Pro Tyr Leu Tyr Lys Ile Ser
                85                  90                  95

Phe Gln Ile Thr Asp Ala Leu Gly Thr Ser Val Thr Thr Thr Met Arg
            100                 105                 110

Arg Leu Ile Lys Asp Thr Leu Pro Ser Glu Arg Arg Trp Ile Ser Gly
        115                 120                 125

Ser Ser Leu Met Ala Pro Arg Pro Trp Leu Leu Gly Ile Ala Leu Leu
    130                 135                 140

Gly Leu Trp Ala Leu Glu Pro Ala Leu Gly His Trp Xaa
145                 150                 155

(2) INFORMATION FOR SEQ ID NO: 344:

(i) SEQUENCE CHARACTERISTICS:
(A) LENGTH: 520 amino acids
(B) TYPE: amino acid
(D) TOPOLOGY: linear
(xi) SEQUENCE DESCRIPTION: SEQ ID NO: 344:

Met Phe Leu Leu Pro Leu Pro Ala Ala Gly Arg Val Val Val Arg Arg
1               5                   10                  15

Leu Ala Val Arg Arg Phe Gly Ser Arg Ser Leu Ser Thr Ala Asp Met

20                  25                  30

Thr Lys Gly Leu Val Leu Gly Ile Tyr Ser Lys Glu Lys Glu Asp Asp
        35                  40                  45

Val Pro Gln Phe Thr Ser Ala Gly Glu Asn Phe Asp Lys Leu Leu Ala
    50                  55                  60

Gly Lys Leu Arg Glu Thr Leu Asn Ile Ser Gly Pro Pro Leu Lys Ala
65                  70                  75                  80

Gly Lys Thr Arg Thr Phe Tyr Gly Leu His Gln Asp Phe Pro Ser Val
                85                  90                  95

Val Leu Val Gly Leu Gly Lys Lys Ala Ala Gly Ile Asp Glu Gln Glu
            100                 105                 110

Asn Trp His Glu Gly Lys Glu Asn Ile Arg Ala Ala Val Ala Ala Gly
        115                 120                 125

Cys Arg Gln Ile Gln Asp Leu Glu Leu Ser Ser Val Glu Val Asp Pro
    130                 135                 140

Cys Gly Asp Ala Gln Ala Ala Ala Glu Gly Ala Val Leu Gly Leu Tyr
145                 150                 155                 160

Glu Tyr Asp Asp Leu Lys Gln Lys Lys Lys Met Ala Val Ser Ala Lys
                165                 170                 175

Leu Tyr Gly Ser Gly Asp Gln Glu Ala Trp Gln Lys Gly Val Leu Phe
            180                 185                 190

Ala Ser Gly Gln Asn Leu Ala Arg Gln Leu Met Glu Thr Pro Ala Asn
        195                 200                 205

Glu Met Thr Pro Thr Arg Phe Ala Glu Ile Ile Glu Lys Asn Leu Lys
    210                 215                 220

Ser Ala Ser Ser Lys Thr Glu Val His Ile Arg Pro Lys Ser Trp Ile
225                 230                 235                 240

Glu Glu Gln Ala Met Gly Ser Phe Leu Ser Val Ala Lys Gly Ser Asp
                245                 250                 255

Glu Pro Pro Val Phe Leu Glu Ile His Tyr Lys Gly Ser Pro Asn Ala
            260                 265                 270

Asn Glu Pro Pro Leu Val Phe Val Gly Lys Gly Ile Thr Phe Asp Ser
        275                 280                 285

Gly Gly Ile Ser Ile Lys Ala Ser Ala Asn Met Asp Leu Met Arg Ala
    290                 295                 300

Asp Met Gly Gly Ala Ala Thr Ile Cys Ser Ala Ile Val Ser Ala Ala
305                 310                 315                 320

Lys Leu Asn Leu Pro Ile Asn Ile Ile Gly Leu Ala Pro Leu Cys Glu
                325                 330                 335

Asn Met Pro Ser Gly Lys Ala Asn Lys Pro Gly Asp Val Val Arg Ala

            340                 345                 350

Lys Asn Gly Lys Thr Ile Gln Val Asp Asn Thr Asp Ala Glu Gly Arg
        355                 360                 365

Leu Ile Leu Ala Asp Ala Leu Cys Tyr Ala His Thr Phe Asn Pro Lys
    370                 375                 380

Xaa Ile Leu Asn Ala Ala Thr Leu Thr Gly Ala Met Asp Val Ala Leu
385                 390                 395                 400

Gly Ser Gly Ala Thr Gly Val Phe Thr Asn Ser Ser Trp Leu Trp Asn
                405                 410                 415

Lys Leu Phe Glu Ala Ser Ile Glu Thr Gly Asp Arg Val Trp Arg Met
            420                 425                 430

Pro Leu Phe Glu His Tyr Thr Arg Gln Val Val Asp Cys Gln Leu Ala
        435                 440                 445

Asp Val Asn Asn Ile Gly Lys Tyr Arg Ser Ala Gly Ala Cys Thr Ala
    450                 455                 460

Ala Ala Phe Leu Lys Glu Phe Val Thr His Pro Lys Trp Ala His Leu
465                 470                 475                 480

Asp Ile Ala Gly Val Met Thr Asn Lys Asp Glu Val Pro Tyr Leu Arg
                485                 490                 495

Lys Gly Met Thr Gly Arg Pro Thr Arg Thr Leu Ile Glu Phe Leu Leu
            500                 505                 510

Arg Phe Ser Gln Asp Asn Ala Xaa
        515                 520

(2) INFORMATION FOR SEQ ID NO: 345:

(i) SEQUENCE CHARACTERISTICS:
(A) LENGTH: 39 amino acids
(B) TYPE: amino acid
(D) TOPOLOGY: linear
(xi) SEQUENCE DESCRIPTION: SEQ ID NO: 345:

Thr Ile Leu Phe Leu Phe Leu Gln Leu Ser Ala Leu Arg Leu Ile Val
1               5                   10                  15

Gly Lys Asp Ser Ile Asp Ile Asp Ile Ser Ser Arg Arg Arg Glu Asp
            20                  25                  30

Gln Ser Leu Arg Leu Asn Ala
        35

(2) INFORMATION FOR SEQ ID NO: 346:

(i) SEQUENCE CHARACTERISTICS:
(A) LENGTH: 234 amino acids

(B) TYPE: amino acid
(D) TOPOLOGY: linear
(xi) SEQUENCE DESCRIPTION: SEQ ID NO: 346:

Met Thr Ser Glu Leu Asp Ile Phe Val Gly Asn Thr Thr Leu Ile Asp
1               5                   10                  15

Glu Asp Val Tyr Arg Leu Trp Leu Asp Gly Tyr Ser Val Thr Asp Ala
            20                  25                  30

Val Ala Leu Arg Val Arg Ser Gly Ile Leu Glu Gln Thr Gly Ala Thr
        35                  40                  45

Ala Ala Val Leu Gln Ser Asp Thr Met Asp His Tyr Arg Thr Phe His
    50                  55                  60

Met Leu Glu Arg Leu Leu His Ala Pro Pro Lys Leu Leu His Gln Leu
65                  70                  75                  80

Ile Phe Gln Ile Pro Pro Ser Arg Gln Ala Leu Leu Ile Glu Arg Tyr
                85                  90                  95

Tyr Ala Phe Asp Glu Ala Phe Val Arg Glu Val Leu Gly Lys Lys Leu
            100                 105                 110

Ser Lys Gly Thr Lys Lys Asp Leu Asp Asp Ile Ser Thr Lys Thr Gly
        115                 120                 125

Ile Thr Leu Lys Ser Cys Arg Arg Gln Phe Asp Asn Phe Lys Arg Val
    130                 135                 140

Phe Lys Val Val Glu Glu Met Arg Gly Ser Leu Val Asp Asn Ile Gln
145                 150                 155                 160

Gln His Phe Leu Leu Ser Asp Arg Leu Ala Arg Asp Tyr Ala Ala Ile
                165                 170                 175

Val Phe Phe Ala Asn Asn Arg Phe Glu Thr Gly Lys Lys Lys Leu Gln
            180                 185                 190

Tyr Leu Ser Phe Gly Asp Phe Ala Phe Cys Ala Glu Leu Met Ile Gln
        195                 200                 205

Asn Trp Thr Leu Gly Pro Val Asp Ser Gln Met Asp Asp Met Asp Met
    210                 215                 220

Asp Leu Asp Arg Asn Phe Ser Arg Thr Xaa
225                 230

(2) INFORMATION FOR SEQ ID NO: 347:

(i) SEQUENCE CHARACTERISTICS:
(A) LENGTH: 169 amino acids
(B) TYPE: amino acid
(D) TOPOLOGY: linear
(xi) SEQUENCE DESCRIPTION: SEQ ID NO: 347:

Met Ala Ala Ala Val Ala Gly Met Leu Arg Gly Gly Leu Leu Pro Gln

1               5                   10                  15

Ala Gly Arg Leu Pro Thr Leu Gln Thr Val Arg Tyr Gly Ser Lys Ala
            20                  25                  30

Val Thr Arg His Arg Arg Val Met His Phe Gln Arg Gln Lys Leu Met
        35                  40                  45

Ala Val Thr Glu Tyr Ile Pro Pro Lys Pro Ala Ile His Pro Ser Cys
    50                  55                  60

Leu Pro Ser Pro Pro Ser Pro Pro Gln Glu Glu Ile Gly Leu Ile Arg
65                  70                  75                  80

Leu Leu Arg Arg Glu Ile Ala Ala Val Phe Gln Asp Asn Arg Met Ile
                85                  90                  95

Ala Val Cys Gln Asn Val Ala Leu Ser Ala Glu Asp Lys Leu Leu Ile
            100                 105                 110

Ala Thr Pro Ala Ala Glu Thr Gln Asp Pro Asp Glu Gly Leu Pro Gln
        115                 120                 125

Pro Gly Pro Glu Ser Pro Ser Trp Arg Ile Pro Ser Thr Lys Ile Cys
    130                 135                 140

Cys Pro Phe Leu Trp Gly Thr Thr Cys Cys Trp Ser Val Lys Ser Pro
145                 150                 155                 160

Arg Ser Arg Arg Trp Tyr Gly Ser Xaa
                165

(2) INFORMATION FOR SEQ ID NO: 348:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 348:

Met Lys Arg Ser Phe Leu Leu Pro Leu Leu Leu Val Gly Phe Leu Asp
1               5                   10                  15

Thr Ala His Leu Ile Leu Leu Glu Thr Leu Ser Val Cys Leu Trp Leu
            20                  25                  30

Pro Ser Leu Ile Asp Ser Arg Cys Val Met Ser
        35                  40

(2) INFORMATION FOR SEQ ID NO: 349:

(i) SEQUENCE CHARACTERISTICS:
(A) LENGTH: 78 amino acids
(B) TYPE: amino acid
(D) TOPOLOGY: linear
(xi) SEQUENCE DESCRIPTION: SEQ ID NO: 349:

(2) INFORMATION FOR SEQ ID NO: 350:

(i) SEQUENCE CHARACTERISTICS:
(A) LENGTH: 24 amino acids
(B) TYPE: amino acid
(D) TOPOLOGY: linear
(xi) SEQUENCE DESCRIPTION: SEQ ID NO: 350:

Met Ser Glu Ile Leu Ser Leu Leu Phe Cys Leu Leu Gly Pro Ala Leu
1               5                   10                  15

Asp Glu Arg Arg Glu Glu Lys Asp
            20

(2) INFORMATION FOR SEQ ID NO: 351:

(i) SEQUENCE CHARACTERISTICS:
(A) LENGTH: 274 amino acids
(B) TYPE: amino acid
(D) TOPOLOGY: linear
(xi) SEQUENCE DESCRIPTION: SEQ ID NO: 351:

Met Ser Ser Ala Gly Thr Ala Thr Pro Leu Glu Met Asp His Lys Leu
1               5                   10                  15

Thr Ser Gln Pro Gly Arg Pro Ser Phe Tyr Cys Asn Ser Arg His Ser
            20                  25                  30

Ile Val Gly Ser Ser His Gln Leu Gly Phe Trp Phe Ser His Leu Glu
        35                  40                  45

Ser Ser Gly Leu Lys Val Phe Gln Val Ser Leu Pro Cys Glu Cys Val
    50                  55                  60

Asn Leu Pro Thr Arg Ile Ala Ser Val Val Leu Ser Leu Met Ser Leu
65                  70                  75                  80

Leu Val Val Gly Gln Ala Pro Ala Trp Glu Gly Ser Leu Leu Arg Gly
                85                  90                  95

Arg Pro Ala Gly Gly Ala His Leu Cys Ala Met Xaa Val Ile Glu Gly
            100                 105                 110

Leu Val Val Asp Val Gly Glu Arg Ile Leu His Gly Gln Arg Glu Val
        115                 120                 125

Gly Gln Val Ser Gln Val Leu Pro Ala Leu Ser Leu Gly Leu Val Phe
    130                 135                 140

Leu Cys Gln Gly Thr Val Glu Lys Val Ser Gly Ala Ala His Cys Ser
145                 150                 155                 160

Ser Leu Leu Cys Cys Leu Pro Trp Gln Cys Ser Gly Gly Gly Phe Pro
                165                 170                 175

Thr Xaa Arg Cys Ser Arg Pro Tyr Phe Ser Ser His Lys Gly Val Ala
            180                 185                 190

Ala Thr Leu Ala Leu Thr Cys His Cys Asp Lys Val His Val Ala Gly
        195                 200                 205

Leu Gly Lys Asp Trp Ala Ile Glu Gln Arg Arg Arg Thr Cys Glu Ser
    210                 215                 220

Asp Xaa Glu Xaa Xaa Pro Phe Thr Leu Ala Gly Leu Val Leu Val Leu
225                 230                 235                 240

Arg Phe Cys Gln Val Val Leu Val Trp Ile Pro Gln Leu Gly Asp Lys
                245                 250                 255

His Trp Arg Gly Met Thr Arg Leu Gly Arg Val Ser Leu Thr Ser Ser
            260                 265                 270

Ile Xaa

(2) INFORMATION FOR SEQ ID NO: 352:

    (i) SEQUENCE CHARACTERISTICS:
        (A) LENGTH: 47 amino acids
        (B) TYPE: amino acid
        (D) TOPOLOGY: linear
    (xi) SEQUENCE DESCRIPTION: SEQ ID NO: 352:

Met Ile Phe Thr Ser Val Thr Lys Gly Ile Leu Leu Ile Ala Leu Trp
  1               5                  10                  15

Val Pro Leu Phe His Phe Met Leu Ile Asp Ser Ile Leu Gly Pro Ser
            20                  25                  30

Arg Leu Leu Thr Asp Gly Val Pro Phe Asn Pro Trp His Val Xaa
        35                  40                  45

(2) INFORMATION FOR SEQ ID NO: 353:

    (i) SEQUENCE CHARACTERISTICS:

(A) LENGTH: 3 amino acids
(B) TYPE: amino acid
(D) TOPOLOGY: linear
(xi) SEQUENCE DESCRIPTION: SEQ ID NO: 353:

Met Lys Thr
1

(2) INFORMATION FOR SEQ ID NO: 354:

(i) SEQUENCE CHARACTERISTICS:
(A) LENGTH: 52 amino acids
(B) TYPE: amino acid
(D) TOPOLOGY: linear
(xi) SEQUENCE DESCRIPTION: SEQ ID NO: 354:

Met Ser Ile Ser Gly Thr Asp Gly Leu Ile Leu Leu Leu Val Gly Leu
1               5                   10                  15

Glu Ala Xaa Val Arg Ser Ser Lys Lys Trp Ile Pro Lys Ala Leu Xaa
            20                  25                  30

Val Thr Gln Ala Lys Trp Asn Ser Trp Pro Ser Arg Arg Asn Ala Gly
        35                  40                  45

Phe Ala Leu His
    50

(2) INFORMATION FOR SEQ ID NO: 355:

(i) SEQUENCE CHARACTERISTICS:
(A) LENGTH: 132 amino acids
(B) TYPE: amino acid
(D) TOPOLOGY: linear
(xi) SEQUENCE DESCRIPTION: SEQ ID NO: 355:

Met Glu His Cys Leu Tyr His Ser Val His Gly Ile Asn Pro Tyr Ile
1               5                   10                  15

His Lys Asn Thr His Pro Ser Ile Asn Ile Tyr Met Val Trp Asp Glu
            20                  25                  30

Gln Val Asn Ser Phe Glu Arg Glu Phe Val Pro Phe Phe Phe Leu Ile
        35                  40                  45

Ile Leu Leu Asn Cys Cys Gln Leu Ser Asn Lys Gln Thr Glu Lys Leu
    50                  55                  60

Phe Gly Lys Thr Leu His Thr Pro Phe Leu Ser Ser Ala Leu Lys Tyr
65                  70                  75                  80

Arg Leu Asn Thr His Ile Leu Pro Val Phe Ser Tyr Ser Asp Ser Ile
                85                  90                  95

Leu Thr Cys His Leu Ile Leu Ala Ser Tyr Phe Ser His Val Tyr Leu
            100                 105                 110

Pro Val Thr Cys Ile Cys Tyr Leu Asn Arg Lys Lys Asn Ile Gln Lys
        115                 120                 125

Lys Lys Asn Xaa
    130

(2) INFORMATION FOR SEQ ID NO: 356:

(i) SEQUENCE CHARACTERISTICS:
(A) LENGTH: 204 amino acids
(B) TYPE: amino acid
(D) TOPOLOGY: linear
(xi) SEQUENCE DESCRIPTION: SEQ ID NO: 356:

Met Gly Ser Arg Asp His Leu Phe Lys Val Leu Val Val Gly Asp Ala
1               5                   10                  15

Ala Val Gly Lys Thr Ser Leu Val Gln Asp Tyr Ser Gln Asp Ser Phe
            20                  25                  30

Ser Lys His Tyr Lys Ser Thr Val Gly Val Asp Phe Ala Leu Lys Val
        35                  40                  45

Leu Gln Trp Ser Asp Tyr Glu Ile Val Arg Leu Gln Leu Trp Asp Ile
    50                  55                  60

Ala Gly Gln Glu Arg Phe Thr Ser Met Thr Arg Leu Tyr Tyr Arg Asp
65                  70                  75                  80

Ala Ser Ala Cys Val Ile Met Phe Asp Val Thr Asn Ala Thr Thr Phe
                85                  90                  95

Ser Asn Ser Gln Arg Trp Lys Gln Asp Leu Asp Ser Lys Leu Thr Leu
            100                 105                 110

Pro Asn Gly Glu Pro Val Pro Cys Leu Leu Leu Ala Asn Lys Cys Asp
        115                 120                 125

Leu Ser Pro Trp Ala Val Ser Arg Asp Gln Ile Asp Arg Phe Ser Lys
    130                 135                 140

Glu Asn Gly Phe Thr Gly Trp Thr Glu Thr Ser Val Lys Glu Asn Lys
145                 150                 155                 160

Asn Ile Asn Glu Ala Met Arg Val Leu Ile Glu Lys Met Met Arg Asn
                165                 170                 175

Ser Thr Glu Asp Ile Met Ser Leu Ser Thr Gln Gly Asp Tyr Ile Asn
            180                 185                 190

Leu Gln Thr Lys Ser Ser Ser Trp Ser Cys Cys Xaa
        195                 200

(2) INFORMATION FOR SEQ ID NO: 357:

(i) SEQUENCE CHARACTERISTICS:
(A) LENGTH: 47 amino acids
(B) TYPE: amino acid
(D) TOPOLOGY: linear
(xi) SEQUENCE DESCRIPTION: SEQ ID NO: 357:

Met Ile Ser Leu Ile Phe Gln Leu Glu Glu Glu Lys Leu Val Glu Lys
1               5                   10                  15

Phe Phe Phe Phe Leu Phe Phe Phe Leu Lys Lys Gly Ser Gln Gly Ser
            20                  25                  30

Asn Leu Lys Ile Val Pro Arg His Met Arg Val Val Leu Arg Gly
        35                  40                  45

(2) INFORMATION FOR SEQ ID NO: 358:

(i) SEQUENCE CHARACTERISTICS:
(A) LENGTH: 73 amino acids
(B) TYPE: amino acid
(D) TOPOLOGY: linear
(xi) SEQUENCE DESCRIPTION: SEQ ID NO: 358:

Met Thr Tyr Val Thr Cys Leu His Val Cys Leu Leu Val Glu Phe Leu
1               5                   10                  15

Asn Ser Gln Leu Thr Asn His Arg Lys Tyr Tyr Phe Leu Ser Tyr Gly
            20                  25                  30

Phe Trp Phe Thr Gly Leu Arg Gly Phe Ser Glu Tyr Leu Trp Pro Gln
        35                  40                  45

Gln His Thr Ser Phe His Pro Asn Arg Asn Glu Ile Asn Phe Val Ser
    50                  55                  60

Thr Asp Asn Arg Ile Trp Val Thr Xaa
65                  70

(2) INFORMATION FOR SEQ ID NO: 359:

(i) SEQUENCE CHARACTERISTICS:
(A) LENGTH: 102 amino acids
(B) TYPE: amino acid
(D) TOPOLOGY: linear
(xi) SEQUENCE DESCRIPTION: SEQ ID NO: 359:

Met Ser Asp Gln Glu Ala Lys Pro Ser Thr Glu Asp Leu Gly Asp Lys
1               5                   10                  15

Lys Glu Gly Glu Tyr Ile Lys Leu Lys Val Ile Gly Gln Asp Ser Ser
            20                  25                  30

Glu Ile His Phe Lys Val Lys Met Thr Thr His Leu Lys Lys Leu Lys
        35                  40                  45

Glu Ser Tyr Cys Gln Arg Gln Gly Val Pro Met Asn Ser Leu Arg Phe

    50                  55                  60

Leu Phe Glu Gly Gln Arg Ile Ala Asp Asn His Thr Pro Lys Glu Leu
65                  70                  75                  80

Gly Met Glu Glu Glu Asp Val Ile Glu Val Tyr Gln Glu Gln Thr Gly
                85                  90                  95

Gly His Ser Thr Val Xaa
            100

(2) INFORMATION FOR SEQ ID NO: 360:

(i) SEQUENCE CHARACTERISTICS:
(A) LENGTH: 48 amino acids
(B) TYPE: amino acid
(D) TOPOLOGY: linear
(xi) SEQUENCE DESCRIPTION: SEQ ID NO: 360:

Met Gly Phe Pro Gln Trp His Leu Gly Asn His Ala Val Glu Pro Val
1               5                   10                  15

Thr Ser Ile Leu Leu Leu Phe Leu Leu Met Met Leu Gly Val Arg Gly
            20                  25                  30

Leu Leu Leu Val Gly Leu Val Tyr Leu Val Ser His Leu Ser Gln Arg
        35                  40                  45

(2) INFORMATION FOR SEQ ID NO: 361:

(i) SEQUENCE CHARACTERISTICS:
(A) LENGTH: 179 amino acids
(B) TYPE: amino acid
(D) TOPOLOGY: linear
(xi) SEQUENCE DESCRIPTION: SEQ ID NO: 361:

Met Ser Ala Glu Val Lys Val Thr Gly Gln Asn Gln Glu Gln Phe Leu
1               5                   10                  15

Leu Leu Ala Lys Ser Ala Lys Gly Ala Ala Leu Ala Thr Leu Ile His
            20                  25                  30

Gln Val Leu Glu Ala Pro Gly Val Tyr Val Phe Gly Glu Leu Leu Asp
        35                  40                  45

Met Pro Asn Val Arg Glu Leu Ala Glu Ser Asp Phe Ala Ser Thr Phe
    50                  55                  60

Arg Leu Leu Thr Val Phe Ala Tyr Gly Thr Tyr Ala Asp Tyr Leu Ala
65                  70                  75                  80

Glu Ala Arg Asn Leu Pro Pro Leu Thr Glu Ala Gln Lys Asn Lys Leu
                85                  90                  95

Arg His Leu Ser Val Val Thr Leu Ala Ala Lys Val Lys Cys Ile Pro
            100                 105                 110

Tyr Ala Val Leu Leu Glu Ala Leu Ala Leu Arg Asn Val Arg Gln Leu
        115                 120                 125

Glu Asp Leu Val Ile Glu Ala Val Tyr Ala Asp Val Leu Arg Gly Ser
    130                 135                 140

Leu Asp Gln Arg Asn Gln Arg Leu Glu Val Asp Tyr Ser Ile Gly Arg
145                 150                 155                 160

Asp Ile Gln Arg Gln Asp Leu Ser Ala Ile Ala Arg Thr Leu Xaa Lys
                165                 170                 175

Asn His Xaa

(2) INFORMATION FOR SEQ ID NO: 362:

        (i) SEQUENCE CHARACTERISTICS:
                (A) LENGTH: 25 amino acids
                (B) TYPE: amino acid
                (D) TOPOLOGY: linear
        (xi) SEQUENCE DESCRIPTION: SEQ ID NO: 362:

Met Lys Ser Ser Ser Leu Phe Phe Phe Phe Leu Ala His Phe Ile His
  1               5                  10                  15

Ser His Asp Leu Pro Gly Leu Cys Arg
            20                  25

(2) INFORMATION FOR SEQ ID NO: 363:

        (i) SEQUENCE CHARACTERISTICS:
                (A) LENGTH: 224 amino acids
                (B) TYPE: amino acid
                (D) TOPOLOGY: linear
        (xi) SEQUENCE DESCRIPTION: SEQ ID NO: 363:

Met Lys Phe Ala Ala Ser Gly Xaa Phe Leu His His Met Ala Gly Leu
  1               5                  10                  15

Ser Ser Ser Lys Leu Ser Met Ser Lys Ala Leu Pro Leu Thr Lys Val
            20                  25                  30

Val Gln Asn Asp Ala Tyr Thr Ala Pro Ala Leu Pro Ser Ser Ile Arg
        35                  40                  45

Thr Lys Ala Leu Thr Asn Met Ser Arg Thr Leu Val Asn Lys Glu Glu
    50                  55                  60

Pro Pro Lys Glu Leu Pro Ala Ala Glu Pro Val Leu Ser Pro Leu Glu
 65                  70                  75                  80

Gly Thr Lys Met Thr Val Asn Asn Leu His Pro Arg Val Thr Glu Glu
                85                  90                  95

Asp Ile Val Glu Leu Phe Cys Val Cys Gly Ala Leu Lys Arg Ala Arg
            100                 105                 110

Leu Val His Pro Gly Val Ala Glu Val Val Phe Val Lys Lys Asp Asp
        115                 120                 125

Ala Ile Thr Ala Tyr Lys Lys Tyr Asn Asn Arg Cys Leu Asp Gly Gln
    130                 135                 140

Pro Met Lys Cys Asn Leu His Met Asn Gly Asn Val Ile Thr Ser Asp
145                 150                 155                 160

Gln Pro Ile Leu Leu Arg Leu Ser Asp Ser Pro Ser Met Lys Lys Glu
                165                 170                 175

Ser Glu Leu Pro Arg Arg Val Asn Ser Ala Ser Ser Ser Asn Pro Pro
            180                 185                 190

Ala Glu Val Asp Pro Asp Thr Ile Leu Lys Ala Leu Phe Lys Ser Ser
        195                 200                 205

Gly Ala Ser Xaa Thr Thr Gln Pro Thr Glu Phe Lys Ile Lys Leu Xaa
    210                 215                 220

(2) INFORMATION FOR SEQ ID NO: 364:

        (i) SEQUENCE CHARACTERISTICS:
                (A) LENGTH: 349 amino acids
                (B) TYPE: amino acid
                (D) TOPOLOGY: linear
        (xi) SEQUENCE DESCRIPTION: SEQ ID NO: 364:

Met Ser Lys Asn Cys Ile Lys Leu Leu Cys Glu Asp Pro Val Phe Ala
  1               5                  10                  15

Glu Tyr Ile Lys Cys Ile Leu Met Asp Glu Arg Thr Phe Leu Asn Asn
            20                  25                  30

Asn Ile Val Tyr Thr Phe Met Thr His Phe Leu Leu Lys Val Gln Ser
        35                  40                  45

Gln Val Phe Ser Glu Ala Asn Cys Ala Asn Leu Ile Ser Thr Leu Ile
    50                  55                  60

Thr Asn Leu Ile Ser Gln Tyr Gln Asn Leu Gln Ser Asp Phe Ser Asn
65                  70                  75                  80

Arg Val Glu Ile Ser Lys Ala Ser Ala Ser Leu Asn Gly Asp Leu Arg
                85                  90                  95

Ala Leu Ala Leu Leu Leu Ser Val His Thr Pro Lys Gln Leu Asn Pro
            100                 105                 110

Ala Leu Ile Pro Thr Leu Gln Glu Leu Leu Ser Lys Cys Arg Thr Cys
        115                 120                 125

Leu Gln Gln Arg Asn Ser Leu Gln Glu Gln Glu Ala Lys Glu Arg Lys
    130                 135                 140

Thr Lys Asp Asp Glu Gly Ala Thr Pro Ile Lys Arg Arg Arg Val Ser
145                 150                 155                 160

Ser Asp Glu Glu His Thr Val Asp Ser Cys Ile Ser Asp Met Lys Thr
                165                 170                 175

Glu Thr Arg Glu Val Leu Thr Pro Thr Ser Thr Ser Asp Asn Glu Thr
            180                 185                 190

Arg Asp Ser Ser Ile Ile Asp Pro Gly Thr Glu Gln Asp Leu Pro Ser
        195                 200                 205

Pro Glu Asn Ser Ser Val Lys Glu Tyr Arg Met Glu Val Pro Ser Ser
    210                 215                 220

Phe Ser Glu Asp Met Ser Asn Ile Arg Ser Gln His Ala Glu Glu Gln
225                 230                 235                 240

Ser Asn Asn Gly Arg Tyr Asp Asp Cys Lys Glu Phe Lys Asp Leu His
                245                 250                 255

Cys Ser Lys Asp Ser Thr Leu Ala Glu Glu Glu Ser Glu Phe Pro Ser
            260                 265                 270

Thr Ser Ile Ser Ala Val Leu Ser Asp Leu Ala Asp Leu Arg Ser Cys
        275                 280                 285

Asp Gly Gln Ala Leu Pro Ser Gln Asp Pro Glu Val Ala Leu Ser Leu
    290                 295                 300

Ser Cys Gly His Ser Arg Gly Leu Phe Ser His Met Gln Gln His Asp
305                 310                 315                 320

Ile Leu Asp Thr Leu Cys Arg Thr Ile Glu Ser Thr Ile His Val Val
                325                 330                 335

Thr Arg Ile Ser Gly Lys Gly Asn Gln Ala Ala Ser Xaa
            340                 345

(2) INFORMATION FOR SEQ ID NO: 365:

(i) SEQUENCE CHARACTERISTICS:
(A) LENGTH: 467 amino acids
(B) TYPE: amino acid
(D) TOPOLOGY: linear
(xi) SEQUENCE DESCRIPTION: SEQ ID NO: 365:

Met Leu His Gln Asp His Ile Thr Phe Ala Met Leu Leu Ala Arg Ile
1               5                   10                  15

Lys Leu Lys Gly Thr Val Gly Glu Pro Thr Tyr Asp Ala Glu Phe Gln

20                  25                  30

His Phe Leu Arg Gly Asn Glu Ile Val Leu Ser Ala Gly Ser Thr Pro
        35                  40                  45

Arg Ile Gln Gly Leu Thr Val Glu Gln Ala Glu Ala Val Val Arg Leu
    50                  55                  60

Ser Cys Leu Pro Ala Phe Lys Asp Leu Ile Ala Lys Val Gln Ala Asp
65                  70                  75                  80

Glu Gln Phe Gly Ile Trp Leu Asp Ser Ser Ser Pro Glu Gln Thr Val
                85                  90                  95

Pro Tyr Leu Trp Ser Glu Glu Thr Pro Ala Thr Pro Ile Gly Gln Ala
            100                 105                 110

Ile His Arg Leu Leu Leu Ile Gln Ala Phe Arg Pro Asp Arg Leu Leu
        115                 120                 125

Ala Met Ala His Met Phe Val Ser Thr Asn Leu Gly Glu Ser Phe Met
    130                 135                 140

Ser Ile Met Glu Gln Pro Leu Asp Leu Thr His Ile Val Xaa Thr Glu
145                 150                 155                 160

Val Lys Pro Asn Thr Pro Val Leu Met Cys Ser Val Pro Gly Tyr Asp
                165                 170                 175

Ala Ser Gly His Val Glu Asp Leu Ala Ala Glu Gln Asn Thr Gln Ile
            180                 185                 190

Thr Ser Ile Ala Ile Gly Ser Ala Glu Gly Phe Asn Gln Ala Asp Lys
        195                 200                 205

Ala Ile Asn Thr Ala Val Lys Ser Gly Arg Trp Val Met Leu Lys Asn
    210                 215                 220

Val His Leu Ala Pro Gly Trp Leu Met Gln Leu Glu Lys Lys Leu His
225                 230                 235                 240

Ser Leu Gln Pro His Ala Cys Phe Arg Leu Phe Leu Thr Met Glu Ile
                245                 250                 255

Asn Pro Lys Val Pro Val Asn Leu Leu Arg Ala Gly Arg Ile Phe Val
            260                 265                 270

Phe Glu Pro Pro Pro Gly Xaa Lys Ala Asn Met Leu Arg Thr Phe Ser
        275                 280                 285

Ser Ile Pro Val Ser Arg Ile Cys Lys Ser Pro Asn Glu Arg Ala Arg
    290                 295                 300

Leu Tyr Phe Leu Leu Ala Trp Phe His Ala Ile Ile Gln Glu Arg Leu
305                 310                 315                 320

Arg Tyr Ala Pro Leu Gly Trp Ser Lys Lys Tyr Glu Phe Gly Glu Ser
                325                 330                 335

Asp Leu Arg Ser Xaa Cys Asp Thr Val Asp Thr Trp Leu Asp Asp Thr

340                 345                 350

Ala Lys Gly Arg Gln Asn Ile Ser Pro Asp Lys Ile Pro Trp Ser Ala
        355                 360                 365

Leu Lys Thr Leu Met Ala Gln Ser Ile Tyr Gly Gly Arg Val Asp Asn
    370                 375                 380

Glu Phe Asp Gln Arg Leu Leu Asn Thr Phe Leu Glu Arg Leu Phe Thr
385                 390                 395                 400

Thr Arg Ser Phe Asp Ser Glu Phe Lys Leu Ala Cys Lys Val Asp Gly
                405                 410                 415

His Lys Asp Ile Gln Met Pro Asp Gly Met Gln Ala Arg Gly Val Cys
            420                 425                 430

Ala Val Gly Gly Val Ala Pro Arg His Pro Asp Ala Leu Leu Ala Gly
        435                 440                 445

Pro Ala Gln Gln Arg Arg Glu Ser Pro Pro Tyr His Thr Gly Cys Gly
    450                 455                 460

His Asp Gln
465

(2) INFORMATION FOR SEQ ID NO: 366:

(i) SEQUENCE CHARACTERISTICS:
(A) LENGTH: 152 amino acids
(B) TYPE: amino acid
(D) TOPOLOGY: linear
(xi) SEQUENCE DESCRIPTION: SEQ ID NO: 366:

Met Ala Asp Glu Ala Thr Arg Arg Val Val Ser Glu Ile Pro Val Leu
  1               5                  10                  15

Lys Thr Asn Ala Gly Pro Arg Asp Arg Glu Leu Trp Val Gln Arg Leu
             20                  25                  30

Lys Glu Glu Tyr Gln Ser Leu Ile Arg Tyr Val Glu Asn Asn Lys Asn
         35                  40                  45

Ala Asp Asn Asp Trp Phe Arg Leu Glu Ser Asn Lys Glu Gly Thr Arg
     50                  55                  60

Trp Phe Gly Lys Cys Trp Tyr Ile His Asp Leu Leu Lys Tyr Glu Phe
 65                  70                  75                  80

Asp Ile Glu Phe Asp Ile Pro Ile Thr Tyr Pro Thr Thr Ala Pro Glu
                 85                  90                  95

Ile Ala Val Pro Glu Leu Asp Gly Lys Thr Ala Lys Met Tyr Arg Gly
            100                 105                 110

Gly Lys Ile Cys Leu Thr Asp His Phe Lys Pro Leu Trp Gly Gln Glu
        115                 120                 125

Cys Ala Gln Ile Trp Thr Ser Ser Ser His Gly Ser Gly Ala Gly Ser
    130                 135                 140

Met Xaa Gly Ser Gly Asn Pro Xaa
145                 150

(2) INFORMATION FOR SEQ ID NO: 367:

        (i) SEQUENCE CHARACTERISTICS:
                (A) LENGTH: 373 amino acids
                (B) TYPE: amino acid
                (D) TOPOLOGY: linear
        (xi) SEQUENCE DESCRIPTION: SEQ ID NO: 367:

Met Tyr Asp Gly Thr Lys Glu Val Pro Met Asn Pro Val Lys Ile Tyr
  1               5                  10                  15

Gln Val Cys Asp Ile Pro Gln Pro Gln Gly Ser Ile Ile Asn Pro Gly
             20                  25                  30

Ser Thr Gly Ser Ala Pro Trp Asp Glu Lys Asp Asn Asp Val Asp Glu
         35                  40                  45

Glu Asp Glu Glu Asp Glu Leu Asp Gln Ser Gln His His Val Pro Ile
     50                  55                  60

Gln Asp Thr Phe Pro Phe Leu Asn Ile Asn Gly Ser Pro Met Ala Pro
 65                  70                  75                  80

Ala Ser Val Gly Asn Cys Ser Val Gly Asn Cys Ser Pro Glu Ala Val
                 85                  90                  95

Trp Pro Lys Thr Glu Pro Leu Glu Met Glu Val Pro Gln Ala Pro Ile
            100                 105                 110

Gln Pro Phe Tyr Ser Ser Pro Glu Leu Trp Ile Ser Ser Leu Pro Met
        115                 120                 125

Thr Asp Leu Asp Ile Lys Phe Gln Tyr Arg Gly Lys Glu Tyr Gly Gln
    130                 135                 140

Thr Met Thr Val Ser Asn Pro Gln Gly Cys Arg Leu Phe Tyr Gly Asp
145                 150                 155                 160

Leu Gly Pro Met Pro Asp Gln Glu Glu Leu Phe Gly Pro Val Xaa Leu
                165                 170                 175

Glu Gln Val Lys Phe Pro Gly Pro Glu His Ile Thr Asn Glu Lys Gln
            180                 185                 190

Lys Leu Phe Thr Ser Lys Leu Leu Asp Val Met Asp Arg Gly Leu Ile
        195                 200                 205

Leu Glu Val Ser Gly His Ala Ile Tyr Ala Ile Arg Leu Cys Gln Cys
    210                 215                 220

Lys Val Tyr Trp Ser Gly Pro Cys Ala Pro Ser Leu Val Ala Pro Asn
225                 230                 235                 240

Leu Ile Glu Arg Gln Lys Lys Val Lys Leu Phe Cys Leu Glu Thr Phe
                245                 250                 255

Leu Ser Asp Leu Ile Ala His Gln Lys Gly Gln Ile Glu Lys Gln Pro
            260                 265                 270

Pro Phe Glu Ile Tyr Leu Cys Phe Gly Glu Glu Trp Pro Asp Gly Lys
        275                 280                 285

Pro Leu Glu Arg Lys Leu Ile Leu Val Gln Val Ile Pro Val Val Ala
    290                 295                 300

Arg Met Ile Tyr Glu Met Phe Ser Gly Asp Phe Thr Arg Ser Phe Asp
305                 310                 315                 320

Ser Gly Ser Val Arg Leu Gln Ile Ser Thr Pro Asp Ile Lys Asp Asn
                325                 330                 335

Ile Val Ala Gln Leu Lys Gln Leu Tyr Arg Ile Leu Gln Thr Gln Glu
            340                 345                 350

Ser Trp Gln Pro Met Gln Pro Thr Pro Ser Met Gln Leu Pro Pro Ala
        355                 360                 365

Leu Pro Pro Gln Xaa
    370

(2) INFORMATION FOR SEQ ID NO: 368:

        (i) SEQUENCE CHARACTERISTICS:
            (A) LENGTH: 83 amino acids
            (B) TYPE: amino acid
            (D) TOPOLOGY: linear
        (xi) SEQUENCE DESCRIPTION: SEQ ID NO: 368:

Met Gly Ser Ser Val Leu Pro Phe Cys Val Cys Val Thr Ser Pro Ser
  1               5                  10                  15

Leu Gly Gly Arg Cys Ile Gln Gly Arg Phe Ala Ser His Ser Lys Phe
             20                  25                  30

Trp Gly Phe Gly Arg Lys Thr Ala Ser Phe Gly Ala Val Gly Glu Thr
         35                  40                  45

Pro Pro Asp Gln Glu Pro Gln Lys Glu Thr Glu Pro Ala Thr Ser Ser
     50                  55                  60

His Ala Arg Pro Trp Ala Arg Val Ile Gly Leu Arg Ile Trp Pro Gln
 65                  70                  75                  80

Pro Asn Xaa

(2) INFORMATION FOR SEQ ID NO: 369:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 369:

Met Leu Leu Ser Val Ala Ile Phe Ile Leu Leu Thr Leu Val Tyr Ala
1               5                   10                  15

Tyr Trp Thr Met Xaa
            20

(2) INFORMATION FOR SEQ ID NO: 370:

(i) SEQUENCE CHARACTERISTICS:
(A) LENGTH: 227 amino acids
(B) TYPE: amino acid
(D) TOPOLOGY: linear
(xi) SEQUENCE DESCRIPTION: SEQ ID NO: 370:

Met Gly Ala Ser Ala Arg Leu Leu Arg Ala Val Ile Met Gly Ala Pro
1               5                   10                  15

Gly Ser Gly Lys Gly Thr Val Ser Ser Arg Ile Thr Thr His Phe Glu
            20                  25                  30

Leu Lys His Leu Ser Ser Gly Asp Leu Leu Arg Asp Asn Met Leu Arg
        35                  40                  45

Gly Thr Glu Ile Gly Val Leu Ala Lys Ala Phe Ile Asp Gln Gly Lys
    50                  55                  60

Leu Ile Pro Asp Asp Val Met Thr Arg Leu Ala Leu His Glu Leu Lys
65                  70                  75                  80

Asn Leu Thr Gln Tyr Ser Trp Leu Leu Asp Gly Phe Pro Arg Thr Leu
                85                  90                  95

Pro Gln Ala Glu Ala Leu Asp Arg Ala Tyr Gln Ile Asp Thr Val Ile
            100                 105                 110

Asn Leu Asn Val Pro Phe Glu Val Ile Lys Gln Arg Leu Thr Ala Arg
        115                 120                 125

Trp Ile His Pro Ala Ser Gly Arg Val Tyr Asn Ile Glu Phe Asn Pro
    130                 135                 140

Pro Lys Thr Val Gly Ile Asp Asp Leu Thr Gly Glu Pro Leu Ile Gln
145                 150                 155                 160

Arg Glu Asp Asp Lys Pro Glu Thr Val Ile Lys Arg Leu Lys Ala Tyr
                165                 170                 175

Glu Asp Gln Thr Lys Pro Val Leu Glu Tyr Tyr Gln Lys Lys Gly Val
            180                 185                 190

Leu Glu Thr Phe Ser Gly Thr Glu Thr Asn Lys Ile Trp Pro Tyr Val
        195                 200                 205

Tyr Ala Phe Leu Gln Thr Lys Val Pro Gln Arg Ser Gln Lys Ala Ser
    210                 215                 220

Val Thr Pro
225

(2) INFORMATION FOR SEQ ID NO: 371:

        (i) SEQUENCE CHARACTERISTICS:
                (A) LENGTH: 79 amino acids
                (B) TYPE: amino acid
                (D) TOPOLOGY: linear
        (xi) SEQUENCE DESCRIPTION: SEQ ID NO: 371:

Met Phe Leu Asn Cys Glu Ile Leu Glu Tyr Cys Tyr Tyr Leu Thr Gln
  1               5                  10                  15

Leu Lys Ile Ser Met Gly Lys Tyr Leu Ser Ile Pro Thr Val Leu Leu
             20                  25                  30

Lys Ile Ile Arg Cys Ser Ile Thr Ala Val Ser Asp Ser Ser Thr Ser
         35                  40                  45

Trp Ala Ile Lys Ala Gln Leu Lys Ile Glu Asn Lys Asp Leu Asp Asn
     50                  55                  60

Lys Thr Ala Lys Gly Gly Gly Gln Glu Ala Leu Thr Cys Thr Xaa
 65                  70                  75

(2) INFORMATION FOR SEQ ID NO: 372:

        (i) SEQUENCE CHARACTERISTICS:
                (A) LENGTH: 51 amino acids
                (B) TYPE: amino acid
                (D) TOPOLOGY: linear
        (xi) SEQUENCE DESCRIPTION: SEQ ID NO: 372:

Met Arg Ala Val Phe Pro Cys Cys Pro Phe Leu Thr Leu Met Leu Pro
  1               5                  10                  15

Leu Leu Glu Cys Leu Val Gly Met Ile Met Cys Tyr Leu Gly Ile Ser
             20                  25                  30

Phe Thr Asp Thr Arg Lys Thr Ala Gly Leu Lys Lys Lys Lys Lys Lys
         35                  40                  45

Lys Xaa Xaa
     50

(2) INFORMATION FOR SEQ ID NO: 373:

        (i) SEQUENCE CHARACTERISTICS:
                (A) LENGTH: 61 amino acids

(B) TYPE: amino acid
(D) TOPOLOGY: linear
(xi) SEQUENCE DESCRIPTION: SEQ ID NO: 373:

Met Phe Leu Met Arg Met His Leu Cys Phe Cys Lys Tyr Cys Cys Ser
1               5                   10                  15

Phe Ile Val Thr Pro Thr Ser Thr Ser Asn Thr Ala Ser Tyr Leu Trp
            20                  25                  30

Pro Trp Ile Ser Ala Ser Met Ala Gly Arg Gly Ser Ser Trp Ala Cys
        35                  40                  45

Thr Leu Asn Ala Val Thr Arg Glu Gly Leu Pro Glu Xaa
    50                  55                  60

(2) INFORMATION FOR SEQ ID NO: 374:

(i) SEQUENCE CHARACTERISTICS:
(A) LENGTH: 40 amino acids
(B) TYPE: amino acid
(D) TOPOLOGY: linear
(xi) SEQUENCE DESCRIPTION: SEQ ID NO: 374:

Met Ser Leu Leu Asn Thr His Thr Leu Cys Phe Val Leu Phe Cys Phe
1               5                   10                  15

Thr Leu Ser Ile Asn Gln Glu Lys Leu Ala Asn His Leu Ala Phe Arg
            20                  25                  30

Ile Leu Phe Phe Ile Val Phe Xaa
        35                  40

(2) INFORMATION FOR SEQ ID NO: 375:

(i) SEQUENCE CHARACTERISTICS:
(A) LENGTH: 44 amino acids
(B) TYPE: amino acid
(D) TOPOLOGY: linear
(xi) SEQUENCE DESCRIPTION: SEQ ID NO: 375:

Met Cys Ser Gly Gln Ser Gln Val Trp Lys Met Ala Leu Gln Ala Leu
1               5                   10                  15

Asp Ser Glu Thr Val Val Ile Leu Pro Asp Met His Leu Ile Leu Ser
            20                  25                  30

Leu Arg Leu Ile His Asn Ala Arg Pro Cys Leu Xaa
        35                  40

(2) INFORMATION FOR SEQ ID NO: 376:

(i) SEQUENCE CHARACTERISTICS:
(A) LENGTH: 203 amino acids

(B) TYPE: amino acid
(D) TOPOLOGY: linear
(xi) SEQUENCE DESCRIPTION: SEQ ID NO: 376:

Met Leu Ile Ser Glu Glu Glu Ile Pro Phe Lys Asp Asp Pro Arg Asp
1               5                   10                  15

Glu Thr Tyr Lys Pro His Leu Glu Arg Glu Thr Pro Lys Pro Arg Arg
            20                  25                  30

Lys Ser Gly Lys Val Lys Glu Glu Lys Glu Lys Lys Glu Ile Lys Val
        35                  40                  45

Glu Val Glu Val Glu Val Lys Glu Glu Glu Asn Glu Ile Arg Glu Asp
    50                  55                  60

Glu Glu Pro Pro Arg Lys Arg Gly Arg Arg Arg Lys Asp Asp Lys Ser
65                  70                  75                  80

Pro Arg Leu Pro Lys Arg Arg Lys Lys Pro Pro Ile Gln Tyr Val Arg
                85                  90                  95

Cys Glu Met Glu Gly Cys Gly Thr Val Leu Ala His Pro Arg Tyr Leu
            100                 105                 110

Gln His His Ile Lys Tyr Gln His Leu Leu Lys Lys Lys Tyr Val Cys
        115                 120                 125

Pro His Pro Ser Cys Gly Arg Leu Phe Arg Leu Gln Lys Gln Leu Leu
    130                 135                 140

Arg His Ala Lys His His Thr Asp Gln Arg Asp Tyr Ile Cys Glu Tyr
145                 150                 155                 160

Cys Ala Arg Ala Phe Lys Ser Ser His Asn Leu Ala Val His Arg Met
                165                 170                 175

Ile His Thr Gly Glu Lys His Tyr Asn Val Arg Ser Val Asp Leu Leu
            180                 185                 190

Val Asp Lys Arg His Leu Leu Ile Gly Thr Xaa
        195                 200

(2) INFORMATION FOR SEQ ID NO: 377:

(i) SEQUENCE CHARACTERISTICS:
(A) LENGTH: 29 amino acids
(B) TYPE: amino acid
(D) TOPOLOGY: linear
(xi) SEQUENCE DESCRIPTION: SEQ ID NO: 377:

Met Leu Pro Arg Arg Thr Phe Tyr Phe Tyr Phe Ile Phe Ile Phe Phe
1               5                   10                  15

Leu Ala Ser Phe Trp Gly Phe Thr Leu Arg Ala Ser Phe
            20                  25

(2) INFORMATION FOR SEQ ID NO: 378:

(i) SEQUENCE CHARACTERISTICS:
(A) LENGTH: 136 amino acids
(B) TYPE: amino acid
(D) TOPOLOGY: linear
(xi) SEQUENCE DESCRIPTION: SEQ ID NO: 378:

Met Phe Asp Ser Leu Ser Tyr Phe Lys Gly Ser Ser Leu Leu Leu Met
1               5                   10                  15

Leu Lys Thr Tyr Leu Ser Glu Asp Val Phe Gln His Ala Val Val Leu
            20                  25                  30

Tyr Leu His Asn His Ser Tyr Ala Ser Ile Gln Ser Asp Asp Leu Trp
        35                  40                  45

Asp Ser Phe Asn Glu Val Thr Asn Gln Thr Leu Asp Val Lys Arg Met
    50                  55                  60

Met Lys Thr Trp Thr Leu Gln Lys Gly Phe Pro Leu Val Thr Val Gln
65                  70                  75                  80

Lys Lys Gly Lys Glu Leu Phe Ile Gln Gln Glu Arg Phe Phe Leu Asn
                85                  90                  95

Met Lys Pro Glu Ile Gln Pro Ser Asp Thr Arg Tyr Met Pro Ser Phe
            100                 105                 110

Phe Ser Cys His Leu Phe Cys Thr Leu Arg Trp Lys Tyr Phe Glu Val
        115                 120                 125

Phe Tyr Asn His Lys Phe Leu Xaa
    130                 135

(2) INFORMATION FOR SEQ ID NO: 379:

(i) SEQUENCE CHARACTERISTICS:
(A) LENGTH: 41 amino acids
(B) TYPE: amino acid
(D) TOPOLOGY: linear
(xi) SEQUENCE DESCRIPTION: SEQ ID NO: 379:

Met Ala Trp Arg Arg Arg Glu Pro Ala Ser Gly Leu Ala Ala Cys Trp
1               5                   10                  15

Leu Trp Arg Cys Ser Pro Trp Pro Cys Ala Cys Pro Gly Pro Gly Ala
            20                  25                  30

Gly Leu Ser Ser Gly Ser Arg Pro Trp
        35                  40

(2) INFORMATION FOR SEQ ID NO: 380:

(i) SEQUENCE CHARACTERISTICS:

(A) LENGTH: 468 amino acids
(B) TYPE: amino acid
(D) TOPOLOGY: linear
(xi) SEQUENCE DESCRIPTION: SEQ ID NO: 380:

Met Glu Phe Leu Lys Val Ala Arg Arg Asn Lys Arg Glu Gln Leu Glu
1               5                   10                  15

Gln Ile Gln Lys Glu Leu Ser Val Leu Glu Glu Asp Ile Lys Arg Val
            20                  25                  30

Glu Glu Met Ser Gly Leu Tyr Ser Pro Val Ser Glu Asp Ser Thr Val
        35                  40                  45

Pro Gln Phe Glu Ala Pro Ser Pro Ser His Ser Ser Ile Ile Asp Ser
    50                  55                  60

Thr Glu Tyr Ser Gln Pro Pro Gly Phe Ser Gly Ser Ser Gln Thr Lys
65                  70                  75                  80

Lys Gln Pro Trp Tyr Asn Ser Thr Leu Ala Ser Arg Arg Lys Arg Leu
                85                  90                  95

Thr Ala His Phe Glu Asp Leu Glu Gln Cys Tyr Phe Ser Thr Arg Met
            100                 105                 110

Ser Arg Ile Ser Asp Asp Ser Arg Thr Ala Ser Gln Leu Asp Glu Phe
        115                 120                 125

Gln Glu Cys Leu Ser Lys Phe Thr Arg Tyr Asn Ser Val Arg Pro Leu
    130                 135                 140

Ala Thr Leu Ser Tyr Ala Ser Asp Leu Tyr Asn Gly Ser Ser Ile Val
145                 150                 155                 160

Ser Ser Ile Glu Phe Asp Arg Asp Cys Asp Tyr Phe Ala Ile Ala Gly
                165                 170                 175

Val Thr Lys Lys Ile Lys Val Tyr Glu Tyr Asp Thr Val Ile Gln Asp
            180                 185                 190

Ala Val Asp Ile His Tyr Pro Glu Asn Glu Met Thr Cys Asn Ser Lys
        195                 200                 205

Ile Ser Cys Ile Ser Trp Ser Ser Tyr His Lys Asn Leu Leu Ala Ser
    210                 215                 220

Ser Asp Tyr Glu Gly Thr Val Ile Leu Trp Asp Gly Phe Thr Gly Gln
225                 230                 235                 240

Arg Ser Lys Val Tyr Gln Glu His Glu Lys Arg Cys Trp Ser Val Asp
                245                 250                 255

Phe Asn Leu Met Asp Pro Lys Leu Leu Ala Ser Gly Ser Asp Asp Ala
            260                 265                 270

Lys Val Lys Leu Trp Ser Thr Asn Leu Asp Asn Ser Val Ala Ser Ile
        275                 280                 285

Glu Ala Lys Ala Asn Val Cys Cys Val Lys Phe Ser Pro Ser Ser Arg

290                 295                 300

Tyr His Leu Ala Phe Gly Cys Ala Asp His Cys Val His Tyr Tyr Asp
305                 310                 315                 320

Leu Arg Asn Thr Lys Gln Pro Ile Met Val Phe Lys Gly His Arg Lys
                325                 330                 335

Ala Val Ser Tyr Ala Lys Phe Val Ser Gly Glu Glu Ile Val Ser Ala
            340                 345                 350

Ser Thr Asp Ser Gln Leu Lys Leu Trp Asn Val Gly Lys Pro Tyr Cys
        355                 360                 365

Leu Arg Ser Phe Lys Gly His Ile Asn Glu Lys Asn Phe Val Gly Leu
    370                 375                 380

Ala Ser Asn Gly Asp Tyr Ile Ala Cys Gly Ser Glu Asn Asn Ser Leu
385                 390                 395                 400

Tyr Leu Tyr Tyr Lys Gly Leu Ser Lys Thr Leu Leu Thr Phe Lys Phe
                405                 410                 415

Asp Thr Val Lys Ser Val Leu Asp Lys Asp Arg Lys Glu Asp Asp Thr
            420                 425                 430

Asn Glu Phe Val Ser Ala Val Cys Trp Arg Ala Leu Pro Asp Gly Glu
        435                 440                 445

Ser Asn Val Leu Ile Ala Ala Asn Ser Gln Gly Thr Ile Lys Val Leu
    450                 455                 460

Glu Leu Val Xaa
465

(2) INFORMATION FOR SEQ ID NO: 381:

(i) SEQUENCE CHARACTERISTICS:
(A) LENGTH: 29 amino acids
(B) TYPE: amino acid
(D) TOPOLOGY: linear
(xi) SEQUENCE DESCRIPTION: SEQ ID NO: 381:

Met Arg Lys Glu Asp Gly Phe Trp Phe Phe Phe Phe Leu Phe Phe Phe
1               5                   10                  15

Val Val Gly Ser Lys Phe Val Asn Gly Asn Lys Leu Val
            20                  25

(2) INFORMATION FOR SEQ ID NO: 382:

(i) SEQUENCE CHARACTERISTICS:
(A) LENGTH: 29 amino acids
(B) TYPE: amino acid
(D) TOPOLOGY: linear
(xi) SEQUENCE DESCRIPTION: SEQ ID NO: 382:

Met Pro Leu Ala Pro Tyr Cys Asp Leu Leu Val Ala Leu Ser Phe Ala
1               5                   10                  15

Leu Val Leu Glu Ser Pro Val Asp Ser Ser Asp Phe Thr
            20                  25

(2) INFORMATION FOR SEQ ID NO: 383:

(i) SEQUENCE CHARACTERISTICS:
(A) LENGTH: 138 amino acids
(B) TYPE: amino acid
(D) TOPOLOGY: linear
(xi) SEQUENCE DESCRIPTION: SEQ ID NO: 383:

Met Asn Ser Leu Val Ser Trp Gln Leu Leu Leu Phe Leu Cys Ala Thr
1               5                   10                  15

His Phe Gly Glu Pro Leu Glu Lys Val Ala Ser Val Gly Asn Ser Arg
            20                  25                  30

Pro Thr Gly Gln Gln Leu Glu Ser Leu Gly Leu Leu Ala Pro Gly Glu
        35                  40                  45

Gln Ser Leu Pro Cys Thr Glu Arg Lys Pro Ala Ala Thr Ala Arg Leu
    50                  55                  60

Ser Arg Arg Gly Thr Ser Leu Ser Pro Pro Pro Glu Ser Ser Gly Ser
65                  70                  75                  80

Pro Gln Gln Pro Gly Leu Ser Ala Pro His Ser Arg Gln Ile Pro Ala
                85                  90                  95

Pro Gln Gly Ala Val Leu Val Gln Arg Glu Lys Asp Leu Pro Asn Tyr
            100                 105                 110

Asn Trp Asn Ser Phe Gly Leu Arg Phe Gly Lys Arg Glu Ala Ala Pro
        115                 120                 125

Gly Asn His Gly Arg Ser Ala Gly Arg Gly
    130                 135

(2) INFORMATION FOR SEQ ID NO: 384:

(i) SEQUENCE CHARACTERISTICS:
(A) LENGTH: 74 amino acids
(B) TYPE: amino acid
(D) TOPOLOGY: linear
(xi) SEQUENCE DESCRIPTION: SEQ ID NO: 384:

Met Ser Cys Phe Ile Asp Ser Xaa Asp Ser Lys Ile Leu His Leu Leu
1               5                   10                  15

Val Val Ser Phe Ile Cys Xaa Leu Phe Leu Leu Ile Leu Thr His Gly
            20                  25                  30

Ile Leu Ile Leu Arg Xaa Phe Phe Ser Val Xaa Xaa His Ser Leu Lys
        35                  40                  45

Asn Asn Leu Glu Glu Tyr Leu Ile Leu Met Asn Lys Ala Leu Leu Thr
    50                  55                  60

Arg Glu Asp Phe Phe Val Leu Pro Xaa Ala
65                  70

(2) INFORMATION FOR SEQ ID NO: 385:

    (i) SEQUENCE CHARACTERISTICS:
        (A) LENGTH: 521 amino acids
        (B) TYPE: amino acid
        (D) TOPOLOGY: linear
    (xi) SEQUENCE DESCRIPTION: SEQ ID NO: 385:

Met Ser Ala Gly Glu Val Glu Arg Leu Val Ser Glu Leu Ser Gly Gly
1               5                   10                  15

Thr Gly Gly Asp Glu Glu Glu Glu Trp Leu Tyr Gly Asp Glu Asn Glu
            20                  25                  30

Val Glu Arg Pro Glu Glu Glu Asn Ala Ser Ala Asn Pro Pro Ser Gly
        35                  40                  45

Ile Glu Asp Glu Thr Ala Glu Asn Gly Val Pro Lys Pro Lys Val Thr
    50                  55                  60

Glu Thr Glu Asp Asp Ser Asp Ser Asp Ser Asp Asp Asp Glu Asp Asp
65                  70                  75                  80

Val His Val Thr Ile Gly Asp Ile Lys Thr Gly Ala Pro Gln Tyr Gly
                85                  90                  95

Ser Tyr Gly Thr Ala Pro Val Asn Leu Asn Ile Lys Thr Gly Gly Arg
            100                 105                 110

Val Tyr Gly Thr Thr Gly Thr Lys Val Lys Gly Val Asp Leu Asp Ala
        115                 120                 125

Pro Gly Ser Ile Asn Gly Val Pro Leu Leu Glu Val Asp Leu Asp Ser
    130                 135                 140

Phe Glu Asp Lys Pro Trp Arg Lys Pro Gly Ala Asp Leu Ser Asp Tyr
145                 150                 155                 160

Phe Asn Tyr Gly Phe Asn Glu Asp Thr Trp Lys Ala Tyr Cys Glu Lys
                165                 170                 175

Gln Lys Arg Ile Arg Met Gly Leu Glu Val Ile Pro Val Thr Ser Thr
            180                 185                 190

Thr Asn Lys Ile Thr Val Gln Gln Gly Arg Thr Gly Asn Ser Glu Lys
        195                 200                 205

Glu Thr Ala Leu Pro Ser Thr Lys Ala Glu Phe Thr Ser Pro Pro Ser
    210                 215                 220

Leu Phe Lys Thr Gly Leu Pro Pro Ser Arg Arg Leu Pro Gly Ala Ile
225                 230                 235                 240

Asp Val Ile Gly Gln Thr Ile Thr Ile Ser Arg Val Glu Gly Arg Arg
                245                 250                 255

Arg Ala Asn Glu Asn Ser Asn Ile Gln Val Leu Ser Glu Arg Ser Ala
            260                 265                 270

Thr Glu Val Asp Asn Asn Phe Ser Lys Pro Pro Pro Phe Phe Pro Pro
        275                 280                 285

Gly Ala Pro Pro Thr His Leu Pro Pro Pro Pro Phe Leu Pro Pro Pro
    290                 295                 300

Pro Thr Val Ser Thr Ala Pro Pro Leu Ile Pro Pro Pro Gly Phe Pro
305                 310                 315                 320

Pro Pro Pro Gly Ala Pro Pro Pro Ser Leu Ile Pro Thr Ile Glu Ser
                325                 330                 335

Gly His Ser Ser Gly Tyr Asp Ser Arg Ser Ala Arg Ala Phe Pro Tyr
            340                 345                 350

Gly Asn Val Ala Phe Pro His Leu Pro Gly Ser Ala Pro Ser Trp Pro
        355                 360                 365

Ser Leu Val Asp Thr Ser Lys Gln Trp Asp Tyr Tyr Ala Arg Arg Glu
    370                 375                 380

Lys Asp Arg Asp Arg Glu Arg Asp Arg Asp Arg Glu Arg Asp Arg Asp
385                 390                 395                 400

Arg Asp Arg Glu Arg Glu Arg Thr Arg Glu Arg Glu Arg Glu Arg Asp
                405                 410                 415

His Ser Pro Thr Pro Ser Val Phe Asn Ser Asp Glu Glu Arg Tyr Arg
            420                 425                 430

Tyr Arg Glu Tyr Ala Glu Arg Gly Tyr Glu Arg His Arg Ala Ser Arg
        435                 440                 445

Glu Lys Glu Glu Arg His Arg Glu Arg Arg His Arg Glu Lys Glu Glu
    450                 455                 460

Thr Arg His Lys Ser Ser Arg Ser Asn Ser Arg Arg Arg His Glu Ser
465                 470                 475                 480

Glu Glu Gly Asp Ser His Arg Arg His Lys His Lys Lys Ser Lys Arg
                485                 490                 495

Ser Lys Glu Gly Lys Glu Ala Gly Ser Glu Pro Ala Pro Glu Gln Glu
            500                 505                 510

Ser Thr Glu Ala Thr Pro Ala Glu Xaa
        515                 520

(2) INFORMATION FOR SEQ ID NO: 386:

(i) SEQUENCE CHARACTERISTICS:
(A) LENGTH: 137 amino acids
(B) TYPE: amino acid
(D) TOPOLOGY: linear
(xi) SEQUENCE DESCRIPTION: SEQ ID NO: 386:

(2) INFORMATION FOR SEQ ID NO: 387:

(i) SEQUENCE CHARACTERISTICS:
(A) LENGTH: 186 amino acids
(B) TYPE: amino acid
(D) TOPOLOGY: linear
(xi) SEQUENCE DESCRIPTION: SEQ ID NO: 387:

Met Ala Ala Gln Lys Asp Gln Gln Lys Asp Ala Glu Ala Glu Gly Leu
1               5                   10                  15

Ser Gly Thr Thr Leu Leu Pro Lys Leu Ile Pro Ser Gly Ala Gly Arg
            20                  25                  30

Glu Trp Leu Glu Arg Arg Arg Ala Thr Ile Arg Pro Trp Ser Thr Phe
        35                  40                  45

Val Asp Gln Gln Arg Phe Ser Arg Pro Arg Asn Leu Gly Glu Leu Cys
    50                  55                  60

Gln Arg Leu Val Arg Asn Val Glu Tyr Tyr Gln Ser Asn Tyr Val Phe
65                  70                  75                  80

Val Phe Leu Gly Leu Ile Leu Tyr Cys Val Val Thr Ser Pro Met Leu
                85                  90                  95

Leu Val Ala Leu Ala Val Phe Phe Gly Ala Cys Tyr Ile Leu Tyr Leu
            100                 105                 110

Arg Thr Leu Glu Ser Lys Leu Val Leu Phe Gly Arg Glu Val Ser Pro
        115                 120                 125

Ala His Gln Tyr Ala Leu Ala Gly Gly Ile Ser Phe Pro Phe Phe Trp
    130                 135                 140

Leu Ala Gly Ala Gly Ser Ala Val Phe Trp Val Leu Gly Ala Thr Leu
145                 150                 155                 160

Val Val Ile Gly Ser His Ala Ala Phe His Gln Ile Glu Ala Val Asp
                165                 170                 175

Gly Glu Glu Leu Gln Met Glu Pro Val Xaa
            180                 185

(2) INFORMATION FOR SEQ ID NO: 388:

        (i) SEQUENCE CHARACTERISTICS:
              (A) LENGTH: 1 amino acids
              (B) TYPE: amino acid
              (D) TOPOLOGY: linear
        (xi) SEQUENCE DESCRIPTION: SEQ ID NO: 388:

Met
  1

(2) INFORMATION FOR SEQ ID NO: 389:

        (i) SEQUENCE CHARACTERISTICS:
              (A) LENGTH: 299 amino acids
              (B) TYPE: amino acid
              (D) TOPOLOGY: linear
        (xi) SEQUENCE DESCRIPTION: SEQ ID NO: 389:

Met Leu Ser Ile Phe Tyr Phe Ala Ile Pro Val Gly Ser Gly Leu Gly
  1               5                  10                  15

Tyr Ile Ala Gly Ser Lys Val Lys Asp Met Ala Gly Asp Trp His Trp
             20                  25                  30

Ala Leu Arg Val Thr Pro Gly Leu Gly Val Val Ala Val Leu Leu Leu
         35                  40                  45

Phe Leu Val Val Arg Glu Pro Pro Arg Gly Ala Val Glu Arg His Ser
     50                  55                  60

Asp Leu Pro Pro Leu Asn Pro Thr Ser Trp Trp Ala Asp Leu Arg Ala
 65                  70                  75                  80

Leu Ala Arg Asn Pro Ser Phe Val Leu Ser Ser Leu Gly Phe Thr Ala

85                    90                    95

Val Ala Phe Val Thr Gly Ser Leu Ala Leu Trp Ala Pro Ala Phe Leu
            100                 105                 110

Leu Arg Ser Arg Val Val Leu Gly Glu Thr Pro Pro Cys Leu Pro Gly
        115                 120                 125

Asp Ser Cys Ser Ser Ser Asp Ser Leu Ile Phe Gly Leu Ile Thr Cys
    130                 135                 140

Leu Thr Gly Val Leu Gly Val Gly Leu Gly Val Glu Ile Ser Arg Arg
145                 150                 155                 160

Leu Arg His Ser Asn Pro Arg Ala Asp Pro Leu Val Cys Ala Thr Gly
                165                 170                 175

Leu Leu Gly Ser Ala Pro Phe Leu Phe Leu Ser Leu Ala Cys Ala Arg
            180                 185                 190

Gly Ser Ile Val Ala Thr Tyr Ile Phe Ile Phe Ile Gly Glu Thr Leu
        195                 200                 205

Leu Ser Met Asn Trp Ala Ile Val Ala Asp Ile Leu Leu Tyr Val Val
    210                 215                 220

Ile Pro Thr Arg Arg Ser Thr Ala Glu Ala Phe Gln Ile Val Leu Ser
225                 230                 235                 240

His Leu Leu Gly Asp Ala Gly Ser Pro Tyr Leu Ile Gly Leu Ile Ser
                245                 250                 255

Asp Arg Leu Arg Arg Asn Trp Pro Pro Ser Phe Leu Ser Glu Phe Arg
            260                 265                 270

Ala Leu Gln Phe Ser Leu Met Leu Cys Ala Phe Val Gly Ala Leu Gly
        275                 280                 285

Gly Ala Leu Pro Gly His Arg His Leu His Xaa
    290                 295

(2) INFORMATION FOR SEQ ID NO: 390:

(i) SEQUENCE CHARACTERISTICS:
(A) LENGTH: 49 amino acids
(B) TYPE: amino acid
(D) TOPOLOGY: linear
(xi) SEQUENCE DESCRIPTION: SEQ ID NO: 390:

Met Gly Pro Gln Gly Trp Val Arg Pro Leu Lys Thr Ala Pro Lys Leu
1               5                   10                  15

Gly Glu Ala Ile Arg Leu Ile Leu Phe Leu Asn Phe Val Lys Gln Cys
            20                  25                  30

Ile Ala Ser Val Asn Leu Cys Ile Leu Arg Leu Asn Ile Thr Pro Leu
        35                  40                  45

Leu

(2) INFORMATION FOR SEQ ID NO: 391:

(i) SEQUENCE CHARACTERISTICS:
(A) LENGTH: 61 amino acids
(B) TYPE: amino acid
(D) TOPOLOGY: linear
(xi) SEQUENCE DESCRIPTION: SEQ ID NO: 391:

Met Tyr Val Asn Tyr Gly Thr Arg Asn Tyr Ser Thr Glu Gly Pro Ala
1               5                   10                  15

Ala Leu Leu Asp Gln Ala Lys Leu Ser Leu Leu Val Trp Val Leu Cys
            20                  25                  30

Phe Val Leu Leu Phe Val Cys Phe Cys Gly Leu Ser Tyr Val Val Ile
        35                  40                  45

Ala Gln Val Pro Val Gly Leu Leu Cys Ile Thr Glu Xaa
    50                  55                  60

(2) INFORMATION FOR SEQ ID NO: 392:

(i) SEQUENCE CHARACTERISTICS:
(A) LENGTH: 79 amino acids
(B) TYPE: amino acid
(D) TOPOLOGY: linear
(xi) SEQUENCE DESCRIPTION: SEQ ID NO: 392:

Met Leu Trp Phe Ala Asn Phe Phe Thr Tyr Leu Phe Leu Ser Gln Ser
1               5                   10                  15

Val Ala Phe Val His Ile Ser His Ile Gly Val Arg Gln Val Asn Thr
            20                  25                  30

Asn Cys Tyr Phe Ser Arg Lys Ser Tyr Cys Tyr Gly Ile Leu Asn Pro
        35                  40                  45

Ile Asn Cys Ile Lys Gly Lys Lys Lys Lys Lys Lys Lys Lys Lys Lys
    50                  55                  60

Lys Lys Lys Lys Ile Pro Ala Gly Arg Xaa Leu Phe Pro Phe Gly
65                  70                  75

(2) INFORMATION FOR SEQ ID NO: 393:

(i) SEQUENCE CHARACTERISTICS:
(A) LENGTH: 36 amino acids
(B) TYPE: amino acid
(D) TOPOLOGY: linear
(xi) SEQUENCE DESCRIPTION: SEQ ID NO: 393:

Met Pro Gly Ala Phe Ser Glu Thr Val Ile Asn Asp Leu Leu Ser Leu
1               5                   10                  15

Phe Leu Val Leu Pro Ala Glu Leu Ser Tyr Ser Thr Leu Ser Gly Val
            20                  25                  30

Tyr Arg Asn Ala
        35

(2) INFORMATION FOR SEQ ID NO: 394:

(i) SEQUENCE CHARACTERISTICS:
(A) LENGTH: 180 amino acids
(B) TYPE: amino acid
(D) TOPOLOGY: linear
(xi) SEQUENCE DESCRIPTION: SEQ ID NO: 394:

Met Ala Gln Ser Arg Asp Gly Gly Asn Pro Phe Ala Glu Pro Ser Glu
1               5                   10                  15

Leu Asp Asn Pro Phe Gln Asp Pro Ala Val Ile Gln His Arg Pro Ser
            20                  25                  30

Arg Gln Tyr Ala Thr Leu Asp Val Tyr Asn Pro Phe Glu Thr Arg Glu
        35                  40                  45

Pro Pro Pro Ala Tyr Glu Pro Pro Ala Pro Ala Pro Leu Pro Pro Pro
    50                  55                  60

Ser Ala Pro Ser Leu Gln Pro Ser Arg Lys Leu Ser Pro Thr Glu Pro
65                  70                  75                  80

Lys Asn Tyr Gly Ser Tyr Ser Thr Gln Ala Ser Ala Ala Ala Ala Thr
                85                  90                  95

Ala Glu Leu Leu Lys Lys Gln Glu Glu Leu Asn Arg Lys Ala Glu Glu
            100                 105                 110

Leu Asp Arg Arg Ser Glu Ser Cys Ser Met Leu Pro Trp Xaa Ala Gln
        115                 120                 125

Leu Leu Asp Arg Thr Ile Gly Pro Leu Tyr Leu Leu Phe Val Gln Phe
    130                 135                 140

Ser Pro Ala Phe Ser Arg Thr Ser Pro Trp Arg Ser Pro Lys Asn Phe
145                 150                 155                 160

Arg Arg Leu Tyr Pro Pro Cys Thr Thr Ser Gly Cys Ala Ala Arg Trp
                165                 170                 175

Xaa Phe Ser Xaa
            180

(2) INFORMATION FOR SEQ ID NO: 395:

(i) SEQUENCE CHARACTERISTICS:

(A) LENGTH: 21 amino acids
(B) TYPE: amino acid
(D) TOPOLOGY: linear
(xi) SEQUENCE DESCRIPTION: SEQ ID NO: 395:

Met Pro Thr Pro Cys Thr Ser Leu Pro Ser Cys Cys Gln His Arg Ser
1               5                   10                  15

Ile Thr Met Thr Leu
            20

(2) INFORMATION FOR SEQ ID NO: 396:

(i) SEQUENCE CHARACTERISTICS:
(A) LENGTH: 60 amino acids
(B) TYPE: amino acid
(D) TOPOLOGY: linear
(xi) SEQUENCE DESCRIPTION: SEQ ID NO: 396:

Met Pro Leu Phe Ile Pro Leu Ile Phe Phe Leu Ser Leu Leu His Cys
1               5                   10                  15

Gln Ser Lys His Pro Ile Gln Met Ser Leu Cys Met Cys Val Asn Ile
            20                  25                  30

Ser Leu Val Trp Ser Pro Val Arg Trp Ile Phe Gly Ser Lys Gly Leu
        35                  40                  45

Phe Ser Val His Leu Gln Ser Ser Gln Arg Pro Ser
    50                  55                  60

(2) INFORMATION FOR SEQ ID NO: 397:

(i) SEQUENCE CHARACTERISTICS:
(A) LENGTH: 152 amino acids
(B) TYPE: amino acid
(D) TOPOLOGY: linear
(xi) SEQUENCE DESCRIPTION: SEQ ID NO: 397:

Met Ala Gly Pro Arg Pro Xaa Trp Arg Asp Gln Leu Leu Phe Met Ser
1               5                   10                  15

Ile Ile Val Leu Val Ile Val Val Ile Cys Leu Met Leu Tyr Ala Leu
            20                  25                  30

Leu Trp Glu Ala Gly Asn Leu Thr Asp Leu Pro Asn Leu Arg Ile Gly
        35                  40                  45

Phe Tyr Asn Phe Cys Leu Trp Asn Glu Asp Thr Ser Thr Leu Gln Cys
    50                  55                  60

His Gln Phe Pro Glu Leu Glu Ala Leu Gly Val Pro Arg Val Gly Leu
65                  70                  75                  80

Gly Leu Ala Arg Leu Gly Val Tyr Gly Ser Leu Val Leu Thr Leu Phe
                85                  90                  95

Ala Pro Gln Pro Leu Leu Leu Ala Gln Cys Asn Xaa Asp Glu Arg Ala
            100                 105                 110

Trp Arg Leu Ala Val Gly Phe Leu Ala Val Ser Ser Val Leu Leu Ala
        115                 120                 125

Gly Gly Leu Gly Leu Phe Leu Ser Tyr Val Trp Asn Gly Ser Xaa Ser
    130                 135                 140

Pro Ser Arg Gly Leu Gly Phe Xaa
145                 150

(2) INFORMATION FOR SEQ ID NO: 398:

(i) SEQUENCE CHARACTERISTICS:
(A) LENGTH: 480 amino acids
(B) TYPE: amino acid
(D) TOPOLOGY: linear
(xi) SEQUENCE DESCRIPTION: SEQ ID NO: 398:

Met Ser Asp Gly Phe Asp Arg Ala Pro Gly Ala Gly Arg Gly Arg Xaa
1               5                   10                  15

Arg Gly Leu Gly Arg Gly Gly Gly Gly Pro Xaa Gly Gly Gly Phe Pro
            20                  25                  30

Xaa Gly Xaa Xaa Pro Ala Glu Arg Xaa Arg His Gln Pro Pro Gln Pro
        35                  40                  45

Lys Ala Pro Gly Phe Leu Gln Pro Xaa Pro Leu Arg Gln Pro Arg Thr
    50                  55                  60

Thr Pro Pro Pro Gly Ala Gln Cys Glu Val Pro Ala Ser Pro Gln Arg
65                  70                  75                  80

Pro Ser Arg Pro Gly Ala Leu Pro Glu Gln Thr Arg Pro Leu Arg Ala
                85                  90                  95

Pro Pro Ser Ser Gln Asp Lys Ile Pro Gln Gln Asn Ser Glu Ser Ala
            100                 105                 110

Met Ala Lys Pro Gln Val Val Val Ala Pro Val Leu Met Ser Lys Leu
        115                 120                 125

Ser Val Asn Ala Pro Glu Phe Tyr Pro Ser Gly Tyr Ser Ser Ser Tyr
    130                 135                 140

Thr Glu Ser Tyr Glu Asp Gly Cys Glu Asp Tyr Pro Thr Leu Ser Glu
145                 150                 155                 160

Tyr Val Gln Asp Phe Leu Asn His Leu Thr Glu Gln Pro Gly Ser Phe
                165                 170                 175

Glu Thr Glu Ile Glu Gln Phe Ala Glu Thr Leu Asn Gly Cys Val Thr
            180                 185                 190

Thr Asp Asp Ala Leu Gln Glu Leu Val Glu Leu Ile Tyr Gln Gln Ala

195                 200                 205

Thr Ser Ile Pro Asn Phe Ser Tyr Met Gly Ala Arg Leu Cys Asn Tyr
    210                 215                 220

Leu Ser His His Leu Thr Ile Ser Pro Gln Ser Gly Asn Phe Arg Gln
225                 230                 235                 240

Leu Leu Leu Gln Arg Cys Arg Thr Glu Tyr Glu Val Lys Asp Gln Ala
                245                 250                 255

Ala Lys Gly Asp Glu Val Thr Arg Lys Arg Phe His Ala Phe Val Leu
            260                 265                 270

Phe Leu Gly Glu Leu Tyr Leu Asn Leu Glu Ile Lys Gly Thr Asn Gly
        275                 280                 285

Gln Val Thr Arg Ala Asp Ile Leu Gln Val Gly Leu Arg Glu Leu Leu
    290                 295                 300

Asn Ala Leu Phe Ser Asn Pro Met Asp Asp Asn Leu Ile Cys Ala Val
305                 310                 315                 320

Lys Leu Leu Lys Leu Thr Gly Ser Val Leu Glu Asp Ala Trp Lys Glu
                325                 330                 335

Lys Gly Lys Met Asp Met Glu Glu Ile Ile Gln Arg Ile Glu Asn Val
            340                 345                 350

Val Leu Asp Ala Asn Cys Ser Arg Asp Val Lys Gln Met Leu Leu Lys
        355                 360                 365

Leu Val Glu Leu Arg Ser Ser Asn Trp Gly Arg Val His Ala Thr Ser
    370                 375                 380

Thr Tyr Arg Glu Ala Thr Pro Glu Asn Asp Pro Asn Tyr Phe Met Asn
385                 390                 395                 400

Glu Pro Thr Phe Tyr Thr Ser Asp Gly Val Pro Phe Thr Ala Ala Asp
                405                 410                 415

Pro Asp Tyr Gln Glu Lys Tyr Gln Glu Leu Leu Glu Arg Glu Asp Phe
            420                 425                 430

Phe Pro Asp Tyr Glu Glu Asn Gly Thr Asp Leu Ser Gly Ala Gly Asp
        435                 440                 445

Pro Tyr Leu Asp Asp Ile Asp Asp Glu Met Asp Pro Glu Ile Glu Glu
    450                 455                 460

Ala Tyr Glu Lys Phe Cys Leu Glu Ser Glu Arg Lys Arg Lys Gln Xaa
465                 470                 475                 480

(2) INFORMATION FOR SEQ ID NO: 399:

(i) SEQUENCE CHARACTERISTICS:
(A) LENGTH: 423 amino acids
(B) TYPE: amino acid
(D) TOPOLOGY: linear
(xi) SEQUENCE DESCRIPTION: SEQ ID NO: 399:

Met Glu Pro Lys Thr Ile Thr Asp Ala Leu Ala Ser Ser Ile Ile Lys
1               5                   10                  15

Ser Val Leu Pro Asn Phe Leu Pro Tyr Asn Val Met Leu Tyr Ser Asp
            20                  25                  30

Ala Pro Val Ser Glu Leu Ser Leu Glu Leu Leu Leu Leu Gln Val Val
        35                  40                  45

Leu Pro Ala Leu Leu Glu Gln Gly His Thr Arg Gln Trp Leu Lys Gly
    50                  55                  60

Leu Val Arg Ala Trp Thr Val Thr Ala Gly Tyr Leu Leu Asp Leu His
65                  70                  75                  80

Ser Tyr Leu Leu Gly Asp Gln Glu Glu Asn Glu Asn Ser Ala Asn Gln
                85                  90                  95

Gln Val Asn Asn Asn Gln His Ala Arg Asn Asn Asn Ala Ile Pro Val
            100                 105                 110

Val Gly Glu Gly Leu His Ala Ala His Gln Ala Ile Leu Gln Gln Gly
        115                 120                 125

Gly Pro Val Gly Phe Gln Xaa Tyr Arg Arg Pro Leu Asn Phe Pro Leu
    130                 135                 140

Arg Ile Phe Leu Leu Ile Val Phe Met Cys Ile Thr Leu Leu Ile Ala
145                 150                 155                 160

Ser Leu Ile Cys Leu Thr Leu Pro Val Phe Ala Gly Arg Trp Leu Met
                165                 170                 175

Ser Phe Trp Thr Gly Thr Ala Lys Ile His Glu Leu Tyr Thr Ala Ala
            180                 185                 190

Cys Gly Leu Tyr Val Cys Trp Leu Thr Ile Arg Ala Val Thr Val Met
        195                 200                 205

Val Ala Trp Met Pro Gln Gly Arg Arg Val Ile Phe Gln Lys Val Lys
    210                 215                 220

Glu Trp Ser Leu Met Ile Met Lys Thr Leu Ile Val Ala Val Leu Leu
225                 230                 235                 240

Ala Gly Val Val Pro Leu Leu Leu Gly Leu Leu Phe Glu Leu Val Ile
                245                 250                 255

Val Ala Pro Leu Arg Val Pro Leu Asp Gln Thr Pro Leu Phe Tyr Pro
            260                 265                 270

Trp Gln Asp Trp Ala Leu Gly Val Leu His Ala Lys Ile Ile Ala Ala
        275                 280                 285

Ile Thr Leu Met Gly Pro Gln Trp Trp Leu Lys Thr Val Ile Glu Gln
    290                 295                 300

Val Tyr Ala Asn Gly Ile Arg Asn Ile Asp Leu His Tyr Ile Val Arg
305                 310                 315                 320

Lys Leu Ala Ala Pro Val Ile Ser Val Leu Leu Leu Ser Leu Cys Val
                325                 330                 335

Pro Tyr Val Ile Ala Ser Gly Val Val Pro Leu Leu Gly Val Thr Ala
            340                 345                 350

Glu Met Gln Asn Leu Val His Arg Arg Ile Tyr Pro Phe Leu Leu Met
        355                 360                 365

Val Val Val Leu Met Ala Ile Leu Ser Phe Gln Val Arg Gln Phe Lys
    370                 375                 380

Arg Leu Tyr Glu His Ile Lys Asn Asp Lys Tyr Leu Val Gly Gln Arg
385                 390                 395                 400

Leu Val Asn Tyr Glu Arg Lys Ser Gly Lys Gln Gly Ser Ser Pro Pro
                405                 410                 415

Pro Pro Gln Ser Ser Gln Glu
            420

(2) INFORMATION FOR SEQ ID NO: 400:

        (i) SEQUENCE CHARACTERISTICS:
                (A) LENGTH: 78 amino acids
                (B) TYPE: amino acid
                (D) TOPOLOGY: linear
        (xi) SEQUENCE DESCRIPTION: SEQ ID NO: 400:

Met Leu Arg Leu Asp Ile Ile Asn Ser Leu Val Thr Thr Val Phe Met
  1               5                  10                  15

Leu Ile Val Ser Val Leu Ala Leu Ile Pro Glu Thr Thr Thr Leu Thr
            20                  25                  30

Val Gly Gly Gly Val Phe Ala Leu Val Thr Ala Val Cys Cys Leu Ala
        35                  40                  45

Asp Gly Ala Leu Ile Tyr Arg Lys Leu Leu Phe Asn Pro Ser Gly Pro
    50                  55                  60

Tyr Gln Lys Lys Pro Val His Glu Lys Lys Glu Val Leu Xaa
 65                  70                  75

(2) INFORMATION FOR SEQ ID NO: 401:

        (i) SEQUENCE CHARACTERISTICS:
                (A) LENGTH: 74 amino acids
                (B) TYPE: amino acid
                (D) TOPOLOGY: linear

(xi) SEQUENCE DESCRIPTION: SEQ ID NO: 401:

Met Leu Lys Gln Val Met Phe Val Phe Ser Gly Met Gly Pro Arg Ser
1               5                   10                  15

His Cys Trp Gly Leu Pro Leu His Val Ala Pro Leu Cys Arg Gly His
            20                  25                  30

Gln Ala Asp Ser Ser His Leu Leu Pro Leu Lys His Gln Gly Ala Trp
        35                  40                  45

Asn Arg Asn Leu Ala Asn Gln Arg His Phe Phe Cys Pro Ser Ile Phe
    50                  55                  60

His Thr Cys Pro Thr Val Leu Phe Phe Xaa
65                  70

(2) INFORMATION FOR SEQ ID NO: 402:

(i) SEQUENCE CHARACTERISTICS:
(A) LENGTH: 20 amino acids
(B) TYPE: amino acid
(D) TOPOLOGY: linear
(xi) SEQUENCE DESCRIPTION: SEQ ID NO: 402:

Ala Arg Thr Ile Leu Val Leu Tyr Leu Ser Leu Gln Arg Leu Glu Asn
1               5                   10                  15

Leu Ala Tyr His
            20

(2) INFORMATION FOR SEQ ID NO: 403:

(i) SEQUENCE CHARACTERISTICS:
(A) LENGTH: 87 amino acids
(B) TYPE: amino acid
(D) TOPOLOGY: linear
(xi) SEQUENCE DESCRIPTION: SEQ ID NO: 403:

(2) INFORMATION FOR SEQ ID NO: 404:

(i) SEQUENCE CHARACTERISTICS:
(A) LENGTH: 92 amino acids
(B) TYPE: amino acid
(D) TOPOLOGY: linear
(xi) SEQUENCE DESCRIPTION: SEQ ID NO: 404:

Met Asn Ala Ala Met Val His Ile Asn Arg Ala Leu Lys Leu Ile Ile
1               5                   10                  15

Arg Leu Phe Leu Val Glu Asp Leu Val Asp Ser Leu Lys Leu Ala Val
            20                  25                  30

Phe Met Trp Leu Met Thr Tyr Val Gly Ala Val Phe Asn Gly Ile Thr
        35                  40                  45

Leu Leu Ile Leu Ala Glu Leu Leu Ile Phe Ser Val Pro Ile Val Tyr
    50                  55                  60

Glu Lys Tyr Lys Thr Gln Ile Asp His Tyr Val Gly Ile Ala Arg Asp
65                  70                  75                  80

Gln Thr Lys Ser Ile Val Glu Lys Ile Pro Ser Lys
                85                  90

(2) INFORMATION FOR SEQ ID NO: 405:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 405:

Met Ala Cys Ser Cys Leu Met Ile Gln Ser Phe Ser Thr Ser Ala Leu
1               5                   10                  15

Val Leu Phe Tyr Gly
            20

(2) INFORMATION FOR SEQ ID NO: 406:

(i) SEQUENCE CHARACTERISTICS:
(A) LENGTH: 174 amino acids
(B) TYPE: amino acid
(D) TOPOLOGY: linear
(xi) SEQUENCE DESCRIPTION: SEQ ID NO: 406:

Met Glu Glu Gly Gly Asn Leu Gly Gly Leu Ile Lys Met Val His Leu
1               5                   10                  15

Leu Val Leu Ser Gly Ala Trp Gly Met Gln Met Trp Val Thr Phe Val
            20                  25                  30

Ser Gly Phe Pro Ala Phe Pro Lys Pro Ser Pro Thr Tyr Leu Arg Thr
        35                  40                  45

Ser Ala Glu Gln Thr Leu Pro Leu Leu Leu Pro His Leu His Gly Leu
    50                  55                  60

Cys Leu His Gln Pro Leu His Leu Gly Phe Thr Ala Cys Leu Gly Ser
65                  70                  75                  80

Ala His Ile Leu Gly Gly Gln Pro Ala Leu Pro Ala Val Pro Glu Pro
                85                  90                  95

Tyr Ala Gly His Cys Gln Arg Pro Leu Ala Gly Thr Pro His His Ser
            100                 105                 110

Cys His Val Gly Pro Ala Asn Arg Gly Arg Arg Ser Glu Ala Trp Val
        115                 120                 125

Gly Arg Tyr Gln Ala Ala Asn Arg Phe Pro Ile Leu Asn Ala Xaa Cys
    130                 135                 140

Glu Arg Arg Thr Pro Ser Thr Val Leu Ser Ala Arg Ile Ser Ser Ala
145                 150                 155                 160

Thr Met Gly Cys Pro Leu Phe Ala Ile Trp Ala Ala Ser Xaa
                165                 170

(2) INFORMATION FOR SEQ ID NO: 407:

(i) SEQUENCE CHARACTERISTICS:
(A) LENGTH: 64 amino acids
(B) TYPE: amino acid
(D) TOPOLOGY: linear
(xi) SEQUENCE DESCRIPTION: SEQ ID NO: 407:

Met Ala Phe Ile Leu Leu Phe Tyr Cys Leu Met Thr Phe Leu Ser Leu
1               5                   10                  15

Glu Gln Asn Ser Ala Thr Val Glu Pro Ser Ser His Glu Ile Leu His
            20                  25                  30

Leu Leu Gln Asn Cys Phe Glu Leu Leu Arg Thr Ser Thr Ser Gln Cys
        35                  40                  45

Thr Glu Gly Ile Pro Cys Gln Arg Tyr Gln Asn Gly Leu His Ile Xaa
    50                  55                  60

(2) INFORMATION FOR SEQ ID NO: 408:

(i) SEQUENCE CHARACTERISTICS:
(A) LENGTH: 280 amino acids
(B) TYPE: amino acid

(D) TOPOLOGY: linear
(xi) SEQUENCE DESCRIPTION: SEQ ID NO: 408:

Met Glu Ala Val Val Asn Leu Tyr Gln Glu Val Met Lys His Ala Asp
1               5                   10                  15

Pro Arg Ile Gln Gly Tyr Pro Leu Met Gly Ser Pro Leu Leu Met Thr
            20                  25                  30

Ser Ile Leu Leu Thr Tyr Val Tyr Phe Val Leu Ser Leu Gly Pro Arg
        35                  40                  45

Ile Met Ala Asn Arg Lys Pro Phe Gln Leu Arg Gly Phe Met Ile Val
    50                  55                  60

Tyr Asn Phe Ser Leu Val Ala Leu Ser Leu Tyr Ile Val Tyr Glu Phe
65                  70                  75                  80

Leu Met Ser Gly Trp Leu Ser Thr Tyr Thr Trp Arg Cys Asp Pro Val
                85                  90                  95

Asp Tyr Ser Asn Ser Pro Glu Ala Leu Arg Met Val Arg Val Ala Trp
            100                 105                 110

Leu Phe Leu Phe Ser Lys Phe Ile Glu Leu Met Asp Thr Val Ile Phe
        115                 120                 125

Ile Leu Arg Lys Lys Asp Gly Gln Val Thr Phe Leu His Val Phe His
    130                 135                 140

His Ser Val Leu Pro Trp Ser Trp Trp Trp Gly Val Lys Ile Ala Pro
145                 150                 155                 160

Gly Gly Met Gly Ser Phe His Ala Met Ile Asn Ser Ser Val His Val
                165                 170                 175

Ile Met Tyr Leu Tyr Tyr Gly Leu Ser Ala Phe Gly Pro Val Ala Gln
            180                 185                 190

Pro Tyr Leu Trp Trp Lys Lys His Met Thr Ala Ile Gln Leu Ile Gln
        195                 200                 205

Phe Val Leu Val Ser Leu His Ile Ser Gln Tyr Tyr Phe Met Ser Ser
    210                 215                 220

Cys Asn Tyr Gln Tyr Pro Val Ile Ile His Leu Ile Trp Met Tyr Gly
225                 230                 235                 240

Thr Ile Phe Phe Met Leu Phe Ser Asn Phe Trp Tyr His Ser Tyr Thr
                245                 250                 255

Lys Gly Lys Arg Leu Pro Arg Ala Leu Gln Gln Asn Gly Ala Pro Gly
            260                 265                 270

Ile Ala Lys Val Lys Ala Asn Xaa
        275                 280

(2) INFORMATION FOR SEQ ID NO: 409:

(i) SEQUENCE CHARACTERISTICS:
(A) LENGTH: 284 amino acids
(B) TYPE: amino acid
(D) TOPOLOGY: linear
(xi) SEQUENCE DESCRIPTION: SEQ ID NO: 409:

Met Xaa Leu Trp Pro Gln Thr Cys Ser Gly Lys Phe Asp Gly Thr Leu
1               5                   10                  15

Ala Phe Ser Ile His Xaa Leu Ala Val Ile Leu Gly Asp Gln Leu Thr
            20                  25                  30

Ala Ala Asp Leu Val Pro Ile Phe Asn Gly Phe Leu Lys Asp Leu Asp
        35                  40                  45

Glu Val Arg Ile Gly Val Leu Lys His Leu His Asp Phe Leu Lys Leu
    50                  55                  60

Leu His Ile Asp Lys Arg Arg Glu Tyr Leu Tyr Gln Leu Gln Glu Phe
65                  70                  75                  80

Leu Val Thr Asp Asn Ser Arg Asn Trp Arg Phe Arg Ala Glu Leu Ala
                85                  90                  95

Glu Gln Leu Ile Leu Leu Leu Glu Leu Tyr Ser Pro Arg Asp Val Tyr
            100                 105                 110

Asp Tyr Leu Arg Pro Ile Ala Leu Asn Leu Cys Ala Asp Lys Val Ser
        115                 120                 125

Ser Val Arg Trp Ile Ser Tyr Lys Leu Val Ser Glu Met Val Lys Lys
    130                 135                 140

Leu His Ala Ala Thr Pro Pro Thr Phe Gly Val Asp Leu Ile Asn Glu
145                 150                 155                 160

Leu Val Glu Asn Phe Gly Arg Cys Pro Lys Trp Ser Gly Arg Gln Ala
                165                 170                 175

Phe Val Phe Val Cys Gln Thr Val Ile Glu Asp Asp Cys Leu Pro Met
            180                 185                 190

Asp Gln Phe Ala Val His Leu Met Pro His Leu Leu Thr Leu Ala Asn
        195                 200                 205

Asp Arg Val Pro Asn Val Arg Val Leu Leu Ala Lys Thr Leu Arg Gln
    210                 215                 220

Thr Leu Leu Glu Lys Asp Tyr Phe Leu Ala Ser Ala Ser Cys His Gln
225                 230                 235                 240

Glu Ala Val Glu Gln Thr Ile Met Ala Leu Gln Met Asp Arg Asp Ser
                245                 250                 255

Asp Val Lys Tyr Phe Ala Ser Ile His Pro Ala Ser Thr Lys Ile Ser
            260                 265                 270

Glu Asp Ala Met Ser Thr Ala Ser Ser Thr Tyr Xaa
        275                 280

(2) INFORMATION FOR SEQ ID NO: 410:

(i) SEQUENCE CHARACTERISTICS:
(A) LENGTH: 187 amino acids
(B) TYPE: amino acid
(D) TOPOLOGY: linear
(xi) SEQUENCE DESCRIPTION: SEQ ID NO: 410:

Met Leu Phe Leu Phe Phe Val Ile Ile Phe Leu Phe Val Phe Leu Ile
1               5                   10                  15

Leu Ile Ile Gln Phe Ser Lys Pro Leu Thr Asn Pro His Pro Pro Ala
            20                  25                  30

Gly Xaa Ser Asp Arg Arg Arg Arg Tyr Ser Ser Tyr Arg Ser His Asp
        35                  40                  45

His Tyr Gln Arg Gln Arg Val Leu Gln Lys Glu Arg Ala Ile Glu Glu
    50                  55                  60

Arg Arg Val Val Phe Ile Gly Lys Ile Pro Gly Arg Met Thr Arg Ser
65                  70                  75                  80

Glu Leu Lys Gln Arg Phe Ser Val Phe Gly Glu Ile Glu Glu Cys Thr
                85                  90                  95

Ile His Phe Arg Val Gln Gly Asp Asn Tyr Gly Phe Val Thr Tyr Arg
            100                 105                 110

Tyr Ala Glu Glu Ala Phe Ala Ala Ile Glu Ser Gly His Lys Leu Arg
        115                 120                 125

Gln Ala Asp Glu Gln Pro Phe Asp Leu Cys Phe Gly Gly Arg Arg Xaa
    130                 135                 140

Xaa Cys Lys Arg Ser Tyr Ser Asp Leu Asp Ser Asn Arg Glu Asp Phe
145                 150                 155                 160

Asp Pro Ala Pro Val Lys Ser Lys Phe Asp Ser Leu Asp Phe Asp Thr
                165                 170                 175

Leu Leu Lys Gln Ala Gln Lys Asn Leu Arg Arg
            180                 185

(2) INFORMATION FOR SEQ ID NO: 411:

(i) SEQUENCE CHARACTERISTICS:
(A) LENGTH: 237 amino acids
(B) TYPE: amino acid
(D) TOPOLOGY: linear
(xi) SEQUENCE DESCRIPTION: SEQ ID NO: 411:

Met Lys Leu Pro Gly Lys Phe Arg Arg Ala His Gln Gly Asn Leu Glu
1               5                   10                  15

Ser Gln Leu Thr Ser Glu Ser Tyr Tyr Lys Glu Thr Leu Ser Val Pro
            20                  25                  30

Thr Val Glu His Ile Ile Gln Glu Leu Lys Asp Ile Phe Ser Glu Gln
        35                  40                  45

His Leu Lys Ala Leu Lys Cys Leu Ser Leu Val Pro Ser Val Met Gly
    50                  55                  60

Gln Leu Lys Phe Asn Thr Ser Glu Glu His His Ala Asp Met Tyr Arg
65                  70                  75                  80

Ser Asp Leu Pro Asn Pro Asp Thr Leu Ser Ala Glu Leu His Cys Trp
                85                  90                  95

Arg Ile Lys Trp Lys His Arg Gly Lys Asp Ile Glu Leu Pro Ser Thr
            100                 105                 110

Ile Tyr Glu Ala Leu His Leu Pro Asp Ile Lys Phe Phe Pro Asn Val
        115                 120                 125

Tyr Ala Leu Leu Lys Val Leu Cys Ile Leu Pro Val Met Lys Val Glu
    130                 135                 140

Asn Glu Arg Tyr Glu Asn Gly Arg Lys Arg Leu Lys Ala Tyr Leu Arg
145                 150                 155                 160

Asn Thr Leu Thr Asp Gln Arg Ser Ser Asn Leu Ala Leu Leu Asn Ile
                165                 170                 175

Asn Phe Asp Ile Lys His Asp Leu Asp Leu Met Val Asp Thr Tyr Ile
            180                 185                 190

Lys Leu Tyr Thr Xaa Xaa Ser Xaa Leu Xaa Thr Xaa Xaa Ser Xaa Xaa
        195                 200                 205

Val Glu Xaa Xaa Xaa Xaa Xaa Xaa Xaa Xaa Xaa Gly Xaa Xaa Xaa Xaa
    210                 215                 220

Asp Xaa Xaa Xaa Arg Glu Lys Ala Val Arg Cys Met Xaa
225                 230                 235

(2) INFORMATION FOR SEQ ID NO: 412:

(i) SEQUENCE CHARACTERISTICS:
(A) LENGTH: 192 amino acids
(B) TYPE: amino acid
(D) TOPOLOGY: linear
(xi) SEQUENCE DESCRIPTION: SEQ ID NO: 412:

Met Lys Pro Met Ala Val Val Ala Ser Thr Val Leu Gly Leu Val Gln
1               5                   10                  15

Asn Met Arg Ala Phe Gly Gly Ile Leu Val Val Val Tyr Tyr Val Phe
            20                  25                  30

Ala Ile Ile Gly Ile Asn Leu Phe Arg Gly Val Ile Val Ala Leu Pro
        35                  40                  45

Gly Asn Ser Ser Leu Ala Pro Ala Asn Gly Ser Ala Pro Cys Gly Ser
    50                  55                  60

Phe Glu Gln Leu Glu Tyr Trp Ala Asn Asn Phe Asp Asp Phe Ala Ala
65                  70                  75                  80

Ala Leu Val Thr Leu Trp Asn Leu Met Val Val Asn Asn Trp Gln Val
                85                  90                  95

Phe Leu Asp Ala Tyr Arg Arg Tyr Ser Gly Pro Trp Ser Lys Ile Tyr
            100                 105                 110

Phe Val Leu Trp Trp Leu Val Ser Ser Val Ile Trp Val Asn Leu Phe
        115                 120                 125

Leu Ala Leu Ile Leu Glu Asn Phe Leu His Lys Trp Asp Pro Arg Ser
    130                 135                 140

His Leu Gln Pro Leu Ala Gly Thr Pro Glu Ala Thr Tyr Gln Met Thr
145                 150                 155                 160

Val Glu Leu Leu Phe Arg Asp Ile Leu Glu Glu Pro Gly Glu Asp Glu
                165                 170                 175

Leu Thr Glu Arg Leu Ser Gln His Pro His Leu Trp Leu Cys Arg Xaa
            180                 185                 190

(2) INFORMATION FOR SEQ ID NO: 413:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 413:

Asn Val Val Val Val Ala Phe Gly Leu Ile Leu Ile Ile Glu Ser Leu
 1               5                  10                  15

Gly Glu Gln Cys Pro
            20

(2) INFORMATION FOR SEQ ID NO: 414:

(i) SEQUENCE CHARACTERISTICS:
(A) LENGTH: 51 amino acids
(B) TYPE: amino acid
(D) TOPOLOGY: linear
(xi) SEQUENCE DESCRIPTION: SEQ ID NO: 414:

Met Asn Trp Gly Leu Ser Ile Trp Leu His Tyr Tyr Glu Lys Lys Lys
 1               5                  10                  15

Glu Gln Val Phe Leu Val Ile Leu Ala His Val Val Arg Arg Cys Ala
            20                  25                  30

Ser Asp Gly Ile Leu Gln Phe Glu Ser Ser Leu Leu Lys Met Arg Arg
        35                  40                  45

Ala Pro Xaa
    50

(2) INFORMATION FOR SEQ ID NO: 415:

        (i) SEQUENCE CHARACTERISTICS:
                (A) LENGTH: 32 amino acids
                (B) TYPE: amino acid
                (D) TOPOLOGY: linear
        (xi) SEQUENCE DESCRIPTION: SEQ ID NO: 415:

Met Leu Ile Ile Ser Leu Arg Pro Gln Phe Pro Ser Leu Ile Val Gln
  1               5                  10                  15

Leu Glu Cys Ser Val Leu Phe Leu Pro Ile Ser Leu Asn Leu Leu Leu
            20                  25                  30

(2) INFORMATION FOR SEQ ID NO: 416:

        (i) SEQUENCE CHARACTERISTICS:
                (A) LENGTH: 163 amino acids
                (B) TYPE: amino acid
                (D) TOPOLOGY: linear
        (xi) SEQUENCE DESCRIPTION: SEQ ID NO: 416:

Met Val Lys Val Cys Asn Asp Ser Asp Arg Trp Ser Leu Ile Ser Leu
  1               5                  10                  15

Ser Asn Asn Ser Gly Lys Asn Val Glu Leu Lys Phe Val Asp Ser Leu
            20                  25                  30

Arg Arg Gln Phe Glu Phe Ser Val Asp Ser Phe Gln Ile Lys Leu Asp
        35                  40                  45

Ser Leu Leu Leu Phe Tyr Glu Cys Ser Glu Asn Pro Met Thr Glu Thr
    50                  55                  60

Phe His Pro Thr Ile Ile Gly Glu Ser Val Tyr Gly Asp Phe Gln Glu
 65                  70                  75                  80

Ala Phe Asp His Leu Cys Asn Lys Ile Ile Ala Thr Arg Asn Pro Glu
                85                  90                  95

Glu Ile Arg Gly Gly Gly Leu Leu Lys Tyr Cys Asn Leu Leu Val Arg
            100                 105                 110

Gly Phe Arg Pro Ala Ser Asp Glu Ile Lys Thr Leu Gln Arg Tyr Met

115                 120                 125

Cys Ser Arg Phe Phe Ile Asp Phe Ser Asp Ile Gly Glu Gln Gln Arg
    130                 135                 140

Lys Leu Glu Ser Tyr Leu Gln Asn His Phe Val Gly Ile Gly Arg Pro
145                 150                 155                 160

Gln Val Xaa

(2) INFORMATION FOR SEQ ID NO: 417:

(i) SEQUENCE CHARACTERISTICS:
(A) LENGTH: 174 amino acids
(B) TYPE: amino acid
(D) TOPOLOGY: linear
(xi) SEQUENCE DESCRIPTION: SEQ ID NO: 417:

Met Ala Pro Lys Gly Lys Val Gly Thr Arg Gly Lys Lys Gln Ile Phe
  1               5                  10                  15

Glu Glu Asn Arg Glu Thr Leu Lys Phe Tyr Leu Arg Ile Ile Leu Gly
             20                  25                  30

Ala Asn Ala Ile Tyr Cys Leu Val Thr Leu Val Phe Phe Tyr Ser Ser
         35                  40                  45

Ala Ser Phe Trp Ala Trp Leu Ala Leu Gly Phe Ser Leu Ala Val Tyr
     50                  55                  60

Gly Ala Ser Tyr His Ser Met Ser Ser Met Ala Arg Ala Ala Phe Ser
 65                  70                  75                  80

Glu Asp Gly Ala Leu Met Asp Gly Gly Met Asp Leu Asn Met Glu Gln
                 85                  90                  95

Gly Met Ala Glu His Leu Lys Asp Val Ile Leu Leu Thr Ala Ile Val
            100                 105                 110

Gln Val Leu Ser Cys Phe Ser Leu Tyr Val Trp Ser Phe Trp Leu Leu
        115                 120                 125

Ala Pro Gly Arg Ala Leu Tyr Leu Leu Trp Val Asn Val Leu Gly Pro
    130                 135                 140

Trp Phe Thr Ala Asp Ser Gly Thr Pro Ala Pro Glu His Asn Glu Lys
145                 150                 155                 160

Arg Gln Arg Arg Gln Glu Arg Arg Gln Met Lys Arg Leu Xaa
                165                 170

(2) INFORMATION FOR SEQ ID NO: 418:

(i) SEQUENCE CHARACTERISTICS:
(A) LENGTH: 50 amino acids

(B) TYPE: amino acid
(D) TOPOLOGY: linear
(xi) SEQUENCE DESCRIPTION: SEQ ID NO: 418:

Met Glu Leu Pro Lys Gly Leu Gln Gly Val Gly Pro Val Ala Met Met
1               5                   10                  15

Arg Pro Phe Tyr Leu Leu Leu Pro Val Leu Cys Thr Gln Ala Leu Arg
            20                  25                  30

Gln Ser Gln Gly Lys Ser Pro Leu Leu Trp Lys Arg Thr Cys Cys Leu
        35                  40                  45

Ala Xaa
    50

(2) INFORMATION FOR SEQ ID NO: 419:

(i) SEQUENCE CHARACTERISTICS:
(A) LENGTH: 120 amino acids
(B) TYPE: amino acid
(D) TOPOLOGY: linear
(xi) SEQUENCE DESCRIPTION: SEQ ID NO: 419:

Met Leu Gly Lys Gly Gly Gly Arg Ala Gly Leu Leu Arg Tyr Arg Leu
1               5                   10                  15

Leu Tyr Phe Thr Leu Val Val Gly Glu Gly Glu Pro Gly Glu Asn Lys
            20                  25                  30

Val Thr Ile Pro Phe Phe Glu Thr Gly Lys Lys Ile Ile Phe Cys Ser
        35                  40                  45

Val Lys Met Val Glu Asn Ser Asn Val Pro Ser His Lys Gly Pro Val
    50                  55                  60

Pro Leu Arg Ser Glu Gln Trp Glu Leu Lys Ile Ser Glu Thr Leu Gly
65                  70                  75                  80

Glu Gly Lys Ile Gly Phe Leu Leu Ile Gly Arg Cys Ser Ser Gly Xaa
                85                  90                  95

Gly Gly Leu Cys Phe Cys Trp Asp Val Leu Cys Cys Met Tyr Ala Tyr
            100                 105                 110

Met Asp Arg Ser Leu Leu Ser Leu
        115                 120

(2) INFORMATION FOR SEQ ID NO: 420:

(i) SEQUENCE CHARACTERISTICS:
(A) LENGTH: 159 amino acids
(B) TYPE: amino acid
(D) TOPOLOGY: linear
(xi) SEQUENCE DESCRIPTION: SEQ ID NO: 420:

Met Thr His Leu Leu Leu Thr Ala Thr Val Thr Pro Ser Glu Gln Asn
1               5                   10                  15

Ser Ser Arg Glu Pro Gly Trp Glu Thr Ala Met Ala Lys Asp Ile Leu
            20                  25                  30

Gly Glu Ala Gly Leu His Phe Asp Glu Leu Asn Lys Leu Arg Val Leu
        35                  40                  45

Asp Pro Glu Val Thr Gln Gln Thr Ile Glu Leu Lys Glu Glu Cys Lys
    50                  55                  60

Asp Phe Val Asp Lys Ile Gly Gln Phe Gln Lys Ile Val Gly Gly Leu
65                  70                  75                  80

Ile Glu Leu Val Asp Gln Leu Ala Lys Glu Ala Glu Asn Glu Lys Met
                85                  90                  95

Lys Ala Ile Gly Ala Arg Asn Leu Leu Lys Ser Ile Ala Lys Gln Arg
            100                 105                 110

Glu Ala Gln Gln Gln Gln Leu Gln Ala Leu Ile Ala Glu Lys Lys Met
        115                 120                 125

Gln Leu Glu Arg Tyr Arg Val Glu Tyr Glu Ala Leu Cys Lys Val Glu
    130                 135                 140

Ala Glu Gln Asn Glu Phe Ile Asp Gln Phe Ile Phe Gln Lys Xaa
145                 150                 155

(2) INFORMATION FOR SEQ ID NO: 421:

(i) SEQUENCE CHARACTERISTICS:
(A) LENGTH: 154 amino acids
(B) TYPE: amino acid
(D) TOPOLOGY: linear
(xi) SEQUENCE DESCRIPTION: SEQ ID NO: 421:

Met Asn Val Gly Val Ala His Ser Glu Val Asn Pro Asn Thr Arg Val
1               5                   10                  15

Met Asn Ser Arg Gly Met Trp Leu Thr Tyr Ala Leu Gly Val Gly Leu
            20                  25                  30

Leu His Ile Val Leu Leu Ser Ile Pro Phe Phe Ser Val Pro Val Ala
        35                  40                  45

Trp Thr Leu Thr Asn Ile Ile His Asn Leu Gly Met Tyr Val Phe Leu
    50                  55                  60

His Ala Val Lys Gly Thr Pro Phe Glu Thr Pro Asp Gln Gly Lys Ala
65                  70                  75                  80

Arg Leu Leu Thr His Trp Glu Gln Leu Asp Tyr Gly Val Gln Phe Thr
                85                  90                  95

Ser Ser Arg Lys Phe Phe Thr Ile Ser Pro Ile Ile Leu Tyr Phe Leu
            100                 105                 110

Ala Ser Phe Tyr Thr Lys Tyr Asp Pro Thr His Phe Ile Leu Asn Thr
        115                 120                 125

Ala Ser Leu Leu Ser Val Leu Ile Pro Lys Met Pro Gln Leu His Gly
    130                 135                 140

Val Arg Ile Phe Gly Ile Asn Lys Tyr Xaa
145                 150

(2) INFORMATION FOR SEQ ID NO: 422:

(i) SEQUENCE CHARACTERISTICS:
(A) LENGTH: 204 amino acids
(B) TYPE: amino acid
(D) TOPOLOGY: linear
(xi) SEQUENCE DESCRIPTION: SEQ ID NO: 422:

Met Val Cys Gly Gly Phe Ala Cys Ser Lys Asn Cys Leu Cys Ala Leu
1               5                   10                  15

Asn Leu Leu Tyr Thr Leu Val Ser Leu Leu Leu Ile Gly Ile Ala Ala
            20                  25                  30

Trp Gly Ile Gly Phe Gly Leu Ile Ser Ser Leu Arg Val Val Gly Val
        35                  40                  45

Val Ile Ala Val Gly Ile Phe Leu Phe Leu Ile Ala Leu Val Gly Leu
    50                  55                  60

Ile Gly Ala Val Lys His His Gln Val Leu Leu Phe Phe Tyr Met Ile
65                  70                  75                  80

Ile Leu Leu Leu Val Phe Ile Val Gln Phe Ser Val Ser Cys Ala Cys
                85                  90                  95

Leu Ala Leu Asn Gln Glu Gln Gln Gly Gln Leu Leu Glu Val Gly Trp
            100                 105                 110

Asn Asn Thr Ala Ser Ala Arg Asn Asp Ile Gln Arg Asn Leu Asn Cys
        115                 120                 125

Cys Gly Phe Arg Ser Val Asn Pro Asn Asp Thr Cys Leu Ala Ser Cys
    130                 135                 140

Val Lys Ser Asp His Ser Cys Ser Pro Cys Ala Pro Ile Ile Gly Glu
145                 150                 155                 160

Tyr Ala Gly Glu Val Leu Arg Phe Val Gly Gly Ile Gly Leu Phe Phe
                165                 170                 175

Ser Phe Thr Glu Ile Leu Gly Val Trp Leu Thr Tyr Arg Tyr Arg Asn
            180                 185                 190

Gln Lys Asp Pro Arg Ala Asn Pro Ser Ala Phe Leu
        195                 200

(2) INFORMATION FOR SEQ ID NO: 423:

(i) SEQUENCE CHARACTERISTICS:
(A) LENGTH: 67 amino acids
(B) TYPE: amino acid
(D) TOPOLOGY: linear
(xi) SEQUENCE DESCRIPTION: SEQ ID NO: 423:

Met Leu Gln Ser Ile Ile Lys Asn Ile Trp Ile Pro Met Lys Pro Tyr
1               5                   10                  15

Tyr Thr Lys Val Tyr Gln Glu Ile Trp Ile Gly Met Gly Leu Met Gly
            20                  25                  30

Phe Ile Val Tyr Lys Ile Arg Ala Ala Asp Lys Arg Ser Lys Ala Leu
        35                  40                  45

Lys Ala Ser Ala Pro Ala Pro Gly His His Asn Gln Ile Tyr Leu Glu
    50                  55                  60

Tyr Met Xaa
65

(2) INFORMATION FOR SEQ ID NO: 424:

(i) SEQUENCE CHARACTERISTICS:
(A) LENGTH: 25 amino acids
(B) TYPE: amino acid
(D) TOPOLOGY: linear
(xi) SEQUENCE DESCRIPTION: SEQ ID NO: 424:

Met Leu Gly Val Ser Leu Phe Leu Leu Val Val Leu Tyr His Tyr Val
1               5                   10                  15

Ala Val Asn Asn Pro Lys Lys Gln Glu
            20                  25

(2) INFORMATION FOR SEQ ID NO: 425:

(i) SEQUENCE CHARACTERISTICS:
(A) LENGTH: 299 amino acids
(B) TYPE: amino acid
(D) TOPOLOGY: linear
(xi) SEQUENCE DESCRIPTION: SEQ ID NO: 425:

Met Ala Ala Xaa Glu Pro Ala Val Leu Ala Leu Pro Asn Ser Gly Ala
1               5                   10                  15

Gly Gly Ala Gly Ala Pro Ser Gly Thr Val Pro Val Leu Phe Cys Phe
            20                  25                  30

Ser Val Phe Ala Arg Pro Ser Ser Val Pro His Gly Ala Gly Tyr Glu
        35                  40                  45

Leu Leu Ile Gln Lys Phe Leu Ser Leu Tyr Gly Asp Gln Ile Asp Met

50                  55                  60

His Arg Lys Phe Val Val Gln Leu Phe Ala Glu Glu Trp Gly Gln Tyr
65                  70                  75                  80

Val Asp Leu Pro Lys Gly Phe Ala Val Ser Glu Arg Cys Lys Val Arg
                85                  90                  95

Leu Val Pro Leu Gln Ile Gln Leu Thr Thr Leu Gly Asn Leu Thr Pro
            100                 105                 110

Ser Ser Thr Val Phe Phe Cys Cys Asp Met Gln Glu Arg Phe Arg Pro
        115                 120                 125

Ala Ile Lys Tyr Phe Gly Asp Ile Ile Ser Val Gly Gln Arg Leu Leu
    130                 135                 140

Gln Gly Ala Arg Ile Leu Gly Ile Pro Val Ile Val Thr Glu Gln Tyr
145                 150                 155                 160

Pro Lys Gly Leu Gly Ser Thr Val Gln Glu Ile Asp Leu Thr Gly Val
                165                 170                 175

Lys Leu Val Leu Pro Lys Thr Lys Phe Ser Met Val Leu Pro Glu Val
            180                 185                 190

Glu Ala Ala Leu Ala Glu Ile Pro Gly Val Arg Ser Val Val Leu Phe
        195                 200                 205

Gly Val Glu Thr His Val Cys Ile Gln Gln Thr Ala Leu Glu Leu Val
    210                 215                 220

Gly Arg Gly Val Glu Val His Ile Val Ala Asp Ala Thr Ser Ser Arg
225                 230                 235                 240

Ser Met Met Asp Arg Met Phe Ala Leu Glu Arg Leu Ala Xaa Xaa Gly
                245                 250                 255

Ile Ile Val Thr Thr Ser Glu Ala Val Leu Leu Gln Leu Val Ala Asp
            260                 265                 270

Lys Asp His Pro Lys Phe Lys Glu Ile Gln Asn Leu Ile Lys Ala Ser
        275                 280                 285

Ala Pro Glu Ser Gly Leu Leu Ser Lys Val Xaa
    290                 295

(2) INFORMATION FOR SEQ ID NO: 426:

(i) SEQUENCE CHARACTERISTICS:
(A) LENGTH: 13 amino acids
(B) TYPE: amino acid
(D) TOPOLOGY: linear
(xi) SEQUENCE DESCRIPTION: SEQ ID NO: 426:

Met Arg Asp Leu Gly Thr Leu Leu Ser Pro Val Cys Ser
1               5                   10

(2) INFORMATION FOR SEQ ID NO: 427:

(i) SEQUENCE CHARACTERISTICS:
(A) LENGTH: 198 amino acids
(B) TYPE: amino acid
(D) TOPOLOGY: linear
(xi) SEQUENCE DESCRIPTION: SEQ ID NO: 427:

Met Phe Gly Cys Leu Val Ala Gly Arg Leu Val Gln Thr Ala Ala Gln
1               5                   10                  15

Gln Val Ala Glu Asp Lys Phe Val Phe Asp Leu Pro Asp Tyr Glu Ser
            20                  25                  30

Ile Asn His Val Val Val Phe Met Leu Gly Thr Ile Pro Phe Pro Glu
        35                  40                  45

Gly Met Gly Gly Ser Val Tyr Phe Ser Tyr Pro Asp Ser Asn Gly Met
    50                  55                  60

Pro Val Trp Gln Leu Leu Gly Phe Val Thr Asn Gly Lys Pro Ser Ala
65                  70                  75                  80

Ile Phe Lys Ile Ser Gly Leu Lys Ser Gly Glu Gly Ser Gln His Pro
                85                  90                  95

Phe Gly Ala Met Asn Ile Val Arg Thr Pro Ser Val Ala Gln Ile Gly
            100                 105                 110

Ile Ser Val Glu Leu Leu Asp Ser Met Ala Gln Gln Thr Pro Val Gly
        115                 120                 125

Asn Ala Ala Val Ser Ser Val Asp Ser Phe Thr Gln Phe Thr Gln Lys
    130                 135                 140

Met Leu Asp Asn Phe Tyr Asn Phe Ala Ser Ser Phe Ala Val Ser Gln
145                 150                 155                 160

Ala Gln Met Thr Pro Ser Pro Ser Glu Met Phe Ile Pro Ala Asn Val
                165                 170                 175

Val Leu Lys Trp Tyr Glu Asn Phe Gln Arg Arg Leu Ala Gln Asn Pro
            180                 185                 190

Xaa Phe Trp Xaa Thr Xaa
        195

(2) INFORMATION FOR SEQ ID NO: 428:

(i) SEQUENCE CHARACTERISTICS:
(A) LENGTH: 47 amino acids
(B) TYPE: amino acid
(D) TOPOLOGY: linear
(xi) SEQUENCE DESCRIPTION: SEQ ID NO: 428:

Met Gly Leu Pro Leu Met Ala Leu Met Trp Ser Thr Leu Pro Ala Ser

1                   5                   10                  15

Ala Gly Val Asn Phe Ile Leu Ala Leu Pro Leu Leu Leu Leu Trp Lys
            20                  25                  30

Asn Arg Gly Gly Val Gly Arg Ser Val Met Ser Ala Val Glu Xaa
        35                  40                  45

(2) INFORMATION FOR SEQ ID NO: 429:

(i) SEQUENCE CHARACTERISTICS:
(A) LENGTH: 370 amino acids
(B) TYPE: amino acid
(D) TOPOLOGY: linear
(xi) SEQUENCE DESCRIPTION: SEQ ID NO: 429:

Met Lys Lys Val Glu Glu Lys Arg Val Asp Val Asn Ser Ala Val Ala
1               5                   10                  15

Met Gly Glu Val Ile Leu Ala Val Cys His Pro Asp Cys Ile Thr Thr
            20                  25                  30

Ile Lys His Trp Ile Thr Ile Ile Arg Ala Arg Phe Glu Glu Val Leu
        35                  40                  45

Thr Trp Ala Lys Gln His Gln Gln Arg Leu Glu Thr Ala Leu Ser Glu
    50                  55                  60

Leu Val Ala Asn Ala Glu Leu Leu Glu Glu Leu Leu Ala Trp Ile Gln
65                  70                  75                  80

Trp Ala Glu Thr Thr Leu Ile Gln Arg Asp Gln Glu Pro Ile Pro Gln
                85                  90                  95

Asn Ile Asp Arg Val Lys Ala Leu Ile Ala Glu His Gln Thr Phe Met
            100                 105                 110

Glu Glu Met Thr Arg Lys Gln Pro Asp Val Asp Arg Val Thr Lys Thr
        115                 120                 125

Tyr Lys Arg Lys Asn Ile Glu Pro Thr His Ala Pro Phe Ile Glu Lys
    130                 135                 140

Ser Arg Ser Gly Gly Arg Lys Ser Leu Ser Gln Pro Thr Pro Pro Pro
145                 150                 155                 160

Met Pro Ile Leu Ser Gln Ser Glu Ala Lys Asn Pro Arg Ile Asn Gln
                165                 170                 175

Leu Ser Ala Arg Trp Gln Gln Val Trp Leu Leu Ala Leu Glu Arg Gln
            180                 185                 190

Arg Lys Leu Asn Asp Ala Leu Asp Arg Leu Glu Glu Leu Lys Glu Phe
        195                 200                 205

Ala Asn Phe Asp Phe Asp Val Trp Arg Lys Lys Tyr Met Arg Trp Met
    210                 215                 220

Asn His Lys Lys Ser Arg Val Met Asp Phe Phe Arg Arg Ile Asp Lys
225                 230                 235                 240

Asp Gln Asp Gly Lys Ile Thr Arg Gln Glu Phe Ile Asp Gly Ile Leu
                245                 250                 255

Ala Ser Lys Phe Pro Thr Thr Lys Leu Glu Met Thr Ala Val Ala Asp
            260                 265                 270

Ile Phe Asp Arg Asp Gly Asp Gly Tyr Ile Asp Tyr Tyr Glu Phe Val
        275                 280                 285

Ala Ala Leu His Pro Asn Lys Asp Ala Tyr Arg Pro Thr Thr Asp Ala
    290                 295                 300

Asp Lys Ile Glu Asp Glu Val Thr Arg Gln Val Ala Gln Cys Lys Cys
305                 310                 315                 320

Ala Lys Arg Phe Gln Val Glu Gln Ile Gly Glu Asn Lys Tyr Arg Phe
                325                 330                 335

Phe Leu Gly Asn Gln Phe Gly Asp Ser Gln Gln Leu Arg Leu Val Arg
            340                 345                 350

Ile Leu Arg Asn Arg Asp Gly Ser Arg Trp Trp Arg Met Asp Gly Leu
        355                 360                 365

Gly Xaa
    370

(2) INFORMATION FOR SEQ ID NO: 430:

        (i) SEQUENCE CHARACTERISTICS:
                (A) LENGTH: 30 amino acids
                (B) TYPE: amino acid
                (D) TOPOLOGY: linear
        (xi) SEQUENCE DESCRIPTION: SEQ ID NO: 430:

Met Asn Val Lys Thr Phe Ser Xaa Asp His Met His Phe Leu Cys Cys
  1               5                  10                  15

Leu Tyr Leu Arg Tyr Val Thr Phe Val Tyr Leu Asn Leu Phe
            20                  25                  30

(2) INFORMATION FOR SEQ ID NO: 431:

        (i) SEQUENCE CHARACTERISTICS:
                (A) LENGTH: 24 amino acids
                (B) TYPE: amino acid
                (D) TOPOLOGY: linear
        (xi) SEQUENCE DESCRIPTION: SEQ ID NO: 431:

Met Glu Pro His Leu Arg Cys Arg Val Thr Arg Val Arg Gly Ser Leu
  1               5                  10                  15

Gly Asn Thr Gly Arg Trp Leu Leu

            20

(2) INFORMATION FOR SEQ ID NO: 432:

(i) SEQUENCE CHARACTERISTICS:
(A) LENGTH: 53 amino acids
(B) TYPE: amino acid
(D) TOPOLOGY: linear
(xi) SEQUENCE DESCRIPTION: SEQ ID NO: 432:

Met His Tyr Leu Val Leu Gly Gly Leu Gly Val Phe Leu Phe Phe Ser
1               5                   10                  15

Cys Phe Val Phe Leu Phe Phe Xaa Phe Ser Phe Ala Phe Phe Pro Phe
            20                  25                  30

Tyr Leu Glu Gly Met Gly Gly Ser Gly Asn Arg Glu Val Gly Gly Gly
        35                  40                  45

Phe Cys Leu Phe Phe
    50

(2) INFORMATION FOR SEQ ID NO: 433:

(i) SEQUENCE CHARACTERISTICS:
(A) LENGTH: 176 amino acids
(B) TYPE: amino acid
(D) TOPOLOGY: linear
(xi) SEQUENCE DESCRIPTION: SEQ ID NO: 433:

Met Val Ser Lys Ala Leu Leu Arg Leu Val Ser Ala Val Asn Arg Arg
1               5                   10                  15

Arg Met Lys Leu Leu Leu Gly Ile Ala Leu Leu Ala Tyr Val Ala Ser
            20                  25                  30

Val Trp Gly Asn Phe Val Asn Met Arg Ser Ile Gln Glu Asn Gly Glu
        35                  40                  45

Leu Lys Ile Glu Ser Lys Ile Glu Glu Met Val Glu Pro Leu Arg Glu
    50                  55                  60

Lys Ile Arg Asp Leu Glu Lys Ser Phe Thr Gln Lys Tyr Pro Pro Val
65                  70                  75                  80

Lys Phe Leu Ser Glu Lys Asp Arg Lys Arg Ile Leu Ile Thr Gly Gly
                85                  90                  95

Ala Gly Phe Val Gly Ser His Leu Thr Asp Lys Leu Met Met Asp Gly
            100                 105                 110

His Glu Val Thr Val Val Asp Asn Phe Phe Thr Gly Arg Lys Arg Asn
        115                 120                 125

Val Glu His Trp Ile Gly His Glu Asn Phe Glu Leu Ile Asn His Asp
    130                 135                 140

Val Trp Ser Pro Ser Thr Ser Arg Leu Thr Arg Tyr Thr Ile Trp His
145                 150                 155                 160

Leu Gln Pro Pro Leu Gln Thr Thr Cys Ile Ile Leu Ser Arg His Xaa
                165                 170                 175

(2) INFORMATION FOR SEQ ID NO: 434:

(i) SEQUENCE CHARACTERISTICS:
(A) LENGTH: 77 amino acids
(B) TYPE: amino acid
(D) TOPOLOGY: linear
(xi) SEQUENCE DESCRIPTION: SEQ ID NO: 434:

Met Leu Arg Cys Trp Pro Leu Phe Trp Leu Pro Leu Val Ser Pro Phe
  1               5                  10                  15

Cys Ser Leu Phe Trp Leu Leu Val Glu Trp Phe Gly Thr Asn Ile Asp
             20                  25                  30

Arg Glu Ser Tyr Asp Ala Ile Gly Gly Pro Ser Trp Met Thr Ala Ser
         35                  40                  45

Ser Phe Cys Leu Ser Asn Ser Asn Ile Trp Ser Leu Glu Ile Ser Ser
     50                  55                  60

Gly Ser Thr Ser Val Val His Ser Gln Gln Ala Met Asp
 65                  70                  75

(2) INFORMATION FOR SEQ ID NO: 435:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 435:

Met Arg Ser Cys Glu Ile Gln Leu Cys Val Trp Leu Leu Val Ser Ser
  1               5                  10                  15

His Val Asp Met Val Leu Gly Gly Ser Pro Ser Thr Leu Tyr Met Met
             20                  25                  30

(2) INFORMATION FOR SEQ ID NO: 436:

(i) SEQUENCE CHARACTERISTICS:
(A) LENGTH: 30 amino acids

(B) TYPE: amino acid
(D) TOPOLOGY: linear
(xi) SEQUENCE DESCRIPTION: SEQ ID NO: 436:

Met Val Val Asn Ser Leu Cys Phe Leu Ser Leu Leu Leu Val Ile Leu
1               5                   10                  15

Glu Leu Ser Thr Asp Ser Ser Ala Arg Leu Leu Tyr His Glu
            20                  25                  30

(2) INFORMATION FOR SEQ ID NO: 437:

(i) SEQUENCE CHARACTERISTICS:
(A) LENGTH: 69 amino acids
(B) TYPE: amino acid
(D) TOPOLOGY: linear
(xi) SEQUENCE DESCRIPTION: SEQ ID NO: 437:

(2) INFORMATION FOR SEQ ID NO: 438:

(i) SEQUENCE CHARACTERISTICS:
(A) LENGTH: 19 amino acids
(B) TYPE: amino acid
(D) TOPOLOGY: linear
(xi) SEQUENCE DESCRIPTION: SEQ ID NO: 438:

Met Pro Leu Cys Phe Phe Ser Phe Leu Cys Cys Trp Val Leu Val Phe
1               5                   10                  15

Lys Leu Ile

(2) INFORMATION FOR SEQ ID NO: 439:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 439:

Met Lys Phe Ser Leu Val Leu Leu Ile Lys Ile Ile Ser Phe Glu Arg
1               5                   10                  15

Leu Leu Ile Phe Leu Phe Pro Leu Ser Phe Leu Pro Asn Ile Trp Arg
            20                  25                  30

Arg Val Met Val Asn Leu Asn Ile Leu Phe Xaa
        35                  40

(2) INFORMATION FOR SEQ ID NO: 440:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 440:

Met Leu Leu Phe Pro Ser Leu Leu Phe Ala Ala Thr Tyr Asn Val Ala
1               5                   10                  15

Asn Pro Ser Arg Leu Ile Leu Tyr Met Ile Ser Ala Gly Ala Asp Ser
            20                  25                  30

Gln

(2) INFORMATION FOR SEQ ID NO: 441:

(i) SEQUENCE CHARACTERISTICS:
(A) LENGTH: 53 amino acids
(B) TYPE: amino acid
(D) TOPOLOGY: linear
(xi) SEQUENCE DESCRIPTION: SEQ ID NO: 441:

Met Trp Gln Val Arg Gly Leu Pro Pro Val Pro Leu Leu Leu Thr Met
1               5                   10                  15

Ser Pro Pro Pro Cys Leu Ser Ser Pro Phe Pro Phe Ile Ser Val Pro
            20                  25                  30

Leu Phe Glu Ala Val Pro Ile Ser Val Ser Asp Gln Pro Ser Pro Xaa
        35                  40                  45

Leu Thr Thr Leu Leu
    50

(2) INFORMATION FOR SEQ ID NO: 442:

(i) SEQUENCE CHARACTERISTICS:
(A) LENGTH: 64 amino acids
(B) TYPE: amino acid
(D) TOPOLOGY: linear
(xi) SEQUENCE DESCRIPTION: SEQ ID NO: 442:

(2) INFORMATION FOR SEQ ID NO: 443:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 443:

Met Leu Tyr Ser Cys Glu Pro Tyr Leu Ile Ile Leu Asn Ile Tyr Ser
1               5                   10                  15

Gln Lys Ala Phe Tyr Phe Tyr Phe Phe Glu Gly Ser Phe Ser Val Cys
            20                  25                  30

Thr Leu

(2) INFORMATION FOR SEQ ID NO: 444:

(i) SEQUENCE CHARACTERISTICS:
(A) LENGTH: 89 amino acids
(B) TYPE: amino acid
(D) TOPOLOGY: linear
(xi) SEQUENCE DESCRIPTION: SEQ ID NO: 444:

Met Arg Gln Arg Gln Ala Ala Cys Gln Pro Pro Pro Ser Arg Asn Gly
1               5                   10                  15

Leu Ala Gln Glu Cys Pro Pro His Ile Pro Ser Ser Phe Phe Leu Val
            20                  25                  30

Lys Leu Leu Phe Ile Pro Trp Leu Ala Ser Leu Leu Ser Ser Pro Leu
        35                  40                  45

Asn Leu Leu Leu Leu Val Ser Ile Ser Trp Asp Leu Gly Leu Lys Leu
    50                  55                  60

Asn Leu Gln Gln Cys Arg Gln His Gln Val Leu Gln Glu Lys Asn Thr
65                  70                  75                  80

Lys Lys Phe Asn Lys Lys Lys Lys Lys
                85

(2) INFORMATION FOR SEQ ID NO: 445:

        (i) SEQUENCE CHARACTERISTICS:
                (A) LENGTH: 350 amino acids
                (B) TYPE: amino acid
                (D) TOPOLOGY: linear
        (xi) SEQUENCE DESCRIPTION: SEQ ID NO: 445:

Met Asp Phe Ile Thr Ser Thr Ala Ile Leu Pro Leu Leu Phe Gly Cys
  1               5                  10                  15

Leu Gly Val Phe Gly Leu Phe Arg Leu Leu Gln Trp Val Arg Gly Lys
             20                  25                  30

Ala Tyr Leu Arg Asn Ala Val Val Val Ile Thr Gly Ala Thr Ser Gly
         35                  40                  45

Leu Gly Lys Glu Cys Ala Lys Val Phe Tyr Ala Ala Gly Ala Lys Leu
     50                  55                  60

Val Leu Cys Gly Arg Asn Gly Gly Ala Leu Glu Glu Leu Ile Arg Glu
 65                  70                  75                  80

Leu Thr Ala Ser His Ala Thr Lys Val Gln Thr His Lys Pro Tyr Leu
                 85                  90                  95

Val Thr Phe Asp Leu Thr Asp Ser Gly Ala Ile Val Ala Ala Ala Ala
            100                 105                 110

Glu Ile Leu Gln Cys Phe Gly Tyr Val Asp Ile Leu Val Asn Asn Ala
        115                 120                 125

Gly Ile Ser Tyr Arg Gly Thr Ile Met Asp Thr Thr Val Asp Val Asp
    130                 135                 140

Lys Arg Val Met Glu Thr Asn Tyr Phe Gly Pro Val Ala Leu Thr Lys
145                 150                 155                 160

Ala Leu Leu Pro Ser Met Ile Lys Arg Arg Gln Gly His Ile Val Ala
                165                 170                 175

Ile Ser Ser Ile Gln Gly Lys Met Ser Ile Pro Phe Arg Ser Ala Tyr
            180                 185                 190

Ala Ala Ser Lys His Ala Thr Gln Ala Phe Phe Asp Cys Leu Arg Ala
        195                 200                 205

Glu Met Glu Gln Tyr Glu Ile Glu Val Thr Val Ile Ser Pro Gly Tyr
    210                 215                 220

Ile His Thr Asn Leu Ser Val Asn Ala Ile Thr Ala Asp Gly Ser Arg
225                 230                 235                 240

Tyr Gly Val Met Asp Thr Thr Thr Ala Gln Gly Arg Ser Pro Val Glu
                245                 250                 255

Val Ala Gln Asp Val Leu Ala Ala Val Gly Lys Lys Lys Lys Asp Val
            260                 265                 270

Ile Leu Ala Asp Leu Leu Pro Ser Leu Ala Val Tyr Leu Arg Thr Leu
        275                 280                 285

Ala Pro Gly Leu Phe Phe Ser Leu Met Pro Pro Gly Pro Glu Lys Ser
    290                 295                 300

Gly Asn Pro Arg Thr Pro Ser Thr Leu Thr Ser Gln Gly Gln Gly Arg
305                 310                 315                 320

Glu Ala Ala Leu Leu Gly Leu Leu Thr Leu Gln Gly Thr Val Ala Phe
                325                 330                 335

Val Glu Thr Leu Met Glu Ile Cys Leu Thr Ser Gly Lys Asp
            340                 345                 350

(2) INFORMATION FOR SEQ ID NO: 446:

        (i) SEQUENCE CHARACTERISTICS:
                (A) LENGTH: 49 amino acids
                (B) TYPE: amino acid
                (D) TOPOLOGY: linear
        (xi) SEQUENCE DESCRIPTION: SEQ ID NO: 446:

(2) INFORMATION FOR SEQ ID NO: 447:

        (i) SEQUENCE CHARACTERISTICS:
                (A) LENGTH: 278 amino acids
                (B) TYPE: amino acid
                (D) TOPOLOGY: linear
        (xi) SEQUENCE DESCRIPTION: SEQ ID NO: 447:

Met Ala Ser Ala Glu Leu Asp Tyr Thr Ile Glu Ile Pro Asp Gln Pro
  1               5                  10                  15

Cys Trp Ser Gln Lys Asn Ser Pro Ser Pro Gly Gly Lys Glu Ala Glu
             20                  25                  30

Thr Arg Gln Pro Val Val Ile Leu Leu Gly Trp Gly Gly Cys Lys Asp
         35                  40                  45

Lys Asn Leu Ala Lys Tyr Ser Ala Ile Tyr His Lys Arg Gly Cys Ile
50                  55                  60

Val Ile Arg Tyr Thr Ala Pro Trp His Met Val Phe Phe Ser Glu Ser
65                  70                  75                  80

Leu Gly Ile Pro Ser Leu Arg Val Leu Ala Gln Lys Leu Leu Glu Leu
                85                  90                  95

Leu Phe Asp Tyr Glu Ile Glu Lys Glu Pro Leu Leu Phe His Val Phe
            100                 105                 110

Ser Asn Gly Gly Val Met Leu Tyr Arg Tyr Val Leu Glu Leu Leu Gln
        115                 120                 125

Thr Arg Arg Phe Cys Arg Leu Arg Val Val Gly Thr Ile Phe Asp Ser
    130                 135                 140

Ala Pro Gly Asp Ser Asn Leu Val Gly Ala Leu Arg Ala Leu Ala Ala
145                 150                 155                 160

Ile Leu Glu Arg Arg Ala Ala Met Leu Arg Leu Leu Leu Leu Val Ala
                165                 170                 175

Phe Ala Leu Val Val Val Leu Phe His Val Leu Leu Ala Pro Ile Thr
            180                 185                 190

Ala Xaa Phe His Thr His Phe Tyr Asp Arg Leu Gln Asp Ala Gly Ser
        195                 200                 205

Arg Trp Pro Glu Leu Tyr Leu Tyr Ser Arg Ala Asp Glu Val Val Leu
    210                 215                 220

Ala Arg Asp Ile Glu Arg Met Val Glu Ala Arg Leu Ala Arg Arg Val
225                 230                 235                 240

Leu Ala Arg Ser Val Asp Phe Val Ser Ser Ala His Val Ser His Leu
                245                 250                 255

Arg Asp Tyr Pro Thr Tyr Tyr Thr Ser Leu Cys Val Asp Phe Met Arg
            260                 265                 270

Asn Cys Val Arg Cys Xaa
        275

(2) INFORMATION FOR SEQ ID NO: 448:

    (i) SEQUENCE CHARACTERISTICS:
        (A) LENGTH: 199 amino acids
        (B) TYPE: amino acid
        (D) TOPOLOGY: linear
    (xi) SEQUENCE DESCRIPTION: SEQ ID NO: 448:

Met Ser Phe Ile Phe Asp Trp Ile Tyr Ser Gly Phe Ser Ser Val Leu
1               5                   10                  15

Gln Phe Leu Gly Leu Tyr Lys Lys Thr Gly Lys Leu Val Phe Leu Gly
            20                  25                  30

Leu Asp Asn Ala Gly Lys Thr Thr Leu Leu His Met Leu Lys Asp Asp
        35                  40                  45

Arg Leu Gly Gln His Val Pro Thr Leu His Pro Thr Ser Glu Glu Leu
    50                  55                  60

Thr Ile Ala Gly Met Thr Phe Thr Thr Phe Asp Leu Gly Gly His Val
65                  70                  75                  80

Gln Ala Arg Arg Val Trp Lys Asn Tyr Leu Pro Ala Ile Asn Gly Ile
                85                  90                  95

Val Phe Leu Val Asp Cys Ala Asp His Glu Arg Leu Leu Glu Ser Lys
            100                 105                 110

Glu Glu Leu Asp Ser Leu Met Thr Asp Glu Thr Ile Ala Asn Val Pro
        115                 120                 125

Ile Leu Ile Leu Gly Asn Lys Ile Asp Arg Pro Glu Ala Ile Ser Glu
    130                 135                 140

Glu Arg Leu Arg Glu Met Phe Gly Leu Tyr Gly Gln Thr Thr Gly Lys
145                 150                 155                 160

Gly Ser Ile Ser Leu Lys Glu Leu Asn Ala Arg Pro Leu Glu Val Phe
                165                 170                 175

Met Cys Ser Val Leu Lys Arg Gln Gly Tyr Gly Glu Gly Phe Arg Trp
            180                 185                 190

Met Ala Gln Tyr Ile Asp Xaa
        195

(2) INFORMATION FOR SEQ ID NO: 449:

        (i) SEQUENCE CHARACTERISTICS:
                (A) LENGTH: 258 amino acids
                (B) TYPE: amino acid
                (D) TOPOLOGY: linear
        (xi) SEQUENCE DESCRIPTION: SEQ ID NO: 449:

Met Thr Leu Ser Arg Phe Ala Tyr Asn Gly Lys Arg Cys Pro Ser Ser
  1               5                  10                  15

Tyr Asn Ile Leu Asp Asn Ser Lys Ile Ile Ser Glu Glu Cys Arg Lys
            20                  25                  30

Glu Leu Thr Ala Leu Leu His His Tyr Tyr Pro Ile Glu Ile Asp Pro
        35                  40                  45

His Arg Thr Val Lys Glu Lys Leu Pro His Met Val Glu Trp Trp Thr
    50                  55                  60

Lys Ala His Asn Leu Leu Cys Gln Gln Lys Ile Gln Lys Phe Gln Ile
65                  70                  75                  80

Ala Gln Val Val Arg Glu Ser Asn Ala Met Leu Arg Glu Gly Tyr Lys

                85                  90                  95

Thr Phe Phe Asn Thr Leu Tyr His Asn Asn Ile Pro Leu Phe Ile Phe
            100                 105                 110

Ser Ala Gly Ile Gly Asp Ile Leu Glu Glu Ile Ile Arg Gln Met Lys
        115                 120                 125

Val Phe His Pro Asn Ile His Ile Val Ser Asn Tyr Met Asp Phe Asn
    130                 135                 140

Glu Asp Gly Phe Leu Gln Gly Phe Lys Gly Gln Leu Ile His Thr Tyr
145                 150                 155                 160

Asn Lys Asn Ser Ser Val Cys Glu Asn Xaa Gly Tyr Phe Gln Gln Leu
                165                 170                 175

Glu Gly Lys Thr Asn Val Ile Leu Leu Gly Asp Ser Ile Gly Asp Leu
            180                 185                 190

Thr Met Ala Asp Gly Val Pro Gly Val Gln Asn Ile Leu Lys Ile Gly
        195                 200                 205

Phe Leu Asn Asp Lys Val Glu Glu Arg Arg Xaa Arg Tyr Met Asp Ser
    210                 215                 220

Tyr Asp Ile Val Leu Glu Lys Asp Glu Thr Leu Asp Val Val Asn Gly
225                 230                 235                 240

Leu Leu Gln His Ile Leu Cys Gln Gly Val Gln Leu Glu Met Gln Gly
                245                 250                 255

Pro Xaa

(2) INFORMATION FOR SEQ ID NO: 450:

(i) SEQUENCE CHARACTERISTICS:
(A) LENGTH: 87 amino acids
(B) TYPE: amino acid
(D) TOPOLOGY: linear
(xi) SEQUENCE DESCRIPTION: SEQ ID NO: 450:

Met Ser His Val Leu Leu Cys Pro Ser Leu Ser Cys Ser Asn Leu Leu
1               5                   10                  15

Pro Pro Ser His Ser Leu Gly Thr Met Gly Ser Leu Ser Pro His Leu
            20                  25                  30

Cys Gly His Thr Met Cys Pro Val Asn Pro Glu Leu Pro Leu Ser Ser
        35                  40                  45

Arg Leu Thr Thr Asp Gln Pro Gln Pro Asp Ala Cys Ser Pro Thr Leu
    50                  55                  60

Leu Thr Leu Pro Leu Pro Ser Ser Phe Leu Pro His Ser Lys Pro Thr
65                  70                  75                  80

Phe Xaa His Pro Cys Ser Pro
                85

(2) INFORMATION FOR SEQ ID NO: 451:

        (i) SEQUENCE CHARACTERISTICS:
                (A) LENGTH: 315 amino acids
                (B) TYPE: amino acid
                (D) TOPOLOGY: linear
        (xi) SEQUENCE DESCRIPTION: SEQ ID NO: 451:

Met Phe Ser Ile Asn Pro Leu Glu Asn Leu Lys Val Tyr Ile Ser Ser
  1               5                  10                  15

Arg Pro Pro Leu Val Val Phe Met Ile Ser Val Xaa Pro Met Ala Ile
             20                  25                  30

Ala Phe Leu Thr Leu Gly Tyr Phe Phe Lys Ile Lys Glu Ile Lys Ser
         35                  40                  45

Pro Glu Met Ala Glu Asp Trp Asn Thr Phe Leu Leu Arg Phe Asn Asp
     50                  55                  60

Leu Asp Leu Cys Val Ser Glu Asn Glu Thr Leu Lys His Leu Thr Asn
 65                  70                  75                  80

Asp Thr Thr Thr Pro Glu Ser Thr Met Thr Ser Gly Gln Ala Arg Ala
                 85                  90                  95

Ser Thr Gln Ser Pro Gln Ala Leu Glu Asp Ser Gly Pro Val Asn Ile
            100                 105                 110

Ser Val Ser Ile Thr Leu Thr Leu Asp Pro Leu Lys Pro Phe Gly Gly
        115                 120                 125

Tyr Ser Arg Asn Val Thr His Leu Tyr Ser Thr Ile Leu Gly His Gln
    130                 135                 140

Ile Gly Leu Ser Gly Arg Glu Ala His Glu Glu Ile Asn Ile Thr Phe
145                 150                 155                 160

Thr Leu Pro Thr Ala Trp Ser Ser Asp Asp Cys Ala Leu His Gly His
                165                 170                 175

Cys Glu Gln Val Val Phe Thr Ala Cys Met Thr Leu Thr Ala Ser Pro
            180                 185                 190

Gly Val Phe Pro Val Thr Val Gln Pro Pro His Cys Val Pro Asp Thr
        195                 200                 205

Tyr Ser Asn Ala Thr Leu Trp Tyr Lys Ile Phe Thr Thr Ala Arg Asp
    210                 215                 220

Ala Asn Thr Lys Tyr Ala Gln Asp Tyr Asn Pro Phe Trp Cys Tyr Lys
225                 230                 235                 240

Gly Ala Ile Gly Lys Val Tyr His Ala Leu Asn Pro Lys Leu Thr Val
                245                 250                 255

Ile Val Pro Asp Asp Asp Arg Ser Leu Ile Asn Leu His Leu Met His
            260                 265                 270

Thr Ser Tyr Phe Leu Phe Val Met Val Ile Thr Met Phe Cys Tyr Ala
        275                 280                 285

Val Ile Lys Gly Arg Pro Ser Lys Leu Arg Gln Ser Asn Pro Glu Phe
    290                 295                 300

Cys Pro Glu Lys Val Ala Leu Ala Glu Ala Xaa
305                 310                 315

(2) INFORMATION FOR SEQ ID NO: 452:

(i) SEQUENCE CHARACTERISTICS:
(A) LENGTH: 52 amino acids
(B) TYPE: amino acid
(D) TOPOLOGY: linear
(xi) SEQUENCE DESCRIPTION: SEQ ID NO: 452:

Met Pro Gly Leu Ser Leu Ala Leu Leu Pro Phe Gly Pro Gly Cys Thr
1               5                   10                  15

Glu Ala Leu His Ala Gly Cys Phe Pro Ala Phe Ala Ser Ala Thr Arg
            20                  25                  30

Val Asn Gly Glu Ala Ala Leu Ser Pro Gly Leu Cys Asp Pro Ile Ser
        35                  40                  45

Val Pro Tyr Val
    50

(2) INFORMATION FOR SEQ ID NO: 453:

(i) SEQUENCE CHARACTERISTICS:
(A) LENGTH: 383 amino acids
(B) TYPE: amino acid
(D) TOPOLOGY: linear
(xi) SEQUENCE DESCRIPTION: SEQ ID NO: 453:

Met Ala Val Gly Gln Ile Met Thr Phe Gly Ser Pro Val Ile Gly Cys
1               5                   10                  15

Gly Phe Ile Ser Gly Trp Asn Leu Val Ser Met Cys Val Glu Tyr Val
            20                  25                  30

Leu Leu Trp Lys Val Tyr Gln Lys Thr Pro Ala Leu Ala Val Lys Ala
        35                  40                  45

Gly Leu Lys Glu Glu Glu Thr Glu Leu Lys Gln Leu Asn Leu His Lys
    50                  55                  60

Asp Thr Glu Pro Lys Pro Leu Glu Gly Thr His Leu Met Gly Val Lys
65                  70                  75                  80

Asp Ser Asn Ile His Glu Leu Glu His Glu Gln Glu Pro Thr Cys Ala
                85                  90                  95

Ser Gln Met Ala Glu Pro Phe Arg Thr Phe Arg Asp Gly Trp Val Ser
            100                 105                 110

Tyr Tyr Asn Gln Pro Val Phe Leu Ala Gly Met Gly Leu Ala Phe Leu
        115                 120                 125

Tyr Met Thr Val Leu Gly Phe Asp Cys Ile Thr Thr Gly Tyr Ala Tyr
    130                 135                 140

Thr Gln Gly Leu Ser Gly Phe His Pro Gln Tyr Phe Asp Gly Ser Ile
145                 150                 155                 160

Ser Tyr Asn Trp Asn Asn Gly Asn Cys Ser Phe Tyr Leu Ala Thr Ser
                165                 170                 175

Lys Met Trp Phe Gly Ser Ala Gly Leu Ile Ser Gly Leu Ala Gln Leu
            180                 185                 190

Ser Cys Leu Ile Leu Cys Val Ile Ser Val Phe Met Pro Gly Ser Pro
        195                 200                 205

Leu Asp Leu Ser Val Ser Pro Phe Glu Asp Ile Arg Ser Arg Phe Ile
    210                 215                 220

Gln Gly Glu Ser Ile Thr Pro Thr Lys Ile Pro Glu Ile Thr Thr Glu
225                 230                 235                 240

Ile Tyr Met Ser Asn Gly Ser Asn Ser Ala Asn Ile Val Pro Glu Thr
                245                 250                 255

Ser Pro Glu Ser Val Pro Ile Ile Ser Val Ser Leu Leu Phe Ala Gly
            260                 265                 270

Val Ile Ala Ala Arg Ile Gly Leu Trp Ser Phe Asp Leu Thr Val Thr
        275                 280                 285

Gln Leu Leu Gln Glu Asn Val Ile Glu Ser Glu Arg Gly Ile Ile Asn
    290                 295                 300

Gly Val Gln Asn Ser Met Asn Tyr Leu Leu Asp Leu Leu His Phe Ile
305                 310                 315                 320

Met Val Ile Leu Ala Pro Asn Pro Glu Ala Phe Gly Leu Leu Val Leu
                325                 330                 335

Ile Ser Val Ser Phe Val Ala Met Gly His Ile Met Tyr Phe Arg Phe
            340                 345                 350

Ala Gln Asn Thr Leu Gly Asn Lys Leu Phe Ala Cys Gly Pro Asp Ala
        355                 360                 365

Lys Glu Val Arg Lys Glu Asn Gln Ala Asn Thr Ser Val Val Xaa
    370                 375                 380

(2) INFORMATION FOR SEQ ID NO: 454:

(i) SEQUENCE CHARACTERISTICS:
(A) LENGTH: 186 amino acids
(B) TYPE: amino acid
(D) TOPOLOGY: linear
(xi) SEQUENCE DESCRIPTION: SEQ ID NO: 454:

(2) INFORMATION FOR SEQ ID NO: 455:

(i) SEQUENCE CHARACTERISTICS:
(A) LENGTH: 163 amino acids
(B) TYPE: amino acid
(D) TOPOLOGY: linear
(xi) SEQUENCE DESCRIPTION: SEQ ID NO: 455:

Met Leu Gln Thr Ser Asn Tyr Ser Leu Val Leu Ser Leu Gln Phe Leu
1               5                   10                  15

Leu Leu Ser Tyr Asp Leu Phe Val Asn Ser Phe Ser Glu Leu Leu Gln
            20                  25                  30

Lys Thr Pro Val Ile Gln Leu Val Leu Phe Ile Ile Gln Asp Ile Ala

        35                  40                  45

Val Leu Phe Asn Ile Ile Ile Ile Phe Leu Met Phe Phe Asn Thr Phe
    50                  55                  60

Val Phe Gln Ala Gly Leu Val Asn Leu Leu Phe His Lys Phe Lys Gly
65                  70                  75                  80

Thr Ile Ile Leu Thr Ala Val Tyr Phe Ala Leu Ser Ile Ser Leu His
                85                  90                  95

Val Trp Val Met Asn Leu Arg Trp Lys Asn Ser Asn Ser Phe Ile Trp
            100                 105                 110

Thr Asp Gly Leu Gln Met Leu Phe Val Phe Gln Arg Leu Ala Ala Val
        115                 120                 125

Leu Tyr Cys Tyr Phe Tyr Lys Arg Thr Ala Val Arg Leu Gly Asp Pro
    130                 135                 140

His Phe Tyr Gln Asp Ser Leu Trp Leu Arg Lys Glu Phe Met Gln Val
145                 150                 155                 160

Arg Arg Xaa

(2) INFORMATION FOR SEQ ID NO: 456:

(i) SEQUENCE CHARACTERISTICS:
(A) LENGTH: 46 amino acids
(B) TYPE: amino acid
(D) TOPOLOGY: linear
(xi) SEQUENCE DESCRIPTION: SEQ ID NO: 456:

(2) INFORMATION FOR SEQ ID NO: 457:

(i) SEQUENCE CHARACTERISTICS:
(A) LENGTH: 105 amino acids
(B) TYPE: amino acid
(D) TOPOLOGY: linear
(xi) SEQUENCE DESCRIPTION: SEQ ID NO: 457:

Met Ser Tyr Leu Ala Phe Leu Tyr Met Thr Phe Asp Phe Cys Cys Leu
1               5                   10                  15

Tyr Phe Ser Thr Val Tyr Ala Pro Ser Phe Lys Tyr Ile Cys Val His
            20                  25                  30

Thr Asp Thr His Ile Cys Val Cys Val Cys Ile Tyr Leu Ser Ser Val
        35                  40                  45

Val Ser Lys Ser Ser Ala Glu Ala Asp Gly Val Leu Gln Pro Arg Arg
    50                  55                  60

His Pro Ala Ser Leu Leu Ile Val Phe Ala Thr Ser Ile Ser Glu Ser
65                  70                  75                  80

Ser Leu Leu Ile Phe Ser Phe Gln Lys Thr Glu Ala Lys Leu Ile Val
                85                  90                  95

Phe Ala Val Ser Leu Ala Ala Lys Xaa
            100                 105

(2) INFORMATION FOR SEQ ID NO: 458:

(i) SEQUENCE CHARACTERISTICS:
(A) LENGTH: 70 amino acids
(B) TYPE: amino acid
(D) TOPOLOGY: linear
(xi) SEQUENCE DESCRIPTION: SEQ ID NO: 458:

Met Leu Pro Pro Phe Ser Leu Val Tyr Thr His Phe Leu Val Ala Ser
1               5                   10                  15

Leu Leu Pro Val Ile Leu Ala Val Phe Pro Asp Ser Ala Gln Ile Val
            20                  25                  30

Pro Leu Leu Lys Pro Ile Pro Arg Pro Gln Pro Glu Val Ile Phe Pro
        35                  40                  45

Ser Ser Glu Leu Leu Glu Gln Leu Leu Ser Val Gln Phe Val Trp Gln
    50                  55                  60

Ala His Thr Val Ala Xaa
65                  70

(2) INFORMATION FOR SEQ ID NO: 459:

(i) SEQUENCE CHARACTERISTICS:
(A) LENGTH: 155 amino acids
(B) TYPE: amino acid
(D) TOPOLOGY: linear
(xi) SEQUENCE DESCRIPTION: SEQ ID NO: 459:

Met Ala Leu Leu Leu Ser Val Leu Arg Val Leu Leu Gly Gly Phe Phe
1               5                   10                  15

Ala Leu Val Gly Leu Ala Lys Leu Ser Glu Glu Ile Ser Ala Pro Val
            20                  25                  30

Ser Glu Arg Met Asn Ala Leu Phe Val Gln Phe Ala Glu Val Phe Pro
        35                  40                  45

Leu Lys Val Phe Gly Tyr Gln Pro Asp Pro Leu Asn Tyr Gln Ile Ala
50                  55                  60

Val Gly Phe Leu Glu Leu Leu Ala Gly Leu Leu Leu Val Met Gly Pro
65                  70                  75                  80

Pro Met Leu Gln Glu Ile Ser Asn Leu Phe Leu Ile Leu Leu Met Met
                85                  90                  95

Gly Ala Ile Phe Thr Leu Ala Ala Leu Lys Glu Ser Leu Ser Thr Cys
            100                 105                 110

Ile Pro Ala Ile Val Cys Leu Gly Phe Leu Leu Leu Leu Asn Val Gly
        115                 120                 125

Gln Leu Leu Ala Gln Thr Lys Lys Val Val Arg Pro Thr Arg Lys Lys
    130                 135                 140

Thr Leu Ser Thr Phe Lys Glu Ser Trp Lys Xaa
145                 150                 155

(2) INFORMATION FOR SEQ ID NO: 460:

(i) SEQUENCE CHARACTERISTICS:
(A) LENGTH: 332 amino acids
(B) TYPE: amino acid
(D) TOPOLOGY: linear
(xi) SEQUENCE DESCRIPTION: SEQ ID NO: 460:

(2) INFORMATION FOR SEQ ID NO: 461:

        (i) SEQUENCE CHARACTERISTICS:
                (A) LENGTH: 5 amino acids
                (B) TYPE: amino acid
                (D) TOPOLOGY: linear
        (xi) SEQUENCE DESCRIPTION: SEQ ID NO: 461:

Met Leu Lys Cys Ile
  1               5

(2) INFORMATION FOR SEQ ID NO: 462:

        (i) SEQUENCE CHARACTERISTICS:
                (A) LENGTH: 14 amino acids
                (B) TYPE: amino acid
                (D) TOPOLOGY: linear
        (xi) SEQUENCE DESCRIPTION: SEQ ID NO: 462:

Met Ile Leu Thr Leu Leu Ser Val Val Ser Thr Met Ala Ser
  1               5                  10

(2) INFORMATION FOR SEQ ID NO: 463:

(i) SEQUENCE CHARACTERISTICS:
(A) LENGTH: 285 amino acids
(B) TYPE: amino acid
(D) TOPOLOGY: linear
(xi) SEQUENCE DESCRIPTION: SEQ ID NO: 463:

Met Lys Leu His Pro Pro Pro Pro Ser Pro Val Thr Gln Asp His Arg
1               5                   10                  15

Ser Lys Ser Ser His Ser Asn Trp Met Pro Arg Met Gly Ala Cys Ser
            20                  25                  30

Met Ser Arg Thr Ser Ser Ser Gly Pro Pro Ser Leu Cys Lys Ser Thr
        35                  40                  45

Ser Gly Arg Ser Cys Thr Arg Pro His Cys Trp Pro Ser Leu Pro Ala
    50                  55                  60

Trp Val Ser Val Phe Thr Arg Thr Asn Thr Gly Ser Trp Cys Tyr Pro
65                  70                  75                  80

Ala Trp Gly Gly Ala Phe Ser Arg Pro Trp Met Ser Ala Gln Ser Met
                85                  90                  95

Cys Cys Ala Glu Arg Ser Val Leu Gln Val Ala Cys Arg Leu Leu Asp
            100                 105                 110

Ala Leu Glu Phe Leu His Glu Asn Glu Tyr Val His Gly Asn Val Thr
        115                 120                 125

Ala Glu Asn Ile Phe Val Asp Pro Glu Asp Gln Ser Gln Val Thr Leu
    130                 135                 140

Ala Gly Tyr Gly Phe Ala Phe Arg Tyr Cys Pro Ser Gly Lys His Val
145                 150                 155                 160

Ala Tyr Val Glu Gly Ser Arg Ser Pro His Glu Gly Asp Leu Glu Phe
                165                 170                 175

Ile Ser Met Asp Leu His Lys Gly Cys Gly Pro Ser Arg Arg Xaa Asp
            180                 185                 190

Leu Gln Ser Leu Gly Tyr Cys Met Leu Lys Trp Leu Tyr Gly Phe Leu
        195                 200                 205

Pro Trp Thr Asn Cys Leu Pro Xaa Xaa Glu Asp Ile Met Lys Gln Lys
    210                 215                 220

Gln Lys Phe Val Asp Lys Pro Gly Pro Phe Val Gly Pro Cys Gly His
225                 230                 235                 240

Trp Ile Arg Pro Ser Glu Thr Leu Gln Lys Tyr Leu Lys Val Val Met
                245                 250                 255

Ala Leu Thr Tyr Glu Glu Lys Pro Pro Tyr Ala Met Leu Arg Asn Asn
            260                 265                 270

Leu Glu Ala Leu Leu Gln Asp Leu Arg Val Ser Pro Tyr
        275                 280                 285

(2) INFORMATION FOR SEQ ID NO: 464:

(i) SEQUENCE CHARACTERISTICS:
(A) LENGTH: 80 amino acids
(B) TYPE: amino acid
(D) TOPOLOGY: linear
(xi) SEQUENCE DESCRIPTION: SEQ ID NO: 464:

Met Thr Ser Pro Pro Pro His Gln Gly Trp Glu Gln Arg Gly Cys Gly
1               5                   10                  15

Glu Ser Gln Val Pro Leu Ala Leu Ser Arg Val Phe Ser Thr Ser His
            20                  25                  30

Tyr Cys Leu Leu Leu Val Ala Asn Gln Ser Ile Phe Phe Pro Cys Leu
        35                  40                  45

Trp Ala Val Glu Arg Leu Leu Gly Val Arg Cys Thr Cys Pro Leu Ser
    50                  55                  60

Trp Gly Lys Arg Ile Ile Ser Glu His Cys Ser Ala Gln Ser Ser Xaa
65                  70                  75                  80

(2) INFORMATION FOR SEQ ID NO: 465:

(i) SEQUENCE CHARACTERISTICS:
(A) LENGTH: 47 amino acids
(B) TYPE: amino acid
(D) TOPOLOGY: linear
(xi) SEQUENCE DESCRIPTION: SEQ ID NO: 465:

Met His Thr Trp Tyr Asn Asp Arg Arg Gln Asn Cys His Cys Leu Leu
1               5                   10                  15

Phe Phe Leu Ile Tyr Leu Arg Lys Ile Tyr Gln Val Val Pro His Val
            20                  25                  30

Pro Leu Leu Val Lys Cys Arg Gly Arg Leu Lys Gly Val Asn Ile
        35                  40                  45

(2) INFORMATION FOR SEQ ID NO: 466:

(i) SEQUENCE CHARACTERISTICS:
(A) LENGTH: 96 amino acids
(B) TYPE: amino acid
(D) TOPOLOGY: linear
(xi) SEQUENCE DESCRIPTION: SEQ ID NO: 466:

Met Glu Leu Val Leu Val Phe Leu Cys Ser Leu Leu Ala Pro Met Val
1               5                   10                  15

Leu Ala Ser Ala Ala Glu Lys Glu Lys Glu Met Asp Pro Phe His Tyr
            20                  25                  30

Asp Tyr Gln Thr Leu Arg Ile Gly Gly Leu Val Phe Ala Val Val Leu
        35                  40                  45

Phe Ser Val Gly Ile Leu Leu Ile Leu Ser Arg Arg Cys Lys Cys Ser
    50                  55                  60

Phe Asn Gln Lys Pro Arg Ala Pro Gly Asp Glu Glu Ala Gln Val Glu
65                  70                  75                  80

Asn Leu Ile Thr Ala Asn Ala Thr Glu Pro Gln Lys Ala Glu Asn Xaa
                85                  90                  95

(2) INFORMATION FOR SEQ ID NO: 467:

(i) SEQUENCE CHARACTERISTICS:
(A) LENGTH: 399 amino acids
(B) TYPE: amino acid
(D) TOPOLOGY: linear
(xi) SEQUENCE DESCRIPTION: SEQ ID NO: 467:

Met Ala Ser Gly Ala Asp Ser Lys Gly Asp Asp Leu Ser Thr Ala Ile
1               5                   10                  15

Leu Lys Gln Lys Asn Arg Pro Asn Arg Leu Ile Val Asp Glu Ala Ile
            20                  25                  30

Asn Glu Asp Asn Ser Val Val Ser Leu Ser Gln Pro Lys Met Asp Glu
        35                  40                  45

Leu Gln Leu Phe Arg Gly Asp Thr Val Leu Leu Lys Gly Lys Lys Arg
    50                  55                  60

Arg Glu Ala Val Cys Ile Val Leu Ser Asp Asp Thr Cys Ser Asp Glu
65                  70                  75                  80

Lys Ile Arg Met Asn Arg Val Val Arg Asn Asn Leu Arg Val Arg Leu
                85                  90                  95

Gly Asp Val Ile Ser Ile Gln Pro Cys Pro Asp Val Lys Tyr Gly Lys
            100                 105                 110

Arg Ile His Val Leu Pro Ile Asp Asp Thr Val Glu Gly Ile Thr Gly
        115                 120                 125

Asn Leu Phe Glu Val Tyr Leu Lys Pro Tyr Phe Leu Glu Ala Tyr Arg
    130                 135                 140

Pro Ile Arg Lys Gly Asp Ile Phe Leu Val Arg Gly Gly Met Arg Ala

145                 150                 155                 160

Val Glu Phe Lys Val Val Glu Thr Asp Pro Ser Pro Tyr Cys Ile Val
                165                 170                 175

Ala Pro Asp Thr Val Ile His Cys Glu Gly Glu Pro Ile Lys Arg Glu
            180                 185                 190

Asp Glu Glu Glu Ser Leu Asn Glu Val Gly Tyr Asp Asp Ile Gly Gly
        195                 200                 205

Cys Arg Lys Gln Leu Ala Gln Ile Lys Glu Met Val Glu Leu Pro Leu
    210                 215                 220

Arg His Pro Ala Leu Phe Lys Ala Ile Gly Val Lys Pro Pro Arg Gly
225                 230                 235                 240

Ile Leu Leu Tyr Gly Pro Pro Gly Thr Gly Lys Thr Leu Ile Ala Arg
                245                 250                 255

Ala Val Ala Asn Glu Thr Gly Ala Phe Phe Phe Leu Ile Asn Gly Pro
            260                 265                 270

Glu Ile Met Ser Lys Leu Ala Gly Glu Ser Glu Ser Asn Leu Arg Lys
        275                 280                 285

Ala Phe Glu Glu Ala Glu Lys Asn Ala Pro Ala Ile Ile Phe Ile Asp
    290                 295                 300

Glu Leu Asp Ala Ile Ala Pro Lys Arg Glu Lys Thr His Gly Glu Val
305                 310                 315                 320

Glu Arg Arg Ile Val Ser Gln Leu Leu Thr Leu Met Asp Gly Leu Lys
                325                 330                 335

Gln Arg Ala His Val Ile Val Met Ala Ala Thr Asn Arg Pro Asn Ser
            340                 345                 350

Ile Asp Pro Ala Leu Arg Arg Phe Gly Arg Phe Asp Arg Glu Val Asp
        355                 360                 365

Ile Gly Ile Pro Asp Ala Thr Gly Arg Leu Glu Ile Leu Gln Ile His
    370                 375                 380

Thr Lys Asn Met Lys Leu Ala Asp Asp Val Asp Leu Glu Gln Xaa
385                 390                 395

(2) INFORMATION FOR SEQ ID NO: 468:

(i) SEQUENCE CHARACTERISTICS:
(A) LENGTH: 1 amino acids
(B) TYPE: amino acid
(D) TOPOLOGY: linear
(xi) SEQUENCE DESCRIPTION: SEQ ID NO: 468:

Leu
1

(2) INFORMATION FOR SEQ ID NO: 469:

(i) SEQUENCE CHARACTERISTICS:
(A) LENGTH: 273 amino acids
(B) TYPE: amino acid
(D) TOPOLOGY: linear
(xi) SEQUENCE DESCRIPTION: SEQ ID NO: 469:

Met Ala Ala Pro Lys Gly Ser Leu Trp Val Arg Thr Gln Leu Gly Leu
1               5                   10                  15

Pro Pro Leu Leu Leu Leu Thr Met Ala Leu Ala Gly Gly Ser Gly Thr
            20                  25                  30

Ala Ser Ala Glu Ala Phe Asp Ser Val Leu Gly Asp Thr Ala Ser Cys
        35                  40                  45

His Arg Ala Cys Gln Leu Thr Tyr Pro Leu His Thr Tyr Pro Lys Glu
    50                  55                  60

Glu Glu Leu Tyr Ala Cys Gln Arg Gly Cys Arg Leu Phe Ser Ile Cys
65                  70                  75                  80

Gln Phe Val Asp Asp Gly Ile Asp Leu Asn Arg Thr Lys Leu Glu Cys
                85                  90                  95

Glu Ser Ala Cys Thr Glu Ala Tyr Ser Gln Ser Asp Glu Gln Tyr Ala
            100                 105                 110

Cys His Leu Gly Cys Gln Asn Gln Leu Pro Phe Ala Glu Leu Arg Gln
        115                 120                 125

Glu Gln Leu Met Ser Leu Met Pro Lys Met His Leu Leu Phe Pro Leu
    130                 135                 140

Thr Leu Val Arg Ser Phe Trp Ser Asp Met Met Asp Ser Ala Gln Ser
145                 150                 155                 160

Phe Ile Thr Ser Ser Trp Thr Phe Tyr Leu Gln Ala Asp Asp Gly Lys
                165                 170                 175

Ile Val Ile Phe Xaa Ser Lys Pro Arg Asn Pro Arg Tyr Ala Pro His
            180                 185                 190

Leu Glu Pro Gly Ala Leu Pro Asn Leu Xaa Xaa Xaa Ser Leu Ser Lys
        195                 200                 205

Met Ser Xaa Xaa Ser Xaa Met Arg Asn Ser Gln Ala His Arg Asn Phe
    210                 215                 220

Leu Glu Asp Gly Glu Ser Asp Gly Phe Leu Arg Cys Leu Ser Leu Asn
225                 230                 235                 240

Ser Gly Trp Ile Leu Thr Thr Thr Leu Val Leu Ser Val Met Val Leu
                245                 250                 255

Leu Trp Ile Cys Cys Ala Thr Cys Cys Tyr Thr Leu Leu Asp Ala Val
            260                 265                 270

Xaa

(2) INFORMATION FOR SEQ ID NO: 470:

(i) SEQUENCE CHARACTERISTICS:
(A) LENGTH: 192 amino acids
(B) TYPE: amino acid
(D) TOPOLOGY: linear
(xi) SEQUENCE DESCRIPTION: SEQ ID NO: 470:

Met Met Val Leu Ser Leu Gly Ile Ile Leu Ala Ser Ala Ser Phe Ser
1               5                   10                  15

Pro Asn Phe Thr Gln Val Thr Ser Thr Leu Leu Asn Ser Ala Tyr Pro
            20                  25                  30

Phe Ile Gly Pro Phe Phe Phe Ile Ile Ser Gly Ser Leu Ser Ile Ala
        35                  40                  45

Thr Glu Lys Arg Leu Thr Lys Leu Leu Val His Ser Ser Leu Val Gly
    50                  55                  60

Ser Ile Leu Ser Ala Leu Ser Ala Leu Val Gly Phe Ile Ile Leu Ser
65                  70                  75                  80

Val Lys Gln Ala Thr Leu Asn Pro Ala Ser Leu Gln Cys Glu Leu Asp
                85                  90                  95

Lys Asn Asn Ile Pro Thr Arg Ser Tyr Val Ser Tyr Phe Tyr His Asp
            100                 105                 110

Ser Leu Tyr Thr Thr Asp Cys Tyr Thr Ala Lys Ala Ser Leu Ala Gly
        115                 120                 125

Xaa Leu Ser Leu Met Leu Ile Cys Thr Leu Leu Glu Phe Cys Leu Ala
    130                 135                 140

Val Leu Thr Ala Val Leu Arg Trp Lys Gln Ala Tyr Ser Asp Phe Pro
145                 150                 155                 160

Gly Ser Val Leu Phe Leu Pro His Ser Tyr Ile Gly Asn Ser Gly Met
                165                 170                 175

Ser Ser Lys Met Thr His Asp Cys Gly Tyr Glu Glu Leu Leu Thr Ser
            180                 185                 190

(2) INFORMATION FOR SEQ ID NO: 471:

(i) SEQUENCE CHARACTERISTICS:
(A) LENGTH: 234 amino acids
(B) TYPE: amino acid

(D) TOPOLOGY: linear
(xi) SEQUENCE DESCRIPTION: SEQ ID NO: 471:

Met Arg Lys Thr Arg Leu Trp Gly Leu Leu Trp Met Leu Phe Val Ser
1               5                   10                  15

Glu Leu Arg Ala Ala Thr Lys Leu Thr Glu Glu Lys Tyr Glu Leu Lys
            20                  25                  30

Glu Gly Gln Thr Leu Asp Val Lys Cys Asp Tyr Thr Leu Glu Lys Phe
        35                  40                  45

Ala Ser Ser Gln Lys Ala Trp Gln Ile Ile Arg Asp Gly Glu Met Pro
    50                  55                  60

Lys Thr Leu Ala Cys Thr Glu Arg Pro Ser Lys Asn Ser His Pro Val
65                  70                  75                  80

Gln Val Gly Arg Ile Ile Leu Glu Asp Tyr His Asp His Gly Leu Leu
                85                  90                  95

Arg Val Arg Met Val Asn Leu Gln Val Glu Asp Ser Gly Leu Tyr Gln
            100                 105                 110

Cys Val Ile Tyr Gln Pro Pro Lys Glu Pro His Met Leu Phe Asp Arg
        115                 120                 125

Ile Arg Leu Val Val Thr Lys Gly Phe Ser Gly Thr Pro Gly Ser Asn
    130                 135                 140

Glu Asn Ser Thr Gln Asn Val Tyr Lys Ile Pro Pro Thr Thr Thr Lys
145                 150                 155                 160

Ala Leu Cys Pro Leu Tyr Thr Ser Pro Arg Thr Val Thr Gln Ala Pro
                165                 170                 175

Pro Lys Ser Thr Ala Asp Val Ser Thr Pro Asp Ser Glu Ile Asn Leu
            180                 185                 190

Thr Asn Val Thr Asp Ile Ile Arg Val Pro Val Phe Asn Ile Val Ile
        195                 200                 205

Leu Leu Ala Gly Gly Phe Leu Ser Lys Ser Leu Val Phe Ser Val Leu
    210                 215                 220

Phe Ala Val Thr Leu Arg Ser Phe Val Pro
225                 230

(2) INFORMATION FOR SEQ ID NO: 472:

(i) SEQUENCE CHARACTERISTICS:
(A) LENGTH: 105 amino acids
(B) TYPE: amino acid
(D) TOPOLOGY: linear
(xi) SEQUENCE DESCRIPTION: SEQ ID NO: 472:

Met Leu His Ile Leu Pro Leu Lys Ser Tyr Asp Phe Pro His Phe Ser
1               5                   10                  15

Leu Met Gly Arg Tyr Arg Cys Ala Ser Leu Leu Phe Cys Phe Leu Leu
            20                  25                  30

Leu Phe Phe Phe Phe Cys Ser Val Leu Trp Thr Phe Ser Asp Met His
        35                  40                  45

Arg Ser Gly Glu Asp Gly Pro Trp Thr Pro Cys Val His His Leu Ala
    50                  55                  60

Ala Ser Leu Ile Ser Tyr Gly Gln Pro Gly Phe Ile Cys Ile Ser Leu
65                  70                  75                  80

Phe Ser Pro Val Leu Phe Ile Glu Asn Pro Arg His Tyr Ala Asn Ala
                85                  90                  95

Thr Val Thr Thr Leu Gly Asp Trp Xaa
            100                 105

(2) INFORMATION FOR SEQ ID NO: 473:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 473:

Met Val Phe Leu Lys Tyr Arg Phe Leu Phe Phe Leu Val Phe Leu Ala
1               5                   10                  15

Asn Cys Ile Tyr Ser Leu His Tyr Lys Pro Ser Leu Met Tyr Pro Lys
            20                  25                  30

(2) INFORMATION FOR SEQ ID NO: 474:

(i) SEQUENCE CHARACTERISTICS:
(A) LENGTH: 571 amino acids
(B) TYPE: amino acid
(D) TOPOLOGY: linear
(xi) SEQUENCE DESCRIPTION: SEQ ID NO: 474:

Met Ala Leu Ser Arg Gly Leu Pro Arg Glu Leu Ala Glu Ala Val Ala
1               5                   10                  15

Gly Gly Arg Val Leu Val Val Gly Ala Gly Gly Ile Gly Cys Glu Leu
            20                  25                  30

Leu Lys Asn Leu Val Leu Thr Gly Phe Ser His Ile Asp Leu Ile Asp
        35                  40                  45

Leu Asp Thr Ile Asp Val Ser Asn Leu Asn Arg Gln Phe Leu Phe Gln
    50                  55                  60

Lys Lys His Val Gly Arg Ser Lys Ala Gln Val Ala Lys Glu Ser Val
65                  70                  75                  80

Leu Gln Phe Tyr Pro Lys Ala Asn Ile Val Ala Tyr His Asp Ser Ile
                85                  90                  95

Met Asn Pro Asp Tyr Asn Val Glu Phe Phe Arg Gln Phe Ile Leu Val
            100                 105                 110

Met Asn Ala Leu Asp Asn Arg Ala Ala Arg Asn His Val Asn Arg Met
        115                 120                 125

Cys Leu Ala Ala Asp Val Pro Leu Ile Glu Ser Gly Thr Ala Gly Tyr
    130                 135                 140

Leu Gly Gln Val Thr Thr Ile Lys Lys Gly Val Thr Glu Cys Tyr Glu
145                 150                 155                 160

Cys His Pro Lys Pro Thr Gln Arg Thr Phe Pro Gly Cys Thr Ile Arg
                165                 170                 175

Asn Thr Pro Ser Glu Pro Ile His Cys Ile Val Trp Ala Lys Tyr Leu
            180                 185                 190

Phe Asn Gln Leu Phe Gly Glu Glu Asp Ala Asp Gln Glu Val Ser Pro
        195                 200                 205

Asp Arg Ala Asp Pro Glu Ala Ala Trp Glu Pro Thr Glu Ala Glu Ala
    210                 215                 220

Arg Ala Arg Ala Ser Asn Glu Asp Gly Asp Ile Lys Arg Ile Ser Thr
225                 230                 235                 240

Lys Glu Trp Ala Lys Ser Thr Gly Tyr Asp Pro Val Lys Leu Phe Thr
                245                 250                 255

Lys Leu Phe Lys Asp Asp Ile Arg Tyr Leu Leu Thr Met Asp Lys Leu
            260                 265                 270

Trp Arg Lys Arg Lys Pro Pro Val Pro Leu Asp Trp Ala Glu Val Gln
        275                 280                 285

Ser Gln Gly Glu Glu Thr Asn Ala Ser Asp Gln Gln Asn Glu Pro Gln
    290                 295                 300

Leu Gly Leu Lys Asp Gln Gln Val Leu Asp Val Lys Ser Tyr Ala Arg
305                 310                 315                 320

Leu Phe Ser Lys Ser Ile Glu Thr Leu Arg Val His Leu Ala Glu Lys
                325                 330                 335

Gly Asp Gly Ala Glu Leu Ile Trp Asp Lys Asp Asp Pro Ser Ala Met
            340                 345                 350

Asp Phe Val Thr Ser Ala Ala Asn Leu Arg Met His Ile Phe Ser Met
        355                 360                 365

Asn Met Lys Ser Arg Phe Asp Ile Lys Ser Met Ala Gly Asn Ile Ile
    370                 375                 380

Pro Ala Ile Ala Thr Thr Asn Ala Val Ile Ala Gly Leu Ile Val Leu
385                 390                 395                 400

Glu Gly Leu Lys Ile Leu Ser Gly Lys Ile Asp Gln Cys Arg Thr Ile
                405                 410                 415

Phe Leu Asn Lys Gln Pro Asn Pro Arg Lys Lys Leu Leu Val Pro Cys
            420                 425                 430

Ala Leu Asp Pro Pro Asn Pro Asn Cys Tyr Val Cys Ala Ser Lys Pro
        435                 440                 445

Glu Val Thr Val Arg Leu Asn Val His Lys Val Thr Val Leu Thr Leu
    450                 455                 460

Gln Asp Lys Ile Val Lys Glu Lys Phe Ala Met Val Ala Pro Asp Val
465                 470                 475                 480

Gln Ile Glu Asp Gly Lys Gly Thr Ile Leu Ile Ser Ser Glu Glu Gly
                485                 490                 495

Glu Thr Glu Ala Asn Asn His Lys Lys Leu Ser Glu Phe Gly Ile Arg
            500                 505                 510

Asn Gly Ser Arg Leu Gln Ala Asp Asp Phe Leu Gln Asp Tyr Thr Leu
        515                 520                 525

Leu Ile Asn Ile Leu His Ser Glu Asp Leu Gly Lys Asp Val Glu Phe
    530                 535                 540

Glu Val Val Gly Asp Ala Pro Glu Lys Val Gly Xaa Lys Gln Ala Glu
545                 550                 555                 560

Asp Ala Ala Lys Ser Ile Thr Asn Gly Gln Xaa
                565                 570

(2) INFORMATION FOR SEQ ID NO: 475:

(i) SEQUENCE CHARACTERISTICS:
(A) LENGTH: 312 amino acids
(B) TYPE: amino acid
(D) TOPOLOGY: linear
(xi) SEQUENCE DESCRIPTION: SEQ ID NO: 475:

Met Gln Val Val Thr Cys Leu Thr Arg Asp Ser Tyr Leu Thr His Cys
  1               5                  10                  15

Phe Leu Gln His Leu Met Val Val Leu Ser Ser Leu Glu Arg Thr Pro
             20                  25                  30

Ser Pro Glu Pro Val Asp Lys Asp Phe Tyr Ser Glu Phe Gly Asn Lys
         35                  40                  45

Thr Thr Gly Lys Met Glu Asn Tyr Glu Leu Ile His Ser Ser Arg Val
     50                  55                  60

Lys Phe Thr Tyr Pro Ser Glu Glu Glu Ile Gly Asp Leu Thr Phe Thr
 65                  70                  75                  80

Val Ala Gln Lys Met Ala Glu Pro Glu Lys Ala Pro Ala Leu Ser Ile
                85                  90                  95

Leu Leu Tyr Val Gln Ala Phe Gln Val Gly Met Pro Pro Pro Gly Cys
            100                 105                 110

Cys Arg Gly Pro Leu Arg Pro Lys Thr Leu Leu Leu Thr Ser Ser Glu
        115                 120                 125

Ile Phe Leu Leu Asp Glu Asp Cys Val His Tyr Pro Leu Pro Glu Phe
    130                 135                 140

Ala Lys Glu Pro Pro Gln Arg Asp Arg Tyr Arg Leu Asp Asp Gly Arg
145                 150                 155                 160

Arg Val Arg Asp Leu Asp Arg Val Leu Met Gly Tyr Gln Thr Tyr Pro
                165                 170                 175

Gln Pro Ser Pro Ser Ser Ser Met Thr Cys Lys Val Met Thr Ser Trp
            180                 185                 190

Ala Val Ser Pro Trp Thr Thr Leu Gly Arg Cys Gln Val Ala Arg Leu
        195                 200                 205

Glu Pro Ala Arg Ala Val Lys Ser Ser Gly Arg Cys Leu Ser Pro Val
    210                 215                 220

Leu Arg Ala Glu Arg Ser Ser Ser Arg Cys Trp Leu Ala Ser Gly Arg
225                 230                 235                 240

Pro Cys Val Ala Val Ser Cys Leu Ser Ser Ser Pro Ala Ser Pro Gly
                245                 250                 255

His Ser Gln Pro Val Val Ser Ser Leu Thr Pro Thr Gly Ala Gly Gln
            260                 265                 270

Gln Ala Phe Val Phe Ser Lys Asn Val Leu Ser Ser Leu Trp Tyr Leu
        275                 280                 285

Asn Leu Thr Val Leu Ala Glu Asn Val Asn Met Cys Val Cys Cys Val
    290                 295                 300

Asn Ser Phe Ser Cys Trp Glu Xaa
305                 310

(2) INFORMATION FOR SEQ ID NO: 476:

        (i) SEQUENCE CHARACTERISTICS:
                (A) LENGTH: 329 amino acids
                (B) TYPE: amino acid
                (D) TOPOLOGY: linear
        (xi) SEQUENCE DESCRIPTION: SEQ ID NO: 476:

Met Ala Gln His His Leu Trp Ile Leu Leu Leu Cys Leu Gln Thr Trp
  1               5                  10                  15

Pro Glu Ala Ala Gly Lys Asp Ser Glu Ile Phe Thr Val Asn Gly Ile

            20                  25                  30

Leu Gly Glu Ser Val Thr Phe Pro Val Asn Ile Gln Glu Pro Arg Gln
        35                  40                  45

Val Lys Ile Ile Ala Trp Thr Ser Lys Thr Ser Val Ala Tyr Val Thr
    50                  55                  60

Pro Gly Asp Ser Glu Thr Ala Pro Val Val Thr Val Thr His Arg Asn
65                  70                  75                  80

Tyr Tyr Glu Arg Ile His Ala Leu Gly Pro Asn Tyr Asn Leu Val Ile
                85                  90                  95

Ser Asp Leu Arg Met Glu Asp Ala Gly Asp Tyr Lys Ala Asp Ile Asn
            100                 105                 110

Thr Gln Ala Asp Pro Tyr Thr Thr Thr Lys Arg Tyr Asn Leu Gln Ile
        115                 120                 125

Tyr Arg Arg Leu Gly Lys Pro Lys Ile Thr Gln Ser Leu Met Ala Ser
    130                 135                 140

Val Asn Ser Thr Cys Asn Val Thr Leu Thr Cys Ser Val Glu Lys Glu
145                 150                 155                 160

Glu Lys Asn Val Thr Tyr Asn Trp Ser Pro Leu Gly Glu Glu Gly Asn
                165                 170                 175

Val Leu Gln Ile Phe Gln Thr Pro Glu Asp Gln Glu Leu Thr Tyr Thr
            180                 185                 190

Cys Thr Ala Gln Asn Pro Val Ser Asn Asn Ser Asp Ser Ile Ser Ala
        195                 200                 205

Arg Gln Leu Cys Ala Asp Ile Ala Met Gly Phe Arg Thr His His Thr
    210                 215                 220

Gly Leu Leu Ser Val Leu Ala Met Phe Phe Leu Leu Val Leu Ile Leu
225                 230                 235                 240

Ser Ser Val Phe Leu Phe Arg Leu Phe Lys Arg Arg Gln Asp Ala Ala
                245                 250                 255

Ser Lys Lys Thr Ile Tyr Thr Tyr Ile Met Ala Ser Arg Asn Thr Gln
            260                 265                 270

Pro Ala Glu Ser Arg Ile Tyr Asp Glu Ile Leu Gln Ser Lys Val Leu
        275                 280                 285

Pro Ser Lys Glu Glu Pro Val Asn Thr Val Tyr Ser Glu Val Gln Phe
    290                 295                 300

Ala Asp Lys Met Gly Lys Ala Ser Thr Gln Asp Ser Lys Pro Pro Gly
305                 310                 315                 320

Thr Ser Ser Tyr Glu Ile Val Ile Xaa
                325

(2) INFORMATION FOR SEQ ID NO: 477:

(i) SEQUENCE CHARACTERISTICS:
(A) LENGTH: 178 amino acids
(B) TYPE: amino acid
(D) TOPOLOGY: linear
(xi) SEQUENCE DESCRIPTION: SEQ ID NO: 477:

Met Lys Leu Gln Cys Val Ser Leu Trp Leu Leu Gly Thr Ile Leu Ile
1               5                   10                  15

Leu Cys Ser Val Asp Asn His Gly Leu Arg Arg Cys Leu Ile Ser Thr
            20                  25                  30

Asp Met His His Ile Glu Glu Ser Phe Gln Glu Ile Lys Arg Ala Ile
        35                  40                  45

Gln Ala Lys Asp Thr Phe Pro Asn Val Thr Ile Leu Ser Thr Leu Glu
    50                  55                  60

Thr Leu Gln Ile Ile Lys Pro Leu Asp Val Cys Cys Val Thr Lys Asn
65                  70                  75                  80

Leu Leu Ala Phe Tyr Val Asp Arg Val Phe Lys Asp His Gln Glu Pro
                85                  90                  95

Asn Pro Lys Ile Leu Arg Lys Ile Ser Ser Ile Ala Asn Ser Phe Leu
            100                 105                 110

Tyr Met Gln Lys Thr Leu Arg Gln Cys Gln Glu Gln Arg Gln Cys His
        115                 120                 125

Cys Arg Gln Glu Ala Thr Asn Ala Thr Arg Val Ile His Asp Asn Tyr
    130                 135                 140

Asp Gln Leu Glu Val His Ala Ala Ala Ile Lys Ser Leu Gly Glu Leu
145                 150                 155                 160

Asp Val Phe Leu Ala Trp Ile Asn Lys Asn His Glu Val Met Ser Ser
                165                 170                 175

Ala Xaa

(2) INFORMATION FOR SEQ ID NO: 478:

(i) SEQUENCE CHARACTERISTICS:
(A) LENGTH: 52 amino acids
(B) TYPE: amino acid
(D) TOPOLOGY: linear
(xi) SEQUENCE DESCRIPTION: SEQ ID NO: 478:

Asp Thr Ala Ile Arg Val Ala Leu Ala Val Ala Val Leu Lys Thr Val
1               5                   10                  15

Ile Leu Gly Leu Leu Cys Leu Leu Leu Cys Gly Gly Gly Glu Gly Lys
            20                  25                  30

Val Ala Gly Arg Gln Ala Val Thr Ser Asp Gln Gln Ser Val Gly Arg
        35                  40                  45

Arg Asp Val Tyr
    50

(2) INFORMATION FOR SEQ ID NO: 479:

        (i) SEQUENCE CHARACTERISTICS:
                (A) LENGTH: 62 amino acids
                (B) TYPE: amino acid
                (D) TOPOLOGY: linear
        (xi) SEQUENCE DESCRIPTION: SEQ ID NO: 479:

Met Gln Lys Lys Asn Ser Leu Phe Phe Phe Phe Ala Phe Tyr Tyr Glu
  1               5                  10                  15

Asn Lys Thr Asn Ala Pro Gly Glu Gly Ser Met Ile Thr Arg Asn Ile
            20                  25                  30

Lys Glu Tyr Phe Leu Pro Phe Leu Phe Cys Cys Val Glu Ala Ser Ile
        35                  40                  45

Ala Ile Asn Lys Leu Asn Tyr Leu His Trp Thr His Phe Gln
    50                  55                  60

(2) INFORMATION FOR SEQ ID NO: 480:

        (i) SEQUENCE CHARACTERISTICS:
                (A) LENGTH: 27 amino acids
                (B) TYPE: amino acid
                (D) TOPOLOGY: linear
        (xi) SEQUENCE DESCRIPTION: SEQ ID NO: 480:

Met Pro Gly Leu Ser Leu Ile Leu Thr Val Thr Leu Leu Ala Val Ser
  1               5                  10                  15

Asp Ser Ala Ala Thr Cys Ile Val Ala Lys Gly
            20                  25

(2) INFORMATION FOR SEQ ID NO: 481:

        (i) SEQUENCE CHARACTERISTICS:
                (A) LENGTH: 339 amino acids
                (B) TYPE: amino acid
                (D) TOPOLOGY: linear
        (xi) SEQUENCE DESCRIPTION: SEQ ID NO: 481:

Met Ser Gly Pro Asp Val Glu Thr Pro Ser Ala Ile Gln Ile Cys Arg
  1               5                  10                  15

Ile Met Arg Pro Asp Asp Ala Asn Val Ala Gly Asn Val His Gly Gly
            20                  25                  30

Thr Ile Leu Lys Met Ile Glu Glu Ala Gly Ala Ile Ile Ser Thr Arg
        35                  40                  45

His Cys Asn Ser Gln Asn Gly Glu Arg Cys Val Ala Ala Leu Ala Arg
    50                  55                  60

Val Glu Arg Thr Asp Phe Leu Ser Pro Met Cys Ile Gly Glu Val Ala
65                  70                  75                  80

His Val Ser Ala Glu Ile Thr Tyr Thr Ser Lys His Ser Val Glu Val
                85                  90                  95

Gln Val Asn Val Met Ser Glu Asn Ile Leu Thr Gly Ala Lys Lys Leu
            100                 105                 110

Thr Asn Lys Ala Thr Leu Trp Tyr Val Pro Leu Ser Leu Lys Asn Val
        115                 120                 125

Asp Lys Val Leu Glu Val Pro Pro Val Val Tyr Ser Arg Xaa Glu Gln
    130                 135                 140

Glu Glu Glu Gly Arg Lys Arg Tyr Glu Ala Gln Lys Leu Glu Arg Met
145                 150                 155                 160

Glu Thr Lys Trp Arg Asn Gly Asp Ile Val Gln Pro Val Leu Asn Pro
                165                 170                 175

Glu Pro Asn Thr Val Ser Tyr Ser Gln Ser Ser Leu Ile His Leu Val
            180                 185                 190

Gly Pro Ser Asp Cys Thr Leu His Gly Phe Val His Gly Gly Val Thr
        195                 200                 205

Met Lys Leu Met Asp Glu Val Ala Gly Ile Val Ala Ala Arg His Cys
    210                 215                 220

Lys Thr Asn Ile Val Thr Ala Ser Val Asp Ala Ile Asn Phe His Asp
225                 230                 235                 240

Lys Ile Arg Lys Gly Cys Val Ile Thr Ile Ser Gly Arg Met Thr Phe
                245                 250                 255

Thr Ser Asn Lys Ser Met Glu Ile Glu Val Leu Val Asp Ala Asp Pro
            260                 265                 270

Val Val Asp Ser Ser Gln Lys Arg Tyr Arg Ala Ala Ser Ala Phe Phe
        275                 280                 285

Thr Tyr Val Ser Leu Ser Gln Glu Gly Arg Ser Leu Pro Val Pro Gln
    290                 295                 300

Leu Val Pro Glu Thr Glu Asp Glu Lys Lys Arg Phe Glu Glu Gly Lys
305                 310                 315                 320

Gly Arg Tyr Leu Gln Met Lys Ala Lys Xaa Gln Gly His Ala Xaa Xaa
                325                 330                 335

Gln Pro Xaa

(2) INFORMATION FOR SEQ ID NO: 482:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 482:

Met Leu Asn Ser Asn Ile Asn Asp Leu Leu Met Val Thr Tyr Leu Ala
1               5                   10                  15

Asn Leu Thr Gln Ser Gln Ile Ala Leu Asn Glu Lys Leu Val Asn Leu
            20                  25                  30

(2) INFORMATION FOR SEQ ID NO: 483:

(i) SEQUENCE CHARACTERISTICS:
(A) LENGTH: 48 amino acids
(B) TYPE: amino acid
(D) TOPOLOGY: linear
(xi) SEQUENCE DESCRIPTION: SEQ ID NO: 483:

Met Arg Glu Thr Ser Ile Arg Val Leu Leu Met Leu Pro Ala Leu Glu
1               5                   10                  15

Ser Thr Ser Gly Leu Ser Ala Phe Met Gly Leu Gly Thr Arg Ile Gly
            20                  25                  30

Cys Phe Lys Thr Ile Thr Cys Trp Pro Thr Ser Leu Thr Gln Arg Xaa
        35                  40                  45

(2) INFORMATION FOR SEQ ID NO: 484:

(i) SEQUENCE CHARACTERISTICS:
(A) LENGTH: 38 amino acids
(B) TYPE: amino acid
(D) TOPOLOGY: linear
(xi) SEQUENCE DESCRIPTION: SEQ ID NO: 484:

Met Tyr Met Tyr Ser Leu Asn Val Phe Leu Ser Phe Ile Phe Leu Ala
1               5                   10                  15

Leu Val Phe Lys Cys Val His Val Cys Gln Gly Ala Asn Ala Phe Leu
            20                  25                  30

Phe Leu Lys Leu Val Phe
        35

(2) INFORMATION FOR SEQ ID NO: 485:

(i) SEQUENCE CHARACTERISTICS:
(A) LENGTH: 61 amino acids
(B) TYPE: amino acid
(D) TOPOLOGY: linear
(xi) SEQUENCE DESCRIPTION: SEQ ID NO: 485:

Met Gly Leu Arg Leu Ile Cys Leu Glu Leu Thr Met Val Lys Ala Leu
1               5                   10                  15

Val Cys Glu Met Phe Leu Phe Phe Leu Met Thr Gln Lys Leu Ile Trp
            20                  25                  30

Gln Glu Cys Thr Glu Lys Phe Ala Lys Leu Leu Val Gln Leu Ile Ser
        35                  40                  45

Leu Val Phe Ala Trp Glu Phe Phe Ser Glu Asp Thr Pro
    50                  55                  60

(2) INFORMATION FOR SEQ ID NO: 486:

(i) SEQUENCE CHARACTERISTICS:
(A) LENGTH: 346 amino acids
(B) TYPE: amino acid
(D) TOPOLOGY: linear
(xi) SEQUENCE DESCRIPTION: SEQ ID NO: 486:

Met Leu Ala Ala Arg Leu Val Cys Leu Arg Thr Leu Pro Ser Arg Val
1               5                   10                  15

Phe His Pro Ala Phe Thr Lys Ala Ser Pro Val Val Lys Asn Ser Ile
            20                  25                  30

Thr Lys Asn Gln Trp Leu Leu Thr Pro Ser Arg Glu Tyr Ala Thr Lys
        35                  40                  45

Thr Arg Ile Gly Ile Arg Arg Gly Arg Thr Gly Gln Glu Leu Lys Glu
    50                  55                  60

Ala Ala Leu Glu Pro Ser Met Glu Lys Ile Phe Lys Ile Asp Gln Met
65                  70                  75                  80

Gly Arg Trp Phe Val Ala Gly Gly Ala Ala Val Gly Leu Gly Ala Leu
                85                  90                  95

Cys Tyr Tyr Gly Leu Gly Leu Ser Asn Glu Ile Gly Ala Ile Glu Lys
            100                 105                 110

Ala Val Ile Trp Pro Gln Tyr Val Lys Asp Arg Ile His Ser Thr Tyr
        115                 120                 125

Met Tyr Leu Ala Gly Ser Ile Gly Leu Thr Ala Leu Ser Ala Ile Ala
    130                 135                 140

Ile Ser Arg Thr Pro Val Leu Met Asn Phe Met Met Arg Gly Ser Trp
145                 150                 155                 160

Val Thr Ile Gly Val Thr Phe Ala Ala Met Val Gly Ala Gly Met Leu
                165                 170                 175

Val Arg Ser Ile Pro Tyr Asp Gln Ser Pro Gly Pro Lys His Leu Ala
            180                 185                 190

Trp Leu Leu His Ser Gly Val Met Gly Ala Val Val Ala Pro Leu Thr
        195                 200                 205

Ile Leu Gly Gly Pro Leu Leu Ile Arg Ala Ala Trp Tyr Thr Ala Gly
    210                 215                 220

Ile Val Gly Gly Leu Ser Thr Val Ala Met Cys Ala Pro Ser Glu Lys
225                 230                 235                 240

Phe Leu Asn Met Gly Ala Pro Leu Gly Val Gly Leu Gly Leu Val Phe
                245                 250                 255

Val Ser Ser Leu Gly Ser Met Phe Leu Pro Pro Thr Thr Val Ala Gly
            260                 265                 270

Ala Thr Leu Tyr Ser Val Ala Met Tyr Gly Gly Leu Val Leu Phe Ser
        275                 280                 285

Met Phe Leu Leu Tyr Asp Thr Gln Lys Val Ile Lys Arg Ala Glu Val
    290                 295                 300

Ser Pro Met Tyr Gly Val Gln Lys Tyr Asp Pro Ile Asn Ser Met Leu
305                 310                 315                 320

Ser Ile Tyr Met Asp Thr Leu Asn Ile Phe Met Arg Val Ala Thr Met
                325                 330                 335

Leu Ala Thr Gly Gly Asn Arg Lys Lys Xaa
            340                 345

(2) INFORMATION FOR SEQ ID NO: 487:

(i) SEQUENCE CHARACTERISTICS:
(A) LENGTH: 237 amino acids
(B) TYPE: amino acid
(D) TOPOLOGY: linear
(xi) SEQUENCE DESCRIPTION: SEQ ID NO: 487:

Met Glu Glu Val Leu Leu Leu Gly Leu Lys Asp Arg Glu Gly Tyr Thr
  1               5                  10                  15

Ser Phe Trp Asn Asp Cys Ile Ser Ser Gly Leu Arg Gly Cys Met Leu
             20                  25                  30

Ile Glu Leu Ala Leu Arg Gly Arg Leu Gln Leu Glu Ala Cys Gly Met
         35                  40                  45

Arg Arg Lys Ser Leu Leu Thr Arg Lys Val Ile Cys Lys Ser Asp Ala
     50                  55                  60

Pro Thr Gly Asp Val Leu Leu Asp Glu Ala Leu Lys His Val Lys Glu
65                  70                  75                  80

Thr Gln Pro Pro Glu Thr Val Gln Asn Trp Ile Glu Leu Leu Ser Gly
                85                  90                  95

Glu Thr Trp Asn Pro Leu Lys Leu His Tyr Gln Leu Arg Asn Val Arg
            100                 105                 110

Glu Arg Leu Ala Lys Asn Leu Val Glu Lys Gly Val Leu Thr Thr Glu
        115                 120                 125

Lys Gln Asn Phe Leu Leu Phe Asp Met Thr Thr His Pro Leu Thr Asn
    130                 135                 140

Asn Asn Ile Lys Gln Arg Leu Ile Lys Lys Val Gln Glu Ala Val Leu
145                 150                 155                 160

Asp Lys Trp Val Asn Asp Pro His Arg Met Asp Arg Arg Leu Leu Ala
                165                 170                 175

Leu Ile Tyr Leu Ala His Ala Ser Asp Val Leu Glu Asn Ala Phe Ala
            180                 185                 190

Pro Leu Leu Asp Glu Gln Tyr Asp Leu Ala Thr Lys Arg Val Arg Gln
        195                 200                 205

Leu Leu Asp Leu Asp Pro Glu Val Glu Cys Leu Lys Ala Asn Thr Asn
    210                 215                 220

Glu Val Leu Trp Ala Val Val Ala Ala Phe Thr Lys Xaa
225                 230                 235

(2) INFORMATION FOR SEQ ID NO: 488:

     (i) SEQUENCE CHARACTERISTICS:
          (A) LENGTH: 200 amino acids
          (B) TYPE: amino acid
          (D) TOPOLOGY: linear
     (xi) SEQUENCE DESCRIPTION: SEQ ID NO: 488:

Met Ala Gln Arg Met Val Trp Val Asp Leu Glu Met Thr Gly Leu Asp
1               5                   10                  15

Ile Glu Lys Asp Gln Ile Ile Glu Met Ala Cys Leu Ile Thr Asp Ser
            20                  25                  30

Asp Leu Asn Ile Leu Ala Glu Gly Pro Asn Leu Ile Ile Lys Gln Pro
        35                  40                  45

Asp Glu Leu Leu Asp Ser Met Ser Asp Trp Cys Lys Glu His His Gly
    50                  55                  60

Lys Ser Gly Leu Thr Lys Ala Val Lys Glu Ser Thr Ile Thr Leu Gln
65                  70                  75                  80

Gln Ala Glu Tyr Glu Phe Leu Ser Phe Val Arg Gln Gln Thr Pro Pro

                85                  90                  95

Gly Leu Cys Pro Leu Ala Gly Asn Ser Val His Glu Asp Lys Lys Phe
            100                 105                 110

Leu Asp Lys Tyr Met Pro Gln Phe Met Lys His Leu His Tyr Arg Ile
        115                 120                 125

Ile Asp Val Ser Thr Val Lys Glu Leu Cys Arg Arg Trp Tyr Pro Glu
    130                 135                 140

Glu Tyr Glu Phe Ala Pro Lys Lys Ala Ala Ser His Arg Ala Leu Asp
145                 150                 155                 160

Asp Ile Ser Glu Ser Ile Lys Glu Leu Gln Phe Tyr Arg Asn Asn Ile
                165                 170                 175

Phe Lys Lys Lys Ile Asp Glu Lys Lys Arg Lys Ile Ile Glu Asn Gly
            180                 185                 190

Glu Asn Glu Lys Thr Val Ser Xaa
        195                 200

(2) INFORMATION FOR SEQ ID NO: 489:

(i) SEQUENCE CHARACTERISTICS:
(A) LENGTH: 351 amino acids
(B) TYPE: amino acid
(D) TOPOLOGY: linear
(xi) SEQUENCE DESCRIPTION: SEQ ID NO: 489:

Met Ala Thr Thr Ala Ala Pro Ala Gly Gly Ala Arg Asn Gly Ala Gly
1               5                   10                  15

Pro Glu Trp Gly Gly Phe Glu Glu Asn Ile Gln Gly Gly Gly Ser Ala
            20                  25                  30

Val Ile Asp Met Glu Asn Met Asp Asp Thr Ser Gly Ser Ser Phe Glu
        35                  40                  45

Asp Met Gly Glu Leu His Gln Arg Leu Arg Glu Glu Glu Val Asp Ala
    50                  55                  60

Asp Ala Ala Asp Ala Ala Ala Ala Glu Glu Glu Asp Gly Glu Phe Leu
65                  70                  75                  80

Gly Met Lys Gly Phe Lys Gly Gln Leu Ser Arg Gln Val Ala Asp Gln
                85                  90                  95

Met Trp Gln Ala Gly Lys Arg Gln Ala Ser Arg Ala Phe Ser Leu Tyr
            100                 105                 110

Ala Asn Ile Asp Ile Leu Arg Pro Tyr Phe Asp Val Glu Pro Ala Gln
        115                 120                 125

Val Arg Thr Gly Leu Leu Glu Ser Met Ile Pro Ile Lys Met Val Asn
    130                 135                 140

Phe Pro Gln Lys Ile Ala Gly Glu Leu Tyr Gly Pro Leu Met Leu Val
145                 150                 155                 160

Phe Thr Leu Val Ala Ile Leu Leu His Gly Met Lys Thr Ser Asp Thr
                165                 170                 175

Ile Ile Arg Glu Gly Thr Leu Met Gly Thr Ala Ile Gly Thr Cys Phe
            180                 185                 190

Gly Tyr Trp Leu Gly Val Ser Ser Phe Ile Tyr Phe Leu Ala Tyr Leu
        195                 200                 205

Cys Asn Ala Gln Ile Thr Met Leu Gln Met Leu Ala Leu Leu Gly Tyr
    210                 215                 220

Gly Leu Phe Gly His Cys Ile Val Leu Phe Ile Thr Tyr Asn Ile His
225                 230                 235                 240

Leu His Ala Leu Phe Tyr Leu Phe Trp Leu Leu Val Gly Gly Leu Ser
                245                 250                 255

Thr Leu Arg Met Val Ala Val Leu Val Ser Arg Thr Val Gly Pro Thr
            260                 265                 270

Gln Arg Leu Leu Leu Cys Gly Thr Leu Ala Ala Leu His Met Leu Phe
        275                 280                 285

Leu Leu Tyr Leu His Phe Ala Tyr His Lys Val Val Glu Gly Ile Leu
    290                 295                 300

Asp Thr Leu Glu Gly Pro Asn Ile Pro Pro Ile Gln Arg Val Pro Arg
305                 310                 315                 320

Asp Ile Pro Ala Met Leu Pro Ala Ala Arg Leu Pro Thr Thr Val Leu
                325                 330                 335

Asn Ala Thr Ala Lys Ala Val Ala Val Thr Leu Gln Ser His Xaa
            340                 345                 350

(2) INFORMATION FOR SEQ ID NO: 490:

(i) SEQUENCE CHARACTERISTICS:
(A) LENGTH: 265 amino acids
(B) TYPE: amino acid
(D) TOPOLOGY: linear
(xi) SEQUENCE DESCRIPTION: SEQ ID NO: 490:

Met Arg Gly Ser Arg Gly Gly Trp Ala Gly Glu Met Ala Ala Ser Gly
  1               5                  10                  15

Glu Ser Gly Thr Ser Gly Gly Gly Gly Ser Thr Glu Glu Ala Phe Met
             20                  25                  30

Thr Phe Tyr Ser Glu Val Lys Gln Ile Glu Lys Arg Asp Ser Val Leu
         35                  40                  45

Thr Ser Lys Asn Gln Ile Glu Arg Leu Thr Arg Pro Gly Ser Ser Tyr
     50                  55                  60

Phe Asn Leu Asn Pro Phe Glu Val Leu Gln Ile Asp Pro Glu Val Thr
65                  70                  75                  80

Asp Glu Glu Ile Lys Lys Arg Phe Arg Gln Leu Ser Ile Leu Val His
                85                  90                  95

Pro Asp Lys Asn Gln Asp Asp Ala Asp Arg Ala Gln Lys Ala Phe Glu
            100                 105                 110

Ala Val Asp Lys Ala Tyr Lys Leu Leu Leu Asp Gln Glu Gln Lys Lys
        115                 120                 125

Arg Ala Leu Asp Val Ile Gln Ala Gly Lys Glu Tyr Val Glu His Thr
    130                 135                 140

Val Lys Glu Arg Lys Lys Gln Leu Lys Lys Glu Gly Lys Pro Thr Ile
145                 150                 155                 160

Val Glu Glu Asp Asp Pro Glu Leu Phe Lys Gln Ala Val Tyr Lys Gln
                165                 170                 175

Thr Met Lys Leu Phe Ala Glu Leu Glu Ile Lys Arg Lys Glu Arg Glu
            180                 185                 190

Ala Lys Glu Met His Glu Arg Lys Arg Gln Arg Glu Glu Glu Ile Glu
        195                 200                 205

Ala Gln Glu Lys Ala Lys Arg Glu Arg Glu Trp Gln Lys Asn Phe Glu
    210                 215                 220

Glu Ser Arg Asp Gly Arg Val Asp Ser Trp Arg Asn Phe Gln Ala Asn
225                 230                 235                 240

Thr Lys Gly Lys Lys Glu Lys Lys Asn Arg Thr Phe Leu Arg Pro Pro
                245                 250                 255

Lys Val Lys Met Glu Gln Arg Glu Xaa
            260                 265

(2) INFORMATION FOR SEQ ID NO: 491:

(i) SEQUENCE CHARACTERISTICS:
(A) LENGTH: 25 amino acids
(B) TYPE: amino acid
(D) TOPOLOGY: linear
(xi) SEQUENCE DESCRIPTION: SEQ ID NO: 491:

Asp Ser Met Pro Thr Cys Pro Leu Xaa Ala Ser Leu Glu Cys Gly Pro
  1               5                  10                  15

Leu Leu Pro Val Arg Leu Cys Cys Leu
            20                  25

(2) INFORMATION FOR SEQ ID NO: 492:

(i) SEQUENCE CHARACTERISTICS:
(A) LENGTH: 159 amino acids
(B) TYPE: amino acid
(D) TOPOLOGY: linear
(xi) SEQUENCE DESCRIPTION: SEQ ID NO: 492:

Met Asn Glu Tyr Arg Val Pro Glu Leu Asn Val Gln Asn Gly Val Leu
1               5                   10                  15

Lys Ser Leu Ser Phe Leu Phe Glu Tyr Ile Gly Glu Met Gly Lys Asp
            20                  25                  30

Tyr Ile Tyr Ala Val Thr Pro Leu Leu Glu Asp Ala Leu Met Asp Arg
        35                  40                  45

Asp Leu Val His Arg Gln Thr Ala Ser Ala Val Val Gln His Met Ser
    50                  55                  60

Leu Gly Val Tyr Gly Phe Gly Cys Glu Asp Ser Leu Asn His Leu Leu
65                  70                  75                  80

Asn Tyr Val Trp Pro Asn Val Phe Glu Thr Ser Pro His Val Ile Gln
                85                  90                  95

Ala Val Met Gly Ala Leu Glu Gly Leu Arg Val Ala Ile Gly Pro Cys
            100                 105                 110

Arg Met Leu Gln Tyr Cys Leu Gln Gly Leu Phe His Pro Ala Arg Lys
        115                 120                 125

Val Arg Asp Val Tyr Trp Lys Ile Tyr Asn Ser Ile Tyr Ile Gly Ser
    130                 135                 140

Gln Asp Ala Leu Ile Ala His Tyr Pro Arg Ile Tyr Gln Arg Xaa
145                 150                 155

(2) INFORMATION FOR SEQ ID NO: 493:

(i) SEQUENCE CHARACTERISTICS:
(A) LENGTH: 279 amino acids
(B) TYPE: amino acid
(D) TOPOLOGY: linear
(xi) SEQUENCE DESCRIPTION: SEQ ID NO: 493:

Met Ile Ser Asp Asn Ser Ala Glu Asn Ile Ala Leu Val Thr Ser Met
1               5                   10                  15

Tyr Asp Gly Leu Leu Gln Ala Gly Ala Arg Leu Cys Pro Thr Val Gln
            20                  25                  30

Leu Glu Asp Ile Arg Asn Leu Gln Asp Leu Thr Pro Leu Lys Leu Ala
        35                  40                  45

Ala Lys Glu Gly Lys Ile Glu Ile Phe Arg His Ile Leu Gln Arg Glu
    50                  55                  60

Phe Ser Gly Leu Ser His Leu Ser Arg Lys Phe Thr Glu Trp Cys Tyr
65                  70                  75                  80

Gly Pro Val Arg Val Ser Leu Tyr Asp Leu Ala Ser Val Asp Ser Cys
                85                  90                  95

Glu Glu Asn Ser Val Leu Glu Ile Ile Ala Phe His Cys Lys Ser Pro
            100                 105                 110

His Arg His Arg Met Val Val Leu Glu Pro Leu Asn Lys Leu Leu Gln
        115                 120                 125

Ala Lys Trp Asp Leu Leu Ile Pro Lys Phe Phe Leu Asn Phe Leu Cys
    130                 135                 140

Asn Leu Ile Tyr Met Phe Ile Phe Thr Ala Val Ala Tyr His Gln Pro
145                 150                 155                 160

Thr Leu Lys Lys Gln Ala Ala Pro His Leu Lys Ala Glu Val Gly Asn
                165                 170                 175

Ser Met Leu Leu Thr Gly His Ile Leu Ile Leu Leu Gly Gly Ile Tyr
            180                 185                 190

Leu Leu Val Gly Gln Leu Trp Tyr Phe Trp Arg Arg His Val Phe Ile
        195                 200                 205

Trp Ile Ser Phe Ile Asp Ser Tyr Phe Glu Ile Leu Phe Leu Phe Gln
    210                 215                 220

Ala Leu Leu Thr Val Val Ser Gln Val Leu Cys Phe Leu Xaa Ile Glu
225                 230                 235                 240

Trp Tyr Leu Pro Leu Leu Val Ser Ala Leu Val Leu Gly Trp Leu Asn
                245                 250                 255

Leu Leu Tyr Tyr Thr Arg Gly Phe Gln His Thr Gly Ile Tyr Ser Val
            260                 265                 270

Met Ile Gln Lys Pro Trp Xaa
        275

(2) INFORMATION FOR SEQ ID NO: 494:

        (i) SEQUENCE CHARACTERISTICS:
                (A) LENGTH: 193 amino acids
                (B) TYPE: amino acid
                (D) TOPOLOGY: linear
        (xi) SEQUENCE DESCRIPTION: SEQ ID NO: 494:

Met Ile Arg Cys Gly Leu Ala Cys Glu Arg Cys Arg Trp Ile Leu Pro
  1               5                  10                  15

Leu Leu Leu Leu Ser Ala Ile Ala Phe Asp Ile Ile Ala Leu Ala Gly
            20                  25                  30

Arg Gly Trp Leu Gln Ser Ser Asp His Gly Gln Thr Ser Ser Leu Trp
        35                  40                  45

Trp Lys Cys Ser Gln Glu Gly Gly Gly Ser Gly Ser Tyr Glu Glu Gly

    50                  55                  60

Cys Gln Ser Leu Met Glu Tyr Ala Trp Gly Arg Ala Ala Ala Ala Met
65                  70                  75                  80

Leu Phe Cys Gly Phe Ile Ile Leu Val Ile Cys Phe Ile Leu Ser Phe
                85                  90                  95

Phe Ala Leu Cys Gly Pro Gln Met Leu Val Phe Leu Arg Val Ile Gly
            100                 105                 110

Gly Leu Leu Ala Leu Ala Ala Val Phe Gln Ile Ile Ser Leu Val Ile
        115                 120                 125

Tyr Pro Val Lys Tyr Thr Gln Thr Phe Thr Leu His Ala Asn Xaa Ala
    130                 135                 140

Val Thr Tyr Ile Tyr Asn Trp Ala Tyr Gly Phe Gly Trp Ala Ala Thr
145                 150                 155                 160

Ile Ile Leu Ile Gly Cys Ala Phe Phe Phe Cys Cys Leu Pro Asn Tyr
                165                 170                 175

Glu Asp Asp Leu Leu Gly Asn Ala Lys Pro Arg Tyr Phe Tyr Thr Ser
            180                 185                 190

Ala

(2) INFORMATION FOR SEQ ID NO: 495:

(i) SEQUENCE CHARACTERISTICS:
(A) LENGTH: 205 amino acids
(B) TYPE: amino acid
(D) TOPOLOGY: linear
(xi) SEQUENCE DESCRIPTION: SEQ ID NO: 495:

Met Ala Ala Gly Asp Gln Val Phe Ser Gly Ala Gly His Val Xaa Glu
1               5                   10                  15

His Val Ala Gly Gly Arg His Ala Trp Leu Leu Thr Trp Gln Ser Ala
            20                  25                  30

Cys Pro Ala Asn Arg Leu Ser Leu Val Pro Leu Val Pro Ser Ala Ser
        35                  40                  45

Met Thr Arg Leu Met Arg Xaa Arg Thr Ala Ser Gly Ser Ser Val Ile
    50                  55                  60

Leu Trp Met Ala Pro Ala Ala Ala Pro Thr Pro Ala Arg Ala Pro Glu
65                  70                  75                  80

Ala Ala Pro Thr Pro Ala Arg Ala Pro Ala Ala Ala Arg Thr Pro Ala
                85                  90                  95

Arg Gly Pro Thr Trp Thr Ser Pro Pro Thr Arg Val Leu Leu Gly Thr
            100                 105                 110

Xaa Pro Gly Pro Ser Pro Trp Arg Ser Pro Ala Arg Arg Pro Ala Gln
        115                 120                 125

Leu Pro Pro Pro Asp Ser Asp Leu Cys Ser Gly Pro Leu Leu Pro Gly
    130                 135                 140

Pro Phe Ser Pro Pro Ala Cys His Thr Ala Pro Asn Ser Val Leu Ile
145                 150                 155                 160

Gln Ser Leu Phe Cys Lys Ser Glu Leu Trp Trp Arg Gln Met Arg Ser
                165                 170                 175

Ile Thr Trp Val Pro Ser Pro Lys Ala Gly Trp Arg Trp Thr Lys Gly
            180                 185                 190

Arg Lys Gln Ala Ser Pro His Arg Ile Leu Phe His Xaa
        195                 200                 205

(2) INFORMATION FOR SEQ ID NO: 496:

(i) SEQUENCE CHARACTERISTICS:
(A) LENGTH: 147 amino acids
(B) TYPE: amino acid
(D) TOPOLOGY: linear
(xi) SEQUENCE DESCRIPTION: SEQ ID NO: 496:

Met Ala Leu Thr Leu Leu Pro Ser Val Ser Arg Leu Pro Gly Glu Arg
1               5                   10                  15

Met Ala Ala Ser Gly Leu Pro Tyr Val Leu His His Lys Ser Ser Leu
            20                  25                  30

Met Lys Val Ile Phe Phe Pro Tyr Pro Val Leu Pro Leu Pro Ala Pro
        35                  40                  45

Asn Gly Thr Trp Val Pro Arg Leu Val Leu Gly Leu Gly Ser Gly Asp
    50                  55                  60

Gln Val His Tyr Leu Pro Ile Ser Ser Ser Ile Val Asn Tyr Gly Thr
65                  70                  75                  80

Ser Val Ser Gly Lys Ser Trp Val Phe Leu Val Tyr Pro Leu His Pro
                85                  90                  95

Thr Pro Thr Trp Ser Thr Arg Cys Phe Gln Val Trp Asp Leu Leu Ser
            100                 105                 110

Val Glu Leu Pro Asp Lys Gly Glu Gly Asn Thr Arg Arg Ala Ser Gly
        115                 120                 125

Val Pro Gly Leu Ser Gln Leu Pro Thr Ser His Lys Pro Ile Lys Gln
    130                 135                 140

Glu Tyr Xaa
145

(2) INFORMATION FOR SEQ ID NO: 497:

(i) SEQUENCE CHARACTERISTICS:
(A) LENGTH: 64 amino acids
(B) TYPE: amino acid
(D) TOPOLOGY: linear
(xi) SEQUENCE DESCRIPTION: SEQ ID NO: 497:

Met Val Trp Val Leu Trp Ser Ala Pro Ser Leu Ala Pro Pro Trp Val
1               5                   10                  15

Gly Pro Cys Trp Pro Ser Thr Gly Asn Cys Cys Leu Cys Glu Val Gly
            20                  25                  30

Ala Ala Leu Pro Pro Arg Gly Pro Ser Leu Ser Asp Cys Leu Gly Leu
        35                  40                  45

Pro Pro Trp Thr Pro Trp Gly Pro Ala Trp Thr Leu Ala Gln Ser Xaa
    50                  55                  60

(2) INFORMATION FOR SEQ ID NO: 498:

(i) SEQUENCE CHARACTERISTICS:
(A) LENGTH: 94 amino acids
(B) TYPE: amino acid
(D) TOPOLOGY: linear
(xi) SEQUENCE DESCRIPTION: SEQ ID NO: 498:

Met Ser Thr Gly Ala Leu Asn Thr Ser Pro Pro Ala Ser Asn Arg Leu
1               5                   10                  15

Glu Ser Thr Leu Asn Glu Tyr Leu Ile Gln Pro Gln Leu His Cys Ser
            20                  25                  30

Ser Val Gln Arg Leu Thr Leu Lys Trp Gly Cys Ser Ser Leu Gln Arg
        35                  40                  45

Asp Gly Gln Ala Val Pro Trp Gly Leu Trp Gln Arg Ala Tyr Pro Ser
    50                  55                  60

Leu Leu Pro Thr Leu Pro Ser Asp Leu Leu Arg Pro His Ala Val Thr
65                  70                  75                  80

Pro Ser Val Ser Val Ser Val His Thr Cys Glu Ser Ser Xaa
                85                  90

(2) INFORMATION FOR SEQ ID NO: 499:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 499:

Met Phe Leu Ile Phe Val Tyr Phe Leu Lys Xaa Leu Phe Ser Ser Ser
1               5                   10                  15

Leu Pro Phe Leu Trp Leu
            20

(2) INFORMATION FOR SEQ ID NO: 500:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 500:

(2) INFORMATION FOR SEQ ID NO: 501:

(i) SEQUENCE CHARACTERISTICS:
(A) LENGTH: 28 amino acids
(B) TYPE: amino acid
(D) TOPOLOGY: linear
(xi) SEQUENCE DESCRIPTION: SEQ ID NO: 501:

Met Gln Phe Leu Leu Thr Ala Phe Leu Leu Val Pro Leu Leu Ala Leu
1               5                   10                  15

Cys Asp Val Pro Ile Ser Leu Gly Phe Ser Pro Ser
            20                  25

(2) INFORMATION FOR SEQ ID NO: 502:

(i) SEQUENCE CHARACTERISTICS:
(A) LENGTH: 15 amino acids
(B) TYPE: amino acid
(D) TOPOLOGY: linear
(xi) SEQUENCE DESCRIPTION: SEQ ID NO: 502:

Pro Gly Lys Pro Gln Ala Cys Pro Glu Leu Thr Ser Val Leu Pro
1               5                   10                  15

(2) INFORMATION FOR SEQ ID NO: 503:

(i) SEQUENCE CHARACTERISTICS:

(A) LENGTH: 19 amino acids
(B) TYPE: amino acid
(D) TOPOLOGY: linear
(xi) SEQUENCE DESCRIPTION: SEQ ID NO: 503:

Asn Lys Ser Leu Xaa Ser Cys Leu Phe Val Leu His Phe Val Leu His
1               5                   10                  15

Cys Xaa Phe

(2) INFORMATION FOR SEQ ID NO: 504:

(i) SEQUENCE CHARACTERISTICS:
(A) LENGTH: 29 amino acids
(B) TYPE: amino acid
(D) TOPOLOGY: linear
(xi) SEQUENCE DESCRIPTION: SEQ ID NO: 504:

Met Glu Lys Thr His Arg Leu Arg Ile Arg Asn Pro Cys Leu Gln Phe
1               5                   10                  15

Ser Ile Leu Asn Leu Phe Leu Leu Lys Met Ile Val Ser
            20                  25

(2) INFORMATION FOR SEQ ID NO: 505:

(i) SEQUENCE CHARACTERISTICS:
(A) LENGTH: 75 amino acids
(B) TYPE: amino acid
(D) TOPOLOGY: linear
(xi) SEQUENCE DESCRIPTION: SEQ ID NO: 505:

Met Val Asp Ile Ser Lys Met His Met Ile Leu Tyr Asp Leu Gln Gln
1               5                   10                  15

Asn Leu Ser Ser Ser His Arg Ala Leu Glu Lys Gln Ile Asp Thr Leu
            20                  25                  30

Ala Gly Lys Leu Asp Ala Leu Thr Glu Leu Leu Ser Thr Ala Leu Gly
        35                  40                  45

Pro Ser Ser Phe Gln Asn Pro Ala Ser Ser Pro Ser Ser Trp Thr His
    50                  55                  60

Glu Glu Glu Pro Gly Tyr Phe Pro Gln Tyr Xaa
65                  70                  75

(2) INFORMATION FOR SEQ ID NO: 506:

(i) SEQUENCE CHARACTERISTICS:
(A) LENGTH: 10 amino acids
(B) TYPE: amino acid
(D) TOPOLOGY: linear

(xi) SEQUENCE DESCRIPTION: SEQ ID NO: 506:

Leu Pro Leu Ala Glu Leu Lys Asn Trp Val
1               5                   10

(2) INFORMATION FOR SEQ ID NO: 507:

(i) SEQUENCE CHARACTERISTICS:
(A) LENGTH: 207 amino acids
(B) TYPE: amino acid
(D) TOPOLOGY: linear
(xi) SEQUENCE DESCRIPTION: SEQ ID NO: 507:

Met Leu Trp Phe Gly Gly Cys Ser Ala Val Asn Ala Thr Gly His Leu
1               5                   10                  15

Ser Asp Thr Leu Trp Leu Ile Pro Ile Thr Phe Leu Thr Ile Gly Tyr
            20                  25                  30

Gly Asp Val Val Pro Gly Thr Met Trp Gly Lys Ile Val Cys Leu Cys
        35                  40                  45

Thr Gly Val Met Gly Val Cys Cys Thr Ala Leu Leu Val Ala Val Val
    50                  55                  60

Ala Arg Lys Leu Glu Phe Asn Lys Ala Glu Lys His Val His Asn Phe
65                  70                  75                  80

Met Met Asp Ile Gln Tyr Thr Lys Glu Met Lys Glu Ser Ala Ala Arg
                85                  90                  95

Val Leu Gln Glu Ala Trp Met Phe Tyr Lys His Thr Arg Arg Lys Glu
            100                 105                 110

Ser His Ala Ala Arg Arg His Gln Arg Xaa Leu Leu Ala Ala Ile Asn
        115                 120                 125

Ala Phe Arg Gln Val Arg Leu Lys His Arg Lys Leu Arg Glu Gln Val
    130                 135                 140

Asn Ser Met Val Asp Ile Ser Lys Met His Met Ile Leu Tyr Asp Leu
145                 150                 155                 160

Gln Gln Asn Leu Ser Ser Ser His Arg Ala Leu Glu Lys Gln Ile Asp
                165                 170                 175

Thr Leu Ala Gly Lys Leu Asp Ala Leu Thr Glu Leu Leu Ser Thr Ala
            180                 185                 190

Leu Gly Pro Arg Gln Leu Pro Glu Pro Ser Gln Gln Ser Lys Xaa
        195                 200                 205

(2) INFORMATION FOR SEQ ID NO: 508:

(i) SEQUENCE CHARACTERISTICS:
(A) LENGTH: 36 amino acids

(B) TYPE: amino acid
(D) TOPOLOGY: linear
(xi) SEQUENCE DESCRIPTION: SEQ ID NO: 508:

Met Trp Arg Cys Arg Gly Lys Leu Ser Phe Pro Leu Phe Ala Val Val
1               5                   10                  15

Ile Val Ser Cys Arg Lys Asp Gly Pro Asp Ala Ala Ala Ala Pro Ala
            20                  25                  30

Val Xaa Lys Lys
        35

(2) INFORMATION FOR SEQ ID NO: 509:

(i) SEQUENCE CHARACTERISTICS:
(A) LENGTH: 19 amino acids
(B) TYPE: amino acid
(D) TOPOLOGY: linear
(xi) SEQUENCE DESCRIPTION: SEQ ID NO: 509:

Met Ala Leu Val Ala Leu Phe Thr Gln Leu Met Arg Xaa Leu Gly Arg
1               5                   10                  15

Cys Pro Gln

(2) INFORMATION FOR SEQ ID NO: 510:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 510:

Met Thr Phe Pro Phe Glu Lys Glu Asn Ser Cys Phe Gln Cys Leu Leu
1               5                   10                  15

Phe Asp Ser Trp Arg Glu Gln Thr Arg Thr Asn Ile Gln Pro Gln Arg
            20                  25                  30

(2) INFORMATION FOR SEQ ID NO: 511:

(i) SEQUENCE CHARACTERISTICS:
(A) LENGTH: 28 amino acids
(B) TYPE: amino acid
(D) TOPOLOGY: linear
(xi) SEQUENCE DESCRIPTION: SEQ ID NO: 511:

Met His Leu Leu Asp Phe Phe Arg Asp Leu Val Leu Leu Val Leu Leu
1               5                   10                  15

Ala Leu Leu Asp Ser Phe Trp Leu Glu Val Gln Lys
            20                  25

(2) INFORMATION FOR SEQ ID NO: 512:

        (i) SEQUENCE CHARACTERISTICS:
              (A) LENGTH: 26 amino acids
              (B) TYPE: amino acid
              (D) TOPOLOGY: linear
        (xi) SEQUENCE DESCRIPTION: SEQ ID NO: 512:

Met Cys Leu Ile His Phe Ile Lys Ile Ile Leu Val Phe Ile Leu Lys
  1               5                  10                  15

Leu Trp Leu Tyr Ser Gln Lys Cys Pro Lys
            20                  25

(2) INFORMATION FOR SEQ ID NO: 513:

        (i) SEQUENCE CHARACTERISTICS:
              (A) LENGTH: 33 amino acids
              (B) TYPE: amino acid
              (D) TOPOLOGY: linear
        (xi) SEQUENCE DESCRIPTION: SEQ ID NO: 513:

Met Ile His Val His Glu Trp Asn Asp Gln Met Leu Met Val Tyr Ile
  1               5                  10                  15

Phe Leu Tyr Pro Val Ser Ile Thr Phe Leu Asn Leu Cys Ser Leu Thr
            20                  25                  30

Cys

(2) INFORMATION FOR SEQ ID NO: 514:

        (i) SEQUENCE CHARACTERISTICS:
              (A) LENGTH: 47 amino acids
              (B) TYPE: amino acid
              (D) TOPOLOGY: linear
        (xi) SEQUENCE DESCRIPTION: SEQ ID NO: 514:

Leu Asn Glu Ser Tyr Val Ser Arg Ala Gly Gly Trp Phe Ser Met Phe
  1               5                  10                  15

Xaa Leu Ile Phe Phe Leu Leu Ala Leu Gly Ser Xaa Leu Cys Leu Leu
            20                  25                  30

Leu Cys Leu Pro Ser Phe Asn Lys Thr Arg Arg Lys Gln Lys Pro
        35                  40                  45

(2) INFORMATION FOR SEQ ID NO: 515:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 515:

Ser Ser Lys Thr Pro Leu Pro Ser Glu Arg Arg Trp Ile Ser Gly Ser
1               5                   10                  15

Ser Leu Met Ala Pro Arg Pro Trp Leu Leu Gly Ile Ala Leu Leu Gly
            20                  25                  30

Leu Trp Ala Leu Glu Pro Ala Leu Gly His Trp
        35                  40

(2) INFORMATION FOR SEQ ID NO: 516:

(i) SEQUENCE CHARACTERISTICS:
(A) LENGTH: 3 amino acids
(B) TYPE: amino acid
(D) TOPOLOGY: linear
(xi) SEQUENCE DESCRIPTION: SEQ ID NO: 516:

Leu Asn Trp
1

(2) INFORMATION FOR SEQ ID NO: 517:

(i) SEQUENCE CHARACTERISTICS:
(A) LENGTH: 174 amino acids
(B) TYPE: amino acid
(D) TOPOLOGY: linear
(xi) SEQUENCE DESCRIPTION: SEQ ID NO: 517:

Phe Ala Phe Cys Ala Glu Leu Met Ile Gln Asn Trp Thr Leu Gly Ala
1               5                   10                  15

Val Asp Ser Gln Met Asp Asp Met Asp Met Asp Leu Asp Lys Glu Phe
            20                  25                  30

Leu Gln Asp Leu Lys Glu Leu Lys Val Leu Val Ala Asp Lys Asp Leu
        35                  40                  45

Leu Asp Leu His Lys Ser Leu Val Cys Thr Ala Leu Arg Gly Lys Leu
    50                  55                  60

Gly Val Phe Ser Glu Met Glu Ala Asn Phe Lys Asn Leu Ser Arg Gly
65                  70                  75                  80

Leu Val Asn Val Ala Ala Lys Leu Thr His Asn Lys Asp Val Arg Asp
                85                  90                  95

Leu Phe Val Asp Leu Val Glu Lys Phe Val Glu Pro Cys Arg Ser Asp
            100                 105                 110

His Trp Pro Leu Ser Asp Val Arg Phe Phe Leu Asn Gln Tyr Ser Ala
        115                 120                 125

Ser Val His Ser Leu Asp Gly Phe Arg His Gln Ala Ser Gly Thr Ala
    130                 135                 140

Thr Trp Ala Pro Ser Ala Ala Ala Ser Cys Ala Cys Ile Met Thr Glu
145                 150                 155                 160

Val Pro Pro Asn Ala Pro Pro Thr Leu Thr Ile Lys Leu Leu
                165                 170

(2) INFORMATION FOR SEQ ID NO: 518:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 518:

Met Trp Lys Asn Leu Gly Ser Gly Ser Val Phe Val Thr Trp Phe Ser
1               5                   10                  15

Leu Val Met Ile Leu Ser Gly Ile Gly Pro Leu Gly Asp Ala Glu Asp
            20                  25                  30

Ser Ile Ser Asp Val Ser His Arg Leu Arg Pro
        35                  40

(2) INFORMATION FOR SEQ ID NO: 519:

(i) SEQUENCE CHARACTERISTICS:
(A) LENGTH: 13 amino acids
(B) TYPE: amino acid
(D) TOPOLOGY: linear
(xi) SEQUENCE DESCRIPTION: SEQ ID NO: 519:

Phe Gln Phe Pro Leu Leu Thr Ile Ala Leu Gln Phe Leu
1               5                   10

(2) INFORMATION FOR SEQ ID NO: 520:

(i) SEQUENCE CHARACTERISTICS:
(A) LENGTH: 30 amino acids
(B) TYPE: amino acid
(D) TOPOLOGY: linear
(xi) SEQUENCE DESCRIPTION: SEQ ID NO: 520:

Met His Tyr Val Ile Val Leu Ser Leu Phe Val Val Leu Glu Lys Lys
1               5                   10                  15

Asn Lys Met Gly Ser Asp Gly Cys Leu Arg Lys Asn Gly Ser
            20                  25                  30

(2) INFORMATION FOR SEQ ID NO: 521:

(i) SEQUENCE CHARACTERISTICS:
(A) LENGTH: 47 amino acids
(B) TYPE: amino acid
(D) TOPOLOGY: linear
(xi) SEQUENCE DESCRIPTION: SEQ ID NO: 521:

Met Ser Arg Ser Ile Val Leu Arg Gly Ser Leu Phe Leu Phe Phe Ser
1               5                   10                  15

His Tyr Thr Leu Lys Leu Leu Ser Val Ile Lys Gln Thr Asn Arg Lys
            20                  25                  30

Ile Val Trp Glu Lys Pro Cys Ile Arg Leu Phe Tyr Xaa Val Leu
        35                  40                  45

(2) INFORMATION FOR SEQ ID NO: 522:

(i) SEQUENCE CHARACTERISTICS:
(A) LENGTH: 26 amino acids
(B) TYPE: amino acid
(D) TOPOLOGY: linear
(xi) SEQUENCE DESCRIPTION: SEQ ID NO: 522:

Met Pro Leu Pro Val Leu Leu Cys Leu Thr Leu Pro Met Pro Leu Pro
1               5                   10                  15

Ser Ala Thr Ala Arg Gly Gly Asn Arg Thr
            20                  25

(2) INFORMATION FOR SEQ ID NO: 523:

(i) SEQUENCE CHARACTERISTICS:
(A) LENGTH: 58 amino acids
(B) TYPE: amino acid
(D) TOPOLOGY: linear
(xi) SEQUENCE DESCRIPTION: SEQ ID NO: 523:

Ser Ser Ile Pro Val Ser Ile Leu Ile Gly Met Lys Leu Ile Leu Tyr
1               5                   10                  15

Leu Leu Ile Thr Glu Ser Gly Ser His Glu Lys Lys Ser Phe Tyr Pro
            20                  25                  30

Ser Phe Lys Tyr Met Phe Lys Ile Ile Ile Tyr Val Ser Ala Tyr Cys
        35                  40                  45

Arg Thr Ala Leu Arg Ala Thr Val Ser His
    50                  55

(2) INFORMATION FOR SEQ ID NO: 524:

(i) SEQUENCE CHARACTERISTICS:
(A) LENGTH: 19 amino acids
(B) TYPE: amino acid
(D) TOPOLOGY: linear
(xi) SEQUENCE DESCRIPTION: SEQ ID NO: 524:

Asn Arg Thr Leu Leu Phe Leu Ile Leu Phe Val Leu Phe Gly Leu Gly
1               5                   10                  15

Tyr Gly Phe

(2) INFORMATION FOR SEQ ID NO: 525:

(i) SEQUENCE CHARACTERISTICS:
(A) LENGTH: 40 amino acids
(B) TYPE: amino acid
(D) TOPOLOGY: linear
(xi) SEQUENCE DESCRIPTION: SEQ ID NO: 525:

Met Phe Leu Leu Val Leu Ser Val Phe Cys Asp Phe Met Cys Ser Ile
1               5                   10                  15

Ala Pro Arg Cys His Ala Leu Ser Leu Val Ser Leu Arg Ala Gln His
            20                  25                  30

Leu Ser Leu Phe Ile Thr Cys His
        35                  40

(2) INFORMATION FOR SEQ ID NO: 526:

(i) SEQUENCE CHARACTERISTICS:
(A) LENGTH: 57 amino acids
(B) TYPE: amino acid
(D) TOPOLOGY: linear
(xi) SEQUENCE DESCRIPTION: SEQ ID NO: 526:

Met Leu Leu Phe Ile Leu Leu Thr Leu Ser Ser Gly Cys Arg Leu Leu
1               5                   10                  15

Val Ser Ser Trp Lys Thr Phe Leu Pro His Phe Ser Leu Pro Gly Pro
            20                  25                  30

Arg Glu His Pro Glu Gly Ser Arg Thr Trp Phe Phe Arg Tyr Trp Glu
        35                  40                  45

Pro Gly Ala His Cys Leu His Cys Ala
    50                  55

(2) INFORMATION FOR SEQ ID NO: 527:

(i) SEQUENCE CHARACTERISTICS:

(A) LENGTH: 21 amino acids
(B) TYPE: amino acid
(D) TOPOLOGY: linear
(xi) SEQUENCE DESCRIPTION: SEQ ID NO: 527:

Ala Arg Leu Leu Leu Phe Leu Ser Ser Val His Pro Ser Ile Met Pro
1               5                   10                  15

Ser Cys Asn Gln Leu
            20

(2) INFORMATION FOR SEQ ID NO: 528:

(i) SEQUENCE CHARACTERISTICS:
(A) LENGTH: 39 amino acids
(B) TYPE: amino acid
(D) TOPOLOGY: linear
(xi) SEQUENCE DESCRIPTION: SEQ ID NO: 528:

Met Ser Leu Thr Ser Ser Leu Thr Phe Leu Ser His Ile Leu Leu Leu
1               5                   10                  15

Pro Gln Lys Leu Gln Phe Leu Ser Trp Met Glu Arg Gln Gln Arg Cys
            20                  25                  30

Thr Gly Val Ala Lys Tyr Ala
        35

(2) INFORMATION FOR SEQ ID NO: 529:

(i) SEQUENCE CHARACTERISTICS:
(A) LENGTH: 128 amino acids
(B) TYPE: amino acid
(D) TOPOLOGY: linear
(xi) SEQUENCE DESCRIPTION: SEQ ID NO: 529:

Met Val Leu Arg Leu Ile Gln Leu Ile Phe Leu Ile Phe Phe Ile His
1               5                   10                  15

Ile Ile Ile Leu Leu Ile Pro Gly Ser Arg Pro Cys Gly Ser Trp Val
            20                  25                  30

Asn Asp Arg Xaa Leu Gly Leu Arg Asp Val Thr His Leu Ile Tyr Leu
        35                  40                  45

His Trp Val His Gly His Leu Pro Trp Cys His Pro Tyr Ile Gln Val
    50                  55                  60

Glu Phe Ser Ala Leu Ile Glu Ser Thr Ala Gln Leu Gly Leu Pro Phe
65                  70                  75                  80

Ser Trp Val Arg Val Ile His Pro Phe Leu Val Leu Pro Cys Leu Tyr
                85                  90                  95

Ser Pro Gly Leu Lys Asn Gly Ile Phe Leu Phe Leu Leu Arg Ala Met
            100                 105                 110

Pro Gly Gly Met Phe Pro Gly Asn Leu Glu Ala Phe Arg Val Pro Val
        115                 120                 125

(2) INFORMATION FOR SEQ ID NO: 530:

        (i) SEQUENCE CHARACTERISTICS:
                (A) LENGTH: 82 amino acids
                (B) TYPE: amino acid
                (D) TOPOLOGY: linear
        (xi) SEQUENCE DESCRIPTION: SEQ ID NO: 530:

Met Gly Ser Ser Val Leu Pro Phe Cys Val Cys Val Thr Ser Pro Ser
  1               5                  10                  15

Leu Gly Gly Arg Cys Ile Gln Gly Arg Phe Ala Ser His Ser Lys Phe
             20                  25                  30

Trp Gly Phe Gly Xaa Lys Thr Ala Ser Phe Gly Ala Val Gly Glu Thr
         35                  40                  45

Pro Pro Asp Gln Glu Pro Gln Lys Glu Thr Glu Pro Ala Thr Ser Ser
     50                  55                  60

His Ala Arg Pro Trp Ala Arg Val Ile Gly Leu Arg Ile Trp Pro Gln
 65                  70                  75                  80

Pro Asn

(2) INFORMATION FOR SEQ ID NO: 531:

        (i) SEQUENCE CHARACTERISTICS:
                (A) LENGTH: 20 amino acids
                (B) TYPE: amino acid
                (D) TOPOLOGY: linear
        (xi) SEQUENCE DESCRIPTION: SEQ ID NO: 531:

Met Leu Leu Ser Val Ala Ile Phe Ile Leu Leu Thr Leu Val Tyr Ala
  1               5                  10                  15

Tyr Trp Thr Met
             20

(2) INFORMATION FOR SEQ ID NO: 532:

        (i) SEQUENCE CHARACTERISTICS:
                (A) LENGTH: 75 amino acids
                (B) TYPE: amino acid
                (D) TOPOLOGY: linear
        (xi) SEQUENCE DESCRIPTION: SEQ ID NO: 532:

Asn Cys Glu Ile Leu Glu Tyr Cys Tyr Tyr Leu Thr Gln Leu Lys Ile
1               5                   10                  15

Ser Met Gly Lys Tyr Leu Ser Ile Pro Thr Val Leu Leu Lys Ile Ile
            20                  25                  30

Arg Cys Ser Ile Thr Ala Val Ser Asp Ser Ser Thr Ser Trp Ala Ile
        35                  40                  45

Lys Ala Gln Leu Lys Ile Glu Asn Lys Asp Leu Asp Asn Lys Thr Ala
    50                  55                  60

Lys Gly Gly Gly Gln Glu Ala Leu Thr Cys Thr
65                  70                  75

(2) INFORMATION FOR SEQ ID NO: 533:

(i) SEQUENCE CHARACTERISTICS:
(A) LENGTH: 60 amino acids
(B) TYPE: amino acid
(D) TOPOLOGY: linear
(xi) SEQUENCE DESCRIPTION: SEQ ID NO: 533:

Met Phe Leu Met Arg Met His Leu Cys Phe Cys Lys Tyr Cys Cys Ser
1               5                   10                  15

Phe Ile Val Thr Pro Thr Ser Thr Ser Asn Thr Xaa Ser Tyr Leu Trp
            20                  25                  30

Pro Trp Ile Ser Ala Ser Met Ala Gly Arg Gly Ser Xaa Trp Ala Cys
        35                  40                  45

Thr Leu Asn Ala Val Thr Arg Glu Gly Leu Pro Glu
    50                  55                  60

(2) INFORMATION FOR SEQ ID NO: 534:

(i) SEQUENCE CHARACTERISTICS:
(A) LENGTH: 39 amino acids
(B) TYPE: amino acid
(D) TOPOLOGY: linear
(xi) SEQUENCE DESCRIPTION: SEQ ID NO: 534:

Met Ser Leu Leu Asn Thr His Thr Leu Cys Phe Val Leu Phe Cys Phe
1               5                   10                  15

Thr Leu Ser Ile Asn Gln Glu Lys Leu Ala Asn His Leu Ala Phe Arg
            20                  25                  30

Ile Leu Phe Phe Ile Val Phe
        35

(2) INFORMATION FOR SEQ ID NO: 535:

(i) SEQUENCE CHARACTERISTICS:
(A) LENGTH: 2 amino acids
(B) TYPE: amino acid
(D) TOPOLOGY: linear
(xi) SEQUENCE DESCRIPTION: SEQ ID NO: 535:

Met Leu
1

(2) INFORMATION FOR SEQ ID NO: 536:

(i) SEQUENCE CHARACTERISTICS:
(A) LENGTH: 36 amino acids
(B) TYPE: amino acid
(D) TOPOLOGY: linear
(xi) SEQUENCE DESCRIPTION: SEQ ID NO: 536:

(2) INFORMATION FOR SEQ ID NO: 537:

(i) SEQUENCE CHARACTERISTICS:
(A) LENGTH: 14 amino acids
(B) TYPE: amino acid
(D) TOPOLOGY: linear
(xi) SEQUENCE DESCRIPTION: SEQ ID NO: 537:

Met Phe Asp Phe Leu Ser Tyr Phe Lys Asp Leu Leu Ser Cys
1               5                   10

(2) INFORMATION FOR SEQ ID NO: 538:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 538:

Met Gly Phe Gly Phe Val Leu Asn Ile Phe Ser Phe Phe Leu Xaa Pro
1               5                   10                  15

Pro Leu

(2) INFORMATION FOR SEQ ID NO: 539:

(i) SEQUENCE CHARACTERISTICS:
(A) LENGTH: 11 amino acids
(B) TYPE: amino acid
(D) TOPOLOGY: linear
(xi) SEQUENCE DESCRIPTION: SEQ ID NO: 539:

Leu Leu Leu Trp Thr Leu Leu Ala Xaa Tyr Xaa
1               5                   10

(2) INFORMATION FOR SEQ ID NO: 540:

(i) SEQUENCE CHARACTERISTICS:
(A) LENGTH: 108 amino acids
(B) TYPE: amino acid
(D) TOPOLOGY: linear
(xi) SEQUENCE DESCRIPTION: SEQ ID NO: 540:

Met Ala Ala Gln Lys Asp Gln Gln Lys Asp Ala Glu Ala Glu Gly Leu
1               5                   10                  15

Ser Gly Thr Thr Leu Leu Pro Lys Leu Ile Pro Ser Gly Ala Gly Arg
            20                  25                  30

Glu Trp Leu Glu Arg Arg Arg Ala Thr Ile Arg Pro Trp Ser Thr Phe
        35                  40                  45

Val Asp Gln Gln Arg Phe Ser Arg Pro Arg Asn Leu Gly Glu Leu Cys
    50                  55                  60

Gln Arg Leu Val Arg Asn Val Glu Tyr Tyr Gln Ser Asn Tyr Val Phe
65                  70                  75                  80

Val Phe Leu Gly Leu Ile Leu Tyr Cys Val Val Thr Ser Pro Met Leu
                85                  90                  95

Leu Val Ala Leu Ala Val Phe Phe Gly Ala Cys Xaa
            100                 105

(2) INFORMATION FOR SEQ ID NO: 541:

(i) SEQUENCE CHARACTERISTICS:
(A) LENGTH: 106 amino acids
(B) TYPE: amino acid
(D) TOPOLOGY: linear
(xi) SEQUENCE DESCRIPTION: SEQ ID NO: 541:

Phe Val Phe Leu Gly Leu Ile Leu Tyr Cys Val Val Thr Ser Pro Met
1               5                   10                  15

Leu Leu Val Ala Leu Ala Val Phe Phe Gly Ala Cys Tyr Ile Leu Tyr
            20                  25                  30

Leu Arg Thr Leu Glu Ser Lys Leu Val Leu Phe Gly Arg Glu Val Ser
        35                  40                  45

Pro Ala His Gln Tyr Ala Leu Ala Gly Gly Ile Ser Phe Pro Phe Phe
    50                  55                  60

Trp Leu Ala Gly Ala Gly Ser Ala Val Phe Trp Val Leu Gly Ala Thr
65                  70                  75                  80

Leu Val Val Ile Gly Ser His Ala Ala Phe His Gln Ile Glu Ala Val
                85                  90                  95

Asp Gly Glu Glu Leu Gln Met Glu Pro Val
            100                 105

(2) INFORMATION FOR SEQ ID NO: 542:

        (i) SEQUENCE CHARACTERISTICS:
                (A) LENGTH: 136 amino acids
                (B) TYPE: amino acid
                (D) TOPOLOGY: linear
        (xi) SEQUENCE DESCRIPTION: SEQ ID NO: 542:

Met Asp Arg Phe Thr Val Ala Gly Val Leu Pro Asp Ile Glu Gln Phe
 1               5                  10                  15

Phe Asn Ile Gly Asp Ser Ser Ser Gly Leu Ile Gln Thr Val Phe Ile
            20                  25                  30

Ser Ser Tyr Met Val Leu Ala Pro Val Phe Gly Tyr Leu Gly Asp Arg
        35                  40                  45

Tyr Asn Arg Lys Tyr Leu Met Cys Gly Gly Ile Ala Phe Trp Ser Leu
    50                  55                  60

Val Thr Leu Gly Ser Ser Phe Ile Pro Gly Glu His Phe Trp Leu Leu
65                  70                  75                  80

Leu Leu Thr Arg Gly Leu Val Gly Val Gly Glu Ala Ser Tyr Ser Thr
                85                  90                  95

Ile Ala Pro Thr Leu Ile Ala Asp Leu Phe Val Ala Asp Gln Arg Thr
            100                 105                 110

Gly Cys Ser Ala Ser Ser Thr Leu Pro Phe Arg Trp Ala Val Val Trp
        115                 120                 125

Ala Thr Leu Gln Ala Pro Lys Xaa
    130                 135

(2) INFORMATION FOR SEQ ID NO: 543:

        (i) SEQUENCE CHARACTERISTICS:
                (A) LENGTH: 424 amino acids
                (B) TYPE: amino acid
                (D) TOPOLOGY: linear
        (xi) SEQUENCE DESCRIPTION: SEQ ID NO: 543:
   Met Ala Gly Asp Trp His Trp Ala Leu Arg Val Thr Pro Gly Leu Gly
   1               5                   10                  15
   Val Val Ala Val Leu Leu Leu Phe Leu Val Val Arg Glu Pro Pro Arg
               20                  25                  30
   Gly Ala Val Glu Arg His Ser Asp Leu Pro Pro Leu Asn Pro Thr Ser
           35                  40                  45
   Trp Trp Ala Asp Leu Arg Ala Leu Ala Arg Asn Pro Ser Phe Val Leu
       50                  55                  60
   Ser Ser Leu Gly Phe Thr Ala Val Ala Phe Val Thr Gly Ser Leu Ala
   65                  70                  75                  80
   Leu Trp Ala Pro Ala Phe Leu Leu Arg Ser Arg Val Val Leu Gly Glu
                   85                  90                  95
   Thr Pro Pro Cys Leu Pro Gly Asp Ser Cys Ser Ser Ser Asp Ser Leu
               100                 105                 110
   Ile Phe Gly Leu Ile Thr Cys Leu Thr Gly Val Leu Gly Val Gly Leu
           115                 120                 125
   Gly Val Glu Ile Ser Arg Arg Xaa Arg His Ser Asn Pro Arg Ala Asp
       130                 135                 140
   Pro Leu Val Cys Ala Thr Gly Leu Leu Gly Ser Ala Pro Phe Leu Phe
   145                 150                 155                 160
   Leu Ser Leu Ala Cys Ala Arg Gly Ser Ile Val Ala Thr Tyr Ile Phe
                   165                 170                 175
   Ile Phe Ile Gly Glu Thr Leu Leu Ser Met Asn Trp Ala Ile Val Ala
               180                 185                 190
   Asp Ile Leu Leu Tyr Val Val Ile Pro Thr Arg Arg Ser Thr Ala Glu
           195                 200                 205
   Ala Phe Gln Ile Val Leu Ser His Leu Leu Gly Asp Ala Gly Ser Pro
       210                 215                 220
   Tyr Leu Ile Gly Leu Ile Ser Asp Arg Leu Arg Arg Asn Trp Pro Pro
   225                 230                 235                 240
   Ser Phe Leu Ser Glu Phe Arg Ala Leu Gln Phe Ser Leu Met Leu Cys
                   245                 250                 255
   Ala Phe Val Gly Ala Leu Gly Gly Ala Leu Ser Trp Ala Pro Xaa Ser
               260                 265                 270
   Ser Leu Arg Pro Thr Ala Gly Gly His Ser Cys Thr Cys Arg Ala Cys
           275                 280                 285
   Cys Thr Lys Gln Gly Pro Gln Thr Thr Gly Leu Trp Cys Pro Ser Gly
       290                 295                 300
   Ala Ala Pro Pro Ala Cys Pro Trp Pro Val Cys Ser Ser Glu Arg Leu
   305                 310                 315                 320
   Pro Leu Thr Tyr Leu His Ile Cys His Ser Xaa Pro Trp Ala His Pro
                   325                     330                     335
   Thr Lys Gly Leu Gly Leu Thr Pro Trp Pro Gly Pro Ala Ser Arg Gly
               340                     345                     350
   Thr Leu Gly Arg Val Pro Ala Pro Arg His Tyr Xaa Gly Ser Ser Gly
           355                     360                     365
   Glu Glu Val Gly Val Gln Glu Gly Asp Pro Ser Pro Gln Gly Xaa Pro
       370                     375                     380
   Gln Gly Leu Gly Ala Ile Cys Asn Gly Ile Lys Phe Val Ala Arg Pro
   385                     390                     395                     400
   Gln Val Pro Ala Leu Val Phe Leu Trp Val Ala Ser Asp Leu Ala Pro
                   405                     410                     415
   Arg Leu His Pro Arg Ala Pro Glu
               420

(2) INFORMATION FOR SEQ ID NO: 544:

        (i) SEQUENCE CHARACTERISTICS:
                (A) LENGTH: 39 amino acids
                (B) TYPE: amino acid
                (D) TOPOLOGY: linear
        (xi) SEQUENCE DESCRIPTION: SEQ ID NO: 544:

Met Phe Arg Phe Val Ile Cys Leu Phe Leu Trp Leu Val Leu Cys Arg
  1               5                  10                  15

Asp Ser Thr Ser Ala Ser Arg Ile Ala Leu Tyr Tyr Arg Ile Val Phe
             20                  25                  30

Leu Ile His Gln Cys Ser Ser
        35

(2) INFORMATION FOR SEQ ID NO: 545:

        (i) SEQUENCE CHARACTERISTICS:
                (A) LENGTH: 58 amino acids
                (B) TYPE: amino acid
                (D) TOPOLOGY: linear
        (xi) SEQUENCE DESCRIPTION: SEQ ID NO: 545:

Met Leu Pro Trp Xaa Ala Gln Leu Leu Asp Arg Thr Ile Gly Pro Leu
  1               5                  10                  15

Tyr Leu Leu Phe Val Gln Phe Ser Pro Ala Phe Ser Arg Thr Ser Pro
             20                  25                  30

Trp Arg Ser Pro Lys Asn Phe Arg Arg Leu Tyr Pro Pro Cys Thr Thr
        35                  40                  45

Ser Gly Cys Ala Ala Arg Trp Leu Phe Ser

50                    55

(2) INFORMATION FOR SEQ ID NO: 546:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 546:

Met Gly Leu Ser Val Leu Leu Pro Leu Cys Leu Leu Gly Pro Gly Arg
1               5                   10                  15

Phe Thr Ser Gly Gln Lys Pro Leu Asp Thr Pro Gly Leu Gly Val Pro
            20                  25                  30

Phe

(2) INFORMATION FOR SEQ ID NO: 547:

(i) SEQUENCE CHARACTERISTICS:
(A) LENGTH: 367 amino acids
(B) TYPE: amino acid
(D) TOPOLOGY: linear
(xi) SEQUENCE DESCRIPTION: SEQ ID NO: 547:

Met Ala Lys Pro Gln Val Val Val Ala Pro Val Leu Met Ser Lys Leu
1               5                   10                  15

Ser Val Asn Ala Pro Glu Phe Tyr Pro Ser Gly Tyr Ser Ser Ser Tyr
            20                  25                  30

Thr Glu Ser Tyr Glu Asp Gly Cys Glu Asp Tyr Pro Thr Leu Ser Glu
        35                  40                  45

Tyr Val Gln Asp Phe Leu Asn His Leu Thr Glu Gln Pro Gly Ser Phe
    50                  55                  60

Glu Thr Glu Ile Glu Gln Phe Ala Glu Thr Leu Asn Gly Cys Val Thr
65                  70                  75                  80

Thr Asp Asp Ala Leu Gln Glu Leu Val Glu Leu Ile Tyr Gln Gln Ala
                85                  90                  95

Thr Ser Ile Pro Asn Phe Ser Tyr Met Gly Ala Arg Leu Cys Asn Tyr
            100                 105                 110

Leu Ser His His Leu Thr Ile Ser Pro Gln Ser Gly Asn Phe Arg Gln
        115                 120                 125

Leu Leu Leu Gln Arg Cys Arg Thr Glu Tyr Glu Val Lys Asp Gln Ala
    130                 135                 140

Ala Lys Gly Asp Glu Val Thr Arg Lys Arg Phe His Ala Phe Val Leu
145                 150                 155                 160

Phe Leu Gly Glu Leu Tyr Leu Asn Leu Glu Ile Lys Gly Thr Asn Gly
                165                 170                 175

Gln Val Thr Arg Ala Asp Ile Leu Gln Val Gly Leu Arg Glu Leu Leu
            180                 185                 190

Asn Ala Leu Phe Ser Asn Pro Met Asp Asp Asn Leu Ile Cys Ala Val
        195                 200                 205

Lys Leu Leu Lys Leu Thr Gly Ser Val Leu Glu Asp Ala Trp Lys Glu
    210                 215                 220

Lys Gly Lys Met Asp Met Glu Glu Ile Ile Gln Arg Ile Glu Asn Val
225                 230                 235                 240

Val Leu Asp Ala Asn Cys Ser Arg Asp Val Lys Gln Met Leu Leu Lys
                245                 250                 255

Leu Val Glu Leu Arg Ser Ser Asn Trp Gly Arg Val His Ala Thr Ser
            260                 265                 270

Thr Tyr Arg Glu Ala Thr Pro Glu Asn Asp Pro Asn Tyr Phe Met Asn
        275                 280                 285

Glu Pro Thr Phe Tyr Thr Ser Asp Gly Val Pro Phe Thr Ala Ala Asp
    290                 295                 300

Pro Asp Tyr Gln Glu Lys Tyr Gln Glu Leu Leu Glu Arg Glu Asp Phe
305                 310                 315                 320

Phe Pro Asp Tyr Glu Glu Asn Gly Thr Asp Leu Ser Gly Ala Gly Asp
                325                 330                 335

Pro Tyr Leu Asp Asp Ile Asp Asp Glu Met Asp Pro Glu Ile Glu Glu
            340                 345                 350

Ala Tyr Glu Lys Phe Cys Leu Glu Ser Glu Arg Lys Arg Lys Gln
        355                 360                 365

(2) INFORMATION FOR SEQ ID NO: 548:

(i) SEQUENCE CHARACTERISTICS:
(A) LENGTH: 77 amino acids
(B) TYPE: amino acid
(D) TOPOLOGY: linear
(xi) SEQUENCE DESCRIPTION: SEQ ID NO: 548:

Met Leu Arg Leu Asp Ile Ile Asn Ser Leu Val Thr Thr Val Phe Met
  1               5                  10                  15

Leu Ile Val Ser Val Leu Ala Leu Ile Pro Glu Thr Thr Thr Leu Thr
            20                  25                  30

Val Gly Gly Gly Val Phe Ala Leu Val Thr Ala Val Cys Cys Leu Ala
        35                  40                  45

Asp Gly Ala Leu Ile Tyr Arg Lys Leu Leu Phe Asn Pro Ser Gly Pro

    50                  55                  60

Tyr Gln Lys Lys Pro Val His Glu Lys Lys Glu Val Leu
65                  70                  75

(2) INFORMATION FOR SEQ ID NO: 549:

(i) SEQUENCE CHARACTERISTICS:
(A) LENGTH: 47 amino acids
(B) TYPE: amino acid
(D) TOPOLOGY: linear
(xi) SEQUENCE DESCRIPTION: SEQ ID NO: 549:

Met Leu Lys Gln Val Met Phe Val Phe Ser Gly Met Gly Pro Arg Ser
1               5                   10                  15

His Cys Trp Gly Leu Pro Leu Ala Cys Gly Thr Phe Val Gln Gly His
            20                  25                  30

Gln Ala Asp Ser Ser His Leu Leu Pro Leu Lys His Gln Gly Ala
        35                  40                  45

(2) INFORMATION FOR SEQ ID NO: 550:

(i) SEQUENCE CHARACTERISTICS:
(A) LENGTH: 168 amino acids
(B) TYPE: amino acid
(D) TOPOLOGY: linear
(xi) SEQUENCE DESCRIPTION: SEQ ID NO: 550:

Met Leu Leu Ser Leu Ala Ala Phe Ser Val Ile Ser Val Val Ser Tyr
1               5                   10                  15

Leu Ile Leu Ala Leu Leu Ser Val Thr Ile Ser Phe Arg Ile Tyr Lys
            20                  25                  30

Ser Val Ile Gln Ala Val Gln Lys Ser Glu Glu Gly His Pro Phe Lys
        35                  40                  45

Ala Tyr Leu Asp Val Asp Ile Thr Leu Ser Ser Glu Ala Phe His Asn
    50                  55                  60

Tyr Met Asn Ala Ala Met Val His Ile Asn Arg Ala Leu Lys Leu Ile
65                  70                  75                  80

Ile Arg Leu Phe Leu Val Glu Asp Leu Val Asp Ser Leu Lys Leu Ala
                85                  90                  95

Val Phe Met Trp Leu Met Thr Tyr Val Gly Ala Val Phe Asn Gly Ile
            100                 105                 110

Thr Leu Leu Ile Leu Ala Glu Leu Leu Ile Phe Ser Val Pro Ile Val
        115                 120                 125

Tyr Glu Lys Tyr Lys Thr Gln Ile Asp His Tyr Val Gly Ile Ala Arg
    130                 135                 140

Asp Gln Thr Lys Ser Ile Val Glu Lys Ile Gln Ala Lys Leu Pro Gly
145                 150                 155                 160

Ile Ala Lys Lys Lys Ala Glu Xaa
                165

(2) INFORMATION FOR SEQ ID NO: 551:

(i) SEQUENCE CHARACTERISTICS:
(A) LENGTH: 124 amino acids
(B) TYPE: amino acid
(D) TOPOLOGY: linear
(xi) SEQUENCE DESCRIPTION: SEQ ID NO: 551:

Ser Val Pro Phe His Leu Leu Val Val Leu Arg Ser Arg Ala Val Arg
  1               5                  10                  15

Ala Arg Arg Arg Arg Glu Pro Arg Ser Leu Pro Arg Pro Gly Asp Glu
             20                  25                  30

Glu Leu Gln Leu Leu Leu Cys Gly Ala Arg Ser Asp Phe Leu Glu Arg
         35                  40                  45

Cys Glu Glu Asp Trp Val Cys Leu Trp His His Ala Asp His Ala Ala
     50                  55                  60

Phe Pro Gly Ser Phe Gln Cys His Gln Cys Gly Phe Leu Pro His Pro
 65                  70                  75                  80

Gly Ser Ser Leu Cys His His Gln Leu Gln Asp Leu Gln Val Arg His
                 85                  90                  95

Pro Ser Cys Thr Glu Val Arg Arg Arg Pro Ser Ile Gln Ser Leu Pro
            100                 105                 110

Gly Arg Arg His Tyr Ser Val Leu Arg Ser Phe Pro
        115                 120

(2) INFORMATION FOR SEQ ID NO: 552:

(i) SEQUENCE CHARACTERISTICS:
(A) LENGTH: 177 amino acids
(B) TYPE: amino acid
(D) TOPOLOGY: linear
(xi) SEQUENCE DESCRIPTION: SEQ ID NO: 552:

(2) INFORMATION FOR SEQ ID NO: 553:

(i) SEQUENCE CHARACTERISTICS:
(A) LENGTH: 72 amino acids
(B) TYPE: amino acid
(D) TOPOLOGY: linear
(xi) SEQUENCE DESCRIPTION: SEQ ID NO: 553:

(2) INFORMATION FOR SEQ ID NO: 554:

(i) SEQUENCE CHARACTERISTICS:
(A) LENGTH: 45 amino acids
(B) TYPE: amino acid
(D) TOPOLOGY: linear

(xi) SEQUENCE DESCRIPTION: SEQ ID NO: 554:

Val Leu Arg Ile Ile Cys Leu Trp Pro Cys Gly Thr Thr Leu Pro Leu
1               5                   10                  15

Val Glu Lys Ala His Asp Ser His Ser Ala Asp Pro Val Cys Pro Gly
            20                  25                  30

Leu Thr Ala His Leu Pro Val Leu Leu Tyr Val Gln Leu
        35                  40                  45

(2) INFORMATION FOR SEQ ID NO: 555:

(i) SEQUENCE CHARACTERISTICS:
(A) LENGTH: 251 amino acids
(B) TYPE: amino acid
(D) TOPOLOGY: linear
(xi) SEQUENCE DESCRIPTION: SEQ ID NO: 555:

Met Lys His Ala Asp Pro Arg Ile Gln Gly Tyr Pro Leu Met Gly Ser
1               5                   10                  15

Pro Leu Leu Met Thr Ser Ile Leu Leu Thr Tyr Val Tyr Phe Val Leu
            20                  25                  30

Ser Leu Gly Pro Arg Ile Met Ala Asn Arg Lys Pro Phe Gln Leu Arg
        35                  40                  45

Gly Phe Met Ile Val Tyr Asn Phe Ser Leu Val Ala Leu Ser Leu Tyr
    50                  55                  60

Ile Val Tyr Glu Phe Leu Met Ser Gly Trp Leu Ser Thr Tyr Thr Trp
65                  70                  75                  80

Arg Cys Asp Pro Gln Asp Cys Thr Leu Gly Gln Cys Pro Ser Val Pro
                85                  90                  95

Ser Pro Xaa Thr Pro Val Thr Lys Ala Tyr Val Val Arg Thr Glu Gln
            100                 105                 110

Gly Thr Gly Pro Pro Leu Pro Thr Ala Ala Leu Gln Gly Pro Arg Leu
        115                 120                 125

Trp Phe Leu Thr His Phe Pro Arg Ala Ala Pro Gly Met Trp Pro His
    130                 135                 140

Cys Cys Leu Pro Leu Gln Ser Trp Gly Leu Lys Gly Leu Tyr Ser Tyr
145                 150                 155                 160

Phe Pro Leu Pro Ala Leu Lys Leu Gly Arg Gly Ala Leu Arg Ala Gly
                165                 170                 175

Pro Thr Lys Gly Leu Val Ala Phe Phe Leu Thr Gln Lys Arg Ser Ala
            180                 185                 190

Ile Met Ser Leu Trp Thr Gln Ser His Ser Ser Thr Pro His Thr Glu
        195                 200                 205

Ala Val Ala Ser Gly Pro Lys Val Arg Val Gly Gly Gly Leu Gly Ile
    210                 215                 220

Gln Pro Val Glu Ala Ala Tyr Ser Thr Cys Val Leu Ile Lys Ser Asp
225                 230                 235                 240

Arg Gly Asn His Glu Lys Lys Lys Lys Lys Lys
                245                 250

(2) INFORMATION FOR SEQ ID NO: 556:

        (i) SEQUENCE CHARACTERISTICS:
                (A) LENGTH: 19 amino acids
                (B) TYPE: amino acid
                (D) TOPOLOGY: linear
        (xi) SEQUENCE DESCRIPTION: SEQ ID NO: 556:

Gly Leu Ala Gly Leu Cys Gly Gln Leu Ser Ser Pro Ala Leu Cys Val
  1               5                  10                  15

Asn Arg Leu

(2) INFORMATION FOR SEQ ID NO: 557:

        (i) SEQUENCE CHARACTERISTICS:
                (A) LENGTH: 217 amino acids
                (B) TYPE: amino acid
                (D) TOPOLOGY: linear
        (xi) SEQUENCE DESCRIPTION: SEQ ID NO: 557:

Met Ile Thr Glu Lys Trp Gly Leu Asn Met Glu Tyr Cys Arg Gly Gln
  1               5                  10                  15

Ala Tyr Ile Xaa Ser Ser Gly Phe Ser Ser Lys Met Lys Val Val Ala
             20                  25                  30

Ser Arg Leu Leu Glu Lys Tyr Pro Gln Ala Ile Tyr Thr Leu Cys Ser
         35                  40                  45

Ser Cys Ala Leu Asn Met Trp Leu Ala Lys Ser Val Pro Val Met Gly
     50                  55                  60

Val Ser Val Ala Leu Gly Thr Ile Glu Glu Val Cys Ser Phe Phe His
 65                  70                  75                  80

Arg Ser Pro Gln Leu Leu Leu Glu Leu Asp Asn Val Ile Ser Val Leu
                 85                  90                  95

Phe Gln Asn Ser Lys Glu Arg Gly Lys Glu Leu Lys Glu Ile Cys His
            100                 105                 110

Ser Gln Trp Thr Gly Arg His Asp Ala Phe Glu Ile Leu Val Glu Leu
        115                 120                 125

Leu Gln Ala Leu Val Leu Cys Leu Asp Gly Ile Asn Ser Asp Thr Asn

130                 135                 140

Ile Arg Trp Asn Asn Tyr Ile Ala Gly Arg Ala Phe Val Leu Cys Ser
145                 150                 155                 160

Ala Val Ser Asp Phe Asp Phe Ile Val Thr Ile Val Val Leu Lys Asn
                165                 170                 175

Val Leu Ser Phe Thr Arg Ala Phe Gly Lys Asn Leu Gln Gly Gln Thr
            180                 185                 190

Ser Asp Val Phe Phe Ala Ala Gly Ser Leu Thr Ala Val Leu His Ser
        195                 200                 205

Leu Asn Glu Val Ile Gly Lys Tyr Xaa
    210                 215

(2) INFORMATION FOR SEQ ID NO: 558:

(i) SEQUENCE CHARACTERISTICS:
(A) LENGTH: 82 amino acids
(B) TYPE: amino acid
(D) TOPOLOGY: linear
(xi) SEQUENCE DESCRIPTION: SEQ ID NO: 558:

Leu Leu Lys Val Leu Cys Ile Leu Pro Val Met Lys Val Glu Asn Glu
  1               5                  10                  15

Arg Tyr Glu Asn Gly Arg Lys Arg Leu Lys Ala Tyr Leu Arg Asn Thr
             20                  25                  30

Leu Thr Asp Gln Arg Ser Ser Asn Leu Ala Leu Leu Asn Ile Asn Phe
         35                  40                  45

Asp Ile Lys His Asp Leu Asp Leu Met Val Asp Thr Tyr Ile Lys Leu
     50                  55                  60

Tyr Thr Ser Lys Ser Glu Leu Pro Thr Asp Asn Ser Glu Thr Val Glu
 65                  70                  75                  80

Asn Thr

(2) INFORMATION FOR SEQ ID NO: 559:

(i) SEQUENCE CHARACTERISTICS:
(A) LENGTH: 95 amino acids
(B) TYPE: amino acid
(D) TOPOLOGY: linear
(xi) SEQUENCE DESCRIPTION: SEQ ID NO: 559:

Met Val Leu Ile Leu Leu Asn Leu Leu Leu Gly Gln Phe Ser Cys Met
  1               5                  10                  15

Ser Pro Ala Ser His His Cys His Pro Leu Pro Thr Glu Met Pro Cys
             20                  25                  30

Ser Ser Asp Trp Gly Phe Asp Ser His Thr Val Tyr Pro Ser Cys Val
        35                  40                  45

Asp Ala Leu Leu Pro Lys Pro Ser Ala Asn Ser Phe Pro Asn Gly Ser
    50                  55                  60

Cys His Cys Gln Gly Leu Tyr Asn Gln Gln Gln Gln Asn Leu His Ala
65                  70                  75                  80

Ala Glu Gly Pro Ala Ser Leu Arg Cys Asn Lys Tyr Val Ser Thr
                85                  90                  95

(2) INFORMATION FOR SEQ ID NO: 560:

      (i) SEQUENCE CHARACTERISTICS:
            (A) LENGTH: 54 amino acids
            (B) TYPE: amino acid
            (D) TOPOLOGY: linear
      (xi) SEQUENCE DESCRIPTION: SEQ ID NO: 560:

Met Ile Pro Ala Tyr Ser Lys Asn Arg Ala Tyr Ala Ile Phe Phe Ile
 1               5                  10                  15

Val Phe Thr Val Ile Gly Asp Ala Pro Gly Ala Val Leu Ser Cys Ala
            20                  25                  30

Gly His Pro Cys Val Gly Phe Ala Ala Val Leu Val Ala Pro Leu Thr
        35                  40                  45

Val Ala Val Ser Ser Xaa
    50

(2) INFORMATION FOR SEQ ID NO: 561:

      (i) SEQUENCE CHARACTERISTICS:
            (A) LENGTH: 108 amino acids
            (B) TYPE: amino acid
            (D) TOPOLOGY: linear
      (xi) SEQUENCE DESCRIPTION: SEQ ID NO: 561:

Met Glu Val Pro Pro Pro Ala Pro Arg Ser Phe Leu Cys Arg Ala Leu
 1               5                  10                  15

Cys Leu Phe Pro Arg Val Phe Ala Ala Glu Ala Val Thr Ala Asp Ser
            20                  25                  30

Glu Val Leu Glu Glu Arg Gln Lys Arg Leu Pro Tyr Val Pro Glu Pro
        35                  40                  45

Tyr Tyr Pro Glu Ser Gly Trp Asp Arg Leu Arg Glu Leu Phe Gly Lys
    50                  55                  60

Asp Thr Val Asn Thr Ser Leu Asn Val Tyr Arg Asn Lys Asp Ala Leu
65                  70                  75                  80

Ser His Phe Val Ile Ala Gly Ala Val Thr Gly Ser Leu Phe Arg Ile
                85                  90                  95

Asn Val Gly Leu Arg Gly Trp Trp Leu Val Ala Xaa
            100                 105

(2) INFORMATION FOR SEQ ID NO: 562:

        (i) SEQUENCE CHARACTERISTICS:
                (A) LENGTH: 50 amino acids
                (B) TYPE: amino acid
                (D) TOPOLOGY: linear
        (xi) SEQUENCE DESCRIPTION: SEQ ID NO: 562:

Met Asn Trp Gly Leu Ser Ile Trp Leu His Tyr Tyr Glu Lys Lys Lys
  1               5                  10                  15

Glu Gln Val Phe Leu Val Ile Leu Ala His Val Val Arg Arg Cys Ala
            20                  25                  30

Ser Asp Gly Ile Leu Gln Phe Glu Ser Ser Leu Leu Lys Met Arg Arg
        35                  40                  45

Ala Pro
    50

(2) INFORMATION FOR SEQ ID NO: 563:

        (i) SEQUENCE CHARACTERISTICS:
                (A) LENGTH: 253 amino acids
                (B) TYPE: amino acid
                (D) TOPOLOGY: linear
        (xi) SEQUENCE DESCRIPTION: SEQ ID NO: 563:

Met Val Lys Val Cys Asn Asp Ser Asp Arg Trp Ser Leu Ile Ser Leu
  1               5                  10                  15

Ser Asn Asn Ser Gly Lys Asn Val Glu Leu Lys Phe Val Asp Ser Leu
            20                  25                  30

Arg Arg Gln Phe Glu Phe Ser Val Asp Ser Phe Gln Ile Lys Leu Asp
        35                  40                  45

Ser Leu Leu Leu Phe Tyr Glu Cys Ser Glu Asn Pro Met Thr Glu Thr
    50                  55                  60

Phe His Pro Thr Ile Ile Gly Glu Ser Val Tyr Gly Asp Phe Gln Glu
 65                  70                  75                  80

Ala Phe Asp His Leu Cys Asn Lys Ile Ile Ala Thr Arg Asn Pro Glu
                85                  90                  95

Glu Ile Arg Gly Gly Gly Leu Leu Lys Tyr Cys Asn Leu Leu Val Arg
            100                 105                 110

Gly Phe Arg Pro Ala Ser Asp Glu Ile Lys Thr Leu Gln Arg Tyr Met

115                 120                 125

Cys Ser Arg Phe Phe Ile Asp Phe Ser Asp Ile Gly Glu Gln Gln Arg
    130                 135                 140

Lys Leu Glu Ser Tyr Leu Gln Asn His Phe Val Gly Leu Glu Asp Arg
145                 150                 155                 160

Lys Tyr Glu Tyr Leu Met Thr Leu His Gly Val Val Asn Glu Ser Thr
                165                 170                 175

Val Cys Leu Met Gly His Glu Arg Arg Gln Thr Leu Asn Leu Ile Thr
            180                 185                 190

Met Leu Ala Ile Arg Val Leu Ala Asp Gln Asn Val Ile Pro Asn Val
        195                 200                 205

Ala Asn Val Thr Cys Tyr Tyr Gln Pro Ala Pro Tyr Val Ala Asp Ala
    210                 215                 220

Asn Phe Ser Asn Tyr Tyr Ile Ala Gln Val Gln Pro Val Phe Thr Cys
225                 230                 235                 240

Gln Gln Gln Thr Tyr Ser Thr Trp Leu Pro Cys Asn Xaa
                245                 250

(2) INFORMATION FOR SEQ ID NO: 564:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 564:

Met Ser Phe Leu Met Trp Leu Met Ser Leu Ala Ile Thr Ser Gln Pro
  1               5                  10                  15

Pro Met

(2) INFORMATION FOR SEQ ID NO: 565:

(i) SEQUENCE CHARACTERISTICS:
(A) LENGTH: 80 amino acids
(B) TYPE: amino acid
(D) TOPOLOGY: linear
(xi) SEQUENCE DESCRIPTION: SEQ ID NO: 565:

Met Ala Pro Lys Gly Lys Val Gly Thr Arg Gly Lys Lys Gln Ile Phe
  1               5                  10                  15

Glu Glu Asn Arg Glu Thr Leu Lys Phe Tyr Leu Arg Ile Ile Leu Gly
             20                  25                  30

Ala Asn Ala Ile Tyr Cys Leu Val Thr Leu Val Phe Phe Tyr Ser Ser
        35                  40                  45

Ala Ser Phe Trp Ala Trp Leu Ala Leu Gly Phe Ser Leu Ala Val Tyr
    50                  55                  60

Gly Ala Ser Tyr His Ser Met Ser Ser Met Ala Arg Ala Ala Phe Phe
65                  70                  75                  80

(2) INFORMATION FOR SEQ ID NO: 566:

    (i) SEQUENCE CHARACTERISTICS:
        (A) LENGTH: 73 amino acids
        (B) TYPE: amino acid
        (D) TOPOLOGY: linear
    (xi) SEQUENCE DESCRIPTION: SEQ ID NO: 566:

His Leu Lys Asp Val Ile Leu Leu Thr Ala Ile Val Gln Val Leu Ser
 1               5                  10                  15

Cys Phe Ser Leu Tyr Val Trp Ser Phe Trp Leu Leu Ala Pro Gly Arg
            20                  25                  30

Ala Leu Tyr Leu Leu Trp Val Asn Val Leu Gly Pro Trp Phe Thr Ala
        35                  40                  45

Asp Ser Gly Thr Pro Ala Pro Glu His Asn Glu Lys Arg Gln Arg Arg
    50                  55                  60

Gln Glu Arg Arg Gln Met Lys Arg Leu
65                  70

(2) INFORMATION FOR SEQ ID NO: 567:

    (i) SEQUENCE CHARACTERISTICS:
        (A) LENGTH: 263 amino acids
        (B) TYPE: amino acid
        (D) TOPOLOGY: linear
    (xi) SEQUENCE DESCRIPTION: SEQ ID NO: 567:

Met Asp Cys Pro Ala Leu Pro Pro Gly Trp Lys Lys Glu Glu Val Ile
 1               5                  10                  15

Arg Lys Ser Gly Leu Ser Ala Gly Lys Ser Asp Val Tyr Tyr Phe Ser
            20                  25                  30

Pro Ser Gly Lys Lys Phe Arg Ser Lys Pro Gln Leu Ala Arg Tyr Leu
        35                  40                  45

Gly Asn Thr Val Asp Leu Ser Ser Phe Asp Phe Arg Thr Gly Lys Met
    50                  55                  60

Met Pro Ser Lys Leu Gln Lys Asn Lys Gln Arg Leu Arg Asn Asp Pro
65                  70                  75                  80

Leu Asn Gln Asn Lys Gly Lys Pro Asp Leu Asn Thr Thr Leu Pro Ile
                85                  90                  95

Arg Gln Thr Ala Ser Ile Phe Lys Gln Pro Val Thr Lys Val Thr Asn
            100                 105                 110

His Pro Ser Asn Lys Val Lys Ser Asp Pro Gln Arg Met Asn Glu Gln
        115                 120                 125

Pro Arg Gln Leu Phe Trp Glu Lys Arg Leu Gln Gly Leu Ser Ala Ser
    130                 135                 140

Asp Val Thr Glu Gln Ile Ile Lys Thr Met Glu Leu Pro Lys Gly Leu
145                 150                 155                 160

Gln Gly Val Gly Pro Gly Ser Asn Asp Glu Thr Leu Leu Ser Ala Val
                165                 170                 175

Ala Ser Ala Leu His Thr Ser Ser Ala Pro Ile Thr Gly Gln Val Ser
            180                 185                 190

Ala Ala Val Glu Lys Asn Pro Ala Val Trp Leu Asn Thr Ser Gln Pro
        195                 200                 205

Leu Cys Lys Ala Phe Ile Val Thr Asp Glu Asp Ile Arg Lys Gln Glu
    210                 215                 220

Glu Arg Val Gln Gln Val Arg Lys Lys Leu Glu Glu Ala Leu Met Ala
225                 230                 235                 240

Asp Ile Leu Ser Arg Ala Ala Asp Thr Glu Glu Met Asp Ile Glu Met
                245                 250                 255

Asp Ser Gly Asp Glu Ala Xaa
            260

(2) INFORMATION FOR SEQ ID NO: 568:

(i) SEQUENCE CHARACTERISTICS:
(A) LENGTH: 70 amino acids
(B) TYPE: amino acid
(D) TOPOLOGY: linear
(xi) SEQUENCE DESCRIPTION: SEQ ID NO: 568:

Met Met Arg Pro Phe Tyr Leu Leu Leu Pro Val Leu Cys Thr Gln Ala
  1               5                  10                  15

Leu Arg Gln Ser Gln Gly Lys Ser Pro Leu Leu Trp Lys Arg Thr Leu
             20                  25                  30

Leu Phe Gly Leu Thr His Leu Asn Pro Ser Ala Lys Leu Leu Leu Ser
         35                  40                  45

Gln Met Lys Thr Ser Gly Asn Arg Lys Ser Glu Tyr Ser Lys Tyr Ala
     50                  55                  60

Arg Asn Trp Lys Lys His
 65                  70

(2) INFORMATION FOR SEQ ID NO: 569:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 569:

Met Pro Val Thr Ser Lys Arg Thr Leu Phe Phe Pro Asp Pro Cys Ser
1               5                   10                  15

Tyr Asp Thr Pro Pro Pro Asp Cys His Cys His Ser Phe Arg Ala Glu
            20                  25                  30

Leu Leu

(2) INFORMATION FOR SEQ ID NO: 570:

(i) SEQUENCE CHARACTERISTICS:
(A) LENGTH: 104 amino acids
(B) TYPE: amino acid
(D) TOPOLOGY: linear
(xi) SEQUENCE DESCRIPTION: SEQ ID NO: 570:

Met Asn Ser Arg Gly Met Trp Leu Thr Tyr Ala Leu Gly Val Gly Leu
1               5                   10                  15

Leu His Ile Val Leu Leu Ser Ile Pro Phe Phe Ser Val Pro Val Ala
            20                  25                  30

Trp Thr Leu Thr Asn Ile Ile His Asn Leu Gly Met Tyr Val Phe Leu
        35                  40                  45

His Ala Val Lys Gly Thr Pro Phe Glu Thr Pro Asp Gln Gly Lys Ser
    50                  55                  60

Lys Ala Pro Asn Ser Leu Gly Thr Thr Gly Leu Trp Ser Thr Val Tyr
65                  70                  75                  80

Ile Phe Thr Glu Val Phe His Asn Phe Ser Asn Asn Ser Ile Phe Ser
                85                  90                  95

Gly Lys Phe Leu Tyr Glu Val Xaa
            100

(2) INFORMATION FOR SEQ ID NO: 571:

(i) SEQUENCE CHARACTERISTICS:
(A) LENGTH: 132 amino acids
(B) TYPE: amino acid
(D) TOPOLOGY: linear
(xi) SEQUENCE DESCRIPTION: SEQ ID NO: 571:

Met Trp Leu Thr Tyr Ala Leu Gly Val Gly Leu Leu His Ile Val Leu
1               5                   10                  15

Leu Ser Ile Pro Phe Phe Ser Val Pro Val Ala Trp Thr Leu Thr Asn
            20                  25                  30

Ile Ile His Asn Leu Gly Met Tyr Val Phe Leu His Ala Val Lys Gly
        35                  40                  45

Thr Pro Phe Glu Thr Pro Asp Gln Gly Lys Ala Arg Leu Leu Thr His
    50                  55                  60

Trp Glu Gln Leu Asp Tyr Gly Val Gln Phe Thr Ser Ser Arg Lys Phe
65                  70                  75                  80

Phe Thr Ile Ser Pro Ile Ile Leu Tyr Phe Leu Ala Ser Phe Tyr Thr
                85                  90                  95

Lys Tyr Asp Pro Thr His Phe Ile Leu Asn Thr Ala Ser Leu Leu Ser
            100                 105                 110

Val Leu Ile Pro Lys Met Pro Gln Leu His Gly Val Arg Ile Phe Gly
        115                 120                 125

Ile Asn Lys Tyr
    130

(2) INFORMATION FOR SEQ ID NO: 572:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 572:

Met Asn Lys Trp Ile Cys Glu Met His Cys Tyr Leu Val Leu Leu Ser
1               5                   10                  15

Val Cys Ser Pro Ser Ala Leu Arg Arg Val Arg His Thr Leu Ser Arg
            20                  25                  30

(2) INFORMATION FOR SEQ ID NO: 573:

(i) SEQUENCE CHARACTERISTICS:
(A) LENGTH: 28 amino acids
(B) TYPE: amino acid
(D) TOPOLOGY: linear
(xi) SEQUENCE DESCRIPTION: SEQ ID NO: 573:

Met Pro Val Leu Ser Leu Leu Cys Thr Leu Ile Val Ser Phe Gln Ser
1               5                   10                  15

Ala Asp Ser Cys Glu Val Phe Leu Asn Cys Ser Leu
            20                  25

(2) INFORMATION FOR SEQ ID NO: 574:

        (i) SEQUENCE CHARACTERISTICS:
              (A) LENGTH: 40 amino acids
              (B) TYPE: amino acid
              (D) TOPOLOGY: linear
        (xi) SEQUENCE DESCRIPTION: SEQ ID NO: 574:

Met Lys Val Ser Thr Met Leu Trp Phe Leu Cys Trp Glu Gln Ser His
  1               5                  10                  15

Phe Leu Arg Glu Trp Glu Asp Leu Ser Thr Phe Leu Ile Leu Ile Gln
            20                  25                  30

Met Glu Cys Gln Tyr Gly Asn Ser
        35                  40

(2) INFORMATION FOR SEQ ID NO: 575:

        (i) SEQUENCE CHARACTERISTICS:
              (A) LENGTH: 30 amino acids
              (B) TYPE: amino acid
              (D) TOPOLOGY: linear
        (xi) SEQUENCE DESCRIPTION: SEQ ID NO: 575:

Met Gly Leu Pro Leu Met Ala Leu Met Trp Ser Thr Leu Pro Ala Ser
  1               5                  10                  15

Ala Gly Val Asn Phe Ile Leu Ala Leu Pro Leu Leu Xaa Leu
            20                  25                  30

(2) INFORMATION FOR SEQ ID NO: 576:

        (i) SEQUENCE CHARACTERISTICS:
              (A) LENGTH: 29 amino acids
              (B) TYPE: amino acid
              (D) TOPOLOGY: linear
        (xi) SEQUENCE DESCRIPTION: SEQ ID NO: 576:

Met Lys Arg Gly Cys Leu Gly Leu Leu Phe Phe Ser Cys Cys Ser Ser
  1               5                  10                  15

Ala Pro Thr Met Leu Leu Cys Asp Tyr Leu Asn Trp Phe
            20                  25

(2) INFORMATION FOR SEQ ID NO: 577:

        (i) SEQUENCE CHARACTERISTICS:
              (A) LENGTH: 92 amino acids

(B) TYPE: amino acid
(D) TOPOLOGY: linear
(xi) SEQUENCE DESCRIPTION: SEQ ID NO: 577:

Met Lys Leu Leu Leu Gly Ile Ala Leu Leu Ala Tyr Val Ala Ser Val
1               5                   10                  15

Trp Gly Asn Phe Val Asn Met Arg Ser Ile Gln Glu Asn Gly Glu Leu
            20                  25                  30

Lys Ile Glu Ser Lys Ile Glu Glu Met Val Glu Pro Leu Arg Glu Lys
        35                  40                  45

Ile Arg Asp Leu Glu Lys Ser Phe Thr Gln Lys Tyr Pro Pro Val Lys
    50                  55                  60

Phe Leu Ser Glu Lys Asp Arg Lys Arg Ile Leu Xaa Asn Arg Arg Arg
65                  70                  75                  80

Xaa Val Arg Gly Leu Pro Ser Xaa Leu Thr Asn Ser
                85                  90

(2) INFORMATION FOR SEQ ID NO: 578:

(i) SEQUENCE CHARACTERISTICS:
(A) LENGTH: 42 amino acids
(B) TYPE: amino acid
(D) TOPOLOGY: linear
(xi) SEQUENCE DESCRIPTION: SEQ ID NO: 578:

Met Lys Phe Ser Leu Val Leu Leu Ile Lys Ile Ile Ser Phe Glu Arg
1               5                   10                  15

Leu Leu Ile Phe Leu Phe Pro Leu Ser Phe Leu Pro Asn Ile Trp Arg
            20                  25                  30

Arg Val Met Val Asn Leu Asn Ile Leu Phe
        35                  40

(2) INFORMATION FOR SEQ ID NO: 579:

(i) SEQUENCE CHARACTERISTICS:
(A) LENGTH: 70 amino acids
(B) TYPE: amino acid
(D) TOPOLOGY: linear
(xi) SEQUENCE DESCRIPTION: SEQ ID NO: 579:

Leu Ala Gln Glu Cys Pro Pro His Ile Pro Ser Ser Phe Phe Leu Val
1               5                   10                  15

Lys Leu Leu Phe Ile Pro Trp Leu Ala Ser Leu Leu Pro Pro Leu Ser
            20                  25                  30

Thr Phe Thr Ser Asp Phe Tyr Phe Met Glu Phe Gly Ile Glu Val Lys
        35                  40                  45

Leu Gln Gln Cys Arg Gln His Gln Val Leu Gln Glu Lys Asn Thr Lys
    50                  55                  60

Lys Phe Asn Lys Lys Lys
65                  70

(2) INFORMATION FOR SEQ ID NO: 580:

    (i) SEQUENCE CHARACTERISTICS:
        (A) LENGTH: 110 amino acids
        (B) TYPE: amino acid
        (D) TOPOLOGY: linear
    (xi) SEQUENCE DESCRIPTION: SEQ ID NO: 580:

Met Leu Arg Leu Leu Leu Leu Val Ala Phe Ala Leu Val Val Val Leu
1               5                   10                  15

Phe His Val Leu Leu Ala Pro Ile Thr Ala Leu Phe His Thr His Phe
            20                  25                  30

Tyr Asp Arg Leu Gln Asp Ala Gly Ser Arg Trp Pro Glu Leu Tyr Leu
        35                  40                  45

Tyr Ser Arg Ala Asp Glu Val Val Leu Ala Arg Asp Ile Glu Arg Met
    50                  55                  60

Val Glu Ala Arg Leu Ala Arg Arg Val Leu Ala Arg Ser Val Asp Phe
65                  70                  75                  80

Val Ser Ser Ala His Val Ser His Leu Arg Asp Tyr Pro Thr Tyr Tyr
                85                  90                  95

Thr Ser Leu Cys Val Asp Phe Met Arg Asn Cys Val Arg Cys
            100                 105                 110

(2) INFORMATION FOR SEQ ID NO: 581:

    (i) SEQUENCE CHARACTERISTICS:
        (A) LENGTH: 30 amino acids
        (B) TYPE: amino acid
        (D) TOPOLOGY: linear
    (xi) SEQUENCE DESCRIPTION: SEQ ID NO: 581:

Met Phe Lys Leu Glu Glu Cys Gly Lys Thr Thr Phe Leu Leu Ser Met
1               5                   10                  15

Ala Leu Tyr Phe Trp Trp Ile Val Gln Thr Thr Lys Gly Cys
            20                  25                  30

(2) INFORMATION FOR SEQ ID NO: 582:

    (i) SEQUENCE CHARACTERISTICS:
        (A) LENGTH: 71 amino acids
        (B) TYPE: amino acid

(D) TOPOLOGY: linear
(xi) SEQUENCE DESCRIPTION: SEQ ID NO: 582:

Met Glu Ser Asp Ala Leu Leu Leu Thr Ile Phe Trp Ile Ile Ala Arg
1               5                   10                  15

Ser Ser Val Arg Ser Val Gly Lys Ser Ser Gln Arg Ser Phe Thr Thr
            20                  25                  30

Ile Thr Gln Leu Arg Ser Thr His Thr Gly Pro Ser Arg Arg Ser Tyr
        35                  40                  45

Leu Ile Trp Trp Asn Gly Gly Pro Lys Arg Thr Ile Ser Tyr Val Ser
    50                  55                  60

Arg Arg Phe Arg Ser Phe Arg
65                  70

(2) INFORMATION FOR SEQ ID NO: 583:

(i) SEQUENCE CHARACTERISTICS:
(A) LENGTH: 47 amino acids
(B) TYPE: amino acid
(D) TOPOLOGY: linear
(xi) SEQUENCE DESCRIPTION: SEQ ID NO: 583:

Val Gly Leu Phe Gln Pro Lys Thr Phe Gln Val Pro Val Thr Asp Leu
1               5                   10                  15

Tyr Ile Phe Ile Lys Ile Tyr Ser Glu Ile Gly Pro Ile Met His Val
            20                  25                  30

Leu Cys Pro Gly Tyr Ser Gln Ser Pro Ser Thr Pro Pro Trp Thr
        35                  40                  45

(2) INFORMATION FOR SEQ ID NO: 584:

(i) SEQUENCE CHARACTERISTICS:
(A) LENGTH: 39 amino acids
(B) TYPE: amino acid
(D) TOPOLOGY: linear
(xi) SEQUENCE DESCRIPTION: SEQ ID NO: 584:

Met Trp Phe Gly Ser Asp Arg Ser Asp Leu Arg Ile Gly Thr Ala Phe
1               5                   10                  15

Leu Phe Asp Leu Val Cys Asp Leu Cys Ile His Ala Trp Lys Pro Pro
            20                  25                  30

Gly Leu Val Arg Phe Ser Phe
        35

(2) INFORMATION FOR SEQ ID NO: 585:

(i) SEQUENCE CHARACTERISTICS:
(A) LENGTH: 19 amino acids
(B) TYPE: amino acid
(D) TOPOLOGY: linear
(xi) SEQUENCE DESCRIPTION: SEQ ID NO: 585:

Met Leu Asn Thr Ala Ser Leu Asn Leu Pro Trp Lys Val Gln Leu Phe
1               5                   10                  15

Ala His Ala

(2) INFORMATION FOR SEQ ID NO: 586:

(i) SEQUENCE CHARACTERISTICS:
(A) LENGTH: 23 amino acids
(B) TYPE: amino acid
(D) TOPOLOGY: linear
(xi) SEQUENCE DESCRIPTION: SEQ ID NO: 586:

Met Ser Ala Cys Leu Leu Leu Phe Leu Ala Phe Ser Trp Lys Arg Lys
1               5                   10                  15

Gly Leu Trp Ser Gly Pro Gly
            20

(2) INFORMATION FOR SEQ ID NO: 587:

(i) SEQUENCE CHARACTERISTICS:
(A) LENGTH: 69 amino acids
(B) TYPE: amino acid
(D) TOPOLOGY: linear
(xi) SEQUENCE DESCRIPTION: SEQ ID NO: 587:

(2) INFORMATION FOR SEQ ID NO: 588:

(i) SEQUENCE CHARACTERISTICS:
(A) LENGTH: 77 amino acids
(B) TYPE: amino acid

(D) TOPOLOGY: linear
(xi) SEQUENCE DESCRIPTION: SEQ ID NO: 588:

Met Gly Pro Pro Met Leu Gln Glu Ile Ser Asn Leu Phe Leu Ile Leu
1               5                   10                  15

Leu Met Met Gly Ala Ile Phe Thr Leu Ala Ala Leu Lys Glu Ser Leu
            20                  25                  30

Ser Thr Cys Ile Pro Ala Ile Val Cys Leu Gly Phe Leu Leu Leu Leu
        35                  40                  45

Asn Val Gly Gln Leu Leu Ala Gln Thr Lys Lys Val Val Arg Pro Thr
    50                  55                  60

Arg Lys Lys Thr Leu Ser Thr Phe Lys Glu Ser Trp Lys
65                  70                  75

(2) INFORMATION FOR SEQ ID NO: 589:

(i) SEQUENCE CHARACTERISTICS:
(A) LENGTH: 155 amino acids
(B) TYPE: amino acid
(D) TOPOLOGY: linear
(xi) SEQUENCE DESCRIPTION: SEQ ID NO: 589:

Met Ala Leu Leu Leu Ser Val Leu Arg Val Leu Leu Gly Gly Phe Phe
1               5                   10                  15

Ala Leu Val Gly Leu Ala Lys Leu Ser Glu Glu Ile Ser Ala Pro Val
            20                  25                  30

Ser Glu Arg Met Asn Ala Leu Phe Val Gln Phe Ala Glu Val Phe Pro
        35                  40                  45

Leu Lys Val Phe Gly Tyr Gln Pro Asp Pro Leu Asn Tyr Gln Ile Ala
    50                  55                  60

Val Gly Phe Leu Glu Leu Leu Ala Gly Leu Leu Leu Val Met Gly Pro
65                  70                  75                  80

Pro Met Leu Gln Glu Ile Ser Asn Leu Phe Leu Ile Leu Leu Met Met
                85                  90                  95

Gly Ala Ile Phe Thr Leu Ala Ala Leu Lys Glu Ser Leu Ser Thr Cys
            100                 105                 110

Ile Pro Ala Ile Val Cys Leu Gly Phe Leu Leu Leu Leu Asn Val Gly
        115                 120                 125

Gln Leu Leu Ala Gln Thr Lys Lys Val Val Arg Pro Thr Arg Lys Lys
    130                 135                 140

Thr Leu Ser Thr Phe Lys Glu Ser Trp Lys Xaa
145                 150                 155

(2) INFORMATION FOR SEQ ID NO: 590:

(i) SEQUENCE CHARACTERISTICS:
(A) LENGTH: 24 amino acids
(B) TYPE: amino acid
(D) TOPOLOGY: linear
(xi) SEQUENCE DESCRIPTION: SEQ ID NO: 590:

Met Pro Glu Thr Arg Leu Gly His Arg Gln Gln Phe Ala Val Phe His
1               5                   10                  15

Leu Xaa Pro Val Pro Pro Cys Gly
            20

(2) INFORMATION FOR SEQ ID NO: 591:

(i) SEQUENCE CHARACTERISTICS:
(A) LENGTH: 38 amino acids
(B) TYPE: amino acid
(D) TOPOLOGY: linear
(xi) SEQUENCE DESCRIPTION: SEQ ID NO: 591:

Met Leu Thr Phe Leu Phe Ser Ala Cys Ala Thr Cys Leu Gly Lys Leu
1               5                   10                  15

Ala Ser Pro Leu Ala Pro Val Gly Pro Gln Gln Arg Gly Xaa Pro Pro
            20                  25                  30

Gly Pro Pro Leu Leu Ser
        35

(2) INFORMATION FOR SEQ ID NO: 592:

(i) SEQUENCE CHARACTERISTICS:
(A) LENGTH: 69 amino acids
(B) TYPE: amino acid
(D) TOPOLOGY: linear
(xi) SEQUENCE DESCRIPTION: SEQ ID NO: 592:

(2) INFORMATION FOR SEQ ID NO: 593:

(i) SEQUENCE CHARACTERISTICS:
(A) LENGTH: 308 amino acids
(B) TYPE: amino acid
(D) TOPOLOGY: linear
(xi) SEQUENCE DESCRIPTION: SEQ ID NO: 593:

Asn Leu Arg Val Arg Leu Gly Asp Val Ile Ser Ile Gln Pro Cys Pro
1               5                   10                  15

Asp Val Lys Tyr Gly Lys Arg Ile His Val Leu Pro Ile Asp Asp Thr
            20                  25                  30

Val Glu Gly Ile Thr Gly Asn Leu Phe Glu Val Tyr Leu Lys Pro Tyr
        35                  40                  45

Phe Leu Glu Ala Tyr Arg Pro Ile Arg Lys Gly Asp Ile Phe Leu Val
    50                  55                  60

Arg Gly Gly Met Arg Ala Val Glu Phe Lys Val Val Glu Thr Asp Pro
65                  70                  75                  80

Ser Pro Tyr Cys Ile Val Ala Pro Asp Thr Val Ile His Cys Glu Gly
                85                  90                  95

Glu Pro Ile Lys Arg Glu Asp Glu Glu Glu Ser Leu Asn Glu Val Gly
            100                 105                 110

Tyr Asp Asp Ile Gly Gly Cys Arg Lys Gln Leu Ala Gln Ile Lys Glu
        115                 120                 125

Met Val Glu Leu Pro Leu Arg His Pro Ala Leu Phe Lys Ala Ile Gly
    130                 135                 140

Val Lys Pro Pro Arg Gly Ile Leu Leu Tyr Gly Pro Pro Gly Thr Gly
145                 150                 155                 160

Lys Thr Leu Ile Ala Arg Ala Val Ala Asn Glu Thr Gly Ala Phe Phe
                165                 170                 175

Phe Leu Ile Asn Gly Pro Glu Ile Met Ser Lys Leu Ala Gly Glu Ser
            180                 185                 190

Glu Ser Asn Leu Arg Lys Ala Phe Glu Glu Ala Glu Lys Asn Ala Pro
        195                 200                 205

Ala Ile Ile Phe Ile Asp Glu Leu Asp Ala Ile Ala Pro Lys Arg Glu
    210                 215                 220

Lys Thr His Gly Glu Val Glu Arg Arg Ile Val Ser Gln Leu Leu Thr
225                 230                 235                 240

Leu Met Asp Gly Leu Lys Gln Arg Ala His Val Ile Val Met Ala Ala
                245                 250                 255

Thr Asn Arg Pro Asn Ser Ile Asp Pro Ala Leu Arg Arg Phe Gly Arg
            260                 265                 270

Phe Asp Arg Glu Val Asp Ile Gly Ile Pro Asp Ala Thr Gly Arg Leu

275                 280                 285

Glu Ile Leu Gln Ile His Thr Lys Asn Met Lys Leu Ala Asp Asp Val
    290                 295                 300

Asp Leu Glu Gln
305

(2) INFORMATION FOR SEQ ID NO: 594:

        (i) SEQUENCE CHARACTERISTICS:
              (A) LENGTH: 22 amino acids
              (B) TYPE: amino acid
              (D) TOPOLOGY: linear
        (xi) SEQUENCE DESCRIPTION: SEQ ID NO: 594:

Met Gln Ile Lys Leu Leu Lys Ser Val Lys Thr Val Phe Ala Ile Thr
  1               5                  10                  15

Leu Leu Val Leu Phe Leu
            20

(2) INFORMATION FOR SEQ ID NO: 595:

        (i) SEQUENCE CHARACTERISTICS:
              (A) LENGTH: 24 amino acids
              (B) TYPE: amino acid
              (D) TOPOLOGY: linear
        (xi) SEQUENCE DESCRIPTION: SEQ ID NO: 595:

Met Phe Pro Lys Phe Cys Pro Ile Leu Ser Leu Val Asp Phe Ile Ser
  1               5                  10                  15

His Arg Asp Lys Pro Glu Thr Glu
            20

(2) INFORMATION FOR SEQ ID NO: 596:

        (i) SEQUENCE CHARACTERISTICS:
              (A) LENGTH: 24 amino acids
              (B) TYPE: amino acid
              (D) TOPOLOGY: linear
        (xi) SEQUENCE DESCRIPTION: SEQ ID NO: 596:

Met Leu Ile Glu Cys Ala Trp Gln Leu Met Phe Leu Leu Leu Lys Val
  1               5                  10                  15

Glu Gln Leu Gly Ile Leu Asp Lys
            20

(2) INFORMATION FOR SEQ ID NO: 597:

(i) SEQUENCE CHARACTERISTICS:
(A) LENGTH: 1 amino acids
(B) TYPE: amino acid
(D) TOPOLOGY: linear
(xi) SEQUENCE DESCRIPTION: SEQ ID NO: 597:

Met
1

(2) INFORMATION FOR SEQ ID NO: 598:

(i) SEQUENCE CHARACTERISTICS:
(A) LENGTH: 8 amino acids
(B) TYPE: amino acid
(D) TOPOLOGY: linear
(xi) SEQUENCE DESCRIPTION: SEQ ID NO: 598:

Met Cys Ile Met Ser Ala Leu Val
1               5

(2) INFORMATION FOR SEQ ID NO: 599:

(i) SEQUENCE CHARACTERISTICS:
(A) LENGTH: 25 amino acids
(B) TYPE: amino acid
(D) TOPOLOGY: linear
(xi) SEQUENCE DESCRIPTION: SEQ ID NO: 599:

Met Phe Leu Val Trp Phe Phe Trp Gly Leu Ile Ser Ala Leu Ser Asn
1               5                   10                  15

Val His Thr Pro Ser Arg Leu Pro Ala
            20                  25

(2) INFORMATION FOR SEQ ID NO: 600:

(i) SEQUENCE CHARACTERISTICS:
(A) LENGTH: 27 amino acids
(B) TYPE: amino acid
(D) TOPOLOGY: linear
(xi) SEQUENCE DESCRIPTION: SEQ ID NO: 600:

Met Xaa Gly Leu Ser Leu Ile Leu Thr Val Thr Leu Leu Ala Val Ser
1               5                   10                  15

Asp Ser Ala Ala Thr Cys Ile Val Ala Lys Gly
            20                  25

(2) INFORMATION FOR SEQ ID NO: 601:

(i) SEQUENCE CHARACTERISTICS:
(A) LENGTH: 61 amino acids

(B) TYPE: amino acid
(D) TOPOLOGY: linear
(xi) SEQUENCE DESCRIPTION: SEQ ID NO: 601:

Met Trp Thr Arg Ser Ser Arg Cys Leu Leu Leu Cys Ile Pro Gly Xaa
1               5                   10                  15

Ser Arg Arg Arg Arg Ala Gly Ser Gly Met Lys Pro Arg Ser Trp Ser
            20                  25                  30

Ala Trp Arg Pro Ser Gly Gly Thr Gly Thr Ser Ser Ser Gln Ser Ser
        35                  40                  45

Thr Gln Ser Arg Thr Leu Ser Ala Thr Ala Ser Pro Ala
    50                  55                  60

(2) INFORMATION FOR SEQ ID NO: 602:

(i) SEQUENCE CHARACTERISTICS:
(A) LENGTH: 29 amino acids
(B) TYPE: amino acid
(D) TOPOLOGY: linear
(xi) SEQUENCE DESCRIPTION: SEQ ID NO: 602:

Met Arg Glu Thr Ser Ile Arg Val Leu Leu Met Leu Pro Ala Leu Glu
1               5                   10                  15

Ser Thr Ser Gly Leu Ser Ala Phe Met Gly Leu Gly Thr
            20                  25

(2) INFORMATION FOR SEQ ID NO: 603:

(i) SEQUENCE CHARACTERISTICS:
(A) LENGTH: 69 amino acids
(B) TYPE: amino acid
(D) TOPOLOGY: linear
(xi) SEQUENCE DESCRIPTION: SEQ ID NO: 603:

Met Pro Pro Lys Gln Glu Leu Gly Ser Gly Val Gly Glu Leu Ala Lys
1               5                   10                  15

Asn Ser Lys Arg Gln His Trp Asn His Arg Trp Lys Lys Tyr Leu Lys
            20                  25                  30

Leu Ile Arg Trp Glu Asp Gly Leu Leu Leu Glu Gly Leu Leu Leu Val
        35                  40                  45

Leu Glu His Cys Ala Thr Met Ala Trp Asp Cys Leu Met Arg Leu Glu
    50                  55                  60

Leu Leu Lys Arg Leu
65

(2) INFORMATION FOR SEQ ID NO: 604:

(i) SEQUENCE CHARACTERISTICS:
(A) LENGTH: 24 amino acids
(B) TYPE: amino acid
(D) TOPOLOGY: linear
(xi) SEQUENCE DESCRIPTION: SEQ ID NO: 604:

Lys Ile Val Tyr Ile Leu Gly Asn Pro Leu Lys Phe Asn Ser Arg Val
1               5                   10                  15

Ile His His Leu Val Leu Leu Gln
            20

(2) INFORMATION FOR SEQ ID NO: 605:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 605:

Met Asn Leu His Gln Arg Arg Leu Leu Leu Ile Gly His Leu Met Thr
1               5                   10                  15

Leu Val Lys Ala Ser Lys Ser Phe Ser Phe Thr Glu Ile Thr Ser Ser
            20                  25                  30

Arg Lys Lys
        35

(2) INFORMATION FOR SEQ ID NO: 606:

(i) SEQUENCE CHARACTERISTICS:
(A) LENGTH: 130 amino acids
(B) TYPE: amino acid
(D) TOPOLOGY: linear
(xi) SEQUENCE DESCRIPTION: SEQ ID NO: 606:

Leu Leu Gly Tyr Gly Leu Phe Gly His Cys Ile Val Leu Phe Ile Thr
1               5                   10                  15

Tyr Asn Ile His Leu His Ala Leu Phe Tyr Leu Phe Trp Leu Leu Val
            20                  25                  30

Gly Gly Leu Ser Thr Leu Arg Met Val Ala Val Leu Val Ser Arg Thr
        35                  40                  45

Val Gly Pro Thr Gln Arg Leu Leu Leu Cys Gly Thr Leu Ala Ala Leu
    50                  55                  60

His Met Leu Phe Leu Leu Tyr Leu His Phe Ala Tyr His Lys Val Xaa
65                  70                  75                  80

Glu Gly Ile Leu Asp Thr Leu Glu Gly Pro Asn Ile Pro Pro Ile Gln
                85                  90                  95

Arg Val Pro Arg Asp Ile Pro Ala Met Leu Pro Ala Ala Arg Leu Pro
            100                 105                 110

Thr Thr Val Leu Asn Ala Thr Ala Lys Ala Val Ala Val Thr Leu Gln
        115                 120                 125

Ser His
    130

(2) INFORMATION FOR SEQ ID NO: 607:

        (i) SEQUENCE CHARACTERISTICS:
              (A) LENGTH: 23 amino acids
              (B) TYPE: amino acid
              (D) TOPOLOGY: linear
        (xi) SEQUENCE DESCRIPTION: SEQ ID NO: 607:

Met Leu Val Ile Phe Leu Phe Thr Ser Leu Leu Lys Ile Pro Ser Ser
  1               5                  10                  15

Val Pro Gly Leu Ile Asn Val
             20

(2) INFORMATION FOR SEQ ID NO: 608:

        (i) SEQUENCE CHARACTERISTICS:
              (A) LENGTH: 6 amino acids
              (B) TYPE: amino acid
              (D) TOPOLOGY: linear
        (xi) SEQUENCE DESCRIPTION: SEQ ID NO: 608:

Glu Leu Asp Tyr Ile Leu
  1               5

(2) INFORMATION FOR SEQ ID NO: 609:

        (i) SEQUENCE CHARACTERISTICS:
              (A) LENGTH: 232 amino acids
              (B) TYPE: amino acid
              (D) TOPOLOGY: linear
        (xi) SEQUENCE DESCRIPTION: SEQ ID NO: 609:

Met Ala Pro Pro Gly Trp Gln Xaa Xaa Xaa Xaa Xaa Trp Leu Ala Cys
  1               5                  10                  15

Pro Asp Arg Gly Glu Leu Ser Ser Arg Ser Pro Pro Cys Arg Leu Ala
             20                  25                  30

Arg Trp Ala Glu Gly Asp Arg Glu Thr Arg Thr Cys Leu Leu Glu Leu
         35                  40                  45

Ser Ala Gln Ser Trp Gly Gly Arg Phe Arg Arg Ser Ser Ala Val Ser
     50                  55                  60

Ala Gly Ser Pro Ser Arg Leu His Phe Leu Pro Gln Pro Leu Leu Leu
65                  70                  75                  80

Arg Ser Ser Gly Ile Pro Ala Ala Ala Thr Pro Trp Pro Gln Pro Ala
                85                  90                  95

Gly Leu Pro Val Arg Pro Thr Pro Thr Arg Thr Gly Glu Glu Asp Arg
            100                 105                 110

Thr Leu Asp Ile Ser Ile Cys Thr Glu Val Leu Ala Gly Thr Glu Gln
        115                 120                 125

Pro Pro Pro Pro Arg Met Thr Ser Pro Ser Ser Ser Pro Val Phe Arg
    130                 135                 140

Leu Glu Thr Leu Asp Gly Gly Gln Glu Asp Gly Ser Glu Ala Asp Arg
145                 150                 155                 160

Gly Lys Leu Asp Phe Gly Ser Gly Leu Pro Pro Met Glu Ser Gln Phe
                165                 170                 175

Gln Gly Glu Asp Arg Lys Phe Ala Pro Ser Asp Lys Ser Gln Pro Pro
            180                 185                 190

Thr Thr Glu Arg Glu Gln Val Pro Val Ser Arg Ile Gln Thr Asp Leu
        195                 200                 205

Thr Glu Ile Gly Ser Ser Met Arg Ser Pro Gly Val Ser Pro Arg Ile
    210                 215                 220

Trp Leu Asp Phe Gln Ser Thr Xaa
225                 230

(2) INFORMATION FOR SEQ ID NO: 610:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 610:

Met Val Leu Leu Leu Leu Leu Ala Tyr Val Leu Leu Thr Tyr Ile Leu
  1               5                  10                  15

Leu Leu Asn Met Leu Ile Ala Leu Met Xaa Arg Asp Arg Gln Gln Cys
            20                  25                  30

Arg His

(2) INFORMATION FOR SEQ ID NO: 611:

(i) SEQUENCE CHARACTERISTICS:
(A) LENGTH: 21 amino acids
(B) TYPE: amino acid
(D) TOPOLOGY: linear

(xi) SEQUENCE DESCRIPTION: SEQ ID NO: 611:

Met Val Phe Glu Gly Phe Ser Ser Ala Phe Cys Leu Ser Ser Thr Ala
1               5                   10                  15

Pro Thr Ser His Pro
            20

(2) INFORMATION FOR SEQ ID NO: 612:

(i) SEQUENCE CHARACTERISTICS:
(A) LENGTH: 9 amino acids
(B) TYPE: amino acid
(D) TOPOLOGY: linear
(xi) SEQUENCE DESCRIPTION: SEQ ID NO: 612:

Gly Lys Lys Asn Gln Leu Leu Val Ile
1               5

(2) INFORMATION FOR SEQ ID NO: 613:

(i) SEQUENCE CHARACTERISTICS:
(A) LENGTH: 29 amino acids
(B) TYPE: amino acid
(D) TOPOLOGY: linear
(xi) SEQUENCE DESCRIPTION: SEQ ID NO: 613:

Met Val Trp Val Leu Trp Ser Ala Pro Ser Leu Ala Pro Pro Trp Val
1               5                   10                  15

Gly Pro Cys Trp Pro Ser Thr Gly Asn Cys Cys Leu Cys
            20                  25

(2) INFORMATION FOR SEQ ID NO: 614:

(i) SEQUENCE CHARACTERISTICS:
(A) LENGTH: 30 amino acids
(B) TYPE: amino acid
(D) TOPOLOGY: linear
(xi) SEQUENCE DESCRIPTION: SEQ ID NO: 614:

Met Ala Lys Arg Ser Pro Gly Gly Cys Gly Ser Gly Leu Ile Leu Leu
1               5                   10                  15

Cys Cys Gln Pro Cys Arg Pro Thr Ser Ser Ala Pro Met Arg
            20                  25                  30

(2) INFORMATION FOR SEQ ID NO: 615:

(i) SEQUENCE CHARACTERISTICS:
(A) LENGTH: 113 amino acids
(B) TYPE: amino acid

(D) TOPOLOGY: linear
(xi) SEQUENCE DESCRIPTION: SEQ ID NO: 615:

Ile Thr Ile Ala Ile Gln Met Ile Cys Leu Val Asn Xaa Glu Leu Tyr
1               5                   10                  15

Pro Thr Phe Val Arg Asn Xaa Gly Val Met Val Cys Ser Ser Leu Cys
            20                  25                  30

Asp Ile Gly Gly Ile Ile Thr Pro Phe Ile Val Phe Arg Leu Arg Glu
        35                  40                  45

Val Trp Gln Ala Leu Pro Leu Ile Leu Phe Ala Val Leu Gly Leu Leu
    50                  55                  60

Ala Ala Gly Val Thr Leu Leu Leu Pro Glu Thr Lys Gly Val Ala Leu
65                  70                  75                  80

Pro Glu Thr Met Lys Asp Ala Glu Asn Leu Gly Arg Lys Ala Lys Pro
                85                  90                  95

Lys Glu Asn Thr Ile Tyr Leu Lys Val Gln Thr Ser Glu Pro Ser Gly
            100                 105                 110

Thr

(2) INFORMATION FOR SEQ ID NO: 616:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 616:

Thr Met Lys Asp Ala Glu Asn Leu Gly Arg Lys Ala Lys Pro Lys Glu
1               5                   10                  15

Asn Thr

(2) INFORMATION FOR SEQ ID NO: 617:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 617:

Pro Arg Val Arg Asn Ser Pro Glu Asp Leu Gly Leu Ser Leu Thr Gly
1               5                   10                  15

Asp Ser Cys Lys Leu
            20

(2) INFORMATION FOR SEQ ID NO: 618:

(i) SEQUENCE CHARACTERISTICS:
(A) LENGTH: 52 amino acids
(B) TYPE: amino acid
(D) TOPOLOGY: linear
(xi) SEQUENCE DESCRIPTION: SEQ ID NO: 618:

Gln Ala Asp Asp Leu Gln Ala Thr Val Ala Ala Leu Cys Val Leu Arg
1               5                   10                  15

Gly Gly Gly Pro Trp Ala Gly Ser Trp Leu Ser Pro Lys Thr Pro Gly
            20                  25                  30

Ala Met Gly Gly Asp Leu Val Leu Gly Leu Gly Ala Leu Arg Arg Arg
        35                  40                  45

Lys Arg Leu Leu
    50

(2) INFORMATION FOR SEQ ID NO: 619:

(i) SEQUENCE CHARACTERISTICS:
(A) LENGTH: 232 amino acids
(B) TYPE: amino acid
(D) TOPOLOGY: linear
(xi) SEQUENCE DESCRIPTION: SEQ ID NO: 619:

Glu Gln Glu Lys Ser Leu Ala Gly Trp Ala Leu Val Leu Ala Xaa Xaa
1               5                   10                  15

Gly Ile Gly Leu Met Val Leu His Ala Glu Met Leu Trp Phe Gly Gly
            20                  25                  30

Cys Ser Ala Val Asn Ala Thr Gly His Leu Ser Asp Thr Leu Trp Leu
        35                  40                  45

Ile Pro Ile Thr Phe Leu Thr Ile Gly Tyr Gly Asp Val Val Pro Gly
    50                  55                  60

Thr Met Trp Gly Lys Ile Val Cys Leu Cys Thr Gly Val Met Gly Val
65                  70                  75                  80

Cys Cys Thr Ala Leu Leu Val Ala Val Val Ala Arg Lys Leu Glu Phe
                85                  90                  95

Asn Lys Ala Glu Lys His Val His Asn Phe Met Met Asp Ile Gln Tyr
            100                 105                 110

Thr Lys Glu Met Lys Glu Ser Ala Ala Arg Val Leu Gln Glu Ala Trp
        115                 120                 125

Met Phe Tyr Lys His Thr Arg Arg Lys Glu Ser His Ala Ala Arg Xaa
    130                 135                 140

His Gln Arg Xaa Leu Leu Ala Ala Ile Asn Ala Phe Arg Gln Val Arg
145                 150                 155                 160

Leu Lys His Arg Lys Leu Arg Glu Gln Val Asn Ser Met Val Asp Ile
                165                 170                 175

Ser Lys Met His Met Ile Leu Tyr Asp Leu Gln Gln Asn Leu Ser Ser
            180                 185                 190

Ser His Arg Ala Leu Glu Lys Gln Ile Asp Thr Leu Ala Gly Lys Leu
        195                 200                 205

Asp Ala Leu Thr Glu Leu Leu Ser Thr Ala Leu Gly Pro Arg Gln Leu
    210                 215                 220

Pro Glu Pro Ser Gln Gln Ser Lys
225                 230

(2) INFORMATION FOR SEQ ID NO: 620:

        (i) SEQUENCE CHARACTERISTICS:
                (A) LENGTH: 36 amino acids
                (B) TYPE: amino acid
                (D) TOPOLOGY: linear
        (xi) SEQUENCE DESCRIPTION: SEQ ID NO: 620:

Tyr Gln Ala His His Val Ser Arg Asn Lys Arg Gly Gln Val Val Gly
  1               5                  10                  15

Thr Arg Gly Gly Phe Arg Gly Cys Thr Val Trp Leu Thr Gly Leu Ser
            20                  25                  30

Gly Ala Gly Lys
        35

(2) INFORMATION FOR SEQ ID NO: 621:

        (i) SEQUENCE CHARACTERISTICS:
                (A) LENGTH: 57 amino acids
                (B) TYPE: amino acid
                (D) TOPOLOGY: linear
        (xi) SEQUENCE DESCRIPTION: SEQ ID NO: 621:

Leu Gln Cys Glu Ile Cys Gly Phe Thr Cys Arg Gln Lys Ala Ser Leu
  1               5                  10                  15

Asn Trp His Met Lys Lys His Asp Ala Asp Ser Phe Tyr Gln Phe Ser
            20                  25                  30

Cys Asn Ile Cys Gly Lys Lys Phe Glu Lys Lys Asp Ser Val Val Ala
        35                  40                  45

His Lys Ala Lys Ser His Pro Glu Val
    50                  55

(2) INFORMATION FOR SEQ ID NO: 622:

(i) SEQUENCE CHARACTERISTICS:
(A) LENGTH: 19 amino acids
(B) TYPE: amino acid
(D) TOPOLOGY: linear
(xi) SEQUENCE DESCRIPTION: SEQ ID NO: 622:

Ile Thr Ser Thr Asp Ile Leu Gly Thr Asn Pro Glu Ser Leu Thr Gln
1               5                   10                  15

Pro Ser Asp

(2) INFORMATION FOR SEQ ID NO: 623:

(i) SEQUENCE CHARACTERISTICS:
(A) LENGTH: 19 amino acids
(B) TYPE: amino acid
(D) TOPOLOGY: linear
(xi) SEQUENCE DESCRIPTION: SEQ ID NO: 623:

Asn Ser Thr Ser Gly Glu Cys Leu Leu Leu Glu Ala Glu Gly Met Ser
1               5                   10                  15

Lys Ser Tyr

(2) INFORMATION FOR SEQ ID NO: 624:

(i) SEQUENCE CHARACTERISTICS:
(A) LENGTH: 51 amino acids
(B) TYPE: amino acid
(D) TOPOLOGY: linear
(xi) SEQUENCE DESCRIPTION: SEQ ID NO: 624:

Cys Ser Gly Thr Glu Arg Val Ser Leu Met Ala Asp Gly Lys Ile Phe
1               5                   10                  15

Val Gly Ser Gly Ser Ser Gly Gly Thr Glu Gly Leu Val Met Asn Ser
            20                  25                  30

Asp Ile Leu Gly Ala Thr Thr Glu Val Leu Ile Glu Asp Ser Asp Ser
        35                  40                  45

Ala Gly Pro
    50

(2) INFORMATION FOR SEQ ID NO: 625:

(i) SEQUENCE CHARACTERISTICS:
(A) LENGTH: 60 amino acids
(B) TYPE: amino acid
(D) TOPOLOGY: linear
(xi) SEQUENCE DESCRIPTION: SEQ ID NO: 625:

Ile Gln Tyr Val Arg Cys Glu Met Glu Gly Cys Gly Thr Val Leu Ala
1               5                   10                  15

His Pro Arg Tyr Leu Gln His His Ile Lys Tyr Gln His Leu Leu Lys
            20                  25                  30

Lys Lys Tyr Val Cys Pro His Pro Ser Cys Gly Arg Leu Phe Arg Leu
        35                  40                  45

Gln Lys Gln Leu Leu Arg His Ala Lys His His Thr
    50                  55                  60

(2) INFORMATION FOR SEQ ID NO: 626:

(i) SEQUENCE CHARACTERISTICS:
(A) LENGTH: 31 amino acids
(B) TYPE: amino acid
(D) TOPOLOGY: linear
(xi) SEQUENCE DESCRIPTION: SEQ ID NO: 626:

Asp Gln Arg Asp Tyr Ile Cys Glu Tyr Cys Ala Arg Ala Phe Lys Ser
1               5                   10                  15

Ser His Asn Leu Ala Val His Arg Met Ile His Thr Gly Glu Lys
            20                  25                  30

(2) INFORMATION FOR SEQ ID NO: 627:

(i) SEQUENCE CHARACTERISTICS:
(A) LENGTH: 25 amino acids
(B) TYPE: amino acid
(D) TOPOLOGY: linear
(xi) SEQUENCE DESCRIPTION: SEQ ID NO: 627:

Arg Ser Ser Arg Ser Lys Thr Gly Ser Leu Gln Leu Ile Cys Lys Ser
1               5                   10                  15

Glu Pro Asn Thr Asp Gln Leu Asp Tyr
            20                  25

(2) INFORMATION FOR SEQ ID NO: 628:

(i) SEQUENCE CHARACTERISTICS:
(A) LENGTH: 183 amino acids
(B) TYPE: amino acid
(D) TOPOLOGY: linear
(xi) SEQUENCE DESCRIPTION: SEQ ID NO: 628:

Leu Gln Cys Glu Ile Cys Gly Phe Thr Cys Arg Gln Lys Ala Ser Leu
1               5                   10                  15

Asn Trp His Met Lys Lys His Asp Ala Asp Ser Phe Tyr Gln Phe Ser
            20                  25                  30

Cys Asn Ile Cys Gly Lys Lys Phe Glu Lys Lys Asp Ser Val Val Ala
        35                  40                  45

His Lys Ala Lys Ser His Pro Glu Val Xaa Ile Thr Ser Thr Asp Ile
    50                  55                  60

Leu Gly Thr Asn Pro Glu Ser Leu Thr Gln Pro Ser Asp Xaa Asn Ser
65                  70                  75                  80

Thr Ser Gly Glu Cys Leu Leu Leu Glu Ala Glu Gly Met Ser Lys Ser
                85                  90                  95

Tyr Xaa Cys Ser Gly Thr Glu Arg Val Ser Leu Met Ala Asp Gly Lys
            100                 105                 110

Ile Phe Val Gly Ser Gly Ser Ser Gly Gly Thr Glu Gly Leu Val Met
        115                 120                 125

Asn Ser Asp Ile Leu Gly Ala Thr Thr Glu Val Leu Ile Glu Asp Ser
    130                 135                 140

Asp Ser Ala Gly Pro Xaa Gln Arg Asp Tyr Ile Cys Glu Tyr Cys Ala
145                 150                 155                 160

Arg Ala Phe Lys Ser Ser His Asn Leu Ala Val His Arg Met Ile His
                165                 170                 175

Thr Gly Glu Lys His Tyr Xaa
            180

(2) INFORMATION FOR SEQ ID NO: 629:

(i) SEQUENCE CHARACTERISTICS:
(A) LENGTH: 60 amino acids
(B) TYPE: amino acid
(D) TOPOLOGY: linear
(xi) SEQUENCE DESCRIPTION: SEQ ID NO: 629:

Gln Tyr Val Arg Cys Glu Met Glu Gly Cys Gly Thr Val Leu Ala His
1               5                   10                  15

Pro Arg Tyr Leu Gln His His Ile Lys Tyr Gln His Leu Leu Lys Lys
            20                  25                  30

Lys Tyr Val Cys Pro His Pro Ser Cys Gly Arg Leu Phe Arg Leu Gln
        35                  40                  45

Lys Gln Leu Leu Arg His Ala Lys His His Thr Asp
    50                  55                  60

(2) INFORMATION FOR SEQ ID NO: 630:

(i) SEQUENCE CHARACTERISTICS:
(A) LENGTH: 27 amino acids
(B) TYPE: amino acid

(D) TOPOLOGY: linear
(xi) SEQUENCE DESCRIPTION: SEQ ID NO: 630:

Pro Phe Lys Asp Asp Pro Arg Asp Glu Thr Tyr Lys Pro His Leu Glu
1               5                   10                  15

Arg Glu Thr Pro Lys Pro Arg Arg Lys Ser Gly
            20                  25

(2) INFORMATION FOR SEQ ID NO: 631:

(i) SEQUENCE CHARACTERISTICS:
(A) LENGTH: 110 amino acids
(B) TYPE: amino acid
(D) TOPOLOGY: linear
(xi) SEQUENCE DESCRIPTION: SEQ ID NO: 631:

Glu Met Phe Asp Ser Leu Ser Tyr Phe Lys Gly Ser Ser Leu Leu Leu
1               5                   10                  15

Met Leu Lys Thr Tyr Leu Ser Glu Asp Val Phe Gln His Ala Val Val
            20                  25                  30

Leu Tyr Leu His Asn His Ser Tyr Ala Ser Ile Gln Ser Asp Asp Leu
        35                  40                  45

Trp Asp Ser Phe Asn Glu Val Thr Asn Gln Thr Leu Asp Val Lys Arg
    50                  55                  60

Met Met Lys Thr Trp Thr Leu Gln Lys Gly Phe Pro Leu Val Thr Val
65                  70                  75                  80

Gln Lys Lys Gly Lys Glu Leu Phe Ile Gln Gln Glu Arg Phe Phe Leu
                85                  90                  95

Asn Met Lys Pro Glu Ile Gln Pro Ser Asp Thr Arg Tyr Met
            100                 105                 110

(2) INFORMATION FOR SEQ ID NO: 632:

(i) SEQUENCE CHARACTERISTICS:
(A) LENGTH: 24 amino acids
(B) TYPE: amino acid
(D) TOPOLOGY: linear
(xi) SEQUENCE DESCRIPTION: SEQ ID NO: 632:

Leu Glu Lys Val Ala Ser Val Gly Asn Ser Arg Pro Thr Gly Gln Gln
1               5                   10                  15

Leu Glu Ser Leu Gly Leu Leu Ala
            20

(2) INFORMATION FOR SEQ ID NO: 633:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 633:

Val His Arg Glu Glu Ala Ser Cys Tyr Cys Gln Ala Glu Pro Ser Gly
1               5                   10                  15

Asp Leu

(2) INFORMATION FOR SEQ ID NO: 634:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 634:

Arg Pro Ala Leu Arg Gln Ala Gly Gly Gly Thr Arg Glu Pro Arg Gln
1               5                   10                  15

Lys Arg Trp Ala Gly Leu
            20

(2) INFORMATION FOR SEQ ID NO: 635:

(i) SEQUENCE CHARACTERISTICS:
(A) LENGTH: 12 amino acids
(B) TYPE: amino acid
(D) TOPOLOGY: linear
(xi) SEQUENCE DESCRIPTION: SEQ ID NO: 635:

Ala Val Asn Phe Arg Pro Gln Arg Ser Gln Ser Met
1               5                   10

(2) INFORMATION FOR SEQ ID NO: 636:

(i) SEQUENCE CHARACTERISTICS:
(A) LENGTH: 37 amino acids
(B) TYPE: amino acid
(D) TOPOLOGY: linear
(xi) SEQUENCE DESCRIPTION: SEQ ID NO: 636:

Met Ile Thr Asp Val Gln Leu Ala Ile Phe Ala Asn Met Leu Gly Val
1               5                   10                  15

Ser Leu Phe Leu Leu Val Val Leu Tyr His Tyr Val Ala Val Asn Asn
            20                  25                  30

Pro Lys Lys Gln Glu
        35

(2) INFORMATION FOR SEQ ID NO: 637:

(i) SEQUENCE CHARACTERISTICS:
(A) LENGTH: 342 amino acids
(B) TYPE: amino acid
(D) TOPOLOGY: linear
(xi) SEQUENCE DESCRIPTION: SEQ ID NO: 637:

Glu Glu Met Ala Asp Ser Val Lys Thr Phe Leu Gln Asp Leu Ala Arg
1               5                   10                  15

Gly Ile Lys Asp Ser Ile Trp Gly Ile Cys Thr Ile Ser Lys Leu Asp
            20                  25                  30

Ala Arg Ile Gln Gln Lys Arg Glu Glu Gln Arg Arg Arg Arg Ala Ser
        35                  40                  45

Ser Val Leu Ala Gln Arg Arg Ala Gln Ser Ile Glu Arg Lys Gln Glu
    50                  55                  60

Ser Glu Pro Arg Ile Val Ser Arg Ile Phe Gln Cys Cys Ala Trp Asn
65                  70                  75                  80

Gly Gly Val Phe Trp Phe Ser Leu Leu Leu Phe Tyr Arg Val Phe Ile
                85                  90                  95

Pro Val Leu Gln Ser Val Thr Ala Arg Ile Ile Gly Asp Pro Ser Leu
            100                 105                 110

His Gly Asp Val Trp Ser Trp Leu Glu Phe Phe Leu Thr Ser Ile Phe
        115                 120                 125

Ser Ala Leu Trp Val Leu Pro Leu Phe Val Leu Ser Lys Val Val Asn
    130                 135                 140

Ala Ile Trp Phe Gln Asp Ile Ala Asp Leu Ala Phe Glu Val Ser Gly
145                 150                 155                 160

Arg Lys Pro His Pro Phe Pro Ser Val Ser Lys Ile Ile Ala Asp Met
                165                 170                 175

Leu Phe Asn Leu Leu Leu Gln Ala Leu Phe Leu Ile Gln Gly Met Phe
            180                 185                 190

Val Ser Leu Phe Pro Ile His Leu Val Gly Gln Leu Val Ser Leu Leu
        195                 200                 205

His Met Ser Leu Leu Tyr Ser Leu Tyr Cys Phe Glu Tyr Arg Trp Phe
    210                 215                 220

Asn Lys Gly Ile Glu Met His Gln Arg Leu Ser Asn Ile Glu Arg Asn
225                 230                 235                 240

Trp Pro Tyr Tyr Phe Gly Phe Gly Leu Pro Leu Ala Phe Leu Thr Ala
                245                 250                 255

Met Gln Ser Ser Tyr Ile Ile Ser Gly Cys Leu Phe Ser Ile Leu Phe
            260                 265                 270

Pro Leu Phe Ile Ile Ser Ala Asn Glu Ala Lys Thr Pro Gly Lys Ala
        275                 280                 285

Tyr Leu Phe Gln Leu Arg Leu Phe Ser Leu Val Val Phe Leu Ser Asn
    290                 295                 300

Arg Leu Phe His Lys Thr Val Tyr Leu Gln Ser Ala Leu Ser Ser Ser
305                 310                 315                 320

Thr Ser Ala Glu Lys Phe Pro Ser Pro His Pro Ser Pro Ala Lys Leu
                325                 330                 335

Lys Ala Thr Ala Gly His
            340

(2) INFORMATION FOR SEQ ID NO: 638:

        (i) SEQUENCE CHARACTERISTICS:
                (A) LENGTH: 529 amino acids
                (B) TYPE: amino acid
                (D) TOPOLOGY: linear
        (xi) SEQUENCE DESCRIPTION: SEQ ID NO: 638:

Met Ala Lys Phe Met Thr Pro Val Ile Gln Asp Asn Pro Ser Gly Trp
  1               5                  10                  15

Gly Pro Cys Ala Val Pro Glu Gln Phe Arg Asp Met Pro Tyr Gln Pro
             20                  25                  30

Phe Ser Lys Gly Asp Arg Leu Gly Lys Val Ala Asp Trp Thr Gly Ala
         35                  40                  45

Thr Tyr Gln Asp Lys Arg Tyr Thr Asn Lys Tyr Ser Ser Gln Phe Gly
     50                  55                  60

Gly Gly Ser Gln Tyr Ala Tyr Phe His Glu Glu Asp Glu Ser Ser Phe
 65                  70                  75                  80

Gln Leu Val Asp Thr Ala Arg Thr Gln Lys Thr Ala Tyr Gln Arg Asn
                 85                  90                  95

Arg Met Arg Phe Ala Gln Arg Asn Leu Arg Arg Asp Lys Asp Arg Arg
            100                 105                 110

Asn Met Leu Gln Phe Asn Leu Gln Ile Leu Pro Lys Ser Ala Lys Gln
        115                 120                 125

Lys Glu Arg Glu Arg Ile Arg Leu Gln Lys Lys Phe Gln Lys Gln Phe
    130                 135                 140

Gly Val Arg Gln Lys Trp Asp Gln Lys Ser Gln Lys Pro Arg Asp Ser
145                 150                 155                 160

Ser Val Glu Val Arg Ser Asp Trp Glu Val Lys Glu Glu Met Asp Phe
                165                 170                 175

Pro Gln Leu Met Lys Met Arg Tyr Leu Glu Val Ser Glu Pro Gln Asp

            180                 185                 190

Ile Glu Cys Cys Gly Ala Leu Glu Tyr Tyr Asp Lys Ala Phe Asp Arg
        195                 200                 205

Ile Thr Thr Arg Ser Glu Lys Pro Leu Arg Xaa Xaa Lys Arg Ile Phe
    210                 215                 220

His Thr Val Thr Thr Thr Asp Asp Pro Val Ile Arg Lys Leu Ala Lys
225                 230                 235                 240

Thr Gln Gly Asn Val Phe Ala Thr Asp Ala Ile Leu Ala Thr Leu Met
                245                 250                 255

Ser Cys Thr Arg Ser Val Tyr Ser Trp Asp Ile Val Val Gln Arg Val
            260                 265                 270

Gly Ser Lys Leu Phe Phe Asp Lys Arg Asp Asn Ser Asp Phe Asp Leu
        275                 280                 285

Leu Thr Val Ser Glu Thr Ala Asn Glu Pro Pro Gln Asp Glu Gly Asn
    290                 295                 300

Ser Phe Asn Ser Pro Arg Asn Leu Ala Met Glu Ala Thr Tyr Ile Asn
305                 310                 315                 320

His Asn Phe Ser Gln Gln Cys Leu Arg Met Gly Lys Glu Arg Tyr Asn
                325                 330                 335

Phe Pro Asn Pro Asn Pro Phe Val Glu Asp Asp Met Asp Lys Asn Glu
            340                 345                 350

Ile Ala Ser Val Ala Tyr Arg Tyr Arg Ser Gly Lys Leu Gly Asp Asp
        355                 360                 365

Ile Asp Leu Ile Val Arg Cys Glu His Asp Gly Val Met Thr Gly Ala
    370                 375                 380

Asn Gly Glu Val Ser Phe Ile Asn Ile Lys Thr Leu Asn Glu Trp Asp
385                 390                 395                 400

Ser Arg His Cys Asn Gly Val Asp Trp Arg Gln Lys Leu Asp Ser Gln
                405                 410                 415

Arg Gly Ala Val Ile Ala Thr Glu Leu Lys Asn Asn Ser Tyr Lys Leu
            420                 425                 430

Ala Arg Trp Thr Cys Cys Ala Leu Leu Ala Gly Ser Glu Tyr Leu Lys
        435                 440                 445

Leu Gly Tyr Val Ser Arg Tyr His Val Lys Asp Ser Ser Arg His Val
    450                 455                 460

Ile Leu Gly Thr Gln Gln Phe Lys Pro Asn Glu Phe Ala Ser Gln Ile
465                 470                 475                 480

Asn Leu Ser Val Glu Asn Ala Trp Gly Ile Leu Arg Cys Val Ile Asp
                485                 490                 495

Ile Cys Met Lys Leu Glu Glu Gly Lys Tyr Leu Ile Leu Lys Asp Pro

500                 505                 510

Asn Lys Gln Val Ile Arg Val Tyr Ser Leu Pro Asp Gly Thr Phe Ser
        515                 520                 525

Ser

(2) INFORMATION FOR SEQ ID NO: 639:

      (i) SEQUENCE CHARACTERISTICS:
            (A) LENGTH: 194 amino acids
            (B) TYPE: amino acid
            (D) TOPOLOGY: linear
      (xi) SEQUENCE DESCRIPTION: SEQ ID NO: 639:

Lys Lys Arg His Thr Asp Val Gln Phe Tyr Thr Glu Val Gly Glu Ile
  1               5                  10                  15

Thr Thr Asp Leu Gly Lys His Gln His Met His Asp Arg Asp Asp Leu
             20                  25                  30

Tyr Ala Glu Gln Met Glu Arg Glu Met Arg His Lys Leu Lys Thr Ala
         35                  40                  45

Phe Lys Asn Phe Ile Glu Lys Val Glu Ala Leu Thr Lys Glu Glu Leu
     50                  55                  60

Glu Phe Glu Val Pro Phe Arg Asp Leu Gly Phe Asn Gly Ala Pro Tyr
 65                  70                  75                  80

Arg Ser Thr Cys Leu Leu Gln Pro Thr Ser Ser Ala Leu Val Asn Ala
                 85                  90                  95

Thr Glu Trp Pro Pro Phe Val Val Thr Leu Asp Glu Val Glu Leu Ile
            100                 105                 110

His Phe Xaa Arg Val Gln Phe His Leu Lys Asn Phe Asp Met Val Ile
        115                 120                 125

Val Tyr Lys Asp Tyr Ser Lys Lys Val Thr Met Ile Asn Ala Ile Pro
    130                 135                 140

Val Ala Ser Leu Asp Pro Ile Lys Glu Trp Leu Asn Ser Cys Asp Leu
145                 150                 155                 160

Lys Tyr Thr Glu Gly Val Gln Ser Leu Asn Trp Thr Lys Ile Met Lys
                165                 170                 175

Thr Ile Val Asp Asp Pro Glu Gly Phe Phe Glu Gln Gly Gly Trp Ser
            180                 185                 190

Phe Leu

(2) INFORMATION FOR SEQ ID NO: 640:

(i) SEQUENCE CHARACTERISTICS:
(A) LENGTH: 70 amino acids
(B) TYPE: amino acid
(D) TOPOLOGY: linear
(xi) SEQUENCE DESCRIPTION: SEQ ID NO: 640:

Arg Ser Gly Leu Gly Leu Gly Ile Thr Ile Ala Phe Leu Ala Thr Leu
1               5                   10                  15

Ile Thr Gln Phe Leu Val Tyr Asn Gly Val Tyr Gln Tyr Thr Ser Pro
            20                  25                  30

Asp Phe Leu Tyr Ile Arg Ser Trp Leu Pro Cys Ile Phe Phe Ser Gly
        35                  40                  45

Gly Val Thr Val Gly Asn Ile Gly Arg Gln Leu Ala Met Gly Val Pro
    50                  55                  60

Glu Lys Pro His Ser Asp
65                  70

(2) INFORMATION FOR SEQ ID NO: 641:

(i) SEQUENCE CHARACTERISTICS:
(A) LENGTH: 101 amino acids
(B) TYPE: amino acid
(D) TOPOLOGY: linear
(xi) SEQUENCE DESCRIPTION: SEQ ID NO: 641:

Val Thr Gln Pro Lys His Leu Ser Ala Ser Met Gly Gly Ser Val Glu
1               5                   10                  15

Ile Pro Phe Ser Phe Tyr Tyr Pro Trp Glu Leu Ala Xaa Xaa Pro Xaa
            20                  25                  30

Val Arg Ile Ser Trp Arg Arg Gly His Phe His Gly Gln Ser Phe Tyr
        35                  40                  45

Ser Thr Arg Pro Pro Ser Ile His Lys Asp Tyr Val Asn Arg Leu Phe
    50                  55                  60

Leu Asn Trp Thr Glu Gly Gln Glu Ser Gly Phe Leu Arg Ile Ser Asn
65                  70                  75                  80

Leu Arg Lys Glu Asp Gln Ser Val Tyr Phe Cys Arg Val Glu Leu Asp
                85                  90                  95

Thr Arg Arg Ser Gly
            100

(2) INFORMATION FOR SEQ ID NO: 642:

(i) SEQUENCE CHARACTERISTICS:
(A) LENGTH: 233 amino acids
(B) TYPE: amino acid

(D) TOPOLOGY: linear
(xi) SEQUENCE DESCRIPTION: SEQ ID NO: 642:

Met Glu Ala Gln Gln Val Asn Glu Ala Glu Ser Ala Arg Glu Gln Leu
1               5                   10                  15

Gln Xaa Leu His Asp Gln Ile Ala Gly Gln Lys Ala Ser Lys Gln Glu
            20                  25                  30

Leu Glu Thr Glu Leu Glu Arg Leu Lys Gln Glu Phe His Tyr Ile Glu
        35                  40                  45

Glu Asp Leu Tyr Arg Thr Lys Asn Thr Leu Gln Ser Arg Ile Lys Asp
    50                  55                  60

Arg Asp Glu Glu Ile Gln Lys Leu Arg Asn Gln Leu Thr Asn Lys Thr
65                  70                  75                  80

Leu Ser Asn Ser Ser Gln Ser Glu Leu Glu Asn Arg Leu His Gln Leu
                85                  90                  95

Thr Glu Thr Leu Ile Gln Lys Gln Thr Met Leu Glu Ser Leu Ser Thr
            100                 105                 110

Glu Lys Asn Ser Leu Val Phe Gln Leu Glu Arg Leu Glu Gln Gln Met
        115                 120                 125

Asn Ser Ala Ser Gly Ser Ser Ser Asn Gly Ser Ser Ile Asn Met Ser
    130                 135                 140

Gly Ile Asp Asn Gly Glu Gly Thr Arg Leu Arg Asn Val Pro Val Leu
145                 150                 155                 160

Phe Asn Asp Thr Glu Thr Asn Leu Ala Gly Met Tyr Gly Lys Val Arg
                165                 170                 175

Lys Ala Ala Ser Ser Ile Asp Gln Phe Ser Ile Arg Leu Gly Ile Phe
            180                 185                 190

Leu Arg Arg Tyr Pro Ile Ala Arg Val Phe Val Ile Ile Tyr Met Ala
        195                 200                 205

Leu Leu His Leu Trp Val Met Ile Val Leu Leu Thr Tyr Thr Pro Glu
    210                 215                 220

Met His His Asp Gln Pro Tyr Gly Lys
225                 230

(2) INFORMATION FOR SEQ ID NO: 643:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 643:

(2) INFORMATION FOR SEQ ID NO: 644:

      (i) SEQUENCE CHARACTERISTICS:
            (A) LENGTH: 63 amino acids
            (B) TYPE: amino acid
            (D) TOPOLOGY: linear
      (xi) SEQUENCE DESCRIPTION: SEQ ID NO: 644:

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert: The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

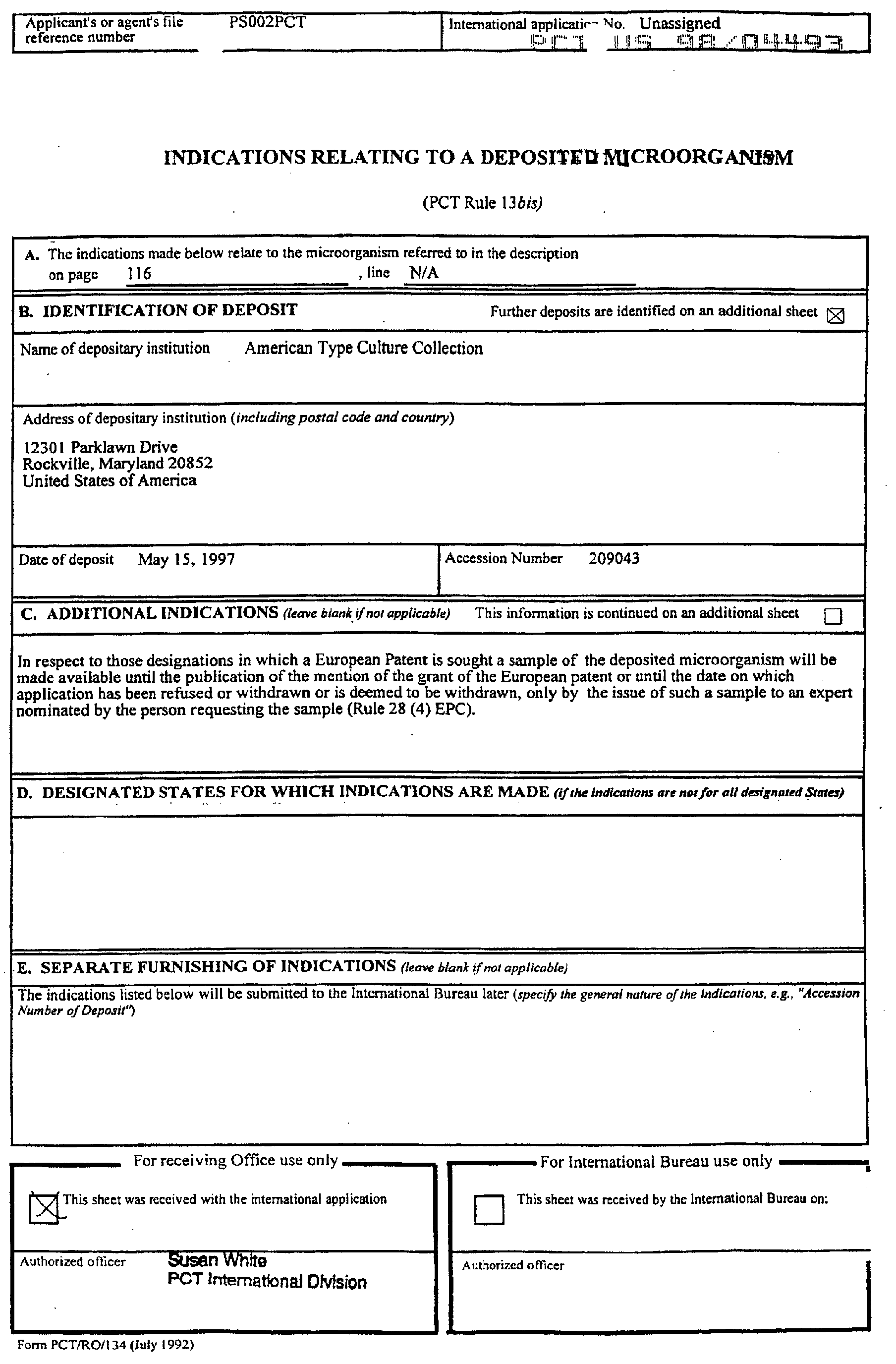

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally-decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

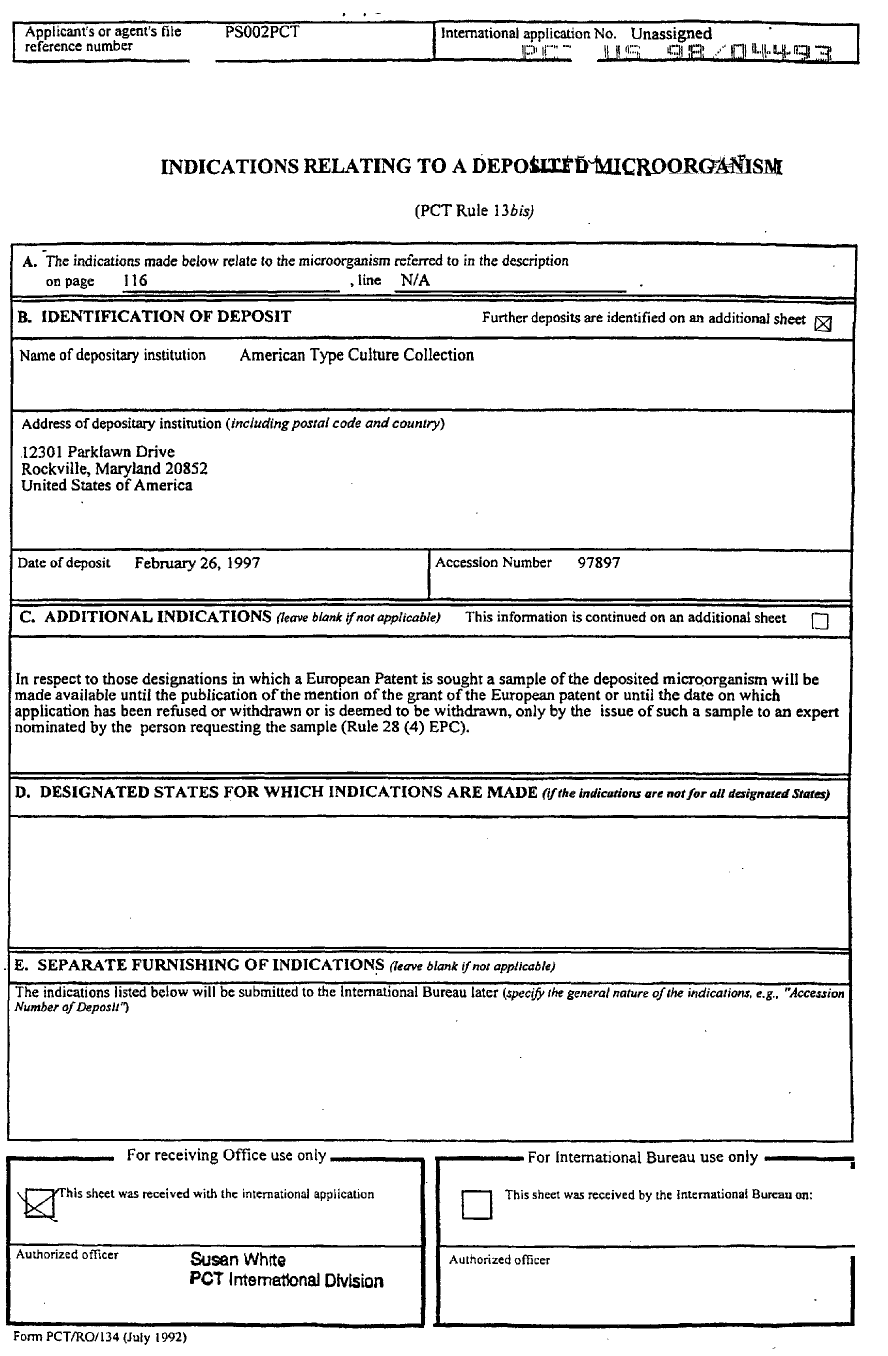

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

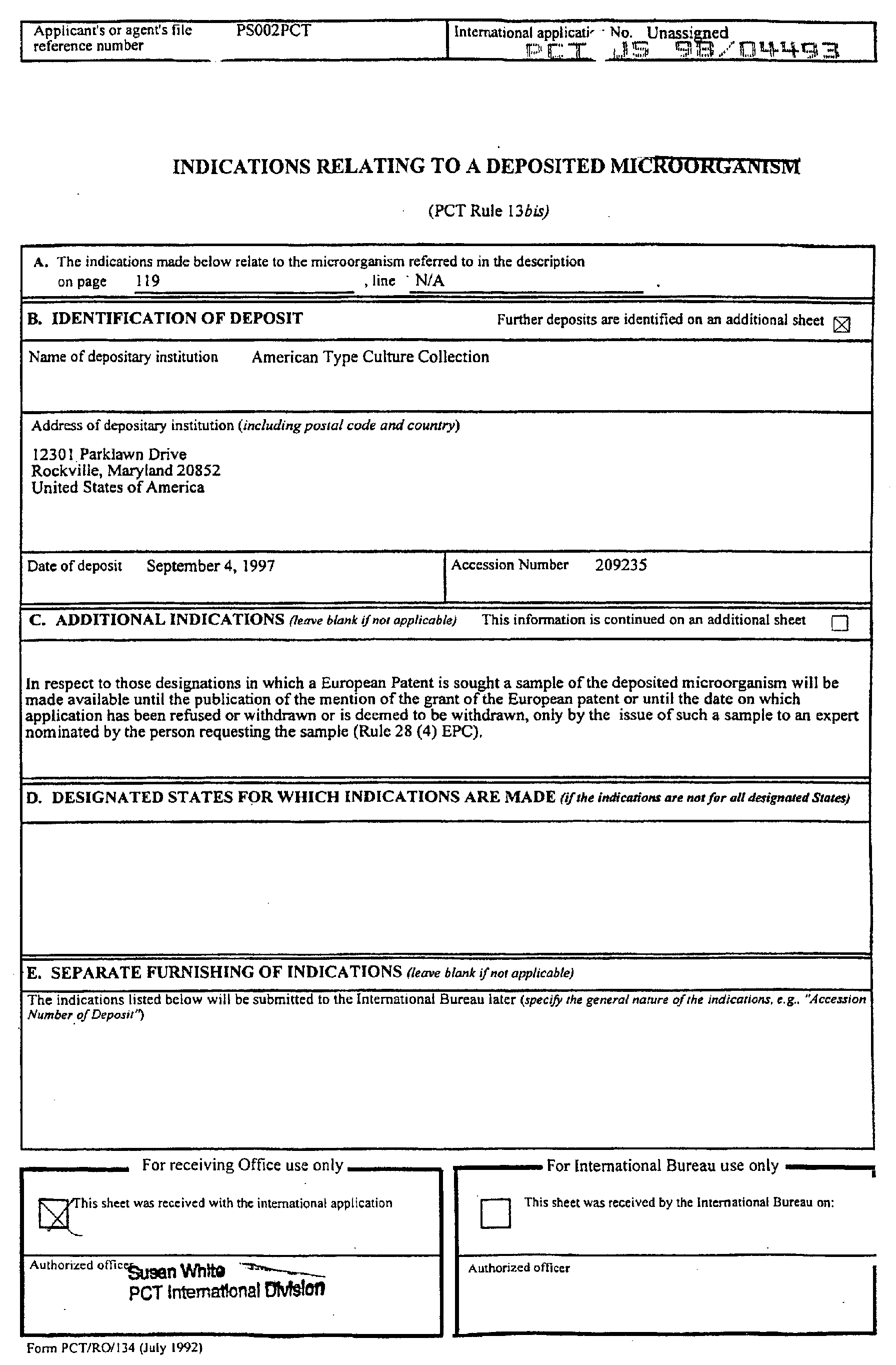

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

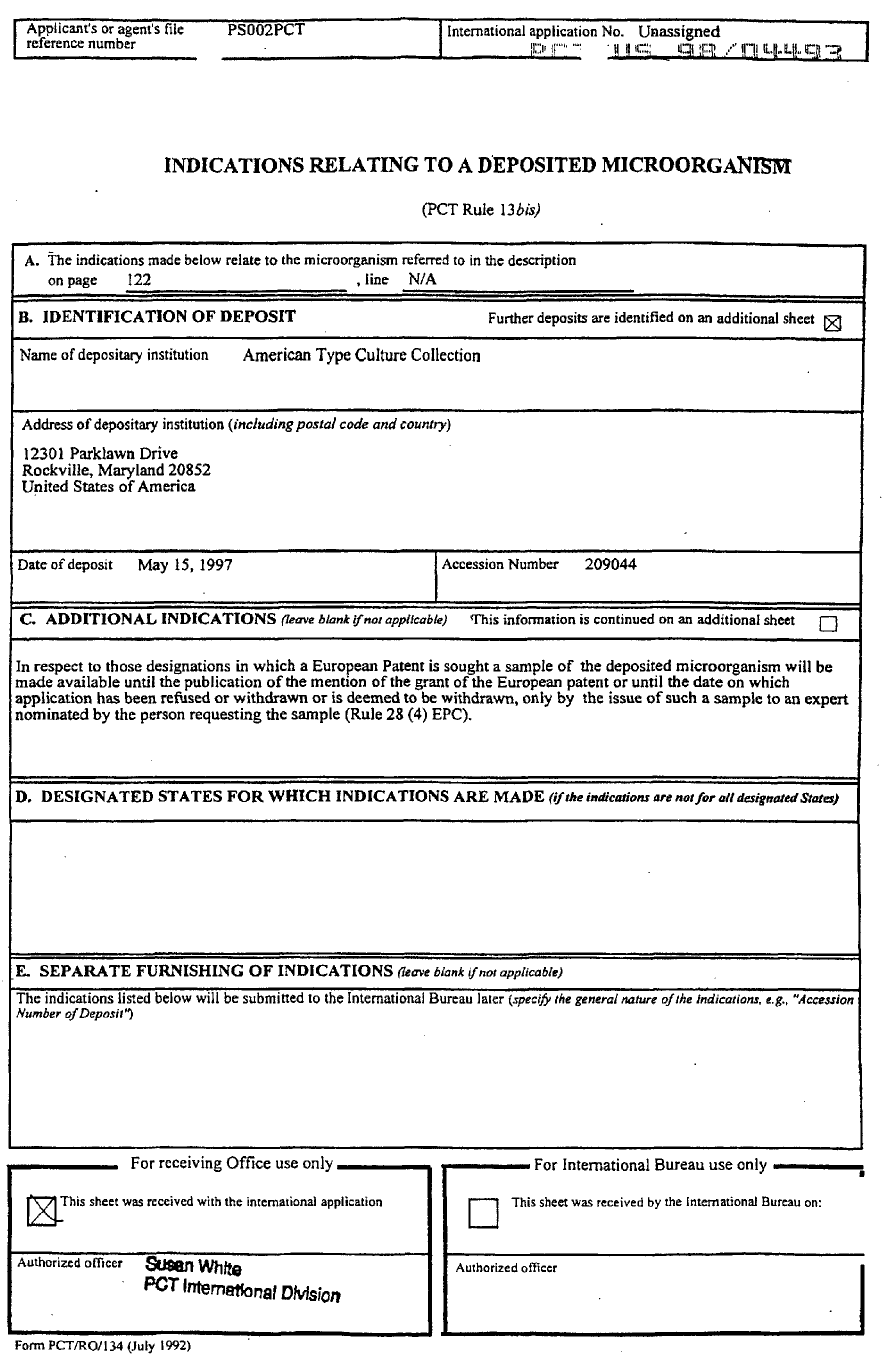

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by. a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

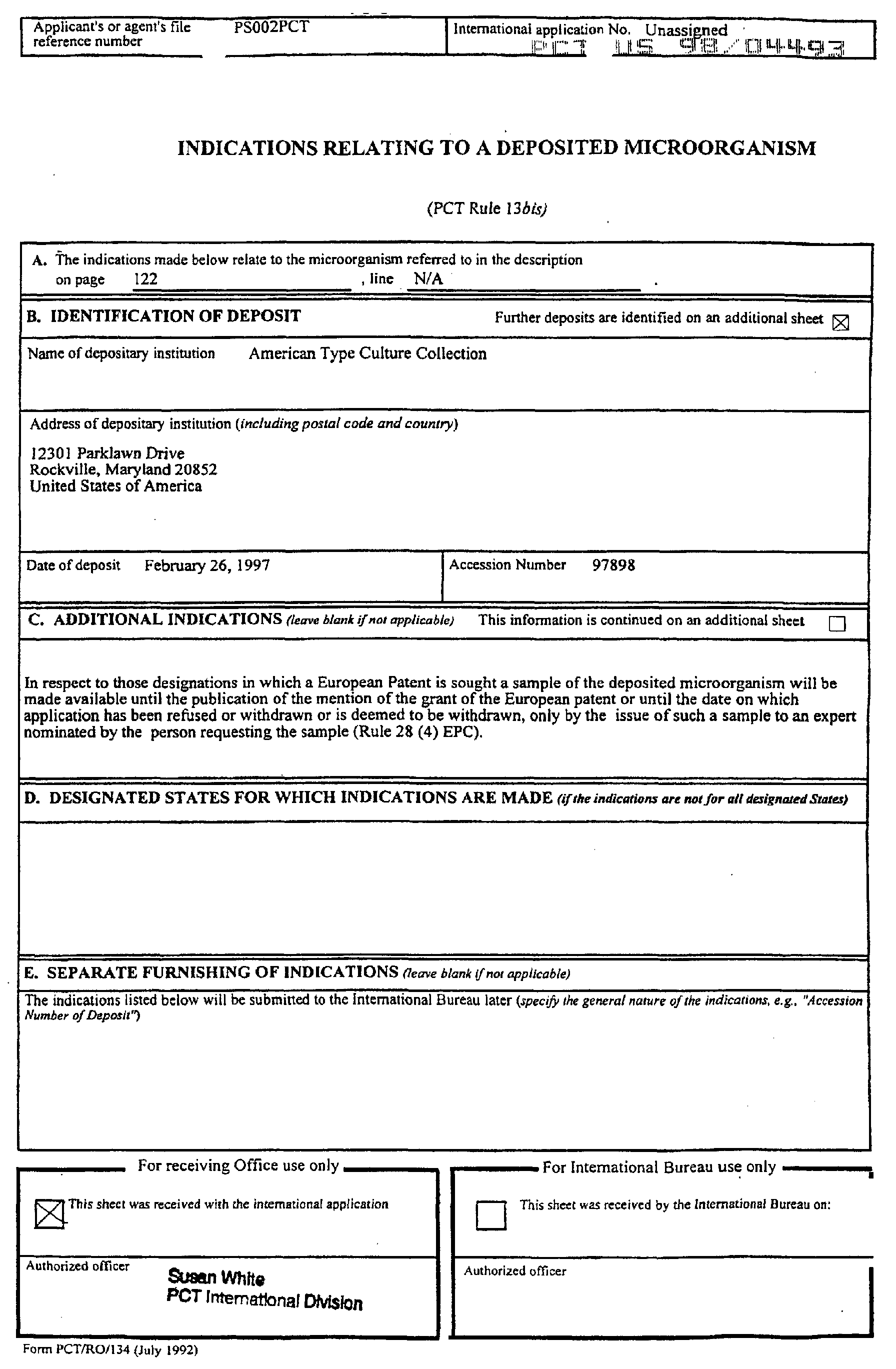

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert: The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

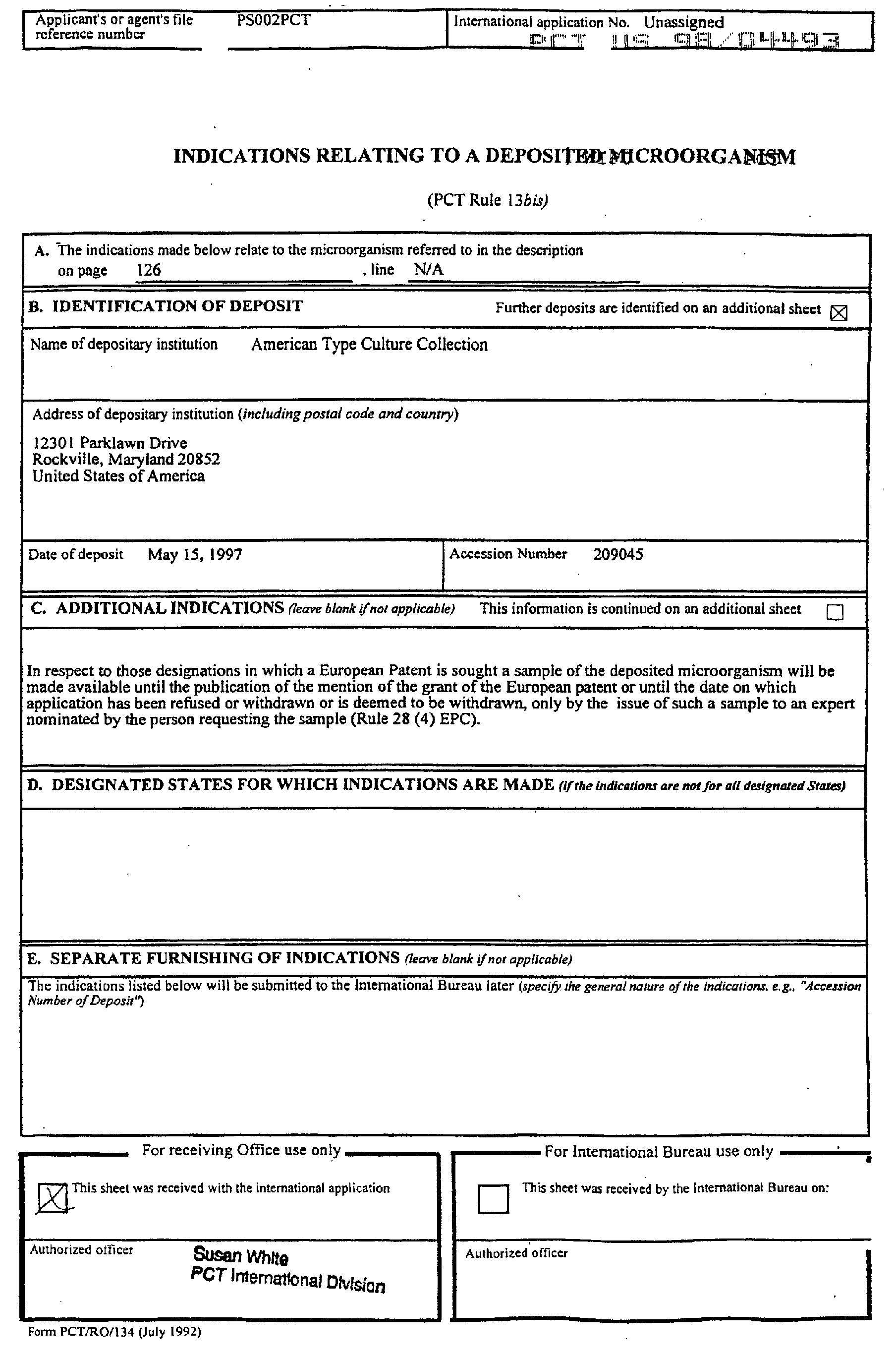

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

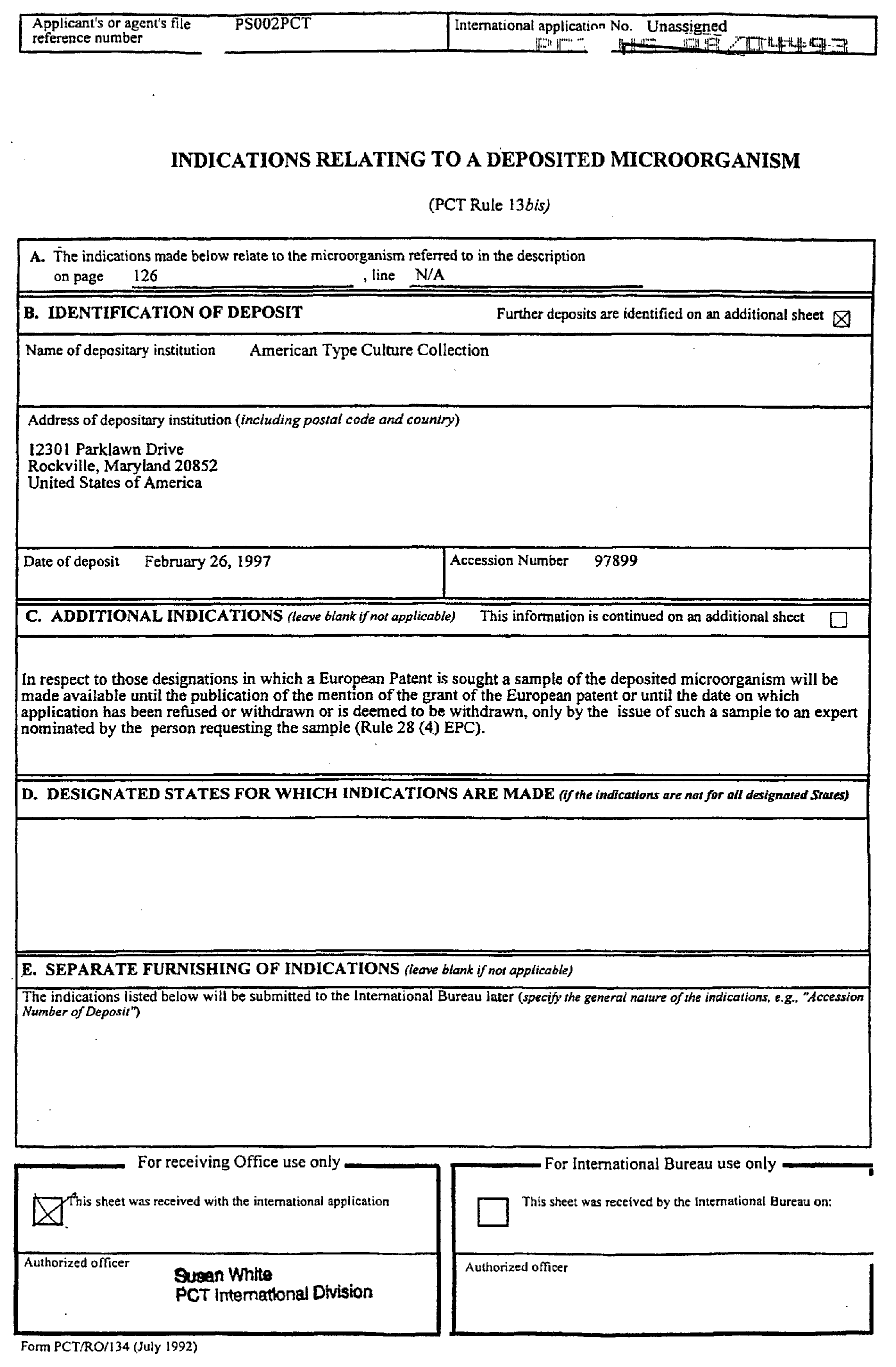

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

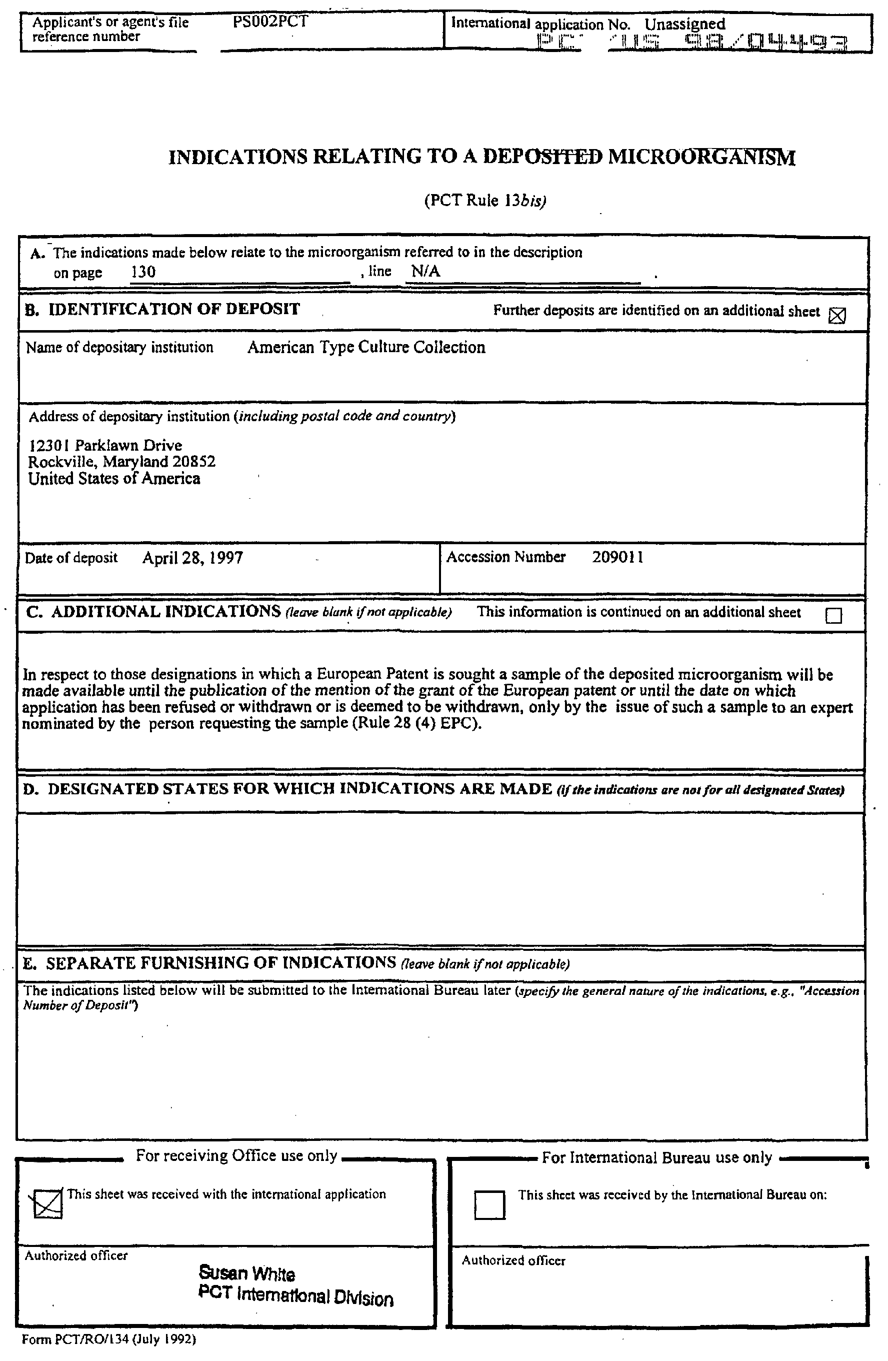

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

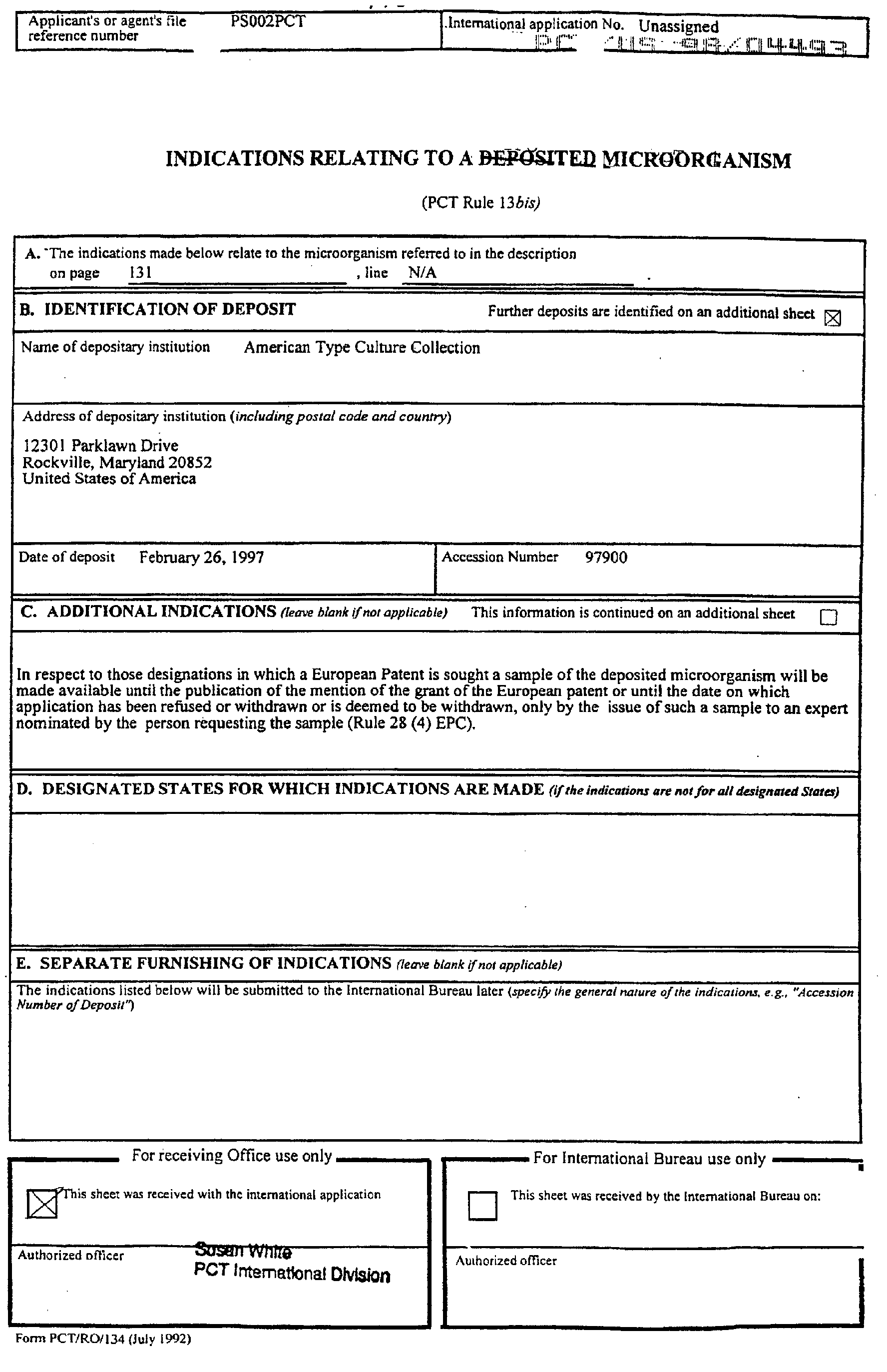

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

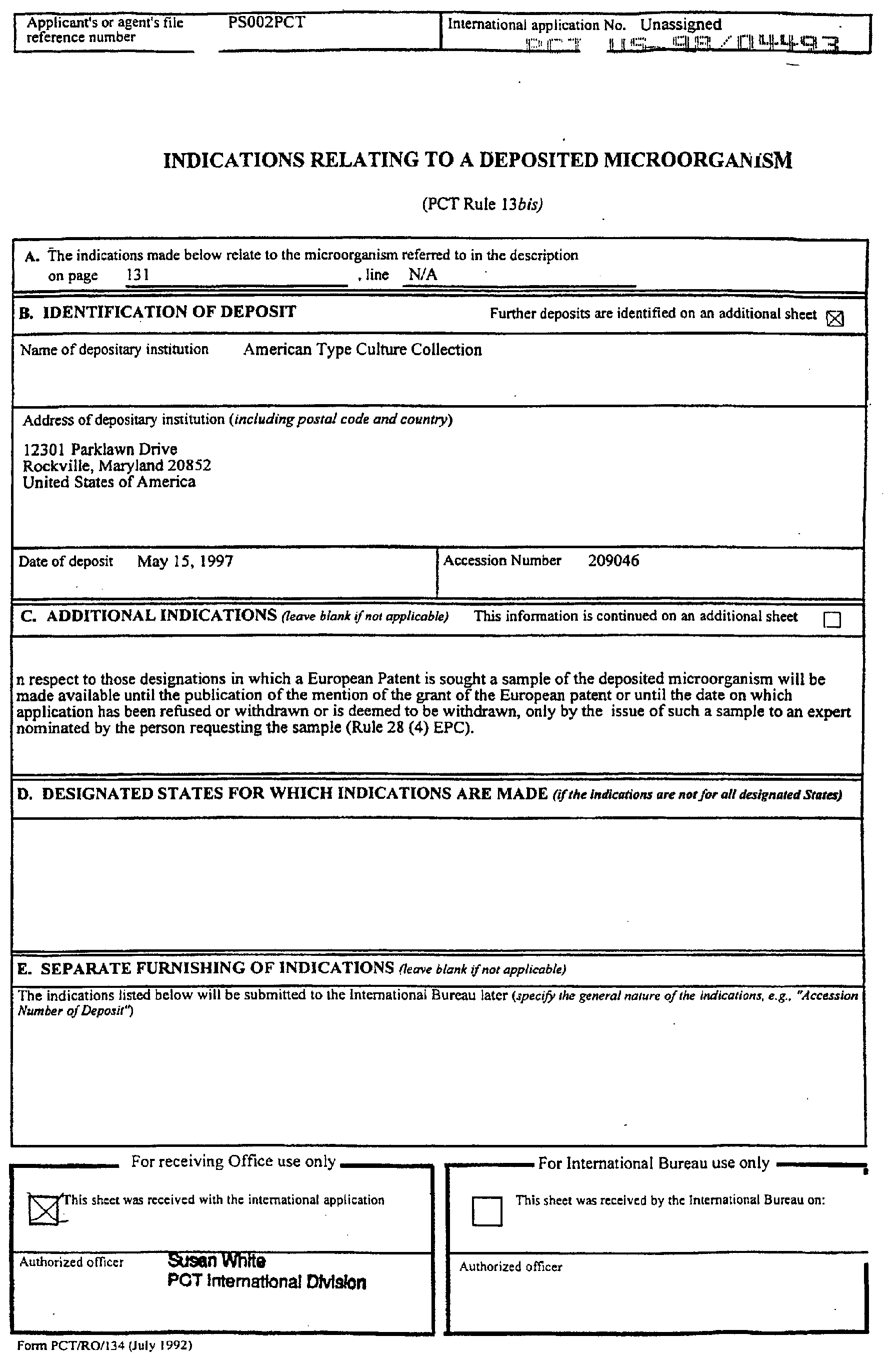

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

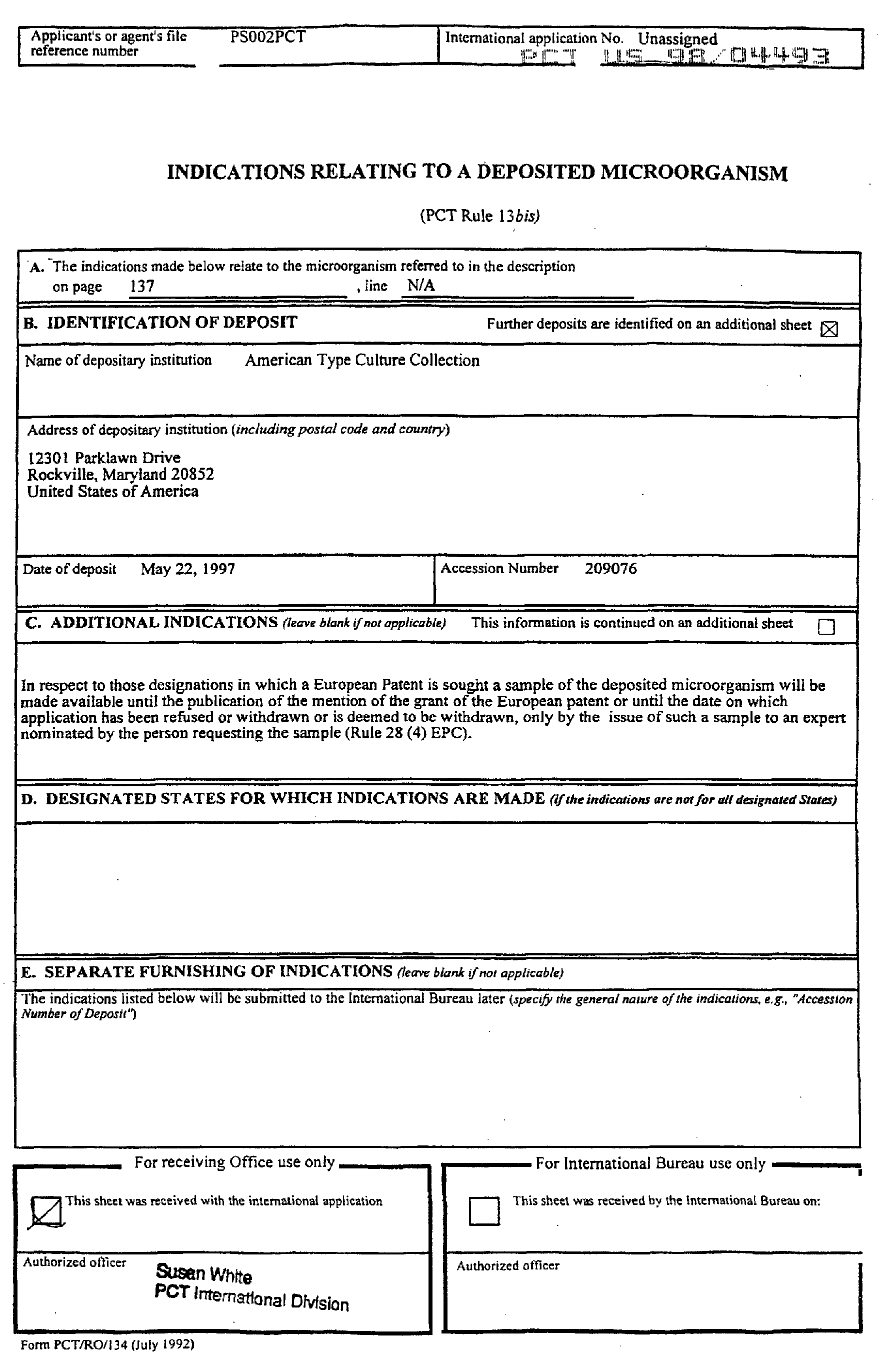

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

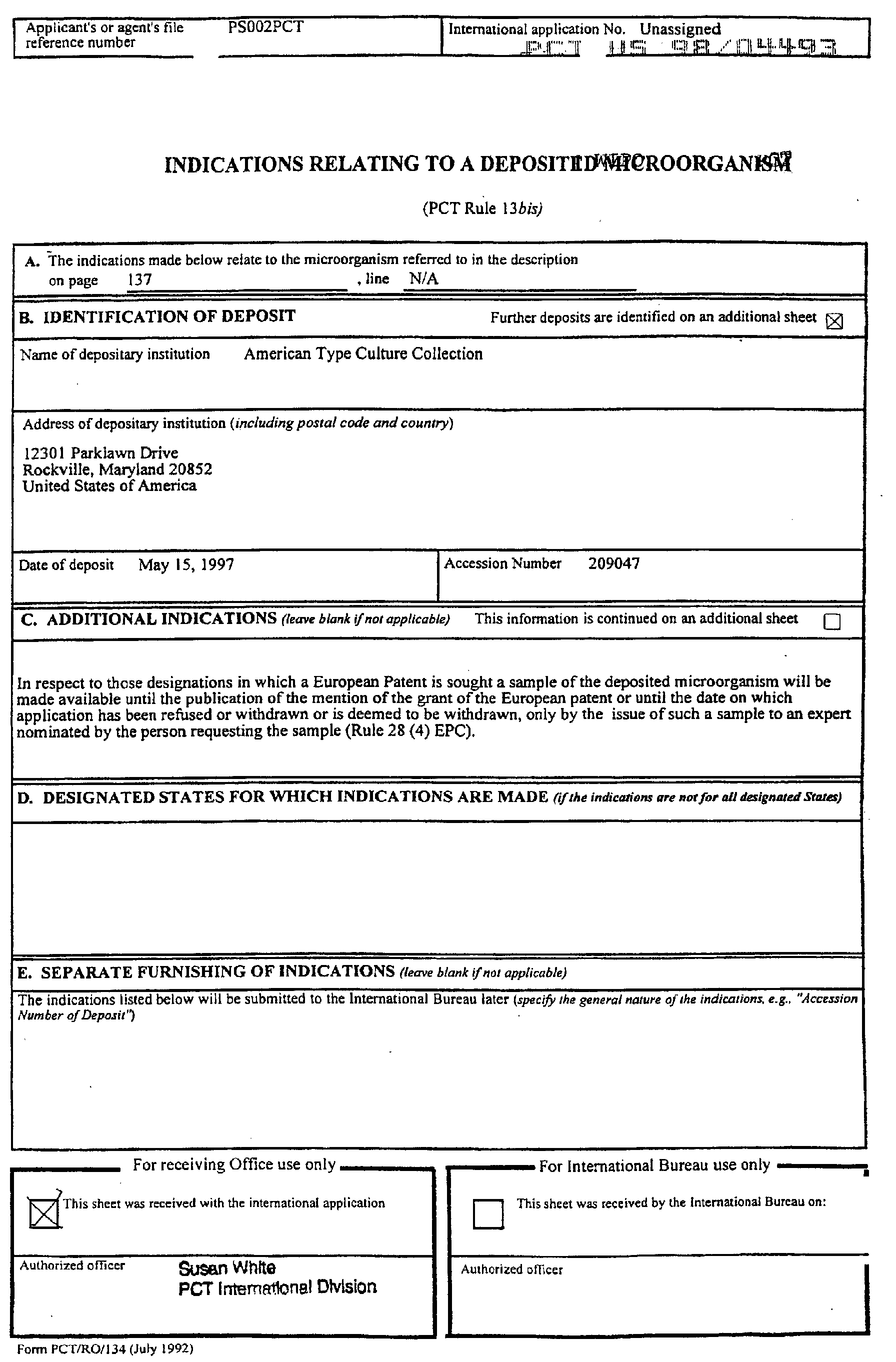

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be. any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

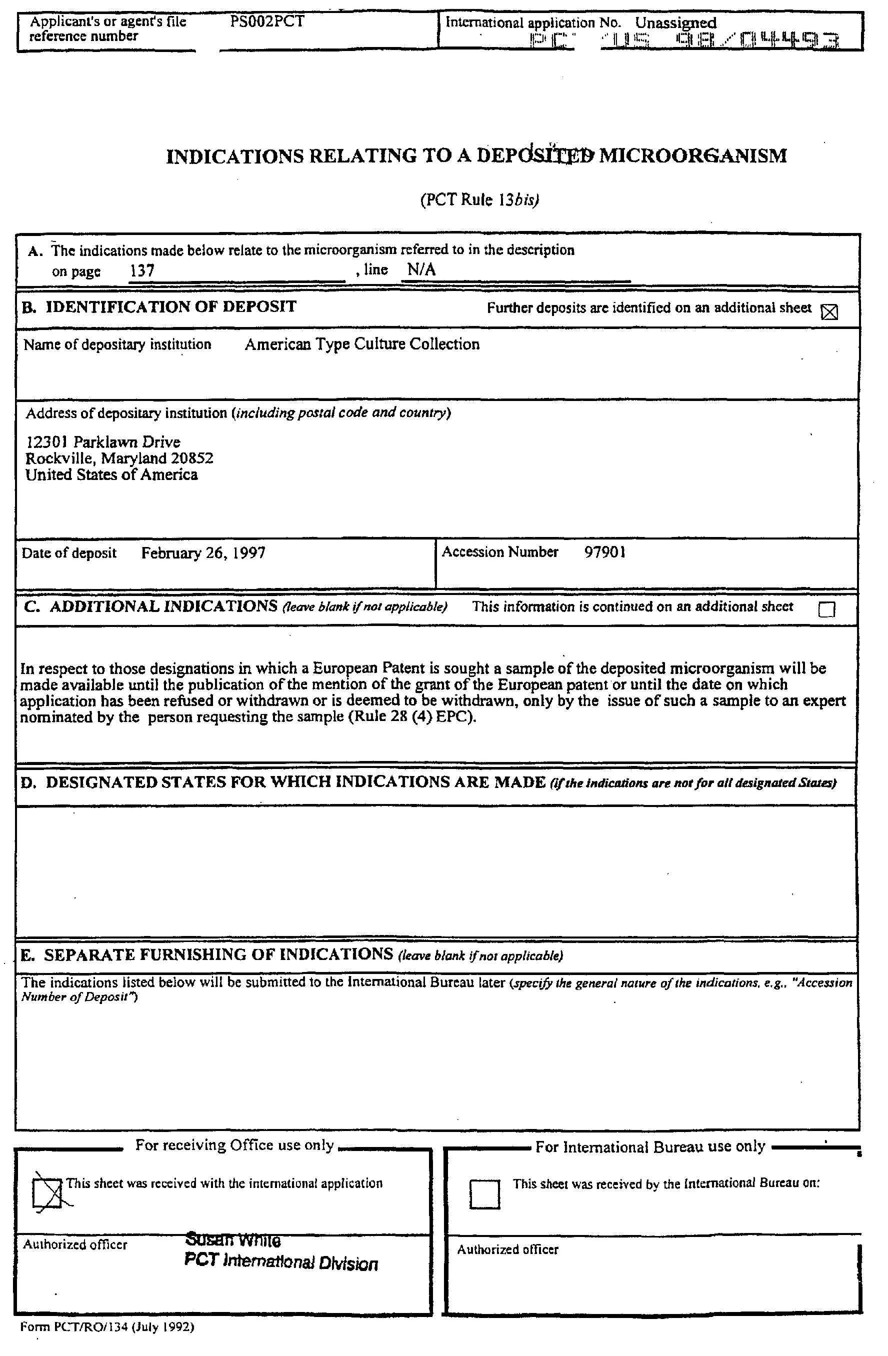

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

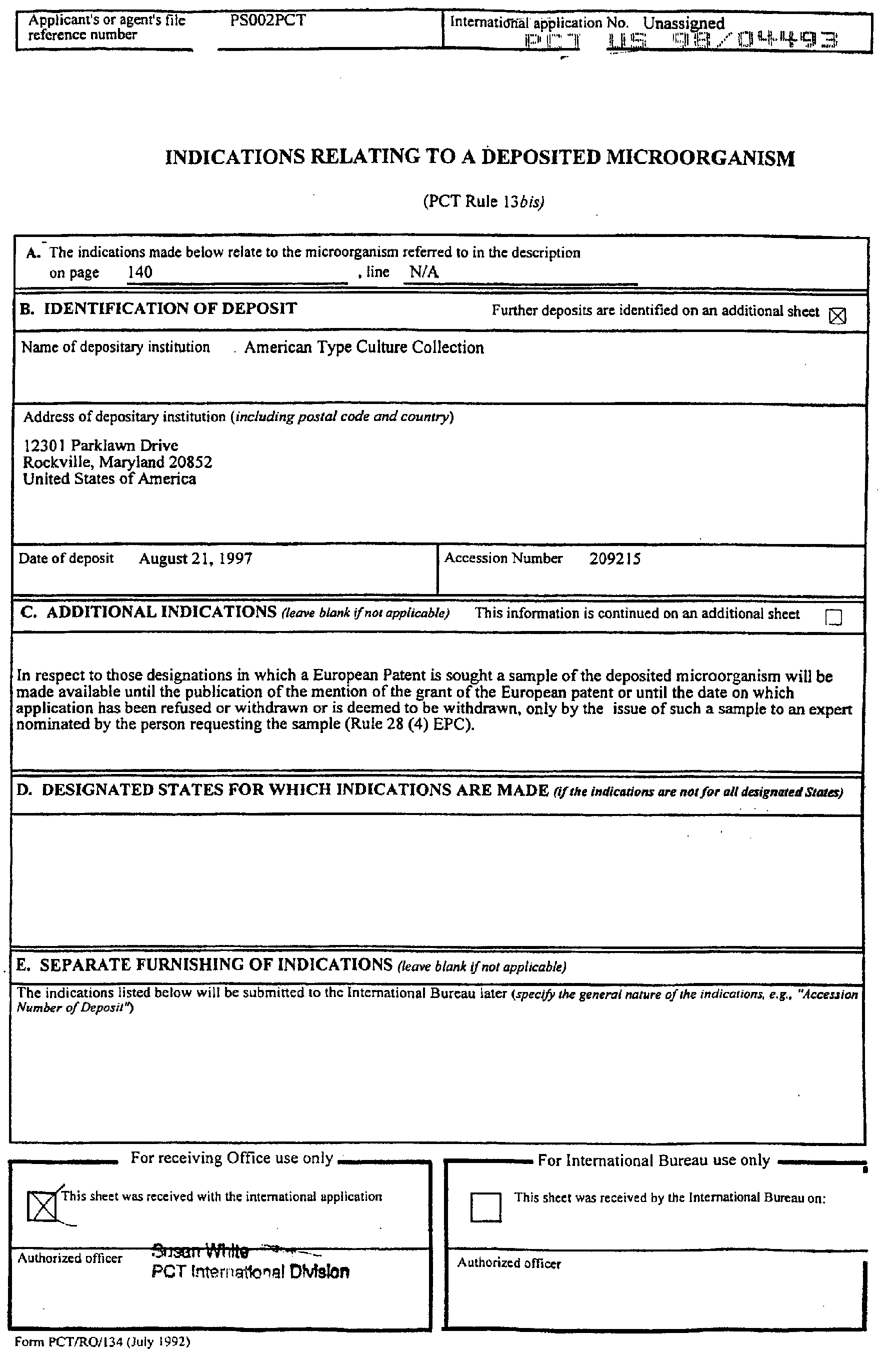

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

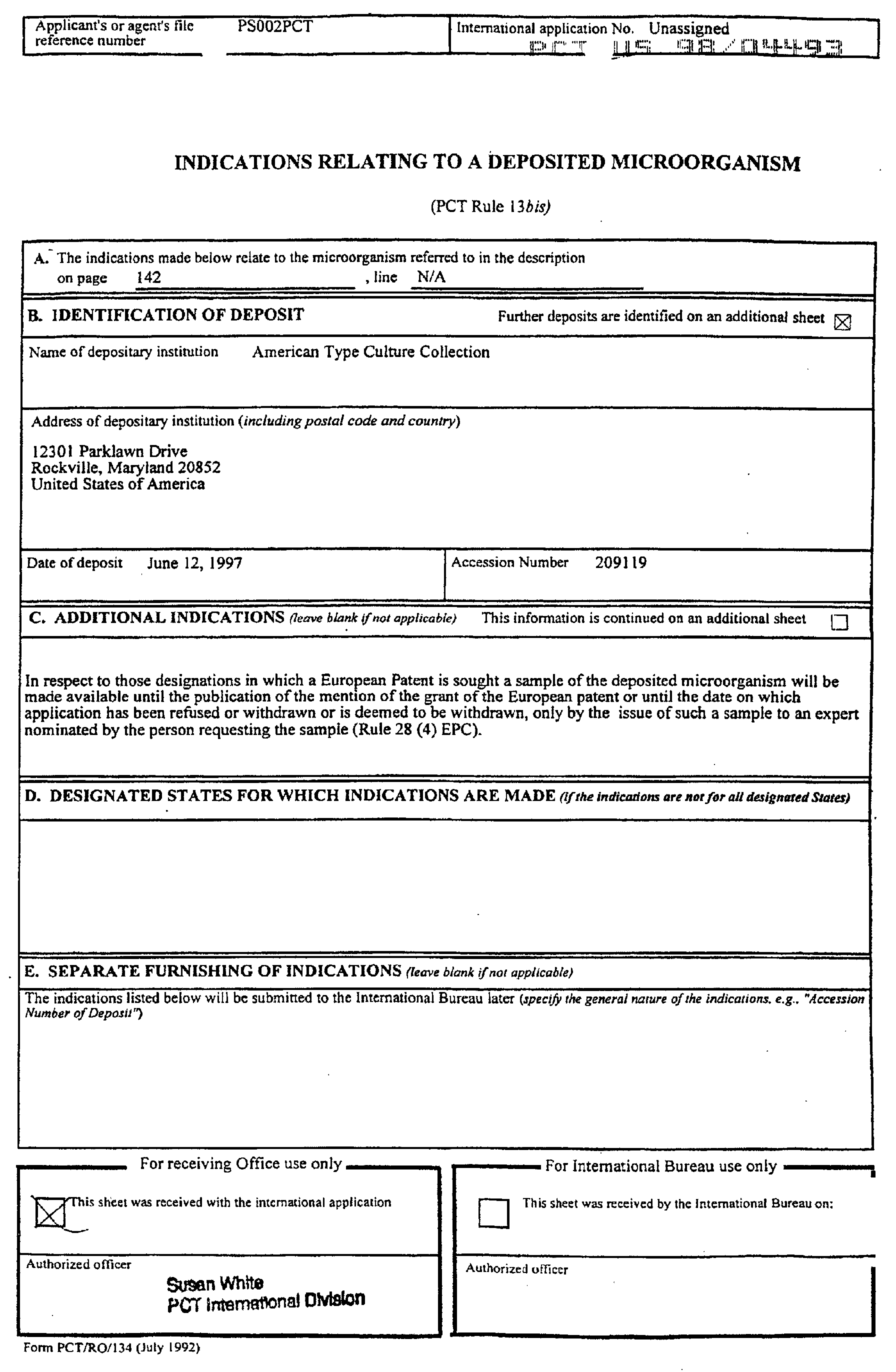

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

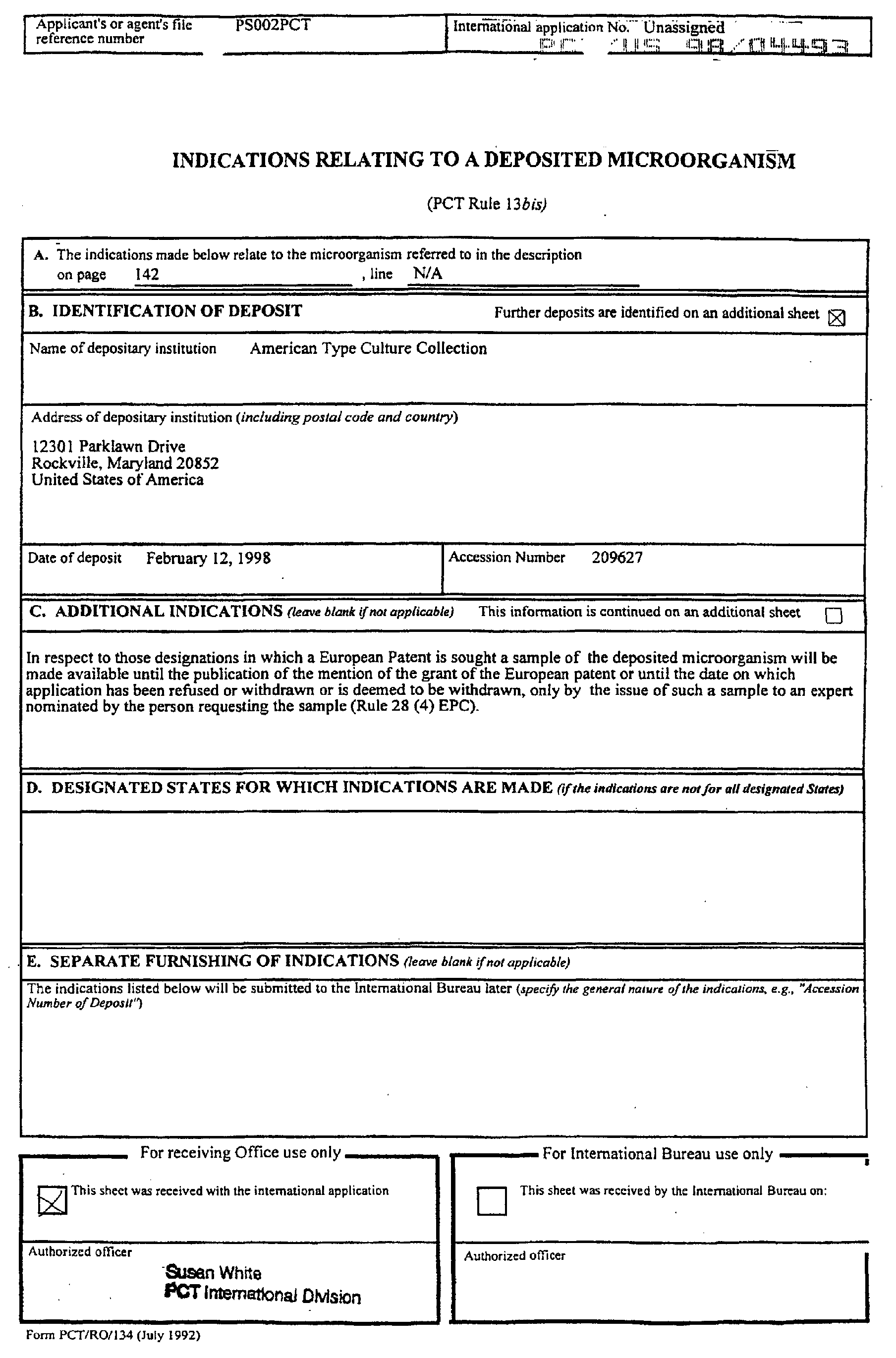

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

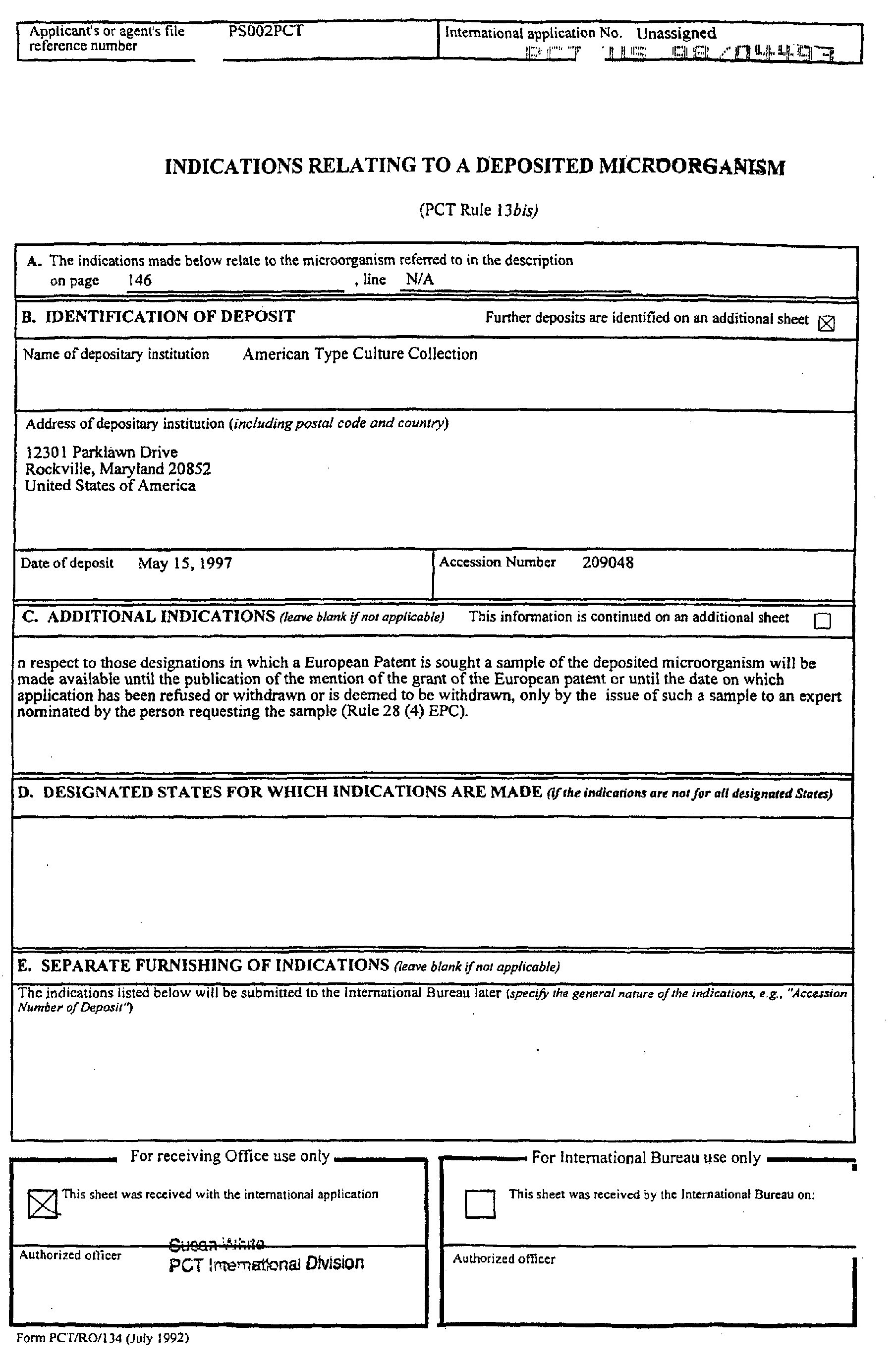

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

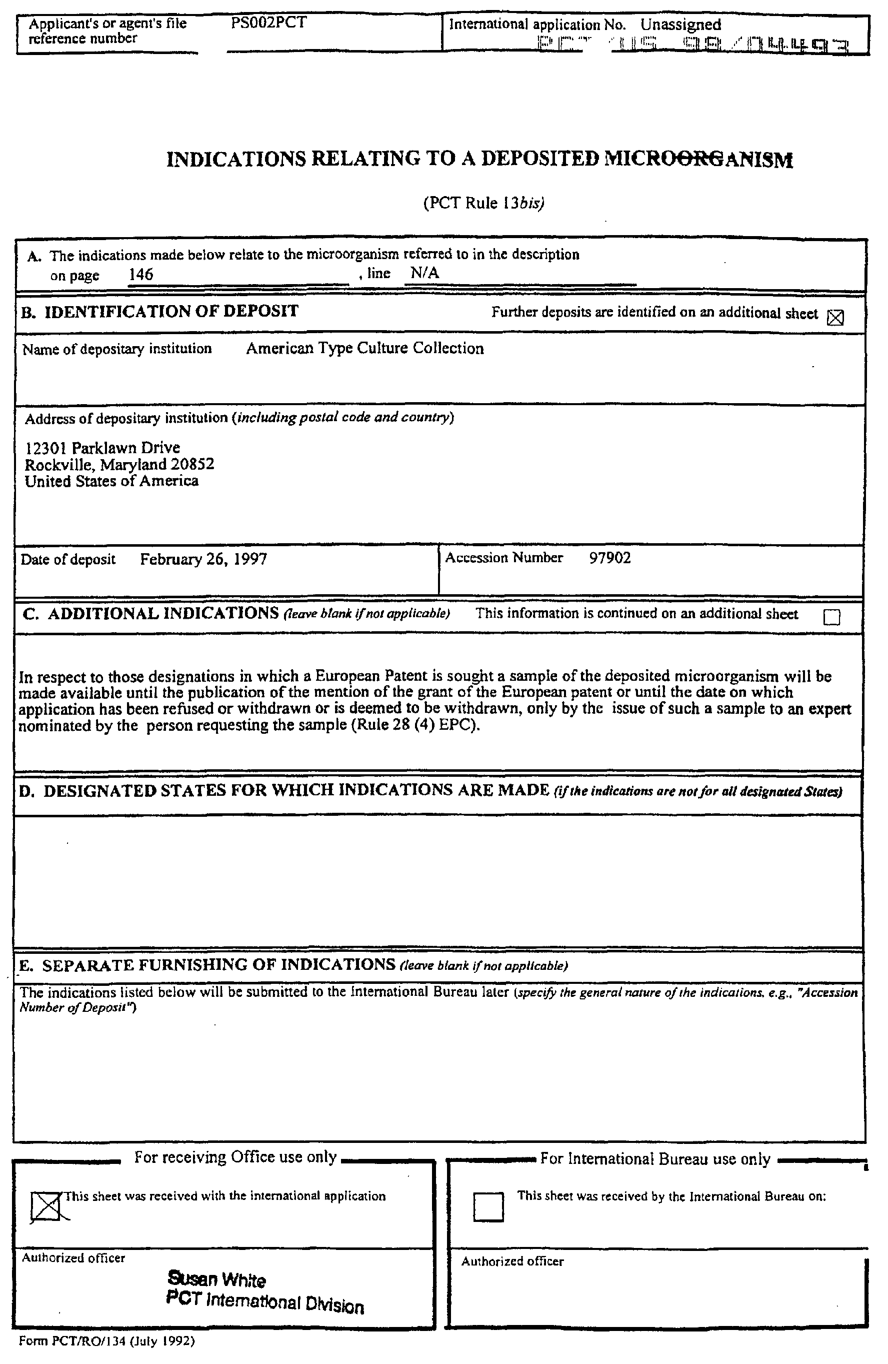

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

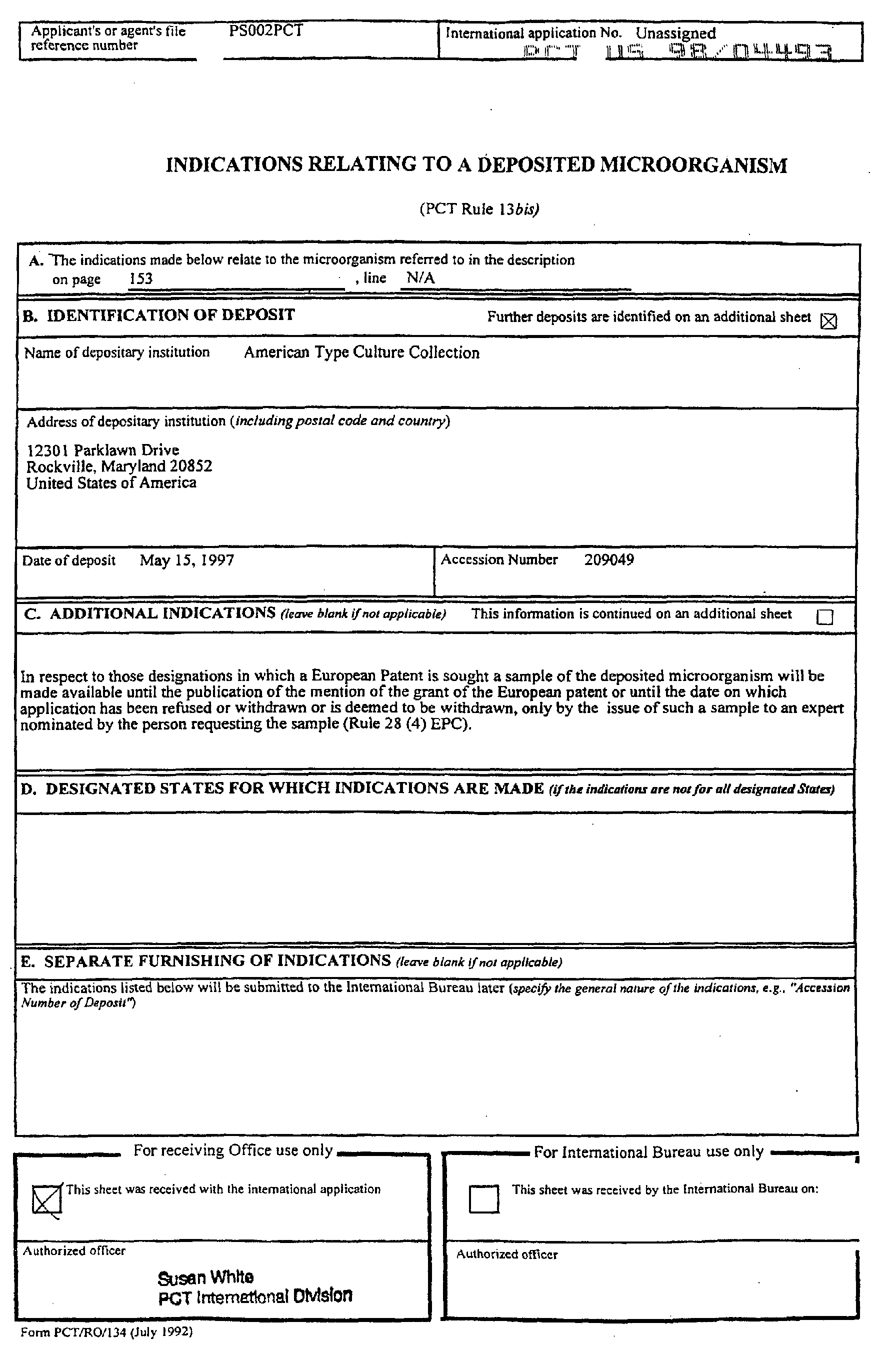

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

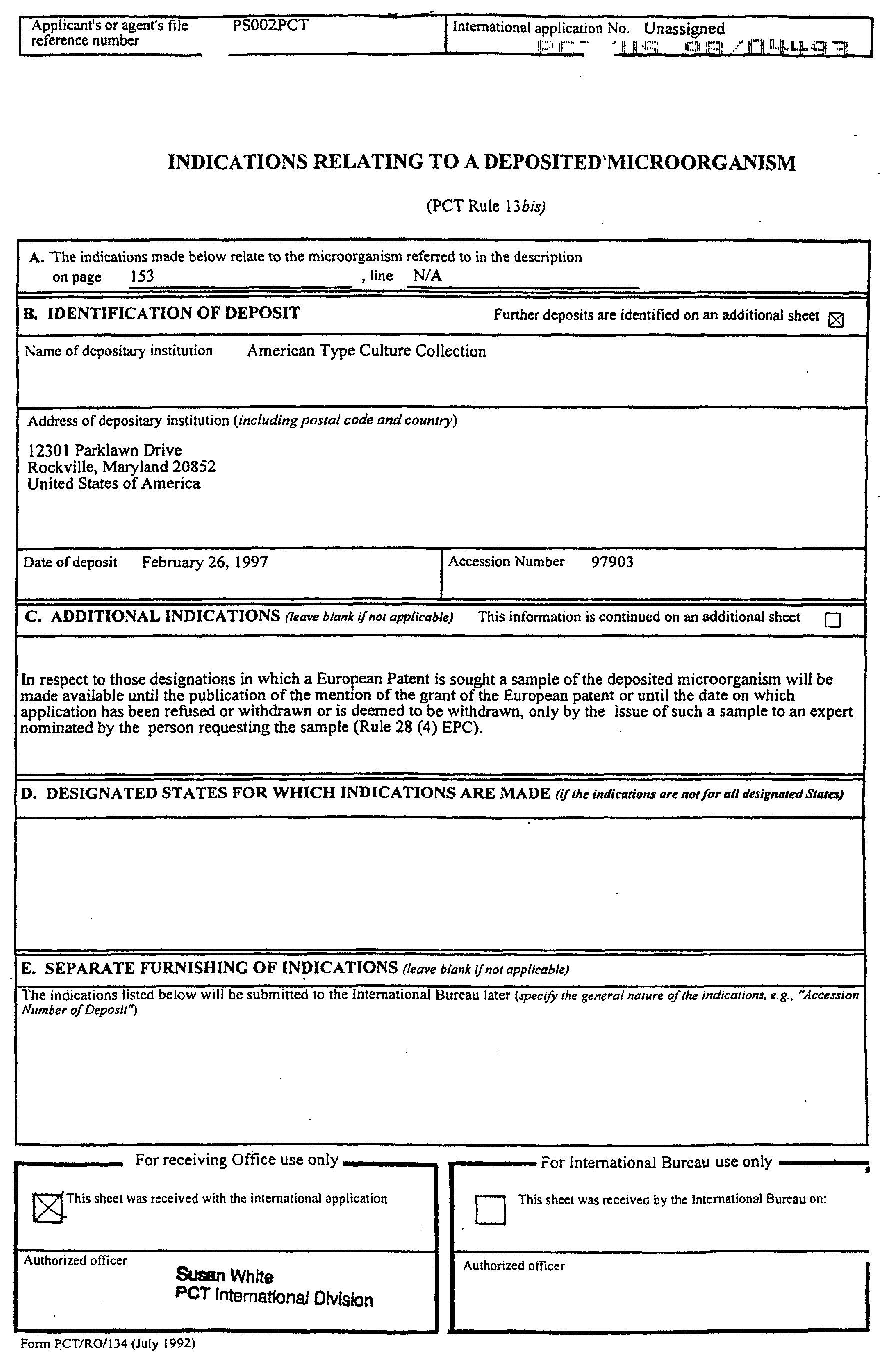

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

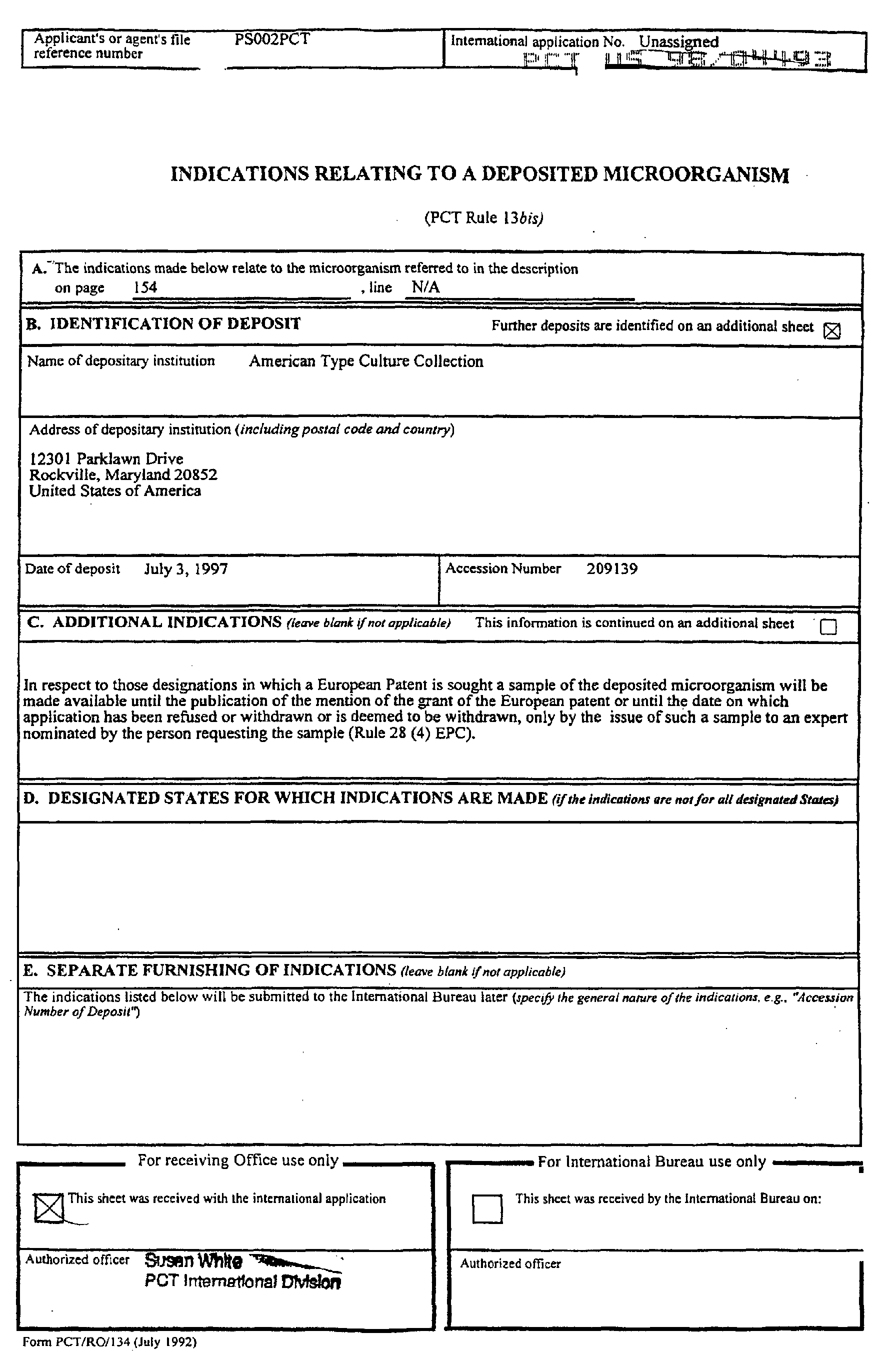

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

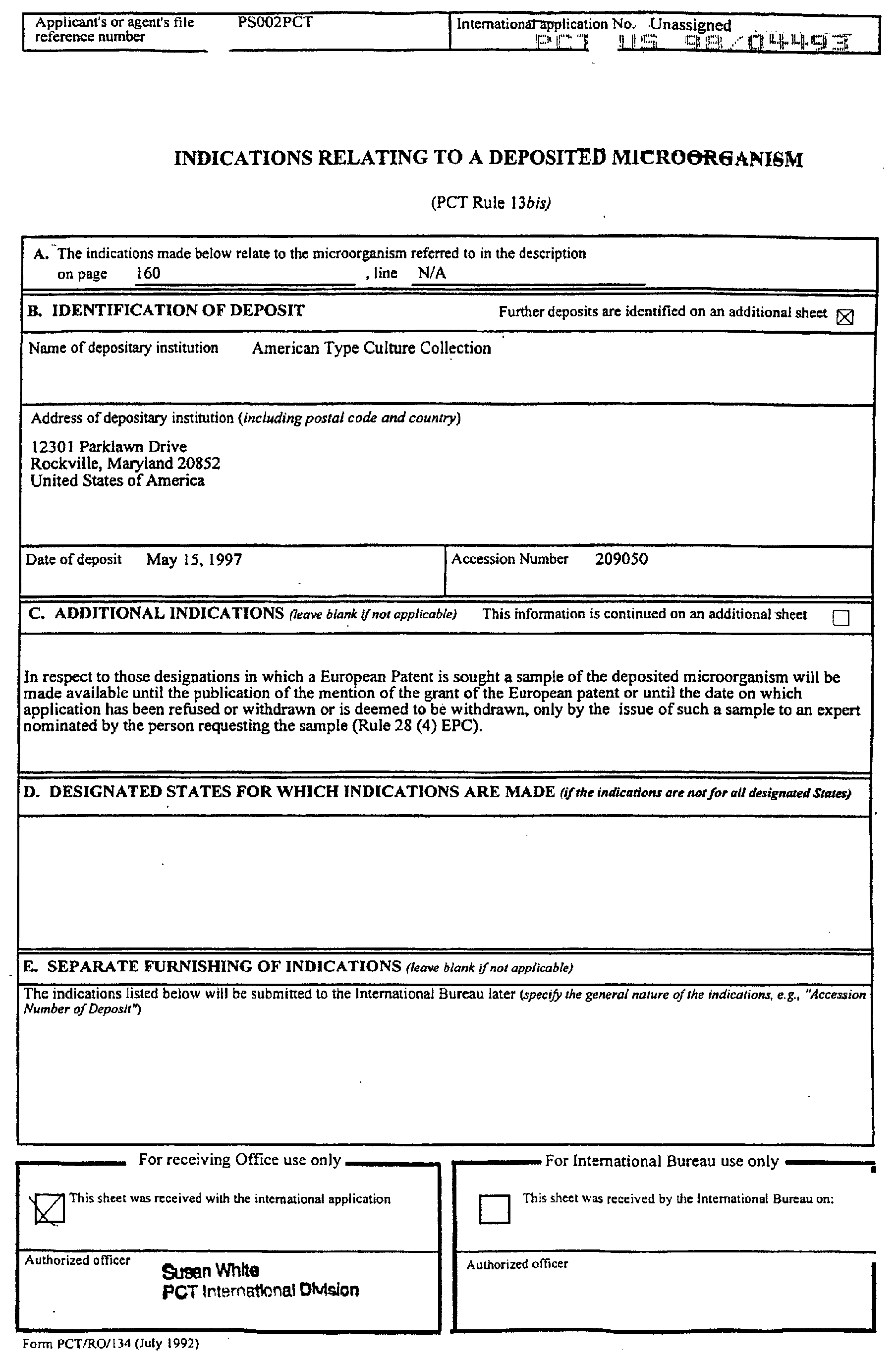

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

## Claims

1. A nucleic acid molecule comprising a polynucleotide having a nucleotide sequence selected from the group consisting of:
(a) a polynucleotide having the entire nucleotide sequence of SEQ ID NO: 169
(b) a polynucleotide having the entire cDNA sequence included in ATCC Deposit No. 97903, which is hybridizable to SEQ ID NO: 169;
(c) a polynucleotide fragment of SEQ ID NO: 169;
(d) a polynucleotide fragment of the cDNA sequence included in ATCC Deposit No. 97903, which is hybridizable to SEQ ID NO: 169;
(e) a polynucleotide encoding a polypeptide fragment of SEQ ID NO: 471;
(f) a polynucleotide encoding a polypeptide fragment encoded by the cDNA sequence included in ATCC Deposit No. 97903, which is hybridizable to SEQ ID NO: 169;
(g) a polynucleotide encoding a polypeptide fragment of SEQ ID NO: 471 or a polypeptide fragment encoded by the cDNA sequence included in ATCC Deposit No. 97903, having biological activity;
(h) a polynucleotide encoding a polypeptide fragment of SEQ ID NO: 471 or a polypeptide fragment encoded by the cDNA sequence included in ATCC Deposit No. 97903, which is secreted;
(i) a polynucleotide encoding a full length polypeptide of SEQ ID NO: 471 or being encoded by the cDNA sequence included in ATCC Deposit No. 97903;
(j) a polynucleotide encoding a polypeptide domain of SEQ ID NO: 471;
(k) a polynucleotide encoding a polypeptide domain encoded by the cDNA sequence included in ATCC Deposit No. 97903, which is hybridizable to SEQ ID NO: 169;
(l) a polynucleotide encoding a polypeptide epitope of SEQ ED NO: 471;
(m) a polynucleotide encoding a polypeptide epitope encoded by the cDNA sequence included in ATCC Deposit No. 97903, which is hybridizable to SEQ ID NO: 169;
(n) a polynucleotide which is a variant of SEQ ID NO: 169;
(o) a polynucleotide encoding a variant of SEQ ID NO: 471;
(p) a polynucleotide which is an allelic variant of SEQ ID NO: 169;
(q) a polynucleotide encoding an allelic variant of SEQ ID NO: 471;
(r) a polynucleotide which encodes a species homologue of the SEQ ID NO: 471;
(s) a polynucleotide which is at least 90% identical to a polynucleotide as defined in any one of (a) to (r);
(t) a polynucleotide encoding a polypeptide having an amino acid sequence at least 90% identical to the amino acid sequence of a polypeptide encoded by a polynucleotide of any one of (a) to (s); and
(u) a polynucleotide capable of hybridizing under stringent conditions to any one of the polynucleotides specified in (a) to (t), wherein said polynucleotide does not hybridize under stringent conditions to a nucleic acid molecule having a nucleotide sequence of only A residues or of only T residues;
or the complementary strand of polynucleotide (a) to (u).

2. The nucleic acid molecule of claim 1, wherein the nucleotide sequence comprises sequential nucleotide deletions from either the 5 prime terminus or the 3 prime terminus.

3. The nucleic acid molecule of claim 1 or 2 which is DNA or RNA.

4. The gene corresponding to the cDNA sequence of SEQ ID NO: 169 or encoding the polypeptide having the amino acid sequence of SEQ ID NO: 471.

5. A vector comprising the nucleic acid molecule of any one of claims 1 to 3.

6. A method of making a recombinant host cell comprising introducing the nucleic acid molecule of any one of claims 1 to 3, or the vector of claim 5.

7. A recombinant host cell containing the nucleic acid molecule of claims 1 to 3 or the vector of claim 5 or produced by the method of claim 6.

8. The recombinant host cell of claim 7 which expresses a polypeptide encoded by the nucleic acid molecule of any one of claims 1 to 3.

9. A method of making a polypeptide encoded by the nucleic acid molecule of any one of claims 1 to 3 comprising:
(a) culturing the recombinant host cell of claim 8 under conditions such that said polypeptide is expressed; and
(b) recovering said polypeptide.

10. A polypeptide encoded by the nucleic acid molecule of any one of claims 1 to 3 or obtainable by the method of claim 9.

11. A polypeptide comprising a polypeptide selected from the group consisting of:
(a) a polypeptide fragment of SEQ ID NO: 471;
(b) a polypeptide fragment of SEQ ID NO: 471 having biological activity;
(c) a polypeptide fragment of SEQ ID NO: 471, which is secreted;
(d) a full length polypeptide of SEQ ID NO: 471;
(e) a polypeptide domain of SEQ ID NO: 471;
(f) a polypeptide epitope of SEQ ID NO: 471;
(g) a polypeptide variant of SEQ ID NO: 471;
(h) an allelic variant of SEQ ID NO: 471;
(i) a species homologue of SEQ ID NO: 471; and
(j) a polypeptide which is at least 90% identical to a polypeptide as defined in any one of (a) to (i).

12. An antibody that binds specifically to the polypeptide of claim 10 or 11.

13. An antagonist of the polypeptide of claim 10 or 11 comprising a molecule closely related to the natural ligand of said polypeptide or a polypeptide related to the natural receptor of the polypeptide of claim 10 or 11 or a fragment thereof.

14. An agonist of the polypeptide of claim 10 or 11 comprising a molecule closely related to the natural ligand of said polypeptide or a polypeptide related to the natural receptor of the polypeptide of claim 10 or 11 or a fragment thereof.

15. A method for identifying antagonists or agonists of the polypeptide of claim 10 or 11 comprising:
(a) producing cells which express said polypeptide either as a secreted protein or on the cell membrane;
(b) contacting the polypeptide produced in step (a) with a test sample potentially containing an antagonist or agonist; and
(c) identifying an antagonist or agonist by observing binding and inhibition or stimulation of activity of said polypeptide.

16. An in vitro method of diagnosing a pathological condition or a susceptibility to a pathological condition in a subject comprising:
(a) determining the presence or absence of a mutation in the nucleic acid molecule of any one of claims 1 to 3 or the gene of claim 4; and
(b) diagnosing a pathological condition or a susceptibility to a pathological condition based on the presence or absence of said mutation.

17. An in vitro method of diagnosing a pathological condition or a susceptibility to a pathological condition in a subject comprising:
(a) determining the presence or amount of expression of the polypeptide of claim 10 or 11 in a biological sample; and
(b) diagnosing a pathological condition or a susceptibility to a pathological condition based on the presence , or amount of expression of the polypeptide.

18. A method for identifying a binding partner to the polypeptide of claim 10 or 11 comprising:
(a) contacting the polypeptide of claim 10 or 11 with a binding partner; and
(b) determining whether the binding partner effects an activity of the polypeptide.

19. A method of identifying an activity in a biological assay, wherein the method comprises:
(a) expressing the nucleic acid molecule of any one of claims 1 to 3 in a cell;
(b) isolating the supernatant;
(c) detecting an activity in a biological assay; and
(d) identifying the protein in the supernatant having the activity.

20. The product produced by the method of claim 19 or identified by the method of claim 15 or 18.

21. A pharmaceutical composition comprising the nucleic acid molecule of any one of claims 1 to 3, the polypeptide of claim 10 or 11, a DNA encoding and capable of expressing said polypeptide in vivo, the antibody of claim 12, the antagonist of claim 13 or the agonist of claim 14, or the product of claim 20 and optionally a pharmaceutically acceptable carrier.

22. Use of the nucleic acid molecule of any one of claims 1 to 3, the polypeptide of claim 10 or 11, the antibody of claim 12, the antagonist of claim 13 or the agonist of claim 14, or the product of claim 20 or a DNA encoding and capable of expressing said polypeptide in vivo for the preparation of a pharmaceutical composition for preventing, treating, or ameliorating a medical condition.

23. A diagnostic composition comprising the nucleic acid molecule of any one of claims 1 to 3 or the antibody of claim 12.

24. A method for the production of a pharmaceutical composition comprising the steps of the method of claim 15, 18 or 19 and the further step of formulating the compound identified in a pharmaceutically acceptable form.

25. The polypeptide of claim 11, wherein the polypeptide is post-translationally modified, fused to a heterologous polypeptide, or coupled to polyethylene glycol.
